# EUROPEAN PATENT SPECIFICATION

(11) **EP 2 634 265 B1**
(45) Date of publication and mention of the grant of the patent: **14.09.2016**
(21) Application number: 13154539.4
(22) Date of filing: 23.07.2007
(51) Int. Cl.: C12Q 1/68, G01N 33/574

(54) **Methods related to the gene GLI3 for detection of colorectal cancer**
Mit dem Gen GLI3 assozierte Verfahren für die Detektion von kolorektalem Karzinom
Procédés associes au gène GLI3 pour la détection du cancer du côlon

(30) Priority: 21.07.2006 US 832509 P; 20.10.2006 US 853097 P
(43) Date of publication of application: 04.09.2013
(62) Divisional of application: 07813223.0
(73) Proprietor: Epigenomics AG, 10829 Berlin (DE)
(72) Inventor: Lofton-Day, Catherine, Seattle, WA 98103 (US); Sledziewski, Andrew, Shoreline, WA 98177 (US); Model, Fabian, 12683 Berlin (DE); Cottrell, Susan, Seattle, WA 98115 (US); Distler, Jürgen, 12163 Berlin (DE); Tetzner, Reimo, 10439 Berlin (DE); Dietrich, Dimo, 10437 Berlin (DE)
(74) Representative: Zwicker, Jörk

(56) References cited:
- WO-A2-01/68912
- WO-A2-02/00927
- WO-A2-2005/013800
- WO-A2-2006/008128
- WO-A2-2008/122447
- Y ZHU: "Functional Smoothened is required for expression of GLI3 in colorectal carcinoma cells", CANCER LETTERS, vol. 207, no. 2, 1 April 2004 (2004-04-01) , pages 205-214, XP055072070, ISSN: 0304-3835, DOI: 10.1016/j.canlet.2003.10.025
- DATABASE Geneseq [Online] 26 July 2000 (2000-07-26), "Human genomic SNP allele specific oligonucleotide SEQ ID NO:410.", XP002704929, retrieved from EBI accession no. GSN:AAA36376 Database accession no. AAA36376
- DATABASE Geneseq [Online] 4 August 1995 (1995-08-04), "Reverse transcription primer used in cDNA analysis technique.", XP002704930, retrieved from EBI accession no. GSN:AAQ75683 Database accession no. AAQ75683
- DATABASE Geneseq [Online] 4 August 1995 (1995-08-04), "Reverse transcription primer used in cDNA analysis technique.", XP002704931, retrieved from EBI accession no. GSN:AAQ75679 Database accession no. AAQ75679
- HESSON L B ET AL: "CpG island promoter hypermethylation of a novel Ras-effector gene RASSF2A is an arly event in colon carcinogenesis and correlates inversely with K-ras mutations", ONCOGENE, NATURE PUBLISHING GROUP, GB, vol. 24, 4 April 2005 (2005-04-04), pages 3987-3994, XP003013793, ISSN: 0950-9232, DOI: 10.1038/SJ.ONC.1208566
- LI-HUI WANG ET AL: "Increased expression of sonic hedgehog and altered methylation of its promoter region in gastric cancer and its related lesions", MODERN PATHOLOGY, vol. 19, no. 5, 1 May 2006 (2006-05-01), pages 675-683, XP055072090, ISSN: 0893-3952, DOI: 10.1038/modpathol.3800573
- M. MICHAEL COHEN,: "The hedgehog signaling network", AMERICAN JOURNAL OF MEDICAL GENETICS, vol. 123A, no. 1, 15 November 2003 (2003-11-15), pages 5-28, XP055072126, ISSN: 0148-7299, DOI: 10.1002/ajmg.a.20495
- ANCA ONISCU ET AL: "Expression of Sonic hedgehog pathway genes is altered in colonic neoplasia", THE JOURNAL OF PATHOLOGY, vol. 203, no. 4, 1 August 2004 (2004-08-01), pages 909-917, XP055072124, ISSN: 0022-3417, DOI: 10.1002/path.1591
- KIM JAE H ET AL: "Gli3 mediates sonic hedgehog dependent development of the glandular stomach", GASTROENTEROLOGY, vol. 128, no. 4, Suppl. 2, April 2005 (2005-04), page A139, XP002704932, & ANNUAL MEETING OF THE AMERICAN-GASTROENTEROLOGICAL-ASSOCIATION/D IGESTIVE-DISEASE-WEEK; CHICAGO, IL, USA; MAY 14 -19, 2005 ISSN: 0016-5085
- VAN DEN BRINK G R ET AL: "Hedgehog Wnteraction in colorectal cancer", GUT, vol. 55, no. 7, July 2006 (2006-07), pages 912-914, XP009171357, ISSN: 0017-5749
- Susan J Clark ET AL: "DNA methylation and gene silencing in cancer: which is the guilty party?", Oncogene, vol. 21, 1 January 2002 (2002-01-01), pages 5380-5387, XP055174183, DOI: 10.1038/sj.onc
- AHUJA N ET AL: "AGING AND DNA METHYLATION IN COLORECTAL MUCOSA AND CANCER", CANCER RESEARCH, AMERICAN ASSOCIATION FOR CANCER RESEARCH, US, vol. 58, no. 23, 1 December 1998 (1998-12-01), pages 5489-5494, XP001026172, ISSN: 0008-5472

## Description

### FIELD OF THE INVENTION

The present invention relates to genomic DNA sequences that exhibit altered expression patterns in disease states relative to normal. The methods for the detection and diagnosis of cell proliferative disorders as provided in the present invention, are used for the diagnosis of colorectal cancer.

### BACKGROUND

*Incidence and diagnosis of cancer.*

Cancer is the second leading cause of death of the United States. Mortality rates could be significantly improved if current screening methods would be improved in terms of patient compliance, sensitivity and ease of screening. Current recommended methods for diagnosis of cancer are often expensive and are not suitable for application as population wide screening tests.

In the United States the annual incidence of colorectal cancer is approximately 150,000, with 56,600 individuals dying from colorectal cancer each year. The lifetime risk of colorectal cancer in the general population is about 5 to 6 percent. Despite intensive efforts in recent years in screening and early detection of colon cancer, until today most cases are diagnosed in an advanced stage with regional or distant metastasis. While the therapeutic options include surgery and adjuvant or palliative chemotherapy, most patients die from progression of their cancer within a few months. Identifying the molecular changes that underlie the development of colon cancer may help to develop new monitoring, screening, diagnostic and therapeutic options that could improve the overall poor prognosis of these patients.

The current guidelines for colorectal screening according to the American Cancer Society utilizes one of five different options for screening in average risk individuals 50 years of age or older. These options include 1) fecal occult blood test (FOBT) annually, 2) flexible sigmoidoscopy every five years, 3) annual FPBT plus flexible sigmoidoscopy every five years, 4) double contrast barium enema (DCBE) every five years or 5) colonoscopy every ten years. Even though these testing procedures are well accepted by the medical community, the implementation of widespread screening for colorectal cancer has not been realized. Patient compliance is a major factor for limited use due to the discomfort or inconvenience associated with the procedures. FOBT testing, although a non-invasive procedure, requires dietary and other restrictions 3-5 days prior to testing. Sensitivity levels for this test are also very low for colorectal adenocarcinoma with wide variability depending on the trial. Sensitivity measurements for detection of adenomas is even less since most adenomas do not bleed. In contrast, sensitivity for more invasive procedures such as sigmoidoscopy and colonoscopy are quite high because of direct visualization of the lumen of the colon. No randomized trials have evaluated the efficacy of these techniques, however, using data from case-control studies and data from the National Polyp Study (U.S.) it has been shown that removal of adenomatous polyps results in a 76-90% reduction in CRC incidence. Sigmoidoscopy has the limitation of only visualizing the left side of the colon leaving lesions in the right colon undetected. Both scoping procedures are expensive, require cathartic preparation and have increased risk of morbidity and mortality. Improved tests with increased sensitivity, specificity, ease of use and decreased costs are clearly needed before general widespread screening for colorectal cancer becomes routine.

Early colorectal cancer detection is generally based on the fecal occult blood test (FOBT) performed annually on asymptomatic individuals. Current recommendations adapted by several healthcare organizations, including the American Cancer Society, call for fecal occult blood testing beginning at age 50, repeated annually until such time as the patient would no longer benefit from screening. A positive FOBT leads to colonoscopic examination of the bowel; an expensive and invasive procedure, with a serious complication rate of one per 5,000 examinations. Only 12% of patients with heme positive stool are diagnosed with cancer or large polyps at the time of colonoscopy. A number of studies show that FOBT screening does not improve cancer-related mortality or overall survival. Compliance with occult blood testing has been poor; less than 20 percent of the population is offered or completes FOBT as recommended. If FOBT is properly done, the patient collects a fecal sample from three consecutive bowel movements. Samples are obtained while the patient adheres to dietary guidelines and avoids medications known to induce occult gastrointestinal bleeding. In reality, physicians frequently fail to instruct patients properly, patients frequently fail to adhere to protocol, and some patients find the task of collecting fecal samples difficult or unpleasant, hence compliance with annual occult blood testing is poor. If testing sensitivity and specificity can be improved over current methods, the frequency of testing could be reduced, collection of consecutive samples would be eliminated, dietary and medication schedule modifications would be eliminated, and patient compliance would be enhanced. Compounding the problem of compliance, the sensitivity and specificity of FOBT to detect colon cancer is poor. Poor test specificity leads to unnecessary colonoscopy, adding considerable expense to colon cancer screening.

Specificity of the FOBT has been calculated at best to be 96%, with a sensitivity of 43% (adenomas) and 50% (colorectal carcinoma). Sensitivity can be improved using an immunoassay FOBT such as that produced under the tradename 'InSure^{™}', with an improved sensitivity of 77 % (adenomas) and 88.9% (colorectal carcinoma.

*Molecular disease markers.* Molecular disease markers offer several advantages over other types of markers, one advantage being that even samples of very small sizes and/or samples whose tissue architecture has not been maintained can be analyzed quite efficiently. Within the last decade a number of genes have been shown to be differentially expressed between normal and colon carcinomas. However, no single or combination of marker has been shown to be sufficient for the diagnosis of colon carcinomas. High-dimensional mRNA based approaches have recently been shown to be able to provide a better means to distinguish between different tumor types and benign and malignant lesions. However its application as a routine diagnostic tool in a clinical environment is impeded by the extreme instability of mRNA, the rapidly occurring expression changes following certain triggers (*e.g*., sample collection), and, most importantly, the large amount of mRNA needed for analysis (Lipshutz, R. J. et al., Nature Genetics 21:20-24, 1999; Bowtell, D. D. L Nature genetics suppl. 21:25-32, 1999), which often cannot be obtained from a routine biopsy.

The use of biological markers to further improve sensitivity and specificity of FOBT has been suggested, examples of such tests include the PreGen-Plus™ stool analysis assay available from EXACT Sciences which has a sensitivity of 20% (adenoma) and 52% (colorectal carcinoma) and a specificity of 95% in both cases. This test assays for the presence of 23 DNA mutations associated with the development of colon neoplasms. The use of DNA methylation as colon cancer markers is known. For example Sabbioni et al. (Molecular Diagnosis 7:201-207, 2003) detected hypermethylation of a panel of genes consisiting TPEF, HIC1, DAPK and MGMT in peripheral blood in 98% of colon carcinoma patients. However, this does provide a suitable basis for a commercially marketable test, as the specificity of such a test must also be sufficiently high.

The current model of colorectal pathogenesis favours a stepwise progression of adenomas, which includes the development of dysplasia and finally signs of invasive cancer. The molecular changes underlying this adenoma-carcinoma sequence include genetic and epigenetic alterations of tumor suppressor genes (APC, p53, DCC), the activation of oncogenes (K-ras) and the inactivation of DNA mismatch repair genes. Recently, further molecular changes and genetic defects have been revealed. Thus, activation of the Wnt signalling pathway not only includes mutations of the APC gene, but may also result from β-catenin mutations. Furthermore, alterations in the TGF-β signalling pathway together with its signal transducers SMAD4 and SMAD2 have been linked to the development of colon cancer.

Despite recent progress in the understanding of the pathogenesis of adenomas and carcinomas of the colon and their genetic and molecular changes, the genetic and epigenetic changes underlying the development of metastasis are less well understood. It is, however, generally well accepted that the process of invasion and proteolysis of the extracellular matrix, as well as infiltration of the vascular basement membrane involve adhesive proteins, such as members of the family of integrin receptors, the cadherins, the immunoglobulin superfamily, the laminin binding protein and the CD44 receptor. Apart from adhesion, the process of metastasis formation also includes the induction and regulation of angiogenesis (VEGF, bFGF), the induction of cell proliferation (EGF, HGF, IGF) and the activation of proteolytic enzymes (MMPs, TIMPs, uPAR), as well as the inhibition of apoptosis (Bcl-2, Bcl-X). More recently other groups have compared the genetic and molecular changes in metastatic lesions to the changes found in primary colorectal cancers. Thus, Kleeff et al. reported the loss of DOC-2, a candidate tumor suppressor gene, both in primary and metastatic colorectal cancer. Furthermore, Zauber et al. reported that in their series of 42 colorectal cancers Ki-ras mutations in the primary cancers were identical in all of the 42 paired primary and synchronous metastatic lesions. Similarly loss of heterozygosity at the APC locus was identical for 39 paired carcinomas and synchronous metastasis. The authors concluded that for Ki-ras and APC genes the genetic changes in metastasis are identical to the primary colorectal cancer. However, other groups have found genetic and molecular changes in metastatic colon cancers, that are not present in the primary cancers. Thus, the development of LOH of chromosome 3p in colorectal metastasis has been reported.

*CpG island methylation.* Apart from mutations aberrant methylation of CpG islands has been shown to lead to the transcriptional silencing of certain genes that have been previously linked to the pathogenesis of various cancers. CpG islands are short sequences which are rich in CpG dinucleotides and can usually be found in the 5' region of approximately 50% of all human genes. Methylation of the cytosines in these islands leads to the loss of gene expression and has been reported in the inactivation of the X chromosome and genomic imprinting.

Recently several groups have also analysed the methylation of various genes in colorectal cancer and reported the transcriptional silencing by promoter methylation for p16INK4, p14ARF, p15INK4b, MGMT, hMLH1, GSTP1, DAPK, CDH1, TIMP-3 and APC among others. Thus apart from mutational inactivation of certain genes, the hypermethylation of these genes also contributes significantly to the pathogenesis of this disease.

In recent years several genes that are methylated in colon cancer have been identified by MS-APPCR. This group of genes, among others, includes TPEF/HPP1 which is frequently methylated in colon cancers and which was independently identified by two different groups using the MS-APPCR method (see, *e.g.,* Young J, Biden KG, Simms LA, Huggard P, Karamatic R, Eyre HJ, Sutherland GR, Herath N, Barker M, Anderson GJ, Fitzpatrick DR, Ramm GA, Jass JR, Leggett BA. HPP1: a transmembrane protein-encoding gene commonly methylated in colorectal polyps and cancers. Proc Natl Acad Scl USA 98:265-270, 2001).

*Multifactorial approach.* Cancer diagnostics has traditionally relied upon the detection of single molecular markers (e.g., gene mutations, elevated PSA levels). Unfortunately, cancer is a disease state in which single markers have typically failed to detect or differentiate many forms of the disease. Thus, assays that recognize only a single marker have been shown to be of limited predictive value. A fundamental aspect of this invention is that methylation-based cancer diagnostics and the screening, diagnosis, and therapeutic monitoring of such diseases will provide significant improvements over the state-of-the-art that uses single marker analyses by the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.
Key to the successful implementation of a panel approach to methylation based diagnostic tests is the design and development of optimized panels of markers that can characterize and distinguish disease states. The present disclosure describes a plurality of particularly efficient and unique panels of genes, the methylation analysis of one or a combination of the members of the panel enabling the detection of colon cell proliferative disorders with a particularly high sensitivity, specificity and/or predictive value.
*Development of medical tests.* Two key evaluative measures of any medical screening or diagnostic test are its sensitivity and specificity, which measure how well the test performs to accurately detect all affected individuals without exception, and without falsely including individuals who do not have the target disease (predicitive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present. In this context: Sensitivity = TP/(TP+FN); Specificity = TN/(FP+TN); and Predictive value = TP/(TP+FP).

Sensitivity is a measure of a test's ability to correctly detect the target disease in an individual being tested. A test having poor sensitivity produces a high rate of false negatives, *i.e*., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, *i.e.,* individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. n example of a test that has high specificity is a gene-based test that can detect a p53 mutation. Specificity is important when the cost or risk associated with further diagnostic procedures or further medical intervention are very high.

*Pronounced need in the art.* It is generally accepted that there is a pronounced need in the art for improved screening and early detection of cancers. As an example, if colon cancer screening specificity can be Increased, the problem of false positive test results leading to unnecessary colonoscopic examination would be reduced leading to cost savings and improved safety.

In view of the incidence of cancers in general and more particularly the disadvantages associated with current colorectal cell proliferative disorder screening methods there is a substantial need in the art for improved methods for the early detection of cancer, in particular colon cancer, to be used in addition to or as a substitute for currently available tests.

*Background of the genes of the present invention.* The human Septin 9 gene (also known as MLL septin-like fusion protein, MLL septin-like fusion protein MSF-A, Slpa, Eseptin, Msf, septin-like protein Ovarian/Breast septin (Ov/Br septin) and Septin D1) is located on chromosome 17q25 within contig AC068594.15.1.168501 and is a member of the Septin gene family. SEQ ID NO: 12 provides the sequence of said gene, comprising regions of both the Septin 9 and Q9HC74 transcripts and promoter regions. SEQ ID NO: 13 and SEQ ID NO: 14 provide particularly preferred CpG rich regions of said gene.
It has been postulated that members of the Septin gene family are associated with multiple cellular functions ranging from vesicle transport to cytokinesis. Disruption of the action of *Septin* 9 results in incomplete cell division, see Surka, M.C., Tsang, C.W., and Trimble, W.S. Mol Biol Cell, 13: 3532-45 (2002). *Septin* 9 and other proteins have been shown to be fusion partners of the proto-oncogene *MLL* suggesting a role in tumorogenesis, see Osaka, M, Rowley, J.D. and Zeleznik-Le, N.J. PNAS, 96:6428-6433 (1999). Burrows et al. reported an in depth study of expression of the multiple isoforms of the *Septin* 9 gene in ovarian cancer and showed tissue specific expression of various transcripts, see Burrows, J. F., Chanduloy, et al. S.E.H. Journal of Pathology, 201:581-588 (2003).
A recent study of over 7000 normal and tumor tissues indicates that there is consistent over-expression of *Septin* 9 isoforms in a number of tumor tissues, see Scott, M., Hyland, P.L., et al. Oncogene, 24: 4688-4700 (2005). The authors speculate that the gene is likely a type II cancer gene where changes in RNA transcript processing control regulation of different protein products, and the levels of these altered protein isoforms may provide answers to the gene's role in malignancy.

Zhu et al, Cancer Letters, vol.207 (2004) 205-214 discloses that GLI3 is not expressed in a number of colorectal carcinoma cell lines and is reexpressed after treatment with a demethylating agent and analyses the methylation status of the SMO promoter in colorectal carcinomas.

### BRIEF DESCRIPTION OF THE DRAWINGS

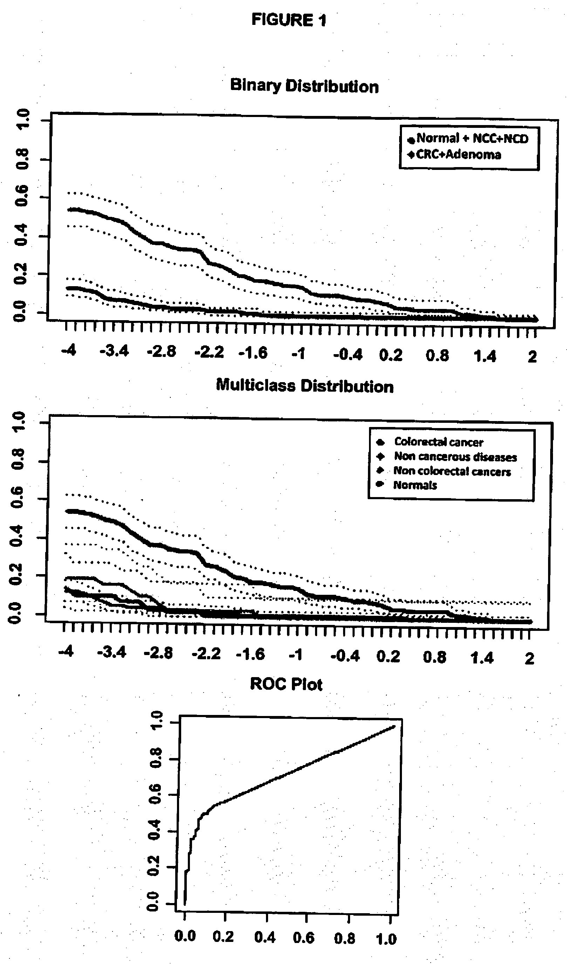

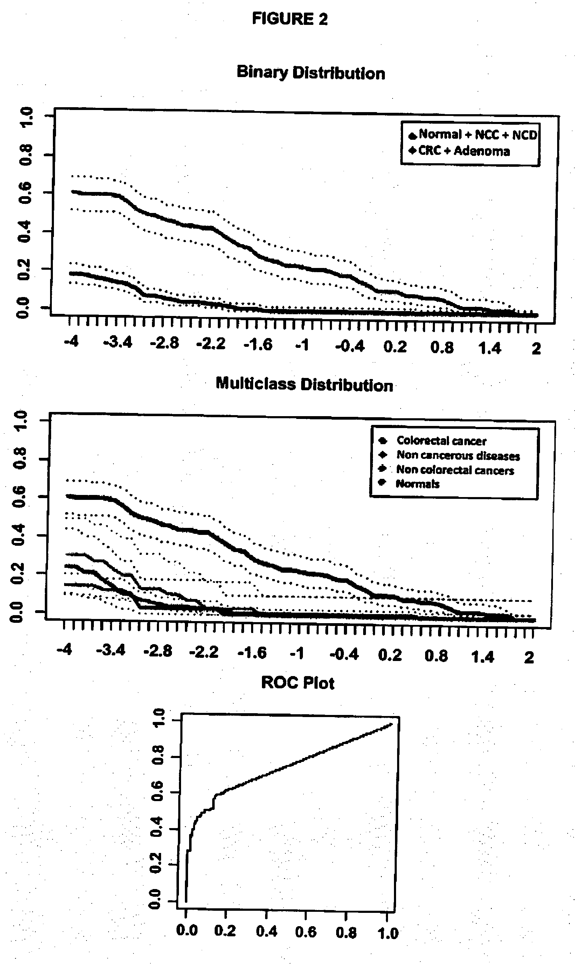

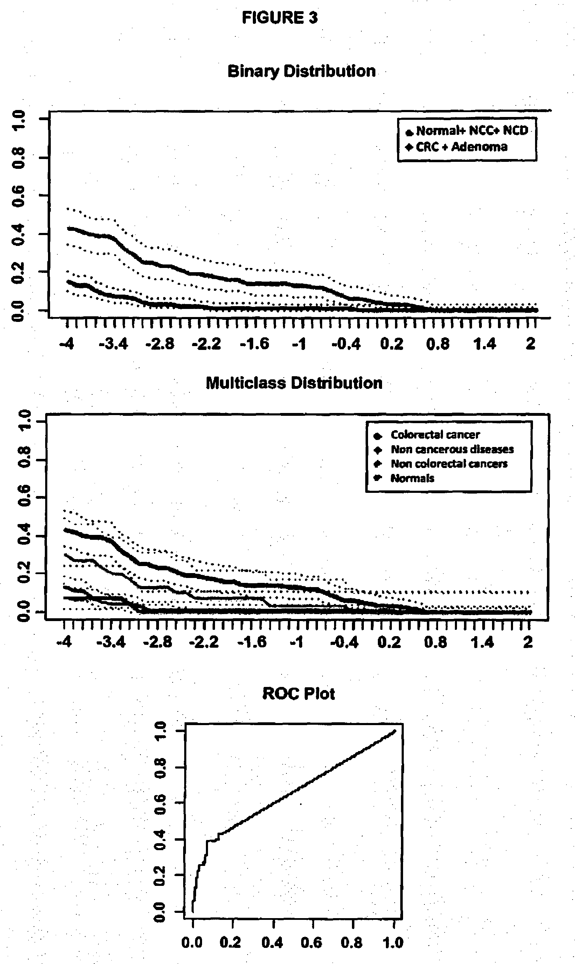

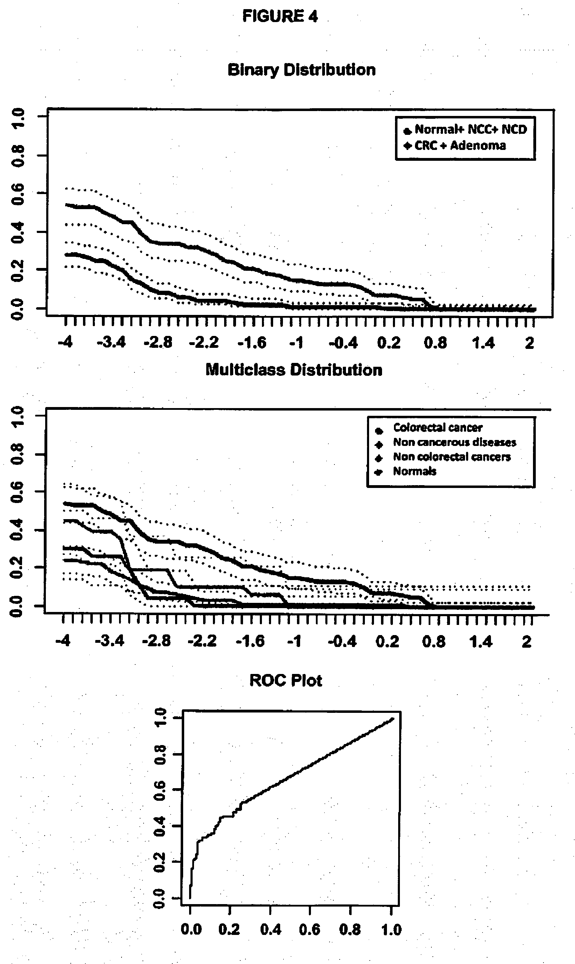

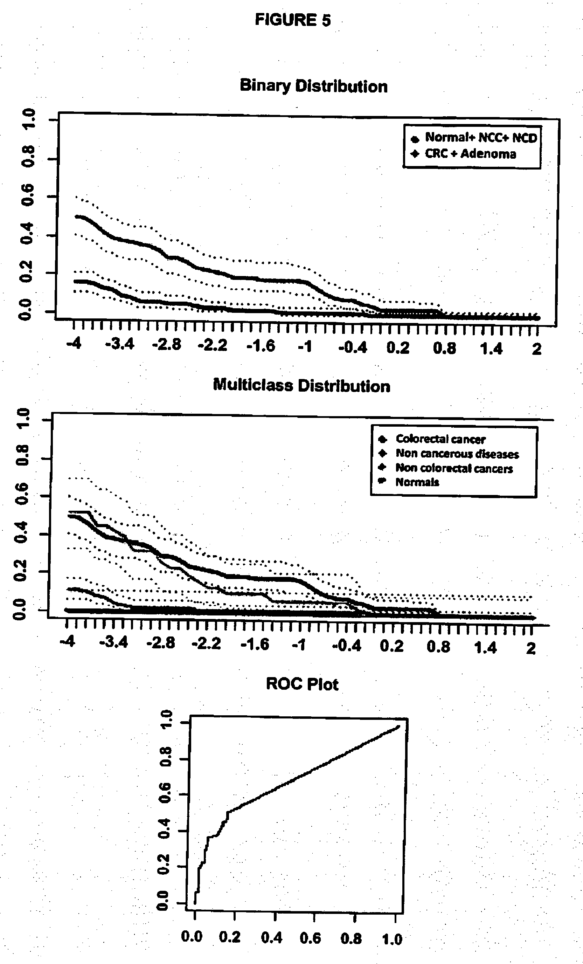

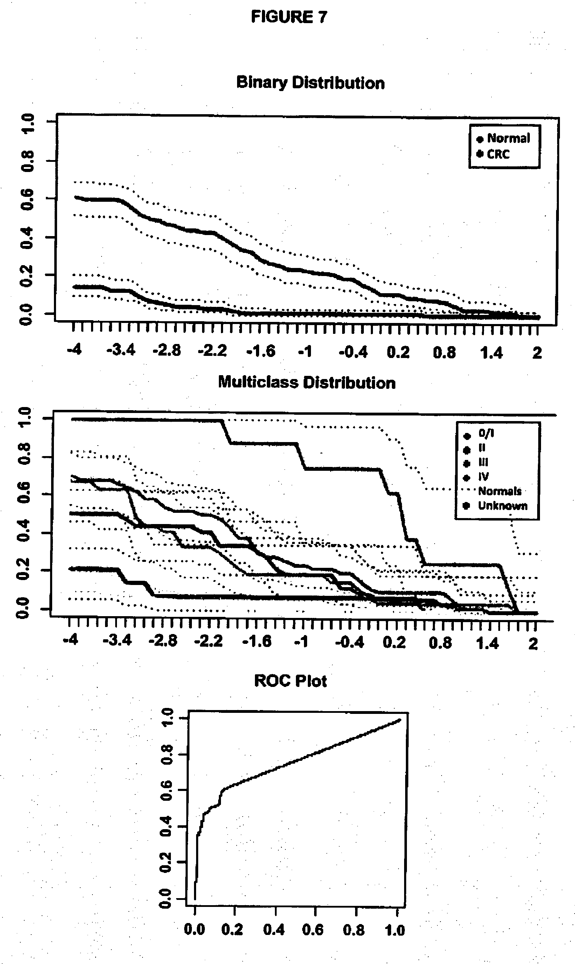

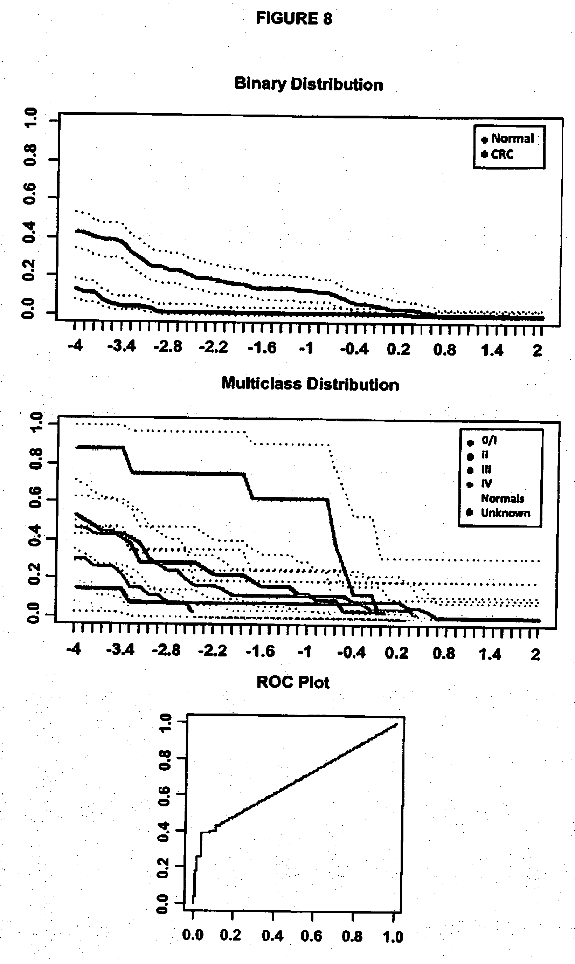

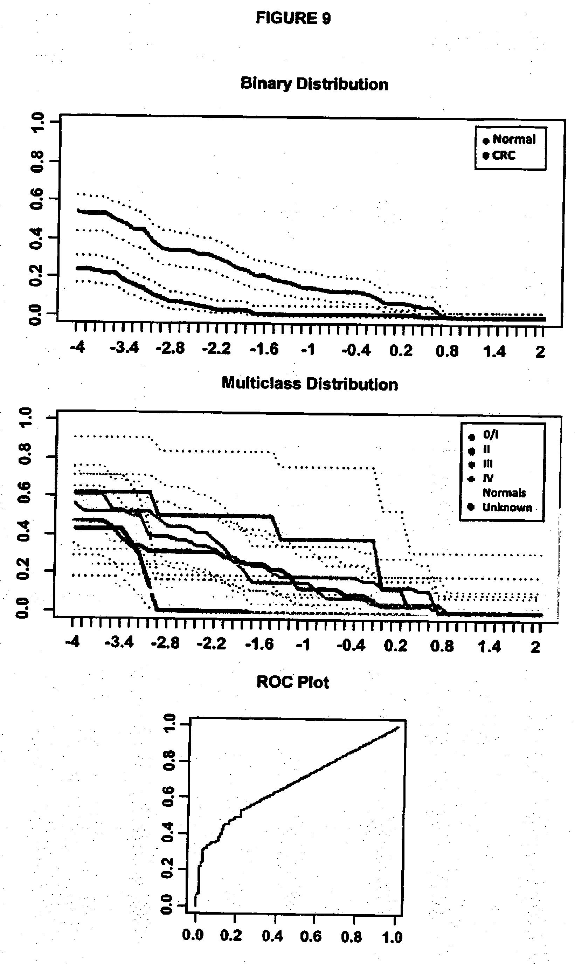

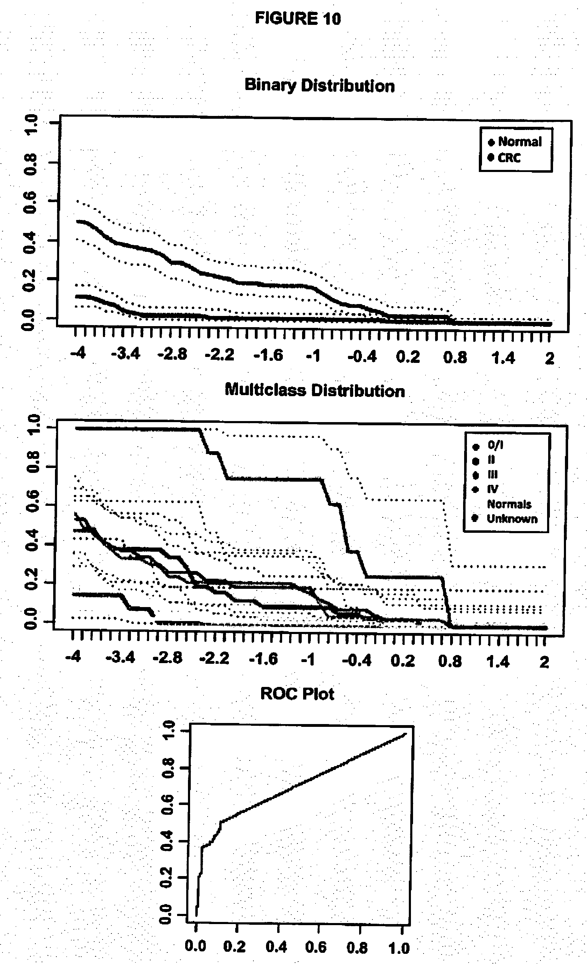

Figures 1 to 10 provide an overview of the log mean methylation measured by means of the HM assay according to Example 2. Each figures consists of three plots, the plots at the top and in the middle provide the binary and multiclass analysis respectively, sensitivity is shown on the Y-axis, DNA methylation measured in (log 10 ng/mL) is shown on the X-axis.

In each figure the plot at the bottom provides an ROC wherein sensitivity is shown on the Y-axis and 1-specificity is shown on the X-axis.
Figure 1 provides an overview of the performance of the RASSF2 HM assay according to Example 2, in all samples.
Figure 2 provides an overview of the performance of the Septin 9 HM assay according to Example 2, in all samples.
Figure 3 provides an overview of the performance of the SND1 HM assay according to Example 2, in all samples.
Figure 4 provides an overview of the performance of the PCDHGC3 HM assay according to Example 2, in all samples.
Figure 5 provides an overview of the performance of the TFAP2E HM assay according to Example 2, in all samples.
Figure 6 provides an overview of the performance of the RASSF2 HM assay according to Example 2, in all colorectal carcinoma and normal colorectal tissue samples.
Figure 7 provides an overview of the performance of the Septin 9 HM assay according to Example 2, in all colorectal carcinoma and normal colorectal tissue samples.
Figure 8 provides an overview of the performance of the SND1 HM assay according to Example 2, in all colorectal carcinoma and normal colorectal tissue samples.
Figure 9 provides an overview of the performance of the PCDHGC3 HM assay according to Example 2, in all colorectal carcinoma and normal colorectal tissue samples.
Figure 10 provides an overview of the performance of the TFAP2E HM assay according to Example 2, in all colorectal carcinoma and normal colorectal tissue samples.
Figure 11 provides an overview of the predictive power of the logistic regression model of combinations of markers. Se is sensitivitiy, sp is specificity, AUC is area under the curve.

Figures 12 to 21 provide an overview of the log majority mean methylation measured by means of the HM assay according to Example 2. Each figures consists of three plots, the plots at the top and in the middle provide the binary and multiclass analysis respectively, sensitivity is shown on the Y-axis, DNA methylation measured in (log 10 ng/mL) is shown on the X-axis.

In each figure the plot at the bottom provides an ROC wherein sensitivity is shown on the Y-axis and 1-specificity is shown on the X-axis.

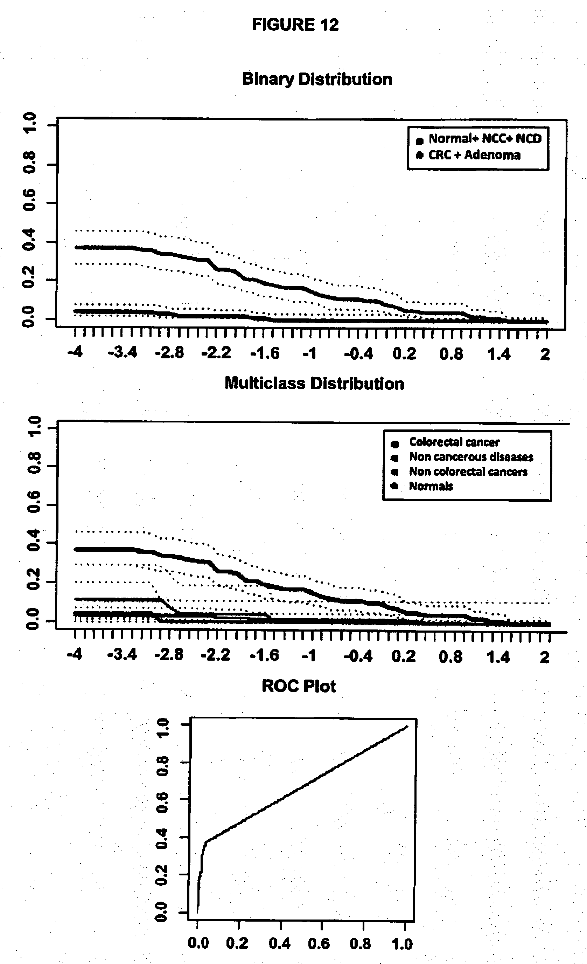

Figure 12 provides an overview of the performance of the RASSF2 HM assay according to Example 2, in all samples.

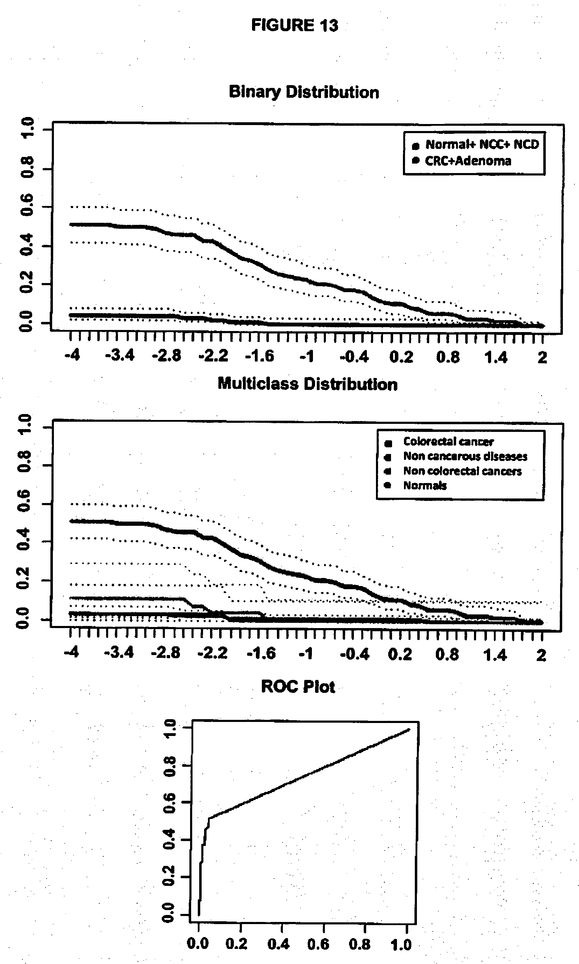

Figure 13 provides an overview of the performance of the Septin 9 HM assay according to Example 2, in all samples.

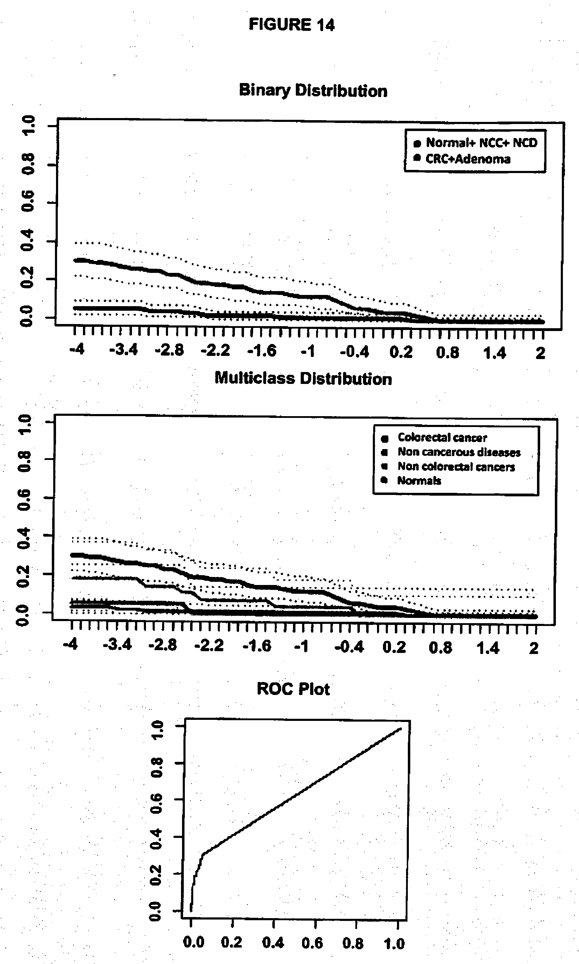

Figure 14 provides an overview of the performance of the SND1 HM assay according to Example 2, in all samples.

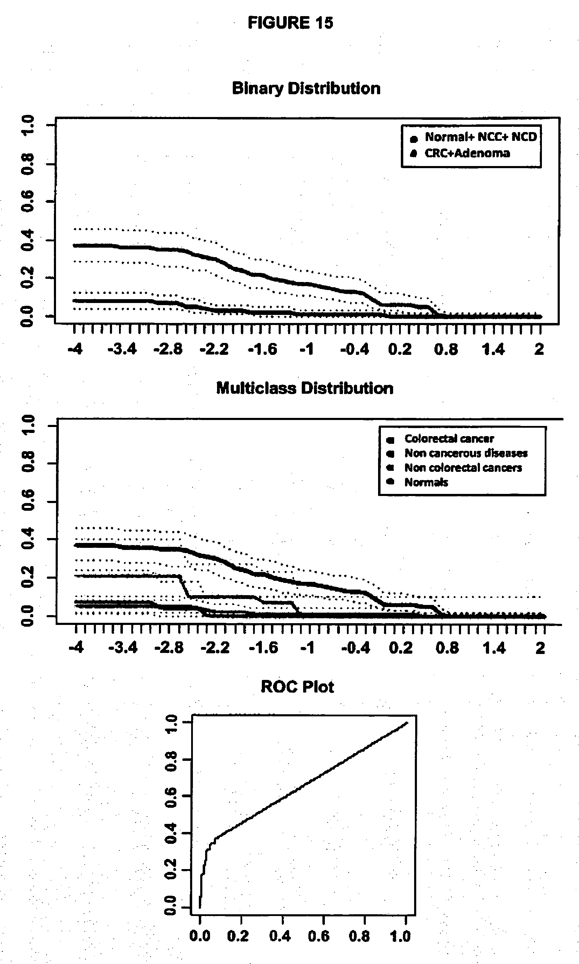

Figure 15 provides an overview of the performance of the PCDHGC3 HM assay according to Example 2, in all samples.

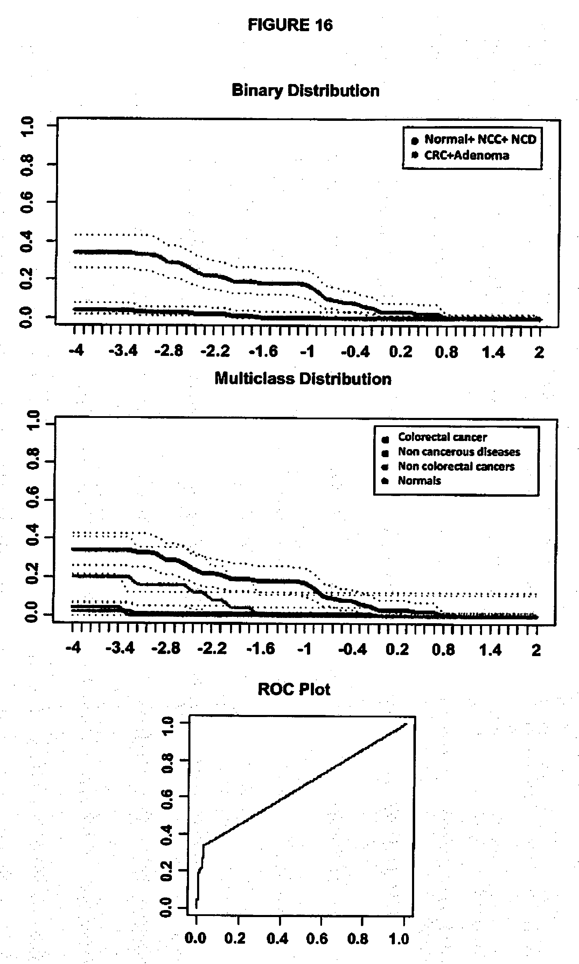

Figure 16 provides an overview of the performance of the TFAP2E HM assay according to Example 2, in all samples.

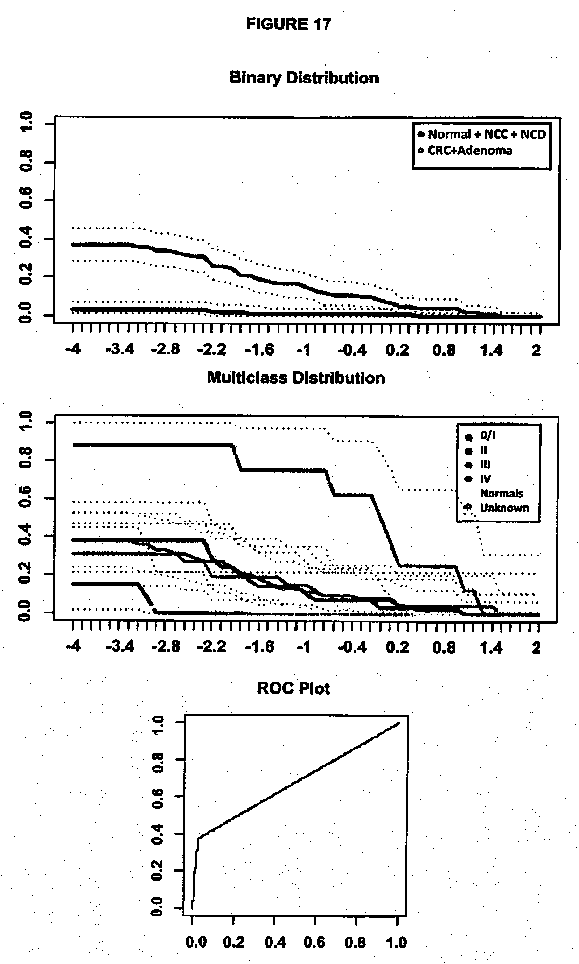

Figure 17 provides an overview of the performance of the RASSF2 HM assay according to Example 2, in all colorectal carcinoma and normal colorectal tissue samples.

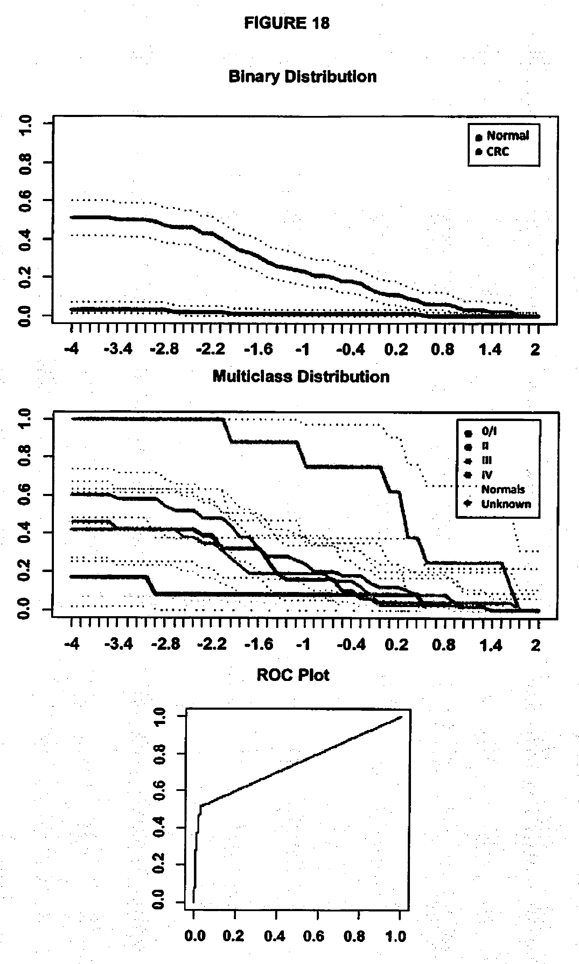

Figure 18 provides an overview of the performance of the Septin 9 HM assay according to Example 2, in all colorectal carcinoma and normal colorectal tissue samples.

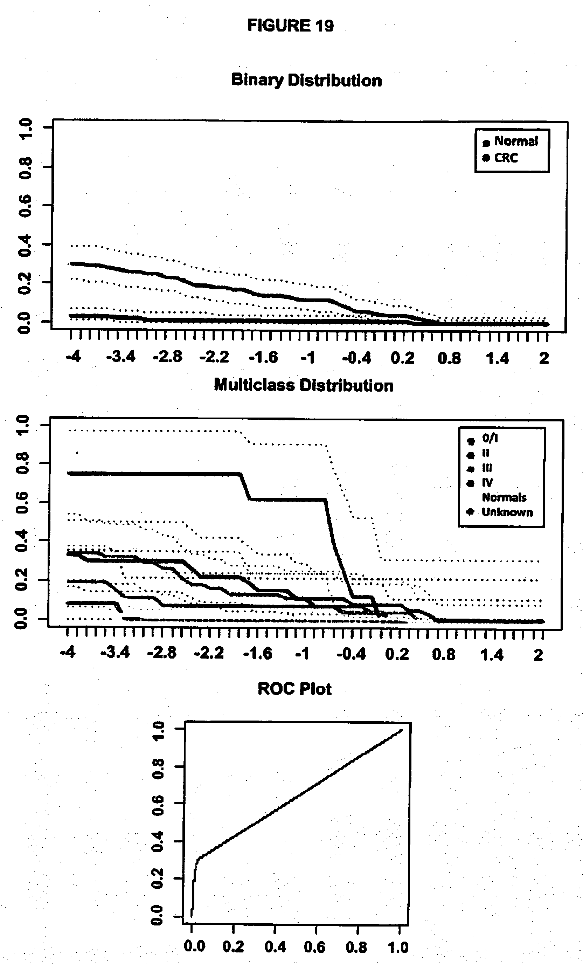

Figure 19 provides an overview of the performance of the SND1 HM assay according to Example 2, in all colorectal carcinoma and normal colorectal tissue samples.

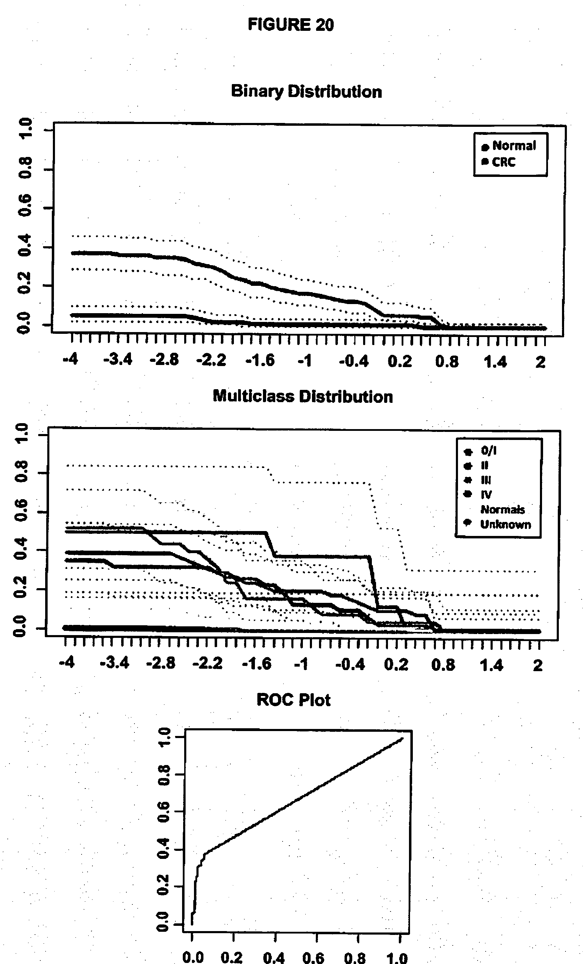

Figure 20 provides an overview of the performance of the PCDHGC3 HM assay according to Example 2, in all colorectal carcinoma and normal colorectal tissue samples.

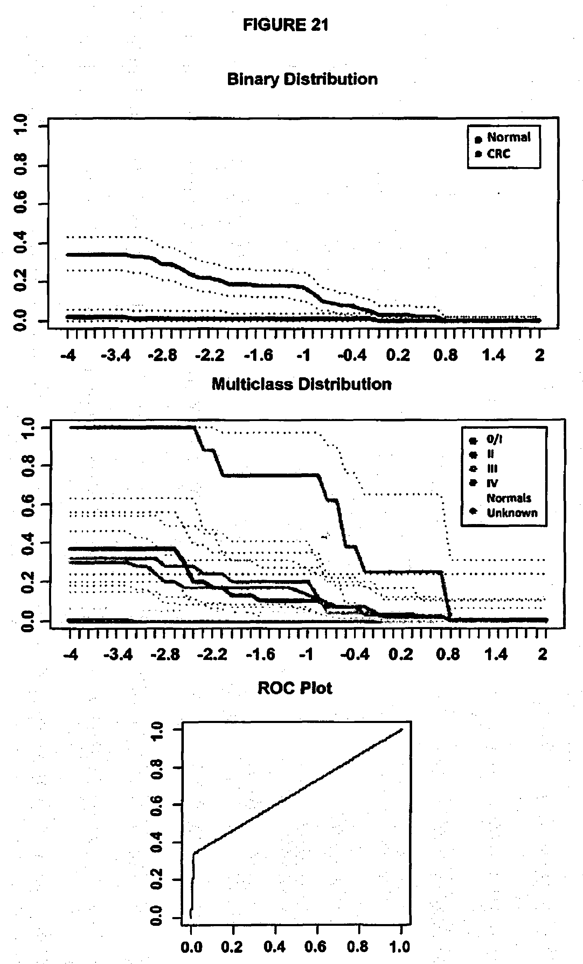

Figure 21 provides an overview of the performance of the TFAP2E HM assay according to Example 2, in all colorectal carcinoma and normal colorectal tissue samples.

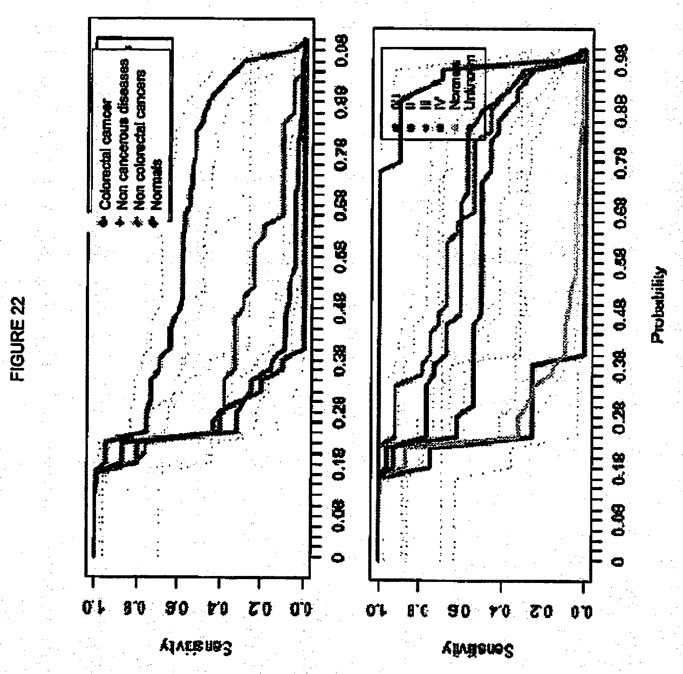

Figures 22 to 26 provide an overview of the log mean methylation measured by means of combinations HM assays (gene panels) according to Example 2. Each figures consists of two plots, The upper plot shows all samples (Normals, Non Colorectal Disease, Non-Coloretal Cancers and all CRC stages), the lower plot shows only Normaland CRC samples.
   sensitivity is shown on the Y-axis, DNA methylation measured in (log 10 ng/mL) is shown on the X-axis.
Figure 22 provides an overview of the performance of the Septin 9+TFAP2E+RASSF2+PCDHGC3+SND1 assays.

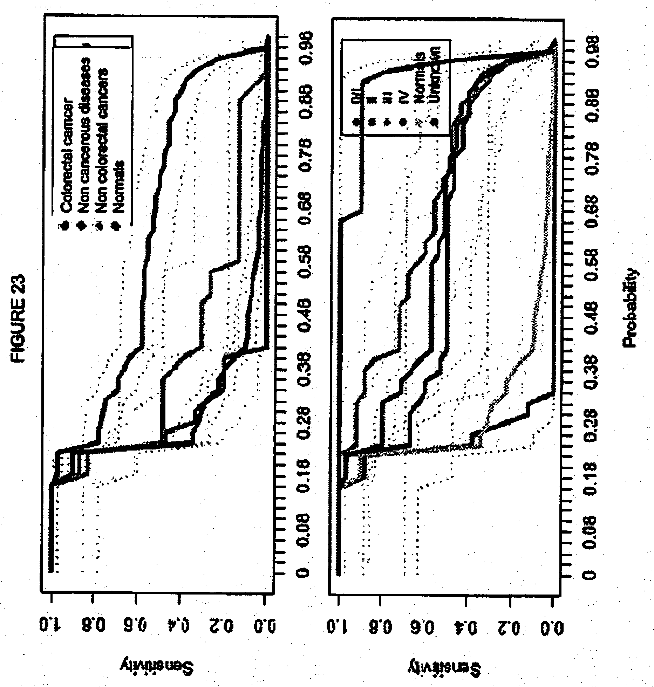

Figure 23 provides an overview of the performance of the Septin 9+TFAP2E+RASSF2+PCDHGC3 assays.

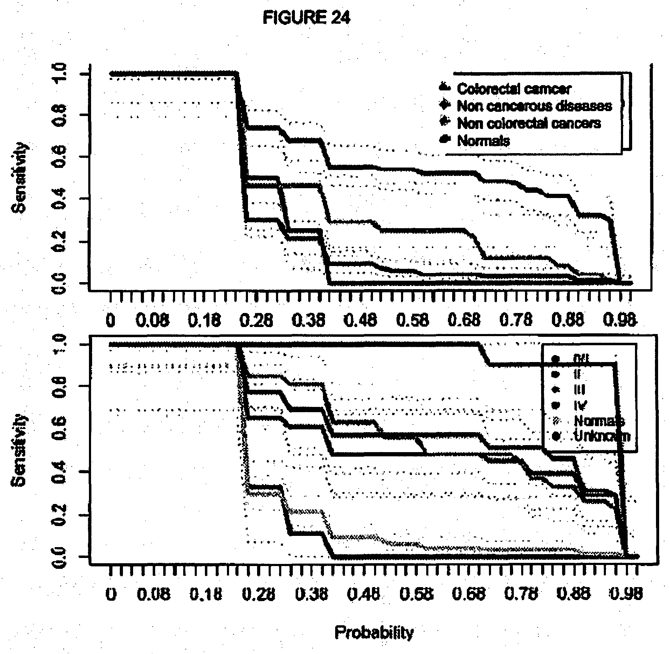

Figure 24 provides an overview of the performance of the Septin 9+TFAP2E+RASSF2 assays.

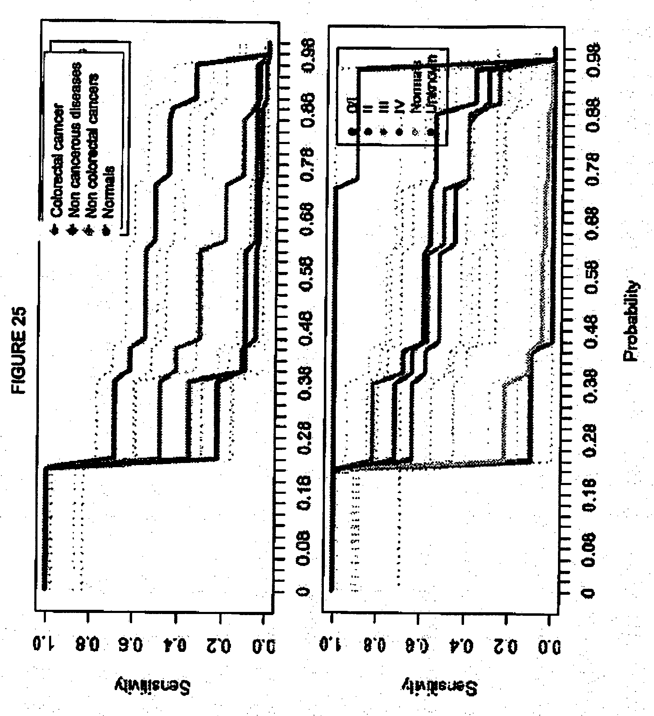

Figure 25 provides an overview of the performance of the Septin 9+TFAP2E assays.

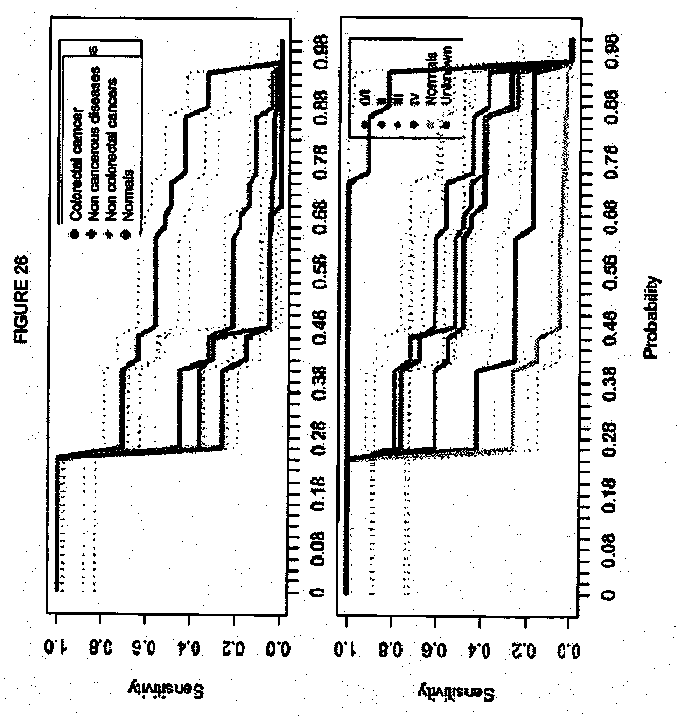

Figure 26 provides an overview of the performance of the Septin 9+RASSF2 assays.

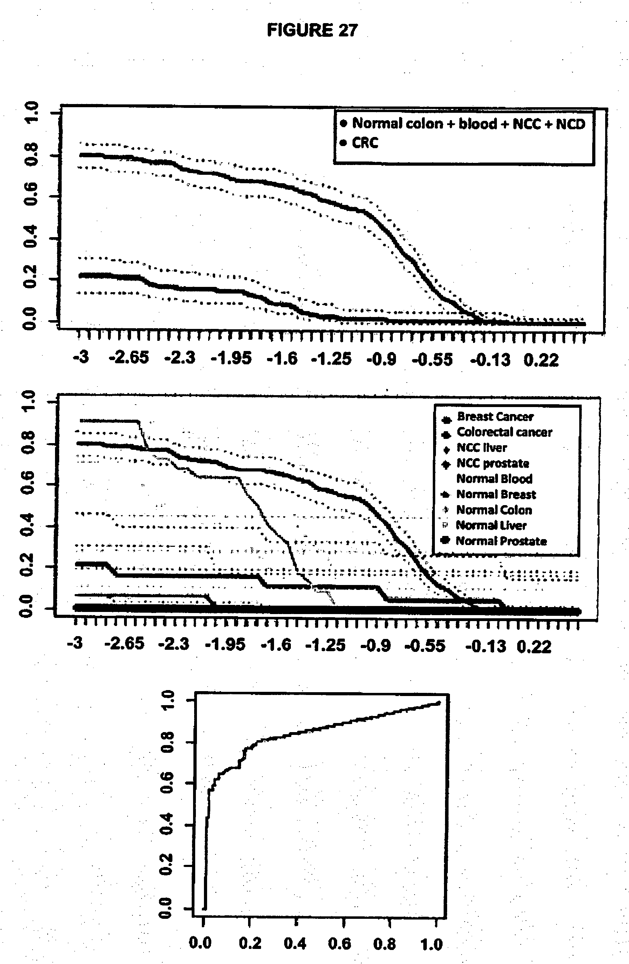

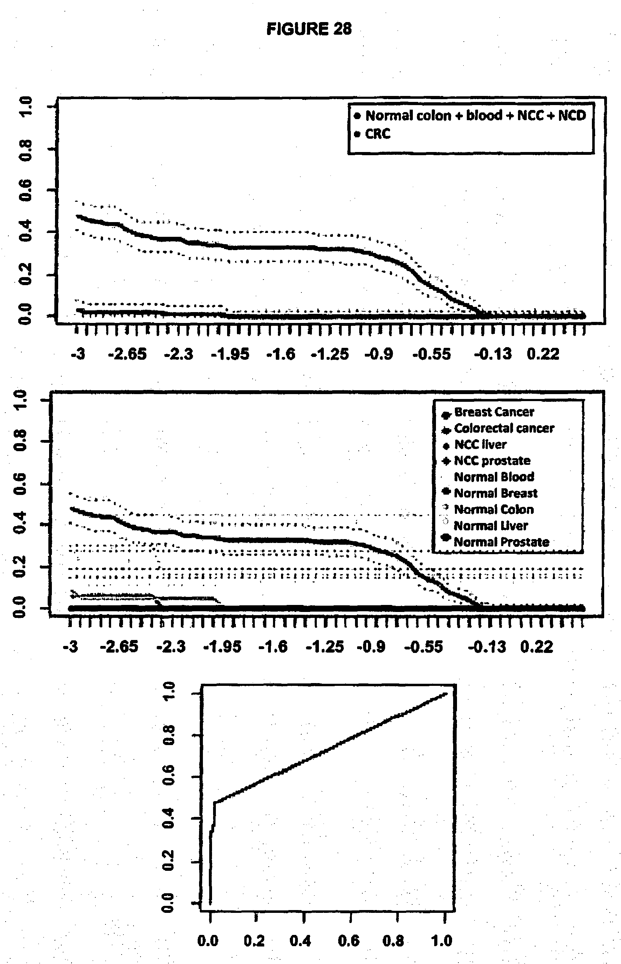

Figures 27 and 28 provide an overview of the methylation measured by means of the MSP assay according to Example 1. Each figure consists of three plots, the plots at the top and in the middle provide the binary and multiclass analysis respectively, sensitivity is shown on the Y-axis, DNA methylation is shown on the X-axis.

In each figure the plot at the bottom provides an ROC wherein sensitivity is shown on the Y-axis and 1-specificity is shown on the X-axis. Figure 27 provides an overview of the MRPS21 assay according to Example 1. Figure 28 provides an overview of the DOCK10 assay according to Example 1.

### SUMMARY OF THE INVENTION

The present invention provides a method for detecting cell proliferative disorders in a subject as defined in the claims.

Various aspects provide an efficient and unique genetic marker, whereby expression analysis of said marker enables the detection of cellular proliferative disorders with a particularly high sensitivity, specificity and/or predictive value. In the context of colorectal carcinoma the inventive testing methods have particular utility for the screening of at-risk populations. The invective methods have advantages over prior art methods (including the industry standard FOBT), because of improved sensitivity, specificity and likely patient compliance.

Preferably, the sensitivity of said detection is from about 75% to about 96%, or from about 80% to about 90%, or from about 80% to about 85%. Preferably, the specificity is from about 75% to about 96%, or from about 80% to about 90%, or from about 80% to about 85%.

The method is novel as no methods currently exist that enable the detection of cancer by analysis of body fluids, with a sensitivity and specificity high enough for use in a commercially available and regulatory body approved assay. For example, current methods used to detect and diagnose colorectal carcinoma include colonoscopy, sigmoidoscopy, and fecal occult blood colon cancer. In comparison to these methods, the disclosed invention is much less invasive than colonoscopy, and as, if not more sensitive than sigmoidoscopy and FOBT. The development of a body fluid assay represents a dear technical advantage over current methods known in the art in that it is anticipated that at least for colorectal carcinoma screening patient compliance for a single body fluid based test will be higher than the triplicate analysis of stool currently recommended for FOBT.

Disclosed is a method for the analysis of biological samples for features associated with the development of cellular proliferative disorders, the method characterised in that at least one nucleic acid, or a fragment thereof, with SEQ ID NO: 5 is contacted with a reagent or series of reagents capable of distinguishing between methylated and non methylated CpG dinucleotides within the genomic sequence, or sequences of interest.

It is further prefered that at least one nucleic acid, or a fragment thereof, of SEQ ID NO: 12 TO SEQ ID NO: 14 and at least one nucleic acid, or a fragment thereof, from SEQ ID NO: 5 is contacted with a reagent or series of reagents capable of distinguishing between methylated and non methylated CpG dinucleotides.

Disclosed is a method for ascertaining epigenetic parameters of genomic DNA associated with the development of neoplastic cellular proliferative disorders (e.g. cancers). The method has utility for the improved diagnosis, treatment and monitoring of said diseases.

Preferably, the source of the test sample is selected from the group consisting of cells or cell lines, histological slides, biopsies, paraffin-embedded tissue, body fluids, ejaculate, stool, urine, blood, and combinations thereof. More preferably, the source is selected from the group consisting of stool, blood plasma, blood serum, whole blood, isolated blood cells, cells isolated from the blood obtained from the subject.

Specifically, the present invention provides a method for detecting neoplastic cellular proliferative disorders (preferably colorectal carcinoma) including at early stages, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within at least one target sequence of the subject nucleic acid, wherein the target sequence comprises, or hybridises under stringent conditions to, a sequence comprising at least 16 contiguous nucleotides of a sequence SEQ ID NO: 5, said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. It is further preferred that at least two target sequences are analysed wherein a first target sequence comprises, or hybridises under stringent conditions to, a sequence comprising at least 16 contiguous nucleotides of a sequence selected from the group consisting SEQ ID NO: 12 TO SEQ ID NO: 14 and a second target region comprises, or hybridises under stringent conditions to, a sequence comprising at least 16 contiguous nucleotides of a sequence SEQ ID NO: 5.

Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence consisting of SEQ ID NO: 23 TO SEQ ID NO: 24 AND SEQ ID NO: 51 TO SEQ ID NO: 52, and contiguous regions thereof corresponding to the target sequence.

It is further preferred that distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within SEQ ID NO: 37 TO SEQ ID NO: 42 AND SEQ ID NO: 65 TO SEQ ID NO: 70 and at least one such CpG dinucleotide sequence to the corresponding converted or non-converted dinucleotide sequence selected from the group consisting of SEQ ID NO: 27 to SEQ ID NO: 24 and SEQ ID NO: 51 to SEQ ID NO: 52, and contiguous regions thereof

corresponding to the target sequence. Disclosed is a method for the detection of neoplastic cellular proliferative disorders, most preferably colorectal carcinoma, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 23 TO SEQ ID NO: 24 AND SEQ ID NO: 51 TO SEQ ID NO: 52 and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides according to SEQ ID NO: 5. It is further preferred that said method additionally comprises contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO: 37 TO SEQ ID NO: 42 AND SEQ ID NO: 65 TO SEQ ID NO: 70, and complements thereof, wherein the treated DNA or the fragment thereof is either amplified to produce an amplificate, or is not amplified; and determining, based on a presence or absence of, or on a property of said amplificate, the methylation state or an average, or a value reflecting an average of the methylation level of at least one , but more preferably a plurality of CpG dinucleotides of a sequence selected from the group consisting of SEQ ID NO: 12 TO SEQ ID NO: 14.

Preferably, determining comprises use of at least one method selected from the group consisting of: I) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 23 to SEQ ID NO: 24 and SEQ ID NO: 51 TO SEQ ID NO: 52 and complements thereof; ii) hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO: 23 to SEQ ID NO: 24 and SEQ ID NO: 51 to SEQ ID NO: 52 and complements thereof; iii) hybridizing at least one nucleic acid molecule comprising a contiguous sequence at least 9 nucleotides in length that is complementary to, or hybridizes under moderately stringent or stringent conditions to a sequence selected from the group consisting of SEQ ID NO: 23 to SEQ ID NO: 24 and SEQ ID NO: 51 to SEQ ID NO: 52, and complements thereof, and extending at least one such hybridized nucleic acid molecule by at least one nucleotide base; and iv) sequencing of the amplificate.

Further disclosed is a method for the analysis (i.e. detection) of cell proliferative disorders, comprising: obtaining a biological sample having subject genomic DNA; extracting the genomic DNA; contacting the genomic DNA, or a fragment according to SEQ ID NO: 5 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of at least one genomic sequence according to SEQ ID NO: 5 or an average, or a value reflecting an average methylation state of a plurality of CpG dinucleotide sequences thereof. Preferably, the digested or undigested genomic DNA is amplified prior to said determining.

It is further preferred that said method additionally comprises determining, based on a presence or absence of, or on property of at least one such fragment, the methylation state of at least one CpG dinucleotide sequence of at least one genomic sequence selected from the group consisting of SEQ ID NO: 12 TO SEQ ID NO: 14, or an average, or a value reflecting an average methylation state of a plurality of CpG dinucleotide sequences thereof.
Additionally disclosed are genomic and chemically modified nucleic acid sequences, as well as oligonucleotides and/or PNA-oligomers for analysis of cytosine methylation patterns within the sequence according to SEQ ID NO: 7.

### BRIEF DESCRIPTION OF THE DRAWINGS

### DETAILED DESCRIPTION OF THE INVENTION

### Definitions:

The term "Observed/Expected Ratio" ("O/E Ratio") refers to the frequency of CpG dinucleotides within a particular DNA sequence, and corresponds to the [number of CpG sites / (number of C bases x number of G bases)] / band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 KB, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG dinucleotide sequences) within a DNA sequence include "unmethylated," "fully-methylated" and "hemi-methylated."

The term "hemi-methylation" or "hemimethylation" refers to the methylation state of a double stranded DNA wherein only one strand thereof is methylated.

The term 'AUC' as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a decreased presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylation. Further epigenetic parameters include, for example, the acetylation of histones which, however, cannot be directly analysed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401 A1.

The term "hybridisation" is to be understood as a bond of an oligonucleotide to a complementary sequence along the lines of the Watson-Crick base pairings in the sample DNA, forming a duplex structure.

"Stringent hybridisation conditions," as defined herein, involve hybridising at 68°C in 5x SSC/5x Denhardt's solution/1.0% SDS, and washing in 0.2x SSC/0.1% SDS at room temperature, or involve the art-recognized equivalent thereof (e.g., conditions in which a hybridisation is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "Methylation-specific restriction enzymes" or "methylation-sensitive restriction enzymes" shall be taken to mean an enzyme that selectively digests a nucleic acid dependant on the methylation state of its recognition site. In the case of such restriction enzymes which specifically cut if the recognition site is not methylated or hemimethylated, the cut will not take place, or with a significantly reduced efficiency, if the recognition site is methylated. In the case of such restriction enzymes which specifically cut if the recognition site is methylated, the cut will not take place, or with a significantly reduced efficiency if the recognition site is not methylated. Preferred are methylation-specific restriction enzymes, the recognition sequence of which contains a CG dinucleotide (for instance cgcg or cccggg). Further preferred for some embodiments are restriction enzymes that do not cut if the cytosine in this dinucleotide is methylated at the carbon atom C5. "Non-methylation-specific restriction enzymes" or "non-methylation-sensitive restriction enzymes" are restriction enzymes that cut a nucleic acid sequence irrespective of the methylation state with nearly identical efficiency. They are also called "methylation-unspecific restriction enzymes."

In reference to composite array sequences, the phrase "contiguous nucleotides" refers to a contiguous sequence region of any individual contiguous sequence of the composite array, but does not include a region of the composite array sequence that includes a "node," as defined herein above.

The term "gene" shall be taken to include all transcript variants thereof (e.g. the term "Septin 9" shall include for example its truncated transcript Q9HC74) and all promoter and regulatory elements thereof. Furthermore as a plurality of SNPs are known within said gene the term shall be taken to include all sequence variants thereof.

### Overview:

The present invention provides a, method for detecting colorectal cancer as defined in the claims.

Preferably a plurality of genes are analysed. Preferably 2, 3 or 4 genes are analysed.

The marker is particularly efficient in detecting or distinguishing between colorectal cell proliferative disorders, thereby providing improved means for the early detection and thus improved treatment of said disorders.

In a first further embodiment the present invention is based upon the analysis of CpG methylaton status of at least GLI3. It is preferred that the sequences of said gene are as according to Table 1 and/or their promotor or regulatory regions. It is preferred that the sequences of said genes are as according to Table 7.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analysed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyse individual cells for methylation status, illustrating the utility and sensitivity of the method. An overview of art-recognized methods for detecting 5-methylcytosine is provided by Rein, T., et al., Nucleic Acids Res., 26:2255,1998.

The bisulfite technique, barring few exceptions (*e.g.,* Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyse individual cytosine positions, or treated by enzymatic digestion (Xiong & Laird, Nucleic Acids Res., 25:2532-4, 1997). Detection by hybridisation has also been described in the art (Olek et al., WO 99/28498). Additionally, use of the bisulfite technique for methylation detection with respect to individual genes has been described (Grigg & Clark, Bioassays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present invention provides for the use of the bisulfite technique, in combination with one or more methylation assays, for determination of the methylation status of CpG dinucleotide sequences as defined in the claims.

Genomic CpG dinucleotides can be methylated or unmethylated (alternatively known as up- and down- methylated respectively). However the methods of the present invention are suitable for the analysis of biological samples of a heterogeneous nature e.g. a low concentration of tumor cells within a background of blood or stool. Accordingly, when analyzing the methylation status of a CpG position within such a sample the person skilled in the art may use a quantitative assay for determining the level (e.g. percent, fraction, ratio, proportion or degree) of methylation at a particular CpG position as opposed to a methylation state. Accordingly the term methylation status or methylation state should also be taken to mean a value reflecting the degree of methylation at a CpG position. Unless specifically stated the terms "hypermethylated" or "upmethylated" shall be taken to mean a methylation level above that of a specified cut-off point, wherein said cut-off may be a value representing the average or median methylation level for a given population, or is preferably an optimized cut-off level. The "cut-off" is also referred herein as a "threshold". In the context of the present Invention the terms "methylated", "hypermethylated" or "upmethylated" shall be taken to include a methylation level above the cut-off be zero (0) % (or equivalents thereof) methylation for all CpG positions within and associated with (e.g. in promoter or regulatory regions).

Determination of the methylation status of CpG dinucleotide sequences within SEQ ID NO: 5 has utility both in the diagnosis and characterization of cellular proliferative disorders. *Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

*COBRA.* COBRA^{™} analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples.

Typical reagents (*e.g*., as might be found in a typical COBRA^{™}-based kit) for COBRA^{™} analysis may include, but are not limited to: PCR primers for specific gene (or bisulfite treated DNA sequence or CpG island); restriction enzyme and appropriate buffer; gene-hybridization oligonucleotide; control hybridization oligonucleotide; kinase labeling kit for oligonucleotide probe; and labeled nucleotides. Additionally, bisulfite conversion reagents may include: DNA denaturation buffer; sulfonation buffer; DNA recovery reagents or kits (*e.g.,* precipitation, ultrafiltration, affinity column); desulfonation buffer; and DNA recovery components.

Preferably, assays such as "MethyLight^{™}" (a fluorescence-based real-time PCR technique) (Eads et al., Cancer Res. 59:2302-2306, 1999), Ms-SNuPE^{™} (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

The "HeavyMethyl™" assay, technique is a quantitative method for assessing methylation differences based on methylation specific amplification of bisulfite treated DNA. Methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers. The HeavyMethyl™ assay may also be used in combination with methylation specific amplification primers.

Typical reagents (*e.g.,* as might be found in a typical MethyLight□-based kit) for HeavyMethyl™ analysis may include, but are not limited to: PCR primers for specific genes (or bisulfite treated DNA sequence or CpG island); blocking oligonucleotides; optimized PCR buffers and deoxynucleotides; and Taq polymerase.

MSP. MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (*e.g*., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MethyLight*^{™}. The MethyLight^{™} assay is a high-throughput quantitative methylation assay that utilizes fluorescence-based real-time PCR (TaqMan□) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed in a "biased" (with PCR primers that overlap known CpG dinucleotides) reaction. Sequence discrimination can occur both at the level of the amplification process and at the level of the fluorescence detection process.

The MethyLight^{™} assay may be used as a quantitative test for methylation patterns in the genomic DNA sample, wherein sequence discrimination occurs at the level of probe hybridization. In this quantitative version, the PCR reaction provides for a methylation specific amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The MethyLight^{™} process can by used with any suitable probes e.g. "TaqMan®" , Lightcycler® etc.... For example, double-stranded genomic DNA is treated with sodium bisulfite and subjected to one of two sets of PCR reactions using TaqMan® probes; *e.g*., with MSP primers and/ or HeavyMethyl blocker oligonucleotides and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e*.*g*., as might be found in a typical MethyLight□-based kit) for MethyLight^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

The QM^{™} (quantitative methylation) assay is an alternative quantitative test for methylation patterns in genomic DNA samples, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the HeavyMethyl™ and MSP techniques), or with oligonucleotides covering potential methylation sites.

The QM^{™} process can by used with any suitable probes e.g. taqMan®", Lightcycler® etc... in the amplification process. For example, double-stranded genomic DNA is treated with sodium bisulfite and subjected to unbiased primers and the TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.
Typical reagents (e.g., as might be found in atypical QM^{™} -based kit) for QM^{™} analysis may include, but are not limited to: PCR primers for specific gene (or bisulfite treated DNA sequence or CpG island); TaqMan® or Lightcycler® probes; optimized PCR buffers and deoxynucleotides; and Taq polymerase.

*Ms-SNuPE*. The Ms-SNuPE^{™} technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is redacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG slte(s) of interest. Small amounts of DNA can be analyzed (*e.g.,* microdissected pathology sections), and it avoids utilization of restriction enzymes for determining the methylation status at CpG sites.

Typical reagents (*e.g*., as might be found in a typical Ms-SNuP E7m-based kit) for Ms-SNuPE^{™} analysis may include, but are not limited to: PCR primers for specific gene (or bisulfite treated DNA sequence or CpG island); optimized PCR buffers and deoxynucleotides; gel extraction kit; positive control primers; Ms-SNuPET^{™} primers for specific gene; reaction buffer (for the Ms-SNuPE reaction); and labelled nucleotides. Additionally, bisulfite conversion reagents may include: DNA denaturation buffer; sulfonation buffer; DNA recovery regents or kit (*e*.*g*., precipitation, ultrafiltration, affinity column); desulfonation buffer; and DNA recovery components.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, *e.g.,* by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising cellular matter are suitable for us e in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood.

The genomic DNA sample is then treated with at least one reagent, or series of reagents that distinguishes between methylated and non-methylated CpG dinucleotides within at least one target region of the genomic DNA, wherein the target region comprises, or hybridizes under stringent conditions to a sequence of at least 16 contiguous nucleotides of at least the sequence according to SEQ ID NO:5 wherein said contiguous nucleotides comprise at least one CpG dinucleotides sequence. It is further preferred that said reagent, or series of reagents distinguishes between methylated and non-methylated CpG dinucleotides within at least two target regions of the genomic DNA, wherein a first the target region comprises, or hybridizes under stringent conditions to a sequence of at least 16 contiguous nucleotides of at least one sequence selected from the group consisting of SEQ ID NO: 12 TO SEQ ID NO: 14 and a second target region that comprises, or hybridizes under stringent conditions to a sequence of at least 16 contiguous nucleotides of the sequence SEQ ID NO:5 , wherein said contiguous nucleotides comprise at least one CpG dinucleotide sequence.

It is particularly preferred that said reagent converts cytosine bases which are unmethylated at the 5'-position to uracil, thymine, or another base which is dissimilar to cytosine in terms of hybridisation behaviour. However in an alternative embodiment said reagent may be a methylation sensitive restriction enzyme.

Wherein the genomic DNA sample is treated in such a manner that cytosine bases which are unmethylated at the 5'-position are converted to uracil, thymine, or another base which is dissimilar to cytosine in terms of hybridization behavior it is preferred that this treatment is carried out with bisulfite (hydrogen sulfite, disulfite) and subsequent alkaline hydrolysis. Such a treatment results in the conversion of SEQ ID NO: 1 - SEQ ID NO: 14 to SEQ ID NO: 43 - SEQ ID NO: 70 wherein said CpG dinucleotides are unmethylated and to SEQ ID NO: 15 - SEQ ID NO: 42 wherein said CpG dinucleotides are methylated.

The treated DNA is then analysed in order to determine the methylation state of the target gene sequences (at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21 prior to the treatment). It is particularly disclosed that the target region comprises, or hybridizes under stringent conditions to at least 16 contiguous nucleotides of at least one gene selected from the group consisting of RASSF2, TFAP2E , SND1, PCDHGC3, EDNRB, STOM, GLI3, RXFP3, LimK1, GPR73L1, CDH10, DOCK10 and MRPS21 . It is disclosed that the sequence of said genes according to SEQ ID NO: 1 TO SEQ ID NO: 11 AND SEQ ID NO: 132 TO SEQ ID NO: 133 is analysed.

The method of analysis may be selected from those known in the art, including those listed herein. Particularly preferred are MethyLightTM, MSP and the use of blocking oligonucleotides (HeavyMethyl™) as described herein. It is further preferred that any oligonucleotides used in such analysis (including primers, blocking oligonucleotides and detection probes) should be reverse complementary, identical, or hybridise under stringent or highly stringent conditions to an at least 16-base-pair long segment of the base sequences of one or more of SEQ ID NO: 27 to SEQ ID NO: 28 and SEQ ID NO: 55 to SEQ ID NO: 56 and sequences complementary thereto.

Aberrant methylation, more specifically hypermethylation of the genes or genomic sequence selected from the group consisting of RASSF2, TFAP2E, SND1, PCDHGC3, EDNRB, STOM, GLI3, RXFP3, LimK1, GPR73L1, PCDH10, DOCK10 and MRPS21 (including their promoter and/or regulatory regions) is associated with the presence of neoplastic cellular proliferative disorders, and is particularly prevalent in colorectal carcinomas. Accordingly wherein a biological sample presents within any degree of methylation, said sample should be determined as cancerous.

Analysis of one the genes or genomic sequence selected from the group consisting of RASSF2, TFAP2E , SND1, PCDHGC3, EDNRB, STOM, GLI3, RXFP3, LimK1, GPR73L1, PCDH10, DOCK10 and MRPS21 enables for the first time detecting, or detecting and distinguishing between or among colon cell proliferative disorders afforded with a sensitivity of greater than or equal to 80% and a specificity of greater than or equal to 80%.

Sensitivity Is calculated as:{detected neoplasia/all neoplasia) e.g. :{detected colon neoplasia/all colon neoplasia); and specificity is calculated as (non-detected negatives/total negatives)

Preferably, the sensitivity is from about 75% to about 96%, or from about 80% to about 90%, or from about 80% to about 85%. Preferably, the specificity is from about 75% to about 96%, or from about 80% to about 90%, or from about 80% to about 85%.

Colon neoplasia is herein defined as all colon malignancies and adenomas greater than 1 cm., or subsets thereof. Negatives can be defined as healthy individuals.

Disclosed is the use of at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21 (or promoter and/or regulatory regions thereof) as a marker for the detection of cellular proliferative disorders (in particular colon disorders). Said method may be enabled by means of any analysis of the expression of an RNA transcribed therefrom or polypeptide or protein translated from said RNA, preferably by means of mRNA expression analysis or polypeptide expression analysis. Further disclosed are diagnostic assays and methods, both quantitative and qualitative for detecting the expression of at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21 in a subject and determining therefrom upon the presence or absence of cancer in said subject Further disclosed are diagnostic assays and methods, both quantitative and qualitative for detecting the expression of the gene SEPTIN 9 in combination with at least one gene selected from the group consisting of RASSF2, TFAP2E, SND1, PCDHGC3, EDNRB, STOM, GLI3, RXFP3, LimK1, GPR73L1, PCDH10, DOCK10 and MRPS21 in a subject and determining therefrom upon the presence or absence of cancer in said subject.

Aberrant expression of mRNA transcribed from the genes or genomic sequences selected from the group consisting of RASSF2, TFAP2E, SND1, PCDHGC3, EDNRB, STOM, GLI3, RXFP3, LimK1, GPR73L1, PCDH10, DOCK10 and MRPS21 are associated with the presence of cancer in a subject.

Under expression (and/or presence methylation) is associated with the presence of cancer, and vice versa over-expression (and/or absence of methylation) is associated with the absence of cancer.

To detect the presence of mRNA encoding a gene, a sample is obtained from a patient. The sample may be any suitable sample comprising cellular matter of the tumour. Suitable sample types include cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sample types are stool or body fluids selected from the group consisting colonic effluent, urine, blood plasma, blood serum, whole blood, isolated blood cells, cells isolated from the blood.
The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligonucleotides dT primer and/or random hexamer primers. The cDNA thus produced is then amplified by means of PCR. (Belyavsky et al, Nucl Acid Res 17:2919-2932, 1989; Krug and Berger, Methods in Enzymology, Academic Press, N.Y., Vol.152, pp. 316-325, 1987 . Further preferred is the "real-time" variant of RT- PCR, wherein the PCR product is detected by means of hybridisation probes (e.g. TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridisation to a labelled probe in the dried gel itself or on a membrane. The resulting signal is proportional to the amount of target RNA in the RNA population.

Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.
The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, mass labels or fluorescent labels). The size of the target RNA, not the probe, will determine the size of the detected band, so methods such as random-primed labelling, which generates probes of variable lengths, are suitable for probe synthesis. The specific activity of the probe will determine the level of sensitivity, so it is preferred that probes with high specific activities, are used..
In an RNase protection assay, the RNA target and an RNA probe of a defined length are hybridised in solution. Following hybridisation, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.
The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length RNA probes and the reproducibility and reliability of RNA:RNA duplex digestion with RNases (Ausubel *et al.* 2003), particularly preferred are probes with high specific activities.

Particularly preferred is the use of microarrays. The microarray analysis process can be divided into two main parts. First is the immobilization of known gene sequences onto glass slides or other solid support followed by hybridisation of the fluorescently labelled cDNA (comprising the sequences to be interrogated) to the known genes immobilized on the glass slide (or other solid phase). After hybridisation, arrays are scanned using a fluorescent microarray scanner. Analysing the relative fluorescent intensity of different genes provides a measure of the differences in gene expression.
DNA arrays can be generated by immobilizing presynthesized oligonucleotides onto prepared glass slides or other solid surfaces. In this case, representative gene sequences are manufactured and prepared using standard oligonucleotide synthesis and purification methods. These synthesized gene sequences are complementary to the RNA transcript(s) of the genes of interest (in this case the genes or genomic sequences selected from the group consisting of RASSF2, TFAP2E , SND1, PCDHGC3, EDNRB, STOM, GLI3, RXFP3, LimK1, GPR73L1, PCDH10, DOCK10 and MRPS21 ,AND PREFERABLY Septin 9) and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. Preferably said synthesized nucleic acids are locked nucleic acids.
In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labelling of the cDNA can be accomplished by either direct labelling or indirect labelling methods. During direct labelling, fluorescently modified nucleotides (*e.g.,* Cy^{®}3- or Cy^{®}5-dCTP) are incorporated directly into the cDNA during the reverse transcription. Alternatively, indirect labelling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.
To perform differential gene expression analysis, cDNA generated from different RNA samples are labelled with Cy^{®}3. The resulting labelled cDNA is purified to remove unincorporated nucleotides, free dye and residual RNA. Following purification, the labelled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridisation using a fluorescent microarray scanner. The fluorescent intensity of each spot indicates the level of expression of the analysed gene; bright spots correspond to strongly expressed genes, while dim spots indicate weak expression..
Once the images are obtained, the raw data must be analysed. First, the background fluorescence must be subtracted from the fluorescence of each spot. The data is then normalized to a control sequence, such as exogenously added nucleic acids (preferably RNA or DNA), or a housekeeping gene panel to account for any non-specific hybridisation, array imperfections or variability in the array set-up, cDNA labelling, hybridisation or washing. Data normalization allows the results of multiple arrays to be compared.
Another aspect of the invention relates to the use of a kit for use in diagnosis of colorectal cancer in a subject according to the methods of present claim 9.

In a preferred embodiment the means for measuring the level of transcription comprise oligonucleotides or polynucleotides able to hybridise under stringent or moderately stringent conditions to the transcription products of GLI3.

It is further prefered that said kit further comprises means for measuring the level of transcription of the gene Septin 9 comprising oligonucleotides or polynucleotides able to hybridise under stringent or moderately stringent conditions to the transcription products of said gene. In one embodiment said oligonucleotides or polynucleotides comprise at least 9,18 or 25 bases of a sequence complementary to or hybridising to said transcription product
In a most preferred embodiment the level of transcription is determined by techniques selected from the group of Northern Blot analysis, reverse transcriptase PCR, real-time PCR, RNAse protection, and microarray. In another embodiment of the invention the kit further comprises means for obtaining a biological sample of the patient. Preferred is a kit, which further comprises a container which is most preferably suitable for containing the means for measuring the level of transcription and the biological sample of the patient, and most preferably further comprises instructions for use and interpretation of the kit results.
In a preferred embodiment the kit comprises (a) a plurality of oligonucleotides or polynucleotides able to hybridise under stringent or moderately stringent conditions to the transcription products of at least GLI; (b) a contained, preferably suitable for containing the oligonucleotides or polynucleotides and a biological sample of the patient comprising the transcription products wherein the oligonucleotides or polynucleotides can hybridise under stringent or moderately stringent conditions to the transcription products, (c) means to detect the hybridisation of (b); and optionally, (d) instructions for use and interpretation of the kit results.

It is further preferred that said kit comprises (e) a plurality of oligonucleotides or polynucleotides able to hybridise under stringent or moderately stringent conditions to the transcription products of the gene Septin 9. Said oligonucleotides or polynucleotides of (e) preferably comprise in each case at least 9, 18 or 25 bases of a sequence complementary to or hybridising to a Septin 9 transcription products.
The kit may also contain other components such as hybridisation buffer (where the oligonucleotides are to be used as a probe) packaged in a separate container. Alternatively, where the oligonucleotides are to be used to amplify a target region, the kit may contain, packaged in separate containers, a polymerase and a reaction buffer optimised for primer extension mediated by the polymerase, such as PCR. Preferably said polymerase is a reverse transcriptase. It is further preferred that said kit further contains an Rnase reagent.

Further disclosed are methods for the detection of the presence of the polypeptide encoded by said gene sequences in a sample obtained from a patient.

Aberrant levels of polypeptide expression of the polypeptides encoded by the genes or genomic sequences selected from the group consisting of RASSF2, TFAP2E, SND1, PCDHGC3, EDNRB, STOM, GLI3, RXFP3, LimK1, GPR73L1, PCDH10, DOCK10 and MRPS21 are associated with the presence of cancer. The accuracy of the diagnosis of colorectal cancer is increased when polypeptides of the gene Septin 9 are analysed in combination with said aforenmentioned polypeptides.

Underexpression of said polypeptides is associated with the presence of cancer.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991). Preferred are binder-ligand immunoassay methods including reacting antibodies with an epitope or epitopes and competitively displadng a labelled polypeptide or derivative thereof.

Disclosed is the use of antibodies specific to the polypeptide encoded by a gene selected from the group consisting of RASSF2, TFAP2E , SND1, PCDHGC3, EDNRB, STOM, GLI3, RXFP3, LimK1, GPR73L1, PCDH10, DOCK10 and MRPS21. Further disclosed is the use of a first species of antibodies specific to the polypeptide encoded by the gene SEPTIN 9 and a second species of antibodies specific to the polypeptide encoded by a gene selected from the group consisting of RASSF2, TFAP2E , SND1, PCDHGC3, EDNRB, STOM, GLI3, RXFP3, LimK1, GPR73L1, PCDH10, DOCK10 and MRPS21.

Such antibodies are useful for cancer diagnosis. Production of monoclonal or polyclonal antibodies can be induced by the use of an epitope encoded by a polypeptide encoded by a gene selected from the group consisting of RASSF2, TFAP2E, SND1, PCDHGC3, EDNRB, STOM, GU3, RXFP3. LimK1, GPR73L1, PCDH10, DOCK10 and MRPS21 as an antigen. Such antibodies may in turn be used to detect expressed polypeptides as markers for cancer diagnosis. The levels of such polypeptides present may be quantified by conventional methods. Antibody-polypeptide binding may be detected and quantified by a variety of means known in the art, such as labelling with fluorescent or radioactive ligands. Further disclosed are kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used In agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (EUSA), fluorescent immunoassays and the like. Polyclonal or monoclonal antibodies or epitopes thereof can be made for use in immunoassays by any of a number of methods known in the art.

Alternatively proteins may be detected by means of western blot analysis. Said analysis is standard in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a blocking agent to bind remaining sticky places on the membrane, commonly used agents include generic protein (e.g. milk protein). An antibody specific to the protein of interest is then added, said antibody being detectably labelled for example by dyes or enzymatic means (e.g. alkaline phosphatase or horseradish peroxidase). The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference in its entirety). Methods for preparation of the polypeptides or epitopes thereof include, but are not limited to chemical synthesis, recombinant DNA techniques or isolation from biological samples.
In the final step of the method the diagnosis of the patient is determined, whereby under-expression (of at least GLI3, and optionally Septin 9) is indicative of the presence of cancer. The term under-expression shall be taken to mean expression at a detected level less than a pre-determined cut off which may be selected from the group consisting of the mean, median or an optimised threshold value. Further disclosed is a kit for use in diagnosis of cancer in a subject comprising: a means for detecting polypeptides at least one gene selected from the group consisting of RASSF2, TFAP2E , SND1, PCDHGC3, EDNRB, STOM, GLI3, RXFP3, LimK1, GPR73L1, PCDH10, DOCK10 and MRPS21. The means for detecting the polypeptides comprise preferably antibodies, antibody derivatives, or antibody fragments. The polypeptides are most preferably detected by means of Western Blotting utilizing a labelled antibody. Preferred is a kit, which further comprises a container suitable for containing the means for detecting the polypeptides in the biological sample of the patient, and most preferably further comprises instructions for use and interpretation of the kit results. The kit may comprise: (a) a means for detecting polypeptide of at least the gene GLI3, (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.
The kit may also contain other components such as buffers or solutions suitable for blocking, washing or coating , packaged in a separate container.

Disclosed is a novel application of the analysis of methylation levels and/or patterns within said sequences that enables a precise detection, characterisation and/or treatment of colorectal cell proliferative disorders. Early detection of cancer is directly linked with disease prognosis, and the disclosed method thereby enables the physician and patient to make better and more informed treatment decisions.

### FURTHER IMPROVEMENTS

Disclosed are novel uses for the genomic sequence SEQ ID NO: 5 as well as oligonucleotides and/or PNA-oligomers for analysis of cytosine methylation patterns within SEQ ID NO: 5.

An objective of the invention comprises analysis of the methylation state of one or more CpG dinucleotides within AT LEAST ONE SEQUENCE SELECTED FROM THE GROUP CONSISTING SEQ ID NO: 1 TO SEQ ID NO: 14 AND SEQ ID NO: 132 TO SEQ ID NO: 133 and sequences complementary thereto.

Disclosed are treated nucleic acids, derived from genomic SEQ ID NO: 5, wherein the treatment is suitable to convert at least one unmethylated cytosine base of the genomic DNA sequence to uracil or another base that is detectably dissimilar to cytosine in terms of hybridization. The genomic sequences in question may comprise one, or more consecutive methylated CpG positions. Said treatment preferably comprises use of a reagent selected from the group consisting of bisulfite, hydrogen sulfite, disulfite, and combinations thereof. Preferred is a non-naturally occurring modified nucleic acid comprising a sequence of at least 16 contiguous nucleotide bases in length of a sequence selected from the group consisting of SEQ ID NO: 23 to SEQ ID NO: 24 and SEQ ID NO: 51 TO SEQ ID NO: 52. Further preferred said nucleic acid is at least 50, 100, 150, 200, 250 or 500 base pairs in length of a segment of the nucleic acid sequence disclosed in SEQ ID NO: 23 TO SEQ ID NO: 24 and SEQ ID NO: 51 to SEQ ID NO: 52.

It is preferred that said sequence comprises at least one CpG, TpA or CpA dinucleotide and sequences complementary thereto. The sequences of SEQ ID NO: 23 to SEQ ID NO: 24 and SEQ ID NO: 51 to SEQ ID NO: 52 provide non-naturally occurring modified versions of the nucleic acid according to SEQ ID NO: 5, wherein the modification of each genomic sequence results in the synthesis of a nucleic acid having a sequence that is unique and distinct from said genomic sequence as follows. For each sense strand genomic DNA, *e.g.,* SEQ ID NO: 1, four converted versions are disclosed. A first version wherein "C" is converted to "T," but "CpG" remains "CpG" (*i.e*., corresponds to case where, for the genomic sequence, all "C" residues of CpG dinucleotide sequences are methylated and are thus not converted); a second version discloses the complement of the disclosed genomic DNA sequence (i.e. antisense strand), wherein "C" is converted to "T," but "CpG" remains "CpG" (*i.e.,* corresponds to case where, for all "C" residues of CpG dinucleotide sequences are methylated and are thus not converted). The 'upmethylated' converted sequences of SEQ ID NO: 1 TO SEQ ID NO: 14 AND SEQ ID NO: 132 TO SEQ ID NO: 133 correspond to SEQ ID NO: 15 TO SEQ ID NO: 42. A third chemically converted version of each genomic sequences is provided, wherein "C" is converted to "T" for all "C" residues, including those of "CpG" dinucleotide sequences (*i.e.,* corresponds to case where, for the genomic sequences, all "C" residues of CpG dinucleotide sequences are unmethylated); a final chemically converted version of each sequence, discloses the complement of the disclosed genomic DNA sequence (i.e. antisense strand), wherein "C" is converted to "T" for all "C" residues, including those of "CpG" dinucleotide sequences (*i.e.,* corresponds to case where, for the complement (antisense strand) of each genomic sequence, all "C" residues of CpG dinucleotide sequences are unmethylated). The 'downmethylated' converted sequences of SEQ ID NO: 1 TO SEQ ID NO: 14 AND SEQ ID NO: 132 TO SEQ ID NO: 133 correspond to SEQ ID NO: 43 TO SEQ ID NO: 70. See Table 1 for further details.

Significantly, heretofore, the nucleic acid sequences and molecules according SEQ ID NO: 15 TO SEQ ID NO: 36 AND SEQ ID NO: 43 TO SEQ ID NO: 64 AND SEQ ID NO: 134 TO SEQ ID NO: 141 were not implicated in or connected with the detection of cellular proliferative disorders.

Further disclosed are oligonucleotides or oligomers suitable for detecting the cytosine methylation state within genomic or treated (chemically modified) DNA, according to SEQ ID NO: 23 to SEQ ID NO: 24 and SEQ ID NO: 51 to SEQ ID NO: 52, SEQ ID NO: 5. Said oligonucleotide or oligomer comprising a nucleic acid sequence having a length of at least nine (9) nucleotides which is Identical to, hybridizes, under moderately stringent or stringent conditions (as defined herein above), to a treated nucleic acid sequence according to SEQ ID NO: 23 to SEQ ID NO: 24 and SEQ ID NO: 51 to SEQ ID NO: 52 and/or sequences complementary thereto, or to a genomic sequence according to SEQ ID NO: 5 and/or sequences complementary thereto.

Disclosed are nucleic acid molecules (*e.g.,* oligonucleotides and peptide nucleic acid (PNA) molecules (PNA-oligomers)) that hybridize under moderately stringent and/or stringent hybridization conditions to all or a portion of a sequence selected from the group consisting of SEQ ID NO: 23 to SEQ ID NO: 24 and SEQ ID NO: 51 to SEQ ID NO: 52, SEQ ID NO: 5 or to the complements thereof. Further disclosed is a nucleic acid molecule that hybridizes under moderately stringent and/or stringent hybridization conditions to all or a portion of the sequences SEQ ID NO: 23 to SEQ ID NO: 24 and SEQ ID NO: 51 to SEQ ID NO: 52 but not SEQ ID NO: 5 or other human genomic DNA.

The identical or hybridizing portion of the hybridizing nucleic acids is typically at least 9, 16, 20, 25, 30 or 35 nucleotides in length. However, longer molecules have inventive utility, and are thus within the scope of the present invention.

Preferably, the hybridizing portion of the hybridizing nucleic acids is at least 95%, or at least 98%, or 100% identical to the sequence, or to a portion thereof of a sequence selected from the group consisting of SEQ ID NO: 5, SEQ ID NO: 23 to SEQ ID NO: 24 and SEQ ID NO: 51 to SEQ ID NO: 52, or to the complements thereof.

Hybridizing nucleic acids of the type described herein can be used, for example, as a primer (*e.g*., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: 5 (such as allelic variants and SNPs), rather than identical, it is useful to first establish the lowest temperature at which only homologous hybridization occurs with a particular concentration of salt (*e.g*., SSC or SSPE). Then, assuming that 1% mismatching results in a 1°C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of inventive oligonucleotides of length X (in nucleotides), as indicated by polynucleotide positions with reference to, *e.g.,* SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:
n to (n + (X-1));
where n=1,2,3,...(Y-(X-1));
where Y equals the length (nucleotides or base pairs) of SEQ ID NO: 1 (2519);
where X equals the common length (in nucleotides) of each oligonucleotide in the set (*e.g.,* X=20 for a set of consecutively overlapping 20-mers); and
where the number (Z) of consecutively overlapping oligomers of length X for a given SEQ ID NO of length Y is equal to Y- (2519-1). For example Z= 2519-19= 2500 for either sense or antisense sets of SEQ ID NO: 1, where X=20.

Preferably, the set is limited to those oligomers that comprise at least one CpG, TpG or CpA dinucleotide.

Examples of inventive 20-mer oligonucleotides include the following set of 2,261 oligomers (and the antisense set complementary thereto), indicated by polynucleotide positions with reference to SEQ ID NO: 1:
1-20, 2-21, 3-22, 4-23, 5-24, ............... and 2499-2519.

Preferably, the set is limited to those oligomers that comprise at least one CpG, TpG or CpA dinucleotide.

Likewise, examples of inventive 25-mer oligonucleotides include the following set of 2,256 oligomers (and the antisense set complementary thereto), indicated by polynucleotide positions with reference to SEQ ID NO: 1:
1-25, 2-26, 3-27, 4-28, 5-29, ...... and 2494-2519.

Preferably, the set is limited to those oligomers that comprise at least one CpG, TpG or CpA dinucleotide.

Encompassed, for *each* of SEQ ID NO: 5, SEQ ID NO: 23 to SEQ ID NO: 24 and SEQ ID NO: 51 to SEQ ID NO: 52 (sense and antisense), multiple consecutively overlapping sets of oligonucleotides or modified oligonucleotides of length X, where, *e.g.,* X= 9, 10, 17, 20, 22, 23, 25, 27, 30 or 35 nucleotides.

The oligonucleotides or oligomers constitute effective tools useful to ascertain genetic and epigenetic parameters of the genomic sequences selected from the group consisting of SEQ ID NO: 5. Preferred sets of such oligonucleotides or modified oligonucleotides of length X are those consecutively overlapping sets of oligomers corresponding to SEQ ID NO: 5, SEQ ID NO: 23 to SEQ ID NO: 24 and ID NO: 51 to SEQ ID NO: 52 (and to the complements thereof). Preferably, said oligomers comprise at least one CpG, TpG or CpA dinucleotide.

Particularly preferred oligonucleotides or oligomers are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, *e.g.,* a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers are typically used in 'sets,' which contain at least one oligomer for analysis of each of the CpG dinucleotide of a genomic sequence selected from the group consisting SEQ ID NO: 5 and sequences complementary thereto, or to the corresponding CpG, TpG or CpA dinucleotide within a sequence of the treated nucleic adds according to SEQ ID NO: 23 to SEQ ID NO: 24 and SEQ ID NO: 51 to SEQ ID NO: 52 and sequences complementary thereto. However, It is anticipated that for economic or other factors it may be preferable to analyse a limited selection of the CpG dinucleotide within said sequences, and the content of the set of oligonucleotides is altered accordingly.

Therefore, disclosed is a set of at least two (2) (oligonucleotides and/or PNA-oligomers) useful for detecting the cytosine methylation state in treated genomic DNA (SEQ ID NO: 23 to SEQ ID NO: 24 and SEQ ID NO: 51 to SEQ ID NO: 52), or in genomic DNA (SEQ ID NO: 5 and sequences complementary thereto). These probes enable diagnosis, classification and/or therapy of genetic and epigenetic parameters of colorectal cell proliferative disorders. The set of oligomers may also be used for detecting single nucleotide polymorphisms (SNPs) in treated genomic DNA (SEQ ID NO: 23 to SEQ ID NO: 24 and SEQ ID NO: 51 to SEQ ID NO: 52), or in genomic DNA (SEQ ID NO: 5 and sequences complementary thereto).

At least one, and more preferably all members of a set of oligonucleotides is bound to a solid phase.

Disclosed is a set of at least two (2) oligonucleotides that are used as 'primer' oligonucleotides for amplifying DNA sequences of one of SEQ ID NO:5, SEQ ID NO: 23 to SEQ ID NO: 24 and SEQ ID NO: 51 to SEQ ID NO: 52 and sequences complementary thereto, or segments thereof.

It is anticipated that the oligonucleotides may constitute all or part of an "array" or "DNA chip" (*i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminium, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labelled probes are often used for the scanning of immobilized DNA arrays. The simple attachment of Cy3 and Cy5 dyes to the 5'-OH of the specific probe are particularly suitable for fluorescence labels. The detection of the fluorescence of the hybridised probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e*., each analyte) can be uniquely bound by a unique label and thus detected (each label is directly counted, resulting in a digital read-out of each molecular species in the mixture).

It is particularly preferred that the oligomers are utilised for at least one of: detection of; diagnosis of; treatment of; monitoring of; and treatment and monitoring of colorectal cell proliferative disorders. This is enabled by use of said sets for the detection of one or more of the following classes of tissues: colorectal carcinoma, colon adenoma, inflammatory colon tissue, grade 2 dysplasia colon adenomas less than 1 cm, grade 3 dysplasia colon adenomas larger than 1 cm, normal colon tissue, non-colon healthy tissue and non-colon cancer tissue.

Particularly preferred are those sets of oligomers according to the Examples.

In the most preferred embodiment of the method, the presence or absence of colorectal carcinoma is determined. This is achieved by analysis of the methylation status of at least one target sequence comprising at least one CpG position said sequence comprising, or hybridizing under stringent conditions to at least 16 contiguous nucleotides of a sequence SEQ ID NO: 5 ) and complements thereof. This is achieved by analysis of the methylation status of a target sequence comprising at least one CpG position said sequence comprising, or hybridizing under stringent conditions to at least 16 contiguous nucleotides of a sequence selected from the group consisting SEQ ID NO: 12 TO SEQ ID NO: 14 and at least one further target sequence comprising at least one CpG position said sequence comprising, or hybridizing under stringent conditions to at least 16 contiguous nucleotides of a sequence SEQ ID NO:5 .

Disclosed is further a method for ascertaining genetic and/or epigenetic parameters of the genomic sequence according to SEQ ID NO:5 within a subject by analysing cytosine methylation and single nucleotide polymorphisms. Said method comprising contacting a nucleic acid comprising SEQ ID NO:5 in a biological sample obtained from said subject with at least one reagent or a series of reagents, wherein said reagent or series of reagents, distinguishes between methylated and non-methylated CpG dinucleotides within the target nucleic acid.

Said method may comprise the following steps: In the *first step,* a sample of the tissue to be analysed is obtained. The source may be any suitable source, such as cell lines, histological slides, biopsies, paraffin-embedded tissue, body fluids, stool, colonic effluent, urine, blood plasma, blood serum, whole blood, isolated blood cells, cells isolated from the blood and all possible combinations thereof. It is preferred that said sources of DNA are stool or body fluids selected from the group consisting colonic effluent, urine, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Wherein the sample DNA is not enclosed in a membrane (e.g. circulating DNA from a blood sample) methods standard in the art for the isolation and/or purification of DNA may be employed. Such methods include the use of a protein degenerating reagent e.g. chaotropic salt e.g. guanidine hydrochloride or urea; or a detergent e.g. sodium dodecyl sulphate (SDS), cyanogen bromide. Alternative methods include but are not limited to ethanol precipitation or propanol precipitation, vacuum concentration amongst others by means of a centrifuge. The person skilled in the art may also make use of devices such as filter devices e.g.ultrafiltration , silica surfaces or membranes, magnetic particles, polystyrol particles, polystyrol surfaces, positively charged surfaces, and positively charged membranse, charged membranes, charged surfaces, charged switch membranes, charged switched surfaces.

Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridisation behaviour. This will be understood as 'pre-treatment' or 'treatment' herein.

This is preferably achieved by means of treatment with a bisulfite reagent. The term "bisulfite reagent" refers to a reagent comprising bisulfite, disulfite, hydrogen sulfite or combinations thereof, useful as disclosed herein to distinguish between methylated and unmethylated CpG dinucleotide sequences. Methods of said treatment are known in the art (e.g. WO 2005/038051 ). It is preferred that the bisulfite treatment is conducted in the presence of denaturing solvents such as but not limited to n-alkylenglycol, particularly diethylene glycol dimethyl ether (DME), or in the presence of dioxane or dioxane derivatives. In a preferred embodiment the denaturing solvents are used in concentrations between 1% and 35% (v/v). It is also preferred that the bisulfite reaction is carried out in the presence of scavengers such as but not limited to chromane derivatives, e.g., 6-hydroxy-2, 5,7,8, -tetramethylchromane 2-carboxylic acid or trihydroxybenzoe acid and derivates thereof, e.g. Gallic acid (see: WO 2005/038051 ). The bisulfite conversion is preferably carried out at a reaction temperature between 30°C and 70°C, whereby the temperature is increased to over 85 °C for short periods of times during the reaction . The bisulfite treated DNA is preferably purified priori to the quantification. This may be conducted by any means known in the art, such as but not limited to ultrafiltration, preferably carried out by means of Microcon^(TM) columns (manufactured by Millipore^(TM)). The purification is carried out according to a modified manufacturer's protocol.

In the *third step* of the method, fragments of the treated DNA are amplified, using sets of primer oligonucleotides, and an amplification enzyme. The amplification of several DNA segments can be carried out simultaneously in one and the same reaction vessel. Typically, the amplification is carried out using a polymerase chain reaction (PCR). Preferably said amplificates are 100 to 2,000 base pairs in length. The set of primer oligonucleotides includes at least two oligonucleotides whose sequences are each reverse complementary, identical, or hybridise under stringent or highly stringent conditions to an at least 16-base-pair long segment of the base sequences of one of SEQ ID NO:23 to SEQ ID NO: 24 and SEQ ID NO: 51 to SEQ ID NO: 52 and sequences complementary thereto. It is preferred that the set of primer oligonucleotides further comprises at least two oligonucleotides whose sequences are each reverse complementary, identical, or hybridise under stringent or highly stringent conditions to an at least 16-base-pair long segment of the base sequences of one of SEQ ID NO: 37 TO SEQ ID NO: 42 AND SEQ ID NO: 65 TO SEQ ID NO: 70 and sequences complementary thereto.
Alternatively, the methylation status of pre-selected CpG positions within at least one nucleic acid sequences selected from the group consisting SEQ ID NO:5 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridises to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridises to a treated nucleic acid sequence according to one of SEQ ID NO: 23 to SEQ ID NO: 24 and SEQ ID NO:51 to SEQ ID NO:52 and sequences complementary thereto, wherein the base sequence of said oligomers comprises at least one CpG dinucleotide .A further preferred embodiment of the method comprises the use of *blocker* oligonucleotides (the HeavyMethyl™ assay). The use of such blocker oligonucleotides has been described by Yu et al., BioTechniques 23:714-720, 1997. Blocking probe oligonucleotides are hybridised to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g.*, with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridises to a treated nucleic acid sequence according to one of SEQ ID NO: 23 to SEQ ID NO: 24 and SEQ ID NO:51 to SEQ ID NO:52 and sequences complementary thereto, wherein the base sequence of said oligonucleotides comprises at least one CpG, TpG or CpA dinucleotide. Also preferred is a blocking oligonucleotides having a length of at least 9 nucleotides which hybridises to a treated nucleic acid sequence according to one of SEQ ID NO: 37 TO SEQ ID NO: 42 AND SEQ ID NO: 65 TO SEQ ID NO: 70 and sequences complementary thereto, wherein the base sequence of said oligonucleotides comprises at least one CpG, TpG or CpA dinucleotide

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labelled amplificates have a single positive or negative net charge, allowing for better delectability in the mass spectrometer. The detection may be carried out and visualized by means of, *e.g.*, matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule Into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analysed by means of based-based methods such as, but not limited to, array technology and probe based technologies as well as by means of techniques such as sequencing and template directed extension.

In one embodiment of the method, the amplificates synthesised in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide. The hybridizing portion of the hybridizing nucleic acids is typically at least 9, 15, 20, 25, 30 or 35 nucleotides in length. However, longer molecules have utility.

Preferably, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within a sequence SEQ ID NO: 51 , and the equivalent positions within SEQ ID NO: 23 to SEQ ID NO: 24 and SEQ ID NO: 51 to SEQ ID NO: 52. Said oligonucleotides may also be present in the form of peptide nucleic acids. The non-hybridised amplificates are then removed. The hybridised amplificates are then detected. In this context, it is preferred that labels attached to the amplificates are identifiable at each position of the solid phase at which an oligonucleotide sequence is located.

The genomic methylation status of the CpG positions may be ascertained by means of oligonucleotide probes (as detailed above) that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labelled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridise to a CpG-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLightTM™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotide.

In a further preferred embodiment of the method, the *fourth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467,1977).

### Best mode

Most preferred the genomic nucleic acids are isolated and treated according to the first three steps of the method outlined above, namely:
a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides,* and further wherein
e) detecting of the amplificates is carried out by means of a real-time detection probe, as described above.
Preferably, where the subsequent amplification of d) is carried out by means of methylation specific primers, as described above, said methylation specific primers comprise a sequence having a length of at least 9 nucleotides which hybridises to a treated nucleic acid sequence according to one of SEQ ID NO: 23 to SEQ ID NO: 24 and SEQ ID NO: 51 to SEQ ID NO: 52 and sequences complementary thereto, wherein the base sequence of said oligomers comprise at least one CpG dinucleotide. It is particularly preferred that the subsequent amplification of d) is additionally carried out by means of methylation specific primers each comprising a sequence having a length of at least 9 nucleotides which hybridises to a treated nucleic acid sequence according to one of SEQ ID NO: 37 TO SEQ ID NO: 42 AND SEQ ID NO: 65 TO SEQ ID NO: 70 and sequences complementary thereto, wherein the base sequence of said oligomers comprise at least one CpG dinucleotide.

Step e) of the method, namely the detection of the specific amplificates indicative of the methylation status of one or more CpG positions of at least one sequence of the group comprising SEQ ID NO: 5 is carried out by means of *real-time* detection methods as described above.

Disclosed is a method for the analysis of the methylation status of genomic DNA (SEQ ID NO: 5 and furthermore SEQ ID NO: 12 TO SEQ ID NO: 14 and complements thereof) without the need for bisulfite conversion. Methods are known in the art wherein a methylation sensitive restriction enzyme reagent, or a series of restriction enzyme reagents comprising methylation sensitive restriction enzyme reagents that distinguishes between methylated and non-methylated CpG dinucleotides within a target region are utilized in determining methylation, for example but not limited to DMH.

In the *first step* of such additional embodiments, the genomic DNA sample is isolated from tissue or cellular sources. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants, *e.g*., by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. All clinical sample types comprising neoplastic or potentially neoplastic matter are suitable for us e in the present method, preferred are cell lines, histological slides, biopsies, paraffin-embedded tissue, body fluids, stool, colonic effluent, urine, blood plasma, blood serum, whole blood, isolated blood cells, cells isolated from the blood and combinations thereof. Body fluids are the preferred source of the DNA; particularly preferred are blood plasma, blood serum, whole blood, isolated blood cells and cells isolated from the blood.

Once the nucleic acids have been extracted, the genomic double-stranded DNA is used in the analysis.

The DNA may be cleaved prior to treatment with methylation sensitive restriction enzymes. Such methods are known in the art and may include both physical and enzymatic means. Particularly preferred is the use of one or a plurality of restriction enzymes which are not methylation sensitive, and whose recognition sites are AT rich and do not comprise CG dinucleotide. The use of such enzymes enables the conservation of CpG islands and CpG rich regions in the fragmented DNA. The non-methylation-specific restriction enzymes are preferably selected from the group consisting of MseI, Bfal, Csp6I, Tru1I, Tvu1I, Tru9I, Tvu9I, Mael and Xspl. Particularly preferred is the use of two or three such enzymes. Particularly preferred is the use of a combination of Msel, Bfal and Csp6I.

The fragmented DNA may then be ligated to adaptor oligonucleotides in order to facilitate subsequent enzymatic amplification. The ligation of oligonucleotides to blunt and sticky ended DNA fragments is known in the art, and is carried out by means of dephosphorylation of the ends (e.g. using calf or shrimp alkaline phosphatase) and subsequent ligation using ligase enzymes (e.g. T4 DNA ligase) in the presence of dATPs. The adaptor oligonucleotides are typically at least 18 base pairs in length.

In the *third step,* the DNA (or fragments thereof) is then digested with one or more methylation sensitive restriction enzymes. The digestion is carried out such that hydrolysis of the DNA at the restriction site is informative of the methylation status of a specific CpG dinucleotide of at least GLI3

It is particularly preferred that the digestion is carried out such that hydrolysis of the DNA at the restriction site is informative of the methylation status of at least one CpG dinucleotide of the gene SEPTIN 9 and furthermore of at least one CpG dinucleotide of at least , GLI3. Preferably said group consists of the genes

Preferably, the methylation-specific restriction enzyme is selected from the group consisting of *Bsi E1, Hga I HinPI*, *Hpy99I, Ava I, Bce AI, Bsa HI, BisI, BstUI*, *Bshl236I, AccII, BstFNI, McrBC, GlaI, MvnI, HpaII* (*HapII*), *HhaI, AciI, SmaI, HinP1I, HpyCH4IV, EagI* and mixtures of two or more of the above enzymes. Preferred is a mixture containing the restriction enzymes BstUI, HpaII, HpyCH4IV and HinP1I.

In the *fourth step,* which is optional but preferred, the restriction fragments are amplified. This is preferably carried out using a polymerase chain reaction, and said amplificates may carry suitable detectable labels as discussed above, namely fluorophore labels, radionuclides and mass labels. Particularly preferred is amplification by means of an amplification enzyme and at least two primers comprising, in each case a contiguous sequence at least 16 nucleotides in length that is complementary to, or hybridizes under moderately stringent or stringent conditions to a sequence SEQ ID NO:5, and complements thereof. Further preferred is amplification by means of an amplification enzyme, at least two primers comprising, in each case a contiguous sequence at least 16 nucleotides in length that is complementary to, or hybridizes under moderately stringent or stringent conditions to a sequence SEQ ID NO: 5, and complements thereof and at least two primers comprising, in each case a contiguous sequence at least 16 nucleotides in length that is complementary to, or hybridizes under moderately stringent or stringent conditions to a sequence selected from the group consisting SEQ ID NO: 12 TO SEQ ID NO: 14, and complements thereof. Preferably said contiguous sequence is at least 16, 20 or 25 nucleotides in length. Alternatively, said primers may be complementary to any adaptors linked to the fragments.

In the *fifth step* the amplificates are detected. The detection may be by any means standard in the art, for example, but not limited to, gel electrophoresis analysis, hybridisation analysis, incorporation of detectable tags within the PCR products, DNA array analysis, MALDI or ESI analysis. Preferably said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence SEQ ID NO:5 , and complements thereof. It is further preferred that said detection is carried out by hybridisation to at least one nucleic acid or peptide nucleic acid comprising in each case a contiguous sequence at least 16 nucleotides in length that is complementary to, or hybridizes under moderately stringent or stringent conditions to a sequence SEQ ID NO:5 , and complements thereof and additionally at least one nucleic acid or peptide nucleic acid comprising in each case a contiguous sequence at least 16 nucleotides in length that is complementary to, or hybridizes under moderately stringent or stringent conditions to a sequence selected from the group consisting SEQ ID NO: 12 TO SEQ ID NO: 14 Preferably said contiguous sequence is at least 16, 20 or 25 nucleotides in length.

Subsequent to the determination of the methylation state or level of the genomic nucleic acids the presence or absence of cellular proliferative disorder (most preferably colorectal carcinoma) is deduced based upon the methylation state or level of at least one CpG dinucleotide sequence of at least one sequence SEQ ID NO:5 , or an average, or a value reflecting an average methylation state of a plurality of CpG dinucleotide sequences of at least one sequence selected from the group consisting SEQ ID NO: 5 wherein methylation is associated with the presence of colorectal carcinoma. It is further preferred that the determination of the methylation state or level of the genomic nucleic acids the presence or absence of cellular proliferative disorder (most preferably colorectal carcinoma) is deduced based upon the methylation state or level of at least one CpG dinucleotide sequence of the gen Septin 9 and furthermore of at least one CpG dinucleotide sequence of at least one sequence selected from the group consisting SEQ ID NO: 12 TO SEQ ID NO: 14, or an average, or a value reflecting an average methylation state of a plurality of CpG dinucleotide sequences of at least one sequence SEQ ID NO: 5 wherein methylation of any of the analysed CpG positions is associated with the presence of colorectal carcinoma.

Wherein said methylation is determined by quantitative means the cut-off point for determining said the presence of methylation is preferably zero (i.e. wherein a sample displays any degree of methylation it is determined as having a methylated status at the analysed CpG position). Nonetheless, it is foreseen that the person skilled in the art may wish to adjust said cut-off value in order to provide an assay of a particularly preferred sensitivity or specificity. Accordingly said cut-off value may be increased (thus increasing the specificity), said cut off value may be within a range selected from the group consisting of 0%-5%, 5%-10%, 10%-15%, 15%-20%, 20%-30% and 30%-50%. Particularly preferred are the cut-offs 10%, 15%, 25%, and 30%.

Disclosed is a method wherein a panel of genes comprising the Septin 9 or its truncated transcript Q9HC74 and at least GLI3, subsequent to the determination of the methylation state of the genomic nucleic acids the presence or absence of cellular proliferative disorders, in particular colorectal cell proliferative disorder is deduced based upon the methylation state of at least one CpG dinucleotide sequence of SEQ ID NO: 12 to SEQ ID NO: 14 and at least one CpG dinudeotide sequence of SEQ ID NO: 5, or an average, or a value reflecting an average methylation state of a plurality of CpG dinucleotide sequences thereof wherein hypermethylation is associated with cancers, in particular colorectal cancer.

### Diagnostic and Prognostic Assays for cellular proliferative disorders

The present disclosure enables diagnosis of events which are disadvantageous to patients or individuals in which important genetic and/or epigenetic parameters within may be used as markers. Said parameters may be compared to another set of genetic and/or epigenetic parameters, the differences serving as the basis for a diagnosis and/or prognosis of events which are disadvantageous to patients or individuals.

More specifically the present disclosure enables the screening of at-risk populations for the early detection of cancers, most preferably colorectal carcinomas. Neoplastic cellular proliferative disorders, most particularly carcinomas, present decreased methylation (i.e. decreased expression) within GLI3, as opposed to benign disorders and normal tissues which do not.

Specifically disclosed are diagnostic cancer assays based on measurement of differential expression (preferably methylation) of one or more CpG dinucleotide sequences of at least one sequence selected from the group consisting SEQ ID NO: 5 that comprise such a CpG dinucleotide sequence. Typically, such assays involve obtaining a sample from a subject, performing an assay to measure the expression of at least GLI3, preferably by determining the methylation status of a sequence SEQ ID NO: 5 , derived from the sample, relative to a control sample, or a known standard and making a diagnosis based thereon.

Preferred oligomers are used to assess the CpG dinucleotide methylation status, such as those based on SEQ ID NO: 5, SEQ ID NO: 23 to SEQ ID NO: 24 and AND SEQ ID NO: 51 to SEQ ID NO: 52, or arrays thereof, as well as in kits based thereon and useful for the diagnosis of cellular proliferative disorders, most preferably colorectal carcinoma.

### Kits

Moreover, an additional aspect of the present disclosure is a kit comprising: a means for determining methylation of at least GLI3.

The means for determining methylation comprise preferably a bisulfite-containing reagent; one or a plurality of oligonucleotides consisting whose sequences in each case are identical, are complementary, or hybridise under stringent or highly stringent conditions to an at least 9 or more preferably 18 base long segment of a sequence selected from SEQ ID NO: 23 to SEQ ID NO: 24 and SEQ ID NO: 51 to SEQ ID NO: 52; and optionally instructions for carrying out and evaluating the described method of methylation analysis. The base sequence of said oligonucleotides comprises at least one CpG, CpA or TpG dinucleotide. Said kit may further comprise one or a plurality of oligonucleotides whose sequences in each case are identical, are complementary, or hybridise under stringent or highly stringent conditions to an at least 9 or more preferably 18 base long segment of a sequence selected from SEQ ID NO: 37 TO SEQ ID NO: 42 AND SEQ ID NO: 65 TO SEQ ID NO: 70.

Said kit may further comprise standard reagents for performing a CpG position-specific methylation analysis, wherein said analysis comprises one or more of the following techniques: MS-SNuPE, MSP, MethyLight™, HeavyMethyl, COBRA, and nucleic acid sequencing. However, a kit can also contain only part of the aforementioned components.

The kit may comprise additional bisulfite conversion reagents selected from the group consisting: DNA denaturation buffer; sulfonation buffer; DNA recovery reagents or kits (*e*.*g*., precipitation, ultrafiltration, affinity column); desulfonation buffer; and DNA recovery components. Alternatively, the kit may contain, packaged in separate containers, a polymerase and a reaction buffer optimised for primer extension mediated by the polymerase, such as PCR. The kit may further comprise means for obtaining a biological sample of the patient. Preferred is a kit, which further comprises a container suitable for containing the means for determining methylation of GLI3, in the biological sample of the patient, and most preferably further comprises instructions for use and interpretation of the kit results. Preferrably, the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to an at least 9 or more preferably 18 base long segment of a sequence selected from SEQ ID NO: 23 to SEQ ID NO: 24 and SEQ ID NO: 51 to SEQ ID NO: 52; and optionally (d) instructions for use and interpretation of the kit results. Alternatively the kit comprises: (a) a bisulfite reagent; (b) a container suitable for containing the said bisulfite reagent and the biological sample of the patient; (c) at least one oligonucleotides and/or PNA-oligomer having a length of at least 9 or 16 nucleotides which is identical to or hybridises to a pre-treated nucleic acid sequence according to one of SEQ ID NO: 23 to SEQ ID NO: 24 and SEQ ID NO: 51 to SEQ ID NO: 52 and sequences complementary thereto; and optionally (d) instructions for use and interpretation of the kit results.

Alternatively the kit comprises: (a) a bisulfite reagent; (b) a container suitable for containing the said bisulfite reagent and the biological sample of the patient; (c) at least one set of primer oligonucleotides containing two oligonucleotides whose sequences in each case are identical, are complementary, or hybridise under stringent or highly stringent conditions to an at least 9 or more preferably 18 base long segment of a sequence selected from SEQ ID NO: 23 to SEQ ID NO: 24 and SEQ ID NO: 51 to SEQ ID NO: 52; (d) at least one oligonucleotides and/or PNA-oligomer having a length of at least 9 or 16 nucleotides which is identical to or hybridises to a pre-treated nucleic acid sequence according to one of SEQ ID NO: 23 to SEQ ID NO: 24 and SEQ ID NO: 51 to SEQ ID NO: 52 and sequences complementary thereto; and optionally (e) instructions for use and interpretation of the kit results.

Said kits may further comprise at least one set of primer oligonucleotides containing two oligonucleotides whose sequences in each case are identical, are complementary, or hybridise under stringent or highly stringent conditions to a 9 or more preferably 18 base long segment of a sequence selected from SEQ ID NO: 37 TO SEQ ID NO: 42 AND SEQ ID NO: 65 TO SEQ ID NO: 70.

The kit may also contain other components such as buffers or solutions suitable for blocking, washing or coating, packaged in a separate container.

Typical reagents (*e*.*g*., as might be found in a typical COBRA^{™}-based kit) for COBRA^{™} analysis may include, but are not limited to: PCR primers for GLI3; restriction enzyme and appropriate buffer; gene-hybridization oligo; control hybridization oligo; kinase labeling kit for oligo probe; and labeled nucleotides. Typical reagents (*e*.*g*., as might be found in a typical MethyLight^{™} -based kit) for MethyLight^{™} analysis may include, but are not limited to: PCR primers for the bisulfite converted sequence of GLI3; bisulfite specific probes (e.g. TaqMan^{™} or Lightcycler^{™)}; optimized PCR buffers and deoxynucleotides; and Taq polymerase.

Typical reagents (*e.g*., as might be found in a typical Ms-SNuPE^{™}-based kit) for Ms-SNuPE7m analysis may include, but are not limited to: PCR primers for specific gene (or bisulfite treated DNA sequence or CpG island); optimized PCR buffers and deoxynucleotides; gel extraction kit; positive control primers; Ms-SNuPE^{™} primers for the bisulfite converted sequence of GLI3; reaction buffer (for the Ms-SNuPE reaction); and labelled nucleotides.
Typical reagents (*e*.*g*., as might be found in a typical MSP-based kit) for MSP analysis may include, but are not limited to: methylated and unmethylated PCR primers for the bisulfite converted sequence of or genomic sequence from GLI3, optimized PCR buffers and deoxynucleotides, and specific probes.
Moreover, an additional aspect of the present invention is an alternative kit comprising a means for determining methylation of at least GLI3, wherein said means comprise preferably at least one methylation specific restriction enzyme; one or a plurality of primer oligonucleotides (preferably one or a plurality of primer pairs) suitable for the amplification of a sequence comprising at least one CpG dinucleotide of a sequence selected from SEQ ID NO: 5; and optionally instructions for carrying out and evaluating the described method of methylation analysis. In one embodiment the base sequence of said oligonucleotides are identical, are complementary, or hybridise under stringent or highly stringent conditions to an at least 18 base long segment of a sequence SEQ ID NO: 5.

Preferably said kit further comprises one or a plurality of primer oligonucleotides (preferably one or a plurality of primer pairs) suitable for the amplification of a sequence comprising at least one CpG dinucleotide of a sequence selected from SEQ ID NO: 12 TO SEQ ID NO: 14.

Further disclosed said kit may comprise one or a plurality of oligonucleotide probes for the analysis of the digest fragments, preferably said oligonucleotides are identical, are complementary, or hybridise under stringent or highly stringent conditions to an at least 16 base long segment of a sequence selected from SEQ ID NO: 5.

Further said kit may further comprise one or a plurality of oligonucleotide probes for the analysis of the digest fragments, preferably said oligonucleotides are identical, are complementary, or hybridise under stringent or highly stringent conditions to an at least 16 base long segment of a sequence selected from SEQ ID NO: 12 TO SEQ ID NO: 14.

Preferred, the kit may comprise additional reagents selected from the group consisting: buffer (e.g. restriction enzyme, PCR, storage or washing buffers); DNA recovery reagents or kits (*e*.*g*., precipitation, ultrafiltration, affinity column) and DNA recovery components.

Alternatively, the kit may contain, packaged in separate containers, a polymerase and a reaction buffer optimised for primer extension mediated by the polymerase, such as PCR. The kit may further comprise means for obtaining a biological sample of the patient Preferrably, the kit comprises: (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridise under stringent or highly stringent conditions to an at least 9 base long segment of a sequence SEQ ID NO: 5; and optionally (d) instructions for use and interpretation of the kit results. In one embodiment said kit further comprises (e) at least one set of oligonucleotides one or a plurality of nucleic acids or peptide nucleic acids which are identical, are complementary, or hybridise under stringent or highly stringent conditions to an at least 9 base long segment of a sequence selected from SEQ ID NO: 12 TO SEQ ID NO: 14.

Alternatively, the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence SEQ ID NO: 5; and optionally (d) instructions for use and interpretation of the kit results. In one embodiment said kit further comprises (e) at least one set of primer oligonucleotides suitable for the amplification of a sequence comprising at least one CpG dinucleotide of a sequence selected from SEQ ID NO: 12 TO SEQ ID NO: 14.

Alternatively, the kit comprises: (a) a methylation sensitive restriction enzyme reagent; (b) a container suitable for containing the said reagent and the biological sample of the patient; (c) at least one set of primer oligonucleotides suitable for the amplification of a sequence comprising at least one CpG dinucleotide of a sequence SEQ ID NO: 5; (d) at least one set of oligonucleotides one or a plurality of nucleic acids or peptide nucleic acids which are identical, are complementary, or hybridise under stringent or highly stringent conditions to an at least 9 base long segment of a sequence SEQ ID NO: 5 and optionally (e) instructions for use and interpretation of the kit results. Said kit may further comprise (f) at least one set of primer oligonucleotides suitable for the amplification of a sequence comprising at least one CpG dinucleotide of a sequence selected from SEQ ID NO: 12 TO SEQ ID NO: 14.

The kit may also contain other components such as buffers or solutions suitable for blocking, washing or coating, packaged in a separate container.

The disclosure further relates to a kit for use in providing a diagnosis of the presence of a cell proliferative disorder in a subject by means of methylation-sensitive restriction enzyme analysis. Said kit comprises a container and a DNA microarray component. Said DNA microarray component being a surface upon which a plurality of oligonucleotides are immobilized at designated positions and wherein the oligonucleotide comprises at least one CpG methylation site. At least one of said oligonucleotides is specific for GLI3 and comprises a sequence of at least 15 base pairs in length but no more than 200 bp of a sequence according to SEQ ID NO: 5. Preferably said sequence is at least 15 base pairs in length but no more than 80 bp of a sequence according to SEQ ID NO: 5 It is further preferred that said sequence is at least 20 base pairs in length but no more than 30 bp of a sequence according to SEQ ID NO: 5. Said kit may further comprise a sequence of at least 15 base pairs in length but no more than 200 bp of a sequence according to one of SEQ ID NO: 12 TO SEQ ID NO: 14. Preferably said sequence is at least 15 base pairs in length but no more than 80 bp of a sequence according to one of SEQ ID NO: 12 TO SEQ ID NO: 14. It is further preferred that said sequence is at least 20 base pairs in length but no more than 30 bp of a sequence according to one of SEQ ID NO: 12 TO SEQ ID NO: 14.

Said test kit preferably further comprises a restriction enzyme component comprising one or a plurality of methylation-sensitive restriction enzymes.

Said test kit is further characterized in that it comprises at least one methylation-specific restriction enzyme, and wherein the oligonucleotides comprise a restriction site of said at least one methylation specific restriction enzymes. The kit may further comprise one or several of the following components, which are known in the art for DNA enrichment: a protein component, said protein binding selectively to methylated DNA; a triplex-forming nucleic acid component, one or a plurality of linkers, optionally in a suitable solution; substances or solutions for performing a ligation e.g. ligases, buffers; substances or solutions for performing a column chromatography; substances or solutions for performing an immunology based enrichment (e.g. immunoprecipitation); substances or solutions for performing a nucleic acid amplification e.g. PCR; a dye or several dyes, if applicable with a coupling reagent, if applicable in a solution; substances or solutions for performing a hybridization; and/or substances or solutions for performing a washing step. The disclosure further provides a composition of matter useful for the detection of colon cell proliferative disorders. Said composition comprising at least one nucleic acid 18 base pairs in length of a segment of the nucleic acid sequence disclosed in SEQ ID NO: 23 to SEQ ID NO: 24 and SEQ ID NO: 51 to SEQ ID NO: 52, and one or more substances taken from the group comprising :
1-5 mM Magnesium Chloride, 100-500 µM dNTP, 0.5-5 units of taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 23 to SEQ ID NO: 24 AND SEQ ID NO: 51 TO SEQ ID NO: 52 and sequences complementary thereto. In one aspect of the disclosure said composition further comprises an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID NO: 37 TO SEQ ID NO: 42 AND SEQ ID NO: 65 TO SEQ ID NO: 70 and sequences complementary thereto. It is preferred that said composition of matter comprises a buffer solution appropriate for the stabilization of said nucleic acid in an aqueous solution and enabling polymerase based reactions within said solution.. Suitable buffers are known in the art and commercially available.

Preferrably, said at least one oligomer is at least 50,100,150,200,250 or 500 base pairs in length of a segment of the nucleic acid sequence disclosed in SEQ ID NO: 23 to SEQ ID NO: 24 and SEQ ID NO: 51 to SEQ ID NO: 52, or SEQ ID NO: 37 to SEQ ID NO: 42 and SEQ ID NO: 65 to SEQ ID NO: 70.

### EXAMPLES

### Example 1

In the following example a variety of assays suitable for the methylation analysis of the genes and genomic sequences according to tables 1 and 2 were designed, in order to validate the suitable of said markers for the detection of colorectal carcinoma. Furthermore the performance of gene panels (combinations of a plurality of markers) was assessed, of particular interest were panels comprising the gene Septin 9 that provided improved accuracy over the use of Septin 9 alone.

The assays were designed to be run on the LightCycler platform (Roche Diagnostics), but other such instruments commonly used in the art are also suitable. The assays were MSP assays. MSP amplificates were designed to be detected by means of Taqman style fluorescent labelled detection probes.

### Samples

In total 314 samples were analysed:
198 colorectal carcinoma of the following stages:
   - Stage 0: 4 samples
   - Stage 1:19 samples
   - Stage 2: 84 samples
   - Stage 3: 57 samples
   - Stage 4:20 samples
   - Stage unknown: 14 samples
22 normal or normal adjacent tissue
26 whole blood samples
40 other cancers (liver, breast & prostate)
28 other normal or normal adjacent tissues (liver, breast & prostate)

### DNA extraction and bisulfite treatment

The DNA was isolated from the all samples according to a modified protocol based on that disclosed in the Qiagen Genomic DNA Handbook (August 2001) (pg 28-31, 44-47). The eluate resulting from the purification was then converted according to the following bisulfite reaction.

The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for a total of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon™ column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50° C). The membrane was turned over according to the manufacturer's instructions. Subsequently a repeated centrifugation was conducted, with which the DNA was removed from the membrane. 10 µl of the eluate was utilized for the Lightcycler Real Time PCR assay.

PCR assay component sequences and thermal cycling conditions are provided in Table 3.

### Control assay

### Control assay

The GSTP1-C3 assay design makes it suitable for quantitating DNAs from different sources, including fresh/frozen samples, remote samples such as plasma or serum, and DNA obtained from archival specimen such as paraffin embedded material. The following oligonucleotides were used in the reaction to amplify the control amplificate:
Control Primer1: GGAGTGGAGGAAATTGAGAT (SEQ ID NO: 71)
Control Primer2: CCACACAACAAATACTCAAAAC (SEQ ID NO: 72)
Control Probe: FAM-TGGGTGTTTGTAATTTTTGTTTTGTGTTAGGTT-TAMRA (SEQ ID NO: 73)

| | |
|---|---|
| Cycle program (40 cycles): | 95 °C, 10 min |
| | 95 °C, 15 sec |
| | 58 °C, 1 min |

### Data interpretation

### Calculation of DNA concentration.

The Cp (crossing point values) as calculated by the Lightcycler instrument software were used to determine DNA concentration. The DNA concentration was calculated by reference of the CP value of each well to a standard curve for both the methylation assays and the C3 assay.

### Percentage methylation

For each sample the detected percentage methylation was calculated as the measured concentration of DNA quantified using the methylation assays over the concentration of DNA in the sample as quantified by the C3 assay.

Detection of methylation was determined at multiple different threshold levels, see tables) as well as at all methylation levels (i.e. any samples wherein methylation was detected were deemed positive).

The sensitivity of each assay was determined from the colorectal carcinoma sample positive detection rate, wherein sensitivity was determined as the % samples wherein methylation was positively detected (i.e. true positives).
The specificity of each assay was determined from the whole blood sample negative detection rate (i.e. true negative detection rate) wherein false positives were discounted from the total number of analysed samples.

### Results

The AUC of each analyzed marker both alone or in some cases in combination with other markers is provided in Tables 4 to 6 (with the exception of MRPS21 & DOCK10). The term 'AUC' is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

Table 4 provides an overview of the measured AUC of each marker in the detection of colorectal carcinoma at all methylation levels, provides information on their complementarity to Septin 9, and also their methylation AUC in whole blood. It is necessary to determine the methylation of a marker in whole blood, as the preferred sample type for a screening assay would be blood, accordingly markers that are methylated in both blood and cancer are not preferred.

Table 5 provides an overview of the measured AUC of each marker in the detection of colorectal carcinoma at methylation cut off values of 10%, 20% and 30%.

Table 6 provides an overview of the measured AUC of panels of markers comprising the gene Septin 9 and 2 further markers in the detection of colorectal carcinoma at methylation cut off values of 0%, 10%, 20% and 30%. The panel data was compiled by determining the proportion or number of the analysed samples with methylation measured within a given threshold using at least one assay of the panel.

For MRPS21 & DOCK10 assay performance is provided in Figures 27 and 28. Sensitivity of the DOCK10 assay was 0.48 as measured at a specificity of 0.97 (cut off was -3). Sensitivity of the MRPS21 assay was 0.63 as measured at a specificity of 0.96 (cutoff was -1.422).

### Example 2

In the following investigation, the performace of selected markers from example 1, according to Table 7 were selected for further analysis by means of the HM (Heavymethy) assay. Target regions of each gene were bisulfite converted and amplified by means of non-MSP primers, in the presence of a blocker oligonucleotides designed to suppress amplificates that had not been methlated prior to bisulfite treatment. Amplificates were then detected by means of Lightcycler (dual) probes.

Plasma samples from the following patient classes were analysed:
- Colorectal carcinoma (131 total)
   Stage 0 = 1
   Stage I = 13
   Stage II = 32
   Stage III = 27
   Stage IV = 8
   Unclassified = 50
- Healthy colorectal (colonoscopy verified) = 169
- Non- cancerous diseases (NCD) = 29
- Cancers of non-colorectal origin (NCC) = 31

In total 360 samples were analysed.

### DNA extraction and bisulfite treatment,

The DNA was isolated from the all samples by means of the Magna Pure method (Roche) according to the manufacturer's instructions. The eluate resulting from the purification was then converted according to the following bisulfite reaction.
The eluate was mixed with 354 µl of bisulfite solution (5.89 mol/l) and 146 µl of dioxane containing a radical scavenger(6-hydroxy-2,5,7,8-tetramethylchromane 2-carboxylic acid, 98.6 mg in 2.5 ml of dioxane). The reaction mixture was denatured for 3 min at 99°C and subsequently incubated at the following temperature program for atotal of 7 h min 50°C; one thermospike (99.9°C) for 3 min; 1.5 h 50°C; one thermospike (99°C) for 3 min; 3 h 50°C. The reaction mixture was subsequently purified by ultrafiltration using a Millipore Microcon ™ column. The purification was conducted essentially according to the manufacturer's instructions. For this purpose, the reaction mixture was mixed with 300 µl of water, loaded onto the ultrafiltration membrane, centrifuged for 15 min and subsequently washed with 1 x TE buffer. The DNA remains on the membrane in this treatment. Then desulfonation is performed. For this purpose, 0.2 mol/l NaOH was added and incubated for 10 min. A centrifugation (10 min) was then conducted, followed by a washing step with 1 x TE buffer. After this, the DNA was eluted. For this purpose, the membrane was mixed for 10 minutes with 75 µl of warm 1 x TE buffer (50°C). The membrane was turned over according to the manufacturer's instructions. Subsequently a repeated centrifugation was conducted, with which the DNA was removed from the membrane. 10 µl of the eluate was utilized for the Lightcycler Real Time PCR assay.

### Reaction solutions and thermal cycling conditions

PCR assay component sequences are provided in Table 10. Each assay was performed twice (independently) in each sample.

Thermal cycling conditions were:

**PCDHGC3**

| | | |
|---|---|---|
| activation: | 95°C | 10min |
| | | |
| 50 cycles: | 95°C | 10 sec (20°C/s) |
| | 56°C | 30 sec (20°C/s) |
| | 60°C | 3 sec (20°C/s)detection |
| | 72°C | 10 sec (20°C/s) |
| | | |
| melting curve: | 95°C | 10sec (20°C/s) |
| | 40°C | 10sec (20°C/s) |
| | 95°C | 0sec (0,1°C/s)Continuous |
| | | |
| cooling: | 40°C 5 sec | |
| | | |
| All other assays: | | |
| activation: | 95°C | 10min |
| | | |
| 55 cycles: | 95°C | 10sec (20°C/s) |
| | 56°C | 30 sec (20°C/s)detection |
| | 72°C | 10 sec (20°C/s) |
| | | |
| melting curve: | 95°C | 10sec (20°C/s) |
| | 40°C | 10sec (20°C/s) |
| | 95°C | 0sec (0,1°C/s)Continuous |
| | | |
| cooling: | 40°C | 5 sec |

### Results:

In order to predict the presence of CRC tumor DNA in the measured plasma samples we use a logistic regression model. The logistic regression model is build as follows.

First the measurement data for each marker assay is encoded in a qualitative way by the following 3 levels:
- Level0 both replicate PCR reactions showed no amplification
- Level1 exactly one of the two PCR replicates showed an amplification curve
- Level2 both of the two PCR replicates showed amplification curves

If any of the two PCR replicates could not be successfully measured the respective marker measurement was regarded as invalid.

The five different DNA methylation markers were used as independent factors with 3 levels in a logistic regression model. An additional intercept factor but no factor interactions were included in the model. The logistic regression model was trained and optimal weights for all factor levels were determined by using the maximum likelihood procedure.

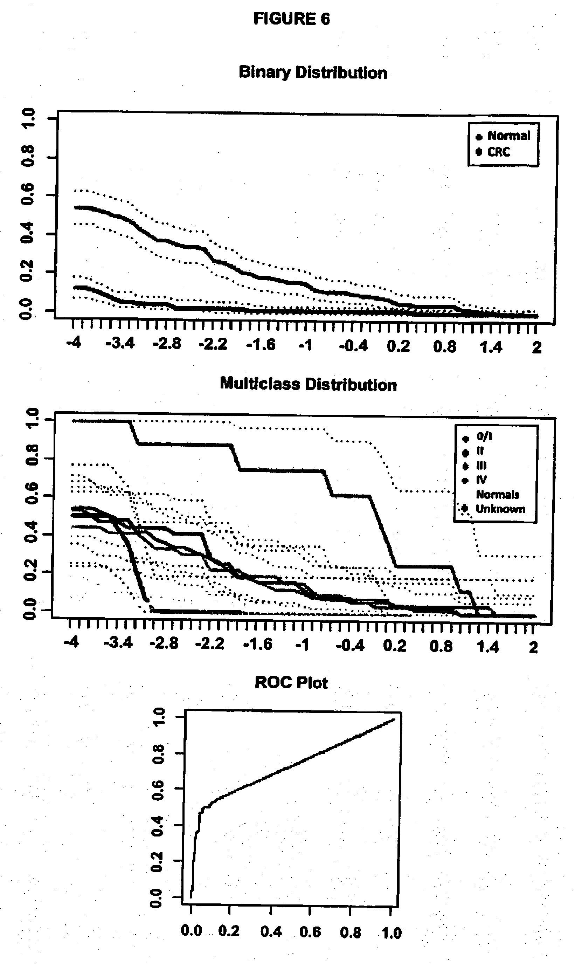

Figures 1 to 10 provide the plots of the measured log mean methylation of the individual assays. Each figures consists of three plots, the plots at the top and in the middle provide the binary and multiclass analysis respectively, the plot at the bottom provides an ROC wherein sensitivity is shown on the Y-axis and 1-specificity is shown on the X-axis. Table 8 and figures 1 to 5 provide an overview of marker performances in all sample groups. Table 9 and figures 6 to 10 provide an overview of marker performances in the colorectal carcinoma and normal colorectal groups.
Figures 12 to 21 provide the plots of the measured log majority mean (analysed sample is only counted as positive if both replicates are positive, the mean of the two measurements is taken as the quantitative methylation measurement) methylation of the individual assays. Each figures consists of three plots, the plots at the top and in the middle provide the binary and multiclass analysis respectively, the plot at the bottom provides an ROC wherein sensitivity is shown on the Y-axis and 1-specificity is shown on the X-axis. Table 12 and figures 12 to 16 provide an overview of marker performances in all sample groups. Table 13 and figures 18 to 21 provide an overview of marker performances in the colorectal carcinoma and normal colorectal groups.
Table 11 provides an overview of the AUC and snd sensitivities of the single assays at 95% specificity (all p-values were less than 0.00001). Wherein said classes are:
   All: Normal + NCD +NCC vs. CRC stages I to IV
   I-IV: Normal vs. CRC stages I to IV
   I-III: Normal vs. CRC stages I to III

From the multiclass distribution of figure 6 (plot in the middle) and table 11 it can be determined that the gene RASSF2 is particularly effective at detecting Stage 1 and early colorectal carcinomas. Accordingly expression, most preferably CpG methylation, of said gene is in additioan to being a preferred diagnostic marker is particularly preferred for the screening of general populations (individuals not displaing any indicators or symptoms of colorectal carcinoma) for the early detection of colorectal carcinomas.

### Marker combinations (panels)

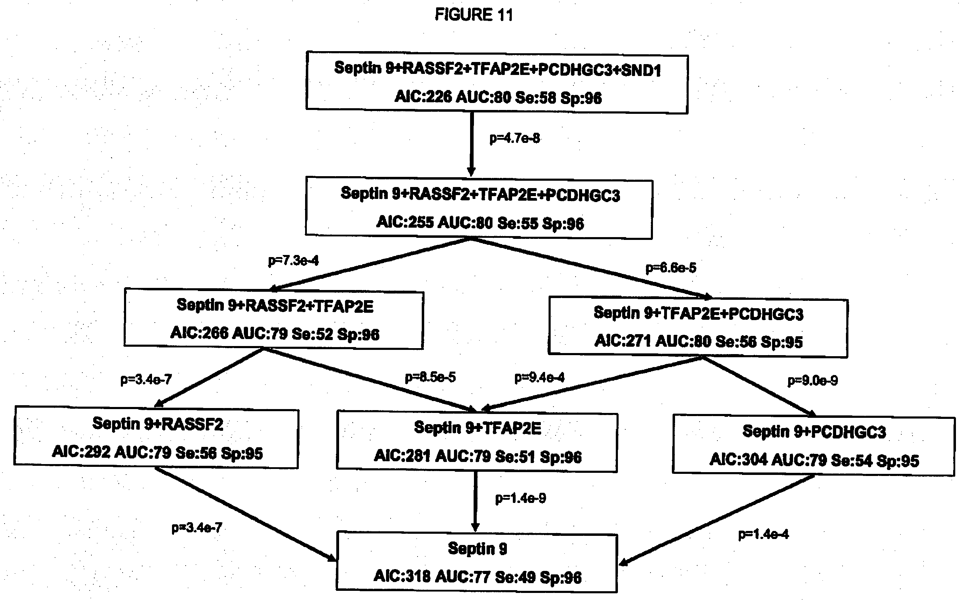

To identify the subset of DNA methylation markers that optimally predicts the presence of CRC we use the backward elimination procedure. In each elimination step the DNA methylation marker with the lowest factor levels was removed from the model. We compared the predictive power of the reduced model with the complete model by using the likelihood ratio test. To identify the subset of DNA methylation markers that optimally predicts the presence of CRC we use the backward elimination procedure. In each elimination step the DNA methylation marker with the lowest factor levels was removed from the model. We compared the predictive power of the reduced model with the complete model by using the likelihood ratio test. Fig. 11 shows that (in the Normal vs. CRC stages I to IV comparison) at each elimination step the predictive power of the logistic regression model was significantly reduced. We conclude that all listed DNA methylation marker models give superior prediction performance as compared to the single marker or the respective simpler marker panels.
We conclude that the following DNA marker models give superior prediction performance as compared to the single marker or the respective simpler marker panels.

Figures 22 to 26 provide an overview of the performance of the following marker combinations:
Septin 9+TFAP2E+Rp,SSF2+PCDHGG3+SND1 (Figure 22)
   All AUC 80
      Sens/Spec 57/95
   All CRC AUC 80
      Sens/Spec 58/96
   CRC I-III AUC 76
      Sens/Spec 50/96
Septin 9+TFAP2E+RASSF2+PCDHGC3 (Figure 23)
   All AUC 80
      Sens/Spec 53/95
   All CRC AUC 80
      Sens/Spec 55/96
   CRC I-III AUC 77
      Sens/Spec 48/96
Septin 9+TFAP2E+RASSF2 (Figure 24)
   All AUC 77
      Sens/Spec 48/96
   All CRC AUC 79
      Sens/Spec 52/96
   CRC I-III AUC 75
      Sens/Spec 42/96
Septin 9+TFAP2E (Figure 25)
   All AUC 77
      Sens/Spec 45/96
   All CRC AUC 79
      Sens/Spec 51/96
   CRC I-III AUC 75
      Sens/Spec 41/96
Septin 9+RASSF2 (Figure 26)
   All AUC 77
      Sens/Spec 43/96
   All CRC AUC 79
      Sens/Spec 56/95
   CRC I-III AUC 74
      Sens/Spec 46/95

In each case the upper plot shows all samples (Normals, Non Colorectal Disease, Non Coloretal Cancers and all CRC stages), the lower plot shows only Normal and CRC samples.

**Table 1: Sequences according to the present invention.**

| Genomic SEQ ID NO: | Gene | Methylated bisulfite converted sequence (sense) | Methylated bisulfite converted sequence (antisense) | Unmethylated bisulfite converted sequence (sense) | Unmethylated bisulfite converted sequence (antisense) |
|---|---|---|---|---|---|
| 1 | SND1 | 15 | 16 | 43 | 44 |
| 2 | PCDHGC3 | 17 | 18 | 45 | 46 |
| 3 | EDNRB | 19 | 20 | 47 | 48 |
| 4 | STOM | 21 | 22 | 49 | 50 |
| 5 | GLI3 | 23 | 24 | 51 | 52 |
| 6 | RXFP3 | 25 | 26 | 53 | 54 |
| 7 | RASSF2 | 27 | 28 | 55 | 56 |
| 8 | Q8N2B6 | 29 | 30 | 57 | 58 |
| 9 | PCDH10 | 31 | 32 | 59 | 60 |
| 10 | LIMK1 | 33 | 34 | 61 | 62 |
| 11 | TFAP2E | 35 | 36 | 63 | 64 |
| 12 | Septin 9 | 37 | 38 | 65 | 66 |
| 13 | Septin 9 CpG island | 39 | 40 | 67 | 68 |
| 14 | Septin 9 CpG island | 41 | 42 | 69 | 70 |
| 132 | 132MRPS21 | 134 | 135 | 138 | 139 |
| 133 | DOCK10 | 136 | 137 | 140 | 141 |

**Table 2: Genes according to the present invention**

| Genomic SEQ ID NO: | Gene | Abbreviation | Chromosomal location* | Contig location* |
|---|---|---|---|---|
| 1 | Staphylococcal nuclease domain-containing protein 1 (p100 co- activator) (100 kDa coactivator) (EBNA2 coactivator p100) | SND1 | 7 127530240 to 127532750 (+) | AC008039.1.1.197346 141230 to 143740 (+) |
| 2 | Protocadherin gamma C5 precursor (PCDH-gamma-C5) | PCDHGC3 | 5 140835658 to 140837940 (+) | AC008781.7.1.205516 172908 to 175190 (-) |
| 3 | Endothelin B receptor precursor | EDNRB | 13 77390505 to 77392740 (+) | AL139002.18.1.183337 92863 to 95098 (+) |
| 4 | Erythrocyte band 7 integral membrane protein (Stomatin) (Protein 7.2b) | STOM | 9 123171727 to 123172921 (+) | x |
| 5 | Zinc finger protein GLI3 | GLI3 | 7 42234131 to 42234437 (+) | AC005158.3.1.266344 110482 to 110788(+) |
| 6 | Relaxin 3 receptor 1 | RXFP3 | 5 -33970963 to 33973309 (+) | AC139777.3.1.169254 141119 to 143465 (+) |
| 7 | Ras association domain family 2 | RASSF2 | 20 4750982 to 4752901 (+) | AL133354.14.1.59726 44336 to 46255 (+) |
| 8 | Prokineticin receptor 2 (PK-R2) (G protein-coupled receptor 73-like 1) (GPR73b) (GPRg2) | GPR73L1 | 20 5245129 to 5245935(+) | AL121757.7.1.167013 166111 to 166917 (-) |
| 9 | Protocadherin gamma C5 precursor (PCDH-gamma-C5) | PCDH10 | 4 134291880 to 134294130 (+) | AC105383.3.1.173956 116476 to 118726 (+) |
| 10 | LIM domain kinase 1 (EC 2.7.1.37) | LIMK1 | 7 73145461 to 73146401(+) | AC005056.2.1.98188 7 to 947(-) |
| 11 | transcription factor AP-2 epsilon (activating enhancer binding protein 2 epsilon) | TFAP2E | 1 35814650 to 35816448 (+) | AC004865.1.1.120088 1091 to 2889 (-) |
| 12 | Septin-9 (MLL septin-like fusion protein) (MLL septin-like fusion protein MSF-A) (Ovarian/Breast septin) (Ov/Br septin) (Septin D1) | Septin 9 | 17 72789082 to 73008258 (+)** | AC068594.15.1.168501 150580 to 151086 (+) to AC111170.11.1.158988 137268 to 138151 (+)** |
| 13 | Septin 9 CpG island | Septin 9 CpG island | 17 72789082 to 72789757 (+) " | AC068594.15.1.168501 150580 to 151255 (+)** |
| 14 | Septin 9 CpG island | Septin 9 CpG island | | |
| 132 | MRPS21 | Dedicator of cytokinesis protein 10 (Protein zizimin 3) | | |
| 133 | DOCK10 | Mitochondrial 28S ribosomal protein S21 (MRP-S21) (MDS016) | | |

| | | | | |
|---|---|---|---|---|
| * Unless otherwise stated all locations refer to Ensembl database v39 (June 2006) ** Ensembl database v31.35d (8 July 2005) | | | | |

**Table 3: Assay components according to Example 1**

| 3ene name | Forward primer SEQ ID NO: | Reverse primer .sEa ID NO: | Probe SEQ ID NO: | PCR conditions | | | | | |
|---|---|---|---|---|---|---|---|---|---|
| | | | | Activation | | Cycling (50x) | | | |
| LHFPL3 | 74 | 75 | 76 | degrees C | time | degrees C | time | degrees C | time |
| PCDHGC3 | 77 | 78 | 79 | 95 | 10 min | 95 | 15 sec | 60 | 60 sec |
| EDNRB | 80 | 81 | 82 | 95 | 10 min | 95 | 15 sec | 60 | 60 sec |
| RASSF2 | 83 | 84 | 85 | 95 | 10 min | 95 | 15 sec | 60 | 60 sec |
| STOM | 86 | 87 | 88 | 95 | 10 min | 95 | 15 sec | 60 | 60 sec |
| TFAP2E | 89 | 90 | 91 | 95 | 10 min | 95 | 15 sec | 60 | 60 sec |
| GLI3 | 92 | 93 | 94 | 95 | 10 min | 95 | 15 sec | 62 | 60 sec |
| RXFP3 | 95 | 96 | 97 | 95 | 10 min | 95 | 15 sec | 60 | 60 sec |
| LimK1 | 98 | 99 | 100 | 95 | 10 min | 95 | 15 sec | 60 | 60 sec |
| GPR73L1 | 101 | 102 | 103 | 95 | 10 min | 95 | 15 sec | 60 | 60 sec |
| PCDH10 | 104 | 105 | 106 | 95 | 10 min | 95 | 15 sec | 62 | 60 sec |
| MRPS21 | 142 | 143 | 144 | 95 | 10 min | 95 | 15 sec | 60 | 60 sec |
| DOCK10 | 145 | 146 | 147/148 | 95 | 10 min | 95 | 15 sec | 62 | 60 sec |

**Table 4: AUC of colorectal carcinoma detection according to Example 1 with methylation above 0% (single genes).**

| Marker | AUC | Complementarity to Septin 9 | Whole Blood |
|---|---|---|---|
| SND1 | 0.92 | 0.04 | 0.00 |
| PCDHGC3 | 0.91 | 0.04 | 0.08 |
| RASSF2 | 0.86 | 0.04 | 0.00 |
| EDNRB | 0.94 | 0.04 | 0.00 |
| STOM | 0.86 | 0.03 | 0.04 |
| Septin 9 | 0.97 | | 0.04 |
| LimK1 | 0.83 | 0.07 | 0.00 |
| TFAP2E | 0.76 | 0.06 | 0.00 |
| GLI3 | 0.71 | 0.04 | 0.00 |
| GPR73L1 | 0.97 | 0.03 | 0.08 |
| RXFP3 | 0.96 | 0.02 | 0.08 |
| PCDH10 | 0.95 | 0.03 | 0.00 |
| MRPS21 | 0.85 | | |
| DOCK10 | 0.73 | | |

**Table 5: AUC of colorectal carcinoma detection according to Example 1 with methylation within various thresholds (single genes).**

| Marker | CRC detection rate at various methylation thresholds | | |
|---|---|---|---|
| | 10% | 20% | 30% |
| Septin 9 | 0.92 | 0.85 | 0.74 |
| SND1 | 0.70 | 0.57 | 0.46 |
| PCDHGC3 | 0.73 | 0.54 | 0.38 |
| RASSF2 | 0.73 | 0.54 | 0.33 |
| EDNRB | 0.71 | 0.54 | 0.41 |
| STOM | 0.71 | 0.57 | 0.42 |
| LimK1 | 0.91 | 0.85 | 0.73 |
| TFAP2E | 0.97 | 0.87 | 0.59 |
| GLI3 | 0.80 | 0.64 | 0.44 |
| GPR73L1 | 0.80 | 0.57 | 0.37 |
| RXFP3 | 0.66 | 0.38 | 0.18 |
| PCDH10 | 0.91 | 0.70 | 0.37 |

**Table 6: AUC of colorectal carcinoma detection samples according to Example 1 with methylation within various thresholds (Septin 9 marker panels).**

| Septin 9+2 markers | CRC detection rate at various methylation thresholds | | | |
|---|---|---|---|---|
| | 0% | 10% | 20% | 30% |
| Septin 9 | 0.97 | 0.92 | 0.85 | 0.74 |
| TFAP2E+LimK1 | 0.84 | 0.98 | 0.97 | 0.89 |
| GLI3+LimK1 | 0.88 | 0.97 | 0.95 | 0.88 |
| EDNRB+SND1 | 0.94 | 0.96 | 0.90 | 0.83 |
| PCDHGC3+RASSF2 | 0.93 | 0.96 | 0.92 | 0.83 |
| PCDHGC3+SND1 | 0.94 | 0.96 | 0.92 | 0.84 |

**Table 7: Particularly preferred genes and sequences thereof according to the present invention**

| Genomic SEQ ID NO: | Gene | Methylated bisulfite converted sequence (sense) | Methylated bisulfite converted sequence (antisense) | Unmethylated bisulfite converted sequence (sense) | Unmethylated bisulfite converted sequence (antisense) |
|---|---|---|---|---|---|
| 1 | SND1 | 15 | 16 | 43 | 44 |
| 2 | PCDHGC3 | 17 | 18 | 45 | 46 |
| 7 | RASSF2 | 27 | 28 | 55 | 56 |
| 11 | TFAP2E | 35 | 36 | 63 | 64 |

**Table 8: HM assay (Example 2) performance in all tissue samples.**

| | RASSF2 | Septin 9 | SND1 | PCDHGC3 | TFAP2E |
|---|---|---|---|---|---|
| AUC (95% confidence interval) | 0.72 (0.67, 0.77) | 0.75 (0.7, 0.79) | 0.66(0.6, 0.71) | 0.66(0.61, 0.72) | 0.69 (0.63, 0.74) |
| Sens/Spec | 0.4/0.95 | 0.47/0.95 | 0.25/0.95 | 0.32/0.95 | 0.29/0.95 |
| Sens/Spec cut | -3.029 | -2.706 | -3.089 | -2.378 | -2.692 |
| Wilcoxon P | 0 | 0 | 0 | 0 | 0 |
| CRC+Adenoma - (pos) | 131 | 131 | 118 | 119 | 119 |
| Normal+non-cancerous diseases (NCD)+carcinoma other than colorectal (NCC)-(neg) | 228 | 228 | 205 | 206 | 206 |

**Table 9: HM assay (Example 2) performance in colorectal carcinoma and normal colorectal tissue samples.**

| | RASSF2 | Septin 9 | SND1 | PCDHGC3 | TFAP2E |
|---|---|---|---|---|---|
| AUC (95% confidence (interval) | 0.73(0.67.0.78) | 0.76(0.7.0.8) | 0.67(0.61,0.73) | 0.68(0.62,0.73) | 0.71(0.65,0.76) |
| Sens/Spec | 0.47/0.95 | 0.48/0.95 | 0.39/0.95 | 0.32/0.95 | 0.39/0.95 |
| Sens/Spec cut off | -3.272 | -2.858 | -3.473 | -2.417 | -3.446 |
| Wilcoxon P | 0 | 0 | 0 | 0 | 0 |
| CRC+Adenoma (pos) | 131 | 131 | 118 | 119 | 119 |
| Normal (neg) | 168 | 169 | 148 | 148 | 148 |

**Table 10: Primer, blocker and probe sequences according to Example 2.**

| | Septin 9 | RASSF2 | SND1 | PCDHGC3 | TFAP2E |
|---|---|---|---|---|---|
| Forward primer SEQ ID NO: | 107 | 112 | 117 | 122 | 127 |
| Reverse primer SEQ ID NO: | 108 | 113 | 118 | 123 | 128 |
| Blocker SEQ ID NO: | 109 | | 119 | 124 | 129 |
| Probe SEQ ID NO: | 110 | 115 | 120 | 125 | 130 |
| Probe SEQ ID NO: | 111 | 116 | 121 | 126 | 131 |

**Table 11: AUC and sensitivity (at 95% specificity) for single assays of markers according to class.***

| | AUC | | | Sensitivity | | |
|---|---|---|---|---|---|---|
| | AII | I-IV | I-II | AII | I-IV | I-II |
| Septin 9 (Majority mean) | 73 | 73 | 67 | 49 | 49 | 37 |
| RASSF2 (Log Mean) | 72 | 73 | 70 | 45 | 48 | 41 |
| TFAP2E (Log Mean) | 68 | 71 | 67 | 32 | 38 | 30 |
| SND1 (Log Mean) | 64 | 65 | 62 | 25 | 35 | 29 |
| PCDHGC3 (Log Mean) | 65 | 66 | 64 | 30 | 32 | 29 |

| | | | | | | |
|---|---|---|---|---|---|---|
| *all p-values were less than 0.00001 | | | | | | |

**Table 12:HM assay (Example 2) performances in all tissue samples.**

| | RASSF2 | Septin 9 | SND1 | PCDHGC3 | TFAP2E |
|---|---|---|---|---|---|
| AUC (95% confidence interval) | 3.67(0.62,0.72) | 0.74(0.69,0.79) | 0.63(0.57,0.68) | 0.65(0.6,0.7) | 0.65(0.6,0.7) |
| Sens/Spec | 0.37(0.96) | 0.51 | 0.28/0.95 | 0.34/0.95 | 0.34/0.96 |
| Sens/Spec cut off | -4 | 0 | -3.45 | -2.523 | -4 |
| Wilcoxon P | 0 | 0 | 0 | 0 | 0 |
| CRC+Adenoma -(pos) | 121 | 127 | 113 | 127 | 120 |
| Normal+non-cancerous diseases (NCD)+carcinoma other than colorectal (NCC) - (neg) | 206 | 220 | 194 | 224 | 203 |

**Table 13:HM assay (Example 2) performance in colorectal carcinoma and normal colorectal tissue samples.**

| | RASSF2 | Septin 9 | SND1 | PCDHGC3 | TFAP2E |
|---|---|---|---|---|---|
| AUC (95% confidence interval) | 0.67(0.61,0.73) | 0.74(0.69,0.79) | 0.64(0.58,0.7) | 0.66(0.6,0.71) | 0.66(0.6,0.72) |
| Sens/Spec | 0.37/0.97 | 0.51/0.97 | 0.3/0.97 | 0.35/0.95 | 0.34/0.98 |
| Sens/Spec cut off | -4 | -4 | -4 | -2.599 | -4 |
| Wilcoxon P | 0 | 0 | 0 | 0 | 0 |
| CRC+Adenoma (pos) | 121 | 121 | 113 | 127 | 120 |
| Normal (neg) | 154 | 164 | 146 | 167 | 154 |

## Claims

1. A method for detecting colorectal cancer in a subject comprising determining the methylation level of at least the GLI3 gene in a biological sample isolated from said subject wherein the presence of CpG methylation is indicative of the presence or class of said colorectal cancer.

2. A method according to claim 1 wherein additionally the methylation level of 1, 2 or 3 genes selected from the group consisting of RASSF2, TFAP2E, SND1, PCDHGC3, EDNRB, Septin 9, STOM, RXFP3, LimK1, GPR73L1, PCDH10, DOCK10 and MRPS21 are determined.

3. A method for detecting colorectal cancer in a subject, according to claim 1, comprising contacting genomic DNA isolated from a biological sample obtained from said subject with at least one reagent, or series of reagents that distinguishes between methylated and non-methylated CpG dinucleotides:
(i) within at least one target region of the genomic DNA, wherein the target region comprises, or hybridizes under stringent conditions to a sequence of at least 16 contiguous nucleotides of the sequence of SEQ ID NO: 5 or
(ii) within at least two target regions of the genomic DNA, wherein a first target region comprises, or hybridizes under stringent conditions to a sequence of at least 16 contiguous nucleotides of at least one sequence selected from the group consisting of SEQ ID NO: 12 TO SEQ ID NO: 14 respectively and wherein a second target region comprises, or hybridizes under stringent conditions to a sequence of at least 16 contiguous nucleotides of the sequence of SEQ ID NO: 5,
wherein said contiguous nucleotides comprise at least one CpG dinucleotide sequence, and whereby detecting colorectal cancer is, at least in part, afforded.

4. A method for detecting colorectal cancer, according to claim 1, comprising:
a. extracting or otherwise isolating genomic DNA from a biological sample obtained from the subject
b. treating the genomic DNA of a), or a fragment thereof, with one or more reagents to convert cytosine bases that are unmethylated in the 5-position thereof to uracil or to another base that is detectably dissimilar to cytosine in terms of hybridization properties;
c. contacting the treated genomic DNA, or the treated fragment thereof, with an amplification enzyme and at least one primer comprising, a contiguous sequence of at least 9 nucleotides that is complementary to, or hybridizes under moderately stringent or stringent conditions to a sequence selected from the group consisting of SEQ ID NO: 23, 24, 51 and 52, and complements thereof, wherein the treated genomic DNA or the fragment thereof is either amplified to produce at least one amplificate, or is not amplified; and
d. determining, based on a presence or absence of, or on a property of said amplificate, the methylation state or level of at least one CpG dinucleotide of the sequence of SEQ ID NO: 5, or an average, or a value reflecting an average methylation state or level of a plurality of CpG dinucleotides of the sequence of SEQ ID NO: 5, whereby detecting colorectal cancer is, at least in part, afforded.

5. The method of any of claim 1 to 4, wherein the biological sample obtained from the subject is selected from the group comprising cell lines, histological slides, biopsies, paraffin-embedded tissue, body fluids, stool, colonic effluent, urine, blood plasma, blood serum, whole blood, isolated blood cells, cells isolated from the blood and combinations thereof.

6. The method of claim 4,
(i) further comprising in step d) the use of at least one nucleic acid molecule or peptide nucleic acid molecule comprising in each case a contiguous sequence at least 9 nucleotides in length that is complementary to, or hybridizes under moderately stringent or stringent conditions to a sequence selected from the group consisting of SEQ ID NO: 15 TO SEQ ID NO: 22, SEQ ID NO: 25 to SEQ ID NO: 36, SEQ ID NO: 43 TO SEQ ID NO: 50, SEQ ID NO: 53 to SEQ ID NO: 64 and SEQ ID NO: 134 TO SEQ ID NO: 141, and complements thereof, wherein said nucleic acid molecule or peptide nucleic acid molecule suppresses amplification of the nucleic acid to which it is hybridized;
(ii) wherein determining in d) comprises hybridization of at least one nucleic acid molecule or peptide nucleic acid molecule in each case comprising a contiguous sequence at least 9 nucleotides in length that is complementary to, or hybridizes under moderately stringent or stringent conditions to a sequence selected from the group consisting of SEQ ID NO: 15 TO SEQ ID NO: 22, SEQ ID NO: 25 to SEQ ID NO: 36 AND SEQ ID NO: 43 TO SEQ ID NO: 50, SEQ ID NO: 53 to 64 AND SEQ ID NO: 134 TO SEQ ID NO: 141, and complements thereof, preferably wherein at least one such hybridizing nucleic acid molecule or peptide nucleic acid molecule is bound to a solid phase or further comprising extending at least one such hybridized nucleic acid molecule by at least one nucleotide base.
(iii) wherein determining in d), comprises sequencing of the amplificate; and/or
(iv) wherein contacting or amplifying in c), comprises use of methylation-specific primers.

7. A method for detecting colorectal cancer, according to claim 1, comprising:
a. extracting or otherwise isolating genomic DNA from a biological sample obtained from the subject
b. digesting the genomic DNA of a), or a fragment thereof, with one or more methylation sensitive restriction enzymes;
contacting the DNA restriction enzyme digest of b), with an amplification enzyme and at least two primers suitable for the amplification of a sequence comprising at least one CpG dinucleotide of the sequence of SEQ ID NO: 5;
c. determining, based on a presence or absence of an amplificate the methylation state or level of at least one CpG dinucleotide of the sequence of SEQ ID NO: 5, whereby detecting colorectal cancer is, at least in part, afforded.

8. Use of a nucleic acid:
(i) derived from the genomic DNA sequence of SEQ ID NO: 5 by treatment that is suitable to convert at least one unmethylated cytosine base of the genomic DNA sequence to uracil or another base that is detectably dissimilar to cytosine in terms of hybridization;
(ii) comprising at least 16 contiguous nucleotides of a DNA sequence selected from the group consisting of SEQ ID NO: 23, 24, 51 and 52, and sequences complementary thereto, or
(iii) comprising at least 50 contiguous nucleotides of a DNA sequence selected from the group consisting of SEQ ID NO: 23, 24, 51 and 52, and sequences complementary thereto
wherein the base sequence of the nucleic acid preferably comprises at least one CpG, TpG or CpA dinucleotide sequence or
(iv) comprising at least 16 contiguous nucleotides of a DNA sequence selected from the group consisting of SEQ ID NO: 23, 24, 51 and 52, and sequences complementary thereto wherein the base sequence of the nucleic acid preferably comprises at least one CpG, TpG or CpA dinucleotide sequence
for determining the methylation of GLI3 and thereby diagnosing or detecting colorectal cancer.

9. Use of a kit suitable for performing the method according to claims 1 to 7 in the diagnosis of colorectal cancer comprising (a) a methylation sensitive restriction enzyme reagent; (b) a container suitable for containing the said reagent and the biological sample of the patient; (c) at least one set of oligonucleotides one or a plurality of nucleic acids or peptide nucleic acids which are identical, are complementary, or hybridize under stringent or highly stringent conditions to an at least 9 base long segment of the sequence of SEQ ID NO: 5; and optionally (d) instructions for use and interpretation of the kit results.

## Patentansprüche

1. Verfahren zum Nachweisen von Dickdarmkrebs in einem Subjekt, umfassend Bestimmen des Methylierungsniveaus wenigstens des GLI3-Gens in einer biologischen Probe, die aus dem Subjekt isoliert wurde, wobei das Vorliegen einer CpG-Methylierung das Vorliegen oder die Klasse des Dickdarmkrebses anzeigt.

2. Verfahren nach Anspruch 1, wobei ferner das Methylierungsniveau von 1, 2 oder 3 Genen ausgewählt aus der Gruppe bestehend aus RASSF2, TFAP2E, SND1, PCDHGC3, EDNRB, Septin 9, STOM, RXFP3, LimK1, GPR73L1, PCDH10, DOCK10 und MRPS21 bestimmt wird.

3. Verfahren zum Nachweisen von Dickdarmkrebs in einem Subjekt nach Anspruch 1, umfassend In-Kontakt-bringen von genomischer DNA, die aus einer biologischen Probe isoliert wurde, die von dem Subjekt erhalten wurde, mit wenigstens einem Reagenz oder einer Reihe von Reagenzien, die zwischen methylierten und unmethylierten CpG-Dinukleotiden unterscheiden:
(i) innerhalb wenigstens einer Zielregion der genomischen DNA, wobei die Zielregion eine Sequenz von wenigstens 16 zusammenhängenden Nukleotiden der SEQ ID NO: 5 umfasst oder unter stringenten Bedingungen daran hybridisiert, oder
(ii) innerhalb wenigstens zweier Zielregionen der genomischen DNA, wobei eine erste Zielregion eine Sequenz von wenigstens 16 zusammenhängenden Nukleotiden wenigstens einer Sequenz ausgewählt aus der Gruppe bestehend aus SEQ ID NO: 12 bis SEQ ID NO: 14 umfasst oder unter stringenten Bedingungen daran hybridisiert und wobei eine zweite Zielregion ein Sequenz von wenigstens 16 zusammenhängenden Nukleotiden der SEQ ID NO: 5 umfasst oder unter stringenten Bedingungen daran hybridisiert,
wobei die zusammenhängenden Nukleotide wenigstens eine CpG-Dinukleotidsequenz umfassen und wobei das Nachweisen von Dickdarmkrebs wenigstens teilweise bereitgestellt wird.

4. Verfahren zum Nachweisen von Dickdarmkrebs nach Anspruch 1, umfassend:
a. Extrahieren oder anderweitiges Isolieren genomischer DNA aus einer biologischen Probe, die von dem Subjekt erhalten wurde,
b. Behandeln der genomischen DNA aus a) oder eines Fragmentes davon mit einem oder mehreren Reagenzien, um Cytosinbasen, die in der 5-Position unmethyliert sind, in Uracil oder eine weitere Base, die hinsichtlich der Hybridisierungseigenschaften nachweisbar von Cytosin verschieden ist, umzuwandeln;
c. In-Konktakt-bringen der behandelten genomischen DNA oder des behandelten Fragments davon mit einem Amplifikationsenzym und wenigstens einem Primer, welcher eine zusammenhängende Sequenz von wenigstens 9 Nukleotiden umfasst, die zu einer Sequenz ausgewählt aus der Gruppe bestehend aus SEQ ID NO: 23, 24, 51 und 52 und Komplementen davon komplementär ist oder unter moderat stringenten oder stringenten Bedingungen daran hybridisiert, wobei die behandelte genomische DNA oder das Fragment davon entweder amplifiziert werden, um wenigstens ein Amplifikat zu erzeugen, oder nicht amplifiziert werden; und
d. Bestimmen, basierend auf dem Vorliegen oder dem Fehlen des Amplifikats oder einer Eigenschaft davon, des Methylierungszustandes oder des Methylierungsniveaus von wenigstens einem CpG-Dinukleotid der Sequenz der SEQ ID NO: 5 oder eines Mittelwerts oder eines Wertes, der einen mittleren Methylierungszustand oder ein mittleres Methylierungsniveau einer Vielzahl von CpG-Dinukleotiden der Sequenz SEQ ID NO: 5 wiedergibt, wobei das Nachweisen von Dickdarmkrebs wenigstens teilweise bereitgestellt wird.

5. Verfahren nach einem der Ansprüche 1 bis 4, wobei die biologische Probe, die von dem Subjekt erhalten wurde, ausgewählt ist aus der Gruppe umfassend Zelllinien, histologische Schnitte, Biopsien, in Paraffin eingebettetes Gewebe, Körperfluide, Stuhl, Darmausfluss, Urin, Blutplasma, Blutserum, Vollblut, isolierte Blutzellen, aus Blut isolierte Zellen und Kombinationen davon.

6. Verfahren nach Anspruch 4,
(i) ferner umfassend in Schritt d) die Verwendung wenigstens eines Nukleinsäuremoleküls oder Peptidnukleinsäuremoleküls umfassend in jedem Fall eine zusammenhängende Sequenz von wenigstens 9 Nukleotiden Länge, die zu einer Sequenz ausgewählt aus der Gruppe bestehend aus SEQ ID NO: 15 bis SEQ ID NO: 22, SEQ ID NO: 25 bis SEQ ID NO: 36, SEQ ID NO: 43 bis SEQ ID NO: 50, SEQ ID NO: 53 bis SEQ ID NO: 64 und SEQ ID NO: 134 bis SEQ ID NO: 141 und Komplementen davon komplementär ist oder unter moderat stringenten oder stringenten Bedingungen daran hybridisiert, wobei das Nukleinsäuremolekül oder das Peptidnukleinsäuremolekül eine Amplifikation der Nukleinsäure, an die sie hybridisiert sind, unterdrücken;
(ii) wobei das Bestimmen in d) eine Hybridisierung wenigstens eines Nukleinsäuremoleküls oder Peptidnukleinsäuremoleküls jeweils umfassend eine zusammenhängende Sequenz von wenigstens 9 Nukleotiden Länge, die zu einer Sequenz ausgewählt aus der Gruppe bestehend aus SEQ ID NO: 15 bis SEQ ID NO: 22, SEQ ID NO: 25 bis SEQ ID NO: 36, SEQ ID NO: 43 bis SEQ ID NO: 50, SEQ ID NO: 53 bis SEQ ID NO: 64 und SEQ ID NO: 134 bis SEQ ID NO: 141 und Komplementen davon komplementär ist oder unter moderat stringenten oder stringenten Bedingungen daran hybridisiert, umfasst, bevorzugt wobei wenigstens ein solches hybridisierendes Nukleinsäuremolekül oder Peptidnukleinsäuremolekül an einer Festphase gebunden ist, oder ferner umfassend ein Erweitern wenigstens eines solchen hybridisierten Nukleinsäuremoleküls durch wenigstens eine Nukleotidbase;
(iii) wobei das Bestimmen in d) ein Sequenzieren des Amplifikats umfasst; und/oder
(iv) wobei das In-Kontakt-bringen oder Amplifizieren in c) eine Verwendung methylierungsspezifischer Primer umfasst.

7. Verfahren zum Nachweisen von Dickdarmkrebs nach Anspruch 1, umfassend:
a. Extrahieren oder anderweitiges Isolieren von genomischer DNA aus einer biologischen Probe, die von einem Subjekt erhalten wurde;
b. Verdauen der genomischen DNA aus a) oder eines Fragmentes davon mit einem oder mehreren methylierungsempfindlichen Restriktionsenzymen;
c. In-Kontakt-bringen des DNA-Restriktionsenzymverdaus aus b) mit einem Amplifikationsenzym und wenigstens zwei Primern, die für die Amplifikation einer Sequenz umfassend wenigstens ein CpG-Dinukleotid der Sequenz der SEQ ID NO: 5 geeignet sind;
d. Bestimmen, basierend auf dem Vorliegen oder dem Fehlen eines Amplifikats, des Methylierungszustandes oder des Methylierungsniveaus von wenigstens einem CpG-Dinukleotid der Sequenz der SEQ ID NO: 5, wobei ein Nachweisen von Dickdarmkrebs wenigstens teilweise bereitgestellt wird.

8. Verwendung einer Nukleinsäure:
(i) abgeleitet von der genomischen DNA-Sequenz der SEQ ID NO: 5 durch eine Behandlung, die geeignet ist, wenigstens eine unmethylierte Cytosinbase der genomischen DNA-Sequenz in Uracil oder eine weitere Base, die sich hinsichtlich der Hybridisierung nachweisbar von Cytosin unterscheidet, umzuwandeln;
(ii) umfassend wenigstens 16 zusammenhängende Nukleotide einer DNA-Sequenz, die ausgewählt ist aus der Gruppe bestehend aus SEQ ID NO: 23, 24, 51 und 52 und dazu komplementären Sequenzen; oder
(iii) umfassend wenigstens 50 zusammenhängende Nukleotide einer DNA-Sequenz, die ausgewählt ist aus der Gruppe bestehend aus SEQ ID NO: 23, 24, 51 und 52 und dazu komplementären Sequenzen;
wobei die Basensequenz der Nukleinsäure bevorzugt wenigstens eine CpG-, TpG- oder CpA-Dinukleotidsequenz umfasst; oder
(iv) umfassend wenigstens 16 zusammenhängende Nukleotide einer DNA-Sequenz, die ausgewählt ist aus der Gruppe bestehend aus SEQ ID NO: 23, 24, 51 und 52 und dazu komplementären Sequenzen, wobei die Basensequenz der Nukleinsäure bevorzugt wenigstens eine CpG-, TpG- oder CpA-Dinukleotidsequenz umfasst, zum Bestimmen der Methylierung von GLI3 und somit zum Diagnostizieren oder Nachweisen von Dickdarmkrebs.

9. Verwendung eines Kits, das zum Durchführen des Verfahrens gemäß der Ansprüche 1 bis 7 geeignet ist, in der Diagnose von Dickdarmkrebs, umfassend (a) ein methylierungsempfindliches Restriktionsenzymreagenz; (b) einen Behälter, der geeignet ist, um das Reagenz und die biologische Probe des Patienten aufzunehmen; (c) wenigstens einen Satz von Oligonukleotiden oder eine Vielzahl von Nukleinsäuren oder Peptidnukleinsäuren, die mit einem wenigstens 9 Basen langen Segment der Sequenz der SEQ ID NO: 5 identisch sind, dazu komplementär sind oder unter stringenten oder hochstringenten Bedingungen daran hybridisieren; und gegebenenfalls (d) Instruktionen zur Verwendung und Interpretation der Kitergebnisse.

## Revendications

1. Procédé pour la détection d'un cancer colorectal chez un sujet, comprenant la détermination du niveau de méthylation d'au moins le gène GLI3 dans un échantillon biologique isolé à partir dudit sujet, tandis que la présence de la méthylation de CpG est indicatrice de la présence ou de la classe dudit cancer colorectal.

2. Procédé selon la revendication 1, dans lequel en outre les niveaux de méthylation de 1, 2 ou 3 gènes choisis dans le groupe consistant en RASSF2, TFAP2E, SND1, PCDHGC3, EDNRB, Septin 9, STOM, RXFP3, LimK1, GPR73L1, PCDH10, DOCK10 et MRPS21 sont déterminés.

3. Procédé pour la détection d'un cancer colorectal chez un sujet, selon la revendication 1, comprenant la mise en contact d'ADN génomique isolé à partir d'un échantillon biologique obtenu à partir dudit sujet avec au moins un réactif, ou une série de réactifs, qui fait la distinction entre des dinucléotides CpG méthylés et non-méthylés:
(i) dans au moins une région cible de l'ADN génomique, la région cible comprenant, ou hybridant dans des conditions stringentes à une séquence d'au moins 16 nucléotides contigus de la séquence selon SEQ ID NO: 5 ou
(ii) dans au moins deux régions cibles de l'ADN génomique, une première région cible comprenant, ou hybridant dans des conditions stringentes à une séquence d'au moins 16 nucléotides contigus d'au moins une séquence choisie dans le groupe consistant en SEQ ID NO: 12 à SEQ ID NO: 14 respectivement et une deuxième région cible comprenant, ou hybridant dans des conditions stringentes à une séquence d'au moins 16 nucléotides contigus de la séquence selon SEQ ID NO: 5,
tandis que lesdits nucléotides contigus comprennent au moins une séquence de dinucléotides CpG, et procurant ainsi la détection, au moins en partie, d'un cancer colorectal.

4. Procédé pour la détection d'un cancer colorectal, selon la revendication 1, comprenant:
a. l'extraction ou l'isolement d'une autre manière d'ADN à partir d'un échantillon biologique obtenu à partir d'un sujet
b. le traitement de l'ADN génomique selon a), ou d'un fragment de celui-ci, avec un ou plusieurs réactifs pour convertir les bases cytosine qui sont non-méthylées sur leur position 5 en uracile ou en une autre base qui est détectable qui est détectable comme n'étant pas similaire à la cytosine en termes de propriétés d'hybridation;
c. la mise en contact de l'ADN génomique traité, ou du fragment traité de celui-ci, avec une enzyme d'amplification et au moins une amorce comprenant une séquence contiguë d'au moins 9 nucléotides qui est complémentaire de, ou hybride dans des conditions modérément stringentes ou stringentes à une séquence choisie dans le groupe consistant en SEQ ID NO: 23, 24, 51 et 52, et leurs compléments, tandis que l'ADN génomique traité ou le fragment de celui-ci soit est amplifié pour produire au moins un produit d'amplification, soit n'est pas amplifié; et
d. la détermination, sur la base de la présence ou de l'absence de, ou sur la base d'une propriété dudit produit d'amplification, de l'état ou du niveau de méthylation d'au moins un dinucléotide CpG de la séquence selon SEQ ID NO: 5, ou d'une moyenne, ou d'une valeur reflétant un état ou un niveau moyen de méthylation d'une pluralité de dinucléotides CpG de la séquence SEQ ID NO: 5, procurant ainsi la détection, au moins en partie, d'un cancer colorectal.

5. Procédé selon l'une quelconque des revendications 1 à 4, dans lequel l'échantillon biologique obtenu à partir du sujet est choisi dans le groupe comprenant lignées cellulaires, coupes histologiques, biopsies, tissus enrobés dans la paraffine, fluides corporels, selles, effluents du côlon, urine, plasma sanguin, sérum sanguin, sang total, cellules sanguines isolées, cellules isolées à partir du sang, et leurs combinaisons.

6. Procédé selon la revendication 4,
(i) comprenant en outre dans l'étape d) l'utilisation d'au moins une molécule d'acide nucléique ou molécule d'acide nucléique de peptide comprenant dans chaque cas une séquence contiguë d'au moins 9 nucléotides de longueur qui est complémentaire de, ou hybride dans des conditions modérément stringentes ou stringentes à une séquence choisie dans le groupe consistant en SEQ ID NO: 15 à SEQ ID NO: 22, SEQ ID NO: 25 à SEQ ID NO: 36, SEQ ID NO: 43 à SEQ ID NO: 50, SEQ ID NO: 53 à SEQ ID NO: 64 et SEQ ID NO: 134 à SEQ ID NO: 141, et leurs compléments, ladite molécule d'acide nucléique ou molécule d'acide nucléique de peptide supprimant l'amplification de l'acide nucléique auquel elle est hybridée;
(ii) tandis que la détermination dans d) comprend l'hybridation à au moins une molécule d'acide nucléique ou molécule d'acide nucléique de peptide dans chaque cas comprenant une séquence contiguë d'au moins 9 nucléotides de longueur qui est complémentaire de, ou hybride dans des conditions modérément stringentes ou stringentes à une séquence choisie dans le groupe consistant en SEQ ID NO: 15 à SEQ ID NO: 22, SEQ ID NO: 25 à SEQ ID NO: 36 et SEQ ID NO: 43 à SEQ ID NO: 50, SEQ ID NO: 53 à 64 et SEQ ID NO: 134 à SEQ ID NO: 141, et leurs compléments, tandis que de préférence au moins une telle molécule d'acide nucléique ou molécule d'acide nucléique de peptide hybridante est liée à une phase solide, ou y inclus en outre l'extension d'au moins une telle molécule d'acide nucléique hybridée par au moins une base nucléotidique;
(iii) tandis que la détermination dans d) comprend le séquençage du produit d'amplification; et/ou
(iv) tandis que la mise en contact ou l'amplification dans c) comprend l'utilisation d'amorces spécifiques vis-à-vis de la méthylation.

7. Procédé pour la détection d'un cancer colorectal, selon la revendication 1, comprenant:
a. l'extraction ou l'isolement d'une autre manière d'ADN génomique à partir d'un échantillon biologique obtenu à partir d'un sujet;
b. la digestion de l'ADN génomique de a), ou d'un fragment de celui-ci, avec une ou plusieurs enzymes de restriction sensibles à la méthylation;
la mise en contact du produit de digestion de l'enzyme de restriction d'ADN de b), avec une enzyme d'amplification et au moins deux amorces convenant pour l'amplification d'une séquence comprenant au moins un dinucléotide CpG de la séquence selon SEQ ID ON: 5;
c. la détermination sur la base d'une présence ou absence d'un produit d'amplification de l'état ou du niveau de méthylation d'au moins un dinucléotide CpG de la séquence SEQ ID NO: 5, ce par quoi la détection d'un cancer colorectal est, au moins en partie, procurée.

8. Utilisation d'un acide nucléique:
(i) dérivé de la séquence d'ADN génomique selon SEQ ID NO: 5 par un traitement qui convient pour convertir au moins une base cytosine non-méthylée de la séquence d'ADN génomique en uracile ou une autre base qui est détectable comme n'étant pas similaire à la cytosine en termes d'hybridation;
(ii) comprenant au moins 16 nucléotides contigus d'une séquence d'ADN choisie dans le groupe consistant en SEQ ID NO: 23, 24, 51 et 52, et des séquences complémentaires de celles-ci; ou
(iii) comprenant au moins 50 nucléotides contigus d'une séquence d'ADN choisie dans le groupe consistant en SEQ ID NO: 23, 24, 51 et 52, et des séquences complémentaires de celles-ci,
tandis que la séquence de bases de l'acide nucléique comprend de préférence au moins une séquence dinucléotidique CpG, TpG ou CpA ou
(iv) comprenant au moins 16 nucléotides contigus d'une séquence d'ADN choisie dans le groupe consistant en SEQ ID NO: 23, 24, 51 et 52, et des séquences complémentaires de celles-ci, tandis que la séquence de bases de l'acide nucléique comprend de préférence au moins une séquence dinucléotidique CpG, TpG ou CpA
pour la détermination de la méthylation de GLI3 et ainsi le diagnostic ou la détection d'un cancer colorectal.

9. Utilisation d'un kit approprié pour la mise en oeuvre du procédé selon les revendications 1 à 7 dans le diagnostic du cancer colorectal comprenant (a) un réactif enzyme de restriction sensible à la méthylation; (b) un récipient approprié pour contenir ledit réactif et l'échantillon biologique du patient; (c) au moins un ensemble d'oligonucléotides dont l'un ou une pluralité des acides nucléiques ou des acides nucléiques de peptide sont identiques, sont complémentaires, ou hybrident dans des conditions stringentes ou fortement stringentes à un segment d'au moins 9 bases de long de la séquence selon SEQ ID NO: 5; et en option (d) des instructions pour l'utilisation et l'interprétation des résultats du kit.


<110> Epigenomics AG
<120> METHODS AND NUCLEIC ACIDS FOR ANALYSES OF CELLULAR PROLIFERATIVE
   DISORDERS
<130> 536-115 EPT2
<150> US 60/832,509
   <151> 2006-07-21
<150> US 60/853,097
   <151> 2006-10-20
<160> 148
<210> 1
   <211> 2519
   <212> DNA
   <213> Homo Sapiens
<400> 1
   gatttatgag tgaatgacta aaagtgcagc tgagtcctgg cagagggcat ggggtcccac     60
   ccagagacag gcagagaaag ttgaagtccc aggattggag gccgttcttc ctcacctccc    120
   caccaggccc aggcagggct tgatctgaac ggaggcctgg gaacctgtgg ccagccttta    180
   cttgttggaa aagagcagtc cttaagctca attgctccag gttgatgctt ccctactttt    240
   ttttatttat ttatttttat tattattttt tttttattga gacggagtct tactctgttg    300
   ccaggttgga gtgcagtggc gcgatctcgg ctcactgcca cctccgcctg ctgagttcaa    360
   gcctcagcct cctgagtagc ctcctgggta gctgggacta caggcgtgcg ccaccacgcc    420
   aggctaattt tttgtatttt agtatagacg gagtttcacc actttggcca ggatggtctc    480
   gatctcctga cctcgtgata ctcccgcctc ggtatcccaa agtgccggga ttacaggcat    540
   gagccacagc gcccggcccc tagttctttt taaaaaacgc tagatccgtc cgctgcgctg    600
   agtggaggcg gggcaggcct ccgttctcca attggcctta tccaccgagc tcttcccttg    660
   tgccgggctc tgtgccaagc acatcacacg ctgtatcctg cggccaggtt gctgtggtcc    720
   agggtcgtac cctggtccaa ggtcgcaaac cgaggtggga ctccgatccg gcaaccacgc    780
   ccgtggcccg gaaacggcgt cccctgaggc ccaggagagg ccgggcggtg agcggctgtg    840
   gagccgagcg cgggcagtgc ggatgctgcc tatgggggag gcagccaagg acggagggcg    900
   agaggcggtt cttccaaggt caccctcttc cgggttgcaa gcaaaggtca ggggatcccg    960
   gaatggttag tgcaggagct tctctgtgcc ttccacgtcc tagatcctca gagcctcaga   1020
   aacggagatc atcgtcccca cccccatttt acagatgaag aaactgagcc gaggaaagga   1080
   agcgacttgg ccaaggtcgg agagctcatt ctttgcaggg cggggtttgg aacccggggt   1140
   ctggctctcg gcaacgcgcc ctcggcccgc agcctcctgc cccctgtgcc ccgcttcggc   1200
   ccccagcgca gctgcagagg ggcccccctc gacgcataca ctcaagagcc cgaccgcgcg   1260
   gctgaaatcg cggagctcgg agccgcggct ggctgagcga tcgcggttcc tgggctgcgt   1320
   gcgcgcccct tggagctgaa aggagcgcca ggatcggggg cgctgcaccg ggctgggccc   1380
   ctcaacgctc gcagaccggg ccgggctgca gctggagatg gcagcaatcc cgggaggtct   1440
   ccgggcctct tcagggtgcg tccaggaggc gggttccgtg cgacgcggtg cagcccaccc   1500
   ccccccccga gaccgcttaa cttcgcgggg gcagcctcgg gcgctcggag acgcggaggc   1560
   ccagactgca gcctccggat gctggaagcc cagactccct ggggtcaccg gctctcccgc   1620
   caccccagct gcagagagtc ccattgcttc accgtccgga gcttagtctc cttgttcctc   1680
   taccagtccc tccctccgca ggtctctggg gacttctgac cgcctgttct tactctcccc   1740
   ctgcccccat acttcccgcc cttgtctcag gaacggtgat acagtcaccg gattgctctc   1800
   catctcctgt tagtctacac tgcacacaac tcaataatcc gcgcccttcc atccgggtga   1860
   cagagacaca gataatctga gctagtggtg ctcaaagtac cggtcccaga acagcagcat   1920
   cagcatctct tgggaacttg ttaaaaatga gaatttgggc cgggcgcggt ggctcacgcc   1980
   tgtaatccca gcactttggg aggccgaggc gggcggatca cgaggtcagg agatcgagac   2040
   catcccggct aaaacggtga aaccccgtct ctactaaaaa tacaaaaaat tagccgggca   2100
   tagtggcggg cgcctgtagt cccagctact tgggaggctg aggcaggaga atggcgtgaa   2160
   cccgggaggc ggagcttgca gtgagccgag atcccgccac tgcactccag cctgggcgac   2220
   agagcgagac tccgtctcaa aaaaaaaaaa aatgcgaatt tgggggcccc accccagatc   2280
   tactgaacag aaactctgtg gagcccagca gatgattccc atgcacacta aagtttgcga   2340
   gccactgatc taaacattct ttcatccatt cattcttcac ctggcccacc cagcattgcc   2400
   agtgggagag acacccgcaa agcaccaggc tgtgagcccc accgccgtgc actctgagac   2460
   actgtccact agctttggga tggcaggcag aggtactcca gcttggtcta gtgcagacc    2519
<210> 2
   <211> 2283
   <212> DNA
   <213> Homo Sapiens
<400> 2
   aatgaagacg ctggagatcg ggcccctgcc cgtccccttt ctgcgccccg ggatgaggca      60
   gagactgaac agccggcgag caaatcaacg gcatccagaa agccatgtcg gactcggcgc     120
   ccagcgccca agcgctaacc cgctgaaagt ttctcagcga aatctcaggg acgatctgga     180
   ccccgctgag aggaactgct tttgagtgag atggtcccag aggcctggag gagcggactg     240
   gtaagcaccg ggagggtagt gggagttttg cttctgcttg gtgccttgaa caaggcttcc     300
   acggtcattc actatgagat cccggaggaa agagagaagg gtttcgctgt gggcaacgtg     360
   gtcgcgaacc ttggtttgga tctcggtagc ctctcagccc gcaggttccg ggtggtgtct     420
   ggagctagcc gaagattctt tgaggtgaac cgggagaccg gagagatgtt tgtgaacgac     480
   cgtctggatc gagaggagct gtgtgggaca ctgccctctt gcactgtaac tctggagttg     540
   gtagtggaga acccgctgga gctgttcagc gtggaagtgg tgatccagga catcaacgac     600
   aacaatcctg ctttccctac ccaggaaatg aaattggaga ttagcgaggc cgtggctccg     660
   gggacgcgct ttccgctcga gagcgcgcac gatcccgatg tgggaagcaa ctctttacaa     720
   acctatgagc tgagccgaaa tgaatacttt gcgcttcgcg tgcagacgcg ggaggacagc    780
   accaagtacg cggagctggt gttggagcgc gccctggacc gagaacggga gcctagtctc     840
   cagttagtgc tgacggcgtt ggacggaggg accccagctc tctccgccag cctgcctatt     900
   cacatcaagg tgctggacgc gaatgacaat gcgcctgtct tcaaccagtc cttgtaccgg     960
   gcgcgcgtcc tggaggatgc accctccggc acgcgcgtgg tacaagtcct tgcaacggat    1020
   ctggatgaag gccccaacgg tgaaattatt tactccttcg gcagccacaa ccgcgccggc    1080
   gtgcggcaac tattcgcctt agaccttgta accgggatgc tgacaatcaa gggtcggctg    1140
   gacttcgagg acaccaaact ccatgagatt tacatccagg ccaaagacaa gggcgccaat    1200
   cccgaaggag cacattgcaa agtgttggtg gaggttgtgg atgtgaatga caacgccccg    1260
   gagatcacag tcacctccgt gtacagccca gtacccgagg atgcccctct ggggactgtc    1320
   atcgctttgc tcagtgtgac tgacctggat gctggcgaga acgggctggt gacctgcgaa    1380
   gttccaccgg gtctcccttt cagccttact tcttccctca agaattactt cactttgaaa    1440
   accagtgcag acctggatcg ggagactgtg ccagaataca acctcagcat caccgcccga    1500
   gacgccggaa ccccttccct ctcagccctt acaatagtgc gtgttcaagt gtccgacatc    1560
   aatgacaacc ctccacaatc ttctcaatct tcctacgacg tttacattga agaaaacaac    1620
   ctcccCgggg ctccaatact aaacctaagt gtctgggacc ccgacgcccc gcagaatgct    1680
   cggctttctt tctttctctt ggagcaagga gctgaaaccg ggctagtggg tcgctatttc    1740
   acaataaatc gtgacaatgg catagtgtca tccttagtgc ccctagacta tgaggatcgg     1800
   cgggaatttg aattaacagc tcatatcagc gatgggggca ccccggtcct agccaccaac    1860
   atcagcgtga acatatttgt cactgatcgc aatgacaatg ccccccaggt cctatatcct    1920
   cggccaggtg ggagctcggt ggagatgctg cctcgaggta cctcagctgg ccacctagtg    1980
   tcacgggtgg taggctggga cgcggatgca gggcacaatg cctggctctc ctacagtctc    2040
   ttgggatccc ctaaccagag cctttttgcc atagggctgc acactggtca aatcagtact    2100
   gcccgtccag tccaagacac agattcaccc aggcagactc tcacggtctt gatcaaagac    2160
   aatggggagc cttcgctctc caccactgct accctcactg tgtcagtaac cgaggactct    2220
   cctgaagccc gagccgagtt cccctctggc tctgcccccc gggagcagaa aaaaaatctc    2280
   acc                                                                  2283
<210> 3
   <211> 2236
   <212> DNA
   <213> Homo Sapiens
<400> 3
   caggtgccaa cgaccgcgcc agactggcgt tggaaccctt gggccataag gtcttagtgg      60
   gtggcgtcat tatctctgcg gtttgcaaaa gcggagtggc cctgtcaggc gggaagcctc     120
   tctcctctcc ccagatccgc gacaggccgc aggcaagaac cagcgcaacc agggcgcgtc     180
   cgcacagact tggaggcggc tgcatgctgc tacctgctcc agaaggcgtc cggtggccgc     240
   tccgcagttt cagagcctag agacaagcag aggaaggaag acaggacact tgggtcagct     300
   gcccgagcca agtcgctgca aacgctaata ccgcccgcag cctcttcgcc agtatccacg     360
   ctcaaaagta actcaagttt gcgcgccagt gggaaacttg gcgctcatga ctcgccagcg     420
   cgggtcgaaa ctccttcctg atgccctctc agctgttttt cttcccccgc gtggccagga     480
   ggggtaaata atatgcagtg gggcggggcg ttctggaagg ggtgtgccag ggagggaatg      540
   atgggggtcc caggcaaagc tccgaactct ttcacgtaac gggaggaata cagacacgtc     600
   ttagttaagc gtgcgtggga accgcggaat taaggcacct agaggccaag ggcttgtgtc     660
   aagctctgca ttttgccccg caggggaacc tctatacccc gcgattgaac tcgaaagact      720
   cctcccggcg gatgaaagcc gctactccct ggctggctga gctacagctc ccgcagcgcg      780
   cccaggagtg cgccggagat tcggaaaccc gcagagactt ctcaagtcag caggaacttg      840
   gaaaccgctg ttccctccaa acggcttcaa acactcgcgc tccttccttt acccaaccta      900
   attttaattt gccctctcta taggcttaat ttttattttt ttaaaatccc aaactagtaa      960
   gccaaaccga agccccagaa taaggttcaa tcaatgattt taattcaaca ccaagcccga     1020
   atgtttacct ctcggatctg acaaaccaga tgcagagcga cgaatggaga ccaccttccc     1080
   gggaggcgga acccagctgg gttccagcct gctctgggag aggagcacgc ctcccttgag     1140
   ctgcaggcgg gtccggctct gacgaaacgc tgcgagaatg ccagacagtg tagcctccac     1200
   cgtttgctcg gggtctctgc tgccatcaga caagtacttt tattcattca tcccttccca     1260
   tcaatcaccg ccagactcct cccgcgcctt ccgaccccgc tgcaaccttt ccccttgggc     1320
   gatgcctgga cagcatcagt agtagttgcc cgagggaggg gggcagtcct cgtccgggga     1380
   caggtcccaa aaggctgttt atttgttaga gttctacgct cttcaaatga acccaaatca     1440
   agggcaagtt atcacgcacc atgtgacacc cggcagctcc agactagaac aaggctctgc     1500
   actgaacgtg ataattgaac ttttaatgcc aagtgtcact tttctttcag gtaaatgttc     1560
   cgccgctgtg atatttaagg ttggtttgtg ggcgctgctc tttgctctgc ttcattttgc     1620
   cctgaagttt catttcaagc caacattaac cccaacacgg aacagctcga tcttttcttc     1680
   ccaggccctc tcactgcccc gagagaggat tacaagtact cctcattttc cccagtacag     1740
   ctcttctcct ctcttcctgc cacagtatcg gtgttaggca atctcaggca tttttatact     1800
   gtacctatga cagacttgat gcttatcgga gacatgaaaa taatcagaaa gctgccatgc     1860
   aaattcaaca tgatatttat ttaccaggta gagcaataca ataaggtctt tctatccatt     1920
   cttgagctcc atgtttaccg cagagaggaa aataacagcg actggtagtt ttgcccagag     1980
   attgtataaa agaggtcagg aagtcaggct tggagatgca ctggagagta ctgtgccgtc     2040
   ctccaatcag caaaatccct actggatctg tggaacttca aacccagaag cacccattcc     2100
   atgtagcagt ttcacccaga gacaggaggc tcgttttttg cttcatgtta gttaatggaa    2160
   tagatgggac aagaaccttg gatttttggc tcttaggcaa tgctttctgg gacagtggaa     2220
   gtagaaaatc aatttc                                                     2236
<210> 4
   <211> 2204
   <212> DNA
   <213> Homo Sapiens
<400> 4
   tccttctgca aatcggtcta agtttcggga ggtgggattg ggggaggagg gggttcaccc      60
   tggggccagg ccagggctgg gtggaggagg cggccttcga gagagtgagt cgggaaagaa     120
   ggggagtggg caaagggtga gatgtggggt cccggtcccc gcctcgctgg ctgccagttc     180
   cctctgcgtt tcctagtccg cgtgcgtgac acgagatagt gcagccctgg catggggctt     240
   ggcatctggc ggccgcgaga taaccagggc ccgtgcgatg ggaaacgtgc cgttggcagc     300
   ccgccacccc tccatcgtga cctcagtctg ccaaacaggg ttcgtgatgc tcagtttact     360
   agggaggagc cgcggctttg agatctcgcc cagcccgaag cagcggagac tggccaggag     420
   cccggctgtc agacctcgga gcgcacgctg cgggcggggg ctcggtccac gggggagggt     480
   caggggacgc gggactcacc cttgaaggag tcggggagcc gctgggcttc ggagtcccgt     540
   gtgtgccgct tctcggccat gctgcccgag acgcagtcgc actcccccgt cctcgttgcc     600
   aaacccggag ccgccgggaa tgccctgagg agccagaggc acaagaccca gattgccgcc     660
   ccgcccccaa tcgaggtccc gccctcgtga gagttggcca atcaacgccc aggatcgagg     720
   aggacccacc tcccaatccc cgccctcctg tccctccacg gcgcgactgc ttcgcagtca     780
   cttcccgcta gttctgtggg ctgaggggcg tggcctgagg ggcgtggcct gaggggcgag     840
   agcccccaag gctgtgggta tgaggccggg gccctgcacc ggaagggagg gcggaagcca     900
   cgggacgtgg agcagatggt gaggatagtg gggaggtagc agcagaggat gacccgaggc     960
   aaaagtgcaa gtgaagaaaa gtcgccttgc cgggcagagg tacgggacca aggcgcctcc    1020
   tctgggctga ggagttagag ggaagaaaat ggcgccctgt catgagggcg cgaggtccga    1080
   gttgagggga gaagactgag ggacacagaa gtggccgaag tgagggcgct gaggtcagcc    1140
   tgagctgaga gggccggcag atcacgccgc agccgaagtg aacgaactcg agtgaggtgt    1200
   gttaagaaaa tcaaaatgta taaatttgga aaaggagagg aggccgggcg cggtggctca    1260
   cgcctgtaat cccagcacgt tggaaggccg aggcgggtag atcatctgag gttagaagtt    1320
   cgagaccagc ttggccaaca tggtgaaacc ccgtctctgc taaaaataca aaaattagcc    1380
   gggcgtgtgg cgcgcgcctg taatcccagc tacttgggag gctgaggcag gagaatcgct    1440
   tgaacttggg aggcagaggt tgcagtgagc cgagatggcc tgggtgacag agcgagactc    1500
   cgtctcaaaa aaacaaaaca aaaaacaaaa aaacaggaga ggagacttta tttcttgtaa    1560
   ggggttacag cctgcaaggt ggccattccg caggctggag aatggccagg accagagaca    1620
   ggcatttcgt tggaggaggg gttggagtag cggctttatg ctgaacagct tgggtagaca    1680
   tgcatattca gcaggtgaca ggaggagcta tgaatattca tgagggtggt cctgacgcat    1740
   gcccattgag caaacatgca tgtaacatac aacccatgtt cattttggag tgaagagtta    1800
   ccaattaaat gttaaatgcc tggcctgcat ggtgacgtcc ccatctctac aaaaagtaaa    1860
   aaaaaaatag ccaggtgggg tggtgtgtgc ttgcggtccc aactagtcag gaggctgggg    1920
   caggaggatc acttgagccc aagagttgga ggctgcagtg atgtatgatc agatcactgc    1980
   actccagcct gatggactct aaaaaataat aacaatacat gtattacagt tagaccccat    2040
   gcgtcaaaag gtcttttcag gacaggaagg tgtgcaagta tgcagtctgt gtaaactagc    2100
   cagagccagt ccatggtcgg tagttttcct atgaggagaa agttactgaa atcagcctct    2160
   tgtccaatta aagctgtaat gatggctggt ggaatagggg ttcc                      2204
<210> 5
   <211> 2137
   <212> DNA
   <213> Homo Sapiens
<400> 5
   tctcattagt tagtacctaa ttatttaaaa gtgtggttta tgcttggaac tgggctataa      60
   agatggcaat gtgccaaatc agacatctgc ttcctgtaca ctcctataaa ttatatcagc     120
   ttattttaca actttgtccc tgtcagttat aaagaagaaa gtttaacatt ctagacgccc     180
   acacctcatc aaactgtggg tgaagagtca gtgagcggca aaaggtggcc gctttgagtt     240
   gtggtgatgg aattttcagg gaatagtcag gagcttggaa agtcactcct gagctggtcc     300
   attcaagcat tcgggataga agtggtcagt agaaattcct catggaatct caaaacacca     360
   ctaggacagt agctccacga cacttttaaa acctggagtt taaaaaataa tgaagtataa     420
   tttatttaga acttaagcat caagggaaaa cgttaagttc tgcttcactt caaagacttt     480
   tccctgagga aaacatgcca ctgtttttca gatgtgaatg aaatgatact ttttcccccg     540
   acatagtttc cttgcttacg actccacact gttctccttc aatccctttc accactgaaa     600
   cattacatgc aagatttgct ggcatgcccc ttgacttttc atgcttaagg ggaaaaatgt     660
   ttgtagctga tgtttgagtc ccaggagctc ggtgaagaaa aaaaaaaaaa gcaacgcaag     720
   gaaaattgct ctaatggtat atccttccta aagcttgaag tttcataggt aaaaggaggg     780
   ataaacattc cttcctgaaa gcaaacagtc aaggcttttt cccactcaac tatccctgaa     840
   ccttttgtaa tttccccgag gcctaaaccc agggcacaat taattccaga ccctatttta     900
   aatctaatcc gtctcctatt tgcaacctgc ctgcaggtgg gtcttttctt tctaaattat     960
   aaatctgaac ctgcgtcaac ctcttacaac acaaggcatt taaagcttcc tggagccact    1020
   gacctggtga cgctgtagtg cacgtctgca tttgaaagta agcaagagaa cagaaagcga    1080
   gcaagagaac cgcaaccttt accaccttgg cgaattcttg ccgggaccta catattaaac    1140
   agcacttgca cacagacgcc tcaagtcgtc ctctttagag tcgtcactta gacctgcaag    1200
   gggcggggga ccagtggaaa cccaaacaga aactttcacc attacaaagg cgagtcccca    1260
   ttgattatgg taattgtttt gctcaggttg tacgggtatg gccaattttg tttgaaagct    1320
   cgctctttct ctgcgctttc ctgtcgagtt cccttcaggt tctggtttcc tccgacggtg    1380
   gcaagggatg tttttcacgc ctttcaccac ttttgcccgc gcctgcgtcc cctgcccgag    1440
   gtcccaggcc gcggcggcgg cgcggtgggt tcattagcgg gcgcgccggg cccgcgcagc    1500
   gctgtctgca gccgcgtcgc cgcatccatc atcattacat cagcgcgggg aagtccacgc    1560
   cgagttctcc cggccccggc gcgccccagt cagcgcgcca gcaaacagaa catgctggat    1620
   tgctcagaga cggctctggg tgtctggggg cagaaggggt gctcccaggc ccggctttga    1680
   tccgcatttc tgaagctggc tttgggagac aatgcatctc cgccagccag gccgcgggaa    1740
   aagtgaaaaa agggctgggg gccatgtggg ggtggggagg tgagctccag cgccagcagc    1800
   gcctggcagt ctccctccag tgcagagaag cggtctgtat caaggcgtat cagagtggtc    1860
   accctgctcc tcggaatatt cacgggcatt tgcaaactct acagttggcc cagccaaatt    1920
   tccagcaaac actggcaagg gccggccctg gttgcgttca gagggctctt ttaactggct    1980
   gaattcaggg agctgcaacc ttccaggtac tttgcaatgc aaccaccctg agtgtcgcat    2040
   tataataaaa cattggggat cccttgaaaa gccttcacaa aaccaatagg tgtgtgcttc    2100
   gctcacaaaa cgtcgaattc accttctatg aacctgg                             2137
<210> 6
   <211> 2461
   <212> DNA
   <213> Homo Sapiens
<400> 6
   tttttttctt ttttcctttt tctctttctt ttcttttctt tctttctttc tttctttctt      60
   tctttctttc tttctttctt tttcgttttt ctctttcttt ctttttctgt ttcttatctc     120
   ctcagcttga aggttgcatc ctctttatct ccttaagtaa agctgggagt tctgtagtac     180
   tccaggagtg aggacaatta tgcttaagaa actttggttt cagtttagtc ccagaccaag     240
   aggaaaacat ttcaaaattt taaagtaaat cctaaaagtg taatttacac cagagcgatt     300
   gacctggcat ttatgtagac tgaagcttga gaagtaattc aagtggtttc cactcattcc     360
   cttggaagag tccttagaat gtacttccat tggtctgaaa aacgcctgaa aaagagtcct    420
   gatgctaaca gtaatggaaa taggagggct ggtggttcac gatcagccca gtggtatcct    480
   ctattctatt tgcatctagg taaagagact gtggcctgta tgtgatcaaa caatttgttg    540
   aagcgctttg ggcttagggc atttaatttc tgtgaagtag gaccatcagt tgtttgctct    600
   gtttttgtgt cacctctcag aatacattaa agtaggggca acccttgagc ccagacttct    660
   gccatgtgaa gaccctttga aaatcctgac aaacacaggt actgcgtaag tggtcagcta    720
   attaaagagg ggaggtggag ctgtcctttg tgtatccaat aagtacccat tatctcattt    780
   gagcatgaaa agaggccact gttattactt tcaagaagga aagtaagcag gatagctcat    840
   atttttagaa caattcctca ccaaatggaa taattccggt gaaaagtggg agtgaggaag    900
   aaagaaaaaa aaaacttcta atcataatgt ttgggaataa gaaaggaaga agaaactcac    960
   gtcaaagccg actttctcct gcagctgtaa aataaactct taagaccctt cctgctgaaa   1020
   ctctggagag gaaaactgga gtggcgggtg ggctttgcct gcagctcaac tctccctcgc   1080
   ggcgcgggcg cggctgggtt cagcacctcg gaaagcgccc ctcgcggcgc cccgggatta   1140
   cgcatgctcc ttggggcccg ccgccttggc cgtgcaagtg ccaccgtaac tggtgagagc   1200
   cgctggcaac ccacccggag ttgacaaccg cggagagacg cagacaccca ctgacctcca   1260
   ggaagctgag cgtggtggat ggaactctac gatctctttc tctccaagga cggaaacctc   1320
   atccaagcag tcccagagga aacggataaa ggtatttgaa agggagcgag cggccccaaa   1380
   tcgcacaatt gagcggctgg gggagttatg cgccagtgcc ccagtgaccg cgggacacgg   1440
   agaggggaag tctgcgttgt acataaggac ctagggactc cgagcttggc ctgagaaccc   1500
   ttggacgccg agtgcttgcc ttacgggctg cactcctcaa ctctgctcca aagcagccgc   1560
   tgagctcaac tcctgcgtcc agggcgttcg ctgcgcgcca ggacgcgctt agtacccagt   1620
   tcctgggctc tctcttcagt agctgctttg aaagctccca cgcacgtccc gcaggctagc   1680
   ctggcaacaa aactggggta aaccgtgtta tcttaggtct tgtcccccag aacatgacct   1740
   agaggtacct gcgcatgcag atggccgatg cagccacgat agccaccatg aataaggcag   1800
   caggcgggga caagctagca gaactcttca gtctggtccc ggaccttctg gaggcggcca   1860
   acacgagtgg taacgcgtcg ctgcagcttc cggacttgtg gtgggagctg gggctggagt   1920
   tgccggacgg cgcgccgcca ggacatcccc cgggcagcgg cggggcagag agcgcggaca   1980
   cagaggcccg ggtgcggatt ctcatcagcg tggtgtactg ggtggtgtgc gccctggggt   2040
   tggcgggcaa cctgctggtt ctctacctga tgaagagcat gcagggctgg cgcaagtcct   2100
   ctatcaacct cttcgtcacc aacctggcgc tgacggactt tcagtttgtg ctcaccctgc   2160
   ccttctgggc ggtggagaac gctcttgact tcaaatggcc cttcggcaag gccatgtgta   2220
   agatcgtgtc catggtgacg tccatgaaca tgtacgccag cgtgttcttc ctcactgcca   2280
   tgagtgtgac gcgctaccat tcggtggcct cggctctgaa gagccaccgg acccgaggac   2340
   acggccgggg cgactgctgc ggccggagcc tgggggacag ctgctgcttc tcggccaagg   2400
   cgctgtgtgt gtggatctgg gctttggccg cgctggcctc gctgcccagt gccattttct   2460
   c                                                                   2461
<210> 7
   <211> 1920
   <212> DNA
   <213> Homo Sapiens
<400> 7
   ccaggctgcc gtagacacag cctttgctct cccgaaaaac acgttctagg cgccgggatt      60
   ccagatacct gggaaataga gtgcacgcag ctgttgagag gcctcgcgct tggcttctcc     120
   tatcactgag gcgcagaggt gctgtggaca gcccagaccc acacggcgcc cgaggtgaaa     180
   cagaaccctc agtctcccta tgaggccact ggcactctcg gctgtcccca gagctctccg     240
   acttagagct gaatgcaaag taagcgctcg aaatgcagaa gtagccgggg ccgcccacgg     300
   cacctgcctc gctcggggcg agagaagacg ccaggctgag gtcccagcga cctcaggcac     360
   cagctccgaa ggagggcggg gagaccgcaa aggggaagtg cccggagggc caacggcccc     420
   cgcgcaccct gcgcccctct gaagcgcgcc gcctccccgc gccggggact gggacctgcc     480
   tctggggaat ccgcctagaa gacggcggcg gactggggtc gggcactctc cagggctgtc     540
   aggccctccc cagccctgca cctgccgcgc cgccccacct cgccaggaag tctcagagac     600
   cccggggatg gggtgggagc gccttcccat cgcgggctca aaaagaagga aggacgcccc     660
   caggggtcgt agaaggagga ctagctccaa gccacaactt tcttcggacc caaggcaggc     720
   cggctggggc tccgcgccta cacggcccct ggcgggggtc cgcgcgcccc gggagccccg     780
   cggctcgggg aggaaagagg agacaagaga caggcgagga ttacggggct gacccagccg     840
   gggtagggac catcgtggaa aaactttggc gaggtggggg gacgcggaaa gagagcggcc     900
   cgcgccctgc accttgcgcc gggcatcccg cgccagtgcc tcgctcccag tgccccgcgc     960
   cccgcgcccc gcgccttgcc ttcaccccgg gccagctgca tcgcgcccgc gccgcaggaa    1020
   ccgtggagtt ggaaagtggg ggcgccgcgg ctggggggct gcttcagctg cgcctcggcc    1080
   agcgatcggc gggccgggct caaatccagc caggctgggc aggcggtggc cgcgcgactg    1140
   gggaccgggc gccccgccct cctcgctccc ctcctccttc ctctccctcc ctccagcccc    1200
   ttggcctttt tcagccccta ccggatctgc tcgtccgctg tcctctcttt tctctcgctc    1260
   ttcatatcac tctccacccc ttcgccttgc cttcgccttt cttcctcccc ttgtctcctg    1320
   ccccctcctc ttctcccctc ccctctaggg gcggagcttc tcccctccct cccagacaat   1380
   gctgtggctg cgtccccttc cccgccagct cgtccaggct cccgccgcca gcgattcttc    1440
   cgggctgggg gtggggaggt ggggggggag tgcagggttg gggaggatga gctggctccc    1500
   ctcacctcct tgctgctgcc ctctccaaga gggatggaga cttggcccaa gctcctcggt    1560
   tcacccggag ctgtgacagc cactcccagg gaacagtcac gctgccctac caagcccacc    1620
   tccagcggcc tggattcccc aggcagaggt tgtgggattt tgttttttct aacatcccag    1680
   cttattccca aaagggtttg agccggacag gggctaaaca ggccccttcg acttggcggg    1740
   ccggccagac gtgacagcaa tgccaaggag gccaagtttc tttgtccatt tctcacctcc    1800
   cccttttcca tccctggacc tcctggcgcc cccagtacac agaggccctt gagcagcccg   1860
   gctgcaggtt ccctatctac tcagagttct ccccctcacg tgcctatccc caaccctgca    1920
<210> 8
   <211> 2225
   <212> DNA
   <213> Homo Sapiens
<400> 8
   atcttcctgt aaaccaaatt cctgctgccc agtccagtgc ctggtgcata gtgggggcta      60
   gacagacatc tgttgagtgc atgaccaggg cgctgtaggt ggaggaggtg gtggtggggt     120
   gggggagggg aattcccctg ctgctccccg ttctgtggga accagctcca gaaagaaatc     180
   ctctgccgcc cttcccaccc tactccaact ttggatctgc tctctgacct ccttcacttg     240
   gccctagttc tccacccaga gtcaaccctg cctgaaaggc accatgtcct gtcaccaagg     300
   tctcaaaata gggaaaatag tctctgaagc cctcagcccg caggagatag ggcaccccag     360
   ggtacaaggc ttcaccgagg ggctggaaaa agagcccaga tcccaacacc tatagaaagg     420
   ggccctggct gtccagctgt gagtggcctt cccctcgcct ctcagcacac ctggaaacga     480
   ctccacatgt gggcgccgcg ggatgctggg tagcaaggag atgacccagc tgaacaatgc     540
   atttggtcca ctcctgcgag gggaggagaa cgccgaggga caggaactgc gcgccagcaa     600
   aagacaccct aacctggggt gcgcccgcca gctgctttcc caaggtgaaa acctccaaga     660
   ggcgagatgc aaatcccagt ggcgtccaca cccggaggtg tcttctctcc aggtcgcccc     720
   tccgtctcat gcgacacccc aatccccgca gcctctgctc agcgtgggac caagaatggg     780
   gagtcctacc cctcctcggc tttccagccc cctaacccaa gacaagaggg cgcgcgcccg     840
   ggctgggaca ggtcccagcc gcattttcgt agattgcact ccccgcccgg gagcgacccg     900
   ttccagcagc ccccttcccg ccccatcaac gcggccgcac tgtcggcgtc tagaggcgac     960
   atcctggcaa agacaggaat caagtcagaa attcaggctc tagaagcaaa ggctgcggtg    1020
   cctcctggct ggaagaaaac ggcgggtggg tggaggatgc ggtgcgcggg aatttcccca    1080
   cggacgcttg ctgtttcctg ctcacctttc aggaaggtgc ccctctacct gcaccctccc    1140
   ctgcaatttg gagctcgtcc gcgagctcag ctacccgctg gctccctctg cccgcgcccg    1200
   cacaccacag actccgctta ccgtacccta cccggtccta gagggcccag aggggatctc    1260
   ctgaaaccaa ctcgcctgct tggagccagc cccgacgcat atctcgagag tggcgggagg    1320
   caaagcagcc ggaatgcccg agaaaaaagt gggcgtcaga ggcccttgtc ctagcgactc    1380
   ccccaccgca ccgactgagt cccgggactg cagggatcta agcccttacc tgtctcggcg    1440
   cctcctttct gtgcgctctg ctgctccgga agccggatcg agagtcacag tgtggttggg    1500
   cgcaggcggg ccgctcggtt ttcacctcga ggacccggac ttgcacttgc agagccgctg    1560
   cgtgggggat gtccctcttt tttcctcgcc ccggcgggct cctccggcgt gtcagggtct    1620
   ctgggttcca gctggagttg gcgctccggc cccacagctc tttccagcgt ctcggacctg    1680
   tgcgccccgc cccgcgctgg actccgcccc ggggtccccg cctgcctcct agtccaggcc    1740
   aagggtggat gcccagctcc cccaccccac cgcgtgctct cgggctggtg cgtccagggc    1800
   gcgggcaccg tagctccggc cgcgctgacg cgagtccccg gcagcggggg cagcctctcg    1860
   cacggatttc agcgccttcg catcccgtct gaatcggacc acacagtagc cgggtttcta    1920
   ccgtgcgctt gggaacagtg agcaccgccc tctgccgcca gtcgtggcac cgcacccggg    1980
   tcgcgcgcgc aggaggggag agtcctgtct tccggaggtg cccagtgcca gcgtccgcgc    2040
   ccactagaag gtgacccttt caaggaaagc ccggctccga cgagtgcggt gtgggccttt    2100
   ggagatggcg gtgaaggagg aggcccccag gaggaagctg ggatggggga cagtttgctc    2160
   tgctcaaagc ccctgaagtc ctcacccaaa ctttgcgatg accaccacta tcagattcca    2220
   tggcc                                                                2225
<210> 9
   <211> 2251
   <212> DNA
   <213> Homo Sapiens
<400> 9
   cttttcagcg tgactgaccg cgactcagag gagaatgggc aggtgcagtg cgagctactg      60
   ggagacgtgc ctttccgcct caagtcttcc tttaagaatt actacaccat cgttaccgaa     120
   gcccccctgg accgagaggc gggggactcc tacaccctga ctgtagtggc tcgggaccgg     180
   ggcgagcctg cgctctccac cagtaagtcg atccaggtac aagtgtcgga tgtgaacgac     240
   aacgcgccgc gtttcagcca gccggtctac gacgtgtatg tgactgaaaa caacgtgcct     300
   ggcgcctaca tctacgcggt gagcgccacc gaccgggatg agggcgccaa cgcccagctt     360
   gcctactcta tcctcgagtg ccagatccag ggcatgagcg tcttcaccta cgtttctatc     420
   aactctgaga acggctactt gtacgccctg cgctccttcg actatgagca gctgaaggac     480
   ttcagttttc aggtggaagc ccgggacgct ggcagccccc aggcgctggc tggtaacgcc     540
   actgtcaaca tcctcatagt ggatcaaaat gacaacgccc ctgccatcgt ggcgcctcta     600
   ccagggcgca acgggactcc agcgcgtgag gtgctgcccc gctcggcgga gccgggttac     660
   ctgctcaccc gcgtggccgc cgtggacgcg gacgacggcg agaacgcccg gctcacttac     720
   agcatcgtgc gtggcaacga aatgaacctc tttcgcatgg actggcgcac cggggagctg     780
   cgcacagcac gccgagtccc ggccaagcgc gacccccagc ggccttatga gctggtgatc     840
   gaggtgcgcg accatgggca gccgcccctt tcctccaccg ccaccctggt ggttcagctg     900
   gtggatggcg ccgtggagcc ccagggcggg ggcgggagcg gaggcggagg gtcaggagag     960
   caccagcgcc ccagtcgctc tggcggcggg gaaacctcgc tagacctcac cctcatcctc    1020
   atcatcgcgt tgggctcggt gtccttcatc ttcctgctgg ccatgatcgt gctggccgtg    1080
   cgttgccaaa aagagaagaa gctcaacatc tatacttgtc tggccagcga ttgctgcctc    1140
   tgctgctgct gctgcggtgg cggaggttcg acctgctgtg gccgccaagc ccgggcgcgc    1200
   aagaagaaac tcagcaagtc agacatcatg ctggtgcaga gctccaatgt acccagtaac    1260
   ccggcccagg tgccgataga ggagtccggg ggctttggct cccaccacca caaccagaat    1320
   tactgctatc aggtatgcct gacccctgag tccgccaaga ccgacctgat gtttcttaag    1380
   ccctgcagcc cttcgcggag tacggacact gagcacaacc cctgcggggc catcgtcacc    1440
   ggttacaccg accagcagcc tgatatcatc tccaacggaa gcattttgtc caacgaggta    1500
   aggctgaagc gaaaggacca ccatctctca tctcctccat cagaaagcct cctctagccc    1560
   ggcccttgta tctctggtgc actgtatcta tttttaggat attagcttat gtgtatcgtt    1620
   gtgggagcag agatgggcgg tcaccttctc ccactccttc gtgtgtaacc taactttcgc    1680
   gttgttccac cctttcacat ttattttcat tccgtcccct tggtactttg ccaccttgga    1740
   gctccctcct ttgctcttcc atcctgtcag tcctttccct tctcagtaac ctgggcatga    1800
   agggaaactg cgtgaaggga gagggaaatg tggaggaggg acttactttc tagcactggc    1860
   aaaggtcttt tttctttgcg tctgtcccag gcattaataa agttggctct attttgcttt    1920
   gtttaacgat gcttttagtc gcgtgtacaa gtaagctata gattgtttaa ctttacacag    1980
   ttgtctaacc tcgaattcat gttttagtga acaagttacc agatccttct cctctgaact    2040
   cgggttgcaa aaaaagcctt cagttcggct ctggacagca tttacagacg ctcttgaagc    2100
   cgagcgccca cacagtgtga atttgaatga agctgcgttg gcacaaaccc ctgttaagag    2160
   taaactaacg aaaggcttaa acaattgttg tcttaaatat atctttttag ctaataatca    2220
   ccttcttgcc actgttttgg ataaatcacg c                                   2251
<210> 10
   <211> 2553
   <212> DNA
   <213> Homo Sapiens
<400> 10
   ctggccaccc ctgccctccc ttagacctcc agagccccca gtgtagccac agaggatgct      60
   gttggcttca gccccaagaa gacgccgctt cctccagagg gctaagtaag tgggaatccc     120
   cctccctact tgtcctgggc tccaggcagg gcccctggtg taaggcctgg ggctggaagc     180
   cgacccacct aggtccaggc tctggggcag aactgaaact ccttggttac tgtcggctgc     240
   agcctgggag caggccactg ccaaagctgt gggtccttcc aggacagtct ccccatgagg     300
   ccggtcctcc acctgctgtt tcttcacacc tggtggccag ggatgtggcc ctgggtagaa     360
   cgatgattct ccactcctgt cattatggaa gccaccgctg tctcccagcc cagccagcca     420
   cctgggctgc agagcacccc tttcatgccc tccgggtgcc tcccccttct cctgccccag    480
   cctggctttg tcctaccctg ctctcaggga ggggtaccct ggagtggggc cagggcatgg     540
   ctctcccccg agggagttcc tctctggctg tccccagggc agctctgcac agcctcagta    600
   cctggcgcac ctcccttgac atccttctta gggacagtca ggcactctgt gtggggcact     660
   caagagagcc aggcccgtca gcctctagct cctgccagaa tgcaggcctg aggggtgagg     720
   ggcggggcag gggcagggac aggaactccg gcgtgctctc catccgcaaa ggttcactga     780
   ggccccgagc cccagccact gagccaccaa gtcagcctgg gccaggcctg ggtgccctgt     840
   ctgcaatgga ggcagagacg gggtctcggg gcagttctga ggatgctggg tgcacagcgg     900
   gggcctcgcc ggcaggaatc acttatgctc tctcctgggc caagctttgt ggatgcccag     960
   cctggggccg cggggagctg gcaggtcagt ggcagacact ggtgggcaga cctagtgtct    1020
   ggtagaacag gcatcaagga agtggtgacc ggagggaagc caagtgcact caaaccctcg    1080
   ggtgagtcat caccgccggg tctttcacag ctgctgaaag tgagcaacag tgatgaaggt    1140
   ttgtgagttt ctgcgtgagc gagtgaatgg accagtagca gtttccaggt tgtggaagag    1200
   cgttccctcc ccgggatggg gacacttggt tacagcaatt cctaatcccc cacccaccca    1260
   ccgcccactg cagaggtatg cgggggccct gcttcctgca ggcaggagtg aggggcactc    1320
   ctgtgatgtg gcacccctgt gaccgaggtc atgtgtgatc ggtgtaaggg caggaagcga    1380
   gtcattggtc tgcaccaggc gtgggggctt ctgcgagggc aggacccaaa gtcggcctgg    1440
   cctcccggct gcagcactcc tttccctttc gaattaggtt agagccctgg gacgggaggt    1500
   gccctgtaga ccacccccct caccaacttc cgtcctccgc cccacccccg cggtgatccg    1560
   gtgaactgcc ggccccctgc tgtgcaccga gtggggcagt gaccctgacg tggcgtctcc    1620
   tgccgcccct gccaccgcca ccacctccgg tggcccagcc tccgcattcc ccacccccat   1680
   ggaggaatgc accaggcctc ccttcctgga tgcacccctc acccacatgc ttccaaaccc    1740
   tggcattttc tgctccccct ttactcccac cccttcccct aggctcccag acaaagggga    1800
   agtggctgga tcctcttaaa gggacagtgt cccaccagct tactgctgaa ctcccctcct    1860
   caaccccagt tccctagtta cagttaatta gcattagcag acagcccatg agtgataccc    1920
   atgcaggccc caggctgtgg agagtttcct gggtaggaaa cagcccttaa ggtccctcat    1980
   ctcatccagg tcccagtctt tcctacctgc ctctctccta gattgtggcc ctttggagcc    2040
   tggttcttct gtccctgtgt gaccgacaca tagcacccaa acagtggcag agcgggacgg    2100
   accccctagc ctgttctctg tgtgggtctg taccctgacc cagacatgcc cccccacagc    2160
   aggacccagg ggggcacatg tgtgcctgcg ggttcactgg ggcacccgca tttggtttat    2220
   tttatttttt agagagaggg tcttgctgtg tcacccagct ggagtgcagt ggtgtaatca    2280
   tagcacactg cagccttcaa ctcctgggct caagcgatcc tccctcccca gcctccctag    2340
   tagctgggag tacaggaccc actgtatcct ggctaatttt ttaataattt tttaagagat    2400
   ggggtcttac tgtgttgccc aggctggcct caaacctctg gcctcaagtg atcctcccac    2460
   cttcgcctcc tgaagtgctg agattacagg catgagccac catgcccatc ccagactgac    2520
   atttctatat ttgttcatcc tggctgggca ggg                                 2553
<210> 11
   <211> 2001
   <212> DNA
   <213> Homo Sapiens
<400> 11
   ggcaaaagcc tgcctggact tcctggccac cagaaatatg agcatggtgg tggtccccag      60
   ttccctattc atgcttgggc tcaagagact gggagtctag gttcactgac tccctgagaa     120
   agactaagac cctgcatttt agaaagaggt ttggggatct ctgccctgcg caagggtaga     180
   aggatcagct gttcctctga gcaccttaac ccggaacccc ggtccgaagc cgagacagga     240
   gactggatgc gaggccctcc cagagctggt ttctctcaaa caacttccaa aactcctaga     300
   tcctaggggt acgccgaaat cccccaaagc agtccaaaga acacaacgag agtcctaaca     360
   tcccaggtgg cggcgcgctg gctccctgga gcggggcggg acgcggccgc gcggactcac     420
   gtgcacaacc gcgcgggacg gggccacgcg gactcacgtg cacaaccgcg ggaccccagc     480
   gccagcggga ccccagcgcc agcgggaccc cagcgccagc gggaccccag cgccagcggg     540
   accccagcgc cagcgggacc ccagcgccag cgggacccca gcgccagcgg gtctgtggcc     600
   cagtggagcg agtggagcgc tggcgacctg agcggagact gcgccctgga cgccccagcc     660
   tagacgtcaa gttacagccc gcgcagcagc agcaaagggg aaggggcagg agccgggcac     720
   agttggatcc ggaggtcgtg acccagggga aagcgtgggc ggtcgaccca gggcagctgc     780
   ggcggcgagg caggtgggct ccttgctccc tggagccgcc cctccccaca cctgccctcg     840
   gcgcccccag cagttttcac cttggccctc cgcggtcact gcgggattcg gcgttgccgc     900
   cagcccagtg gggagtgaat tagcgccctc cttcgtcctc ggcccttccg acggcacgag     960
   gaactcctgt cctgccccac agaccttcgg cctccgccga gtgcggtact ggagcctgcc    1020
   ccgccagggc cctggaatca gagaaagtcg ctctttggcc acctgaagcg tcggatccct    1080
   acagtgcctc ccagcctggg cgggagcggc ggctgcgtcg ctgaaggttg gggtccttgg    1140
   tgcgaaaggg aggcagctgc agcctcagcc ccaccccaga agcggccttc gcatcgctgc    1200
   ggtgggcgtt ctcgggcttc gacttcgcca gcgccgcggg gcagaggcac ctggagctcg    1260
   cagggcccag acctgggttg gaaaagcttc gctgactgca ggcaagcgtc cgggaggggc    1320
   ggccaggcga agccccggcg ctttaccaca cacttccggg tcccatgcca gttgcatccg    1380
   cggtattggg caggaaatgg cagggctgag gccgacccta ggagtataag ggagccctcc    1440
   atttcctgcc cacatttgtc acctccagtt ttgcaaccta tcccagacac acagaaagca    1500
   agcaggactg gtggggagac ggagcttaac aggaatattt tccagcagtg agcaggggct    1560
   gtatgggacg cgggaggagc tcagaggagg cgcggagagt gcccgaggtt gggtgagtgc    1620
   ctagagggga gatagttgaa ccgggttcaa gaggtgctta gtgggtgttt gttgaatgaa    1680
   tgagtgatgg gctttgaagt ctgagtgcat tgaaagaggg ggtgtgtaaa aagggctcct    1740
   ttcatcacac aggacacagc atatgcaaat cctctccctg tggaaaagcc agacaggtta    1800
   aaaaggttac aaacaaatta gccgggcatg gtggtgcgcg tctgtagtcc cagctactag    1860
   ggaggctgag ccaggggaat cgcttgaacc cgggaggcgg agattgcagt gagccaagat    1920
   cgcgccactg cactccagcc tggaaacaga gcgagactcc gtctcggaaa aaaaaaaaaa    1980
   aagttacaaa ccgtgtgtgg g                                              2001
<210> 12
   <211> 219909
   <212> DNA
   <213> Homo Sapiens
<400> 12
<210> 13
   <211> 1405
   <212> DNA
   <213> Homo Sapiens
<400> 13
   cgttacccga gttgtaaagg gcggctccct gtgtctgccc cgctgcaccg atacaccgag      60
   ctgcgcacgg tgcccagcgc agggagaaca aatgatcatc tgtccaacgc gcccatttac     120
   aggtgaggaa actaaggctc caactcaatc gacgcactct gcccttttga ttaccagaaa      180
   agtagcagga caggtgtcct gtcccgccct accccggccc actaagccgg caccccggct     240
   ccgacccccg gctgtgcccg gcgccgccgc ggtgcccggc gccgccgcct cgcccggcgg     300
   ggccgcccgg agcgcccgca cctccgcccg cttccacctg gccgggcccg ccccgcccgg     360
   actcgggact gggaagtgcg gcgactcccg gaaccagcca ttggcgccag cgcggggagc     420
   tgggggtgca gagctgcggg cgcggcgggc cacgcaggcg gcccccaccc ccggcctggc     480
   ctggtctggt ctggtctgcg ctgccgcgcg ggggcgcccc ctcccaggcc cggcgcccgc     540
   cagccccgct ccgccaggtg cagcgcagcg caggggtggg cgggggtggg gctcggcgcg     600
   cacgttcacg gggcggggag ggggcgggtc aggggcggga ccacagccgg ctgggccggg      660
   gttctatgcg catctccggg gaggggcggg gcgggggcgg ggccggggcg gggcccggtc      720
   ggtgcactcc agacggcggg ccgccccctc ttcccgcctt cctactaccg gcccaggatt    780
   agcgccctgg gagcgcgcgc cccgctgcct cgccgccaca ctttcctggg agcggcggcc     840
   acggaggcac catgaagaag tcttactcag gtgggcttcg cgcccggggt ggggaggggt     900
   cggtgtcccg ggaccagcgc tgctcacctg agtgcctgcg gccgggagtg gcgaggcgcc     960
   cccggagctg agcgagtccc cgcggcgggc acactgcagg tcgagttcct cccaggacag    1020
   ggccgctgtc gggccgcttt cgacctgagc cgaccgtccc ctgcgctgtc tccagccctt    1080
   gctcgagtgt cggaggggct gccctggggg acgctccctc ttcctcgccc cttgcaccct     1140
   cgcaggaatc gctgactttc caggtcggcc gggtgctttg ggtccctgtg cgtctgtgtg    1200
   ggtgaatggg gtcggggcta ggtggagggg tgtccttggg ttcagcctct agggctggtg    1260
   gtccaggccg cagcatcctt tcttcggatt ctcttcggtt tctcctctac ttagtggggc    1320
   acgggacggc ctccagatgg gaccgtccag cagcgcccaa acttggcgac tcgggttcac    1380
   gttttgcgct caggacgccg cccgc                                          1405
<210> 14
   <211> 1818
   <212> DNA
   <213> Homo Sapiens
<400> 14
   cgtcgttcgt ttttggtttt tttgatagtc gtgttttatt ttcgttttgt gttttttttt      60
   tcggatagtg ttttttttag ggtttattta ggagggtgta gcggtggttt tcggggcggt     120
   ggtcgtggtg ggggtgttag ttgtaggggt gttttcggtg ggtgggagtt ggtggttttt     180
   cgttggtgtt atgggattcg tatgttcgtt ttgcgttttt cggtttttga gtttataggt     240
   cgggattttg tttgttagtc gcgtgcgttg tcgtttaatt tttgtaggcg tagagcgcgc      300
   ggcggcggtg atagagaatt ttgtttggtt gtttaaatat agttttttgt agaaggattt      360
   tgcgttcggg gaaggggagg aatttttttt tttttgggcg ttcgtttttt tcgttatggt     420
   tcggttttta tattcgttta tatttggtcg tagcggggcg ttcgggggga ggggttgagg     480
   tcgcgttttt cgtcgttttt tgggcgcggg ttaggcgggg aggagggggg cgtttcggtc      540
   gtgtgtttag gattgttttt tagcggttat tcgggtttta gttttttagg tttggttttg      600
   ataggcgggc ggagtagtta gtgcgagata gggaggtcgg tgcgggtgcg ggaatttgat     660
   tcgttcggga ggcgggggcg gggcgggggc gtagcgcgcg gggaggggtc ggcgttcgtt      720
   tttttttttt atttatttag ttgagttagg gggtttaggg gttttttcgg cggttagttt       780
   tgtattgtag gagcgcgggc gcggcgtttt agttagcgcg tagggttcgg gtttcgtcgg     840
   gggcgttttt tcgtcgttgt ttttcgcgcg attcgttgtt tattagttat tatgtcggat     900
   ttcgcggtta acgcgtagtt ggatgggatt atttcggatt tcgaaggtgg gtgttgggtt     960
   ggttgttgcg gtcgcggacg tgttggagag gattttgcgg gtgggtttgg cgcgggacgg    1020
   gggtgcgttg aggggagacg ggagtgcgtt gaggggagac gggattttta atttaggcgt    1080
   tttttcgttg agagcgtcgc gcgttttcgg tttcgtgttc gcgtcgttta cgtgggggat    1140
   tttgttaggg gtattcgcgt agattttgcg cgtttttata ggattttgtg ttcgttttgc      1200
   gtattgtcgt ttgggttttt ttttttttat tgttgtttgt gtttgttaag cgatagcgat    1260
   tttttcgagg gttcgcgagg ttgtttcgga attttttagg acgtatagtt ttattttggg    1320
   aaatttatcg gttttttttt tttggttttt ttcggcggtt ttcgggtttc gtttggattc   1380
   ggtaacggga tagggaggtc gttttttatt ttcgattgag tggatagtcg cgttttgttc   1440
   gggtggatag tttttttttt ttttacgtta gtttcggggt cgttaagttg tgtagttcgt    1500
   gggtcgggag tatcgaacgg atatagttta ggtcgtggta gggtttagag tgggatgttt    1560
   tatggttttt atttaggttt ggggatattt ttattcgttt tttagaatcg ggtcgtgggg    1620
   gatagaaggg gtttgcgtgc gggtagggag agtattttgg tttttttttg ttttcggggt    1680
   ttataaagtg tgttgggatt tgcggggttg ttttgtttaa gtttgggttt ggcgttcgcg    1740
   tttttgagtt tgtgagtgcg tgcgtttttt tgcgtttttt tgattgtcgg tgttggggtt    1800
   ttgcgttttg cgttcgcg                                                  1818
<210> 15
   <211> 2519
   <212> DNA
   <213> Artificial Sequence
<220>
   <223> chemically treated genomic DNA (Homo sapiens)
<400> 15
   gatttatgag tgaatgatta aaagtgtagt tgagttttgg tagagggtat ggggttttat      60
   ttagagatag gtagagaaag ttgaagtttt aggattggag gtcgtttttt tttatttttt     120
   tattaggttt aggtagggtt tgatttgaac ggaggtttgg gaatttgtgg ttagttttta     180
   tttgttggaa aagagtagtt tttaagttta attgttttag gttgatgttt ttttattttt     240
   ttttatttat ttatttttat tattattttt tttttattga gacggagttt tattttgttg     300
   ttaggttgga gtgtagtggc gcgatttcgg tttattgtta ttttcgtttg ttgagtttaa     360
   gttttagttt tttgagtagt tttttgggta gttgggatta taggcgtgcg ttattacgtt     420
   aggttaattt tttgtatttt agtatagacg gagttttatt attttggtta ggatggtttc     480
   gattttttga tttcgtgata ttttcgtttc ggtattttaa agtgtcggga ttataggtat      540
   gagttatagc gttcggtttt tagttttttt taaaaaacgt tagattcgtt cgttgcgttg     600
   agtggaggcg gggtaggttt tcgtttttta attggtttta tttatcgagt tttttttttg     660
   tgtcgggttt tgtgttaagt atattatacg ttgtattttg cggttaggtt gttgtggttt     720
   agggtcgtat tttggtttaa ggtcgtaaat cgaggtggga tttcgattcg gtaattacgt     780
   tcgtggttcg gaaacggcgt tttttgaggt ttaggagagg tcgggcggtg agcggttgtg      840
   gagtcgagcg cgggtagtgc ggatgttgtt tatgggggag gtagttaagg acggagggcg      900
   agaggcggtt tttttaaggt tatttttttt cgggttgtaa gtaaaggtta ggggatttcg      960
   gaatggttag tgtaggagtt tttttgtgtt ttttacgttt tagattttta gagttttaga     1020
   aacggagatt atcgttttta tttttatttt atagatgaag aaattgagtc gaggaaagga     1080
   agcgatttgg ttaaggtcgg agagtttatt ttttgtaggg cggggtttgg aattcggggt     1140
   ttggttttcg gtaacgcgtt ttcggttcgt agttttttgt tttttgtgtt tcgtttcggt     1200
   ttttagcgta gttgtagagg ggtttttttc gacgtatata tttaagagtt cgatcgcgcg     1260
   gttgaaatcg cggagttcgg agtcgcggtt ggttgagcga tcgcggtttt tgggttgcgt     1320
   gcgcgttttt tggagttgaa aggagcgtta ggatcggggg cgttgtatcg ggttgggttt     1380
   tttaacgttc gtagatcggg tcgggttgta gttggagatg gtagtaattt cgggaggttt     1440
   tcgggttttt ttagggtgcg tttaggaggc gggtttcgtg cgacgcggtg tagtttattt     1500
   tttttttcga gatcgtttaa tttcgcgggg gtagtttcgg gcgttcggag acgcggaggt     1560
   ttagattgta gttttcggat gttggaagtt tagatttttt ggggttatcg gttttttcgt     1620
   tattttagtt gtagagagtt ttattgtttt atcgttcgga gtttagtttt tttgtttttt     1680
   tattagtttt ttttttcgta ggtttttggg gatttttgat cgtttgtttt tatttttttt     1740
   ttgtttttat atttttcgtt tttgttttag gaacggtgat atagttatcg gattgttttt     1800
   tattttttgt tagtttatat tgtatataat ttaataattc gcgttttttt attcgggtga    1860
   tagagatata gataatttga gttagtggtg tttaaagtat cggttttaga atagtagtat     1920
   tagtattttt tgggaatttg ttaaaaatga gaatttgggt cgggcgcggt ggtttacgtt     1980
   tgtaatttta gtattttggg aggtcgaggc gggcggatta cgaggttagg agatcgagat     2040
   tatttcggtt aaaacggtga aatttcgttt ttattaaaaa tataaaaaat tagtcgggta     2100
   tagtggcggg cgtttgtagt tttagttatt tgggaggttg aggtaggaga atggcgtgaa     2160
   ttcgggaggc ggagtttgta gtgagtcgag atttcgttat tgtattttag tttgggcgat     2220
   agagcgagat ttcgttttaa aaaaaaaaaa aatgcgaatt tgggggtttt attttagatt     2280
   tattgaatag aaattttgtg gagtttagta gatgattttt atgtatatta aagtttgcga     2340
   gttattgatt taaatatttt tttatttatt tattttttat ttggtttatt tagtattgtt     2400
   agtgggagag atattcgtaa agtattaggt tgtgagtttt atcgtcgtgt attttgagat     2460
   attgtttatt agttttggga tggtaggtag aggtatttta gtttggttta gtgtagatt      2519
<210> 16
   <211> 2519
   <212> DNA
   <213> Artificial Sequence
<220>
   <223> chemically treated genomic DNA (Homo sapiens)
<400> 16
   ggtttgtatt agattaagtt ggagtatttt tgtttgttat tttaaagtta gtggatagtg      60
   ttttagagtg tacggcggtg gggtttatag tttggtgttt tgcgggtgtt ttttttattg     120
   gtaatgttgg gtgggttagg tgaagaatga atggatgaaa gaatgtttag attagtggtt     180
   cgtaaatttt agtgtgtatg ggaattattt gttgggtttt atagagtttt tgtttagtag     240
   atttggggtg gggtttttaa attcgtattt tttttttttt tgagacggag tttcgttttg     300
   tcgtttaggt tggagtgtag tggcgggatt tcggtttatt gtaagtttcg tttttcgggt     360
   ttacgttatt tttttgtttt agttttttaa gtagttggga ttataggcgt tcgttattat     420
   gttcggttaa ttttttgtat ttttagtaga gacggggttt tatcgtttta gtcgggatgg     480
   tttcgatttt ttgatttcgt gattcgttcg tttcggtttt ttaaagtgtt gggattatag     540
   gcgtgagtta tcgcgttcgg tttaaatttt tatttttaat aagtttttaa gagatgttga     600
   tgttgttgtt ttgggatcgg tattttgagt attattagtt tagattattt gtgtttttgt     660
   tattcggatg gaagggcgcg gattattgag ttgtgtgtag tgtagattaa taggagatgg     720
   agagtaattc ggtgattgta ttatcgtttt tgagataagg gcgggaagta tgggggtagg     780
   gggagagtaa gaataggcgg ttagaagttt ttagagattt gcggagggag ggattggtag     840
   aggaataagg agattaagtt tcggacggtg aagtaatggg attttttgta gttggggtgg     900
   cgggagagtc ggtgatttta gggagtttgg gtttttagta ttcggaggtt gtagtttggg     960
   ttttcgcgtt ttcgagcgtt cgaggttgtt ttcgcgaagt taagcggttt cggggggggg    1020
   ggtgggttgt atcgcgtcgt acggaattcg ttttttggac gtattttgaa gaggttcgga    1080
   gatttttcgg gattgttgtt atttttagtt gtagttcggt tcggtttgcg agcgttgagg    1140
   ggtttagttc ggtgtagcgt tttcgatttt ggcgtttttt ttagttttaa ggggcgcgta    1200
   cgtagtttag gaatcgcgat cgtttagtta gtcgcggttt cgagtttcgc gattttagtc    1260
   gcgcggtcgg gtttttgagt gtatgcgtcg aggggggttt ttttgtagtt gcgttggggg    1320
   tcgaagcggg gtataggggg taggaggttg cgggtcgagg gcgcgttgtc gagagttaga    1380
   tttcgggttt taaatttcgt tttgtaaaga atgagttttt cgattttggt taagtcgttt    1440
   ttttttttcg gtttagtttt tttatttgta aaatgggggt ggggacgatg attttcgttt    1500
   ttgaggtttt gaggatttag gacgtggaag gtatagagaa gtttttgtat taattatttc    1560
   gggatttttt gatttttgtt tgtaattcgg aagagggtga ttttggaaga atcgtttttc    1620
   gtttttcgtt tttggttgtt ttttttatag gtagtattcg tattgttcgc gttcggtttt    1680
   atagtcgttt atcgttcggt tttttttggg ttttagggga cgtcgttttc gggttacggg    1740
   cgtggttgtc ggatcggagt tttatttcgg tttgcgattt tggattaggg tacgattttg    1800
   gattatagta atttggtcgt aggatatagc gtgtgatgtg tttggtatag agttcggtat    1860
   aagggaagag ttcggtggat aaggttaatt ggagaacgga ggtttgtttc gtttttattt    1920
   agcgtagcgg acggatttag cgttttttaa aaagaattag gggtcgggcg ttgtggttta    1980
   tgtttgtaat ttcggtattt tgggatatcg aggcgggagt attacgaggt taggagatcg    2040
   agattatttt ggttaaagtg gtgaaatttc gtttatatta aaatataaaa aattagtttg    2100
   gcgtggtggc gtacgtttgt agttttagtt atttaggagg ttatttagga ggttgaggtt    2160
   tgaatttagt aggcggaggt ggtagtgagt cgagatcgcg ttattgtatt ttaatttggt    2220
   aatagagtaa gatttcgttt taataaaaaa aaaataataa taaaaataaa taaataaaaa    2280
   aaagtaggga agtattaatt tggagtaatt gagtttaagg attgtttttt tttaataagt    2340
   aaaggttggt tataggtttt taggttttcg tttagattaa gttttgtttg ggtttggtgg    2400
   ggaggtgagg aagaacggtt tttaattttg ggattttaat tttttttgtt tgtttttggg    2460
   tgggatttta tgttttttgt taggatttag ttgtattttt agttatttat ttataaatt     2519
<210> 17
   <211> 2283
   <212> DNA
   <213> Artificial Sequence
<220>
   <223> chemically treated genomic DNA (Homo sapiens)
<400> 17
   aatgaagacg ttggagatcg ggtttttgtt cgtttttttt ttgcgtttcg ggatgaggta      60
   gagattgaat agtcggcgag taaattaacg gtatttagaa agttatgtcg gattcggcgt     120
   ttagcgttta agcgttaatt cgttgaaagt tttttagcga aattttaggg acgatttgga     180
   tttcgttgag aggaattgtt tttgagtgag atggttttag aggtttggag gagcggattg     240
   gtaagtatcg ggagggtagt gggagttttg tttttgtttg gtgttttgaa taaggttttt     300
   acggttattt attatgagat ttcggaggaa agagagaagg gtttcgttgt gggtaacgtg     360
   gtcgcgaatt ttggtttgga tttcggtagt tttttagttc gtaggtttcg ggtggtgttt     420
   ggagttagtc gaagattttt tgaggtgaat cgggagatcg gagagatgtt tgtgaacgat     480
   cgtttggatc gagaggagtt gtgtgggata ttgttttttt gtattgtaat tttggagttg     540
   gtagtggaga attcgttgga gttgtttagc gtggaagtgg tgatttagga tattaacgat     600
   aataattttg ttttttttat ttaggaaatg aaattggaga ttagcgaggt cgtggtttcg      660
   gggacgcgtt tttcgttcga gagcgcgtac gatttcgatg tgggaagtaa ttttttataa    720
   atttatgagt tgagtcgaaa tgaatatttt gcgtttcgcg tgtagacgcg ggaggatagt     780
   attaagtacg cggagttggt gttggagcgc gttttggatc gagaacggga gtttagtttt     840
   tagttagtgt tgacggcgtt ggacggaggg attttagttt ttttcgttag tttgtttatt     900
   tatattaagg tgttggacgc gaatgataat gcgtttgttt ttaattagtt tttgtatcgg     960
   gcgcgcgttt tggaggatgt atttttcggt acgcgcgtgg tataagtttt tgtaacggat    1020
   ttggatgaag gttttaacgg tgaaattatt tattttttcg gtagttataa tcgcgtcggc     1080
   gtgcggtaat tattcgtttt agattttgta atcgggatgt tgataattaa gggtcggttg     1140
   gatttcgagg atattaaatt ttatgagatt tatatttagg ttaaagataa gggcgttaat    1200
   ttcgaaggag tatattgtaa agtgttggtg gaggttgtgg atgtgaatga taacgtttcg    1260
   gagattatag ttattttcgt gtatagttta gtattcgagg atgttttttt ggggattgtt    1320
   atcgttttgt ttagtgtgat tgatttggat gttggcgaga acgggttggt gatttgcgaa    1380
   gttttatcgg gttttttttt tagttttatt ttttttttta agaattattt tattttgaaa    1440
   attagtgtag atttggatcg ggagattgtg ttagaatata attttagtat tatcgttcga    1500
   gacgtcggaa tttttttttt tttagttttt ataatagtgc gtgtttaagt gttcgatatt    1560
   aatgataatt ttttataatt tttttaattt ttttacgacg tttatattga agaaaataat    1620
   tttttcgggg ttttaatatt aaatttaagt gtttgggatt tcgacgtttc gtagaatgtt    1680
   cggttttttt tttttttttt ggagtaagga gttgaaatcg ggttagtggg tcgttatttt    1740
   ataataaatc gtgataatgg tatagtgtta tttttagtgt ttttagatta tgaggatcgg    1800
   cgggaatttg aattaatagt ttatattagc gatgggggta tttcggtttt agttattaat    1860
   attagcgtga atatatttgt tattgatcgt aatgataatg ttttttaggt tttatatttt    1920
   cggttaggtg ggagttcggt ggagatgttg tttcgaggta ttttagttgg ttatttagtg    1980
   ttacgggtgg taggttggga cgcggatgta gggtataatg tttggttttt ttatagtttt    2040
   ttgggatttt ttaattagag tttttttgtt atagggttgt atattggtta aattagtatt    2100
   gttcgtttag tttaagatat agatttattt aggtagattt ttacggtttt gattaaagat    2160
   aatggggagt tttcgttttt tattattgtt atttttattg tgttagtaat cgaggatttt    2220
   tttgaagttc gagtcgagtt tttttttggt tttgtttttc gggagtagaa aaaaaatttt    2280
   att                                                                  2283
<210> 18
   <211> 2283
   <212> DNA
   <213> Artificial Sequence
<220>
   <223> chemically treated genomic DNA (Homo sapiens)
<400> 18
   ggtgagattt tttttttgtt ttcggggggt agagttagag gggaattcgg ttcgggtttt      60
   aggagagttt tcggttattg atatagtgag ggtagtagtg gtggagagcg aaggtttttt   120
   attgtttttg attaagatcg tgagagtttg tttgggtgaa tttgtgtttt ggattggacg   180
   ggtagtattg atttgattag tgtgtagttt tatggtaaaa aggttttggt taggggattt   240
   taagagattg taggagagtt aggtattgtg ttttgtattc gcgttttagt ttattattcg   300
   tgatattagg tggttagttg aggtatttcg aggtagtatt tttatcgagt ttttatttgg   360
   tcgaggatat aggatttggg gggtattgtt attgcgatta gtgataaata tgtttacgtt   420
   gatgttggtg gttaggatcg gggtgttttt atcgttgata tgagttgtta atttaaattt   480
   tcgtcgattt ttatagttta ggggtattaa ggatgatatt atgttattgt tacgatttat   540
   tgtgaaatag cgatttatta gttcggtttt agttttttgt tttaagagaa agaaagaaag   600
   tcgagtattt tgcggggcgt cggggtttta gatatttagg tttagtattg gagtttcggg   660
   gaggttgttt tttttaatgt aaacgtcgta ggaagattga gaagattgtg gagggttgtt   720
   attgatgtcg gatatttgaa tacgtattat tgtaagggtt gagagggaag gggtttcggc   780
   gtttcgggcg gtgatgttga ggttgtattt tggtatagtt tttcgattta ggtttgtatt   840
   ggtttttaaa gtgaagtaat ttttgaggga agaagtaagg ttgaaaggga gattcggtgg   900
   aatttcgtag gttattagtt cgttttcgtt agtatttagg ttagttatat tgagtaaagc   960
   gatgatagtt tttagagggg tattttcggg tattgggttg tatacggagg tgattgtgat  1020
   tttcggggcg ttgttattta tatttataat ttttattaat attttgtaat gtgttttttc  1080
   gggattggcg tttttgtttt tggtttggat gtaaatttta tggagtttgg tgttttcgaa  1140
   gtttagtcga tttttgattg ttagtatttc ggttataagg tttaaggcga atagttgtcg  1200
   tacgtcggcg cggttgtggt tgtcgaagga gtaaataatt ttatcgttgg ggtttttatt  1260
   tagattcgtt gtaaggattt gtattacgcg cgtgtcggag ggtgtatttt ttaggacgcg  1320
   cgttcggtat aaggattggt tgaagatagg cgtattgtta ttcgcgttta gtattttgat  1380
   gtgaataggt aggttggcgg agagagttgg ggttttttcg tttaacgtcg ttagtattaa  1440
   ttggagatta ggttttcgtt ttcggtttag ggcgcgtttt aatattagtt tcgcgtattt  1500
   ggtgttgttt tttcgcgttt gtacgcgaag cgtaaagtat ttatttcggt ttagtttata  1560
   ggtttgtaaa gagttgtttt ttatatcggg atcgtgcgcg ttttcgagcg gaaagcgcgt  1620
   tttcggagtt acggtttcgt taatttttaa ttttattttt tgggtaggga aagtaggatt  1680
   gttgtcgttg atgttttgga ttattatttt tacgttgaat agttttagcg ggttttttat  1740
   tattaatttt agagttatag tgtaagaggg tagtgtttta tatagttttt ttcgatttag  1800
   acggtcgttt ataaatattt tttcggtttt tcggtttatt ttaaagaatt ttcggttagt  1860
   tttagatatt attcggaatt tgcgggttga gaggttatcg agatttaaat taaggttcgc  1920
   gattacgttg tttatagcga aatttttttt tttttttttc gggattttat agtgaatgat  1980
   cgtggaagtt ttgtttaagg tattaagtag aagtaaaatt tttattattt tttcggtgtt  2040
   tattagttcg tttttttagg tttttgggat tattttattt aaaagtagtt ttttttagcg   2100
   gggtttagat cgtttttgag atttcgttga gaaattttta gcgggttagc gtttgggcgt  2160
   tgggcgtcga gttcgatatg gttttttgga tgtcgttgat ttgttcgtcg gttgtttagt  2220
   ttttgtttta tttcggggcg tagaaagggg acgggtaggg gttcgatttt tagcgttttt  2280
   att                                                                2283
<210> 19
   <211> 2236
   <212> DNA
   <213> Artificial Sequence
<220>
   <223> chemically treated genomic DNA (Homo sapiens)
<400> 19
   taggtgttaa cgatcgcgtt agattggcgt tggaattttt gggttataag gttttagtgg    60
   gtggcgttat tatttttgcg gtttgtaaaa gcggagtggt tttgttaggc gggaagtttt   120
   tttttttttt ttagattcgc gataggtcgt aggtaagaat tagcgtaatt agggcgcgtt     180
   cgtatagatt tggaggcggt tgtatgttgt tatttgtttt agaaggcgtt cggtggtcgt     240
   ttcgtagttt tagagtttag agataagtag aggaaggaag ataggatatt tgggttagtt     300
   gttcgagtta agtcgttgta aacgttaata tcgttcgtag ttttttcgtt agtatttacg     360
   tttaaaagta atttaagttt gcgcgttagt gggaaatttg gcgtttatga ttcgttagcg     420
   cgggtcgaaa tttttttttg atgttttttt agttgttttt tttttttcgc gtggttagga     480
   ggggtaaata atatgtagtg gggcggggcg ttttggaagg ggtgtgttag ggagggaatg     540
   atgggggttt taggtaaagt ttcgaatttt tttacgtaac gggaggaata tagatacgtt     600
   ttagttaagc gtgcgtggga atcgcggaat taaggtattt agaggttaag ggtttgtgtt     660
   aagttttgta ttttgtttcg taggggaatt tttatatttc gcgattgaat tcgaaagatt     720
   tttttcggcg gatgaaagtc gttatttttt ggttggttga gttatagttt tcgtagcgcg     780
   tttaggagtg cgtcggagat tcggaaattc gtagagattt tttaagttag taggaatttg     840
   gaaatcgttg ttttttttaa acggttttaa atattcgcgt tttttttttt atttaattta     900
   attttaattt gtttttttta taggtttaat ttttattttt ttaaaatttt aaattagtaa     960
   gttaaatcga agttttagaa taaggtttaa ttaatgattt taatttaata ttaagttcga    1020
   atgtttattt ttcggatttg ataaattaga tgtagagcga cgaatggaga ttattttttc    1080
   gggaggcgga atttagttgg gttttagttt gttttgggag aggagtacgt tttttttgag    1140
   ttgtaggcgg gttcggtttt gacgaaacgt tgcgagaatg ttagatagtg tagtttttat    1200
   cgtttgttcg gggtttttgt tgttattaga taagtatttt tatttattta ttttttttta     1260
   ttaattatcg ttagattttt ttcgcgtttt tcgatttcgt tgtaattttt ttttttgggc    1320
   gatgtttgga tagtattagt agtagttgtt cgagggaggg gggtagtttt cgttcgggga    1380
   taggttttaa aaggttgttt atttgttaga gttttacgtt ttttaaatga atttaaatta    1440
   agggtaagtt attacgtatt atgtgatatt cggtagtttt agattagaat aaggttttgt    1500
   attgaacgtg ataattgaat ttttaatgtt aagtgttatt ttttttttag gtaaatgttt    1560
   cgtcgttgtg atatttaagg ttggtttgtg ggcgttgttt tttgttttgt tttattttgt    1620
   tttgaagttt tattttaagt taatattaat tttaatacgg aatagttcga tttttttttt    1680
   ttaggttttt ttattgtttc gagagaggat tataagtatt ttttattttt tttagtatag    1740
   tttttttttt ttttttttgt tatagtatcg gtgttaggta attttaggta tttttatatt     1800
   gtatttatga tagatttgat gtttatcgga gatatgaaaa taattagaaa gttgttatgt    1860
   aaatttaata tgatatttat ttattaggta gagtaatata ataaggtttt tttatttatt    1920
   tttgagtttt atgtttatcg tagagaggaa aataatagcg attggtagtt ttgtttagag    1980
   attgtataaa agaggttagg aagttaggtt tggagatgta ttggagagta ttgtgtcgtt    2040
   ttttaattag taaaattttt attggatttg tggaatttta aatttagaag tatttatttt    2100
   atgtagtagt tttatttaga gataggaggt tcgttttttg ttttatgtta gttaatggaa    2160
   tagatgggat aagaattttg gatttttggt ttttaggtaa tgttttttgg gatagtggaa    2220
   gtagaaaatt aatttt                                                     2236
<210> 20
   <211> 2236
   <212> DNA
   <213> Artificial Sequence
<220>
   <223> chemically treated genomic DNA (Homo sapiens)
<400> 20
   gaaattgatt ttttattttt attgttttag aaagtattgt ttaagagtta aaaatttaag      60
   gtttttgttt tatttatttt attaattaat atgaagtaaa aaacgagttt tttgtttttg     120
   ggtgaaattg ttatatggaa tgggtgtttt tgggtttgaa gttttataga tttagtaggg     180
   attttgttga ttggaggacg gtatagtatt ttttagtgta tttttaagtt tgattttttg    240
   atttttttta tataattttt gggtaaaatt attagtcgtt gttatttttt tttttgcggt    300
   aaatatggag tttaagaatg gatagaaaga ttttattgta ttgttttatt tggtaaataa     360
   atattatgtt gaatttgtat ggtagttttt tgattatttt tatgttttcg ataagtatta     420
   agtttgttat aggtatagta taaaaatgtt tgagattgtt taatatcgat attgtggtag     480
   gaagagagga gaagagttgt attggggaaa atgaggagta tttgtaattt tttttcgggg     540
   tagtgagagg gtttgggaag aaaagatcga gttgtttcgt gttggggtta atgttggttt     600
   gaaatgaaat tttagggtaa aatgaagtag agtaaagagt agcgtttata aattaatttt     660
   aaatattata gcggcggaat atttatttga aagaaaagtg atatttggta ttaaaagttt     720
   aattattacg tttagtgtag agttttgttt tagtttggag ttgtcgggtg ttatatggtg     780
   cgtgataatt tgtttttgat ttgggtttat ttgaagagcg tagaatttta ataaataaat     840
   agttttttgg gatttgtttt cggacgagga ttgttttttt ttttcgggta attattattg     900
   atgttgttta ggtatcgttt aaggggaaag gttgtagcgg ggtcggaagg cgcgggagga     960
   gtttggcggt gattgatggg aagggatgaa tgaataaaag tatttgtttg atggtagtag    1020
   agatttcgag taaacggtgg aggttatatt gtttggtatt ttcgtagcgt ttcgttagag    1080
   tcggattcgt ttgtagttta agggaggcgt gttttttttt tagagtaggt tggaatttag    1140
   ttgggtttcg tttttcggga aggtggtttt tattcgtcgt tttgtatttg gtttgttaga    1200
   ttcgagaggt aaatattcgg gtttggtgtt gaattaaaat tattgattga attttatttt    1260
   ggggtttcgg tttggtttat tagtttggga ttttaaaaaa ataaaaatta agtttataga    1320
   gagggtaaat taaaattagg ttgggtaaag gaaggagcgc gagtgtttga agtcgtttgg    1380
   agggaatagc ggtttttaag tttttgttga tttgagaagt ttttgcgggt tttcgaattt    1440
   tcggcgtatt tttgggcgcg ttgcgggagt tgtagtttag ttagttaggg agtagcggtt    1500
   tttattcgtc gggaggagtt tttcgagttt aatcgcgggg tatagaggtt tttttgcggg    1560
   gtaaaatgta gagtttgata taagtttttg gtttttaggt gttttaattt cgcggttttt   1620
   acgtacgttt aattaagacg tgtttgtatt tttttcgtta cgtgaaagag ttcggagttt    1680
   tgtttgggat ttttattatt ttttttttgg tatatttttt ttagaacgtt tcgttttatt    1740
   gtatattatt tatttttttt ggttacgcgg gggaagaaaa atagttgaga gggtattagg   1800
   aaggagtttc gattcgcgtt ggcgagttat gagcgttaag ttttttattg gcgcgtaaat    1860
   ttgagttatt tttgagcgtg gatattggcg aagaggttgc gggcggtatt agcgtttgta    1920
   gcgatttggt tcgggtagtt gatttaagtg ttttgttttt ttttttttgt ttgtttttag    1980
   gttttgaaat tgcggagcgg ttatcggacg ttttttggag taggtagtag tatgtagtcg    2040
   tttttaagtt tgtgcggacg cgttttggtt gcgttggttt ttgtttgcgg tttgtcgcgg    2100
   atttggggag aggagagagg tttttcgttt gatagggtta tttcgttttt gtaaatcgta    2160
   gagataatga cgttatttat taagatttta tggtttaagg gttttaacgt tagtttggcg    2220
   cggtcgttgg tatttg                                                    2236
<210> 21
   <211> 2204
   <212> DNA
   <213> Artificial Sequence
<220>
   <223> chemically treated genomic DNA (Homo sapiens)
<400> 21
   tttttttgta aatcggttta agtttcggga ggtgggattg ggggaggagg gggtttattt     60
   tggggttagg ttagggttgg gtggaggagg cggttttcga gagagtgagt cgggaaagaa    120
   ggggagtggg taaagggtga gatgtggggt ttcggttttc gtttcgttgg ttgttagttt    180
   tttttgcgtt ttttagttcg cgtgcgtgat acgagatagt gtagttttgg tatggggttt    240
   ggtatttggc ggtcgcgaga taattagggt tcgtgcgatg ggaaacgtgt cgttggtagt    300
   tcgttatttt tttatcgtga ttttagtttg ttaaataggg ttcgtgatgt ttagtttatt    360
   agggaggagt cgcggttttg agatttcgtt tagttcgaag tagcggagat tggttaggag    420
   ttcggttgtt agatttcgga gcgtacgttg cgggcggggg ttcggtttac gggggagggt    480
   taggggacgc gggatttatt tttgaaggag tcggggagtc gttgggtttc ggagtttcgt    540
   gtgtgtcgtt tttcggttat gttgttcgag acgtagtcgt attttttcgt tttcgttgtt    600
   aaattcggag tcgtcgggaa tgttttgagg agttagaggt ataagattta gattgtcgtt    660
   tcgtttttaa tcgaggtttc gttttcgtga gagttggtta attaacgttt aggatcgagg    720
   aggatttatt ttttaatttt cgtttttttg tttttttacg gcgcgattgt ttcgtagtta    780
   tttttcgtta gttttgtggg ttgaggggcg tggtttgagg ggcgtggttt gaggggcgag    840
   agtttttaag gttgtgggta tgaggtcggg gttttgtatc ggaagggagg gcggaagtta    900
   cgggacgtgg agtagatggt gaggatagtg gggaggtagt agtagaggat gattcgaggt    960
   aaaagtgtaa gtgaagaaaa gtcgttttgt cgggtagagg tacgggatta aggcgttttt   1020
   tttgggttga ggagttagag ggaagaaaat ggcgttttgt tatgagggcg cgaggttcga   1080
   gttgagggga gaagattgag ggatatagaa gtggtcgaag tgagggcgtt gaggttagtt   1140
   tgagttgaga gggtcggtag attacgtcgt agtcgaagtg aacgaattcg agtgaggtgt   1200
   gttaagaaaa ttaaaatgta taaatttgga aaaggagagg aggtcgggcg cggtggttta   1260
   cgtttgtaat tttagtacgt tggaaggtcg aggcgggtag attatttgag gttagaagtt   1320
   cgagattagt ttggttaata tggtgaaatt tcgtttttgt taaaaatata aaaattagtc   1380
   gggcgtgtgg cgcgcgtttg taattttagt tatttgggag gttgaggtag gagaatcgtt   1440
   tgaatttggg aggtagaggt tgtagtgagt cgagatggtt tgggtgatag agcgagattt   1500
   cgttttaaaa aaataaaata aaaaataaaa aaataggaga ggagatttta ttttttgtaa   1560
   ggggttatag tttgtaaggt ggttatttcg taggttggag aatggttagg attagagata   1620
   ggtatttcgt tggaggaggg gttggagtag cggttttatg ttgaatagtt tgggtagata   1680
   tgtatattta gtaggtgata ggaggagtta tgaatattta tgagggtggt tttgacgtat   1740
   gtttattgag taaatatgta tgtaatatat aatttatgtt tattttggag tgaagagtta   1800
   ttaattaaat gttaaatgtt tggtttgtat ggtgacgttt ttatttttat aaaaagtaaa   1860
   aaaaaaatag ttaggtgggg tggtgtgtgt ttgcggtttt aattagttag gaggttgggg   1920
   taggaggatt atttgagttt aagagttgga ggttgtagtg atgtatgatt agattattgt   1980
   attttagttt gatggatttt aaaaaataat aataatatat gtattatagt tagattttat   2040
   gcgttaaaag gtttttttag gataggaagg tgtgtaagta tgtagtttgt gtaaattagt   2100
   tagagttagt ttatggtcgg tagttttttt atgaggagaa agttattgaa attagttttt   2160
   tgtttaatta aagttgtaat gatggttggt ggaatagggg tttt                     2204
<210> 22
   <211> 2204
   <212> DNA
   <213> Artificial Sequence
<220>
   <223> chemically treated genomic DNA (Homo sapiens)
<400> 22
   ggaattttta ttttattagt tattattata gttttaattg gataagaggt tgattttagt      60
   aatttttttt ttataggaaa attatcgatt atggattggt tttggttagt ttatatagat      120
   tgtatatttg tatatttttt tgttttgaaa agatttttgg acgtatgggg tttaattgta      180
   atatatgtat tgttattatt ttttagagtt tattaggttg gagtgtagtg atttgattat      240
   atattattgt agtttttaat ttttgggttt aagtgatttt tttgttttag ttttttgatt     300
   agttgggatc gtaagtatat attattttat ttggttattt tttttttatt ttttgtagag      360
   atggggacgt tattatgtag gttaggtatt taatatttaa ttggtaattt tttattttaa      420
   aatgaatatg ggttgtatgt tatatgtatg tttgtttaat gggtatgcgt taggattatt      480
   tttatgaata tttatagttt tttttgttat ttgttgaata tgtatgttta tttaagttgt      540
   ttagtataaa gtcgttattt taattttttt tttaacgaaa tgtttgtttt tggttttggt      600
   tattttttag tttgcggaat ggttattttg taggttgtaa ttttttataa gaaataaagt       660
   tttttttttt gtttttttgt tttttgtttt gtttttttga gacggagttt cgttttgtta       720
   tttaggttat ttcggtttat tgtaattttt gttttttaag tttaagcgat ttttttgttt       780
   tagtttttta agtagttggg attataggcg cgcgttatac gttcggttaa tttttgtatt     840
   tttagtagag acggggtttt attatgttgg ttaagttggt ttcgaatttt taattttaga     900
   tgatttattc gtttcggttt tttaacgtgt tgggattata ggcgtgagtt atcgcgttcg     960
   gttttttttt tttttttaaa tttatatatt ttgatttttt taatatattt tattcgagtt       1020
   cgtttatttc ggttgcggcg tgatttgtcg gtttttttag tttaggttga ttttagcgtt     1080
   tttatttcgg ttatttttgt gttttttagt tttttttttt taattcggat ttcgcgtttt       1140
   tatgataggg cgttattttt ttttttttaa ttttttagtt tagaggaggc gttttggttt      1200
   cgtatttttg ttcggtaagg cgattttttt ttatttgtat ttttgtttcg ggttattttt      1260
   tgttgttatt tttttattat ttttattatt tgttttacgt ttcgtggttt tcgttttttt      1320
   tttcggtgta gggtttcggt tttatattta tagttttggg ggttttcgtt ttttaggtta     1380
   cgttttttag gttacgtttt ttagtttata gaattagcgg gaagtgattg cgaagtagtc     1440
   gcgtcgtgga gggataggag ggcggggatt gggaggtggg tttttttcga ttttgggcgt      1500
   tgattggtta atttttacga gggcgggatt tcgattgggg gcggggcggt aatttgggtt     1560
   ttgtgttttt ggttttttag ggtattttcg gcggtttcgg gtttggtaac gaggacgggg     1620
   gagtgcgatt gcgtttcggg tagtatggtc gagaagcggt atatacggga tttcgaagtt     1680
   tagcggtttt tcgatttttt taagggtgag tttcgcgttt tttgattttt tttcgtggat      1740
   cgagttttcg ttcgtagcgt gcgtttcgag gtttgatagt cgggtttttg gttagttttc      1800
   gttgtttcgg gttgggcgag attttaaagt cgcggttttt ttttagtaaa ttgagtatta     1860
   cgaattttgt ttggtagatt gaggttacga tggaggggtg gcgggttgtt aacggtacgt      1920
   tttttatcgt acgggttttg gttatttcgc ggtcgttaga tgttaagttt tatgttaggg      1980
   ttgtattatt tcgtgttacg tacgcggatt aggaaacgta gagggaattg gtagttagcg     2040
   aggcggggat cgggatttta tattttattt tttgtttatt tttttttttt ttcgatttat      2100
   tttttcgaag gtcgtttttt ttatttagtt ttggtttggt tttagggtga attttttttt      2160
   tttttaattt tatttttcga aatttagatc gatttgtaga agga                      2204
<210> 23
   <211> 2137
   <212> DNA
   <213> Artificial Sequence
<220>
   <223> chemically treated genomic DNA (Homo sapiens)
<400> 23
   ttttattagt tagtatttaa ttatttaaaa gtgtggttta tgtttggaat tgggttataa      60
   agatggtaat gtgttaaatt agatatttgt tttttgtata tttttataaa ttatattagt     120
   ttattttata attttgtttt tgttagttat aaagaagaaa gtttaatatt ttagacgttt     180
   atattttatt aaattgtggg tgaagagtta gtgagcggta aaaggtggtc gttttgagtt     240
   gtggtgatgg aatttttagg gaatagttag gagtttggaa agttattttt gagttggttt     300
   atttaagtat tcgggataga agtggttagt agaaattttt tatggaattt taaaatatta     360
   ttaggatagt agttttacga tatttttaaa atttggagtt taaaaaataa tgaagtataa     420
   tttatttaga atttaagtat taagggaaaa cgttaagttt tgttttattt taaagatttt     480
   tttttgagga aaatatgtta ttgtttttta gatgtgaatg aaatgatatt ttttttttcg     540
   atatagtttt tttgtttacg attttatatt gttttttttt aatttttttt attattgaaa     600
   tattatatgt aagatttgtt ggtatgtttt ttgatttttt atgtttaagg ggaaaaatgt     660
   ttgtagttga tgtttgagtt ttaggagttc ggtgaagaaa aaaaaaaaaa gtaacgtaag     720
   gaaaattgtt ttaatggtat atttttttta aagtttgaag ttttataggt aaaaggaggg     780
   ataaatattt ttttttgaaa gtaaatagtt aaggtttttt tttatttaat tatttttgaa     840
   ttttttgtaa ttttttcgag gtttaaattt agggtataat taattttaga ttttatttta     900
   aatttaattc gttttttatt tgtaatttgt ttgtaggtgg gttttttttt tttaaattat     960
   aaatttgaat ttgcgttaat tttttataat ataaggtatt taaagttttt tggagttatt    1020
   gatttggtga cgttgtagtg tacgtttgta tttgaaagta agtaagagaa tagaaagcga    1080
   gtaagagaat cgtaattttt attattttgg cgaatttttg tcgggattta tatattaaat    1140
   agtatttgta tatagacgtt ttaagtcgtt ttttttagag tcgttattta gatttgtaag    1200
   gggcggggga ttagtggaaa tttaaataga aatttttatt attataaagg cgagttttta    1260
   ttgattatgg taattgtttt gtttaggttg tacgggtatg gttaattttg tttgaaagtt    1320
   cgtttttttt ttgcgttttt ttgtcgagtt ttttttaggt tttggttttt ttcgacggtg    1380
   gtaagggatg ttttttacgt tttttattat ttttgttcgc gtttgcgttt tttgttcgag    1440
   gttttaggtc gcggcggcgg cgcggtgggt ttattagcgg gcgcgtcggg ttcgcgtagc    1500
   gttgtttgta gtcgcgtcgt cgtatttatt attattatat tagcgcgggg aagtttacgt    1560
   cgagtttttt cggtttcggc gcgttttagt tagcgcgtta gtaaatagaa tatgttggat    1620
   tgtttagaga cggttttggg tgtttggggg tagaaggggt gtttttaggt tcggttttga    1680
   ttcgtatttt tgaagttggt tttgggagat aatgtatttt cgttagttag gtcgcgggaa    1740
   aagtgaaaaa agggttgggg gttatgtggg ggtggggagg tgagttttag cgttagtagc    1800
   gtttggtagt ttttttttag tgtagagaag cggtttgtat taaggcgtat tagagtggtt    1860
   attttgtttt tcggaatatt tacgggtatt tgtaaatttt atagttggtt tagttaaatt    1920
   tttagtaaat attggtaagg gtcggttttg gttgcgttta gagggttttt ttaattggtt    1980
   gaatttaggg agttgtaatt ttttaggtat tttgtaatgt aattattttg agtgtcgtat    2040
   tataataaaa tattggggat tttttgaaaa gtttttataa aattaatagg tgtgtgtttc    2100
   gtttataaaa cgtcgaattt attttttatg aatttgg                             2137
<210> 24
   <211> 2137
   <212> DNA
   <213> Artificial Sequence
<220>
   <223> chemically treated genomic DNA (Homo sapiens)
<400> 24
   ttaggtttat agaaggtgaa ttcgacgttt tgtgagcgaa gtatatattt attggttttg      60
   tgaaggtttt ttaagggatt tttaatgttt tattataatg cgatatttag ggtggttgta     120
   ttgtaaagta tttggaaggt tgtagttttt tgaatttagt tagttaaaag agttttttga     180
   acgtaattag ggtcggtttt tgttagtgtt tgttggaaat ttggttgggt taattgtaga     240
   gtttgtaaat gttcgtgaat atttcgagga gtagggtgat tattttgata cgttttgata     300
   tagatcgttt ttttgtattg gagggagatt gttaggcgtt gttggcgttg gagtttattt     360
   ttttattttt atatggtttt tagttttttt tttatttttt tcgcggtttg gttggcggag     420
   atgtattgtt ttttaaagtt agttttagaa atgcggatta aagtcgggtt tgggagtatt     480
   ttttttgttt ttagatattt agagtcgttt ttgagtaatt tagtatgttt tgtttgttgg     540
   cgcgttgatt ggggcgcgtc ggggtcggga gaattcggcg tggatttttt cgcgttgatg     600
   taatgatgat ggatgcggcg acgcggttgt agatagcgtt gcgcgggttc ggcgcgttcg     660
   ttaatgaatt tatcgcgtcg tcgtcgcggt ttgggatttc gggtagggga cgtaggcgcg     720
   ggtaaaagtg gtgaaaggcg tgaaaaatat tttttgttat cgtcggagga aattagaatt     780
   tgaagggaat tcgataggaa agcgtagaga aagagcgagt ttttaaataa aattggttat     840
   attcgtataa tttgagtaaa ataattatta taattaatgg ggattcgttt ttgtaatggt     900
   gaaagttttt gtttgggttt ttattggttt ttcgtttttt gtaggtttaa gtgacgattt     960
   taaagaggac gatttgaggc gtttgtgtgt aagtgttgtt taatatgtag gtttcggtaa    1020
   gaattcgtta aggtggtaaa ggttgcggtt tttttgttcg ttttttgttt ttttgtttat    1080
   ttttaaatgt agacgtgtat tatagcgtta ttaggttagt ggttttagga agttttaaat    1140
   gttttgtgtt gtaagaggtt gacgtaggtt tagatttata atttagaaag aaaagattta    1200
   tttgtaggta ggttgtaaat aggagacgga ttagatttaa aatagggttt ggaattaatt    1260
   gtgttttggg tttaggtttc ggggaaatta taaaaggttt agggatagtt gagtgggaaa    1320
   aagttttgat tgtttgtttt taggaaggaa tgtttatttt tttttttatt tatgaaattt    1380
   taagttttag gaaggatata ttattagagt aatttttttt gcgttgtttt tttttttttt    1440
   ttttatcgag tttttgggat ttaaatatta gttataaata tttttttttt taagtatgaa    1500
   aagttaaggg gtatgttagt aaattttgta tgtaatgttt tagtggtgaa agggattgaa     1560
   ggagaatagt gtggagtcgt aagtaaggaa attatgtcgg gggaaaaagt attattttat    1620
   ttatatttga aaaatagtgg tatgtttttt ttagggaaaa gtttttgaag tgaagtagaa    1680
   tttaacgttt ttttttgatg tttaagtttt aaataaatta tattttatta ttttttaaat    1740
   tttaggtttt aaaagtgtcg tggagttatt gttttagtgg tgttttgaga ttttatgagg    1800
   aatttttatt gattattttt atttcgaatg tttgaatgga ttagtttagg agtgattttt    1860
   taagtttttg attatttttt gaaaatttta ttattataat ttaaagcggt tattttttgt    1920
   cgtttattga ttttttattt atagtttgat gaggtgtggg cgtttagaat gttaaatttt    1980
   tttttttata attgataggg ataaagttgt aaaataagtt gatataattt ataggagtgt    2040
   ataggaagta gatgtttgat ttggtatatt gttattttta tagtttagtt ttaagtataa    2100
   attatatttt taaataatta ggtattaatt aatgaga                             2137
<210> 25
   <211> 2461
   <212> DNA
   <213> Artificial Sequence
<220>
   <223> chemically treated genomic DNA (Homo sapiens)
<400> 25
   tttttttttt tttttttttt tttttttttt tttttttttt tttttttttt tttttttttt      60
   tttttttttt tttttttttt tttcgttttt tttttttttt ttttttttgt tttttatttt     120
   tttagtttga aggttgtatt ttttttattt ttttaagtaa agttgggagt tttgtagtat     180
   tttaggagtg aggataatta tgtttaagaa attttggttt tagtttagtt ttagattaag     240
   aggaaaatat tttaaaattt taaagtaaat tttaaaagtg taatttatat tagagcgatt     300
   gatttggtat ttatgtagat tgaagtttga gaagtaattt aagtggtttt tatttatttt     360
   tttggaagag tttttagaat gtatttttat tggtttgaaa aacgtttgaa aaagagtttt     420
   gatgttaata gtaatggaaa taggagggtt ggtggtttac gattagttta gtggtatttt     480
   ttattttatt tgtatttagg taaagagatt gtggtttgta tgtgattaaa taatttgttg     540
   aagcgttttg ggtttagggt atttaatttt tgtgaagtag gattattagt tgtttgtttt     600
   gtttttgtgt tattttttag aatatattaa agtaggggta atttttgagt ttagattttt     660
   gttatgtgaa gattttttga aaattttgat aaatataggt attgcgtaag tggttagtta     720
   attaaagagg ggaggtggag ttgttttttg tgtatttaat aagtatttat tattttattt     780
   gagtatgaaa agaggttatt gttattattt ttaagaagga aagtaagtag gatagtttat     840
   atttttagaa taatttttta ttaaatggaa taatttcggt gaaaagtggg agtgaggaag     900
   aaagaaaaaa aaaattttta attataatgt ttgggaataa gaaaggaaga agaaatttac     960
   gttaaagtcg attttttttt gtagttgtaa aataaatttt taagattttt tttgttgaaa    1020
   ttttggagag gaaaattgga gtggcgggtg ggttttgttt gtagtttaat tttttttcgc    1080
   ggcgcgggcg cggttgggtt tagtatttcg gaaagcgttt ttcgcggcgt ttcgggatta    1140
   cgtatgtttt ttggggttcg tcgttttggt cgtgtaagtg ttatcgtaat tggtgagagt    1200
   cgttggtaat ttattcggag ttgataatcg cggagagacg tagatattta ttgattttta    1260
   ggaagttgag cgtggtggat ggaattttac gatttttttt tttttaagga cggaaatttt    1320
   atttaagtag ttttagagga aacggataaa ggtatttgaa agggagcgag cggttttaaa    1380
   tcgtataatt gagcggttgg gggagttatg cgttagtgtt ttagtgatcg cgggatacgg    1440
   agaggggaag tttgcgttgt atataaggat ttagggattt cgagtttggt ttgagaattt    1500
   ttggacgtcg agtgtttgtt ttacgggttg tattttttaa ttttgtttta aagtagtcgt    1560
   tgagtttaat ttttgcgttt agggcgttcg ttgcgcgtta ggacgcgttt agtatttagt    1620
   ttttgggttt tttttttagt agttgttttg aaagttttta cgtacgtttc gtaggttagt    1680
   ttggtaataa aattggggta aatcgtgtta ttttaggttt tgttttttag aatatgattt    1740
   agaggtattt gcgtatgtag atggtcgatg tagttacgat agttattatg aataaggtag    1800
   taggcgggga taagttagta gaatttttta gtttggtttc ggattttttg gaggcggtta    1860
   atacgagtgg taacgcgtcg ttgtagtttt cggatttgtg gtgggagttg gggttggagt    1920
   tgtcggacgg cgcgtcgtta ggatattttt cgggtagcgg cggggtagag agcgcggata    1980
   tagaggttcg ggtgcggatt tttattagcg tggtgtattg ggtggtgtgc gttttggggt    2040
   tggcgggtaa tttgttggtt ttttatttga tgaagagtat gtagggttgg cgtaagtttt    2100
   ttattaattt tttcgttatt aatttggcgt tgacggattt ttagtttgtg tttattttgt    2160
   ttttttgggc ggtggagaac gtttttgatt ttaaatggtt tttcggtaag gttatgtgta    2220
   agatcgtgtt tatggtgacg tttatgaata tgtacgttag cgtgtttttt tttattgtta    2280
   tgagtgtgac gcgttattat tcggtggttt cggttttgaa gagttatcgg attcgaggat    2340
   acggtcgggg cgattgttgc ggtcggagtt tgggggatag ttgttgtttt tcggttaagg    2400
   cgttgtgtgt gtggatttgg gttttggtcg cgttggtttc gttgtttagt gttatttttt    2460
   t                                                                      2461
<210> 26
   <211> 2461
   <212> DNA
   <213> Artificial Sequence
<220>
   <223> chemically treated genomic DNA (Homo sapiens)
<400> 26
   gagaaaatgg tattgggtag cgaggttagc gcggttaaag tttagattta tatatatagc      60
   gttttggtcg agaagtagta gttgtttttt aggtttcggt cgtagtagtc gtttcggtcg     120
   tgttttcggg ttcggtggtt ttttagagtc gaggttatcg aatggtagcg cgttatattt     180
   atggtagtga ggaagaatac gttggcgtat atgtttatgg acgttattat ggatacgatt     240
   ttatatatgg ttttgtcgaa gggttatttg aagttaagag cgttttttat cgtttagaag     300
   ggtagggtga gtataaattg aaagttcgtt agcgttaggt tggtgacgaa gaggttgata     360
   gaggatttgc gttagttttg tatgtttttt attaggtaga gaattagtag gttgttcgtt     420
   aattttaggg cgtatattat ttagtatatt acgttgatga gaattcgtat tcgggttttt     480
   gtgttcgcgt tttttgtttc gtcgttgttc gggggatgtt ttggcggcgc gtcgttcggt     540
   aattttagtt ttagttttta ttataagttc ggaagttgta gcgacgcgtt attattcgtg     600
   ttggtcgttt ttagaaggtt cgggattaga ttgaagagtt ttgttagttt gttttcgttt     660
   gttgttttat ttatggtggt tatcgtggtt gtatcggtta tttgtatgcg taggtatttt     720
   taggttatgt tttgggggat aagatttaag ataatacggt ttattttagt tttgttgtta     780
   ggttagtttg cgggacgtgc gtgggagttt ttaaagtagt tattgaagag agagtttagg     840
   aattgggtat taagcgcgtt ttggcgcgta gcgaacgttt tggacgtagg agttgagttt     900
   agcggttgtt ttggagtaga gttgaggagt gtagttcgta aggtaagtat tcggcgttta     960
   agggttttta ggttaagttc ggagttttta ggtttttatg tataacgtag attttttttt    1020
   ttcgtgtttc gcggttattg gggtattggc gtataatttt tttagtcgtt taattgtgcg    1080
   atttggggtc gttcgttttt ttttaaatat ttttattcgt tttttttggg attgtttgga    1140
   tgaggttttc gtttttggag agaaagagat cgtagagttt tatttattac gtttagtttt    1200
   ttggaggtta gtgggtgttt gcgttttttc gcggttgtta atttcgggtg ggttgttagc   1260
   ggtttttatt agttacggtg gtatttgtac ggttaaggcg gcgggtttta aggagtatgc    1320
   gtaatttcgg ggcgtcgcga ggggcgtttt tcgaggtgtt gaatttagtc gcgttcgcgt    1380
   cgcgagggag agttgagttg taggtaaagt ttattcgtta ttttagtttt tttttttaga    1440
   gttttagtag gaagggtttt aagagtttat tttatagttg taggagaaag tcggttttga    1500
   cgtgagtttt tttttttttt tttattttta aatattatga ttagaagttt tttttttttt    1560
   ttttttttat ttttattttt tatcggaatt attttatttg gtgaggaatt gttttaaaaa    1620
   tatgagttat tttgtttatt tttttttttg aaagtaataa tagtggtttt tttttatgtt    1680
   taaatgagat aatgggtatt tattggatat ataaaggata gttttatttt ttttttttaa   1740
   ttagttgatt atttacgtag tatttgtgtt tgttaggatt tttaaagggt ttttatatgg    1800
   tagaagtttg ggtttaaggg ttgtttttat tttaatgtat tttgagaggt gatataaaaa    1860
   tagagtaaat aattgatggt tttattttat agaaattaaa tgttttaagt ttaaagcgtt    1920
   ttaataaatt gtttgattat atataggtta tagttttttt atttagatgt aaatagaata    1980
   gaggatatta ttgggttgat cgtgaattat tagttttttt atttttatta ttgttagtat    2040
   taggattttt ttttaggcgt tttttagatt aatggaagta tattttaagg atttttttaa    2100
   gggaatgagt ggaaattatt tgaattattt tttaagtttt agtttatata aatgttaggt    2160
   taatcgtttt ggtgtaaatt atatttttag gatttatttt aaaattttga aatgtttttt     2220
   ttttggtttg ggattaaatt gaaattaaag ttttttaagt ataattgttt ttatttttgg   2280
   agtattatag aatttttagt tttatttaag gagataaaga ggatgtaatt tttaagttga    2340
   ggagataaga aatagaaaaa gaaagaaaga gaaaaacgaa aaagaaagaa agaaagaaag    2400
   aaagaaagaa agaaagaaag aaagaaaaga aaagaaagag aaaaaggaaa aaagaaaaaa    2460
   a                                                                    2461
<210> 27
   <211> 1920
   <212> DNA
   <213> Artificial Sequence
<220>
   <223> chemically treated genomic DNA (Homo sapiens)
<400> 27
   ttaggttgtc gtagatatag tttttgtttt ttcgaaaaat acgttttagg cgtcgggatt      60
   ttagatattt gggaaataga gtgtacgtag ttgttgagag gtttcgcgtt tggttttttt     120
   tattattgag gcgtagaggt gttgtggata gtttagattt atacggcgtt cgaggtgaaa     180
   tagaattttt agttttttta tgaggttatt ggtattttcg gttgttttta gagtttttcg     240
   atttagagtt gaatgtaaag taagcgttcg aaatgtagaa gtagtcgggg tcgtttacgg     300
   tatttgtttc gttcggggcg agagaagacg ttaggttgag gttttagcga ttttaggtat     360
   tagtttcgaa ggagggcggg gagatcgtaa aggggaagtg ttcggagggt taacggtttt     420
   cgcgtatttt gcgttttttt gaagcgcgtc gttttttcgc gtcggggatt gggatttgtt     480
   tttggggaat tcgtttagaa gacggcggcg gattggggtc gggtattttt tagggttgtt     540
   aggttttttt tagttttgta tttgtcgcgt cgttttattt cgttaggaag ttttagagat     600
   ttcggggatg gggtgggagc gtttttttat cgcgggttta aaaagaagga aggacgtttt     660
   taggggtcgt agaaggagga ttagttttaa gttataattt ttttcggatt taaggtaggt     720
   cggttggggt ttcgcgttta tacggttttt ggcgggggtt cgcgcgtttc gggagtttcg     780
   cggttcgggg aggaaagagg agataagaga taggcgagga ttacggggtt gatttagtcg     840
   gggtagggat tatcgtggaa aaattttggc gaggtggggg gacgcggaaa gagagcggtt     900
   cgcgttttgt attttgcgtc gggtatttcg cgttagtgtt tcgtttttag tgtttcgcgt     960
   ttcgcgtttc gcgttttgtt tttatttcgg gttagttgta tcgcgttcgc gtcgtaggaa    1020
   tcgtggagtt ggaaagtggg ggcgtcgcgg ttggggggtt gttttagttg cgtttcggtt    1080
   agcgatcggc gggtcgggtt taaatttagt taggttgggt aggcggtggt cgcgcgattg    1140
   gggatcgggc gtttcgtttt tttcgttttt tttttttttt tttttttttt ttttagtttt    1200
   ttggtttttt ttagttttta tcggatttgt tcgttcgttg tttttttttt tttttcgttt    1260
   tttatattat tttttatttt ttcgttttgt tttcgttttt tttttttttt ttgttttttg    1320
   tttttttttt tttttttttt ttttttaggg gcggagtttt tttttttttt tttagataat    1380
   gttgtggttg cgtttttttt ttcgttagtt cgtttaggtt ttcgtcgtta gcgatttttt    1440
   cgggttgggg gtggggaggt ggggggggag tgtagggttg gggaggatga gttggttttt    1500
   tttatttttt tgttgttgtt ttttttaaga gggatggaga tttggtttaa gtttttcggt    1560
   ttattcggag ttgtgatagt tatttttagg gaatagttac gttgttttat taagtttatt    1620
   tttagcggtt tggatttttt aggtagaggt tgtgggattt tgtttttttt aatattttag    1680
   tttattttta aaagggtttg agtcggatag gggttaaata ggttttttcg atttggcggg    1740
   tcggttagac gtgatagtaa tgttaaggag gttaagtttt tttgtttatt ttttattttt    1800
   ttttttttta tttttggatt ttttggcgtt tttagtatat agaggttttt gagtagttcg    1860
   gttgtaggtt ttttatttat ttagagtttt tttttttacg tgtttatttt taattttgta    1920
<210> 28
   <211> 1920
   <212> DNA
   <213> Artificial Sequence
<220>
   <223> chemically treated genomic DNA (Homo sapiens)
<400> 28
   tgtagggttg gggataggta cgtgaggggg agaattttga gtagataggg aatttgtagt      60
   cgggttgttt aagggttttt gtgtattggg ggcgttagga ggtttaggga tggaaaaggg     120
   ggaggtgaga aatggataaa gaaatttggt ttttttggta ttgttgttac gtttggtcgg     180
   ttcgttaagt cgaaggggtt tgtttagttt ttgttcggtt taaatttttt tgggaataag     240
   ttgggatgtt agaaaaaata aaattttata atttttgttt ggggaattta ggtcgttgga     300
   ggtgggtttg gtagggtagc gtgattgttt tttgggagtg gttgttatag tttcgggtga     360
   atcgaggagt ttgggttaag tttttatttt ttttggagag ggtagtagta aggaggtgag     420
   gggagttagt ttattttttt taattttgta tttttttttt atttttttat ttttagttcg     480
   gaagaatcgt tggcggcggg agtttggacg agttggcggg gaaggggacg tagttatagt     540
   attgtttggg agggagggga gaagtttcgt ttttagaggg gaggggagaa gaggaggggg     600
   taggagataa ggggaggaag aaaggcgaag gtaaggcgaa ggggtggaga gtgatatgaa     660
   gagcgagaga aaagagagga tagcggacga gtagattcgg taggggttga aaaaggttaa     720
   ggggttggag ggagggagag gaaggaggag gggagcgagg agggcggggc gttcggtttt     780
   tagtcgcgcg gttatcgttt gtttagtttg gttggatttg agttcggttc gtcgatcgtt     840
   ggtcgaggcg tagttgaagt agttttttag tcgcggcgtt tttatttttt aattttacgg     900
   tttttgcggc gcgggcgcga tgtagttggt tcggggtgaa ggtaaggcgc ggggcgcggg     960
   gcgcggggta ttgggagcga ggtattggcg cgggatgttc ggcgtaaggt gtagggcgcg    1020
   ggtcgttttt ttttcgcgtt tttttatttc gttaaagttt ttttacgatg gtttttattt    1080
   cggttgggtt agtttcgtaa ttttcgtttg ttttttgttt tttttttttt tttcgagtcg    1140
   cggggttttc ggggcgcgcg gattttcgtt aggggtcgtg taggcgcgga gttttagtcg    1200
   gtttgttttg ggttcgaaga aagttgtggt ttggagttag tttttttttt acgatttttg    1260
   ggggcgtttt tttttttttt tgagttcgcg atgggaaggc gtttttattt tattttcggg    1320
   gtttttgaga ttttttggcg aggtggggcg gcgcggtagg tgtagggttg gggagggttt    1380
   gatagttttg gagagtgttc gattttagtt cgtcgtcgtt ttttaggcgg attttttaga    1440
   ggtaggtttt agttttcggc gcggggaggc ggcgcgtttt agaggggcgt agggtgcgcg    1500
   ggggtcgttg gtttttcggg tatttttttt ttgcggtttt ttcgtttttt ttcggagttg    1560
   gtgtttgagg tcgttgggat tttagtttgg cgtttttttt cgtttcgagc gaggtaggtg    1620
   tcgtgggcgg tttcggttat ttttgtattt cgagcgttta ttttgtattt agttttaagt    1680
   cggagagttt tggggatagt cgagagtgtt agtggtttta tagggagatt gagggttttg    1740
   ttttatttcg ggcgtcgtgt gggtttgggt tgtttatagt atttttgcgt tttagtgata    1800
   ggagaagtta agcgcgaggt tttttaatag ttgcgtgtat tttatttttt aggtatttgg    1860
   aatttcggcg tttagaacgt gtttttcggg agagtaaagg ttgtgtttac ggtagtttgg    1920
<210> 29
   <211> 2225
   <212> DNA
   <213> Artificial Sequence
<220>
   <223> chemically treated genomic DNA (Homo sapiens)
<400> 29
   atttttttgt aaattaaatt tttgttgttt agtttagtgt ttggtgtata gtgggggtta 60
   gatagatatt tgttgagtgt atgattaggg cgttgtaggt ggaggaggtg gtggtggggt 120
   gggggagggg aatttttttg ttgtttttcg ttttgtggga attagtttta gaaagaaatt 180
   ttttgtcgtt ttttttattt tattttaatt ttggatttgt tttttgattt tttttatttg 240
   gttttagttt tttatttaga gttaattttg tttgaaaggt attatgtttt gttattaagg 300
   ttttaaaata gggaaaatag tttttgaagt ttttagttcg taggagatag ggtattttag 360
   ggtataaggt tttatcgagg ggttggaaaa agagtttaga ttttaatatt tatagaaagg 420
   ggttttggtt gtttagttgt gagtggtttt tttttcgttt tttagtatat ttggaaacga 480
   ttttatatgt gggcgtcgcg ggatgttggg tagtaaggag atgatttagt tgaataatgt 540
   atttggttta tttttgcgag gggaggagaa cgtcgaggga taggaattgc gcgttagtaa 600
   aagatatttt aatttggggt gcgttcgtta gttgtttttt taaggtgaaa atttttaaga 660
   ggcgagatgt aaattttagt ggcgtttata ttcggaggtg tttttttttt aggtcgtttt 720
   ttcgttttat gcgatatttt aattttcgta gtttttgttt agcgtgggat taagaatggg 780
   gagttttatt ttttttcggt tttttagttt tttaatttaa gataagaggg cgcgcgttcg 840
   ggttgggata ggttttagtc gtattttcgt agattgtatt tttcgttcgg gagcgattcg 900
   ttttagtagt ttttttttcg ttttattaac gcggtcgtat tgtcggcgtt tagaggcgat 960
   attttggtaa agataggaat taagttagaa atttaggttt tagaagtaaa ggttgcggtg 1020
   ttttttggtt ggaagaaaac ggcgggtggg tggaggatgc ggtgcgcggg aattttttta 1080
   cggacgtttg ttgttttttg tttatttttt aggaaggtgt ttttttattt gtattttttt 1140
   ttgtaatttg gagttcgttc gcgagtttag ttattcgttg gttttttttg ttcgcgttcg 1200
   tatattatag atttcgttta tcgtatttta ttcggtttta gagggtttag aggggatttt 1260
   ttgaaattaa ttcgtttgtt tggagttagt ttcgacgtat atttcgagag tggcgggagg 1320
   taaagtagtc ggaatgttcg agaaaaaagt gggcgttaga ggtttttgtt ttagcgattt 1380
   ttttatcgta tcgattgagt ttcgggattg tagggattta agtttttatt tgtttcggcg 1440
   tttttttttt gtgcgttttg ttgtttcgga agtcggatcg agagttatag tgtggttggg 1500
   cgtaggcggg tcgttcggtt tttatttcga ggattcggat ttgtatttgt agagtcgttg 1560
   cgtgggggat gttttttttt ttttttcgtt tcggcgggtt ttttcggcgt gttagggttt 1620
   ttgggtttta gttggagttg gcgtttcggt tttatagttt tttttagcgt ttcggatttg 1680
   tgcgtttcgt ttcgcgttgg atttcgtttc ggggttttcg tttgtttttt agtttaggtt 1740
   aagggtggat gtttagtttt tttattttat cgcgtgtttt cgggttggtg cgtttagggc 1800
   gcgggtatcg tagtttcggt cgcgttgacg cgagttttcg gtagcggggg tagtttttcg 1860
   tacggatttt agcgttttcg tatttcgttt gaatcggatt atatagtagt cgggttttta 1920
   tcgtgcgttt gggaatagtg agtatcgttt tttgtcgtta gtcgtggtat cgtattcggg 1980
   tcgcgcgcgt aggaggggag agttttgttt ttcggaggtg tttagtgtta gcgttcgcgt 2040
   ttattagaag gtgatttttt taaggaaagt tcggtttcga cgagtgcggt gtgggttttt 2100
   ggagatggcg gtgaaggagg aggtttttag gaggaagttg gggatgggga tagtttgttt 2160
   tgtttaaagt ttttgaagtt tttatttaaa ttttgcgatg attattatta ttagatttta 2220
   tggtt 2225
<210> 30
   <211> 2225
   <212> DNA
   <213> Artificial Sequence
<220>
   <223> chemically treated genomic DNA (Homo sapiens)
<400> 30
   ggttatggaa tttgatagtg gtggttatcg taaagtttgg gtgaggattt taggggtttt      60
   gagtagagta aattgttttt atttttagtt tttttttggg ggtttttttt tttatcgtta      120
   tttttaaagg tttatatcgt attcgtcgga gtcgggtttt ttttgaaagg gttatttttt     180
   agtgggcgcg gacgttggta ttgggtattt tcggaagata ggattttttt tttttgcgcg     240
   cgcgattcgg gtgcggtgtt acgattggcg gtagagggcg gtgtttattg tttttaagcg     300
   tacggtagaa attcggttat tgtgtggttc gatttagacg ggatgcgaag gcgttgaaat     360
   tcgtgcgaga ggttgttttc gttgtcgggg attcgcgtta gcgcggtcgg agttacggtg     420
   ttcgcgtttt ggacgtatta gttcgagagt acgcggtggg gtgggggagt tgggtattta     480
   tttttggttt ggattaggag gtaggcgggg atttcggggc ggagtttagc gcggggcggg      540
   gcgtataggt tcgagacgtt ggaaagagtt gtggggtcgg agcgttaatt ttagttggaa     600
   tttagagatt ttgatacgtc ggaggagttc gtcggggcga ggaaaaaaga gggatatttt     660
   ttacgtagcg gttttgtaag tgtaagttcg ggttttcgag gtgaaaatcg agcggttcgt     720
   ttgcgtttaa ttatattgtg attttcgatt cggttttcgg agtagtagag cgtatagaaa     780
   ggaggcgtcg agataggtaa gggtttagat ttttgtagtt tcgggattta gtcggtgcgg     840
   tgggggagtc gttaggataa gggtttttga cgtttatttt tttttcgggt atttcggttg     900
   ttttgttttt cgttattttc gagatatgcg tcggggttgg ttttaagtag gcgagttggt     960
   tttaggagat tttttttggg ttttttagga tcgggtaggg tacggtaagc ggagtttgtg    1020
   gtgtgcgggc gcgggtagag ggagttagcg ggtagttgag ttcgcggacg agttttaaat    1080
   tgtaggggag ggtgtaggta gaggggtatt tttttgaaag gtgagtagga aatagtaagc    1140
   gttcgtgggg aaattttcgc gtatcgtatt ttttatttat tcgtcgtttt tttttagtta    1200
   ggaggtatcg tagtttttgt ttttagagtt tgaatttttg atttgatttt tgtttttgtt    1260
   aggatgtcgt ttttagacgt cgatagtgcg gtcgcgttga tggggcggga agggggttgt    1320
   tggaacgggt cgttttcggg cggggagtgt aatttacgaa aatgcggttg ggatttgttt    1380
   tagttcgggc gcgcgttttt ttgttttggg ttagggggtt ggaaagtcga ggaggggtag   1440
   gattttttat ttttggtttt acgttgagta gaggttgcgg ggattggggt gtcgtatgag   1500
   acggaggggc gatttggaga gaagatattt tcgggtgtgg acgttattgg gatttgtatt    1560
   tcgttttttg gaggttttta ttttgggaaa gtagttggcg ggcgtatttt aggttagggt    1620
   gttttttgtt ggcgcgtagt ttttgttttt cggcgttttt tttttttcgt aggagtggat  1680
   taaatgtatt gtttagttgg gttatttttt tgttatttag tatttcgcgg cgtttatatg    1740
   tggagtcgtt tttaggtgtg ttgagaggcg aggggaaggt tatttatagt tggatagtta    1800
   gggttttttt ttataggtgt tgggatttgg gttttttttt tagtttttcg gtgaagtttt    1860
   gtattttggg gtgttttatt ttttgcgggt tgagggtttt agagattatt ttttttattt   1920
   tgagattttg gtgataggat atggtgtttt ttaggtaggg ttgattttgg gtggagaatt    1980
   agggttaagt gaaggaggtt agagagtaga tttaaagttg gagtagggtg ggaagggcgg    2040
   tagaggattt ttttttggag ttggttttta tagaacgggg agtagtaggg gaattttttt    2100
   tttttatttt attattattt tttttattta tagcgttttg gttatgtatt taatagatgt    2160
   ttgtttagtt tttattatgt attaggtatt ggattgggta gtaggaattt ggtttatagg    2220
   aagat                                                                2225
<210> 31
   <211> 2251
   <212> DNA
   <213> Artificial Sequence
<220>
   <223> chemically treated genomic DNA (Homo sapiens)
<400> 31
   ttttttagcg tgattgatcg cgatttagag gagaatgggt aggtgtagtg cgagttattg      60
   ggagacgtgt tttttcgttt taagtttttt tttaagaatt attatattat cgttatcgaa   120
   gtttttttgg atcgagaggc ggggattttt tatattttga ttgtagtggt tcgggatcgg   180
   ggcgagtttg cgttttttat tagtaagtcg atttaggtat aagtgtcgga tgtgaacgat   240
   aacgcgtcgc gttttagtta gtcggtttac gacgtgtatg tgattgaaaa taacgtgttt   300
   ggcgtttata tttacgcggt gagcgttatc gatcgggatg agggcgttaa cgtttagttt   360
   gtttatttta ttttcgagtg ttagatttag ggtatgagcg tttttattta cgtttttatt   420
   aattttgaga acggttattt gtacgttttg cgttttttcg attatgagta gttgaaggat   480
   tttagttttt aggtggaagt tcgggacgtt ggtagttttt aggcgttggt tggtaacgtt   540
   attgttaata tttttatagt ggattaaaat gataacgttt ttgttatcgt ggcgttttta     600
   ttagggcgta acgggatttt agcgcgtgag gtgttgtttc gttcggcgga gtcgggttat     660
   ttgtttattc gcgtggtcgt cgtggacgcg gacgacggcg agaacgttcg gtttatttat     720
   agtatcgtgc gtggtaacga aatgaatttt tttcgtatgg attggcgtat cggggagttg     780
   cgtatagtac gtcgagtttc ggttaagcgc gatttttagc ggttttatga gttggtgatc     840
   gaggtgcgcg attatgggta gtcgtttttt ttttttatcg ttattttggt ggtttagttg     900
   gtggatggcg tcgtggagtt ttagggcggg ggcgggagcg gaggcggagg gttaggagag     960
   tattagcgtt ttagtcgttt tggcggcggg gaaatttcgt tagattttat ttttattttt    1020
   attatcgcgt tgggttcggt gttttttatt tttttgttgg ttatgatcgt gttggtcgtg   1080
   cgttgttaaa aagagaagaa gtttaatatt tatatttgtt tggttagcga ttgttgtttt    1140
   tgttgttgtt gttgcggtgg cggaggttcg atttgttgtg gtcgttaagt tcgggcgcgt    1200
   aagaagaaat ttagtaagtt agatattatg ttggtgtaga gttttaatgt atttagtaat    1260
   tcggtttagg tgtcgataga ggagttcggg ggttttggtt tttattatta taattagaat    1320
   tattgttatt aggtatgttt gatttttgag ttcgttaaga tcgatttgat gttttttaag    1380
   ttttgtagtt tttcgcggag tacggatatt gagtataatt tttgcggggt tatcgttatc    1440
   ggttatatcg attagtagtt tgatattatt tttaacggaa gtattttgtt taacgaggta    1500
   aggttgaagc gaaaggatta ttatttttta ttttttttat tagaaagttt tttttagttc  1560
   ggtttttgta tttttggtgt attgtattta tttttaggat attagtttat gtgtatcgtt    1620
   gtgggagtag agatgggcgg ttattttttt ttattttttc gtgtgtaatt taattttcgc   1680
   gttgttttat ttttttatat ttatttttat ttcgtttttt tggtattttg ttattttgga    1740
   gttttttttt ttgttttttt attttgttag tttttttttt ttttagtaat ttgggtatga   1800
   agggaaattg cgtgaaggga gagggaaatg tggaggaggg atttattttt tagtattggt    1860
   aaaggttttt tttttttgcg tttgttttag gtattaataa agttggtttt attttgtttt    1920
   gtttaacgat gtttttagtc gcgtgtataa gtaagttata gattgtttaa ttttatatag    1980
   ttgtttaatt tcgaatttat gttttagtga ataagttatt agattttttt tttttgaatt    2040
   cgggttgtaa aaaaagtttt tagttcggtt ttggatagta tttatagacg tttttgaagt    2100
   cgagcgttta tatagtgtga atttgaatga agttgcgttg gtataaattt ttgttaagag    2160
   taaattaacg aaaggtttaa ataattgttg ttttaaatat atttttttag ttaataatta    2220
   tttttttgtt attgttttgg ataaattacg t                                   2251
<210> 32
   <211> 2251
   <212> DNA
   <213> Artificial Sequence
<220>
   <223> chemically treated genomic DNA (Homo sapiens)
<400> 32
   gcgtgattta tttaaaatag tggtaagaag gtgattatta gttaaaaaga tatatttaag      60
   ataataattg tttaagtttt tcgttagttt atttttaata ggggtttgtg ttaacgtagt     120
   tttatttaaa tttatattgt gtgggcgttc ggttttaaga gcgtttgtaa atgttgttta     180
   gagtcgaatt gaaggttttt tttgtaattc gagtttagag gagaaggatt tggtaatttg     240
   tttattaaaa tatgaattcg aggttagata attgtgtaaa gttaaataat ttatagttta     300
   tttgtatacg cgattaaaag tatcgttaaa taaagtaaaa tagagttaat tttattaatg     360
   tttgggatag acgtaaagaa aaaagatttt tgttagtgtt agaaagtaag tttttttttt     420
   atattttttt ttttttttac gtagtttttt tttatgttta ggttattgag aagggaaagg     480
   attgatagga tggaagagta aaggagggag ttttaaggtg gtaaagtatt aaggggacgg     540
   aatgaaaata aatgtgaaag ggtggaataa cgcgaaagtt aggttatata cgaaggagtg     600
   ggagaaggtg atcgtttatt tttgttttta taacgatata tataagttaa tattttaaaa     660
   atagatatag tgtattagag atataagggt cgggttagag gaggtttttt gatggaggag     720
   atgagagatg gtggtttttt cgttttagtt ttatttcgtt ggataaaatg ttttcgttgg     780
   agatgatatt aggttgttgg tcggtgtaat cggtgacgat ggtttcgtag gggttgtgtt     840
   tagtgttcgt atttcgcgaa gggttgtagg gtttaagaaa tattaggtcg gttttggcgg     900
   atttaggggt taggtatatt tgatagtagt aattttggtt gtggtggtgg gagttaaagt     960
   tttcggattt ttttatcggt atttgggtcg ggttattggg tatattggag ttttgtatta    1020
   gtatgatgtt tgatttgttg agtttttttt tgcgcgttcg ggtttggcgg ttatagtagg    1080
   tcgaattttc gttatcgtag tagtagtagt agaggtagta atcgttggtt agataagtat    1140
   agatgttgag tttttttttt ttttggtaac gtacggttag tacgattatg gttagtagga    1200
   agatgaagga tatcgagttt aacgcgatga tgaggatgag ggtgaggttt agcgaggttt    1260
   tttcgtcgtt agagcgattg gggcgttggt gtttttttga tttttcgttt tcgttttcgt     1320
   tttcgttttg gggttttacg gcgttattta ttagttgaat tattagggtg gcggtggagg    1380
   aaaggggcgg ttgtttatgg tcgcgtattt cgattattag tttataaggt cgttgggggt   1440
   cgcgtttggt cgggattcgg cgtgttgtgc gtagtttttc ggtgcgttag tttatgcgaa    1500
   agaggtttat ttcgttgtta cgtacgatgt tgtaagtgag tcgggcgttt tcgtcgtcgt    1560
   tcgcgtttac ggcggttacg cgggtgagta ggtaattcgg tttcgtcgag cggggtagta    1620
   ttttacgcgt tggagtttcg ttgcgttttg gtagaggcgt tacgatggta ggggcgttgt    1680
   tattttgatt tattatgagg atgttgatag tggcgttatt agttagcgtt tgggggttgt   1740
   tagcgtttcg ggtttttatt tgaaaattga agttttttag ttgtttatag tcgaaggagc    1800
   gtagggcgta taagtagtcg tttttagagt tgatagaaac gtaggtgaag acgtttatgt    1860
   tttggatttg gtattcgagg atagagtagg taagttgggc gttggcgttt ttatttcggt    1920
   cggtggcgtt tatcgcgtag atgtaggcgt taggtacgtt gtttttagtt atatatacgt    1980
   cgtagatcgg ttggttgaaa cgcggcgcgt tgtcgtttat attcgatatt tgtatttgga    2040
   tcgatttatt ggtggagagc gtaggttcgt ttcggtttcg agttattata gttagggtgt    2100
   aggagttttt cgtttttcgg tttagggggg tttcggtaac gatggtgtag taatttttaa    2160
   aggaagattt gaggcggaaa ggtacgtttt ttagtagttc gtattgtatt tgtttatttt    2220
   tttttgagtc gcggttagtt acgttgaaaa g                                  2251
<210> 33
   <211> 2553
   <212> DNA
   <213> Artificial Sequence
<220>
   <223> chemically treated genomic DNA (Homo sapiens)
<400> 33
   ttggttattt ttgttttttt ttagattttt agagttttta gtgtagttat agaggatgtt      60
   gttggtttta gttttaagaa gacgtcgttt tttttagagg gttaagtaag tgggaatttt     120
   tttttttatt tgttttgggt tttaggtagg gtttttggtg taaggtttgg ggttggaagt    180
   cgatttattt aggtttaggt tttggggtag aattgaaatt ttttggttat tgtcggttgt     240
   agtttgggag taggttattg ttaaagttgt gggttttttt aggatagttt ttttatgagg     300
   tcggtttttt atttgttgtt tttttatatt tggtggttag ggatgtggtt ttgggtagaa     360
   cgatgatttt ttatttttgt tattatggaa gttatcgttg ttttttagtt tagttagtta     420
   tttgggttgt agagtatttt ttttatgttt ttcgggtgtt tttttttttt tttgttttag     480
   tttggttttg ttttattttg tttttaggga ggggtatttt ggagtggggt tagggtatgg    540
   ttttttttcg agggagtttt tttttggttg tttttagggt agttttgtat agttttagta    600
   tttggcgtat tttttttgat atttttttta gggatagtta ggtattttgt gtggggtatt    660
   taagagagtt aggttcgtta gtttttagtt tttgttagaa tgtaggtttg aggggtgagg     720
   ggcggggtag gggtagggat aggaatttcg gcgtgttttt tattcgtaaa ggtttattga     780
   ggtttcgagt tttagttatt gagttattaa gttagtttgg gttaggtttg ggtgttttgt     840
   ttgtaatgga ggtagagacg gggtttcggg gtagttttga ggatgttggg tgtatagcgg     900
   gggtttcgtc ggtaggaatt atttatgttt ttttttgggt taagttttgt ggatgtttag       960
   tttggggtcg cggggagttg gtaggttagt ggtagatatt ggtgggtaga tttagtgttt    1020
   ggtagaatag gtattaagga agtggtgatc ggagggaagt taagtgtatt taaattttcg    1080
   ggtgagttat tatcgtcggg ttttttatag ttgttgaaag tgagtaatag tgatgaaggt   1140
   ttgtgagttt ttgcgtgagc gagtgaatgg attagtagta gtttttaggt tgtggaagag    1200
   cgtttttttt tcgggatggg gatatttggt tatagtaatt tttaattttt tatttattta    1260
   tcgtttattg tagaggtatg cgggggtttt gttttttgta ggtaggagtg aggggtattt  1320
   ttgtgatgtg gtatttttgt gatcgaggtt atgtgtgatc ggtgtaaggg taggaagcga    1380
   gttattggtt tgtattaggc gtgggggttt ttgcgagggt aggatttaaa gtcggtttgg   1440
   tttttcggtt gtagtatttt tttttttttc gaattaggtt agagttttgg gacgggaggt    1500
   gttttgtaga ttattttttt tattaatttt cgtttttcgt tttattttcg cggtgattcg    1560
   gtgaattgtc ggttttttgt tgtgtatcga gtggggtagt gattttgacg tggcgttttt   1620
   tgtcgttttt gttatcgtta ttattttcgg tggtttagtt ttcgtatttt ttatttttat   1680
   ggaggaatgt attaggtttt tttttttgga tgtatttttt atttatatgt ttttaaattt    1740
   tggtattttt tgtttttttt ttatttttat tttttttttt aggtttttag ataaagggga   1800
   agtggttgga tttttttaaa gggatagtgt tttattagtt tattgttgaa tttttttttt   1860
   taattttagt tttttagtta tagttaatta gtattagtag atagtttatg agtgatattt    1920
   atgtaggttt taggttgtgg agagtttttt gggtaggaaa tagtttttaa ggttttttat  1980
   tttatttagg ttttagtttt ttttatttgt ttttttttta gattgtggtt ttttggagtt   2040
   tggttttttt gtttttgtgt gatcgatata tagtatttaa atagtggtag agcgggacgg    2100
   attttttagt ttgttttttg tgtgggtttg tattttgatt tagatatgtt tttttatagt  2160
   aggatttagg ggggtatatg tgtgtttgcg ggtttattgg ggtattcgta tttggtttat    2220
   tttatttttt agagagaggg ttttgttgtg ttatttagtt ggagtgtagt ggtgtaatta    2280
   tagtatattg tagtttttaa tttttgggtt taagcgattt ttttttttta gtttttttag  2340
   tagttgggag tataggattt attgtatttt ggttaatttt ttaataattt tttaagagat    2400
   ggggttttat tgtgttgttt aggttggttt taaatttttg gttttaagtg atttttttat   2460
   tttcgttttt tgaagtgttg agattatagg tatgagttat tatgtttatt ttagattgat    2520
   atttttatat ttgtttattt tggttgggta ggg                                  2553
<210> 34
   <211> 2553
   <212> DNA
   <213> Artificial Sequence
<220>
   <223> chemically treated genomic DNA (Homo sapiens)
<400> 34
   ttttgtttag ttaggatgaa taaatataga aatgttagtt tgggatgggt atggtggttt      60
   atgtttgtaa ttttagtatt ttaggaggcg aaggtgggag gattatttga ggttagaggt     120
   ttgaggttag tttgggtaat atagtaagat tttatttttt aaaaaattat taaaaaatta     180
   gttaggatat agtgggtttt gtatttttag ttattaggga ggttggggag ggaggatcgt     240
   ttgagtttag gagttgaagg ttgtagtgtg ttatgattat attattgtat tttagttggg     300
   tgatatagta agattttttt tttaaaaaat aaaataaatt aaatgcgggt gttttagtga     360
   attcgtaggt atatatgtgt ttttttgggt tttgttgtgg gggggtatgt ttgggttagg     420
   gtatagattt atatagagaa taggttaggg ggttcgtttc gttttgttat tgtttgggtg     480
   ttatgtgtcg gttatatagg gatagaagaa ttaggtttta aagggttata atttaggaga     540
   gaggtaggta ggaaagattg ggatttggat gagatgaggg attttaaggg ttgtttttta     600
   tttaggaaat tttttatagt ttggggtttg tatgggtatt atttatgggt tgtttgttaa     660
   tgttaattaa ttgtaattag ggaattgggg ttgaggaggg gagtttagta gtaagttggt     720
   gggatattgt ttttttaaga ggatttagtt attttttttt tgtttgggag tttaggggaa     780
   ggggtgggag taaaggggga gtagaaaatg ttagggtttg gaagtatgtg ggtgaggggt     840
   gtatttagga agggaggttt ggtgtatttt tttatggggg tggggaatgc ggaggttggg     900
   ttatcggagg tggtggcggt ggtaggggcg gtaggagacg ttacgttagg gttattgttt     960
   tattcggtgt atagtagggg gtcggtagtt tatcggatta tcgcgggggt ggggcggagg    1020
   acggaagttg gtgagggggg tggtttatag ggtatttttc gttttagggt tttaatttaa    1080
   ttcgaaaggg aaaggagtgt tgtagtcggg aggttaggtc gattttgggt tttgttttcg    1140
   tagaagtttt tacgtttggt gtagattaat gattcgtttt ttgtttttat atcgattata    1200
   tatgatttcg gttatagggg tgttatatta taggagtgtt ttttattttt gtttgtagga    1260
   agtagggttt tcgtatattt ttgtagtggg cggtgggtgg gtgggggatt aggaattgtt    1320
   gtaattaagt gtttttattt cggggaggga acgttttttt ataatttgga aattgttatt    1380
   ggtttattta ttcgtttacg tagaaattta taaattttta ttattgttgt ttatttttag    1440
   tagttgtgaa agattcggcg gtgatgattt attcgagggt ttgagtgtat ttggtttttt    1500
   ttcggttatt atttttttga tgtttgtttt attagatatt aggtttgttt attagtgttt    1560
   gttattgatt tgttagtttt tcgcggtttt aggttgggta tttataaagt ttggtttagg    1620
   agagagtata agtgattttt gtcggcgagg ttttcgttgt gtatttagta tttttagaat    1680
   tgtttcgaga tttcgttttt gtttttattg tagatagggt atttaggttt ggtttaggtt    1740
   gatttggtgg tttagtggtt ggggttcggg gttttagtga atttttgcgg atggagagta    1800
   cgtcggagtt tttgtttttg tttttgtttc gttttttatt ttttaggttt gtattttggt    1860
   aggagttaga ggttgacggg tttggttttt ttgagtgttt tatatagagt gtttgattgt    1920
   ttttaagaag gatgttaagg gaggtgcgtt aggtattgag gttgtgtaga gttgttttgg    1980
   ggatagttag agaggaattt tttcggggga gagttatgtt ttggttttat tttagggtat    2040
   ttttttttga gagtagggta ggataaagtt aggttggggt aggagaaggg ggaggtattc    2100
   ggagggtatg aaaggggtgt tttgtagttt aggtggttgg ttgggttggg agatagcggt    2160
   ggtttttata atgataggag tggagaatta tcgttttatt tagggttata tttttggtta    2220
   ttaggtgtga agaaatagta ggtggaggat cggttttatg gggagattgt tttggaagga    2280
   tttatagttt tggtagtggt ttgtttttag gttgtagtcg atagtaatta aggagtttta    2340
   gttttgtttt agagtttgga tttaggtggg tcggttttta gttttaggtt ttatattagg    2400
   ggttttgttt ggagtttagg ataagtaggg agggggattt ttatttattt agttttttgg    2460
   aggaagcggc gtttttttgg ggttgaagtt aatagtattt tttgtggtta tattgggggt    2520
   tttggaggtt taagggaggg taggggtggt tag                                 2553
<210> 35
   <211> 2001
   <212> DNA
   <213> Artificial Sequence
<220>
   <223> chemically treated genomic DNA (Homo sapiens)
<400> 35
   ggtaaaagtt tgtttggatt ttttggttat tagaaatatg agtatggtgg tggtttttag      60
   ttttttattt atgtttgggt ttaagagatt gggagtttag gtttattgat tttttgagaa     120
   agattaagat tttgtatttt agaaagaggt ttggggattt ttgttttgcg taagggtaga     180
   aggattagtt gtttttttga gtattttaat tcggaatttc ggttcgaagt cgagatagga     240
   gattggatgc gaggtttttt tagagttggt tttttttaaa taatttttaa aatttttaga     300
   ttttaggggt acgtcgaaat tttttaaagt agtttaaaga atataacgag agttttaata     360
   ttttaggtgg cggcgcgttg gttttttgga gcggggcggg acgcggtcgc gcggatttac     420
   gtgtataatc gcgcgggacg gggttacgcg gatttacgtg tataatcgcg ggattttagc     480
   gttagcggga ttttagcgtt agcgggattt tagcgttagc gggattttag cgttagcggg     540
   attttagcgt tagcgggatt ttagcgttag cgggatttta gcgttagcgg gtttgtggtt     600
   tagtggagcg agtggagcgt tggcgatttg agcggagatt gcgttttgga cgttttagtt     660
   tagacgttaa gttatagttc gcgtagtagt agtaaagggg aaggggtagg agtcgggtat     720
   agttggattc ggaggtcgtg atttagggga aagcgtgggc ggtcgattta gggtagttgc     780
   ggcggcgagg taggtgggtt ttttgttttt tggagtcgtt tttttttata tttgttttcg     840
   gcgtttttag tagtttttat tttggttttt cgcggttatt gcgggattcg gcgttgtcgt     900
   tagtttagtg gggagtgaat tagcgttttt tttcgttttc ggttttttcg acggtacgag     960
   gaatttttgt tttgttttat agattttcgg ttttcgtcga gtgcggtatt ggagtttgtt    1020
   tcgttagggt tttggaatta gagaaagtcg ttttttggtt atttgaagcg tcggattttt    1080
   atagtgtttt ttagtttggg cgggagcggc ggttgcgtcg ttgaaggttg gggtttttgg    1140
   tgcgaaaggg aggtagttgt agttttagtt ttattttaga agcggttttc gtatcgttgc    1200
   ggtgggcgtt ttcgggtttc gatttcgtta gcgtcgcggg gtagaggtat ttggagttcg    1260
   tagggtttag atttgggttg gaaaagtttc gttgattgta ggtaagcgtt cgggaggggc    1320
   ggttaggcga agtttcggcg ttttattata tattttcggg ttttatgtta gttgtattcg    1380
   cggtattggg taggaaatgg tagggttgag gtcgatttta ggagtataag ggagtttttt    1440
   attttttgtt tatatttgtt atttttagtt ttgtaattta ttttagatat atagaaagta    1500
   agtaggattg gtggggagac ggagtttaat aggaatattt tttagtagtg agtaggggtt    1560
   gtatgggacg cgggaggagt ttagaggagg cgcggagagt gttcgaggtt gggtgagtgt    1620
   ttagagggga gatagttgaa tcgggtttaa gaggtgttta gtgggtgttt gttgaatgaa    1680
   tgagtgatgg gttttgaagt ttgagtgtat tgaaagaggg ggtgtgtaaa aagggttttt    1740
   tttattatat aggatatagt atatgtaaat tttttttttg tggaaaagtt agataggtta    1800
   aaaaggttat aaataaatta gtcgggtatg gtggtgcgcg tttgtagttt tagttattag    1860
   ggaggttgag ttaggggaat cgtttgaatt cgggaggcgg agattgtagt gagttaagat    1920
   cgcgttattg tattttagtt tggaaataga gcgagatttc gtttcggaaa aaaaaaaaaa    1980
   aagttataaa tcgtgtgtgg g                                               2001
<210> 36
   <211> 2001
   <212> DNA
   <213> Artificial Sequence
<220>
   <223> chemically treated genomic DNA (Homo sapiens)
<400> 36
   tttatatacg gtttgtaatt tttttttttt tttttcgaga cggagtttcg ttttgttttt      60
   aggttggagt gtagtggcgc gattttggtt tattgtaatt ttcgtttttc gggtttaagc     120
   gatttttttg gtttagtttt tttagtagtt gggattatag acgcgtatta ttatgttcgg     180
   ttaatttgtt tgtaattttt ttaatttgtt tggttttttt atagggagag gatttgtata     240
   tgttgtgttt tgtgtgatga aaggagtttt ttttatatat tttttttttt aatgtattta     300
   gattttaaag tttattattt atttatttaa taaatattta ttaagtattt tttgaattcg     360
   gtttaattat ttttttttta ggtatttatt taatttcggg tatttttcgc gttttttttg     420
   agtttttttc gcgttttata tagtttttgt ttattgttgg aaaatatttt tgttaagttt     480
   cgttttttta ttagttttgt ttgttttttg tgtgtttggg ataggttgta aaattggagg     540
   tgataaatgt gggtaggaaa tggagggttt ttttatattt ttagggtcgg ttttagtttt     600
   gttatttttt gtttaatatc gcggatgtaa ttggtatggg attcggaagt gtgtggtaaa     660
   gcgtcggggt ttcgtttggt cgtttttttc ggacgtttgt ttgtagttag cgaagttttt     720
   ttaatttagg tttgggtttt gcgagtttta ggtgtttttg tttcgcggcg ttggcgaagt     780
   cgaagttcga gaacgtttat cgtagcgatg cgaaggtcgt ttttggggtg gggttgaggt     840
   tgtagttgtt tttttttcgt attaaggatt ttaattttta gcgacgtagt cgtcgttttc     900
   gtttaggttg ggaggtattg tagggattcg acgttttagg tggttaaaga gcgatttttt     960
   ttgattttag ggttttggcg gggtaggttt tagtatcgta ttcggcggag gtcgaaggtt    1020
   tgtggggtag gataggagtt tttcgtgtcg tcggaagggt cgaggacgaa ggagggcgtt    1080
   aatttatttt ttattgggtt ggcggtaacg tcgaatttcg tagtgatcgc ggagggttaa    1140
   ggtgaaaatt gttgggggcg tcgagggtag gtgtggggag gggcggtttt agggagtaag    1200
   gagtttattt gtttcgtcgt cgtagttgtt ttgggtcgat cgtttacgtt tttttttggg    1260
   ttacgatttt cggatttaat tgtgttcggt ttttgttttt ttttttttgt tgttgttgcg    1320
   cgggttgtaa tttgacgttt aggttggggc gtttagggcg tagttttcgt ttaggtcgtt    1380
   agcgttttat tcgttttatt gggttataga ttcgttggcg ttggggtttc gttggcgttg    1440
   gggtttcgtt ggcgttgggg tttcgttggc gttggggttt cgttggcgtt ggggtttcgt    1500
   tggcgttggg gtttcgttgg cgttggggtt tcgcggttgt gtacgtgagt tcgcgtggtt    1560
   tcgtttcgcg cggttgtgta cgtgagttcg cgcggtcgcg tttcgtttcg ttttagggag    1620
   ttagcgcgtc gttatttggg atgttaggat tttcgttgtg ttttttggat tgttttgggg    1680
   gatttcggcg tatttttagg atttaggagt tttggaagtt gtttgagaga aattagtttt    1740
   gggagggttt cgtatttagt tttttgtttc ggtttcggat cggggtttcg ggttaaggtg    1800
   tttagaggaa tagttgattt ttttattttt gcgtagggta gagattttta aatttttttt    1860
   taaaatgtag ggttttagtt ttttttaggg agttagtgaa tttagatttt tagttttttg    1920
   agtttaagta tgaataggga attggggatt attattatgt ttatattttt ggtggttagg    1980
   aagtttaggt aggtttttgt t                                              2001
<210> 37
   <211> 219909
   <212> DNA
   <213> Artificial Sequence
<220>
   <223> chemically treated genomic DNA (Homo sapiens)
<400> 37
   cgttattcga gttgtaaagg gcggtttttt gtgtttgttt cgttgtatcg atatatcgag      60
   ttgcgtacgg tgtttagcgt agggagaata aatgattatt tgtttaacgc gtttatttat     120
   aggtgaggaa attaaggttt taatttaatc gacgtatttt gtttttttga ttattagaaa     180
   agtagtagga taggtgtttt gtttcgtttt atttcggttt attaagtcgg tatttcggtt     240
   tcgattttcg gttgtgttcg gcgtcgtcgc ggtgttcggc gtcgtcgttt cgttcggcgg     300
   ggtcgttcgg agcgttcgta ttttcgttcg tttttatttg gtcgggttcg tttcgttcgg     360
   attcgggatt gggaagtgcg gcgattttcg gaattagtta ttggcgttag cgcggggagt     420
   tgggggtgta gagttgcggg cgcggcgggt tacgtaggcg gtttttattt tcggtttggt     480
   ttggtttggt ttggtttgcg ttgtcgcgcg ggggcgtttt tttttaggtt cggcgttcgt     540
   tagtttcgtt tcgttaggtg tagcgtagcg taggggtggg cgggggtggg gttcggcgcg     600
   tacgtttacg gggcggggag ggggcgggtt aggggcggga ttatagtcgg ttgggtcggg     660
   gttttatgcg tattttcggg gaggggcggg gcgggggcgg ggtcggggcg gggttcggtc     720
   ggtgtatttt agacggcggg tcgttttttt ttttcgtttt tttattatcg gtttaggatt     780
   agcgttttgg gagcgcgcgt ttcgttgttt cgtcgttata tttttttggg agcggcggtt     840
   acggaggtat tatgaagaag ttttatttag gtgggtttcg cgttcggggt ggggaggggt     900
   cggtgtttcg ggattagcgt tgtttatttg agtgtttgcg gtcgggagtg gcgaggcgtt     960
   ttcggagttg agcgagtttt cgcggcgggt atattgtagg tcgagttttt tttaggatag    1020
   ggtcgttgtc gggtcgtttt cgatttgagt cgatcgtttt ttgcgttgtt tttagttttt    1080
   gttcgagtgt cggaggggtt gttttggggg acgttttttt tttttcgttt tttgtatttt    1140
   cgtaggaatc gttgattttt taggtcggtc gggtgttttg ggtttttgtg cgtttgtgtg    1200
   ggtgaatggg gtcggggtta ggtggagggg tgtttttggg tttagttttt agggttggtg    1260
   gtttaggtcg tagtattttt ttttcggatt tttttcggtt ttttttttat ttagtggggt    1320
   acgggacggt ttttagatgg gatcgtttag tagcgtttaa atttggcgat tcgggtttac    1380
   gttttgcgtt taggacgtcg ttcgcggttg atatttttat tttattttag tagggttcgt    1440
   tttaagtagg cgaagggggt ttgatttggg ttttattatg agtcggttta ggagttttta    1500
   ttttataaga ggttttcgtt ttggtatttt gaagttttat tttatagatg ggtatatcga    1560
   ggttataagg gtaggttaag agaaatttag atttttatta tttgtttttt tttttttttt    1620
   tttttttttt tgtggttttt tttaaaggat ttaggtaggt aggatttttt gtttttttta    1680
   gtttttaaat tataaatagc gttttttttt ttaaatttag aaatcgtttg tttaatgttt    1740
   tttttttaat ttatttagat agaaaggtat ttgttaagga tttatgaatt aatttttatt    1800
   gataggagaa tagaattatg ggttttagtt ggaggtgaag gagttatcgg ggttattttt    1860
   ttgtttttgg gttattgtcg gttttttttg gttttttata tttaggaatt aaaatgagtg    1920
   ataaagtatt ttgttttaaa gaggattatt tagaattttt tcgaaaatat ttttttgggt    1980
   tagtagttta gaattttaat ttttattcgg ttgttgcgaa taagatggtt ttttacgtat    2040
   tcggagtata gttttgttag gagcgtgggt gggttggtgt gttggtgggc gggagcgggt    2100
   ttttaggtgg tatatgtttg gttttggtat cggggttttt tttttaatat gttttaaata    2160
   tttgagattg ttgtggggat cgcgcggtgt atggtttttt ttaggggatt tttagttcgt    2220
   ttagggaggg ttttttgttt ttggtttttt atattttatt ttttattggt gggtttagat    2280
   agtgggggtg atcggggttt ttttaggtcg tgggttttgg gttttttttg cggagatttt 2340
   tttgtataga tgtcggaagg gttttgaggt aggtttggag tttttgtatt tgcggtttat 2400
   tgggaggtgg ggagattatt atttgttttt tttttttttt ttttatatat atttatttta 2460
   ttttttattg ggtttttttt tttttttttt ttttaaaaaa aaggatagaa ttttttgtag 2520
   taggtggaag tagagtttgt ggagtgagta ggaattaatt tttgtatagt agtttcggtg 2580
   tttttttgtg tttatttaaa ggggtaggtt tttttgagga tgggggaagg gagggtttag 2640
   attatttttg gtttgttttt tgtttggtag tggtttaagg tgtgttgggt tattgtgttg 2700
   tagttgtatg gagtgggatt ggagggggtt gatggtgggg gtggtttttt gtttttattt 2760
   tgagtgtttt ggagttgttt tggtattagt tttttttcgg gtacgggtat tcgtaggagt 2820
   taggttgatt ttttaataag ttatcgatat tatgaaatta aggaaagtag taaaagatgt 2880
   taaaaatttt ttaaggtttt gataatttaa ggataatagg aaggttttta tttttttatt 2940
   tagattgtgc gggattattg tttttagatt tgtatgtggg ttttagaata aacggaatga 3000
   ttataatgtt gttttcggta gatttggttt gaatattcgt gagtttttga aaaggagaaa 3060
   gattttagaa ggatgtgttg gggtaatttt aagtatcgtg ggtttataga tagttatcga 3120
   agatatttta tgtgttgggt atatatggtt aagtttgagt gggtatagaa atggtaatga 3180
   gatatagttt ttgtttgtag agaatttata tttttatcgg gagttaggat gagggcgaga 3240
   tattagttta ttaatatttt ttttttttga gtagtttagt ttttaaatat tataggaatg 3300
   tttcggaggt tttaggtgga ggtgagattg agttgggttt tgaggaagga gaagttttga 3360
   gtagtggaga ataaaaaggg atttggttag gtgcgtttga agttttagtt atttaggagg 3420
   ttgagatacg aggatcgttt gagtttagga ggttatttag gaggttgaga tacgaggatc 3480
   gtttgagttt aggaggttat ttaggaggtt gagataggag gatcgtttga agttaggagt 3540
   ttaaggttgt agttttgtga tagcgtttgg aaatagatac ggtattttag tttgggtggc 3600
   gttatgagat tttatttgta agaaggtttt ttttaatgcg gaaggtagta agggtttata 3660
   tagggtagtt gtaggattta gggggatatt ggggataggt tttttttggt agttttattg 3720
   ttgttttagg ttttttcgat tgttttattc ggttttttaa tggggtggag gtagtggtag 3780
   tttataggta gtgtggttta gtggttatat ttgggggttt agagtcgtta gttttttggg 3840
   attaaatttt attaggttgc gttttttttt gtggtataag gtttttattt tatgtatttt 3900
   atttgtttta tttgtttttt tgtgtgtgtg gtgtttttta attttattta tttatttatt 3960
   tttgagacgg agttttattt tgttttttag gttggagtgt aatggtatga ttttagttta 4020
   ttgtaatttt cgttttttag gtttaagtga tttttttgtt ttagtttttt gagtggttgg 4080
   gattgtagga acgtgttatt gcgtttagtt aatttttata tttttattag agatggggtt 4140
   ttattatgtt ggttaggttg atttcgaatt tttgatttcg tgatttgttt gttttggttt 4200
   tttaaagtgt tgtgattata ggtatgagtt attacgttta gtttgtttta tttgttaaat 4260
   gggatagtgt tatgttgttg taaaggttaa acgagtttat atttgaaaaa gttgagaaat 4320
   gtttgttttg taatttgtta gttgttgtta tgattatgat tgttattgat aggaggatgg 4380
   agaaggttgg ggttacgtta ttgttaggac gttttagaag tttttatgat ttttttttat 4440
   tgtggttaga gtatggtgta gggttttttt tttagttttt tagtattgga gttagttgta 4500
   ttgtattcgt tttggttttt taggttttga gttttgggat tttttttgta tattaggtta 4560
   tatggaattt gtcgttattt ttaggttttt ttggaaattt tttgtgggtg gtagcggtag 4620
   gaggatttat tcgaggtcga tgagttatag aagcgggatt gaggttgcgt tatattttta 4680
   ttggtatttg ggaattattt tggagatttt ttttttattt tttatttttt tgagatggag 4740
   ttttgttttg ttgtttaggt tggagtgtag tggtacgatt tcggtttatt gtaatttttg 4800
   ttttttaggt ttaagtaatt tttatgtttt agttttttga gtagttggga ttataggtat 4860
   gtgttatcgt gtttagttaa tttttgtatt ttttagtaga gatggggttt tattatattg 4920
   gttaggttgg tttcgaattt cggattttag gtgatttatt cgttttagtt ttttaaagtg 4980
   ttgggattat aggtatgagt tatcgtgttt agttattttg gagttttttt gtggttttta 5040
   ggagattttt gaaggggtat tagttacgat taaaaagttt tatgttgtcg tgatttttga 5100
   gttttgggta gggagggagg ttgtatttat ataggaagta gtttttgtcg tgatttagtg 5160
   tgttggttcg tcgtggtgga tagggttgta tttttattta gcgtttcggg agttgattta 5220
   cgggaagggt aaggaattgt atttttagag aatatttttt tagttagtta attcggtgat 5280
   aggcgagcgt gggaagggta taagtatagg gttttttttt tttttttttt agtttcgttt 5340
   tcgcgtttgt tttcgttgtt ttttgttttt ttacgtttaa gtttgtttgt tttggcgtat 5400
   ttaggggatt tggattggag agaaaaagtt atttcgtacg gttagtttgt ggttttgttt 5460
   gatttgtgtt tttcgtcgag gttttttagt tgtgagatag ttggttgagt ttaagttttg 5520
   gggttagttt tttttttagt ttgttgtaaa ggtagaaagg tatgtttagg taggggttat 5580
   ttgttttgtt cgtttacgtt agatcgtagg tatttgttcg ttagattttt tcggtttgtt 5640
   tagagggatt ttgatagtcg agggtaatgt atttggtagc ggagtattgc gtttgcgata 5700
   gtttattttt tttttggaat tattttttat aagtgtgatt agtttaagtt aggataggta 5760
   tagggggatt taggattgtt atttaggggt agtggtttgg aatttaggtt taggtgatat 5820
   gggagttaat tttttgtttg gagattttag gtgggtgttt gtagaggata ttttatttga 5880
   ggatttatgg aaggggatat tttttatttt ttatttggtt tttgggttgt ttttttttga 5940
   tatttttttg ttattttagg tagttttgat agttttatgt gattgggatt tagaggtggt 6000
   ttttgttttc gttttagtgt ttgtttatag gtagtgttgc gtgggttttg aagtttgttt 6060
   aagatggatt tgataagggg agtttagtat atattagtgt aggttcggtt agggggtgat 6120
   gatggtagtt tttgggtaga tttagttggt tttagggtag gaggggtttt agagagaggt 6180
   tttaggtttt taggggtgtg aatttttggg gtgaggcggg aagtgacgcg ttgggttttt 6240
   gcgtttttgt ttgttggagt tattattttg gaaatatttt agttttaggc ggttgttagg 6300
   gatttatggg gattagtggg tgttaatttt tgggtgaagt tttcggggtt tgtatatagt 6360
   atatagcgtt gtggttttat ggtttatttt gttgtgtgaa gtggtttagt tttcgggaga 6420
   gtttttagtt aggtaggttt gacggtaatt gttaggttat ggtgatttgg aatcgttgat 6480
   taaagggagt tagttttggt gggtggaggg atttggtggg tagttatcgt gatgttaggt 6540
   atggggatcg tgtgttattt aggtttgtgg gtgtcgagtt tttgtgggag tagtgaagtg 6600
   ttttcgtggt cgatgggttg gttatttttg tttttttttt gatgtgatag ttgtgtttgg 6660
   gtttcgggaa atttgttagg aggtttggta ttggtttgtt ttattggagc ggttttgttg 6720
   agttgtgagt tgttcgtttt tttgaggttt ttagtttatt ttaatttttt atgtggattt 6780
   ggttatttat tttagaagcg tttgtcgggc gttgttgagt gttaggtttt gtttgttttt 6840
   ttttttgtac gttggaggtt ttgggtatta gttttgtagg gtggtttggt tttgggtttt 6900
   tagggtagag tatggtattt tttgttttta gtttagtttt tttttgtttt atttttttga 6960
   ttttattttg ggtatgggtt tatttgtagt ttttgtgaag ggagatattg agttgttgtt 7020
   ggtagaggag gggcggtttt tgggtattgt ttgttaatgg tttttcggta gttttgtgat 7080
   agttgagttt tgtttttagt ttaatttgat ttggtttttt aaagtatgat tgttggtagt 7140
   ttgaaaattt ttaggatagg aaataggagt ggcgtaggga atgtaagatg attttagttt 7200
   taagttattg ggcggttttt agatttaggt tgtttgttgt ttttggtagg aatttaagtc 7260
   gttggttatt tttagagcgg tgttggttcg gggtttttag tggtttttgt gtttgggtga 7320
   gaggaatttt ggatggttat tttgttttga gtgtgtgggt ttttagaagt gttgggttag 7380
   ggggtacgga gggttagaaa gttttttttg gagcgttgga ttttttcgtt gatttttatt 7440
   ttatttcggt ttggggtttt agagaagggg tttaggtagg agtgttattt ttttttaatg 7500
   gggatgtggt tttagttttt gtttaggtgg gtgtattgtt tttttttcgg ttttttttag 7560
   gttgtttaaa tgatttgtga cgaggtttgc gttatggtcg gggttttttg ttttttttag 7620
   cgttgtattt gggaattagg ggttgggttt cggaagtcga ggatgttttt gaatgttatg 7680
   tttgggtatt tgttttggtt aggcgaggcg tgttggggta gcggttgaga tgggagtagt 7740
   agtattgttt gttaggtttg agatgggttt cgggtgagtg tggttattag gaagggttgg 7800
   gtagagtagg cgggtgggtc gtttttgggg ttttgtttcg tagattaatt tgattcgtat 7860
   tttggggtcg gggttttttt ttatagtttt tttatgaatt atggtttttg tttttatttt 7920
   aaattagatt ttagaagagg aataatgttt atgtaattta agttagttgg aagtgggtta 7980
   tatttaattt ttgttatttt ttagtttgtt aaggatttga ttaatttaat ttagagggat 8040
   ttggagggtt gtttgtaaaa tttattaata atagggaatt ttagttttgt agttataggg 8100
   tttatagagg tgtcgggttg tcgggttatt gttgggaggt gagtcggtgg cgtagatatt 8160
   atggaggtat cggtagtgga agagttgtaa gtgtatatag aggtcggtat cgaggggcgg 8220
   ggaggaggag gtggtggatt tttcgttagt gttggtatta tgttgttttt agtattagag 8280
   atttaaggtg tggttgattt tggttggggc gatgagttat tggatagtag agtttttttg 8340
   gtagttgggg gttaacgttt taattgtcgg gattttgtat tatgtcgttt tttttttttg 8400
   ttttgtttgg tttggtttgg tttggttttt gagacggagt tttattttgt tatttaggtt 8460
   ggagtgtggt ggtttaattt tggtttattg taatttttgt tttttgggtt taagtaattt 8520
   ttttgtttta gttttttgaa tagttgggat tataggtacg cgttattatg tttggttaat 8580
   ttttgtattt ttagtagaga tggggtttcg ttatgttggt cgggttggtt ttaaattttt 8640
   gattttaggt gatttatttg tttcggtttt ttaaagtgtt gggattatag gtatgagtta 8700
   tcgcgtttag tttgtattta tgtttttttt tttttttttt tgagatggag tttcgttttt 8760
   gttgtttagg ttggagtgta aaggcgcgat tttagtttat tgtaattttt atttttcggg 8820
   tttaagcgat ttttttgttt tagtttttta agtagttggg attataggtt tgcgttatta 8880
   tgttcggtta atttgtattt tttagtatag acggggtgtt tttatgttgg ttaggttggt 8940
   ttcgaatttt cgattttagg tgattcgttc gttttggttt tttaaagtgt tgggattata 9000
   ggcgtgagtt atcgtattcg gtttatgttt tttattatta ttatttaatt ttttaaggaa 9060
   agtaatttaa attgtatatt gtattttttt tttttttttt gttttttgtt gttagttttt 9120
   tgtattattt attgttaaat aatatgaata ggaaattgaa aggaaaattt taataattat 9180
   tggttgtagt attttttttg tgttttattt agtgtttgtt tttttaggaa tatgatatag 9240
   ggtttgattt aattatgaaa ggcgtatatt tttagagttt tatatttttt atagaatgaa 9300
   tgttgaattt tgtttttttt atttgaaatt agattttagg tatggatttt atgattatta 9360
   ttataatggt tttttagagt ggatattgta ttttgtaggg aattattttt tttttagttg 9420
   ttggtgtgga cgtttttttt tgatggtgtg ggtttgggtt tttgaagggt ggtgttttga 9480
   gtaaagatgt tatagtagtt atgtttatag ttttgggttt ggggttaggt ggagattggt 9540
   tttaatttta gagtttgttg ttttaaaata aatttttttt tttttttttt tgagatggag 9600
   tttcgttttg ttgtttaggt tggagtgtag tggcgcgatt ttcgtttatt gtaagtttcg 9660
   tttttcgggt ttatattatt tttttgtttt agtatttcgt atagttggga ttataggtat 9720
   tcgtttttac gttcggttaa ttttttgtat ttttttagta gaggcggggt tttattatgt 9780
   tagttaggat ggttttaatt ttttgatttc gtgatttatt cgttttagtt tttaaagtgt 9840
   tgggataaaa taaattttta gggtcgggag cggtggttta tatttgtaat tttagtattt 9900
   tgagaggtcg aggcgggtag attataaggt tagaagatcg agattatggt gaaatttcgt 9960
   ttttattaaa aatataaaaa attagttggg tgtagtggcg ggcgtttgta gttttagtta 10020
   ttcgggaggt tgaggtagga gaatggtatg aattcggaag gcggagtttg tagtgagtcg 10080
   agattgcgtt agtgtatttt agtttgggcg atagagtgag attttgtttt ataaataaat 10140
   aaataaataa ataaataaat aaatttttat atttatttgt attatttatt attattatta 10200
   ttaaataggg ttttgttgtg ttgtttaggt tggagtgtag tggcgtgatt atgatttttt 10260
   agtttcgatt ttttagtttt aattggtttt ttttttttag ttttttgagt agttgggatt 10320
   ataggtgtgc gttgtatgtt tggttaattt tttttttttt tttttttttg agatagagtt 10380
   ttattttgtt gtttaggttg gagtgtcgtg gtgtaatttc ggtttattgt aattttcggt 10440
   ttttggatta aagtaatttt tttgttttag ttttttgaat agttgcgatt ataggtatgt 10500
   gtcgttatat ttggttaatt tttgtatttt tagtagggac ggggttttat tatgttggtt 10560
   aggttggttt taaatttttg atttcgtgat ttatttgttt tggtttttta aagtgttggg 10620
   attataggtg ggagttatcg cgtttggtat gtttggttaa tttttaaaaa atttttgtag 10680
   tagtcgggcg cggtggttta tgtttgtaat tttagtattt tgggaggtta aggaggtcgg 10740
   gagttcgaga ttagtttgat taatatggag aaattttgtt tttattaaaa ttataaaatt 10800
   agttgggtat ggtggtatat gtttgtaatt ttagttattt aggaggttga ggtaggagaa 10860
   ttgtttgaat ttaggaggcg ggggttgtag tgagttgaga tcgtgttacg gtattttagt 10920
   ttgggtaaag agagtgaaat ttcgttttaa aaaatttttg tttttgtttt tgtagcgata 10980
   gggttttgtt ttgttgttgt ttaggttggt attgaatttt tagttttaag cgattttttt 11040
   gttttagttt tttaaagcgt tgggagtata ggcgtgagtt attgtgtttg gtttttattt 11100
   gttttggttg tttttttttg ttttaggtaa ttatatattt ttttaggtaa ttaagaaaag 11160
   attgcgtatg atgagttttt tttttggtat ttttgggatt gtttttaaaa ttagataagt 11220
   gtttttattt tgatatttta ttaagatttt aatgaataga atgtcgtttg tttttagttt 11280
   gttggttttt ttgcgaaggt ttagggtttt tttgtttgaa aagttatttt tttgtcgagg 11340
   tttttgattt ttttttatta cggtagttat gtcgagggtt tatagagtta tttttttttt 11400
   attttttttt cggtttttta agacgtattt gtattttcgg agatgttttt ttgttttttt 11460
   gggttatttt ttttggtcgt tatttatttt gttgatttat tatttgtgta atagtttttt 11520
   ttggaatttt taggtttttg ttatttattt tttgtagttt tcgttttgtt ttttttgttt 11580
   tagttttttt ttttattatt tttttatttg ttggttttta tttttgttta tggtatattt 11640
   ttgtgttata ttttgatggt tggataaagg ttgagtgaaa cgtatttatt tttttggtga 11700
   ttggggtgtt tattaattag aagggattta aggttggcgt agtttttttt cgtattttgg 11760
   ggtatcgttt tgtggttttt ttttgttgga gtttgtagtt ttttgttttt ttttttttaa 11820
   attttttgag atggagtttc gttttgtcgt ttaggttgga gtgtagtggt gagattttag 11880
   tttattgtaa ttttcgtttt tcgggtttaa ttaatttttt tgttttagtt ttttaagtag 11940
   ttgggattat aggcgttcgt tattatgttt ggttaatttt ggtattttta gtagagacgg 12000
   ggttttttta tattggtcgg gttggtttcg aatttttgat tttaggtgat aggttcgcgt 12060
   cggtttttta aagtgttgag attacgggta tgagttatta ggttcggtcg ttgagattat 12120
   ttttataagt tattaggttt ggttgtagtt ggtagttttt gattattggt ttcgttttac 12180
   gtgatttgtg attttttttt tggttttttg gttttgtttt gtttatttgt ttattttttt 12240
   tatgtatgcg gtttgtttat agggtatgta atggggttta attaggaaat ggaaatcgtt 12300
   tgagtattta tttatttatt tatttattta tttatttatt tatttttttt aagataaggt 12360
   tttattttgt tacgtaagtt gaagtgtagt ggtgtgatta tagtttatgg tagttttgaa 12420
   tttttgagtt taagcgattt ttttttttta gttttttaag tagttcggat tataggtgta 12480
   tattatatgt ttgtttaatt ttttttttta tttttgtaga gatagggttt tgttttgttg 12540
   tttaggttgg ttttgaattt ttaattttaa gtaatttttt tgttttagtt ttttaaagta 12600
   ttggagttat aggtatgtgt tattacgttt tgttaatttt tgtatttttt gtgtgtgtgt 12660
   aaagataagg ttttattatg ttgtttaggt tggttttgta tttttgggtt tatgtgattt 12720
   ttttgtttgg gttttttaaa gtgttgggat tataggcggg agttatagtg tttggtttat 12780
   ttgagtattt agaatagaag gatttaaagg cggggaatta gttatataga tgatgggaga 12840
   ggtgaaggtt agatagggaa tagagaggta attagtagga aattaaagtg gtgtgggttt 12900
   atatagtttt ttaaaggtat ggatgtaatg atttatttag aatgagaaaa taaataatag 12960
   taaattataa attataagta gttgttaaat attataaata ttattatatg tagaagagta 13020
   ttagttaatt gttagttata tttttaaagt tttatttttt ttatattttt ggtattattt 13080
   ttttaatatg atgattttgt aatatcgttt tttatttcga gaatagaaaa ttgattaggt 13140
   tggatatagt ggttcgtgtt tgtaatttta gtattttggg aggttaaggt aggtggatta 13200
   tttgaggtta ggagtttaag atcggttcgg gtagcgtggc gaaatttcgt ttttattaaa 13260
   aatataaaaa ttagtagggt atggtggtat atatttgtag ttttagttat ttaggaggtt 13320
   gaggtgggag gattgtttga gtttaggagg tcgaggttgt agtgagtcgt gattatgtta 13380
   ttgtatttta gtttgggtga gagagtgaga ttatgtttta aaaaaaaaaa aaaaataata 13440
   aaaaaaaata taggttgggc gtggtggttt acgtttgtaa ttttagtatt gtgggaggtt 13500
   gagatgggcg gattatttga ggttaggagt ttgaaattag tttggttaat atggtgaaat 13560
   tttattttta ttaaaaatat aaaaaattag ttaggtgtga tgacgtacgt ttgtaatttt 13620
   agttatttgg gaggttgagg taggagaatg gtttgaattt aggaggtaga ggttgtagtg 13680
   ggttaagatc gtgttattgt attttagttt gggtaataga gtgagatttt attttaaaaa 13740
   taaataaata aataaagcga atgaattttt ttagttgatt aaattttgtt tttatttttg 13800
   gtagttggga tgtatataaa agtggcggtt aatatggagt tggtggtgtt gttataattg 13860
   ttttgtttaa gatatatagg aattttggta aattttgttt tttttttttt ttttaaatgt 13920
   gatttttatt aagaaagaag taggaaaatg ggggagattt ttaattgtat atgaggtatt 13980
   tgtgtatgta tttttgatta gagagatttt tttgttttga ttttatattg aaggaattga 14040
   attttttgtt tataatttta tttttggtga tgggagtaat tttttataga tttgtttttg 14100
   gattcgtgta ttttaaattt tgtttttttt tattgcgtac gttttttcgg cgttaggtat 14160
   cgtaggatat gttcgtgttt tgtgattttt gattttgcgc ggtaggtgga attggaacgg 14220
   tgagtggagc gagtattgtt ggaattcgtt tttatatcgg gatagcgagt agtaattgaa 14280
   ttatatatgg aaataaatgg gaattatata aatataattc gattaaagtt aaattaaata 14340
   tttttttagt ttaatttttt tttagtagga gtttttacgt ggtagtaagt tttcgttttt 14400
   attttatata agagaaaatg tgacgggtag gaatttaaga ttggaaatga acggggacgt 14460
   taattgattg tagggaaaat attttttatt ttgtaaattt tataaaaaat gtttgatttt 14520
   gtggatatgt ggttagggtt ttttttagtg ttttgagagg gaattatgta ggtagaggga 14580
   gggtttttag tattgtgggg gtgatttgga gattggtagt agggtgttat tattattttt 14640
   aggtggggat gagagggagg aagttttgtg attagagttt aggggttaga gttatttgtt 14700
   gggagtttaa ataaaggtgg gattttggta ggagttggaa ttatatagag gattattttt 14760
   tttgttggag aagttgtttg aggtagagga ggtggagagt tttcgcgttt ttttttgttt 14820
   ttatttttta ggtttttttt aagtttttcg ttggttgaat ttagtttaaa gttagtaggt 14880
   ttggggattt ggatatttga gttagtagag gtcggcgttt tttttatata aaggagggta 14940
   taagaagtgg gaggtacgga gggggattag gttaggattt atataggatg tgtaaatagg 15000
   agtttttttt tttttttatt ttaaatggta tttgtagtcg gttttatttt tttgttgtta 15060
   gataggaaga ggaattttgt tttcggtatt agttgttttt atcgtttttt attttacgga 15120
   ggtttttttt ttttttggtt tttgtttgta attattggcg acgttgatgg attatatttt 15180
   ttggggagtt tggttatgaa tatggttttt tatttgttgt ggattaaatt gtgttttttt 15240
   ttttgaattt ttgcgttgaa gttttaattt ttaatgtagt tgtaattgaa gatagggttt 15300
   ttagggaggt aattaaggtt aaatgaagtt atagggtagg attttaattt aataggattg 15360
   atgtttttat aagaagaggg agagatatta gagatttttt tttttttttt atttgtaaat 15420
   agaggaaaaa tgacggagga tatagtaggg aggtgggtat aagttaggaa gaggggtttc 15480
   gtcgggaatt aattttgacg gtattttgat tcgggatttt gggtttttta aattgagaaa 15540
   tatatgtttg ttgtttaagt tatttttttt atagtaattt ggatggtagt ttgagtttat 15600
   taaggtatcg tttgtattag ttagggtttt ttagggagat agttaatagg agatggatgg 15660
   atggatggat ggttagatag atagataagc ggatagagag agagagagat aggtagatat 15720
   agatagatag gtagataaat ggttagatag ataagtagat agagagatag agatagatag 15780
   atataaatag gtaaatagat aggtagataa atggatagat agatgataga tagttaggta 15840
   ggtagataga tatagatagg tagatagata gatagataga tgataggttg ggtttagtga 15900
   tttttatttg taataattag tatattggta ggttgaggtc ggtggattat ttgaggttag 15960
   gagtttgaga ttagtttggt taatagggtg aaattttatt tttattaaaa atagaaaaat 16020
   tagttgggcg tggtggtgta tgtttgtaat tttagttatt cgggaggttg aggtacgaga 16080
   attatttgaa tttgggaggt ggaggttgta gtgagttgag attacgttat tgtattttag 16140
   tttaggcgat aaagtgagat tttattttaa aaagaaaaaa aagatagata gatgaatgga 16200
   tagatagtta gatagatgat agatggatga taggtagata gataattgat aaatagatat 16260
   aaatgatcga tggataggta gatatagata tattgattag gtagatgata gatggataga 16320
   tagaagatag atggtggatg ggtgggtaga taggtagata gatagttaga aagatagatg 16380
   atagatagat aggaaggaat ttattagggg aattggttta tattattgtg gaggttaaga 16440
   tgtattatag aaggtcgttc gtaagttgga ggtttaggaa agttagtatc gtggtttagt 16500
   ttaagtttgt aggttttagg attgggaagt tgttgatgta atttttagtt taaggttaaa 16560
   ggtttgagag tttggggaat tgttggagaa ttgggagttt taagtttaag ggtaggagag 16620
   gaaggtattt tatttttagg agaaagaggg aatttatttt ttttttgttt ttttgttgtt 16680
   tggtaatgga atggtgttat ttatattgag ggaggatttt tattatttag tttattaatt 16740
   tatatgttta ttttttttag aaatattttt atagatatat ttagaagcga tgcgttatta 16800
   gttatttttt aatttagtta aattgatatt tgtcgttagt tattatatag gtttagaaga 16860
   atcgtggttg tatggtgatg gtttttattt cgtttaaaga agttgaagat gaattgttgg 16920
   tataagttat cgttttttta ggtttttttt agtcgtttat ttttttaaaa atgtatttat 16980
   tgagtatttg ttgtatgtta ggtgttgtga taagtattgg ggtatagtag tgagtaaata 17040
   aggatttttg ttttttggag tttatatgta gtagaaggaa atagaattaa tgaatttatt 17100
   agttttagaa attattaaaa taggattaag agttgtggtg agaattaaga taaggtgatt 17160
   gtatagaaag tgattgatgg ttgttttgga atgagtggtg aggggttata gagatgatga 17220
   tagttgagtt gagattggga agataggaag gagttagtag tgtaaggatt aggttggaag 17280
   taagtttggt gtattttagg aatagtaagg aggttagagg ggttgtagta gagtaggcga 17340
   ggaggaggag ggagacgtag gttaaagagg agtttggggt tagatggagg attttttttt 17400
   taaagtttag ttattttttt tttaaaggag aatgtgttat ttttaaaagt tatatatgtt 17460
   agtatagtgt ttggtttatt atatgttttt agtaggcggt gagtatggtt gtttttgtta 17520
   ttattatatt ataattatta ggaataatgg agttattagg ttttgtattt atagtaatgt 17580
   gagggagtta gtaagttttt ttggggaatt tatttttttt tattaagttt ataagagttt 17640
   gataattagg ttaatgtagt gagagttata gttgtttagt gtattggatt ttttagttac 17700
   gggttaggat atttttattt tagtagaaat ttttaagggg attggggagt gtgttgtgga 17760
   tgtttttggg ttatttagat tttttgggga gtttttttta tcgtagttgt tgtagggagt 17820
   ttagttgtag tgatgaattg cggtttagtt ttgtttatgg gaaaatttta tatgggagat 17880
   gggacggttt tttgttggtt ttttcggttt taggttgttt ttataagtgt agggattttt 17940
   atttgggtgt gtgaatgggg tttaggatgt tttaagggta tgtggagatg ggggttagtt 18000
   gttttttttg atgtttttga attttttttt tttgtttttt tgtattttat tgttttttgg 18060
   ttttttttat tttgggtgtt ttggaataat atgtaattta atatataggt tgattttatt 18120
   attttgtttt gttttatttt attttatttt ggaaataaag gttttgttat ttagtttgga 18180
   gtgtagcggt gtaattgtaa tttattgtag ttttgaattt ttgggtttaa gtgatttttt 18240
   cgttttagtt ttttaaagtg ttgggattat aggtatgagt tgttgtgttt ggtttaggtt 18300
   ggttttaaaa gttttttatt ttttttgtat tttgatgata tttatttttt ggagtaggtt 18360
   tttagttatt ggtttcggat attttgtagt tattattggt tagttgtggt atgtgaagtt 18420
   gaggtttagg agaagggtag ttttgtttaa ggttatacgg tttagtgtta atatttagat 18480
   tttagtgttt ttattttttt tttggtcgtt tttattttta gtgtattttt ggaggtttaa 18540
   gggagttaaa ggttaggttt tggttagtta agaatttaaa aaagttgttt ttgtgttttt 18600
   ttgttaattt attattttat tatttttttc gataatgaat ttagtttgaa ttttagataa 18660
   ataaggatta atttttagta taagtatatt ttataataga tttgggatat atttatgttt 18720
   aaaaatgatt tattgtttgt tggttttagt agtttatgtt tataatttta gtgttttggg 18780
   aggttatagt tggagaattg tttgagttta ggagtttaag gttatagtga gttatgatta 18840
   tgttattgag ttttagtttg ggtgaagtag taagattttt atttttttaa aaaattgttg 18900
   attgtttatt tgaaatttag gtttaattga gtattttgta ttttatatga gaattttata 18960
   taggtgagat ttagagtttt aatgtttatt ggtaagtttt tgtagggagg ttttgtgggt 19020
   ttttggttgg tattgatgtt ttgtgatgag tagggttatt tggtaaggtg gacgtttgtt 19080
   gtttatgtag gtagagtaag gttttgggta ggtatgagtt gattttattt tttttagttt 19140
   gggagttaga tattgttggt agtttgtcgt taagagaggg gttggttatt aagagagggg 19200
   cggtttttat gggttttttt tggtttttgg ttttttttta gttttttttt tattttaaat 19260
   agaggttaac ggtatcgtat tagaattagt gtgttgtttt ttttggttgg tattttgtag 19320
   ttgggtagat tgtttttttt ttataggagt tggtggtgtt tttttcgttt tatagggagg 19380
   atggttgaga tttttgattt tgatataggg tgttgttttt tttttgtttt gagatagagt 19440
   tttggttttt ttatttaggt tggagtgtag tggtatgatg ttggtttatt gtagttttta 19500
   ttttttgggt tttagtaatt tttttgtttt agttttttga gtagttgaga ttataggcgt 19560
   gtattattat tgagaataat gttttttata gtgtttattt ttttgtagga gtaagttttt 19620
   aaggtaagga ggttgttttt tttatggatg aataaattga ttttgattta tagttttacg 19680
   tgttagtttt gagggtgggg aaaggttgtt ttgtgtttag gtagggggtg ggggtaaggt 19740
   taggagggtt tggtgtttag tgtatttttt taggatataa ttttaggtag tagaggttaa 19800
   ggatagaggg gaggagaaga gggaggtggt tattattttt ttattttagt tttttttgga 19860
   gataagtttg agtattttag ttgtttatta agtttggaga taagtttgga atttaaattt 19920
   ttgattttga tttagttttt tatagttttt gtgagtaaat tgagttttag aaaaagggag 19980
   tgaagtttgt gtattttgga cgtttttatt ttaggtttaa atcgttttta ttttaggttt 20040
   aaatcgtttt tattttagtt taaatcgttt ttattttagg tttaaagcgt ttttatttta 20100
   gtttaaagcg tttttatttt aggtttaaag tgtttttatt ttaggtttaa agcgttttta 20160
   ttttaggttt aaatcgtttt tattttagtt taaatcgttt ttattttagt ttaaattatt 20220
   tttaatatag gtttaaatta tttttaattt aggtttaaag tatttttatt ttaggtttaa 20280
   agtattttta ttttaggttt aaagtgttcg taatttaggt ttaaagcgtt tttattttag 20340
   tttaaatcgt tttattttag gtttaaatcg tttttatttt aggtttaaat tatttttatt 20400
   ttaggtttaa atcgttttta ttttaggttt aaagtgggaa ggagttatag ggaaataaac 20460
   gggggaggta cggatgatta ggatgagggt ttttatgatt tatttttttt taggggttat 20520
   tttaagagtt ttttaaattt ttattatttt ttaatagaga tggggttttg ttgtgttgtt 20580
   taggttgatt tttaattttt agttttaagt tattttttta ttttagtttt ttaaattgtt 20640
   gaggttatag gtgtgagtta ttgtatttgg ttttttttag gagttttaag aaaaaaaatg 20700
   ggaatagatt tgtgtgtgtg tgtgtgtgtg tgtgtgtgtg tgtgtgtgtg tgtgttttga 20760
   gaaagagttt cgttttgtta tttaggttgg agtgtaatgg tacgatttcg gtttattgta 20820
   attttcgttt ttcgggttta agtgtttttt ttgttttagt tttttgagta gttgggatta 20880
   taaatatgta ggttgatttt gtatttttat tagagacggg gttttattat gttggttagg 20940
   ttgatttcga atttcggatt ttagatgatt tgtttgtttt ggttttttaa agtgttggga 21000
   ttataggttt gagttatcgt gttcggttag gaatagattt ttttttgtag atgatttttg 21060
   ttattattga aaatggatgt ttatgtataa ttgaattgtt ataacgtttt attttaaaaa 21120
   gttttttttt gagaaaatgt gatttttgat agaggagaga ttgtttaggt tttatttatt 21180
   gttagtataa aggtagagta ggggtgtggc ggtgttattt ttttttgaga tagttttatt 21240
   tttgttttgt ttaggggttt agaagtttgg tttattcgag tttatagagg ggtttggagt 21300
   ggttgtttta tattataggt ggggtttgta aggatttgaa atgatttttg agaaggcggg 21360
   tgtggtgggt gtattttaat tattaaagta ataataatga tatattaaat agtgtttgtt 21420
   tagattttga gtgtttgttt aaatattttt attttgtgat ttgttagatt ttagtataat 21480
   aatttaggta aaagaaaaaa tagttgggcg tggtggtttg tgtttgtaat tttagcgtga 21540
   ggttaaggta ggaggattgt ttgagattag gagtttgaga ttagtttggg ttatttgaga 21600
   ttttattttt gaaaataaat aaattaataa aagattttta tgttttatgt tttttttata 21660
   tttaggtttt ttaaattata tgtattgggg aaagtttatt aagagaagtt tttggtcggg 21720
   cgtggtggtt tacgtttgta attttagtat tttgggaggt taaggtggga ggattataag 21780
   gttaggagtt cgagattagt ttggttaata tggtgaaatt ttatttttat taaaaatata 21840
   aaaattagtc gggcgtggta gtgtatgttt gtaattttag ttacgtagga ggttgaggta 21900
   ggagaattat ttgaatttgg gaggtagagg ttgtagtgag ttaagttggt gttattgtat 21960
   tttagtttgg gcgatagaaa aagattttat ttttaaataa ataaataaag ggagagaagt 22020
   ttttgggttt ttagatatat gggttagtgt ttatttttgt aaagttgttg tgtttaaaga 22080
   ggggttgtat ttggtggaat tggggtaaaa gagtagggag ggtattgggt agttgatttg 22140
   gtttttatta ttggaaaggg gttgtgttgt tggtgttttg agttggtttt ataaggaaga 22200
   tattgtatag ggcgttttaa gtttgttttt tttgtttagg tttatttttt tttttttagg 22260
   attagggagt ttcgttcgtt ttttgttttc ggtatttgtt ttttggtttg tgagtattgt 22320
   attttttttt gtttggaacg tcggtttatg gtgggttttc ggatttttgt tttttgttgt 22380
   tttttgttat atttcgttaa agtgtgtttt atttatttat tgttattatt ttttgaaata 22440
   gagttttgtt ttgtcgttta ggttggagtg taatggtgtg atttcggttt attgtaattt 22500
   tcgttttttg ggtttaagcg atttttttgt tttggttttt cgagtagttg ggattatagg 22560
   cgaatgttat tacgttcggt taatttttgt atttttagta gagatggggt ttttttatgt 22620
   tggttaggtt ggtttcgaat tgatttcggg tgattggttt acgttagttt tttaaattgt 22680
   tgggattata ggcgtgagtt attatgtttg gttaattatt ataatattga tattataata 22740
   atattatagt tagataacgg tgattaacgt ttgtaattcg aatattttgg gaggttgagg 22800
   taggtagatt atttgaggtt agttcgagat tagtttggtt aacgtggtga aattttgttt 22860
   ttatcgaata tataaaaatt agtcggagcg tgatggtagg tgtttgtaat tttagttatt 22920
   tgggaggcgg aggttgcggt gaattgagat cgtgttattg tagtttaatt tgggtgatag 22980
   agtaagattt tattttaaaa gtagtaataa taataataat aataataata ttatttattg 23040
   agttttaatt ttgtgttagg tattgtgtta gtatttgcgt tatgtgattt tattgattcg 23100
   gaataattta tgagtaagta ttatttttgt ttttaaggaa gttgagattt aggaaatgga 23160
   agttattttt tagggtttat agtaagggtt tggcgaggtt tgcgtttaaa ttttgattat 23220
   ttgattttag gtttttttgt tttttttgtt ttttaaaaat ttaaatattt ttatttggga 23280
   tgattttaga tagttagatt ttaattgttt tttttttttt tgagtatgtt ttattttaaa 23340
   attatataaa gataatttat tttttaattg gagttacgga atcgttagat gttttggaga 23400
   tttttttagt tatttttttg ttggatttag atataggttt gaataattag gtttaaggag 23460
   ttgaattgga gtcgttcgtg gttagaggtt tttttttggg aatagtagtg gcggtagcgg 23520
   ttgcgttgag tttggtaatt tgtttaagag taggggatgt ttaggttgcg ttcgtttttt 23580
   ttttatgaac ggtttgtttt gcgttttttt tgtgttaagg gttgttaggt attagatgtt 23640
   tattttagga aattagaaaa ataaatagtg taaataaaat aaaaatttta tttaagaaat 23700
   ttgagataga tgttgttttt tttatcgttt ttttattttt atgttgggtt tggagagagt 23760
   gggttagtat gttttttacg gataagaggt tttggggtat ttaatttgga tggaattttt 23820
   tttatttttt ttttgtttgg tgttaggggg tagaaggtag gggtggtgtt tatttggttt 23880
   agggaggagg atagggtagt ttttgtcgtc ggtatttttt gtagagatgt tttagttaga 23940
   agtgatttta tttttaggaa cgtgtgtttt gttttttgtt gggcggtttt tttttatttt 24000
   ttattgtttt tatagtgggg tgtgttagat ttatgggtag agttttggtt tatttttagt 24060
   ttcggatagt agaatgtttt agtagtgtat tgatggtgtg tgcgtgggga tgtgtggaat 24120
   aggggatagt gatttcgtgt gcgttatatg tagtgcggtt tttagtcggg tgtgtgaagg 24180
   gggtgtttat ggttatcgtg gaagtgagtt tagttggggt tggagggttt aggtttattg 24240
   tatgttattt agtagttttg ggagtggggt ttattttttt tttcgttttt tttttttttt 24300
   ggttttgggt agtttatcgg agttgtattt tgggttattt tttttggttt taaggaggtt 24360
   gttatttttg ggaggtagat gttggttttt ttatttttgt tttttggaga tttgcgtcga 24420
   ggtttgtttt tttgtttgga gggattggtt aggaattttt gggttttttt ggtggggttt 24480
   ttgttgttta gtagtgagta gagtgagtgg gtattaagag gaggatgttg ggtgaggtta 24540
   cggatatggt tggggtaggt tgggggtgtt ggaggtgaat ttcgtagttg tgcgattttg 24600
   ggtagtttgt ttagttttgg gtttcgtttt tttttttatg aaatggggcg ggagtatttg 24660
   tttgttgggg ttgtgggttg ggtgaatcgg agttaaggta tagtttatag tttgtcgtta 24720
   gattttcgta agtagaagtg ggtgtttttg tttagcgttt ttcggttttt tttttaaggt 24780
   ttagttttgt ttttattttt tttatgaagt ttttttggtt attttaggag ttacgattta 24840
   tatttttttt ggagttttgt gttatacgta ttttttacgg tgttgttttt tttggaggat 24900
   gggtttaatt aaaattgggg tttatttggg ggtttttttt agtattagag aagtttaggt 24960
   ttagtagaga tgggtatttt gttaatatta gtttcgcgaa ggatatattg tgtttatttt 25020
   gttgttagtt gttgagtttg gtgttttgta gttatggaat atgtgtatag gtttgggttg 25080
   ttggatacga aggttagatg ggtagtttat tttgttttcg ttttgagttt ttagaaataa 25140
   gttggttgta tttattttta tttggttata gtttaggagt ggggttgtgg aatggatgga 25200
   aggaaggatg agtgggtggg tgggggggtg ggtgggtggg tggatggatg gatggatgat 25260
   gggtggatgg gttgatggat gggtggatta ggtggatagg tgaatggatt ggtgggtggg 25320
   tgggtgggtg aagggttgaa taggtgggtt gggtggatgg gtggatggat gtgtggaggg 25380
   gtggtggggt ggatggggtt tatgggtgaa tggatggatg gatgggtgaa tggagtaaat 25440
   gagtgggtgg atggatggat ggatgttgta agagtttttt tggagggttt tagggaggaa 25500
   ttggtatttt tgattagtag ttagtatggt gattagatat agggaggtgt ttgggtttgt 25560
   ttgatttttt attttattgt tggtgggatt gggttggtta tgtgtttttg aagttttttg 25620
   aagggtggtt gtatggtatt gtttagggga tatttttaga gtgtcgtgtt ttatagtaat 25680
   agggtggtcg tttgtgtttt tagggtcgtt ttttttggta gtttttcggg tttgtttggt 25740
   ttagtatttt agtaaatttt ttcgagttag gatttgtttg ttatgagttt ttttaataag 25800
   tagttttagg ttaaggtttg tgtgtgggta gagggagcgt atgtcgcggg aggttgtatt 25860
   aatttgaggt tttgtttttg tttttttttg gggtaaaagg taaaaatagt ttgttttatt 25920
   ttatttttaa atttagttcg gttgtattta gttttgggag tgaagggggt tgggttagga 25980
   ttgttcgtga gttgtagatt ttggtgtagg gaaattttga tttataagac gggtggatat 26040
   ggaggaagga ggagatttcg tttagtagag atacgtttgg gtgtagttta gtttggagtt 26100
   tgatggacgt gggtttattt gtgtgtaatt tttatttatt gtgtggttat agggaagtga 26160
   tattttgtgg agatttagat tttttatttg taaaataggg gtagtagggt ttgggggagg 26220
   atgtgtgtga aggtttggtt tagggttttt ttagtagtta gatagtagtg gttgttattt 26280
   ttttagcgtt tagggttatt tgggggagta gggtttttgt ttttagatgg gtttcgtttt 26340
   tggttttgga atttatagta ggagtaaagg cgaggatatt tttggtgtta attatttatt 26400
   cggagggaga gcgttagtgg ttttgtgtga tttttgtttt ttgtttttgt ttttaggagg 26460
   tacgcggatt tttagtggtc ggtttcggag gtttggtgat tttagtggtt taggtaggtg 26520
   gttcgtttcg ttttggtttt atttagttta tgggtgtgtt tgtgttgggg tttttttatt 26580
   ttggtttggg atttgttttt ttatttggtt tttttggatg agtggggtta tttgtttatt 26640
   ttttagggag acgtagtttt tgacggtttt ggatttaggt ggtttttagg tttgtcgacg 26700
   ttgttttgtt ggagatgatt tttgtggggg taggggtagt tatttttagg tttagggagt 26760
   aagttcgtgt gtgcgggata gaggagagaa taggagatag atagggttgg atgatttggt 26820
   ttgagatgtt gaggggatag atgtgttatg gataggtggt tttggtcgtg agtttttcgg 26880
   tgttggttta gtatttgtgg gttttttgta attttgtagg atggaagatg ttttaggata 26940
   gagtagatac gttcggtgtt atttaggagg aaataagtgg gtttttggta gtggttattg 27000
   tgttattttt tgtgtttttt ttatgtgttt ggaaattttt tatattaatt agtagggaag 27060
   ggaaatagat gtatttatta gagttttggg gttggtcgtt taggatacga gttatagttt 27120
   ataggtttga agggatttta ttaatattgg gtgtagtttg ggtaagggag tgggatagag 27180
   aggttagggt tttgttttta gtttttgttt tttagggttg agtttgcgcg ttattttttt 27240
   tttattgggt gtttttgatt ttttttgttt gattttagtg tggttatgtt agtgtttttg 27300
   tttttgggag tttacgttgt ttttggttgt tatttttagc ggttaggttg ggataggtgt 27360
   ttatttagtg gtttttttat ttggagagga tgatgggtta tagtaatgaa tggatttatt 27420
   ttttttagga gacgaggagt tttgagaaga gtagagatgg cgtggttatt tgtgtatatg 27480
   tgttgggggt taattgggtg ttagattttt ggttgttatt atggtggtag ataggtaggg 27540
   attgtgtttt tatgaattag ttattttaga ggagataaag ttattaaagg atacggattc 27600
   ggagttaaat ttatataaat agagcgtttg tggattaggg ttgtttttag aataaggtga 27660
   gatagtcgtt ttgttaggat aaggcgttta cgggggtaag ggtatgtgtt tatttggagt 27720
   cgttattttt atgttttaga ttagtttcgt ttaatagaat tttttgtgat ggtgggtttg 27780
   ttttatatat ttgtatttcg ttattttgta tggtagtcgt ttagtcgttt gggtttattg 27840
   tatatttaaa gtataaatgt ttacgtttat tttatttatt tatttatttg tatagaatta 27900
   tttatttatt ttaaatggat ttttatttat atttacgtgt ggttagtggt tatgaaattg 27960
   aatagtttta gattatattg gtatttgaga ttttagaatt ttagatttaa agtttgtttt 28020
   tgattttgag tttgtttttg tggggatagt atagacgttt tttaagtttg ttttttagag 28080
   gtggatgtag tgttcgtgga ttgtgtatta ttaggtttgg atagtggaga gggtggtttt 28140
   ttatagggag tgggagtgga ggggatgtcg tgtttatatt gagtgtatgt gaggtttttt 28200
   gtagtatagg atgggattgt atgtgggaag aggggggtga tgttttggtt ggaggttttt 28260
   gggtagttgt atgatttagc gttgtggtta agaggggttt cgtttatcgt agttatgttg 28320
   ggtattaatt attggatagt ttagtcgttg ttgtgttttt ggggagtttt ttttcggttt 28380
   cgtatttgat ttgttttttt attttttttt tttttattgg ttagtttttg gtggaatggg 28440
   tggggagggt tttttagttt ttgtgggggt tgtattttgg gaatttggta ttttgggttg 28500
   tatggatata gtttttttcg gcggttgttt ttttttgttt agattggttg gttttgtgtt 28560
   gttagtattt ggtagttacg attatagttt agtttggaag tttaaggtat aatttgttga 28620
   attttaaagt tgtaagttgg tgatttgggt ttgcgtggga ttgtttgttg atatacgggg 28680
   tttttttttt gtgggttttt gtgggttttg gttcgagata tttttttttt atttttttgg 28740
   tttttttttt tttttttgtt ttttttttgt taagattttg gtttaggtag gagtttattt 28800
   ttagacgttt tagtattttc ggttttttta tttgatattt tttagacgat tgtttgttat 28860
   tttatttttt tagtttagtt tttttgtaga tttatatttg gttaattttt tatgtttggg 28920
   atttttgttt tttttagaag gacgaaggtt tttttcgagg ttgtgtttgg gattgggtta 28980
   gggatgatat ttttggtaaa agtttaaatg atattatgat taacgttttg gttagtttat 29040
   atattttttt tttttttaaa aaaaaaatgg aatttttaga gttataaaat aaaaatgtaa 29100
   agaataaggt aataaaatat ttgtgtattt attatttcgt ttaagaagtg agatattgta 29160
   ggtgtagtcg tagttttggg tgattttttt tattttagtt ttatttttgt tgcggtgttc 29220
   gtttttttgg attgggattt ggtttcgggt aggtatattt gtgttgggta gtggtgtgtt 29280
   tgagaagttt aggttttttt tttttttttt ttgagataga gtttcgtttt atcgtttagg 29340
   ttggagtgta gtggtattat ttcggtttat tgtaattttt gtttttgggg tttaagcgat 29400
   ttttttgttt tagttttttt agtagttagg attataggta ttcgttatta tgtttggtta 29460
   attttttttg tatttttagt agatataggg ttttattatg ttggttaggt tggttttata 29520
   tttttgattt taggtgattt atttgttttg gttttttaaa gtgttgggat tataggtgtg 29580
   agttatcgta ttcggtttgg tttttttttg tatacggtta ttagtatcgt gtgattgagt 29640
   agtttaggtt tgtttttttt tgaatgtcgt tattagtatt atatttttgg tgttttattg 29700
   gagttagtgg ttgttttgtt tgtgtttata acgtgacggc gtggagattt attcgtgtag 29760
   atttacgaag ttttggttta tttattttgg tagttagcga gtgttttttt gtgtggggag 29820
   gtagagtttg ttcgtttcgt ggtttattgg tgatatcgtc gaaggttttt tttgttatta 29880
   gtgttgtcgg gaacgtgtgt ttttttaggt ggatttggtt gggttgtgga gatgcgtttt 29940
   ttagtttttt tagatgttat tattacgttt tttcgagtgg ttttttggta ggttttttta 30000
   tgttcgttta ttttttgtgt tttaggtagt cgtttttttg attgagcgtt tagcggattt 30060
   ttgttttgtt ttttacggtg tacggagtat tttattttag tggtgtttga gttttttttt 30120
   ttttggtttt tagatttcgg ttgggatttt tggagttaat ttttttgttt gatatttcgt 30180
   gtcgttaggt tttgtttttt tattttttaa tatttttggg gtgtgtggtt ttttttttag 30240
   tgtttttatt attttttttg ttttaggttg aatcgtgttt tttataaatt tatgtttatt 30300
   aaaaatttta gaatgtgatt tgggagttgg agatggggtt tttgtagatg taagtagtta 30360
   aggattttga gatgaggtta ttttggatta gggtgggttt taaatttaat aattggtgtt 30420
   tttaaaagaa gtagagaggt tatagatata tcggggagag ttacgtgaag gtaggagtag 30480
   atgatggagt gacgtagttt taggttaggg agcgtttggg aagaggtaag gaaggatttt 30540
   ttttttattt tattttagag tttttttagg gagtgtggtt ttgttgatat tttgattttg 30600
   gatttttggt ttttagtatg ggagagaatt cgtttttatt tttttaagtt ttatagttgt 30660
   ggtttattat ggtagttata ggatattgat gtatttttta tttggtcgtt gtggatggac 30720
   gatttcgttc gtaggaagtt ttttttaagt attgtaggtg tttttgtttt tataggggac 30780
   gtttgtataa gtaggtgtga aaaaagggtt ttgtgggggt aaggtttggg ttggtgttcg 30840
   ttgttttttt ttattttatg ttagttttta tgtttttttc ggtaggagtg tttggtttta 30900
   tttggtgaat tagagttaaa tttttttgat aattggttgt tatttattta attgttatta 30960
   tttttaggag atagaaaatg ttagaagggt ggggtttgtg tagtgttttt tcgttatttt 31020
   tttagggtat cgtgttacgt tgttttaatg tatagagtag ttgtttaaag tgtgtcggac 31080
   gaataggtgt tttatgttta gttatgtaga taaaagtgtg ttacgtggag ggagttggtc 31140
   gtggaagaga ttattagtat tttggttgtt cgaggatttt gtttttgggt ttttgtaggt 31200
   atttttagtt tattattttt ttttttattt atagttattt attttttttt tatttttttg 31260
   atattagttt tgttatttat ttaggtttgt aagtaggaaa ttttagagtt gattttgatt 31320
   tttgtttttt tagtattttt aggttttaat aggttgttaa gattttatta attcgttttt 31380
   tgtatattga tttttggata ggttgtttcg gttacggtta tgttttggtt gggtttttat 31440
   tatttttttt tggattagta ggtgttattt tttttatttt tgttttgttg tggttttttt 31500
   ttttttacgg ttaatattta gaggacgatt attatttttt tttttgtgtg ttggggatcg 31560
   ggtgatagga tttttttatt cgattatttt tattagattt gggtcgggtt tttttattta 31620
   tagagttttt agttttcgtt tttaaagttt cgagggtgga ttcgtgttgt cgtttagttt 31680
   ggttttgata tcgtttttta gtttggagtt ttttgttttt gcgtttttgg ttttgagtgt 31740
   tcgggttttt tttggatggg ttgttatttg ttttttggaa agtagggttt ttatgttttt 31800
   ggatttttta gttgatttag tgattttggg ttattttgtt atattttttt tattagtttg 31860
   gttttggtta tttttgtgtt ttttatagtt tcggattaga gttacggcgg gatgtttttt 31920
   tggtgcgtgg taggtagtta ggaaatattg ttggattgat tttgatagga gatgtttggg 31980
   agaattgttt aaaaaattag tttaaaatgt aaatattggt ttttttttgc gttggttaaa 32040
   gagtagtatt ttttcgtttg tttttcgggt aagaaattga tggcgaatgt aatgttttta 32100
   gggaggagaa gaaggtgata aaaagggttt gtataacggg gtatacgtta ggtatttgtg 32160
   attatttaag tttaaattat aatataaatg cgaaattttt tgttttggta gtttttttat 32220
   attgtaagcg tttattgtta tatgtggttg ttatattgat tacggtagat atagaatatt 32280
   gatattattg cggagagttt tatggggggg cgttggttta gattattttt acgttggatt 32340
   tataaattta ttgtttattt agtgaacggt tattgggtat ttattgagta agtaggatga 32400
   aagatattcg ttttcggggg agtttttcgt tggttgtaga gaagtttaaa tttaggggat 32460
   ttggggtaaa ttttgtaagg gtttttggtt tttgtatttt tagatttttt tcgttttgta 32520
   ggggtagtcg gggttttttt ttaggaatgg agcgttagtt ttagtttttg ttaggttata 32580
   gtttagatgg ttggttgagt acgagtatgt gagagtggaa ttgggtttaa tcgggttgtt 32640
   ttttttcgtt gttttggttt ttttttacgt tgatttggaa ggatttttaa agtaataggt 32700
   ttgtttaggc ggtttgtatt agtaaatttt gtagcggttt taagggtaga gatagttgga 32760
   ttgtatttat ttaggaatag ttaaagtttg aggtataata aaaagtggag aaagatttta 32820
   gagaggaatg tagttggttt tagtttggga attcgttttg tttgggaggt tgtaggcggg 32880
   ttaggttggg gttagtttgg agggttttgg ttggttttgt tggtaggtat agtagtaggg 32940
   ttagtaggag ggagtttagg acggagtttt tttattttta atttagggaa gtaaatttta 33000
   gtggttgagt ttgaggatga gtaggaaatg aatggaaaag tttttttttt tttttttttt 33060
   tttttaggta gggttttttt ttgtttttta ggttggagtg tagtggtata attatagttt 33120
   attgtagttt taatttgtta ggtttaaggg atttttttgt tttagttttt taagtagttg 33180
   ggattatagt tgtgcgttat tatgattggt taatttttaa aagaaatgtt tttaggttag 33240
   gtatggtgat ttatgtttgt aattttagta tttttggagt cgaggtggga ggattatttg 33300
   agtttaggtg ttttagatta gtttgggtaa tatgacgaga tttcgttttg attaaaaata 33360
   taaaaagtta gttgggcgtg gtggtatagt tttgtagttt tagttattcg ggaagttgag 33420
   gttgtagtga gttgtgatta cgttattgtt ttttagtttg ggtaaaggga gtaaggtttt 33480
   gttttaaaaa aaaaaatttt tttttttttt gaggtggagt tttgttttgt tgtttaggtt 33540
   ggagtgtagt ggtatgattt tggtttattg taatttttgt tttttaggtt taagtgattt 33600
   ttttgtttta gttttttaag tagttgggat tataggtatg agttattgtg tttggatttt 33660
   tttttttttt tttttttttt gtagagatgg gattttttta tgttgtttag gttggttttg 33720
   aatttttggt tttaagtgat ttttttattt tggtttttta aagtgttgga attataggtg 33780
   tgagttatta tatttagtag gaaaagttat tttttaaagc gagaggttgt gcgtttttta 33840
   tagtttggcg tttattagta tggaggattt attggaacgt gtgttaggtg aggggatatg 33900
   gaagatggta gtaaattcga gtttagagta ttagaaagta tgtttttagt taaagatgcg 33960
   ttttttttat tttcgcgtag tatttttttt tagtgtgtat ttttggagta aggtggggtc 34020
   gattttacga agattttttt tcgaaggttt ttgttgttta tattagtgtt atttggatcg 34080
   tttgtttttt tttttggatg gttggattag ttttagggtt ttttggtttg gtgtggggag 34140
   tatagtttat tttggggaat gtttggtatg tgtttttttt ttattgggta ttaggggatg 34200
   aagttgtgtt tcgtggaagg ggatagaggc gttggtgttt aggatggtgt atttggggcg 34260
   gttgaattgg atttggtttt tattgggttt ttgtggagtt tagattgttg tgagaagggg 34320
   tggggaaagg taggtgattc gtatttttta tttttgtttt atatgggaat aggggtttat 34380
   tttgtatata tatttagttt ggagtatgaa tttttatgaa gaggaaaatt tttattaggt 34440
   tttgatgtta ggattgttcg tgagggtcgg gtcgtgtttt ggggtaagga taggagtgga 34500
   gggggtaatt agggaaggtt ttttaatgga ggaggtattt gatagtattt tgaaaaagaa 34560
   gtagttttta ggttaagtta gagattgaag aatgattatt taattttttt tttttttttt 34620
   tgagatggaa tttcgcgttg tcgtttaggt tggagtgtag tggcgtgatt taggtttatt 34680
   gtaattttta ttttttaggt ttaagtaatt tttttgtttt agttttttga atagttggga 34740
   ttataggtat gcgttattat gtttagttaa tttttgtatt tttagtagag acggggtttt 34800
   attatgttgg ttaggttggt ttggaatttt tgattttgtg atttatttgt ttcggttttt 34860
   taaagtgttg ggattatagg tatgagttat tgtattcggt tacgattatt taattttttt 34920
   ttataagatg ttttttggtt gacgtaaagt aggttgagtg agtgtgatat tttttttttg 34980
   tttgtgggtt tcggtttttt tagagttcgg ttagtgttat tggagataaa gtggttttcg 35040
   tttttttttt tcgttttgtt aggtttagtt tgtgtcgttg agttttattt tgtaattaag 35100
   aggagttgtg aattagtgat gttgatgtcg tagaaaagtg gttaggggtt tatggggggt 35160
   gaaggatagg gatgtcgttt tgagaattgt ttggaagggg tttttgggta atagtgttta 35220
   ttttagggtg tgggtaggtt ggggtggttg ggagttgggt ttataggttg gtataattag 35280
   agttttttaa agtcgtgggt ttgagggttt tgtttttttg ggatagtaat ttttggttta 35340
   atttgggaaa aggtgggtta aattaagttc ggttttttta ttgtaggatt atttagtgcg 35400
   tttagtatgt aaatgtgttt ttgaatttaa gataggatta tagaaatttt tttagtgttt 35460
   ttttggaggg agggatcgcg ggggatttat cgtaggggtt tgggtttgta ttggtttggt 35520
   gaagtgtttt tttagtgttt atattatgcg cggtgagagg ttggttttcg ttttttttat 35580
   tttttgttgg ttttatttta ttatttttgt attcgttttt tagtcggttt atgattaatt 35640
   tttgtttttt tagagttaat cggtgttgtt aggtttttta ttttaagtga tttttcgttt 35700
   gggttaaaat gtttggattt ttaggtacgg aggggagatg gattttaggt taaattatgt 35760
   tgggtaaaat tgagggttaa ggttgtgttt agtaagtgtt ttagttttgg tcgttaaatt 35820
   tatagtgtgt atagcgtgta ggaggtggtt gggtgaatga atttagttat agttcggttt 35880
   tttattcggg gttgttttta agtttttatc gttagttagg ttgggttaga gtttcgtggg 35940
   gtttttttgg gtttttttgt tttatagtag gagttcgtat ggagagtttc gagtgaggtt 36000
   aatgggtttt tttttttttt tttttttttt tttttaattt gagatagggt tttatttttt 36060
   ttatttagtt agggtgtagt agtgtaatta tagtttatag tagtttcgaa tttttggttt 36120
   taagtaattt tttcgtcgta gttttttaaa gtgttgagat tataggtacg tgttattttg 36180
   tttagttggg gtttttgttt tttatttttt tttttgtttt taaatttaat ataaagaaag 36240
   atattatatt tataattaga aaaattaata aggaatatta ttttaaaatt ggaaaaggga 36300
   ttaaagaaat aaattaagaa atttttgcga tttttgttta tcggagttag tggttgttag 36360
   tggttggttt aatataatgt tttaggaaga aatagaataa tagaaagtaa ttttagtgag 36420
   ttgagttttg gtttcgtttg tggcgttgta ttagtttttt gtagttgttg taacgagtgg 36480
   ttataagttg gttgggttat aataacgttt agttattttt ttatagtttt gtaggttata 36540
   tattcgagat tagggttatt aggttgaaat tagggtgttt attgggtata gttttttgga 36600
   ggttttaggg taggggtttt tagtttcgtg tgttttatta ggaattggtt tgtatagtag 36660
   gaggtgagtg attggtaagc gagcgaagtt ttatttgtat ttatagttat tttttattgt 36720
   tcgtattatt gtttaagttt tgttttttgt ttgattagtt atggtattgg atttttatag 36780
   gagagtgaat cgtattgtga acggtatatg tgagggattt aggttgcgtg ttttttatga 36840
   gaatttgatg tttgatgatt tgttatcgtt ttttattatt attagatggg atcgtttggt 36900
   tgtaggaaaa taagtttagg gttttaattg attttatatt atggtgagtt gtataattat 36960
   tttattatat attataataa taatagaaat aaagtgtata attaatataa cgtgtttgtg 37020
   gtcgggtgta gtggtttata tttgtaattt tagagtgaaa ttttgtttta aaaaataaaa 37080
   aataaaggtt aggtatagtg gtttatgttt gtaattttag tattttggga ggttaaggtt 37140
   ggcggattac gaggttagga gtttaagatt agtttggtta atatggtgaa atttcgtttt 37200
   tattaaaaat ataaaaatta gtcggatacg gtggtaggcg tttgtagttt tagttttttg 37260
   ggaggttgag gtagaagaat tgtttgaatt taggtggcgg aggttgtagt gaattgagat 37320
   agtgttattg tattttagtt tgggtggtag agcgagattt tgttttaata aaataaaata 37380
   aaataaaata ataaatataa tatatttgaa ttattttaaa attatttttt tattttttgg 37440
   tttgtggaaa aattgttttt tttgaaatcg attttaggtg ttaaaaaggt tgggggttat 37500
   tgtttttatc gtttgttttt ttgagttttt ggtggttgtg ggtgtttttt ggtttgtggt 37560
   tgtatggttt ttatttttaa tattagtatt tatagatttt ttcgggtttt atttttattt 37620
   ggttattttt gtttgtgtta aatttttttt tgttttcgtt ttgtatgagt atcgagattg 37680
   tttttaggga ttatttagat tatttagggt gttttcgtta tagtaagatt tgtaattgaa 37740
   ttatagatgt aaaaattttt gttttaaata aggttttatt tgtaggttta gggattagga 37800
   tatgttattt ttggggttat tatttagttt tttttcgtga ttatgatttt tttttttttt 37860
   tttagttttt tatttcggtt gagatgttgt ttttattttt tttattgttt ggttgtttgt 37920
   ggggttgttt tttaggtttt ttgggaggag gtggttgttt gatgtttttt agaaggttgg 37980
   gaatggtttt ttttattttt taggaagatg ttttttttgt taaggagatt gtatttgggg 38040
   ttttatttta ggtagagagc ggacgggatt gtggtttgaa gtggtatttt agggtttttg 38100
   gttttgttat ttagtcgagg gttgtgtatg ggttttattt ggttataggt aatttatttg 38160
   gttttgtggg gtagggattg taaatttaga ggtttgcggg gtgtgtcgag gtagaggggt 38220
   gtaaatattg taaatttgtt gggggttttg gggagtagta tagattgaaa agtgttttgt 38280
   ttgaagagta gaattgtttt atattagttt tgtgcggtta ggtaggaagg ttttcgtggt 38340
   gcggttacgc gttttttttt ttttaataga agttgatagt ttggagtttt tagagagatt 38400
   gtttgagttt ttagttttgg tataaatata tatttttttt aaataggttt gggttaatag 38460
   aattagtttt cgtgtttcgg gtttgaatga atttttgagg tgaagtttaa agatagagag 38520
   gatgtttttg atttttattt tagggattta tattgtaaat ttttatttgt tttgttgagt 38580
   ttttggtttt agtatggatt ttgattatgt gttggtgggg attttttagg taagtaagta 38640
   gtttttgagg atttcggatg aatagtgggg aatagtattg attttttagt ggggtttcga 38700
   gttttcggag aggaagattc gttttttttt aggggacggt tagagattta ttgatttatg 38760
   cgtgtgtggt aggaattttt taggaagttt tcgtttcgtt cgtttttttt gtttgggcgg 38820
   ggacggtaat tgtttttgtt attgtagatt aatgggacgg aggggggtga ttttttaggg 38880
   ttttttttgg gtaggtgttt cggaattttt ttttagtacg ttggtttggg gtacggtcgt 38940
   tttttttgtt tagttacgtt ggggtatagg gtgaagaagg gttggggtta gtttaggata 39000
   gaggaaggcg aggtaggtac gtaggaattg ggttttttaa atttttaagt ttaaggaaat 39060
   cgtagtatcg cgggataggg aaaatgaaag attttggaag tcgttaggaa tttgattttg 39120
   tgagttggtt tttaagagtt taagttaagt atttttaagt ggatattaaa aaggagtagt 39180
   aagtttcggg gcggcggggg ttggaggagg tggagagagg aggttgtcgg aagtcgtatt 39240
   cgggattttt gtagttatcg attagatcgg gcggtcggga ttttgggatt ttcgtaggta 39300
   gattcggtgg tttgtcggat ttttcggggt ttatttcggg tttttttttt tgttttttat 39360
   ttttggattt tttttttttg tttttttttt tttttcgttt tgaagagata atgttatttt 39420
   agtttggagt ataaatatat gattagtata tggaaatgtg gtaattcgga tgtattcgtg 39480
   attgtaatag attgaagaaa ttagattaga taaagagtgt ttttagagga ggaggaggag 39540
   gaggaggagg ttgagagagg gagggcgacg ggggtgagaa aggggaggtc gtttttgagc 39600
   gggacgtcgg gattttcgtc gttgttaaat atattcgtag gaatggagag ggatcggatt 39660
   ttaggtacgt agatagtttt ttttttatcg gtcgtttttt attgttttgg attcggggtt 39720
   ttatttatgt ttgggggaat aagtatgtaa agggcgtttt tgtttatttt ttatagaaat 39780
   attttttttt tttttgacgg ttttaagttc gtgggatgaa tttcgtttta gaagttaagt 39840
   ttttttgagt ttggttttta tttaaattcg gttttttttt tttttggtta tttttttttt 39900
   attttttagt gtgttatttt tgtatgttat ggttattgtt aatgttgagg ttgttggcgt 39960
   cgaaattttg ggagggtgtg ggggatttgt ttttttggtt cgggtttggg gcggcggcgt 40020
   ttggatttgg ttggtggttg gtttgtttat tgtgtggttt gttttttgtt tttagttaaa 40080
   gtttgttttt aaggttgttt cggtttttta tttaaaatag tatttttgtg tttttttggt 40140
   agttgattat agagggggaa gttttcggcg tttagaggag tttggcgtgt gtgtgtacgt 40200
   gtgtgtgtat gtgtgtgcgt gtgtttattt acgttggtag tttttttgtc gtaggtatag 40260
   atttattagg gcgtgaatag gtaaatgaag agaaaggggg tttttttgtt tttagtttat 40320
   tttagttttt taggtttttt tttttaggtt tagttttttt ggttttttta tttatatgcg 40380
   gtgtcgtggt tgttatttta cgtggttagc gtcgttatcg gtaattagta gtgatgatag 40440
   tttttgtcgt ttatgtcgtt ttttgagttt agcgttggga gatatggggt ggttgagtta 40500
   agttttaggt tttgggtatt ttgaagtgat ttacgggtta tggagcggga ggtcgttaag 40560
   agtgttaatt tttacgtgtt ttgggttttg gtttgttggt tgttattatg tgtgtgttgg 40620
   ggggtttttg agaggtttga gagagggagg tttatttcgg tggttttttt ttaggacgtg 40680
   tggttttttt tattattgtt tttagggtta tatttttttt tttttttttt tttgagacgg 40740
   agtttggttt tgttgtttag gttggagtgt agtggcgcga ttttagttta ttgtaagttt 40800
   cgttttttta gtttatgtta tttttttgtt ttagtttttt aagtagttgg gattataggc 40860
   gttcgttata tttttttgta tttttagtag agatggggtt ttatcgtgtt aattaggatg 40920
   gtttcgattt tttgatttcg tgattcgttc gttttagttt tttaaagtgt tgggattata 40980
   ggcgtgagtt acggcgttcg gttttagggt tatattttga ttgttacgtt gggattggtg 41040
   gagcgggtgt gtgttgtgtc gttgagtata tttagtcggg tatagtaggc ggattttagg 41100
   agggggttag gggagacggt agaaggtttg ggtggtagtg aaattgtatt ttatttgttt 41160
   gagttcgggg gtttaggttc gttgggacgg gtgggtgtgg agtttagcgt tttgtttttc 41220
   gtttagtagg agtcgtaggg tagggagcgt tgagagggat tttcgttagg tttgtatagt 41280
   ggagtcgttt tcggagtgat tattttatta ggtagtttgg gtagattata gtgggttttt 41340
   tgttttttgg gttattagtt ttaggaggac gttattttat tcgtttggtt ggtaggggtt 41400
   tcggggagtt tcgggttcgg tttttatttt atagatgatt tagtcgaggt ttagatgggt 41460
   gattagtagg ggatttgaga gttgagtaga gaggcgtggg tttgggggtg gtgttttttg 41520
   gggagttgga agtgatggta tttttttagg tttataaaat acgggttttt tatgaattga 41580
   ataaaatatt ttttgaaagt agttagtgtt gtgttttaga gattattttg atttttgttg 41640
   tttttggttg aggatagtat agttagggat gaagcgggga ttttttttcg ggttgtgttt 41700
   tgatgaaaga tgttttttat aaaattttat tcggatgtgt ttttatgtaa gaaaacgtat 41760
   atttttagaa atttagttgt aaattggttg tttttcgaat gaatacgata gtttcggagt 41820
   tgtttttttt aatgaggttt tcgttttggg taaattttag cgttggtttt taattgttat 41880
   tgaattttcg tatagttttt ggggaagtga tttttttttt gtttttgttg ggtggttttg 41940
   agtaagttat ttggtagttt tgggtcgtag tttcgttatt tgtaaggtga ggggtgggag 42000
   cggtacggtt tttaggtagc gtttttattt tttgcgggtt tttgcgcgtg ttttgagttt 42060
   tgtgggcgtt ttttttttta ttgaggggtt tttttttttt gtagggtatg gtttacgtcg 42120
   cgttgttagg ttgtaagtta ttgcgagagt tgttttgatt tttaagtatg ttttggtttg 42180
   aggaggagtt atggggagga cgttgtgttt gtgtgataga ggtttgttcg gtagatgtcg 42240
   gttcgttgat ttttagacgt ggttgtgggt gttttatgtt ttattgggtt ggcgtgtgag 42300
   gcgtgtttag tttaggttgt tttatagttg tttaggaagg gtttgtggga gggggtggtt 42360
   ttattttggt gagtttacgg tggtttgtta gttttaggga aggtttagga gttttaagat 42420
   ttgttttttt gttttttttt cggtagtgtt tggtttcggt ttggtttgtg aacgggggtt 42480
   tgggtatttt ttcgtttttt ttgggggttg tggttttttt ttgggaaggt gttggagggt 42540
   ttgttagtag ggtcggtatg agttatggag tggtgattgg tcgttgaggt atggaaggtt 42600
   atttttttta gttaagggtc ggagtttttt ttttgttttg gaacggtttt gtggttttta 42660
   taaattaagt agttttattt tgttttaaga gtttgtattt tagtagggag agagttttta 42720
   agaagggaat tttttattgg ggagttttgt tgggttggga ggttaggaat aggttacgag 42780
   ggagggttgt aggtagggga agtggtcgtt tgtgtatggt tatgaatgta ggttcggggt 42840
   tttggagggg gtttggttac gttgtcgttg gacgtttgtt tttattaggg tttatttgat 42900
   gtagtacggt atagggtaaa gtttagtttt ttttacgggg tttttgggtt attagtgggc 42960
   ggggaatcga ggttttttgg agggcggtgt tttagttttt attttttttt tttgtttttc 43020
   gttgtgttgt tttcgatggt ttttgttatt ttttgggagt ttttattttt ggacgttcgg 43080
   tgtttgttcg gttttaattt ttttagtttt gtttttttat tttcgaggtg agaggtttgg 43140
   tttgacgcgt tttgtattcg ttttttgttt agtttcgtgg gaattttggt ttttgtagtg 43200
   gttttttggg gttgttatta ttaagtgtta taaattgggt ggtttaaaat attagatatt 43260
   ttttttttcg tcgttttgga ggttagaagt tcgaaattac ggtattagta ggtttggttt 43320
   ttttcggagg tttcgagggg gagtttgttt ttcgtttttg gtagtttttg gcggttttcg 43380
   aaatttttgg cgttttttgg ttcgtggtta tattattcga tttttgtttt cgttgttacg 43440
   gggttttttt ttgtgtttta aatttttttt tgtttgtttt ttatacggat agtttttttc 43500
   ggatttaggg tttattgggt aatttaaaat ggttttattt taagattttt aattttataa 43560
   cgtttgtaaa gatttttttt ttaaataggt tatgtttata ggttttgggg tggatgtaaa 43620
   ttttaggggt tattttttat ttttttgttg ttttcgtgtg tttggatgta tagtatggtt 43680
   ttatgtttta gattttttgt tggatttttt tggttttttg gggtggtttt tggttttttt 43740
   tattcgtttt tgtgtttttg gtgaggtgtc ggggtcgggt ttagtttttg taggaatttt 43800
   ggggatagta tggagtcggt aattttttat taggcgtttt tttatttgtt tagagtttat 43860
   ggttgtgagg ggtttttttt tgtggttttg atttgtaatt ttttagtggt tagtgatgtt 43920
   gagtattttt tcgtgtttat tgtttatttg tatatatttt tgtagaaatg tttgtttaag 43980
   ttttttattt gtgtttgatt tggatgattg ttgttgttga gtgttaggag atttgtagat 44040
   taatatttag atattaattt tatatttgat ttatgatttg ttgttatttt atggattgtt 44100
   ttttttttac gttgttagtg ttttttgata tgtaattttt tttttttttt ttgagatgga 44160
   gtttgatttt gttgtttagg ttggagtgta gtggtatgat tttggtttat tgtaattttc 44220
   gtttttttgg ttttagcgat ttttttgttt tagttttttg agtagttggg attataggta 44280
   ttcgttatta tatttagtta atttttgtat ttttagtaga gacggggttt tattgtgttg 44340
   attaagttgg tttcgaattt ttgattttaa gtaatttatt cgtttcggtt ttttaaagtg 44400
   tcggagttat aggcgtgagt tatcgtattt aggttgatat gtaaatattt taaattttta 44460
   tgacgtttta ttttatttat ttgttttttt gttgtttgtg tttttggcgt tatatttaag 44520
   aaattattgt taaatgtaac gttaggaagt tttttttttg tgtttttttt taagagtttt 44580
   gtggttttag tttttgagtt taggtttttg atgtaagttg agttgatttt tgtatgtggt 44640
   gtaagggttg gtttagtttt atgttttggg tttttgattt attttttttt tttttttacg 44700
   tttagttggt ttcgttgggt ggcggggagg agtggggaag tttcgggttg ggtttgtatt 44760
   cgattatttt tttttaggtt agttagggag ttttagtttt tgtttaggat ttggttggac 44820
   gtgtttttta tcgggaaagt ttgggtcgtt tttttaggtt gatggtaggg ttagttcggg 44880
   gcgttttgag gtttgttttg cggatatgtt ttttgtttta ggtggtgtgg ttgttcggtt 44940
   tgcgtgtgag attagttgtt tgtgttttag gttatggagg ttgagtgttt ttagtttgtt 45000
   tttttgttcg gttttttttt tggggaagtt tttgtagttt attttttgtt ttcgtttttg 45060
   ttatttgtgt ttttgtttgt tttttgtttg gaggtggtta tttttggggt tattttttat 45120
   gatttggata taagttttta ttttgaagtt attatttaaa tttttgtgtt ttaaattttt 45180
   tttatttatt atatgggttt ttattgtgat taatttagta gttgatgaag ttttttttgg 45240
   ggttttttta gtaacgggga gttggttttt tcggaggttt ggtttttttt taagtggaag 45300
   tggggcgtga gggtgttagt ttttttttgt tgtttggtgt tttaggttgg tttgttattt 45360
   ttggaagtat ttgttatttt tatatagtat tttatattta ttttttcgtt ttttattttt 45420
   ttttttaagg ggttattttt gttttttttt ttatttaatt ttatttatgt ggttcgttta 45480
   gtaatttttg atttttaata tgataaaata aatttttttt gtcggttatt tatttattta 45540
   tttagtattg ggtgtttttt gtggatttgg cgttggggtt tcgtggagga taaagttaga 45600
   tatagttttt gtttttatgg gattgtataa gtgtaagatt atattagtaa acgtgaaata 45660
   taggaagtga taggtgtgat aaaggggatt agtggtagga tagaatttgc ggttcgtagg 45720
   attaggttag gagggttgtt tcggggggat attttcgggt tgagcgtaga aggatgaggg 45780
   gagtaaatta ggtttaaatt tagtaggtag aggcgatcgt tgtaggtaat cggtaatgtg 45840
   tttaaaggtt ttggggcgcg gggggttgag gtcggtagta cggtaggaag taagattggg 45900
   gttgaaagag attgattgtt atgttgtgaa atatatattt ggtttttatt tttatttttt 45960
   ggtatataat ttttaaaatt tttggaattt ttaaagtgat gtttttttgg atgtttatga 46020
   ttgatagatt agttggtagt tttaggatgg tttttaggga agattaggta gaattataag 46080
   gtttagtatt atttcgtaat ttttaggtag gggagagggg ttgaaggtta agtagattat 46140
   tagcggttaa tgattgaatt aattatgttt tcgtaatgag gttttcgtga atatttagaa 46200
   ggatggggtt tcgggagttt ttggatggat gagtatgtgg aggtttttgg agggtggagc 46260
   gtttggggag tatatggaag ttttgcgttt tttttttata ttttgtttta tatatttttt 46320
   tttttgtatt ttttgtaata ttttttataa taaattagta aattttatga gttttaggaa 46380
   tatgtgtaaa agaaaaaaaa aagaataaat tatagttgtt gtagtaagtt aattttatta 46440
   gtattggttt ttatttgtcg attttatttt tggtaatgtt tttttttttt gggtttttgg 46500
   gattttttcg gttttattat tttttttgta gtggtttcgt tggttgttgt ttttaggtta 46560
   gtttattttt attcggtttg tttttattag gggttgaggt ttttttgttt attttagttt 46620
   tttggttcgt tttttgtttt gggtattttt ttatttcgaa cggttagagt ttttgaagtc 46680
   gttcgttgtg tgttttcgtt tggttttatg tatttcgtac ggtgtttttt agggtttttt 46740
   attggtaagg gtattggtat agattttggg gttgttttgg tttaagttta ttttgatttg 46800
   gagtttgagg gtttttgagg gggttttgaa ggttagggta ggtaaggttg gtatgttttg 46860
   ggtagtgggg gttgggtatg ggaggggatc gtgggtgggg agattttttt taattagcgg 46920
   tttacgtttg gagattgatt tgggttttag gttacgtttt gaaattggga tagagagtgg 46980
   cgtagatttt agttcggaga ttttttttgt ttgtggacgt ttttatattt ttattcgtgg 47040
   agttattttt tttttttttt ttttaaatgg ggaaattttg atttgaatat tgaataaata 47100
   atatattttc gaaattagcg aagaggtatt ggttttgaaa ttttgttatg aaagtttgaa 47160
   aatgtttttt ggatcgattt gttggaatag acgggtgtga tggggttgcg gggttttagg 47220
   tggtaggagg cgttttcgtg tttagggtat ttcgatgttt agagtttttt cgtgtttagt 47280
   gtgtttttgt atttagggta tttttatgtt tagggtgttt tcgtgtttag tgtgtttttg 47340
   tatttagggt atttttatgt ttagggtttt ttcgtgttta gggtgttttt gtatttagag 47400
   tatttttatg tttagggtgt tttcgtattt agggtgtttt cgtttttagg gcgttttgtg 47460
   tttagggtgt tttcgtgttt agtgtgtttt tgtatttagg gcgtttttat gtttagggtg 47520
   tttttatgtt taggtattgt ttttttttat ttttttaatt tttttttatt aataggtgtt 47580
   agttgtgaaa tgtttgttgt gtgtgttttt tggttgagtt tattttgtgt ttttaagttt 47640
   ttgttagata taggtaggtt gttttagttt ttttggtaaa tgttttgttg ttgttgtttg 47700
   atacggggtt ttatttcgtt atttaggttg gagtgtagtg gcgttatttc gttttattgt 47760
   aattttcgtt ttttaggttt aagcgatttt tttgttttag ttttttaaat agttgggatg 47820
   ataggcgttc gttattatgt ttggttaatt tttgtatttt tagtagagat ggggttttat 47880
   tatgttggtt aggttggttt cgattttttg attttaagtg atttatttag tttggttttt 47940
   taaagtgttg ggattatagg tatgagttat cgtgtttggt tcgtttggtg aatgtttatt 48000
   gaggtttatt atgtgtagtt ttttttttag gtgtttggtt tgtaatgagg agtaataaag 48060
   atatagtcgt tggtcgtttg ttatttaata gttggaattg agaggtagtt tataaatgag 48120
   taaatttata ggtggtttta gatgaggatt gtgaagaagt taagaatagg ttgttggggg 48180
   tgtatatttg tgtgtgtggc gaggggatta atggaagttt tttttgagtg gtgatattga 48240
   gttgaagttg taatgattag aaggatttag ttaggttaaa atttaaggga agagtaattt 48300
   ggtaaaggaa atagtaggtg tgaaggttcg aggttggagt ttggggaagg tgggagtggt 48360
   tggagtgtgt ttttagagac gggttatggt gatattaatg cgataggtag ggttaggtcg 48420
   tgtcgggttt tggaggtttt ggtgagggtt tgattttatt tttattatgg tgggatttta 48480
   gtgttttgtg gttgttgaga atggttaggg gtaggtaggg aggtttgtta gggaggaggt 48540
   tgttgtaggt tttgtagttg gagagggagg attaggattt ttttagagag gaagttggag 48600
   agggaggatt tttttgattg ttggatggat ttatttgttt ggttgttttc gttagtattt 48660
   agtagaggtt attggatgtt gataggtggt cggttttgtt tagttttgta ttgttttgtg 48720
   ttgttttggt ggttgtcggt tttaggagga gcgtttttgg tgtttgagtt tagtggtttt 48780
   ggttgtggtt tggtatattt ggtcgtgggt gaggttaagg tggtttggat tttgggaagg 48840
   ttgggtattt ggtagtttta aggtaggagt tggggtgttg gaggaggaag tacgtttatt 48900
   tttcgttttt ttttgtttcg tgggatgttg agtttttggg tagggatatg gtgagttttg 48960
   gtgtgatgta tttaatattt gttggtgttt ttatttttaa gtaggaattt tggtgtttgg 49020
   tgtttgagga ttgttgttta tcgttttatt attttttagg ttttttttta gtttttgatt 49080
   cggggtaagg tttagatatt tagaatattt ttggttggtt tttggagttg gggtgaggta 49140
   gaattttttt tttatattta tagtattagt taaggtttcg gggtttggat attatttttg 49200
   ggaggggtga gtaggataag tgaggttttt agatttgggg tggtttcgga ttggttaggt 49260
   aggtgggtgg gggttgtagt ttatgttttc gttttaggta atatagtcga gttgtagttt 49320
   tcgttttatt tttagttggg gttttttgtg tttatttttt gttttttttt tttttttcgg 49380
   aatggggggt ggatatttta agattagttt tttagttatt ttttttgttt tttgtttttt 49440
   ttttttcggg atagattttt gtgatcgttg ggtgtttttg atgttttgtt ttttttgcgt 49500
   tttgtgtcgt gggtgagaga gggtttcggg gggatttttt ttttttggag tatttgttgt 49560
   ttcgggtgtt tttgggggcg ggtttttatt ttatattgtg gagttggtgt aggaaggaag 49620
   gtgtcgggag ttagttttaa tttacgtgtt ggttgtggtt tttaaggtgt ggatggtagg 49680
   aggtggtgag atagggattt attgattatg ggattgatat ttttttatgt tgatgtgggt 49740
   tttgagttat aattttatta gtttttgagt tttggcgttg tttttggcga tggcgtggat 49800
   ttagttaggt tttcgggtag ttttttttgg attttttttt cgtttttttt tgggtagtgt 49860
   ttttgtttat ttggaaagga tgggtaggat ttggagataa tagggattta aagaagtcgg 49920
   gagtgggggt atttgtgtag tttattttgt agaatagata atttggttaa agtttaaagt 49980
   ttgttgttag gagtttcgtt tgttttttag agagtatatg gtttgattat tacggggatt 50040
   gggggagagg tataggtatg tttgttcggt agtagaaggg aatttcgggt ttgggttttt 50100
   ttattttgtg gcgttgtttt tttttagagt tgtattgttt tttttattgt ttttgtttgg 50160
   ttttagtgtt tatgtggttt gtggttttta gtttgggttt ggtttttcgt aatttatttt 50220
   gtttagtttt atatagtttg tttttttagt tgggaatcgg agtttatttg gagttttagg 50280
   tagtagattc gatgtagtga tgtagggttg ttagttatgt tagtaggttt tgttgtttat 50340
   agagggatgg gtattgttgt tgataataat taaagaatta cggttttagg ttgggtatag 50400
   tggtttatat ttgtaatttt agaattttgg gaggtcgagg taggagaatt aattgaattt 50460
   aggagtttta gattaggttg ggtaatatag taaggttttg tttttataaa aaataaaaaa 50520
   taataataat aataaaaatt atagttttag aatgattgat tttagtatgt ttataaagtt 50580
   aagggttttt agtgttttgg gtttatgtta ttgcgttaga gggagagagg aagaggttgg 50640
   ggtgatgggt attggggttt atgttttttt tggaatgggt ggttttggat tgtgtttgtg 50700
   tgatatgttt gtcgagcggg ggcggtgttt attgaggatt ggatgttgag gtgtgggtgg 50760
   ggggaggtta tgaagtattg ggtatttttc gtgtattagt gtttcgagat tgtagagagg 50820
   ggattcgttt agtttgggta gggaggcggg tggtagcgag gaaggggaga ggaattcggg 50880
   tgtagaaagt ttttttttta gtaggtggcg tcgtaagttt tcgagagttt agtttttgcg 50940
   gggtttgtat ttttattgaa tttgtgggta gaggttgttt gggtgtagtt ttcgttaatt 51000
   gtgattgttt attgcgggcg aggggaagtg gagtgtagag aatgtatcgg agggtagatg 51060
   attagttttc gtataggtac gggggtgttt tttttttcgt ataggtacgg ggggttcgag 51120
   tttttggggt ttttgtttta attgggcggg gaaagggagg gttggaagtt gagttagggg 51180
   gaaagggggt ggttttttgt tatttttttt attttatttt ttttattttt ggttgatagg 51240
   ttttaatttt ttttatattt ttttaatttt ttgtcgtaaa attttttaaa tatagaaaag 51300
   atgaaagagg gttgagtgtg gtggtttatg attgtaattt tagtattttg ggaggttgag 51360
   gtaggaggat tatttgagtt taggagttta agattagttc gggtaatata gtgagatttt 51420
   atttttataa aggatataaa aaattagtta ggtatggtgg tgtaggtttg gagttttagt 51480
   tattcggaag gttgaggtga gaggattgtt tgaatttggg aggttgaggt tgtagtgagt 51540
   tgggattgtg ttaatgtatt ttagtttggg cgatagaata aaattttgtt ttaaaaataa 51600
   aataaaataa aattataaaa aagaaattat gaaagatttt aaagtaaata tttatgtatt 51660
   tattatttag attttatagt aattattttg ttgtttgttt tattataaat ttttttggtt 51720
   attagtattt acgttggttt tgatgtattt aaaataagtt gtagaaatta gtatataata 51780
   ttttgttaat attttagttt gtattttatt aattagagtt cggtatttgt ttgtgattat 51840
   ttttgttttt ttttgaagta aaatgtatat attttgagat gtataggtgt gaaacgtttc 51900
   gttttttgaa ttttaataaa ggtatttacg tgtattttag tttgattaga atatagaata 51960
   ttattattat tttagtaagt tttttatgtt tttttaggtg tattatagtt tttatttttt 52020
   ttagggtaat tataattgtg tttttttttt attacgaaat aattttgtta gtttgagtgt 52080
   tttttagagt tatttatgtt gttgtaggtg ttagcgattt gttttgtttt attgttgagt 52140
   aacgtttatt gtgtgaatat tttgtgggtt tttagttatt tttttgttga aagatttttg 52200
   gttgttttta tttggaggtt tttatgagta aatttgttac gttggttttt tttgtataag 52260
   tttttttgtg tatatgggcg tttatttatt ttgtagaaat atttagagta ggtatagggt 52320
   aggtatatat ttagttttat aagaaattgt tagatttttt tttaaagtgg tttttttatt 52380
   ttatattttt attagtaatg gatacgagtt ttagttgttt tatattttgg ttaatatttg 52440
   gtgttgttat tttgtgaaat tttagttatt ttggtgggcg tgtagtggta ttattttatt 52500
   atggttttaa tttgttttgg tttgatgatt aatgcggttg agtatatttt tatgattggt 52560
   tttttatata tttttttttt gtgaaatgtt tttttaaatt tgttgtttat tttaaaattg 52620
   ggttgtttga tttttttttt tggatattga ttttttgtta gatacgtgtt ttataaatat 52680
   tttattttag tttgtggttt gtttgtttat tttttttttt tttttaagaa taaggtttcg 52740
   ttgtattgtt taagtaggtt tgaaattttt gggtttaagt tgtttttttg tttttgtttt 52800
   tttaagagtc ggttttatag gtatgagtta ttatgttcgg tttgtttatt tttttagtgg 52860
   tattttttaa taagtaaaag tgtgttattt taatttttat gaagtttaat ttaataaatt 52920
   tttttttttg tggttttttt tttgtgtttt ttgtaagaaa tttttgttta ttttgttaaa 52980
   ttgtaaagat atttatgttt ttttttagaa ggtttagagt tatatagttt ttatgaatta 53040
   atttttgtat atggtgtgag gtagagggtt agggtttatt ttgttgtcgt tttatgtgtt 53100
   tatttagttg ttttaaattt tatttttttt taaagttttt attttgtatt tttttggtga 53160
   tttatgaggt cgtataattt tttaagattt ttgttggtta tttacgtatt tttttttgta 53220
   aaattatttg tttacgtttt attttttttt aaaattatgt tgttttaaga tcgagtgatt 53280
   tttgaagttt ttattttata atgtttgaaa gaatgaatat tttgtttttt agttataggt 53340
   ttttgttttt tgttttgtag ttgtatagtg gggtaagatt tgggttttgt tttgttgttg 53400
   ttttgttgtt tttttatttg attgtttttt tatgggtttt atttaaaaaa aaaattggtc 53460
   gggtatagtg gtttacgttt gtaattttag tattttggga ggtcgaggcg ggtagattac 53520
   gaggttagga gattaagatt attttggtta atacggtgaa attttatttt tattaaaaat 53580
   ataaaaaatt agtcgggcgt ggtggcgggc gtttgtagtt ttagataatc gggaggtcga 53640
   ggtaggaaaa tggtgtgaat ttgggaggcg gagtttgtag tgagtcgaga ttgtattatt 53700
   gtattttagt ttaggtgata gagtaagatt ttgttttaaa aaaaaaaaaa aaaaaaaatg 53760
   ttgttttata tgttttttat atattttgga tataagtttt ttattagata tatgtattat 53820
   aagttttttt ttttatattg tttttttttt tttggttttt ttaatgatgt tatttaatga 53880
   gtaagagtat tttgttttta taaagtttaa tttattaatt ttttttagtt tcgatttatg 53940
   atttttgtga tttatggagg aaatttttgt ttatattaag gttaaaagaa gtttttttat 54000
   tttttagaaa ttttatagtt gtatatttta tgattttttt tattttttaa ttttttaaat 54060
   ttttatattt tttaatttat tatagtcgtt aggatgttgt atgatttatt ttaaattagt 54120
   ttttgtattt ggtttgaggt tagggttaat ttttgttttt ttatatttat atttagtttt 54180
   agtatcgttt gttgaaaaga tatattttat tttattgaat tatttgatat ttttgttaaa 54240
   aattattgat tgtatatgtg gttttatatt agtatatgta cgtatatata tgtaggtttt 54300
   gtgttgattg tatgtgtggt tttatattag tatatgtacg tatatatata ttggttttat 54360
   gttgattgta tatgtggttt tgtattagta tatatatatt agttttatat tgattgtata 54420
   tgtggttttg tattagtata tgtatatata tatatgtagg ttttatgttg attgtatatg 54480
   tggttttata ttagtatatg tatatttata tatgtaggtt ttgtgttgat tgtatatgtg 54540
   gttttgtatt agtatatgta tatatatata tataggtttc gtgttgattg tatatgtggt 54600
   tttgtattag tatatgtata tatatatatg taggttttat gttgattgta tatgtggttt 54660
   tgtattagta tatgtatata tatatatata ggtttcgtgt tgattgtata tgtggttttg 54720
   tattagtata tgtatatata tatatgtagg ttttatgttg attgtatatg tggttttgta 54780
   ttagtatatg tatatatata tatgtaggtt ttatgttgat tgtatatgta gttttatatt 54840
   agtatatgta tatatatata tgtaggtttt atgttgattg tatatgtagt tttatattag 54900
   tatatgtata tatatatatg taggttttat gttgattgta tatgtggttt tgtattagta 54960
   tatgtatata tatatatgta ggttttatgt tgattgtata tgtggttttg tattagtata 55020
   tgtatatata tatatgtagg ttttatgttg attgtatatg tggttttgta ttagtatatg 55080
   tatatatata tatgtaggtt ttatgttgat tgtatatgtg gttttgtatt agtatatgta 55140
   tatatatata tgtaggtttt atgttgattg tatatatata tgtggttttg tattagtata 55200
   tgtatatata tatatgtagg tttcgtgttg attgtatatg tggttttgta ttagtatatg 55260
   tatatatata tatgtaggtt ttatgttgat tgtatatgtg gttttatatt agtatatgta 55320
   ttgtatatat gtaggtttta tgttgattgt atatgtggtt ttatattagt atatgtattg 55380
   tatatatgta ggttttgtgt tgagatatta ttgtgttttt ttgatatttt gtttattttt 55440
   ttgttgatgt tatattttga ttaatgtggt tttatagtaa gttttaatat aaagtttttt 55500
   aaatatttaa aatattattt tgaatatttt aggttttttg agttttttta taaatttaag 55560
   aagtagtttg ttgatgttta taaaaatgta gtgtgaattt tggttgagat tgtgttgaat 55620
   ttgtagatta attaaggaag aattggtatt ttaataatat tgagtttttt gtatagatgg 55680
   tatattttcg ttttaaaggt ttttttgtat tttttttatt tttttattga aaaaaaattt 55740
   ttatagaatt atattgtttt gtagttttta agaaaaagat tttgtatgtt atttgttaaa 55800
   tttattttta agaattttat ttttggtatt attttaagtt gaaatagatt ttaaatttta 55860
   tttttaatta tttgttgtta gtatgtaaaa atataataga tttttgtatg taaattttat 55920
   attttatgat tttttttaaa gttatttatt attttggtaa tggttttgta ggttttttag 55980
   gatttaaata tatagtttta tttttttttg attttgatgt tttgtttttt ttttttgttt 56040
   tattgtattg gttagagttg ttagtttagt attgaatagt agtgattagt ggatattttt 56100
   gtttgtgtta aatttataat gttaatttta gatttgttat atatgatttt tgttagattg 56160
   aggaaatttt tttttatttt taatttgttg aaatttttta atatgaatgg gtatgatttt 56220
   agtaaatgtt ttttttgtat ttatggaaat gattattgtt ttgtttttta ttcgtttaat 56280
   aggataaatt ataatgattg atttttgttt ttcgtttttt tttttttttt ttttaaagaa 56340
   atagagtttt atttttgttt aggttgtagt atagtggtgt tatcgtagtt tattgtagtt 56400
   tgaatttttg gatttaagta atttttttat tttagttttt taagtagtag ggattatagg 56460
   tatatgttat tatgttaagt taatttttaa tttttaaaat tttttgtata gatggggttt 56520
   tattgtgttg tttaggttgg tttgtaattt ttgagtttaa gtaatatttt tatttttgtt 56580
   ttttaaagtg ttgagattat aggtgtgagt tattgtgtag gtttgatttt ttaatattga 56640
   attaattttg tatttttgga agaagtttaa tttgtttatg agatattatt ttttttatat 56700
   attgttaaat ttgatttgtt tagtttttaa ggatttttgc gtgttgattt atgagggata 56760
   ttggtgtggt attttttttg tttgttagtt tgtttcggta atgtttttgt tagttttatt 56820
   ttggttttat aaaattagtt tggaagtgtt tttttttttt tgttttttgt gtttttataa 56880
   ggttgatgtt gttttttttt taaatgtttg atagaattta ttaagaaaat cgtttggatt 56940
   tagaattttt tattaggaaa ggtgattgtt tgtttttaat tataaattta atttaatgta 57000
   tagaaagagt tatttaggtt tttttatttt tttttgtttt atttttggta agttgtattt 57060
   tgaaggaatt tgtttttttt aggtaagttg ttgaatttat ttatttaaag ttgttcgtaa 57120
   tataagtata tttagggttt gtttttagat ttcggtaata aagtaaatat tgtaataaag 57180
   tacgttatat aaattttttg gttttttagt atatataaaa gttatgttta ggttaggtat 57240
   ggtggtttat gtttgtaatt ttagtttttt gggaggttaa ggtgggcgga ttatttgagg 57300
   ttaggagttt gagaatagtt tggttaatat ggtgaaattt tatttttatt agaaatataa 57360
   aaattagttg ggtgtggtgg tatatgtttg tagttttagt tgtttgggag gttgaggtgg 57420
   gagaattgtt cgaatttggg aggtggaggt tgcggtgagt tgagattata ttattgtatt 57480
   ttagattggg tgatagggcg agattttatt ttaaaaaaaa aagaaaaaag ttatgtttat 57540
   attatattat agtttattaa agtgtataag attattatgt ttttttgtta aaaaatgtta 57600
   ataattattt gagtttttag taagttataa tttttttttt ggtagaaggt ttgttttaat 57660
   gttgatggtt gttgattagg tggtggttgt tgaaagttga aggtagttgt ggtagtagtt 57720
   tttttgtttt tttttttttt tttttttttt ttgagataga gttttgtttt attgtttagg 57780
   ttggagtgta gtggtacgat tttagtttat tgtaattttt gtttttcggg tttaagtaat 57840
   ttttttgttt tagtttttcg agtagttggg attataggtg tttgttatta cgtttggtta 57900
   atttttgtat ttttagtaga gatagggttt tattttgttg gttaggttgg ttttaaattt 57960
   ttgattttag gtgatttatt tgttttggtt ttttaaagtg ttgggattat aggcgtgagt 58020
   tattgtattt ggtcggtagt tttttaattg aagtttttga tatgggttga tttttttttt 58080
   tatgaaagat ttttttgtgg tacgtattat gtttgatagt attttattta taatagagtt 58140
   ttttttaaaa ttagagttaa tttttttaat tttggttacg gattgattaa ataagttgat 58200
   gtaatatttt aaattttttg ttgttatttt aataatgttt atagtatttt cgtttggagt 58260
   agattttatt tttaggaatt attttgtttg tttatttata agaagtaatt ttttatttgt 58320
   ttaagtttga ttatgggatt gtagtaattt agttttattt ttaggtttta tttttaattt 58380
   tagttttttg ttatttttat tatatttgta gtgatttttt ttgttgaagt tttaaatcgt 58440
   ttatttatga gggttggaat taattttttt taaatttttg ttaatgttga tattttaatt 58500
   tttttttgtg aattataagt gtttttaatg gtatttagaa tggggaattt tttttagaag 58560
   gtttttaatt tattttgttt agatttatta gaggaattat taattatggt agatattgtt 58620
   ttataaaata tattttttaa ataataagat ttgaaaatta aatttatttt ttttttatgg 58680
   gttgtagaat gggtgttata ttagtaggta tgaaaataat atttattttt atatatattt 58740
   tcgttagagt ttttgggtta taaggtgtat tgttaatgag taataatatt ttgaaaggat 58800
   tttttttttt tgagtagtag gttttaatga tttttttttt tttttgatgg agtttcgttg 58860
   tgttatttag aatggaatgc ggtggtatga tttcggttta ttgtaatttt tgtttttcgg 58920
   gtttaagtga tttttgtgtt ttagtttttt gagtagttgg gattataggt atatattatt 58980
   atgtttggtt agtttttgta ttttttgtag aaatagggtt ttattatgtt ggttaggttg 59040
   gtttcgaatt tttgatttta ggtgatttat ttgttttagt tttttaaagt attgggatta 59100
   taggtatgag ttattgtatc gggttttatt tgtttttttt ttgttgttgt tttttaattg 59160
   agatagagtt tcgttttgtt gtttagattg gagtgtagta gcgttatttt agtttatcgt 59220
   agttttcgtt ttttaggttt aagtgatttt tttgttttag ttttttgagt agttgggatt 59280
   gtaggtgtgt attattatgt ttggttaatt tttgtatttt taatagagat gaggttttat 59340
   tattatgttg gttaggttgg ttttgaattt ttgaatttag gtgatttgtt tgttttggtt 59400
   ttttaaaatg ttgggattat agttgtgagt tatcgtgttt ggtttttttt tgtttttttg 59460
   ttttttttta aattgagata gagttttttt ttgttgttta ggttggagta tagtggtacg 59520
   atttcggttt attgtaattt tcgttttttg ggtttaagcg gtttttttgt tttagttttt 59580
   taagtagttg gaattatagg cgtgtgatat tatgtttgaa taatttttgt atttttagta 59640
   gagatgcggt tttattatgt tggttaggtt ggtttcgaat ttttgatttt aagtgatttt 59700
   ttcgttttag tttttaaaag tgttgggatt ataggcgtga gttgtcgtat taggttttaa 59760
   tgggttttta atatttagta aattatgttg taaatagatg tgttgttaag tagggtttgt 59820
   tattttattt ttgaagtata gatagaatag atttggtaga atttttaagg gttttaggat 59880
   ttttggaacg gttaatgagt attggtttta atttacggtt aatagttgcg ttagtttttt 59940
   atgagagtta gtttgttttt tgaagtttta aagataggta ttgatttttt ttttttagtt 60000
   atgaaaattt tagatgatat ttttttttag tagaaggtgg gatatttatg ttgaaatttt 60060
   gttgtttagt gtagttattt ttattagtgt tttgagttag attttttggg taatttgttg 60120
   tcgttttttt attagtattt gttgttttat tttgtatttt tatattatag agatggtttt 60180
   tttttttaaa ttttatgatt tgatttttgt tagttttaaa tttttttttt ttagtttttt 60240
   tatttttttt agtttttata ttattgaaga gagttagggt tttgttttgg attaggtttt 60300
   tgtttaaggg aatgttgtgg ttgatttgat tttttattta gattatttaa atttttttta 60360
   tattagtatt aaggttgttt tgtttttttt ttttttatgt gtgtattgga atcgtatttt 60420
   taattttttt taataatttt tttttggtat ttataatttg gttagttatt tggtataaga 60480
   gattttattt ttgtttgttt tggttttcga tacgtttttt ttatttagtt taattatttt 60540
   tagtttttga tttaaagtga gagacgggtg attttttttt ttatttgaat atttagaggt 60600
   tattgtaggg ttattaattg gtttaatttt aatattgttg agttttaggg aatagggatg 60660
   tttgagtaga gggagagaga gcggggaata gttagttagt ggagtagtta gaatatataa 60720
   cgtttattga ataagtttat tgttttattt ggatgtggtt cgttatgttt taaaaataat 60780
   tgtaataatg gtattgaaga ttattgatta ttgggcgcgg tggtttatgt ttgtaatttt 60840
   agtattttgg gaggtcgagg taggtggatt atttgaggtt aggagtttga gattagtttg 60900
   gttaatatgg tgaaatttcg tttttattaa aaatattaaa attagttagg cgtggtggcg 60960
   ggtgtttgta gttttagtta ttggggaagt tggggtagaa gagtcgtttg aatttgggag 61020
   gtggaggttg tagtgagtcg agattatatt attgtatttt attgtatttt agtttgggta 61080
   atagagcgag attttatttt aaaaaaaaaa aaaaagggtc gggcgtagtg gtttacgttt 61140
   gtaattttag tattttggga ggtcgaggcg ggtagattat gaggttaggg gattgagatt 61200
   attttggtta atacgatgaa atttcgtttt tattaaagat acgaaaaaaa tagtcgggcg 61260
   tggtggtggg tgtttgtagt tttagttatt cgggaggttg aggtaggaga atggcgtgaa 61320
   ttcgggaggc ggagtttgta gtgagtcgag atcgcgtcgt tgtattttag atagagtaag 61380
   attttgtttt aaaaaaaaaa aaataaagaa agaaaaaaga aaaaagagat tatagattat 61440
   tatagtagat ataataataa tgaaaaagtt tgaattattt taagaattat taaattttta 61500
   tatagagata tagtgttaat atgttgttgg aaaaatggtg ttagtagatt tatttgatgt 61560
   agggttgtta cgaattttta atttgtaaaa agtgtagtat ttgtgaagta taatgaaatg 61620
   aggtgtgttt gtgttttttg ttattgtaat gtcggtagga ttcgtaagga aaatatttta 61680
   ttttttttat tgtgatttta tgttcgttat ttttttttta ttagtagttt ggttaggagg 61740
   ttattaattt agattttttt aaagaattag tattggattt tttttttttg gtgtttattt 61800
   ttttgagttt ttttgttatt tttattattt ttttttttgt atttgtttta gcgtattagt 61860
   tagggtttag ttaggaaata aaaattatat cggtaatttg aatagtaatt tgtaatataa 61920
   agtgttttta attgtaagag gtagttaatt attaaaggga ttataaaggg gtgatattgt 61980
   gatataagaa agatgtattt gggttgggtt cggtgtagtg gtttatgttt gtaattttag 62040
   tattttggga ggttgaggta ggcggatttt ttgagtttag gagtttgaga ttagtttggg 62100
   taatatggtg aaattttatg tttattaaaa atataaaaaa ttattttggc gtggcggtgt 62160
   gtgtttgcgg ttttagttat ttgggaggtt gaggtgagag gatagtttga gttcgggaag 62220
   gtagacgttg tggtgagtcg agattgtatt attgtatttt agtttggatg atagagggag 62280
   attttgtttt aaaaattaat gtatttgttt gtttgtttgt ttatttattt atttatttat 62340
   ttatttattt atttatttag tttttgtttt ttaattttgt ttggtatgta gtttttaaaa 62400
   tttttgaaat ttttaaagtg atgggtgttt ttttgtttgt taaggaggcg attggtggtt 62460
   ggaggttttt ggatagtttg gggataaggg ttggttgtta ggggatttag ttttgtgatt 62520
   agaaggttag aatttgtagt tttttttatt tattttttgc gaggtaagag gggatgaagg 62580
   ttgagttgat tattaatggt taaagatttt atgttatgtt tatgtagtga agtttttttg 62640
   aaaatttaaa aggatgaggt ttgtagagtt ttaggttgtt gaatatttgt aggtgttggg 62700
   agggtagtgt attttttcgt ggaagtttta tgtcgttttt tttatatttt ttttatgtat 62760
   tttttttagt tggttgttta tttgtatttt ttgaaatatt atttgtaata agttagtaat 62820
   tttaagtaaa ttgatttttt gggttttgtg agttatttaa gtaaattatt gaatttgaga 62880
   agggagttgt gaagatttta aatttgtagt taagttagat agaagttatg ggtaatttgg 62940
   tgttttatta tttgcgattg atttttgaag ttgagggtag ttttgtgaga tggaattttt 63000
   aatttatggg atttgtatta attttggttg gtgttagaat tgaattgaat tgtaggatat 63060
   ttagttgggg attgttgagt attggagaat tgtttggggg tcggcgggag gaaattatat 63120
   atttggtgtt agagttgaag tattgagtgt tgtgaatgag agtggagaga tagagtttgt 63180
   tttgttttat gtaagaggat tttaaggagt atagaaagag taaatatgag aagtagttat 63240
   tttagaagaa aatataggag gaaagtttcg tgatattgga gttggtaatg tttttttgga 63300
   tgtgatatcg aaaatataat aaaagaaata aatgataaat tggattttat taaaatttaa 63360
   aatttttgtg tattaaggaa tattattaat ggcgtgaaaa ggaaatttat agaatggaag 63420
   aaagtatttg aaaatcgtat attcgataaa ggattagtat ttagaatata taaagaattt 63480
   ttataattaa gttataaaat aaaaaaaaat aatttagtga atagatattt ttttatagaa 63540
   gatatattaa tggttaagaa gtataggaag agatgcgtag tgttattaat tattagggaa 63600
   atgaaaatta aagttataat aaaatatgat tttatatttt ttaaggtggt tgttagttaa 63660
   aatatggata atagtaagtg ttggtaagga tatggagaaa ttggagtttc gtgtattgtt 63720
   ggtgggaatg taaaatggtg tagttgttgt ggaaaagata taataattgt ttaaaaaatt 63780
   aaatttagaa tgattataat atttagtgat tttattttta gatgtatatt ttaaagaatt 63840
   gatagtaggg atttgaatag gtgtgttata tttttgtttg tagtagtatt ttttttataa 63900
   ttaaaagatg taggtaattt aggtgtttat tagcggatga atgaatagat aaaatgtggt 63960
   ttatttgtat agtggaatat tattttgttt taaaaaggaa ggagaggtta ggtgtggtgg 64020
   tttacgtttg taattttagc gttttggaag gtcgaggtgg gtagattatt tgaggttagg 64080
   agtttgagat tagtttggtt agtatggcga aattttgttt ttgttaaaaa tataaaaatt 64140
   agttaggtat gggggtatgt gtttgtaatt ttagttatta gggaggttga ggtaggagaa 64200
   tcgtttgaat tagggaggtg gaggttgtag tgagtcgaga ttatgttatt gtattttagt 64260
   ttgggcgata gagtaagatt gttttaaaaa aaaaaaaaaa aaaaaaaaaa aaggagattt 64320
   tgatatatgt tatagtatag atgagttttg aagatatgtt aagtgataga agttagatat 64380
   gaaaagataa atattgtatg attttatttt tatgaggttt ttagagtagt taaatttaga 64440
   gagatagaaa gtgggatgga ggttgttaga ggttggggga agggagaatg aatgggaagt 64500
   tggtgtttaa tggggataga attgtagttt gggaagatga gatggatggt ggtgatggtt 64560
   atataatagt gtgaatgtgt tttatgttat taaattgtat atttgaaaat ggttaaaatg 64620
   gtaaattttt tgtgatggat attttaaggt atgtatgtaa atatatagaa gtagttatta 64680
   ttattggggt tgatggaaaa gaaattaaga attcggagcg ttttttatat tttgttacgt 64740
   tttacgttta ggttttgttg gagagtgtgt gattgttata gaaatttgta gtttattggt 64800
   aataaaaagg tttttgttag aaggtgtaga aagagtggat ttgtaggatt agtttattgg 64860
   gtagtcgaga aatttattag agggtatggg tgggtaggag ttgggtgttt gagaagtttg 64920
   ttgaggtttt attgggttag agttggggtg ttggagaaat ttagtataga gtaggtttta 64980
   ttgggtatac gttattggta gttgtgtttt tagaggaaaa agagattttg gaattaggaa 65040
   gagaagttgt tattttagta aggtttggta gttttttata gtgttgtttt ttgtggatag 65100
   agtttgatat gattgtattt ggttaaggag aaagatttat aaggtttcgg tttagtatta 65160
   cggagcgggg tttggtagga tgtatgtaga gttaagaggt aatatatggg taattggtat 65220
   atttttttag attttttgtt ttttttgttg ttttgttttg ttttgttttg ttttgttttg 65280
   ttttgagaga gtattttgtt ttgttgttta ggttggagtg tagtggtatg atttaggttt 65340
   attgtaattt ttatttttcg ggtttaagtt atttttttgt tttagttttt taagtagttg 65400
   gagatgttta ttatgtttag ttaatttttg tatttttagt agagataggg ttttattatg 65460
   ttggttaggt tggttttgaa tttttgattt taggtgattt atttgttttg gttttttaaa 65520
   gtgttgggat tataggcgtg agttatattg tatttggttt atttttttag atttttaaag 65580
   tagattttta ggtttttttt tttttttttt tttttttttt tttatttttt ttttaatata 65640
   agtatttata gttttttttt aagtattagt tttagtttac gtattttagt atgttgtgtt 65700
   tttattatta ttttatttga aatattgtaa aatttttttt gtgatttttt ttttgattta 65760
   tgggttattt agaagtatgt tgtttaattt gtaaatagtt aaggtttttt tgagtatttt 65820
   agagttattg atttttaatt taattttatt ttggttagat aagtatattt tgtatgtttt 65880
   taaaatttga gatttatggt ttagaatgtg tattttggtg aatgtattgt ttttatttga 65940
   gaaaaaaata tgtatatata ttttagattt tttgagtata gagttttata attgttgatt 66000
   aggttaattg ttagttatta gtgtttttta gattatttga tgtttgtttt ttagatgttg 66060
   agagaatggt gtttgttatg gtttatttgt ttgtgttttt taaaatttat atgttggagt 66120
   ttaatttttg gtgtagtggt gttgggaatt ggggtattag ggaagtattg gtttatgagg 66180
   gtagagttat cgtgaatggg attagttttt tttgttatgt ggggatatag taggaaaagg 66240
   atatttttga agtagggagt tagtttttat tagatattga gtattttgat tttggatttt 66300
   ttagttttta gaattgtgag tattataatt tgtttataaa ttatttagtt taaggtattt 66360
   tgttttaata ttttgaatga attgaggtgg tgttaaaatt tttaattatg tatttaggcg 66420
   tagtggttta tatttgtaat tttagtattt taggaggtta aggcgggtgg attatttgag 66480
   gttaggagtt tgagattagt ttggataata tggtgaaatt ttgtttttat taaaaatata 66540
   aaaattagtt gggcgtggtg gtaggttttt gtaattttag ttattcggga ggttgaggta 66600
   ggagaatcgt ttgaatttag gaggtggagg ttgtagtgag tcgagattgt attattgtat 66660
   tttagtttgg gtaataagag tgaaatttcg ttttaaaaaa aaaaaaaaag agtttttatt 66720
   tatgattgtg gaattgttta tttttttttt taattttttt aggttttata ttatgtattt 66780
   tgaagttttt ttggttaggt gtatatatat ttatgattat aatggtgttt cgatgaattg 66840
   attatttaat tatgatgaat gtattttttt atttcgtaat aattttattt tggatttaat 66900
   tttattgata tttatgtagt ttttttagtt tttttaaata tgttgtttgg gatgtataag 66960
   aaggttggag aggtttatgg agaaatttat gatggtttga tttatagttt tttaatttta 67020
   ttatggtgcg aacgtggtat gtatatagta gaaattgtat tttaagtatg tgtgaaatta 67080
   ttgtgttttt tatttttagt atagtattta ataatttata tgagatagtt aatattttat 67140
   tataaaatag gttttgtgtt tgttgatttt gtttaattgt aggttgaagt aagtgtttta 67200
   attatgttaa ggtaggttag gttaagttag aatgttcggt aagttagatg tattaagtgt 67260
   atttttgaga tgatatttat tttttattta tgatgggttt gttaagatat aattttatta 67320
   ttatttaagt agtattagta tgtttttttt taaatagaaa attttaattt gtgtttatat 67380
   tgaaatagta ttttttgtag atagtatata gttgagtttt gttattttat ttattttgat 67440
   agttttcgtt ttttaattgg aatatttagg ttatttttgt ttaatatagt tatagatatg 67500
   attgggttta gagttattat tttgtttttt gatatttatt ttttttattt tgtttttttg 67560
   gtttttttta tgttaattaa gtatttttta gaattttatt ttaatttatg tttttttttt 67620
   tttttttttt ttaagataga gttttatttt gttgtttagg ttggagtgta atagtatgat 67680
   tttggtttat tgtaattttt gttttttggg tttaagtgat ttttttgttt tagttttttt 67740
   agtatattgg gattatagac gtttgttatt acggtcggtt aatttttgta tttttagtaa 67800
   agacggggtt ttattatgtt ggttaggttg gtttcgaatt tttgatttta ggcgattcgt 67860
   ttattttggt tttttaaagt gttgggatta taagcgtgag ttattgagtt cggtttgttt 67920
   tgatttttta attgtatttt tttgtattgt tattttagta gttattttat agattttatt 67980
   gtatattttt agttttttat agtttattta gaattaatat tgtttttttt tatggaaaaa 68040
   aagtgtaaga attaggtaat tttatgggtt tatttattat tttttatttt gtatgtgatg 68100
   gttgttagat atttattata tttatatata ttgtaaatta gataatataa taaggaaatt 68160
   tttgttttaa atagttttat gtattttaaa gaaattaaaa gtaaaataga atttttttta 68220
   tttgtttatg tatttgttat attttgtgtt ttttattttt ttttgaagag ttgagttttt 68280
   atttggtttt attttttatg attttggaga atttgtttta cggtttttgt agtgtaggtt 68340
   tgtagatagt taattttttt ttatttttat ttatttgtaa atttttattt tgttgttatt 68400
   tttttttttt ttatttttat ttttattttt gagatagagt tttattttgt ttttttatag 68460
   gttatagtgt aatggtgtga ttttaaaatt tttgtttaat tttaatttat taattttttt 68520
   ttttcgggtt taaacgattt ttttgtttta gttttttaag tagttgggat tataggtatg 68580
   tgttattata tttagttaat ttttgtattt ttagtagaga tgggatttta ttatgatggt   68640
   taggttggtt tcgaattttt gattttaagt gatttgtttg ttttggtttt ttaaaatgtt   68700
   gggattatag gtgtgagtta ttgtgtttag tttgttgtta tttttgaatt ataattttat   68760
   tggttataga attttgagtt gttagttttt ttttttagga ttttaaaata tagggtttta   68820
   gtgtttttgg ttttcgttgt ttttgttaag gaattagtat ttatttagtt atttttttgt   68880
   agattatatg ttgttttttt ttggatgttt ttaagatttt ttatttaagt ttgattgttt   68940
   agtatttatt tttgggtaaa aaagttttta gaaattagaa atttatttag ttttattttt   69000
   ttttgttagg tgtagatttt attttagttt ttatttattt gagatatttt atattgattt   69060
   taggttgttg ttattttgtt tttttaaata ttttgtttag agttataatg ttttttgtag   69120
   aattgattag atagtagtta ttttttgtga ttaggagttt tgatttttta gatttgttga   69180
   tgggtttata aagtttttgt tcgtggtttt tagttggtag tggttttttt gttttgaagt   69240
   tgttatgagt gtgtaaaatt tataggttta atttttttta ttagtatagt ttagaaaagt   69300
   ggaatttttg gtttgggtgg ttatggtgtt ttagattatt ggttagatta gggtggtgat   69360
   tgttagttgg tcgaatgtgg ggtttttagt ttagtatttt tggattttat tttaggagtt   69420
   atggttgttt tagggttagg ggtttggttt tagtttttgt tttttttttt ttttttttaa   69480
   gttgtttagt attaggtttt aattagtatt ttttgtagat agggtgggtc ggttttttat   69540
   tagaggggta gggttgggcg ggaagtttgt tgagtttttt tttttgtgta gtttatagtt   69600
   ttggggttag ttttagaggg gtgtagaaag gagtttttaa aggtagaaat ttattttttt   69660
   ttttgttttt gtgtttttag ggataaagga gaggtggttt tagttggggg gttattagtg   69720
   gtatgtagga ggtttatatg attattgttt gttggggttt tttttttttt gtgagagaga   69780
   cgagagagag gagagagagt gtgagttagg gtagtgtttt tttttagtaa cgtagatttt   69840
   tgtacgtgtt ttttaggaga aatttcgtta ttcgttagag ttttagattt ggaagaagtt   69900
   ttgttttttt tttttttttt agtgtagtgt gtgtgtgtgt gtgtgtgtgt gtgtttttat   69960
   atgtattata tttttttata gaggaaataa ttttcgttta agtgtaaagg ttgggattgg   70020
   agtaggtagt atgtgggtta aaggagtaga gaggaggaaa cgggggcggg ggtgagggtg   70080
   ggaataggag gtgggtagat gttacgaggt atgtttttta taattagaag ggttggtgga   70140
   gatgattaga atgttttaga tagatttttt attaaatttt taaaatttaa ttttgtttta   70200
   tgaagtttga ggattttgta gagtgaaaga taaggaaggg tttttttgta ggggggtgtg   70260
   tgtgtttgtg tgtgtttgtg tgttgtgtgt ttttatgtgt gtgtacgtgt ggatttgtgt   70320
   ggtgtgtgtt tgtatatgtg tgtacgtgtg gatttgtgtg gtgtgtgttg agtgtgtata   70380
   tgtgtgtgtt tgtgtgttgt gtatgtattt gtatatgtgt atatagattt tatttttttt   70440
   ttttttggtt tattaagtgt tagaaagaat tttcgtgagt ttttttattt tttttaaggt   70500
   tgtatatgga gggatttgcg taggtatatg tgcgcgtgta tataatatat atatatatat   70560
   atatatatat atatatatat atatacgagt taggggcgag tatttttgtg gattgtattt   70620
   aggtttggta ggaaatcggg ttttgggaaa ttgtaggttt tatttttggt agaaaattat   70680
   gtttttgttg ttgttcgttg aagggaatat taagtggggt tggggatagt ttatgttgag   70740
   aagagtaaat tttgtttatg gtattgggga tattgagtgg tttttgtttc gggttttgga   70800
   gggtttggga gatacgtttg tagttttggg tattttttaa ggttacgggc ggttatattt   70860
   aaggttaggt ttttttttgt gtgagttgcg gttagtagga aggttgattt tagaggcggt   70920
   tttagggttt ttggaggttt tgtttttgag atatgggtta aggttagaga gtcgagaagg   70980
   ttgttgttga ggattttggt tggcgtttag tttagcggtt ttttaggcgg gttttgtgta   71040
   ggggttgatg aagttagttg ggaaatttgt gaaataaagt ataggttttt taggtttgtt   71100
   tagagtttcg gtattagatg aggtgtagag aagggaattg agttgttaag tgtgaatagg   71160
   agagaggagt tttaggatta tataggggtt tcgatatggt aggtgtttgg ttagtagcgg   71220
   tgttgttttt attgttggac gtgggaaaat gtattgatat ttgttgatta gagtcggggt   71280
   gagtatttgg gtgattttgt agatgagata aaataagggt tgtcgtgggg gaggggtttt   71340
   tgtcgggtta ttttgttgag gttttgtgtt attgttttgc ggttgttata ataaaggatt   71400
   ataaatgagg tgatttaaaa tattcgaaat cggttacgtg cggtggttta tgtttgtaat   71460
   tttagtattt tgggaggtcg aggcggttgg attacgaggt taggagatcg agattatttt   71520
   ggttaatatg gtgaaatttc gtttttatta aaaatataaa aaaaaaaaaa aaattagtcg   71580
   ggcgtggtgg tgggtgtttg tagttttagt tgtttaggag gttgaggtag gagaatggta   71640
   tgaattttgg aggtggaggt tgtagtgagt cgagatcgcg ttattgtatt ttagtttggg   71700
   tgatagagta agatttcgtt ttaaaaataa aaataaaaat aaaaataaaa atattcgaaa   71760
   tgtttttttt aaagtttttg gagttagaaa ttttagatcg aggtgtttga aggtttattt   71820
   tttttggggg tttcgaggga gattgtttta tgtttttttt cgagttttgc ggcggttagt   71880
   aatttttggt atttttcggt ttgtagttcg gtttattttt tatttggttt ttattttgtg   71940
   tttttttttg tgtttttgtt taaattgttt tttttgttgt ttttttaatt tatttttagg   72000
   gataggtttt tattttgttt tttaggttgg agtgtagtgg cgtgattatg gtttattgta   72060
   gttttagttt tttaggttta agtaattttt ttattttagt tttttaagta gttgggatat   72120
   aggcgtacgt tattatgttt ggttaatttt ttaatttttt tgtagagacg agggtttagg   72180
   ttggttttaa atttttgcgt ttaggtaatg agattttgtt ttttaaaata ttgggattag   72240
   agatatgagt tgttgtgttt agtttatttt tgttttataa agatatttgt tattggattt   72300
   agggtttatt ttgatttaat atgtttttaa ataatttaat tatattttta aagattttat   72360
   gtttatataa agttatattt ataggtattg gggggttagg atttgtatat gttttttggg   72420
   ggaatataat ttaatttata atgggtttta tttatagaac gaacggtttt tttagggtgt   72480
   gcgtaggtgg gtgaggggtt taggagaggg atttgcgtgg taagagggtt tgtgtttacg 72540
   ttagagattt tagaaggaga ttggatgggg tggttgtatg gagtaagggg ttatagttat 72600
   tgtttttaag tagttggtgg gttgtttggt agaaagggag tatttatttt gagttttttt 72660
   aagatatgaa gttagtatta ggtatttgga aggtgttagc gagtagagtt tgatttttta 72720
   tagaaagagg tgtttttttt ataatgttta tttgaggacg gtggaatagg ttattttata 72780
   aggtagtgag ttttttatgt ttggaagtat ttaagtaggg gttatgtggg agttgtttag 72840
   gagtgtatag aagggatttt agaggttttt gttaatatta tttttgtttg ttttatttta 72900
   cgataatttg aattttagaa tgtaatgtta ttggggatat gtttattaga ggggttttta 72960
   gtattaggtt ttatatatta tgtattattt ttagggtagg gaatgagagt aggttttatt 73020
   ttttttggtt gtttgtgagg attaggaggt ttgttgttga ggtagttttt tgtggaaata 73080
   gtgttttagt ttcgtaattt atttatcgga gttttaatat tttaatgaaa tattgtgtta 73140
   gatgggtttt gggtatgtgt gtgtagattg ttataagttt atgagaatgt atatacgtat 73200
   attgtatgtg tgtgtatata tgtatgtata tatgtataag aatacgaaga ttcgaggtag 73260
   gtatagttcg tgagatagat ttttttaggt tgaaagattt tttataaggg tttttagaat 73320
   aaggttttag tttcggtagt tttttttatt tttggtggga ggatttttat agagaagaag 73380
   ggttaagaag tatagttttt gatgtttacg gtgtattaag ttttgtttta agtttgtgtt 73440
   ttatttttta tttatttagt aaatttgtcg tttttgtttt gtgttagatg ttgtttttag 73500
   tattttataa atattgatgt atgtttgttt tatgataatt ttgtgaagta ggtagtgtta 73560
   tttcgtttta tagatgagga aataggcgta gagggataaa gggatttgtt taaggtttgt 73620
   ttttatttta tacgtatatg aacgtttatt tatgcggtag gcgttagttt ttttttgatg 73680
   tgagttaggt tttgagttgg ttggggtttg ggttttattt tggttgtgtt ttgttgtggg 73740
   tattaaggaa gtttttagta gttttatttt tttggtatga tttagtttta gggattggat 73800
   ttttttaaat gtttttgttt agtttggagt ttttttttta gttgggggag ggttgaacga 73860
   ttttttattt ttggaggttg tcggagtttt ttttgtttgt atgcggtcgt tgtcgggtgg 73920
   ataggtttgt ttgtgggttg aggtttttag tagttggggt ggattatttt tttttttaag 73980
   aagttttggg agaagtagtt gagggggtta gcgttttttt gaggttaatt ttttgttttt 74040
   tttagcgtag gcgcgaagtt atttttgttt gtgtgtttgt tatagatagt ggagggcggt 74100
   gtaatcgtga cgtttgtttg ttgtattttg cgtttttgag gacgttgatt tttggtcggt 74160
   gttgggcgtt tgttttttag ttattttgtt ttgatttggc gtggtaggtt cgagtattgt 74220
   ttttttataa ggaaataggt ttagaggttt tgagttattt gttcgggttt ttttagttaa 74280
   taattggttg agttaggatt tttatttagt tttgtgattt tagaagttta agtttttttt 74340
   attacgttag ggttaggaaa ggattagaat gtttttttac gtgtcgatta tttggatgat 74400
   ttgtgtagtt tagttcgttg tagttgttta ataaaaatgt ggaatgaatg ggtttatatg 74460
   ttttattgtg tgatttagag ttaatgattt tggttgattt ttttgttttt atttcgtggt 74520
   ttagtatttt tttttggggt gtgtgatttt ggaagtagtg atttttttta ttttattttg 74580
   aatggtgttt gcgtcgtagt aggggagtag ttggggattg ttagcgtttg ttgttggtgg 74640
   gttgtgcgtt agatttcgtt ttggatgtgt gggtgattgt tagttatatg tgtgtgaagg 74700
   gttgtgcgtg taggatttgt gtaaatgtgt ttttttttgt tgttagttta tgggaggttg 74760
   ttaagatttt gtttttgtat attagtttat attttttttg tggtttattg ttgtggttgt 74820
   ttttttgtgt tacgtggttg taggtatagt ttttaagtgg atggggatcg tgggttgaat 74880
   gtgtattaga tgtttttgtt ttttggtggg tggagatttt taatttatgt atcgtggttg 74940
   tcgtatttga aaggtttttt atggtgaagg atttggattt gagtaatttt tcgtagaggt 75000
   tttagaagtg ttttttggtc gggcgcggtg gtttatattt gtaattttag tattttggga 75060
   ggtcgaggtg ggcggattat aaggttagga gatcgagatt attttggtta atacggtgaa 75120
   atttcgtttt tattaaaaat ataaaaaatt agtcgggcgt ggtggcgggt gtttgtagtt 75180
   ttagttattc gggaggttga ggtaggagaa tggcgtgaat ttaggaggcg gagtttgtag 75240
   tgagtcgaga tcgcgttatt gtattttagt ttgggcgata gagcgagatt tcgttttaaa 75300
   aaaaaaaaaa aaatgaagtg tttttagttt ttttttttgt ggtgaggttt tgagttttta 75360
   tttttagaag aaaatttggt tggggagggt atttttagat tcggatataa acgttaggag 75420
   attagggttg tttttttagg tgattaatta tagagattta gggaaagtta cgtttggaag 75480
   aggcgatatt gtgttttagt tttatttatt tttttatttt atagatattg tttggtgttt 75540
   agttcgtatc gcgaggtttt ttaagtagtt ttgggtatcg tgtatttggt atggttttta 75600
   gttttatttt tttgttttgg gagattcggt ggggatttcg ggttgagttt ttgtggttgt 75660
   ggaattttgg tggtttattg tggtggttga gaatagggac gtggggttgg acgggttgag 75720
   tgtaattttt agtggtttcg tgaattgttt gggagttttg ggtagttttt ttttattttg 75780
   tgttttcgtt ttttgatgga aaaatggggg ttagggggga gcgattttga aggtattcgt 75840
   gggaattaag cgaggtgata aatgtagtgt ttttggtaga gaattggata tagagtttat 75900
   ttttatgaag ggggttgttt tttttttttt tatgtttttt tttgttttta tttttttttt 75960
   tgtatttttt ttttttgttt tttttttttt tttttttttt ttttatatgt aggtatattt 76020
   tatttggtgt tttaattgtt tttttttatt gtttttttgt aagtggtttt ttaagtggga 76080
   atttaggttt ggaaagggtg ggtattttgt ttggtgggtt ttggtaggtt aggcgttgag 76140
   tagttttagg agatataatt ttaggaaaag gtttatgtag gttggttgtt atgacggatt 76200
   gttggtggga gaggggaagg cgtcgtggta ggggaagttt tggaaatatt tattagtggt 76260
   tttatttatg atttaggata tttgttaggt tattagaata tagttgagga agttgtcggt 76320
   tagacgttta gaagtattaa tttggtggtt gggttgtggg gggatttttt tggataggta 76380
   gataaggtgt cggaggaagg ggttgatatt tatttagtgt ttgttatgta ttgagggtat 76440
   aaagggtagg atggagagtt ggtttacgtt atttgtggaa ttttttaggt gtgggggtag 76500
   atggggaagt aagggggttt gaagatatat gcgtttatta aatgttttta aattttaaga 76560
   agaagaataa ggatatattt ataggggtag ggttgaaagt ttgatgtatg gtgttttggg 76620
   gtttgttttt gttgtaatag ttatagtggt ttttttttat tttttttttt tttttatttt 76680
   gttgtgaaaa tgtttaaaat atatggttaa gttgaaagta ttttataggg aatatattta 76740
   gttattattt agattttatt attaatatta agttatattt gttttattat tttatcgttt 76800
   atttattttt ttttcgtttg gttatgaatt tattttattt ttattttttg gtgtatttta 76860
   aagtaatttt aggttatttt gattattttt ggaattattg ttagaaattt tttttttttt 76920
   tttttttttt tttttttttt ttttttttga gatgggattt tgttaggtta tttaggttga 76980
   agtgcggtgg tgtagttata gtatattgta gtttttaatt ttcgagttta agtaattttt 77040
   ttgttttagt tttttgagta gttgggatcg taggtattta ttattatatt tagttttatt 77100
   tttaaaattt tttattaatt ttttattttt tgtatttatt taggattatt ttttataaag 77160
   ttagggtgtt gtaggagggg ttagaatttt aagttttaat tttggttttg ttattaatat 77220
   tgtgcgattt ttagtaaatt attttgtttg tttaggtttt gtttttattt ttatgaaacg 77280
   agggcgttaa attgtatgtt ataaataatg agggttatta gttattaagt ttttatttta 77340
   ggttaggtat tttttatatg ttattatgag tttttttaat ttaggtattg tttttaatgg 77400
   atagtttagt taacggaagt ttagagaggt ggtgtaattt gttaaaagtt ttattattta 77460
   gtgaatgttt ttacggggtt tgtatttagg agtttgatat agagtttagg ttttagtatt 77520
   tggcgatgtt ttgggggtgt gagtagttta gtttattttg ggtacgtgtt tatttgttgt 77580
   tttttttgtt ttatgtttgt gtttgttttt tttgaagttt agattgttat tttttagttt 77640
   taaatattaa aattggttag gtacggtggt ttatgtttgt aattttagta ttttgggaga 77700
   ttaaggcggg tggattattt gaagttagga gtttaagatt agtttggtta atatggtaaa 77760
   attttatttt tataaaaaat ataaaaatta gttagatatg gtggcgtgta tttgtagttt 77820
   tagttattta aggagaattt agttatttag ttattgaggt acgagaatta tttaaatttg 77880
   ggaggtggag gttgtagtga gtcgagatcg tattattttt ttttaggtta ggtaataagt 77940
   gtgagatttt gttttaaaaa taataaataa ataaaataaa ataaaaatta aaattaaatg 78000
   ttaaaattaa tttataaaat aaattttggt taaagtagtt tgtttatttt gttttaatta 78060
   tgtatattgt attagatttt gttaggggtt tagcgtataa agatgagatt tttgttttgt 78120
   tagataggtg ggatgatggt tatattttga gttagttaag gtattatttg gttgtgtaat 78180
   aaaagttaaa atattagagg ttgagtagta ttcggattta cgttttagtt taagtgttag 78240
   tcgttatggg ggtttaggat agttcggatt tagtttattt ttttggtgta ttttcgatat 78300
   atagttttta ttttttgagt tgagatggtt ttagtttttg tcgttatgtt tgtattttag 78360
   ttagagggaa gggaagaagg gagaggggaa agggtaagtg ttttttttta tttttaaggg 78420
   tatgatttga aagttaaata ttttagtttt tttttattgg ttagaattta gttgtatggt 78480
   cgtttttgtt attagggagg ttgagtaatg tattttttag ttgggttttt ttgtgtataa 78540
   ttaaaatttt agttattata gaaaaaaggg agtttagata ttggggaaat aattagtatt 78600
   tgtattatat gtattgatag ttttttttgt atgaatagag ttgtggtagg ttttatgtta 78660
   gggagaaagt aagattggaa aagagtttat taaggaggtg gtatttgtat tgtgtttaag 78720
   gggtaagaaa aacgtttttt aattaggagt tataaatgtt tggttgaagt gttattgttt 78780
   ttttattttg gagttggaat agacgttatt agttaattat gatggttgtt gggtgtattg 78840
   gttaatattt ataattttag tattttgtga ggttgagggt gggaagattg tttggggtta 78900
   ggagtttgag attagtttgg gtaaattgta agattttgtt tttgtaaaaa aatataaaat 78960
   gtagttgagt gtggtggtat ttgtagattt agttttagtt attcgagagg ttgagatggg 79020
   aggatcgttt gggtttagga gttcgaggtt gtagtgagtt atgattgtat tattgtattt 79080
   tagtttgggt gatagagtag gatttgtttt taaaaataat aaaataaaat taaagtttta 79140
   aaatggaaaa aaaattatga gtatataatt ttagatgtat ttttaagata gtaaattcgg 79200
   gtgggcgtag tggtttatgt ttgtaatttt ggtattttgg gaggttaagg tgggtggatt 79260
   atttgaggtt aggagtttga gattagtttg gttaatagtt ttatttgtat tataaatata 79320
   aaattagttg ggtatggtgg tgggtatttg tgattttagt tatttggaag gttgaggtag 79380
   gagaattgtt tgaatttagg aggcggaggt tgtagtgagt taagattata ttattgtatt 79440
   ttagtttgga gaaaaaaagt aaaattttgt tttaaaaaaa aaaaaaaaaa gtaaatttag 79500
   ggttaggttt gtagttatat ggttattttt ttttttgagt atggaggtag ttttagaaat 79560
   ttgttttata ttatattagt gggtagttga ttgggttagt ttgttagttg aattttattt 79620
   gttatagtta ttttttatag taaggggtat tttagatata tttttttttt tagggaagaa 79680
   taagtcgtag ttgtttgagc gttttattta gtggtgtgta tgtttttttt taattttgtg 79740
   tttagtgatg ttttgtttgt agtttgaaat ttgttatagt gggtgtgttt atattatagg 79800
   aattggtaaa tattatatat taggttttat tttttgtttt ttattagggt aggttgtttt 79860
   ttttttgttg tttttgtttt ttgttttttt ttttttttga gacggagttt cgttttgtcg 79920
   tttaggttgg cgtggagtga tgcgattttc gtttattgta agtttcgttt ttttggttta 79980
   tattattttt ttgtttgagt ttttagaata gttgggatta taggcgttcg ttattatatt 80040
   cggttaattt tttgtatttt tagtagagac ggggttttat cgtgttagtt aggatggttt 80100
   cgattttttg atttcgtaat ttgttttttt cggtttttta aaatgttggt attatgttat 80160
   tgcgttcggt ttagggtagg ttgttagaaa tgtattagtt tgttagtttt agttttgttt 80220
   gtttttattt tttttttttt tttttttttt ttttgagacg gggttttttt ttgttgtttt 80280
   ttaggttgga gtgtagtggt gtgattttgg tttattgtaa tttttgtttt ttgggattaa 80340
   gtgatttttt tgttttagtt ttttaagtag ttgggattat aggtgtttat tattacgttt 80400
   agttaaattt tgtattttta gtagagatgg ggttttatta tattggttag gttggtatcg 80460
   aattttttat ttaaggtgat ttattcgttt tggtttttta aagtgttggg attataggcg 80520
   tgagttatcg tattgggttt ttttcgtttt tttttaatga aaattttttt gaaagatttg 80580
   tttattgatg tttgtgttaa taggtattgt ttgcgggata gagtttgttt tttttttgaa 80640
   ggtttttttt tgtttgggtg attatttgta tgttattttt tgttgtttag aattaaagat 80700
   gtagttatgt ttggggtagg ttgggaaaat ttgtgggaat tatagagttt gttgttatgt 80760
   tgtgttttgg atatttgaag tttggtaggt aggtgggatc gagggtgggt attaggaaag 80820
   tgatgtggtt ttataggaag gggatatagg ttttgagatt ttaggttttt aattggataa 80880
   agttgttttt tttttttgga taaatgtttt agtttattta taatcgagtg gttagttttt 80940
   tttttttttt tttttttttt ttttttgaga tggagtttcg ttttttcgtt taggttggag 81000
   tgtagtggcg ttatttcggt ttattgtaag tttcgttttt taggtttacg ttattttttc 81060
   gttttagttt tcggagtagt tgggattata ggcgtttgtt atcgcgttcg gttaattttt 81120
   tgtattttta gtagagatag ggttttatcg tgttagttag gatggtttcg attttttgat 81180
   ttcgtgattc gttcgtttcg gttttttaaa gtgttgggat tataggcgtg agttatcgcg 81240
   ttcggttaag tggttagttt ttttagttgt cgggttttgt atattttgtt ggttttggag 81300
   attatatgta ttatgtagat tttatttgtt ttttattttt agtgttgggg gaggggttaa 81360
   gggtttgtcg atgtgttgat atttggttat ttattggttt tgaatgttta tatttatttt 81420
   tttgaaatcg atgtttcgta tcggaggttt tatgtggatt tgggggtaga ggtttgtatt 81480
   ttagatggtg gggtggtttt tattaattag gtagattttt tgttttgttt tgttttttgg 81540
   gttttgtagt tttagatgtg tgtttaagat ttgtggttat atggatggga taagttatag 81600
   agatttagat ttcgtggttt ttgtgattag agtagtcgtg gttttgtttg ggaatgtttt 81660
   tattttgttg ttgaaggtga gaaatttttt tgtgttggag aatagttgtg ttgagaagtt 81720
   ggtttatttg gtttgtagga ttgttggggt aagggaggtt tgttgtcggt tttttttttt 81780
   tttttttgat tgggtgtttt ttgtttaggt tttttgtagt aaaagaattt tttaatttat 81840
   tttgtttttt tattgagttt ttattttatg aagtttattt tttaatttta gttgattggt 81900
   ttttattttt ttaagaggaa ggttaggggt agacgaggtt gaaggaggtt gatttttttg 81960
   tgttttcgta gtttgagttg atgttgttgt atgtttatag ggttttttta gtttttaaaa 82020
   aggttttaat acgttcgtta tacgtttgtg gtttttttgg atgttatatt aatttagtaa 82080
   ggtttgagtt agaggttgtt tttgaggttg tgaaaataga agaagggttt agaggagaat 82140
   ttttttattt gacgatttta ggaaggtttt ttagaagaag tagtttttga gttgagattt 82200
   gaagatagat taggtttgag ttaggttagg gagggtgtga ggggtttatt aggtagaggg 82260
   aataggatag ggtaggtata gaggtttggt atttggtttg tggggaagtg tgagtttttg 82320
   ggtatgatta aggcggggag aagtggcgtg agaggaggta gatgtggttg gtaggagatc 82380
   ggcggtttcg gttttgttga gttttatttt gtatgtatta ggtgatagag gttgtttagg 82440
   acggtgggta gggaagtgat aggttatatg gttattttat taatagtcgg gagtttagtt 82500
   tggggggatt taagattttg gggaaggtac gagttagggg tttgttagag ttaggagtta 82560
   gtgggagaag gaaatagtgt tgtttatgaa ttataaatag atattttcgt aatttttttt 82620
   gacgtgatat aattttaaag tgggttattt agaggtggga aggtgatttt tggtttttta 82680
   ggttttgggt ttgttttttt tttttttttt tttttttttt tgagatggag ttttattttg 82740
   ttgtttaggt tggagtgtag tggtgtaatt ttggtttatt attatttttg ttttttgcgt 82800
   ttaagtaatt tttttgtttt agttttttga gtagttggga ttataggtat ttgttattat 82860
   attcggttaa tttttgtatt tttagtagag atggtgtttt attatgttgg ttaggttggt 82920
   ttcgaatttt tgattttagg tgattcgttt attttggttt tttaaagtgt tgggattata 82980
   ggtatgagtt attgtgttta gtcgagttta tttttaagtt ttttttggtt tttagtgttt 83040
   ttgtattagt ttattttttt ggttttttgt ttttaagagt tttttttaag attgttttta 83100
   agtggatagg tttttagaat taaggagtag ttttaggagt ttttagttat agatatttta 83160
   gggggttcgt gtggttttta gtttttggtt tggtttcggt agttttggtt ttttgtgttg 83220
   ttttttatat agtttggtta gagttggtta ggttttttgg ttgggtcgag ttatcgtggt 83280
   tagtgattta tgtaggggga gaaaagtgag gagggggaat agagtgcgag ggggattgtg 83340
   ttaataataa atttattagg tttttgaggt tattttatag gagggataaa tggtttattt 83400
   aggttgattt ggttgtcggt tggtttttag agatatttag ggtggggaag attttattat 83460
   ttggggaaag taatattaga ttgtttggag gggtttagtt agcgtatttc gcgttttttg 83520
   tttgtttttt acgtagagcg ttttgttttt aaggcggtag gttaagagcg cgagtcgagg 83580
   tttaggtgta aagaggggtg cggtatgggg gtattgtagg gtacggggtg tatgtatttt 83640
   tatttatttt tttattgagt gttagggtaa tttgttaatg gggtatattt ggtgatataa 83700
   aattttttgt cgatttatgg tagtttggaa cggagtggaa gcgttttatt tttggggaat 83760
   ttttatatat atttggttgt gaacgtcggt gtttttgtat tggagttttt ttggagtgtg 83820
   ttgggtagag cggagaggtt tttttttttt tttttttatt attttgtgga ggtgttaata 83880
   tgtagttaga gatggttagt ggtagtgagg agaggggggt aggaagagat tagggttggg 83940
   ggtttgttag tttatagttt tatttttgaa tatttatggt ttttttgttt aggttagcga 84000
   ggttgggggt agagacgggt aggggttttt taatgttggg tattttttcg tttttttata 84060
   ttttggattt gttttttgga gaaagttttg gagtatttag aagttttggg gttgttggtt 84120
   gaggtgggga gtagagttta gttgtgtttg gaggtttttt atttttattt agtttttagg 84180
   gatagtttta gttgtgtagg tttaggtcgt tgaggttggt tttagaaatt tttttataat 84240
   tattattgga tggaaaatta gttttttaaa gggttttaga gttagttttg tttttttttt 84300
   tataggtttt tatagttaga gtatcgggtt tttgttagag atgtagaaga gacgatgttt 84360
   ttagttttta tgtggttcgg tttttattgt ggatgttggt gattttagtt ttttttttaa 84420
   aattaagagg gttgatgttt tatattttag gtttttagaa agagtcgtta tttatttata 84480
   tcgttatatt ttttttttat ttttttgttt cggaaagagt agagtggata tgcgtttttg 84540
   tagaaattta tttatagttt gattgtagat gtattttgtt tttttattga gtttttgttt 84600
   tatgaagttt gttttgtttg tatttttttt tttttttttt tttttttttt tttttttttt 84660
   tttttttttc gttttttttt tttttttttt ttttttgttt tatttttttt tttttttttt 84720
   tttttttttt tttttttttt tttttttttt gtttttttta tttttttttt tttttttttt 84780
   tttttttttt gaatagttaa agatagaatg aatggtatag gtaggtattg agttttttgt 84840
   aattggaggt gtttaagtgt agggtggatg ggtatttata gggtaggtat tgaggaagga 84900
   tttttttggg attgtggatg gtaggatagg ttttgtggag ttttttgagc gggtgtagaa 84960
   gagggagggt atgtttttgt tttttgtttt tttcgggttg ttaggtcgtt atttttaatt 85020
   tttatttttt ttttttttat ttgttgggtt ttacgggatg ggtagaggta ttgttagaat 85080
   atggagtcgc ggtttataga gggttagtag ggaggagagt tttttgggga ttacgtcgtt 85140
   ttagttattg tggtgatttt gaaaataaat gttttttttt ttgtttgagt ttttgttgtt 85200
   tttgttgacg tggaattagg gtaggttggt gatttagttt ttatttttgt attgtatcgg 85260
   tggtaagttg tttttagttt tagattttat agagtagggt gtttttttta gaaaaatagt 85320
   agggaggtta ggatattaag ggttttaggg atagtttgtt ttttttggtt ggtatgagtt 85380
   ttagttaagt tttttggtat tttgggggtg aggaggtatt agttgtgtgt tttagttatt 85440
   ggttgttttg aggttgggtc ggtgttaggt tggttatttt tttttgtttt ttagtgtgat 85500
   tttgtagagt tttcggaagg gttagagggt ttttttttat tattggtgtt tatgggatat 85560
   tgtgttttta ttttattttt gtattttagt tttagacggg tgttaggaag ttggagattt 85620
   tagttatttt acgattgtgt tttgcggggt tggagtagga ataggagtag gaggaacgtt 85680
   ttggttatgg gattgtgtga agagagaagg attaggaaga aaatatgagt tattatgttt 85740
   gggttatatt ttagtagagt cgttggtggt tttttaggta tttgaattta ttaattataa 85800
   agtttattag gtgtgtaaat aaataaatgt tgtttaggag gtatagttag ggttttgttg 85860
   gagagggatt attttggagg ggttatattt tttatttagt tttttagttt ttttgttttg 85920
   tgtgtgtgta gtttttgatt attattattt ttgtttttgt tatttgtttt tagggtttta 85980
   ttaggttgga tgttattgta ataaaatgta gtaatagaag gtttaggtag tgttgtagtt 86040
   tttggtggta ttattagagt tagtaggtag tagggttggg gaattttaag ttgatttagt 86100
   tttaagtgtc ggagcgggga gttcggatga ggtgggggtg gggaatcggg gagttatgga 86160
   gtagagtatt tgttgtgttg aaggttagga gtttttttgg gagattatga atttagaata 86220
   ttaatatttt gtttaaaaag ttttttgttt tggagataag agtttaggtc gttgtggtta 86280
   ggttttgttg taaatgtttt gtataaagta ggttgggggg attattggga agtttgggga 86340
   atagattggt tatgtgtttt tttgagtgaa attttatggt tgtattgaag cggttaggta 86400
   gagggtgtga tggggtgtgg tagttgtata tcggtttagt taggttgaat ggggaaatgt 86460
   aaatttaggt ggtttgaaag agatttagtg gtttatagtt tggtggttgt ttttagtata 86520
   ggtgatgtaa agagagaagg ggtttcgggg gtttggttta ggtggaaatt atgtattgtt 86580
   tgtgattttg ggtatgtcga ggttggagta ggtttgtttg gtggttggta gaacgtatat 86640
   gaagaattta tgtgtgtttt gaacgttttt tttgtttttt tgggtttatg ttttattagg 86700
   agtttttcga ttttgttatt ttgtaaagga aattgcgttt tgttattgta taggatcgaa 86760
   taggtgtgtg tggagttttt aaagttattg ttttaagggg ttttatggga taatgggaga 86820
   gttgttgtga tgtttaagat taaagtgatg gttgggcgta gtggtttacg tttgtaattt 86880
   tagtattttg ggaggttgag gaggatggat tatttgaggt taggagttcg agattatttt 86940
   ggttaatacg gtgaaatttt atttttatta aaaaaataaa aattagtcgg gtatggtagc 87000
   gggtgtttgt aattttagtt attcgggagg ttgaggtagg agaattgttt cgatttggga 87060
   ggcggaggtt gtagtgagtc gagattacgt tattatatta tagttcgggc gatagagtga 87120
   gattgtttaa aataataata atgttaataa taaataaaat aataataata aaataataaa 87180
   agtaaggatt tgaataaata tttgtatatt tatgtttgta gtagttttat ttttaattgt 87240
   ttaaatgtgg aaggaatgga tatataaagt gtgtgtatat atagtggaat attatttagt 87300
   ttttaaaagg aacggaattt tgttattata tgtgaatttt gaataatgaa ttttgaagat 87360
   tttatgttac gtaagtttgt tttataagga taaatattgt atgattttat ttatgtgaag 87420
   ttaaatttat agagaaattg gaatgacggt tgttagtagt taggggaagg aaggatgggg 87480
   agttattgtt taatgaggat agagttttag tttgggaagt tggaagaagt ttcggaggtg 87540
   gatggtgttg atgggtgtat aatgtgaatg tatttaatgt tattaatatg gaaatttggt 87600
   taaaatggta aatttgtttt ttttgttttt gttttttttt tttttgagac ggagtttcgt 87660
   tttgtcgttt aggttggagt gtattggcgt gattttggtt tattgtaatt ttcgtttttt 87720
   aggtttaggc gattttcgtg ttttagtttt ttagagagtt gggattatag gcgtttgtta 87780
   ttacgttcgg ttgatttttg tatttttagt agagataggg ttttattatg ttggttaggt 87840
   tggttttgaa tttttgattt taggtgattt atttattttg gttttttaaa gtgttgggat 87900
   tataggcgtg agttatggtg gttggttaaa aatggtaaat tttatattat atatatttta 87960
   ttatagtaaa aatgtattgt tttattagaa aaatgataga tttttataat atgaattaaa 88020
   ttataagtta tgaaggaaaa attaagtaaa atattaaaaa attatttata agtaattata 88080
   tttaaaataa aagataataa atatataatt tttattattt tttaggtttt ttgtaatatt 88140
   taattattgt ttgttttgtt gcggttatgt ttttgaggtt gtatttgtat gatagaaata 88200
   ttatattcgt ggtgattaaa tatttttttt tagttttatt tttaatgatg ttatatagat 88260
   aatttgaaat tggtcggttg ggtacgatgg tttatgtttg taattttagt attttgggag 88320
   gttaaggtag gtagattatt tgagtttaaa attagtttgg ttagtagggt gaatttttat 88380
   ttttagtaaa aatataaaaa ttagttagga gtggtggcgg gtgtttgtaa ttcgagttat 88440
   ttgggaggtt gaggtaggag aattatttga atttgggagg tggagtttgt agtgagttag 88500
   gattgtttta ttgtatttta gatcgggtga tagagtgaga ttttgttaaa aaagaaaaga 88560
   aaagaaagaa attggttaag gtaggaatat tgatattata ggaataggta agcgttataa 88620
   atcgggaaat tgagtgttgt tgtttttgtt ggttttaggt ggtcggttgt tgatcgttaa 88680
   ttggtatatt attggaggag ggaggtttag attgattcgt agagagatta gaggtagaaa 88740
   cgtattatgg tttggatgtt gagggtgagg gaaagagagg agttaatagt ggtgttcgga 88800
   gatttggttt gagtaattag gtggatggta gtatcgtttt ttaagatgag gggttgtggg 88860
   aatttgaggg gttgtggaag gttttgttat tgacggattt agggttcggt agttttggga 88920
   ggtttatttt gtgttaggtt acgtagagat aagattattt gggttttgtt tttgggaagt 88980
   ttttgtttat gaggatatag tttgttattt aatggggtgt ttcgtttgga gttttagtgt 89040
   tgttgggaga aagtttttta ttttttttcg tttttcgagt tttttttgtt tttttgttag 89100
   ttgagttttt ttatggaggt tgtgtttgga ggggggttat ggatatggat gtgtgcgtta 89160
   aacgttgttt tttcgtggat ttttttttta tttcgttttt acgttttgta agttttattt 89220
   tttcggggtt tgatttgtgg ggtttttttt tagatttgtt tttttttatt tttagaaagg 89280
   cgtgtagggg ttttttatgg tttttttttt tgtttttatt tttttttttt tgggatagtg 89340
   atttgttgga cgggtgggtt atttataggg tttgtgattg tttagatatt tttgttgtat 89400
   gaagttttga ttttaaggta tgatgtagat gggaataagg tacgagtttt ttttggttgt 89460
   tgtaataatt gtcgaaattg ggtggtttat agtaagggga atgtgttttt ttatagtttt 89520
   ggaggttaga agtttgaaat taaggtgtta gtagggttag ttttttttga agattttagg 89580
   ggaaaatttt tttttgtttt ttttagtttt tggtggtttt aggtgttttt tggtttgtgg 89640
   ttgtattatt tcggtttttg tttttgtttt tataaggttt ttttttgtgt ttttgtattt 89700
   ttttttttgt tttttagaag gatatagtat tgaatttagg gtttatttta ttttgagatt 89760
   tttgtttaaa ttttttttgt agagattttt aaatagaatt aggttatatt tgaggtttta 89820
   agtgggtata tttttgtttt gggggggatt atcggtttat tgtaggtttc gttgtgtatt 89880
   gaagacgtag gcggtttttt tggttaggtt tgtagtcggt tttgtttttt tttgtgatcg 89940
   tggaagaatg tattttgttg gatgtatagg tttttatagt ttttttgttt ttgttaaatt 90000
   ttagtttagt tttttttttg ttgtcggcgt gtattttttt tgtttagtgt tttttttggg 90060
   attgtgtgtt taggttttga ggttttagaa aaggggggtg gagggttttt ttattatttt 90120
   gtagtaatga ggttttatta aaattttgga gttttagatt ggatttagtt tttagttatg 90180
   attaaaggat agttcgggat atgattaaag ggttagagaa agatgattta ggttgagaaa 90240
   gaggaggtta aggagtaatt ggattgtttt tgaatgtgga aaaggtaatt atgatttgaa 90300
   gggtggtgtt tagttgtttt ttattttttt tgaggatagg aaaaaaaagg aaataaatga 90360
   tgtataatta gagtagttgg ttagatacga gggtgaattt tttggttggg agtatggtta 90420
   agttttgatt tgggtttttt gaaagagttc ggggaatttt tttatttgga ggtttttagg 90480
   tttggttatg tggttttgag tgattatatt gaaggggtag tggttggggt tggagtcggt 90540
   gggttttgag tatagttacg aaggtatgcg tattttttgg ttgttttttt gttattgttt 90600
   tttgggtttt tattttggtg aggcgtgtat tttggcggcg ttttttaggg aattaaatat 90660
   gtttgttgta ttgtgcgtgg agatggagaa tgtataattg gttgattttg tgttaatttg 90720
   gtggaatttt atgttagttt tgggaaagaa taattgtatg ggtgtgttta tatttattag 90780
   gtgtttttta gaaaaatatt cgagaataat gttgtggttt aggatggttg ttgtgtcgga 90840
   ttcggtattt ttttaggggg gttgtgttgt tgggttgagt tttttaggta ttggattttt 90900
   aaatttttaa atacggcgtg gataggtggt ttagtagggg ttggattatt cgataggttt 90960
   aggtgttgga gtttagataa gatatatttt ggtttggcgt ggaagatacg gggtgttatt 91020
   aatggtagta atggttgtat ttttgaaatt cgggttttta ggtcgacgag ggtgtgtacg 91080
   tatttgaaat gtttgtggtt ttgtagtttt tatgtttata aatttatttg gttgaaaata 91140
   gtttaaaata tttaaagtat gagggaggga gtgtttgttt tttttaaaaa ggaaggattt 91200
   gattttattt atttaaaaag ttatttaaat ttagaatatt tttcgtaaga gattttttgt 91260
   ttttcgtttt tttagaatgg ttggagagtt ttagtatttt tgtatatttg ggatatttta 91320
   gagggggtgg ggaggggtaa gtgggtagcg agcgatttta gatttaggat gagttgttag 91380
   gcgtttttcg gttatatatt taagggatcg gagtgtagtt gtagcgttgc ggtttgttgt 91440
   ttcgggggtg ggggtgttgt tttatgttgt gaatttttat atggtttttg attttgggta 91500
   gaggtcgagg gtttaaggga cggggtgata gggagagtat gtaggagtgg gtttttggtt 91560
   ttttagggcg agtggaagaa gcgttttttt tttttgtagg tgatagattt gggggttttt 91620
   ttttgaggat gagagtttgt tgttttttaa gttttgtgtt taatttaggt ttttaggttt 91680
   attttagttt ttcggttttg tttgttttgt ggatgatata gtttaagggt agagatcgtt 91740
   ggtttggagg gaaggttagg ttttaggtta gggtttagaa gggagggaga agtttttggg 91800
   gtagtttttt ttttgtttat ttattgttta gttttttttt ttatattttt tttcggaaac 91860
   gtttgttttt gataaggttt attttttgtt tttaggaggt ttttattgtg gaggaaggga 91920
   ggcgtcgttc gtttttggtt tttttgatag tcgtgtttta ttttcgtttt gtgttttttt 91980
   tttcggatag tgtttttttt agggtttatt taggagggtg tagcggtggt tttcggggcg 92040
   gtggtcgtgg tgggggtgtt agttgtaggg gtgttttcgg tgggtgggag ttggtggttt 92100
   ttcgttggtg ttatgggatt cgtatgttcg ttttgcgttt ttcggttttt gagtttatag 92160
   gtcgggattt tgtttgttag tcgcgtgcgt tgtcgtttaa tttttgtagg cgtagagcgc 92220
   gcggcggcgg tgatagagaa ttttgtttgg ttgtttaaat atagtttttt gtagaaggat 92280
   tttgcgttcg gggaagggga ggaatttttt tttttttggg cgttcgtttt tttcgttatg 92340
   gttcggtttt tatattcgtt tatatttggt cgtagcgggg cgttcggggg gaggggttga 92400
   ggtcgcgttt ttcgtcgttt tttgggcgcg ggttaggcgg ggaggagggg ggcgtttcgg 92460
   tcgtgtgttt aggattgttt tttagcggtt attcgggttt tagtttttta ggtttggttt 92520
   tgataggcgg gcggagtagt tagtgcgaga tagggaggtc ggtgcgggtg cgggaatttg 92580
   attcgttcgg gaggcggggg cggggcgggg gcgtagcgcg cggggagggg tcggcgttcg 92640
   tttttttttt ttatttattt agttgagtta gggggtttag gggttttttc ggcggttagt 92700
   tttgtattgt aggagcgcgg gcgcggcgtt ttagttagcg cgtagggttc gggtttcgtc 92760
   gggggcgttt tttcgtcgtt gtttttcgcg cgattcgttg tttattagtt attatgtcgg 92820
   atttcgcggt taacgcgtag ttggatggga ttatttcgga tttcgaaggt gggtgttggg 92880
   ttggttgttg cggtcgcgga cgtgttggag aggattttgc gggtgggttt ggcgcgggac 92940
   gggggtgcgt tgaggggaga cgggagtgcg ttgaggggag acgggatttt taatttaggc 93000
   gttttttcgt tgagagcgtc gcgcgttttc ggtttcgtgt tcgcgtcgtt tacgtggggg 93060
   attttgttag gggtattcgc gtagattttg cgcgttttta taggattttg tgttcgtttt 93120
   gcgtattgtc gtttgggttt tttttttttt attgttgttt gtgtttgtta agcgatagcg 93180
   attttttcga gggttcgcga ggttgtttcg gaatttttta ggacgtatag ttttattttg 93240
   ggaaatttat cggttttttt tttttggttt ttttcggcgg ttttcgggtt tcgtttggat 93300
   tcggtaacgg gatagggagg tcgtttttta ttttcgattg agtggatagt cgcgttttgt 93360
   tcgggtggat agtttttttt ttttttacgt tagtttcggg gtcgttaagt tgtgtagttc 93420
   gtgggtcggg agtatcgaac ggatatagtt taggtcgtgg tagggtttag agtgggatgt 93480
   tttatggttt ttatttaggt ttggggatat ttttattcgt tttttagaat cgggtcgtgg 93540
   gggatagaag gggtttgcgt gcgggtaggg agagtatttt ggtttttttt tgttttcggg 93600
   gtttataaag tgtgttggga tttgcggggt tgttttgttt aagtttgggt ttggcgttcg 93660
   cgtttttgag tttgtgagtg cgtgcgtttt tttgcgtttt tttgattgtc ggtgttgggg 93720
   ttttgcgttt tgcgttcgcg ggagtaaata tagtaggcga aggggaagtt tatataatgg 93780
   tttttagcgt tttggggtag ggtttttgag gggcgggttt gtttttgtcg ggatttggag 93840
   ttttcgtttt tcggagaggt ttttaggttg atttgggtag agttttttgg tgggtcggga 93900
   gggggaaagg ttgtgttgaa atgagtaaat tgtttaggtg ttaggttaag ttgggaggtg 93960
   attagtttga ggtttttttc gttttatggt tagaattagg gttgatattt gggtgttttg 94020
   agtttagttg tttatacggt ttatttgggg ttagttttat ttgagtgggg gaggcggggt 94080
   tttttggggg attagaattt tggttggacg ttaagtagag tgttagtggt tgttttttag 94140
   ggttgggttt gaggagggtg tggggcggcg aagggacggg agggggttgt gatttagtgg 94200
   ttattggcgt tgtgtagagt gtgagttgga aatatcgtag ttattttgtt agtttagtgg 94260
   tgaaagtttt tttttaggtt ttattttttt gtatttttgt tttttagagg gaggggaggt 94320
   ttgggtttgt agagttggga gggtttgttg ttttcgtttt ttttttttat aatatttttt 94380
   tatttggata tttttgggta tatgtttata ttggggtttt tttaggttta ttgtgtttcg 94440
   ttgagttttt tgtagttttc gagtgaatgt gatttttttg tttttgtttt tttgtaattt 94500
   ttttttgcga tcgttttttt aggggttttt tttgttttaa atgtttaagt ggtacgattt 94560
   agtcggtttg attatttttt agtaagtttt tatggagaga ggttttgtgt tgtgtagagt 94620
   tttttttttg tttgcgggat cgaggttttt gtttttagtt tttaatagaa agtgtcgggt 94680
   ttttagtggg atttttgggg aagaattttc gtgttttaac gggagttttg tggcgggagg 94740
   ggaggttagg gtttggggtt gtgttcgttg tatagttgtt attatttgta ttatgaaagt 94800
   tgttagtgtt ttttttttgg gtttttgggt gtaattttat ttttgttttt atgtgttttt 94860
   atttggagtt gtttttgcgg ttgtttttta agttagtttt gtgattttgt aatttagttt 94920
   aagataatgg gtttattgag attattttgg tgtagtagtt ggtaattttt tggttttggg 94980
   ggaaggtttt ttagtttcgg ggagtggggt tttaatttgt tggttttttg tgtttattag 95040
   tttttttttt gtgtgttttg aatggttttg ttgggaattt tggttttaga gttattaggt 95100
   ggttcgagtc gataggcgtg agagagtgtg tgtgtgtatg agtgcgtatg tgtatggggg 95160
   ttgatttggg gtatggaaag gtggtttttt ttggtgttta aggagtttgg agtatagttg 95220
   gagggtgtgg gggtgtgtat atgggagttg gataattttg ggtggataga tagacgtggg 95280
   gaagggatga ttgaaggagg tggaggagag agtgtgattt agtttagtta ggggtgatgt 95340
   ggataggtag ttttcgaatt agggtagaga aaagttatta ttagttagta ggggagaagt 95400
   tagtatggag gaggcggatt ttgagggaga gtaggaattg gattgtaaga ggaaggagag 95460
   ttttttggtt agtagtagtt agtagtagtg ggggaggttg gaatgagttg gttggagagg 95520
   gggttggggt ataaggaggg gtttgtttgt gaagattata tgggttaggt tgcggagggt 95580
   taggtatgtt cgtcgggagt gtagttggtt tacgggaagt atttggagtg gttgggaatg 95640
   ggcgtaggag tagcgtcgtg ggagtatagg tttttttttc ggggcggttt atttggtgtt 95700
   ttggtttttg taaggtaggt cgaaagggtg gggaggaaat tgttagtttt ttatagcgtt 95760
   gggatggtgg ttttagggtt tttgaggtta gcggatgtgg gtgtttgtta ttatgtgggt 95820
   tgttgagggg cggagatttt aggggttatt ttaaagtagg acgagttttg agttacggta 95880
   tttttggggg tagtttttta atcgagtaga cgtttaggtt tggaattttg taatagaggt 95940
   tatagggttt tgattagggt gttttgggag gtttagaatt agtggtagta tatagggtag 96000
   acggtaagtg atttggtatg gggaaagagg taggtgttta ggtcggtata gtatattcgt 96060
   aaggaatagg tagacgggaa gtcgttcgtg ggtttgtgtg tgtgttcgga gttaaaattt 96120
   tgttaatgtt ttatgttttg ggtatattta tttttttttt ggggagtatt ttttttttta 96180
   tttttttttt tttcgtttgt tttttttatt tagggttttt tttatttttt tcgttttggg 96240
   gatcgagggt attatggttt tatgttttat tatcgatgag ttgtataggg atttagtttt 96300
   ttcgttgttt aggtcgggtt ttttaggttt agggttttta ggaatggaga gggtattagt 96360
   gttttttatg gatttaaatt tttcgtattt cgtttttgtt ttttttttaa gataggtttt 96420
   cgagttttaa ggttttaggg ttttgtggag gtcgttacgt agtagtaagg agaatgtttt 96480
   gtatttggtt gatgagattt ttagagtttt attttttatt tttttattgt ataaacgggt 96540
   ttttaggcga ttgtagtatt cgttattgtt cgtaataggg tgataagagg gatgattttt 96600
   tttttttttt tttttttggt tggtggaggt acggggttgg cggacggtat gtgttttcgt 96660
   gaatttaggt taaatttgtt atcgtaaata cgattataat tcgggttttt gtgtaataaa 96720
   agttttttta agtattagtt gttggtttgt tttgtttagc ggtgtttgtt gtaattagat 96780
   ttgtatatcg agaaagaatt taaaagtttt tgatgtttgt tgaaataatt tggtttagga 96840
   tttacgtgtt tagattttag agttgtgtgg tatttgagtt tttttcgagt ttttattgtc 96900
   gttcgaggag gatttttaga tttgtgtttt ggaggtagag taggttgtgg gacgggtttt 96960
   tgggtgggaa ggattatgtg gatatgtttt tttgtttgag agttttaata ttttcgggac 97020
   gtgggagttg gcgcgttggt aggatttagg tgtttttttt ttttttagag aaaaaggttt 97080
   cgttgtttgg taataggtgt agatttgttt ttaattaatg ttagtaggtt ttttgcgtga 97140
   tgaattttgt tttttagtta agatttaagg tattttgtga atattgtttt tttgtagttt 97200
   gagtttttgt ggtgggaggt aggagttatg gggagtgggg gtaggttttt tatacgggtt 97260
   ttatagttat tggtagtatt gatttgatgt tttttgagtt tagagtttag ggttagatag 97320
   atttattgtt tcgattacga gttggtttat ttagaggggg gcggatatag tatttaggta 97380
   gtagatgtat tgtgattagt tttgtagcgg ggttgtgggt tttttgggtt ggatgttcgg 97440
   gaagaggtag gtggaggtaa acgttaggat atttttgtag tgattgggtg attgtaggtt 97500
   ggaaatgttt tttgtgggtt gtggttgttt aggaaggttt tgaatggggt tagtggatag 97560
   agtttgtatt tagaggggta gtgttttgga ggagtgaggg gtatggtagt gtagggatgt 97620
   ttaggtcgtt tttattttgt tattggaaag ttgggcggtt tcggtttttt tagttttttc 97680
   gtttgttttt ttgtttgtaa agtggggtta gaaatagttt tttttgaggg ttgttggggg 97740
   attttgagat gtagtttatg gcgttgagta cgggttttgt tttttacggg tgtggtgggt 97800
   gtcgcggttg gtgtggtatt tgggcgggaa aagggggtat ttgtaaagga taggtaggtt 97860
   tggatgttta aatatgtaga tttggggatg ggaggtttta ggtaagggtt tgtgtgatgt 97920
   tattgtatga atgaggtcgg atagtatggt tattattaat tatggaaagt acggtagata 97980
   ttagtgttag tagcgttcgt ttgggggttt ttttgagttc gataggtatt taggttttta 98040
   gtgtagttat gggatttttt tttttttgtt tggttgatat tttggaggtt ggcgttatgt 98100
   tggttgtagg ttttttttat tgggaggttg ttttggtttt ttttgttatt atttattttt 98160
   atttttattt tggagtgtta tagatgggtg gattatatgt ttatttgtag ggttaagcgt 98220
   tgataatgag gtgagtattt gttggatgat cggattatga ggatagcggg ggcgttggta 98280
   gtttgcgttc ggttttgtag gttggttttt ttttgaaaag cggttgtggt agagtgtgta 98340
   ttttagggag tggtgtttgt tgttttgaat atttttattt agtaggtttt tgtaagggtt 98400
   tagttaagtg tggtgtagag ttgggttggt agttggaata gttttatttt gtttagtggt 98460
   gagattaagg gttgatgggt taaggttgcg tgttgtttgt ttagttcgtg gatttattta 98520
   ttgagtattt ttatattttt atattgggtt cggggtgtta ggaggtggag gggggattgg 98580
   gtgtttggaa aaataagttt atcgagagtt tgagttttta ggggttagta ttaggtagtt 98640
   attacggtgt tcggtatata gtggttattt ggtttcgtcg gttgttttgg tggtggagag 98700
   cgtgtatggt ggtcgtacgt gatgggtatt atttagggta atatagatat gtagattgtg 98760
   tatttgtttg taggagttag ggatttttta gtttggtaat ttgtgatttt tgtttattcg 98820
   ttgggagaga ggtgttgttt tttgatagtt tggtgtcggg agagtagaat tagggttttt 98880
   tttttttttt tttatttttt attttttata tgtttaggta ttaggggagg ttatatttag 98940
   ggatgagttt tggttattgg gtttagggtt ttattgttgt tatttttttt ttttgttagt 99000
   tagttaaatg ttcgtttatt atcgtgtcgg ggatggggtt ggtttgtttt tttgaagttt 99060
   ttttggtgta tagtttaggt tggagtttcg gacgatttcg ttattttttt tttcgatttt 99120
   ttttttttat tttgatttat tttagaaatt tagttaaaaa ttaagttttt gtgttttttt 99180
   ttgataaagt tattgaggaa aaatggttaa atattgtttt tttttttaaa gtttttttgg 99240
   ttttttttaa atttggaggt tttgagttgt ttatgagatt aaagggttcg gaattttttg 99300
   gaatatagtt ttagaagtgg gaggatttgt tattttttta gttaaataga tttattagtt 99360
   ggtttttaga tatttttagg cgtttggggt atttttgggt ttaggttgtt taatttggtt 99420
   tgtagagttt cgaggtttcg tttagattgg gttagtagat agttttagag attttaagtt 99480
   tgttgtcgtt tttaaaagtt ttgttaatgt ttagaaattt agagggataa gaagatgatt 99540
   tcgatgttag tcgggattat aaatagtgag gttcgttttt ttttttattt atgtttgttt 99600
   ttattgtttt cgtagttggg tttttagggg attgttcgtg gttatggggg aggtcgggaa 99660
   tgcggggttt agattttatt gggtttttcg tatttttttg tttttttttg tttatttttt 99720
   ttttcggaaa tttttgaata tttttttata gtttttggta ttttgttatt tttggtattt 99780
   ttgggttaat tgggaaagtt attatttgtt taatatttag aaaattaagg ggtttggttt    99840
   taaatatatt gtagtgtttt tttttttata tggtatcgaa ttttttgtgt ttttaaagtt    99900
   aggagtttgt gttttggatg tgggcgatgg agttttgtag atatttggag cggttttttt    99960
   gggagagttt ttttcgtttt tttatttatt cgtttttttt tttttgtttt aagttttggt   100020
   tatagtttcg gaggggttat ttgagtgtag gttgttttta ggtgtatagt tggagggggg   100080
   tagttatttt atattttttt gtttgttagg ggtattttta gggatttagg ttttgaggga   100140
   tagcggaagg gtggggtggg gagtaggtcg ttgtttcgtg ttatttgggt gttatgtaga   100200
   ttttgaatgg gtagtttttg ggagttggtt tttttttttt atttttttta tagtgttgtt   100260
   tttttgaggg gttggagttt tttggagttt tttgtagttt gtggtaggtt tttttgattt   100320
   tgtgtttggt ttcgtagttt ttaatttata gttagtggta tgaagaatat ttttggttgt   100380
   ttttatttgt ggtttttgta ggtagtagtt ttttggggta gggtttgggg gttttggtat   100440
   ttttttgtgg gattttttgg gtatgaggga gggagttatt tgggttatag atagattttt   100500
   tttttgtatt taggaggttt aggatggatt tgttttattt ttttttttgg tgggaagaga   100560
   gtatttttgg ttatggggaa tgtggtttgg atagagttat ttagtttttt ttttttttta   100620
   ggtagggttt tgaaagatgg agatggtttt gtttttgtta ggttaatagt tagtagtttg   100680
   gtggattttt atgtagttta ggtaggattt ttttttgttt ttatttttta ttttatgtgt   100740
   aggaagttgg ttttaagtag ggatttttgg tttcgttttt tacgtgggtg ggtagttttt   100800
   ttgcgagggg taggttaggt ttttgtttta tgttttttag agatcgatgt tatttatagt   100860
   aagagtatat aagttttttt ggttttaaat taggtttgga ggggtggttt ttttttttgt   100920
   tttttgtttt ttgtttttta ttaaaggtta gggaatggag tgttttgtat gtttgttggg   100980
   gttgggtttt tttttttggt ttaaaatgaa gggttggaat ttttggtgta gattgtacgg   101040
   tgagaggtgt tttttgggat ttattcgagg agtattattg ttgaatgttt tgttaggtta   101100
   ggagtatcgt tgggggtggg agaggtagtt ggtttatttg tttatttaat agttatttat   101160
   gggtatttta agggttagtt ttgttttagg tattgggggt ttaggtatgg gtttaggaga   101220
   aggggtagaa cgggagggtt ttttggagga aggtttttta attagagatc ggggtaggag   101280
   tttgttaggt aggtgatgtt ggttagtttt ttttgttatt tttttttttt tttttttttt   101340
   tttttttttt tttttttttt gagatagagt ttcgtttttg ttgtttaggt tgtagtgtag   101400
   tggcgtgatt tcggtttatt gtatttttcg ttttttgggt ttaagcgatt tttttgtttt   101460
   agttttttga gtagttgaga ttttaggtac gtattattac gtttagttaa ttttttgtat   101520
   ttttagtaga gataaggttt tattttgttg gttaggttgg ttttaaattt tttattttag   101580
   gtgattcgtt tattttggtt ttttaaagtg ttgggattat aggtgtgagt tattgcgttc   101640
   ggtttttttt tgttattttt gtagatttat agtgtgtgtg gaagattgta tgtttgtgtt   101700
   tggtatagtt gttttcggta tttaggtggg aggtggggga gagggttgtt ggtgttagta   101760
   taggttatgg tatgagatag gttttcgtag ttaagcgggg tgtatcgtag tattggaggg   101820
   gttttgttgc gtacgagatt taggttttgt atttgttttt ttttttagga ggtttggagg   101880
   gttgggaggg attagggtgg tgaaataatt taggtgggag agaggtattg ttggagaggt   101940
   agagggagtt gtaggtttgt agtagcgagt tttaaatttt gggtttttat tagaaaattt   102000
   tagaaagggt tgttttgttt ttttttttaa attataaaag aaatttttgg tcgtttgggt   102060
   tttttggggt cgggtggggg aggagatttt ttattcgtat ttttgtaggt ttagtattgt   102120
   gtagagggag gagggtggag gtaaaggcga gggaggaggg tggaagtaaa ggcggggttt   102180
   ttttttagtt tcgttgtttt gtttggttgt tgtttttaga tttttatttt tggttcggaa   102240
   atatggaatt ttgggatagt tggggtaggg ggtatagaac ggagattttg gtgagggagg   102300
   aatttttttt ttagttttgg ttgtggttat agtaaggtta ggttgggagg attttattta   102360
   aatatttata ttttttttaa tatttaaatg atgaagtagg tttttgtata tagttgtttt   102420
   ggagttagtt gttgtttgtt ttattttttt tttttttttt tttttttttt tttttttttt   102480
   tttttttttt tttttttttt tttttttttt ttttttttgt attttttgtt tttgatttaa   102540
   gtttaggttt ttttagttgt ttttttttta tttataggta ttttttgtga ttgagttttt   102600
   ttttgttatt ttttataatt atttttttta attgagtatt ttttaggtgt taggttttgg   102660
   gttggtattt tgtatataag gtgtttttta atttttacga gagtttaata aggtagattg   102720
   ggttgttgag gttttagcgt tggaaggtcg gttcgggttt ttttagttcg tatttgtatt   102780
   tatttattat ttttttattg tggagatttg ttttttggtt tttggttgag tacgatggaa   102840
   atttttgttt attagttttt ggagagtggg gatggttgag ggtttcgatt ttggggtttg   102900
   acgggggttg tttttgtttt attttagttt ggttgttttt ttatttaatg attgagtttg   102960
   taaaattaga ataatagtgt tatttggttg gtattttggg tggttgggag gcgtgggaga   103020
   gggtgtacgg aggggttgag cgaggtgtta gtatcgtggg gattttcgag ggtagttttt   103080
   ttaggtttag ggcggagtgt aggtagaggg cgcgatggtt tagcgtgtcg gtttatattt   103140
   ggttttgttt ttgtttatac ggtaatgtgg gggagttatt agttttttag tgttttagtt   103200
   ttttatttgt gtaatgggga taagagtaaa ttattgtttt taggtatggt aagtatttat   103260
   atatattgat tattgtagaa tgaatatagt gagttttttt taggttttta ttatatgtta   103320
   ggtagggtgt taggttttag tggagagggg ataatgatgg gtaagttggt ttttgttttt   103380
   aagggttgtc gagtagggag gaaggcggcg gttgttttga ttgtgattcg gagtggggtg   103440
   attcgtaaag aggttggcgg gaggttggtt gggggttggt tgggggttgt tttttttttt   103500
   gtttgtgttt cgtggtaggg tattaaattg gtttttttta tggattttcg aggttttgat   103560
   tgtgggattt cgggtaggtt ttgggatttt gtgattttgt tttcgtttgt tatttaggtt   103620
   tttgttttta gtagtgttta gtatacggcg atttttgtat gttttttagt gtgtgattga   103680
   ttttggagtt gagatttggt agttgaggtt ggatattgtt tttggataat tttcggggag 103740
   gtgggggtag ggtggggttg tgagttggta atttcgagtt tttagtagtt ttcgaatgga 103800
   ggtttttttt ttgtaggatt ttcggggagg aaggagggga tgggggagat gaggtttgtg 103860
   agtttttagg agggtagagg tttatatttg tagcgtttta gaaatatttc ggggtgttta 103920
   ggtttattta gtttttttga gttagaatta ttaagagttg gggattagta gtttattttt 103980
   tagtaagatt tagaaatgat ttttttgaat agttcgagtt aagaatgata tatttggaat 104040
   tagaatatta gaattataga ggaatcgaga gaacgggttt aaaatgtgta gggattttag 104100
   ttatatggcg ttatatggag gaaagtattt aatattatta ttgtatgaat ttgtttaaat 104160
   atatatatat atatatatat atatatatat atatatatat atatttttta gtaaagagtg 104220
   gaaagatatt tgttagaata ttgatggaag ttatttgggt tgtgggttta tagatttttt 104280
   ttttttgttt ttgtgtattt tgtaaatttt cggttttgtg tttaaatttt ttttatagtt 104340
   aggaataaag taatagatgt tattttttaa agtttatggg ttcgtgttcg taattttaat 104400
   attttgggag gttgaggtag gaggattttt taagtttagg agtttgagat tagtttgggt 104460
   aatataggga gatttttatt tttttttttt tttttttttg atagagtttt attgtgtttt 104520
   aggttgaagt ggcggtggta cgtttttggt ttattgtaat ttttgttttt taggtttagg 104580
   tgatttttat gttttagttt tttgagtagt tggattatag atatttatat ttgtaggatt 104640
   tgagaatttt ataagaggaa agtttttatt cggaggttgt tggggatttg gagttgttag 104700
   tttatatatt ttttagtaga gatagggttt tattatgttg attaggttgg ttttaaattt 104760
   ttgtttttaa gtgatttatt tatttcggtt ttttaaggtt ttaggattat aggcgtgagt 104820
   tattgcgttc ggttatggga gatttttttt taaaataaga ataaaaataa aaataaataa 104880
   aagtttattg ttaaaaagaa aaatatatta tttaatttaa ttttttttta ggattgagag 104940
   atgaggaagt agaggtcgga aagtgttata ttttttggtc gttggtagtt aggggatttg 105000
   aggtttgggt tggttttttc gtgttttagg tttggggtgt taggtggagt ttttgttttg 105060
   tttaaaggtt taggtgtttt tgttagtatt gtttgttttt gtatttttgg ttataaggaa 105120
   tgtgggttaa aggtagggtt tttgattttt gtttaggagg gtgaagtggg ttggtttttt 105180
   ttgtttttgt tttaaggttt ttgcggttgt gtttttggaa attgttggtt cgagttagtg 105240
   tgcggagttg ggatagattt tatcgtggtt ttcgtggggt tattttatgt tcgggttgga 105300
   agggattttt cggtttagtt ttttttgttt tttattagga aattgagatt agagaggtta 105360
   tttgtttggt tcggggtggg gtgtagatgg cgtttgtgat ttttgtatgt tacgtgtaga 105420
   tagtttttta gttgtgggtt ttttacgtaa gtagttatgt tggggataat ttggttgaaa 105480
   tttgcgattt aatgaagaga agaatgattt tttttgtagt tttgtgtttt ataggttttt 105540
   ttttgttttt attttttttg tagaggggtt ttatgttggt gggtgggttt ttgttgttgt 105600
   aatgggttta ggtagttttt gggtagtgga ggtttggttg tttttttgtt taatgttttt 105660
   tgtagtttac gttgttcggg aagggcgggg aagtatttag ttattgggga ttttataaga 105720
   tgggtatttg ggagatgttg attggtaggt taggttttcg tggtttatag gtaggttaga 105780
   ggttatagtg ttgagttggg ttgagttggt agtttagggt agtgggggtg ggggtatttt 105840
   gtttagttat gggttttttt ggtataaggg tttggagttt attgtaaata atttttttag 105900
   tttttttttt ttttataggt tattagtaga attgaggggt tttttgttga tgaggtgtgg 105960
   tttgggaggg gttagttgtt tgtttatttg atttttgcgg ataggtgatt ataggttttt 106020
   tgttttaggg ggttggggga aagatcgggg aggttttttt ggttttttta tttggaagga 106080
   aaatacgggt ataggggaga tattttatag ggatatattt tagttagtat tttttggttg 106140
   tgttgggggt atagtgtttg ttttgtttat aagaggggtt agtttttttt aggatttaat 106200
   tttagttttt agtgtagggt agatgagtat ttagaggttt agaggattta tattacgaga 106260
   gagaagttgt gattatatag gtattgaagg ttaagtagaa gagtgggata tacgggtgat 106320
   ttgaaagttt tagggttagt gtggttagtt ttagatagtt ggggagtttt gggtattagg 106380
   tttagtaggg gaggagggtt ttcgtgatag tttgttgggg ttgttgtaat aaagttttac 106440
   gaattttggg ggttgaagat ggttttagag gttagaagtt taaaattaag gtgtcgatta 106500
   ggttgatttt ttggggtttt tgcgggaggt ttcggtttag gttttttttt tagtttttgg 106560
   tggttttcgg gtgtttttgg gtatgtagat gcgttacgtt attttttgtt tcgtttttat 106620
   atggtttttt ttttgtgttt ttgtgtttcg taaggatgtt tgttattgta tttagagttt 106680
   attttaaatt taggatattt tttttttgag attttttatt gtatttgtaa agattttatt 106740
   ttaatttttg ttatatttat aggttcgagg gttagggttt tggtatgttt tttgggggtt 106800
   attatttagt ttattgcggt agttgatttt aggggaagta gaatgtttta agtttggggt 106860
   gagcggaggg aacgttgttg ggtggggagt gggagattag gttcggagat atcgcggatt 106920
   ttttttgttt gatgagggcg tgatgaggtt tttattttag gttttttgaa tggcggttgt 106980
   ttttgaggag attttttttt ttggatttgt ttttttagaa atttttattt aggtttaggt 107040
   tttaaggtgg atggtaagag gcggttgttt ttgttatatt tgatgggtga gtgtgtgaac 107100
   ggggttggga gtaggatatt tgttattttt gtggatagat tttagaggtt tatgaatttg 107160
   ttgttgggga aggtgtttga tttagtttga ttttttattt ttattttttt tttttcgttt 107220
   ttttttatta gttttgtttt tagagtttta tttttttaaa ggtaggcgtc gttttttgga 107280
   ttttttagta gttatttttt attttagatt ttttttttgt aggttttttt ggttttagcg 107340
   tttatttttt tttgggtttt tgttttttta tcgtgaggtt tttgggtaga agtgtggggt 107400
   tttttcgttg tgtttggttt tcgcggtata ttaggggttt ttttaggggt tcgttttgtt 107460
   cgttaggttt ttagggagtt ttttaagttt ttagatgggt ttgatggagt tttttaatgc 107520
   gttgttttag agtaggaggg gtttcggttg agttcgagag tttttttagt tatattttgg 107580
   gttgtttggg gtagttaggt agttcgtttt ttggaggtgt tgtttttttt tggttatttt 107640
   ttttggaatt ttttgcgttt agttgcggtt ttttttatat agtatattta ttattttttt 107700
   ttatttgtga tttttttttg ttaatttttt tatgtttttg agaatggaga ttaggttttt 107760
   tttattttgg agtttagtag aatttaaatt aggtagattc gtgtaggttt acgtgggggt 107820
   tttgtggtta ttgtggtttt gtcgttattt ttagtttagt ttggcgcgtt ttggattatg 107880
   ttatttagtg tcggtttttt aaattttttt tttggttttg taggtttggg aagtagttgg 107940
   tttttgggtg tagttgtgat cggagtttga attttgtttt ttggggttat cgttttttgt 108000
   aattaggttg tagtttatat tggttcgggt agaggttatc ggttgttcgt tttggggtta 108060
   taatatagag agtttcgtga ttaatggagg ttttatttgg gttaagtggt ttttggtgtt 108120
   aggcgttttt tttttttttt tttttttttt gtatttttta agtgtttgta atattttttt 108180
   taatggataa tgagacgagg agggagggag gggtggtttt tttgtttagg tagattagga 108240
   atggttatcg tttgtgtgtg tagtgatagg tttgggaata gataagggag agtttgttcg 108300
   ggaggttgga tttttatatc gatgttgttt gtgtttggta gttttgtttc gggttttaga 108360
   atagatattg attaatacgt tttttagggt ttttaggttc gggttttggt tttcggtaga 108420
   gttttttatt tatgggaaga agtatcgagt agttttgttg gtttttcgta gggagttggg 108480
   atgtattttt aagagtagta tgcgtgaagt tcggggtttg ggtaaaggag tgtttgggga 108540
   agagtttgtt gttgttggag ggtcggaagt agaggggaag gaggggtacg cggtaggttg 108600
   aggaggtggt tggcgatttt gtggtgggga cgagggggtt atgagtagat taggagttag 108660
   tattacgttt ttttgttttt agaatcgagg acggagagtt gttagagagg ttttggcggg 108720
   gtgtttgggg ttatttaaag ggcgtgggtg gattaaagtt gaggaaagtt ttattttggg 108780
   ggatttcgta tttaattttt gaggttttta gagttgtagt ttgtggtttt tcgaagttaa 108840
   ttatttttta gggtttcgtt tttgttttta gcgatagtta tttattaggg agtttttgat 108900
   ttaaggagat ttgaaaggtt atttggtttt ttttttattt ttaggtaggt tatatttaga 108960
   tttttttata tagattcgtt ttattttttt gttgtttttt attagggacg gagggtgggc 109020
   ggttttttta tttaggtttt ggcggtttta ttttagttag gaaatttttt ttttttattt 109080
   gttttatatt tttttttttg cggttttcgt ttattttttt ttaatttgtt tttagtggag 109140
   tagttagtag ttggtttaga attcgggggt ggggggagcg ttagattagt gtagtatatt 109200
   tgagagtgtg ttagaggtgt ttttattttt atagttgatt tagtaaagaa aagaggaagt 109260
   ttagtaattt agcgttattt taaagttcgt ttggaatgat tttgtggttg tcggaattag 109320
   gggtacgcgg tagatgacgg gagtaggtat agagaggttt tcgtttcgga ttagtcgttg 109380
   ttgttttttt ggaggttggg ttgtttttta agaagaggat ttaaggtttt gtgagcggtg 109440
   gttttttttg gagtttttat tgtggttttg gttttgtttt gcgtaaagag agatggtttg 109500
   aagtagaagt tgttacgtgg ttgttttagg taggcgttcg tagttttagt tattcgggag 109560
   gcggaggtag gagaatggta tgaattcggg aggcggagtt tgtagtgagt agagatggta 109620
   ttattgtatc gtagtttggg cgatagagta agatttcgtt ttaaaaaaaa aaaaaaaaaa 109680
   aaggagtaga gaaatttttt ttagaaggtt tgtagttatt ttttttttat gttttagagg 109740
   taaaaaaaaa aaaaattgta tatttcgttt tttttttttt tttttgagac ggagtttcgt 109800
   tttgtcgttt aggttggagt gtagtggcgc gatttcggtt cgttgtaagt ttcgtttttc 109860
   gggtttacgt tatttttttg ttttagtttt tcgagtagtt gggattatag gcgttcgtta 109920
   ttacgttcgg ttaatttttt gtgtttttag tagagacggg gttttattgt gttagttagg 109980
   atggtttcga ttttttgatt tcgtgatttg ttcgtttttg ttttttaaag tgttgggatt 110040
   ataggcgtga gtcgtcgcgt tcggttatac gtttttgttt ttgaataggt ggttttttga 110100
   ttttgggtgt ggttatttta agattttgtt ttggtggggt ataattttgg ttttaggtag 110160
   ggcgatcgta taatttattg ttttttgggg gatacgttag tgaaagggat ttttgaatga 110220
   ttacggtggg ataatagatg tgagttgggt ttgttttagg aaggttggat gtatgggttt 110280
   tttgagttag aggagataat agtagaaagt tttgcggttg atgggatcga gtaggagaag 110340
   ggtcggcgga ggttggggta ataacgtatt tgtttattgt gaagggtttg tgtgagttga 110400
   tggttgatga gagggtagtg gggcgaggtt gttttttgtt aggttagatt agttttagga 110460
   tatttatatt ttttttttag aatcggtgat ttagtggtta gaaggggttt tagaattgac 110520
   gggatattga gggagtcgaa tggaatggtt ggagtggtta tagtaggttt taggtttttt 110580
   ttcgtttttt ttttgtgttt tgtttttggg gtttatattt tggggttgtt gtaatagagt 110640
   gttataattt gggggtttat aaatatagtt ttcggcggtt tttggagatt tttggcgttt 110700
   tttggtttgt agacgtatta ttattatttt tgatttttgg tttttgtttt tacgtggttt 110760
   tttttttttg tttgtgtgtt tttttttttt tttgtttttt ataaagatgt ttattattgg 110820
   atttagggtt tattaaattt agaatgattt tattttgaga tttttatttt aattatattt 110880
   gtaaagattc gttttttaaa tgtgattatg ttcgtaggtt ttaggagtta ggatttaaga 110940
   tatatcgttt tgggggatat tgttgaattt attatattta ataaatttag agttggtgtt 111000
   aatgtttttt gcggatatta tagtagattt tggaggtttt ttgtaaagga attcgtagga 111060
   tttttttttt tttttggagg gatgagattg tagttgggtt ggggaaagtt gttggtttaa 111120
   gttggggttg gtttagttgg tgttggtttg ttaggttgtt agagagtttt atttagggag 111180
   aaattaatat tataaagttg ggggataaga gaaggtttgg aattttaagg gttggttgtt 111240
   gaggttagtt gtttattttt ttgtgtttta ttttataggg atttaggttt gttagtgttt 111300
   cgtttttgga aggaatgttg tgggttgttg tgttttatag gtagggtgga gggttttttt 111360
   gtagttagcg agttgttttt tttgtagcgt gtagagggaa ggaggaggtt tgtttatttt 111420
   ttttggtttt agtcgaaaat ttaattatag gttttggtat taggtagatt tgtttatttt 111480
   tatattatta ttgtagagtg agttaggttt tttgggtggg gttgtgatga gtaggaagga 111540
   ttttcggagt gtttgggatt ttttgggttt agggttgtta gaggtagaga gaaagaagtt 111600
   tttttttaat atttgagtaa agatagttat tagaatttat tagagtggtt ttttgtggtt 111660
   tttttttggg tgttgatttt tttggtgtat ttgaagtttt tagggatatt attgacgttt 111720
   tttgtatttt tatttagggg ttagtggaaa tggttttttt tttttttgtt tagaggtatt 111780
   gtggatgatg attttaggtt ttagatttta gttattgggt tacgtgtgta gataagtttg 111840
   ggtagttagt aatttttggt tattaagttg gttttttgcg gattgggtcg gtattggagg 111900
   cgggttttag ttgttagtat tgtggtttag ttaggtttgg ttttgggaag gtagaggggg 111960
   aggggattgg gaaagggatt gagttggggg tggggtggta ttttggtttt ttttttttag 112020
   atttttggta ggtttgaatt atttggttgc ggttttttaa ggagggagcg tttttttcgt 112080
   tttgatgtgg attttgtttt ttgtttacgt tgtttttgtt tttaaataaa tgtggttttt 112140
   tgtggggagt ttgggggtcg ttttttattg atattgttgt tttttggttt ttttttgtag 112200
   gtttcgttta gttttattgc gtttattttt aagttttttt ttttggacgg ttttgttttt 112260
   tgagaatata ttttttttta ttattttaat tgtgtttttt tttttattcg gagtttgttt 112320
   tatttgtttt aggagttatt tttttttttt attaagtagt ttaggttaat tttggagggt 112380
   taatttgtgt ttttttagaa gtaggttttg agatagggat tttagtggga ggggatatta 112440
   ggggatgttt gagtgtggga atgggttagg tagggaattt agttaataaa gggtgtttta 112500
   ttagtttatt gtgattatag gtggttgttt agtttttata tcgggtagta tggggagatt 112560
   gtagatcgtg tttttagttt tgttttattt ggggttgggt aagggagttg gggtttttat 112620
   attttagttt ttggttaagg attgtattgt ttattttttt tagtttttcg agggtttggg 112680
   tattaggaaa ggttttgggt ggagatacgg aaataggtta tgtgaagtta gtgggcgtga 112740
   ttggtagtag taaggttagg atagttttgg ttattagtaa ggtgattata tattttagta 112800
   tgtatagttt ttatagatat atatttagga gatggtaatt atattagtaa tagttatttt 112860
   tgtcgcgtgt tatgtttgta tttagggaat tttatttttt atttatttat ttatttttat 112920
   ttgagatgga gttgcgtttt tgttgtttag gatagagtgt aatggtataa tttcggttta 112980
   ttataatttt cgttttttgg gtttaagtta ttttgtttta gtttttcgag tagttgggat 113040
   tataggtatg tattattacg tttgattatt tttgtatttt tagtagagat ggggtttttt 113100
   tattttggtt aggttggttt cgaatttttg attttaggtg atttatttat tttagttttt 113160
   taaagtgtta ggattatagg tatgagttat cgcgtttggt tttaggggta ttttattatt 113220
   ttattatttt ttatagtagg tgatattgag gatattgagg tttagagtag attgtggtta 113280
   tgttattttt ttgtttggtt tttttgtttt tagttttgaa ataaagagta cggtttttgt 113340
   gtaggtttat acgttttgtt tttagttttg gagtagtttt taagtttttt gaaggtatat 113400
   taggtttttg ggtttcgttt tttaagtttt ggataaatag gaggtgaagg agtttttgta 113460
   attggatttt attttgttag cgttaaggtt ttgtttgatt atttatttta ttttatatag 113520
   attagggatt tgtgttaagg tttagtgtta taggagagtg aagataagga ggtgtttatg 113580
   gcgtatatgt tgttggattt attttgtaga ttttaagggt tatggggagt tatttgttag 113640
   gtgaggtttt ttcgtggttt ttttttgtag gtttagggta gtatataggg aggtgttttt 113700
   ttattttttg gtattatttg ttcggttttt ttgagttttg attttggttt ttagatagtt 113760
   agtgtttttt tagggataga atttgttttg ttgttgttta gaggagattt agtttgatta 113820
   gggttagcgt ttttggaatt tgagtttgga attttatttt gttttgggta gaggttacgt 113880
   tgtttttttt tacgagagtt gagttgcgta tgagattatt ttgattttgg gttttattat 113940
   ttttttgtat gatatttgtt ttaggttgga gtagggtttt gtggttcgtt tggtgttttt 114000
   tttttagatt gaggtttgat atgtatatta ggaagtgttt gatttggttt taggagttgg 114060
   gagcggtttg ttaggtagtt tgtggttaga gtaggggttt taataatggt tttttgtgcg 114120
   gtatattttt ttttaaatag gtttaggttt aggttgtttt tgtttcgatt tcgttatggg 114180
   tttggagggg aaagtttgta tttggagggt ggtttattgt ggtttttttt tgtgtagtag 114240
   ttgagatcgt aggtgtttga gaataggaga agttttgtgt ttattttggg ttgtgagatg 114300
   tttttttaga ggtggggatg gggatgggtt agtatgtagt tatttatatt ttttagtttg 114360
   ttggtattaa ggtttgtgta tgtgtgtgtt tttttttttt taattttgtt tttttaatat 114420
   ggagttttgt tatttgttta ggttggtttc ggatttttgg tttaagcgat tttttagttt 114480
   taggttttta aagtgttggg attataggtg tgagttatta tgtttagtta gtattaaggt 114540
   gtgtttttta agagtaatat tggtcgggtg tagtggttta cgtttgtaat cgtagtattt 114600
   tgggaggtcg aggtaggcgg attatgaggt tagtagattg agattatttt ggttaatacg 114660
   gtgaaatcgt gtttttatta aaaatataaa aaaattagtt agacgtggtg gtcgggtatt 114720
   tgtagtttta gttattcggg aggttgaggt aggagaatgg cgtgaatttg ggaggtggag 114780
   tttgcggtga gtcgagattg cgttattgta ttttagtttg ggcgatagag cgagattgtt 114840
   ttaaaaaaaa aaaaaaaaaa gaataatatc gatggtttta ttttttgaat atgttaagtg 114900
   atttttatgt agtataagta ttttagaagg taggaaaatg taatgaataa tatttatcgt 114960
   tatttatttt atttagagat cgttattttt tgttcgggtg tgagtgtgtg tgtgcgtgta 115020
   aattgtatat gtgtgtatga aatggaatta ggatttttgt atatattttg tgttttgtta 115080
   tttttattta atattttcgg tattttttat ttttaaaaat gtgattttaa tgatggtgta 115140
   ggtttttgat atatgggttt agaaaatttg cggaaattcg gattgttttt aggttttgtt 115200
   agtttaatgt tgatgtaaat gttttcgtac gtaaattttt ttgtattttt gattattttt 115260
   ttgggataaa tttttagaag tgggttaaag tatatgaacg gtttttaggt ttttggagta 115320
   agttgagatc ggtggggatt ggagaaggga tttagggagg tttttgggag tttgcgagat 115380
   tggagtttgg ttttagggga tttgaattta tagatgaagg agatggttgg taaaggtggg 115440
   aattttttgt tttatgggtg ggggggtgat attgttttat tttttagtga tagtatgggg 115500
   atttttttaa tatgatttgg tttcgtattt ttttgatatt gtgtacggtt tatagttgtg 115560
   atgtttagtt gggagttttt tttgtgtttt ttagttgtat taattttttg tagatagagg 115620
   aggaagttgt tcggtgtttt tttttgttgc gtgggttttt tgaatttgtt tagttgggtt 115680
   tgtggatttg tgtagttacg tacggggttt taaaatttgt aaaatgataa gtttacgaat 115740
   attttcgttt ttgtgggtga atgtggatat tttttggttg cgtttttttt tatagggaag 115800
   atggagacgt gtaaagtgat aaggatttgt tatttttggg taggttatgt cggttttatt 115860
   gtgaggatgg tggtaggggg ttttttagga ggtttgtata ttgggaagag aggggcgatc 115920
   gtgtgtgatt gtaggattta gggcgtagtt ttaggatttt gatgggggag gagatgttgg 115980
   ggagttttgt tttgttagtt tggtgataga tattttttaa tgtttttcgg ttgggtataa 116040
   tttataatgt ttttcgtggg gaggtaattg tttatgaggt atttttggta gggatgattt 116100
   tgttttattg ttatatattt tagtaatgaa ataagaattt agagtgatgt gattttttcg 116160
   atgatttttt taattatttg gggtttttgt gcgattttaa gaagggggaa ggaggtagtt 116220
   ttcgtttttt tgggtttttg atgtggtagt tttggaatgg ttacgtgttc gttggtttat 116280
   agtgggttat ttgtcgaatg tggttgtggt gggattgggg ttgggttgga gagtgggtga 116340
   ttatattagt tagttttatt ttgggttatt gtgtagttag gggttttatt tttttagggg 116400
   tttcgttgtg agtgttttgg ggttgttgga ataaattatt ggaaattaag tggtttaata 116460
   aagattgatt cgtttatggt tttggaggtt ataagtttga tattaaggta ttggtaggat 116520
   tatatttttt tcgatagttc gaggggagga tttttttttg ttttttttag tttgcgttgg 116580
   tttaggcgtt ttttggtttt ggtagtattt gtagtttttg tttttattac gtagtttttt 116640
   ttatcgtgtt tttttgtgtt ttaagttttt ttttgttttt tttttataag gatatcgagt 116700
   attggattta gagtttattt taaatttagg atagttttat ttcgggattt ttaattatat 116760
   ttgtaaagat ttttttttta aataaggtta tattcgtggg tttttggggt taggtattag 116820
   atatgttttt ttgggggtta ttagttaatt ttttatagaa ttttagacga aagtgaattt 116880
   ttataggttg aaatgggggt gaaggtttag tgcgtttttt ttttttggga gtttttttag 116940
   ggtttgtgtt tattttgtga ttttgtgatt tagttttttt tttgagatga agttttgttt 117000
   tgtcgtttag gttggagtat tgtggtacga tttgggttta ttgtaatttt tgtttttcga 117060
   atttaagtga tttttttgtt ttagtttttt aagtagttgg gattataggc gtttattttt 117120
   atgtttagtt aattttttgt gtttttagta gagatagggt tttattatgt tggttaggtt 117180
   ggtttcgaat ttttgatttt aggtgattcg tttgttttgg gtttgttttg gttttttaaa 117240
   gtgttgggat tataggtatg agttattacg tttggtattt agtaggtttt ggttgttttt 117300
   tttttttgag atagagtttt attttgttat ttaggttgga gtgtagtggt gtgattatgg 117360
   tttattgtag ttttgatttt ttggttttaa gtgatttttt tattttagtt tttcgagtag 117420
   ttgggattat aggtatatgt tattattttt ggttaatttt tgtattgttt agtggagtcg 117480
   gggttttgtt atgttgttta ggtttgtttt gaatttttgg gtttaagcga tttgtttatt 117540
   ttggtttttt aaagtgttgg ggttataggc gtgagttatt acgtttggtt tagtaggttt 117600
   tttagaagcg tttgaggttt ggtgaaggta tggtgttatg gagtagagaa tatttaattt 117660
   tgttttcggg gagtcggaat atatttttcg tagttaattt taatagttat gacgtggtta 117720
   ggatgatatg gggagggggt ggtgtggatt gtgtgggagg ttagtttttc gggaaggggg 117780
   ttggagtggg tttggttttg aggaggattt ttaagaggag gaggaggcgg tggtatgttc 117840
   gtttgggggg atggcgtgag ttagggtggg tattttttta tttaggtgtt atttttttga 117900
   tattttagtc gggatatggt ttttttgggg aagatggata tttttagagt ggggtatttt 117960
   ttggggaaga tggatatttt cggaatgggg cgttttttgt ttttattgtt tttttagtat 118020
   atagttgtat ttttttgtag ttttggaggt tgtgagattt tttggttgtt atttagaata 118080
   aattcgtttt tgagtagcgt cgttttgatg gtggggtggt ttttgcgggt ggaatagttt 118140
   ttgttgtttt agagtttatt gagagttagt gggtatggga gtggtgttgg atcgaaatat 118200
   ttagagggat tatagagtta ggtagtaagt atagaggtta gttagtgggg agggagggat 118260
   ttatttatga aaagatatta agtattgaaa gagaggagag gatggttggg gatcggtata 118320
   tatgtgtatt ttgcgtagat ggttaaggtg gtgaggcgtt agttacgtag ataattggag 118380
   gaaggaggag tgttttaggg gaggggttag tttcggtttt agaatgattc gtttgtgtac 118440
   gtgggtttgg ttggaataaa ttattgtatt cgtatcgtag atttaatcgt aagtttatat 118500
   tagggtagtt tttgttttgg tgtagtgttt tcgattatga gttggttatg gtgggggtga 118560
   ttttaagggg atttttaagg agggggttta tcggattttt tagggaaggt tatttgtttt 118620
   tttgggttgg atttgggata aggttttttt ttgtttagtt tttgtttttt aatttgatcg 118680
   tacggaagga tgttgaggat atttggaagt cggtttttgt tttatagtat ttataggata 118740
   gaggtatatt atgtggggat agtttggata gtgtttagga ggtagggagt agattagagg 118800
   atcgcgggag gttttgggaa tcgtacgagg atttaggtat tgagttttgt attttttggt 118860
   attagttcga tgtatagttt ggttttgtgt ttatttcgta ttttattttt tttatttgta 118920
   cgggggcggt aataatattt tttatagttt aggtttaagc gggtgtagat gtgatgagtt 118980
   tggtaagttt attttattat atttagtttt gggtattgaa ttttggattt tttggttggt 119040
   agaagtggat atttaatttt tttttggggt tttggggttt ttgtagaatt agaatatagt 119100
   tagtagtttg tatgtagggt gtagagggat agagggatat tatgtcggga gtttatattt 119160
   ggtatatttt tgattttggg gtttagtgtt ttgttgtttt gcggttacgt gattagttag 119220
   aggttatggt tggatttata ggcgaatata gtttgtggat ttgattttgt ttggttttgc 119280
   ggtgttttat tttattttta gttttagttt gtggagatag agtttcgttt tgttagttag 119340
   gttgagtgta gtgatacgat tttagttttg taatttttgt ttttcgggtt taagcgattt 119400
   ttttgtttta gttttttgag tagttgggat tataggcgag tgttattatg tttagttaat 119460
   tttttatttt tagtaaagat ggggttttat tatattagtt aggttgttta gtgttttaaa 119520
   aagggtttgt tttgttttgt gttagtattt aaagattagg agatattata taaagattta 119580
   gatttttatt tttttgagaa gtgaatagtt ttggttacgt cgggtttata ttttttaggg 119640
   tagtgacgga gttgagatag ggtttgtgat aattgagagt tttttggagt ttatacggtt 119700
   ttttgtttag tttttggtgg aggttgagtt tagtgattta ggatttcgtt tgtgtttatt 119760
   aagaaggaga ggtgtttgtg gtttagagag ggcgtttata ggagtgtttt gtttaggaag 119820
   ttggtagttg aggcggttag ggttaggggg ttgggttttt tttttttttt tttagtgttt 119880
   gagtgtattt ggaggaagag agacgggtga ggggaaggta ttgaaattgg ttttggagcg 119940
   ttattattgg gggttgggga ttagttgtta tttttttttt tgtgggtagg ggaggtagtg 120000
   gtgggatttt atgagattcg gattttgtgg tttggtgagg tagggagcgg ttggggagat 120060
   attgtgggtt tttatagggg tgcgttgtgg gtttggtttg gggaaggagt tggtgaatgt 120120
   gtttgttatg tgggtttttt tttgttttgg ttttgataat taggagcggt tggtttttat 120180
   ttggagtgcg gttgtgggga ttgtggaggt ggtagttttt taggtattat ttttattgtt 120240
   ttcgagggag tagattttgg tggagaggta ggatggttgt agggcgggag ttggtggttg 120300
   gtattattgt acgggtttgt agtagtggat gggaagatgt aggagaggtt gatgattgag 120360
   aaagttggtt tttgtggatt aagttggggg agtcggataa ttaagtggtt agaattttgg 120420
   gttttggtat tagtttgatg tatagtttgg ttttgtgttt atttcgtgtt ttattttttt 120480
   tatttgtatt gggggtattg gtatttttta tattttaggg ttaagcggtt gtagatgtga 120540
   tgagttcggt aggttcggcg tttcgtcggt attgaaagtt ttagtgttcg tttttagtgt 120600
   tattgatggg gttttgtttt tgttgcggtt ttgattggtt atagttgcgg ttatttatta 120660
   ttggaggtat gtttttcgtt gtggttgatg gtagaggtgt gtttgttggt ggtgttttag 120720
   tgtgtgtttt ggttggaggt gaggagggat aaaaatagtc gcgataagaa ggaaattaag 120780
   gtcgggtacg gtggcgtacg tttgtaattt tagtattttg ggaggtcgag gcgggtggat 120840
   cgtttgaggt taggagttcg agattagatt gattaatatg gtgaaatttt atttttatta 120900
   aaaatataaa aattagttag gtatggtggt tcgtgtttat aattttagtt attggggagg 120960
   ttgaggtagg agaatcgttt gaattcggga ggtgtcgttg tattttagtt agggtaatag 121020
   agtaagattt tattttaaaa aaaaaaagaa ggaaataaaa gttttttttg gaattagaac 121080
   gtttttatgt agacgtagtt ggagggggag gttgtgtggt ttgcgtcgtg gttatggtaa 121140
   gatacgacgg gtagtatttt tttattttag tttgtatttt tgttttagaa tttatgatgg 121200
   tttttttagg atggggttgt ttttttcgtt tgttgagata tgaaggacgg gaagagattt 121260
   tcggttttta tcgtcgtatc gtttgttttt ttttgttgtt ttttagtagg aaatatgtcg 121320
   gagtgttttt tagttattta tttattaatt gtattttttt tttttatttt ttagttttga 121380
   aaagattttt tgaggtcgag gaggtcgaga tatttaattt tattttattt cggagggttt 121440
   agattttttt atttcgagtt attgtggtta gttttattta gaaattttag gatttgggcg 121500
   tgaagaattt agaattttcg gttcgttatg tggatttttt aagttaacgt ttttttaagg 121560
   cgtttttgcg gagggtggag ttttcgggtt ttaaggcggt cgagtcggtg tttcggcgta 121620
   ttgagttgtt tattgatatt tcgtttaagt aggtggagaa cgtcggggtt atcggttcgt 121680
   ttcggttcgg gtttaagagg gtcgaggtgt tgggttataa gacgttagaa tcggtttttc 121740
   ggaggacgga gattattatc gttaaatttt aggagttagt ttatcggagg atggagtttt 121800
   ttgtttttaa ggttttcgag gtgtttattg tttttgttat cgacgtagtt tttaagaggg 121860
   tggagattta gatgtttaag tttgttgagg cgtttatcgt ttttagttta gtttagattt 121920
   tggagaattt agagtttgtt tttgtgtttt agttgtagag taggttggag tttaagtttt 121980
   agttttttgt ggttgaggtt atatttcgga gttaggaggg tgagtcgtag agcgttaggt 122040
   tttggatgtt gtggtaatgg ggggtttggt tgttggtttt gttatagaat tttggaggaa 122100
   gaagttggat atggggtgga gggtgttatt ttggagattt agaaagatcg gaatgtatgg 122160
   gggtgggggt ttgtaagttt aggagaggtg gttttttttg tttgatggga atatgttaag 122220
   atgttttaag cgggatttga atgagagttt ggaaagattt tttgggtttt aaggagtttt 122280
   ttttatgggt tatcgtgggt tttgttggga tttttgtttg ttgtgtgttt ttggagggtt 122340
   tgggaggttt agagaatgag gagttacgta ggaattaggg taggtttatg taggggtgta 122400
   ggatagggtg gagttgagat ttttagaagg gttgttatat tttggtggtt tttgtttgag 122460
   gaattgtagg ttgtttggat atgtttagta gaggtcgagg gtttggggaa gggaggaggg 122520
   acgtatgttt tttgaggttt gtattggagt ttatggtttg gttttttttt tttaggaggg 122580
   atggttgtgg tagagggagg ggaatatgag gagtatttta gtttgtagga taaaattttt 122640
   agggtattgg gaaggggtgt ttggattagg gggttgtgtg atagttgtat acggttttat 122700
   ttgggggtgt ttttttggcg agaggagaga ggttatgggg ttttagtagc gttttgggag 122760
   ggtttaagtt tatagtcgtg ttgatatgga gtttataggt aggggattgg tgttagtttt 122820
   gtgggatata gggtttgttt ggaaatgtta ggttgtggga tatggttggg ttttttttta 122880
   gaagcgtgtt ttttggtttt agtagggatc gtggttttat ggggttatag taaggtgtta 122940
   gttgggttag aagtttttta agagttaggt ttgattttgg gtataggatt agaatttagt 123000
   aatgtttagt aagttttttt taggttggat aaatagtata gtttgggttt ttgatttagt 123060
   tgttgttttt tatttttaat cgtggtgaaa tatataatat ataatgtgta attttagtta 123120
   ttttgtagtg tacgttttag cggtatttag tatatttacg gtgttgcgta gttgttatcg 123180
   ttgtttattt tcggtatttt ttttattttt ttaaattgag tttcgttttc gttagattaa 123240
   gttttttatg tttttttttt agttgttggt agttattatt ttattttttg tttttattat 123300
   gaatttattt gattatttta ggaattttgt gtgtgtggaa aaatataata attgtttatt 123360
   ttgtgtttgg tttattttat ttagtatgac gttttaaagg tttattcgtc gtgttgtagt 123420
   atggggtaga attttttttt ttttagaggt tgaataatat tttgttgtgc gatgtatttg 123480
   tgttgtgtag ttgttttttt atcggtggat ttttgggttg tttttatatt tttttaagtt 123540
   ttttttttta ttttttttga tttttttttg atttttttga gatagggttt tgttttgttt 123600
   tttaggttgg cgtgtagtgg tgtgattatt gtttattgta atttttgttt tttgggttta 123660
   agtgattttt tagttttagt tttttaagta tttaggatta taggttgcgc gttattatgt 123720
   ttggttaatt tttaaatttt ttttgtagag ataggttttt attaggttgt ttaggttggt 123780
   tttaaatttt tgggtttaaa tgagtttttt gtttcggttt tttaaagtgt tgggattata 123840
   ggtatgagtt atttaatttt tttttggttt ttttttttga atgagagttt tttagtggtt 123900
   agtgggtttg ggggttggga ggatgtgggg tttggagagg gtgtgggttt ggggggagat 123960
   tagtgttttt tatgttttta tgggtatgtg gagtgtatat ttgttttggc gggtttgggg 124020
   tttgggattt tgtatttttt atagaggggg tatcggaata tattgagtag ttagaggtta 124080
   gtgggttttg gaaggttttt ttcggttttt agtgtgcgag tttcgtgttt ttttttttat 124140
   ttttttaagt ttgattttga agagttattt tgttttagtt atttagattt agtgatagtg 124200
   gttgttggtg gtatggtgtt aggttaaagg tatgggtagt ttgtagagtg gtaggaggtt 124260
   gttttttttt ttgttttttg ttggtgtagg atttttgttt gacgtgtcgg atatattttt 124320
   attttgggtt gatgtgttta tatttagttg agttaaatag gatgtggttg gggaggcggt 124380
   tgttatcgag ttatttaaat ttcggtgatg gttggtgttg gatgtatagg gacgtggttt 124440
   tggttttggg ggataggtag ggggatgttt atggggatgt ataagaatat ttttttttag 124500
   gggtagatat agtttagttt ttaaattgtg ttagagattg tgtcgggagt taggtttagg 124560
   gatataattt gcgggttttg ttttttggag tttatcgagt cgtgagtagg atggttggga 124620
   tataaggagt gtttagggag tttttttagt agtggaagta taattttatt aggttaggaa 124680
   ggggagagaa aggtaggtta cgtggaggta gtagcgttta gagttgttag gaatttggcg 124740
   ttttgggggg acggtggttt tttttatgga ataggatgta tacgttggag tcggggtatg 124800
   ggagaaaatt attagtattt tagattcggt tatttacgga tagggatttg tgtaggtggg 124860
   agtttagttt acgtggagag gttcgtgggt tgggttgata gttttgaggg tttaagtaag 124920
   tttaggtgga ggaaatgtag ggatagacga gggtagggtt tttttaatgg aggagtgttt 124980
   ttgaaattcg atgggagtgg tttttcgggg ggtttaggtt tgagttttat tagttttttt 125040
   ttcgaggtat tatttgtttg ttttttcgtt tttttttttt gtgttacgat cgttttggat 125100
   acggatttag ttttttttaa tttagttttt tgtttatttt ttttttaggt ttattttttt 125160
   ttgttttttt atgtggggtt gggttgggag ttttttgagt ttcgatatgt atagttttga 125220
   ttaatttttg cggttttttt gtttttcgag tattagggat ttagttggta ttatggggaa 125280
   aggaattatg gagggagttt cgtgtttttt gagtagacgt ttagtttttt ggtttttgtt 125340
   tgcgggaggg tatttgtgtt gtggtttgtt tttattttag agttgtgggt gtagggtagg 125400
   gttttttggg gttttagttg aattgttttt agattgattt ttttaaagta tagtattgtt 125460
   gagatttttt ttcgtgttga gttttttttg tttatttatt ataggttttt ttacggttga 125520
   tttatttttt tgtttagttt atattttttt tttttttagt tttttttgtt ttggtttttt 125580
   tttggttttt tatatttttt tttataattt ttttgttttg attttttgaa ttttgatttt 125640
   tgtttttttt tatattattg ttttggtgaa tttttatgta ttttttaaaa tttggtttaa 125700
   atatttcgtg tttggtttgg gatataaatg atttgtgtat aaattttttt ttttttgtta 125760
   gatttttttt tttaaatgtg aaagtagttt tttagattat aaaaagtaat cgagtatttt 125820
   ttttttaaaa tgtaggaagt atagaaatga gaaagaattt aaaatttatt tgtaagttag 125880
   cgatatttat gtttgaggtt ttgttgtata gtttttaaaa ttttttttat gtttttatta 125940
   gattttgagg gtagggttgt tgtttttgtt tttttttgtg tttttttttt gttttttgtt 126000
   ttttgagatg gagttttttt ttattattta ggttggagtg tagtggtatg attttagttt 126060
   attgtaattt ttgtttttta ggtttaagcg atttttttgt tttagttttt cgagtagttg 126120
   ggattatagg cgtgggttat tacgtttggt taatttttgt atttttagta gagacggggt 126180
   tttattatat aggttaggtt ggtttcgaat ttttgatttc gtgatttatt tattttagtt 126240
   tttttgattt tttaaagtgt tgggattatg ttatcgtgtt tagtttgttt tggtttttat 126300
   aaagaatttt ataaggggtt aggtgtagtg gtttatgttt gtaaatttag tattttggag 126360
   gtcgaagtag gtagattgtt tgagtttagg agtttgggat tagtttgggt aatatagaga 126420
   aattttattt ttataaaaaa tataaaaaat tagttaggta tgatagtatg cgtttgtagt 126480
   tttagttatt ggggaggttg aggtgggagg attatttaag tatgggaggt tgaggttgcg 126540
   gtgagttgag attgtgttat tgtattttag tttaggagat agagtgagat tttgttttaa 126600
   aaaaaaaaaa aaaaaaaaaa aaaattttat aagatattaa gtaagtgatt tggatataat 126660
   aggtgtgtta tgaaattatg ttgaaggagt gagttattgg tggtgagttg ggatggtttt 126720
   tttgtggttt ttgtaggtag atgtagtgat ttttatgggt cgtttttaga gattaagtag 126780
   aaattttttt ggttaatttt atttttagga tatttggttt tttttttgga ggttgtgttg 126840
   gagggtttag tgggtgggtg ttggttttag tgtgggggtt ttggaaggag ttagtatttt 126900
   aggagggagt gtgggtattt agatggttga aggggaggga gaggaagaga ggcgttgtag 126960
   agggtagagg tatttttgtt tgttttttcg ttttagggtt tggtgaggtt tgagttttat 127020
   gttggtgggg tggattttcg gttttcgagg ttttggtttt gtttttggat ttggtatttt 127080
   ttttgttgtt tttttataga agggcgttta ttttttgggg ttatttttgg tttttttagg 127140
   ttttgggtgg ggacggtttt gttttttttt ttggattttt gttttttttt tgttttttaa 127200
   tatgggtgtt tttgggtgga gtggggtggg gttgggttgt agtatttagt attgagtttg 127260
   taagggttgg ttgttggttt agagtttttt ttattagagt tatttttttt cggtttttta 127320
   gttatattag ttaagttttt ttagagttgt tttataaggc gggtgtttag cgttcgttta 127380
   tgggtttggt tttttgtttt tttgggatgt ttattggggg taagagggag gttaggttta 127440
   gtgtttagtt tagttttggg tatgttttag ttttgtttta tgagttaatt ggttttgaat 127500
   tattatttta ttttttttag ttttaatttt ttgatttgtt aaagaggtat ttgtagaggt 127560
   ggttgggggt tgagttttta tgtatttatt tattttaaga attaagaagg gagtttatag 127620
   tggttttggg gttatttgtg aatatggatg tgaaattggg gcggggtaga tatggtagta 127680
   tgttttagta gtttttttgt gatttagagg gtttggaaat ttttgttatg tgggtggagc 127740
   gttgagggag tagggggtta ggttttttcg agttgatgat ggtgggcgtg gtttttcgta 127800
   tatttggttc gtatcgggtc gtgtgtttta ggggtagaat agggaggaat tttagtatag 127860
   atagtagggt tgttagtata gcgttgagta tagggttaat taaagtatag ggttttttgg 127920
   aattatttaa tatagagtag tgaagaggtg gttttgggga aagtggggtt agagttgagt 127980
   ttgagtgggg ggtggggagg gcgggtatta gtttgtaagg gagggatatt tattgggttt 128040
   tagaagtttt tggtggggat tagggattgt gaagttgggg agagggtgat tattgtgatt 128100
   tagggaggaa gggttttata gatagtttag tttttggttt cgtgtttata ggatatttta 128160
   tataggtttt gagtttattt ttggattgtg gattttttga ggtagggtgt gtaggttcgt 128220
   ttagtattag gtgtaaggta gggggttttg gtaagcgtcg agggatttat tggtgtttga 128280
   tgaagtagtg agtttggatc gtgttatgtt tcgggttttg agtttgtagg atttttttgt 128340
   ggggttgtgt gtttttgttt ttggaggtgt ttagaaggta ttgacgtttt tgtcgggatg 128400
   ttttattcgg aaggtttcgt tgtttttatt tttttttatt tttttgagtt gtttgtagag 128460
   ggtatttagg ttttttttta ttttgggatt gggttttttt tttttttttt ttttaagaat 128520
   aagtattttt ttttttcgaa ttgtttttgg agttttttag atttaggttt agagtgggaa 128580
   gtttcgtatg tgttggggga ataggaatta atgcgggagg attggttagg ttcggtgtgt 128640
   ttttttcgtg gtgggacggg gtggggttag agaatagagt ttggttttag agtttaggaa 128700
   gagaattttt tttttcgtcg gtttgttaag tatcgtgtag tggtgagagg aaaggagggt 128760
   tggttcggga atgtagcggg tgtcgagtag ttgtggaata ttttaaatag agcgtagttg 128820
   cggggaaggg tggggttagg aggtaggcgt tgggggcggt ttttttgttt cgttttttgt 128880
   agttttgtgg ttttgattat ttagtttggt tttaggatgg agtttcggtt tttagatata 128940
   tggtattggg tgaggtcgtt ttttagtatt tgttttagga agttagagta gttgttagaa 129000
   gagagaggag ttgtgtttgt attcgtcgtt ttttaggttc gggatagttg tcgggattga 129060
   gggtttgtat aatggcgagt ttgttcggag gggcgcgtcg cggttagata gttatgtttt 129120
   gtgcggtgtg gatagagggt atatttagtt ttggaggagc gtatttagat ttttcgtttc 129180
   ggggtttaaa gtgtgggtag tttttaatta gattttggat tttagtagat agtttgggag 129240
   gatgttatgt ggtttatttt gatatattgt tttatttgtg attttatttt tgagagttgt 129300
   agggaggcgt ttttttagtt ggattttatt tttttttcgg ggttttttgt gggtgttata 129360
   agtgattttg ttttttttag tttaaggggg gtttttgggg tatgggttgt aattgatagg 129420
   ttagagtagt ttatggttag ggttgagttt ttttttcgtt ttttttgaat attgggttta 129480
   ggaaggggtt aaagtggaga tgttttgatt aaagttgagg gaggtatgga gtttttttga 129540
   ttttgttttt ttattttatt gttgttattg ggtgtatttt gtggagtttt aggattttta 129600
   aagaatttag agtggagatt ttcggtttta tttagtgtag gtgaggggga aagtgatttg 129660
   tttatagtta tatggttatt attagtatat ttgggttaag ttcgatttta ttttacggtt 129720
   tttgtttgaa atttggaatt ggaaatggtt ttggaaatta ttgattttat ttgtcgtttt 129780
   ttaaaatggt tcgtagaggt taagatatag atttttgtaa tgttttgttt attcgtgtgt 129840
   ggggaaatgg tagaggagta tcggttttgt ttttatattg tttgggttgt cgtttttgtt 129900
   ttgtttagtg gttttttttt ttggttcgtt tttttattgt ggaagtgggt ataatttttt 129960
   ttgtgtagcg tggttagagt ggatagtgat gattcgtgaa gtagggtgga gcgtttgggg 130020
   gattcgtgtt aaataagtat ataaataaaa gttaatataa tttttattta attagtaagt 130080
   aggttttgtt tgaagttttt gggtagtatt ttatgttatt tttaggtttt aggtcggtgg 130140
   tgtcgtttcg tgtttttttt attcgtaagt tggtgtttag aagttttgtt ttgtgttttg 130200
   gtttttattt tttttgtgat tttttttttt ttttatttta aaaagtaatg tacgacgggg 130260
   cgtggtggcg ggtatttgta gttttagttg tttgggaggt tgaggtagga gaattatttg 130320
   aatttaggag gcggaggttg tagtgagttg agattgtgtt agtgtagttt agtttgggta 130380
   atagaataag atttttattt gggaaaaaaa aaaaaaagcg atgtttagtt atttttagat 130440
   agaaaatgaa gattatatga aatttggaaa gaagtttttt ttttttattt tttaattttt 130500
   ttttaagggg tggttatgtt gatagttggg tgtggtattt ttttgaattt tatttttgta 130560
   gagttattta gggtttgtgt aaaaaaagtt aatgtgtaaa tatagatttt ttaagaatat 130620
   aaaggaaatt agatttttgt tattagtttt tatttttttt atttaataat attgttgtta 130680
   tttgtagatg tagtacgttt tttttttttt tttttttttt ttttttttga tagagcgtgg 130740
   tttaggttgg agtgtagtgg tgcgatttta gtttattgtg gtttattgta atttttattt 130800
   ttcgtttttc gtttttaagc gatttttttg ttttagtttt ttaagtagtt gaaattatag 130860
   gtgtttgtta ttatatttgg ttaatttttg tatttttagt agagacgggg ttttattatg 130920
   ttggttaggt tgtttttgaa tttttgattt tgagattcgt ttatttcggt tttttaaagt 130980
   agtacgtttt ttttaatggt tgtgttatta gttgttttat ggttttgtta ggtttgttta 131040
   gttttttatt gtgattgaga ggttgtaata gatgttttcg tatttgtata tgtgtatata 131100
   tgattgaagg atgagttttt agtgaatttt tgtgttaagg atgtgtgtat ttaagttttg 131160
   atttgttggg tatagtggtt tatatttgta attttagtat tttgggaggt taaggcgggt 131220
   tgattatttg aggttaggag tttaagatta gtttggttaa tatggtgaaa ttttattttt 131280
   attaaaaata taaaaattag ttgggtatgg tggtttgtgt ttgtagtttt agttatttag 131340
   gaggttaagg taggagattt gtttgaattt agtaggtaga ggttgtagtg agttaagatc 131400
   gtgttattgt attttagttt gggtgataga gcgagatttt ttattaaaaa aaaaaaaaaa 131460
   aaaaaaaaat tgatttttgt ttttgaattg gtttttaaag tggttattag aattttttag 131520
   gatttttttt ttcgtcgatt tgtttagttg tttgttgggt tttgtgtagt gatggtttag 131580
   tagggaggtt ttatgttttt tatttttggt tgagggttaa atgtttttta attggtgttt 131640
   tattttgtgt atttcggtta agcgagaggg attatttttt cgtatgttat aggttagggt 131700
   tttgtgtttt ttttttattt ggaaggtgta tttttttttt gttgaaattt tttaaaaaga 131760
   gttttttgta agttaggcgt gatggttaat atttataatt ttagtatttt gggaggttga 131820
   agtgggcgga ttgtttgagt ttaggagttc gagattagtt taggtaatat ggtaaaatcg 131880
   tatttttgta aaaattagtt gggtgaggtg gtgcgtttgt agtttcggtt attcgggagg 131940
   ttgatgtggg aggattattt gagtttggga gtttgaggtt gttgtgagtt gtggttatat 132000
   tattgtattt tagtttgggt gatagagtga gattttgttt aaaaaaaaaa aagagttatt 132060
   tgtatatttt tattatagta ggtacgagta ttttgttttt agttgtttat ttttgatttt 132120
   gtttttggga tgttttttta tatagaatat tttaattttg atattgttaa gtttttgttt 132180
   ttttttttag tttttggaat ttatattttg tttagaggtt ttttgcgttt tgaaattata 132240
   ggaaagaatt ttatttacgt ttttttttag tatttttgtg gttttatttt tatatgttga 132300
   aattatgaat ttattggaat gtattttggt tttagtagcg aggtaggggt agatggtatt 132360
   gtttggatat atagtagcga gtttggtttg gagcgggtgg ggcgtgtgtg tgtgtgtgtg 132420
   tgtgtgtgtg tgtgtgtgtt atttttatta gttttggtgt ttgtattgtg ttggtttagt 132480
   aaaaataatt tggatgtatt ttttttattt tttgtttttt ggaatatttt aagtagtaat 132540
   ataataattt ttttgttaaa attttttaag aatttattcg tgaaattatt tgagtttgat 132600
   atttttgggg gagtttagta ttttaaaaat ttaaattttt tttatgggtt tgtgagtttg 132660
   ggagtttttt ttttttttta aaatttatta ttattttttt tttagaaaag tgttttttag 132720
   tttatttaaa attcgttagt ttatttcgag ggggttgttt tagttcgttt agtgaggata 132780
   gtgtgtagcg cggtgattga tatgattgat tagtagagta cgatcgatta gtcggtgtgt 132840
   aaatatgatt gattgtggtt agcgttttgg gatgttttat gttgaggtat atgtgtgtag 132900
   tgtagggtga tgtgtatttt gagaggggat gaagttatag gatttttgta tttttaggag 132960
   agagtttttt tttttaaggt tgtagttttg agtaggtagt gacgggattg ggtttagggg 133020
   gttgagtggt tttagtaggt taagggtttt aggaattatg agaagtttgg agttgtttag 133080
   agagtagttt ttaaaattag tttggattgt agtttttaga aagatatgcg attgatatta 133140
   cgaattagga tatatttata tatggggtgg gggtggggat gggatgtatt aatgaaatta 133200
   aattttcgtg atggaatttt tatttttatt atgcgtgtgt tatatttagt attttttttt 133260
   tttttttttt tttttttttt tttttttttt ttttttgttt gtttgttttg gagagaggga 133320
   aggttttttt ttattttttt ttaaatagtt ttattgagat ttaagtaagt tattatataa 133380
   tttattaatt tatttattta tttatttttt tgagacggag ttttattttt gtcgtttagg 133440
   ttggagtgta gtggcgttat ttcggttgat tgtaattttc gttttttggg tttaattaat 133500
   ttttttgttt tagtttttcg agtagttgag attatagata tgtattatta cgttcggtta 133560
   attttgtatt tttagtagag acggggtttt tttatgttga ggttggtttc gaatttttga 133620
   ttttaggtga tttattcgtt ttagtttttt aaagtgttgg gattataggt atgagttatc 133680
   gcgtggttta atttatttat ttaaagtgta taattttatg gtttttagtt tatttagaga 133740
   gtagtgtaat tatcgttatt ataattaatt ttagaaaatt gaaaatttga gaaatttaga 133800
   aaattttttt tttaagaaga aatttcgtag tttttagtta ttaattttgt tttttttttt 133860
   tatatttttt ttttatttgt gtggattttt tttttttttt tttttttttt tgagataggg 133920
   ttttattttg ttatttaggt tggggtgtaa tggtttaatt tttgtttatt gtaattttta 133980
   ttttttaggt ttaagttatt tttttattat ggtttttcga gtagttgaga ttataggttt 134040
   gtgttattat gttcggttaa tttttgtatt ttttgtagag atggggtttt attatgttgt 134100
   ttaggttggt tttgaatttt tgggtttaaa tgattttttt gttttggttt tttaaagttt 134160
   tggaattata ggcgtgagtt attgcgtttg gtcgatttta gatatttttt gtatagagaa 134220
   ttgtacgttt ggtttttgtg tttggttttt ttatttggtg ttatgttttt aaggtttatt 134280
   tatattgtgg taggtgttag tgcgtttttg agtaatattt tattatggta tggattattt 134340
   tattgtggat agatatgttg tttttattag ttgagggata ttgggttgtt gttatcgttg 134400
   gtgagtgtat tttttttaaa ttaaaaaaaa aatgttggtt attatttatt agatcgtttt 134460
   tttggtttat agggtgggat aagtcggttt gggtgagggt taggaggtgg ttaggtggag 134520
   gcggttggag cggttttggg ttgtttttgg gcgtagtagg tagaggtgta atggtagagt 134580
   ttgagtaggt tttttgggcg gatttttatg tcggtaatgt ttgtttattt aagaagtttc 134640
   ggttaggcgt ggtgttttat atttgtaatt ttagtatttt gggaagttga ggtaggtaga 134700
   ttatttgagg ttaggagttc gagattagtt tggttaatat ggtaaaattt cgtttttatt 134760
   aaaaatataa aaattagttg ggtgtggtgg cgtgtatttg tagttttagt tattcggtag 134820
   gttgaggtag gagaatttgt ttgattttag gaggtagagt ttgaatttag gaggtggagg 134880
   ttgtagtgag ttaaaattgc gttattgtat tttagtttgg gtgatagagt gagattatgt 134940
   tttaaaaaaa aaaaaaaaaa gaaaaaaaaa gaaattttta gtgttgtttt gtgttcggta 135000
   tcgaagtcga gggaaggtag tattagtatg agtttttagt ttatcgggtt tttgtttgga 135060
   gttgacgtgc gattaaggta tacgagggaa gtatggcgag gtggacggtg cggaggtata 135120
   aggagtaaaa taaggtagat gtagtggttt agtggttttt ggtggttgcg aggagcgtgg 135180
   gtaggatagt tttaggaggg tatttggaga gtagggtcgg ttggggtaag gtgtgttagg 135240
   tatttgttag gagtaaaatg taaggggata ttaaagagtt taggaattaa tatagatagt 135300
   atttaatgta gtatttttta atatcgaaat gatgtatagt aatttttatg atgaataaat 135360
   gttggaattt taaattaaga taggtttagt tttgtacgtg tatagttttg ttagagtttg 135420
   tttagcgtgg aatttgattt gtttaaaatg ttgttttaaa atattgtttg atttttggat 135480
   tttttggtat tgttttaatt tttgtatttg gacgaaggtt ttattttaat tttagttgga 135540
   gagggttaag ggttggtgaa tttggttttg gatttgtaga gtttgtggta tttgagaggt 135600
   ggggttagga ataggaagga gttggtgaga acggtattcg gtatgggatg gggttagatt 135660
   agagtagtat tgtggttttt tttaattcgt ggttttgttg ttggatgttt ggcgttggtg 135720
   ttgagttttc ggttttttga acggagtgtg atgaataacg gggtcgggga gtttttgggt 135780
   ttttttttga agtagtagtg aagttaggga tatgtcggta gtaggtaggg agagggacgt 135840
   ttggtttgtg tttggagtta gttgttttat ggaggagttg ggtgaggatg cggtgggagg 135900
   aagatttttt tagttttgta tttttcgaag tataattgag tgtttgtgtg gtttgggggt 135960
   tattttggtt cgaggatagt tttgttttgt ttagttaggt tttgaagagt tatcgggata 136020
   cggatagtag ttttgttggg tggtaggttg tttattttcg tttatttaac gttggaattg 136080
   gatgtagagt atttggatgg ttagttttag agtgtttttg atatttttag ttattttatt 136140
   ttttagatta ggataagatg cgggatttta atttcgagtt ttttatgggt tttattttat 136200
   gtgtttttat aagttattgg gtttattttt tggttttagg gttgtttggt tatttggtat 136260
   tttggttttt ttatttgtat aatgaaaata atagttttag ttttttcgag gagttataag 136320
   atgtttttcg tgtgaaaaat tgggatttgg tattagatta tttggatttg aattttggtt 136380
   ttattttttt taatttgtgg ttttgggtta atttggtttt tttgttttta gtttttttat 136440
   ttatagagtt attttattga gttgttggga ggatgaatat aaggaagtgt atttatgtgt 136500
   ttagagtatt gtgattttat ttattattat taaacgaggt atataataag tgtttaatag 136560
   atgtttagtg ttattttacg gattttgtgt gttttgtgtg tttttagtag ttaagttttg 136620
   gaggtgggga agttgttgac gagttgtacg aggtaattag aaagaggagg ttataggtgt 136680
   aagggtttgg gtgatatttg tggatagaga gaggtcgatg gattttggta gaggggaagg 136740
   gggtttttgt atatagaaaa tgttttttgt atttaagatt tttgtgattt tggaatagag 136800
   gtagtagagg tttgtaaatt ttaaattata aatttagaaa gtatagaata ttcgtagaga 136860
   ggattagaaa tattatatat tttagtgtgt atagaatttt ttatatagtt aaataaaagt 136920
   acgtttttaa ttgaagttgt tggagagttt ttgaggattt tatattagat aagggtcggt 136980
   aagtggaagt ttaatacgta ggagttattt tttttgttta taaatgtttg tcgttttttt 137040
   tgtttatatt cgtatttatg tgagggtatt tataggttta tcgtttagtt ttatgttttg 137100
   tttgttatta aaaaattttt taagaatttt ttagttgtaa atttttttat cggagggggg 137160
   gttttaggaa tttagttttg ttcggggttg gggtatttta gtatttgttg tataggcgtt 137220
   ggttattaaa ggttatgtgt cgtattcgtg ggttttcggg gtttggtggt atttgggtat 137280
   atagtttagt ggcggttttt tatttgttag atattgggag tggggggttt tttatttttg 137340
   ttttttggcg gtggggggag tgtttcgagt tgttatatat tagaatttat cgatttatac 137400
   gttgcggtat tttatagttt tttgatagtt ttttagtttg agagattatt aaattgagtt 137460
   tttggagagg gtggttgttg aggtttggat ataggtgtgg ataggatagt tttgttttat 137520
   ttcggagttt cgtttcggtt tagttttttg tgggtttgaa gtttaggttg ggttagtttt 137580
   tgttttttag atggtcggaa aggtagttta gttttacggt ttttttgtta ggttttgaga 137640
   gtcgtttggt ttttagtttt gttttttttt ttttttaaga tggagtttta ttttgttgtt 137700
   taggttggag tgtagtggtg ttattttggt ttattgtaat ttttattttt tgggtttaag 137760
   taattttttt gttttagttt tttgagtagt tgggattata ggcgcgtgtt attatggtag 137820
   gttagttttt gtatttttag tagggatcgg tttttattat gttggttagg ttggttttga 137880
   atttttgatt ttaagtgatt tgtttatttc ggttttttaa agtgttggga ttataggtgt 137940
   gagtcgttac gtttagtttt tagttttgtt ttgatttttt tttaaggtcg tgaggatgtt 138000
   tacgtttttt ttttttttta cggtttattg atgtttttag atgttttgta aagttatttt 138060
   tttttcggga gttttttagt tggtgacggg ggattttttg gggattattg tttaggtagt 138120
   tatgtaggtt tgtgggtcgt gtttttaggt tggatcgaga ttcggtgtat ggggtgtttt 138180
   tggtttgagt tgaggttttt ggaagttata gttaggaggt agtaagggac ggttgttatg 138240
   tttttttttt tttgtttttt agggtttttg cgggatagat ttttttgttt gagttttttg 138300
   gttgtttata ttttggtttt ttaaagttta gtttgaagtt ttaggtattt tataatttgt 138360
   tttttttagt agtttgtttt gttttttttt tgtcgatgtt tttgaaattt ttttgttttt 138420
   ttttattttt tggttagaga gagattggag attttgtggg aattttcgag gggagggaga 138480
   aggtcgttgt ttgaacggag gtggtttttt tttttgtttt tttttaggcg ggttatattt 138540
   gtaggtttag ttttttgttg tggtagcgtt tggggtgtcg gttgtttttt gggtacgagt 138600
   tgggagttga gggaggcgtt tttaggatcg gagtggtaat ttagttggag gaggagtaat 138660
   aggttcgtgg ttagtagtag tcgtcgtagg gatttcggcg ttgtatttgt gttttagttg 138720
   atttttttcg tttttttagg gtgggtcggg gatggtggtt tgttgtttag tttaggaaga 138780
   agaggggtcg taattagtag tagttatttt aggggatttt agcgttgtat ttgcgattta 138840
   gttgattttt tttttttttt tagggttggt tgggaatggt ggtttgttgt ttagtttagg 138900
   aggaagaggg gttgtggttt ttattttaga ggtttttttt tcggtttttt tttcggtgtt 138960
   ggtttaattg taggtttgtt ttaggtcgtg tcggtatttg ttgattgacg tttagcggat 139020
   gttgatagga gagggttggg atggggaagt agtaagtttc ggttattgtg ggttcgggtt 139080
   atagatggtt gaggtatttt ttggttattt ttaggaagta aggttttacg tagttttcga 139140
   tttatttatt gttcgtgtgt attttaggag gttgtatttt ttaaggtttt tattaggtaa 139200
   ttttattggg ttgggtgtcg ttgtaaaatt atttattggg tgcggtggtt ttttggtggg 139260
   gggtatggtt ggggaggatg cggtggagga gaagttagtt tcgtggttgg ggggagggcg 139320
   gattattcgt tttttgtttg ttttttgaat tggagtttat ggtttttttt ttaggtgttt 139380
   gggttatgga agaggttata gttttgtttt atttttgtgg tagtttaggg gagttttggt 139440
   tagtttcgtg ttttttggga gggcgatagt ttggttaggt tgtagggtag gattggaatg 139500
   tagatttttg tttggttatg ttgttgtttg gttgttttgt ttggggattt tttgtttagg 139560
   aagagagaag cgggtcggat agttcggttt agtgattgga agtttagttt tttatgttta 139620
   gtttagtagg aagtttgttt tttcggaata tttgcgtgtt tttgtatgtg tttttttggt 139680
   tggtggtttt gcgaggtttt tattgggttg cgttatgttt tttggttatt ttggttttgg 139740
   tgtttggttt ttgtgaaata gagatatttg ggtagttttt tatttgtgtt tgtaagtttg 139800
   tgtggtttta gtgatggttg attttgtttt atttagagga tttgggaatt tttagtttaa 139860
   ttgtttagag gagttttttt tagtggggag agtttgagtt gtttttttgg tggatagttt 139920
   tttagatttt aggcggattt ttttgtcggt cgtttttttt gttgtttttg tttttttgtt 139980
   ttttatttgt tttttatttt tttttttgta gtagttagtt ggtacgggga tttttttttt 140040
   tttttgggat tgtgaatgag acgatttttt tggttttttt gggtttagat ttttttggta 140100
   atagttagtg aaatttttgt tttaggtggg aggagaaggt ttagtatgtg gttataataa 140160
   cggttttaat tatattattt ttaaacgttg gtttttattt attttttgtt ttgtttaata 140220
   gttattttta ttattttatt ttttagatta ggaaattaag gcgtagagag ttttagttat 140280
   ttgtttgagg tttttatagt taagtgtttg gatttgaatt ttggagtagt ttggttgttg 140340
   agttgttttg gagacggggt attgtgtagt ggtcggggta ggggtggagt tttgtcgtcg 140400
   ttgattttcg ggttatttgg agtgataata tagtttagtg tttgggtttt tgtttgagat 140460
   tttggttgga tgttaggagt tcgagatttt ttagtttttt tttgtgtaag gcgttatggg 140520
   gtgaaatgaa agaaaatttt agttagaagg tgtttttttt tttttttagt tttttagttg 140580
   ttttggattt tgaaataaga tatcgttcgt ttgtggggtg agtaggaata gattggaatc 140640
   gtattttttt ttttttagtt atagttgtta ggagtttatt tttttttgtt tttttttttt 140700
   gtcgtttttt tttataagat tgggttggat gttgttggga aagttaatag gaagtttttt 140760
   ggttgcggtg gaggtggggg tttgtgtttt tttggggatc gagatgaggg ttttaggttg 140820
   gtttcggtaa gagtagggag gtttacgttt ttgtttgttg gttttgtttt ttgttttggg 140880
   agaaggtatt tatatgtttt agaatgtatt ttttgcgtgt atagggatgg tttttgggtt 140940
   atttatttga ggtttattga tttttttttt tggaattgga gtggtttttt tacggtggat 141000
   tgggttgagt tttttgtttc ggtcgacgtt tttttttagg tatttttttt tatcgtgtta 141060
   gggtgtaggt tgttagtgga tgggttttta ttttcgggga ttggtagttg ttacgtttat 141120
   tttatagtcg agatattttg gtcgaaggtt ttgttaagtt ttatggagtg tagggatttt 141180
   ttttgagacg gttttttttt gtcgtttagg ttggagttta atggtttaat gtttcgttta 141240
   ttgtgatttc gattttttag gtttagtagt ttttttattt tagtttatta agtagttggg 141300
   attataggtg tgtgttatta tgtttggtta attttttaat tttaattttt ttttgtagaa 141360
   atggagtttt attatatttt ttaggttgaa ttttaatttt tgggtttaaa tgattttttt 141420
   gttttggttt gttaaagtgt tgggatttta ggtatgagtt attgtgttta gttggggttt 141480
   ttttttaatt ttttttggtt ttttttaggt gtaggattcg ggttttagtt ttatgtgttt 141540
   ttgaatagtt ttgggttttg ttttgtattt tttgggcgcg tggttaggtt tttcgtgata 141600
   ttagtaggag atttaggtga agaagtaggt agaacggaat gtttgtttat ttagtttcgg 141660
   ggttaggcgg ggcggttagg atagtagtag tggttggtgt ggttttttgg gaggttagtt 141720
   tattggcgtg gttagcgaat ggggagggga gttttttttt ttgttttttt ttatttttgg 141780
   tttttgggtt tttagggtta ggtttttaga gttttaggtg gaggatggag gttagggaag 141840
   ttggggtaag agcgggttgg gggtgggtgg ttaggcggat tttggaaaat tattttttgt 141900
   aggtttcgtt ttttttaggg agtatttagg gttggtttcg gggagtcggg ttaatggtta 141960
   tttcggatgg tgtaggggtt tatttttatg aaatcgggtt tatttttatg aaattgggtt 142020
   tatagaagtt tttggtttcg gttttgtttt gttttttggt ttttgttttt aaagtaggat 142080
   ttaggatagt tttgaatttt ttgtcgaaat gtagtttttt gggttttaat ttagtttttt 142140
   agaattagag tttttggggt gggttttttg tttgtttagt agtttttagg ggatatttgt 142200
   gtttgttaga gtaagtttaa taagtattgt tgtaaatttt tcgtttttat agttttttgg 142260
   ttcgtttttt tttttttgtt gggatttgta ttaggattaa atgttaatat tagggattag 142320
   gttatatttg tgaattttga gataatattt gtattttttt tttttttgag gttgttttgt 142380
   tcgttttgtt atttaggttg aagtataatg gtgtgatatt tatttatggt aatttttatt 142440
   tttagggttt aagtaatttt tttgttttag ttttttgagt agttgggatt ataggtttat 142500
   attattatat ttggttaatt tttgtatttt tagtagagat ggagttttat tatgttggtt 142560
   aggttggttt cgaatttttg gttttaagcg attcgttcgt tttagttttt taaagtgttg 142620
   ggattatagg cgtgagttat tgcgttcggt ttgatattta tattttgatt aagttgttgt 142680
   ggaaatattg agtttttttt gatagttttg tttatagagt gaatgtttag gaatcggcga 142740
   ggtatttttt atttttattt agatggcggt ttggaggtag cgtttttgat tttgtgatgg 142800
   gatttgtttt ttttgtttaa gagattttag agatgtggtt tttgaatgtg ggggtttttg 142860
   ttggggtggg aacgggagtt ggttttggtt attgtttagt gattttattt tagtgtttcg 142920
   attttagtat tttcgatttt agcgtttttt ttttttcggt ttcgcgcgga gattacgtgg 142980
   tttgcgttga gtaatttttt attgttttcg tcgcgttttt gttgtaggga agttttttta 143040
   gggagttagt tgatatattt ggtttttatt atttgttttt gaataggaat gaaaggagag 143100
   agtgagtaag aaagtggtag gaggtggggg ttgtagagta ggggaaggtt agagagaggg 143160
   gaggaaagat ttggaaagtt taggtatgtg ttttggaagt aggtgttagc gatgagttta 143220
   tttggagggt ggtcgttcgt tttgagtttt gtttggggtt agtagggaat attttgttag 143280
   ggtatgtcgt tgggagtttg tttttggttt attttttgtt gttttgagcg ttgagtagat 143340
   ttgtttttat tttagggata acgtaaggag gttgttattt tattttttat tttattaata 143400
   ttttatgaat tataagtgat tatttattgc gttttgacgt attttaggcg ttgggttcga 143460
   agttttatgc gttttatggg atgtatttgt attatcgtcg tagaaaacgg tttttgttat 143520
   tattgtagag aaggtatcga ggtatagaga ggttgaggaa ttggttttag gttatatagt 143580
   tagaagcggt tgattttggt tgtgaatata gggttgtttg attttaaagt ttgagttggt 143640
   ttgtagcgag gtttatggtt tttgtgttat tgtttgagtt tttggtcgag ttttttggtt 143700
   tagtgggggg tttagtgatt tattagttgt tttattatat gttgtggata tttttacggt 143760
   ttatttgggt gggagggaag gggttttagg aggtgggagg ggtataggga atatagttta 143820
   gtttgtttgg gtttaatatt ttgattttta aggtattttt tgagggtagt atgaggttaa 143880
   gtttttgttt ttttttagga ttttgttttt gtgagggttt cggggttttt tgtttttttt 143940
   tgggggtttt gttggttagg attatagaga agttttttgt tgttgatttt tttgtttttg 144000
   tattttttgg taagttattt tatgatttag ttgttttgtt tgtaaaatgg gttgaataat 144060
   attgttttgt ttcggagggt ttttgggagg attaaatgag atttgttgta tagaagtttt 144120
   ttgtaagtta tgttgattgg cggtcgtcgg gtttttaagt gaatgggtat tcgggtattg 144180
   agtgtgtgga tttagtattc ggaaggaggg gttggggggt atttagaatt tttgggaggt 144240
   ttggattttt gttttgttta gattttgagt ttttttttgg ttagtttaat ttttattagt 144300
   agggtgaggg taattaggat ttacgtatag ttgggcgaag tcgagtgatt atatgtggaa 144360
   gtttagcgtt ttttatgtaa agtgggtagg cggttaaagt gagttttgtt ttagtttttt 144420
   tattttcggg gttgtttttg gcgttttgtt tttttttaga gttttattga tttgtattat 144480
   gtgattcggg tttgggaaga tgggtttagt tgtgggaggt cgtgcgggtt ggggattgag 144540
   agtgtcggag gagggttttt taggaaaatt tagttgtata ggaagtcggg ttagcggttt 144600
   tcggaagttt ttgggttttg taagttggat tttttagatt tgtggttgtg gggttttttt 144660
   ttgtttgtag ttgagagttt ttgattcgta tttgagggtt ttagaggttc gggaggggga 144720
   tgtgatcgtg tttttagggt gaagtttatt cgagtgttat tttggatata gtttttattt 144780
   ttttttggtt ttttttttgg ttatggggac gttgtttggg gggggttttt ttatatggaa 144840
   ggagttgtag ttgagttggc gggtttcgta tatttttaag gtcgtttgat ttttagtttt 144900
   taagtggggt tgtatttatt atttaggatt tggagtttta gttagagagg gattttttat 144960
   tgtttttttt tttgtttttt gggaaaggta gggtttttgt gttttttttt atttagggat 145020
   ttggtatgtt gggaggttaa gaggaagttg tttttcgttt atttattttt taattttttt 145080
   agttagtaga gttttatttg tgtgtttaga atttgtgcgt ggagttagaa gttataaggt 145140
   tggtttagtt gtgtttgatt ttggttgtgc ggttttggat agttttagtt tttttggagt 145200
   tgagtatttt gatttgtaga ggagaggttg ttgtgtcggt ttcgtagggt tttgtgtaga 145260
   ggggagtgtg tgcggttgtg agtttaggtt atgtttttag attggaggat aggatcggaa 145320
   tagtgggtta ggatggttag agggttttag ttttaggagg aggtttttat attttttagt 145380
   atttttatat taggaaatgt agttaggttt ttaggttttt tatgttagga agtagtattt 145440
   cgttttttat ttttggttgt ttgggtttga gaagttgagt atttttttcg aattcgagtg 145500
   ttttttgatt tttgtttggg tagttgtttt ttttgaattg atcgtttgtt ttatatagtt 145560
   ttttgtggtt tgttcgatag ggtggttagc gagttttttg tatagtatat tttttttagg 145620
   agtgttgttt gtgatgatgg tgtaaagggt gttttttcga gttttcggag gtatgtagtg 145680
   gcgtaatagc gtgagtttag gagtttcggt gagtttttgg atggagaatt tgtttagatt 145740
   attttttagt ttaaattttt tttgggtgtt tgttgtgttt ggggcgttga tttatcgtgg 145800
   ggcggtttgt gagattttac gttatttggt tatttttttt agtttttgtt tattgtatcg 145860
   gttttttggt ttggttgttt ttggaatatg agggtttatt tttacggaag aatatttatt 145920
   tgtttttttt tggaaagttt ttttttagat tttttggtcg ttttatttag gttttgttga 145980
   agcgttgtta ttttggagag gtttttttgt tattcgtagt taaaatagtt tttttatgtt 146040
   tgtgggtggt tttttatggg gtatggatga gtattggaga gttttgtcgt ttatttgttt 146100
   atatgttttt tgggagggta ggggatattg atttgtgttg tgggtgtttt gtatgttacg 146160
   ggtgtttagt gaatgtttgt taagtattag gggagtataa aggagggaag tttttgttta 146220
   attggagtag ttagggaaga ttttttggag gaagtggtat ttggtttgtt tagaatgtgg 146280
   cgatttgttt gtatgtagat attagattag agggtaaagg ttattattat aatttagtag 146340
   atagttataa gtagttaagg aattgttatg atatggattg ttcgtgattg gagttaagaa 146400
   ggagttagag atagaggttt ttgtgttttg gcgtgagttt ttgggggagg ttgagttttt 146460
   agaatgtggg gtcgtttgtg tagaggtcgg aggtgggaga tatatttgtg agtgtcggtg 146520
   agtgtggggt gagttattgg ggttggatta gaagatggga atttttaaat ttattaatta 146580
   gttttggtgt tttgggagtg ataggagacg gggtttttgg gggattaatt tagtataagt 146640
   ggacgagatg ggatgtaggg gaaaggataa agttttttga ggttttggga agttgagtta 146700
   gggtagagtt atgatagggt gagttttaga agtaatataa gaaaaaatag ttagattggg 146760
   tttttttttt attgtgggag atgttttatt ttattttttt cgtttttcgg gttttggtaa 146820
   tgggttgggt atagtgaatg ggttttattt gtaggagtgg ttatattttt tgggtatagt 146880
   tgttttatat attgtattga gatgggattc gaggtcggga agaatagttt tttttgtagg 146940
   ttgatattgt tagttttcgg ggagggaagg acggtagggg gtttggagta tttttttttg 147000
   ttatttatat tttgtgtttg tttgtagggt tttgagtttt gtttgttttg ttttagtttt 147060
   ggttaggaat ttgttggagt ttataggggt agttaggttg gaataggagg agattaggtc 147120
   ggtaggggag tcgagagggg gttttggata gtatttaggg ttgagttatt ttgggtttgt 147180
   tcgatttgtt atttggagtt gttttcgttt gtttattatg tgtaaggttt tgatttattc 147240
   gggtttgttt aattagcgtt tttttttagg tttagttagt ttggttttcg ttcgatttat 147300
   ttattgttat tagtgttatt tttgtattac gtaattagtt tttgtagtat gttagttatt 147360
   aagtttaggg ttttttgtgt attttttgta atttttatag taattcggga ggtatagaga 147420
   ggttgagtcg ttggtttata gttatatagt aggtatgggg tattgttagg atttagtcgg 147480
   atttttgatt ttagggtttt tttagggttt tgggtgggat attggtaagg aagtaaggta 147540
   tgaatattgg ttttgttttg gatagagttc ggggacggtg gtttggtagg tttagttgat 147600
   atagtgattt tggtgtaggt ttaattattg gtaaagtgtt taagtatata tagaatttta 147660
   attggggaga taggcgaagc ggttaaggga gggttgtgag tgataggtgg atattgtatt 147720
   tattggtaga ttagttgatt ttggtgtttt ttggagtttt ttaatttttt tatttagaaa 147780
   aagaggaatt aatagtttta tttttaaatt gggtgggagt attatattcg aaattgaagt 147840
   aaagttggtc gtgttatagg ttcggtagat gttagttgtg taggtaattt agtgattatt 147900
   tgaagtaggt tgtgcggcgg tatttttatt ttgtagatgt agtttagaga ggttagggta 147960
   ttaattttgg gttatatagc gatagtttag gttatagaga gtagtgttgg atgggagggt 148020
   gttggtagtt ttaggtatag gttgggaggg ttgtggggtt agtagaggga gggtatattt 148080
   tttagttggt ggggtttttt tgtgttttag tatttatgtg ggtttaaagg agttggaagg 148140
   agggaggtta gagttattta tgggtttaga agttgataag taggttattg attttgaatt 148200
   tgttttatcg tatgtaaatt ggggataaat ttggggattt aatgatataa tgtaagaagt 148260
   agatatgtta tagttttggt tatatagtta gtgtataata agtggtaagt cgttagagga 148320
   ggtagtcggt gagaatggga gtattttagt agtttttcgt tttttgtttt tggttgtaga 148380
   tgtatttgtt tttggttatt tgtgttttag tttcgtgttt ttcggtgtgt gtgaggttaa 148440
   gtttttgggg tggggatttg ggggtgtgtt tgtagttttt gaggttaaga ttaaggttga 148500
   ggtcgaggtt gaggttgagg ttattttggt gaggaggaaa ttgtaggtgg agaagttcgt 148560
   tgatcgtgat tttgatttga tcgtaatttt gatggtgtcg atgtcgttag tacgtaggtt 148620
   ttttgttttc gttacgattg gtttttgtag tttatttggt tgagaggtgt gttggttttc 148680
   gtaggttgta aaatggggat attatcgttc gtttgggtta tagcgtgttc gtcgattgta 148740
   tttggggagg gattgagggt tgattgtgtt gtggggatgt tggtagagaa ttagagaggg 148800
   tatttaaaaa ggttaatagt tcggtgggga gaggaggcgt aaggttggtt tttgtttttt 148860
   gggttttgat cgaaagggaa taggggatat atttggggat agtttttttt ttggaggaag 148920
   aagggtttag ggtgttggtg tggagtttgt atagatgggg ataatgagtt ggagggtgtt 148980
   gggtaggagt tggttagtaa taggttgacg agggaggttt tgttggttgg gtttggtttt 149040
   taagtgagta ttttgagttg cggggattta gggggtttta gagattttag agttgagatt 149100
   cgggattata gaggggtggg ttgtgtttag ggtaatatag cggatggggt agatgttgcg 149160
   aaatttgttt ttttgttttt tttttttttg ttatagaatt ggattttttt agtttatggt 149220
   tgttaaattt tttagatgtt tttgggtttt atgggcgttt tatattggtt tttttgtagg 149280
   atagtagatt ttgggggttg gttttgatgg tatttattat ggggtatttt atgtaagtat 149340
   agtgttttta cgtttagttt tttagtttgt ggagtaggtt tgtgtttggt tagttttatt 149400
   ttgtggttag aggagggaga tagtggtttt ggagatagga gttggtttgg ggttgttttt 149460
   ttagtttttg tttttttagt ttattttttt gttttgcggg tgttggttat ttttttttcg 149520
   ttagttttat ttttagtttt ttttgttcga gtcgtgattt gttagtaggg ggtagtggag 149580
   gttttgaaag tttttggaga ggattttgaa gtagttttta taaatttgtt tttggaaagt 149640
   tttttttgtt atatttttag cggttttttt ttttttaata ttttttttgg ggttgttatg 149700
   tagcgggggg tggaggaatt tattaggtcg tggatagagt agagttatgt tgatataaat 149760
   tttggttgaa tttatatgtt ttttatagtt agcggtcgat tattggaatt agggaagtga 149820
   ttttatagta agggttcgta gagtagttat tacgtatttt cgattttggt ttttggattt 149880
   agttttggaa aagaagttag attttagtta tttggagttg tttagggaag attttagttt 149940
   ggtgtcggtt gatagtttat tggaattttt tatttgtttg gttaggtgat gttaaggggt 150000
   agaggttaga agtgttaaga agtaggtcga gggtggtggt ttacgtatgt aattttaata 150060
   ttttgggagg ttaaggcggg tgaatttttt aaggttagga gtttaagatt aggttggtta 150120
   atatggcgaa attttatttt tattgaaaat ataaaaatta gttaggtatg gtggtgtacg 150180
   tttgtaattt tagttatttt ggaggttgag gtaggagaat tttttgaatt cgggaggtag 150240
   aggttgtagt gagtcgagat tgtgttattg tattttagtt tgggtaatag agtaatattt 150300
   cgtattaaaa aaaaaaaaaa gaagaagaat aagaagtgtt aagaagtatt tgtttttttg 150360
   attgaaaagt aatttttgtt gggcgtggtg gtttacgttt gtaattttag ttttgggagg 150420
   ttgcggtggg agagtcgttt gaggttaggc gttcgagatt agtttgggta atagaggtgt 150480
   taggggtagt ggcgtttgtt tgtagtttta gttttttagg aggtttaggt aggaggattg 150540
   tttgagttag gagtttgagg ttgtagtgag ttatgattat attattgtat tttagttttt 150600
   gggtgatagg atgagatttt gttttaaaaa ataaaatata aataaaaaat taacgtttat 150660
   tttttgtgat tttaaaaatt atatttattt gtgataaaat aattaaatta gaaaatgtaa 150720
   aggaaatgat taataataat tgcgtttttt agataaatat tgttaatatg tttacgtttt 150780
   tttttagttt ttttttatat agatatttaa tatttattaa tttgttttga attaaagatg 150840
   gttttttttg ttttgtatga gaattatttt agttaggtac ggtggtttac gtttataatt 150900
   ttagaaagtt gaggtaggta gattgtttga gtttacgagt ttgagattag tttgggtaat 150960
   atggcgaaat tttattttta taaaaaaata taaaaattag tttggtgtgg tggtatatgt 151020
   ttgtgttttt agttttttag gaggttgagg taggagaata gtttgagttc gggaggtaga 151080
   ggttgtagtg agttaagatc gtgttattgt attttagttt aggtaacggg agtgaaatgt 151140
   tgttttaaaa aaaaaaaaaa aaaaaaaaaa gttgttacgt tgttattttt tttagggatt 151200
   tttatttttt ttatttttcg tttaagattt gttttgtaaa aggatgaggt taaatagtta 151260
   tgggagggtg gtattggatg tttaaaaaaa tgtttcgtgg ttggcgggat ttagaaatag 151320
   gttggggagg ttttagtaga gggtggattt tagttggggg tggggggtag taggggggtt 151380
   ttttatatgt tttttttgcg tgttttatgg ggttgtttgt gaggtcgtgt gaattagagt 151440
   tcggttggga agttatatgt tatttggatt ttgatttttt ttggtagtat gattttagtt 151500
   aatacgttta attatgtttt tttttgtaaa atgggttaat agaaatgttt attcgagata 151560
   tgatgagaaa taaaggtgtt tatagagtaa agtgtttaga acggttttgg tatgatagga 151620
   gtttatatgt tagtgttgtc gttggtatcg ttattattac ggcggtattg gttgttttgt 151680
   tttttttttg tttttgttat tttttttttt tagtaggttt ttatttattt gtttatttat 151740
   ttatggtcga ggtttcgttt gtgttaggtt tggtgttgag aacgggggta gagagatggg 151800
   ggttgtgttt tgttttttga tttaagggtt ttttttgtaa tgtgaagttt ttggagattg 151860
   gatttttagg ttgttttatt agtttttttt taggttagtt taattttttc gtgttcgttt 151920
   tttgagttgt tagaggaaga gagcgggagg aaggatggag tggggttttt gtggggattg 151980
   agcggggggt gtgtgaggtt ttggaaatgg tgtttgcggt atttacgtag tattagtttt 152040
   tgttgtagtt gttgtttgga gaatattttt ggattttagg atattgatga tagaaggtgg 152100
   aacgtttttt ttggaagtta ttaagagggg attatcgcgg cggttgagta gagttgttga 152160
   tttgtgagtt gtggtttgat tatagttatt gtttttatga gcgaggtttt ttgtggtttt 152220
   ttttttagga atttttggtt ttttggtagg ggatagttta gggttggggg tgggtagtgg 152280
   tgtttgtagg tttttttgga gttagagaat tttttgtcgt cgtggcgggg ttgttttttt 152340
   ttgttttttt tttttcgttg tgtttttagt tgtttcgata ggatgttttt ggtttgagag 152400
   atagaaattt taattttgaa tggttttagt ttttaagaga atttggagtt tttttgtgta 152460
   aaatgagatg ttttgagtat cgaggatgtt gtgggggatt ttttatttta ttatttttag 152520
   tcgtggtgtt tttttcggtt ataggatgtt gtagttacgt tgtagggaag gagagttttt 152580
   ttttttgtga gtttagggag tttttttatg tagttttttt attggttatt gtttgagatt 152640
   cgatttggtg gtttttgttt taaggagtag aaagggggtt cgttaggtta gggtggggtt 152700
   gggtgcgagg atttttttcg gttaataggg tttgtttagg tgttcgtttt gttggggtag 152760
   tagggaaggg atttatatgt ggtattggga tacggagagg tttttgtgtt tgtgacggtt 152820
   aaaagggatg gcgttttagt tacgttttta tttatttcga ggtttagtat ggttatgtcg 152880
   gaaatagtag tatcgttttt tttattgttt tggtttgttt ttaataggtt agaggggtgg 152940
   ggttggagcg ggcgtttgat ttgtggttgg atttagttgt tcgtggatag gttgtgagtg 153000
   aatttgtaga ggaagggaga tgggaatgtc gggatttttt tttgtatttt tcgaattttc 153060
   gttttttgtg agttagatgg ggagtgtatt tttgtttttt ttttttgggt attttttgcg 153120
   tttttcgttt taggtatttg atgttgtttg tttatttttt ttttttgttt ttttcgttgg 153180
   ttttttttat atggttttaa ttaggatttt tcgttcgttg gtggatgggt agagagggga 153240
   gtttgttgtg gggttttttt cgatcggggg attttttagt atagagtttt ttggtttttg 153300
   tagggacgtt gtgtgtgttt gtgtgagttt ggcgtgttag aagcgttaag ttgtagttgt 153360
   tttgtgcgtt ttttatattt tttatttttt aaaggttttg ggtttggtgt ttgtgggttg 153420
   gggatttgtg ttggggtttt taggaggttt ttggtgggag aggtagggtt tttttttttt 153480
   taatttgggt aattttgttt tggtttgttg tttgcggaaa gtgttttagg atacggtggt 153540
   aggattggtt ggggttttgg ttttaggttt agttttagtt tttgttagat ttattttttt 153600
   tgtgttttcg tttattttgt ttattcgtag ggtttgtagg agtattttga gagttggtag 153660
   ggtggtttgt tcggcgttcg tatagggttt gttggtgggt ggttgaggtt gcggtgaggt 153720
   ttttatggtt ttttcgtttt gtatattttt tttttgttgt gttgtttcgt tagtatatga 153780
   gttcgtattg tttttgagtt tgtagtttgt tttttttatt aggtggttaa gtagggtggt 153840
   agttagggtg gtagagtttt attggtttgt aggggatttt ttgtgggttg taggtagttt 153900
   tttgtggttg aaggtgtttt ggtaggattt gtattatgat tttcgttaaa gtttacgttt 153960
   aaggtatttg tgggtatttt cgttttagac gtgagggttg gtattttttt ttgtacgtga 154020
   agtaaaggtt tgtttgaagg agcgtgtgga agtttgggtg tgatgaaggt aaggatcggt 154080
   ggtttatttt ttttgtattt ggattttggg tggtaggttt ggggttttta gtttttaatt 154140
   taggagggta gttagtcgtt ggaattaaga gtgaatttta gttgttgttg ttatagttgg 154200
   agggaagagg ggaaaggagg cgttgtaggg gagtagagat tttatttttt ttttgtcgat 154260
   attaggttgt cggtgttgga cggggttttg gtaatcgtgg taggagtggt gatgttcgat 154320
   gatggtaata agggtttttt gaggatttcg agttgtttta agggtttttt atttggagta 154380
   agtatttttt ttaatttttt taataatatt gtgaaattga ttttgttgtt atttttattt 154440
   tatcgatgag gaatttatag tttagagatg ttaagtaatt tgtttaagtt tttttttgag 154500
   tatggtagag ggagggttcg aatgcggaaa gtttggtttt agtgttttta tttttaatta 154560
   ttgttattta ttttttcggt tagttttttg attgtaaaat ttgaggttta gaggagtgta 154620
   gtgatttggt tagtttcgta cggtaggtta gtggtagagt tgagtagtgt tttttatgtt 154680
   ttgattttgt ggttttttga ggtggtttat ttttaaatac gttttttatg gtggttttgt 154740
   tttttttttt tttttgaatt tgttagtttg tgtttaggta gggggagaag tagagtaatt 154800
   atgtcgggcg ggtcgtcgtg ttttagtgtc gtttgtttta tatttgattg gtagtagagt 154860
   ggtttttttt tggtgtggtt tggaggtgtt agtggttatt tattagtata gtttagtttt 154920
   atatatgtta tttttaaagg atgttgatat agaatgaagt agttagggga ggcggacgtt 154980
   ttggtacgag ggtggcgttt ttggtaattc gtagtgttgt tgtttgtggt tgtttttggt 155040
   tggtatttta tttttttttg gttgttttgt agttatttga gggaggttag tatgtcgttt 155100
   ttattttagg atagtgtgaa tgtagtagcg taagtttgtt agcgtcgtat tgcgggattt 155160
   ggaggttttt gtttttgagt tttgtttttg tgtaattttt aaagtacgga ttttttaggg 155220
   gtttttattt ttttatttgg aaaaatttta tatcgtttag ggtttgaggt agggttagag 155280
   tatggagacg ttgttggagg attaagggag aattgagcgc gtgttttttt ggttttagtt 155340
   tcgtgtgtgt gtttatgtat gggtattttg ttatttatat tggtttttac ggttgggtgg 155400
   gagttgagtt gagttcggcg gttgtttttt tattttgcgg gtttggttgg ggagtgtgag 155460
   gatgtttgtt ggaagttaac gtgtacgaaa gagttaatgg tggattttat ttttttagtt 155520
   ttttttttag tttttttagg gaaagggtag gaatagagag gttttatttt tagaggaagg 155580
   ggtgggcggg gatatagcgt tgttcggtat agtttttttt gagttatttt gtatgtttta 155640
   ttttatcgat ttttgtagtt tagttgttta gagcggattt agaaggtgga gagagggttt 155700
   ttaataagta gaggatggag tgaatgtgag atttattatt gggatttggt aaagtaaaga 155760
   gttttatttt tttttcgtag atgggacggg ggcggggttg gagttagcgg ggtgggggta 155820
   tttagtaaga gttttttttt atttttttat tttttttgtt atgggttttt ttttttacgt 155880
   agatttttag tttggaatga ttagtgttta gtttttttcg tttttttttt gtttcgttgg 155940
   agggagtgat ttggtttttg gtttcggtta tttatatttt ttgttttgtt gtattagttt 156000
   cgtggtaggg gttgcggcgg aggtgggtaa aagtcgtttt cgggtagtta gtttttattt 156060
   tgggagtgat tgaaagtttt tgggggtttt tgtagggtga gttgtttatt ttggttttgg 156120
   taaggatatt taggagaggg ttagggtgtt tgtatttggg gtatagtata aagaatagtc 156180
   gtaagtgttt tttaggtagg agggtgtcga agtattggga agttgggatt tagaagtatt 156240
   gtttggggtg gtgagtggaa gatgtaggat atagatttac gcgattgtgt agattatttg 156300
   gttttatttt ttcggtttat agtggaggaa attagaggtc gagagaggtg aagtgagttt 156360
   tttggggtta tatagtttag ttaggtttag ggcgtcgatt ttttgttttg ttttttcgtg 156420
   tttattcggt tggtattagt atttttgttt tgatattttc gtgtggtagt tgttgtgatt 156480
   ttgtgatttt tttttatttt attttagtgg ggttttgcga gggaaagaag aatgttttat 156540
   gtagaagtgg ttggaaagta agaagtttgg agagaagtga ggtttttgtg gtttggtttg 156600
   agtttaggat ttatggtttg gtttttaggg atggagtatc gatttttttt ttttatattt 156660
   ttattttacg tttttaggag ttttttatgg ggaagtcgga atggatttgg gtttagagat 156720
   tgtaaattta ttttgttgga tgtgtggttg tttttatgta attaattttt ttacggagaa 156780
   gttaggggat ggtttttagt attttcgggg gttttttagt gaggtgggag gatttcgaga 156840
   ggttaggagg ttgtttaaga ttttttgttt tttagggatt aggtgagggt ttgtcgattg 156900
   ttttgatttt ttgatgtttt tggtggggag attttagagg ggtttgtcgt tattatcgtt 156960
   agtggttttg gtttgtttag ttattttttt ggggtggtta aattttatgt tgtaaaagtt 157020
   taagtgaatt ttagtagtgg tttgggagaa ttatttgtta cgagttaagg atggagtttt 157080
   tggggaggat tttttgttgt tttttaattt ttttcgggta tatttttttg tagttttatt 157140
   ttttgtaaga aagttaggtg tttttcgaag tttttttttg gttttaggtt agaggtgata 157200
   gtgggggttt tttgtttgcg ttggtgattg tggttggttg ttggtgtttg gttttgtttt 157260
   gtattgtgta tttattgaag gcggtattat tcgggttttt tcggtggtta tgtatgggtg 157320
   agtagtttac gatatatttg tttttagtat cgtgtgggtc ggggttttta gaggggttga 157380
   gggtttggat ggttttgtag gggggtgttt ttggttttgt gatcgaaagg agttgaaggt 157440
   ttggagaagt cgttgggtcg aggttagttt ttatagattt ggcgaggagg gaatagatgt 157500
   ttacggagtt gttttaggta ttgtatatat tgttttattt tgcgttgata gttaagaggt 157560
   tggtttttcg tcgattttta ttttatagat aatgtgtttt tttagttttt tagtggtgga 157620
   gttaggattc gaatttacgt aggtagtttt ttgggtttgt agttttattt tttatttttt 157680
   tttaggattt atgttttggg gttggtagga taatgatttt tagagttgag tatattttaa 157740
   tttttagtat ttgggattac gttaggtgat atggtagggt tagttaaggt tattaattag 157800
   ttggttttaa aatagggaga tgattttgga tgatttatac gggttcgttg ttattataag 157860
   agtttttaaa agcgtaatag attatttgag gggtgtagtg ggagaggttt ggaaggagga 157920
   aggaggttat aagttaagaa atgtaggtga atagaaaatg taggaaaacg tagttttttt 157980
   tgtagtttcg ggagggtgcg tagttttgtg gatatttttt ttgtagttta gtgaggtgga 158040
   gtgtgggatt aatttgtgtt gtttgatatt ataagtgtgt ggttatttgt tatagtagtg 158100
   atgggaattg aatgttatta ggtagtagtg tttttatttc ggaagaatta ggataaattt 158160
   ttttgtggat tttaagtttt gggagatttt gggttttttt ggattgtttt agttttgtaa 158220
   ttataggaaa ggttttttat gtttgtttgg gagaagaaaa tttcgtagta aatttatttt 158280
   tttttttgat aaatgaaggc gagggtgtgg gttggatata tgtttttttt ttgtggattt 158340
   tttttgtttt aatatttaga attttgtagt tgaaggagat tttggggggt tgtttgtttg 158400
   atttgtaagt gaagaaatgg aggtttaggg aagttaagtg attggtttag ggttatgtag 158460
   agttatgttg gaatattaag ttagatagtt tagtttttgt tttaaagtgt tttttttttt 158520
   ggttggggag gagggttgga gggagtggat ttcgagtaat agtagtattg gtaggggttg 158580
   gaagagtagt tattgtattt aggtgttgga gtttttggtt attttttgta ggaattaggt 158640
   gatttattat ggtataggtt tttttttgtt tatttgtagt tttgggggtt ttagggatta 158700
   gaggatattt tggtttgagg gttggggttt gagagaagag atggaagtat ttttagtttg 158760
   gtttgttttc gtggcgtttt gtaggtatat tcggtagttt aggggtttta gagaatttgg 158820
   ttggtgggcg tttttatttt gtcgtagttg tattttgtgg ttagtagttt gtaggatttg 158880
   ggttgttgtt tgtggttttg aaatagttat tttttgagtt gagtggggat taggtgagta 158940
   gatttttata ttttaattgt tgtgattttt ttgttttgga ggttggtggg tagtttggtt 159000
   ggtgttttat ggtattttat gatcggtttt gttattaggt tcgtgagttg tttttttttt 159060
   ttcgggtatt ttgaacggtt tttttgagag tttcgttttg ggttttcgtt tttgtaggtt 159120
   ttcgttgttt ggggcgtttt ttttttttgg aagttgggtt tttttgggga tttattaaga 159180
   agggttttgt cgagtgttgt taaattgttt tatttggttt ttatttgttt tatttattat 159240
   tttattttaa tttttatgtt ttttttttgg ttttaagatt tgagtaggta gggtttgtga 159300
   ggtattttag gttttttgat gttgttagta tttaggggat tgttagtata tatgtaggga 159360
   gggaaggaat gaaggaagag atgatttggg gagtttttta gtttttattt gtaattggta 159420
   tattgcgata aaatatttat ggtattttat tttttttata tatttttttt ttaaatttat 159480
   ttttatttga gatggagttt cgtttcgtta tttaggttgg agtgtagtag tataattttg 159540
   gtttattgta atttttattt ttcgggttta agcgagtttt ttgttttagt tttttaagta 159600
   gttgggatta taggcgttta ttattacgtt cggcgaattt ttgtattttt agtagagatg 159660
   gggttttatt aagttggtta ggttggtttc gaattttcga ttttaggtga ttcgtttgtt 159720
   ttcgtttttt aaagtgttgg gattataggc gtgagttatt gtatttggtt ttcgtatatt 159780
   tataaaagag aatttgttta gaaaattttt tagatgtatt tttagggttt taaggtatag 159840
   atatatgaag tttgttagtg agtagttgtt gtttattttt tttttttttt taatttagag 159900
   cggtttgcgt tttaagcgga tagttgtttt tttttttttg gttttttgtt gtttacgttt 159960
   ttatagaagt agggagaaga ggtaggaagg tgacgcgtga ttattcgttg ggcgttcgta 160020
   tagtgtcgtt tgtataggtg ttattttttt tatttgttat ttttttcgtt gagttttttt 160080
   ttttttttgt agatgagaga gtattgtttt ttagaggtta agaaatgggt ttagagttat 160140
   ttagttgatt agcgtgtagt tagatttgaa ttatattttg ttgattttta tgtttgtagg 160200
   tttggtttga tgagatggat agttgggtgg tggagatttt gagattttgg atggttttta 160260
   aatagggttt gtagtttttg gatagggaga gtttggtata aagggtttgt ggagtgagtt 160320
   gggggagaag gtggtttagg gtttttgttt ttgaataaat agatggaggg gttattttag 160380
   cgttttttta gtttttggtg tgttgggttt ttggttttgg gaggagagaa tggagttggt 160440
   ttttattttg ggttgggttg gtttgggttt aatttagata agttttttag tttgttatga 160500
   gtgatatttt tgtttgtttt ggtgtttttg ttaaaaatat gaagggtttt agaattttta 160560
   tttcggtttg tttttttttt agatttggag tgggtttggg gttgataata gttttatgtt 160620
   agatagtttg agtagttatt agtgttatta ggtttgatat tgcggttttt gtttcggagg 160680
   gaaaaataag gttttttttg ggggattttt tgttagtttg tgaaaatagg tttggagtta 160740
   gttagcgttt ttatgtttag atatttcgtg tggtttttaa agtgttttta tgttgttagt 160800
   ttatatgatt ttttttaaaa ttttataagg gagggtagga agtttgagta atagtttgtt 160860
   aagtatttat atagtaggtg gttttattgt tttttattta tagataagta aattgagttt 160920
   tagggtggtt atcgtttgtt ttttaagtta tttagttatt tagttacgga agaggttatg 160980
   attttttata ttttagtgaa gttgaagatt ttttttttcg tttttaaaaa tgtttatttt 161040
   ttagggtagt gaatatttgg tgtttttgtt tagcgtaaga ggtgaacgtt tgttattttc 161100
   gttttttttt ttttattaga tttgattttt tttttgtgac gggaatattt tgtaggtagt 161160
   ttgttttgtt tttatagatt ttgagaaacg gggtttaaat tgtcggttgt gggtgggata 161220
   ggcgatattg tagtgaagag gtggagtatt tttgtagcgg ggaagaaata tatttttttt 161280
   atttttttag gtttagtgtt tgggggtttg tgaattaaat ttatttaaga taaattaatg 161340
   agagaaaaga tagatttaat tatatacgta tgggatttta ttgaaaaacg tgatttaaaa 161400
   gagtaggtag aaggtggggt tcgtgattgg ttcgaaggta gtgagttatt ttagttgatt 161460
   gtttatagtt agttatagat tagatttttt gttttattgt tttttttttt tttatttgtt 161520
   ttgttttttt tgagttagag ttttgtattg ttgtttgggt tggagtgtaa tggtacgatt 161580
   tcggtttatt ttaattttcg tttttcgggt ttaagcgatt tttttgtttt agtttttcga 161640
   gtagttagga ttataggtat ttattattac gtttggttaa ttttttgtat ttttagtaga 161700
   gatagggttt tattatgttg gttaggttgg ttttaaattt ttgatttcgt gatttgttta 161760
   tttcggtttt ttaaagtggt gggattatag gtatgagtta ttgtatttag ttaattagtt 161820
   ttaaaaaaaa aaaaaaaaaa aaaagaattt agggttcgta tattatttta ataggaaaaa 161880
   aggaaggggt gaaagtatat attatgggtc gggtgcgatg gtttacgttt gtaatttcgg 161940
   tatttcggga ggtcgaggta ggtggattat ttgaggtcgg gaatttaaga ttagtttggt 162000
   taatatggtg aaatttcgtt tttattaaaa atataaaaat tagtcgggta tggtggtgta 162060
   tatttgtaat tttagttatt tgggaagttg aggtaggaga attgtttgaa tttacgaggc 162120
   ggaggttgta gtgagttgag attatattat tgtattttag tttgggtaat agagtgagat 162180
   tgtgttttaa aaaaataaat aaaaaaaaaa aagtatatat tatggtaaaa taaatgagtt 162240
   tttggaaaga taaataggtt tttaggagtt gagatgggag atgtgaagtt ttgtgataat 162300
   gtttttttag gtgtggtgtg gaaattattt atatttttta gggaagagtt agttgttttt 162360
   agggagtaga tttgggtttg tatattattt taatagggga aatggaaggg gtaaaagtat 162420
   atattatggg ttgggtgtaa tgtgtttagt gtaggaggta gacgtggttt atggttgttg 162480
   ttgaaattag tgtagggttc gattgttttg tattgagtgt tgaggatttt ggttttgtat 162540
   tagttatgag tttgtagttt tgggatatat ttgatgttgg tttaagggga aatgaggttg 162600
   gaggggtagg gtggttggat tggatgtggg tttggggtta ttgttgattt ataaagtggt 162660
   tattatacgg gaataataga gttggttata gattataatt agtaatatta tttaaaagta 162720
   aggttatatt tttaataata agttatattt taagttatta ttaatttata aaatatattg 162780
   ttggtataat gatagttttt taagaaggat ataaaaaggt atgttgggtt gggtgtagtg 162840
   atttacgttt ataattttag tattttgaga ggttgaggtc ggcggattat tttaagttag 162900
   gagtttgaga ttagtttggt taatatggtg aaattttgtt tttgttaaaa atataaaaaa 162960
   aaattagttt ggcgtggcgg cgtatgtttg taattttagt tacgggggag gttgtggtag 163020
   aagaattatt taaaatttgg gaggtggagg ttgtagtgag ttgagatcgc gttattatat 163080
   tttagtttgg gtaatagagt tagatttcgt tttaaaaaaa aagaaaaaaa agaaaaaaag 163140
   taaaaaggta tgttgaaggt atttgtttgt ggaaagttgt aatttgacgg gaggtagggc 163200
   gagtattttt tagaaaggtg gaaatgggtt tttttgttat agagatttat aaatatagat 163260
   tgaggtttgt agtttaggag atttaaaaga ggtaaaggtg aataagagtt aaggttttta 163320
   agttagggtt tgattttggg ggtgggggtt atatatgatg gaggatgtta ggagggtttt 163380
   ttggaggtgg tgatatttat taggggggat tttaggtggg gtttgcgaga tagtaggatt 163440
   tggttgaaga gcgtaggttt tttagatagg agtgggaatt taggtttgaa aatgagttgg 163500
   ttagattgta ggggttttcg aatgtaaggg aagaatttta attttaatag aggagtttgg 163560
   gaagttgttt gggaaaagga gtgataaagc gggaggggtt ttttagtaga gttttgtatg 163620
   ggtaggaggg agcgagatgg gaggtgggtg ttggtaaggg atgattttag tggtttaggg 163680
   gagtagttat agggtggaaa ttaattttgt ggggtttggg taggaaggga ttgatttgag 163740
   atatcgggaa gaaatagtta gtaggatttt ggtgagaggg ttgggggtcg gggagggggg 163800
   tgtggagagt gggagtttga gtttttgaaa atagatagtg ttttaagggg gagatttgag 163860
   gtaagatgtg gagaggtagt tgagttggga agttggaacg gaggtgtggt tattttagga 163920
   gattaagtta gggggcgagg ggatttttgg gagttattta gattttagta ttagggaata 163980
   cggaggggag aaggagggga gaagaattag ttggtgcgtg ttaggtattg ttttcgaggt 164040
   tagatggggt ggggttggat attggttatg aggatttttt taagagttgg gttaatagtg 164100
   agacgggcgg atattagatg gttcgggtta agaagtaagt gggcggtggg aagttagaag 164160
   tcgtggtgta gatttatttt gagaggttgg gttgtttaga tagggagaaa gtttatagtt 164220
   tgaggaggta gataggttaa aggaagaggg tttgtttgtt tttgagattg gggtattttg 164280
   aacgtttttt taggttaagt gagaagaaat tagaagagga atatgttatt taaatgtgtt 164340
   gaaatagttt agtagttgcg ttgttttata gaaaatgtgt agaggttttt ttgttttatt 164400
   atgtttgttt ggggacggtt tttattcggg ttatttaggg tttttttagt ttgttaattt 164460
   aagggtggta ggagttgtgg gtgtttttag ttttttttta aggttggttt gtgggtagaa 164520
   atgtgggttg tttcggggcg ttatagttat aaatgtatat tagtttttag aattatattt 164580
   ttgtttttta gttttttttt tcgtattttt atgtagaagc gcggtcgtta gtttataaag 164640
   gatagggagg gatattgttt ttggtatttg atgggaagta tttgttgtat tttattgggg 164700
   tgttgtttag gatggattgg aaaagagttg gtaggaaggt tgagttttgt gtttataatt 164760
   tggtttggtg gtggtcgagg agtttggtag gagtagagtg taggatttgg gaattggggg 164820
   ttggtgtatg tgtgtacgta cgtgtgtgtg tgtgtgcgtg cgtgttgggt gggtagggag 164880
   gaagttgtga aattatattt tttttttttt gttgttgtgt tgttgtgtgt tttagtagta 164940
   cgtgggtgtt attatatttt ttagtaggtg ttaattttta agattgtttt gggttttttt 165000
   tttagttggt tgagttggag gtgggagttt taattgtttt ttgtggtttt tagagtggga 165060
   ttttgttgtg ggataggttg gttaatggtg tttttttttt tgtgattttt ttgttgggtg 165120
   ggttacgagg aaggattgtg ggtgttgttt atagataggt ggatatgtgg taaggatatt 165180
   ttgggatttt ttttttgacg ttttttgaag ggggtatttt tttagttttg agatgagttt 165240
   ttgtggatgt gggaagttta ttattttaag agtagtagtt ttggaaaatt taatatagaa 165300
   tttcgagtag gggcgggaag gggttttgtt tcgtttattg gttgtttggt agagttttgt 165360
   ataaggaagc gtttgtgttg ttgtgggcgg aggaatggat tgagggttat attcgttttt 165420
   tgttgtcgtt gtaattgttt attataaatt tagtggttta aagtaataga ggtttttttt 165480
   tttatagtgt taagggttag aagtcgatta gttttatcgg attaaagtta aggtgttggt 165540
   agaatttatt tttgtttttt ttagttttgg gtgggaggtt ttgttggagt tttttggttt 165600
   gcggttgtat ttttttagtt tttgttttta tttttttata gttttttttt tttttgtagt 165660
   tagatttttt tttgtttttt tttttttttt ttttgagacg gagttattta ggttggagtg 165720
   tagtggtata attttggttt attgtagttt tcgttttttg ggtttaagcg atttttttgt 165780
   tttagttttt cgagtagttg ggattatagg tatgtgttat tatatttggt taatttttgt 165840
   atttttagta gagatagggt tttgttatgt tggttaggtt ggttttgaat ttttgatttt 165900
   aggtgatttg tttgttttag ttttttaaag tgttgggatt gtagttatga gttattatat 165960
   ttggtttgtt ttttttttaa aggacgtttg tgatttgggg tttatttggg taattttttt 166020
   attttaatat ttttagttat atttatagag tttttgttgt tatatgaggt aatatagttt 166080
   gggaagggag agttatttag tttattttag gggtttgtgg tgtattttag ggtttttttg 166140
   attttaagat ataaagtaag aaaataaatt ggtttaaggg gaaaaaagga tacgttgaat 166200
   tttgttgttt taaatgtata tttttttatt gtgttaaaat gtatagaata taaaatttgt 166260
   tattagtaat attgagtata tttatagtgt cgtgtaatta ttagtattgt ttagcgttag 166320
   aattttttta ttattttaaa gggaaatttc gtatttatga aggatttatt ttttattcgt 166380
   tttttttagt ttttggtagt tattagaatg ttttttgttt ttataaattt atttttaata 166440
   agtgtaattt tgtgtgattt taaaataaat aaatatgagt acgatgagtt gtttattgga 166500
   aggatattta tgcggggagg tcggcgtgtg gagtgcgtag gttttcggac gggtaggagt 166560
   tgaaggggcg tggatgtgtc gttttttttt ttttttgttt tttttttggg gttattgttt 166620
   gagtattttt ttttgtaaat ggttttaaat aatgttttag tttttacgtt tgtatcgttt 166680
   tttagtttta ggatttttta ttaaaaaata ttttaagttt tagatttatt tttgggaaaa 166740
   ttttaatggt tttatttttt tttttgagtt agttagattt tatggtttgt ggcgggttgt 166800
   ttttgagttt tgagtatttg tgagttaggg aagtaggata ggtatattta gggaagggga 166860
   agagtcgtcg ttagttatag taattgatat ttttggaatc gtttaagaga tatttagcgt 166920
   gtgtttaaaa tatttatttt tttttttgtt tgttttttga gacgaagttt cgttttgtcg 166980
   tttaggttgg agtgtagtgg cgtgatttta gtttatcgta attttttttt ttcgggttta 167040
   agcgattttt ttgttttaat tttttgagtg gttgggatta taggtatata ttattacgtt 167100
   tggcgaattt ttgtattttt agtagtgatt gggttttatt atgttggtta ggttggtttt 167160
   gaatttttga ttttaggtaa tgtgttcgtt ttggtttttt aaagtgttgg gatgataggt 167220
   gtgagttaat atatttggtt atatttattt tttaggagaa gttttgtatt ttttgaaagt 167280
   gaaatgtttt taggcgggtg atattgatgg tgggatttgt ggttttatta gtgttttatg 167340
   agagatgtcg ttgtgtgttt tattaagttg tattttattg ttttttaaat gtggtttgta 167400
   gatatggtgt cgagaatgat ttgggaggtt taaattataa tatggtttaa attaatagtt 167460
   ttttgatgat ttggtattat tttaggatta aattgagatt attcgaggat gggcgtggtg 167520
   gtttacgcgt gtaattttag tattttagga ggttgaggcg ggcggattaa ttgaggtttg 167580
   tagtttgaga ttagtttggt taatatggtg aaattttatt tttattaaaa atataaaaag 167640
   tagtcgtcgg atatggtggt gtacgtttgt agttttagtt atttgggagg ttgaggcggg 167700
   agaattgttt gaatttagga gacggaggtt gtagtgagtt aagattacgt tattgcgttt 167760
   tagtttgggt gatagagtaa gattttgttt ttcggtaatt aaaaaaaaaa aaaaaaattg 167820
   agatttttcg agttgtattg tttagtatga tagttattgg ttgtacgggg ttatttgaat 167880
   atttgaaatg tggtgatttt aagtggagag gtgtcgtgtg tgtgaagtgt attttgggtt 167940
   ttagagtttt agtaaggggg aaagaaaaga atattaaata tttgttattt ttatattgat 168000
   tatatgttga attgataata ttttgggttt tgttaagtga atgatattaa aattaatatt 168060
   atttgttttt atatttttta atgatttttt tttttttttt tttttttttt ttttttaaat 168120
   atggggtttt atattgttgt ttaggttgga gtgtagtggt acgatttcgg tttattgtaa 168180
   tttttgtttt ttaggtttga gcgatttttt tgttttagtt ttttaagtag ttgggattat 168240
   aggtggttgt tattatgttt ggttaatttt tgtattttta gtagagatag ggttttgtta 168300
   tgttggttag gttggttttg aatttttgat tttaggtgat ttgtttattt tagtttttta 168360
   aagtgttggt atgataggcg tgagttattg tgtttggttt gtttttatat tttttattgt 168420
   aggtgttgga aaatgtaagg ttgcgtttgt ggtttatgtg tcgtttttgt tggagggttg 168480
   gatcgagggt tagtcgagaa ggttttgggg ttgttttatt tgaatgttga gtgtgtgttg 168540
   agcggtagag aattttgaga agtgttgaag gatagaggtt ttgtgtgggg gatgtagttt 168600
   taggtcggag atgttttttt tgtgttttgg agatttttat ttggtgatag taagaggaat 168660
   tattgtaaga ggggcggtta ttttagtaaa gtatttagga tgttggtttt ttagtttagg 168720
   aatttagagg tagaggaggg gaggaggttg ggtttggagg agagtagggt tttgggtttt 168780
   gggaaaagtt tggggggtta gaaagttggg ggatagattg aggttagagg taaagagaag 168840
   aaggttcgga gaggagggtt aaggaagaag ggttttgttt acggggaggg agaggtttac 168900
   ggggtagggg cgtggtggga gttgtatttt cgaggttttt tgttgatttt gatatttggt 168960
   tagaaaggag ttttttttgg tattttttgt tagcggtttt tggagttgtt ttttaggggt 169020
   tagtgatgag ggtgttttgg gttgggttgt agatagtgga aaagataggt tttttttatt 169080
   tttagtgttg gggaaattag ttttttggag gaagggggag ttgtattttt tgagtggtcg 169140
   tattttttgg tttagacgtg tgtgttttgt tgtaattttg gcgattggtg ttggtggtgt 169200
   ttattttatg gatggggaag gttaaatttt atagatattg agtgataggt ttagggtttt 169260
   ggattttagt gtttgattta gaatttaggt ttttgatttg ttattagttt tttttttatt 169320
   tagatgggaa tagggatttc gggtatgtag ttttttttta gatatttttt cggttttggt 169380
   ttggcgttcg gagattagta gtagtagttt tttacggtcg tttattgtga gggtggggag 169440
   ggtcgtggga ttaggaggtt ttagggggaa agttggttat gaggagtggg aagcgacggg 169500
   ttagatgtta gtgattgttg gttggggagt cgttggacgt ggttggtttg tttggttagt 169560
   gtggcgggtg tttcggggtt ttagttattt ttagttacgt gtgggggtgc gggatgggaa 169620
   aggtaagagt gtgtggagtt tagttagtat ttaatatgag gtttcgtggg ggttgtttga 169680
   gggcgtagtt tttagaattt agtttttggt ttttagaagg tgttattaaa ggtttttttt 169740
   tatttgagtg aatttttttt agtttttagt aagttttcgg aacgagttta tttttaggcg 169800
   ttttttttta gtgtgggagt ggttacgttt ttgttgggag tgatttattt gggtttagag 169860
   gtcgtggtat tgagatgggt ttggtagatt ttagcgttta ggtttagttt ttatagtgtt 169920
   agtttttttt tttggggatt tttttgtttg tgtttttttg ggttttagta tattttaggt 169980
   ttgtagggag ggggagagga agagattgat ttattggtta ggttttttag gggttggaga 170040
   ggttggagag gtaggagttg gattagattt gaatttagag gttttcggag gaagagttta 170100
   gggtgagttg cgtttttatt ttttgttttt ttttttttgt tttttttttt ttttatggtt 170160
   ttagttagta agtatttggg gtagagggga taaatttagg tggttgtgtt ttagttttgg 170220
   ttgtaggttt gaatggtttt ttggggtggt tggttatgtt ttttgagagt ttagttgtgg 170280
   cgatgtttga gtaggtaggt gggggagtat ttaggaagta ggggtgttag gtagagtata 170340
   aggagagagg gtgtttaggt tagttttagg atttggttga gaggaggggg ttttttacgg 170400
   gtatcgtttt tggtaagtat agggataagg gtaaggacgg tatggttaga ggtttttggg 170460
   agtttttttt tttttttttt ttttagtagt ttttttttat tttttttagg gattttgtta 170520
   ttttttttta gcgtgtggta gttttttggc gtttttttcg tgtttttagg ttgtttttgc 170580
   gtcgggtttg tcgttgggcg tttttatttt tgtttgtttt ttttttttgt ttttttcgtt 170640
   ttgttttttt ggagtaattt tttatcgagt tttttttttt aggtagtcga ggtttttttt 170700
   tatttttgtt tcgcgttttg ggaggttttt tgtttcgcga ttataaagtt tttttgattt 170760
   tttgttcggt tttgagttat gtgattcggt gggcgggtcg cggttttcgg cgcgtttagc 170820
   gtagttcgac gtttcgttgt tggggtgagt tttgtttttt tgtttttttt agttttgtat 170880
   tattggttcg ggggttttta ggtggcgcgg tcgcgaggcg gattttgatg gttatggtgg 170940
   cggtgtcggg agttacgttg tttttgggtt tcggttcgag gtcggtagga tcgagcgggg 171000
   tttttaggag aggggtggcg gggagttcga tttttacgcg gggattagat tttcggtttt 171060
   aaaatagaag aatagggttt tgtgtggtta tagttatttt tttgtaaata tttggttaat 171120
   taagttgagt ggttaagttt ttttgttgtt cggagttttt tggaatatgt ttttttttcg 171180
   taaggggttt ttttggtttt taggagggtt aggaagaaat tcgaattggt tatcgttttt 171240
   tttaaaatta tttcgtttaa gttattattt ttttgggttt tcgaagatcg gttggttgga 171300
   ggttggagat agttttaatg ttcgaaatgt cgtaatcgaa gtttttcgcg gcgtcggtat 171360
   tgggatttag ggagttgttg ttatagcgta gttttggatt tttggatgtg ttggatatgt 171420
   gtagggcgtt tttgggagga gcggggaggg agggtgttgt tggcggggtt ggtttgcgtg 171480
   tgttttgttt ttttataatg gtatgttgcg tgtcggttat gtagaggtat gttagtgagt 171540
   aggggttgag ggattttttt aacggatttg tttttagagg gtttttttat gttgggagaa 171600
   ttttagagat taaattatgt agttaacggg gtggttttcg gttttaaagt agggaggggc 171660
   gaggagtttt gtaggtaatg ttatttgttt ttgaaacgtc gttttagtga ttttggggat 171720
   tgatttagtt tttagtttgt tgtattttat ttttggtttt tttttttttg tttttttatg 171780
   tgaaatttgt tgtgttttgg ttagaggttt ggggaatagt tttttgttta tttaagataa 171840
   gtttgtaatt ttttttttgg agaaaaatat gttattcgga agtaggttga gtagtggttt 171900
   tttggtaggt ttgttcgggg ttgtggagat agtatttgtt ggattaggat ggagttggaa 171960
   tttggtttgg attttatatg agaaaattcg ttggaagagg aggaagtaga aaaaggtttt 172020
   attttttaat aatgttgtgg gttttttttt tttatttatt gttattttgt tttgaaattt 172080
   aatggggtta cgtgtgtttt tatgaatgtt attttttttg gttaattttt tcggtagttt 172140
   taggagtttt tttttaggaa aaggaagaag gtgtttgttt agtatttagt atacgagttt 172200
   ttgttggaaa ggtgggattt ttgttttttt ttgggttttt tttaggttga gatgtggtta 172260
   aggaaggttt gtggtgtggt tcgttttttt taatgttcgt ttttggagat ttgagttgtt 172320
   gttgattttt agtttgtttt ttttgttttt tggttatttt tgtgtcgggg tggttgagat 172380
   attattgtat tggttatttt gtgtttttat tagttttcgt ttttgtaggt tgttattgtg 172440
   atggtgatgg ggttaattta gttgcggtgg ggaggattta ttgtgtgcgt ttggggggtg 172500
   gcgtggggta ttttgggatg agaagatgcg atttttgttt tgagagtttt gttttaatgg 172560
   aaggcggggg tatagttgta ggtagaagtt attaagtatt gtggttagat tagtatttta 172620
   tgttaaaaat ggttttgttg gtcgggcgta gtggtttatg tttgtaattt taatatttta 172680
   ggaggttaag gtaggtagat tatttgaggt taagagttcg agattagttt ggttaatatg 172740
   gtgaaatttt gtttttatta aaaatattgg ttaattcggg tatggtggcg cgtgtttgta 172800
   atggtagtta ttcgggaggt tgagatagga gaattatttg aatttgggag gtggaggttg 172860
   tagtgagttg agattgtgtt attgtatttt agtttgggta ataagagtga aattttgttt 172920
   taaaaaaaaa aaaaaaaagt tttatttggg tttgggattt gtttttcgaa tattgggttt 172980
   attgtgtttt ataaggattt ttatttcgga agttttagat ttatttgggt aattagagtt 173040
   tttgggttga ttatttttta gttttggtta gttttttggg ttatcgtatt tttttaattt 173100
   tttgttgtgt tacggttggt ttaagttgtt tttattagat aatgcgaggt atttttatag 173160
   gaggggataa tttcgttttt aggagttttt tttttttgtt tttaggtttg gtgtagtgtg 173220
   tggttttttt gtcggtgtta aaggttttaa ggttttttta agaagtttgt tattattatt 173280
   agtagttttc gttatttggt ttttttgtat atgtaagttt tttttatata tttcggatgt 173340
   ttagggtttt cgtttttttt ttttggtcgg gagtgtggga agagggtttt attttaatgt 173400
   ttgagagtag ggttaatggt tagggtaagt tgtgggggtt ttttttcgtc ggtttttttt 173460
   tgtagttttt attttaatat agagtatcga tattatttgg gttttgggaa gatttggagg 173520
   ttgattattt tggaggtttt tagcgtttta tagttatttt ttggggaaga ggttttttga 173580
   gggatttgtg gtttttggtt gggattttag ttttagagtt ttgtttttcg gcgtcgagtt 173640
   ttgatttgtt tattgagtgt tagttcggtt ttcggatagg tagggatttg ttttcggagt 173700
   ttagtagagt tttgggaagt ttttttagga gggttttcga gggtggcggt tatgtttagt 173760
   tcgttgtacg tgtgagtttg tttgatgagg aattggttgg gaaggcgcgg gtttaattat 173820
   aggtttatga gggttttttt cgtttttatt taggaagtag cggagatttt ggtatattta 173880
   gtatttgggt ttgtgtttgt ttagttgttg gtggaattat gggagttttg tttcggggtt 173940
   tgtagagtag tagtgggaat tttttttatt ttagtttttt ttagagttgg ttgtttaaat 174000
   tcgtcgtcgt ttagtttttt tttttttttt tttattaatt ttgggaatcg tatattgttt 174060
   attttatcgg gtttcggagc gggtgtagga agagagagtt taatatttag aggtttgggt 174120
   tttttgtagg cgtttgagtt gttgtggttt gagttgggtt ttttgttgga attattatag 174180
   tagatttaat gatttgtttg taggagttgt tttattttag tcgatttcgg ggttgcggga 174240
   aggggttggg ttttggtttt gttgggggtg gggggttagt gtaaagggtt tgaaaagtgg 174300
   tagaggtgat ggtttatttg tttttttttt ggtcggtcgg tttaaacgcg attggttttt 174360
   tttttttttt tttttttttt tttttttttt tataaataat attaaatgcg atttgttaat 174420
   gtatttattt gggtttagta gttggattat gggtagaagt attttagatt tgttattttt 174480
   tttaagttaa gggtggggaa tattttttgt gagattttgg tttttgagcg tttaagattg 174540
   tttttggaat tttaattttg ggtcggtttt aggggattga cgttgatgga gttgttgata 174600
   ttgattttcg gtttttgtat ggtttttagg aagggggttt ttcgtttttt gtttcgtcga 174660
   gtttggttgg tagtattttt tttttaggtt tgtgttttcg gtagggtttg ttgttttcgt 174720
   tttgttttgt gggtgttagg ttttaagtat taggtgattc ggttattgaa tggttattga 174780
   atagttattt ttgttggtgt ttttagtgga atgtgtatgg acgttttttt ttttttagat 174840
   ttttgttttt tattttttag tttttgtttt aggggttatt ataggattag gaggtacgta 174900
   gacgtgggtg gtatatttat tgggtagatt tagttttgaa tttgtacgtt ttttttaagg 174960
   gggtttttag cgggcggggt tgatggtgtc gttttttttt ttgttattag aacggatatg 175020
   aggattgtgt tttgtcgatt tacgttttga gtttttagac gttataggtg tttgtttaac 175080
   gagtgtttga atgaatggtt agtattgtgt gatcgtagat ttttaatttt tatttaaata 175140
   gtttgtggtt tttgtgaggt tatttcgaga tagttttata agaattttta ttttttttgt 175200
   acgtttcgta taaggttttt ttggggggtt ttttagaaaa agggttaaag agtagtattt 175260
   tttcggggat ttgaatacga tttttttcgg ggtaggggat tgttttggaa gattttcggt 175320
   atcgtttttt atgcgttacg tgattaggag ggttttgggg taggatggtg ttttttgtgt 175380
   tttgtgtgtt ggggtcggag gaaatattgt tcgtggtcgg tgttatttgt tagttttgaa 175440
   gattaatgat gggttggata gtggttttat tgagtgggag ggagaatcgt tgttgtttgt 175500
   agttgggggg tcgggtgggt gggagtcgag gttagggaga atagcgtaga tttggtacgg 175560
   agaggtttgg aggaggagta gggggtttta gttatttagg gtgtagggac gttatacgag 175620
   gagtaggtgt ggtgtgcgtt tttttgcggg ttttttattt tttggtttcg ttatttgtat 175680
   taggttttgt ggtgagaggg tatagagtgg aggaaggtgt cgtggttagg gtaggttata 175740
   cgttgtgtta atggattgtt gttatttgtc gtttttgtgg ttttgttgtt aggatttttt 175800
   agtattaggt ttttattatt aggtatgggt tttataggta ttattggttt ttcgaagtta 175860
   gggatagatt ttatagataa tattgagttt ttgtagttag ggatgggttt ttacggttag 175920
   tattagattt tagggattag tatcgggtat ttatagttag ggataggttt ttatggttag 175980
   tattaggttt tagggattag tatcgggtat ttatagttag ggacgggttt ttatggttag 176040
   tattagattt tagggattag tatcgggtat ttatagttag ggacgggttt ttatggttag 176100
   tattaggttt tagggattag cgttgggttt ttatagttaa ggataagttt ttacggttaa 176160
   tattagattt tagtgattag tattagatgt ttatagttag agatgggttt ttatggttag 176220
   tattaggttt tagggattag cgttgggatt ttggatgatt gaaggagggt acggttgatt 176280
   aaagttgtag gagttagttt ggttattttg aggtattaag ttattattgc ggttttaggg 176340
   gatttagatt tttttatttg tttttttgaa ttttggaatt agatatattt gggtttagtt 176400
   tttgttttcg gggttgtgtg tttgggatgg ttgttttatt tttttgaagt ttagtgagat 176460
   ggggacggtt gtatagggtt cggtatagat ggtattcgat ggtatagggt tatttgtttg 176520
   tatttatata gtttcggttt tcgttttggt tgcgtatcga gagttttatg tttagatttt 176580
   tgttgagttt ttaagttttt ggttatgttt taggtttttc gtgagaagta ttattgtatg 176640
   gattatgttt cgggatttta ttttgggttg ggaagtttgt tttatttttt taagtttttt 176700
   ttagaggcgt tttatgtttt attattttta ttgtttgagg ggttagatag gggaatttag 176760
   tgtgggaggt ttagaggtgt cggagtagac gttaagcgag tttcgtgttt atgcgttggg 176820
   ttttggtaga tattttgaat gagttttttt tttaggaagg agattggggt tttagtttaa 176880
   gttgtggttt taaattttgt gattaagagt ggagaaaagg taagattttt ttttttagtt 176940
   ttttagaata gtcgatagtg gtggaaatat ggggtttata ttgaattttt ttgtaatttg 177000
   ttgaagagaa tttagtttat atttttaggg gtggaggttt ggtcgatttg gtagtgtgtt 177060
   cgtttttttg gttatagttt attttggatc gggtattttg gtgttttttt ttagtgacgt 177120
   agtgaagtta ttatgacgtg tttagttggt gagaaagtgt tttttgtaat gttttatttt 177180
   ttttagaggg tgaataagtt ttgtttatta taaataatta tttatagaat atatgaggtt 177240
   taatttaatt ttgggttacg gtagcgtttt ttgggtaaat ttacgcgtta ttttagttag 177300
   agtttgtgtt tttataggtt ttttggattt attttgaagg ggggttggat tttttgtttg 177360
   tttcgatgag agggttgtag gatagttttt agcgagtttt tttgagggtt gtaatttttt 177420
   tttttggtat ttttttgttc gttaggaatg ttttaagttt ttagtagaag tttatttatt 177480
   atagtcgttt tttattttgt ttattatttt ttttgttttt atttatttag tagatttcgt 177540
   gtatagttta gtgtaggatt tagggtggga aggtgtttcg ggtgggttgg agtaaggttt 177600
   ttagttaggg cggggtttta aggtgggttt atagttagga ttgtgatttg ggaggttttt 177660
   ttttgttttg ttttagtttc ggtatcgtcg ttgtttttgt gtttagtgga gatttattgg 177720
   ttttaggttt taaaatttta gcgaggtttg tagataggga ggagttttat tcgagggtag 177780
   agtgattgtc gggtttattt gtttgttacg aattacgtgg ttttagattt gttatttttt 177840
   tttttttaga gttatggttt tttgtttgaa aaatggataa tgatggttat tatgttaggg 177900
   attattttga ggataaacgg ggatgaaatt gaagaggttt agtacggaat ttggatatag 177960
   aagtgttaat tgtgtgttat tagttgcggt ggttgttaag gtttaattat atggtagtta 178020
   cgggttgagt ggcgggcggg aagtggggag taggagtatg taagatgggt atttttgaag 178080
   tttttgattt agtgagataa gtatatattt agttttaaga tggagtgttt taggaaggtg 178140
   gagattgtaa aggttttgtg gcgtaggagt gtgagttgta tttttgggtt ttttagaatt 178200
   ttattagagt ttagggagga ggttagttta gatatagggg taacgggaga gatgggagtg 178260
   agaaaatggt ttggcgtttg gaagtggagt ttggaaaggt gggcgtgggg atagaggagg 178320
   gttttggggg ttatgggttt atgcgtttag gaggtggagg tagtgaacgg ggttacggtc 178380
   ggttttatac gtggtattat tagttttgtt gagtttgtat ttgtttttta ggttttttga 178440
   gtgttattat tatttggtat tttttggttt gatttttgtt ttttgttcgt ttgggaagag 178500
   tgagattatt tgtttaaagt tttttagttg tttttaatta ttttggttgt tttttggttt 178560
   tgtttagggt tagtcgttga tttgaattgt ttggtgttat ttttttagat ttttggggtt 178620
   ttgttttggt attaatagtt atttttattt ttattattta ttcgtatatt tatttagtag 178680
   tgttttttgg gtatttattt agtaatatta aggcgttgag atgcggttgt ggttaagatt 178740
   cgggttcggt ttgtgaggat ttcgtagttg ttattttttt agagtatagt tttagttagg 178800
   tatattgtgg ggtttagaaa gagaaattaa tatattgtgt tcgtttattt tgagttgtta 178860
   taaggaatag tcgaggttgg ggggttattt gatttacggt tttgtaggtt gtataagtgg 178920
   tgttagtatt tgtttggttt ttggggaggt tttaggaagt ttttttttat ggtagaaggt 178980
   gaagggggag ttggtacgtt atatggcgag agagagaatt tttagatttt tttttttttt 179040
   gaaatggagt ttcgttgtgt tagttaggtt ggagtgtagt ggtataattt tcggtttatt 179100
   gtaattttag ttttttgagt agttgggatt ataggcgtat gttatcgcgt tcggttaatt 179160
   tttttgtatt tttattagag atggggtttt attatgttgg tcgggttggt tttaaatttt 179220
   taattttgtg atttatttat tttggttttt taaagtgttg ggattatagg tgtgagttat 179280
   tttatttagt ttttttagat tttatagtaa ttagattttt cggattttgt gatcgtgggg 179340
   agggtattaa attatttatg agggatttat ttttatgatt taagttttat tttttttagg 179400
   ttttattttt aatattaggg attatatttt agtatgagat ttggagggga taacgtttaa 179460
   attatattat acgtgatttt tttgtgcgtg cgttagtttt tatatttggt taagtagtag 179520
   aagttttttt tttgtttttt gttgagagag cggtggggga gaaatgattt agggatggga 179580
   ggttggtttt tcgaggggtt ttggtgtttg tggagtggga gtgcgggaag gtttttacgt 179640
   tgtgtgtgtc gtgttgggtt ggttttttat agtttatcgt ggtgtttttc gaggattgtt 179700
   ggggaggaaa gagttaatta atttggggat ttagttgttg aagttattat taaatttgag 179760
   tagtttaggg gttttttttt ttttgggttt tatgttattt agtatagtgg ggtttaattg 179820
   aatttttttt tattttttaa ggtttttggt tggcgtggtt ggggtttggg atacggggag 179880
   cgagagggtt tgggtttttt ttagatttgg agtgggttta ttagaaatgg tttaggaaat 179940
   cgcgggggtt atttttagta gttgggggtt tttgtggtta tttttttttg gttttgttga 180000
   gtagttttgg tttttggagt ttttagtaag ggatgtgggg gtttgttagg ttagggtttt 180060
   tattgttgtc gttatgtata gttgtatttt gtattcgggg agtggggtgt cgagtggggt 180120
   gtttgggagg taggtttttt tttgtttttt ttgtttagag attttgggga tgagttgagt 180180
   gagatttttt cggtttttga ttggttgtgt ttttgttgtt attttttatt ttttttaagt 180240
   ggaggtttat gatttttttt tttatttaaa agggttattt agtgatttag tagattttag 180300
   tttgtgttat ttagggtttt tttaaaaatt tgtggggagg ttgggtttgg tggtttatat 180360
   ttttaatttt agtattttgg gaggttgagg cgggtggatt atttgaagtt aggagttcga 180420
   gattagtttg gttaatatgg agaaatttta tttttagtaa aaatataaaa aattggttgg 180480
   gtgtggtggt acgtgtttgt aattttagtt attcgggagg ttgaggtagg agaattattt 180540
   aaatttagga ggcggaggtt gtagtgagtc gagattatat tattgtattt tagtttggac 180600
   gatagagtga gattttgttt taaaaaaata aaaataaata aataaaaata aaaatttatg 180660
   gggggatttt tgtgtttatt ggttggtgtg aggagagggt gaggagttgt agagaaggta 180720
   ttaggttgag gatcgttttt atttttgggt tatagaattg aaggagggga tatatgaaag 180780
   atgtttttag attcgggttg gaggttgggg atattcgagg gtaaggacga cgttgttttt 180840
   gtttttgttt gtagaggagt gtatagggtt tattagtttt ttgttgttgt ttaagtgata 180900
   gttttatgga gatagaattt atatattgtg acgttgattt ttttaaaatt tatagtttaa 180960
   tggttttgtt gtatttataa agtgattgta ttagggttat ttagagaaat agaattaata 181020
   gaaggtgtgt acgtgtgttt agataaagag atttattatt atgaggagtt ggtttatttt 181080
   attatagagg ttggtaagtg ttaagattgg tagagtaaat tagtaggttg gagatacggg 181140
   agagtttatg ttgcggtttt ggtgtttatt tgaaggttta agatttagga gagttaatgg 181200
   cgtcgtttta gtttcgagat ttaggaaaag cgtatgtgtt agtttgagtt tgaagtagga 181260
   aaaatttgat gttttaggtt gaaggcggtt aagcgggaag aatttttttt tatttaggtt 181320
   ttgtgttttt ttatttagat ttttaggttt ttgttataaa attataaaga tttgattaga 181380
   ataaaatttg agaaaaaatt tttttttttt atcggttttg gatttattgg ttgggatttt 181440
   ggaaattaga ttgatagaga ttaataggaa ataagtataa taatttaagt aagttttatt 181500
   tgatatcgag tttttataag gaaatgaaga tttagagaaa tcgttagttt gagcggtttt 181560
   tgttttgttt tgagataaag ttttattttg tcgtacgttt aggttggaga gtagtggttc 181620
   gattacggtt tattgtagtt ttgatttttt gggtttaagt tggggtttag ttgtcggttt 181680
   cgggagttgt tttgaattta tttttaagtt ttagtttaga tttttaagta gttgggatta 181740
   taggtatgtg ttatttcgtt tggttaattt tttatttttt gtagagatag ggttttatta 181800
   tgttgttcgg gttggtttta aatttttggg ttcgagtaat ttttttgttt tggtttttta 181860
   aagtgttggg gttataggtg tgagttattg tgttcggttt aggtttgaat agtttttatt 181920
   tgttttgttt gttttttaaa tttgtttgtt gtttgttttg agtagttttt ttgtttattt 181980
   tttttgtttg ttttttgttt aggttggagt gtagtggtat gatttttgtt tattgtaatt 182040
   tttgtttttt gggtttaagt tatttttatg ttttagtttt ttaagtagtt gcgattatag 182100
   gcgtgtgtta ttatattcgg ttgatttttg tatttttagt agggatgggg ttttattatg 182160
   ttggttaggt tgttttttaa tttttgattt taagtgattt ggttattttg gttttttaaa 182220
   gtgttgggat tgtaggtgtg agttgttgtg tttagtttag gtttgagcgt ttttatgttg 182280
   ggtttgatga agagtggaaa gtcgtgggaa attgtgatag agtaataaaa gatgagttga 182340
   atagtgagtt cgtgggggtt tcggtaagat ttgtgtattg gggttttttt tagatttttt 182400
   gtttttggag atgaggttgt tttttttcgt tgggtgtagg gagggtattt tttacgtgag 182460
   gggtttatga tttattttag agaaggggta ggttagtgag ttttttttgg atttgttatt 182520
   ttttaaattt ttttagttga aaatatttat tatgttaagg tattgtattt tggagtgtgc 182580
   gttttaagtt ttgttgtttt taattgatcg ggtgaggttt atttttatta gagtatatag 182640
   tttgttttat ttaggttttt gattgtattt tttttttgtt tgtttcgaga atatttatta 182700
   gtagtgtttg tggttgtagc gtttatttta agataaattt gtcgggaagt attttgtttt 182760
   tattattatt tttatattat tttagtatat cgattttgga aataaaagat attattttgt 182820
   ttatagtatt ttatttttag tagtggtatt ttttttaaaa aaaaatggta atttttagtt 182880
   gggtgtggtg gtttatgttt gtaattttag tatttgggaa agttgaggta ggttgattat 182940
   ttgaggttag gagttggaga ttagtttggt taatatggtg aaattttatt tttattaaaa 183000
   atataaaaat tagttaggcg tggtggtagg tatttgtaat tttagttatt tgggaggtcg 183060
   aggtcggaga attgtttgaa ttcgggaggt agaggttgta gtgagtcgag atcgcgttat 183120
   tgtattttag tttggacgat agagtaagat tttattttaa aaaataaata aataaataaa 183180
   atttaaaaaa aaatagtaat tttcgatcgt tgaaaatgtt aaattttaga gaatatagta 183240
   tttttataag tgatgttaat attgtttttg attagttgtt ggttgaagat ttatttgatg 183300
   aatttgattt ttttagaata gacgatttag atgattttgt tgttttgttt agaaataatt 183360
   gtaggaataa tttaaaaaat tttttttttt tttaattttt ttgttttttg agacggattt 183420
   tttttgttgt ttaggttgga gtgtagtggt acgatttcgg tttattgtaa tttttgtttt 183480
   ttgggtttaa gtgatttttt agttttagtt ttttgagttt agttgggatt ataaggtgtt 183540
   tgttattata cgtggttaat tttttgtatt tttagtagag acggtgtttt attaggttga 183600
   ttaggttagt ttcgaatttt tgattttaaa taattggttt atgtcggttt tttaaagcgt 183660
   tgggattata ggcgtgagtt attacgtttg gttagttttt atatttaatt tttatataga 183720
   gtatttagtt agatttgttt tagttttaaa gagcgtgttt atgtaaaatt aagtgagtgt 183780
   tggtagtgag ttgaattttt taaaaggtaa aagggttaaa tattaaattt atttaaaaat 183840
   atttttatag atatatttag aataatgttt gatcggttat ttaggtgttt tatagtttag 183900
   tttagttgat atagaaattt tttttttttt ttttgagacg gagtttcgtt ttgtcgttta 183960
   ggttggagtg tagtggcgcg attttggttt attgtaagtt tcgtttttta ggtttacgtt 184020
   atttttttgt tttagttttt cgagtagttg ggattatagg tgtttattat tatattcggt 184080
   taattttttt ttttgtattt ttagtagaga tggggtttta tcgtggtttt aattttttga 184140
   tttcgtgatt tattcgtttt agttttttaa agtgttggga ttataggtat gagttattgc 184200
   gttcggttga tataggaaat taattattat agttatatag ttatcgttat taattttagt 184260
   atattttatt atttttaaaa gagattttgt ttttattagt taatttttat ttttaatttc 184320
   gggtttttag tttttagtta tcgtttattt gttttcgttt ttatgaattt atttgtttta 184380
   gatagtttgt ataagtggaa tcgtatagta tgtggttttg gtgtttggtt ttttttattt 184440
   ggtagaatat ttttaaggtt tatttatatt gtagtaggtg ttggtgtttt attttttttt 184500
   atggtcgaat aatattttat cgtggataga ttatattgtg tattttttta tttgttgatg 184560
   gatatttggg ttgtttttgt tttttggtat aattttgttg tgaagaattt tgtagtgagt 184620
   gttggtttat aggtttttgt gtgaatatgt ttttattttt tttgggtaat tgtaagatta 184680
   tgtggtttag ttaattagtt tttaagttat gtgtttgttg agggatttag gtttggtggg 184740
   ggtgtattgt tgtgtttggt tagttaggtg ttgcgagttc gtgtcgtagt tatttttttg 184800
   tgttgtgttt tgaggttgtt tcgtcggtat cgggtattgt gagttttagg aggttaaagt 184860
   tttttttgtt ttattttgag tttaggtttc gttttatgaa ttaggatgaa tggatattga 184920
   attaacgtag ggatgggtgg ggttggggtg gggacgtaga gtgatttcgg gggtttgtag 184980
   ttgggttttg gttgttgatt tttagttttt tttgtttgtt ggagtttaga ataagttaga 185040
   aaggggaaat gtaaagttcg agtgtttttt ttgtggtaga ttggtaggtt tgggtgggtg 185100
   agttatttgt agttagttgt agcgtgagag aggaagtggg aggggaggga gagggtgagt 185160
   ttggtgtaat ttatttttgg aaggaggttg ggttgaggtt gggggaagtg atttttattt 185220
   ttttgagagg attttttttt ggggttaatt ttaaattaag ggagataaga tttttttggg 185280
   ggaggttgga agtgttgggg tttttaggtg ggtaggtttt ttagggttaa cgttttgggg 185340
   aagttataag ttattatgtt tttggattgt tatatatata tatatatata tatatatata 185400
   tatatatata tatatatata tacgagtaaa ttgtaatata aatgtgtgtt tatgaaagtg 185460
   tttattttaa attattatat agtttgtaaa gcgaaggttt tggttcgtat ttgggtattt 185520
   attgtttagt tagtcggtag gtagtttgta ttgtagtttg ttattatttt ttagagatgt 185580
   agtcgaggag tttagttggt ttggagggtt tagtgggcgt agttttagga aagtggttag 185640
   gttcgggtta ggtttttttt tttttttagt tttgggagaa attattatat gaatgtagtt 185700
   ttttttcgga gttcgggttg gaagggtttg ggaggggata ggtttttatt ttaggagttt 185760
   ttaggtttcg gttgttgttt gtgttagttg cgtgagcgtg agcgagttag gggttattat 185820
   tgggtggaag gaagaagcgg gtattttttt taggaggtta tcgttggagt tttatagtag 185880
   gttaatttta ggttttgggt tttcgggagg aggagaaaga agagaagtag gaggtgtgga 185940
   gggtaggtta ggattagaag gtatataggg tcggggtagt gtgggagatt ttgggggttt 186000
   ttttatttaa ttagttaata ttatagcgtt tataagaata cgttattatt gtagttttat 186060
   tattttgttt tgattttaga aggtataatt gatgatttta gggaacggag aatttgagga 186120
   aggtttcgta ggagagggtt agatgcgatc gagtaaatgt gagtcgtttg taaattgtag 186180
   agtgtacgta tagtatcggt tagttttggg gttagtgttt ttttggtgtt tagttcggtt 186240
   tttgtatttt ttagtgttcg gttgggtttt gtgggggtta gggttgggtt tggtatatta 186300
   gaggattttt attaagtttt tggtagtgtt ttagttatta aatttagttt gttagttatt 186360
   aaattcgttt tgtcggtttt cgaattattt tttcgttggg gtcggttatg ttacgttagg 186420
   ttttttatgt aggacggtag atggatagac gttgggatta agttattttt gggtcgcgag 186480
   acgtttgatt ttttgttggt gttttagaag atgttagttt ttcgtggtat gttttttacg 186540
   cggtgtttgg ggattagtgg tttttaaggg gtttttttgt tttagcgtgg ggagtggttt 186600
   ggttaaggtt ttagtgtttg gttggagtta ggtataggtt taggtgtatt ggttggaggt 186660
   tttttgttta tgggttttgt ttgttttgtg tttttggttt ttattgtgtt ttttgtgttt 186720
   ttgggagaga ttttaggggt tttgagttta gtattggggt tggggagtgg gcggggaaga 186780
   gggtggggat tagggttttt ggttgtagaa tagttaggtt ttaagttttt attttatttt 186840
   ttttttatcg ggcgttttat tttatattga ttattttttg aagaaaattt tttgaatcgg 186900
   ttttttattt cggttgtttt aggaagttta tttatttttt cgttttaatg ttttgtggtt 186960
   ttagaaggat ggtggaagtc ggaaattatg agttttattt ataggcggtt atatatcgaa 187020
   gtcgcggaga cgtcgtggga aaggattttg gttagcgcgg tcggtataga gggagcggtg 187080
   ttatcggggt agacgggaat cgtggttgtt tttatgttgg tttgggagag acgaggggtc 187140
   ggacgaaagg atagtagatt ggttatttat ttatttattt atttatttat ttatagcgtt 187200
   ttattgggtg tttcggacgt gtaaggtttt gtgggtagtg aatgcgatag attttagata 187260
   gtaatcggag ggttacgtgt gtaagaattg agggatttag gttgggcgtg gtgttttaag 187320
   tttgtaattt tagtattttg ggaggtagag gcgggcggat tacgaggtta ggagatcgag 187380
   attattttgg ttaatacggt gaaatttcgt ttttattaaa aatataaaaa attagtcggg 187440
   cgtggtggcg ggcgtttgta attttagtta tttaggaggt tgaggtagga gaatggcgtg 187500
   aattcgggag gtagagtttg tagtgagttt agatagtgtt attgtatttt agtttgggcg 187560
   atagagtgag attttatttt aaaaaaaata aaataaaata aaataaaata aaaattgagg 187620
   aatttagtgt agatgagtta tttcgggaaa ataagtggga gttttaggaa tagtatgggg 187680
   aagggatggt ttcggtaggg aggatttttg tgggtagttt tgttggaaga gttgaaaggt 187740
   agttagagag gttgtgcgtg gatggaagtt gaagagtagg ttaggggcgt gtgttttgtt 187800
   aataaagatt ttaagtattt attgattagg tataatatta tgtgttttat aaataaatat 187860
   tttatttgat ttttagtatt tttatgagat agatgttatt attttatttt atatgtaaga 187920
   atattgaggt atagagtggt taagttattt gtttagggtt atatagtacg tcggtggtag 187980
   gtaggatttt ggagttatag gaatttggta tattttaggt gtaatggaaa ttagttacgg 188040
   ttttatagag ggaaattatt agattagtaa attttatata cgttttttgg tgttttgagg 188100
   aggagggatt cggatagggg tagttaatgg tggtggttgt gagatgtgtt gggttaatat 188160
   tgatgagagt ttgggatgga tgagtggatt cggagtaatg gaaatggagg gtttaggttc 188220
   ggtttttgga gatttagttg taatagttgt tggttggaga ggttgtttat ttagatgggg 188280
   gatggggtgg gaggtaggtg agatgagagt tttagagttt agtttggggt acggttattg 188340
   ggtttaagat gtttttagat gttggggttt gatagtgata gtggttgtag atatttagga 188400
   tttagtagag ggtttggtat agcggggttt agggatacgt ttgtgagtta ggttggtatt 188460
   taagtgtgaa tttggataaa gaaggtggta ttgttggtat tgaatgttta ggggttgggt 188520
   gaagttagtt agggagaggg gtttttaagg tattaggatt gatttcgtgg gtgagggcgg 188580
   aggaggagac ggtagaggtt gagagaatag ttagagatag gaggaaggtt agtagagtta 188640
   tgtatagaag tttggagaag atttgaggaa aggaagggtt gggtggtcgt tattttttgt 188700
   agttaggagg taagggtgat ttttgtgtgt tttatttttt agaggattgg tggttttatt 188760
   tggttagaat tttaagttta gagatgtatt tgagttttat gttatggtgt aggtagtagg 188820
   agttatggag taggtttgcg gggttacggt ttgtttgttg ttttttggtg tttttatttt 188880
   gagtttaggg gttgtttgga tgttttgtgt tgtatttttt taggcggtgt tttagagagg 188940
   atgaaagtgg ttattttaag gtgtgattag gtagcggttt atgtatattg tttttttttg 189000
   gggagagttt gggatggaag ggtatgtgtg tgtttttgag attttttgaa taggttttat 189060
   gtatttattt atttatttta ttatttattt atttatttat ttatttattt atttacgtat 189120
   ttatttattt atttatttat ttatttattt gttaatttat ttatttattt atttatttat 189180
   ttatttattt atttatttat agttatttat ttatttattt atttatttat ttatttattt 189240
   atttatttat ttatttattt gtttatttat ttatttattt atttatttat ttatttattt 189300
   atttatttat ttatgtattt atttatttat ttatttattt atttatttat ttatttattt 189360
   atttatttat ttatttattt atttatttat ttatttattt atttatttat ttatttattt 189420
   atttatttat ttatttgttt atttatttat ttatttattt atttatttat ttatttattt 189480
   atttatttaa ttatttattt atttatttat ttatttatta ttcgtttatt tatttattta 189540
   tttatttatg tatttattta tttatttatt tatttattta tttgtttatt tatttattta 189600
   tttatttatt tatttattta tttatttatt tatttattta tttatttttt tatttattta 189660
   tttatttatt tatttattta tttatttatt tatttattta ttcgtttatt tatttattta 189720
   cgtatttatt tatttattta tttatttatt tatttattta tttatttatt tgtttattta 189780
   tttatttatt tatttattta tttatttatt tatttattta tttatttatt tatttattta 189840
   tttatttatt tatttattaa tttatttatt tatttattta tttatttatt tatttattta 189900
   gttatttatt tatttgttta tttattcgtt aatttattta tttatttgtt tgtttattta 189960
   tttatttatt tatttattta tttgtttatt tatttattta tttatttatt tatttatata 190020
   tttatatatt tatttattta tttaattatt tatttttatt tttattttag aaagtatttg 190080
   aagttgttgg ggtgtgtatt tggaaaatgc gttataagtt tttataggtt tttttttggt 190140
   ttttttattt ttttgattgg tttttttttc ggatatgtag gattttgagt agtttttttg 190200
   ggaggtgggt gtagtttagg taggttgttt gtaaagttag gatcgaggga gttatattta 190260
   aggtattgtg ggtttttaag gcgatggtat tgtgggttta ttgtttttag atttttttaa 190320
   tttttaggtt tttgagtagg aggatttttg gggtagggtt tggagacggg aagagaaaat 190380
   aattttagtt ttttaatttt tgttagttta ggtgggtgtt gtatggattg ggggtatttg 190440
   gaatagttgg tttttttatt tttttttttt atttttttag tttttttggt tataattaga 190500
   tagttgtgtg cgatgttttt tttttttttg aaggggttat ttagttaggt ttttatttat 190560
   ggtttaggaa tgtgtttttt tttgtttgtt ttttattttt aagtaggtcg tgtttttagt 190620
   tttttgttgt ttttgaaggg tttgggtatg gggtagtttt tgtagtattt ttagggttcg 190680
   agttgatttg tatagtagaa gggaattggg ggaaagtttt ttttttattt agtatttggt 190740
   agggggttat tttgttgtat tttagagatt tttgtttaag ttttgttatt ttagatgatt 190800
   ttataagttg ggggaggcgg agttttagtt tttatagttt ttgttgttgt tttattgttg 190860
   agttttgttg tgtatttgtt gataggtttg ttgatagtag aaggggttgg agagagggaa 190920
   gttttgggtt ttgttttttt taatttttgt ttaggggata tttttgataa gaggaaattt 190980
   tagtttgtga tagatggtta gtgtttattt atttacgggg gaattttttt ttttttttgt 191040
   aagtttgtag atttgttagg aattaaagga atttttggtt gataggttat tgtggatatg 191100
   gttggagggt tggtacgggt tggattttgg ggttgttttg aggtaggggt cgtttttgtt 191160
   ggaaggtttt ttggggtggg gaaggtatta gggttatttt ttgatttttt ttttgtagtg 191220
   gatttatttt agagtttgag ataaggtaag ggggtagtag aaaatagaat ataaaatagt 191280
   tttttttatt atatatcgta ttagaagtta gaggatttat ttttggtttg ttttttttgt 191340
   agtttaagtt ttgagagtgg ttttttagga agtgaatggt gatggtttgt aggttagagt 191400
   ttgttttggt ttaagattga tgttttaata ggtcgcggag tttgggtgaa aaaatttagg 191460
   ttagggaggt ggtgggttgt aaatgaaaga atagaggagg gtttttttta gaaagcggcg 191520
   ggtggtgtga ggagtttttg gattttgttt aaatttgatt ttatcgattg gggttaattg 191580
   aaatcgtgtt ggtagaaagg taaaattgtt tgagattttt ttttttataa tagaggagag 191640
   gtttgtttga aagggtttgg cgagggaaaa gttggaggaa ttttggtcgg tagtaaaagt 191700
   ggggattttc gttttttgtt tcgggggcgt ttcggttttt gaatgaatga aagaggaggt 191760
   tggatggaga tagatttata ttatttgttt tttttaggtt ttgtaaagga gtatataggg 191820
   tttttgttgg tttttttatt ttgttagtta ttaagaagga gaaaatatta ataggggaat 191880
   tgtaaagttg tagttttgtt taagttttat agtggaaatc ggaaagagtt ttagttcgga 191940
   gaaaaatgta gttttttgtt tttgtaaatt cgtgtttttt tttgggaggt tagagttatt 192000
   gtgttcgatt tttaagtagg gttttagagt gttttaggag gggaaggtag agttttagat 192060
   tttcggtatt ttaggtttta attaggtttt gaggagacgt tttttgtttt atataaagtg 192120
   tgaagtgtaa tttgatttgg agttagttaa taaattagat gagttgtgtg tattttaaaa 192180
   atttagtttt tgatttttta tttatatata tttgtataat ttggttattt atatttagtg 192240
   tttttatttt tagaagagaa taggaagttt ttagtgatat ttatggagtg tttgtagtag 192300
   tttgggtagg ttagtaggaa tataatgtag tgaaaagtag agttgagatg ggaaaatatt 192360
   ttgtttttag agttttttag ggatgatatt ttttatcgtt tttgtttatt cgttttggag 192420
   tttatttcgt aaatagtaat ttgtttttat ttgagagttt ttttttgtcg gagtttaggg 192480
   atggagagta gttggtttta gttttttttt tttatttttt tgttttttag atttattttt 192540
   tattttgaat aattgttttt aatttgtttt tttttttttt tagttgtaga atttagaatt 192600
   ttgtgtgtgt ggttaaaagt ttagagagtg ttgtaggtaa tgttgagata attttattaa 192660
   attttgtaag tttttattat attaagtttg tttgtttttt ttaattttta ttttatttta 192720
   ttttatttta tagggatggg gtttcgttac gttgtttagg ttggttttga atttttgggt 192780
   ttaaacgatt ttttcgtttc ggttttttag agtgttaagt tattttttta attgggattt 192840
   acgggtttag ggattttttg atgaatcgtt taaaatacgt gtgtgtttgt ttgtttgttt 192900
   ttaatgtata tttttttagg gagagttttt attttttatt agaggtttgt ggtatatata 192960
   tgttttgtat tattgtatta gattgtgaat tttttatatg ttaaagattg ttttaaattt 193020
   gttcggaagt ttcggttaat aaatatttaa ataaatgaat tttaaatttt tatgtgttat 193080
   tattgttttt attttgatta tttttaagtt tgtagtgtta tttttttgtt attgtgaagt 193140
   agatatttaa taaatatttg ttaaatgaat taatgttatg gttttggttt attaggattt 193200
   taggattatc gtttattatt attgtttata gtttttttat tattttgttt tagagtagtt 193260
   ttttttttga ggtttgttta tttttgtacg tgtgtttagt ttttagtttt gtcgttttat 193320
   tatttttttt tttttatttg tattttttga attgttaata gttaataata gttttttttt 193380
   ttatttattt atttatattt gaagtgagaa gttattattg aggtttttgt ggattttgtt 193440
   agtttaattt ggggcggttt tttggaggtg gagtttttag atgagggatg gatattttgg 193500
   tgtttgttgg gtagatttat ttgggagttt tggtaggggt tttagggtag ttttgttttt 193560
   ttattttggt tgtgggattt ttggtgtatt agggtttttt aagtaagtat ttgtgttttg 193620
   ggagagttat aggtaggttt gagagggttt ttggaatttg ggatttttag ttcggtttag 193680
   tagttggtat tattattttt tttgtaaggg aagttatgga aattaagatg ttaatgtgtt 193740
   ttgataggtt tggggtggtt ttaagttttg ggaagagttt tggtaatttt ttttggttgg 193800
   cgggtagaat ttttttttgg tagtttaggg ttttgttttt agtttttggg gggagagtaa 193860
   ttgagggagg agttggaggt taaggtttta gtgttgagtt gtttgtatcg tagttgtatt 193920
   ttttgggttt tggttgtagg aattttatat ttttttagtt tttttatggt tttcgaagat 193980
   ttggtttggg gagggggtta gtagtttggg gtttatttat ttggttttaa attttggttt 194040
   tatcgtttat tcgttttgcg atttttataa ttgataaaag ttttttcggt ttcggttttt 194100
   ttatttgtga aatgggataa taatagtggt tattttatag gattgtgtga tgatttatta 194160
   gttataatag tttttgagta gcgtttagag taatatttgg ggttcggtgt agtggtttat 194220
   gtttataatt ttagtatttt gggaggttaa ggtgggagga tcgtttgagg ttaggagttt 194280
   gaaattagtt tgattaatat tagtgagatt tattttttaa aaaaaaaaat tagagtaacg 194340
   tttggtatag agagttttta gtaaatttcg gttgttgtga tgagttttgt ttttttattt 194400
   gtttatttat ttattagatt ttttgtcggg gtttgtcgtg agttttacgt tgttttgaag 194460
   aaagtcgggt tggggtgagc gtgatggatg aggtgtttgg ttttataaat tttgtgtggg 194520
   ggggttgtgg tgagaggaaa tcgtatatat tattatttag ttattatcgt ggggagattg 194580
   tttggacgta gagagtaggt tttgtgtggg agcggggagg gtaagttttg gttgcgaggt 194640
   agggtttttt taggatttag tagggatttg aaggattttg taggtattta gggagatagg 194700
   agaggaggag ggagtagttt tggtcggata ttgttttttt agtattgttt gtattaggga 194760
   tttatttagt tttaagaagt ttttttgtgg gggataggga gtatttgtta gtttatagga 194820
   tttgaagtgt ttttatgggt ttaagttttt gggaaggttt gtaggtggtc gtagggttgt 194880
   cgtaggggtg ttggttttag ggtttggatt taggggagtt tgtagaggga gggtagttgg 194940
   aggttgtttt agtgtgtatt gttacgaggt aaagtaagga gattgttggg tttacgttgg 195000
   gtcggggtgg atggaggtaa ggaagttttc gtcggggtaa gggtattagt tgtagatgtc 195060
   ggtagttttt ttttggatac gggtttggaa ggttgatagg gtgtggtgag tgttatcggt 195120
   ttttttgtcg tggtttggtt agtggtttta taggttttcg tgggtaggag gaggatgatt 195180
   ttgtattttg tttggttatt attggtagtg atagataagg tgtgtgggga tgtggttatt 195240
   tcggttcgtg ttttgagagt taggtgtggg ttgggtttgg ggaagatagg ggaatggtat 195300
   tgatttgatt ttgagtattt tgttttgggg tgtaggttcg gttgttattt ttttggagtt 195360
   gggaatggta ttgggcggtg gtttagggtt ttttgtttta gcggtattat ttttgtattt 195420
   ggttagtaag gttgatggga tttgtgtgga tatttttggg acgtagatta ggtagtatga 195480
   tatataggga ggggtaggta gagaagggcg gtttttgggt tttggttttt aagttaggtt 195540
   ttgtaagttt gttgtgttaa gttttttttc ggtattaggt taggtgggtt agggttagtt 195600
   ttggggttgt gatgagtgtg agtgttttta aagtgtgtta tgtttaatag ttgttttttt 195660
   tttgggtatt gttttttagt tagatatttt atagttttcg ttttagttat agatagtaat 195720
   ttgatttttt tattttcggt aggttttggg gttggggtaa gaatttgtta ttaagtgtag 195780
   atgtttttag agggtgttgg tttagggttg cgtggattat agttttttta tttttgtttt 195840
   agatttattt gtaattttgg agaaaagatg gtttagtgga cgtttgcggg gaggagggag 195900
   ttggttttta ggatgagtag atttgggggt agtttttatt acgggatcgg tgatttattg 195960
   ttattagcgt tagtgaatgg tagaggggtt ttggtataag gtttgggttt agaatgggaa 196020
   tttgtagaga aattgttaag gtttttcgtt atttgatatt ttcgtttggt tgttatttta 196080
   gaaatatttt tgttgtaaga agatggggga gtttgtttgg ggtatggttt ggcgatatta 196140
   atttgagaat tttaggttta agtgttggtt tttgggtttt agttgttttt tgagttgtaa 196200
   agttggtttt tgggtatttg tggaaaatat tggatttttt attttatttt atttatattt 196260
   ttgggatata tgtgtagaac gtgtaggttt gttatatagg aatataagtg ttatagtggt 196320
   ttgttgtatt tattggattt tttaatttag ttttattaga aatgattgtt tttttttttt 196380
   tttttcgttt tttttttttt tttttttttt tttttttttt tttttgtttt tttttttttt 196440
   tttttttttt tttttttttt ttttttttcg tttttatggg gatataattt ttatattata 196500
   ttattcgtcg atttagaaag tatagttgag tggtattttt ttataatttt ttataagtat 196560
   tatttttaat tttagaacgt tttttttatt ttaaaaggaa acgttatttt cggtatatag 196620
   ttattttttt ttttttagtt tttggaaatt attaatttgc gttttgtttt tatagatgta 196680
   tttattttga atatatgatt agaattttaa aagatgtgat tttttgtgtt tggttttttg 196740
   tagttggaat gtttttagag tttatttgtg ttggcgtacg ttcgtgtttt tttatggttg 196800
   agaaatagtt cgttgtgttg atttagtacg ttgggttttt ttacgtattt gttggcggat 196860
   gtgttagtta tttttggagt cgggtgtgtt gtgttattta cgggttgtat taggagttgg 196920
   ggatattatt gagtaagtta tagtttcggg tatttaggag ttcgtagagt aggagttggg 196980
   agggaggtta ggaggtgtat ggtttgtttg ttaggttgga gagatttgta aaaagttatt 197040
   ttgagttaga aattagagtt tgaggttttt gatttttagt tgcggagtcg ggagggaagt 197100
   tttgtatttt gttttgattg tggggtgacg tgttagttat tgtttaaatt agaatttttt 197160
   tgagagtcga cgtgggtatt gttgataatt agtttggata ttcgaaagaa tggaaagtag 197220
   ggatttagat agatttttgt ataatcgtgt tcgtagtagg gcgaggtttg gaggtgcgat 197280
   ggggatttta acggggtagt tgtcgttttg ttggagttta cggtttttgg ggggggttat 197340
   aggtgttaat cgtatattat ataagtagga gtgtgaaatt ttagggttgt taaatttagt 197400
   gagggttttg tgttggtttg gttatttata gtggtatttt aggaaaaaaa attattttat 197460
   ggattttagt ttttttatta gtaaagagta ggttggattg gaattttgta ttatttagta 197520
   tagttgttat ttatgaaatg tggttattta aatttaaata aaaatagaaa tttcgttttt 197580
   tagttggatt agtttagtgt taagtgttta gaagttattt gtgaatatat tgtggtaatt 197640
   ttttaaaaaa cgaaaattag aattatcgta gtattttata attttatttt tgggtgtgga 197700
   tttaaaagaa tgtatagtat tttaaagagt tagagatatt tgtatattta tatttatagc 197760
   ggttttgttt atagtagtta gcgggtagaa ataattcgaa tgttcgcggt taaatgaatg 197820
   gagaaattaa aggcgggata ggtcgggcgt ggtggtttac gtttgtaatt ttagtatttt 197880
   gggaggtcga ggcgggtgga ttatttgagg ttataagttt aagattagtt tgattagtat 197940
   ggagaaattt cgtttttatt aaaataaaat tagtcgggta tggtggcggg tgtttgtaat 198000
   tttagttatt tgagaggttg aggtaggaga attgtttgaa tttggggagg tagaggtttc 198060
   ggtgagtcga gatcgcgtta ttgtatttta gtttgggtaa taagagtaaa attttgttta 198120
   aaaaaaaaaa aaaaaagggt ggaatatata aggaatttga tttagtcgta aagaggaagt 198180
   taggatattt gtaatagtaa gggtgaattt ttcgggtatt acgttaagtg aaataatttg 198240
   gttataaaag gataattttg taggatttta tttatattag gttcgtagag tagttagatt 198300
   tagaaaaata gtagaatgga ggttgttaga ggtttggggg atggggttgg ggagttgttt 198360
   aatgaggata gtttgtttta taagatgaaa aagttttgga gatttgttat ataataatgc 198420
   gaatgtattt aatattattg gattgtatat tttaaaatag tgatcgtggt aaacgttatg 198480
   ataattaaaa ggaaaagtta cgtggttagt ggttattacg ttgagtagcg taggtttcgg 198540
   acggtgtcgt ggttatagat aatggagtgg gtgattttta ttattgtttt tagttgtttg 198600
   agtattatgg tgttttgggg ttaggatgaa agtgtttttg ggtatggggg tttttgtttt 198660
   ttttatttgg gagttttgaa ggtagatttt aaagtttatt tggttaggta ggagagcgag 198720
   gcgggttttt gtattgtttt ttgcgtgcgt gtttgtttgc gggttagtcg gggtttgtaa 198780
   ggtcgttttt cgagggaggg gttgtaggaa tggtttttat ttgtgtagaa ggtaagttat 198840
   tgttttacgt taatggtaaa gatggggttc ggttgcgttg tattattatt ttttaagcgg 198900
   ggtttttggt gggtaggatg tttggtttga tttgagtttt tgtagtgagt gttggtgttt 198960
   gtggagggat ttagttgtag ttgagtcggt ttttttttcg ttttgggttt taggggcgtt 199020
   gggggcgaga aaggagagtt tagggggtag cgtttttttt taggttttgt tggggagggt 199080
   tgagggatgt tggggggagg tgggggcggg taggggttgg ttttagtttt aggtaaaatg 199140
   gtttttagat ttataggtag atggaagcga ttttcggtta cgtgggtggg gattgaattg 199200
   ttgtttcgta aggtaaggtt ttatgtttgg ttttaggaag cggttttaaa gttatatggt 199260
   ttatggttag atgtagggtt gtgtttttgt ttatttaaag gttttggaag tttaggggag 199320
   agtatttttg aagggggagt aggagtagga gtaaagtttt gaaggttttt aggttgcgtg 199380
   gtttgggtgg gggttacgtg ggggtataga gagaggagtt ttggttttta ggttcgtttg 199440
   tgggtattgt ttttagtagt ggagatgtgt ggattttttt ttggtatttt ttaataagga 199500
   attatattgt ttttttgttt ttttaatttt ataagatagg aatttttgtg ttttgtggag 199560
   gttgagaaga cggggaggtg tttgtgagga taaattagtt tttagttttt ggtttttaga 199620
   gttttttttt tttttatttt tgatgtttta ggtttgggga gttttttttc gatatttttt 199680
   gatcgtttta gtgaaatagg tcgagaggga tggtttgaat cggttgggga tgtaggttgt 199740
   agttaggggg tttagtgggt attttttgag ttcggttttt gtttcgtaag tggaagggta 199800
   gttgaattga agttttttat tgtttttttt ggtttggaag tttgtttttt attttttggg 199860
   tagagtgagg agtgggcgtg ggtcgggagg gttgtttttt gtttgggagg gttgtttatt 199920
   tgtttgttac gggggatgag ttttttgtag tttttcgtgt tgtggttttg gtttttacgt 199980
   cggttttatt tttttgtggt tgtttttgtt gggtgtttat cggttttagg gaaaagttat 200040
   ttttttttcg gaggattggc gggaggggtt ttgtttggga gcgtgtcgtg tggtttttgt 200100
   tttagcgtag gatgtttgag gttagatttt ttttgggagg taggatgggg taaaagtttt 200160
   ttcgtaggta gagtagggtt tggatgggtt taagtaggga cgggggtata gaatagtagt 200220
   cgtttagttt gcgtgggagt tgagggttat taagggtagt tttagtttgt ggggattagt 200280
   agaggtatta tttttgtgat tagcgagttg tttgtatttg tatgcgtatg tattagtgag 200340
   tgtggcgagt ttgaggtgtg ttgggatttg gttcgtgtat tagttagttt tgatgggaat 200400
   tagttttatt ttgggtattt ttggaatttt attgtgtggg agtatttggg tttttagtag 200460
   gcgtttttcg ggcgttcgtt tttagtttta ggtttttgat tcgtgtgtgg tgttttaata 200520
   gtagttagga gtagggttat cgaagggatt tatagataga agaggtttta gtgatttcgg 200580
   gaagtttagt tttacgtgtt ttatatattt aagagtaaaa tttggttttg gtttgtgggt 200640
   gtgtagttcg tgggcgttgg ttaggtatat aagacggtcg tttgggttag tttaggggga 200700
   ggtagatgtg gtgtaggggt tttttttttt tatgagttta ggaatagttt tgtatttagg 200760
   tattcgtttt gtatttagta tttttttttt ttttaggatt gttggttcgt tttatgggat 200820
   tgaatatata attttatggg gatcgacgtt ggttttattt ttttttttta taagattatt 200880
   tatggggatg tgggaggcgg ggatatacgt tggttttatt ttcgtttttt ataagattat 200940
   tttttttgtg ttttggttcg tggatcggtt tttaaggttt ttgagtggta gtaggcggta 201000
   tagtttattt gagtagaggt gaaggagagg gttaggttta tttgggtggt tcgaggtagt 201060
   gtggagggtt aggagaggag agtatttttt atttttgttt gagtagtttt ttagggtacg 201120
   gagataggag tttagggtta gttttgtttt ggagcgtagg gggtttttag tttggtatag 201180
   tttttagaag aggagatgaa aaagtttggt tttgggagtt tggggatgga gttttgttgg 201240
   ggaggtagcg tggttgtagg atggtagttt tttagaggat attacggtta gggatattta 201300
   ttcgggggaa tttttagggg tcgtttgggt aagtcgttta gtttttttga gttttagtgt 201360
   tttagttttt ttatttaagg atgggtgtga taattatttg gtaggcgttt gtgtgtgagt 201420
   ttttgtttcg ttttaatttt tttgttgttt tttttttagt tattgaggcg gtttttagtt 201480
   gcgttggcga tatggtcgat atttttagag atgtcgggtt taagtaggcg tttgtattac 201540
   ggaacgagaa ggtttcggtg gatttcggtt acgtggggat tgattttatt ttggagtaga 201600
   tgcgtcggaa ggttatgaag tagggtttcg agtttaatat tatggtggtc ggtgagtttt 201660
   tattttgtat gttatttaat taatgtcgga aattagtttt agtttttagg gcggtttata 201720
   agtttttggg tttggttttt ttttttgtaa aatggggtag taattttgat tttaaggacg 201780
   gattgttagt gatattgcgt gtgtatgtat gcgtttggga ggagtttata gtttttagcg 201840
   gttttttaga ggggtttgtt ttgtatattt agggtttttg agatgatttt ggggagttta 201900
   ggtcggtttg gagtaggttt tgatgagtgg tcgttgtttt tgagtaatga cgttagtttg 201960
   tgtagagggt tttgtggtag ttgttttttt attgttgttt tagtttggtt ttttaggttt 202020
   tttatcgtat tttatcgtat ttcggttaga ggttttttag tagtttagtt ttagttttgt 202080
   tttggaattg gggtttcgtg gtttttatat tttttggtgg tttcggagag ttggaggtta 202140
   ttacgggagg tcgtcgatta gtacgatttg ggtagggtga gggggttagg ttggagttta 202200
   tagtgggtcg tttgttgttt ttaaagtttt ttaggtttta tttaaatttt atagggattt 202260
   tgtgaagggg gcgtttcgtt ttttaggtag aagcgaggtt tagggaaggt tgttaaggtt 202320
   ttttagttta tagggtagag aatcgtagtt atcgttttga tttttagaat tgtgttttgt 202380
   ttattttacg gggtttaggg aggggtttgg ggaagtgttt tcgttaggtt atattttaaa 202440
   taaaggtgag tttgcggggt ttttgtaagg tgtttgggta ttttttaggt cgggtttgtt 202500
   tttttgattt ttgttagtcg tgtacgtttt ttttgtttag ttttgtggac gtaaatttat 202560
   atttgttagt tcgatatttt tttttttttt tttgtattga tttgggtttt ggtttttttg 202620
   gggttttggt gttttttttg ttttttgtag ttttatggga ttcggttgtt tatggttttg 202680
   ttgtgtttat agttgtttgg aagttggtag tttgttttaa gggagggcgg aggtgtgggg 202740
   ttttttttta cggtttttat tttgttttgt ttgttgtggg ggacgtgttt gtattttgag 202800
   gatttagggt tgtttttcgg gaagatggtt ggagagaaag agaggggaga ggtttaaagg 202860
   tagtggttag gttgggattt atttttgtgg gttacggagg ggttgttttg ttttttagtt 202920
   agggataggg tgagaaggat ttgggaattt ttgcgtttta gttatggtat aggttttttt 202980
   tcggagtttt ggttttaagg agagggagag gtagtttagt agttttcgtt tttcgtggtt 203040
   gttattgtga tagttttgtt ttttgggaat ggtgcgtagg aggttatttt atttgtttgg 203100
   gttttttcgt ttttttaatt ttgagaagaa tagtggtttt gattttttat aaaattattt 203160
   ttttttaggg atgtcgagtg aggaataaga cgtggggtag ttttgttttt tttttttttt 203220
   ttttttatgg tagttgtgtt tatttgtaga ggtgtaggta aggtggaggg gtatttgttt 203280
   ggaggtttat agatttcggg gagaatttta gttttgtttt aatttagcgg ggtgaggttt 203340
   gtgtgttttt ttttgggttt tttttttgtg agttttagtt ttttcgttta taaaatggga 203400
   ttattttata aagtgagatt aagcgagtta ggtttcgggt agggttttta ataggttgtg 203460
   gttgttcgag aatttgtagt agtggtgatg gtggttgtgg tgatgaggat gatgggggtg 203520
   gtaatgacgg tgatgatgat ggggtgatgg tgttagtgga gatgatgggg atgatggtga 203580
   tggtggtggt gatagtggtg ggatggtagt ggtgatgtgg gtgatggtga ttgtgatggt 203640
   ggtggtggtg gtgatggagg tgatggtgat agtgattatg agggtgatgg ggatggtggt 203700
   ggtggtgatg gtgatagtgg tgggatggta gtgatgatgt gggtgatggt gatggtggtg 203760
   atggtggtgg tggtgattgt ggtggtggtg gtgatgatgg tggtgatgat ggtgattgtg 203820
   gtgatggtga tggtggtaat tgtgatggtg gtggtggtga tggtggtgat gatggtgatt 203880
   gtggtgatgg tggttatgat ggtggtaatt gtgatggtgg tggtgatggt ggtgatgggg 203940
   atgatgatgg tgattgtgat gggggtggtg gtggtgattg tgatggtggt gatgtgggtg 204000
   gtggtggtga ttgtgatggt ggtgatgtgg gtggtggtgg tgattgtgat ggtggtggtg 204060
   gtggttgtga tggtggtgat gtgggtggtg gtggtgattg tgatggtggt gatgtgggtg 204120
   gtggtggtga ttgtgatggt ggtggcgatg gtggttgtga tggtggtgat gtgggtggtg 204180
   gtgattgtga tggttgtgat tgtgatggtg gtggtggtga tggtgatgat ggtggtgatt 204240
   gtgatggtgg tgatgggagt gatgttggtg attgtggtgg tgaagggggt gatggtggtg 204300
   attgtgatgg tggtgaaggg ggtgatggtg gtgattgtga tggtggtgaa gggggtgatg 204360
   gtggtgattg tggtggtggt aatggtgatg gtggtaaagg ggtgatggtg gtgattgtgg 204420
   tgatgtgggt ggtggtggtg atgtgggtgg tggtggtgat tgtgatgggg gtgatgatgg 204480
   taatgtgggt gatggtgatt gtggtggtgg tggtgatggt gatgattgtg atggtggtga 204540
   agggggtgat ggtggtgatt gtgatggtgg tgattgtgat ggtgatgtgg gtgatggtgg 204600
   tgttgatggg ggtgatgatg ttagtagtgg tggtgatagt gatggggatg atggtggtgt 204660
   tgatggtgat agtggtggga tggtagtggt gatgtggttg atgatggtgt tggtgttgat 204720
   gattgtgatg atagtggtgg tggtgatgag ggtgacggaa gtggtgatga tggcgggagt 204780
   gatgggatga tggtaatagt agtgatggta ttgtgatgat ggtgttgatg gggatggtgg 204840
   tgatgataag ggtgatgatg atggtgatga taataataaa agtaataata ataaagggat 204900
   atattttgta gtattttagt tggtattttt tagatttaag gtgtcgtggt tggaatattg 204960
   atataatttt tgatttgagg ttggttagga tatagatttg gtggatagag agagatggac 205020
   gaggagtgtt cgtttttggg ttattaaata gtttaggtta gggattttta gtattagttt 205080
   ttgttttttt aggagtttat atttttttta gaaaggtgtt attaaagtta gaatattagt 205140
   gtaatttagg gtgttttttg tgttgtgttt ttttgagata gggttttatt gttgtttagg 205200
   ttggagtgta gtggtatggt tttgatttat tgtagttttt atattttggg tttaagtgat 205260
   tttcgtgttt taggtatgtg ttattatatt tggttaattt ttgtgttttt tggtagagat 205320
   agggttttat tatgttggtt aagttggttt cgaatttttg attttaagta atttattttt 205380
   tttagttttt taaagtgttg ggattatagg cgtgagttat ttcgtttggt cgatttttgt 205440
   ttttttaaaa gatagggttt ttgttatttt ggagtggtaa tgattttagt ttattgtagt 205500
   ttcgtatttt tgggtttgag taattttttt attttagttt tttgcgtagt tgggattata 205560
   ggtgttcgtt attatattta gttaatgttt atattttgat gtagagatag ggtttttttg 205620
   tgttgtttag gttggttttg aatttttggg tttaagtaat ttttttgttt tagtttttta 205680
   aagtgttggg attataggta tgagttatta tatttggttt agggtatttt ttttggtgtg 205740
   ttgttagcgt agtttaaggt agttttgtgt agggtaaagt aatggggttt cgagtttgga 205800
   ggggtgtgtg tgtgtgtgtg tgtgtgtgtg tgtgtgtgcg cgtacgcgcg cgcgtgttat 205860
   atgtgatttg tttgtgtgtg ttgtgagagt tgtgtgtgag gtatgtgtaa gaggtatgtt 205920
   tgaggtgagt tgtgtgtgtg ttgtgagttg tgtatgtgaa gtgtgtgtgt tgtgaggtgt 205980
   gtgtatgagg tgtatgtgag ttgtgtgtgt tgtgttgtat atgaatgtta tttttttatt 206040
   ttcgtttgtt ttagttattt gtggattggt tgtttgtata tacggttatt gtttttcgag 206100
   tggtattatt ttgtaggtta gcgtttttta gtatatggtt ttatagattt tatgggatat 206160
   gcgtgagttt gtttgatgtt tatattagtt ttacggggga aggattgttt attttatttt 206220
   gtagattagg aaattgagat acggtgaggt taggtgattt gtttaggttt tggtcggtta 206280
   gggaggagta gtttgaatat ttgggttttg gttgtaatta ttttggtgaa ttgtaggaat 206340
   ttcggttatt tggtgtattt tgggtagttt tgggtttttt cgttagttcg gtttttgttt 206400
   tgtttaaaat ttgagttatt aggagatttt ttgtttagaa agtataaggg gtataagaga 206460
   tgtcgatttt tttaggttcg gtttattttt taggagagga ttgtggtttg ggttgtgttg 206520
   gtattagagt tcggttgtgt gggtttatat agttattgga tattcggttc gagggtgaag 206580
   ttttcggggg ttgtttggta gggatattgt cgggaggtag tttaggtatt gttgaatttt 206640
   agattggaga gtttcgtgtt tgggtgggag gggttttatg tgtagatttt gttggttttt 206700
   tcggagagat ttttgatcgg ttttttattt tttttttagg gtagagcggt ttgggtaaat 206760
   ttattttaat taatattttt tttaaattta aaattagtcg gaagtcggtg tagtttattt 206820
   tagaggagcg tatttttaag attatcgaga ttaagtttat tacgtacggt tagtggtcgg 206880
   gagtgggttg ggggtgtagg acgtttttgt ttttttggag tataggggtt gggggttaag 206940
   attattatat atagttagtg gttaggggcg ggttgggggt gtaggatttt tttgtttttt 207000
   tggagtatag agtggggggt taagattatt atatatagtt agtggtcggg agtgggttgg 207060
   ggatgtagga cgtttttgtt tttttggagt ataggggttg ggggttaaga ttattatata 207120
   cggttagtgg ttggggatgg gttggggatg tgggatattt atgttttttt ggagtattgg 207180
   tgcggtgtga gggtgttcga gttaggtaat tttaggtttt taagacgggg attcgttgtg 207240
   tttattattt ttagtatgtt gagtatgttg gggttttggt tgtgatgggt tagtgtttcg 207300
   gatttttgtg ggtttcgtta tattgagttt tttatagata attgagtttt ttattaaaag 207360
   tttgataggt aaggggtttt taggggaatc gttatcggtt tgtgtcggag ttggttgagg 207420
   aaggttatta atttttcggt gtttttttgg ttgttatcgt tgtttatttg gggttgtttc 207480
   ggggttagtt aggtgtgggg tgtttgtgag ggtttttcgt tttttttttg attttgcgtt 207540
   cgtggttttg tgtagatatt gaggagaaag gcgttcggat gaagttgata gtgattgata 207600
   tattagggtt cggggattat attaataacg agaattggtg aggttttttt agggggagga 207660
   gtattagcgg gggttttagg gttttttgga ttttatttag agttagtttt agaggttttt 207720
   cggttcgcgg gggtgtaggg tttatttttt gggatttgat atgttgttaa agtcgtacgt 207780
   tttaggggtt tgaaaatttt ggattaaagt agaattattt ggggaatttt gttgttttta 207840
   atttattatt tgaggttagt tcggggaggt tggtttggga ggtcgagggt aaggttgtgg 207900
   tattttcgtt tagtaagtta gattttttag ggtatgtatt ttattggaag gtggggtgtt 207960
   gggttttgag ttttgggaat gtacgatttg tttggggagg gtattttatg tgtttgttga 208020
   ttcgttttta ttttttacgt agttggtagt ttattatgaa gtttattaat gattagtacg 208080
   agaaatattt gtaggaggag gttaatatta atcgtaagaa gcgtatttcg gatattcgcg 208140
   tttattgttg tttttatttt attttcgtta tcggttattc gtacgttttt gtagtgtcgg 208200
   cgtttttatg tcgggtgttt tgggtttttt ttgttttttg ggattgtaag acggtgttgt 208260
   ttgttttgtt gttgggtgta gaggggttga gttaggggtt atggtcgtta gttatgaagt 208320
   tgggggtgtg ttatgtttgt atttttaaag ttttatattg atttttatgt aagtgttttg 208380
   tttagttttt attttttagt tgcgttttgg ggagttgagg gttaggtagg ttttgggttg 208440
   ttttggagtt tggtggtttg tgttttgttt ttttaggtta ggttttggta ggtggagtgg 208500
   gagtttttag ttaagtattc gtagcggtga ttgatttttt tattcgggtt ttgttttatg 208560
   ggatttttta gagggtttgg tgcgttttta gacgggaagt atagagatta gtgttggttt 208620
   tttttagggt tggacggggt tgttgttggt gttggtgaga gttttttttt ttcgtcggtt 208680
   ttgttcgtat ttgacgtcgt ttttagagat tagtaagggc gggaggttgt atgtattttg 208740
   gtaggtggat tttttatggt tttacggatt ttttgttttt agttttttgt gtattttttg 208800
   ttagatattt gtggtttatt ttgagttagg gttgggagga ggaggattag aggttagtag 208860
   gagtggttgg tggaggtagt cgtgggtggt ttgtttgtat gggtgggtgg agaaggtttt 208920
   tcgtatttgt tgttttagat gtattttttt gggcggagga agtttagggg tttggggaaa 208980
   ttatattttg tgggtattag gtgtaggaag gagtttggtt ggtgggtagc gggttttatt 209040
   tagagtttgt gggaaaattt aagtcggagt tttcgagttt attttttggt attatttttt 209100
   gtgggttagt aggagttagg ggtagggggt tagttcgttg tttatgggtt ttttttgaat 209160
   tgaatatttg tttttttagg tagaacggtt tatttgcggt tttgtggtag ttgttttagt 209220
   tgttttaagt tttgttttag ttgaggttga tggtaggagg tagaagtttt ttttgatgtt 209280
   agttgggtaa gtcgagggcg ggcgggcggt ttgtggtatt tttgttattt tttagtattg 209340
   attttttggt tgggatttta gttaggtatg ttgtttggtt tcgtttaagg cgtttagttt 209400
   tatggggtag gggcgggttt tggttatttt tgtttaaatt tttgttcgga gcgggttttt 209460
   ttttagttat gtgtgtgatc gatttatttt tattgtggga aaagttcggt tttagaattg 209520
   gggaagatgg tgttttagtt ttagggtttt gttagttttt gttatttata gtttttttta 209580
   tattagattt taggtttatt ggggttaagt aggtaagggg agtagttcgt ttggtgtagg 209640
   ggagtagttc gtttggtgta ggggtatcgt atattggtgt tagtttttat tttttttgtt 209700
   tttttttttt tagttaaagt tttatagtaa gacggttgtt gagttaacga tagttttttt 209760
   gtgtggatat gtaggggtgt ggttgggatt ttagaattta tggacgaggg tgtttggagt 209820
   tggttttggt tcgtttggtt tgtgtagtgg tgttatttgt gtttgggata tattcgtgtt 209880
   ttttttttgt tttatattta tagcgtggtc gattcggttc ggggttttgt ttttgttagt 209940
   agggattttc gtgagtttcg agttagtatt tgagagtgtc gatatttgtt tgggggtggg 210000
   tgttttttta ttgtttcgtt tttttagatg agttttacgt atattttttt gttttagttt 210060
   taggtttttg gatatcgagt ttatgaaacg tttgagtaag gtggttaata tcgtttttgt 210120
   tatcgttaag gcggatatat ttattttgga ggagagggtt tattttaaat agcgggtagg 210180
   gttttatttt tatttgtttt agtttttttg tgtaatttgg agacgttgta ggtttggtag 210240
   gggttatttc gtttaaagga gttatattgt tagggagatc gaatcgttgg ttattttttt 210300
   tagcgggtgg ttggtttggt ggtttttggt ttaggtggtt gttgggggtc gtttttgaga 210360
   tggttttttt agttttttta tattttaaaa gtagttttgt gggagtgtta attttttagt 210420
   agagtttttg aggggggttt tggagaagat ttggattagg tagatggtga attttttttt 210480
   tagggagtga ggtcgagtag gggtcgtgcg atatggttta ttttgtttta tgtttttttt 210540
   tttttttttt gagaaagggt ttggtttagt tgttaaggtt gtaatgtagt ggtataatta 210600
   tggtttattg tagtttttat tttttaggtt tagttttcga gtagttggaa ttatagacgt 210660
   atattattat gtttggttaa tttttgtatt tttagtagag acgaggtttt gttatgttgt 210720
   ttaggttggt ttttaatttt tgggtttaag ggattttttt gttttagttt tttaaagtgt 210780
   tggggttata ggtatgagtt attttatttc gttttgtttg gtttttatta gatggaagag 210840
   aagttgggtt aggtgtggtg gtttacgttt gtaattttag tattttggga ggtcgaggcg 210900
   ggtagattat ttgaggttag gagtttgagg ttaatatgat gaaattttat ttttattaaa 210960
   aatataaaaa tattagtcgg gtatggtggc gggcgtttgt agttttagtt atttgggagg 211020
   ttgaggtagg agaatttttt gaattcggga ggcggaggtt ttagtgagtc gagattgagt 211080
   tattgtattt tagtttgggt gatagagcga gattttattt taaaaaaaaa aaaaaaaaaa 211140
   aaagttttag gagggatggt ttttgttatt ttttttttcg ttatcgtttg aggtttttgt 211200
   tgtgggatag ggaggggtat ttaagtaaga gggtagtttt gggttttttt ttttgatagg 211260
   gtagtatatt agagttatat ggggtgtttt ttaaaatgaa atcgaggtgt ttgggttgcg 211320
   gtattatatt tgtttcggtg ggtgtgggtg gggtttgtaa tttatttcgt tttggggtta 211380
   ggtgttaggg atttttttag tatgtgtagg ggaagatagt ttatataaag tgggtgtgtt 211440
   tgtcggaggt tatatacggg ttgtggtttg tgtttgggta ggcgagtagt cgtttattgg 211500
   gatgtttttg ttgtacgtat ttatgtcggg ttgttggtag ggagttgaga ggttattgta 211560
   ggcggggttt ttgttggaat tggggattgt gacgagggtt ttttaggaag ggttttagga 211620
   ggggaggttt cggtaatttt gagtattttt ttggggttgt gatgagtagg agaagagagg 211680
   tgggaggtgt ttaggacgat gattttaagt ttttggggag ttgtagcgtc gtttaggata 211740
   gtaggtgtat ttgtagttgt tttttttttt tttaggaggt ataggagttg gaggtgattg 211800
   gtgttatagt tttttagagt ttgtttttga attcgagttt ggggtagtat atagtgtgga 211860
   ggttatgtgg taaatattag tgggtgggta gagtttgtta tagggatggg tttatttttt 211920
   ttttttttta ttttagatta tcgtagattt gttgtttaac ggtatcgacg tgtattttta 211980
   gaaggaattt gatgaggatt cggaggatcg gttggtgaac gagaagtttc gggtgagtgg 212040
   atttattagg atgttgtttt taggggattt ttagtttttg ttgaagggtg ggttgggggt 212100
   ttggaggatt ttaagttatg aaattatatt tttggggtta gagggtattg agtttaggtg 212160
   tttgtattta gtgttgttag gttgaggttt tcgtttttgg gggattttag gtttagggta 212220
   gtttttttcg ggggtttagg ggagggaatt gttttttcgt atatgtgtaa ttaatatcgt 212280
   ttgttcgttt tttaggagat gattttattt gttgtggtgg gtagtgatta cgagtattag 212340
   gttaacggta agaggatttt tgggaggaag attaagtggg gtattatcga aggtattcgt 212400
   cgtaggcgtc ggggttttag atagatggga agatagtttt ttttgttgat ttagagtttg 212460
   tgggttacgt ttgagttagg gattcgtgga attttattta ttgttttgtt ttatttagag 212520
   ggaggggttt ttttgttttt atttaatttt tgggaaagtg agagggtaag tggtttgtgt 212580
   agatgttttt attttagatt ttttttcggg tagggaaaga tttagggaga taggagttgg 212640
   gatgggggtc gttaagtttt ggattttggt gtaggttttt ttataggtat ttgtgtggtt 212700
   ttggataaat cgtttaattt ttttgggttt agaaaagggg tgggttgtag ttttgtgagt 212760
   tagatggatt ttcggttttt tattttgttg aagtttttgg gatttttata ttttagtttt 212820
   ttgagtggtt gttgtaatta attagtataa acgaggggat ttaaaataat ataaacgtat 212880
   ttttatagtt ttagaggtta gaggttcgaa attaggggtt gttttttttt gaggttttga 212940
   ggattagttt tatttatgtt tttttttttt tggtggcgtt tggtattttt agttgtattg 213000
   taaaggtatt atatgtttta atttttgttt tattgttata gggttttttt ttttgtgttt 213060
   ttgtttcgtt tttttttttt tttttttttt tttttttttt ttgtttgaga cggagtttcg 213120
   ttttgttatt taggttggag tgtagtgttg taattttagt ttattgtaat ttttgttttt 213180
   tgggtttaag taattttttt gttttagttt ttcgagtatt tgggattata gttacgtatt 213240
   atttatttga tttaattttt gtatttttag tagagatagg atttcgttat gttggttagg 213300
   ttggttttaa atttttgatt ttaggtgatt tgtttatttt gtttggtttt ttaaagtgtt 213360
   gggattatag gaatgagtta tcgagtttgt tttttttttt ttttgttttt ggataaggat 213420
   gtttgttttt ggatttaggg tttattttaa gtggatgatt ttatttggag atttttaatg 213480
   aatttataaa gaaagacgtt ttttaagtaa ggttataatt atgggtttta aggtttggat 213540
   tttgattatg tttttgaatg gatgggagtt tttggggggt tatagtttaa tttatttttg 213600
   gcggtgatat ttcgggaggg atgggttcga acgttttttt tttttttttt tttttgtttt 213660
   tttatttttt attagaatgg aagaggggaa ggtgtagagg gaaatgtagt aggaaaagtt 213720
   attttgtttt gggagagtat ttggttgaaa ggtttagtag agtaggaagt ataagttttt 213780
   taatttttta gggttttagt ttttattatt tataaagtgg gtgtagtgtg ttaagatttt 213840
   agtgagttga gagattgttt taaagattat agagtttttt gggaagttgt tgttttaaaa 213900
   aaatggttat aatgataatt attaggaggt attagatatt tttgtgttat tggttagata 213960
   tgggttattt cgttttatgt tcgaagtttt tcgtatttat tttttttgta gagttgaaga 214020
   ggtgttgtga gtagaaaatt tgttaaggga tttagaacgg gaggcggttt tagaatttat 214080
   agttttagtt ttgtattaga ttttttcgag atgggagttt atgtttggag gagggtgtgg 214140
   agaggtatac gggttttttt tttgtttgta tttttttttt attattattt ttgtcgggtt 214200
   tattttgcgg gagttgttta gttgggtgta gtttgggtgt tttttagtat tcgtttgtgg 214260
   tgttaggttg gtttgttttt ttttggtttt tttgtttgta gttttttttt ttttttttgt 214320
   ttaaagagtt tttgttgttt aggaaatttt ttatgaaagg taggtttggg agagttaggg 214380
   atggatgggt ttgggacggg tcggcggttt tgttatgggc gtgtttttgt tgtattttgg 214440
   tggttaattt tgagtatcgt aggtggtcgg tgatagaaat tgttaggaaa tgtataagag 214500
   agaggttttc gtattttttt gatttgtgcg ttgttgagta ttattggttt tagttgagga 214560
   agagtttgga atttgttagg atagttgtga ggtggtgggg tttatttttt agtagtttta 214620
   tatgaggatt ttggaaatgt gtttttagta ttgttgggtt tttatattag gggttttatg 214680
   agtttatagt tgatttgata ttcggattag taagggttgt gttttgtttt tgttttttat 214740
   ttggtgaata gtattttgag gtattttttt tatttttaga aattatttta agtattgtta 214800
   atagaaatgt ggggtgtaat tgattacgtt atgaatttat ttaattttta tgtttttgag 214860
   aggcgggtga cgttattatt tatattttat ggatgaggaa attgagggaa tgagagtaat 214920
   ttgttcgagt tagggagtgg cgattatata ttttaatttt agtttgagag ttttattttt 214980
   tttgtcggtt ttcgttgttc gttagtgtta gatgagagtg ttgttggtta gtttgtagga 215040
   ggttttagtg tggatatcgt tttatatagg gtcgtggtag gagggtttag gtaaaggtaa 215100
   tcggtatagt attgagagta tttgagggtt gggtaaggtg gaggtttcgg ttttgtttgt 215160
   tggtttatta agtaaaaatt agatattttt aatttgggga aaagttatgt ggttttgcga 215220
   tggggtagtt tggagttagg tgtatgggtt ttgtatttcg ttttagtttt atgtattgtg 215280
   gagttgagat agatatagtt tttgcgtttt ttatgtttta tttttttttt tttttttttt 215340
   tttttttttt tttttttttt ttgagacgaa gttttatttt tttgtttagg ttggagtgtc 215400
   gtggcgtgat tttggtttat tataattttt attttttggg ttttagtgat ttttttgttt 215460
   tagttttttg agtagttggg attataggcg tttggtatta ggtttggtta atttttgtat 215520
   ttttaataga gacgagattt tattatgttg gttaggttgg tttcgaattt ttgatttcgt 215580
   gatttattta ttttagtttt ttaaagtgtt gggattgtag acgtgagtcg tggtgtttag 215640
   cgtttacgtt ttattttcgt aagataatgg ggataataaa ggaattataa gagatatttg 215700
   taggtgtgtg aagacgaagt ttattatatg ggagggtatc gggtgttttt atttgttttt 215760
   tttgttcggt tttttatttt tagtgatgtc gtttattgtt ttttttgtta aaaggttttt 215820
   tgttatataa tagttatata tgttaggtat cgcgtgttta tataggagtt acgaagtata 215880
   gtaataaaat gattattttg agatggttat tagttcgagt tcgagggtat ttgtgggtgt 215940
   tcgtggtttt ttgtttagtt tttttgagaa gtggttattg tttcgagttt gggtttgtat 216000
   gtattgtgtg aagtgtatat gtttttgagt aatgtttagt attgttgatt tttgagtttg 216060
   tgaatggtcg tttatagtac gtagtttttg tgtgtgtttt tgtgtgtgtg tgttttgagc 216120
   gtatataagg tggatggggt cgtgggggta atattttgtg ggttataggt tttttttaaa 216180
   attttagttt cgatttttgt tttattttat ttttagtagt ttagtttttt gtttgttttt 216240
   aggtttttgt tgggacgttt tgtttttttt ggttatttta gtggtgcggg ggtttattgt 216300
   tttggttttt ttattgtggt ttttttttat gtttttattt ggtttggtta ttgtgttatt 216360
   ttttattagt taggaatttt ttggagagta ggagttgagt tgtttaggag taggagttgg 216420
   tgttggttat tttagtattt tttagattga gggtagtgtt aggtgtttta tatttagttg 216480
   tagagtaggt ggtttttttg tttttttgtt tatggggttg tttttagatt ttgtttttgg 216540
   tattagtatt tttgggtagg gggagagagg tggggttcgg gtagagtttt tttattggga 216600
   tattaaagtt ttttttgttt ttttttttag ttgaaaatat tatatattgt gagtttgttt 216660
   atttgcggga tttttttatt aggtgagaga tagggtgttt gggtggggtt gacggtttta 216720
   tttttaagag ggttttatag cgggtggggg tagtgggtgt ggggtcgaag ttttgggtag 216780
   agtgggtgtt ttttgttatt tgtttgtttt gttttcggga agattttgga agtttatttt 216840
   ttgaaaaaga aaatttattg ggaaatgaag taggtagtga agattttttt taatttttgc 216900
   gaagttggtt ggatgggaag gttgagtttt gatttattgt ggtttggttc gttagagttg 216960
   ttcggtggtt attaagggtt tagtgaggtt ttatgtgtag gttttgtcgg tagcgtgggg 217020
   cgtttttagt ttcgatttta ttggtttttg atttttattt tttttatttg gttgaggttt 217080
   ttttttgttt tttttgagtt taggtaagga ggggaagttt tgatggagag ggtgttgggt 217140
   gggtggtttt tggagtgtgg gttttggttt gggtaggttt ttttttagta agtagttggt 217200
   atggatgatg ggaatgttat taagaggagg ggtttttagg ttggcggggg taggggtggg 217260
   gggatttcgg gagtcgtttt gttattgtgg ggatattggg ttttttaatt tttttttaga 217320
   ttattttttt tgagttgtag aggaatgaaa attcgagttt aggatttttt ttttttgggg 217380
   ataagggtaa tttagttttt tgttttaggt tatttgtttg ttataggtta tagttttgga 217440
   ggtcggtggt tatttattgt ttttattttg ttagtatttt ggatatcggt ttggagtaat 217500
   aatgttttag ttttgttttt ttgagtttat gtattgtttt tgggttttgg atttagggtt 217560
   atagggtgga tttagaggga gatatatgta ttggaatatg tgtgttttga tcgagtttgg 217620
   gtttattagg gggattgatt ttttttttaa aacgtgtatt agatttggtt taatatggtg 217680
   ggtttggggg attttatggg gagttaagta gtcgggatgg ggagtggggg tgggggtagg 217740
   cgggtttgag ttcgaggtag gtcgagtagg gtttttgttt cgttgttttt atttcgttgc 217800
   gtttatttta ttgattcgtt cgttttttat ttttatagga cgtatatgta gaatattaag 217860
   gatattatta gtagtattta tttcgaggcg tatcgtgtga agcgttttaa cgagggtagt 217920
   agcgttatgg ttaacggtat ggaggagaag gagttagaag tttcggagat gtagacgtta 217980
   ttttgtttat tttcgggatt ttgtttttaa gttattttcg ttttttttta ggttttttta 218040
   ttattttatt ttattttata tgattttttt tatttgttat cgttttttat tttttcgata 218100
   tgttgttagg aaataaggga aggggttttt tttcgagtga gttagtgatg aggtcgcggt 218160
   tttttcgagg ttgtggggag gttgtattgg agttataggt aggggtgaga gtatttattg 218220
   aattgatatg attttttgtt tttaggtttg gtttttcgag ggtttagaag agtagtttcg 218280
   gtgtgtagat tattcgtttg tgtggggttt ttagtgtcgg aggttttggg gtgggggtta 218340
   ggtttcgtat ttgtagagga gtttagtggg ttgtacgttt ttttttattt ttatcggttt 218400
   tgttttttgg agtgtgttag agtttagggg agaatgtagt ttattaggag tatttggatt 218460
   ttttgttcgt tatatggtgt ggttattatt tagtttttat ttttgttttg tttttaaggg 218520
   tagaaaattt tagggttttt tgttatcgat tgtttagtta ttttaagttt tttggtagtt 218580
   gttttttttg gagtagaaag tgtttttatt ttagttattc gtagattgtt tggttagatg 218640
   cggggatagg ttggaatgag ggaggcgttt ttattttttt gttatttttt ttttacgtta 218700
   ttttcgtttt tatcgggttg taggtgttgt tgatgcgttg ggatttgatt gaggataaaa 218760
   aggaaggaga gatgattttt atttttttat tttttagttt tgaaaaggtt taagtaagtg 218820
   agtttggtgg aggagttgag ggagggagtt atggaaggtg ttagaaggaa ggttggcggg 218880
   ggtacgtgtg ggtcgtggtt tgggttggtt agagtggcgt gagttgttcg gcgtttgttt 218940
   tgtttaagtg attagggaag tgtgtgtgtg tttatgtgta tgcgtgttcg tttgtttgtt 219000
   tagtgtttgg gtttggttta agattgggtt gtagttatat taatgtttag ttagttattt 219060
   ttgttattta ttttttttgg tttaggtttt gttgattttt tgagttgggg aggtgggagg 219120
   ttaggcgagt ttgattttgt tgatttattc gtgtttgggt tttttttttt agagtttatg 219180
   gtaacgaatt tttagaaagg agagaacggg cgttaggggt gtatagttta tagttgaaga 219240
   gtaaggtttc gtggtagtac ggttcggttt tttatttttt gtttttacga ggggatttat 219300
   gggggttgtt tttgtagggt atagatgatt aaagtttttt tttgtttttt gttatttgtt 219360
   ttgtttttgg ggagaaagag gggtttgatg agattttatt taggtgtata tattattagg 219420
   tgtatttgta ggtatcgggt tggttgtttg tagttaggag aaggttagcg agaaggagtg 219480
   tatgagtgtg agtgtgtgtg tatggaagtt ggggtattgg gcgtttgatt ttttttttat 219540
   ttaagagagg aaggattttt tattattttt attggtgaga tagtttattt tgttaatttg 219600
   ttatgttttt gttaaaatta agattttgaa ggaaatgagt ggttagcgtt agggtttagg 219660
   ttatgtggtt tgtttagttt taatgttatt tggtggcggg gtggggtggg ggtgggtagt 219720
   agtattttag ttttgagatg ttttattttt tttttttgta attagatttt gaaaaattgt 219780
   tcgttttatt aggttttgtt ttttaatggt tttttgttac gtgtttttcg agaaatttgt 219840
   ttttttgtat aaatgataaa gtgttgaaaa tgtatttttt gaaataaatg ttttaaatgt 219900
   agaaattta 219909
<210> 38
   <211> 219909
   <212> DNA
   <213> Artificial Sequence
<220>
   <223> chemically treated genomic DNA (Homo sapiens)
<400> 38
   tgggtttttg tatttgaaat atttatttta ggaaatatat ttttaatatt ttgttattta 60
   tataaaaaga taaatttttc gggaggtacg tagtaaaagg ttattgagga atagagtttg 120
   atgaaacgaa taatttttta aagtttggtt atagagaagg aaagtgaagt attttaaggt     180
   tgggatgttg ttgtttattt ttattttatt tcgttattaa gtgatattga ggttgggtag     240
   gttatatggt ttgggttttg gcgttggtta tttatttttt ttaaaatttt ggttttggta     300
   aaaatatggt aagttggtaa agtaaattgt tttattagtg ggggtggtga ggggtttttt     360
   tttttttggg tggggaggga gttagacgtt tagtgtttta atttttatgt atatatattt     420
   atatttatat attttttttc gttgattttt ttttggttgt aagtagttag ttcggtgttt     480
   gtagatgtat ttggtgatgt gtgtatttga gtggagtttt attaggtttt tttttttttt     540
   taggagtaag ataggtaata ggaagtagga agggattttg gttatttgtg ttttgtaaag     600
   atagttttta tgggtttttt cgtggggata gagggtgagg ggtcgggtcg tgttgttacg     660
   aaattttgtt ttttagttgt ggattgtgta tttttgacgt tcgttttttt ttttttaggg     720
   gttcgttatt atgggttttg aggggagggg tttaggtacg ggtagattaa tagagttagg     780
   ttcgtttggt tttttatttt tttagtttag agagttagta aggtttgggt taggaagggt     840
   gagtggtagg ggtggttggt tgggtattaa tgtaattata gtttagtttt gggttaaatt     900
   tagatattag atagatagac ggatacgtat atatatggat atatatatat ttttttggtt     960
   atttgggtag ggtaggcgtc gggtagttta cgttattttg attagtttaa gttacggttt    1020
   atacgtgttt tcgttaattt tttttttggt attttttatg gttttttttt ttagtttttt    1080
   tattagattt atttgtttag atttttttaa aattggggga tgagggggta ggggttattt    1140
   tttttttttt tttattttta attagatttt agcgtattag tagtatttgt agttcggtgg    1200
   gggcgggggt ggcgtgagag ggggatggta gggagatgaa gacgtttttt ttattttagt    1260
   ttgttttcgt atttggttaa gtagtttgcg gatggttgag ataaaggtat tttttgtttt    1320
   aggaggggta gttgttaggg ggtttggagt ggttgggtag tcggtggtag gggattttgg    1380
   agttttttat ttttaggagt agggtagggg taggggttga gtgatggtta tattatgtgg    1440
   cgggtagggg gtttaggtgt ttttggtggg ttgtattttt ttttgggttt tgatatattt    1500
   taggggatag ggtcgatggg gatggagggg agcgtgtagt ttattgggtt tttttgtaag    1560
   tgcgaggttt ggtttttatt ttaaggtttt cggtattgag aattttatat agacggatga    1620
   tttgtatatc gaagttgttt ttttgagttt tcggggagtt aggtttgggg atagagggtt    1680
   atgttaattt agtgggtgtt tttatttttg tttgtggttt tagtgtagtt tttttataat    1740
   ttcggggagg tcgcggtttt attattgatt tattcggagg gaggtttttt tttttgtttt    1800
   ttggtagtat gtcgaagggg tggggaacga tgataaatgg agaaaattat ataaaataaa    1860
   atggggtggt gggagggttt ggggggggac ggaaatgatt tgggggtagg atttcggggg    1920
   tgggtagggt ggcgtttata ttttcggggt ttttggtttt ttttttttta tgtcgttggt    1980
   tatggcgttg ttgttttcgt tgaggcgttt tatacggtac gtttcgaagt ggatgttgtt    2040
   ggtgatgttt ttgatgtttt gtatgtgcgt tttgtggggg tggggggcgg gcgggttagt    2100
   gaggtgggcg tagcggggtg ggggtagcgg ggtaggggtt ttgttcggtt tgtttcggat    2160
   ttaggttcgt ttgtttttat ttttattttt tatttcgatt gtttggtttt ttatggggtt    2220
   ttttaggttt attatgttgg attagatttg gtatacgttt tgggggaagg gttagttttt    2280
   ttggtaagtt tagattcggt tagaatatat atattttagt gtatgtgttt ttttttgggt    2340
   ttattttatg gttttgggtt tagggtttag ggatagtgta tagatttaga gggatagggt    2400
   tggagtattg ttgttttagg tcggtgttta gggtgttggt agggtggaag tagtgggtga    2460
   ttatcggttt ttaggattat ggtttgtggt aggtaggtgg tttggggtaa agggttgggt    2520
   tgtttttgtt tttaggaaaa aaaagttttg ggttcggatt tttatttttt tgtagtttag    2580
   gaggaataat ttggagggag gttggaaagt ttagtatttt tataatggta gaacggtttt    2640
   cggaattttt ttatttttgt tttcgttaat ttggaggttt ttttttttga taatattttt    2700
   attatttatg ttagttgttt gttgaggaag aatttgttta ggttagggtt tatattttag    2760
   ggattattta tttagtattt tttttattag ggtttttttt ttttgtttgg atttaaggga    2820
   aataaggaga agttttagtt aggtgaaagg ggtaggggtt agaggttagt gggatcggag    2880
   ttggagacgt tttacgttgt cggtagggtt tgtatatgag gttttattgg gtttttagtg    2940
   gttatcgggt agttttaacg ggttagatta tagtggatta aagtttagtt tttttattta    3000
   gttaatttcg taggggttga aaaggatttt tattgtttgt tttatttttt agtgggtttt    3060
   tttttttaga agatgggttt ttaagatttt ttcgagggta gggtaggtag gtggtagggg    3120
   gtatttattt tgtttagggt ttcggtttta tatttattgt ttttattcgt tgtagggttt    3180
   ttttaggggt gaagtcgtta gttttattta agtattttgt tttttatttg atgagaaggt    3240
   ttcgtaggta ggtaaattta tagtgtgtgg tgtttttaat taggagagga gataggaggg    3300
   gttttaatgt tttagtggag aaattttgtt cggattttat tttttttttt ttgtttagaa    3360
   atgttggtgt taaaagtaga gtttgagggt agttttatgg gtaggagggt aggggaatta    3420
   tttgttttgt agttgagtat aggatatttg gtattgtttt tagtttgggg gatattggag    3480
   tgattagtat tagtttttgt ttttggatag tttagttttt gttttttaga aagtttttgg    3540
   ttaatgggag atggtatagt gattaggtta aataggagta tggaaggaag ttatagtgga    3600
   gaagttaggg tagtgggttt tcgtattatt ggggtgatta gggagagtag gacgttttag    3660
   taggggtttg aagataggta gggggttggg ttgttgggaa tgggatgaag taggaatcgg    3720
   agttgggatt ttaaagaaaa tttgtggttt atagggtgtt atttttacgg ttttatttat    3780
   tttgtgtgcg tttagagtat atatatatag ggatatatat aggggttacg tattgtggac    3840
   gattatttat aagtttaaga attagtaatg ttaaatattg tttagggata tgtgtatttt    3900
   atataatata tataagttta agttcgggat agtggttatt ttttagggag attgggtagg    3960
   agattacggg tatttataag tgttttcggg ttcgagttgg tggttatttt agagtggtta 4020
   ttttattgtt gtgtttcgta atttttatat gaatacgcgg tgtttggtat gtgtaattgt 4080
   tatataatag aaggtttttt aataaggagg gtagtgaacg gtattattgg aggtggggag 4140
   tcgggtaggg aaggtaaata aagatattcg gtgttttttt atgtgatgaa tttcgttttt 4200
   atatatttgt aagtgttttt tgtgattttt ttgttatttt tattatttta cgaaaatggg 4260
   gcgtagacgt tgggtattac ggtttacgtt tgtaatttta gtattttggg aggttgaggt 4320
   gggtggatta cgaggttagg agttcgagat tagtttggtt aatatgatga aatttcgttt 4380
   ttgttaaaaa tataaaaatt agttaggttt ggtattaggc gtttgtaatt ttagttattt 4440
   aggaggttga ggtaggagaa ttattggaat ttaggaggtg gaggttgtgg tgagttaaga 4500
   ttacgttacg gtattttagt ttgggtaaaa gagtgaaatt tcgttttaaa aaaaaaaaaa 4560
   aagaaaaaga aaaagaaaag aaaagaaaat gaggtataga gaacgtaaga attgtgtttg 4620
   ttttagtttt atagtgtata aggttggagc gggatgtaga atttatatat ttggttttag 4680
   gttgttttat cgtagagtta tatggttttt ttttaagtta aaaatgttta gtttttattt 4740
   ggtaggttag taggtagggt cggggttttt attttgttta gtttttaagt gtttttagtg 4800
   ttatgtcggt tgtttttgtt taggtttttt tgttacggtt ttgtgtaggg cggtgtttat 4860
   attagaattt tttgtagatt gattagtagt atttttattt aatattgacg ggtagcgaga 4920
   gtcggtagga agaatggggt ttttaggttg gagttggagt gtgtgatcgt tattttttgg 4980
   ttcgggtaag ttatttttat ttttttagtt tttttattta taaaatgtgg ataataacgt 5040
   tattcgtttt ttagggatat gaggattaaa tgagtttatg gcgtgattaa ttatatttta 5100
   tatttttatt gataatattt ggggtggttt ttaaggatgg gaaagatatt ttaagatgtt 5160
   gtttattaag tgggaggtaa gaataaggta tagtttttat tagttcgggt gttaggttag 5220
   ttatgaattt atggggtttt tgatataaaa atttagtagt gttgaaggta tatttttagg 5280
   gtttttatat aagattgttg gaaagtgggt tttattattt tataattgtt ttgataggtt 5340
   ttagattttt ttttagttgg agttagtggt gtttaataac gtataggtta gagaggtgcg 5400
   gggatttttt ttttgtgtat tttttgatag tttttgttat cggttatttg cggtatttaa 5460
   ggttggttat tagggtgtag tagaggtacg tttatagtaa agtcgtcggt tcgttttagg 5520
   tttatttatt tttaattttt ttaaatttgt tttttataaa aagtttttta aatagtaaga 5580
   atttttgggg tagagaggaa gagaggaatt gtaggtagag gggttaaggg gaggtaagtt 5640
   agtttgatat tataggcgga tattggaaaa tatttaggtt gtatttagtt gggtagtttt 5700
   cgtagggtgg gttcggtagg ggtggtggta ggggaggagt gtagataggg gaaaggttcg 5760
   tgtgtttttt tatatttttt tttaggtatg aatttttatt tcgagggagt ttggtgtagg 5820
   gttggagttg tagattttag agtcgttttt cgttttgggt tttttggtag gttttttgtt 5880
   tatagtattt ttttaatttt gtaaggaggg tgggtgcggg gagtttcgga tatgaaacga 5940
   gataatttat gtttagttag tggtatagga gtgtttgatg ttttttggta attgttatta 6000
   tgattatttt tttagagtaa tagtttttta aaaagttttg tggtttttgg gatagttttt 6060
   taatttatta aaattttggt atattgtatt tattttgtgg atgatgaaaa ttgaggtttt 6120
   ggaagattaa gagatttgtg ttttttgttt tattgggttt tttagttaag tgttttttta 6180
   gaataaagtg gttttttttg ttgtattttt ttttgtattt tttttttttt tattttaatg 6240
   agaaatgaag ggatagagag aggaagggga aggaaacgtt cgggtttatt tttttcgggg 6300
   tgttatcgtt aggagtgggt tgaattgtgg ttttttaggg gtttttattt atttaaagat 6360
   atggttaagg tttaggtttt ggaatttatg attgtgattt tatttggaaa acgttttttt 6420
   ttatagattt attaaggatt tttaaatggg attatttatt tagggtaggt tttaagttta 6480
   aagataggta tttttgttta aagataagaa ggaaagaggg taggttcggt ggtttatttt 6540
   tgtaatttta gtattttggg aggttaagta aggtgggtag attatttgag gttaggagtt 6600
   taagattagt ttggttaata tggcgaaatt ttgtttttat taaaaatata aaaattaagt 6660
   taggtgggtg gtacgtgatt gtaattttag atattcggga ggttgaggta ggagaattat 6720
   ttgaatttag gaggtagagg ttgtagtgag ttgagattgt aatattgtat tttagtttgg 6780
   gtgatagaac gagatttcgt tttaaataaa aaaaaaaaaa aaaaaaaagg aagaggaggg 6840
   acgggataga gatataggga agaaggtttt gtgataatgg ggtagagatt agagtatgtg 6900
   atgtttttat aatataatta ggaatgttag gcgttattag aaggaggaag gtatggatga 6960
   ggttggtttt tagggtttta gaaggaaata gtttttgatt tcggattttt ggtttttgga 7020
   attgtgaaaa tacgtttgtg ttgttttaag ttttttcgtt tgtgttaatt ggttataata 7080
   gttatttaag agattaaggt gtgagggttt taggaatttt agtagggtaa ggggtcgagg 7140
   gtttatttgg tttatagagt tatagtttat ttttttttta ggtttagaga ggttgggcga 7200
   tttgtttagg gttatatagg tgtttatgaa agagtttgta ttaggattta gaatttggcg 7260
   gtttttattt tagtttttgt ttttttgaat ttttttttgt tcggggggag gtttggggtg 7320
   gaggtatttg tataggttat ttgttttttt atttttttaa gggttgaatg gggataagga 7380
   gatttttttt tttgggtggg gtaaggtagt ggatgaggtt ttacgagttt ttggtttagg 7440
   cgtggtttat aggttttggg ttagtagaag agattgtttt tttatttgtt tggagtttcg 7500
   gcgtttgcgg cgagtatttt cgatggtatt ttatttggtt ttttttttaa ggattttttt 7560
   gtcgttgatt tggtattcgt ggttattgtt tattatagta aatgggatta ttttttgggg 7620
   aacgggtaga cggtattggt tatatatgtg cgggaaggta attttttttt ttgggttttc 7680
   gggaggagtt gttttgagtt tggggttttt taaaaacgag agttttagtt tgatagtatt 7740
   gggtatagat atttgggttt agtgtttttt ggttttaaga atataatttt atggtttaag 7800
   gttttttaaa tttttaattt attttttagt aggaattggg ggttttttgg gaataatatt 7860
   ttagtggatt tatttattcg gaatttttcg tttattagtc ggtttttcga gtttttatta    7920
   aatttttttt gggggtatac gtcgatgtcg ttggatagta ggtttgcggt gatttgggat    7980
   aaggagggag agagatgggt ttatttttgt ggtaagtttt atttatttat tggtgtttgt    8040
   tatatgattt ttatattgtg tgttgtttta ggttcgggtt taagggtagg ttttgggggg    8100
   ttgtgatatt aattattttt aatttttgtg ttttttggag ggaagagggg tagttgtagg    8160
   tgtatttgtt gttttgagcg acgttgtaat tttttagggg tttaaggtta tcgttttgag    8220
   tattttttat tttttttttt ttgtttatta tagttttaag aaagtattta gggttatcga    8280
   gatttttttt tttgaaattt tttttaaaaa attttcgtta tagtttttag ttttagtagg    8340
   ggtttcgttt gtagtaattt tttagttttt tgttagtagt tcgatatggg tacgtgtaat    8400
   agaaatattt tagtgagcgg ttgttcgttt gtttaggtat agattatagt tcgtgtgtag    8460
   ttttcggtag atatatttat tttgtgtgga ttgttttttt ttgtatatgt tgggaaggtt    8520
   tttgatattt ggttttagga cggagtaggt tataggtttt atttatattt atcgaggtag    8580
   atgtggtgtc gtagtttagg tatttcggtt ttattttgaa aggtatttta tgtggttttg    8640
   atgtgttgtt ttgttaggag gaagagttta gggttgtttt tttgtttgga tgtttttttt    8700
   tattttatag taggggtttt agacggtggc ggagaagaag gtggtagaga ttattttttt    8760
   tgaaattttt tttttttttt ttttttttga gatggagttt cgttttgtta tttaggttgg    8820
   agtgtagtgg tttaatttcg gtttattgaa gttttcgttt ttcgggttta agagattttt    8880
   ttgttttagt tttttaagta gttgggatta taggcgttcg ttattatgtt cgattaatat    8940
   ttttgtattt ttagtagaga tggggtttta ttatgttggt tttaaatttt tgattttaag    9000
   tgatttgttc gtttcggttt tttaaagtgt tgggattata ggcgtgagtt attatatttg    9060
   gtttagtttt ttttttattt aataaaaatt aggtagggcg aggtgaggtg gtttatgttt    9120
   gtaattttag tattttggga ggttgaggta ggaggatttt ttgagtttaa gggttagaga    9180
   ttagtttggg taatatggta aaatttcgtt tttattaaaa atataaaaat tagttagata    9240
   tggtggtgtg cgtttgtaat tttagttatt cggggattga gtttgggagg tggaggttgt    9300
   agtgagttat gattgtgtta ttatattgta attttggtaa ttgagttaga ttttttttta    9360
   aaaaaaaaaa gggggggtat aaaataaggt gggttatgtc gtacggtttt tgttcggttt    9420
   tattttttgg aagagagatt tattatttgt ttggtttagg ttttttttaa aatttttttt    9480
   agagattttg ttggagaatt ggtattttta taaagttgtt tttgagatat gggggagttg    9540
   gagaggttat tttagggacg gtttttagta gttatttggg ttagggatta ttaggttagt    9600
   tattcgttgg ggagagtgat tagcggttcg gtttttttga tagtgtgatt tttttagacg    9660
   gggtggtttt tgttaggttt gtagcgtttt taggttgtat agaagggttg gggtaagtag    9720
   agatggaatt ttattcgttg tttgaagtgg attttttttt ttagggtgag tgtgttcgtt    9780
   ttggcgatga tagggacgat gttgattatt ttgtttaggc gttttataaa ttcgatgttt    9840
   aggggtttga ggttgaagta gagggatgtg cgtgaggttt atttggggga gcggggtagt    9900
   gggggggtat ttatttttaa gtaggtgtcg atatttttag gtgttggttc ggggtttacg    9960
   ggggtttttg ttggtaagga tagggtttcg ggtcgagtcg gttacgttgt gagtgtgggg   10020
   tagaggaagg atacgggtgt gttttagata taggtgatat tattgtatag gttaggcggg   10080
   ttagggttag ttttaagtat tttcgtttat gagttttggg gttttaatta tatttttata   10140
   tgtttatata gaagggttgt cgttggttta gtagtcgttt tgttgtgggg ttttggttgg   10200
   gaggggagag gtaagagaaa tagaagttag tattagtgtg cggtgttttt gtattaggcg   10260
   ggttgttttt ttgtattagg cgggttgttt tttttgtttg tttggtttta gtgagtttgg   10320
   ggtttgatgt ggggaaagtt gtgggtggta ggggttggta gggttttgga attggggtat   10380
   tatttttttt agttttggag tcgagttttt tttatagtga gaataaatcg gttatatata   10440
   tggttgggga gaagttcgtt tcgggtagaa gtttaagtag agatggttaa ggttcgtttt   10500
   tgttttatgg gattggacgt tttgggcggg attaggtaat atgtttagtt ggagttttag   10560
   ttaaagggtt agtgttgggg agtggtagaa gtgttataga tcgttcgttc gttttcggtt   10620
   tgtttagttg gtattaggag aggtttttgt tttttgttat tagttttagt tgaggtaaag   10680
   tttggagtag ttggaatagt tgttatagga tcgtaggtga gtcgttttgt ttgggagggt   10740
   agatgtttag tttaggagag gtttatggat agcgggttgg ttttttgttt ttgatttttg   10800
   ttggtttata gaggatgatg ttagagggtg ggttcgaaaa tttcggtttg ggttttttta   10860
   tagattttgg gtgaggttcg ttgtttatta gttaggtttt tttttgtatt tggtgtttat   10920
   agagtatggt ttttttaaat ttttgggttt ttttcgttta gggaagtata tttgggatag   10980
   taggtgcgaa ggattttttt tatttattta tgtagataag ttatttacgg ttgtttttat   11040
   tagttatttt tgttggtttt tggttttttt ttttttagtt ttggtttagg gtgagttata   11100
   ggtgtttggt agggggtgta tagggagttg aggatagagg gttcgtgagg ttatgaagag   11160
   tttatttgtt agggtatatg tagtttttcg tttttgttga tttttagggg cgacgttagg   11220
   tgcggataga gtcggcggga ggaaagggtt tttattaata ttaatagtag tttcgtttag   11280
   ttttggaagg ggttagtatt gatttttgtg tttttcgttt aagaacgtat taaatttttt   11340
   aaggggtttt atgaggtagg gttcggatga gaaggttagt tatcgttacg ggtgtttggt   11400
   tgggggtttt tattttattt gttagagttt ggtttgagaa ggtaggatat agattattag   11460
   gttttagaat agtttagggt ttgtttgatt tttagttttt taagacgtag ttgaaggatg   11520
   ggggttgggt aggatatttg tataggggtt agtgtgaggt tttggaggtg tagatatggt   11580
   atatttttaa ttttatagtt ggcggttatg gtttttgatt tagttttttt gtatttagta   11640
   gtagggtaga tagtatcgtt ttgtagtttt aggggataga gggaatttag ggtattcggt   11700
   atgaggacgt cgatattgta gggacgtacg agtggtcggt ggcggggatg aagtagaggt   11760
   agtagtggac gcgggtgttc gggatgcgtt ttttgcggtt gatgttgatt tttttttgta 11820
   ggtatttttc gtattggtta ttgatgaatt ttatgatggg ttgttagttg cgtgggggat 11880
   ggggacgagt tagtaggtat atgagatgtt ttttttaagt aggtcgtgta tttttaggat 11940
   ttagagttta atattttatt ttttaatgaa atgtatgttt tgggaggttt ggtttgttga 12000
   gcggagatgt tatagtttta ttttcggttt tttaaattag ttttttcggg ttggttttag 12060
   gtgatgagtt ggggatagta agatttttta ggtaattttg ttttggttta gaatttttag 12120
   gtttttgaga cgtgcggttt tggtagtatg ttaggtttta ggaggtgggt tttgtatttt 12180
   cgcgggtcgg gaaattttta gggttgattt tgggtggggt ttagggagtt ttgaagtttt 12240
   cgttagtgtt tttttttttg gaggggtttt attagttttc gttgttgatg tggttttcga 12300
   attttggtgt gttaattatt gttagtttta ttcggacgtt ttttttttta atatttgtat 12360
   agagttacgg acgtagagtt agaggggaga cggaagattt ttatagatat tttatatttg 12420
   attgatttcg gggtaatttt aggtaggtag cgatgataat taaggagata tcgggaaatt 12480
   aatggttttt tttagttaat ttcggtatag gtcggtgacg gtttttttgg gagttttttg 12540
   tttgttagat ttttggtgga aaatttagtt gtttgtgggg agtttagtgt gacggggttt 12600
   ataggggttc gggatattga tttattatag ttagagtttt aatatgttta atatgttggg 12660
   gatggtgggt atagcgagtt ttcgttttgg ggatttggga ttgtttgatt cgggtatttt 12720
   tatatcgtat tagtgtttta ggaaggtatg ggtattttat atttttagtt tatttttagt 12780
   tattgatcgt gtgtgatggt tttgattttt aatttttgtg ttttaggaag gtaggggcgt 12840
   tttgtatttt tagtttattt tcggttattg attgtgtgtg atggttttga ttttttattt 12900
   tgtgttttag gaaggtagag gagttttgta tttttagttc gtttttggtt attgattgtg 12960
   tgtgatggtt ttgattttta atttttgtgt tttaggaagg taggggcgtt ttgtattttt 13020
   agtttatttt cggttattga tcgtgcgtga tggatttgat ttcgatggtt ttggggatgc 13080
   gtttttttga ggtgggttgt atcgattttc ggttgatttt ggatttgaag agggtgttga 13140
   ttaaggtgga tttatttaag tcgttttgtt ttggggagag gatgggaggt cggttagggg 13200
   ttttttcgga gagattagta gggtttgtat atggggtttt ttttatttag gtacgaggtt 13260
   ttttagtttg agatttagta gtgtttgggt tatttttcgg taatattttt gttaagtagt 13320
   tttcgaggat tttattttcg agtcgggtgt ttagtgattg tgtaaattta tatagtcggg 13380
   ttttgatgtt agtataattt aggttatagt ttttttttga gaggtgagtc gggtttggga 13440
   gagtcggtat tttttgtgtt ttttgtgttt tttggatagg aggttttttg gtggtttaga 13500
   ttttggatag gatagaggtc gggttggcgg aggagtttag ggttgtttag ggtgtattag 13560
   gtggtcggga tttttgtagt ttattagggt ggttgtaatt aaagtttagg tgtttaaatt 13620
   gttttttttt gatcggttag ggtttgggta agttatttga ttttatcgtg ttttagtttt 13680
   ttgatttgta aaatgggata aatagttttt ttttcgtgga gttggtgtga gtattaggta 13740
   ggtttacgta tgttttatgg ggtttgtggg gttatgtgtt agggggcgtt ggtttgtagg 13800
   gtggtgttat tcgagggata gtgatcgtgt gtgtagatag ttagtttata ggtgattgag 13860
   ataaacgagg atgaggaagt agtatttata tgtaatataa tatatataat ttatatatat 13920
   tttatatata tattttataa tatatatatt ttatatgtat aatttataat atatatataa 13980
   tttattttaa atatattttt tatatatatt ttatatataa tttttataat atatataaat 14040
   aaattatata taatacgcgc gcgcgtgcgc gtatatatat atatatatat atatatatat 14100
   atatattttt ttagattcgg agttttattg ttttattttg tatagagttg ttttgggtta 14160
   cgttagtaat atattaaaaa aaatatttta ggttaggtgt ggtggtttat gtttgtagtt 14220
   ttagtatttt gggaggttga ggtaggagga ttgtttgagt ttaggagttt aagattagtt 14280
   tgggtaatat agagagattt tgtttttata ttaaaatgta aatattggtt aggtgtagtg 14340
   gcgagtattt atagttttag ttacgtagga ggttgaagta ggagaattgt ttaagtttag 14400
   gagtgcgagg ttgtagtgag ttaggattat tgttatttta gagtgataga gattttgttt 14460
   tttaaaaaaa taaaaatcgg ttaggcgaga tggtttacgt ttgtaatttt agtattttgg 14520
   gaggttgagg agggtggatt atttgaggtt aggagttcga gattagtttg gttaatatgg 14580
   tgaaattttg tttttattaa aaaatataaa aattagttag gtatggtggt atatgtttga 14640
   ggtacgagaa ttatttgaat ttaggatgtg gaggttgtag tgagttaaga ttatgttatt 14700
   gtattttagt ttgggtaata atgagatttt gttttaaaaa aatataatat aaaaaatatt 14760
   ttaaattata ttgatgtttt gattttgata atattttttt aggaaaagtg tgggtttttg 14820
   ggaggataga ggttggtgtt gggaattttt ggtttgaatt gtttagtagt ttaaggacgg 14880
   atatttttcg tttatttttt tttgtttatt aggtttgtgt tttggttaat tttaggttag 14940
   gagttatgtt aatgttttag ttacgatatt ttagatttag gagatgttaa ttgggatatt 15000
   gtaaggtgta tttttttatt attgttattt ttattattat tattattatt attattattt 15060
   ttattattat tattattttt attagtatta ttattataat attattattg ttattattat 15120
   tattttatta ttttcgttat tattattatt ttcgttattt ttattattat tattattgtt 15180
   attataatta ttagtattag tattattatt aattatatta ttattattat tttattatta 15240
   ttattattaa tattattatt atttttatta ttattattat tattattaat attattattt 15300
   ttattaatat tattattatt tatattatta ttataattat tattattata attattatta 15360
   ttattttttt tattattatt ataattatta ttattattat tattattata attattatta 15420
   tttatattat tattattatt tttattataa ttattattat tatttatatt attattatta 15480
   tttatattat tataattatt attattattt ttttattatt attattatta ttattattat 15540
   aattattatt attatttttt ttattattat tataattatt attattattt tttttattat 15600
   tattataatt attattatta ttttttttat tattataatt attagtatta tttttattat 15660
   tattattata attattatta ttattattat tattattatt attattataa ttataattat 15720
   tataattatt attatttata ttattattat tataattatt atcgttatta ttattataat 15780
   tattattatt atttatatta ttattattat aattattatt attatttata ttattattat 15840
   tataattatt attattatta ttataattat tattattatt tatattatta ttattataat 15900
   tattattatt atttatatta ttattattat aattattatt attattttta ttataattat 15960
   tattattatt tttattatta ttattattat tattattata attattatta ttataattat 16020
   tattattata attattatta ttattattat tattattatt attattataa ttattattat 16080
   tattattatt ataattatta ttattattat tattattatt attattatta taattattat 16140
   tattattatt attattatta ttattattta tattattatt attattttat tattgttatt 16200
   attattatta ttattatttt tattattttt atgattatta ttattattat ttttattatt 16260
   attattatta ttattataat tattattatt tatattatta ttattatttt attattatta 16320
   ttattattat tattattatt tttattattt ttattagtat tattatttta ttattattat 16380
   cgttattatt atttttatta tttttattat tataattatt attattattg ttgtaaattt 16440
   tcgaataatt ataatttgtt ggggattttg ttcggggttt agttcgtttg attttatttt 16500
   atgggatgat tttattttat ggacgaggaa gttaaggttt atagaggagg agtttagggg 16560
   aagatatata agttttattt cgttgagtta ggataaagtt gggatttttt tcggggtttg 16620
   tggattttta aataggtgtt tttttatttt gtttgtattt ttgtaggtgg gtatagttgt 16680
   tatgggaggg aggagggagg agagtagagt tgttttacgt tttgtttttt attcggtatt 16740
   tttgggaggg agtgattttg tggggaatta ggattattgt tttttttagg gttgaaaggg 16800
   cgaagaggtt tagataggtg aagtggtttt ttgcgtatta tttttagagg gtaaggttgt 16860
   tatagtggta gttacggaag gcgagggttg ttgggttgtt tttttttttt tttggggtta 16920
   aagtttcgga agaaggtttg tgttatggtt gggacgtaga ggtttttagg ttttttttat 16980
   tttgtttttg gttagggagt agggtagttt tttcgtagtt tatagagatg agttttagtt 17040
   tggttattgt ttttgggttt tttttttttt ttttttttta gttatttttt cgggaggtag 17100
   ttttgaattt ttagggtata agtacgtttt ttataatagg tagaataggg tgggggtcgt 17160
   aggaggggat tttatatttt cgtttttttt tgaagtaggt tgttagtttt taggtagttg 17220
   tgagtatagt agggttatga gtagtcgggt tttatgaggt tataggaagt agagaggata 17280
   ttaagatttt aggggagtta gggtttagat tagtgtagaa gaggaaagaa aggtgtcggg 17340
   ttggtaggtg tgggtttgcg tttatagagt tgggtaggaa ggacgtgtac ggttggtaaa 17400
   ggttagagaa ataggttcgg tttgagaggt gtttagatat tttgtagaaa tttcgtaagt 17460
   ttatttttgt ttagggtgtg atttggcggg ggtatttttt tagatttttt tttggatttc 17520
   gtggagtggg tagaatatag ttttgggagt taaaacggtg gttgcggttt tttgttttgt 17580
   gagttaagga attttggtag ttttttttga gtttcgtttt tgtttggaaa acgggacgtt 17640
   ttttttatag ggtttttgtg aggtttgaat gggatttggg gggttttgga aataataggc 17700
   ggtttattgt agattttagt ttggtttttt tattttgttt aggtcgtgtt gatcgacgat 17760
   ttttcgtggt gatttttagt ttttcggggt tattaggggg tgtggaagtt acgaggtttt 17820
   aattttaggg tagagttgaa gttgagttgt tgggaagttt ttggtcgggg tgcggtgggg 17880
   tgcggtgggg agtttggagg gttaggttgg agtaatagtg aggggatagt tgttatagaa 17940
   ttttttgtat aagttgacgt tattgtttag aggtagcgat tatttattag gatttgtttt 18000
   aggtcggttt gggtttttta aagttatttt aggggttttg gatgtgtagg ataggttttt 18060
   ttgaggagtc gttggaggtt gtgggttttt tttaggcgta tgtatgtata cgtaatgtta 18120
   ttggtaattc gtttttgagg ttagggttgt tgttttattt tatagaaggg aagattaagt 18180
   ttaaaggttt gtgggtcgtt ttgggggttg aggttggttt tcggtattgg ttgagtggta 18240
   tgtaaggtga ggatttatcg attattatga tgttgaattc gaagttttgt tttatggttt 18300
   ttcggcgtat ttgttttagg atggagttaa tttttacgta gtcgaagttt atcggggttt 18360
   tttcgtttcg tgatgtaggc gtttgtttga gttcggtatt tttgggggtg tcggttatgt 18420
   cgttaacgta gttgggagtc gttttagtgg ttggggaagg aatagtagag gagttagagc 18480
   ggaatagggg tttatatata ggcgtttgtt aggtggttgt tatatttatt tttggatggg 18540
   gaaattgagg tattgaggtt tagaaaggtt aagcgatttg tttaggcgat ttttgagaat 18600
   tttttcgggt ggatgttttt ggtcgtggtg ttttttaaaa agttgttatt ttgtagttac 18660
   gttgtttttt tagtaaagtt ttatttttag atttttaagg ttaggttttt ttattttttt 18720
   ttttaggagt tgtgttaagt tgggggtttt ttgcgtttta aggtagggtt gattttgaat 18780
   ttttgttttc gtattttgga gggttgttta gataagggtg gagggtgttt tttttttttg 18840
   gttttttata ttgtttcggg ttatttaggt ggatttggtt ttttttttta tttttattta 18900
   gataagttgt atcgtttgtt gttatttaaa ggttttagaa gtcgatttac gagttaagat 18960
   ataggaggga tggttttgta agaaacgggg atagaattag cgtgtgtttt cgttttttat 19020
   atttttatgg atggttttgt aagaaagggg gatagaatta gcgtcggttt ttatggagtt 19080
   gtgtgtttaa ttttatagaa cgagttagta gttttgggag aaggaaaagt gttgggtgta 19140
   gggcgggtgt ttggatgtag agttgttttt gggtttatgg aggagggggg tttttgtatt 19200
   atatttgttt ttttttggat tgatttaggc ggtcgttttg tgtgtttggt tagcgtttac 19260
   gggttgtata tttataggtt aagattaagt tttatttttg ggtatgtggg gtacgtggag 19320
   ttgaattttt cgaaattatt aaagtttttt ttgtttgtag gttttttcga tggttttgtt 19380
   tttggttgtt gttaagatat tatatacggg ttagaggttt ggggttgggg gcggacgttc 19440
   ggaagacgtt tgttgaggat ttaggtgttt ttatatagtg gggttttagg ggtatttagg 19500
   atggggttgg tttttattag ggttggttgg tatacgggtt aggttttagt atattttaga 19560
   ttcgttatat ttattggtgt atgcgtatgt aggtgtagat aattcgttgg ttatagaggt 19620
   ggtgtttttg ttagttttta taggttggag ttgtttttgg tggtttttag tttttacgta 19680
   ggttgggcgg ttgttgtttt gtgttttcgt ttttgtttgg gtttatttag gttttgtttt 19740
   gtttgcgggg agatttttgt tttattttgt tttttaggga ggatttggtt ttaggtattt 19800
   tgcgttaaga tagggattat acggtacgtt tttaggtagg gtttttttcg ttagtttttc 19860
   gggaggggag tggttttttt ttgggatcgg tgggtattta gtaggggtag ttatagggga 19920
   gtggagtcgg cgtgggagtt agggttatag tacgggaggt tgtagggggt ttatttttcg 19980
   tgataggtaa gtgagtagtt tttttagata ggaggtagtt ttttcggttt acgtttattt 20040
   tttattttgt ttagaaggtg ggaggtaggt ttttaggtta ggaggggtag tgggagattt 20100
   tagtttaatt gtttttttat ttgcggggta agaatcgaat ttaggaaatg tttattgagt 20160
   tttttggttg tagtttgtat ttttagtcga tttaagttat ttttttcggt ttattttatt 20220
   ggagcggtta gagggtgtcg gagggaggtt ttttaggttt gaggtattag aggtgagaga 20280
   ggaaaagatt ttggaagtta gaaattgggg gttggtttat ttttataggt attttttcgt 20340
   ttttttagtt tttatagggt ataggaattt ttattttatg gaattggggg aataaaggaa 20400
   taatataatt ttttgttggg aaatgttaag ggggggttta tatattttta ttgttgagag 20460
   taatatttat aggcggattt ggaggttagg gttttttttt ttgtgttttt acgtggtttt 20520
   tatttaggtt acgtagtttg gaagttttta gggttttgtt tttgtttttg tttttttttt 20580
   agaaatattt ttttttgggt ttttaggatt tttaaataaa taaagatata gttttgtatt 20640
   tggttatgga ttatgtggtt ttggggtcgt tttttgaagt tagatatggg gttttgtttt 20700
   gcggggtagt agtttagttt ttatttacgt gatcgaaaat cgtttttatt tgtttgtggg 20760
   tttgagagtt attttgtttg ggattgggat tagtttttat tcgtttttat ttttttttag 20820
   tattttttaa ttttttttag taggatttgg ggggaggcgt tgttttttga attttttttt 20880
   ttcgttttta acgtttttgg agtttagagc gagggagggg tcggtttagt tgtagttggg 20940
   tttttttata ggtattagta tttattgtag aaatttaaat taaattaaat attttgttta 21000
   ttaggggttt cgtttgagaa ataataatat aacgtagtcg agttttattt ttgttattga 21060
   cgtagggtag tggtttgttt tttgtatagg tgagagttat ttttgtaatt tttttttcga 21120
   gaggcggttt tgtaggtttc ggttggttcg taggtaggta cgtacgtaga gagtaatgta 21180
   agagttcgtt tcgttttttt atttggttag gtgagttttg gggtttgttt ttaggatttt 21240
   taagtgggaa aagtagagat ttttatattt agagatattt ttattttggt tttaggatat 21300
   tatggtgttt agatagttgg aaataatggt aaagattatt tattttattg tttgtgatta 21360
   cgatatcgtt cgaggtttgc gttgtttagc gtggtagtta ttggttacgt gatttttttt 21420
   tttaattgtt ataacgtttg ttacggttat tattttgaag tgtgtagttt agtggtgtta 21480
   agtatattcg tattgttgtg taataaattt ttagaatttt tttattttgt aaaataaatt 21540
   gtttttatta aataattttt tagttttatt ttttaagttt ttggtaattt ttattttgtt 21600
   gtttttttga atttgattat tttacggatt tggtataagt ggaattttat aggattgttt 21660
   ttttgtgatt aaattatttt atttagcgta atattcgaaa ggtttatttt tgttgttgta 21720
   ggtattttaa tttttttttt acggttgagt taaatttttt gtatgtttta tttttttttt 21780
   tttttttttt agatagagtt ttgtttttgt tgtttaggtt ggagtgtaat ggcgcgattt 21840
   cggtttatcg aaatttttgt ttttttaggt ttaagtaatt tttttgtttt agttttttaa 21900
   gtagttggga ttataggtat tcgttattat gttcggttaa ttttgtttta gtagagacgg 21960
   ggttttttta tgttggttag gttggttttg aatttgtgat tttagatgat ttattcgttt 22020
   cggtttttta aagtgttgag attataggcg tgagttatta cgttcggttt gtttcgtttt 22080
   tgattttttt atttatttga tcgcgggtat tcgggttgtt tttattcgtt ggttattgtg 22140
   aatagagtcg ttatgaatgt gggtgtataa atatttttag ttttttaaga tgttgtgtat 22200
   ttttttgaat ttatatttag aagtggaatt atgggatgtt acggtggttt tagttttcgt 22260
   tttttgagga attgttatag tgtgtttata agtgattttt gagtatttga tattgagtta 22320
   gtttaattga ggaacggaat ttttattttt atttaaattt aaatagttat attttataaa 22380
   tggtaattat attggatggt atagaatttt agtttaattt attttttatt aatggggaaa 22440
   ttgaggttta tagagtgatt tttttttttg aaatgttatt gtaagtggtt aggttagtat 22500
   aagattttta ttgaatttgg taattttgaa attttatatt tttgtttgta tgatatgcgg 22560
   ttaatatttg tgattttttt taaagatcgt gggttttagt aagacggtag ttgtttcgtt 22620
   gagattttta tcgtattttt aggtttcgtt ttgttacgaa tacggttgtg taaggatttg 22680
   tttgggtttt tgttttttat tttttcgggt atttagatta attattagta gtatttacgt 22740
   cgatttttag aaaggttttg atttggatag taattgatac gttattttat agttaaagta 22800
   aaatgtagag tttttttttc ggtttcgtag ttgggagtta gaggttttag gttttggttt 22860
   ttggtttaaa gtgatttttt gtaagttttt ttagtttggt aaataggtta tgtatttttt 22920
   gatttttttt ttagtttttg ttttgcgagt ttttgggtgt tcgaggttgt ggtttattta 22980
   gtgatatttt tagtttttag tgtagttcgt gagtagtata atatattcgg ttttagaaat 23040
   aattaatata ttcgttaata gatgcgtgga gaaatttaac gtgttgagtt aatatagcgg 23100
   attatttttt agttataaaa aggtacgagc gtgcgttaat atagatgaat tttgaaaata 23160
   ttttaattgt aagaaattaa gtataaaaag ttatattttt tagggtttta gttatatgtt 23220
   tagaataagt atatttgtag ggataaagcg tagattggtg atttttaggg gttgggggag 23280
   ggggagtgat tgtgtatcga ggatggcgtt tttttttggg gtgaggaaaa cgttttggaa 23340
   ttagaggtga tgtttataga aggttgtgaa aaaatgttat ttaattgtgt tttttgaatc 23400
   ggcggatggt gtaatatgga aattatattt ttatgaggac gggagaggaa gggaaagaag 23460
   ggaaggaggg agggggaagg aaatagggaa ggaaggaaaa aagggagggg aggagggagg 23520
   gacggaagaa gggaaagaag gtagttattt ttagtggagt tggattgaag agtttaatgg 23580
   gtgtagtaaa ttattatggt atttgtattt ttatgtaata aatttgtacg ttttgtatat 23640
   gtattttaga atgtaaagta aaataaaata aagagtttag tgttttttat aagtgtttag 23700
   gagttagttt tatagtttag aggatagttg aggtttagag gttaatattt aggtttgagg 23760
   tttttaagtt ggtgtcgtta gattatgttt tagataaatt tttttatttt tttgtagtaa 23820
   gggtgttttt agggtggtag ttaggcggag gtgttaggtg gcgggagatt ttgatagttt 23880
   ttttgtaggt ttttattttg aatttaggtt ttatgttagg gtttttttgt tatttattgg 23940
   cgttggtggt agtaggttat cggtttcgtg atggggattg tttttaaatt tgtttatttt 24000
   ggggattagt tttttttttt tcgtaggcgt ttattgggtt attttttttt tagagttgta 24060
   ggtgggttta aggtagggat gaaagggttg taatttacgt agttttgagt tagtattttt 24120
   tgggaatatt tgtatttagt gataaatttt tattttaatt ttaggatttg tcgaggatga 24180
   gggaattaga ttgttgtttg tggttgggac gggggttgta ggatatttgg ttgggaggta 24240
   gtgtttagag aggaggtagt tattggatat gatatatttt aggggtattt atatttatta 24300
   tagttttagg gttgattttg gtttatttgg tttggtgtcg agagagggtt tgatatagta 24360
   ggtttgtaaa gtttgatttg gaaattaaga tttaggggtc gttttttttt gtttattttt 24420
   ttttgtgtgt tatgttgttt ggtttacgtt ttagaaatgt ttatataagt tttattagtt 24480
   ttgttggtta aatgtagaga tggtgtcgtt ggggtaaggg attttgagtt atcgtttaat 24540
   gttattttta gttttagggg agtgatagtc gggtttgtat tttaggataa agtgtttagg 24600
   gttaagttaa tgttattttt ttgttttttt taaatttaat ttatatttgg tttttagagt 24660
   acggatcgaa atggttatat ttttatatat tttgtttgtt attgttaatg gtggttaagt 24720
   agaatgtaag gttatttttt ttttgtttac ggaggtttgt gaagttattg attaggttac 24780
   ggtaggggag tcggtggtat ttattatatt ttgttagttt tttaggttcg tgtttaggag 24840
   aaagttgtcg gtatttatag ttggtgtttt tgtttcggcg aagatttttt tgtttttatt 24900
   tatttcggtt tagcgtgggt ttagtagttt ttttattttg tttcgtaata atgtatattg 24960
   gagtagtttt tagttgtttt ttttttgtag gtttttttga atttagattt tggggttagt 25020
   atttttgcgg tagttttacg gttatttgta ggttttttta gaaatttgag tttatggggg 25080
   tattttaggt tttataaatt aatagatgtt ttttgttttt tataaagggg ttttttaaag 25140
   ttgagtgagt ttttgatgta ggtaatgtta ggaggatagt gttcggttag ggttgttttt 25200
   tttttttttt ttattttttt gggtgtttgt aaagtttttt agatttttgt tgaattttgg 25260
   ggaagttttg tttcgtaatt agggtttgtt tttttcgttt ttatatagag tttgtttttt 25320
   gcgtttagat agttttttta cgatggtaat tgaataatga tgtgtgcggt ttttttttat 25380
   tataattttt ttatataggg tttgtgaagt tagatatttt atttattacg tttattttag 25440
   ttcggttttt tttagagtag cgtagggttt acggtaagtt tcggtaaaaa gtttggtgaa 25500
   tgaatgaata aatgaagaaa taaagtttat tatagtagtc gaggtttatt gggggttttt 25560
   tgtgttaggc gttattttaa tttttttttt tagagagtgg gttttattaa tgttggttag 25620
   gttggtttta aatttttggt tttaagcgat ttttttattt tggtttttta aagtgttggg 25680
   attatagata taagttattg tatcgagttt taggtgttat tttaaacgtt gtttaggagt 25740
   tattataatt gatggattat tatataattt tataaggtag ttattgttat tgttttattt 25800
   tatagataag gaaatcgaga tcgagagggt ttttgttaat tatgagggtc gtaaagcgag 25860
   taagcggtag agttaggatt tgaagttagg taggtgagtt ttaggttgtt gatttttttt 25920
   ttaggttagg ttttcgggaa ttatggaaag gttggagaag tatggggttt ttgtagttag 25980
   gatttagaag gtgtagttgc ggtgtaggta gtttagtatt gaggttttgg tttttagttt 26040
   tttttttagt tgtttttttt tagggagttg gaggtagagt tttgaattgt taggagaaga 26100
   ttttattcgt tagttaggga aagttgttag ggtttttttt agggtttaga gttattttag 26160
   gtttattaaa gtatattagt attttagttt ttataatttt ttttgtaaga aagatgatgg 26220
   tattagttat tgggtcgggt tgggggtttt agattttagg ggttttttta ggtttgttta 26280
   tggttttttt aggatataga tgtttatttg agaaatttta gtgtattagg ggttttatag 26340
   ttaaaataaa gaggtagagt tgttttgggg tttttgttaa agtttttagg tagatttatt 26400
   tagtagatat taaaatgttt attttttatt tgaaaatttt atttttagga ggtcgtttta 26460
   ggttgggttg ataaaattta tagagatttt aatgatgatt ttttatttta ggtatgagtg 26520
   agtgggtgga gggagaagtt attgttaatt attaatagtt taggaaatgt aggtaaagga 26580
   aagagaatgg taaggcgata gggttggaaa ttagatatac gtgtagaggt gagtagattt 26640
   tagagaaaga attgttttgg aataagatgg taaagaaatt atagataata atggtagacg 26700
   atgattttgg ggttttagtg gattaggatt ataatattga tttatttgat aagtatttat 26760
   tgagtgttta ttttatagta ataaaaagat gatattatag gtttgagagt agttagaatg 26820
   aaaatagtgg tggtatatgg gaatttgaag tttatttatt taaatgttta ttagtcgaga 26880
   ttttcggata gatttgaagt agtttttaat atgtaaggaa tttataattt gatatagtag 26940
   tataggatat gtgtgtgtta taaatttttg gtgaaaagta aagatttttt ttagaaaagt 27000
   atatattgaa gatagataga tagatatata cgtattttga acggtttatt aaagaatttt 27060
   taggttcgtg gattttagtt aaaaaaataa tttggtattt tgggaggtcg aggcgggagg 27120
   atcgtttgag tttaggagtt taagattagt ttgggtaacg tagcgagatt ttatttttat 27180
   aaaatgaaat aaaataaaat gagaattaaa aaaaataaat aaatttggtg tgatgagaat 27240
   ttatagaatt taatagggtt attttagtat tatttgtagt attttttaaa tttttgatta 27300
   tatatataaa gttttgggtt ttatagttag agaagaggga gagtaggtta aaaataatta 27360
   tttaaggtgg aagatgagtt tgagaaataa agaggtaaga ggagaagatt gaggttagtt 27420
   gttttttatt tttgagtttc ggtaggagga agtttttaag tgagaataag ttgttgtttg 27480
   cggagtggat tttagagcgg gtggatagag acgataagga atgttatttt tggggggttt 27540
   tgaaaatagg atattttttt attttaattt tgttttttat tgtattgtgt ttttattgat 27600
   ttatttaagt tattataaat attttataaa tgttattggg gattttttgt ttttttttaa 27660
   agatggaaat attgaatata aatgattaaa ttgtgtaaat gtgtgtgggt gggggattaa 27720
   ggattgaatt tttaaaatgt atatagttta tttaatttgt taattaattt taaattaaat 27780
   tatattttat attttgtatg ggataggaag cgttttttta aagtttggtt aggatttggg 27840
   atgtcggaaa tttgagattt tgtttttttt ttttgagata ttttgaaatt ttgtttagaa 27900
   atcgagtata atggttttgg ttttttagag aaagatacgg atttgtaaag gtaaagagtt 27960
   gtattttttt tcgagttgga gtttttttcg gtttttatta tggggtttaa ataaaattgt 28020
   agttttatag tttttttgtt ggtgtttttt tttttttggt gattaataag gtggaagagt 28080
   taataagaat tttgtgtgtt tttttgtagg atttggaagg gataggtggt gtgagtttgt 28140
   ttttatttag tttttttttt tatttattta ggggtcgagg cgttttcgag gtagggggcg 28200
   ggggttttta tttttattgt cggttaaggt ttttttagtt tttttttcgt taaatttttt 28260
   taagtaaatt ttttttttat tgtggaggaa ggaattttaa gtagttttgt ttttttgtta 28320
   gtacggtttt aattggtttt agtcggtaaa gttaaatttg gataaggttt aagggttttt 28380
   tatattattc gtcgtttttt ggaagaggtt tttttttgtt tttttatttg taatttatta 28440
   tttttttagt ttgagttttt ttatttaggt ttcgcggttt attgagatat taattttggg 28500
   ttagagtaag ttttgatttg taaattatta ttatttattt tttaaagagt tatttttaga 28560
   atttaggttg tagggagggt aggttaagga tgaatttttt gatttttggt gcggtgtgtg 28620
   gtgggggggg ttattttata ttttgttttt tgttgttttt ttgttttgtt ttaggttttg 28680
   aagtggattt attgtagagg gagggttagg gaatggtttt gatgtttttt ttattttaag 28740
   gaatttttta gtaggagcgg tttttatttt aaagtaattt taagatttag ttcgtgttag 28800
   ttttttagtt atgtttatag tggtttgtta attagagatt tttttagttt ttggtaaatt 28860
   tgtaaattta taaagagaaa gaaaagtttt ttcgtgggtg ggtggatatt ggttatttgt 28920
   tataggttgg aatttttttt tattagggat gttttttaag taggggttgg ggagggtagg 28980
   gtttagagtt tttttttttt tagttttttt tgttattagt agatttgtta gtagatatat 29040
   agtagagttt agtagtgagg taataatagg ggttgtgggg gttggggttt cgtttttttt 29100
   aatttatgaa attatttgaa ataataaaat ttagataagg gtttttgggg tgtaataggg 29160
   tggttttttg ttaggtgttg ggtagaaaaa aagttttttt ttaatttttt tttgttgtgt 29220
   agattagttc gggttttggg gatgttgtag aggttgtttt atgtttaggt tttttagaga 29280
   tagtaggggg ttggaggtac ggtttgtttg ggggtagggg ataggtaggg aggggtatat 29340
   ttttgggtta tgagtaggag tttgattaaa tgattttttt aggagggaga aagatatcgt 29400
   atataattgt ttgattatag ttagaggggt tggaaaggtg ggggaaaggg gtgaaggggt 29460
   taattgtttt aggtgttttt aatttatgta gtatttattt ggattgataa gagttgagag 29520
   attggaattg tttttttttt tcgtttttag gttttgtttt aggagttttt ttgtttagga 29580
   gtttgggagt tgaggaggtt tggaagtagt gggtttatag tgttatcgtt ttgggaattt 29640
   atagtgtttt ggatgtggtt ttttcggttt tagttttgta ggtagtttat ttggattgta 29700
   tttatttttt agaagagttg tttagaattt tgtatgttcg aaagaaagat taattaaaga 29760
   agtgaggaag ttaaagagag gtttatgaaa gtttatgacg tattttttaa gtgtatattt 29820
   tagtaatttt aaatgttttt tgaagtaagg atggggatgg atggttggat aaatggatgg 29880
   gtgtatgggt gtgtgaatgg gtgagtgggt gggtggatgg atggatagat ggatgaatgg 29940
   atgggtaggt ggatgaatag gtaggtgaat ggatgggttg gcggatgaat gagtaaatgg 30000
   atgaatgatt ggatggatgg gtggatgagt agataggtgg atgagtgaat tggtggatga 30060
   gtggatagat ggatgagtgg atgagtagat gagtgaatgg gtggatgagt ggatgggtgg 30120
   gtgagtggat gagtagataa gtggatgggt ggatgagtgg atgggtagat ggatgggtga 30180
   gtggatgcgt ggatgagtgg atggacggat gagtggatgg atggataagt ggatgggtag 30240
   atgagtggat ggatagatga gaggatgggt agatggatgg gtgagtggat gggtgggtga 30300
   gtagatgggt ggataagtgg atggatagat gagtggatgg gtggatgagt ggatggatat 30360
   atgggtggat agatgggtga gtggacggat gatgagtgga tgggtggatg agtggatggt 30420
   tgagtggatg gataaatggg tgaatgagtg gatgggtaga tgagtggatg gatagatgag 30480
   tgaatgagtg gatgggtgga tgagtggatg gatagatggg tgaatgagtg gatgggtgga 30540
   tgagtgaatg agtgaatgag tggataggtg gatgagtgga tgggtggatg agtgtataga 30600
   tagatgggtg gatgggtgga tgagtggatg ggtgggtgag tggatgggta ggtggatgag 30660
   tggatggata gatgggtaaa tgagtggatg ggtggatgag tgaatgattg tagataggtg 30720
   gatgagtgga tgggtgaatg agtggataga tgggttgata ggtggatgag tggatgggtg 30780
   gatgggtggg tgcgtggatg ggtgagtgag tggatgggtg ggtgggtggt aagatgggtg 30840
   gataggtata tggagtttat ttaaggagtt ttagaaatat atatatattt ttttatttta 30900
   gatttttttt aggaaaaagt agtgtgtatg gatcgttatt tggttatatt ttgagatagt 30960
   tatttttatt ttttttggaa tatcgtttgg ggggatgtag tatagggtat ttaagtaatt 31020
   tttgagttta gggtagagat attaggaagt agtaggtagg tcgtgatttc gtaggtttgt 31080
   tttatggttt ttgttgtttg tattatggta tgaagtttag atgtattttt ggatttgggg 31140
   ttttgattag atggagttat taattttttg aagaatgaaa tatatagggg ttatttttgt 31200
   tttttagttg tagggaatga cgattattta gttttttttt tttttaaatt ttttttaggt 31260
   ttttgtgtat ggttttgttg attttttttt tgtttttggt tattttttta gtttttgtcg 31320
   tttttttttt cgtttttatt tacggagtta gttttggtgt tttgagagtt ttttttttta 31380
   gttgatttta tttagttttt gagtatttaa tgttaataat gttatttttt ttgtttagat 31440
   ttatatttga gtgttagttt gatttataga cgtgtttttg ggtttcgttg tgttaggttt 31500
   tttgttgggt tttgggtatt tgtagttatt gttattgtta agttttaata tttgaaggta 31560
   ttttgaattt aatgatcgtg ttttaggttg aattttggag tttttatttt atttgttttt 31620
   tattttattt tttatttaag tgaatagttt ttttagttaa tagttattgt agttagattt 31680
   ttaggggtcg ggtttgggtt ttttattttt attatttcga atttatttat ttattttaaa 31740
   tttttattaa tgttaattta atatatttta tagttattat tattagttgt ttttgttcga 31800
   attttttttt tttaaagtat tagggggcgt gtgtaaaatt tattaatttg atgatttttt 31860
   tttatgaagt cgtgattggt ttttattgta tttgggatat attagatttt tgtggtttta 31920
   gaattttgtt tattatcgac gtgttgtgtg attttgagta agtaatttaa ttattttgtg 31980
   ttttaatgtt tttatatgtg aaatgggata ataatatttg ttttataaga atgttgggga 32040
   ttaaatgaga tatttattta tgaagtatat agtgttgtgt ttggttagta aatgtttgaa 32100
   gtttttgtta ataaaatata cgtttttaat ttatttttta gtttttattt acgtatagtt 32160
   tttttggttg ttttttagtt tttttaataa ggttatttat agaggttttt tttgtcggga 32220
   ttattttttt tttatgttgt ttttagaatt tttatttgtt ttttcgggat agtttattta 32280
   tattgggttt tttagttttt gttttgtttt gttttgtttt gttttttttg agatggagtt 32340
   ttattttgtc gtttaggttg gagtgtagtg gtattatttg ggtttattgt aagttttgtt 32400
   tttcgggttt acgttatttt tttgttttag ttttttgagt agttgggatt ataggcgttc 32460
   gttattacgt tcggttaatt ttttatattt ttagtagaaa cggggtttta tcgtgttagt 32520
   taggatggtt tcgatttttt gatttcgtaa ttcgttcgtt tttgtttttt aaagtgttga 32580
   gattataggt ttgagatatt acgtttagtt taggtttttt agtttttata tacgtggttt 32640
   ttcgattgtt gtttggggtt tgtcgtattt attatttata aaattttata cgttcgaagt 32700
   atttagtaag acgttgtgaa tgagtgaatg aataaatgaa tgaatggtta gtttgttgtt 32760
   ttttcgttcg gtttttcgtt ttttttagat tagtatgggg gtagttacgg ttttcgttta 32820
   tttcggtaat atcgtttttt ttgtgtcggt cgcgttggtt agagtttttt tttacggcgt 32880
   tttcgcggtt tcggtgtgtg gtcgtttgtg aatgagattt atgattttcg gtttttatta 32940
   tttttttgga gttataaagt attaaggcgg aaggatggat gggttttttg ggatagtcgg 33000
   ggtgggaggt cggtttagga aatttttttt aggaaatggt tagtgtggga tgaggcgttc 33060
   ggtgggaggg gagtggagtg gggatttggg gtttggttgt tttgtagtta gggattttaa 33120
   tttttatttt ttttttcgtt tattttttaa ttttagtgtt gggtttaggg tttttggggt 33180
   tttttttagg aatatagaag atatagtaag gattagggat ataggatagg tagagtttat 33240
   gggtaggagg tttttagtta gtatatttga gtttatgttt ggttttaatt agatattgag 33300
   attttggtta ggttattttt tacgttgagg taggggggtt ttttagaggt tattggtttt 33360
   taagtatcgc gtggaagata tgttacggag agttagtatt ttttgagata ttagtagaag 33420
   gttagacgtt tcgcggttta aggatgattt ggttttagcg tttatttatt tgtcgttttg 33480
   tatgaaaagt ttgacgtgat atggtcggtt ttagcggaag agtgattcgg gaatcggtag 33540
   agcggattta gtaattggta aattggattt agtaattgag atattgttag gggtttggta 33600
   gaagtttttt aatgtattag atttagtttt gatttttata gagtttagtc gagtattaaa 33660
   aggtgtagag gtcgagttga gtattagggg aatattgatt ttagggttga tcggtgttgt 33720
   gcgtgtattt tatagtttat agacggttta tatttattcg atcgtatttg attttttttt 33780
   gcgaggtttt ttttagattt ttcgtttttt aaagttatta gttgtgtttt ttgaaattag 33840
   agtaaagtaa taaaattgta gtaatagcgt gtttttatga acgttgtgat gttagttggt 33900
   taaatgaggg agtttttagg gttttttata ttgtttcgat tttgtgtgtt ttttggtttt 33960
   ggtttatttt ttatattttt tgtttttttt tttttttttt ttttcgagga tttagggttt 34020
   ggggttgatt tattgtggga ttttaacggt gattttttgg aagaaatgtt cgtttttttt 34080
   ttttatttag tagtgatttt tggttcgttt acgtttacgt agttaatata ggtagtagtc 34140
   ggaatttgga agtttttggg gtgggagttt gttttttttt aggttttttt agttcgaatt 34200
   tcgggaagga gttatattta tatggtaatt ttttttaagg ttgggagggg gaaggggttt 34260
   ggttcgggtt tgattatttt tttgaggttg cgtttattgg gttttttaag ttagttgggt 34320
   ttttcggttg tatttttgag ggatggtaat agattatagt gtaaattgtt tatcggttgg 34380
   ttgggtagtg ggtgtttaga tgcgggttag ggttttcgtt ttataggttg tgtggtggtt 34440
   taaagtgggt atttttatgg gtatatattt gtgttatagt ttattcgtgt gtgtgtgtgt 34500
   gtgtgtgtgt gtgtgtgtgt gtgtgtgtgt gtgtgtgtga tagtttagaa atatggtaat 34560
   ttatagtttt tttaagacgt tggttttggg agatttgttt atttgggggt tttagtattt 34620
   ttagtttttt ttaggaaggt tttgtttttt ttggtttgaa gttggtttta gggaagaatt 34680
   tttttagaaa aataaaagtt atttttttta gttttagttt agtttttttt taggggtgag 34740
   ttatattaaa tttatttttt tttttttttt ttattttttt ttttacgttg taattagttg 34800
   taagtggttt atttatttag gtttgttagt ttattataga aaagatattc gaattttgta 34860
   tttttttttt ttgatttgtt ttgggtttta ataggtaaag gaaattggaa gttagtagtt 34920
   aggatttagt tgtaggtttt cggggttatt ttgcgttttt attttagttt tatttatttt 34980
   tgcgttgatt taatgtttat ttattttagt ttatggagcg aggtttggat ttaagataaa 35040
   ataaaggggg ttttggtttt ttggggttta tagtgttcgg tgtcggcgag ataattttag 35100
   agtatagtat agagaaatgg ttgcgatacg gattcgtagt atttggttgg ttaagtatag 35160
   taatgtattt ttattaggtt tgggtttttt agtagatata tggtttaaag gttggttggt 35220
   taaattatat gattttgtaa ttatttaaga gaaatgaaaa tatgtttata taaaaatttg 35280
   tagattagta tttattgtag aattttttat aatagaatta tgttaaaaag tagaaataat 35340
   ttaaatgttt attagtagat gaagagatat atagtgtggt ttatttacga tggaatatta 35400
   ttcggttatg aaaaagaatg aagtattagt atttgttata atgtggatga gttttgaaaa 35460
   tattttatta agtgaaagga gttagatatt aaggttatat attgtacgat tttatttata 35520
   taaattattt agaataggtg aatttataga gacgaaggta gatgagcggt ggttaggggt 35580
   tgagagttcg gagttgggaa tggggattgg ttaatggggg tagggttttt tttggggatg 35640
   atgaaatgtg ttgggattaa tggcgatggt tgtgtaattg tgatggttaa ttttttgtgt 35700
   tagtcgggcg tagtggttta tgtttgtaat tttagtattt tgggaggttg aggcgggtgg 35760
   attacgaggt taggagattg agattacggt gaaattttat ttttattaaa aatataaaaa 35820
   aaaaaattag tcgggtgtgg tggtgggtat ttgtagtttt agttattcgg gaggttgagg 35880
   taggagaatg gcgtgaattt gggaggcgaa gtttgtagtg agttaagatc gcgttattgt 35940
   attttagttt gggcgataga gcgagatttc gttttaaaaa aaaaaaaaaa aatttttgtg 36000
   ttaattggat tgggttatgg ggtatttagg taatcggtta aatattattt tgagtgtgtt 36060
   tgtgaaggtg tttttggatg agtttaatat ttaatttttt tattttttag aaagtttagt 36120
   ttattgttag tatttattta attttatata aatacgtttt ttgaggttga agtaaatttg 36180
   attggatgtt ttgtgtgaaa attaaatata aaaattggtt aggcgtggtg gtttacgttt 36240
   gtaattttag cgttttggga agtcgatatg ggttaattat ttgaggttag gggttcgaga 36300
   ttagtttggt taatttggtg aaatatcgtt tttattaaaa atataaaaaa ttagttacgt 36360
   gtggtggtag gtattttgta attttagtta aatttaggag gttaagattg gagaattatt 36420
   tgaatttagg aggtagaggt tgtagtaagt cgagatcgtg ttattgtatt ttagtttggg 36480
   taatagagag agttcgtttt aaaaaataaa aaaattaaaa agaaaaaaaa ttttttaaat 36540
   tgtttttgta gttattttta agtaaaataa tagagttatt tgagtcgttt attttggaaa 36600
   aattagattt attaaatgaa tttttagtta ataattgatt aagaataatg ttaatattat 36660
   ttgtaggaat gttgtgtttt ttaggatttg atatttttag cgatcgagaa ttattatttt 36720
   tttttaaatt ttatttattt atttattttt tgagatggaa ttttgttttg tcgtttaggt 36780
   tggaatgtaa tggcgcgatt tcggtttatt gtaatttttg tttttcgggt ttaagtaatt 36840
   tttcggtttc ggttttttaa gtagttggga ttataggtgt ttattattac gtttggttaa 36900
   tttttgtatt tttagtagag atgggatttt attatgttgg ttaggttggt ttttaatttt 36960
   tggttttagg tgattagttt gttttagttt ttttaagtgt taggattata ggtatgagtt 37020
   attatattta gttgagaatt attatttttt tttaaaggaa atattattat taaaaatgga 37080
   atgttataaa tagaatgatg ttttttgttt ttaaagtcga tatattagag tgatgtgaaa 37140
   ataataataa aagtaagata tttttcggta aatttatttt ggggtgaacg ttgtagttat 37200
   aagtattgtt ggtgagtatt ttcggggtaa ataggaaaag ggtgtaatta ggagtttggg 37260
   taaagtagat tgtgtgtttt gatgggggtg ggttttattc gattagttga aggtaatagg 37320
   gtttggaacg tatattttaa gatatagtgt tttggtatag taaatatttt tagttgaagg 37380
   aatttaagaa atggtaggtt taagaagaat ttattgattt gttttttttt tgaagtagat 37440
   tataagtttt ttacgtaaga ggtgtttttt ttatatttag cggaaaggaa tagttttatt 37500
   tttagagata gaggatttga gaggggtttt agtatatagg ttttgtcgag atttttacgg 37560
   gtttattgtt tagtttattt tttgttgttt tgttatagtt ttttacgatt ttttattttt 37620
   tattaaattt agtataaaaa cgtttaggtt taggttaggt atagtagttt atatttataa 37680
   ttttagtatt ttggaaggtt aaggtggtta gattatttga ggttaggagt tgaagaatag 37740
   tttggttaat atggtaaaat tttattttta ttaaaaatat aaaaattagt cgggtgtggt 37800
   ggtatacgtt tgtagtcgta gttatttggg aggttgaggt atgagaatgg tttgaattta 37860
   ggagatagag gttgtagtga gtagagatta tgttattgta ttttagtttg ggtaaaagat 37920
   aagtaaaaga aataaataaa aaaattgttt aaaataagta ataaataaat ttaaaaaata 37980
   agtaaaataa ataaaaattg tttaggttta ggtcgggtat agtggtttat atttgtaatt 38040
   ttagtatttt gggaggttaa ggtaggagga ttgttcgagt ttaggagttt gagattagtt 38100
   cgggtaatat agtgggattt tgtttttata aaaaataaaa aattagttag gcggggtggt 38160
   atatgtttgt agttttagtt atttgggagt ttgagttggg atttaagagt gggtttagag 38220
   tagttttcgg ggtcggtagt tgggttttag tttgagttta ggaagttaag gttgtagtga 38280
   gtcgtgatcg ggttattgtt ttttagtttg gacgtgcgat agagtgagat tttgttttaa 38340
   aataaaataa aaatcgttta ggttaacggt ttttttgggt ttttattttt ttatgaaggt 38400
   tcggtgttaa gtaaaattta tttgaattgt tatgtttgtt ttttgttaat ttttgttagt 38460
   ttaattttta gggttttagt taatgaattt aaaatcgata gaaggaaagg gttttttttt 38520
   agattttgtt ttgattaggt ttttgtaatt ttgtgataaa ggtttgaagg tttgaatagg 38580
   aggatataag gtttgagtga gagagaattt ttttcgttta atcgttttta atttggaata 38640
   ttagattttt tttgttttag atttaaattg atatatgcgt tttttttggg tttcgggatt 38700
   ggaacgacgt tattggtttt tttgggtttt gggtttttag gtggatatta gagtcgtaat 38760
   atgggttttt tcgtgttttt agtttgttga tttattttgt taattttggt atttgttaat 38820
   ttttgtaatg aggtgagtta attttttata ataataaatt tttttattta aatatacgta 38880
   tatatttttt attggttttg tttttttgga tgattttaat ataattattt tgtgaatata 38940
   gtaaaattat tgaattgtag attttaaaaa ggttaacgtt atagtatgtg aattttattt 39000
   ttataaagtt attatttaaa tagtaataag aagttggtga gttttgtgta tttttttgta 39060
   aataagagta gggatagcgt cgtttttgtt ttcggatgtt tttagttttt agttcgagtt 39120
   taggaatatt ttttatgtgt tttttttttt aattttgtag tttaagggtg ggggcggttt 39180
   ttagtttggt attttttttg tagtttttta tttttttttt atattagtta atggatatag 39240
   aggttttttt atggattttt atttttattt atttattttt atttttttga gatagagttt 39300
   tattttgtcg tttaggttgg agtgtagtgg tgtgatttcg gtttattgta attttcgttt 39360
   tttggatttg agtgattttt ttgttttagt ttttcgagta attgggatta taggtacgtg 39420
   ttattatatt tagttaattt tttgtatttt tattagaggt agggtttttt tatgttggtt 39480
   aggttggttt cgaatttttg attttaggtg atttattcgt tttagttttt taaagtgttg 39540
   ggattagagg tgtgagttat taagtttagt ttttttatag atttttaaaa gggttttggg 39600
   taatatagat tgggatttgt taagttatta aatgattttt ttaaataaag aaggaagtta 39660
   taaattttta tttaaaagga gtgggggatg gtaatagaaa tatagttagt tagaagtcgg 39720
   aagggtttta tttaatttat ttttaaggtt tttgggtaga agggataagg gaaaatttgt 39780
   tttttaaata ttttattcgg tattttattt ttcggatgta agatgtagtt gtgtatagcg 39840
   gtagtagtga ggattttggt ttggtaggtt tttatatttt ttgttgaggg ttttaggggt 39900
   tagggttgtt taataggatt aagaggaaat gattatagga gtttttagtt gttgggggtg 39960
   gttttcgcgg ttttttgggt tatttttggt gaatttattt taaatttgga agaggtttaa 40020
   gttttttcgt ttttcgtatt ttaaatttta gttacgttag ttaggagttt tgggggatga 40080
   gaaaaaattt aattaagttt tattgtgttg ggtgatatag gatttagaag gagaggggtt 40140
   tttggattgt ttaagtttaa tgataatttt agtagttggg tttttaagtt aattagtttt 40200
   ttttttttta gtagttttcg gggagtatta cggtgagttg tgaggagtta gtttagtacg 40260
   gtatatataa cgtaggggtt ttttcgtatt tttattttat aaatattagg gtttttcggg 40320
   ggattagttt tttatttttg agttattttt tttttatcgt ttttttaata aggaatagag 40380
   aaggagtttt tattgtttaa ttagatgtag aaattgacgt acgtatagag aggttacgtg 40440
   tgatatggtt tggacgttgt tttttttaaa ttttatgttg aaatgtgatt tttgatgttg 40500
   gaggtaaggt ttgggggggg tggggtttgg gttatggggg tggatttttt atggatggtt 40560
   tggtgttttt tttacggtta taaagttcgg gagatttgat tgttatagag tttgggaggg 40620
   ttgggtgagg tggtttatat ttgtaatttt agtattttgg gaggttaagg tgggtggatt 40680
   ataaggttag gagtttggga ttagttcgat taatatggtg aaattttatt tttagtaaaa 40740
   atataaaaaa attagtcggg cgcggtggta tgcgtttgta attttagtta tttaggaggt 40800
   tgaggttgta gtgagtcgag gattgtgtta ttgtatttta gtttggttga tatagcgaga 40860
   ttttatttta aaaaaaaaaa gagtttggga gttttttttt tcgttatgtg acgtgttagt 40920
   ttttttttta ttttttatta tgaggaaaag ttttttgggg tttttttaga agttaagtag 40980
   atgttggtat tatttgtata gtttgtagaa tcgtgagtta aataattttt tagtttcggt 41040
   tattttttat agtaatttaa aatggacgaa tataatgtat tagttttttt ttttggattt 41100
   tatagtgtgt ttggttgggg ttgtgttttg gggaaatggt aattgcgagg tttttataga 41160
   tcgggttcgg gttttaatta tagtcgtatt ttagcgtttt gatattgttg agtggatgtt 41220
   taaaaagtat tgttgggtga atgtgcgagt gaatgatgaa gatggaggtg gttgttggtg 41280
   ttagggtaag gttttaggag tttggggagg tggtattagg tagtttaggt taacggttaa 41340
   ttttggatag ggttaggagg tagttagaat ggttaaggat agttaaggga ttttaggtaa 41400
   gtggttttat tttttttagg cgggtaggaa gtaggaatta gattaagggg tgttaggtgg 41460
   tagtggtatt tagggggttt gaggagtagg tgtaggttta gtagggttgg tggtgttacg 41520
   tgtggggtcg atcgtggttt cgtttattgt ttttattttt tggacgtatg ggtttatggt 41580
   ttttaaggtt tttttttgtt tttacgttta tttttttagg ttttattttt agacgttaag 41640
   ttattttttt atttttattt ttttcgttgt ttttatattt gagttgattt tttttttgag 41700
   ttttggtgaa attttagaga gtttagaggt gtagtttata tttttgcgtt ataaggtttt 41760
   tgtagttttt atttttttgg gatattttat tttagagttg ggtgtgtatt tattttattg 41820
   gattagaaat tttaggaatg tttattttgt atgtttttgt tttttatttt tcgttcgtta 41880
   tttagttcgt ggttgttatg tggttgggtt ttggtagtta tcgtagttgg taatatataa 41940
   ttagtatttt tgtgtttagg tttcgtgttg agttttttta gttttatttt cgtttgtttt 42000
   taaaatggtt tttgatatgg taattattat tatttatttt ttagataaaa gattatagtt 42060
   ttaggagagg aggaatggta ggtttgaggt tacgtggttc gtaataggta aatggattcg 42120
   gtagttattt tattttcgag tggggttttt ttttgtttgt agatttcgtt gaggttttag 42180
   agtttggagt tagtgagttt ttattgagta taaggatagc ggcggtgtcg gggttggaat 42240
   aaggtaggga ggggtttttt aaattatagt tttggttgtg gatttatttt gggatttcgt 42300
   tttggttggg aattttgttt tagtttattc gggatatttt tttattttga gttttgtatt 42360
   gggttgtgta cggggtttgt tgaatgagtg agggtaggag ggatggtgag tagggtggaa 42420
   agcgattatg gtgggtaggt ttttgttgga agtttgaaat atttttggcg agtaggaaaa 42480
   tattagggga agaggttata gtttttagaa gggttcgttg gggattgttt tgtagttttt 42540
   ttatcgggat aggtagaaaa tttagttttt ttttagggtg agtttaggaa gtttgtgggg 42600
   gtataggttt tagttagggt ggcgcgtggg tttatttagg ggacgttgtc gtggtttaga 42660
   gttaggttaa attttatgta ttttgtaaat aattatttat aataaataag gtttatttat 42720
   tttttaagaa gagtaaaata ttataggagg tattttttta ttaattaaat acgttatagt 42780
   gattttattg cgttattgag ggagggtatt agggtgttcg gtttagagta aattgtggtt 42840
   agggggacgg gtatattgtt aaatcggtta gatttttatt tttgaaaatg tgggttaaat 42900
   tttttttaat aagttataag gaaatttagt gtgaatttta tgtttttatt attatcggtt 42960
   gttttggaag attgggagga ggggttttgt ttttttttta tttttggtta tagggtttga 43020
   ggttataatt tggattggga ttttaatttt ttttttgggg aagaggttta tttagggtgt 43080
   ttgttaaggt ttagcgtata aatacggaat tcgtttaacg tttgtttcgg tatttttggg 43140
   ttttttatat tgggtttttt tatttgattt tttaagtagt gagagtgatg ggatatgggg 43200
   cgtttttagg ggagatttgg ggagatggag taagtttttt agtttaaggt gaggtttcga 43260
   ggtatggttt atgtagtgat gttttttacg ggaggtttga ggtatgatta ggagtttgga 43320
   gatttagtag aggtttgggt atgggatttt cggtacgtag ttagagcgag ggtcgaggtt 43380
   gtatggatgt aagtaggtga ttttgtgtta tcgggtgtta tttgtgtcgg gttttgtgta 43440
   gtcgttttta ttttattgag ttttagggag gtgaggtaat tattttaggt atatagtttc 43500
   gggggtaaag gttggattta ggtgtgtttg attttagaat ttaagaagat aagtagagga 43560
   gtttgagttt tttgagatcg tagtgatggt ttagtgtttt aggatggtta ggttggtttt 43620
   tgtagtttta gttaatcgtg ttttttttta gttatttagg gttttagcgt tggtttttga 43680
   gatttggtat tggttatagg ggtttatttt tggttgtgga tatttagtgt tggttattgg 43740
   gatttggtat tggtcgtggg gatttgtttt tggttgtggg gatttagcgt tggtttttga 43800
   gatttggtat tggttatggg gattcgtttt tggttgtggg tattcggtgt tggtttttga 43860
   gatttggtat tggttatggg ggttcgtttt tggttgtggg tattcggtgt tggtttttga 43920
   gatttggtat tggttatgga ggtttgtttt tggttgtggg tattcggtgt tggtttttga 43980
   gatttggtat tggtcgtggg ggtttatttt tggttgtagg gatttagtat tgtttatggg 44040
   gtttgttttt ggtttcgggg agttagtagt gtttgtgggg tttatatttg gtggtgggga 44100
   tttggtgttg gaggattttg atagtaaaat tatagaggcg ataggtaata gtaatttatt 44160
   agtatagcgt gtggtttgtt ttagttacgg tatttttttt tattttgtgt ttttttatta 44220
   taaagtttag tgtaggtaac gaagttaaga ggtggagaat tcgtaggagg acgtatatta 44280
   tatttgtttt tcgtgtggcg tttttgtatt ttgggtggtt gagatttttt gttttttttt 44340
   taggtttttt cgtgttaggt ttgcgttgtt ttttttggtt tcggttttta tttattcggt 44400
   tttttagtta tagatagtag cgattttttt ttttatttaa tggggttatt gtttagttta 44460
   ttattagttt ttagggttgg tagatgatat cggttacggg tagtgttttt ttcggtttta 44520
   gtatataagg tataaaagat attattttgt tttaagattt ttttagttac gtggcgtatg 44580
   ggaagcggtg tcgggaattt tttagggtag ttttttgttt cgggggagat cgtgtttaaa 44640
   ttttcgagga aatgttgttt tttggttttt tttttgaaaa attttttaag gagattttgt 44700
   gcggagcgtg tagggagaat ggagattttt gtgagattgt ttcgggatag ttttatagaa 44760
   attataaatt atttggataa aagttgaaaa tttgcggtta tataatgttg attatttatt 44820
   taaatattcg ttgagtaagt atttatgacg tttgggaatt taggacgtgg atcgataaga 44880
   tataattttt atgttcgttt tagtgataga gagagagacg gtattattaa tttcgttcgt 44940
   tgggggtttt tttggagggg gcgtgtaggt ttagggttga atttgtttag tggatgtgtt 45000
   atttacgttt gcgtattttt tggttttgtg gtggtttttg gaataaaggt tgagagataa 45060
   aagataagga tttgggggag ggaggacgtt tatgtatatt ttattgggga tattagtaag 45120
   gatgattgtt tagtgattat ttagtgatcg agttatttgg tgtttggagt ttggtattta 45180
   tagagtaggg cgggggtagt aggttttgtc gggggtatag gtttgagaag ggagtgttgt 45240
   tagttaggtt cgacgggata gagagcgaag ggtttttttt ttgaaggtta tgtagaggtc 45300
   ggaggttaat gttagtaatt ttattaacgt tagttttttg agatcgattt agagttgggg 45360
   ttttaagagt agttttgggc gtttaggagt taagatttta tagagggtgt tttttatttt 45420
   tagtttaggg aaagtgataa atttggagtg tttttgttta tggtttaatt attgaattta 45480
   aatgaatgta ttgataaatc gtatttagtg ttatttatag ggaaaaggga gggaaaaaaa 45540
   aaaaagaaag agagttaatc gcgtttaggt cggtcggtta gggggaggat agatgggtta 45600
   ttatttttgt tattttttag gttttttgta ttgatttttt atttttagta aaattaaggt 45660
   ttagtttttt ttcgtagttt cggagtcggt tgggatgaag taatttttgt aggtagatta 45720
   ttaggtttat tgtgatggtt ttagtaggga atttagttta ggttatagta gtttaggcgt 45780
   ttgtagaagg tttagatttt tgggtgttaa gttttttttt tttgtattcg tttcggggtt 45840
   cggtggggtg ggtagtgtgc gatttttagg gttgatgaaa gagaggagga agaggttgag 45900
   cggcggcgag tttgaatagt taattttggg aggggttgag gtggggaagg tttttattgt 45960
   tgttttatag gtttcggagt aaggttttta tgattttatt agtagttggg taggtatagg 46020
   tttaggtgtt gagtgtgtta ggattttcgt tgttttttgg gtgggggcga gagggatttt 46080
   tatgggtttg taattagatt cgcgtttttt tagttaattt tttattaaat aaatttatac 46140
   gtatagcgga ttgggtatgg tcgttatttt cgggagtttt tttgggaaag ttttttaagg 46200
   ttttgttgag tttcgggaat aggtttttat ttgttcggga atcgggttgg tatttaataa 46260
   ataaattaaa gttcggcgtc ggaaggtaga attttgggat tagagtttta gttaagggtt 46320
   ataagttttt taggaagttt tttttttaaa gagtggttgt gggacgttgg gagtttttag 46380
   ggtggttagt ttttaggttt ttttaaagtt taggtggtat cgatgttttg tgttaaggtg 46440
   agggttgtag agaggggtcg gcggggagga gtttttatag tttattttgg ttattgattt 46500
   tatttttaag tattgaaata aagttttttt tttatatttt cggttaggga gagagggcgg 46560
   aagttttaaa tattcggggt gtatgggaaa ggtttatatg tatagaagaa ttagataacg 46620
   gaagttgttg gtggtggtga taggtttttt aaaggggttt tgagattttt gatatcggta 46680
   gggaagttat atattgtatt aggtttgggg gtagggagaa ggggttttta aggacggggt 46740
   tgtttttttt tgtggaagtg tttcgtattg tttgataaga gtagtttgga ttagtcgtgg 46800
   tataatagga agttgggaga atacggtggt ttaggaggtt ggttagggtt ggggagtggt 46860
   taatttagag attttggttg tttaagtggg tttagggttt tcggggtgaa agtttttgtg 46920
   gagtatagtg gatttagtgt tcggaggata gattttaggt ttagataagg tttttttttt 46980
   ttttttttga gatagagttt tatttttgtt gtttagattg gagtgtagta gtataatttt 47040
   agtttattgt aatttttatt ttttaggttt aagtgatttt tttgttttag tttttcgagt 47100
   agttgttatt ataggtacgc gttattatgt tcggattagt tagtattttt agtagagata 47160
   gggttttatt atgttggtta ggttggtttc gaatttttga ttttaggtga tttgtttgtt 47220
   ttggtttttt gaagtgttgg gattataggt atgagttatt gcgttcggtt aataaggtta 47280
   tttttgatat ggaatgttgg tttgattata gtgtttgatg gtttttgttt gtaattatgt 47340
   tttcgttttt tattaaagta gggtttttag ggtagaagtc gtattttttt attttagagt 47400
   gttttacgtt attttttagg cgtatatagt gggttttttt tatcgtagtt gaattggttt 47460
   tattattatt ataatgatag tttatagggg cgggggttag tgggaatata aagtggttaa 47520
   tgtaatagtg ttttagttat ttcgatatag aaatggttag gggatagaag gggtaagttg 47580
   ggagttagta gtaatttagg tttttagaga cgagtattaa aaaaggcgag ttatattata 47640
   ggtttttttt gattatattt tagtttaaaa gagatttaag gagaaataag aattttattt 47700
   ttttaataag gattcgtata ttagatgtta agtaaatatt tttttttttt ttttgaaaag 47760
   gagtttttgg ggttgtcggg ggagttggtt aaagagaatg atatttatgg aaatatacgt 47820
   ggttttatta agttttaagg taaggtggta atgaatggag gaggaaaatt tatagtatta 47880
   ttagggaatg gggttttttt ttgttttttt tttttttaac gggttttttt atgtgggatt 47940
   taaattaaat tttaatttta ttttgattta gtaggtgttg tttttatagt ttcgaataga 48000
   tttgttagaa aattattgtt taatttgttt tcgggtggta tatttttttt taggaaagga 48060
   attataaatt tattttagat gagtagagag ttgtttttta aatttttgat taaaatatag 48120
   tagattttat atgaaaaagt aaagagagag gggttaggga tgaagtgtag taggttggag 48180
   gttgagttag tttttagagt tattgggacg gcgttttagg agtagatggt attgtttata 48240
   aagtttttcg tttttttttg ttttgaggtc ggggattatt tcgttggttg tatgatttag 48300
   tttttggggt ttttttagta tgaaaagatt ttttgaaagt aggttcgtta gggagatttt 48360
   ttagtttttg tttattgata tgtttttgta tggtcgatac gtagtatgtt attgtagaga 48420
   agtaaagtat acgtagatta gtttcgttag tagtattttt ttttttcgtt ttttttagga 48480
   acgttttgta tatatttaat atatttagga atttagagtt gcgttgtagt agtagttttt 48540
   tggattttag tgtcggcgtc gcggggagtt tcggttacgg tatttcgagt attgagatta 48600
   tttttagttt ttagttagtc ggttttcggg agtttagagg ggtgataatt tgagcggagt 48660
   gattttagag aaagcggtgg ttaattcgaa tttttttttg gtttttttgg aagttaggga 48720
   aattttttgc gaaaaggaaa tatgttttaa gaagtttcgg gtagtaggag aatttagtta 48780
   tttagtttag ttggttaaat atttataaag agatagttgt gattatataa agttttattt 48840
   ttttattttg aggtcgaaaa tttggttttc gcgtggagat cgagtttttc gttatttttt 48900
   ttttggggat ttcgttcggt tttgtcggtt tcgggtcggg gtttagggat agcgtggttt 48960
   tcggtatcgt tattatggtt attagggttc gtttcgcggt cgcgttattt gagagttttc 49020
   gagttagtgg tgtaaggttg ggaagaataa aggagtagga tttattttag tagcggaacg 49080
   tcgggttgcg ttggacgcgt cgagagtcgc ggttcgttta tcgggttata tggtttagag 49140
   tcgagtagag gattaaaggg gttttgtggt cgcggaataa ggagtttttt agagcgcggg 49200
   gtagaggtag ggagggattt cgattgtttg ggaagggagg ttcggtgggg aattgtttta 49260
   agggggtagg gcgaggggag taggaggagg gggtaggtag agataggggc gtttagcggt 49320
   aggttcggcg tagaagtaat ttgaaggtac gggagggacg ttaaggagtt gttatacgtt 49380
   aaaggaaagt gatagggttt ttgggaagag tgagggggag ttgttaggaa gaagagaggg 49440
   gaagaggttt ttagggattt ttggttatgt cgtttttgtt tttgtttttg tgtttgttag 49500
   aggcggtgtt cgtgaggagt tttttttttt tagttaggtt ttgaaattga tttggatatt 49560
   tttttttttt gtgttttgtt tgatattttt gttttttagg tgttttttta tttatttgtt 49620
   tagatatcgt tatagttggg tttttaggga gtatggttag ttattttaga aagttattta 49680
   gatttgtagt tagggttgga atatagttat ttgggtttgt tttttttatt ttaggtgttt 49740
   gttggttggg attatggaag ggagaaggag taggagagga ggggtagggg atgggggcgt 49800
   agtttatttt gagttttttt ttcgagagtt tttggattta gatttgattt agtttttgtt 49860
   tttttagttt ttttagtttt tgggggattt ggttagtgag ttagtttttt tttttttttt 49920
   tttttgtagg tttgggatgt gttgggattt agaggggtat aagtaagggg gtttttagag 49980
   gagggagttg atattatagg ggttgggttt ggacgttggg atttgttaga tttattttag 50040
   tgttacgatt tttgaattta agtgagttat ttttagtagg ggcgtggtta tttttatatt 50100
   aggagagagc gtttgggagt gggttcgttt cgagggtttg ttgggggttg ggagggattt 50160
   atttaaatgg aaagaagttt ttggtgatat tttttgaggg ttaggggttg aattttggaa 50220
   gttacgtttt taggtagttt ttacgggatt ttatgttgag tgttgattgg gttttatata 50280
   tttttatttt ttttatttcg tatttttata cgtggttggg ggtgattgaa gtttcggggt 50340
   attcgttata ttggttaaat aggttagtta cgtttagcgg ttttttagtt agtagttatt 50400
   ggtatttgat tcgtcgtttt ttatttttta tggttaattt tttttttaag gttttttggt 50460
   tttacggttt tttttatttt tatagtgaac ggtcgtgggg agttgttgtt attggttttc 50520
   gggcgttagg ttagagtcgg gggagtgttt gggagaagat tgtatgttcg gagtttttgt 50580
   ttttatttga atgggaggga ggttggtggt agattagaga tttgggtttt gagttaaata 50640
   ttgaaattta aggttttggg tttgttattt aatatttgtg gaatttgatt ttttttattt 50700
   ataaaatggg tattattaat attagtcgtt aaaattatag taagatatat acgtttaggt 50760
   taggaagtgc ggttatttag aaaatatagt tttttttttt tttaagaagt taattttttt 50820
   agtattggga gtgagaagag tttgtttttt ttattgtttg tagtttagtt tagggtattt 50880
   ttattattgg tttttaaagg gtagttttag aaatcgttga taaaggatgt tagaaggaat 50940
   ttttttttgg ttaggtgtta gggttaatag aaagtttcga ggatgtagtt tttattacgt 51000
   ttttgtttcg tgggtttttt ttttttcgtg ggtagaattt tttttttttg gttttttttt 51060
   tcgaattttt ttttttttgt ttttggtttt aatttgtttt ttagtttttt ggttttttag 51120
   gtttttttta gggtttaaga ttttgttttt ttttaagttt agtttttttt ttttttttgt 51180
   ttttggattt ttgagttggg aggttagtat tttgggtgtt ttgttgaggt ggtcgttttt 51240
   tttgtagtgg ttttttttgt tgttattaaa tgagggtttt tagagtatag agaaggtatt 51300
   ttcggtttgg ggttgtattt tttatataaa gtttttgttt tttagtattt tttaaagttt 51360
   tttatcgttt agtatatatt tagtatttag gtagagtagt tttagagttt tttcggttgg 51420
   ttttcggttt aattttttag tagaaacggt atatgggtta tagacgtaat tttatatttt 51480
   ttagtattta taataaaaaa tgtaagaata agttaggtat agtggtttac gtttgttata 51540
   ttagtatttt gggaggttga ggtgggtaga ttatttgagg ttaggagttt aaaattagtt 51600
   tggttaatat ggtaaaattt tgtttttatt aaaaatataa aaattagtta ggtatggtag 51660
   tagttatttg taattttagt tatttgggag gttgaggtag gagaatcgtt taaatttggg 51720
   aggtaaaggt tgtagtgagt cgagatcgtg ttattgtatt ttagtttggg taatagtgtg 51780
   agattttata tttaaaaaaa aaaagaaaag aaaagaaaaa ggaaattatt aaaaaatgta 51840
   agaataggtg atattaattt taatattatt tatttaatag agtttaaaat attattaatt 51900
   taatatgtaa ttaatgtaaa aatgatagat atttgatatt tttttttttt ttttttgtta 51960
   ggattttgaa atttagagtg tattttatat atacggtatt tttttatttg gaattattat 52020
   attttaggtg tttaaatggt ttcgtgtagt tagtggttat tatgttggat agtgtagttc 52080
   ggagagtttt aatttttttt tttttttttg gttgtcgggg aatagagttt tgttttgtta 52140
   tttaggttgg agcgtaatgg cgtgattttg gtttattgta attttcgttt tttgagttta 52200
   agtaattttt tcgttttagt tttttaagta gttgggatta taggcgtata ttattatgtt 52260
   cggcggttat tttttgtatt tttagtagag atggggtttt attatgttgg ttaggttggt 52320
   tttaaattat agattttaat tgattcgttc gttttagttt tttgaagtgt tgagattata 52380
   cgcgtgagtt attacgttta ttttcggata gttttaattt gattttagga taatattagg 52440
   ttattagaga gttgttgatt tggattatat tatgatttga attttttaga ttattttcgg 52500
   tattatgttt ataagttata tttagaggat aataggatat agtttggtga aatatatagc 52560
   ggtatttttt ataagatatt ggtgaggtta tagattttat tattagtgtt attcgtttgg 52620
   ggatatttta tttttagaaa atgtagagtt ttttttaaga aatgaatgtg gttaggtgtg 52680
   ttggtttata tttgttattt tagtattttg ggaggttaag gcgagtatat tgtttgaggt 52740
   taggagttta agattagttt gattaatatg gtgaaattta gttattatta aaaatataaa 52800
   aattcgttag gcgtggtagt gtgtgtttgt agttttagtt atttaggagg ttgaggtagg 52860
   agaatcgttt gaattcggga ggaggaggtt gcggtgagtt gagattacgt tattgtattt 52920
   tagtttgggc gatagagcga gatttcgttt taaaaaataa ataaaaaaag aaatgaatgt 52980
   tttaggtata cgttaagtat tttttagacg attttaaaga tattaattat tgtgattgac 53040
   gacgattttt tttttttttt gggtgtgttt gttttgtttt tttagtttat aggtgtttag 53100
   gatttaaagg tagttcgtta taggttatgg gatttggttg gtttaaaagg gagagtgagg 53160
   ttattggaat ttttttagga gtgagtttga agtttggaat gttttttggt ggagggtttt 53220
   gaagttagga ggcgatgtag acgtgggggt tgagatatta tttggggtta tttgtaaaga 53280
   gggatattta ggtagtgatt ttaaggaaag agtaagggga ggagagggcg gtatatttac 53340
   gtttttttaa tttttgttcg ttcggaggtt tacgtatttt atacgtcggt tttttcgtat 53400
   ggatattttt ttaataagta atttatcgtg tttatgttta tttattttaa agttatatag 53460
   aattgtattt attaaaaatg aatttatgga gatagaaagt attttggtgg ttgttaaggg 53520
   ttggagaggg cgaatgggga gtgagttttt tatggatacg ggtttttttt ttggggtgat 53580
   gaaaaagttt tggcgttaga tagtgttgat ggttgtacga tattgtgaat gtatttagtg 53640
   ttattaatgg taaattttat gttttgtgta ttttagtata ataaaaaaat atatatttaa 53700
   agtaatagaa tttaacgtgt tttttttttt ttttgagtta gtttgttttt ttgttttata 53760
   ttttaaaatt agaagggttt tgagatatat tataggtttt tagggtaggt tgaataattt 53820
   ttttttttta aattgtgtta ttttatgtgg taatagggat tttgtagatg taattgaaga 53880
   tgttgagatg aagagattat ttaggtgggt tttaaattat aagcgttttt taagagggag 53940
   gtaggttagg tgtgatggtt tatggttgta attttagtat tttgggaggt tgaggtaggt 54000
   agattatttg aggttaggag tttaagatta gtttggttaa tatggtaaaa ttttgttttt 54060
   attaaaaata taaaaattag ttaggtgtag tagtatatgt ttgtaatttt agttattcgg 54120
   gaagttgagg taggagaatc gtttgaattt aggaggcgga ggttgtagtg agttaagatt 54180
   gtgttattgt attttagttt gggtgatttc gttttaaaaa aaaaaaagag gaaggtagag 54240
   ggagatttga ttgtagagaa ggaggaggtt gtaggaggat ggaggtagag gttggaggga 54300
   tgtagtcgta agttaaggaa ttttagtaga gttttttatt taaagttgga agaggtagga 54360
   atggattttg ttaatatttt gattttagtt cggtgagatt gatcggtttt tgatttttag 54420
   tattgtaaag gaaggagttt ttgttgtttt aagttattga gtttgtgata agtagttata 54480
   gcggtaatag gaagcgaatg tagtttttag tttatttttt cgtttatagt aatataggcg 54540
   tttttttgtg tagaattttg ttaggtagtt agtgagcggg ataggatttt ttttcgtttt 54600
   tattcggggt tttgtgttgg attttttaag gttgttgttt ttgagatagt gaatttttta 54660
   tatttataga gatttatttt aaagttgaga aagtgttttt tttaaggggc gttagaaaga 54720
   aggttttaag gtgtttttgt tatatgttta tttgtttgtg ggtaatattt atagtttttt 54780
   ttcgtgattt atttaataga agggttatag gggagggagt attattggtt agtttatttt 54840
   atagtaaggt tttattttgg aagttatagg ggatagttgg gatttttatt tttaatttag 54900
   ttaattggga gaagagttta gaatagtttt ggaggttgat atttgttagg aagtgtggtg 54960
   atatttacgt gttgttgaaa tatatagtaa tatagtagta gagaggaggg gatgtggttt 55020
   tatagttttt tttttattta tttagtacgt acgtatatat atatatacgt gcgtgtatat 55080
   atgtattaat ttttagtttt taggttttgt attttgtttt tgttaagttt ttcggttatt 55140
   attaagttag gttgtgagta tagagtttag tttttttgtt aatttttttt taatttattt 55200
   tgggtaatat tttaatggga tgtagtagat gttttttatt aaatgttaag agtaatattt 55260
   ttttttattt tttgtgagtt ggcggtcgcg tttttgtatg ggggtgcgga ggaaagggtt 55320
   ggggagtagg aatgtggttt tgagggttgg tatgtatttg tagttgtgac gtttcgaggt 55380
   agtttatatt tttatttata gattaatttt gagaaggggt tgggagtatt tatagttttt 55440
   gttatttttg ggttggtagg ttggggaaat tttgagtagt tcgagtgaag gtcgttttta 55500
   ggtaggtatg gtgggataga ggggtttttg tatatttttt gtgaggtagc gtagttgttg 55560
   ggttgtttta gtatatttgg gtgatatgtt ttttttttgg ttttttttta tttggtttgg 55620
   gagaacgttt aggatgtttt aattttaaaa ataagtaaat tttttttttt tgatttattt 55680
   atttttttaa attgtgaatt ttttttttgt ttgaatagtt taatttttta aagtaggttt 55740
   atattacggt ttttagtttt ttatcgttta tttatttttt agttcgagtt atttggtgtt 55800
   cgttcgtttt attattaatt tagtttttga aaaaattttt atggttagta tttagtttta 55860
   ttttatttga tttcgagggt agtgtttgat acgtattagt tggttttttt tttttttttt 55920
   tttttttcgt attttttagt gttggggttt agatagtttt taagggtttt ttcgtttttt 55980
   gatttagttt tttggagtga ttatattttc gttttagttt tttagtttag ttgttttttt 56040
   atattttgtt ttaggttttt tttttggaat attgtttgtt tttagagatt taagttttta 56100
   ttttttatat tttttttttc gatttttagt ttttttatta aagttttatt ggttgttttt 56160
   tttcggtgtt ttaaattagt ttttttttgt ttagatttta taaggttagt ttttatttta 56220
   tgattgtttt tttggattat tgggattatt ttttattaat atttattttt tatttcgttt 56280
   ttttttgttt atgtagggtt ttgttggagg atttttttcg ttttattatt ttttttttta 56340
   aatagttttt taggtttttt tgttaaagtt gaaatttttt ttttgtattc gaggattttt 56400
   gtaatttggt taatttattt ttaagtttga atttttattt ttgtttaagg agtttacgtt 56460
   ttttagttag gttttattgt ttcgtaaatt ttatttaaaa ttttttttgg taaatgttat 56520
   tatttttagg aagttttttt gatatttttt attatatgtg atttttattt ttagagttag 56580
   attttgattt ggagattttg atttttattt atttttgttt tttttgagtt ttttaggttg 56640
   taggttttag tttatgtttg tgggtttttg tagtaggagg gtttattttt atttttttaa 56700
   gaagtgttcg ttttattttt cgttaagttg taatttttta taagtaggta tttttaatat 56760
   gtttttttat tttttttttt tttttttttt ttttttgaga cggagtttgg ttttgttgtt 56820
   taggttggag tgtagtggcg cgattttagt ttattgtaat ttttattttt taggttttaa 56880
   gtgatttttt tgttatagtt tttttcgtag ttgggattat aagtatgcgt cgttacgtta 56940
   agttaatttt tttttgtatt tttagtagag atagggtttt attatgttgg ttaggttggt 57000
   tttaaatttt tggtttgaag tgattcgtcg attttagttt tttaaagtgt tgggattata 57060
   ggcgtgagtt attgtattta gtttaatatg tttttttata ttttttttga aaagttatta 57120
   ttatattaat agtgtgtttt ataaattgat aataatttaa aatgtgattt attattaaaa 57180
   gtgtgatttt atttttaaat aatattgttg gttgtggttt gtagttagtt ttattatttt 57240
   cgtgtgatga ttattttgta gattagtaat ggttttaggt ttatatttaa tttaattatt 57300
   ttgttttttt aattttattt ttttttgagt taatattaag tgtgttttag agttgtaaat 57360
   ttatggttga tataaaatta ggatttttaa tatttaatat aaagtaatcg ggttttgtat 57420
   tggttttaat agtaattata aattacgttt gttttttgta ttgggtatat tgtatttagt 57480
   ttataatatg tgtttttgtt ttttttattt tttttattaa gatggtatat aggtttaaat 57540
   ttgttttttg ggagtaattg attttttttt ggaagatgtg agtagttttt atattatatt 57600
   taagaagata ttgttataag attttatatt ttttatttta gtttttaagg gtttatttat 57660
   ttttttaaaa atttatttgt tttgttataa tatgtgtttt ttttttttta tttgtttttt 57720
   taagatatag ttttattttg ttgtttagat tggaatgtaa tggtatgatt ttagtttatt 57780
   gtaattttcg tttcgtgggt ttaagtaatt tttttgtttt agttttttaa gtagttggga 57840
   ttataggtgt gtattattat attcggttaa tttttgtatt tttagtagag acggggtttt 57900
   attatgttgg ttaggttggt tttgaatttt cgattttagg tgatttattt gtttcggttt 57960
   ttcgaagtgt cgggattata ggcgtgagtt atcgtattcg gtttataata tgtgttttta 58020
   tttttttttt tttttttatt aagatagtat acgggtttta agtttttttt tttttttttt 58080
   tttttttaag attagttggt tgggtgtagt ggtttatgtt tgtaatttta ttattttggg 58140
   aggtcgaggt aggtagatta cgaggttagg agtttgagat tagtttggtt aatatagtga 58200
   aattttgttt ttattaaaaa tataaaaaat tagttaggcg tggtggtggg tgtttgtaat 58260
   tttagttatt cgggaggttg aggtagggga atcgtttgaa ttcgggaggc ggaggttaga 58320
   gtgagtcgag atcgtgttat tgtattttag tttaggtaat agtgtaagat tttgatttaa 58380
   aaaaaataaa ataagtggga agaggggaga tagtggaata aggagtttaa tttgtaattg 58440
   attgtgaata attaattgag ataatttatt attttcggat tagttacggg ttttattttt 58500
   tatttgtttt tttgagttac gttttttagt gaaattttat acgtatgtga ttaaatttgt 58560
   tttttttttt attaatttat tttgagtgag tttaatttat agatttttaa atattgaatt 58620
   taagagggta gaggaaatgt attttttttt cgttataaaa atattttatt tttttattgt 58680
   agtgtcgttt gttttattta tagtcggtaa tttggatttc gttttttaaa gtttatgaaa 58740
   gtaggataga ttgtttgtag gatgttttcg ttataaaagg agggttaggt ttggtaggag 58800
   gaaagaaacg gaagtggtaa acgtttattt tttgcgttag atagaggtat taggtgttta 58860
   ttgttttggg gagtaaatat ttttaaaaac ggaaagaaaa atttttagtt ttattggggt 58920
   gtaaaaagtt atgatttttt tcgtggttaa atggttgggt ggtttgggag ataagcggta 58980
   gttattttga agtttagttt atttgtttgt aaatggaggg taatgaaatt atttgttgta 59040
   tgggtgtttg ataggttgtt gtttaaattt tttgtttttt tttatggggt tttggggaag 59100
   attatatgag ttagtaatat aaaagtattt tgaaaattat acgaagtgtt taagtatggg 59160
   ggcgttggtt gattttaggt ttgtttttat aagttggtag aaaatttttt agagagggtt 59220
   ttgttttttt tttcggggta ggggtcgtag tgttagattt gatggtattg gtggttgttt 59280
   aggttatttg gtatgagatt gttgttagtt ttagatttat tttaagtttg ggagggaagt 59340
   aggtcggggt gggggttttg aaatttttta tatttttggt agagatatta gggtaggtaa 59400
   aagtgttatt tatggtaaat taagaagttt gtttgaattg ggtttaaatt agtttagttt 59460
   agaatggagg ttagttttat tttttttttt tagaattagg agtttagtat attagaggtt 59520
   gagaaaacgt tggaatagtt tttttatttg tttgtttaga gatagggatt ttaagttatt 59580
   ttttttttta gtttatttta taaatttttt gtgttagatt ttttttgttt aggagttata 59640
   ggttttattt gggagttatt tagggtttta aggtttttat tatttagttg tttattttat 59700
   taggttaggt ttgtaggtat ggagattagt aggatgtggt ttaaatttgg ttgtacgttg 59760
   attagttgag tgattttggg tttatttttt aatttttggg aggtaatgtt tttttatttg 59820
   taaaaagggg aaagggttta gcgaggagag tgataagtaa aggagatggt atttgtgtag 59880
   acggtattgt gcgggcgttt agcggatgat tacgcgttat ttttttgttt tttttttttg 59940
   tttttgtggg agcgtgggta gtagggagtt aagaggaagg gagtagttgt tcgtttggga 60000
   cgtaggtcgt tttgaattgg aagagaaaaa ggaataagta gtagttgttt attgataagt 60060
   tttatgtatt tgtgttttaa aattttaaag gtgtatttga aagatttttt gggtaaattt 60120
   ttttttatag atatacgaaa gttaggtgta gtggtttacg tttgtaattt taatattttg 60180
   ggaggcgaag gtaggcggat tatttgaagt cgggagttcg agattagttt ggttaatttg 60240
   gtgaaatttt atttttatta aaaatataaa aattcgtcgg gcgtggtggt gggcgtttat 60300
   aattttagtt atttaggagg ttgaggtagg agattcgttt gaattcgaga ggtggaggtt 60360
   gtagtgagtt aagattgtgt tattgtattt tagtttaggt gacggagcga gattttattt 60420
   taaataaaaa taaatttaaa aaaaaggtat atgaaaaagg taaaatgtta tgagtgtttt 60480
   gtcgtaatgt gttagttata aatgggaatt ggaggatttt ttaagttatt ttttttttta 60540
   tttttttttt ttttgtatat gtattaatag ttttttgggt gttggtagta ttaggggatt 60600
   tggggtgttt tataggtttt gtttgtttag attttagagt taaggaggag atataaaagt 60660
   taaaataggg tggtaagtgg aataaatggg gattagatgg ggtaatttgg tagtattcgg 60720
   taaggttttt tttaatgagt ttttaaagag gtttagtttt taggggaagg aggcgtttta 60780
   ggtagcgggg gtttgtagag gcgaaggttt agggcggggt ttttagggaa gtcgtttagg 60840
   atgttcgaaa ggaggggagt aatttacgag tttggtgata gggtcggtta taaagtgtta 60900
   tgaagtatta attaggttgt ttattagttt ttaggataga aagattatag tagttaaggt 60960
   gtggggattt gtttatttga tttttattta gtttagaaaa tgattgtttt agggttatag 61020
   gtaatagttt aagttttgta agttgttggt tatagggtgt agttgcggta aggtggggac 61080
   gtttattagt taagtttttt aggatttttg ggttgtcgga tatgtttgta ggacgttacg 61140
   ggggtaggtt agattagggg tatttttatt ttttttttta ggttttagtt tttaggttag 61200
   agtgtttttt ggtttttggg gtttttaggg ttgtagatag atagggagag gtttgtgtta 61260
   tggtgggtta tttggttttt gtaggaagtg gttagaggtt ttagtatttg gatatagtag 61320
   ttattttttt aatttttgtt aatgttgttg ttgttcgggg tttatttttt ttaatttttt 61380
   tttttaatta gggaggaaag tattttggag taggaattgg gttatttagt ttgatatttt 61440
   agtatgattt tgtatgattt tgggttagtt atttaatttt tttgggtttt tattttttta 61500
   tttgtaaatt aggtagataa ttttttaaag ttttttttag ttatagaatt ttaagtgtta 61560
   aaataaaaaa aatttataaa aaggaaatat gtgtttagtt tatattttcg tttttatttg 61620
   ttaaaagggg aaatggattt gttacggggt tttttttttt taaatagata tgggagattt 61680
   tttttatgat tgtagaattg gagtagttta gaggagttta gaatttttta gagtttgaga 61740
   tttatagggg aatttatttt aattttttcg ggatagaagt attgttgttt aatggtattt 61800
   aatttttatt attgttgtag taaatgatta tatatttgtg atgttaaata atatagatta 61860
   gttttatatt ttattttatt aggttatagg gaaggtgttt atagggttgc gtattttttc 61920
   gaggttgtag gggagattgc gtttttttgt attttttgtt tatttgtatt ttttggtttg 61980
   tggttttttt ttttttttta agtttttttt attgtatttt ttagatggtt tattgcgttt 62040
   ttaaaaattt ttgtgatgat agcgggttcg tgtggattat ttaggattat ttttttattt 62100
   taagattagt tgattagtga ttttagttgg ttttgttatg ttatttaacg tagttttagg 62160
   tgttggggat taggatgtgt ttaattttgg gagttattat tttgttaatt ttaagatata 62220
   ggttttggag ggagatgaag ggtaggatta taggtttagg aggttgtttg cgtgggttcg 62280
   aattttggtt ttattattaa ggaattgggg gagtatatta tttgtaaaat gggggtcggc 62340
   ggggaattag ttttttggtt gttagcgtag agtgaggtag tgtgtgtagt gtttggagta 62400
   gtttcgtgag tatttgtttt tttttcgtta aatttataag aattgatttc ggtttaacgg 62460
   tttttttaaa tttttagttt ttttcggtta taaggttagg agtatttttt tgtagggtta 62520
   tttaagtttt tagttttttt gaaagtttcg gtttatacga tgttggaagt agatgtgtcg 62580
   tgggttgttt atttatgtat ggttatcgga gaggttcgga tgatgtcgtt tttaatgggt 62640
   gtatagtgta gggtagggtt aggtattaat aattagttat agttattagc gtaagtaagg 62700
   ggtttttatt gttatttttg gtttggggtt agggagaagt ttcgggaggt atttgatttt 62760
   tttataggga gtggggttat aggagaatgt attcgaaagg ggttgggggg tagtagaaag 62820
   ttttttttag aaattttatt tttggttcgt gatagatgat ttttttaggt tattattggg 62880
   gtttatttgg gtttttgtag tatggagttt ggttatttta gaaggatgat tgggtaggtt 62940
   agggttattg gcgatggtga cggtaggttt ttttgaggtt tttttattag aagtattagg 63000
   aggttagagt agtcgataag tttttatttg gtttttgagg ggtagaaggt tttgagtagt 63060
   tttttgattt ttcgaggttt ttttatttta ttgagagatt ttcgagagta ttgagagtta 63120
   ttttttggtt ttttcgtgag agagttggtt atataaaggt agttatatat ttagtaagat 63180
   gaatttataa tttttgagtt taggtttatt tcggtttttt tatagggagt ttttgggggc 63240
   gtggggtggg ggtgtagaag ggaggggtcg gtgttttatt tttgggaatt aggttatgag 63300
   ttttggattt aggttaaatt atagaggttt tatttttttt taggtttttt gttttttaat 63360
   tatttttgta tagggtattt tttttttttt cgtagggttt tattggggtg gggtgaggag 63420
   gagttataga attatagtag ttgttatacg ggggtgttaa ggtagggatg ttgatgttaa 63480
   tcgaatgggt acgggaaggt agagtaggag gtcggcgttt tgggtttggt taggttgtgt 63540
   gattttaggg aatttatttt atttttttcg gtttttagtt ttttttattg taaatcgagg 63600
   gagtgaggtt aggtgatttg tataatcgcg tgaatttgtg ttttgtattt tttatttatt 63660
   attttaaata atgtttttaa attttagttt tttaatgttt cggtattttt ttgtttgaga 63720
   aatatttacg gttgtttttt atgttgtgtt ttaaatatag gtattttggt ttttttttgg 63780
   gtgtttttat tagggttaga atgggtagtt tattttgtag gaatttttaa ggatttttag 63840
   ttatttttag gatgggggtt ggttgttcgg aagcggtttt tgtttatttt cgtcgtagtt 63900
   tttattacga ggttagtgta gtaaaatagg aggtgtaggt ggtcggggtt aggggttagg 63960
   ttattttttt tagcggggta ggagaggggc gagaaaagtt ggatattgat tattttaggt 64020
   tgagggtttg cgtggaggag ggggtttatg gtaaaggaag tgggaggatg ggaaggagtt 64080
   tttgttggat atttttattt cgttggtttt agtttcgttt tcgttttatt tgcggggaga 64140
   aagtgaggtt ttttgttttg ttaaatttta gtaatgaatt ttatatttat tttatttttt 64200
   gtttattgga aatttttttt ttattttttg gattcgtttt gaatagttgg gttgtagaag 64260
   tcggtggaat gaaatatgta gggtggttta gaggggattg tgtcgaatag cgttgtgttt 64320
   tcgtttattt ttttttttga agataaggtt tttttgtttt tgtttttttt ttgggaaagt 64380
   tggagagggg gttgaggaaa tgggatttat tattaatttt ttcgtgtacg ttggttttta 64440
   gtaggtattt ttatattttt taattaggtt cgtagggtgg aaaggtagtc gtcgagttta 64500
   atttaatttt tatttagtcg tggaagttag tgtggatggt aaagtattta tgtataaata 64560
   tatatacgag gttgaggtta agagaatacg cgtttagttt ttttttggtt ttttagtaac 64620
   gtttttatgt tttagttttg ttttaagttt taaacggtgt aaggtttttt taggtggagg 64680
   aatggaagtt tttaagaagt tcgtgtttta gggattatat agaggtaggg tttaggagta 64740
   aaggttttta gatttcgtag tgcggcgttg gtaagtttgc gttgttgtat ttatattgtt 64800
   ttgggatgaa ggcgatatgt tgattttttt taggtggttg taggatagtt aggaggaggt 64860
   gagatgttag ttaaaggtag ttatagataa tagtattacg gattattaga aacgttattt 64920
   tcgtgttaaa gcgttcgttt tttttagttg ttttattttg tgttaatatt ttttgggagt 64980
   gatatgtgtg agattgggtt gtgttgatga gtgattattg gtatttttag attatattaa 65040
   ggaggggtta ttttgttgtt aattaggtgt agggtaggcg gtattggggt acgacgattc 65100
   gttcggtatg gttgttttat tttttttttt gtttgagtat aggttggtag gtttagagga 65160
   gaggaggaaa tagagttatt atggggggcg tgtttagggg tgagttattt taagaagtta 65220
   tagaattagg gtatgggaga tattgtttag ttttgttatt aatttgtcgt gcgaagttgg 65280
   ttaagttatt gtattttttt gggttttagg ttttgtaatt agagggttgg tcgaagaggt 65340
   aaatgatagt ggttagggat gggggtattg aagttagatt tttcgtattc ggattttttt 65400
   tttgttatgt ttagagggag gtttgggtag gttatttaat atttttgggt tgtgggtttt 65460
   ttatcggtga aatgggaata ataatagaat taattttata gtgttgttgg gaggattaaa 65520
   ggggatattt attttaggta aagagttttt aagatagttc ggggttttta ggaagttttt 65580
   gttattatta tcggatatta ttatttttgt tacggttgtt agggtttcgt ttagtatcgg 65640
   tagtttgatg tcggtagagg aagggtgagg tttttgtttt tttgtagcgt tttttttttt 65700
   tttttttttt tagttatggt aataatagtt gaagtttatt tttagtttta gcgattggtt 65760
   atttttttga attaaggatt gaaggtttta ggtttgttat ttaaggttta ggtgtaagag 65820
   gggtgagtta tcggtttttg tttttattat atttaggttt ttatacgttt ttttaaatag 65880
   gtttttgttt tacgtgtaag agaagatgtt aatttttacg tttggggcgg gggtgtttat 65940
   aaatgttttg gacgtgagtt ttgacggggg ttatggtgta ggttttgtta gggtattttt 66000
   aattataggg gattatttgt agtttataga gggttttttg taggttaatg ggattttgtt 66060
   attttggttg ttattttgtt tagttatttg gtaaggaaaa taggttgtag atttagaggt 66120
   agtacgggtt tatatgttag cgagataata taataaggga aggatgtgta ggacgaggga 66180
   gttatggaga ttttatcgta gttttagtta tttattagta gattttgtgc ggacgtcggg 66240
   taggttattt tattaatttt taggatgttt ttgtaggttt tgcgggtgga tagggtgaac 66300
   ggaggtataa ggagggtaag tttgataggg gttggggttg ggtttggagt tagaatttta 66360
   gttagttttg ttatcgtatt ttagggtatt tttcgtagat agtaggttaa agtaaggttg 66420
   tttagattaa aggggaaggg gttttgtttt ttttattaag ggttttttgg agattttaat 66480
   atagattttt agtttataga tattaaattt agggtttttg agaagtgaga aatgtgaaaa 66540
   acgtatagaa tagttgtagt ttagcgtttt taatacgtta gatttatata ggtatatata 66600
   gcgtttttgt agaggttaga aagttttgtg ttgggaagtt tttcgatcgg gggaggtttt 66660
   atagtaagtt tttttttttg tttatttatt aacgggcgga aggttttaat taggattata 66720
   tgggaagggt tagcgggagg ggtagagagg gaaaatgaat aggtaatatt aagtgtttgg 66780
   ggcggggggc gtaggggata tttaggaggg gagggtaggg gtgtattttt tatttaattt 66840
   atagggaacg ggggttcggg aggtgtagga gaaggtttcg gtatttttat tttttttttt 66900
   ttgtagattt atttatagtt tgtttacggg tagttagatt tagttatagg ttagacgttc 66960
   gttttagttt tatttttttg gtttattgaa agtaagttag gatagtgagg gggacgatgt 67020
   tgttattttc ggtatggtta tgttgggttt cggggtgggt gggaacgtgg ttggggcgtt 67080
   attttttttg gtcgttatag gtataaaagt tttttcgtgt tttagtgtta tatgtgggtt 67140
   ttttttttgt tgttttaata ggacggatat ttaagtagat tttgttggtc ggggggggtt 67200
   ttcgtattta gttttatttt gatttagcga attttttttt tgttttttgg ggtaggagtt 67260
   attaggtcgg gttttaggta atggttagtg aaggggttgt atggaggggt tttttaggtt 67320
   tataggaagg gaggtttttt ttttttgtaa cgtggttgta gtattttgtg atcgagaggg 67380
   atattacggt tggagatggt gggatggagg attttttata gtattttcga tgtttagaat 67440
   attttatttt gtataaaaaa gttttaaatt tttttgaggg ttgaagttat ttagagttgg 67500
   ggtttttgtt ttttaggtta aaagtatttt gtcggagtag ttgagagtat agcgggaggg 67560
   gaggggtagg agggaatagt ttcgttacgg cgatagggga ttttttgatt ttaagagggt 67620
   ttgtagatat tattatttat ttttagtttt gaattgtttt ttgttaaggg gttagaaatt 67680
   tttgggaggg ggattatagg gggtttcgtt tatgagaata gtgattgtgg ttaggttata 67740
   gtttataagt tagtaatttt gtttagtcgt cgcggtggtt tttttttgat ggtttttaga 67800
   aaaagcgttt tattttttgt tattagtgtt ttgaaattta agaatatttt ttaaatagta 67860
   attgtagtaa aagttaatgt tgcgtgagtg tcgtaggtat tatttttagg gttttatata 67920
   tttttcgttt agtttttata aaaattttat tttatttttt ttttcgtttt ttttttttaa 67980
   tagtttagaa aacgagtacg gaagggttaa gttggtttga ggaggggttg gtggaatagt 68040
   ttgagggttt agtttttaag aattttatat tgtaaaggga atttttgggt tagggagtag 68100
   ggtatagttt ttattttttt attttcgttt ttagtattag gtttgatata gacggggttt 68160
   cggttatgaa taaatgaata agtgaatgag agtttgttga gggaagaagg tgatagggat 68220
   aagggagaaa taaagtagtt aatgtcgtcg tgatgatgac gatgttaacg gtagtattga 68280
   tatgtgggtt tttgttatgt taggatcgtt ttaagtattt tgttttatga atatttttgt 68340
   tttttattat gtttcgagtg gatattttta ttagtttatt ttatagaggg gaatatagtt 68400
   gagcgtattg gttaaggtta tgttgttagg aaaggttaga gtttaggtag tatgtggttt 68460
   tttagtcggg ttttggttta tacggtttta taggtagttt tatgaaatac gtagagagag 68520
   tatgtgaggg gtttttttgt tgttttttat ttttagttgg ggtttatttt ttgttgaaat 68580
   ttttttaatt tgtttttaag tttcgttagt tacgaaatat ttttttgaat atttagtatt 68640
   atttttttat ggttatttgg ttttattttt ttatagggta agttttgggc gaaaagtaga 68700
   aaggatgaaa atttttgaga gaggtgataa cgtggtaatt tttttttttt tttttttttt 68760
   tttgagatag tattttattt tcgttgttta ggttggaatg tagtggtacg attttggttt 68820
   attgtaattt ttatttttcg ggtttaagtt atttttttgt tttagttttt tgaggagttg 68880
   ggagtatagg tatgtgttat tatattaggt taatttttgt gtttttttgt agagatggag 68940
   tttcgttatg ttgtttaggt tggttttaaa ttcgtgggtt taagtaattt gtttgtttta 69000
   gttttttggg attatagacg tgaattatcg tgtttggttg aggtggtttt tatataaagt 69060
   agagaaaatt atttttagtt taaaataaat taataagtat tgagtgttta tgtagaaaaa 69120
   gattagaagg aaacgtgaat atgttaatag tgtttgtttg gaggacgtaa ttattattga 69180
   ttattttttt tatatttttt aatttaatta ttttgttata agtaggtgtg atttttaaaa 69240
   ttataaaaga taaacgttgg ttttttgttt gtgttttgtt ttttgagata gggttttatt 69300
   ttgttattta ggagttggag tatagtggtg tggttatagt ttattgtagt tttaaatttt 69360
   taatttaagt aattttttta tttgagtttt ttgaggagtt gggattatag gtaagcgtta 69420
   ttatttttag tatttttgtt gtttagattg gtttcgaacg tttggtttta agcgattttt 69480
   ttatcgtagt tttttaaagt tgggattata ggcgtgagtt attacgttta gtaaagattg 69540
   ttttttaatt aggaagatag atgtttttta gtattttttg tttttttttt tttttttttt 69600
   tttgatacgg agtattgttt tgttgtttag gttggagtgt agtagtatag tttcggttta 69660
   ttgtaatttt tgtttttcgg gtttaagaga tttttttgtt ttagttttta gagtagttgg 69720
   gattataagc gtgtattatt atatttggtt aatttttgta tttttagtag agatggggtt 69780
   tcgttatgtt gattagtttg gttttgaatt tttgatttta agggatttat tcgttttggt 69840
   tttttaaagt gttgggatta tatgcgtgag ttattatttt cggtttgttt tttagtattt 69900
   ttaatttttg ttttttggta ttatttggtt aagtagatga aaagttttag tgagttgtta 69960
   gtcggtatta ggttggggtt ttttttaggt agttttaagt ggttaggatt tggttttttt 70020
   tttagagttg ggtttagaaa ttaagatcgg gaatgcgtga tggttgtttt gcgggttttt 70080
   gttatgaggt tatttttttg gttttagtga tcggtcgttg gttgtaggga gtatgtgggt 70140
   ttagttaggg tttgtgttaa tatggttttg ttttatttac ggtttagtgg gtttttttat 70200
   ttttcgttgt atgataattt taggaaaaat gttaggaggg agggagtcgt tgggggtgtg 70260
   ataggagaga ttttttaggg atagatttgt aaaaattgtt ttaaagtttt ttttaaggat 70320
   ttttaaggtt tttattgttt tttattgata ggttacggtt cgggtagaga aagttgggga 70380
   tggagttggc ggggggaggg tggttagtat tcgtaaggta aggagatgag ttgggagaat 70440
   aggggttggg agggtagttt taggttagtt tttgttttta gaattattgt tttttttttt 70500
   tggttataaa gtgaggttgg ttagatatag gtttatttta taggttggga ggttgggcgt 70560
   gaaagtattg tgtttgtatg gggtatttta tggtgaatat tattagagtt aatttttagg 70620
   gtttgttgtt ttatagggaa gttagtgtaa ggcgtttatg gaatttagaa gtatttaaag 70680
   gatttgatag ttatgagttg ggaggattta gttttgtagt aggagaaaga gaggtagaga 70740
   gataggtttc gtaatatttg ttttattcgt tgtgttgttt tgagtatagt ttattttttt 70800
   gtggtttcga gttttagttt tgaggttttt aagatttttt gggttttcgt agtttaggat 70860
   gtttatttga aaattaggtt tagttagtag ggtttttttc gttagtttat tattggttag 70920
   tttttgttta gtatttttta gtttattatt tttatttgtg taaattttat attagtattt 70980
   taagtttttt tttttttagg aaggagattg tttttagatg tgttttttgt tttttttcga 71040
   ttaaagttta gaaaatagga attagttttg cgtttttttt ttttatcgaa ttgttggttt 71100
   ttttggatgt tttttttgat tttttgttaa tatttttata atataattag tttttagttt 71160
   ttttttaaat gtaatcggcg agtacgttgt agtttaggcg gacggtgatg tttttatttt 71220
   gtaatttgcg agagttagta tattttttag ttaggtgggt tgtaggagtt agtcgtggcg 71280
   agggtaggag gtttgcgtgt tgacggtatc ggtattatta aaattacggt tagattaagg 71340
   ttacggttaa cgagtttttt tatttgtaat ttttttttta ttaaggtggt tttagtttta 71400
   gtttcggttt tagttttggt tttggtttta ggagttgtag atatattttt aagtttttat 71460
   tttaggagtt tggttttata tatatcggag gatacggagt tggggtataa atagttaaga 71520
   ataggtatat ttgtaattag gagtaggggg cggagggttg ttgagatgtt tttattttta 71580
   tcggttgttt tttttgacgg tttattattt attatatatt gattgtataa ttagggttgt 71640
   gatatgtttg ttttttatat tatattatta aatttttaaa tttgttttta atttatatac 71700
   gatgaggtaa atttagagtt aataatttgt ttattagttt ttgaatttat gggtggtttt 71760
   aatttttttt tttttagttt ttttgggttt atatgagtat tgaggtataa gggaatttta 71820
   ttagttggga agtgtgtttt ttttttgttg attttatagt ttttttagtt tgtgtttgaa 71880
   gttgttaata tttttttatt tagtattgtt ttttgtagtt tgagttgtcg ttgtgtgatt 71940
   tagggttagt gttttaattt ttttggatta tatttgtaag atgaggatgt cgtcgtatag 72000
   tttattttaa atggttatta agttgtttgt atagttaata tttgtcgagt ttgtgatacg 72060
   attagttttg ttttaatttc gaatgtggta tttttattta gtttgggggt ggaattgtta 72120
   gttttttttt ttttggataa ggaagttgaa aggttttaga aggtattaag gttagttgat 72180
   ttgttagtag gtatagtgtt tatttgttat ttatagtttt tttttgatcg tttcgtttgt 72240
   ttttttagtt gagattttgt gtgtatttga atattttgtt aatggttgaa tttgtattag 72300
   ggttattgtg ttagttgggt ttgttaggtt atcgttttcg agttttgttt aaggtagggt 72360
   tagtgtttat attttgtttt tttgttagtg ttttatttaa ggttttggaa aggttttgga 72420
   gttagagatt cggttaaatt ttggtaatgt tttatatttg ttgtgtgatt atgggttagc 72480
   gatttagttt ttttgtgttt ttcgggttgt tgtgagggtt ataggagatg tatagagggt 72540
   tttgagtttg gtggttggta tgttgtaaag gttagttacg tggtgtaagg atggtattga 72600
   tgataataaa tgaatcgaac gaaaattagg ttaattgggt ttggggaggg gcgttggtta 72660
   aatagattcg agtggattag gattttgtat atgatgagta agcgggaata gttttaaata 72720
   gtaagtcggg taggtttagg atgatttagt tttgggtgtt gtttagggtt ttttttcggt 72780
   ttttttgtcg gtttggtttt tttttgtttt agtttgattg tttttatggg ttttaataag 72840
   tttttgatta ggattgaggt agaatagata gggtttaaga ttttgtaggt aggtatagag 72900
   tgtggatggt aaaaggagat gttttaggtt ttttgtcgtt tttttttttt cgaaggttga 72960
   taatgttaat ttatagaaga agttgttttt ttcggtttcg gattttattt taatatagtg 73020
   tatgaggtag ttgtgtttaa ggggtgtgat tatttttgta gatggaattt atttattgtg 73080
   tttagtttat tgttagagtt cggaggacgg gggaagtgag gtggggtatt ttttatagtg 73140
   aagggaaaat ttagtttagt tatttttttt tgtgttgttt ttggaattta ttttgttatg 73200
   gttttatttt aatttagttt tttaaagttt tagagagttt tgtttttttt tttgtatttt 73260
   atttcgttta tttgtgttgg gttagttttt tagaagtttc gttttttgtt atttttaggg 73320
   tattaaggtt ggttggtggg tttagggatt tttatttttt gatttagttt tagtggttta 73380
   ttttatattt atcggtattt ataggtgtgt tttttatttt cggtttttgt ataggcggtt 73440
   ttatattttg ggagtttagt tttttttaag gatttacgtt agagtatagg gatttttgtt 73500
   tttgattttt ttttagtttt aattacgagt agtttatgtt atagtaattt tttagttgtt 73560
   tgtagttgtt tgttaagtta tgataatggt ttttgttttt taatttaatg tttgtatata 73620
   gataaatcgt tatattttaa gtagattaga tattattttt tttaggaagt tttttttgat 73680
   tgttttagtt ggatagaggt tttttttttt tgtgtttttt taatgtttaa taggtattta 73740
   ttgagtattc gtgatatgta aggtatttat aatataagtt agtgtttttt gtttttttag 73800
   agggtatatg aataaataaa cggtaagatt ttttaatatt tatttatgtt ttatagaaag 73860
   ttatttatag gtatgggagg gttgttttaa ttgcgggtga tagggaggtt tttttaagat 73920
   ggtagcgttt tagtaagatt tgaatgaggc ggttaggagg tttagggagg gattttttag 73980
   gagggaataa gtgaatattt tttcgtgagg atgagttttt atgttttagg agtagttagg 74040
   ttaggggatc ggtgtagtga ataggggttg aggggagtgg ttaggtgacg tagggtttta 74100
   taggtcgttt tacgatgagt tagcgtttta agtatagtag atatttaggg aaggtttagg 74160
   ttggggagtg atttgaatag attttttatt taggagttta tcggggtttt tgggtttacg 74220
   ttgttgcgtt attgtatgtt ttcgggggtt cgggggggta ttttttatat tattattata 74280
   ggtagtattt ttgggggagg tgtgttgtgt aggggattcg ttggttattt tgtcgggtaa 74340
   attatagggg attgtgtagg ataggcggtt agtttaggag ggataattat ttaagtaggg 74400
   gttagagggt attcggattc gggaggagtg tttaattttt taggtttagg taattagagg 74460
   tggggggcgg ggtgttgttt tttgatatgg gaggtttggg ggtttggttg tattttttgg 74520
   tgtggaggtg ttgggaggtg tggaggtttt tttttgaggt tgggattttt tggttatttt 74580
   gatttattgt ttcgatttta ttttttagtt tgagggtatg gtttgaattt atagtcgtat 74640
   atattttttt ttgtataggg ttttgcgggg tcggtatagt agtttttttt ttatagatta 74700
   ggatgtttag ttttaggagg gttgggattg tttaagatcg tataattagg gttagatata 74760
   gttgggttaa ttttgtgatt tttggtttta cgtatagatt ttaagtatat aggtaaaatt 74820
   ttgttggttg ggagggttgg ggggtagatg agcggggggt agtttttttt tagtttttta 74880
   atatgttagg tttttgggtg ggagggggta taaagatttt atttttttta agagatagag 74940
   aaagaggtag tggagggttt ttttttggtt gaggttttag gttttgagtg gtgaatgtaa 75000
   ttttatttgg aggttggaag ttaggcggtt ttgggggtgt gcgaggttcg ttagtttagt 75060
   tgtagttttt tttatgtaag ggaatttttt ttaggtagcg tttttatgat tagagagggg 75120
   gttaggaaga ggtgagggtt gtatttagag tgatattcgg gtgggtttta ttttggggat 75180
   acggttatat ttttttttcg gatttttgaa gtttttaggt gcgggttaag ggtttttagt 75240
   tgtaggtagg agagggtttt atagttatag atttggggag tttagtttgt aaagtttaga 75300
   ggttttcgga ggtcgttggt tcggtttttt gtgtagttgg gtttttttgg gaggtttttt 75360
   ttcggtattt ttagttttta gttcgtacga ttttttatag ttgggtttat ttttttaggt 75420
   tcgggttata tggtgtaggt tagtggggtt ttgggggagg gtagggcgtt aggaatagtt 75480
   tcgggggtgg ggggattgga gtagagttta ttttggtcgt ttgtttattt tgtatgaagg 75540
   acgttagatt tttatatgtg gttattcgat ttcgtttaat tatgcgtaag ttttggttat 75600
   ttttatttta ttgatgggaa ttaaattggt taaggagaag tttagaattt gagtagagta 75660
   ggggtttaag ttttttagga gttttaggta ttttttagtt ttttttttcg agtattgagt 75720
   ttatatattt agtgttcgga tgtttattta tttgggggtt cggcgatcgt tagttagtat 75780
   ggtttataaa gggtttttgt gtaataggtt ttatttgatt tttttaaaaa tttttcgagg 75840
   taggataatg ttatttaatt tattttatag ataaagtaat tgagttataa aatggtttgt 75900
   taaaagatat agaggtagaa agattaatag taaagagttt ttttgtgatt ttgattagta 75960
   aggtttttag aaaaaagtaa gggatttcga ggtttttata ggaatagagt tttggaggag 76020
   aataagagtt tagttttatg ttatttttaa ggggtatttt gaaggttaag gtattgggtt 76080
   taagtaggtt ggattgtgtt ttttgtattt tttttatttt ttgaagtttt ttttttttta 76140
   tttaggtggg tcgtggaggt gtttataata tataatggaa tagttggtag gttattgggt 76200
   tttttattga gttaaggggt tcgattagag gtttaaatag tggtatagaa attatgagtt 76260
   tcgttgtaaa ttagtttagg ttttggagtt aagtagtttt gtgtttatag ttagggttag 76320
   tcgtttttag ttgtgtggtt tgaggttagt tttttaattt ttttgtgttt cggtgttttt 76380
   tttgtagtag taataggaat cgttttttac ggcgatagtg tagatatatt ttatgaagcg 76440
   tataaagttt cgagtttagc gtttggagta cgttaggacg tagtaagtgg ttatttatga 76500
   tttatgaaat attaataagg tgaggggtgg aatgatagtt tttttgcgtt gtttttaaaa 76560
   tggaaataaa tttgtttagc gtttaaagta gtagaaagtg agttaaggat aggtttttag 76620
   cgatatgttt tggtagagtg ttttttgttg gttttaggta gggtttaggg cgggcggtta 76680
   ttttttagat gagtttatcg ttggtatttg tttttagagt atatgtttgg attttttaga 76740
   tttttttttt tttttttttg attttttttt attttgtaat ttttattttt tgttattttt 76800
   ttatttattt ttttttttta tttttgttta aaaataaata atggaggtta aatgtgttag 76860
   ttggtttttt ggaaaggttt ttttgtagta ggaacgcgac gggggtagtg gaggattgtt 76920
   tagcgtaagt tacgtgattt tcgcgcggag tcggggaagg ggaggcgttg agatcgaggg 76980
   tgttaggatc gaggtattga gatggggtta ttggataatg gttagggtta gttttcgttt 77040
   ttattttaat aggggttttt atatttaggg gttatatttt taaagttttt tggataagag 77100
   agataaattt tattataggg ttagaaacgt tgtttttagg tcgttatttg ggtgggggtg 77160
   agagatgttt cgtcggtttt taaatattta ttttgtggat aaaattatta aggggaattt 77220
   aatgttttta tagtagtttg gttaagatat aggtattagg tcgggcgtag tggtttacgt 77280
   ttgtagtttt agtattttgg gaggttgagg cgggcggatc gtttgaggtt aggagttcga 77340
   gattagtttg gttaatatgg tgaaatttta tttttattaa aaatataaaa attagttagg 77400
   tgtggtggtg tgagtttgta gttttagtta tttaggaggt tgagatagga gaattgtttg 77460
   aattttggag gtggaggttg ttatgagtgg atattatatt attgtatttt agtttggatg 77520
   atagagcgag taagatagtt ttaagaaaaa aaaagatgta ggtattgttt tagggtttat 77580
   aagtataatt tgatttttga tgttggtatt taattttgat gtagatttta gtaggaaaag 77640
   ggagacgaat tagaaggttg tggggacgag gggtttatag tagtgtttat tagatttgtt 77700
   ttggtaggta taagtgtttt ttggaggttg ttagataagt agagggttta ttttagagat 77760
   tttggttttg gagggttggg ttagggttta ggaagttgta tttcggtagg gaatttagag 77820
   ttattttgag ttttattttg gaagtaagag ttagggggta gaatagaatc ggaattaaaa 77880
   gtttttgtgg gtttagtttt atggggatgg gttcggtttt atggggatgg atttttgtat 77940
   tattcgaggt gattattagt tcggtttttc gggattagtt ttgagtgttt tttggggaaa 78000
   gcgggatttg tagaggatgg ttttttagga ttcgtttgat tatttatttt tagttcgttt 78060
   ttattttagt ttttttgatt tttatttttt atttagaatt ttggagattt ggttttggag 78120
   gtttagagat tagaaatgag gggagataag gggagaagtt ttttttttta ttcgttggtt 78180
   acgttaatgg gttgattttt taggaagtta tattagttat tgttattgtt ttggtcgttt 78240
   cgtttggttt cgaggttggg tgagtaagta tttcgttttg tttgtttttt tatttgagtt 78300
   ttttgttgat gttacgagag gtttgattac gcgtttagga ggtataggat agagtttaga 78360
   gttgtttaag aatatataag gttgaggttc gggttttgta tttgggaagg gttaggaagg 78420
   gttaaaggaa aattttagtt gagtataatg gtttatgttt ggaattttag tattttggta 78480
   ggttaaggta ggaggattat ttaagtttag gagttggagt ttagtttggg aaatatagta 78540
   agattttatt tttataaaaa aaaattaaaa ttaaaaaatt aattaggtat ggtggtatat 78600
   atttgtagtt ttagttattt ggtaggttga gatgggagga ttgttgagtt taggaggtcg 78660
   agattatagt gagcgggata ttgggttatt gaattttagt ttgggcgata gagagagatc 78720
   gttttaaaaa aaatttttat attttatgga gtttagtaga attttcggtt agggtgtttc 78780
   gattgtgaaa tgagcgtggt agttgttaat tttcgagggt gaagatttat ttattagtaa 78840
   tttgtatttt ggtacggtga gaggaagtgt ttggggaaag acgtcggtcg aggtagagag 78900
   tttagtttaa tttatcgtgg aggagttatt ttagttttaa agagaaagat taataaattt 78960
   taggtgaatg gtttaaaagt tatttttgta tacgtagagg gtgtattttg ggatatgtgg 79020
   atattttttt ttaggatagg aagtaaaatt agtaggtaga gacgtgagtt tttttgtttt 79080
   tgtcggagtt aatttggagt ttttatttcg gtttttaggg aagtatagat ttttattttt 79140
   atcgtagtta aaagattttt tgttgatttt tttagtagta tttagtttag ttttgtgggg 79200
   agaggcggta gggggaggag atagagaaag atgagttttt ggtagttgtg attgggggga 79260
   gagaaatgcg gttttaattt gtttttgttt attttataga cgggcggtgt tttattttaa 79320
   aatttagggt agttgaggga ttgaaggaag agggggatat tttttaattg gggttttttt 79380
   ttattttatt ttatggcgtt ttatatagga aagagttgga aggtttcggg tttttggtat 79440
   ttagttaaag ttttaggtag gggtttagat attgggttgt gttattattt taggtagttc 79500
   gggagttagc gacggtagag ttttattttt atttcgatta ttgtatagta tttcgttttt 79560
   aggatagttt agtagttagg ttgttttagg gtttaaattt agatatttaa ttgtgggggt 79620
   tttaggtaag tggttgaagt tttttgcgtt ttgatttttt aatttggaaa atgggatgat 79680
   gaaagtaatt gttgggtagg gtaaggggta agtgaaagtt agcgtttgag agtgatgtgg 79740
   ttaggatcgt tgttgtgatt atatattgag tttttttttt ttatttgaga taagggtttt 79800
   attgattgtt gttagaaaga tttagattta gaaggattag gaaagtcgtt ttatttataa 79860
   ttttagaggg aaggggaggt tttcgtgtta gttggttgtt gtaggggaga gggtgggaga 79920
   tagatggggg atagaggagt aggggtagta ggaggaacgg tcggtaggga agttcgtttg 79980
   agatttaaag gattgtttat taggaggata gtttaggttt tttttattga gaaagatttt 80040
   tttggatagt tgggttgagg atttttaggt tttttgggtg gagtagaatt agttattatt 80100
   gaagttatat aggtttgtaa gtataagtgg gggattgttt aggtattttt gttttatagg 80160
   ggttaggtat taaggttagg gtggttagag gatatgacgt agtttagtag gggtttcgta 80220
   gaattattag ttaagaagat atatgtagaa gtacgtaagt gtttcgagga ggtaaatttt 80280
   ttgttggatt gaatatgggg agttgagttt ttaattattg agtcgggttg ttcggttcgt 80340
   tttttttttt ttgaatagaa ggtttttaga tagagtagtt aggtaatagt atggttaagt 80400
   aggggtttgt attttagttt tgttttgtag tttgattagg ttgtcgtttt tttaggagat 80460
   acgggattgg ttagggtttt tttaggttgt tataggaata aggtagggtt gtgatttttt 80520
   ttatggttta ggtatttggg ggagggatta tgagttttag tttaggggat agataggaaa 80580
   cggatagttc gttttttttt taattacgga gttggttttt tttttatcgt atttttttta 80640
   gttatgtttt ttattagaag attatcgtat ttaatgggtg gttttgtagc gatatttagt 80700
   ttaatgaagt tatttggtgg gggttttggg ggatgtagtt ttttgaaata tatacgggta 80760
   gtgggtgagt cggaggttgc gtggggtttt gttttttggg gatggttagg aggtgtttta 80820
   attatttgta attcgagttt atagtgatcg ggatttgttg tttttttatt ttagtttttt 80880
   tttattagta ttcgttaagc gttagttagt aggtgtcggt acggtttaag gtaggtttgt 80940
   agttagatta gtatcggggg agggatcggg agagagattt ttagagtgga agttatagtt 81000
   tttttttttt ttaggttggg taatagatta ttatttttag ttagttttgg ggaaggggga 81060
   ggggttagtt gggtcgtagg tatagcgttg gggttttttg aggtggttgt tgttgattac 81120
   ggtttttttt ttttttgggt tgggtaatag attattattt tcggtttatt ttagggaagc 81180
   gggaggggtt agttggggta taggtatagc gtcggggttt ttgcggcggt tgttgttgat 81240
   tacgggtttg ttattttttt tttagttggg ttgttatttc ggttttgaaa gcgttttttt 81300
   tagtttttaa ttcgtgttta ggaggtagtc gatattttaa gcgttgttat agtaggaggt 81360
   tgggtttgta agtgtgattc gtttggggag gggtagaggg aaagattatt ttcgtttaaa 81420
   tagcggtttt tttttttttt tcgggggttt ttatagggtt tttagttttt ttttggttaa 81480
   gaggtaagga gaggtaggaa agttttagga atatcggtag agaagagata gggtaggttg 81540
   ttagaggaag taggttgtag ggtgtttaag gttttaggtt gggttttggg aggttagggt 81600
   atgggtaatt aggaggttta ggtagggaaa tttgtttcgt agggattttg ggagataggg 81660
   gagaggggat atgatagtcg ttttttgttg ttttttggtt gtggttttta ggggttttag 81720
   tttagattag aagtatttta tgtatcgggt ttcggtttag tttggaagta cggtttatag 81780
   atttatatga ttatttgggt agtgattttt aaaaggtttt tcgttattag ttggggagtt 81840
   ttcgagggga gggtggtttt gtagagtatt tggagatatt aatgggtcgt gaggaaagga 81900
   ggaggcgtgg atatttttac ggttttaaga gaaaattaag gtagagttgg aggttgggcg 81960
   tggcggttta tatttgtaat tttagtattt tgggaagtcg aggtgggtag attatttgag 82020
   gttaggagtt taagattagt ttggttaata tggtgaaaat cggtttttgt taaaaatata 82080
   aaaattagtt tgttatggtg atacgcgttt gtagttttag ttatttagga ggttgaggta 82140
   ggagaattgt ttgaatttag gaggtggaga ttgtagtgag ttaagatggt attattgtat 82200
   tttagtttgg gtaatagagt aaaattttat tttaagaaaa aaaaagagta gagttggagg 82260
   ttaggcggtt tttagagttt gataaggagg tcgtggggtt gggttgtttt ttcggttatt 82320
   tgaggggtag ggattggttt agtttgagtt ttagatttat aagaggttga atcggagcgg 82380
   gatttcgaga tgaggtaggg ttattttgtt tatatttgtg tttaggtttt aatagttatt 82440
   ttttttagga atttagtttg atggtttttt aggttgagag gttgttaagg ggttgtaagg 82500
   tgtcgtaacg tgtgagtcgg tgggttttga tgtgtgatag ttcggggtat tttttttatc 82560
   gttagggaat aggaatgagg agttttttat ttttagtgtt tggtaggtag gaggtcgtta 82620
   ttggattgtg tgtttaggtg ttattagatt tcgagggttt acggatacgg tatatgattt 82680
   ttaatgatta acgtttgtgt agtaggtgtt gaggtgtttt aatttcgagt aaggttgggt 82740
   ttttggaatt tttttttcgg taggaggatt tgtagttgag gaatttttaa gaaatttttt 82800
   ggtagtaaat aaagtatggg gttggacggt ggatttatga gtgtttttat atgaatgcgg 82860
   atatagatag aaaagacggt aaatatttgt gaataaaagg aatggttttt gcgtattgaa 82920
   tttttattta tcggtttttg tttggtataa agtttttagg aattttttaa tagttttagt 82980
   tagaaacgtg tttttgtttg gttatgtgga aaattttata tatattaaga tatatgatgt 83040
   ttttggtttt ttttacgaat gttttatgtt ttttgggttt atgatttgga atttgtagat 83100
   ttttgttgtt tttgttttag agttataagg attttggatg taggaaatat tttttatgtg 83160
   taagggtttt tttttttttt gttagggttt atcggttttt ttttgtttat aggtgttatt 83220
   taaatttttg tatttgtggt tttttttttt taattatttc gtgtaattcg ttaatagttt 83280
   ttttattttt agagtttggt tgttggagat atataaggta tatagaattc gtgaaatgat 83340
   attgagtatt tattaggtat ttattgtgtg tttcgtttgg taataataag taaaattata 83400
   atgttttggg tatatgagta tattttttta tgtttatttt tttagtaatt taatgagatg 83460
   gttttataaa tggggaaatt gaggataggg aggttagatt ggtttaaagt tataggttag 83520
   gaggggtagg gttaaaattt aaatttagat agtttggtat taaattttag ttttttatac 83580
   gaaaggtatt ttgtaatttt tcgagaaggt tgggattatt atttttattg tatagatgag 83640
   gaaattaaga tgttaaatgg ttagatagtt ttggagttag gaggtgggtt tagtggttta 83700
   tgggagtata tggaatggag tttatgggag gttcgaggtt gggatttcgt attttatttt 83760
   ggtttaaggg atgaggtgat tgggagtgtt agagatattt tgggattggt tatttaggtg 83820
   ttttgtattt agttttagcg ttgagtgggc gagggtggat aatttgttat ttagtagggt 83880
   tgttgttcgt atttcggtgg ttttttaaag tttggttaag taaagtaaga ttgttttcgg 83940
   attagagtga tttttaagtt atataagtat ttagttatgt ttcggaggat gtaggattgg 84000
   gaaggttttt tttttatcgt atttttattt agttttttta tggggtagtt ggttttaggt 84060
   atagattaga cgtttttttt tttgtttgtt gtcggtatgt ttttggtttt attgttgttt 84120
   tagggagggg tttaagagtt tttcggtttc gttatttatt atatttcgtt tagagggtcg 84180
   ggggtttagt attaacgtta agtatttagt agtaaaatta cgggttggaa agagttatag 84240
   tgttgttttg atttggtttt attttatatc gggtgtcgtt tttattagtt tttttttgtt 84300
   tttggtttta ttttttaggt attataagtt ttgtaggttt aaagttaaat ttattagttt 84360
   ttgatttttt ttagttggga ttagggtgag gttttcgttt aggtgtaaaa gttaaggtag 84420
   tgttaaaaaa tttaggaatt aaataatatt ttgaagtaat attttaaata aattaaattt 84480
   tacgttgaat aaattttgat agggttgtgt acgtgtaggg ttgggtttgt tttaatttaa 84540
   aattttaata tttgtttatt atagagatta ttatgtatta tttcgatatt gaaaaatatt 84600
   gtattaaata ttatttgtgt tgatttttga gttttttggt gtttttttat attttgtttt 84660
   tgataagtgt ttgatatatt ttgttttagt cggttttgtt ttttaggtgt ttttttgggg 84720
   ttgttttgtt tacgtttttc gtagttatta ggagttattg agttattgta tttgttttat 84780
   tttgtttttt atgttttcgt atcgtttatt tcgttatgtt tttttcgtgt gttttggtcg 84840
   tacgttagtt ttagatagga gttcggtggg ttgggagttt atattaatgt tgtttttttt 84900
   cggtttcggt gtcgggtata gggtagtatt ggagattttt tttttttttt tttttttttt 84960
   ttttttgaga tatagtttta ttttgttatt taggttggag tgtagtggcg taattttggt 85020
   ttattgtaat ttttattttt taggtttaag ttttgttttt tggaattaag tagatttttt 85080
   tgttttagtt tatcgagtag ttgggattat aggtatacgt tattatattt agttaatttt 85140
   tatattttta gtagagacgg ggttttgtta tgttggttag gttggtttcg aatttttgat 85200
   tttaggtgat ttgtttgttt taatttttta aagtgttggg attataggtg tgaggtatta 85260
   cgtttggtcg agattttttg aataaataaa tattatcggt atggagattc gtttagggaa 85320
   tttgtttaga ttttgttatt gtatttttgt ttgttgcgtt tagaggtaat ttagggtcgt 85380
   tttagtcgtt tttatttggt tattttttgg tttttattta ggtcggtttg ttttattttg 85440
   tgggttaaga aaacgattta gtgggtggtg attagtattt tttttttaat ttgaaaagaa 85500
   tatatttatt agcggtggta gtaatttaat gttttttagt tgatgaaggt aatatgtttg 85560
   tttataatgg aatggtttat gttatggtgg aatattattt agggacgtat tggtatttgt 85620
   tataatgtgg atggattttg gaaatatgat attaagtgga gaagttagat ataaaggtta 85680
   aacgtgtaat tttttgtata agaaatgttt agaatcggtt aggcgtagta gtttacgttt 85740
   gtaattttag aattttggga ggttaaggta ggaggattat ttgagtttag gagtttaaga 85800
   ttagtttggg taatatggtg aaattttatt tttataaaaa atataaaaat tagtcgggta 85860
   tggtggtata ggtttgtagt tttagttatt cgggaggtta tggtgggagg atggtttgag 85920
   tttgggaggt ggaggttgta gtgagtagag attgagttat tgtattttag tttgggtgat 85980
   agagtgagat tttattttaa aaaaaaaaaa aaaaaaaaaa agaatttata tagataggaa 86040
   ggaaatatgg aggaggaggg taggattgat agttaaaggt tacggggttt ttttttaaga 86100
   aaaaaatttt ttaaattttt taaattttta attttttaaa attggttgtg gtgacgatgg 86160
   ttgtattatt ttttgaatag attgaaaatt atggaattat gtattttaaa tgggtggatt 86220
   gggttacgcg gtgatttatg tttgtaattt tagtattttg ggaggttgag gcgggtggat 86280
   tatttgaggt taggagttcg agattagttt taatatggag aaatttcgtt tttattaaaa 86340
   atataaaatt agtcgggcgt agtggtatat gtttgtaatt ttagttattc gggaggttga 86400
   ggtaggagaa ttggttgaat ttaggaggcg gaggttgtag ttagtcgaga tggcgttatt 86460
   gtattttagt ttaggcgata agagtgaaat ttcgttttaa aaaaataaat aaataaataa 86520
   attggtggat tgtatagtag tttatttgga ttttaataaa gttgtttaaa aaaaaatgaa 86580
   aaaaagtttt tttttttttt aaaataaata aataaaaaga aaaaaagaga gaggggaaaa 86640
   aaaaaaaaaa aaagaaaata ttgagtgtag tatacgtatg gtaaaggtaa gaattttatt 86700
   acggaaattt ggttttatta atatatttta tttttatttt tattttatat ataggtgtgt 86760
   tttggttcgt ggtgttaatc gtatgttttt ttggaggttg tagtttagat tggttttgaa 86820
   agttgttttt tgggtaattt taaatttttt ataatttttg aagtttttgg tttgttggga 86880
   ttatttagtt ttttagattt agtttcgtta ttatttattt aagattgtag ttttggggag 86940
   gggggttttt ttttaagggt gtaggggttt tgtggtttta ttttttttta gaatatatat 87000
   tattttgtat tgtatatatg tgttttaata tggagtattt taggacgttg gttatagtta 87060
   gttatgtttg tatatcgatt gatcgatcgt gttttgttga ttaattatgt taattatcgc 87120
   gttgtatatt gtttttatta agcgagttaa agtaattttt tcggaataag ttgacggatt 87180
   ttaaataagt taaaagatat ttttttagga aaaaaatagt aatgaatttt aagaagaaag 87240
   aaaaattttt aaatttatag atttatggaa aaagtttaag tttttaaagt gttagatttt 87300
   tttaaaagta ttaggtttag atggttttac gagtgaattt ttgaaaagtt ttaatagaga 87360
   gattattatg ttgttgttta aaatgtttta gaaggtagag ggtaaaagaa atgtatttaa 87420
   attgtttttg ttaagttagt ataatgtaga tattaaaatt gataaaaata gtatatatat 87480
   atatatatat atatatatat atatacgttt tattcgtttt agattaagtt cgttgttgtg 87540
   tatttaagta atattattta tttttatttc gttgttgaaa ttaaaatata ttttaatgga 87600
   tttatgattt taatatgtaa aaatgaaatt atagaagtat tagaagaaaa cgtgggtgaa 87660
   attttttttt ataattttag aacgtaggag atttttaaat aggatgtgaa ttttaaaagt 87720
   taaaagggaa agtagagatt tgataatatt aggattaaaa tgttttgtgt agaagaatat 87780
   tttagaaata aaattaaaag taggtagttg gggataagat attcgtattt gttgtgataa 87840
   agatgtataa ataatttttt ttttttttta gataggattt tattttgtta tttaggttgg 87900
   agtgtagtgg tatgattata gtttatagta gttttaaatt tttagattta aatgattttt 87960
   ttatattagt ttttcgagta gtcgggatta taggcgtatt attttattta gttaattttt 88020
   gtagagatgc ggttttgtta tgttgtttag gttggtttcg aatttttaag tttaagtaat 88080
   tcgtttattt tagtttttta aagtgttggg attataggtg ttagttatta cgtttggttt 88140
   ataaagaatt ttttttaaaa aattttaata agaaaaggat atatttttta aatgaaaaga 88200
   gaatataggg ttttggttta taatatacga agagataatt tttttcgttt gatcgagatg 88260
   tataaaataa aatattaatt ggagagtatt tggtttttag ttaggggtaa ggggtatagg 88320
   atttttttgt tggattatta ttgtataggg tttagtagat agttgagtag gtcgacgagg 88380
   ggaggggttt taaaaaattt tagtaattat tttagaggtt aatttaggaa taaggattaa 88440
   tttttttttt tttttttttt ttttgatagg gagtttcgtt ttgttattta ggttggagtg 88500
   taatggtacg attttggttt attgtaattt ttgtttgttg ggtttaagta agttttttgt 88560
   tttagttttt tgagtaattg ggattatagg tataagttat tatgtttagt taatttttgt 88620
   atttttagta gagatggggt tttattatgt tggttaggtt ggttttgaat ttttgatttt 88680
   aagtgattag ttcgttttgg ttttttaaag tgttgggatt ataggtgtga gttattgtgt 88740
   ttagtagatt aaaatttaaa tgtatatatt tttgatatag aaatttatta agagtttatt 88800
   ttttagttat gtgtgtatat gtataagtgc gagaatattt gttgtagttt tttaattata 88860
   gtgggaagtt ggataagttt ggtagggtta tgggatagtt gatgatataa ttattaaaag 88920
   gaacgtgtta ttttgggagg tcgaggtggg cggattttaa ggttaggagt ttaagagtag 88980
   tttggttaat atggtgaaat ttcgttttta ttaaaaatat aaaaattagt taggtgtggt 89040
   ggtaagtatt tgtgatttta gttatttggg aggttgaggt aggagaatcg tttgaaagcg 89100
   gaaggcgaaa ggtggaggtt atagtgagtt atagtgagtt gagatcgtat tattgtattt 89160
   tagtttgggt tacgttttgt taaaaaaaaa aaaaaagaaa aagaaaaagg aacgtgttat 89220
   atttataagt ggtaatagtg ttgttaaatg gaaaaaataa aagttggtag tagaagtttg 89280
   attttttttg tgtttttgaa aagtttatat ttatatattg atttttttta tataagtttt 89340
   gaatgatttt gtaggaataa aatttaggag aatgttatat ttagttgtta atatggttat 89400
   tttttgggag agagttgggg aatgggagag aaaaattttt ttttaaattt tatgtgattt 89460
   ttatttttta tttaagaatg attgggtatc gttttttttt ttttttttta gatgggagtt 89520
   ttgttttgtt gtttaggttg gattgtattg gtataatttt agtttattgt aattttcgtt 89580
   ttttgggttt aagtgatttt tttgttttag ttttttaagt agttgggatt ataggtgttc 89640
   gttattacgt ttcgtcgtgt attgtttttt aggatgaaga aagggaaagg ttatagggag 89700
   aatgggagtt agaatataga gtagggtttt taaatattag tttgcgggtg agagagatac 89760
   ggggcgatat tatcggtttg gaatttaggg atggtatgag gtgttgttta ggggttttag 89820
   ataaggtttg tttgttgatt aagtaagggt tgtgttggtt tttgtttatg tgtttgttta 89880
   atacgaattt tttaggcgtt ttattttgtt ttacgaatta ttattgttta ttttaattac 89940
   gttgtatagg aagaattgtg tttattttta taatgagaaa acggattaga gaggaaagtt 90000
   attaggtagg gtaggggcgg tagtttaggt agtgtgaggg taaagtcggt gtttttttgt 90060
   tattttttta tatacgagtg ggtaaggtat tataggagtt tatattttaa tttttgcggg 90120
   ttattttgga aggcggtaga tggaattagt gatttttagg gttattttta gttttaggtt 90180
   ttagataggg gtcgtggagt ggaatcgagt ttgatttagg tgtgttgatg gtagttatgt 90240
   aattgtggat aaattatttt tttttttatt tgtattggat gagatcggga atttttattt 90300
   tgggtttttt aagagttttg gggttttata gagtatattt agtagtagta gtgggatggg 90360
   gggatagggt taggggagtt ttatgttttt tttaatttta attagagtat ttttatttta 90420
   attttttttt gggtttagtg tttagagaga acgggaagga agtttagttt tggttatggg 90480
   ttattttagt ttgttagtta tagtttatat tttaggagtt ttttttggat tgaaggagat 90540
   agagttattt ataatattta tagaaaattt cggagaggaa gtggagttta attgaggaag 90600
   cgtttttttg tagtttttag aaatgaggtt ataggtgaga tagtgtgtta gggtggatta 90660
   tatggtattt ttttaggttg tttgttggaa tttagggttt ggttaggggt tgtttatatt 90720
   ttgggtttcg aggcggggag tttgagtgcg tttttttagg gttgaatgtg ttttttgttt 90780
   atatcgtata gagtatggtt gtttggtcgc ggcgcgtttt ttcggatagg ttcgttattg 90840
   tgtagatttt taatttcggt agttgtttcg ggtttgggag gcggcgggta tagatatagt 90900
   tttttttttt tttggtagtt gttttgattt tttgggataa gtgttgagag gcggttttat 90960
   ttagtgttat gtgtttgggg gtcggggttt tattttgggg ttaggttggg tggttagggt 91020
   tatagagttg tagggggcgg agtagagaaa tcgtttttag cgtttgtttt ttggttttat 91080
   tttttttcgt agttgcgttt tatttaaggt attttatagt tgttcggtat tcgttgtatt 91140
   ttcggattag tttttttttt tttttattat tgtacggtgt ttggtaggtc ggcgagggga 91200
   ggggtttttt ttttgggttt tggagttagg ttttgttttt tggttttatt tcgttttatt 91260
   acgaaggaga tatatcgggt ttggttaatt ttttcgtatt gatttttgtt tttttagtat 91320
   atgcgggatt ttttatttta gatttaggtt tgagaggttt tagggatagt tcggagaagg 91380
   agggtatttg tttttagaag gggaaagaga aagagattta attttaaagt aaaaggggat 91440
   ttgggtgttt tttgtaagta gtttaggaaa atgggaggag gtgggagtag cgggattttt 91500
   cgagtaaggt atttcggtag gaacgttagt gttttttgga tatttttaga agtagaagta 91560
   tatagtttta tagaaaaatt ttgtaggttt aggattcgag gtatgatacg atttaaattt 91620
   attgttttat tagatattag tgagtttttc gacgtttatt aaggtttttt gttttatatt 91680
   tggtattgga cgaatttgta tattttgttt tagggagttt atagtttagg gataggttta 91740
   gagtttatgt ggggtgtttt gtaaatacgg ggttagaagt tgggttgttt atggggtttt 91800
   ttttttttaa attatagtgg ttattttttt tttaatttta tagtttttga tttttattag 91860
   gaatttttgg ggtttagtag gtgttttttt tttgtaggtt gatgttcgtt ttttttattt 91920
   tttatttagg tttagttttg attttatttt ttttaaagtt atttttttat tgttttgtgt 91980
   tgggtggttt tagggagttt tgtgttttga ttaattttat atttaacgtt gtattggtag 92040
   ttttgttgtt tgtgttgaga tttttttttg ttttgttttt ggaatatacg gttcggtgcg 92100
   gattaagtat gcgaagagtt acgtttatta ttattagttc gggggggttt gattttttgt 92160
   ttttttaacg ttttatttat atgataaaga tttttagatt ttttgggtta taggaaaatt 92220
   attgagatat gttgttatgt ttatttcgtt ttaattttat atttatgttt ataagtggtt 92280
   ttagaattat tgtggatttt ttttttggtt tttggggtgg gtggatatat gagagtttag 92340
   tttttagtta tttttgtaga tgtttttttg atagattaag aaattgaggt tgggaggggt 92400
   ggaatgatag tttagaatta gttgatttat ggggtagagt tgggatatgt ttagaattga 92460
   gttgggtatt gggtttggtt ttttttttgt ttttagtgag tattttaaga aggtagagaa 92520
   ttagatttat gaacgagcgt tgagtattcg ttttataagg tagttttgga gaaatttggt 92580
   tggtgtggtt gaggagtcgg aggggggtaa ttttggtggg gggggttttg agttagtagt 92640
   tagtttttgt aggtttagta ttgggtgttg tagtttagtt ttattttatt ttatttagag 92700
   gtatttatgt tggggggtag gaaaagagta gggatttagg agaggaggta aggtcgtttt 92760
   tatttagagt ttgagaggat tagggatgat tttaaaggat gagcgttttt ttgtgggagg 92820
   atagtaagga gggtgttaag tttaggggta gggttagagt ttcggaaatc gagaatttat 92880
   tttattagta taaagtttag attttattag gttttggggc ggaggagtag gtagggatgt 92940
   ttttgttttt tgtagcgttt tttttttttt tttttttttt taattattta ggtgtttata 93000
   tttttttttg aaatgttgat tttttttaga atttttatat tgaagttaat atttatttat 93060
   tgagtttttt agtataattt ttagaggaga gattaggtgt tttgggggta aagttgatta 93120
   ggaaagtttt tgtttggttt ttagggacga tttatggggg ttattatatt tgtttataag 93180
   aattataaaa aagttatttt aatttattat tagtggttta tttttttagt ataattttat 93240
   ggtatattta ttatgtttaa gttatttatt tggtatttta tgggattttt tttttttttt 93300
   tttttttttt tgagataggg ttttattttg ttttttaggt tggagtgtag tggtataatt 93360
   ttagtttatc gtagttttaa ttttttatgt ttaggtggtt tttttatttt aattttttta 93420
   gtagttggga ttataagcgt atgttattat gtttggttaa ttttttgtat tttttgtaaa 93480
   gatagggttt ttttatgttg tttaggttag ttttaaattt ttgggtttaa gtaatttgtt 93540
   tgtttcggtt tttaaagtgt tggatttata ggtatgagtt attgtatttg gttttttatg 93600
   ggatttttta taaaaattaa gataggttgg gtacggtggt atgattttag tattttggga 93660
   ggttagggaa gttgaggtgg gtgggttacg aggttaggag ttcgagatta gtttggttta 93720
   tatggtgaaa tttcgttttt attaaaaata taaaagttag ttaggcgtgg tagtttacgt 93780
   ttatagtttt agttattcgg gaggttgagg tagaagaatc gtttgaattt gggaggtaga 93840
   ggttgtagta agttgagatt atgttattgt attttagttt gggtgataga gagagatttt 93900
   attttaaaaa ataaaaaata aaaaaaaaat ataaaaaaaa ataaagataa tagttttatt 93960
   tttagaattt agtgggggta tgaaaggaat tttgaagatt atatagtaaa attttaagta 94020
   tggatatcgt tggtttatag gtgaatttta ggtttttttt tatttttgta ttttttgtat 94080
   tttaaagaaa gggtattcga ttatttttta tgatttgaaa aattattttt atatttaaaa 94140
   aaaaaagttt agtagaggaa agaaagttta tatataaatt atttatattt taagttaaat 94200
   acgaggtatt tggattaggt tttgagggat gtataggagt ttattaaggt agtgatgtaa 94260
   agagaagtag aggttagggt ttaggaggtt agggtaaagg ggttgtgagg aaaggtgtga 94320
   gagattaagg gaagattaaa gtaggagaag ttgggaagga gagaggtgtg agttgagtag 94380
   gagagtgaat taatcgtgga ggggtttatg gtgagtgagt agagaaggtt tagtacggga 94440
   agagatttta gtagtgttgt gttttgggga agttagtttg ggagtaattt agttggggtt 94500
   ttaggagatt ttgttttgta tttatagttt tggggtggag ataaattata gtatagatgt 94560
   tttttcgtag gtagaagtta aggggttggg cgtttgttta gagggtacgg aatttttttt 94620
   atgatttttt tttttatggt gttagttgga tttttggtgt tcgggggata gaggggtcgt 94680
   agagattggt tagagttgtg tatgtcgggg tttagggaat ttttagttta gttttatatg 94740
   gaggggtaga aggggatggg tttgaaggaa agatgggtag gaagttgagt tggagagaat 94800
   tggattcgtg tttagaacgg tcgtgatata gagagggagg acggagaagt aagtaggtgg 94860
   tgtttcggag gaagaattag tggggtttag gtttgggttt ttcgagaagt tatttttatc 94920
   ggattttaag agtatttttt tattgggaag gttttgtttt cgtttgtttt tgtatttttt 94980
   ttatttggat ttgtttaggt ttttaggatt gttagtttag tttacggatt tttttacgtg 95040
   agttggattt ttatttgtat aggtttttgt tcgtgggtgg tcgggtttgg gatgttgatg 95100
   gttttttttt atgtttcggt tttaacgtgt atattttatt ttatggagaa agttatcgtt 95160
   tttttagagc gttaggtttt tggtagtttt gaacgttgtt gtttttacgt ggtttgtttt 95220
   tttttttttt ttttggtttg atgaaattgt atttttattg ttggggaagt tttttggata 95280
   ttttttgtgt tttagttatt ttgtttacgg ttcggtgagt tttagaaagt agagttcgta 95340
   ggttgtgttt ttgggtttgg ttttcggtat agtttttggt ataatttagg ggttaaattg 95400
   tgtttgtttt tggaggagaa tgtttttgta tatttttatg gatatttttt tatttgtttt 95460
   ttagagttag gattacgttt ttgtgtattt agtattagtt attatcgaga tttagatggt 95520
   tcggtgataa tcgttttttt agttatattt tgtttgattt agttgagtgt gaatatatta 95580
   atttaggatg ggggtgtatt cggtacgtta aataaaggtt ttgtattaat agaaggtagg 95640
   ggaggaaata gttttttgtt attttgtaaa ttgtttatgt ttttggtttg atattatgtt 95700
   attagtaatt attgttattg agtttgggtg gttggggtag ggtggttttt tagagttaag 95760
   tttagagaga tgggagaggg aatacgggat tcgtatattg agggtcggaa aggatttttt 95820
   aaggtttatt agtttttggt tatttagtgt gtttcgatgt tttttttgta ggaaatgtag 95880
   agttttaggt tttagattcg ttaagataaa tgtgtatttt atatgtttat gaaagtatga 95940
   ggagtattgg ttttttttta aatttatatt ttttttaggt tttatatttt tttagttttt 96000
   aagtttattg attattggag aatttttatt taaaaagaaa aattaaaaag gggttgggtg 96060
   gtttatgttt ataattttaa tattttggga ggtcgaggta ggaggtttat ttgaatttag 96120
   gagtttgaga ttagtttggg taatttagtg agaatttgtt tttataaaaa aaatttaaaa 96180
   attagttagg tatggtggcg cgtaatttgt ggttttaggt atttgggagg ttgaggttgg 96240
   ggaattattt gagtttaggg ggtagaggtt gtagtaagta gtgattatat tattgtacgt 96300
   tagtttggag gatagagtaa gattttgttt taaaaaagtt aaagaaaagt taaagaagat 96360
   ggaaagaggg atttagagag atgtggaagt aatttaaggg tttatcgatg gaggaatagt 96420
   tatataatat aagtgtatcg tataatagaa tattatttag tttttgaaag gaaggaaatt 96480
   ttgttttatg ttatagtacg acggatgaat ttttaggacg ttatgttgag tgaaatgagt 96540
   tagatataaa atgggtagtt attgtatttt tttatatata taaggttttt agagtggtta 96600
   aataaattta taataaagat agaaaataga atggtggttg ttagtagttg ggggaggggt 96660
   atggggagtt tggtttaacg gggacggagt ttagtttggg aggatgaaaa gggtatcgga 96720
   gatggatagc ggtggtagtt gcgtaatatc gtgaatgtat tgaatgtcgt tgaaacgtat 96780
   attgtaaagt ggttaaaatt gtatattgta tgttgtgtat tttattacgg ttagaaataa 96840
   gaagtagtag ttaaattaaa aatttagatt gtgttgtttg tttaatttaa agaaggttta 96900
   ttgagtatta ttgagttttg attttgtgtt tagaattaga tttggttttt gaggggtttt 96960
   taatttagtt ggtattttat tatagtttta tggagttacg atttttgttg gggttagggg 97020
   gtacgttttt gagaagggat ttaattatgt tttatagttt ggtattttta aataggtttt 97080
   gtgttttata ggattggtat taattttttg tttgtaagtt ttatgttagt acggttgtgg 97140
   gtttgagttt ttttaggacg ttgttggagt tttatggttt tttttttttc gttagaaggg 97200
   tatttttaaa taaaatcgtg tgtagttatt atatagtttt ttggtttagg tatttttttt 97260
   tagtgttttg agaattttgt tttgtaggtt aaggtgtttt ttatgttttt ttttttttgt 97320
   tataattatt ttttttgggg gaggaggatt agattatggg ttttaatgta agttttagga 97380
   ggtatacgtt tttttttttt tttttaagtt ttcggttttt gttgggtata tttaggtagt 97440
   ttgtaatttt ttaagtagga gttattagaa tgtgatagtt tttttagggg ttttaatttt 97500
   attttgtttt atatttttgt atgagtttgt tttggttttt gcgtggtttt ttatttttta 97560
   agttttttag gttttttagg gatatatagt agatagaggt tttagtaagg tttacggtga 97620
   tttataaagg aggttttttg aaatttagaa aattttttta aatttttatt taagtttcgt 97680
   ttggggtatt ttggtatgtt tttattagat agagagagtt attttttttg agtttgtaga 97740
   tttttatttt tatgtatttc ggtttttttg ggtttttagg gtagtatttt ttattttata 97800
   tttagttttt ttttttaaga ttttgtggta aagttagtaa ttaggttttt tattattata 97860
   gtatttaaga tttagcgttt tgcgatttat ttttttggtt tcggggtgta gttttagtta 97920
   tagggggttg gggtttgggt tttagtttgt tttgtagttg agatataggg gtaggttttg 97980
   aattttttaa ggtttgggtt gggttggggg cggtgggcgt tttagtaggt ttgggtattt 98040
   ggatttttat ttttttgggg gttgcgtcgg tggtaggggt agtgggtatt tcggggattt 98100
   tggaggtagg gggttttatt tttcggtggg ttgatttttg gggtttgacg atggtgattt 98160
   tcgtttttcg aggggtcggt tttggcgttt tgtggtttaa tatttcggtt tttttgagtt 98220
   cgaatcggga cgggtcgatg gtttcggcgt tttttatttg tttggacgag atgttaatgg 98280
   atagtttagt gcgtcgggat atcggttcgg tcgttttggg gttcgagagt tttatttttc 98340
   gtagggacgt tttgggggag cgttggttta gggagtttat atggcgggtc gagggttttg 98400
   agttttttac gtttaggttt tggaattttt gggtggagtt ggttatagtg gttcggagta 98460
   ggggagtttg gatttttcgg ggtggggtgg agttgggtgt ttcgattttt tcgattttaa 98520
   aagatttttt taaggttgaa aaataaagag aggggatgta attggtgaat ggatggttag 98580
   ggaatatttc ggtatgtttt ttgttaagga gtagtagagg aaggtaggcg atgcggcggt 98640
   gagggtcggg ggtttttttt cgttttttat gttttagtaa gcggaaggga tagttttatt 98700
   ttaggagaat tattatgagt tttggggtag agatgtaggt tggagtggga agatgttgtt 98760
   cgtcgtgttt tgttatggtt acggcgtaaa ttatatagtt ttttttttta gttacgttta 98820
   tataggaacg ttttagtttt agagaagatt tttatttttt tttttttttt tttgagatgg 98880
   agttttgttt tgttgttttg gttggagtgt agcggtattt ttcgggttta agcgattttt 98940
   ttattttagt ttttttagta gttgggatta taggtacggg ttattatgtt tggttaattt 99000
   ttgtattttt agtagagatg gggttttatt atgttggtta gtttggtttc ggatttttga 99060
   ttttaagcga tttattcgtt tcggtttttt aaagtgttgg gattataggc gtgcgttatc 99120
   gtgttcggtt ttgatttttt ttttgtcgcg gttgttttta ttttttttta tttttagtta 99180
   aggtatatat tgggatatta ttagtaaata tatttttatt attagttata gcgggggata 99240
   tgtttttaat aataagtgat cgtaattgta attaattaga atcgtagtag gaataaaatt 99300
   ttattaatga tattaagggc ggatattgga gtttttagtg tcggcgaggc gtcgggttta 99360
   tcgagtttat tatatttgta gtcgtttaat tttgggatgt aaagaatatt agtattttta 99420
   gtatagatgg ggaaagtggg gtacggggta gatatagagt tagattgtat attaggttga 99480
   tattagagtt taaggtttta attatttggt tgttcggttt ttttagttta gtttatagga 99540
   attaattttt ttagttatta gttttttttg tattttttta tttattatta taagttcgtg 99600
   tagtgatgtt aattattagt tttcgttttg tagttatttt gtttttttat tagggtttgt 99660
   tttttcggag gtagtgggga tggtatttgg gaagttgtta tttttatagt ttttataatc 99720
   gtattttaaa tgagagttag tcgtttttgg ttattagggt tagagtaggg agggatttat 99780
   atggtaggta tatttattag tttttttttt aggttagatt tatagcgtat ttttgtggag 99840
   atttatagtg tttttttagt cgttttttgt tttattaggt tatagagttc gggttttatg 99900
   ggattttatt attgtttttt ttgtttatag gaaaagggat gatagttggt ttttagtttt 99960
   taataatagc gttttaggat taattttagt gttttttttt tattcgtttt ttttttttta 100020
   aatgtattta ggtattgggg aggggaagag gaagtttaat tttttaattt tggtcgtttt 100080
   agttgttagt tttttgggta aggtattttt gtgggcgttt tttttaagtt ataggtattt 100140
   tttttttttg gtggatatag acgaaatttt gggttattaa gtttaatttt tattagaagt 100200
   tgggtaagga gtcgtgtgga ttttagagaa tttttagttg ttataggttt tgttttagtt 100260
   tcgttattgt tttagaggat atgggttcgg cgtggttaga gttgtttatt ttttaagaga 100320
   gtagaaattt ggatttttat atgatatttt ttgattttta aatgttggta taaagtaaaa 100380
   taagtttttt ttaaaatatt gagtagtttg gttgatgtgg tgaaatttta tttttattaa 100440
   aaataaaaaa ttagttgggt atggtgatat tcgtttatag ttttagttat ttaggaggtt 100500
   gaggtaggag aatcgtttga attcgggagg tagaggttgt agagttgaga tcgtgttatt 100560
   gtatttagtt tgattgatag agcgagattt tgtttttata aattaaaatt aaaaataaaa 100620
   taaaatatcg tagggttaaa taaaattaaa tttataggtt gtattcgttt atgggtttag 100680
   ttatgatttt tagttgatta cgtggtcgta gggtagtagg atattgagtt ttagagttag 100740
   gggtgtgtta ggtgtgggtt ttcgatatgg tgtttttttg tttttttgta ttttgtatgt 100800
   aaattgttgg ttgtgttttg attttgtaga agttttaggg ttttaaggaa aggttgaatg 100860
   tttattttta ttagttagaa agtttagggt ttaatgttta gaattgaatg taatgagatg 100920
   ggtttattaa gtttattata tttatattcg tttaaatttg ggttgtaaag aatattatta 100980
   tcgttttcgt atagatgggg aaagtgaagt acggggtaga tatagagtta gattgtatat 101040
   cgggttgatg ttagaaagtg tagagtttaa tgtttgggtt ttcgtgcggt ttttagggtt 101100
   tttcgcggtt ttttgatttg ttttttgttt tttgagtatt gtttaaattg tttttatatg 101160
   gtgtgttttt gttttatggg tgttgtagag taggggtcgg tttttaggtg tttttagtat 101220
   tttttcgtgc ggttaagttg gagaatagaa attagatagg agaaggtttt gttttaggtt 101280
   taatttaaga aaataaatga ttttttttgg gagattcggt gaattttttt tttgggagtt 101340
   tttttggagt tatttttatt atggttaatt tatgatcgaa gatattgtat taaagtagag 101400
   gttgttttgg tgtgagttta cgattgagtt tacggtgcgg atgtaatgat ttattttagt 101460
   taggtttacg tgtataagcg gattatttta gaatcggagt tggttttttt tttagggtat 101520
   tttttttttt tttagttgtt tgcgtggttg gcgttttatt attttggtta tttgcgtaaa 101580
   atgtatatgt gtgtcggttt ttaattattt tttttttttt tttaatgttt ggtgtttttt 101640
   tatggatggg tttttttttt tttattagtt ggtttttgtg tttattgttt ggttttatga 101700
   tttttttggg tgtttcggtt tagtattatt tttatgttta ttagttttta gtaagttttg 101760
   agatagtagg ggttgtttta ttcgtagggg ttattttatt attaggacgg cgttgtttaa 101820
   aggcgagttt gttttagata atagttagga gattttatag tttttagaat tgtaagagaa 101880
   tataattgtg tgttagaaga atagtgagaa taggaggcgt tttatttcgg aggtgtttat 101940
   tttttttagg agatgtttta ttttgggggt gtttattttt tttaaggaaa ttatgtttcg 102000
   gttggaatgt taagaaggtg atatttaaat gaagaggtgt ttattttggt ttacgttatt 102060
   tttttaaacg agtatgttat cgtttttttt tttttttggg aatttttttt aaagttaagt 102120
   ttattttagt ttttttttcg gggagttggt tttttatata gtttatatta tttttttttt 102180
   atgttatttt ggttacgtta tgattgttga gattggttac gaggggtgtg tttcggtttt 102240
   tcgggaatag agttgaatgt tttttatttt atgatattat gtttttatta ggttttaggc 102300
   gtttttagaa aatttattaa gttaggcgtg gtggtttacg tttgtaattt taatattttg 102360
   ggaagttaag gtggatagat cgtttgagtt taggagttta agataaattt gggtaatatg 102420
   gtaaagtttc gattttatta aataatataa aaattggtta ggggtggtgg tatatgtttg 102480
   taattttagt tattcgagag gttgaggtga gaggattatt tgaggttagg agattaaggt 102540
   tgtagtgagt tatgattata ttattatatt ttagtttgga tgatagagtg agattttgtt 102600
   ttaaaaaaaa aaaataatta aaatttatta aatgttaggc gtggtggttt atgtttgtaa 102660
   ttttagtatt ttgggaggtt aaggtaggtt taaggtaggc ggattatttg aggttaggag 102720
   ttcgaaatta gtttggttaa tatggtaaaa ttttgttttt attaaaaata taaaaaatta 102780
   gttaggtatg ggggtgggcg tttgtaattt tagttatttg gaaggttgag gtagaagaat 102840
   tatttaaatt cgggaggtag aggttgtagt gagtttagat cgtgttatag tattttagtt 102900
   tgggcgataa agtaagattt tattttaaaa aaaaggttgg attataggat tataggatag 102960
   gtataagttt tgagggagtt tttaaggagg agggacgtat tagattttta tttttatttt 103020
   agtttgtgaa gatttatttt cgtttgaggt tttgtagagg gttgattagt gatttttaaa 103080
   aagatatgtt taatatttaa ttttaagaat ttacgaatgt gattttattt ggaaaaaggg 103140
   tttttgtaga tgtgattagg gatttcggga tgagattatt ttggatttgg gatgggtttt 103200
   aaatttagtg ttcggtgttt ttataggaga aaggtagaag gggatttggg atataaagag 103260
   atacggtggg gagggttacg tgatgagagt agagattgta gatgttgtta gagttaagga 103320
   gcgtttgggt taacgtaagt tggaggaggt aaggaagggt ttttttttcg agttgtcgga 103380
   gggagtgtgg ttttgttaat attttgatgt taaatttgtg gtttttagaa ttatgagcga 103440
   attaattttt gttgggttat ttggttttta gtaatttgtt ttagtagttt taggatattt 103500
   ataacgagat ttttgggaag atgaaatttt tggttgtata gtggtttagg gtgaggttag 103560
   ttggtgtggt tatttatttt ttagtttagt tttaatttta ttataattat attcgataaa 103620
   tggtttattg tgggttaacg gatacgtggt tattttagag ttgttatatt aggagtttag 103680
   aggagcgaga gttgtttttt tttttttttt tggagtcgta taaaaatttt agatggttgg 103740
   agaagttatc ggaggaatta tattatttta gatttttgtt ttattgttgg gatatgtggt 103800
   aatgaaataa gattattttt gttagggatg ttttatgggt aattgttttt ttacggagga 103860
   tattgtgagt tgtatttagt cgggaaatat taggagatgt ttgttattag gttggtagaa 103920
   tagggttttt tagtattttt ttttttatta gagttttaag attgcgtttt gggttttgta 103980
   gttatatacg gtcgtttttt tttttttaat gtgtaggttt tttgggaagt tttttgttat 104040
   tatttttata gtggaatcgg tataatttgt ttaggggtag taggtttttg ttattttata 104100
   cgtttttatt ttttttgtgg agggagacgt agttaagggg tatttatatt tatttataga 104160
   agcgagagtg ttcgtgagtt tgttattttg tagattttag aatttcgtac gtgattgtat 104220
   agatttatag gtttaattgg gtagatttaa aggatttacg tagtagggag gagtatcgga 104280
   tagttttttt ttttgtttgt aaggagttaa tgtaattgaa ggatatagaa gaggttttta 104340
   gttgagtatt ataattgtgg atcgtgtata gtgttagaaa gatgcggagt taggttatgt 104400
   tgggaggatt tttatgttgt tattggaagg taaagtagtg ttattttttt atttatagag 104460
   taggaggttt ttatttttgt tagttatttt ttttatttgt gaatttaagt tttttaaagt 104520
   tagattttaa tttcgtaagt ttttaaaagt ttttttgggt ttttttttta atttttatcg 104580
   attttaattt gttttaaaag tttgaagatc gtttatgtgt tttggtttat ttttgggaat 104640
   ttattttaag gaaataatta gaagtataga aagatttacg tacgaggata tttgtattag 104700
   tattaaattg ataaaatttg gaaatagttc gggttttcgt agatttttta aatttatatg 104760
   ttaaaggttt gtattattat taaaattata tttttgaaga tgagaaatgt cgagaatatt 104820
   aagtaaaaat agtaggatat aagatgtata taaagatttt aattttattt tatatatata 104880
   tgtgtagttt gtacgtatat atatatttat attcgggtag aaaataacga tttttggatg 104940
   gaatgaatgg cggtgggtgt tgtttattat atttttttgt tttttgagat gtttatatta 105000
   tatggggatt atttagtatg tttagggaat aaaattatcg atattgtttt tttttttttt 105060
   tttttttgag atagtttcgt tttgtcgttt agattggagt gtagtggcgt aatttcggtt 105120
   tatcgtaagt tttatttttt aggtttacgt tatttttttg ttttagtttt tcgagtagtt 105180
   gggattatag gtgttcggtt attacgtttg gttaattttt ttgtattttt agtagagata 105240
   cgattttatc gtgttagtta ggatggtttt aatttgttga ttttatgatt cgtttgtttc 105300
   ggttttttaa agtgttgcga ttataggcgt gagttattgt attcggttaa tattgttttt 105360
   aaaaaatata ttttggtatt ggttgggtat ggtggtttat atttataatt ttagtatttt 105420
   gggaatttga ggttagagga tcgtttgagt taggagttcg agattagttt gggtaaatgg 105480
   taagatttta tgttggaaaa ataaaattaa aaaaaaaaaa atatatatat atatagattt 105540
   tgatgttaat aagttggagg gtgtgggtaa ttgtatgttg gtttattttt atttttattt 105600
   ttggaaaaat attttatagt ttaggatggg tatagggttt tttttatttt taagtattta 105660
   cggttttagt tgttatataa aaagaggtta tagtgggtta ttttttagat gtaaattttt 105720
   ttttttaggt ttatggcggg gtcgaaatag gagtagtttg gatttgggtt tatttagaga 105780
   ggaatgtgtc gtataaggga ttattgttaa ggtttttgtt ttgattatag gttgtttggt 105840
   aaatcgtttt tagtttttga aattagatta agtatttttt ggtgtatata ttaggtttta 105900
   gtttggggag ggaatattaa gcgagttata gagttttgtt ttagtttggg ataaatgtta 105960
   tgtaaagaaa tggtagagtt taaggttaga ataattttat gcgtagttta gttttcgtag 106020
   gagggagtaa cgtggttttt gtttagggta gggtggaatt ttaggtttag gttttaggaa 106080
   cgttagtttt ggttaaattg ggtttttttt gagtagtagt aaagtaggtt ttgtttttga 106140
   agaaatattg gttatttagg agttagggtt agggtttagg gaagtcggat agatggtgtt 106200
   aaggagtagg gaagtatttt tttgtatgtt attttgagtt tgtaggaaaa agttacggga 106260
   aaattttatt tggtagataa ttttttataa tttttaggat ttatagagtg agtttagtag 106320
   tatgtacgtt atgggtattt ttttgttttt atttttttgt gatattggat tttggtatag 106380
   gtttttgatt tgtgtagggt ggggtgggta gttaggtagg gttttggcgt tggtagagtg 106440
   gggtttagtt gtaaaggttt ttttattttt tatttattta ggatttgggg gacgaggttt 106500
   aaaggtttgg tgtgttttta aggagtttaa ggattgtttt agggttggag ataggacgta 106560
   tgggtttgta tagggatcgt gttttttatt ttagggttga gagtaggaga attaaatagg 106620
   agagtgatat aattatagtt tattttgagt tttagtgttt ttagtgttat ttgttgtagg 106680
   aggtgatggg gtaataaggt gtttttgagg ttaggcgcgg tggtttatgt ttgtaatttt 106740
   aatattttgg gaggttgagg tgggtggatt atttgaggtt aggagttcga gattagtttg 106800
   attaaaatgg agaaatttta tttttattaa aaatataaaa atagttaggc gtggtggtgt 106860
   atgtttgtaa ttttagttat tcgggaggtt gaggtagaat agtttgaatt taggaggcgg 106920
   aggttgtggt gagtcgagat tgtgttattg tattttattt tgggtaataa gagcgtaatt 106980
   ttattttaaa taaaaataaa taaataaata aaaaataaga ttttttgggt gtaggtatgg 107040
   tacgcggtag gggtgattgt tgttggtgta attattattt tttgggtgtg tgtttgtggg 107100
   ggttgtgtat gttgaaatgt gtggttattt tgttggtgat taaggttgtt ttggttttat 107160
   tgttattaat tacgtttatt gattttatat ggtttatttt cgtgttttta tttagagttt 107220
   tttttagtgt ttaagttttc ggaaggttgg aaggagtgag tagtgtagtt tttggttaag 107280
   aattgaagta taaaaatttt agttttttta tttaatttta ggtgggataa ggttgggggt 107340
   acggtttgta gtttttttat gttgttcggt atgggggttg ggtagttatt tgtggttata 107400
   atgagttggt gaggtatttt ttattggttg gattttttgt ttggtttatt tttatattta 107460
   ggtatttttt ggtgtttttt tttattgaaa tttttgtttt aggatttgtt tttggaggga 107520
   tataaattag ttttttagaa ttggtttggg ttgtttggtg gaggagggag gtggtttttg 107580
   gggtaggtgg agtaggtttc ggatagggag gaggatatag ttggggtgat ggggaggggt 107640
   gtgtttttag gaagtaaaat cgtttaaaag gaaaggttta aaaataggcg tagtgaagtt 107700
   gagcggagtt tgtaaaggga agttaggaaa tagtagtgtt agtggagggc ggtttttagg 107760
   ttttttatag agagttatat ttgtttaggg atagggataa cgtgaataga aggtaaagtt 107820
   tatattaagg cggaggaggc gttttttttt tagagagtcg tagttagata gtttaaattt 107880
   gttaaaggtt tgggagggaa gaattagggt attattttat ttttagttta attttttttt 107940
   tagttttttt tttttttgtt tttttaaggt taggtttggt tgggttatag tgttagtagt 108000
   taagattcgt ttttagtgtc ggtttagttc gtaggaggtt agtttggtgg ttaaaggtta 108060
   ttgattgttt aggtttattt gtatacgtgg tttaatgatt gaggtttggg atttggggtt 108120
   attatttata gtatttttgg ataaggagga aaagaattat ttttattaat ttttggatgg 108180
   aggtgtaggg agcgttaata gtatttttga aaattttaaa tgtattaggg gggttagtat 108240
   ttagggaaga gttataggag gttattttga tggattttga tgattatttt tgtttaagtg 108300
   ttaggaggag gttttttttt ttttgttttt ggtaattttg ggtttaagaa gttttaggta 108360
   tttcggaggt tttttttgtt tattatagtt ttatttaaga ggtttggttt attttgtaat 108420
   gatgatgtga gaatgagtag gtttgtttag tgttagagtt tgtggttgga ttttcggttg 108480
   aggttaagag aaataaataa gttttttttt tttttttgta cgttatagga agagtagttc 108540
   gttggttgta aggggatttt ttattttgtt tatggggtat agtagtttat agtatttttt 108600
   ttagaaacgg agtattggta ggtttgggtt tttgtaagat ggaatataaa ggggtgggta 108660
   gttgatttta atagttagtt tttgggattt taaatttttt tttatttttt aattttgtga 108720
   tgttaatttt tttttggata aagtttttta ataatttggt aagttagtat tagttgaatt 108780
   agttttagtt tgaattagta gtttttttta gtttagttat agttttattt ttttagaagg 108840
   gaaaagaagt tttacggatt tttttataag gaatttttag aatttgttat ggtgttcgta 108900
   ggggatattg gtattaattt tgggtttatt aaatgtagtg ggtttaatag tgttttttaa 108960
   aacgatatgt tttaagtttt aatttttgga atttgcgaat atgattatat ttggaaaacg 109020
   gatttttgta gatgtaatta aggtaagggt tttaaggtga gattattttg aatttagtgg 109080
   gttttaaatt taatggtgag tatttttata aaagatagaa aaggagaaag atatatagat 109140
   agggggagaa agttacgtgg agataggggt tagagattag agatgatggt gatgcgtttg 109200
   taggttaagg aacgttagga atttttagga gtcgtcggaa gttatgtttg taagttttta 109260
   ggttgtggta ttttgttata gtaattttag gatatagatt ttaggaatag aatatagaaa 109320
   agaaacggga ggaggtttag agtttgttgt ggttatttta gttattttat tcgatttttt 109380
   tagtgtttcg ttaattttga agtttttttt agttattgag ttatcggttt tgaaaaggag 109440
   atatgggtgt tttggagttg gtttagtttg gtaggaggta gtttcgtttt attgtttttt 109500
   tattagttat tagtttatat aggtttttta tagtgggtaa gtgcgttgtt gttttagttt 109560
   tcgtcggttt tttttttgtt cggttttatt agtcgtagag ttttttgttg ttgttttttt 109620
   tggtttaaga ggtttatata tttagttttt ttgggataaa tttagtttat atttgttgtt 109680
   ttatcgtagt tatttaaggg ttttttttat taacgtgttt tttaagggat agtaagttat 109740
   acggtcgttt tatttggggt tagaattgtg ttttattaga gtaaggtttt ggagtaatta 109800
   tatttagggt tagggagtta tttgtttaag aatagaaacg tgtggtcggg cgcggcggtt 109860
   tacgtttgta attttagtat tttgggaggt agaggcgggt agattacgag gttaggagat 109920
   cgagattatt ttggttaata tagtgaaatt tcgtttttat taaaaatata aaaaattagt 109980
   cgggcgtggt ggcgggcgtt tgtagtttta gttattcggg aggttgaggt aggagaatgg 110040
   cgtgaattcg ggaggcggag tttgtagcga gtcgagatcg cgttattgta ttttagtttg 110100
   ggcgatagag cgagatttcg ttttaaaaaa aaaaaaaaaa agcgaaatgt gtagtttttt 110160
   ttttttttat ttttgaaata tgaaggggag gtgattgtag gttttttggg aaagattttt 110220
   ttattttttt tttttttttt tttttttaag acggagtttt gttttgtcgt ttaggttgcg 110280
   gtgtagtggt gttatttttg tttattgtaa gtttcgtttt tcgggtttat gttatttttt 110340
   tgttttcgtt tttcgagtag ttgggattac gggcgtttgt ttgggatagt tacgtagtag 110400
   tttttgtttt aagttatttt tttttacgta gagtagaatt aggattatag taaaggtttt 110460
   agaggaggtt atcgtttata gggttttggg tttttttttt gaaaggtagt ttagttttta 110520
   agagggtaat agcgattgat tcgggacgaa ggtttttttg tgtttgtttt cgttatttat 110580
   cgcgtgtttt tggtttcggt agttataggg ttattttagg cggattttaa agtggcgtta 110640
   agttgttgga tttttttttt tttttattaa attagttatg ggaatgggag tatttttggt 110700
   atatttttag atgtgttgta ttggtttagc gtttttttta ttttcgggtt ttaaattagt 110760
   tgttggttgt tttattagga gtagattgag gggaaatggg cggaagtcgt aggggaagag 110820
   atgtgaggta gataaagagg aagaattttt tggttggggt ggggtcgtta gggtttgggt 110880
   gggagggtcg tttatttttc gtttttggtg gaaggtagta gaagggtggg acggatttgt 110940
   gtgggagggt ttaggtgtgg tttgtttggg ggtaggagag ggattagatg attttttagg 111000
   tttttttggg ttaaagattt tttgataaat ggttgtcgtt gaaggtagga gcgaggtttt 111060
   gggaggtgat tgatttcgaa aaattatagg ttgtagtttt gagggtttta aaggttaagt 111120
   acggagtttt ttaaaatgag gtttttttta gttttaattt atttacgttt tttaggtggt 111180
   tttagatatt tcgttagggt ttttttggta gtttttcgtt ttcgattttg gagatagaga 111240
   aacgtgatgt tggtttttgg tttgtttata gttttttcgt ttttattata gggtcgttag 111300
   ttattttttt agtttatcgc gtgttttttt ttttttttta ttttcgattt tttaataata 111360
   gtagattttt ttttaaatat ttttttgttt agatttcggg ttttacgtat gttgttttta 111420
   gaaatgtatt ttagtttttt gcgaggagtt agtaaggttg ttcggtgttt ttttttatgg 111480
   atgggaagtt ttgtcgggga ttaggattcg gatttggaga ttttgaggag cgtgttggtt 111540
   agtgtttgtt ttgaggttcg ggatagagtt gttaggtata ggtagtatcg gtgtgagggt 111600
   ttagtttttc ggataagttt ttttttgttt gtttttagat ttgttattgt atatataggc 111660
   ggtgattatt tttgatttgt ttgggtagag aagttatttt tttttttttt ttcgttttat 111720
   tgtttattag gggaaatgtt ataagtattt aaaaaatgta aaaaaaaaaa aaaaaaaaaa 111780
   aaagcgttta atattagggg ttatttggtt taggtggagt ttttattaat tacggggttt 111840
   tttgtgttgt ggttttaagg cgggtagtcg atggtttttg ttcgagttaa tatgagttgt 111900
   agtttaatta taggggacgg tgattttagg gagtagagtt taggtttcgg ttatagttgt 111960
   atttaggagt tagttgtttt ttagatttgt agagttaggg gagaggtttg gaggatcggt 112020
   attgaatgat atgatttaaa gcgcgttaag ttgggttgga ggtgacggta gagttatagt 112080
   ggttatagag tttttacgtg aatttatacg gatttgtttg gtttaggttt tgttgaattt 112140
   tagggtgagg aggatttggt ttttattttt aaagatatga agaggttgat aaagaagagt 112200
   tataggtaaa agggggtggt gggtgtgttg tgtaggaggg gtcgtaattg gacgtagaag 112260
   attttagaag gggtaattag agagaggtag tatttttaga agacgggttg tttggttgtt 112320
   ttaaatagtt tagggtgtga ttgaggggat tttcggattt agtcggggtt ttttttgttt 112380
   tgagataacg tattgggaaa ttttattaag tttatttaag aatttgaaaa gttttttgga 112440
   aatttagcgg gtagggcggg tttttgggag gatttttggt atatcgcgag agttagatat 112500
   agcgaggggg ttttatattt ttgtttagaa gttttacggt agaggggtag aagtttaggg 112560
   aggagtaggc gttgagatta gaggggttta taagagaggg gtttggggta gggggtagtt 112620
   gttgagaagt ttaggaaacg gcgtttgttt ttggggaagt gaagttttgg gaatagggtt 112680
   ggtgaagggg ggcgggaagg gggaggtggg ggtggagggt taggttagat taggtatttt 112740
   ttttagtagt agatttatga gtttttggaa tttgtttata gaagtgataa gtgttttgtt 112800
   tttagtttcg tttatatatt tatttattaa gtgtggtagg gatagtcgtt ttttattatt 112860
   tattttggga tttggattta agtggaggtt tttggagggg tagatttagg ggaaagggtt 112920
   tttttagaag tagtcgttat ttagaggatt tgagatgaga attttattac gtttttatta 112980
   gatagaggag attcgcggtg ttttcgagtt tggtttttta ttttttattt agtagcgttt 113040
   ttttcgttta ttttaagttt agaatatttt gtttttttta agattagttg tcgtagtgga 113100
   ttgaatagtg atttttaaaa gatatgttag agttttagtt ttcggatttg tgaatgtgat 113160
   aggagttgga atagggtttt tgtagatgta ataaaggatt ttaagaggag agtattttgg 113220
   atttagggtg ggttttaaat gtaatgataa gtatttttac gagatataga gatatagaga 113280
   aaaggttatg tgaagacgga gtagggagtg gcgtgacgta tttatatgtt taggagtatt 113340
   cgggagttat tagaagttgg gagagaggtt tgggtcggag tttttcgtag aggttttaaa 113400
   ggattagttt ggtcgatatt ttgattttgg atttttggtt tttagaatta tttttagttt 113460
   ttaaagttcg tggggttttg ttgtagtagt tttagtaaat tgttacgggg gttttttttt 113520
   tttgttaagt ttggtattta aagtttttta gttatttggg gttaattata ttgattttgg 113580
   gatttttagg ttattcgtgt gttttatttt tttgtttaat ttttagtgtt tgtgtggtta 113640
   tagttttttt ttcgtgatat gagttttttg ggtttttgag tgtttatttg ttttgtattg 113700
   aaggttgaag ttaaattttg gagagaattg attttttttg tgggtagggt aggtattgtg 113760
   tttttagtat agttagaggg tattggttga aatgtgtttt tgtaaagtgt tttttttatg 113820
   ttcgtatttt ttttttaagt ggggaagtta gagaggtttt ttcgattttt tttttagttt 113880
   tttggggtag gaagtttgtg gttatttgtt cgtaaggatt aggtggatag ataattagtt 113940
   ttttttaggt tatattttat taatagaggg ttttttaatt ttgttggtgg tttatgagag 114000
   aaggaaaaat tgaaggagtt gtttatagta aattttaaat ttttgtgtta gagaggttta 114060
   tggttgagta aggtattttt atttttattg ttttgagttg ttaatttagt ttagtttagt 114120
   attgtgattt ttgatttgtt tgtgggttac gggagtttgg tttgttaatt agtatttttt 114180
   agatatttat tttgtgaagt ttttagtggt tgagtgtttt ttcgtttttt tcggatagcg 114240
   tgggttatag gggatattgg gtaggaagat agttaagttt ttattgttta gaaattgttt 114300
   gggtttattg tagtaatagg gatttattta ttagtatgag gtttttttgt aggaggggtg 114360
   ggggtaggag ggagtttgtg gggtataggg ttgtaggagg agttattttt ttttttatta 114420
   aatcgtaggt tttagttaag ttatttttag tatggttgtt tgcgtgggaa gtttatagtt 114480
   gagggattgt ttgtacgtgg tatgtaggag ttataagcgt tatttatatt ttatttcggg 114540
   ttaggtagat gatttttttg gttttagttt tttgatgagg aataggaagg gttggatcga 114600
   aagatttttt ttagttcgga tatggaatga ttttacgagg gttacggtga ggtttgtttt 114660
   agtttcgtat attagttcgg gttagtagtt tttaaaggta tagtcgtagg ggttttggag 114720
   tagggataag gaggattagt ttattttatt tttttgggta gaggttaagg attttgtttt 114780
   tggtttatat tttttgtggt taaaaatgta gaagtaagta gtgttggtag aggtatttag 114840
   atttttgggt aaagtagggg ttttatttga tattttaggt ttggagtacg agaaaattag 114900
   tttaggtttt aggttttttg attgttagcg attagggaat gtgatatttt tcggtttttg 114960
   tttttttatt ttttaatttt agggggagat taggttgggt gatgtgtttt tttttttgat 115020
   agtaaatttt tatttatttt tatttttatt tttgttttaa gagggggttt tttatggtcg 115080
   ggcgtagtgg tttacgtttg taattttgga attttgggag gtcgagatgg atggattatt 115140
   tgaggatagg agtttgagat tagtttggtt aatatggtga aattttgttt ttattaaaaa 115200
   atatgtgagt tggtaatttt aagtttttag tagttttcga atggaggttt tttttttgta 115260
   ggatttttaa attttataaa tatgagtgtt tgtaatttag ttatttagga ggttgaggta 115320
   tgagaattat ttgaatttgg gaggtagagg ttgtagtgaa ttaagaacgt gttatcgtta 115380
   ttttagtttg ggatatagtg aaattttgtt aaaaaaaaaa aaaaaaaaag gtgggggttt 115440
   ttttatgttg tttaggttgg ttttaaattt ttgggtttaa ggaatttttt tgttttagtt 115500
   ttttagagtg ttgggattac gggtacgagt ttatgaattt taaaaaataa tatttattgt 115560
   tttgtttttg attgtaaaag gaatttaggt ataaagtcga aaatttgtaa aatgtataaa 115620
   aataaaggga aaaaatttat aaatttataa tttaggtaat ttttattaat attttgataa 115680
   atgttttttt attttttatt ggggagtgtg tgtgtgtgtg tgtgtgtgtg tgtgtgtgtg 115740
   tgtgtgtgtg tttaaatagg tttatatagt gatgatgttg ggtgtttttt tttatgtggc 115800
   gttatgtggt tgaggttttt atatattttg ggttcgtttt ttcgattttt ttgtagtttt 115860
   gatgttttga ttttagatat gttatttttg gttcggattg tttaggagaa ttatttttgg 115920
   attttgttaa aaaatggatt gttggttttt aatttttggt ggttttggtt taggagggtt 115980
   gggtaagttt gggtatttcg gagtgttttt ggggcgttgt aggtgtgaat ttttgttttt 116040
   ttgaagattt ataagtttta ttttttttat tttttttttt tttttcgaga attttataag 116100
   aggaaagttt ttattcggag gttgttaggg attcggagtt gttagtttat agttttattt 116160
   tgtttttatt ttttcgggag ttatttaagg ataatgttta gttttagttg ttaagtttta 116220
   gttttagggt tagttatata ttgaaggata tgtagaagtc gtcgtgtgtt gggtattgtt 116280
   gaggataaag gtttgaatga taagcgggaa taaagttata aggttttaaa atttgttcga 116340
   ggttttatag ttagggtttc ggaaatttat gaaaaaagtt agtttaatat tttgttacgg 116400
   aatataggta ggagagggag tagtttttag ttagttttta gttagttttt cgttagtttt 116460
   tttgcgaatt attttatttc gggttatagt tagggtagtc gtcgtttttt tttttgttcg 116520
   atagtttttg agggtaggag ttagtttgtt tattattgtt ttttttttat tagggtttgg 116580
   tattttgttt ggtatgtagt agaagtttaa gaagggttta ttgtgtttat tttgtaatag 116640
   ttaatgtgta tgagtgttta ttatatttga agatagtgat ttatttttgt ttttattgta 116700
   tagatgggaa attgaggtat tgagaggttg atgatttttt tatattgtcg tgtgggtaag 116760
   ggtaaggtta gatgtgagtc ggtacgttgg gttatcgcgt tttttgttta tatttcgttt 116820
   tgggtttggg aaggttattt tcggagattt ttacgatgtt gatatttcgt ttagtttttt 116880
   cgtgtatttt tttttacgtt ttttagttat ttaaagtatt agttaggtag tattgttatt 116940
   ttggttttgt aaatttagtt attgagtgga agagtagtta ggttgaagtg gggtagaggt 117000
   agttttcgtt agattttagg gtcgggattt ttagttattt ttatttttta aggattagta 117060
   gatagagatt tttatcgtgt ttagttaggg gttagggaat aggtttttat agtgggggag 117120
   tgatggatgg atgtaggtgc ggattgggag gattcgggtc ggttttttag cgttggagtt 117180
   ttagtagttt aatttatttt gttgggtttt cgtggggatt aaaagatatt ttgtatataa 117240
   agtattagtt tagggtttgg tatttaggag gtgtttaatt aaaggaaatg gttatgaaaa 117300
   ataataagga gaagtttagt tataggaggt gtttgtgggt aaggagagaa tagttggaag 117360
   ggtttggatt tgggttagag ataaagaatg taagagggag ggagggagag aaggagggag 117420
   ggagagaaag agggagggaa agaaagaggg agggagagag ggagggtagg gtaggtaata 117480
   gttggtttta gggtagttgt gtgtaggagt ttgttttatt atttaaatat taggagagat 117540
   gtaaatattt gagtgaggtt tttttagttt ggttttgttg tggttatagt tagggttgga 117600
   gaaagagttt tttttttatt agggttttcg ttttgtgttt tttattttag ttgttttaag 117660
   atttatgttt ttcgggttag gagtaggggt ttgagagtag taattaggta gggtagcggg 117720
   gttagggagg gatttcgttt ttgtttttat tttttttttt cgtttttgtt tttatttttt 117780
   tttttttgta tagtattggg tttgtaaagg tgcgaatgaa ggattttttt ttttattcga 117840
   ttttaagggg tttaggcggt taagggtttt ttttgtagtt tagaaaaaaa agtaaagtag 117900
   ttttttttga ggttttttgg tggaggttta gggtttaaag ttcgttgtta taaatttgta 117960
   gttttttttg tttttttaat agtgtttttt ttttatttgg gttattttat tattttggtt 118020
   ttttttagtt ttttaggttt tttggggagg aggatagatg tagagtttgg atttcgtgcg 118080
   tagtagggtt tttttaatgt tgcggtgtat ttcgtttggt tgcgggagtt tgttttatgt 118140
   tatgatttgt gttggtatta atagtttttt tttttatttt ttatttgagt gtcgagggta 118200
   gttgtgttag gtatagatat atagtttttt atatatatta tgagtttgta gaaatggtaa 118260
   gagaaggtcg agcgtagtgg tttatatttg taattttagt attttgggaa gttaaggtgg 118320
   gcggattatt tgaggtaagg agtttgagat tagtttggtt aataggatga aattttgttt 118380
   ttattaaaaa tataaaaaat tagttgggcg tggtggtgcg tgtttggagt tttagttatt 118440
   taggaggttg aggtaggaga atcgtttgaa tttaggaggc ggaggatgta gtgagtcgag 118500
   attacgttat tgtattgtag tttgggtaat aagagcgaaa ttttgtttta aaaaaaaaga 118560
   aagaaagaaa aagaaaaaaa gaaaaggaaa atggtaagag aagttggtta gtattatttg 118620
   tttggtaaat ttttatttcg gtttttggtt aggaagtttt tttttaggaa gttttttcgt 118680
   tttatttttt tttttgagtt tatgtttaga tttttagtgt ttagaataga gttggttttt 118740
   ggggtattta tggatggttg ttggataaat aaataagtta attgtttttt ttatttttag 118800
   cgatgttttt ggtttggtag ggtatttagt agtaatgttt ttcgagtgga ttttaaggag 118860
   tattttttat cgtgtagttt gtattaggga ttttagtttt ttattttaaa ttaaaggaaa 118920
   aaatttaatt ttagtaaata tatagagtat tttatttttt gatttttaat ggggggtaag 118980
   aggtagagga tagaggagaa gattattttt ttaggtttgg tttggagtta aaaagatttg 119040
   tatgttttta ttgtagataa tatcggtttt tgggggatat agggtagggg tttggtttgt 119100
   ttttcgtagg gaaattgttt atttacgtgg aaagcggggt taagggtttt tgtttggaat 119160
   tagttttttg tatatggggt ggggggtggg gatagaagga gatttgtttt ggattgtatg 119220
   gaaatttatt agattgttgg ttgttgattt ggtaggagta gagttatttt tattttttag 119280
   ggttttgttt gggggagggg agaggttagg tggttttgtt taggttatat tttttatggt 119340
   taggaatgtt ttttttttat tagaaagaaa gatgagatag gtttattttg aattttttgg 119400
   atataggaag gggtttattt tgtggtttag gtggtttttt tttttatatt taaggggttt 119460
   tataaggagg tgttaggatt tttaggtttt gttttaggga attgttgttt gtaaaggtta 119520
   taggtagaga tagttaggaa tgttttttat gttattggtt atgggttgga ggttgcgaaa 119580
   ttaggtatag aattagaaag atttgttata ggttgtaaag ggttttagag agttttagtt 119640
   ttttagaggg gtagtattgt agaaggggtg agaaggaaaa attagttttt aggaattgtt 119700
   tatttagagt ttatataata tttaggtggt acgagataac ggtttatttt ttattttatt 119760
   ttttcgttgt tttttaagat ttgagttttt gggggtgttt ttagtagata gggggatgtg 119820
   gggtaattgt ttttttttaa ttgtatattt ggaggtaatt tatatttaga tgattttttc 119880
   gaggttgtga ttagagtttg gggtagggga ggggaagcgg gtggatggga aggcgaggga 119940
   gattttttta ggaaagtcgt tttaggtgtt tgtaaggttt tatcgtttat atttaaggta 120000
   taggtttttg gttttgagga tataggaaat tcgatgttat gtggggagga gggtattgta 120060
   atgtatttaa ggttaggttt tttgattttt tggatattgg ataaatgatg gtttttttag 120120
   ttggtttaga gatgttagga gtgatagaat gttaaaggtt gtagggggat atttagaaat 120180
   tttcgagaaa gaagatggat aaggaaaaat aaaaaaatac gagaagttta gtagagtttg 120240
   agtttcgtat tttcggtttt ttttatggtt acgggtaatt ttttggagat ttagttacgg 120300
   gagtagtgga ggtaggtatg ggtggggggg ggggcgggtt ttattgttta tggtttcggt 120360
   tgatatcgag gttatttttt tgttttttta agtttttggg tattggtagg atttttaaaa 120420
   acgataataa atttgaagtt tttagaattg tttgttggtt tagtttaaac ggagtttcgg 120480
   ggttttgtag attaaattaa atagtttgag tttagaaatg ttttaggcgt ttgggggtgt 120540
   ttaggggtta attggtgggt ttgtttggtt ggagagatga taaatttttt tatttttggg 120600
   attgtgtttt agagagtttc ggattttttg gttttataag taatttagag tttttaaatt 120660
   tgggaaaaat taagagggtt ttggaaagaa aggtagtgtt tgattatttt tttttagtgg 120720
   ttttgttaag ggagagtata ggaatttggt ttttggttgg gtttttggga tgaattaaaa 120780
   taaaaagaaa aagtcggggg agggggtggc ggaatcgttc ggggttttag tttaggttat 120840
   atattaagga gattttagag gagtaggtta gttttatttt cggtacgatg atggacgggt 120900
   atttggttgg ttggtaaagg gaagagatgg taatagtgag gttttgaatt tagtggttaa 120960
   gatttatttt tgagtgtgat tttttttgat gtttgggtat gtaagaaatg ggaggtggag 121020
   aaaggaggag gagattttag ttttgttttt tcggtattag gttattaggg aatagtattt 121080
   tttttttaac gggtaggtag aggttatagg ttgttaggtt gagaagtttt tggtttttat 121140
   agataaatgt atagtttgta tatttgtatt attttgggtg atgtttatta cgtacggtta 121200
   ttatgtacgt tttttattat taagatagtc gacgagatta ggtggttatt gtgtatcggg 121260
   tatcgtggtg attgtttgat attgattttt gggggtttag gttttcggtg ggtttgtttt 121320
   tttaggtatt tagttttttt tttatttttt gatatttcgg gtttagtgta ggggtatggg 121380
   ggtgtttagt ggatgggttt acgggttggg taagtagtac gtagttttgg tttattagtt 121440
   tttggtttta ttattagata agatgagatt gttttagttg ttagtttagt tttgtattat 121500
   atttgattgg gtttttgtag aggtttgttg gatgagaatg tttagggtag taggtattat 121560
   tttttgggat gtatattttg ttataatcgt tttttaggga aaggttagtt tgtagagtcg 121620
   aacgtaggtt gttagcgttt tcgttgtttt tatggttcgg ttatttagta agtgtttatt 121680
   ttattgttag cgtttggttt tgtagatggg tatatggttt atttatttgt gatattttag 121740
   ggtgggggtg ggggtagatg gtggtaggga agattaggat aattttttag taagaggaat 121800
   ttgtagttaa tatgacgtta gtttttagaa tgttagttag gtaggaaaag gggggtttta 121860
   tggttgtatt gggagtttga gtatttgtcg ggtttagagg gatttttagg cgggcgttat 121920
   tggtattagt gtttgtcgtg ttttttataa ttagtggtga ttatgttgtt cggttttatt 121980
   tatatagtgg tattatatag gtttttgttt gagatttttt atttttagat ttgtatattt 122040
   aagtatttaa atttatttgt tttttgtaaa tgtttttttt tttcgtttag atgttatatt 122100
   agtcgcgata tttattatat tcgtaggagg taggattcgt gtttagcgtt atgggttgta 122160
   ttttagagtt ttttagtaat ttttaaagag agttgttttt aattttattt tgtaggtagg 122220
   gaaataggcg aagaaattga agaaatcgaa gtcgtttagt tttttaatgg tagagtgagg 122280
   gcggtttggg tatttttata ttgttatatt ttttattttt ttaaagtatt gtttttttgg 122340
   atgtaaattt tgtttattag ttttatttaa aatttttttg gatagttata gtttatagaa 122400
   agtattttta gtttgtagtt atttagttat tgtaggggtg ttttggcgtt tatttttatt 122460
   tgtttttttt cggatattta gtttagagaa tttatagttt cgttgtaagg ttgattatag 122520
   tgtatttgtt gtttgggtgt tgtgttcgtt tttttttaaa tgaattaatt cgtagtcggg 122580
   atagtgggtt tgtttggttt tgggttttga atttaaagag tattaagtta gtgttgttag 122640
   tggttgtggg attcgtgtaa gaggtttgtt tttatttttt atggtttttg ttttttatta 122700
   taaaagttta aattgtagag aggtagtatt tataggatgt tttgagtttt ggttgaaagg 122760
   tagggtttat tacgtaagag gtttattggt attaattagg gataggtttg tatttattat 122820
   taagtaacgg ggtttttttt tttagagagg aaagaggtat ttgaattttg ttagcgcgtt 122880
   agtttttacg tttcggaggt gttgggattt ttagataaga aggtatattt atatggtttt 122940
   ttttatttag aagttcgttt tatagtttgt tttgttttta gaatatagat ttgggggttt 123000
   ttttcgggcg atagtggggg ttcgggagaa gtttagatgt tatatagttt tgggatttga 123060
   gtacgtgggt tttgggttag attgttttaa taaatattaa ggatttttgg attttttttc 123120
   ggtgtgtaaa tttggttata gtaaatatcg ttgagtaaag taggttaata gttggtattt 123180
   ggaaaggttt ttgttatata aagattcgag ttgtaatcgt gtttgcgata atagatttgg 123240
   tttgaattta cgaaagtata tgtcgttcgt tagtttcgtg tttttattaa ttagaaggga 123300
   gaaagaggaa agggttattt tttttgttat tttattacgg gtagtggcgg gtgttgtagt 123360
   cgtttggagg ttcgtttgta tagtggaaaa gtagaaaatg agattttaaa aattttatta 123420
   gttaaatata aaatattttt tttgttgtta cgtggcggtt tttatagagt tttaaaattt 123480
   tgaagttcgg agatttattt tgaaggggga gtaaaggcgg gatgcgggaa gtttgagttt 123540
   atggaagata ttgatatttt ttttattttt gggaattttg agtttgaagg attcggtttg 123600
   aatagcggaa ggattgaatt tttgtgtaat ttatcggtga tggaatataa agttatggtg 123660
   ttttcggttt ttaagacggg agggatgggg aaggttttgg gtgaagggga taggcgggga 123720
   aggaaaaggt gaagagaaag gtgtttttta gggagggggt agatgtgttt aggatatggg 123780
   atattggtag aattttaatt tcgagtatat atataggttt acggacggtt tttcgtttat 123840
   ttgtttttta cgggtgtgtt gtgtcggttt gggtatttgt tttttttttt atgttaggtt 123900
   atttgtcgtt tattttgtat attgttatta gttttaagtt ttttagaata ttttgattag 123960
   ggttttgtgg tttttgttat aggattttag gtttagacgt ttgttcgatt ggaaaattgt 124020
   ttttagagat gtcgtggttt agagttcgtt ttgttttggg gtggtttttg gagttttcgt 124080
   tttttagtaa tttatatggt gataggtatt tatattcgtt ggttttagga attttggggt 124140
   tattatttta gcgttgtaag gagttggtag tttttttttt attttttcgg tttattttgt 124200
   aggaattaag atattaggtg agtcgtttcg ggaaagagat ttgtgttttt acggcgttgt 124260
   ttttgcgttt atttttagtt attttagatg tttttcgtgg attagttgta ttttcggcgg 124320
   gtatgtttgg tttttcgtag tttggtttat atgattttta taggtaggtt tttttttatg 124380
   ttttagtttt ttttttagtt agtttatttt agtttttttt attgttgttg gttgttgttg 124440
   gttagaaggt tttttttttt tttgtaattt aatttttatt tttttttaag gttcgttttt 124500
   tttatattga tttttttttt gttagttagt ggtgattttt ttttattttg attcggaagt 124560
   tgtttgttta tattattttt ggttgggttg aattatattt ttttttttat tttttttaat 124620
   tatttttttt ttacgtttgt ttgtttattt aaggttgttt aatttttata tatatatttt 124680
   tatatttttt aattatattt taggtttttt gggtattagg gagggttatt tttttatatt 124740
   ttaggttagt ttttatgtat atgcgtattt atgtatatat atattttttt acgtttatcg 124800
   attcgggtta tttgatggtt ttaagattag aatttttagt aaaattattt aggatatata 124860
   agggggagat tgataaatat agggaattag tagattgagg ttttattttt cgagattgag 124920
   aaattttttt ttagagttaa aggattgtta attgttgtat taggatggtt ttaatgagtt 124980
   tattgttttg gattaaatta tagggttata aaattggttt gggagatagt cgtagaagta 125040
   gttttagatg gaggtatatg ggggtaaggg tggagttata tttagagatt taaggaaggg 125100
   gtattaataa tttttatagt gtaaatggtg atagttgtat aacgaatata attttaaatt 125160
   ttggtttttt ttttcgttat agggttttcg ttgagatacg agagtttttt tttagaaatt 125220
   ttattggggg ttcgatattt tttgttagga attgagagta gagatttcga tttcgtaggt 125280
   aggaggagag ttttgtataa tatagggttt ttttttataa gggtttattg gaaagtggtt 125340
   agatcggtta agtcgtgtta tttggatatt tgggataagg aaggtttttg gaggagcggt 125400
   cgtagggagg agttgtaaag aggtaggggt agggaggtta tatttattcg gggattgtag 125460
   gaggtttaac ggaatatagt ggatttaggg agattttagt atgagtatgt gtttaaagat 125520
   gtttagatga ggaggtgttg tgggggaggg gggcgggaat agtaagtttt tttagttttg 125580
   tagatttaga tttttttttt ttttgggaag tagggatgta aagggataga gtttgaaaaa 125640
   gggtttttat tattaagttg ataaagtaat tacgatgttt ttagtttata ttttgtatag 125700
   cgttagtggt tattggatta taattttttt tcgttttttc gtcgttttat atttttttta 125760
   ggtttagttt tgaagaatag ttattggtat tttgtttggc gtttagttaa agttttggtt 125820
   ttttaggagg tttcgttttt tttatttaga tagggttgat tttaggtggg tcgtgtgggt 125880
   agttgggttt aggatattta gatgttagtt ttggttttgg ttatggagcg gggaggattt 125940
   taggttggtt attttttagt ttggtttgat atttggatag tttgtttatt ttaatatagt 126000
   tttttttttt ttcggtttat tagagggttt tgtttaagtt agtttaggag ttttttcgag 126060
   gggcgggggt tttaggtttc ggtagaggta ggttcgtttt ttagaagttt tgttttagag 126120
   cgttggagat tattgtgtga gttttttttt cgtttgttgt atttattttc gcggacgtag 126180
   gacgtagagt tttagtatcg gtagttaaga ggacgtagga aagcgtacgt atttataggt 126240
   ttagagacgc ggacgttaga tttaggtttg gatagagtag tttcgtaagt tttaatatat 126300
   tttgtaaatt tcgaagatag gagagagtta aaatattttt tttgttcgta cgtaggtttt 126360
   ttttgttttt tacggttcga ttttgggagg cggatgagga tatttttagg tttggatggg 126420
   ggttatggga tattttattt tagattttgt tacgatttgg gttgtgttcg ttcggtgttt 126480
   tcggtttacg ggttgtataa tttggcggtt tcgaaattgg cgtgggggag gggagggttg 126540
   tttattcgag taggacgcgg ttgtttattt agtcggaggt gaggaacgat ttttttattt 126600
   cgttgtcggg tttaagcggg gttcgagagt cgtcggggag agttaaaggg aggggatcga 126660
   tggatttttt agagtgaaat tgtgcgtttt ggagagtttc gaggtagttt cgcgagtttt 126720
   cgaggaggtc gttgtcgttt ggtaaatata aataataata aaaggaagga aatttaggcg 126780
   gtagtgcgta gaacgagtat agggttttgt gagggcgcgt agggtttacg cgggtgtttt 126840
   taatagggtt ttttacgtag gcggcgcggg tacggggtcg ggggcgcgcg gcgtttttag 126900
   cgggagggcg tttggattag gggtttcgtt tttttttagc gtattttcgt ttttttttag 126960
   cgtattttcg tttcgcgtta ggtttattcg tagggttttt tttagtacgt tcgcggtcgt 127020
   agtagttagt ttagtattta ttttcgaagt tcgaaatgat tttatttagt tgcgcgttga 127080
   tcgcggggtt cgatatgatg gttggtgggt agcgggtcgc gcggagggta gcggcgagga 127140
   agcgttttcg gcggggttcg ggttttgcgc gttggttggg gcgtcgcgtt cgcgtttttg 127200
   tagtgtagag ttagtcgtcg gaggagtttt taggtttttt ggtttagttg aatgaatggg 127260
   ggaggaaggc gggcgtcggt tttttttcgc gcgttgcgtt ttcgtttcgt tttcgttttt 127320
   cgggcggatt aggttttcgt attcgtatcg gtttttttgt ttcgtattgg ttgtttcgtt 127380
   cgtttgttaa ggttaggttt ggggggttgg ggttcgagtg gtcgttgggg gatagttttg 127440
   ggtatacgat cggagcgttt tttttttttt cgtttggttc gcgtttaggg gacggcgaga 127500
   gacgcggttt tagttttttt tttcgggcgt ttcgttgcgg ttagatgtgg gcggatgtgg 127560
   aggtcgggtt atggcgagga gggcgggcgt ttagagggga agagattttt tttttttttc 127620
   gggcgtaggg tttttttgta ggaggttgta tttgggtagt taaataaagt tttttgttat 127680
   cgtcgtcgcg cgttttgcgt ttgtaagggt taaacggtag cgtacgcggt tggtaggtag 127740
   gatttcggtt tgtgggttta agagtcgagg ggcgtagggc gaatatgcga gttttatggt 127800
   attagcgaga ggttattaat ttttatttat cgagggtatt tttgtagtta atatttttat 127860
   tacgattatc gtttcggggg ttatcgttgt atttttttgg gtgagttttg gagaaggtat 127920
   tgttcgggag aaggggtata gggcggggat ggaatacggt tgttagagaa gttagggacg 127980
   ggcgacgttt tttttttttt ataataaggg ttttttgaga gtaggaagta gattttgtta 128040
   ggagtagacg ttttcgaagg aaggtgtgaa ggaaggagtt aggtagtgag tgagtagaaa 128100
   gggagttgtt ttaagggttt tttttttttt tttgggtttt aatttgaggt ttagtttttt 128160
   ttttaggtta gcggttttta tttttaaatt atattattta taaggtaggt agggtcgagg 128220
   ggttggggta gatttgggga tttgggttag atatagaatt tgagaagtaa taggttttta 128280
   tttttaagaa gggtttttta ggtttgttat ttataagaga gagaggcgtt ttttttattc 128340
   gttttggaaa gttagaaatt tatttttgta tgtttttttt gttatttcgt tttttagatt 128400
   ttcggttttt gtttagagtt aggggttatg tgagaattta tagtatggaa taatattttt 128460
   attttcgaag tagtaggtcg taacgttata attgtatttc ggttttttga atgtgtggtc 128520
   ggggagcgtt tgatagttta ttttgggttt gaggtcgttc gttgtttatt tgtttttttt 128580
   tatttttttt gaaatatttt aaatgtgtag gagtgttgag attttttagt tattttggag 128640
   aggcgggggg tagggggttt tttgcggaaa atgttttagg tttgggtggt tttttaagta 128700
   gatgaaatta agtttttttt ttttaagaaa agtaggtatt ttttttttta tgttttggat 128760
   attttgaatt atttttaatt aagtgagttt gtggatatgg gaattgtaaa attatagata 128820
   ttttagatgc gtgtatattt tcgtcggttt gggagttcgg gttttagaaa tgtagttatt 128880
   gttgttatta atagtatttc gtatttttta cgttaggtta gggtatgttt tgtttgaatt 128940
   ttagtatttg ggtttatcgg atagtttagt ttttattggg ttatttgttt acgtcgtatt 129000
   tggggatttg ggggtttagt atttaagaaa tttagtttaa taatatagtt tttttgggaa 129060
   gatgtcgggt tcggtataat agttatttta agttatagta ttattttcga gtgttttttt 129120
   aaaaagtatt tggtgagtgt ggatatattt atgtaattgt ttttttttag ggttggtatg 129180
   gagttttatt agattagtat agggttagtt aattgtatat tttttatttt tacgtatagt 129240
   gtagtaaata tatttagttt tttggggagc gtcgttaggg tgtacgtttt attaaaatgg 129300
   gaatttaagg gataatggta ggagagtagt tagaaagtgc gtatgttttc gtgattgtgt 129360
   ttagagttta tcggttttag ttttagttat tgttttttta atgtaattat ttagagttat 129420
   atgattaggt ttagaaattt ttaggtaagg aggtttttcg agttttttta ggaaatttaa 129480
   attagggttt ggttatattt ttagttagga agtttatttt cgtatttaat taattgtttt 129540
   aattgtatat tatttatttt tttttttttt ttgtttttag aggagatggg aaatagttgg 129600
   gtattatttt ttaagttata attatttttt ttatatttaa ggataattta gttgtttttt 129660
   ggtttttttt tttttagttt aaattatttt tttttagttt tttaattata tttcgagttg 129720
   ttttttgatt atagttaagg gttgggttta gtttggggtt ttaggatttt aatgaggttt 129780
   tattgttata ggatagtgag gaggtttttt attttttttt tttagggttt tagggtttgg 129840
   atatatagtt ttagaagggg tattgggtag agaagatgta cgtcggtagt agagaggaaa 129900
   ttgagttgaa atttggtaag aataaaagaa ttataaaggt ttgtgtattt agtaaggtgt 129960
   atttttttac ggttataaaa gaaggtaaga tcgattgtag gtttggttaa agggatcgtt 130020
   tgcgttttta gtgtataacg agatttatag tgggtcggtg atttttttta aaataaagat 130080
   atgtttattt ggaattttaa atgtgatttg attttgttta agggtttttg tagaaagaat 130140
   ttaggtaagg attttaagat aaggtgggtt ttaaatttaa tgttgtgttt ttttaagaga 130200
   tagaaaagaa agtatagaga tatagaaaaa ggttttgtga agatagaggt agagatcgga 130260
   gtgatgtagt tataggttaa ggaatatttg gggttattag aagttggaag aggtaaagaa 130320
   aggttttttt ttggagtttt tagaggaagt tggttttgtt ggtattttga ttttagattt 130380
   ttggttttta gaattgtgag aggatatatt ttttttgtta taagttattt agtttcgata 130440
   gttgttatag tagttaggag aaattcgtgt tttattttta tttgtattat gttttgaggt 130500
   taaagtttta tatagtaaag atgtttaagt aattataaat tttgtgggta gtttattcgt 130560
   ttagtagatt attgttttaa gggaaggggg atggggatag ggaagagagt tataggaaat 130620
   ttttgtacgt ttttttggaa atgggaagga gtaggtttgg agagaaattt tataggttag 130680
   gtttcgggga aatgaagttt ataaagcgtg ggaacggagt ggggaggaaa tttacgagga 130740
   agtagcgttt ggcgtatata tttatattta tgattttttt ttaggtatag tttttatgga 130800
   gaggtttagt tggtagggaa gtaaaagagg ttcggagagc gaaggaaggt gaggggtttt 130860
   tttttagtaa tattgaggtt ttagacggga tattttatta ggtggtaggt tgtgttttta 130920
   tagatagaga ttttttaagg gtagggttta gatggtttta tttttgcgtg gtttgatata 130980
   aagtaggttt tttagagttg tcgagttttg ggttcgttag tggtagagtt ttttatagtt 131040
   ttttaagttt ttatagtttt ttattttagg aaacggtgtt attatttatt tagttgttta 131100
   agttaagttt tcgggtatta ttgttgattt tttttttttt tttattttta gtatttaaat 131160
   tatggtgcgt ttttattttt agtttttttg cgaattagtt tgaatttttt ttttttagta 131220
   gtgtgttagt taacggttaa taatcggtta tttggggtta gtaaaagtaa taatatttag 131280
   tttttcgatt tgtagcgttt gtttattttt gtggtgttaa tatttttgtt ttggttaatt 131340
   tttttttttt tttttttttt ttgatagggt tttattttgt tattcggttt ggagtgtagt 131400
   ggggtaattt tggtttattg taaattttat tttttaggtt taagtgattt ttttgtttta 131460
   gttttttaag tagttcggat tataggtatt cgttattatt tttggttaat ttttgtattt 131520
   ttattagaga tgaggattta ttttgttggt taggttggtt ttgaatttag atgatttgtt 131580
   tgttttggtt ttttaaagtg ttgggattat aggtatgagt tatcgtgttt agtcggttaa 131640
   ttttaagtta tttgtatgat attattggag gtgagattga gaagagatgt ttagttatta 131700
   cgaatatagt gtttttatta tataaatata attttaagaa tataatcgta ataggataga 131760
   taatagttaa atgttgtaaa aaatttaaga ggtgatgaaa gttgtgtatt tattattttt 131820
   tgttttaaat ataattattt ataagtaatt ttttaatgtt ttgtttaatt ttttttttat 131880
   aatttgtaat ttaatttata ttgtaaaaat ttattatttt tttaatagag taatgtattt 131940
   ttattgtgat aaaatatatg taatataaaa tttattattt ttggttagtt attatggttt 132000
   acgtttgtaa ttttagtatt ttggaaggtt aaggtgggtg gattatttga ggttaggagt 132060
   ttaagattag tttggttaat atggtgaaat tttgttttta ttaaaaatat aaaaattagt 132120
   cgggcgtggt ggtaaacgtt tataatttta gttttttggg aggttgaggt acgagaatcg 132180
   tttgaatttg ggaggcggag gttatagtga gttaagatta cgttagtgta ttttagtttg 132240
   ggcgatagag cgagatttcg ttttaaaaaa aaaaaaaata aaaataaaaa agataaattt 132300
   attattttaa ttaagttttt atgttagtga tattaagtat atttatattg tatatttatt 132360
   aatattattt attttcgaaa ttttttttaa ttttttaaat tgaaattttg tttttattaa 132420
   ataataattt tttatttttt ttttttttag ttgttggtaa tcgttatttt aattttttta 132480
   tgaatttgat tttatataag tgaaattata taatatttgt ttttgtgaga taggtttacg 132540
   tagtatgagg tttttaaggt ttattattta aggtttatat gtagtgatag aatttcgttt 132600
   tttttgaagg ttgaatgata ttttattgtg tgtatatata ttttgtatat ttattttttt 132660
   tatatttgag taattgagag taaagttgtt ataaatatgg gtgtgtaaat atttgtttaa 132720
   gtttttgttt ttattatttt attattatta ttttatttat tattagtatt attattattt 132780
   tagatagttt tattttgtcg ttcgggttgt agtgtagtgg cgtgatttcg gtttattgta 132840
   attttcgttt tttagatcgg agtaattttt ttgttttagt ttttcgagta gttgggatta 132900
   taggtattcg ttattatgtt cggttaattt ttgttttttt agtagagatg gggttttatc 132960
   gtgttggtta gggtagtttc gaatttttgg ttttaagtga tttatttttt ttagtttttt 133020
   aaagtgttgg gattatagac gtgagttatt gcgtttagtt attattttag ttttaaatat 133080
   tatagtaatt tttttattgt tttatgagat tttttgagat agtggtttta gggattttat 133140
   atatatttgt tcgattttgt gtaatgataa agcgtagttt tttttgtaga atggtagagt 133200
   cgggagattt ttgatggggt atgagtttaa aggggtagag gggacgttta gaatatatat 133260
   ggatttttta tgtgcgtttt gttagttatt aagtagattt gttttagttt cgatatgttt 133320
   aggattatag atagtgtatg gtttttattt gggttaagtt ttcggaattt tttttttttt 133380
   tatattattt gtattgggag tagttattag gttgtggatt attaagtttt ttttaggtta 133440
   tttgagtttg tattttttta tttagtttga ttgagtcggt gtgtagttat tatattttat 133500
   tatatttttt gtttggtcgt tttaatgtaa ttataaggtt ttatttaaag aagtatatga 133560
   ttaatttgtt ttttagattt tttagtgatt tttttagttt gttttgtgta gaatatttat 133620
   agtaaaattt gattatagcg gtttgagttt ttatttttaa ggtaggggat tttttaaata 133680
   aagtattagt gttttaaatt tatgattttt taaggaggtt tttggttttt agtatagtaa 133740
   atgttttgtt ttatggtttt tcggtttttt atttttattt tattcgagtt tttcgtttcg 133800
   gtatttggga ttgaattagt ttggagtttt ttagttttgt tgtttgttgg ttttgatgat 133860
   attattaggg attgtagtat tgtttggatt ttttgttgtt atattttatt gtaataatat 133920
   ttaatttgat ggaatttggg gagtaagtgg taggggtagg gatggtggtg gttaggggtt 133980
   gtatatatat aggatagaag gattgaagga ttgggtggaa agtatgattt ttttaagatg 134040
   attttttttt agtagaattt tggttgtgtt ttttgaatag tatttgttta tttgtatatt 134100
   tggtgagttt tgtgattagt aaatttagat gtttgggagg ttattagcgg ttttgttgag 134160
   gtataattta gatatgatag tttatgtttt ttttttgatt tttttttttt tatatagttt 134220
   tatgattaga gcgttttttt tatttttgtt tttattttag tttcgtaaga tatagtcgtg 134280
   aaatggttaa agtttttaat tttttagtat tcgtttgaga ttggagtata aaaatgagat 134340
   ggggatatag tattttatgg atattagtga tggaaaaaaa ttttttgatt ttttcgggag 134400
   ttttgtaaag ttatattagg ggataagaag gagtggttag tttggtatcg atttagtttt 134460
   aaagtagtta gtgattaaag tatatagttg gtattttttt atttttaggg tattaggagg 134520
   tttggttaga atttatgtta gttaggagaa ataggttgtt tttggggttt ttggtgtttt 134580
   ggttttttta ttgttttttt gaagaaggta ttttgttttg tggggtttga ggttggaggt 134640
   aatttgttat cgatgtagtg tagggatgag ggttgagtta ttagtttgtt ttagttttac 134700
   gttagtagga ataataggga tttaagtaga gaagaaagta tttattttta aagttattat 134760
   agtgattgag gcgacgtggt ttttagggag tttttttttt tgttggtttt ttgtggatcg 134820
   cggttttatg ttttaataat gtttttgttt atttcgtaag gtttagtagg tggaaggaaa 134880
   gagggtggag gttgggagtg gcggtttggt aattcgggaa gggtagggag taggaatatg 134940
   tttttttttt tttgtattcg tttagaaggt tttatagggt ttgttttgtt atttataatt 135000
   ttagggagat ttttttttaa tgtttgtttt ataaatgttt atttatttta tatttgaata 135060
   tttttagtta tagggagttt agtatttatt tatgttattt attttatttt tggttgttta 135120
   aaaaagagga aagagagaga aagaaaagat aggaaagata gagaaagggg gaaagagaga 135180
   gagaggaaag aaagaaagag gaaagaataa gataggaagg aaagaaaaag gaagggaacg 135240
   gaagggaagg aagagaggga agaaagagag aaagagagag aaagaggtat aaataaggta 135300
   ggttttatgg agtagagatt taataaggga gtaaaatata tttgtagtta ggttgtagat 135360
   ggatttttat aggagcgtat atttatttta tttttttcgg aatagaaaag tggaaagaag 135420
   atgtagcggt gtgggtgggt ggcggttttt tttgggggtt tggaatgtgg ggtattagtt 135480
   tttttggttt tgagaaggag attaagatta ttaatattta tagtggagat cgaattatat 135540
   aagggttggg gatatcgttt tttttgtatt tttaataaag attcgatgtt ttaattatgg 135600
   gaatttgtgg aagaagaaat aaaattggtt ttggggtttt ttagagaatt ggttttttat 135660
   ttagtggtgg ttgtgaagaa atttttggag ttagttttag cggtttgggt ttgtatagtt 135720
   gagattgttt ttggaagttg agtggaagtg gggagttttt aggtatagtt gggttttgtt 135780
   ttttatttta gttagtagtt ttaggatttt tgggtgtttt agagtttttt ttagagggta 135840
   aatttagagt atgagggagc ggggagatgt ttaatattgg gggatttttg ttcgtttttg 135900
   tttttagttt cgttgatttg aatagggagg ttatgggtgt ttaggggtag aattgtgggt 135960
   tggtaagttt ttagttttgg tttttttttg tttttttttt tttattgtta ttgattattt 136020
   ttggttatat gttgatattt ttataggatg atggagaaga gagaagagga attttttcgt 136080
   tttgtttagt atattttaaa gaggttttag tgtaaaggta tcgacgttta tagttaaatg 136140
   tgtgtggggg ttttttagga gtgggacgtt tttatttcgt tttagattgt tatgaatcga 136200
   tagaaggttt tatgttatta aatatatttt attaataaat tattttaata tttagtaaaa 136260
   aaataaataa aaatgtatgt atttcgtgtt ttgtagtgtt tttatgtcgt attttttttt 136320
   gtatttgagt ttcggttcgc gtttttggtt tgtcgttttg ggaatagaac gttttgcgtg 136380
   ggaggtagat aggaagcgcg gggtgcgttg gttgggtttt tttagataat ttggtattgt 136440
   tttttttaga taatgaaatt ttttttattt tgagtgtttt tggaagttag tcggtagtta 136500
   gattaattta aatgaattat ttgttttttt tgtgaagtga ttttagggat ttaatgggtt 136560
   tgttattaat atagtttttt tcgtattttg tttttttttt ttattttttt ttttttgtat 136620
   gagttattgg ttacgatgat tcggtttagt taagggattt ggttagtttt ggttaggtta 136680
   tgtgagagat agtataaagg attaaggttg tcggaattag gttagaggtt gagggttata 136740
   cggatttttt ggagtgtttg taattgggga tttttaaggt tgttttttgg ttttggggat 136800
   ttgtttattt ggggatagtt ttaagggaga tttttaaaag tagggagtta aaagggtgaa 136860
   ttaatataga aatattgagg gttagggaga atttgaagat gggttcggtt gggtatagtg 136920
   gtttatgttt gtaattttag tattttggga ggttaaggtg ggcggattat ttgaggttag 136980
   gaattcgaga ttagtttggt taatatggtg aaatattatt tttattaaaa atataaaaat 137040
   tagtcgggtg tggtggtagg tgtttgtaat tttagttatt taggaggttg aggtaggagg 137100
   attgtttgaa cgtaggaggt agaggtggta gtgagttaag attatattat tgtattttag 137160
   tttgggtaat agagtgagat tttattttaa aaaaaaaaaa aaaaaaaaaa aaaagatagg 137220
   tttaaggttt ggagagttag aagttatttt tttatttttg ggtggtttat tttgagattg 137280
   tattacgtta gagaaagttg cgggagtgtt tgtttataat ttataagtaa tattgttttt 137340
   tttttttatt agtttttggt tttagtaggt ttttgattcg tgtttttttt agggttttgg 137400
   gtttttttaa gttggatttt cggttgttgg tgggatgatt atgtgatttg ttattttttt 137460
   gtttatcgtt ttgggtagtt tttgttattt ggtgtatata gagtggagtt tagtaaggtc 137520
   ggggtcgtcg attttttgtt agttatattt gttttttttt acgttatttt ttttcgtttt 137580
   ggttatgttt aggggtttat atttttttat aagttaggtg ttagattttt gtgtttgttt 137640
   tattttgttt tttttgtttg gtgggttttt tatatttttt ttggtttgat ttaggtttga 137700
   tttgttttta ggttttagtt tagaggttgt tttttttagg aagttttttt gggatcgtta 137760
   gatgaaggga tttttttttg gatttttttt ttatttttat agttttagga atagttttta 137820
   atttaggttt tattgagtta gtgtggtatt taggagagtt ataagcgtgt gacgaacgtg 137880
   ttgaagtttt tttaaaagtt gaaggagttt tgtgaatata tagtagtatt agtttagatt 137940
   gcgaaggtat aggggagtta gtttttttta gtttcgtttg tttttggttt tttttttgag 138000
   gaggtgggag ttagttagtt gggattagga ggtgggtttt atggagtaga gatttaataa 138060
   gggaataaaa taaattagag aatttttttg ttgtaaggaa tttagataag gagtatttag 138120
   ttagaggagg aaggaagaga tcggtagtag attttttttg ttttagtagt tttgtaagtt 138180
   agatgggtta attttttagt atagttgttt tttagtatag agaaattttt tatttttagt 138240
   aatagggtgg gaatattttt agatagggtt acggttattt tggttatagg gattacggag 138300
   tttgagtttt tgtggtttgt tttatttatg tggttataga ttttgagtat atatttggag 138360
   ttatagagtt taaagggtag ggtagggtag ggaatttgtt tgattggtga aggttatttt 138420
   attatttgga gtataggttt ttgtttttaa atttatatgg agttttcgat acggggtatc 138480
   gattttaagg aggtgaatgt ggatatttaa ggttagtgaa tggttaggtg ttaatatatc 138540
   gataggtttt tggttttttt tttaatattg agggtaggaa ataaatgggg tttatatggt 138600
   gtatatggtt tttagagtta gtaaggtgtg taagattcgg tagttgagaa aattgattat 138660
   ttggtcgggc gcggtggttt acgtttgtaa ttttagtatt ttgggaggtc gaggcgggcg 138720
   gattacgagg ttaagagatc gagattattt tggttaatac ggtgaaattt tgtttttatt 138780
   aaaaatataa aaaattagtc ggacgcggtg gtaggcgttt gtagttttag ttatttcgga 138840
   ggttgaggcg ggagaatggc gtgaatttgg gaggcggagt ttgtagtgag tcgagatagc 138900
   gttattgtat tttagtttgg gcgaaagagc gagattttat tttaaaaaaa aaaagaaaag 138960
   aaaagaaaag aaaattgatt attcgattat gaataagtta gaatatttat ttagaaaaaa 139020
   aaaatagttt tgtttaattg gggatttaaa attttaggat ttatgttttt tttttataga 139080
   gttatattat ttttttaatg tttattttcg attttatttg tttgttaagt tttaggtatt 139140
   tagaatatag tatgatagta agttttgtga tttttatagg tttttttaat ttattttagg 139200
   tatagttata tttttagttt tgagtagtag ggagtgatat gtagatggtt atttagatag 139260
   agaaagattt ttaaaaggag ggtaggtttt gtttcgtagg tagtatttat tgatatagat 139320
   attagtaggt aaatttttta ggaagatttt tattaagaaa gaacgaaaga ggtttagtgc 139380
   ggtggtttac gtttgtaatt ttagtatttt gggaggttaa ggcgggtgga ttattttagg 139440
   taaggagttc gatattagtt tggttaatgt ggtgaaattt tatttttatt aaaaatataa 139500
   aatttagttg gacgtggtgg tgggtatttg taattttagt tatttgggag gttgaggtag 139560
   gagaattatt tgattttagg aggtagaggt tgtagtgagt taggattata ttattgtatt 139620
   ttagtttggg gggtaataga gagagatttc gttttaaaaa aaaaaaaaaa aaaaaaaagg 139680
   atgaaagata aataaggttg aaattggtag gttggtatat ttttaataat ttattttagg 139740
   tcgggcgtag tggtataatg ttagtatttt gggaggtcga ggagggtaga ttgcgaggtt 139800
   aggagatcga gattattttg gttaatacgg tgaaatttcg tttttattaa aaatataaaa 139860
   aattagtcgg gtgtggtggc gggcgtttgt agttttagtt attttggaag tttaggtagg 139920
   agaatggtgt gaattaggga ggcggagttt gtagtgagcg gagatcgtat tattttacgt 139980
   tagtttgggc gatagagcga gatttcgttt taaaaaaaaa aagaaataaa aaataaaaat 140040
   aataaaaaaa aaataattta ttttagtagg gagtaggagg taaggtttga tatgtagtgt 140100
   ttgttaattt ttgtggtgta aatatattta ttgtggtaaa ttttaagtta taaataggat 140160
   attattaaat atagagttgg gaaaagatat gtatattatt ggataaaacg tttaggtagt 140220
   tacggtttat ttttttttgg gaaggggggt atgtttagaa tgttttttat tatggagaat 140280
   gattgtggta aatagggttt aattggtagg ttgatttaat taattgttta ttgatgtgat 140340
   gtggggtagg tttttgaggt tgtttttatg tttagggaag aaggtggtta tgtggttata 140400
   gatttggttt tgggtttgtt tttttttttt ttttttgaga tagagttttg tttttttttt 140460
   ttaggttgga gtgtaatggt gtgattttgg tttattgtaa ttttcgtttt ttgggtttaa 140520
   gtaatttttt tgttttagtt ttttaagtag ttgggattat aggtgtttat tattatgttt 140580
   agttaatttt gtatttgtag tatagatggg gttgttggtt aggttagttt taaatttttg 140640
   attttaggtg atttatttat tttggttttt taaagtgtta ggattatagg tatgagttat 140700
   tgcgtttatt cgggtttgtt attttgagga tatatttgga gttatgtgtt tatgattttt 140760
   tttttatttt aaaattttga ttttatttta ttgtttttag agataggttt tgttttgtta 140820
   tttaggttgg agtgtagtgg tgtaattata gtttattgta gtttcgaatt tttaggttta 140880
   agcgattttt ttattttagt ttttcgagta gttggggttg ggtttatagg tgttattata 140940
   tttagttata ttttatattt ttttgtagag atagggtttt gtaatttgtt taaattggtt 141000
   ttaaattttt ggttttaagt aattttttta tttttagttt tataaagtgt tgggattata 141060
   gatgttagtt agtgtattta gtagttatta tgattgattg gtgacgtttg ttttagtttt 141120
   aggatgaaag agtagtagta ttttagttaa gtatttgtgg tttttgattg gaagacgttt 141180
   tttttgtttt ttaagtatag tgtaaatgtt atttttttgg taagtttttt tttaatttta 141240
   tttttttttt ggtatagggt ttgttatagt tttgtttata taaagagagt tattagtgta 141300
   tgtggtatag atgttagttg tttttttaat atttgagttt tttttttttt atagtaattg 141360
   aagttttggt tgtgtatagg gaagtttagt tgaagaatat attgtttagt ttttttgatg 141420
   ataggagcgg ttatataatt gggttttggt tagtgggaag gagttgaagt gtttaatttt 141480
   taggttatgt ttttaaaaat aaagagggat atttgttttt tttttttttt tttttttttt 141540
   tttttttggt tggagtgtag atatgacggt aggagttgga attattttag tttaagagat 141600
   ggaagttgtg tgtcgaggat gtattaaagg aatgaattag gttcgggttg ttttgggttt 141660
   ttataacgat tgatatttgg attgggacgt gggttcgagt gttgtttagt ttttgatgtt 141720
   ttagtttttg ttatatagtt agatggtgtt ttgattaatt taaggtatgg ttattatttt 141780
   atttgtttgg tagggtaggg gttttatttt tatacgttga gtttttggta gaatttggtg 141840
   taatatatat agttaaagta aaatgaatag gttattttaa ttaaggttta ttttgtgggt 141900
   tggttttggt atttggtttt aatttttatt ttattttatt tatttattat ttttgagata 141960
   gagttttata tttgttgttt agtttagaga ggagtggtgc gatttcggtt tattgtaatt 142020
   tttatttttt aggtttaagt gattttcgtg ttttagtagt tgggtagttg ggtttttttt 142080
   gagtagttgg gattataggt gtacgttatt atgtttggtt aatttttgta ttttttgtag 142140
   agatggggtt ttattatgtt ggttaggttg gttttgaatt tttgatttta agtgatttat 142200
   tcgttttggt tttttaaagt gttgggatta taggtatgag ttatcgtgtt tagttagttt 142260
   tggtatttgg aattggaaaa taatagtttg agttttagag gggataaata taaatatgag 142320
   gtagaaggaa tagtaagtaa atacgtgttt agagtaggtt gggttgttta tatttttaaa 142380
   atatcgttag gtgttgaggt ttgggttttg tgttagattt ttgggtgtag atttcgtggg 142440
   gatatttatt gggtagtggg atttttggta agttatatta tttttttgag ttttcgttga 142500
   ttaagttgtt tattgagaat aatatttaga ttgaaaggat ttataataat atgtgaaaag 142560
   tgtttggttt aaaataggag tttggtaatt ggtagttttt attatttata atatatagtt 142620
   taacgttttc gttttatagg aatgaaaata ggatttagat aggtaaaatg atttgttgag 142680
   ggtcgtatag tgttaatgat agagttagga ttagggtttg gggttttgat tttttttgta 142740
   gtattttggt tttgtgagaa gtggttttgg gtgaatataa ggagtgagga gttggtgaga 142800
   aattttgaaa atgaagttgg gtgtggtggt ggatgtttgc ggttttagtt atttaggagg 142860
   ttgaggtagg aggattgttt gagttcggga gttggaggtt gtagtgtgtt atgattgtat 142920
   tatcgtattt tagtttaggt gatttagtaa gattttattt tagaaaagga aaggaaagga 142980
   gaggggaggg gaagggaggg aatttttgat agtgatttta gagataatta ggatgatttg 143040
   aaattatttt gaaatgtatt aaaaaataaa aataaaatgg atttatggtt aggcgaagga 143100
   gggatagatg gacggtgagg tgataaagta ggtgtagttt ggtgttagtg gtagaattta 143160
   ggtgatggtt gaatgtgttt tttgtaaaat gtttttaatt tgattatgtg ttttgaatat 143220
   ttttataata gaatggaggg aagagaaagg taagagggag ttattgtagt tgttgtagta 143280
   ggagtagatt ttaaaatatt atgtattaga tttttagttt tgtttttgtg gatgtgtttt 143340
   tgtttttttt tttgggattt aaggatattt aatgagcgta tgtgttttta agtttttttg 143400
   tttttttatt tgtttttata tttggaagat tttataggtg gcgtgggtta gttttttatt 143460
   ttgttttttg tgtttttagt gtatgataga tattgagtga gtgttagttt tttttttcga 143520
   tattttgttt gtttgtttag agaggttttt ttatagttta gttattaggt tgatgttttt 143580
   ggacgtttgg tcgatagttt ttttagttgt gttttagtga tttggtaggt gttttgagtt 143640
   ataaataagg ttattagtgg atgtttttaa agtttttttt attacggcgt tttttttttt 143700
   tttattaata gttcgttatg atagttagtt tgtatgagtt tttttttagg gttgtgtttt 143760
   ttgaggttgt ttagcgtttg gtttgttaga gtttattaga tagggtgttt atttttttta 143820
   ggtttgagtt tttatttaga gaattattta tagaaggata atggagaaag gtagttgggg 143880
   tattaaatgg agtgtgtttg tatgtggggg gggaggggag ggggagggaa agtaggaaga 143940
   gaagatataa gaggagaaat aagaataaga ggaaatatag aaggaggagg agtagttttt 144000
   tttatggggg tggattttgt gtttagtttt ttattaagga tattatattt attatttcgt 144060
   ttggttttta cgaatatttt taaggtcgtt tttttttggt ttttattttt ttattaggaa 144120
   gcggaggtat agagtgagaa agagttgttt aaggttttta ggtagtttac ggagttattg 144180
   ggagttatat ttaattcgtt taattttacg tttttgtttt tagttattat aataaattat 144240
   taaggtttta tagttatagg agtttagttc ggagttttta tcgggttttt taaggtaggg 144300
   agatggggtt ggaggttatg ttaggtgtac ggtgtttaga gttgtttagg aagtttcgcg 144360
   gtgcgggttg ggtattaggt agtgtttgta ggatggagaa atgaatgagg ttgagatata 144420
   gtgtcgtttt ttttaaacgt gatttttttt gagtttttgt gattggttat ttgagagggt 144480
   agttttggtt ttttggcgtt tatgttcggg tttagagatg ttttttttag ttaggttttt 144540
   ttttgaggat ggaaatttag aattttatta taggaggaag agttgggagt attttatttt 144600
   tttttttttt ttgagacgga gtttcgtttt gtcgtttagg ttggagtgta gtgacgcgat 144660
   ttcggtttat tgtaagtttc gttttttggg tttacgttat ttttttgttt tagtttttcg 144720
   agtagttggg attataggta ttcgttatta cgttcggtta attttttgta tttttagtag 144780
   agacggggtt ttatcgtgtt agttaggatg gtttcgattt tttgattttg tgattcgttt 144840
   atttcggttt tttaaagtgt tgggattata ggtgtgagtt atcgcgttcg gttagggagt 144900
   atttttaaga tttttgcgga gggttgttta ggtttaagtt ttttattatg gaaggttttt 144960
   tagatgcggt agttacggtg tatgggttga gggtttttat ttattaagaa gtaggagtat 145020
   ttgatgtata tttagtttac gatttttatt tatttggggg ttgtgtttgt agttacgtga 145080
   tataagggaa tagttatagt agtgggttat aaagagagtg tgggttaatg tgtaggggta 145140
   gaattttgat agttttttat gggttggtag tagggagggg tatatttgta taaattttat 145200
   acgtatagtt ttttatatat atgtggttga taattattta tatatttaag acgggatttg 145260
   gcgtatagtt tattagtaat aggcgttggt aatttttagt tgtttttttg ttgcggcgta 145320
   gatattattt aaggtgggat ggagagagtt attgttttta aggttatata ttttaaaggg 145380
   agatgttggg ttacggaatg aaaataggag gattagttag agttattggt tttgagttat 145440
   ataatgggat atataggttt atttatttta tatttttatt gagtaattat aacgagttaa 145500
   gttgtataga ttatttaaat aatcggtacg tagaaaagta ttttaatttt tttttggttt 145560
   tgacgtgatg ggaagaattt ggatttttgg agttatagag ttggatggaa attttggttt 145620
   agttaattat tggttggggg agttcgggta agtgatttaa ggtttttgag tttgtttttt 145680
   tgtaaaagag tagtgttcgg gtttgttacg ttaggttagg gtaagatgat tgaagagtaa 145740
   gcgtttagta tcggttaggg gttaacgttt ttaaggacgt aaagtgtaat aagtaggcgt 145800
   tacgattgta tcgtttttta ttgtttgtgg taaatatata agtaaagatg gtttcgcgtt 145860
   tgcgttgggg gaggtaggga attggtttta ggaggacgtt ggttttttta gttgtttttt 145920
   ttaaagtttt ttgggaggag aaatgattta ttttagttgt tgggagtttt aatttataag 145980
   taggtttgtt tattcggtag cggtcgtatg taggtaggag gggtttcggt agtttttaag 146040
   agtgaggggt cgtttagttt ttttttagtt ggaaaggagg ttttaggttg ggtaggagta 146100
   tttggggaaa tttagttttt ggagttaggt tatattaggg agatgaggtt gttagaagtt 146160
   tttttggtgt ttatagtaaa atataattaa agtgggattt agattttaat tagtttaagg 146220
   tttgatttat attagaggga aattagcgtt tgtcgtatga gtgagcgttt atgtgcgtgt 146280
   aaggtagaga tagattttgg gtaagttttt ttattttttt acgtttgttt ttttatttat 146340
   aaaacggggt aatattattt attttatagg gttgttatga aataaatatg tattaatatt 146400
   tgtaaagtgt tgagaatagt atttggtata aaataagagc gatagatttg ttaaataagt 146460
   aaaaaataag atataagttt aaagtagagt ttggtatatc gtgagtatta agagttgtgt 146520
   tttttgattt tttttttttg tggagatttt tttattagaa gtgggaggga ttgtcgaggt 146580
   tggagttttg ttttagggat ttttgtgggg agttttttag tttagaggga tttgttttac 146640
   ggattatatt tgtttcgggt tttcgtgttt ttgtgtatgt gtgtatatat gtgtgtatat 146700
   atatgtaatg tgcgtgtgtg tatttttatg gatttgtgat aatttgtata tatatgttta 146760
   gaatttattt gatatagtgt tttattaaga tgttggagtt tcgatgaatg gattgcgggg 146820
   ttgagatatt gtttttatag gaagttgttt tagtagtaag ttttttggtt tttatagata 146880
   attaaaaaga atggagtttg tttttatttt ttgttttaaa aatgatgtat agtgtgtggg 146940
   gtttgatgtt ggaggttttt ttagtagata tatttttaat aatattatat tttgaaattt 147000
   aagttgtcgt aaaataagat aagtaaagat ggtattggta gggatttttg aggttttttt 147060
   tatgtatttt tgaataattt ttatatggtt tttgtttgaa tgtttttagg tatggggaat 147120
   ttattatttt gtgaaatagt ttgttttatc gtttttaagt gaatattgtg agaaaaatat 147180
   ttttttttgt aaagagttaa gttttattcg ttagtatttt ttaggtattt ggtattagtt 147240
   ttatattttg gggaatttta aaataaatgt ttttttttta ttagataatt tattaattat 147300
   ttggagatag tggttgtagt tttttgtttt atgtagttat tttatttagt ttttttttgg 147360
   aatttttgac gtgaatataa gtttttttgt tacgtaggtt ttttttttgg gttttttatt 147420
   tatttgcgta tattttggag aggtcgttcg ttttgtgggt gagatttatt gtgggttgaa 147480
   ttgtgttttt ttaaaagata tatgtaagtt ttgatttttt agtatttatg aatgtgattt 147540
   tatatggata tagggttttt gaagatgtaa ttgggttatt tgagggtata ttgaattaga 147600
   gtgggtttta aatttaatga taggtgtttt tataagatag aggtaggttg ggtatagtag 147660
   tttatgtttt taattttagt gttttgggag gtaaagtttt attgtttgag cgtaggagtt 147720
   tgagattagt ttgggttttc gtttttataa aaaaattaaa aaattagtta ggtatggtgg 147780
   cgtgcgtttg tgttttagtt atttggaagg ttgaggtggg aggattgttt gagtttggga 147840
   ggttgaggtt gtagtgagtt atgattacgt tattgtattt tagtttggag gatagaatga 147900
   gaatttgttt ttaaaaataa attaaaaaga taatagagag ggtagtttgg atagagatat 147960
   aagaagagat atagggtgaa ggttaggtgg ggggtaggtc gagttgtaag tcgaggaata 148020
   ttaggggttg ttggtcgtcg tagagttcgg agaaaggtat gggatagttt ttttcggaat 148080
   ttttaggagg aatgaatttt tagatatttc ggtttgggat ttttggtttt agaaatttta 148140
   gggaagatat ttcggatgtt tttgtttttg tttttgtttt tgtttttgag acggagtttt 148200
   gttttgttat ttaggttgga gtgtaatggc gcgatttcgg tttattgtaa tttttatttt 148260
   taaggtttat gttatttttt tgttttagtt ttttgagtag ttgggattat aggtatttat 148320
   tattacgttc ggttaatttt tttttttttt ttgtattttt agtagagacg gggttttatt 148380
   atgttagtta ggatggtttc gattttttga tttcgtgatt taatcgtttc ggttttttaa 148440
   agtgttggga ttataggtat gagttatcgt acgtggtcga tttcggatgt tttgagttat 148500
   tttatttgtg gttttttgtt atgatagtcg taggatagta atatagggtt ttagtagggt 148560
   ggttcggtaa gggttttttt tttacggtag tttttatttt gttttatttg tagggttatt 148620
   tagatgttta tttcggtttt ggttagtaag tgttagtgta tttttttacg tttagtagta 148680
   agggtagtat cgttgttgat taagtatttg ttatgtcggg gtttttgtgt ggttttgggg 148740
   tttttttttt ttgtttatat ttggtagttt agtttttttt tttgtatttt atttgatgtc 148800
   ggggttttgg gtaagtttgg gggatttgtg ttttatttta tagatttttt agttgatttt 148860
   attagttttt gtatagggtt cgtttgaagg gtcgttgggt tgggcgttaa ttagagtttt 148920
   tagtagtagt tttttcggtt ttttgatttt ggtttatgtt ttaggggtag ggtttttaag 148980
   ggttttgggg tcgtttttga ggttagtttt tttgttgatc gtagtttata tagaggaggg 149040
   tttaattttg agtgtggtcg ttcgtgattt tgagagatgt ttagagttgt agacgtgttt 149100
   tttaagtttt ttagagttcg ggataggagt tatttaatgt ttttagtgtt ataggtaaaa 149160
   tttgtttttt ttaatataaa ttgtttttag ttttatttaa tgtttttttt aacgagtagt 149220
   aataaaaata tggttttttg ttaaaggtgg ggtttatagt tttttagaat tcggtttttt 149280
   gttaggttta ggtataattt ataggagtgt tcgtttttga ttcgtgtgtg tgtgtgtgtg 149340
   tgtgtgtgtg tgtgtgtgtg tgtgttgtgt gtacgcgtat atgtgtttgc gtaagttttt 149400
   ttatgtgtag ttttgggaag ggtggagagg tttacggaaa ttttttttgg tatttagtaa 149460
   gttagaaaaa aggaaaatga gatttgtgta tatatgtgta agtgtatata tagtatatag 149520
   gtatatatat atatatattt agtatatatt atataggttt atacgtgtat atatgtgtag 149580
   gtatatatta tataggttta tacgtgtata tatatggggg tatatagtat ataggtatat 149640
   ataggtatat atattttttt gtaggaaagt ttttttttgt tttttatttt gtagggtttt 149700
   taggttttat gaaataagat tgaattttgg aagtttgata aaaggtttgt ttggagtatt 149760
   ttggttattt ttattagttt ttttagttgt ggaaggtatg tttcgtggta tttatttatt 149820
   ttttgttttt atttttattt tcgttttcgt tttttttttt ttgttttttt agtttatatg 149880
   ttgtttgttt taattttaat ttttgtattt ggacgggaat tatttttttt gtagaagggt 149940
   gtaatgtatg tggggatata tatatatata tatatatata tattgtattg gaggagagaa 150000
   gagaggtaga gtttttttta gatttagagt tttagcgagt gacgggattt tttttagaga 150060
   atacgtgtag aagtttgcgt tattggaaga agatattatt ttgatttata tttttttttt 150120
   tttttttcgt tttttttata gagaaggaaa ggttttagta aatagtggtt atgtgggttt 150180
   tttgtatgtt attggtagtt ttttagttag gattattttt tttttatttt tggaagtata 150240
   agaatagaga gaagggtgag tttttgtttt tgggggtttt tttttgtatt tttttggagt 150300
   taattttagg gttgtgggtt gtataggaag gagggtttaa taggtttttc gtttaatttt 150360
   gtttttttag tgaggagtcg atttattttg tttgtaaaag atgttggttg ggatttgatg 150420
   ttgggtaatt tgaggagaag agaaggggga taggggttga ggttaggttt ttggttttga 150480
   aatagttata atttttgggg tagagtttaa gggtgttagg ttggagattt tatattcggt 150540
   tagttggtag ttattatttt ggtttagtta gtggtttagg gtattatagt tatttagatt 150600
   aggaatttta tttttttgga ttgtattaat gaggaaggtt gagtttgtag gttttatata 150660
   tttatggtaa ttttaaggta gagaggttat tgttaattga gggttacggg taggagtttt 150720
   gtgagtttat tagtaagttt ggaagattaa ggtttttggt tatagagagt agttgttatt 150780
   tggttaattt tgtagaaaat attatagttt tggataaaat atttgaaaga ataaaataat 150840
   aatagtttgg agttagtata gaatatttta agtaggtaga agttggagta gagtttatat 150900
   ttgatagagg ggaataaaat tagataagtt tttggttttt ggaggttttt ttgtttagag 150960
   gtaggtattg agtaattaaa tttggataaa aagttttaaa agtatttagg gaaaaataat 151020
   atgtggttta taggggaata gttagatgaa tgttgatttt ttgatagaaa taacggaggt 151080
   taaagatatt ggaattttat attttaaagt tttgaaagaa aaaattgata atttagaatt 151140
   ttataattag tgaaattatg gtttaagaat gatagtaggt tgggtatagt ggtttatatt 151200
   tataatttta gtattttagg aggttaaagt aggtagatta tttgaggtta ggagttcgag 151260
   attagtttgg ttattatggt gaaattttat ttttattaaa aatataaaaa ttagttgggt 151320
   atgatggtat atgtttgtag ttttagttat ttgggaggtt gaggtaggag aatcgtttga 151380
   gttcgggagg agaaggttgg tgagttgaga ttgggtagga gttttgaaat tatattattg 151440
   tattgtagtt tgtggggaga tagagtgaga ttttgtttta aaaataaaaa taaaaataaa 151500
   aaaagaaaga atgatagtaa aataaagatt tgtagataag tgaaaatgaa agggaattag 151560
   ttgtttgtaa atttgtatta taaaaatcgt gaagtaagtt ttttaagatt atgggaaatg 151620
   agattagatg ggaatttagt tttttaggaa ggaatgaaga gtataggata tagtaaatat 151680
   atgggtaaat agaaaagatt ttgttttatt tttaattttt ttaaaatgta tggggttgtt 151740
   taaagtaaaa atttttttat tgtattgttt agtttataat gtgtatggat gtagtaaata 151800
   tttaataatt attatatata ggatggaaag tggtaaatga atttatagga ttgtttagtt 151860
   tttatatttt ttttttatga agaaaaataa tgttaatttt aagtagattg tgaaaagtta 151920
   agaatgtgta atagaattta taaagtaatt attaaaatag taatataaag agatataatt 151980
   aaaaagttaa aataggtcgg gtttagtggt ttacgtttgt aattttagta ttttgggagg 152040
   ttagggtggg cggatcgttt gaggttagga gttcgaggtt agtttggtta gtatggtgaa 152100
   atttcgtttt tattaaaaat ataaaaatta gtcggtcgtg gtggtaggcg tttgtaattt 152160
   tagtatatta gggaagttga ggtaggagaa ttatttgaat ttaggaggta gaggttgtag 152220
   tgagttaaga ttatgttatt gtattttagt ttgggtaata gagtgagatt ttgttttaaa 152280
   aaaaaaaaaa aaaaaaaaat ataaattaaa atagaatttt aaaaaatatt taattaatat 152340
   aaaagaagtt aagaaagtaa gatgaaagaa ataaatatta aagagtaagg tagtagtttt 152400
   aaatttagtt atatttataa ttatgttaaa tagaaatagt ttgaatattt taattaaaag 152460
   gcggaggttg ttagaatgaa taaaatagta agatttaatt atatattatt tataaaagat 152520
   gttgttttag tataaatata ggttgaaatt ttttgtttgg aaaaagatat attgatgttg 152580
   tttgaatgat gatggagtta tgttttgata aatttattat aagtggaaaa taaatattat 152640
   tttaaaaatg tatttaatat atttaattta tcgaatattt tagtttagtt tagtttattt 152700
   taatatgatt agaatattta ttttagttta tagttgagta aaattaataa atataaagtt 152760
   tattttataa taaagtgttg attattttat gtaagttatt gaatattgta ttgaaagtga 152820
   gaaatataat ggttttatat atatttaaag tatagttttt attgtatata tattacgttc 152880
   gtattatggt aaagttgaaa aattgtaagt taaattatta taagtttttt tataagtttt 152940
   tttagttttt ttatgtattt taaatagtat gtttaagaag gttggagagg ttatataaat 153000
   attaataaag ttgagtttaa gataaaatta ttacgagata aaggaatata tttattatgg 153060
   ttaaatgatt agtttatcga gatattatta taattataaa tgtgtatgta tttaattaaa 153120
   ggagttttaa aatatatgat ataaaatttg aaagaattaa agggagaaat gggtaatttt 153180
   ataattatag gtggagattt tttttttttt tttttgagac ggagttttat ttttgttgtt 153240
   taggttggag tataatggtg taatttcggt ttattgtaat ttttattttt tgggtttaag 153300
   cgattttttt gttttagttt ttcgagtagt tgggattata ggagtttgtt attacgttta 153360
   gttaattttt gtatttttag tagagataag gttttattat gttgtttagg ttggttttaa 153420
   atttttgatt ttaggtgatt tattcgtttt ggttttttga agtgttggga ttataggtgt 153480
   gagttattgc gtttggatgt atagttggag attttaatat tattttagtt tatttaggat 153540
   gttaaaataa aatgttttag gttgggtaat ttataaatag attgtagtgt ttatagtttt 153600
   agaggttggg aagtttaaaa ttagggtgtt tagtgtttgg tgagagttag ttttttgttt 153660
   tagagatgtt ttttttttgt tgtgttttta tatggtagaa gggattaatt ttatttacga 153720
   tgattttgtt tttatgagtt aatatttttt tgatgtttta atttttaata ttattgtatt 153780
   agagattaag ttttaatata tgaattttgg gggatataaa taggtaggtt atagtaaata 153840
   ttattttttt aatatttgag aaataagtat tagataattt gaagaatatt ggtaattagt 153900
   aattgattta attagtagtt ataaagtttt atatttagga agtttagaat atatatatat 153960
   attttttttt taagtgaaaa tagtgtattt attaagatat atattttggg ttataagttt 154020
   taaattttaa aagtatgtaa aatatgtttg tttggttaaa gtaagattaa attagaaatt 154080
   aatagtttta agatatttag aggagttttg attatttgta aattaaataa tatattttta 154140
   aataatttat gagttaaaga ggaaattata agaaaaattt tataatattt taaatgaaat 154200
   gataatgaaa atatagtata ttaaaatacg taggttgaag ttagtgttta gagggaaatt 154260
   gtaagtgttt atattagaaa agaagtaaaa aaaaaaaaaa aaaaaaaaaa aaaaagattt 154320
   aaggatttgt tttaagaatt tggaaaaatg ggttaggtgt agtgtagttt acgtttgtaa 154380
   ttttagtatt ttgggaggtt aaggtaggtg gattatttga gattaggagt ttaagattag 154440
   tttggttaat atggtgaaat tttgttttta ttaaaaatat aaaaattagt tgggtatggt 154500
   gggtattttt agttatttag aaggttgagg taggagaatg gtttgaattc gggaggtgga 154560
   ggttgtagtg agtttagatt atgttattgt attttagttt gggtaataga gtaggatgtt 154620
   tttttaaaat aaaataaaat aaaataaaat aaaataaaat aataaaaaaa ataaagaatt 154680
   tggaaaaata tgttagttat ttatgtattg ttttttaatt ttgtatatat tttgttaggt 154740
   ttcgtttcgt gatattggat cgaggtttta tgagtttttt tttttggtta aatatagtta 154800
   tgttaggttt tatttatagg ggataatatt gtggggagtt gttaggtttt gttgaggtag 154860
   tagttttttt ttttggtttt agggtttttt ttttttttga aggtatagtt gttagtggcg 154920
   tgtgtttagt ggggtttgtt ttgtattgag tttttttagt attttagttt tagtttaatg 154980
   ggattttagt aaatttttta gatatttagt ttttgtttat ttatattttt tagtaggttt 155040
   ttcggttatt tagtaagttg attttgtaga tttatttttt ttatattttt tggtaagaat 155100
   ttttttgtta ttagtaggtt gtaggttttt gtggtagtta tatatttttt aataaggttt 155160
   gaacgtaaga cgtggtaagg tgtggaggac gtttcgagtt tttggttttt tttttattaa 155220
   ttttagtggt agtaattgtt tttgtgtgtt tgtatatatg ttttaaagta tttattatag 155280
   aaaatttatt attttaatta tttttaggtg tgtagtttgg tggtatgaag tatatttata 155340
   ttgttgtgta attattatta ttatttattt tattttttta aattgtaatt ttatttttat 155400
   taaatattaa ttttttattt attttttttt tttttagttt ttggtaattt ttattttatt 155460
   ttttgttttt ttgaatttga ttattttagg gattttataa aagtgaaatt atatagtatt 155520
   tgttttttta tgtttggttt ttgttattta gtatgttttt aaggtttatt tatgttatag 155580
   tatgtgttag aatttttttt tttttttttt tttttttttt ttttgaggta gttttgtttt 155640
   gtcgtttaag ttggagtgta gtggtatggt ttcggtttat tgtaattttt atttttttag 155700
   tttaagcgat ttttttgttt tagttttttt agtagttggg attataggta tatgttttta 155760
   tgtttggtta atttttgtat ttttagtaga gatagggttt cgttatgttg gttaggttag 155820
   ttttaaattt ttgattttaa gtgatttgtt tatttcggtt ttttaaagcg ttgggattat 155880
   aggcgtgagt tattatattt ggtttttttt ttttttttaa ggtagaataa tattttattg 155940
   tatagatgga ttatattttg tttgtttatt tattcgttgg tggatatttg ggttgtttgt 156000
   attttttggt tatgggaaaa gtgttgttat aaataagaat gtagtatatt tgtttaagtt 156060
   tttgttgtta gttttttgga gtatatattt agaagtggaa ttattggatg ttatggttat 156120
   tttgagttta attttttgag taattgttat atttttttta tagtagttgt attattttat 156180
   atttttatta gtagtgtacg gggttttaat ttttttatat ttttgttaat atttgttgtt 156240
   atttatgttt tggttaatag ttattttaaa gggtgtgaaa ttatatttta ttgtggtttt 156300
   gatttttatt tttttgatga ttagtgatat tgcgtatttt tttttgtgtt ttttggttat 156360
   tggtatgttt tttgtggaaa aatgtttgtt tattgggttg tttttttttg ttttgtggtt 156420
   tagttgtagg agttttttat atattttgga tattagtttt ttatcggata tacgattttt 156480
   aaatattttt ttttattttg tggatttttt ttttacgtta ttggtagtat tttttgatgt 156540
   ataaaagttt taagttttgg tgaagtttaa tttattattt attttttttg ttgtattttc 156600
   ggtgttatat ttaagaaagt attgttaatt ttaatgttac gaagtttttt ttttatgttt 156660
   ttttttaaag tagttgtttt ttatatttgt tttttttata ttttttagag tttttttgta 156720
   taaggtaaaa taaattttat ttttttattt ttatttataa tatttagtgt tttagttttg 156780
   atattagatg tgtggttttt tttcgtcgat ttttaagtaa ttttttaata tttagtagtt 156840
   tttaattggg tgttttgtaa tttagtttaa ttttgatatt aattagagtt agtgtagatt 156900
   ttataggtta agggttttat tttataagat tgtttttaat tttagagatt agtcgtaagt 156960
   ggtaagatat taggttattt ataatttttg tttgatttgg ttataaattt gaggttttta 157020
   taattttttt tttaggttta ataatttgtt tgagtgattt atagaattta ggaaattagt 157080
   ttatttaaaa ttgttgattt attataaatg atattttaaa ggatataaat gaatagttaa 157140
   ttggaagaga tgtataagaa aggtatgaag gaagcggtat ggagttttta cgggagggta 157200
   tattgttttt ttagtatttg taggtattta ataatttgaa attttataaa ttttattttt 157260
   ttgggttttt agggaggttt tattatataa gtatgatatg aaatttttgg ttattgatga 157320
   ttaatttaat ttttattttt ttttgtttcg taggaggtgg atgggaaagg ttgtaagttt 157380
   tagtttttta attataaggt tgggtttttt ggtaattagt ttttattttt aggttgttta 157440
   ggagttttta gttattagtc gtttttttag taaataaaaa gatatttatt attttggaga 157500
   ttttaagagt tttaggagtt atatgttaaa taaagttggg ggatagagat tagatagata 157560
   gatagataga tagatagata gatagataga tagatagata gatagatata ttgatttttg 157620
   agatagagtt tttttttgtt atttaggttg gagtgtagta gtgtaatttc ggtttattat 157680
   aacgtttgtt ttttcgggtt taaattattt ttttatttta attttttaag tagttggaat 157740
   cgtaggtata tatcgttacg ttaggataat tttttgtatt tttggtagat atgaggtttt 157800
   attatgttgt ttaggttggt tttaaatttt tgagtttaag ggattcgttt gttttagttt 157860
   tttaaagtgt tgggattata aatatgagtt attgtatcga gtttagttta aatatatttt 157920
   ttttatatta taatgttatt tttttgtgat ttttttaata gttaattatt ttttatagtt 157980
   aagaatattt tatattataa gttgttattt aaattgtcgg tgtggttttt gttttttgat 158040
   tgggttttga ttaatgcgtt gaagtaagta taaggaagga agtaatgaag ataatagaga 158100
   aatttaagga aatggatatt agaaaagggg agtttaatgt tggtttttta aaaagattta 158160
   gattgataat tttttagtta ggttgttgat gagagggaga tggcgaatat aaagttataa 158220
   taaaaggaat gaagtgtttt ttttacggat tttatcgata ttatagtgat aaggaatata 158280
   ggtatatttt attttattgt gttttataga tattgtattt tttataaatt gaaggttcgt 158340
   ggtagttttg tattaagtaa gtttgttggt attatttttt taataatatg ttaatattgt 158400
   gtttttgtgt aggagtttga tgatttttga aatagtttaa atttttttat tattattata 158460
   tttgttatgg tgatttgtga tttttttttt tttttttttt tttttgtttt tttttttttg 158520
   agatagagtt ttgttttgtt tggagtgtag cggcgcgatt tcggtttatt gtaagtttcg 158580
   tttttcgggt ttacgttatt tttttgtttt agtttttcga gtagttggga ttataggtat 158640
   ttattattac gttcggttat ttttttcgta tttttagtag agacggggtt ttatcgtgtt 158700
   agttaggatg gttttaattt tttgatttta tgatttgttc gtttcggttt tttaaagtgt 158760
   tgggattata ggcgtgagtt attgcgttcg gttttttttt tttttttttg agatggagtt 158820
   tcgttttgtt gtttaggttg gagtgtagtg gagtgtagtg gtgtgatttc ggtttattgt 158880
   aatttttatt ttttaggttt aagcgatttt tttgttttag tttttttagt agttgggatt 158940
   ataggtattc gttattacgt ttggttaatt ttggtatttt tagtagagac gaggttttat 159000
   tatgttggtt aggttggttt taaatttttg attttaggtg atttatttgt ttcggttttt 159060
   taaagtgttg ggattatagg tatgggttat cgcgtttagt gattagtgat ttttaatgtt 159120
   attattgtag ttgtttttgg ggtatgacga gttatattta gataagatag tgaatttatt 159180
   taataaacgt tgtatgtttt gattgtttta ttgattggtt gtttttcgtt tttttttttt 159240
   ttgtttaggt atttttattt tttgagattt aataatattg aaattaggtt aattaataat 159300
   tttataatgg tttttaagtg tttaagtgaa aggaagagtt attcgttttt tattttaaat 159360
   taaaagttag aaatgattaa gttgagtgag gaaggcgtgt cgaaagttaa gataggtaaa 159420
   agtgagattt tttatgttaa atagttagtt aagttgtgaa tgttaagaaa aagttattga 159480
   aagaaattaa aagtgcgatt ttagtgtata tatgaagaag aagaaagtaa aatagtttta 159540
   atgttgatat gaagaaagtt tgagtggttt ggatagaaga ttaaattagt tataatattt 159600
   ttttaaataa aagtttaatt tagggtaaag ttttaatttt ttttaatggt atgaaggtta 159660
   agagaagtga ggaagttgga gaaggaaagt ttgaggttag tagaggttag gttatgaagt 159720
   ttaaggaaag aagttatttt tataatgtaa aagtgtaagg tgaagtagta ggtgttgatg 159780
   gagaagcggt agtaagttat ttagaagatt tagtttagag tattgatgaa ggtggttata 159840
   ttaagtaata gaattttagt atagatgttt tattttttat tggaagaaga tgttatttag 159900
   gatttttata gttagagggg agaagttaat gtttgttttt aaagttttaa aggataggtt 159960
   gatttttatg agaggttaac gtagttgttg atcgtaagtt aaagttaata tttattgatc 160020
   gttttaaaaa ttttagggtt tttaagaatt ttgttaaatt tattttgttt atgttttaga 160080
   aatggaataa taggttttat ttgatagtat atttgtttat agtatggttt attgaatatt 160140
   aaaaatttat tgaggtttgg tgcggtaatt tacgtttgta attttagtat ttttgaaggt 160200
   tggggcgggg ggattatttg aggttaggag ttcgagatta gtttggttaa tatggtgaaa 160260
   tcgtattttt attaaaaata taaaaattat ttaggtatgg tgttatacgt ttgtaatttt 160320
   agttatttgg gaggttgagg taggagaatc gtttgagttt aggaggcgga ggttgtagtg 160380
   agtcgagatc gtgttattgt attttagttt gggtaataga gggagatttt gttttaattt 160440
   gaaaaaaaat aaaaaaataa aaaagggtta agtacggtgg tttatagttg taattttagt 160500
   attttgggag gttaaggtag gtaaattatt tgagtttagg agtttaagat tagtttggtt 160560
   aatatggtgg tgaaatttta tttttattaa aaatataaaa attagttagg tatggtagtg 160620
   tatatttgta gttttagtta tttaggaggt tgaggtagga gaattatttg aatttgggag 160680
   gcggaggttg cggtgagttg agatagcgtt gttgtatttt agtttaggta ataaagcgag 160740
   atttgttttt aattaaaaaa taataataaa aaaaaaataa atagggttcg gtgtagtggt 160800
   ttatgtttgt agttttaatg ttttgagagg ttgaggtagg tggattattt gaggttagga 160860
   gttcgagatt agtttggtta atatggtgaa attttgtttt tataaaaaat ataaaaatta 160920
   gttaagtatg gtggtgtgtg tttgtaattt taattattta ggaggttgag gtataagaat 160980
   tatttgaatt cgggaggtag aggttataat gagtcgagat tatgttatcg tattttattt 161040
   tgggtgatat agcgagattt tattaaaaaa aaaaaaaaat tattgagatt tattgtttag 161100
   gaaaaaagaa tttttttaaa atattattgt ttattgataa tgtattttgt gatttaagag 161160
   ttttgacgga gatgtatgta aagatgaatg ttgtttttat gtttgttaat gtaatattta 161220
   ttttgtagtt tatggagaag ggataaattt gatttttaag ttttattatt taagaaatat 161280
   attttataaa gtagtatttg ttatagttag tgattttttt aatggatttg ggtaaagtaa 161340
   attgaaaatt ttttggaaag gattttttat tttagatgtt attaagaata tttgtgattt 161400
   ataagaagag gttgaaatat taatattaat aggagtttgg aagaagttga ttttaatttt 161460
   tatggatgag cggtttaaga ttttagtaga ggaagttatt gtagatgtgg tggaaatagt 161520
   agagaattag aattagaagt ggagtttgga gatgggattg gattgttgta attttatgat 161580
   taaatttgaa tagatgagga gttgtttttt atggatgagt agataaagtg gtttttggag 161640
   atgggattta ttttaggcga agatgttgtg aatattgttg aaatgataat aaaagattta 161700
   gaatattata ttaatttatt tgattaattc gtggttaggg ttgaaagaat tgattttaat 161760
   tttgaaggaa gttttattgt gggtaagatg ttattaaata taatgcgtgt tatagaaaaa 161820
   ttttttatga aagaaagagt taatttatgt taaaaatttt agttaagaaa ttgtcggtta 161880
   ggtgtagtgg tttacgtttg taattttagt attttgggag gttaaggtag gtggattatt 161940
   tgaggttagg agtttgagat tagtttggtt aataaggtga aattttgttt ttattaaaaa 162000
   tataaaaatt agttaggcgt ggtggtaggt atttgtagtt ttagttattc gggaggttga 162060
   ggtaggagaa ttgtttgaat tcgggaggta gaggttgtag tgagttgaaa tcgtgttatt 162120
   gtattttagt ttgggtaata gagtaagatt ttgttttaaa aaaaaagaaa aagaaaaaag 162180
   aaataaagaa attgttgtta tagttatttt taatttttag taattattat ttggttagta 162240
   gttattaata ttgaggtaga ttttttatta agaaaaagat tatggtttgt tgaaggttta 162300
   gatgattatt agtatttttt agtaaaaaga tataatgatt ttatatattt taatagatta 162360
   tagtatagtg taaatataat tttttttttt tttttttgag atggagtttc gttttgttat 162420
   ttagtttgga gtatagtggt gtgattttag tttatcgtaa tttttatttt ttaggttcga 162480
   gtaatttttt tattttagtt ttttaagtag ttgggattat aggtatgtgt tattatattt 162540
   agttaatttt tgtattttta atagagatga ggttttatta tgttggttag gttgttttta 162600
   aatttttaat tttaaatgat tcgtttattt tggtttttta aagggttagg attataggta 162660
   tgagttatta tgtttggttt aaatataatt tttatatgta ttgggaaatt aaaaagtttg 162720
   tgtgacgtgt tttattgtaa tatttgtttt attgtcgagg tttggaagta aattttaggt 162780
   atgtttgtat tacgaataat tttaaatgag taaatttaat aatttatttg aaagggataa 162840
   atttttttaa agtataattt attaaaagta agataagggg aagtaaaaaa atttgaataa 162900
   ttttttttat atattaaatt gaatttgtga ttaaaaataa ataattattt tttttagtag 162960
   aaaattttag gtttagacgg tttttttggt ggattttatt aaatatttaa ggaagaaata 163020
   atattaattt tatgaaaata tagaaaataa agagaaggga atatttttaa attaatttta 163080
   tgaagttaaa atgaggttga taaagatatt atcgaaataa attaataaat aaagaaaata 163140
   ttatattaat attttttatg aattaatacg taaaaatttt taaaaattgg gtaaattaaa 163200
   tttagtaata tataaaaagg atgatatttt ataagtaagt tggatttttt ttagaaatgt 163260
   aaggttggtt taatattaaa aaattaggtt tgtatagtgg tttatatttg taattttagt 163320
   attttgggag gtagaggtgg gagtattgtt taagtttaag aattgtagat tagtttgagt 163380
   aatataatga gattttattt gtataaaaaa ttttaaaaat taaaaattag tttggtatgg 163440
   tggtatgtgt ttatagtttt tgttatttag gaggttgagg tgggaggatt gtttaagttt 163500
   aggagtttag gttgtagtga gttacgatgg tattattgtg ttatagtttg ggtaagagtg 163560
   agattttgtt tttttaaaaa aaaaaaaaaa aaaaacgggg ggtaaaaatt aattattgta 163620
   atttatttta ttaaacgaat aaaagataaa ataatgatta tttttataga tgtagaaaaa 163680
   gtatttatta aaattatatt tatttatgtt aaaaaatttt agtaaattaa gaatagaagg 163740
   gaattttttt aatttgataa aggttatata tgataaattt aaagttaata ttataagttt 163800
   ggtataggta aggatgttta ttaattatta ttatttaata ttgaattggt agttttaatt 163860
   agtgtaataa agtaagaaaa aaaaataagg tattaagatt agaaagaagt aaaattatgt 163920
   atttaaattt taaaagattt ataaaattat tattagagta ataaatgatt ttggaaaaga 163980
   ttataggata taagatttat atataaaaat ttattgtatt tttatatatt agtagtaaat 164040
   aattaaaaat gaaatttaaa atttatttta gtttaaaata gtgttaaaag taaaattttt 164100
   aggaataaat ttaataaatg gtatgtaaga tttttttttt gaaaattata aaataatgta 164160
   attttgtaga gatttttttt tagtagagaa ataaaagaaa tataaaagga tttttaaaac 164220
   ggagatatat tatttatata aaagatttaa tattgttaaa atgttaattt ttttttaatt 164280
   gatttataga tttaatataa ttttaattaa aatttatatt gtatttttgt agatattaat 164340
   aagttatttt ttaaatttat aaggaaattt aaaggattta gaatatttaa agtaatattt 164400
   tagatatttg agagatttta tattaagatt tattgtaaag ttatattaat taagatgtgg 164460
   tattagtaaa ggaatggata aaatattaag aaaatataat agtgttttaa tatagggttt 164520
   atatgtatat agtatatata ttaatatagg attatatata tagttaatat aggatttata 164580
   tgtatatagt atatatatta atataggatt atatatatag ttaatatagg atttatatgt 164640
   atatatgtat atatattaat ataggattat atatatagtt aatacggggt ttatatgtat 164700
   atatgtatat atattaatat aggattatat atatatatat agttaatata gggtttatat 164760
   gtatatatgt atatatatta atataggatt atatatatag ttaatatagg gtttatatgt 164820
   atatatgtat atatattaat ataggattat atatatagtt aatatagggt ttatatgtat 164880
   atatgtatat atattaatat aggattatat atatagttaa tatagggttt atatgtatat 164940
   atgtatatat attaatatag gattatatat atagttaata tggggtttat atgtatatat 165000
   gtatatatat taatatagga ttatatatat agttaatata gggtttatat gtatatatgt 165060
   atatatatta atataggatt atatatatag ttaatatagg gtttatatgt atatatgtat 165120
   atatattaat ataagattat atatatagtt aatatggggt ttatatgtat atatgtatat 165180
   atattaatat aggattatat atatagttaa tacggggttt atatgtatat atgtatatat 165240
   attaatatag gattatatat atagttaata tggggtttat atgtatatat gtatatatat 165300
   taatatagga ttatatatat agttaatacg ggtttatatt gtatatatgt atatatatta 165360
   atataggatt atatatatag ttaatatagg gtttatatgt ataaatgtat atatattaat 165420
   ataggattat atatatagtt aatatagggt ttatatgtat atatgtatat atattaatat 165480
   aggattatat atatagttaa tatagggttg atatgtatat attaatatag gattatatat 165540
   atagttaata tagggttaat atgtatatac gtatatatat taatatagga ttatatatat 165600
   agttaatata gggtttatat gtatatacgt atatatatta atatagggtt atatatatag 165660
   ttaatgattt ttagtaaagg tgttaggtaa tttaatagag taaggtatgt ttttttaata 165720
   aacggtgtta gaattggata tgagtataga aaaataagaa ttaattttga ttttaaatta 165780
   aatataaaaa ttgatttaaa atggattata taatattttg acggttataa taggttaaaa 165840
   aatgtaaaaa tttaaaaaat taaaaaataa aaggaattat agaatatata attataaaat 165900
   ttttagaaaa taagagaatt ttttttgatt ttagtgtagg taaagatttt ttttataggt 165960
   tataaaaatt ataaatcgaa attggaaaaa gttgataaat tggattttat gaaaataaaa 166020
   tatttttgtt tattaaataa tattattaag aaaattaaaa ggaaaggagt aatgtggaaa 166080
   aaaaaattta tgatatatat atttgataaa ggatttatat ttagaatata taaaaaatat 166140
   atgaaataat attttttttt tttttttttt ttgagataga gttttgtttt gttatttagg 166200
   ttggagtgta gtggtgtaat ttcggtttat tgtaagtttc gttttttagg tttatattat 166260
   ttttttgttt cggtttttcg attatttggg attataggcg ttcgttatta cgttcggtta 166320
   attttttgta tttttagtag agatggggtt ttatcgtgtt agttaggatg gttttgattt 166380
   tttgatttcg tgatttgttc gtttcggttt tttaaagtgt tgggattata ggcgtgagtt 166440
   attgtgttcg gttaattttt tttttaaatg agatttatgg ggaaatagtt aaatgaagaa 166500
   ataataaagt agtaataaaa taaaatttaa gttttatttt attgtgtagt tgtaggataa 166560
   agagtagaag tttgtagttg gggaataagg tgtttatttt tttaagtatt ataaaatgaa 166620
   ggttttagaa attattcgat tttgaaataa tataatttta aaaagaagta agacgtggat 166680
   agataatttt ataaaggaaa atacgtaaat gattaataag gattttggaa aattgtgcga 166740
   ttttataagt tattaagaaa atataaaata aaagttttaa aaaaaaatga agtttggaat 166800
   aattgaataa atatataaaa cgataataaa atgaattttg attttttatt ttatattata 166860
   tgtaaaagtt aatttataaa agttatataa ttttaagttt tttaaaggaa aatataaata 166920
   tttttgtaat ttggtagggt aggtaaaggt tttttataaa gaatatagag agagaattat 166980
   aaagaaagga gtttgttaaa ttagatttta taaaaattaa agtgatatat ttttgtttat 167040
   tggaaaatat tattaagaaa atgaataggt cgggtatggt ggtttatgtt tgtaaaatcg 167100
   gtttttgggg aggtagaggt aggaggatag tttgagttta ggagttttag atttgtttgg 167160
   gtaatatagc gagattttgt ttttgaaaaa ggaaagaaaa tgaataggta agttatagat 167220
   tggaataaaa tatttgtaaa atacgtattt gataaaagat tagtatttag aagaaaaagt 167280
   taagtaattt aattttaaaa tgagtaatag atttgaaaag atattttata aaggaaaaat 167340
   atatgaagag ttaattatga aaatgtgttt aatcgtatta gttattaggt taaagtaaat 167400
   taaagttata atgagatgat attattatac gtttattaga atgattaaga ttttatagag 167460
   tgatagtatt aaatgttggt taggatgtgg agtaattaga attcgtattt attgttggtg 167520
   ggagtgtgaa atggaagggt tattttggga aaagatttgg tagtttttta taaaattaaa 167580
   tgtatattta ttttatattt attttaggtg tttttataga ataaatgaac gtttatatgt 167640
   atagaaaaat ttgtataaga agaattagcg taataggttt atttataaga gtttttagat 167700
   ggaaatagtt aggggttttt taataagaga atgattaaaa atttatagaa tgtttatata 167760
   gtggacgtta tttagtaata aaatagaata aatcgttggt atttgtagta atatgggtag 167820
   ttttaagaaa tatttaaatt ggtaaaatta tttcgtggtg gaaaaaaagt ataattgtgg 167880
   ttgttttggg gaaggtgggg attgtgatgt atttggaggg gtatgaggaa tttattgggg 167940
   tgatagtgat gttttatgtt ttggttaggt tgggatatac gtggatattt ttgttaaaat 168000
   ttaaagaacg gaacgtttta tatttgtgta ttttagggta tatatatttt attttaaaga 168060
   aaaataaaaa taattataga taaatgtcgg attttagtta atgaaatgta aattgaggtg 168120
   ttgataggat gttgtgtgtt gatttttgta gtttatttta agtgtattaa aattagcgtg 168180
   gatgttggtg gttagaggga tttgtggtga agtaaatagt aaaatggttg ttgtggaatt 168240
   taggtggtag gtgtatgggt gtttatttta gaatttttta tggttttttt tttgtggttt 168300
   tgttttgttt tgtttttgag atagggtttt gttttgtcgt ttaggttgga gtgtattggt 168360
   ataattttag tttattgtag ttttaatttt ttaggtttaa gtaatttttt tattttaatt 168420
   tttcgagtag ttgggatttt aagtttgtat tattatgttt gattaatttt ttgtattttt 168480
   tatagagatg ggattttatt atattgttcg ggttggtttt gaatttttgg gtttaagtga 168540
   tttttttatt ttaatttttt aaagtgttgg gattatagtt atgagttatt atatttagtt 168600
   tttttttatt ttttttatgt ttgaaaagtt ttacggtaaa aagttggaaa aatgtaaaaa 168660
   agattgaaat ttattaatta aaaataggaa aaataaaata aaagagatgg taggagatta 168720
   tttttttttt ttttaattta gtttttaatt tttttttttt tcgtttagtt aggataggaa 168780
   ttttaagggt tcgggttttt cgtgtttgtg cggggaagga ggtattttcg tgtttgtgcg 168840
   ggggttggtt atttgttttt cggtgtattt tttgtatttt attttttttc gttcgtagtg 168900
   agtagttata gttggcggaa attatattta ggtagttttt gtttataaat ttagtgaaaa 168960
   tgtaaatttc gtaggagttg gattttcgag agtttgcggc gttatttgtt ggaggaaagg 169020
   ttttttgtat tcgagttttt tttttttttt tcgttgttat tcgttttttt gtttaggttg 169080
   ggcgggtttt ttttttgtaa tttcggggta ttggtgtacg ggaaatgttt agtgttttat 169140
   aatttttttt tatttatatt ttaatattta gtttttagtg gatatcgttt tcgttcgata 169200
   aatatattat ataggtatag tttaagatta tttattttag gaggagtata agttttagtg 169260
   tttattattt tagttttttt tttttttttt ttgacgtagt gatataggtt taggatattg 169320
   aagatttttg attttataaa tatgttgaag ttaattattt tgaagttgta gtttttatta 169380
   ttattattat tttttatttt ttgtaaagat aaagttttgt tatgttgttt agtttggttt 169440
   ggaatttttg gatttaattg atttttttgt ttcggttttt taaagttttg ggattgtagg 169500
   tgtgagttat tgtatttagt ttgaagtcgt agttttttaa ttattgttaa tagtaatatt 169560
   tatttttttg tgagtagtag ggtttgttgg tatggttgat agttttgtat tattgtatcg 169620
   aatttgttgt ttgggatttt aggtgagttt cgatttttag ttggagggat aagttgtgtg 169680
   gggttgggta gggtgaattg cgggagatta agtttagatt gagggttata agttatatga 169740
   gtattggggt taggtaggga tagtgaagga gatagtgtaa ttttgaggaa agataacgtt 169800
   atagaatgga gaggtttagg ttcggggttt ttttttgttg tcggataggt atgtttgtgt 169860
   ttttttttta gttttcgtga tggttaagtt atgtgttttt tgagaggtaa gcgaggtttt 169920
   tgatagtagg ttttaggttt taattaaatt gtttgttttg tagggtgagt tgtataggtg 169980
   tttttatttt cgattttttt gaatttttgt tgtttttaag ttttatttat tttttttagg 170040
   tgaatagaaa tattgtttag gggagaacgg aggaagaatt taggaaaggt tgttcgagag 170100
   tttggttgag tttacgttat cgttaagagt agcgttaaaa tttaggagtt ggtgagatta 170160
   tggtttaggg tttatattaa tatgaggggg tgttagtttt atgattaatg agtttttgtt 170220
   ttattatttt ttgttattta tattttgagg gttatagtta gtacgtgaat tggagttggt 170280
   tttcggtatt ttttttttta tattagtttt ataatgtgag gtgggggttc gtttttagag 170340
   gtattcggag tagtaggtgt tttaagaggg ggaagttttt tcgaagtttt tttttattta 170400
   cggtatagga cgtagagggg atagagtatt aggaatattt agcgattata ggggtttgtt 170460
   tcgaaggaga ggaggtagga ggtagggaga gtggttggga ggttagtttt ggagtgttta 170520
   ttttttattt cgagggagag gaaggaagta gaaggtgagt atagggagtt ttagttgggg 170580
   atggagcggg ggttgtagtt cgattgtgtt gtttggagcg ggggtatgga ttgtagtttt 170640
   tatttatttg tttggttagt tcgaagttat tttaagtttg gagattttat ttgttttgtt 170700
   tatttttttt agggatggtg tttaaatttc gaagttttgg ttggtgttgt aaatatggga 170760
   gggaggtttt gttttatttt agttttaaga gttagttaag aatgttttgg atatttgaat 170820
   tttgtttcga gttagaggtt gggaagaaat ttgaaggatg gtgggacggt ggatagtagt 170880
   ttttaggtat taggtattag ggtttttgtt tggggatggg ggtattagta ggtgttaagt 170940
   gtattatatt agggtttatt atgtttttgt ttagagattt agtattttac gaaataggaa 171000
   ggaacgaggg atgagcgtgt tttttttttt aatattttag tttttgtttt gaggttgtta 171060
   ggtatttaat ttttttagga tttaaattat tttggtttta tttacggtta agtgtattaa 171120
   attatagtta ggattattgg gtttagatat taaaagcgtt ttttttaaag tcggtagtta 171180
   ttagggtagt atagggtagt gtaaggttgg gtagggtcgg ttatttgtta gtatttagtg 171240
   gtttttgttg gatgttgacg ggggtagtta ggtaggtggg tttatttagt aattaaagag 171300
   attttttttt tttaattttt tttttaaaga gattttgatt tttttttttt agttgtaggg 171360
   tttgtagtag tttttttttt agtaggtttt tttgtttgtt tttggttatt tttagtagtt 171420
   atagggtatt gaggttttat tatagtgaga ataaaattaa gtttttatta gggtttttaa 171480
   ggttcggtac gatttggttt tgtttgtcgt attggtgtta ttatggttcg tttttgaaaa 171540
   tatattttag ttatttttat tttttttagg ttttagtttc gggtttttat atttgttgtt 171600
   ttttttgtta ggttgttttt tttttggatt ttggtttggt tgggtttttt tggttattat 171660
   agttttagtt tagtgttatt atttagaaag ggtttttatt aattttttcg ttatatatat 171720
   agatatgtat ttttagtaat ttgtttttag tttttttata gtttttattt aaaattattt 171780
   atggatttgt ttatttgtgg attgtttttt agttttaatt gttaagtaat aagcgattag 171840
   cgattgtgtt tttgttgttt tttattatag gttaagtatt taagagaggg gttgtatata 171900
   gtaggtttta ataaatattt attagacggg ttaggtacgg tggtttatgt ttgtaatttt 171960
   agtattttgg gaggttaagt tgggtggatt atttgaggtt aggagatcgg gattagtttg 172020
   attaatatgg tgaaatttta tttttattaa aaatataaaa attaattagg tatggtggcg 172080
   gacgtttgtt attttagtta tttgggaggt tgaggtagga gaatcgtttg aatttgggag 172140
   gcggaggttg tagtgaggcg agatggcgtt attgtatttt agtttgggtg acggagtgag 172200
   atttcgtatt aaataataat aataaaatat ttgttagaag aattaaagta gtttgtttgt 172260
   gtttaatagg aatttaagag tataagatga atttaattaa aggatatata tagtaggtat 172320
   tttatagttg atatttgttg gtggagggag attaggaaag tggggagagg tagtgtttgg 172380
   gtatggggat attttgggta tggggacgtt ttggatatag ggatatattg ggtacgggga 172440
   tattttgggt ataggacgtt ttgggaacgg ggatattttg ggtacgggga tattttgggt 172500
   atggggatgt tttggatata gggatatttt gggtacgggg aaattttggg tatggggatg 172560
   ttttggatat agggatatat taggtacggg gatattttgg gtatggggat gttttggata 172620
   tagggatata ttgggtacgg ggaaattttg agtatcggga tgttttaggt acggggacgt 172680
   tttttgttat ttgaaatttc gtagttttat tatattcgtt tgttttagta aatcggttta 172740
   aagagtattt ttaagttttt atggtaaagt tttagagtta gtgttttttc gttgatttcg 172800
   agggtgtgtt gtttatttag tgtttaagtt agagtttttt tatttagaaa aaaaaagagg 172860
   agggtggttt tacggatgga ggtatgaggg cgtttatagg tagagggagt tttcgggttg 172920
   agatttgcgt tattttttgt tttaatttta aggcgtagtt tggagtttaa attagttttt 172980
   aaacgtgggt cgttggttaa gaggaatttt tttatttacg attttttttt atatttagtt 173040
   tttattgttt agagtatgtt agttttgttt gttttggttt ttaggatttt tttagggatt 173100
   tttaggtttt aagttagggt gggtttgaat taaggtagtt ttagaattta tgttaatgtt 173160
   tttgttagtg ggagattttg gggagtatcg tgcgggatgt atggggttag gcgggggtat 173220
   atagcgagcg gttttagaag ttttggtcgt tcggagtggg ggggtgttta gagtaagaga 173280
   cgggttagga agttggagtg agtaaggagg ttttagtttt tggtgggagt aggtcgggta 173340
   aaaataaatt aatttgaggg tagtagttag cgagattatt ataggagggg tggtggggtc 173400
   ggaggggttt taggagttta ggagagggag gtattgttag aggtaaggtc ggtagatgag 173460
   agttagtgtt ggtaggatta atttgttata gtagttatgg tttatttttt tttttttttt 173520
   ttatatatgt ttttggggtt tatggaattt attagtttat tataaaggat attataaagg 173580
   atatagggga agagatgtgt agggtaaggt atgggggagg gacgtagagt ttttatgtgt 173640
   tttttaggcg ttttattttt taggagtttt tatatgttta tttatttaga agttttcgga 173700
   attttatttt tttgagtgtt tacggaggtt ttattacgaa ggtatgattg atttaattat 173760
   tggtcgttga tgatttgttt aatttttagt tttttttttt tatttggagg ttgcggggtg 173820
   gtgttgaatt ttgtgatttt atttggtttt ttttgggaat tattttgaag ttgttagttg 173880
   gtttgttaat tatgagtatt taaaaagata ttattttgga gattttaagg attttaggag 173940
   ttgtgtgtta ggaaatggag atgaaaatta agtgtatatt ttataatatg atagttagtt 174000
   ttttttaatt ttagttttat tttttgtcgt gttgtcggtt ttagtttttc gcgttttagg 174060
   gtttttgaat atattgtcgg ttgtttgtag cgatcgtttt tgtttgttgg gtttgagttt 174120
   ggtttatttt ttttattttt ttgcgtttag ttcgaaggtg ttttttcgag gtagtttttt 174180
   tgatttggtt ttacgaatcg taggttttgt tttgttattg gttttttttg ttatatttgt 174240
   tattttttgt gttttacgtt tattgatgtg gttttgtatt tgtgtagttt tatgagggta 174300
   aggattgtgt ttggttttgt tttttacggg attttagcgt taagtttata gggagtattt 174360
   aatgttgaat gaatgaatga gtgatcggta agggaggttt attttgttat gttgggggtt 174420
   aaaggttgtt gggcggatta tatgggtgag gttgggtggg gagggaagta gagatgattt 174480
   tttggaagaa ggggtaggag gcggggaggt gggtgtgggg tgttgtataa ggatggtaag 174540
   tgtttttagg ggtgataagt taatttagag tattaggtag tagaaaaagg ttgatatttt 174600
   tacgttttat ttttatttag ggagaggtta ggttttcggg aaagttagtt tttcgttgtt 174660
   aggagagttt taagggaagt tttattagtt gttgagttgg ttatagtgga aatttatgtg 174720
   gtgggtggga ggggtttgag gtataggggt ttgggtggtg gttttaggat ggagatttgt 174780
   gtttagatta tgaggggtgg ttttagggat gattattttt aaataggaag tagataaagg 174840
   tatagatggt agaagcggag gtagagaatg ggttgtaggg gtttttttag agggagagtc 174900
   gagtaagggg ataggttgga aatatttagt ttttatggtt tgaagtatag atagttggtt 174960
   ttatacgtag gtcgggtagt tatattattt agaataaggg gtatgttcgt agggtaggtt 175020
   ttaggacgtt tcgggttggt tttgttatta gtttagaaag acggtttagg ttttttcggt 175080
   agggagtacg tttagttagg ttttgggtag gagttgagat tttttggttg gtttgagagg 175140
   ggatgatcga gtgtaggttt agttcgggat ttttttattt tttttcgtta tttagcggaa 175200
   ttagttgggc gtgagaaaag aagagaagtg aattaagagt ttagagtatg aggttggatt 175260
   agtttttata ttatatgtaa aaattaattt aatttgtatt aaagatttaa atttaagagt 175320
   taaaattata aaatttttag aagaaaatat aggggaaaag ttttttgacg ttgtatttgg 175380
   taatgatttt ttggatatgg cgttaaaagt ataggtaata gaagaataaa tagataaagt 175440
   ggaacgttat agaagtttaa aatatttgta tattagtttg ggtgcggtga tttacgtttg 175500
   taatttcggt attttgggag gtcgagacgg gtggattatt tgaggttagg agttcgagat 175560
   taatttagtt aatatagtga aatttcgttt ttattaaaaa tataaaaatt agttgggtgt 175620
   ggtggcgggt gtttgtaatt ttaattattt aggaggttga ggtaggaaaa tcgttggaat 175680
   tagggaggcg gaggttgtag tgagttaaga ttatgttatt gtattttagt ttgggtaata 175740
   gagttagatt ttattttaaa aaaaaaaaaa aaaattgtat attaaagggt attaataacg 175800
   tggggagaag gtaatttatg gaatggtagt aaattatagg ttagatatgg gattagtatt 175860
   tggatattaa tttatagatt ttttaatatt tagtaataat aattatttaa attaaatata 175920
   ggtaaaggat ttggatagat atttttgtaa gaatatgtgt aaatgagtaa taaatacgaa 175980
   aagatgttta atattattaa ttattgggga attgtaaatt aaaattataa ggagagattt 176040
   tttataatta tgagttttgg gtaggtagga agacgtttgg tggggagttg tcggttttat 176100
   gttgttttta gaatttttgt aggggttaag ttcgatttcg atattttatt aggagtatag 176160
   aaacgagtgg aaaaggttaa aggttatttt aaagggttag aggggtttag taaggagttt 176220
   gggatatgga attatgttgt gtatttagat atacggggat aataggggga tgaaaggtgg 176280
   tttttaaaat ttatatttat tttagagttt atgaatatgg tttatttgga aaaagggttt 176340
   ttgtagacgt tatgaagtta aggattttga gatgggatta ttttgggtta tttagtgggt 176400
   tttaaattcg agaaagattg ttcgtatgag agatagatag ggggagattt agggtataga 176460
   aggaggtttc gtgataacgg aggtagaggt cgagtggtgt ggttacgagt taaggaacgt 176520
   taagggtttc gggagtcgtt agaagttgtt agaggcgaga agtaggtttt ttttcggagt 176580
   tttcggaaag aattaggttt gttgatgtcg tgatttcgga tttttggttt ttagaacggc 176640
   gagagaagaa atgtttggtg ttttgagtta tttagtttgt ggtatttggt gatggtagtt 176700
   ttaggaaatt attataggga ttagagtttt tacggggttg ggtaggaagc gggtgtagag 176760
   cgcgttaggt taggtttttt atttcggaga tggaaaagta gagttggaga ggttgagatc 176820
   ggataggtat cgagcgttta ggggtaagaa tttttagggg atggtagagg ttatcgggga 176880
   tagtatagcg agggatagga gggaggggtg agggttgagg tatcgttttt taggaggttt 176940
   cggtttttcg tttattgatg atttaggaat ttcgtaagag gagttgggtt ttattttgtg 177000
   tcgtattgta ttagatggat tttggtgggg atagacgttt agcgatagcg tggttaagtt 177060
   ttttttagga tttcgggttt gtatttatga ttatgtatag acggttattt tttttgtttg 177120
   tagttttttt tcgtggtttg tttttagttt tttagtttaa tagagttttt tagtggaaag 177180
   tttttttttt ggagattttt tttttgttga aatataagtt tttggaataa gataaaattg 177240
   tttaatttgt aaaagttata aagtcgtttt agaataaaaa aaaagtttcg atttttggtt 177300
   ggaaggagtg gttttttatg ttttagcggt tagttattat tttatgattt atgtcggttt 177360
   tgttgataaa ttttttagta tttttttagg ggaaggttat agtttttagg gagggcggga 177420
   gagtatttag gttttcgttt ataaattaga tcggagttag atattgtcga gggagggata 177480
   agagggtagg ttttggggtt tttaagtttt ttttggggtt ggtagattat cgtggattta 177540
   ttagggtagg attatttttt tttataaatt ttttttgggt agttgtaggg tagtttgagt 177600
   tggatacgtt ttatacgtta gtttagtggg gtatgaggta tttatagtta cgtttggaag 177660
   ttagcgggtc ggtatttgtc gggtaggttt ttgttatata ggtatagcgt tttttttatg 177720
   gttttttttt aggttagagt atgtttgggg attagagtag ttttcgtagt ggtttgtagt 177780
   ttgataacgc ggcgtgagtt atgttttgta gagaagaaga gttttttagt gagggaagag 177840
   gcgtttataa aatttaggat acgcgtagaa attcgtagga ggtgggggcg ttgtttaggg 177900
   gtcgtgtcgt ttttattttt tattttgtag atgacggaat tgcggtttag agttgttaag 177960
   tggtttgttt aaggttattt agtagggata ggaaggaagt tattttttta gggattgtgc 178020
   gggagtttag tgataattaa agattagcgt taaagtttgt ttaggacgag aattttatta 178080
   agggaaatag tttcgggatt gtcgtgttta ttcggaaggt agttagttta taattgagtt 178140
   tttaaaaatg tacgtttttt tatatgaaaa tatattcgaa tgggattttg tagaaagtat 178200
   tttttattaa ggtatagttc gaggggaggt tttcgtttta tttttggttg tgttgttttt 178260
   agttaagggt agtagggatt agagtggttt ttggggtata gtattggttg tttttaaaga 178320
   gtgttttgtt tagtttatga aaagttcgtg ttttgtgagt ttgggagggt gttattattt 178380
   ttagtttttt agggagtatt atttttaaat ttacgttttt ttgtttagtt tttagatttt 178440
   ttgttggtta tttatttggg tttcggttag attatttata aagtgaaggt cgaattcgag 178500
   gtttttcgag gtttttgtta gttaggcggg tggagtgacg tttttttggg gttggtggtt 178560
   taggaggtag gggatttatt gtggtttgtt taggttgttt ggtggggtgg ttatttcgag 178620
   gacggtttta ttgtgtaagt ttgacgaagg ttttttttag cgttttttgt tttgcggttt 178680
   ttgttgggcg gagagtagga cgttgggttt tatatttatt cgttttagcg ggtttgagtt 178740
   ttcgagttta aatagataag atgtagtttt attgttattt aagttttttg tcgttttttt 178800
   tggttttttt ttgaggttcg tttgttgtgt tcgattgggt gtgtttagcg gtataatata 178860
   tattcgtttt attagtttta acgtgataat taagatgtgg ttttgaggtc gggcgtcgtg 178920
   gtttacgttt gtaattttag tattttggga ggttgaggcg ggcggattac gagattagga 178980
   gatcgagatt attttggttg atacggtgaa attttatttt tattaaaaat ataaaaaaat 179040
   atggcgggcg tttatagttt tagttgtttg ggaggttgag gtaggagaat ggtatgaatt 179100
   agggaggcgg agtttgtagt gagttgagat cgcgttattg tattttagtt tgggtaatag 179160
   agttagattt cgttttaaag aaaaaaaaaa aaaaagatgt ggttttggga gtagtggtgg 179220
   aggggattat acgttttaga gaggagttat cgagatgggt tttttttttt taggtttttt 179280
   aggatttttt taatatatat atagtggtaa ttaatagatt agggtttaag gtacgtaggg 179340
   gttgatattt ttagcgattt ttcgttttat ggttcgtagg ttattttaga atatttaaag 179400
   tttggagttt ggtttagtta ttttatgttt tttagcgttg ggtttagagg gcggtatgag 179460
   cggtagaaat tgttattatt gttaattatc gatgacggcg ttggttacgt gagatggtag 179520
   ttacggtatc gtatgtgagt gaagggatta aggaggttgg gtttgggagg gagggtttgg 179580
   aaagttgggg tgaattaaag gtaggggagt tttttttttt tttatttgtt tgtttacgtt 179640
   ttgatgggtt tgtgtttgcg gtaagggggt tgttagcgtg aatggatata cgtatatata 179700
   tgtatatata cgtgtatata tacgttaggt ttttttgagc gtcgggagtt ttttttttta 179760
   taattaattg ttaggaaggt atagaaatgt tattttaggt aggagatcga ggtagttttg 179820
   aagatagatt ttggttggaa gtaaaaaata aattatatag tggataaatt aattattagt 179880
   taggtttaag cgtcgtcgtt ttagattcga gttagggggg taggtttttt atattttttt 179940
   aaggtttcgg cgttaatagt tttagtattg atagtggtta tgatatgtaa gaatggtata 180000
   ttggaaaatg aaagggaagt agttaggaga gaggagagtc gagtttaaat aaaagttaga 180060
   tttaggagag tttagttttt gagacggagt ttattttacg agtttggaat cgttaaaaag 180120
   aaaagaaata tttttgtgaa aaatgagtaa aagcgttttt tgtatgttta tttttttagg 180180
   tataaataaa gtttcgagtt tagggtagtg gggggcggtc gatggggagg gggttgtttg 180240
   cgtatttgag attcggtttt tttttatttt tacggatata tttagtagcg gcgggagttt 180300
   cggcgtttcg tttagaggcg gttttttttt ttttattttc gtcgtttttt tttttttagt 180360
   tttttttttt tttttttttt tttttaaaaa tattttttgt ttggtttaat ttttttaatt 180420
   tgttgtaatt acgaatgtat tcgaattatt atatttttat gtgttgatta tatgtttgtg 180480
   ttttaaattg aagtagtatt gtttttttaa aacgggagga aaaagggagt aaaggagaga 180540
   aatttaaaaa taggaggtag gagagagggt tcgaagtggg tttcgaggag ttcggtagat 180600
   tatcgggttt gtttgcgaga gttttagagt ttcggtcgtt cggtttggtc ggtggttgta 180660
   gaggtttcga gtgcggtttt cggtagtttt ttttttttat tttttttagt tttcgtcgtt 180720
   tcgaggtttg ttgttttttt ttaatattta tttggagatg tttaatttag atttttggaa 180780
   attaatttat agagttaaat ttttgacgat ttttaaagtt ttttattttt tttgtttcgc 180840
   gatgttacga tttttttggg tttaagggtt taaaaagttt agtttttgcg tgtttgtttc 180900
   gttttttttt gttttgggtt ggttttagtt tttttttatt ttgtatttta acgtggttgg 180960
   gtaggaggaa cggtcgtgtt ttaggttagc gtgttgagaa aaggtttcgg agtatttgtt 181020
   taggaggaat tttgagaagt tatttttttt cgttttatta gtttgtagtg atagaggtag 181080
   ttgtcgtttt cgtttaggta gagagggcga acgggacggg gattttttgg aagattttta 181140
   ttatatacgt atgagttagt gagtttttag tcgttttttg ggagggagcg agtttttttt 181200
   ttcgggaatt cggggtttta ttgggggatt agtgttgttt tttattgttt attcggggtt 181260
   tttagaggtt gtttatttat ttggggggtt tttattagta tatggttagg gtttatgttg 181320
   aggttaaggg tttagtagag taggtggggg tttataatgt gggtttttgg ggtgggaatt 181380
   aggggtattt tttttgtttt taagttttat tttaagggtt tatttaaatt cgagatacgg 181440
   aggttggttt tgttggttta agtttgttta aagagaatgt gtgtttgtgt taaggttgag 181500
   aatttaggta atttttttga aaattttaga ttgttagttt ttgttgagaa agggaggacg 181560
   cgtggtcgta ttacggaagt ttttttgttt ggtcgtatag ggttggtata agatagtttt 181620
   gttttttaga taaaatattt tttagtttgt gttgtttttt aaggttttta ataaatttat 181680
   aatatttgta tttttttatt tcgatatatt tcgtaggttt ttgagtttat aatttttgtt 181740
   ttatagagtt aggtgagtta tttgtgatta ggtgaggttt atgtatagtt ttcggttaga 181800
   tgatagagtt aaaggttttg aagtgttatt ttaggttata gtttcgttcg ttttttgttt 181860
   gggataggat tttaaatgta atttttttgg tagaaaggat atttttttgg ggggtgggga 181920
   ggattatttt tagttttttg ggagatatta agtaattatt tttttttaaa gagtttggag 181980
   gatagtttta taggtagtta gatagtgagg gaggtgggga tagtatttta gtcgaggtgg 182040
   gaggttgggg aggaagggag ggattatagt tacgagagaa ggttgaataa tggttttagg 182100
   gatagtatgt tttaattttt agatttgtaa atgggatttt gtttggaata agagtttttg 182160
   tatttgtgat ttagttgtag attttgttgt ggcgagagta ttttggatgg tttaggtggt 182220
   ttttaaaggt agtttcggtg tttatataag acgggggtag agggagattt gatatagata 182280
   gggatggtta agtggagatg gggttcggag agatttgtag atgttggtgt tgaaggtaag 182340
   ggttatgtag ttataagtta agggatattt atagttatta gaggtttaaa ggggtaggcg 182400
   atgaaggtag tggtttttaa tttttttggt atttgggatc ggttttaggg aagataattt 182460
   ttttatagat tagggagtgg ggagatggtt ttgagatgat ttaagtgtat tatatttatt 182520
   attttatttt attttatttt attgagatag agtttcgttt tgttatttag gttggagtat 182580
   agtggtatta ttttagttta ttgtaatttt cgttatttgg gtttaagtaa tttttttgtt 182640
   ttagtttttt aagaagttgg gattatagac gtttgttatc gtgttcggtt aatttttgta 182700
   tttttagtag aaacggggtt ttattatatt ggttaggttg attttgaatt tttgatttcg 182760
   tgattcgtta gttttggttt tttaaagtgt tgggattata ggtataagtt attgtgtttg 182820
   gtttttattt tttatttttt gagatagagt tttattttgg gattataggt gtgagttatt 182880
   gtattcggtt ataagtacgt tgtattgatt gtgtatttta tttttattat tattataata 182940
   tataatgaga taattatata atttattata atgtagaatt agttggagtt ttgagtttat 183000
   ttttttgtaa ttagacggtt ttatttggtg gtgatgggag acggtgatag attattaggt 183060
   attagatttt tataagaagt acgtaattta gattttttat atgtgtcgtt tataatacgg 183120
   tttatttttt tataagaatt taatgttatg gttgattaga taggaggtag agtttgggta 183180
   gtaatgcgag taatggggag tgattgtaaa tatagatgaa gtttcgttcg tttgttagtt 183240
   atttattttt tgttgtgtag gttagttttt aataggatat acggggttgg gaatttttgt 183300
   tttagagttt ttagagggtt atgtttagtg gatattttga ttttagtttg gtgattttga 183360
   tttcggatgt gtggtttgta gagttgtgag aggatggttg ggcgttgttg tgatttagtt 183420
   agtttgtggt tattcgttat agtagttgta ggaaattgat gtagcgttat agacggagtt 183480
   agggtttagt ttattggagt tgttttttgt tattttattt ttttttaaaa tattgtgtta 183540
   agttaattat taataattat tgatttcgat gggtaggaat cgtagaggtt ttttggtttg 183600
   tttttttaat ttttttttta attttgaaat aatatttttt gttggttttt ttgattataa 183660
   atgtaatatt tttttttgta ttgagtttag aaataagaga aaaagtaaaa agtagaaatt 183720
   ttagttgggt aaggtggtac gtgtttgtaa ttttagtatt ttgggaggtt gcggcggaag 183780
   gattgtttga agttaggagt tcgaggttgt tgtgagttat aattgtatta ttgtattttg 183840
   gttgggtaaa aagagtgaga ttttgtttta aattaaaaaa aaaaaaaaag aagaggaaga 183900
   aaatttatta attttattcg agatttttta tacgaatttt tgttgtgggg tagggaagtt 183960
   taggaaggtt ttacgaggtt ttgatttagt ttggttggcg gtgggaattt gggaatagtt 184020
   tcgagtgagg ggtcgggtta tgattgagtt tatttattta gttatttttt gtacgttgtg 184080
   tatattatgg gtttgacggt tagggttgga gtatttgttg agtatagttt tgatttttag 184140
   ttttatttag tatggtttgg tttgaggttt attttttttt cgtgtttgaa aatttaaata 184200
   ttttggttta ggcgggggat tatttgaaat ggaaaatttg gtagtatcga ttggttttga 184260
   agaggtagga gttagttatg agtcggttgg gaggcgagtg tagaggtggt gaggtaaggt 184320
   tagtagaggg tgggggaggc gagggttaat tttttatcgc gtatggtgta agtattaagg 184380
   gggtatttta ttaggttagt gtagatttag gtttttgcgg tggatttttc gcggtttttt 184440
   tttttagagg aatattgaag aggtttttgt ggttttgttt tagatttaga ggtatatttg 184500
   tatattaaac gtattgagta attttatagt gagggagtcg ggtttggttt aatttatttt 184560
   tttttaaatt ggattaaagg ttgttgtttt aggaaggtag ggtttttagg tttacggttt 184620
   tgggaaattt tggttgtgtt agtttatgag tttagttttt agttatttta gtttgtttat 184680
   attttggagt gggtattgtt atttaaaagt tttttttaga tagtttttag ggcggtattt 184740
   ttgtttttta ttttttatag atttttggtt atttttttgc gatattagta ttattggttt 184800
   atagtttttt ttaattgtaa agtggggttt agcggtatag gttgggtttg gtagggcggg 184860
   aaggaagagc gaaggttatt ttgtttttaa tagtattggt cgggttttgg aaaggtcgga 184920
   atttatagat aggaaggaag tgttatattt atttaatttg ttttgcgtta gttagggggt 184980
   attttgtaga aagaagttag gtggtcgtgg tcgggtgtag tggtttatgt ttgtaatttt 185040
   agtattttgg gaggtcgagg taggtggatt ataaggttag gggttttaga ttagtttgat 185100
   taatatggtg aaatttcgtt tttattaaaa atataaaaat tagttgggta tggtggcgta 185160
   tgtttgtaat tttagttatt tagaaggttg aggtaggaga attgtttgaa tttgggaggt 185220
   ggaggttgta gtgagtttag attacgttat tgtattttag tttgggcgat agcgcgagat 185280
   tttattttaa aaaaaaaaaa aagaagttag gtggttattt tttagttttt ggtttggttt 185340
   aggaattgtt ttttttttaa gatgttatta aatgtttttt ttattaggaa gttttttttg 185400
   gttgtttttt ttatttttgt ttttgtttta gaatacggtt cggtttttac ggataatttt 185460
   gatattagag tttaatagag attttttttt ttatggaaat ttatatttta aattgaatat 185520
   gtgtgtagga taagtttttg tttttatgtg aaataaaagt gaaaggtgcg gattatttgt 185580
   ttttttttat ttttttttat aataatttgg gttttatagg ggtttagtag gagttaggtt 185640
   tagtttagtc gttttaggtg tattattttg ggtattagcg tttttgtttt tttttacgaa 185700
   gtatagtttt attttttggt atttagtggg gaggggatat atattaggta ttttttaggg 185760
   tggattgtgt tttttatatt aggttaggag gttttggggt tggtttagtt atttagaaaa 185820
   gggagtaggc ggtttaggtg atattgatgt gggtagtagg ggttttcggg aaagggtttt 185880
   cgtaggatcg gttttatttt gttttaggag tatatattgg ggaagggtgt tgcgcggagg 185940
   tggagagaac gtatttttgg ttgaaagtat gttttttgat gttttgggtt cggatttgtt 186000
   gttatttttt atgttttttt atttaatata cgttttagta aattttttat gttgatgagc 186060
   gttaagttat agaggacgta taatttttcg ttttaaaaag tgattttttt tgttgggtgt 186120
   ggtggtttat atttgtaatt ttaatatttt gggaggttaa agtgggagga ttatttgagg 186180
   ttaagagttt aagattagtt tgggtaatat agagaaattt tatttttata aaaaaaaaaa 186240
   aaaaaaaaaa aagtttaggt atagtggttt atgtttgtaa ttttagttat ttgggaggtt 186300
   gaggtaggag aattatttga atttgggagg tagagattgt agtgagttaa gattatgtta 186360
   ttgtatttta gtttgggtaa tagagtaaga ttttatttta aaaaaaaaaa aaattttttt 186420
   ttttgaaata gggttttatt ttttttgttt aggttggagg atagtgacgt gattataatt 186480
   tattgtagtt ttagtttttc gggtagttgg aattataggg ttgtattatt acgtttagtt 186540
   aattttttgt atttttagtt aagacggggt ttcgttatgt tgtttaggtt ggtttggagt 186600
   atttgagttt aagtgatttt tttatttcgg ttttaaaagt attgggatta taggtatggg 186660
   ttattatgtt tagtttgaaa atattttttt taagaattag ttagttatgg tggcgtataa 186720
   ttgtagtttt agttatttag gaggttaagg taggaggatt ttttgagttt ggtaggttga 186780
   ggttgtagtg agttataatt gtgttattgt attttagttt gggggataga gaaagatttt 186840
   gtttagaaag aaaaaagaaa aaaaaaagat ttttttattt attttttgtt tatttttaga 186900
   tttaattatt gaagtttgtt tttttgaatt aaaaatgaag gagtttcgtt ttggattttt 186960
   ttttgttgat tttgttattg tatttgttag tagagttagt taagattttt taggttggtt 187020
   ttagtttggt tcgtttatag ttttttaagt aagacggatt tttaggttga ggttagttgt 187080
   attttttttt ggaatttttt tttatttttt attgtgtttt agattttggt tgtttttaaa 187140
   taaatataat ttagttattt ttgtttttgg ggtcgttgta gggtttattg gtgtaaatcg 187200
   tttgggtagg tttgttgttt taggagtttt tttaggttag cgtggaagga aattaagata 187260
   acggggagaa gtagttcggt tgggtttaat tttattttta tatgttcgtg tttagttaat 187320
   tatttgggtt gtagtttgat aggagttgga gttggcgttt tatttttaga gggggttttc 187380
   gattgttttt gtagagcgag ggaggtttgg gggtgtagag gttaagggtt tttgtagagt 187440
   ttgttttagg ttttttgagt ttaagttttt ttgtagttaa cggaagattt tttcgagagc 187500
   gggtgttttt tattttgttt gtttagtagg tgtttagtaa tcgtttattg gataaataat 187560
   gaatttgtgg atttaacgtg aagatgattt aaattagcgt ttttttatag aatttttcgt 187620
   aatgatgtta atgttttgta tttgtcgtag ttaatatggt agttatatgt ggtagtgaac 187680
   gtttgtaatg tggaagggtt attaaggtaa agagtttcgt atttatgtta taatttaaat 187740
   ttaaatagtt ataaatgttt agcgtgtgtt tcgttatata gatttttttt gttatttttt 187800
   tttttttttt agagatattg tattcgttat taatttttta ttcgggaggt aaacggagaa 187860
   atgttatttt ttagttaacg taaaagaagg ttaatatttg tattttaagt tagttttttg 187920
   ggtaattttt ttaagtattt tttattaagg ttagtttagt aatatttttt gattgtttgt 187980
   tacgtattag gaaagtattt cgtcgtggtt ttggttcgag gttatggagg atatagaaat 188040
   aattaaggtt aagttgatag aaggggtgtg ataagatggt ttaaagttat tgagttagtt 188100
   aggaggttta ggagtatgga agttttgttt tttagaggat aagtggtaat ttatttaggg 188160
   ggggttcgag tatttagagt taggagcgta gaagtagggg gttttaggtt ggaaagcggt 188220
   attagggtta ggttgggcgg tagtacgaat ttattttcga ggttttgggg gcggaaatta 188280
   agggttttat gagtaagaag attcggttta ggtttaatgg aaatgatcgg gtggagggat 188340
   tttattattc ggtttttagt atatagggag ggaagtggta atcgtttttt aaatgttggt 188400
   cgtggagggg aaggagttat agtagggtag gggtggggag gatggtattt gttggtttaa 188460
   ggagggatga tggaggttta attaagatat gatcgtgatc ggggtagttt gtttaagggt 188520
   tagtatgtag agggcgagtt gatgagattt tggtagtttg ttgagatttg ggggtgttgg 188580
   ggaggtagag gttaaggtta attttgagat tttttgtttg taaatttggg tgggtggtaa 188640
   ggttgatatt agagaaatag aggaagagtg ggtagttatg ggtgagagag gaagtaatga 188700
   attaggggtg tttgtaagag tttaggggta aggttttcgg gtagttaaga tgttggtggt 188760
   tttttttacg attagttttt tttacgtggt atatttttgt ttgtatggtt gggtatgggg 188820
   tatttgttcg ttcgatatat tttgagtagt tattttgtgt attgaggtaa cgtggtacgg 188880
   tgttttgggg aaatagcgag gaggtattgt ataggtttta tttttttggt attttttgtt 188940
   ttttggggat ggtggtagtt aaataagtga tagttaattg ttaggagagt ttggttttga 189000
   tttattaggt ggagttaggt atttttgtcg gaagaggtat gggaattagt ataggatgaa 189060
   gggaggtagc gagtattagt ttaggttttg tttttatagg gttttttttt tatatttatt 189120
   tgtgtaggcg ttttttgtga gggtagagat atttgtagtg tttgagggag attttttgcg 189180
   ggcggggtcg tttatttata gcggttaggt gggaggtgta ttagtgtttt gtggttgtta 189240
   taatggatta tagttgtggg gtttgaaaaa atggaagcgg gttttttttt atgttggagg 189300
   ttagaggttt aaaattaagg tgttagtaag gttatatttt ttagggaggt tttggggtgg 189360
   ggtggggggg gggttttttt ttgttttttt ttagacgttt tttggtttgg ggttgcgtta 189420
   ttttattatt tgtttttgtt tttacgtggt ttttttcggt gtgtttgtgg tttttttgtt 189480
   ttttttaagg atattagttg ttggatttag ggtttatttt aatttagaat gattttattt 189540
   taagattttt aattatttat atttgtaaag attttatttt taatttttag gttatatttt 189600
   gaggtttttg gtggatatga gtttgtgggg gatacggttt aatttaggat agaagggatg 189660
   atgggggtat tggggaaaag gttatatatt ttagaggtat tggaagatgg aagagtagga 189720
   tttagcgata cgggatgtta ggtagaggag ttggttttaa ggattttagt cggggtttga 189780
   gggttagagg gagggaagtt taggtattat tgagatgaaa tgtttcgtgt atcgtgggaa 189840
   atagggtaaa agttcgttgg acgtttagtt agagaggcgg ttgtttgagg tatagaaaat 189900
   gagcgaatat gggagagttt attagaaggt tattcgggag ggcgtggtgg taatatttag 189960
   aagggttgag aagcgtattt ttatagttta gttaaattta tttggggaaa tatacgtttt 190020
   cggtagtatt agtaatagag aagattttcg gcgatgttat tagtgggtta cgggacgggt 190080
   aaattttgtt tttttatata gaggaatatt cgttagttgt taaaataagt gaattaaggt 190140
   ttcgtggatt tatacggata agtttttacg tcgttacgtt gtgggtatag gtagggtagt 190200
   tattgatttt agtgaggtat taaggatgtg atgttgatga cggtatttaa gaggagatag 190260
   atttaggttg tttagttata cgatgttgat gatcgtatgt aagaggagat taggtcggat 190320
   gcggtggttt atatttataa ttttagtatt ttgggaggtt aaggtaggtg gattatttga 190380
   ggttaggggt atgagattag tttgattaat atggtgaaat tttgtgttta ttaaaaatat 190440
   aaaaaaaatt agttagatat ggtggcgggt gtttgtaatt ttggttattg aggaggttga 190500
   ggtagaagaa tcgtttgaat ttaggaggta gaggttgtag tgagtcgaga tggtgttatt 190560
   gtattttagt ttgggcgata gagcgagatt ttattttaaa aaaaaaaaaa aggagattta 190620
   ggttttttag atatattatt gtttagtata gatgtgtttg ttcggggtta gattttaatt 190680
   tagaggggcg ggtatcgtag taggagtggg gttaggatgg agaaggttat ttagggttgc 190740
   ggttatattt gtaatgtttt atttttaaag cgggatggtg agtatatagg tgttttatta 190800
   ttttattttt tatatttttg ttttgtagtt ttaagaattt tatttttttt ttaaaaggaa 190860
   agaaagtgtg tgagttgatt aaagcgttga ttatggtgtt atttaagttt ttgttagaaa 190920
   tgttattttt ggtttagttt taaatataat ttcgagaaga gttttcgttt ttttggggag 190980
   ggtaagagtt ttagatatag gagattggtt agatgtggat ttatagagaa gttgggttgg 191040
   gaggatggga tggtagatag tcgtttgggg agtgttaaat ggggaggtcg gagatgttgg 191100
   ggcgtttagg aatgaatttt tgtttggatt agggttttag taaaagggag gtagggagaa 191160
   agaaagaagt taagaagatg aggaaagggt gtttcgggtt agagtttata gaggtttata 191220
   gagggaggat ttcgtgtgtt agtaggtagt tttacgtaaa tttagattat tagtttgtag 191280
   ttttgggatt tagtaaatta tgttttaggt ttttaagttg agttgtgatc gtggttgtta 191340
   agtgttgata gtatagaatt agttagtttg agtagaggga agtaatcgtc gggggaggtt 191400
   gtgtttatgt agtttagggt gttaggtttt taagatgtaa tttttataag aattggggga 191460
   ttttttttat ttattttatt agaagttggt taatgagagg gaaggagatg gagaggtaga 191520
   ttagatgcgg agtcggagaa aagtttttta gaaatatagt agcggttggg ttgtttagtg 191580
   gttagtgttt aatatgattg cgatgggcgg agtttttttt aattatagcg ttgaattatg 191640
   tagttgttta ggggttttta gttagagtat tatttttttt tttttatata tagttttatt 191700
   ttgtattgta aggggtttta tatgtattta atgtggatac ggtatttttt ttatttttat 191760
   tttttgtaaa aagttatttt ttttattgtt taggtttggt ggtgtatagt ttacgggtat 191820
   tgtatttatt tttggaaggt agatttagga ggcgtttgtg ttgtttttat agaagtaagt 191880
   ttaggattag aagtaggttt tgggtttaga attttgaggt tttaggtgtt agtgtggttt 191940
   ggaattgttt agttttatag ttattagtta tacgtagata taagtggaaa tttatttaaa 192000
   ataaataaat aattttatgt aagtaaatga gtgaataaag taaacgtaaa tatttatatt 192060
   ttgagtgtgt aatagattta ggcggttaag cggttattat ataggatggc ggagtgtaga 192120
   tgtatagaat agatttatta ttatagaagg ttttattaga cggagttggt ttagagtatg 192180
   gaggtggcgg ttttagatgg gtatatgttt ttgttttcgt aggcgttttg ttttggtagg 192240
   acggttgttt tattttgttt tgaaggtagt tttggtttat aggcgtttta tttgtgtgga 192300
   tttgatttcg ggttcgtgtt ttttggtaat tttgtttttt ttagaatggt taatttatga 192360
   aggtatagtt tttgtttgtt tgttattatg atggtagtta ggggtttggt atttagttgg 192420
   tttttagtat atatgtatag gtgattacgt tatttttgtt ttttttagag tttttcgttt 192480
   tttaggggag gtaagtttat ttattgttgt gatttattat tttttttaaa tagaaaagtt 192540
   attagatggg tatttgtttt agtttggtcg ttggaagtga tagttaagaa taacgtaggt 192600
   ttttagaaat agaggtatta gtatggttat attggggtta ggtaggggag attagggata 192660
   tttagtgggg agaagatgac gcgtagattt agttttgggg ggtaagggtt ggaggtagag 192720
   ttttgatttt tttgttttat ttttttgttt aggttgtatt tagtgttggt agagtttttt 192780
   taagtttata agttgtaatt cgtgttttgg acggttagtt ttagagtttt gatgggtgta 192840
   tttgtttttt ttttttattg gttagtgtgg aaaattttta aatatatgga aaaagtataa 192900
   ggagtagtat agtgattatt gttagaggtt tatttatttt tttttagata atatcggacg 192960
   tatttgtttt attttggagt attttttatt ttgtaaggtt gtagggagtt tatagatatt 193020
   gaattaatat cgagaggttt acggttaaag ttatttgttt atggtatatt tgttttttta 193080
   gtattttaaa ttaaattatt tagttttatt tattttttgt tttttttttt gtttcgtata 193140
   tacgggtttg ttttttgagt ttgggggtgg ttgtttttgt ttttataagg attattttta 193200
   gtagggtagc gtcgataggt ttggaagtta tttgagttta aagtcgttaa agattgcgtt 193260
   tttttgggaa atgggtaagt ggttttattt atttagggaa gttaggtggg gaggtaggtt 193320
   ttagattaga atgaagggat tttagtataa atatatttat gagttgggtg gggttagagc 193380
   ggagcgagtt atttatttgg gttattggag ttattaagtt ttcggagtcg gttattggag 193440
   gttcgcgtgt tttttaaaga tagagataaa gggtaaaggt tatataaggt tattggcgtt 193500
   tttttttcgg gtggatgatt aatattagga atgttttcgt ttttgttttt gttgtgagtt 193560
   ttagagttag aaacggggtt tatttgggga tagaggtttt atttttttag gtaattttgg 193620
   acgttggagg gatgatagtt attgttgttt ggttgttgga ggggttttgg gttaaatttt 193680
   tatatatatt tttttttaag ttttattatt tttgttttgt agataaggaa tttgagtttt 193740
   tatagagtgt tatttttttg tggttatata gtgaatgaga gttgtatata ggtgagttta 193800
   cgtttattag attttaagtt gggttgtatt taagcgtgtt tttgttggac ggagtttttt 193860
   ttttttttta tgtttattcg ttttatgaat tagggttttt ttatattagg gtttataatt 193920
   tacgggtagt tttgatttag ttttttttat ttttagggtt gggtgtagtc gggttggatt 193980
   tggggatgga gtgggatagg ttatttttat tttttgtttt aggagggaat aaaaatagaa 194040
   ttttagatta atgtagtttt tcgcggtatg cgtttttttt gtttatatat aggttttggt 194100
   ttggggttgt ttgttggaga ggtttatggt aggtaagttt taattcgagg agatttgttg 194160
   aggtgttaag ttaagtaaat tcgaaaggtt gttaggagga gcggttttgg gagtataggc 194220
   ggttatttta ttgttgtaga gtacggtatt ttggggatgt tttttgggta atgttatata 194280
   gttatttttt agagagtttt agggatatat aattagttta gttttattaa tagtgagata 194340
   agggattagg tagatttaga tatttttttg tatttgatta ttatgttggt tgttggttaa 194400
   agatgttagt ttttttttgg aattttttag aaaggttttt gtagtattta tttatttatt 194460
   tatttattta tttattttat ttatttattt atttatttat ttatttatgg attttattta 194520
   ttttattatt tttttatata tttatttatt tatttattta atttatttat ttagtttttt 194580
   atttatttat ttatttatta atttatttat ttgtttattt aatttattta tttattaatt 194640
   tatttattta ttatttattt atttatttat ttatttattt atttttttat ttatttattt 194700
   attttttttt ttatttattt tataatttta tttttaaatt gtgattagat ggaaatgggt 194760
   gtagttagtt tgtttttaaa ggtttagagc ggaggtagag tgaattgttt atttggtttt 194820
   cgtgtttagt agtttaggtt tgtatatatg ttttataatt ataggatatt agatttagta 194880
   attggtagta gaatgagtat agtgtatttt tcgcggaatt agtattgata gggtatttat 194940
   ttttattaag tttgagtttt tttggtgttg gagggaattt ttagatgggt tttaatttta 195000
   gttgaattta ttttttaggg aaagtaatat cgtgagaaat gcgtgtgata taaggtttta 195060
   aagagggtgt gggtcgtgat ttttggagta attagggagg ttttatggag gagatgagga 195120
   taaagttgag ttttgaagga gggatcggag gacgttgagt agggatattt atttttgttt 195180
   gcgagggttt gacggtaggt tgtgagttgt attttagttt cgatttattt aatttataat 195240
   tttagtaggt aggtgttttc gttttatttt atagaggagg aagcgaggtt tagggttggg 195300
   taaattgttt aaggtcgtat agttgcggaa tttattttta gtatttttag tttgttttag 195360
   ttatgttcgt gattttattt agtatttttt ttttggtgtt tatttatttt gtttattgtt 195420
   aagtaataaa aattttatta ggaaaattta ggggtttttg gttagttttt ttagatagaa 195480
   aggtagattt cggcgtagat ttttaggaag taaggatgga agggttagta tttgtttttt 195540
   agaaatgata gtttttttaa gattaggaga gatggtttag agtgtagttt cggtgggttg 195600
   tttaggatta ggaggggaga ggagcgggga ggaggatgaa ttttattttt aaggttgttg 195660
   agtggtatat agtgagtttg gattttttag ttttagttga gtttattttt acgatggtta 195720
   tgagtatttt ttttatatat tcgattggaa gtcgtattgt atataacgta tacggggtta 195780
   ttattttttg ttttatatat ttttacgtat atattattag tgtattattg ggatattttg 195840
   ttgttcgggg ttgagagtga attagaattt tgtttataaa tttagtatat tttattgtgg 195900
   aaataatggg gggtaaggag gggtcgttta gtaaagaata ggatatacgt ttttgaggat 195960
   ggagttattt ttggttgaga tatttttgta ggggatgtcg gcgataaagg ttgttttatt 196020
   tttttttttg ggttagatgg atattatttt tgttttttgt tttttagtat taggtaaaag 196080
   gaaaatagaa gaggttttat ttaggttaaa tattttaggg ttttttattc gtaggaaata 196140
   tgttggttta ttttttttaa atttagtatg ggggtggaga aacggtgggg aaaatagtat 196200
   ttgttttaga ttttttgggt ggagtttttg ttttatttat attgtttatt tttttgattt 196260
   tttaaaatgg gtatttggtg tttggtagtt tttggtatag agaagacgta aagtaaatcg 196320
   tttataagaa gggggcgagc gtagtttgag tattttttgt ttttgaataa attgttaggt 196380
   ttagcgtagt cgttgtcgtt attgttgttt ttaggaaggg atttttggtt acgagcggtt 196440
   ttagtttagt tttttggatt taattattta ggtttgtgtt tgagtttagt aaagaggtag 196500
   ttagaaaagt ttttaagata tttaacgatt tcgtggtttt agttgagaag tgagttattt 196560
   ttatgtgatt ttaaaataaa atatatttag aaggagagaa gataattgaa atttagttgt 196620
   ttggagttat tttagatgaa agtatttgag tttttgggag gtaaaggagg taggaaagtt 196680
   tggagttaga tgattagaat ttgaacgtag atttcgttag gtttttgttg tgggttttgg 196740
   gagatgattt ttattttttg agttttagtt tttttgggga tagagatggt gtttatttat 196800
   aggttgtttc gagttaatga ggttatatgg cgtaagtatt agtatagtgt ttggtataaa 196860
   attaaaattt aataagtgat attattatta ttattattat tattattatt tttgagatgg 196920
   agttttgttt tgttatttag gttggattgt agtggtacga ttttagttta tcgtaatttt 196980
   cgttttttaa gtagttgaga ttataggtat ttgttattac gtttcggtta atttttgtat 197040
   attcggtaga gatagggttt tattacgttg gttaggttgg tttcgaattg attttaggtg 197100
   atttgtttgt tttagttttt taaagtgttc ggattatagg cgttagttat cgttatttgg 197160
   ttgtgatatt attataatat taatattata atagttggtt aggtatggtg atttacgttt 197220
   gtaattttag taatttggga ggttgacgtg ggttagttat tcgaggttag ttcgagatta 197280
   gtttgattaa tatagggaaa ttttattttt attaaaaata taaaaattag tcgggcgtgg 197340
   tggtattcgt ttgtaatttt agttattcgg gaggttaagg taggagaatc gtttgaattt 197400
   aggagacgga ggttgtagtg agtcgagatt atattattgt attttagttt gggcgataga 197460
   gtaagatttt gttttaaaaa ataataataa taaataaata aaatatattt tgacgagata 197520
   tggtaaaaga tagtagaagg taggagttcg gggatttatt atgggtcggc gttttaagta 197580
   gaaagaaatg tagtgtttat aagttagaga gtagatgtcg aagataggag acgagcgggg 197640
   ttttttggtt ttggagagga aagggtgagt ttaggtagaa agagtaaatt tagggcgttt 197700
   tgtgtagtgt tttttttgtg gaattagttt agggtattag tagtatagtt tttttttagt 197760
   agtggagatt aggttaattg tttagtgttt tttttgtttt tttgttttaa ttttattaag 197820
   tatagttttt ttttgggtat agtagttttg taggggtgga tattgattta tgtatttagg 197880
   agtttagagg tttttttttt tttatttatt tatttagaga tggagttttt ttttgtcgtt 197940
   taggttggag tgtagtggta ttaatttggt ttattgtaat ttttattttt taggtttaag 198000
   tgattttttt gttttaattt tttgcgtagt tgggattata agtatgtatt attacgttcg 198060
   gttaattttt gtatttttag tagagatggg gttttattat gttggttagg ttggtttcga 198120
   atttttgatt ttgtgatttt tttattttgg ttttttaaag tgttgggatt atagacgtga 198180
   gttattacgt tcggttaggg gtttttttta atggattttt tttaatgtat ataatttggg 198240
   gaatttagat ataagaaaga tatagagtat gaaggttttt tattggtttg tttattttta 198300
   gaggtggggt tttaggtggt ttaggttggt tttaaatttt tggttttaag taattttttt 198360
   gttttggttt tacgttggga ttataggtat aagttattac gtttagttat tttttttttt 198420
   atttgggttg ttatgttaga atttggtaag ttataagatg gaaatgttta aataggtatt 198480
   tagaatttga gtagatatta tttgatgtat tattgttgtt attttaatga ttgaaatgta 198540
   tttattatat tcgttttttt aaaggttatt ttagattttt atagatttta tttgtgatgt 198600
   aagatagtta ttttaaattt ttttgtgggt tcgaatagat tagatttttg aatttttggg 198660
   tagagtaggg atggggttgt tttagaggag ggtgatatcg ttatattttt gttttgtttt 198720
   tgtgttgata atgaatgagg tttggatagt ttttttttta ttaaaagtta tattttttta 198780
   aaaaggagtt ttttaggatg aggcgttatg ataatttaat tgtgtataaa tatttatttt 198840
   taataatgat agaagttatt tatagaaaag aatttatttt tggtcgggta cggtggttta 198900
   ggtttgtaat tttagtattt tgagaggtta aggtaggtaa attatttgag gttcggagtt 198960
   cgagattagt ttgattaata tggtgaaatt tcgtttttaa tgaaaatata aaattagttt 199020
   gtatgtttgt aattttagtt atttaggaag ttgaggtaga agaagtattt gagttcgaga 199080
   ggcggaggtt gtagtgagtc gagatcgtgt tattgtattt tagtttgggt gatagagcga 199140
   aatttttttt taaaatatat atatatatat atatatatat atatatatat atatatataa 199200
   atttattttt attttttttt ttgaaatttt tgagagaggt taggtgtagt ggtttatatt 199260
   tgtaatttta gtagtttggg aggttgaggt gggaggatgg tttgaggtta ggagttggag 199320
   attagtttgg gtaatatagt aagattttat ttttattaaa aaataataaa agtttaaaaa 199380
   gtttttgaga tagtttttgg ggaaaggtgg gttatggaga tttttatttt ggttattcgt 199440
   gtttttttcg tttgtttttt tgtggttttt ttttattttg agtttggggt gggggcggtt 199500
   tgagtttagg atgggggtgg tttgagtttg gggtgggagc ggtttgagtt tgggataggg 199560
   cggtttgagt tggggtgggg gcgttttaag tttgggttgc gggtattttg agtttggggt 199620
   ggggatgttt tgagtttggg gtgggggtgt tttgagtttg ggttgagggt ggtttgagtt 199680
   tgtgttgggg gtggtttgag ttgggatggg ggcggtttga gttgggatgg gggcggtttg 199740
   agtttggggt gggggcgttt tgagtttggg atgggggtat tttgagtttg ggatgggggc 199800
   gttttgagtt gggatggggg cgttttgagt ttgggatggg ggcggtttga gttgggatgg 199860
   gggcggtttg agtttggggt gggggcggtt tgagtttggg gtgggggcgt ttaggatgta 199920
   taggttttat tttttttttt tgaggtttag tttgtttata ggagttgtga agggttggat 199980
   taaagttaaa ggtttaaatt ttagatttat ttttagattt agtggataat taaaatattt 200040
   aagtttattt ttaaaggaag ttgagataag gagataatgg ttattttttt tttttttttt 200100
   tttttgtttt tggtttttgt tatttggagt tgtgttttga agaggtatat tgggtattag 200160
   gtttttttgg ttttgttttt attttttgtt tagatataga ataatttttt tttattttta 200220
   aggttgatac gtggggttgt ggattaaagt taatttattt atttatgagg ggagtaattt 200280
   ttttgtttta ggaatttgtt tttataggag aataaatatt gtagaaggta ttgtttttag 200340
   tggtggtgta cgtttgtaat tttagttatt taggaggttg aggtagaaga attgttagaa 200400
   tttaggaggt ggaggttgta gtgagttaat attatgttat tgtattttaa tttgggtgaa 200460
   aagattaaga ttttgtttta aaataaaaaa agaatagtat tttgtgttaa ggttaggggt 200520
   tttagttatt ttttttgtga agcggaaggg gtattattag tttttgtgag aaggaaatag 200580
   tttatttaat tataaaatgt taattaaagg aggtagtata ttggttttgg tgcggtgtcg 200640
   ttggtttttg tttggggtag gaggagggtt gggggagagt taaaagttaa agaaggttta 200700
   tggaggtcgt tttttttttg gtgattagtt ttttttttga cggtaagttg ttaatagtat 200760
   ttggttttta ggttggggaa ggtgagatta gtttatattt gtttagggtt ttgttttgtt 200820
   tgtatgggta gtaggcgttt attttgttag atggttttgt ttattatagg gtattagtgt 200880
   tagttaagga tttatagagt ttttttgtaa aggtttatta gtgggtattg ggattttggg 200940
   ttttatttat atagggtttt tatataaaat ataggatgtt tagttaaatt tggattttag 201000
   ataaataatt aataattttt tgaagagata aggattttgt tgttttattt aagttggagt 201060
   ttagtggtat gattatggtt tattgtaatt ttgaattttt ggatttaagt aattttttaa 201120
   ttgtagtttt ttaaagtatt gggattgtag gtatgagtta ttaaggttag taaataatga 201180
   attattttta ggtataagta tattttaagt ttgttgtggg atatgtttat attaaaaatt 201240
   aatttttgtt tatttgaaat ttaaattgaa tttattgtcg ggggagataa tggagtgatg 201300
   gattgataga ggagtataga gatagttttt ttaaattttt agttgattaa agtttggttt 201360
   ttagtttttt tgggttttta gagatgtatt aagggtggaa gcgattagga gggagataaa 201420
   aatattgaag tttgagtgtt agtattgagt cgtgtggttt tggataggat tgtttttttt 201480
   ttgagtttta gttttatatg ttataattgg ttaataatag ttataaagta ttcgaagtta 201540
   atggttggga gtttgtttta ggaggtaggt attattagga tgtagagaga gtgaggagtt 201600
   tttaaagtta gtttaggtta ggtatagtag tttatgtttg taattttagt attttgagag 201660
   gttgaggcga gagaattatt tgagtttaga agtttaaggt tgtagtgagt tgtaattgta 201720
   tcgttgtatt ttaggttgaa tgatagagtt tttgttttta aaataaaata aaataaaata 201780
   aaataaaata ataaagttag tttgtgtatt ggattgtatg ttgttttaaa gtatttaaag 201840
   tgagagaggt taggaagtaa tgagatgtag aaaggtaggg ggaggaggtt tagaggtatt 201900
   agagaagata gttgattttt atttttatat atttttgggg tattttgagt tttatttata 201960
   tatttaggtg agaatttttg tatttgtgaa agtaatttga ggtcgagaga attaatagga 202020
   aatcgtttta ttttttatgt agggtttttt tataaataag gttgagtcgt agtttattat 202080
   tgtagttgag ttttttgtag tagttgcggt ggggaaggtt ttttaggagg tttgagtaat 202140
   ttagaaatat ttatagtata ttttttagtt tttttagagg tttttgttga gatgaaagtg 202200
   ttttggttcg tggttaaaga gtttagtata ttgggtagtt atagttttta ttatattgat 202260
   ttggttatta ggtttttatg aatttagtga ggggagatgg gttttttagg gaagtttgtt 202320
   gattttttta tattgttgta aatgtaaagt ttggtgattt tattattttt agtaattgtg 202380
   atgtagtaat aataagaata gttatgttta tcgtttattg aaggtatatg atgagttaga 202440
   tattgtgttg gtatatgtga tttttggagg taatatattt ttttttaagg aggaaatgat 202500
   tgagttttgg agggagggtt ttttatttgg ttttaggttt ttttttgatt tgcgtttttt 202560
   tttttttttt cgtttgtttt attgtagttt ttttggtttt tttgttgttt ttggaatgta 202620
   ttaagtttgt ttttagttta gtttttgtat tgttggtttt tttttgtttt tttaatttta 202680
   atttagttgt tattattttt gtgatttttt attatttatt ttaaagtagt tattagttat 202740
   tttttatgta attattttat tttaattttt attatagttt ttagttttat tttgatagtt 202800
   tttagaattg atgaatttat tgattttgtt tttttttatt gtatgtaaat tttaagaaat 202860
   aagagttttt gtttgtttat tgttgtgttt tagtgtttgt tatagtattt ggtatgtagt 202920
   aggtgtttaa tgaatatatt tttaaaagaa tgaacgattg gaaggagttt gaaagaacga 202980
   tggtttgtgt taatagttta tttttaattt ttttagacgg gatggagatt attattatgt 203040
   aattacgatt tttttgagtt tgtgtggtgg ttgacggtag gtgttagttt ggttggatta 203100
   agggatggtt ggtaacgtat cgtttttggg tgtgtttgtg agggtgtttt tggaggagat 203160
   gggtatgtga gttggtgggt tgagtggtaa agattttttt ttaatatgga tgatattatt 203220
   ttattgttag ataataaaaa ggtagagaaa aggtgaattt tttttttttt ttggaggtgg 203280
   gatgtttttt ttttttgttt ttggatttag aatttttagt tttttagtag ttttttaggt 203340
   ttttaaattt ttggttttaa attaagagtt atattaatag ttttttagtt ttgaggtttg 203400
   tagatttgga ttgagttacg atattggttt ttttgggttt ttagtttgcg gacggttttt 203460
   tgtggtatat tttggttttt ataatggtat gagttaattt ttttaataaa ttttttttta 203520
   tttatttatt atttattttt ttaattgttt atttgtttat ttatttattt atttattatt 203580
   tattttttat ttatttattt attatttatt taattaatgt atttatgttt gtttgtttat 203640
   cggttattta tatttattta ttaattattt atttatttat tatttatttg ttatttattt 203700
   agttatttat ttatttattt atttattttt tttttttttt tgagatggag ttttattttg 203760
   tcgtttaggt tggagtgtag tggcgtgatt ttagtttatt gtaattttta ttttttaggt 203820
   ttaagtgatt ttcgtgtttt agtttttcga gtggttggga ttatagatat gtattattac 203880
   gtttagttaa tttttttatt tttagtagag atggggtttt attttgttag ttaggttggt 203940
   tttaaatttt tggttttaag tgatttatcg gttttagttt attaatgtgt tggttattat 204000
   aggtaagagt tattgaattt aatttattat ttgtttgttt gtttgtttgt ttgtttgtgt 204060
   ttatttgttt gtttggttat ttattattta tttatttatt tgtttgtttg tttgtttgtt 204120
   tgtttgtgtt tgtttgtttt tatttttttg tttgtttgtt tatttagtta tttgtttgtt 204180
   tgtttgtttg tgtttgtttg tttttttttt tttttgttcg tttgtttgtt tatttagtta 204240
   tttatttatt tatttatttt ttattggttg tttttttgga gagttttggt tgatgtagac 204300
   ggtgttttgg taagtttagg ttgttattta gattattgtg gagggagtga tttaaataat 204360
   agatatgtat tttttagttt gagaggttta aagtttcgga ttaaggtgtc gttagggtta 204420
   gttttcggcg aggttttttt ttttggtttg tatttatttt tttgttgtgt ttttcgttat 204480
   tttttttttg tttataagtg aggggggagg agagattttt ggtgtttttt ttttttttta 204540
   taaggatatt aattttatta gattagggtt ttgttttatg attttattta attttaatta 204600
   tttttttaaa agttttattt ttaattatag ttgtattggg agttagggtt ttaacgtagg 204660
   aatttaaggg gaagggtata gtttggttta taataaatgg gaaattatgt ttatgattaa 204720
   attttttaga agatatggtt tattagcgtc gttaatgatt gtaagtaaag gttagagagg 204780
   aaaggggttt tcgtggggtg gggggcggtg ggggtagttg gtgtcgggaa taaggttttt 204840
   ttttttgttt aatagtaggg aagtgaggtc ggttgtagat gttatttggg gtgggaagag 204900
   aggggagttt ttgtttgtat attttgtgtg ggttttggtt tgattttttt tcgtgttttt 204960
   tattttttgt gttttttttt gtgtgggaga aacgtcgatt tttgttggtt taggtgttta 205020
   ggtttttagg tttgttggtt ttgggttggg tttaattaac gagaggtttg ggaggaattt 205080
   gggaggtgga agtaagggag ggcgcgggag ttttttattt tttttgtttt agatagtttt 205140
   tttagtagga gaggtggttt tttgtgtagt tttagttttt gttagagttt tatttttgtt 205200
   tgggttttta gtaggtgatt ttggtttttg gattttggtt ataggatttt ttttttttta 205260
   tttttattta ggggtggtgg tggtattttg ttgttaattt ttaggttatt tttataatgt 205320
   taagaatttt ttttttattt gtatggtttt tttttaaggt attgagaagg gttttaatta 205380
   tatgtttata gggttaaata ttttttgtaa aatttataaa atggaaaata ttttttttat 205440
   aattaattaa cgttttcgtt tatttttagt tttgagtttt tgttcgttat attttttttt 205500
   gtgtgaggtg gaaacggagg tttgttgtta cgtgggggtt tttgttaagg agaagttgaa 205560
   ttggaaagat atttagtttg gttttggtcg ggttatattt atgtagtttt tatttatttt 205620
   tatgtatagt ttagttattg ttcgttattt cggtgtggaa acggatttta gtaatattcg 205680
   ttttatttat cgttttaatt ttatttatcg cgtagaatta aaaattataa gatacgaata 205740
   tattttgcgg tgtttggcgt cggaaaaacg tgcgtagtgg agagaagtaa gatttgaaat 205800
   gtacgggttt agaagtaaat ttgtggaaaa ttatttttat tattagaggt gaagttgtaa 205860
   gtaaaggatt tagttttttt aatgtggagt taaaatagaa aagttttttt ggttagaaat 205920
   atatgtataa atgttttata tgtagttaga aatttttttt atttttttat tttttttttg 205980
   atgggaatta tatttaaaaa aaaagaaaaa aataggattt attagaattt ttgtgtattt 206040
   tgaataaaat aattgtagta gtattattaa ttttatgttg atcgttattt ttgtatatat 206100
   tttaattatt aaaaataaaa ataaaatttg attaattaag gagatttatt cgttttattt 206160
   atttatttat ttttgagatg gagttttatt ttgttgttta ggttggagtg taatggtacg 206220
   attttggttt attgtaattt ttgttttttg ggtttaagtt atttttttgt tttagttttt 206280
   taagtagttg ggattatagg cgtgcgttat tatatttggt taatttttta tgtttttggt 206340
   agagatgggg ttttattatg ttgattaggt tggttttaaa tttttgattt taagtgattc 206400
   gtttatttta attttttata gtgttggaat tataggcgtg agttattacg tttagtttat 206460
   gttttttttt gttgtttttt tttttttttt gagatatggt tttatttttt tatttaggtt 206520
   ggagtgtagt ggtatgatta cggtttattg tagtttcgat tttttgggtt taagtaattt 206580
   ttttatttta gttttttaag taattgggat tataggtgta tgttattatg ttttgttagt 206640
   ttttgtattt ttagtagaga cggggtttcg ttacgttgtt cggatcggtt ttgaattttt 206700
   ggttttaagt gatttatttg ttttggtttt ttaaagtgtt gggattatag gtacgagtta 206760
   ttgtgtttag tttgattagt tttttgtttt cgaaatagaa aacgatatta taaagttatt 206820
   atattgaaga agtaatgtta aaaatgtggg agaaatagaa ttttggagat atgattgata 206880
   gttgattggt atttttttgt atgtagtgat gtttgtgata tttaatagtt gtttataatt 206940
   tataatttgt tgttatttat ttttttattt taaatgaatt attgtattta tatttttaga 207000
   gggttgtata agtttatatt attttgattt tttgttgatt gtttttttgt tttttgtttg 207060
   gtttttattt tttttattat ttgtgtaatt agtttttcgt ttttaagttt ttttgtttta 207120
   aatgtttaag tggattaggt attgtggttt tcgtttgtaa ttttagtatt ttgggaggtt 207180
   taggtaggag gattatataa atttaggagt ataagattaa tttgggtaat atagtaagat 207240
   tttgttttta tatatatata aaaaatataa aaattagtaa ggcgtgatgg tatatgtttg 207300
   tagttttagt attttgggag gttgaggtag gaggattgtt tgaggttggg agtttaggat 207360
   tagtttgggt aataaagtaa gattttgttt ttataaaaat aaaagaaaaa attaggtagg 207420
   tatatagtgt gtatttatag ttcgagttat ttgggaggtt gagaagggag gatcgtttga 207480
   gtttaggagt ttaaggttgt tatgagttgt gattatatta ttgtatttta gtttgcgtga 207540
   tagagtgaga ttttgttttg ggaaaaataa ataaataaat aaataaataa ataaataaat 207600
   aaatgtttaa gcggttttta ttttttggtt agattttatt atatatttta tgaataaatc 207660
   gtatgtatga gaaaggtgag tagatgaata gggtagggtt agggggttaa ggggaagatt 207720
   atagattacg tggagcgaga ttagtggtta aaaattgtta attatagtta ggtttggtgg 207780
   tttatgaagg taattttaac ggtcgggttt ggtggtttat gttcgtaatt ttagtatttt 207840
   gggaggtcga cgcgagttta ttatttgagg ttaagagttc gagattagtt cggttaatgt 207900
   agggaaattt cgtttttatt aaaaatatta aaattggtta ggtatggtgg cgggcgtttg 207960
   tagttttagt tatttgggag gttgaagtag gagaattggt tgaattcggg aggcggaggt 208020
   tgtagtgagt tgggatttta ttattgtatt ttagtttggg cgatagagcg agattttatt 208080
   ttaaaaaatt taaaaaaaaa aaaataaaaa attgtagatt ttaataagga agagttatag 208140
   gacgatattt taggatgcgg gaaaaggtta cgttagtttt ggattttttt tgattaatgg 208200
   gtattttagt tattaaaggg gtagatgcgt tttatttaat ttttatttag ttattagagt 208260
   ataatatagg gatgtgttat agatagagat ggagattaat aggtagaggg gtgatgggga 208320
   ggaagattga ggtaagagga ataaaacggg gattgtagag ggtaggtggt aggagtttga 208380
   gggttttaga agaaattgtt gtataagtaa tgagttagta gggtgagtga cggttaggag 208440
   aggtggttta agaaaataaa gagatatttt cgggggtata gatgcgtttt gaggggtcga 208500
   ggaagagatg gggaaggggt ggttttgtgg attttcggta tgattgtcgt ggtgaaaagg 208560
   aattaaagat ttcgataagg ggatgatttt ttaaataagg aagttttggg ttttcgtagg 208620
   gggattagta gattgagggt aaacggtatt ttgtttatta aagttttaat aaaatgttaa 208680
   ggtaagaata tttgtttggt tttgggggta attttagaaa tattagaaaa gggatttatt 208740
   atgcgtaatt tttttttaat tatttggaga aatgtatggt tatttaagat agagaaaggt 208800
   agttaaagta agtggaggtt aggtatagtg atttacgttt gtatttttag cgttttggga 208860
   ggttgaggta ggaggatcgt ttgaggttgg aaatttagta ttagtttagg taataataaa 208920
   ataagatttt gtcgttataa aaataaaaat aaaaattttt tgagacggag ttttattttt 208980
   tttgtttagg ttggagtgtc gtggtacgat tttagtttat tgtaattttc gttttttaga 209040
   tttaagtaat ttttttgttt tagttttttg agtagttggg attataggta tgtgttatta 209100
   tgtttagtta attttgtagt tttagtagag atagggtttt tttatgttgg ttaggttggt 209160
   ttcgaatttt cgattttttt ggttttttaa agtgttagga ttataggtat gagttatcgc 209220
   gttcggttgt tataaaaatt ttttaaaaat tagttaggta tgttaggcgc ggtggttttt 209280
   atttgtaatt ttagtatttt gggaggttaa ggtaggtgga ttacgaggtt aggagtttga 209340
   gattagtttg attaatatgg tgaaatttcg tttttattaa aaatataaaa attagttagg 209400
   tgtggcggta tatgtttgta atcgtagtta tttaggaggt tgaggtagga gaattgtttt 209460
   aatttaggag tcgggggttg tagtgagtcg agattgtatt acggtatttt agtttgggta 209520
   atagagtaag attttgtttt aaaaaaaaaa aaaaaaaaaa aattagttag gtatgtagcg 209580
   tatatttgta gttttagtta tttaggaggt tgagaaggga ggattaattg aaattgggag 209640
   gtcgagattg ggaggttatg attacgttat tgtattttaa tttgggtaat atagtaagat 209700
   tttgtttaat aataataata ataaataatg taagtgggta taagaattta tttatttatt 209760
   tatttattta tttatttatg agatagggtt ttattttgtc gtttaggttg gagtgtattg 209820
   gcgtaatttc ggtttattgt aagtttcgtt tttcgggttt atgttatttt tttgttttag 209880
   tttttcgagt agttgggatt ataggcgttc gttattgtat ttagttaatt ttttgtattt 209940
   ttagtagaga cggggtttta ttatggtttc gattttttga ttttgtgatt tgttcgtttc 210000
   ggttttttaa agtgttggga ttataggtgt gagttatcgt tttcggtttt aagaatttat 210060
   tttattttag tattttggag gttgaggcga gtggattacg aggttagaag attgagatta 210120
   ttttggttaa tatggtgaaa tttcgttttt attaaaaaaa tataaaaaat tagtcgggcg 210180
   tgggggcggg tgtttgtagt tttagttatg cgggatgttg aggtaggaga atggtgtgaa 210240
   ttcgggaggc ggagtttgta gtgagcggag atcgcgttat tgtattttag tttgggtaat 210300
   agagcgagat tttattttaa aaaaaaaaaa aaaagaattt attttaagat agtaagtttt 210360
   agggttgaag ttagttttta tttagtttta ggtttaaagt tataagtatg gttattgtaa 210420
   tatttttatt taggatatta ttttttaggg gtttagattt atattattaa ggaggagcgt 210480
   ttatattagt agttggggag agggtggttt tttataagat gtaatgttta ttttgaagag 210540
   ttattatagt gataattatg aaatttatgt ttgagattta attttaagtg aaaagaatag 210600
   aatttaatat ttattttatg gggagtataa aattttgaaa atatacgttt tttataatta 210660
   aattaaattt tatgttatgt ttttgaggag ataagtattg aatggaatat agaagaaatg 210720
   ttatagttaa tgattgttga agtttttttt ttaatttttt gtttatgttg tttggtaata 210780
   agtaatatag aaaattgata ataaaaagta aaagagaaag aaaagatata gtgtatagtt 210840
   tagattgttt tttttaaaaa attaaataat aataataaaa agtataggtc gggtgcggtg 210900
   gtttacgttt gtaattttag tattttggga ggttaaggcg ggcggattat ttgaggtcgg 210960
   gagttcgaga ttagtttgat taatatgggg atatttcgtt tgtattaaaa atataaaatt 211020
   agtcgggtat ggtgacgtag gtttgtaatt ttagttattt gggaggttga ggtaagagaa 211080
   tcgtttgaat tcgggaggtg gaggttgtag tgagttgaga tcgcgttttt gtattttagt 211140
   ttgggtaata agagcgaaat tttattttaa aaaaaaaaaa aaaaaagtat aaatgtaagt 211200
   tgggcgcggt agtttatgtt tgtaatttta gtattttggg aggtcggggt aggtggatta 211260
   tttgaggtta ggagtttgaa attaattcgg ttaatatggc gaaattttat ttttattaaa 211320
   aatataaaaa ttagttaggt atggtggcgc gtgtttgtaa ttttagttat ttaggaggtt 211380
   gaggtaggag aattgtttga atttaggagg tagaggttgt agtgagttaa gattgagtta 211440
   ttatatttta gtttgggtga tagagtgaga tttcgtttta aaaattaaat taaattaaat 211500
   taaataaaat aaaaaaaaga agcggtataa tataaagttt cggtagttaa agcgttaatt 211560
   tttagttatt aggaaggttt tgttatttaa tgatttatcg ttttagttaa ggttagttat 211620
   attttgggtt tttgatgttg gaggtaatat gatgttaata ttggcgagaa atttattatt 211680
   tttttttttt cgtttttcgg tgtcggtttt tgtgtgtatt tgtagttttt ttattatcgg 211740
   tgtttttatg gtgtttacgt tatcggttta ttttttagta ataattcgat agttcgatat 211800
   ttttgtagat tttgtggtta tagagttaaa gttttttgtt attagtgggt tttgtagata 211860
   attttttaaa tttttttggg ttgagttagt taagttttta ataagttgga agatgatagg 211920
   ggttggatgt aatttatttt taattggttt gggttatatg gatattattt tttttttggg 211980
   atttgatttg ggatggaaat aggagttata gtttatggga aggttgtgaa aggaagtttc 212040
   ggttttaggg tgcggattag gttgatttgc ggaataaggt tttaggggcg gtttattcgt 212100
   ttgttttgtt tagttttttt tggtaattat atttattcgg ggtttatttt aaatttggta 212160
   ggtaatgttg ttgtttttat tttagtcgtt gttttaatac gtttcgtttg gttaggatag 212220
   gtgtttaggt atggtattta gggatatttt cggttttcgg ggtttaattt ttggttttta 212280
   ggtgtagcgt tgggaaaagt agagggtttc ggttatggcg taggtttcgt tataggttat 212340
   ttaagtagtt tgggaggagt cggaaagaga gtaatgtatt tatttggata gagattgaag 212400
   ttatattttt attgagaaaa gatgatattt ttgtttggat tttttttttg aaattttagg 212460
   tcggggtggg gtaggagtta gcgagagagt ttagcgtttt aaaggggatt ttttggtttt 212520
   tcgtgttttt taatttagta tttttgggga tttatatatt tagggtagag tggttattta 212580
   gggttttttt tatttagata taaaggttat tgaaggtttc gggttaatat cgttttgagg 212640
   atgattagcg atttgagttt ttgttagaga tagtaggtag tttgggttta agggtcgttt 212700
   agtgatttga agttgggatt attttatatt ttttgcgtta tttttgtttt ttgttttgga 212760
   ggtttttagg ttgttaatag ttatgttttg gagaattagg ttagattagg ttgggaataa 212820
   agtttagttg ttatagagtt gtcgagaggt tattggtaag tagtgtttag gagtcgtttt 212880
   ttttttgtta gtaatagttt aatgtttttt tttatagggg ttgtaaatgg gtttatgttt 212940
   agggtggggt tagggagata aagtagagag aaattgggtt gggagtagag agtgttatgt 213000
   tttattttgg agatttaagg ttaggttatt ttgtagagtt ggtgtttaga gtttttagcg 213060
   tataggagag gaggtaaata gaatttgata tttagtagcg ttcggtagac gtttttggaa 213120
   tgaatgatta aatttatatg gagagttgaa atgggttagg aattttaggg agacgggtag 213180
   tttatagttt agtagggtcg ttttagtaga gtaggttaat gttaggtttt ttggtaaatt 213240
   tttcggggtt taggtataat tgttatatta ggaaaggggt aggggtgatt agtttatcgg 213300
   ttacgggggt attttattgt ttttatagaa gttcggtatt tataggtttg ggtgatatac 213360
   gatttttatg tttggtatta cgatggttgt ttattaagtt tttttattta ttaggattga 213420
   ttttttttgg ttagcgattt tagattatta tgatttggta gttgtcgtta ggtttgtttg 213480
   gttaaggatt ttttcggaga ttgagttatt ttatatagta aagtgagtta tggggttata 213540
   gcgttgtgtg ttgtgtataa gtttcgaggg ttttatttag ggattggtat ttattgattt 213600
   ttataaattt ttggtaatcg tttagagttg ggatgttttt aaggtaatag ttttaataaa 213660
   tagaggcgta ggaatttagc gcgttatttt tcgttttatt ttaaaggttt atatttttag 213720
   gggtttggag tttttttttg gagttttttt tattttaaag ttagttggat ttgtttagga 213780
   attgttatta ttattttttg gtcgggtttg tattgatgtg tattgaattt tttttattag 213840
   gtttatttta ggtaaatttt agagtttacg taatattgtt tgtggataaa tattggggcg 213900
   gaaatagggg ttatttttag gttttagtta tatggggttg ttagggttgt ttgggatggt 213960
   aggagagtgt tagaggaggg tagtttaagg attaggtagg gggtgggaag tgtttttttt 214020
   tatgggtttt taagtgagat gttttttgta aatatttatt tgggattttt aagtagaggg 214080
   ttggttttta tgttatttgg atttggattt tagattattg tttttaagtg gtaattttgg 214140
   gtttttttgt atttgttttg gtttgagttg gttatatttg tggggggtgg ttttaggaag 214200
   aaggtggatt gtcgtaaacg taatgtttcg ttgttaggtg tattgttttc ggttattaga 214260
   atttttttgg gtaggtcggg gagatttgac gggtaagtgt ttgcggtttg gcgtgagcgg 214320
   gtagggtagg tggtttttgt ttgggtatgt ttttttattt ttgtaataga ttagggagag 214380
   ggttggtttt aggatttagg tttagttagt tgttttataa ttgaggggtt tcggcggagg 214440
   atataagtta gatagagtta tagattaatc gtacgaggtg gttttttttt tttagtttag 214500
   gttttttgaa tgcgttagag tagataagtt tggacgtgag gaggtaagag gtagcgagaa 214560
   taggcgcgga gacggggttg ggggagaaga ggaagaaatt ttgtgtttgt gtttttttta 214620
   cgttcgtttg ttatcgggtt agttgattgg ggaagtgttt tttgagggtg taattttttg 214680
   tttttttcgt aagttagttt tcggggcgtt gggtgagggt gtagttttgt ttattacggc 214740
   gagttagtat attgaattac ggtagaagtt gttttttgtg tgggtgtagt tttttttttt 214800
   gtttaggatt tagaagttac gatagtatag agttttttaa tcgtggttgg tgttttttta 214860
   ggaatttttt gaaagttata gagaaatttt agagtggttg ggtacggtgg tttatgtttg 214920
   tgattttagt attttgggag gttgaggcgg gtggattatt tgaggttcgg agttcgagat 214980
   tagtttggtt agtatggtaa aattttattt ttattaaaaa atataaaaat tagttgggta 215040
   cggtggtata tgtttgtaat tttagttatt taggaggttg aggtatgaga attgtttgaa 215100
   tttgggaggt agaggttgta gtgaatcgag atcgtgttat tgtattttag tttgggtaat 215160
   agagtaagat tttattttaa aaaaataaaa aataaaaaaa gaatttttag agtaattttt 215220
   aggtgttagt aggggtgtga cgtaatttta gtttcgtttt tgtggtttat cggtttcggg 215280
   taagtttttt tgtcgttgtt atttatagag agtttttagg ggagtttggg aatgacggta 215340
   agttttatgt gatttagtat gtaaagggag ttttaaaatt tagaatttag agagttaggg 215400
   cgggtatagt gtagttggtt ttagtgttga ggggttgggg gagagatttt gtattatgtt 215460
   ttgattatag taagggaaga ttatggaggt ttttggaacg ttttagtaat ggcgtaattt 215520
   tagttttttt tatttttttg ttaatgataa ttatgattat gatagtagtt aataggttat 215580
   agagtaggta ttttttagtt tttttaaata tgaattcgtt taatttttat aataatatag 215640
   tattgtttta tttaataaat gaggtaggtt gggcgtggtg gtttatgttt gtaattatag 215700
   tattttggga ggttaaggta ggtagattac gaggttagga gttcgagatt agtttgatta 215760
   atatggtgaa attttatttt tagtaaaaat ataaaaatta gttgggcgta gtggtacgtt 215820
   tttataattt tagttattta ggaggttgag gtaggagaat tatttgaatt tgggaggcgg 215880
   aggttgtagt aagttgagat tatgttattg tattttagtt tgggagatag agtgagattt 215940
   cgttttaaaa ataaataaat aaataaaatt aaaaaatatt atatatataa aaaaataaat 216000
   gaggtaaatg agatgtatga ggtgagaatt ttgtgttata gggagaggcg tagtttggtg 216060
   agatttaatt ttaaggggtt ggcggttttg agtttttaag tgtaattatt ggattatatt 216120
   gtttgtagat tattattatt tttattttat tggaaggtcg ggtgaggtaa tcgggaaagt 216180
   ttggagtagt agtggagttg ttagagagaa tttgttttta gtgttttttt ggattttgta 216240
   gttgttttgt gtgggttttt gttgtttttc gtattggaaa agattttttt atagatgggg 216300
   ttttatgacg ttatttaggt tggagtgtcg tgtttatttt taggcgttgt tatagggttg 216360
   tagttttgaa tttttggttt taagcgattt ttttgtttta gttttttgag tagttttttg 216420
   ggtttaagcg attttcgtgt tttagttttt tgagtagttt tttgggttta agcgattttc 216480
   gtgttttagt tttttgagta gttgggattt taagcgtatt tggttaagtt tttttttatt 216540
   ttttattatt taagattttt ttttttttaa ggtttagttt aattttattt ttatttaaaa 216600
   ttttcggggt atttttgtag tatttggagg ttgaattgtt taggagggaa gggtgttaat 216660
   gagttggtgt ttcgttttta ttttgatttt cggtgagaat gtaagttttt tgtaggtaag 216720
   gattatattt tattattatt tttgtattta tttagatttg gttatgtgtg tttagtatat 216780
   aaagtgtttt cgatgattgt ttatagattt acggtatttg agattgtttt aatatatttt 216840
   tttaaaattt tttttttttt tagaggttta cgaatgttta aattaggttt atcgggaata 216900
   atattatggt tatttcgttt attttgaagt ttatatatag gtttggaagt aataatttcg 216960
   tatagtttag atgaaaaaat aaagattttt ttgttgtttt tggattgtta gagttttggg 217020
   ggatttttaa tattttttgt tgtttttttt aattttatgg tgtcgatggt ttgttggggg 217080
   gttagtttga tttttgcgag tgttcgtatt cggaaaaggg ttggtgttaa gatagtttta 217140
   ggatatttag aatgagggta agaggttatt tttattatta gtttttttta gttttatttt 217200
   atataattgt aatatagtga tttagtatat tttgggttat tgttaagtag ggagtaaatt 217260
   agaaatggtt tgagtttttt ttttttttat ttttagaggg gtttgttttt ttaggtaaat 217320
   ataaggaggt atcgaggttg ttgtataaga gttggttttt gtttatttta taaattttat 217380
   ttttatttat tgtaaaaggt tttgtttttt tttttaaaaa aaaaaaaaaa aaagaaattt 217440
   agtgaggggt ggggtaggtg tgtgtggagg agaggagaaa aggataagtg atggtttttt 217500
   tattttttag taagtcgtag gtgtagagat tttaagtttg ttttagggtt ttttcggtat 217560
   ttgtgtagga gggttttcgt agaaaaaatt taggatttac gatttgggag gatttcggtt 217620
   atttttattg tttggattta ttagtgggag gtaaagtgta agaaattaag ggtaaagggt 217680
   tttttttggg cgggttagag attttttgga aagggttatg tatcgcgcga tttttataat 217740
   aattttaagt atttggagta tgttggggag gaagtttcgg tgttaaaatt aagtatgtgt 217800
   tatttggaag ttcgttttcg tttattagta tattaattta tttacgtttt tgatagagtt 217860
   gtgtttcggg tgcgtagagg gttattttgt tcgtaataat cgggtgaaag ttgaaatttt 217920
   aagttattga tttagaaggg tattttcggg gaagttttga gtaatttttt ttgagataag 217980
   gtattttatt atttattttg gtttttaaat atggaaagtt agaggaaatc gataatggtt 218040
   taaaggtagg gggatggttt cgatgatttt tttattttta attaaggttt atgattttgt 218100
   ttttttatta ataaaaatta atttatggat ttttggtagg tattttttta tttgaatgaa 218160
   ttaaaggagg gatattaaat aaacggtttt tagatttaga agaggagacg ttatttataa 218220
   tttgaggatt gaggaggata aaggattttg tttgtttgga ttttttggag aaaattataa 218280
   aggaaaaaaa aaaggaagga aaagataaat aatgaagatt tggatttttt ttaatttgtt 218340
   tttgtggttt cggtgtgttt atttgtgaaa tggaatttta gagtgttaga gcgggagttt 218400
   tttatgaaat gaaagttttt gaatcggttt atggtgaggt ttaagttagg tttttttcgt 218460
   ttatttaagg cgggttttgt tagggtggag tgaggatgtt agtcgcgggc ggcgttttga 218520
   gcgtaaaacg tgaattcgag tcgttaagtt tgggcgttgt tggacggttt tatttggagg 218580
   tcgtttcgtg ttttattaag tagaggagaa atcgaagaga attcgaagaa aggatgttgc 218640
   ggtttggatt attagtttta gaggttgaat ttaaggatat tttttatttt agtttcgatt 218700
   ttatttattt atatagacgt atagggattt aaagtattcg gtcgatttgg aaagttagcg 218760
   atttttgcga gggtgtaagg ggcgaggaag agggagcgtt ttttagggta gttttttcga 218820
   tattcgagta agggttggag atagcgtagg ggacggtcgg tttaggtcga aagcggttcg 218880
   atagcggttt tgttttggga ggaattcgat ttgtagtgtg ttcgtcgcgg ggattcgttt 218940
   agtttcgggg gcgtttcgtt attttcggtc gtaggtattt aggtgagtag cgttggtttc 219000
   gggatatcga ttttttttta tttcgggcgc gaagtttatt tgagtaagat ttttttatgg 219060
   tgttttcgtg gtcgtcgttt ttaggaaagt gtggcggcga ggtagcgggg cgcgcgtttt 219120
   tagggcgtta attttgggtc ggtagtagga aggcgggaag agggggcggt tcgtcgtttg 219180
   gagtgtatcg atcgggtttc gtttcggttt cgttttcgtt tcgttttttt tcggagatgc 219240
   gtatagaatt tcggtttagt cggttgtggt ttcgtttttg attcgttttt ttttcgtttc 219300
   gtgaacgtgc gcgtcgagtt ttattttcgt ttatttttgc gttgcgttgt atttggcgga 219360
   gcggggttgg cgggcgtcgg gtttgggagg gggcgttttc gcgcggtagc gtagattaga 219420
   ttagattagg ttaggtcggg ggtgggggtc gtttgcgtgg ttcgtcgcgt tcgtagtttt 219480
   gtatttttag tttttcgcgt tggcgttaat ggttggtttc gggagtcgtc gtatttttta 219540
   gtttcgagtt cgggcggggc gggttcggtt aggtggaagc gggcggaggt gcgggcgttt 219600
   cgggcggttt cgtcgggcga ggcggcggcg tcgggtatcg cggcggcgtc gggtatagtc 219660
   gggggtcgga gtcggggtgt cggtttagtg ggtcggggta gggcgggata ggatatttgt 219720
   tttgttattt ttttggtaat taaaagggta gagtgcgtcg attgagttgg agttttagtt 219780
   tttttatttg taaatgggcg cgttggatag atgattattt gttttttttg cgttgggtat 219840
   cgtgcgtagt tcggtgtatc ggtgtagcgg ggtagatata gggagtcgtt ttttataatt 219900
   cgggtaacg 219909
<210> 39
   <211> 1405
   <212> DNA
   <213> Artificial Sequence
<220>
   <223> chemically treated genomic DNA (Homo sapiens)
<400> 39
   cgttattcga gttgtaaagg gcggtttttt gtgtttgttt cgttgtatcg atatatcgag 60
   ttgcgtacgg tgtttagcgt agggagaata aatgattatt tgtttaacgc gtttatttat 120
   aggtgaggaa attaaggttt taatttaatc gacgtatttt gtttttttga ttattagaaa 180
   agtagtagga taggtgtttt gtttcgtttt atttcggttt attaagtcgg tatttcggtt 240
   tcgattttcg gttgtgttcg gcgtcgtcgc ggtgttcggc gtcgtcgttt cgttcggcgg 300
   ggtcgttcgg agcgttcgta ttttcgttcg tttttatttg gtcgggttcg tttcgttcgg 360
   attcgggatt gggaagtgcg gcgattttcg gaattagtta ttggcgttag cgcggggagt 420
   tgggggtgta gagttgcggg cgcggcgggt tacgtaggcg gtttttattt tcggtttggt 480
   ttggtttggt ttggtttgcg ttgtcgcgcg ggggcgtttt tttttaggtt cggcgttcgt 540
   tagtttcgtt tcgttaggtg tagcgtagcg taggggtggg cgggggtggg gttcggcgcg 600
   tacgtttacg gggcggggag ggggcgggtt aggggcggga ttatagtcgg ttgggtcggg 660
   gttttatgcg tattttcggg gaggggcggg gcgggggcgg ggtcggggcg gggttcggtc 720
   ggtgtatttt agacggcggg tcgttttttt ttttcgtttt tttattatcg gtttaggatt 780
   agcgttttgg gagcgcgcgt ttcgttgttt cgtcgttata tttttttggg agcggcggtt 840
   acggaggtat tatgaagaag ttttatttag gtgggtttcg cgttcggggt ggggaggggt 900
   cggtgtttcg ggattagcgt tgtttatttg agtgtttgcg gtcgggagtg gcgaggcgtt 960
   ttcggagttg agcgagtttt cgcggcgggt atattgtagg tcgagttttt tttaggatag 1020
   ggtcgttgtc gggtcgtttt cgatttgagt cgatcgtttt ttgcgttgtt tttagttttt 1080
   gttcgagtgt cggaggggtt gttttggggg acgttttttt tttttcgttt tttgtatttt 1140
   cgtaggaatc gttgattttt taggtcggtc gggtgttttg ggtttttgtg cgtttgtgtg 1200
   ggtgaatggg gtcggggtta ggtggagggg tgtttttggg tttagttttt agggttggtg 1260
   gtttaggtcg tagtattttt ttttcggatt tttttcggtt ttttttttat ttagtggggt 1320
   acgggacggt ttttagatgg gatcgtttag tagcgtttaa atttggcgat tcgggtttac 1380
   gttttgcgtt taggacgtcg ttcgt 1405
<210> 40
   <211> 1405
   <212> DNA
   <213> Artificial Sequence
<220>
   <223> chemically treated genomic DNA (Homo sapiens)
<400> 40
   gcgggcggcg ttttgagcgt aaaacgtgaa ttcgagtcgt taagtttggg cgttgttgga      60
   cggttttatt tggaggtcgt ttcgtgtttt attaagtaga ggagaaatcg aagagaattc     120
   gaagaaagga tgttgcggtt tggattatta gttttagagg ttgaatttaa ggatattttt     180
   ttatttagtt tcgattttat ttatttatat agacgtatag ggatttaaag tattcggtcg     240
   atttggaaag ttagcgattt ttgcgagggt gtaaggggcg aggaagaggg agcgtttttt     300
   agggtagttt tttcgatatt cgagtaaggg ttggagatag cgtaggggac ggtcggttta     360
   ggtcgaaagc ggttcgatag cggttttgtt ttgggaggaa ttcgatttgt agtgtgttcg     420
   tcgcggggat tcgtttagtt tcgggggcgt ttcgttattt tcggtcgtag gtatttaggt     480
   gagtagcgtt ggtttcggga tatcgatttt tttttatttc gggcgcgaag tttatttgag     540
   taagattttt ttatggtgtt ttcgtggtcg tcgtttttag gaaagtgtgg cggcgaggta     600
   gcggggcgcg cgtttttagg gcgttaattt tgggtcggta gtaggaaggc gggaagaggg     660
   ggcggttcgt cgtttggagt gtatcgatcg ggtttcgttt cggtttcgtt ttcgtttcgt     720
   tttttttcgg agatgcgtat agaatttcgg tttagtcggt tgtggtttcg tttttgattc     780
   gttttttttt cgtttcgtga acgtgcgcgt cgagttttat tttcgtttat ttttgcgttg     840
   cgttgtattt ggcggagcgg ggttggcggg cgtcgggttt gggagggggc gttttcgcgc     900
   ggtagcgtag attagattag attaggttag gtcgggggtg ggggtcgttt gcgtggttcg     960
   tcgcgttcgt agttttgtat ttttagtttt tcgcgttggc gttaatggtt ggtttcggga    1020
   gtcgtcgtat tttttagttt cgagttcggg cggggcgggt tcggttaggt ggaagcgggc    1080
   ggaggtgcgg gcgtttcggg cggtttcgtc gggcgaggcg gcggcgtcgg gtatcgcggc    1140
   ggcgtcgggt atagtcgggg gtcggagtcg gggtgtcggt ttagtgggtc ggggtagggc    1200
   gggataggat atttgttttg ttattttttt ggtaattaaa agggtagagt gcgtcgattg    1260
   agttggagtt ttagtttttt tatttgtaaa tgggcgcgtt ggatagatga ttatttgttt    1320
   tttttgcgtt gggtatcgtg cgtagttcgg tgtatcggtg tagcggggta gatataggga    1380
   gtcgtttttt ataattcggg taacg                                          1405
<210> 41
   <211> 1818
   <212> DNA
   <213> Artificial Sequence
<220>
   <223> chemically treated genomic DNA (Homo sapiens)
<400> 41
   cgtcgttcgt ttttggtttt tttgatagtc gtgttttatt ttcgttttgt gttttttttt      60
   tcggatagtg ttttttttag ggtttattta ggagggtgta gcggtggttt tcggggcggt     120
   ggtcgtggtg ggggtgttag ttgtaggggt gttttcggtg ggtgggagtt ggtggttttt     180
   cgttggtgtt atgggattcg tatgttcgtt ttgcgttttt cggtttttga gtttataggt     240
   cgggattttg tttgttagtc gcgtgcgttg tcgtttaatt tttgtaggcg tagagcgcgc     300
   ggcggcggtg atagagaatt ttgtttggtt gtttaaatat agttttttgt agaaggattt     360
   tgcgttcggg gaaggggagg aatttttttt tttttgggcg ttcgtttttt tcgttatggt     420
   tcggttttta tattcgttta tatttggtcg tagcggggcg ttcgggggga ggggttgagg     480
   tcgcgttttt cgtcgttttt tgggcgcggg ttaggcgggg aggagggggg cgtttcggtc     540
   gtgtgtttag gattgttttt tagcggttat tcgggtttta gttttttagg tttggttttg     600
   ataggcgggc ggagtagtta gtgcgagata gggaggtcgg tgcgggtgcg ggaatttgat     660
   tcgttcggga ggcgggggcg gggcgggggc gtagcgcgcg gggaggggtc ggcgttcgtt     720
   tttttttttt atttatttag ttgagttagg gggtttaggg gttttttcgg cggttagttt     780
   tgtattgtag gagcgcgggc gcggcgtttt agttagcgcg tagggttcgg gtttcgtcgg     840
   gggcgttttt tcgtcgttgt ttttcgcgcg attcgttgtt tattagttat tatgtcggat     900
   ttcgcggtta acgcgtagtt ggatgggatt atttcggatt tcgaaggtgg gtgttgggtt     960
   ggttgttgcg gtcgcggacg tgttggagag gattttgcgg gtgggtttgg cgcgggacgg     1020
   gggtgcgttg aggggagacg ggagtgcgtt gaggggagac gggattttta atttaggcgt    1080
   tttttcgttg agagcgtcgc gcgttttcgg tttcgtgttc gcgtcgttta cgtgggggat    1140
   tttgttaggg gtattcgcgt agattttgcg cgtttttata ggattttgtg ttcgttttgc    1200
   gtattgtcgt ttgggttttt ttttttttat tgttgtttgt gtttgttaag cgatagcgat    1260
   tttttcgagg gttcgcgagg ttgtttcgga attttttagg acgtatagtt ttattttggg    1320
   aaatttatcg gttttttttt tttggttttt ttcggcggtt ttcgggtttc gtttggattc    1380
   ggtaacggga tagggaggtc gttttttatt ttcgattgag tggatagtcg cgttttgttc    1440
   gggtggatag tttttttttt ttttacgtta gtttcggggt cgttaagttg tgtagttcgt    1500
   gggtcgggag tatcgaacgg atatagttta ggtcgtggta gggtttagag tgggatgttt    1560
   tatggttttt atttaggttt ggggatattt ttattcgttt tttagaatcg ggtcgtgggg    1620
   gatagaaggg gtttgcgtgc gggtagggag agtattttgg tttttttttg ttttcggggt    1680
   ttataaagtg tgttgggatt tgcggggttg ttttgtttaa gtttgggttt ggcgttcgcg    1740
   tttttgagtt tgtgagtgcg tgcgtttttt tgcgtttttt tgattgtcgg tgttggggtt    1800
   ttgcgttttg cgttcgcg                                                  1818
<210> 42
   <211> 1818
   <212> DNA
   <213> Artificial Sequence
<220>
   <223> chemically treated genomic DNA (Homo sapiens)
<400> 42
   cgcgaacgta aaacgtaaaa ttttaatatc gataattaaa aaaacgtaaa aaaacgtacg      60
   tatttataaa tttaaaaacg cgaacgttaa atttaaattt aaataaaata atttcgtaaa     120
   ttttaatata ttttataaat ttcgaaaata aaaaaaaatt aaaatatttt ttttattcgt     180
   acgtaaattt tttttatttt ttacgattcg attttaaaaa acgaataaaa atatttttaa     240
   atttaaataa aaattataaa atattttatt ttaaatttta ttacgattta aattatattc     300
   gttcgatatt ttcgatttac gaattatata atttaacgat ttcgaaatta acgtaaaaaa     360
   aaaaaaaatt atttattcga ataaaacgcg attatttatt taatcgaaaa taaaaaacga     420
   tttttttatt tcgttatcga atttaaacga aattcgaaaa tcgtcgaaaa aaattaaaaa     480
   aaaaaaatcg ataaattttt taaaataaaa ttatacgttt taaaaaattt cgaaataatt     540
   tcgcgaattt tcgaaaaaat cgttatcgtt taataaatat aaataataat aaaaaaaaaa     600
   aaatttaaac gataatacgt aaaacgaata taaaatttta taaaaacgcg taaaatttac     660
   gcgaatattt ttaataaaat tttttacgta aacgacgcga atacgaaatc gaaaacgcgc     720
   gacgttttta acgaaaaaac gtttaaatta aaaatttcgt ttttttttaa cgtattttcg     780
   ttttttttta acgtattttc gtttcgcgtt aaatttattc gtaaaatttt ttttaatacg     840
   ttcgcgatcg taataattaa tttaatattt attttcgaaa ttcgaaataa ttttatttaa     900
   ttacgcgtta atcgcgaaat tcgatataat aattaataaa taacgaatcg cgcgaaaaat     960
   aacgacgaaa aaacgttttc gacgaaattc gaattttacg cgttaattaa aacgtcgcgt    1020
   tcgcgttttt ataatataaa attaatcgtc gaaaaaattt ttaaattttt taatttaatt    1080
   aaataaataa aaaaaaaaaa cgaacgtcga ttttttttcg cgcgttacgt tttcgtttcg    1140
   ttttcgtttt tcgaacgaat taaattttcg tattcgtatc gattttttta tttcgtatta    1200
   attatttcgt tcgtttatta aaattaaatt taaaaaatta aaattcgaat aatcgttaaa    1260
   aaataatttt aaatatacga tcgaaacgtt tttttttttt tcgtttaatt cgcgtttaaa    1320
   aaacgacgaa aaacgcgatt ttaatttttt ttttcgaacg tttcgttacg attaaatata    1380
   aacgaatata aaaatcgaat tataacgaaa aaaacgaacg tttaaaaaaa aaaaaatttt    1440
   tttttttttt cgaacgtaaa atttttttat aaaaaattat atttaaataa ttaaataaaa    1500
   ttttttatta tcgtcgtcgc gcgttttacg tttataaaaa ttaaacgata acgtacgcga    1560
   ttaataaata aaatttcgat ttataaattt aaaaatcgaa aaacgtaaaa cgaatatacg    1620
   aattttataa tattaacgaa aaattattaa tttttattta tcgaaaatat ttttataatt    1680
   aatattttta ttacgattat cgtttcgaaa attatcgtta tattttttta aataaatttt    1740
   aaaaaaaata ttattcgaaa aaaaaaatat aaaacgaaaa taaaatacga ttattaaaaa    1800
   aattaaaaac gaacgacg                                                  1818
<210> 43
   <211> 2519
   <212> DNA
   <213> Artificial Sequence
<220>
   <223> chemically treated genomic DNA (Homo sapiens)
<400> 43
   gatttatgag tgaatgatta aaagtgtagt tgagttttgg tagagggtat ggggttttat      60
   ttagagatag gtagagaaag ttgaagtttt aggattggag gttgtttttt tttatttttt     120
   tattaggttt aggtagggtt tgatttgaat ggaggtttgg gaatttgtgg ttagttttta     180
   tttgttggaa aagagtagtt tttaagttta attgttttag gttgatgttt ttttattttt     240
   ttttatttat ttatttttat tattattttt tttttattga gatggagttt tattttgttg     300
   ttaggttgga gtgtagtggt gtgattttgg tttattgtta tttttgtttg ttgagtttaa     360
   gttttagttt tttgagtagt tttttgggta gttgggatta taggtgtgtg ttattatgtt     420
   aggttaattt tttgtatttt agtatagatg gagttttatt attttggtta ggatggtttt     480
   gattttttga ttttgtgata tttttgtttt ggtattttaa agtgttggga ttataggtat      540
   gagttatagt gtttggtttt tagttttttt taaaaaatgt tagatttgtt tgttgtgttg      600
   agtggaggtg gggtaggttt ttgtttttta attggtttta tttattgagt tttttttttg     660
   tgttgggttt tgtgttaagt atattatatg ttgtattttg tggttaggtt gttgtggttt     720
   agggttgtat tttggtttaa ggttgtaaat tgaggtggga ttttgatttg gtaattatgt     780
   ttgtggtttg gaaatggtgt tttttgaggt ttaggagagg ttgggtggtg agtggttgtg     840
   gagttgagtg tgggtagtgt ggatgttgtt tatgggggag gtagttaagg atggagggtg     900
   agaggtggtt tttttaaggt tatttttttt tgggttgtaa gtaaaggtta ggggattttg     960
   gaatggttag tgtaggagtt tttttgtgtt ttttatgttt tagattttta gagttttaga    1020
   aatggagatt attgttttta tttttatttt atagatgaag aaattgagtt gaggaaagga    1080
   agtgatttgg ttaaggttgg agagtttatt ttttgtaggg tggggtttgg aatttggggt    1140
   ttggtttttg gtaatgtgtt tttggtttgt agttttttgt tttttgtgtt ttgttttggt    1200
   ttttagtgta gttgtagagg ggtttttttt gatgtatata tttaagagtt tgattgtgtg    1260
   gttgaaattg tggagtttgg agttgtggtt ggttgagtga ttgtggtttt tgggttgtgt    1320
   gtgtgttttt tggagttgaa aggagtgtta ggattggggg tgttgtattg ggttgggttt    1380
   tttaatgttt gtagattggg ttgggttgta gttggagatg gtagtaattt tgggaggttt    1440
   ttgggttttt ttagggtgtg tttaggaggt gggttttgtg tgatgtggtg tagtttattt    1500
   ttttttttga gattgtttaa ttttgtgggg gtagttttgg gtgtttggag atgtggaggt     1560
   ttagattgta gtttttggat gttggaagtt tagatttttt ggggttattg gtttttttgt     1620
   tattttagtt gtagagagtt ttattgtttt attgtttgga gtttagtttt tttgtttttt    1680
   tattagtttt tttttttgta ggtttttggg gatttttgat tgtttgtttt tatttttttt     1740
   ttgtttttat attttttgtt tttgttttag gaatggtgat atagttattg gattgttttt     1800
   tattttttgt tagtttatat tgtatataat ttaataattt gtgttttttt atttgggtga     1860
   tagagatata gataatttga gttagtggtg tttaaagtat tggttttaga atagtagtat    1920
   tagtattttt tgggaatttg ttaaaaatga gaatttgggt tgggtgtggt ggtttatgtt    1980
   tgtaatttta gtattttggg aggttgaggt gggtggatta tgaggttagg agattgagat    2040
   tattttggtt aaaatggtga aattttgttt ttattaaaaa tataaaaaat tagttgggta    2100
   tagtggtggg tgtttgtagt tttagttatt tgggaggttg aggtaggaga atggtgtgaa    2160
   tttgggaggt ggagtttgta gtgagttgag attttgttat tgtattttag tttgggtgat     2220
   agagtgagat tttgttttaa aaaaaaaaaa aatgtgaatt tggggttttt attttagatt    2280
   tattgaatag aaattttgtg gagtttagta gatgattttt atgtatatta aagtttgtga    2340
   gttattgatt taaatatttt tttatttatt tattttttat ttggtttatt tagtattgtt    2400
   agtgggagag atatttgtaa agtattaggt tgtgagtttt attgttgtgt attttgagat    2460
   attgtttatt agttttggga tggtaggtag aggtatttta gtttggttta gtgtagatt     2519
<210> 44
   <211> 2519
   <212> DNA
   <213> Artificial Sequence
<220>
   <223> chemically treated genomic DNA (Homo sapiens)
<400> 44
   ggtttgtatt agattaagtt ggagtatttt tgtttgttat tttaaagtta gtggatagtg     60
   ttttagagtg tatggtggtg gggtttatag tttggtgttt tgtgggtgtt ttttttattg   120
   gtaatgttgg gtgggttagg tgaagaatga atggatgaaa gaatgtttag attagtggtt    180
   tgtaaatttt agtgtgtatg ggaattattt gttgggtttt atagagtttt tgtttagtag    240
   atttggggtg gggtttttaa atttgtattt tttttttttt tgagatggag ttttgttttg    300
   ttgtttaggt tggagtgtag tggtgggatt ttggtttatt gtaagttttg ttttttgggt    360
   ttatgttatt tttttgtttt agttttttaa gtagttggga ttataggtgt ttgttattat    420
   gtttggttaa ttttttgtat ttttagtaga gatggggttt tattgtttta gttgggatgg   480
   ttttgatttt ttgattttgt gatttgtttg ttttggtttt ttaaagtgtt gggattatag    540
   gtgtgagtta ttgtgtttgg tttaaatttt tatttttaat aagtttttaa gagatgttga    600
   tgttgttgtt ttgggattgg tattttgagt attattagtt tagattattt gtgtttttgt    660
   tatttggatg gaagggtgtg gattattgag ttgtgtgtag tgtagattaa taggagatgg    720
   agagtaattt ggtgattgta ttattgtttt tgagataagg gtgggaagta tgggggtagg   780
   gggagagtaa gaataggtgg ttagaagttt ttagagattt gtggagggag ggattggtag    840
   aggaataagg agattaagtt ttggatggtg aagtaatggg attttttgta gttggggtgg   900
   tgggagagtt ggtgatttta gggagtttgg gtttttagta tttggaggtt gtagtttggg    960
   tttttgtgtt tttgagtgtt tgaggttgtt tttgtgaagt taagtggttt tggggggggg   1020
   ggtgggttgt attgtgttgt atggaatttg ttttttggat gtattttgaa gaggtttgga   1080
   gattttttgg gattgttgtt atttttagtt gtagtttggt ttggtttgtg agtgttgagg   1140
   ggtttagttt ggtgtagtgt ttttgatttt ggtgtttttt ttagttttaa ggggtgtgta   1200
   tgtagtttag gaattgtgat tgtttagtta gttgtggttt tgagttttgt gattttagtt   1260
   gtgtggttgg gtttttgagt gtatgtgttg aggggggttt ttttgtagtt gtgttggggg   1320
   ttgaagtggg gtataggggg taggaggttg tgggttgagg gtgtgttgtt gagagttaga   1380
   ttttgggttt taaattttgt tttgtaaaga atgagttttt tgattttggt taagttgttt   1440
   tttttttttg gtttagtttt tttatttgta aaatgggggt ggggatgatg atttttgttt   1500
   ttgaggtttt gaggatttag gatgtggaag gtatagagaa gtttttgtat taattatttt   1560
   gggatttttt gatttttgtt tgtaatttgg aagagggtga ttttggaaga attgtttttt   1620
   gttttttgtt tttggttgtt ttttttatag gtagtatttg tattgtttgt gtttggtttt 1680
   atagttgttt attgtttggt tttttttggg ttttagggga tgttgttttt gggttatggg 1740
   tgtggttgtt ggattggagt tttattttgg tttgtgattt tggattaggg tatgattttg   1800
   gattatagta atttggttgt aggatatagt gtgtgatgtg tttggtatag agtttggtat   1860
   aagggaagag tttggtggat aaggttaatt ggagaatgga ggtttgtttt gtttttattt   1920
   agtgtagtgg atggatttag tgttttttaa aaagaattag gggttgggtg ttgtggttta    1980
   tgtttgtaat tttggtattt tgggatattg aggtgggagt attatgaggt taggagattg   2040
   agattatttt ggttaaagtg gtgaaatttt gtttatatta aaatataaaa aattagtttg   2100
   gtgtggtggt gtatgtttgt agttttagtt atttaggagg ttatttagga ggttgaggtt   2160
   tgaatttagt aggtggaggt ggtagtgagt tgagattgtg ttattgtatt ttaatttggt   2220
   aatagagtaa gattttgttt taataaaaaa aaaataataa taaaaataaa taaataaaaa   2280
   aaagtaggga agtattaatt tggagtaatt gagtttaagg attgtttttt tttaataagt   2340
   aaaggttggt tataggtttt taggtttttg tttagattaa gttttgtttg ggtttggtgg  2400
   ggaggtgagg aagaatggtt tttaattttg ggattttaat tttttttgtt tgtttttggg   2460
   tgggatttta tgttttttgt taggatttag ttgtattttt agttatttat ttataaatt    2519
<210> 45
   <211> 2283
   <212> DNA
   <213> Artificial Sequence
<220>
   <223> chemically treated genomic DNA (Homo sapiens)
<400> 45
   aatgaagatg ttggagattg ggtttttgtt tgtttttttt ttgtgttttg ggatgaggta      60
   gagattgaat agttggtgag taaattaatg gtatttagaa agttatgttg gatttggtgt     120
   ttagtgttta agtgttaatt tgttgaaagt tttttagtga aattttaggg atgatttgga     180
   ttttgttgag aggaattgtt tttgagtgag atggttttag aggtttggag gagtggattg     240
   gtaagtattg ggagggtagt gggagttttg tttttgtttg gtgttttgaa taaggttttt     300
   atggttattt attatgagat tttggaggaa agagagaagg gttttgttgt gggtaatgtg     360
   gttgtgaatt ttggtttgga ttttggtagt tttttagttt gtaggttttg ggtggtgttt     420
   ggagttagtt gaagattttt tgaggtgaat tgggagattg gagagatgtt tgtgaatgat     480
   tgtttggatt gagaggagtt gtgtgggata ttgttttttt gtattgtaat tttggagttg     540
   gtagtggaga atttgttgga gttgtttagt gtggaagtgg tgatttagga tattaatgat     600
   aataattttg ttttttttat ttaggaaatg aaattggaga ttagtgaggt tgtggttttg     660
   gggatgtgtt ttttgtttga gagtgtgtat gattttgatg tgggaagtaa ttttttataa     720
   atttatgagt tgagttgaaa tgaatatttt gtgttttgtg tgtagatgtg ggaggatagt     780
   attaagtatg tggagttggt gttggagtgt gttttggatt gagaatggga gtttagtttt     840
   tagttagtgt tgatggtgtt ggatggaggg attttagttt tttttgttag tttgtttatt     900
   tatattaagg tgttggatgt gaatgataat gtgtttgttt ttaattagtt tttgtattgg     960
   gtgtgtgttt tggaggatgt attttttggt atgtgtgtgg tataagtttt tgtaatggat   1020
   ttggatgaag gttttaatgg tgaaattatt tatttttttg gtagttataa ttgtgttggt   1080
   gtgtggtaat tatttgtttt agattttgta attgggatgt tgataattaa gggttggttg    1140
   gattttgagg atattaaatt ttatgagatt tatatttagg ttaaagataa gggtgttaat    1200
   tttgaaggag tatattgtaa agtgttggtg gaggttgtgg atgtgaatga taatgttttg    1260
   gagattatag ttatttttgt gtatagttta gtatttgagg atgttttttt ggggattgtt    1320
   attgttttgt ttagtgtgat tgatttggat gttggtgaga atgggttggt gatttgtgaa    1380
   gttttattgg gttttttttt tagttttatt ttttttttta agaattattt tattttgaaa    1440
   attagtgtag atttggattg ggagattgtg ttagaatata attttagtat tattgtttga    1500
   gatgttggaa tttttttttt tttagttttt ataatagtgt gtgtttaagt gtttgatatt    1560
   aatgataatt ttttataatt tttttaattt ttttatgatg tttatattga agaaaataat    1620
   ttttttgggg ttttaatatt aaatttaagt gtttgggatt ttgatgtttt gtagaatgtt   1680
   tggttttttt tttttttttt ggagtaagga gttgaaattg ggttagtggg ttgttatttt    1740
   ataataaatt gtgataatgg tatagtgtta tttttagtgt ttttagatta tgaggattgg   1800
   tgggaatttg aattaatagt ttatattagt gatgggggta ttttggtttt agttattaat   1860
   attagtgtga atatatttgt tattgattgt aatgataatg ttttttaggt tttatatttt   1920
   tggttaggtg ggagtttggt ggagatgttg ttttgaggta ttttagttgg ttatttagtg   1980
   ttatgggtgg taggttggga tgtggatgta gggtataatg tttggttttt ttatagtttt   2040
   ttgggatttt ttaattagag tttttttgtt atagggttgt atattggtta aattagtatt   2100
   gtttgtttag tttaagatat agatttattt aggtagattt ttatggtttt gattaaagat   2160
   aatggggagt ttttgttttt tattattgtt atttttattg tgttagtaat tgaggatttt   2220
   tttgaagttt gagttgagtt tttttttggt tttgtttttt gggagtagaa aaaaaatttt    2280
   att                                                                  2283
<210> 46
   <211> 2283
   <212> DNA
   <213> Artificial Sequence
<220>
   <223> chemically treated genomic DNA (Homo sapiens)
<400> 46
   ggtgagattt tttttttgtt tttggggggt agagttagag gggaatttgg tttgggtttt      60
   aggagagttt ttggttattg atatagtgag ggtagtagtg gtggagagtg aaggtttttt     120
   attgtttttg attaagattg tgagagtttg tttgggtgaa tttgtgtttt ggattggatg     180
   ggtagtattg atttgattag tgtgtagttt tatggtaaaa aggttttggt taggggattt     240
   taagagattg taggagagtt aggtattgtg ttttgtattt gtgttttagt ttattatttg     300
   tgatattagg tggttagttg aggtattttg aggtagtatt tttattgagt ttttatttgg     360
   ttgaggatat aggatttggg gggtattgtt attgtgatta gtgataaata tgtttatgtt     420
   gatgttggtg gttaggattg gggtgttttt attgttgata tgagttgtta atttaaattt     480
   ttgttgattt ttatagttta ggggtattaa ggatgatatt atgttattgt tatgatttat     540
   tgtgaaatag tgatttatta gtttggtttt agttttttgt tttaagagaa agaaagaaag     600
   ttgagtattt tgtggggtgt tggggtttta gatatttagg tttagtattg gagttttggg     660
   gaggttgttt tttttaatgt aaatgttgta ggaagattga gaagattgtg gagggttgtt     720
   attgatgttg gatatttgaa tatgtattat tgtaagggtt gagagggaag gggttttggt     780
   gttttgggtg gtgatgttga ggttgtattt tggtatagtt ttttgattta ggtttgtatt     840
   ggtttttaaa gtgaagtaat ttttgaggga agaagtaagg ttgaaaggga gatttggtgg     900
   aattttgtag gttattagtt tgtttttgtt agtatttagg ttagttatat tgagtaaagt     960
   gatgatagtt tttagagggg tatttttggg tattgggttg tatatggagg tgattgtgat    1020
   ttttggggtg ttgttattta tatttataat ttttattaat attttgtaat gtgttttttt    1080
   gggattggtg tttttgtttt tggtttggat gtaaatttta tggagtttgg tgtttttgaa    1140
   gtttagttga tttttgattg ttagtatttt ggttataagg tttaaggtga atagttgttg    1200
   tatgttggtg tggttgtggt tgttgaagga gtaaataatt ttattgttgg ggtttttatt    1260
   tagatttgtt gtaaggattt gtattatgtg tgtgttggag ggtgtatttt ttaggatgtg    1320
   tgtttggtat aaggattggt tgaagatagg tgtattgtta tttgtgttta gtattttgat    1380
   gtgaataggt aggttggtgg agagagttgg ggtttttttg tttaatgttg ttagtattaa    1440
   ttggagatta ggtttttgtt tttggtttag ggtgtgtttt aatattagtt ttgtgtattt    1500
   ggtgttgttt ttttgtgttt gtatgtgaag tgtaaagtat ttattttggt ttagtttata    1560
   ggtttgtaaa gagttgtttt ttatattggg attgtgtgtg tttttgagtg gaaagtgtgt    1620
   ttttggagtt atggttttgt taatttttaa ttttattttt tgggtaggga aagtaggatt    1680
   gttgttgttg atgttttgga ttattatttt tatgttgaat agttttagtg ggttttttat    1740
   tattaatttt agagttatag tgtaagaggg tagtgtttta tatagttttt tttgatttag    1800
   atggttgttt ataaatattt ttttggtttt ttggtttatt ttaaagaatt tttggttagt    1860
   tttagatatt atttggaatt tgtgggttga gaggttattg agatttaaat taaggtttgt    1920
   gattatgttg tttatagtga aatttttttt tttttttttt gggattttat agtgaatgat    1980
   tgtggaagtt ttgtttaagg tattaagtag aagtaaaatt tttattattt ttttggtgtt    2040
   tattagtttg tttttttagg tttttgggat tattttattt aaaagtagtt ttttttagtg    2100
   gggtttagat tgtttttgag attttgttga gaaattttta gtgggttagt gtttgggtgt    2160
   tgggtgttga gtttgatatg gttttttgga tgttgttgat ttgtttgttg gttgtttagt    2220
   ttttgtttta ttttggggtg tagaaagggg atgggtaggg gtttgatttt tagtgttttt    2280
   att                                                                   2283
<210> 47
   <211> 2236
   <212> DNA
   <213> Artificial Sequence
<220>
   <223> chemically treated genomic DNA (Homo sapiens)
<400> 47
   taggtgttaa tgattgtgtt agattggtgt tggaattttt gggttataag gttttagtgg      60
   gtggtgttat tatttttgtg gtttgtaaaa gtggagtggt tttgttaggt gggaagtttt     120
   tttttttttt ttagatttgt gataggttgt aggtaagaat tagtgtaatt agggtgtgtt     180
   tgtatagatt tggaggtggt tgtatgttgt tatttgtttt agaaggtgtt tggtggttgt     240
   tttgtagttt tagagtttag agataagtag aggaaggaag ataggatatt tgggttagtt     300
   gtttgagtta agttgttgta aatgttaata ttgtttgtag tttttttgtt agtatttatg     360
   tttaaaagta atttaagttt gtgtgttagt gggaaatttg gtgtttatga tttgttagtg     420
   tgggttgaaa tttttttttg atgttttttt agttgttttt ttttttttgt gtggttagga     480
   ggggtaaata atatgtagtg gggtggggtg ttttggaagg ggtgtgttag ggagggaatg     540
   atgggggttt taggtaaagt tttgaatttt tttatgtaat gggaggaata tagatatgtt     600
   ttagttaagt gtgtgtggga attgtggaat taaggtattt agaggttaag ggtttgtgtt     660
   aagttttgta ttttgttttg taggggaatt tttatatttt gtgattgaat ttgaaagatt     720
   ttttttggtg gatgaaagtt gttatttttt ggttggttga gttatagttt ttgtagtgtg     780
   tttaggagtg tgttggagat ttggaaattt gtagagattt tttaagttag taggaatttg     840
   gaaattgttg ttttttttaa atggttttaa atatttgtgt tttttttttt atttaattta     900
   attttaattt gtttttttta taggtttaat ttttattttt ttaaaatttt aaattagtaa     960
   gttaaattga agttttagaa taaggtttaa ttaatgattt taatttaata ttaagtttga    1020
   atgtttattt tttggatttg ataaattaga tgtagagtga tgaatggaga ttattttttt    1080
   gggaggtgga atttagttgg gttttagttt gttttgggag aggagtatgt tttttttgag    1140
   ttgtaggtgg gtttggtttt gatgaaatgt tgtgagaatg ttagatagtg tagtttttat    1200
   tgtttgtttg gggtttttgt tgttattaga taagtatttt tatttattta ttttttttta    1260
   ttaattattg ttagattttt tttgtgtttt ttgattttgt tgtaattttt ttttttgggt    1320
   gatgtttgga tagtattagt agtagttgtt tgagggaggg gggtagtttt tgtttgggga    1380
   taggttttaa aaggttgttt atttgttaga gttttatgtt ttttaaatga atttaaatta    1440
   agggtaagtt attatgtatt atgtgatatt tggtagtttt agattagaat aaggttttgt    1500
   attgaatgtg ataattgaat ttttaatgtt aagtgttatt ttttttttag gtaaatgttt    1560
   tgttgttgtg atatttaagg ttggtttgtg ggtgttgttt tttgttttgt tttattttgt    1620
   tttgaagttt tattttaagt taatattaat tttaatatgg aatagtttga tttttttttt    1680
   ttaggttttt ttattgtttt gagagaggat tataagtatt ttttattttt tttagtatag    1740
   tttttttttt ttttttttgt tatagtattg gtgttaggta attttaggta tttttatatt    1800
   gtatttatga tagatttgat gtttattgga gatatgaaaa taattagaaa gttgttatgt    1860
   aaatttaata tgatatttat ttattaggta gagtaatata ataaggtttt tttatttatt    1920
   tttgagtttt atgtttattg tagagaggaa aataatagtg attggtagtt ttgtttagag    1980
   attgtataaa agaggttagg aagttaggtt tggagatgta ttggagagta ttgtgttgtt    2040
   ttttaattag taaaattttt attggatttg tggaatttta aatttagaag tatttatttt    2100
   atgtagtagt tttatttaga gataggaggt ttgttttttg ttttatgtta gttaatggaa    2160
   tagatgggat aagaattttg gatttttggt ttttaggtaa tgttttttgg gatagtggaa    2220
   gtagaaaatt aatttt                                                    2236
<210> 48
   <211> 2236
   <212> DNA
   <213> Artificial Sequence
<220>
   <223> chemically treated genomic DNA (Homo sapiens)
<400> 48
   gaaattgatt ttttattttt attgttttag aaagtattgt ttaagagtta aaaatttaag      60
   gtttttgttt tatttatttt attaattaat atgaagtaaa aaatgagttt tttgtttttg     120
   ggtgaaattg ttatatggaa tgggtgtttt tgggtttgaa gttttataga tttagtaggg     180
   attttgttga ttggaggatg gtatagtatt ttttagtgta tttttaagtt tgattttttg     240
   atttttttta tataattttt gggtaaaatt attagttgtt gttatttttt tttttgtggt    300
   aaatatggag tttaagaatg gatagaaaga ttttattgta ttgttttatt tggtaaataa     360
   atattatgtt gaatttgtat ggtagttttt tgattatttt tatgtttttg ataagtatta     420
   agtttgttat aggtatagta taaaaatgtt tgagattgtt taatattgat attgtggtag     480
   gaagagagga gaagagttgt attggggaaa atgaggagta tttgtaattt ttttttgggg     540
   tagtgagagg gtttgggaag aaaagattga gttgttttgt gttggggtta atgttggttt     600
   gaaatgaaat tttagggtaa aatgaagtag agtaaagagt agtgtttata aattaatttt     660
   aaatattata gtggtggaat atttatttga aagaaaagtg atatttggta ttaaaagttt     720
   aattattatg tttagtgtag agttttgttt tagtttggag ttgttgggtg ttatatggtg     780
   tgtgataatt tgtttttgat ttgggtttat ttgaagagtg tagaatttta ataaataaat     840
   agttttttgg gatttgtttt tggatgagga ttgttttttt tttttgggta attattattg     900
   atgttgttta ggtattgttt aaggggaaag gttgtagtgg ggttggaagg tgtgggagga     960
   gtttggtggt gattgatggg aagggatgaa tgaataaaag tatttgtttg atggtagtag    1020
   agattttgag taaatggtgg aggttatatt gtttggtatt tttgtagtgt tttgttagag    1080
   ttggatttgt ttgtagttta agggaggtgt gttttttttt tagagtaggt tggaatttag    1140
   ttgggttttg ttttttggga aggtggtttt tatttgttgt tttgtatttg gtttgttaga    1200
   tttgagaggt aaatatttgg gtttggtgtt gaattaaaat tattgattga attttatttt    1260
   ggggttttgg tttggtttat tagtttggga ttttaaaaaa ataaaaatta agtttataga    1320
   gagggtaaat taaaattagg ttgggtaaag gaaggagtgt gagtgtttga agttgtttgg    1380
   agggaatagt ggtttttaag tttttgttga tttgagaagt ttttgtgggt ttttgaattt    1440
   ttggtgtatt tttgggtgtg ttgtgggagt tgtagtttag ttagttaggg agtagtggtt    1500
   tttatttgtt gggaggagtt ttttgagttt aattgtgggg tatagaggtt tttttgtggg    1560
   gtaaaatgta gagtttgata taagtttttg gtttttaggt gttttaattt tgtggttttt    1620
   atgtatgttt aattaagatg tgtttgtatt ttttttgtta tgtgaaagag tttggagttt    1680
   tgtttgggat ttttattatt ttttttttgg tatatttttt ttagaatgtt ttgttttatt    1740
   gtatattatt tatttttttt ggttatgtgg gggaagaaaa atagttgaga gggtattagg    1800
   aaggagtttt gatttgtgtt ggtgagttat gagtgttaag ttttttattg gtgtgtaaat    1860
   ttgagttatt tttgagtgtg gatattggtg aagaggttgt gggtggtatt agtgtttgta    1920
   gtgatttggt ttgggtagtt gatttaagtg ttttgttttt ttttttttgt ttgtttttag    1980
   gttttgaaat tgtggagtgg ttattggatg ttttttggag taggtagtag tatgtagttg    2040
   tttttaagtt tgtgtggatg tgttttggtt gtgttggttt ttgtttgtgg tttgttgtgg    2100
   atttggggag aggagagagg ttttttgttt gatagggtta ttttgttttt gtaaattgta    2160
   gagataatga tgttatttat taagatttta tggtttaagg gttttaatgt tagtttggtg    2220
   tggttgttgg tatttg                                                    2236
<210> 49
   <211> 2204
   <212> DNA
   <213> Artificial Sequence
<220>
   <223> chemically treated genomic DNA (Homo sapiens)
<400> 49
   tttttttgta aattggttta agttttggga ggtgggattg ggggaggagg gggtttattt      60
   tggggttagg ttagggttgg gtggaggagg tggtttttga gagagtgagt tgggaaagaa     120
   ggggagtggg taaagggtga gatgtggggt tttggttttt gttttgttgg ttgttagttt     180
   tttttgtgtt ttttagtttg tgtgtgtgat atgagatagt gtagttttgg tatggggttt     240
   ggtatttggt ggttgtgaga taattagggt ttgtgtgatg ggaaatgtgt tgttggtagt     300
   ttgttatttt tttattgtga ttttagtttg ttaaataggg tttgtgatgt ttagtttatt     360
   agggaggagt tgtggttttg agattttgtt tagtttgaag tagtggagat tggttaggag     420
   tttggttgtt agattttgga gtgtatgttg tgggtggggg tttggtttat gggggagggt     480
   taggggatgt gggatttatt tttgaaggag ttggggagtt gttgggtttt ggagttttgt     540
   gtgtgttgtt ttttggttat gttgtttgag atgtagttgt atttttttgt ttttgttgtt     600
   aaatttggag ttgttgggaa tgttttgagg agttagaggt ataagattta gattgttgtt     660
   ttgtttttaa ttgaggtttt gtttttgtga gagttggtta attaatgttt aggattgagg     720
   aggatttatt ttttaatttt tgtttttttg tttttttatg gtgtgattgt tttgtagtta     780
   ttttttgtta gttttgtggg ttgaggggtg tggtttgagg ggtgtggttt gaggggtgag     840
   agtttttaag gttgtgggta tgaggttggg gttttgtatt ggaagggagg gtggaagtta     900
   tgggatgtgg agtagatggt gaggatagtg gggaggtagt agtagaggat gatttgaggt     960
   aaaagtgtaa gtgaagaaaa gttgttttgt tgggtagagg tatgggatta aggtgttttt    1020
   tttgggttga ggagttagag ggaagaaaat ggtgttttgt tatgagggtg tgaggtttga    1080
   gttgagggga gaagattgag ggatatagaa gtggttgaag tgagggtgtt gaggttagtt    1140
   tgagttgaga gggttggtag attatgttgt agttgaagtg aatgaatttg agtgaggtgt    1200
   gttaagaaaa ttaaaatgta taaatttgga aaaggagagg aggttgggtg tggtggttta    1260
   tgtttgtaat tttagtatgt tggaaggttg aggtgggtag attatttgag gttagaagtt    1320
   tgagattagt ttggttaata tggtgaaatt ttgtttttgt taaaaatata aaaattagtt    1380
   gggtgtgtgg tgtgtgtttg taattttagt tatttgggag gttgaggtag gagaattgtt    1440
   tgaatttggg aggtagaggt tgtagtgagt tgagatggtt tgggtgatag agtgagattt    1500
   tgttttaaaa aaataaaata aaaaataaaa aaataggaga ggagatttta ttttttgtaa    1560
   ggggttatag tttgtaaggt ggttattttg taggttggag aatggttagg attagagata    1620
   ggtattttgt tggaggaggg gttggagtag tggttttatg ttgaatagtt tgggtagata    1680
   tgtatattta gtaggtgata ggaggagtta tgaatattta tgagggtggt tttgatgtat    1740
   gtttattgag taaatatgta tgtaatatat aatttatgtt tattttggag tgaagagtta    1800
   ttaattaaat gttaaatgtt tggtttgtat ggtgatgttt ttatttttat aaaaagtaaa    1860
   aaaaaaatag ttaggtgggg tggtgtgtgt ttgtggtttt aattagttag gaggttgggg    1920
   taggaggatt atttgagttt aagagttgga ggttgtagtg atgtatgatt agattattgt    1980
   attttagttt gatggatttt aaaaaataat aataatatat gtattatagt tagattttat    2040
   gtgttaaaag gtttttttag gataggaagg tgtgtaagta tgtagtttgt gtaaattagt    2100
   tagagttagt ttatggttgg tagttttttt atgaggagaa agttattgaa attagttttt    2160
   tgtttaatta aagttgtaat gatggttggt ggaatagggg tttt                      2204
<210> 50
   <211> 2204
   <212> DNA
   <213> Artificial Sequence
<220>
   <223> chemically treated genomic DNA (Homo sapiens)
<400> 50
   ggaattttta ttttattagt tattattata gttttaattg gataagaggt tgattttagt      60
   aatttttttt ttataggaaa attattgatt atggattggt tttggttagt ttatatagat     120
   tgtatatttg tatatttttt tgttttgaaa agattttttg atgtatgggg tttaattgta     180
   atatatgtat tgttattatt ttttagagtt tattaggttg gagtgtagtg atttgattat     240
   atattattgt agtttttaat ttttgggttt aagtgatttt tttgttttag ttttttgatt     300
   agttgggatt gtaagtatat attattttat ttggttattt tttttttatt ttttgtagag     360
   atggggatgt tattatgtag gttaggtatt taatatttaa ttggtaattt tttattttaa     420
   aatgaatatg ggttgtatgt tatatgtatg tttgtttaat gggtatgtgt taggattatt     480
   tttatgaata tttatagttt tttttgttat ttgttgaata tgtatgttta tttaagttgt     540
   ttagtataaa gttgttattt taattttttt tttaatgaaa tgtttgtttt tggttttggt     600
   tattttttag tttgtggaat ggttattttg taggttgtaa ttttttataa gaaataaagt     660
   tttttttttt gtttttttgt tttttgtttt gtttttttga gatggagttt tgttttgtta     720
   tttaggttat tttggtttat tgtaattttt gttttttaag tttaagtgat ttttttgttt     780
   tagtttttta agtagttggg attataggtg tgtgttatat gtttggttaa tttttgtatt     840
   tttagtagag atggggtttt attatgttgg ttaagttggt tttgaatttt taattttaga     900
   tgatttattt gttttggttt tttaatgtgt tgggattata ggtgtgagtt attgtgtttg     960
   gttttttttt tttttttaaa tttatatatt ttgatttttt taatatattt tatttgagtt    1020
   tgtttatttt ggttgtggtg tgatttgttg gtttttttag tttaggttga ttttagtgtt    1080
   tttattttgg ttatttttgt gttttttagt tttttttttt taatttggat tttgtgtttt    1140
   tatgataggg tgttattttt ttttttttaa ttttttagtt tagaggaggt gttttggttt    1200
   tgtatttttg tttggtaagg tgattttttt ttatttgtat ttttgttttg ggttattttt    1260
   tgttgttatt tttttattat ttttattatt tgttttatgt tttgtggttt ttgttttttt    1320
   ttttggtgta gggttttggt tttatattta tagttttggg ggtttttgtt ttttaggtta    1380
   tgttttttag gttatgtttt ttagtttata gaattagtgg gaagtgattg tgaagtagtt    1440
   gtgttgtgga gggataggag ggtggggatt gggaggtggg ttttttttga ttttgggtgt    1500
   tgattggtta atttttatga gggtgggatt ttgattgggg gtggggtggt aatttgggtt    1560
   ttgtgttttt ggttttttag ggtatttttg gtggttttgg gtttggtaat gaggatgggg    1620
   gagtgtgatt gtgttttggg tagtatggtt gagaagtggt atatatggga ttttgaagtt    1680
   tagtggtttt ttgatttttt taagggtgag ttttgtgttt tttgattttt ttttgtggat    1740
   tgagtttttg tttgtagtgt gtgttttgag gtttgatagt tgggtttttg gttagttttt    1800
   gttgttttgg gttgggtgag attttaaagt tgtggttttt ttttagtaaa ttgagtatta    1860
   tgaattttgt ttggtagatt gaggttatga tggaggggtg gtgggttgtt aatggtatgt    1920
   tttttattgt atgggttttg gttattttgt ggttgttaga tgttaagttt tatgttaggg    1980
   ttgtattatt ttgtgttatg tatgtggatt aggaaatgta gagggaattg gtagttagtg    2040
   aggtggggat tgggatttta tattttattt tttgtttatt tttttttttt tttgatttat    2100
   ttttttgaag gttgtttttt ttatttagtt ttggtttggt tttagggtga attttttttt    2160
   tttttaattt tattttttga aatttagatt gatttgtaga agga                     2204
<210> 51
   <211> 2137
   <212> DNA
   <213> Artificial Sequence
<220>
   <223> chemically treated genomic DNA (Homo sapiens)
<400> 51
   ttttattagt tagtatttaa ttatttaaaa gtgtggttta tgtttggaat tgggttataa      60
   agatggtaat gtgttaaatt agatatttgt tttttgtata tttttataaa ttatattagt     120
   ttattttata attttgtttt tgttagttat aaagaagaaa gtttaatatt ttagatgttt     180
   atattttatt aaattgtggg tgaagagtta gtgagtggta aaaggtggtt gttttgagtt     240
   gtggtgatgg aatttttagg gaatagttag gagtttggaa agttattttt gagttggttt     300
   atttaagtat ttgggataga agtggttagt agaaattttt tatggaattt taaaatatta     360
   ttaggatagt agttttatga tatttttaaa atttggagtt taaaaaataa tgaagtataa     420
   tttatttaga atttaagtat taagggaaaa tgttaagttt tgttttattt taaagatttt     480
   tttttgagga aaatatgtta ttgtttttta gatgtgaatg aaatgatatt tttttttttg     540
   atatagtttt tttgtttatg attttatatt gttttttttt aatttttttt attattgaaa     600
   tattatatgt aagatttgtt ggtatgtttt ttgatttttt atgtttaagg ggaaaaatgt     660
   ttgtagttga tgtttgagtt ttaggagttt ggtgaagaaa aaaaaaaaaa gtaatgtaag     720
   gaaaattgtt ttaatggtat atttttttta aagtttgaag ttttataggt aaaaggaggg     780
   ataaatattt ttttttgaaa gtaaatagtt aaggtttttt tttatttaat tatttttgaa     840
   ttttttgtaa tttttttgag gtttaaattt agggtataat taattttaga ttttatttta     900
   aatttaattt gttttttatt tgtaatttgt ttgtaggtgg gttttttttt tttaaattat     960
   aaatttgaat ttgtgttaat tttttataat ataaggtatt taaagttttt tggagttatt    1020
   gatttggtga tgttgtagtg tatgtttgta tttgaaagta agtaagagaa tagaaagtga    1080
   gtaagagaat tgtaattttt attattttgg tgaatttttg ttgggattta tatattaaat    1140
   agtatttgta tatagatgtt ttaagttgtt ttttttagag ttgttattta gatttgtaag    1200
   gggtggggga ttagtggaaa tttaaataga aatttttatt attataaagg tgagttttta    1260
   ttgattatgg taattgtttt gtttaggttg tatgggtatg gttaattttg tttgaaagtt    1320
   tgtttttttt ttgtgttttt ttgttgagtt ttttttaggt tttggttttt tttgatggtg    1380
   gtaagggatg ttttttatgt tttttattat ttttgtttgt gtttgtgttt tttgtttgag    1440
   gttttaggtt gtggtggtgg tgtggtgggt ttattagtgg gtgtgttggg tttgtgtagt    1500
   gttgtttgta gttgtgttgt tgtatttatt attattatat tagtgtgggg aagtttatgt    1560
   tgagtttttt tggttttggt gtgttttagt tagtgtgtta gtaaatagaa tatgttggat    1620
   tgtttagaga tggttttggg tgtttggggg tagaaggggt gtttttaggt ttggttttga    1680
   tttgtatttt tgaagttggt tttgggagat aatgtatttt tgttagttag gttgtgggaa    1740
   aagtgaaaaa agggttgggg gttatgtggg ggtggggagg tgagttttag tgttagtagt    1800
   gtttggtagt ttttttttag tgtagagaag tggtttgtat taaggtgtat tagagtggtt    1860
   attttgtttt ttggaatatt tatgggtatt tgtaaatttt atagttggtt tagttaaatt    1920
   tttagtaaat attggtaagg gttggttttg gttgtgttta gagggttttt ttaattggtt    1980
   gaatttaggg agttgtaatt ttttaggtat tttgtaatgt aattattttg agtgttgtat    2040
   tataataaaa tattggggat tttttgaaaa gtttttataa aattaatagg tgtgtgtttt    2100
   gtttataaaa tgttgaattt attttttatg aatttgg                             2137
<210> 52
   <211> 2137
   <212> DNA
   <213> Artificial Sequence
<220>
   <223> chemically treated genomic DNA (Homo sapiens)
<400> 52
   ttaggtttat agaaggtgaa tttgatgttt tgtgagtgaa gtatatattt attggttttg      60
   tgaaggtttt ttaagggatt tttaatgttt tattataatg tgatatttag ggtggttgta     120
   ttgtaaagta tttggaaggt tgtagttttt tgaatttagt tagttaaaag agttttttga     180
   atgtaattag ggttggtttt tgttagtgtt tgttggaaat ttggttgggt taattgtaga     240
   gtttgtaaat gtttgtgaat attttgagga gtagggtgat tattttgata tgttttgata     300
   tagattgttt ttttgtattg gagggagatt gttaggtgtt gttggtgttg gagtttattt     360
   ttttattttt atatggtttt tagttttttt tttatttttt ttgtggtttg gttggtggag     420
   atgtattgtt ttttaaagtt agttttagaa atgtggatta aagttgggtt tgggagtatt     480
   ttttttgttt ttagatattt agagttgttt ttgagtaatt tagtatgttt tgtttgttgg     540
   tgtgttgatt ggggtgtgtt ggggttggga gaatttggtg tggatttttt tgtgttgatg     600
   taatgatgat ggatgtggtg atgtggttgt agatagtgtt gtgtgggttt ggtgtgtttg     660
   ttaatgaatt tattgtgttg ttgttgtggt ttgggatttt gggtagggga tgtaggtgtg     720
   ggtaaaagtg gtgaaaggtg tgaaaaatat tttttgttat tgttggagga aattagaatt     780
   tgaagggaat ttgataggaa agtgtagaga aagagtgagt ttttaaataa aattggttat     840
   atttgtataa tttgagtaaa ataattatta taattaatgg ggatttgttt ttgtaatggt     900
   gaaagttttt gtttgggttt ttattggttt tttgtttttt gtaggtttaa gtgatgattt     960
   taaagaggat gatttgaggt gtttgtgtgt aagtgttgtt taatatgtag gttttggtaa    1020
   gaatttgtta aggtggtaaa ggttgtggtt tttttgtttg ttttttgttt ttttgtttat    1080
   ttttaaatgt agatgtgtat tatagtgtta ttaggttagt ggttttagga agttttaaat    1140
   gttttgtgtt gtaagaggtt gatgtaggtt tagatttata atttagaaag aaaagattta    1200
   tttgtaggta ggttgtaaat aggagatgga ttagatttaa aatagggttt ggaattaatt    1260
   gtgttttggg tttaggtttt ggggaaatta taaaaggttt agggatagtt gagtgggaaa    1320
   aagttttgat tgtttgtttt taggaaggaa tgtttatttt tttttttatt tatgaaattt    1380
   taagttttag gaaggatata ttattagagt aatttttttt gtgttgtttt tttttttttt    1440
   ttttattgag tttttgggat ttaaatatta gttataaata tttttttttt taagtatgaa    1500
   aagttaaggg gtatgttagt aaattttgta tgtaatgttt tagtggtgaa agggattgaa    1560
   ggagaatagt gtggagttgt aagtaaggaa attatgttgg gggaaaaagt attattttat    1620
   ttatatttga aaaatagtgg tatgtttttt ttagggaaaa gtttttgaag tgaagtagaa    1680
   tttaatgttt ttttttgatg tttaagtttt aaataaatta tattttatta ttttttaaat    1740
   tttaggtttt aaaagtgttg tggagttatt gttttagtgg tgttttgaga ttttatgagg    1800
   aatttttatt gattattttt attttgaatg tttgaatgga ttagtttagg agtgattttt    1860
   taagtttttg attatttttt gaaaatttta ttattataat ttaaagtggt tattttttgt  1920
   tgtttattga ttttttattt atagtttgat gaggtgtggg tgtttagaat gttaaatttt   1980
   tttttttata attgataggg ataaagttgt aaaataagtt gatataattt ataggagtgt    2040
   ataggaagta gatgtttgat ttggtatatt gttattttta tagtttagtt ttaagtataa    2100
   attatatttt taaataatta ggtattaatt aatgaga                             2137
<210> 53
   <211> 2461
   <212> DNA
   <213> Artificial Sequence
<220>
   <223> chemically treated genomic DNA (Homo sapiens)
<400> 53
   tttttttttt tttttttttt tttttttttt tttttttttt tttttttttt tttttttttt       60
   tttttttttt tttttttttt ttttgttttt tttttttttt ttttttttgt tttttatttt      120
   tttagtttga aggttgtatt ttttttattt ttttaagtaa agttgggagt tttgtagtat      180
   tttaggagtg aggataatta tgtttaagaa attttggttt tagtttagtt ttagattaag      240
   aggaaaatat tttaaaattt taaagtaaat tttaaaagtg taatttatat tagagtgatt      300
   gatttggtat ttatgtagat tgaagtttga gaagtaattt aagtggtttt tatttatttt      360
   tttggaagag tttttagaat gtatttttat tggtttgaaa aatgtttgaa aaagagtttt      420
   gatgttaata gtaatggaaa taggagggtt ggtggtttat gattagttta gtggtatttt      480
   ttattttatt tgtatttagg taaagagatt gtggtttgta tgtgattaaa taatttgttg      540
   aagtgttttg ggtttagggt atttaatttt tgtgaagtag gattattagt tgtttgtttt      600
   gtttttgtgt tattttttag aatatattaa agtaggggta atttttgagt ttagattttt      660
   gttatgtgaa gattttttga aaattttgat aaatataggt attgtgtaag tggttagtta      720
   attaaagagg ggaggtggag ttgttttttg tgtatttaat aagtatttat tattttattt      780
   gagtatgaaa agaggttatt gttattattt ttaagaagga aagtaagtag gatagtttat      840
   atttttagaa taatttttta ttaaatggaa taattttggt gaaaagtggg agtgaggaag      900
   aaagaaaaaa aaaattttta attataatgt ttgggaataa gaaaggaaga agaaatttat      960
   gttaaagttg attttttttt gtagttgtaa aataaatttt taagattttt tttgttgaaa     1020
   ttttggagag gaaaattgga gtggtgggtg ggttttgttt gtagtttaat ttttttttgt     1080
   ggtgtgggtg tggttgggtt tagtattttg gaaagtgttt tttgtggtgt tttgggatta     1140
   tgtatgtttt ttggggtttg ttgttttggt tgtgtaagtg ttattgtaat tggtgagagt     1200
   tgttggtaat ttatttggag ttgataattg tggagagatg tagatattta ttgattttta     1260
   ggaagttgag tgtggtggat ggaattttat gatttttttt tttttaagga tggaaatttt     1320
   atttaagtag ttttagagga aatggataaa ggtatttgaa agggagtgag tggttttaaa     1380
   ttgtataatt gagtggttgg gggagttatg tgttagtgtt ttagtgattg tgggatatgg     1440
   agaggggaag tttgtgttgt atataaggat ttagggattt tgagtttggt ttgagaattt     1500
   ttggatgttg agtgtttgtt ttatgggttg tattttttaa ttttgtttta aagtagttgt     1560
   tgagtttaat ttttgtgttt agggtgtttg ttgtgtgtta ggatgtgttt agtatttagt     1620
   ttttgggttt tttttttagt agttgttttg aaagttttta tgtatgtttt gtaggttagt     1680
   ttggtaataa aattggggta aattgtgtta ttttaggttt tgttttttag aatatgattt     1740
   agaggtattt gtgtatgtag atggttgatg tagttatgat agttattatg aataaggtag     1800
   taggtgggga taagttagta gaatttttta gtttggtttt ggattttttg gaggtggtta     1860
   atatgagtgg taatgtgttg ttgtagtttt tggatttgtg gtgggagttg gggttggagt     1920
   tgttggatgg tgtgttgtta ggatattttt tgggtagtgg tggggtagag agtgtggata     1980
   tagaggtttg ggtgtggatt tttattagtg tggtgtattg ggtggtgtgt gttttggggt     2040
   tggtgggtaa tttgttggtt ttttatttga tgaagagtat gtagggttgg tgtaagtttt     2100
   ttattaattt ttttgttatt aatttggtgt tgatggattt ttagtttgtg tttattttgt     2160
   ttttttgggt ggtggagaat gtttttgatt ttaaatggtt ttttggtaag gttatgtgta     2220
   agattgtgtt tatggtgatg tttatgaata tgtatgttag tgtgtttttt tttattgtta     2280
   tgagtgtgat gtgttattat ttggtggttt tggttttgaa gagttattgg atttgaggat     2340
   atggttgggg tgattgttgt ggttggagtt tgggggatag ttgttgtttt ttggttaagg     2400
   tgttgtgtgt gtggatttgg gttttggttg tgttggtttt gttgtttagt gttatttttt     2460
   t                                                                     2461
<210> 54
   <211> 2461
   <212> DNA
   <213> Artificial Sequence
<220>
   <223> chemically treated genomic DNA (Homo sapiens)
<400> 54
   gagaaaatgg tattgggtag tgaggttagt gtggttaaag tttagattta tatatatagt      60
   gttttggttg agaagtagta gttgtttttt aggttttggt tgtagtagtt gttttggttg     120
   tgtttttggg tttggtggtt ttttagagtt gaggttattg aatggtagtg tgttatattt     180
   atggtagtga ggaagaatat gttggtgtat atgtttatgg atgttattat ggatatgatt     240
   ttatatatgg ttttgttgaa gggttatttg aagttaagag tgttttttat tgtttagaag     300
   ggtagggtga gtataaattg aaagtttgtt agtgttaggt tggtgatgaa gaggttgata     360
   gaggatttgt gttagttttg tatgtttttt attaggtaga gaattagtag gttgtttgtt     420
   aattttaggg tgtatattat ttagtatatt atgttgatga gaatttgtat ttgggttttt     480
   gtgtttgtgt tttttgtttt gttgttgttt gggggatgtt ttggtggtgt gttgtttggt     540
   aattttagtt ttagttttta ttataagttt ggaagttgta gtgatgtgtt attatttgtg     600
   ttggttgttt ttagaaggtt tgggattaga ttgaagagtt ttgttagttt gtttttgttt     660
   gttgttttat ttatggtggt tattgtggtt gtattggtta tttgtatgtg taggtatttt     720
   taggttatgt tttgggggat aagatttaag ataatatggt ttattttagt tttgttgtta     780
   ggttagtttg tgggatgtgt gtgggagttt ttaaagtagt tattgaagag agagtttagg     840
   aattgggtat taagtgtgtt ttggtgtgta gtgaatgttt tggatgtagg agttgagttt     900
   agtggttgtt ttggagtaga gttgaggagt gtagtttgta aggtaagtat ttggtgttta     960
   agggttttta ggttaagttt ggagttttta ggtttttatg tataatgtag attttttttt    1020
   tttgtgtttt gtggttattg gggtattggt gtataatttt tttagttgtt taattgtgtg    1080
   atttggggtt gtttgttttt ttttaaatat ttttatttgt tttttttggg attgtttgga    1140
   tgaggttttt gtttttggag agaaagagat tgtagagttt tatttattat gtttagtttt    1200
   ttggaggtta gtgggtgttt gtgttttttt gtggttgtta attttgggtg ggttgttagt    1260
   ggtttttatt agttatggtg gtatttgtat ggttaaggtg gtgggtttta aggagtatgt    1320
   gtaattttgg ggtgttgtga ggggtgtttt ttgaggtgtt gaatttagtt gtgtttgtgt    1380
   tgtgagggag agttgagttg taggtaaagt ttatttgtta ttttagtttt tttttttaga    1440
   gttttagtag gaagggtttt aagagtttat tttatagttg taggagaaag ttggttttga    1500
   tgtgagtttt tttttttttt tttattttta aatattatga ttagaagttt tttttttttt    1560
   ttttttttat ttttattttt tattggaatt attttatttg gtgaggaatt gttttaaaaa    1620
   tatgagttat tttgtttatt tttttttttg aaagtaataa tagtggtttt tttttatgtt    1680
   taaatgagat aatgggtatt tattggatat ataaaggata gttttatttt ttttttttaa    1740
   ttagttgatt atttatgtag tatttgtgtt tgttaggatt tttaaagggt ttttatatgg    1800
   tagaagtttg ggtttaaggg ttgtttttat tttaatgtat tttgagaggt gatataaaaa    1860
   tagagtaaat aattgatggt tttattttat agaaattaaa tgttttaagt ttaaagtgtt    1920
   ttaataaatt gtttgattat atataggtta tagttttttt atttagatgt aaatagaata    1980
   gaggatatta ttgggttgat tgtgaattat tagttttttt atttttatta ttgttagtat    2040
   taggattttt ttttaggtgt tttttagatt aatggaagta tattttaagg atttttttaa    2100
   gggaatgagt ggaaattatt tgaattattt tttaagtttt agtttatata aatgttaggt    2160
   taattgtttt ggtgtaaatt atatttttag gatttatttt aaaattttga aatgtttttt    2220
   ttttggtttg ggattaaatt gaaattaaag ttttttaagt ataattgttt ttatttttgg    2280
   agtattatag aatttttagt tttatttaag gagataaaga ggatgtaatt tttaagttga    2340
   ggagataaga aatagaaaaa gaaagaaaga gaaaaatgaa aaagaaagaa agaaagaaag    2400
   aaagaaagaa agaaagaaag aaagaaaaga aaagaaagag aaaaaggaaa aaagaaaaaa    2460
   a                                                                    2461
<210> 55
   <211> 1920
   <212> DNA
   <213> Artificial Sequence
<220>
   <223> chemically treated genomic DNA (Homo sapiens)
<400> 55
   ttaggttgtt gtagatatag tttttgtttt tttgaaaaat atgttttagg tgttgggatt      60
   ttagatattt gggaaataga gtgtatgtag ttgttgagag gttttgtgtt tggttttttt     120
   tattattgag gtgtagaggt gttgtggata gtttagattt atatggtgtt tgaggtgaaa     180
   tagaattttt agttttttta tgaggttatt ggtatttttg gttgttttta gagttttttg    240
   atttagagtt gaatgtaaag taagtgtttg aaatgtagaa gtagttgggg ttgtttatgg     300
   tatttgtttt gtttggggtg agagaagatg ttaggttgag gttttagtga ttttaggtat     360
   tagttttgaa ggagggtggg gagattgtaa aggggaagtg tttggagggt taatggtttt     420
   tgtgtatttt gtgttttttt gaagtgtgtt gtttttttgt gttggggatt gggatttgtt    480
   tttggggaat ttgtttagaa gatggtggtg gattggggtt gggtattttt tagggttgtt     540
   aggttttttt tagttttgta tttgttgtgt tgttttattt tgttaggaag ttttagagat     600
   tttggggatg gggtgggagt gtttttttat tgtgggttta aaaagaagga aggatgtttt    660
   taggggttgt agaaggagga ttagttttaa gttataattt tttttggatt taaggtaggt     720
   tggttggggt tttgtgttta tatggttttt ggtgggggtt tgtgtgtttt gggagttttg     780
   tggtttgggg aggaaagagg agataagaga taggtgagga ttatggggtt gatttagttg     840
   gggtagggat tattgtggaa aaattttggt gaggtggggg gatgtggaaa gagagtggtt     900
   tgtgttttgt attttgtgtt gggtattttg tgttagtgtt ttgtttttag tgttttgtgt     960
   tttgtgtttt gtgttttgtt tttattttgg gttagttgta ttgtgtttgt gttgtaggaa    1020
   ttgtggagtt ggaaagtggg ggtgttgtgg ttggggggtt gttttagttg tgttttggtt   1080
   agtgattggt gggttgggtt taaatttagt taggttgggt aggtggtggt tgtgtgattg    1140
   gggattgggt gttttgtttt ttttgttttt tttttttttt tttttttttt ttttagtttt    1200
   ttggtttttt ttagttttta ttggatttgt ttgtttgttg tttttttttt ttttttgttt   1260
   tttatattat tttttatttt tttgttttgt ttttgttttt tttttttttt ttgttttttg   1320
   tttttttttt tttttttttt ttttttaggg gtggagtttt tttttttttt tttagataat   1380
   gttgtggttg tgtttttttt tttgttagtt tgtttaggtt tttgttgtta gtgatttttt    1440
   tgggttgggg gtggggaggt ggggggggag tgtagggttg gggaggatga gttggttttt   1500
   tttatttttt tgttgttgtt ttttttaaga gggatggaga tttggtttaa gttttttggt   1560
   ttatttggag ttgtgatagt tatttttagg gaatagttat gttgttttat taagtttatt    1620
   tttagtggtt tggatttttt aggtagaggt tgtgggattt tgtttttttt aatattttag    1680
   tttattttta aaagggtttg agttggatag gggttaaata ggtttttttg atttggtggg    1740
   ttggttagat gtgatagtaa tgttaaggag gttaagtttt tttgtttatt ttttattttt    1800
   ttttttttta tttttggatt ttttggtgtt tttagtatat agaggttttt gagtagtttg     1860
   gttgtaggtt ttttatttat ttagagtttt tttttttatg tgtttatttt taattttgta     1920
<210> 56
   <211> 1920
   <212> DNA
   <213> Artificial Sequence
<220>
   <223> chemically treated genomic DNA (Homo sapiens)
<400> 56
   tgtagggttg gggataggta tgtgaggggg agaattttga gtagataggg aatttgtagt      60
   tgggttgttt aagggttttt gtgtattggg ggtgttagga ggtttaggga tggaaaaggg     120
   ggaggtgaga aatggataaa gaaatttggt ttttttggta ttgttgttat gtttggttgg     180
   tttgttaagt tgaaggggtt tgtttagttt ttgtttggtt taaatttttt tgggaataag     240
   ttgggatgtt agaaaaaata aaattttata atttttgttt ggggaattta ggttgttgga     300
   ggtgggtttg gtagggtagt gtgattgttt tttgggagtg gttgttatag ttttgggtga     360
   attgaggagt ttgggttaag tttttatttt ttttggagag ggtagtagta aggaggtgag     420
   gggagttagt ttattttttt taattttgta tttttttttt atttttttat ttttagtttg    480
   gaagaattgt tggtggtggg agtttggatg agttggtggg gaaggggatg tagttatagt     540
   attgtttggg agggagggga gaagttttgt ttttagaggg gaggggagaa gaggaggggg    600
   taggagataa ggggaggaag aaaggtgaag gtaaggtgaa ggggtggaga gtgatatgaa     660
   gagtgagaga aaagagagga tagtggatga gtagatttgg taggggttga aaaaggttaa     720
   gggttggagg ggagggagag gaaggaggag gggagtgagg agggtggggt gtttggtttt      780
   tagttgtgtg gttattgttt gtttagtttg gttggatttg agtttggttt gttgattgtt      840
   ggttgaggtg tagttgaagt agttttttag ttgtggtgtt tttatttttt aattttatgg      900
   tttttgtggt gtgggtgtga tgtagttggt ttggggtgaa ggtaaggtgt ggggtgtggg      960
   gtgtggggta ttgggagtga ggtattggtg tgggatgttt ggtgtaaggt gtagggtgtg     1020
   ggttgttttt tttttgtgtt tttttatttt gttaaagttt ttttatgatg gtttttattt     1080
   tggttgggtt agttttgtaa tttttgtttg ttttttgttt tttttttttt ttttgagttg     1140
   tggggttttt ggggtgtgtg gatttttgtt aggggttgtg taggtgtgga gttttagttg     1200
   gtttgttttg ggtttgaaga aagttgtggt ttggagttag tttttttttt atgatttttg     1260
   ggggtgtttt tttttttttt tgagtttgtg atgggaaggt gtttttattt tatttttggg     1320
   gtttttgaga ttttttggtg aggtggggtg gtgtggtagg tgtagggttg gggagggttt     1380
   gatagttttg gagagtgttt gattttagtt tgttgttgtt ttttaggtgg attttttaga     1440
   ggtaggtttt agtttttggt gtggggaggt ggtgtgtttt agaggggtgt agggtgtgtg     1500
   ggggttgttg gttttttggg tatttttttt ttgtggtttt tttgtttttt tttggagttg     1560
   gtgtttgagg ttgttgggat tttagtttgg tgtttttttt tgttttgagt gaggtaggtg     1620
   ttgtgggtgg ttttggttat ttttgtattt tgagtgttta ttttgtattt agttttaagt     1680
   tggagagttt tggggatagt tgagagtgtt agtggtttta tagggagatt gagggttttg     1740
   ttttattttg ggtgttgtgt gggtttgggt tgtttatagt atttttgtgt tttagtgata     1800
   ggagaagtta agtgtgaggt tttttaatag ttgtgtgtat tttatttttt aggtatttgg     1860
   aattttggtg tttagaatgt gttttttggg agagtaaagg ttgtgtttat ggtagtttgg     1920
<210> 57
   <211> 2225
   <212> DNA
   <213> Artificial Sequence
<220>
   <223> chemically treated genomic DNA (Homo sapiens)
<400> 57
   atttttttgt aaattaaatt tttgttgttt agtttagtgt ttggtgtata gtgggggtta       60
   gatagatatt tgttgagtgt atgattaggg tgttgtaggt ggaggaggtg gtggtggggt      120
   gggggagggg aatttttttg ttgttttttg ttttgtggga attagtttta gaaagaaatt    180
   ttttgttgtt ttttttattt tattttaatt ttggatttgt tttttgattt tttttatttg     240
   gttttagttt tttatttaga gttaattttg tttgaaaggt attatgtttt gttattaagg      300
   ttttaaaata gggaaaatag tttttgaagt ttttagtttg taggagatag ggtattttag      360
   ggtataaggt tttattgagg ggttggaaaa agagtttaga ttttaatatt tatagaaagg      420
   ggttttggtt gtttagttgt gagtggtttt ttttttgttt tttagtatat ttggaaatga      480
   ttttatatgt gggtgttgtg ggatgttggg tagtaaggag atgatttagt tgaataatgt      540
   atttggttta tttttgtgag gggaggagaa tgttgaggga taggaattgt gtgttagtaa      600
   aagatatttt aatttggggt gtgtttgtta gttgtttttt taaggtgaaa atttttaaga      660
   ggtgagatgt aaattttagt ggtgtttata tttggaggtg tttttttttt aggttgtttt      720
   tttgttttat gtgatatttt aatttttgta gtttttgttt agtgtgggat taagaatggg      780
   gagttttatt tttttttggt tttttagttt tttaatttaa gataagaggg tgtgtgtttg      840
   ggttgggata ggttttagtt gtatttttgt agattgtatt ttttgtttgg gagtgatttg      900
   ttttagtagt tttttttttg ttttattaat gtggttgtat tgttggtgtt tagaggtgat      960
   attttggtaa agataggaat taagttagaa atttaggttt tagaagtaaa ggttgtggtg     1020
   ttttttggtt ggaagaaaat ggtgggtggg tggaggatgt ggtgtgtggg aattttttta    1080
   tggatgtttg ttgttttttg tttatttttt aggaaggtgt ttttttattt gtattttttt   1140
   ttgtaatttg gagtttgttt gtgagtttag ttatttgttg gttttttttg tttgtgtttg    1200
   tatattatag attttgttta ttgtatttta tttggtttta gagggtttag aggggatttt      1260
   ttgaaattaa tttgtttgtt tggagttagt tttgatgtat attttgagag tggtgggagg      1320
   taaagtagtt ggaatgtttg agaaaaaagt gggtgttaga ggtttttgtt ttagtgattt     1380
   ttttattgta ttgattgagt tttgggattg tagggattta agtttttatt tgttttggtg     1440
   tttttttttt gtgtgttttg ttgttttgga agttggattg agagttatag tgtggttggg     1500
   tgtaggtggg ttgtttggtt tttattttga ggatttggat ttgtatttgt agagttgttg     1560
   tgtgggggat gttttttttt tttttttgtt ttggtgggtt tttttggtgt gttagggttt     1620
   ttgggtttta gttggagttg gtgttttggt tttatagttt tttttagtgt tttggatttg     1680
   tgtgttttgt tttgtgttgg attttgtttt ggggtttttg tttgtttttt agtttaggtt      1740
   aagggtggat gtttagtttt tttattttat tgtgtgtttt tgggttggtg tgtttagggt     1800
   gtgggtattg tagttttggt tgtgttgatg tgagtttttg gtagtggggg tagttttttg    1860
   tatggatttt agtgtttttg tattttgttt gaattggatt atatagtagt tgggttttta     1920
   ttgtgtgttt gggaatagtg agtattgttt tttgttgtta gttgtggtat tgtatttggg     1980
   ttgtgtgtgt aggaggggag agttttgttt tttggaggtg tttagtgtta gtgtttgtgt     2040
   ttattagaag gtgatttttt taaggaaagt ttggttttga tgagtgtggt gtgggttttt     2100
   ggagatggtg gtgaaggagg aggtttttag gaggaagttg gggatgggga tagtttgttt     2160
   tgtttaaagt ttttgaagtt tttatttaaa ttttgtgatg attattatta ttagatttta     2220
   tggtt                                                                  2225
<210> 58
   <211> 2225
   <212> DNA
   <213> Artificial Sequence
<220>
   <223> chemically treated genomic DNA (Homo sapiens)
<400> 58
   ggttatggaa tttgatagtg gtggttattg taaagtttgg gtgaggattt taggggtttt       60
   gagtagagta aattgttttt atttttagtt tttttttggg ggtttttttt tttattgtta      120
   tttttaaagg tttatattgt atttgttgga gttgggtttt ttttgaaagg gttatttttt      180
   agtgggtgtg gatgttggta ttgggtattt ttggaagata ggattttttt tttttgtgtg      240
   tgtgatttgg gtgtggtgtt atgattggtg gtagagggtg gtgtttattg tttttaagtg      300
   tatggtagaa atttggttat tgtgtggttt gatttagatg ggatgtgaag gtgttgaaat      360
   ttgtgtgaga ggttgttttt gttgttgggg atttgtgtta gtgtggttgg agttatggtg      420
   tttgtgtttt ggatgtatta gtttgagagt atgtggtggg gtgggggagt tgggtattta      480
   tttttggttt ggattaggag gtaggtgggg attttggggt ggagtttagt gtggggtggg      540
   gtgtataggt ttgagatgtt ggaaagagtt gtggggttgg agtgttaatt ttagttggaa      600
   tttagagatt ttgatatgtt ggaggagttt gttggggtga ggaaaaaaga gggatatttt      660
   ttatgtagtg gttttgtaag tgtaagtttg ggtttttgag gtgaaaattg agtggtttgt      720
   ttgtgtttaa ttatattgtg atttttgatt tggtttttgg agtagtagag tgtatagaaa      780
   ggaggtgttg agataggtaa gggtttagat ttttgtagtt ttgggattta gttggtgtgg      840
   tgggggagtt gttaggataa gggtttttga tgtttatttt ttttttgggt attttggttg      900
   ttttgttttt tgttattttt gagatatgtg ttggggttgg ttttaagtag gtgagttggt      960
   tttaggagat tttttttggg ttttttagga ttgggtaggg tatggtaagt ggagtttgtg     1020
   gtgtgtgggt gtgggtagag ggagttagtg ggtagttgag tttgtggatg agttttaaat     1080
   tgtaggggag ggtgtaggta gaggggtatt tttttgaaag gtgagtagga aatagtaagt     1140
   gtttgtgggg aaatttttgt gtattgtatt ttttatttat ttgttgtttt tttttagtta     1200
   ggaggtattg tagtttttgt ttttagagtt tgaatttttg atttgatttt tgtttttgtt     1260
   aggatgttgt ttttagatgt tgatagtgtg gttgtgttga tggggtggga agggggttgt     1320
   tggaatgggt tgtttttggg tggggagtgt aatttatgaa aatgtggttg ggatttgttt     1380
   tagtttgggt gtgtgttttt ttgttttggg ttagggggtt ggaaagttga ggaggggtag     1440
   gattttttat ttttggtttt atgttgagta gaggttgtgg ggattggggt gttgtatgag     1500
   atggaggggt gatttggaga gaagatattt ttgggtgtgg atgttattgg gatttgtatt     1560
   ttgttttttg gaggttttta ttttgggaaa gtagttggtg ggtgtatttt aggttagggt     1620
   gttttttgtt ggtgtgtagt ttttgttttt tggtgttttt ttttttttgt aggagtggat     1680
   taaatgtatt gtttagttgg gttatttttt tgttatttag tattttgtgg tgtttatatg     1740
   tggagttgtt tttaggtgtg ttgagaggtg aggggaaggt tatttatagt tggatagtta     1800
   gggttttttt ttataggtgt tgggatttgg gttttttttt tagttttttg gtgaagtttt     1860
   gtattttggg gtgttttatt ttttgtgggt tgagggtttt agagattatt ttttttattt     1920
   tgagattttg gtgataggat atggtgtttt ttaggtaggg ttgattttgg gtggagaatt     1980
   agggttaagt gaaggaggtt agagagtaga tttaaagttg gagtagggtg ggaagggtgg     2040
   tagaggattt ttttttggag ttggttttta tagaatgggg agtagtaggg gaattttttt     2100
   tttttatttt attattattt tttttattta tagtgttttg gttatgtatt taatagatgt     2160
   ttgtttagtt tttattatgt attaggtatt ggattgggta gtaggaattt ggtttatagg     2220
   aagat                                                                 2225
<210> 59
   <211> 2251
   <212> DNA
   <213> Artificial Sequence
<220>
   <223> chemically treated genomic DNA (Homo sapiens)
<400> 59
   ttttttagtg tgattgattg tgatttagag gagaatgggt aggtgtagtg tgagttattg       60
   ggagatgtgt ttttttgttt taagtttttt tttaagaatt attatattat tgttattgaa      120
   gtttttttgg attgagaggt gggggatttt tatattttga ttgtagtggt ttgggattgg      180
   ggtgagtttg tgttttttat tagtaagttg atttaggtat aagtgttgga tgtgaatgat      240
   aatgtgttgt gttttagtta gttggtttat gatgtgtatg tgattgaaaa taatgtgttt      300
   ggtgtttata tttatgtggt gagtgttatt gattgggatg agggtgttaa tgtttagttt      360
   gtttatttta tttttgagtg ttagatttag ggtatgagtg tttttattta tgtttttatt      420
   aattttgaga atggttattt gtatgttttg tgtttttttg attatgagta gttgaaggat      480
   tttagttttt aggtggaagt ttgggatgtt ggtagttttt aggtgttggt tggtaatgtt      540
   attgttaata tttttatagt ggattaaaat gataatgttt ttgttattgt ggtgttttta      600
   ttagggtgta atgggatttt agtgtgtgag gtgttgtttt gtttggtgga gttgggttat      660
   ttgtttattt gtgtggttgt tgtggatgtg gatgatggtg agaatgtttg gtttatttat      720
   agtattgtgt gtggtaatga aatgaatttt ttttgtatgg attggtgtat tggggagttg      780
   tgtatagtat gttgagtttt ggttaagtgt gatttttagt ggttttatga gttggtgatt      840
   gaggtgtgtg attatgggta gttgtttttt ttttttattg ttattttggt ggtttagttg      900
   gtggatggtg ttgtggagtt ttagggtggg ggtgggagtg gaggtggagg gttaggagag      960
   tattagtgtt ttagttgttt tggtggtggg gaaattttgt tagattttat ttttattttt     1020
   attattgtgt tgggtttggt gttttttatt tttttgttgg ttatgattgt gttggttgtg     1080
   tgttgttaaa aagagaagaa gtttaatatt tatatttgtt tggttagtga ttgttgtttt     1140
   tgttgttgtt gttgtggtgg tggaggtttg atttgttgtg gttgttaagt ttgggtgtgt     1200
   aagaagaaat ttagtaagtt agatattatg ttggtgtaga gttttaatgt atttagtaat     1260
   ttggtttagg tgttgataga ggagtttggg ggttttggtt tttattatta taattagaat     1320
   tattgttatt aggtatgttt gatttttgag tttgttaaga ttgatttgat gttttttaag     1380
   ttttgtagtt ttttgtggag tatggatatt gagtataatt tttgtggggt tattgttatt     1440
   ggttatattg attagtagtt tgatattatt tttaatggaa gtattttgtt taatgaggta     1500
   aggttgaagt gaaaggatta ttatttttta ttttttttat tagaaagttt tttttagttt     1560
   ggtttttgta tttttggtgt attgtattta tttttaggat attagtttat gtgtattgtt     1620
   gtgggagtag agatgggtgg ttattttttt ttattttttt gtgtgtaatt taatttttgt     1680
   gttgttttat ttttttatat ttatttttat tttgtttttt tggtattttg ttattttgga     1740
   gttttttttt ttgttttttt attttgttag tttttttttt ttttagtaat ttgggtatga     1800
   agggaaattg tgtgaaggga gagggaaatg tggaggaggg atttattttt tagtattggt     1860
   aaaggttttt tttttttgtg tttgttttag gtattaataa agttggtttt attttgtttt     1920
   gtttaatgat gtttttagtt gtgtgtataa gtaagttata gattgtttaa ttttatatag     1980
   ttgtttaatt ttgaatttat gttttagtga ataagttatt agattttttt tttttgaatt     2040
   tgggttgtaa aaaaagtttt tagtttggtt ttggatagta tttatagatg tttttgaagt     2100
   tgagtgttta tatagtgtga atttgaatga agttgtgttg gtataaattt ttgttaagag     2160
   taaattaatg aaaggtttaa ataattgttg ttttaaatat atttttttag ttaataatta     2220
   tttttttgtt attgttttgg ataaattatg t                                 2251
<210> 60
   <211> 2251
   <212> DNA
   <213> Artificial Sequence
<220>
   <223> chemically treated genomic DNA (Homo sapiens)
<400> 60
   gtgtgattta tttaaaatag tggtaagaag gtgattatta gttaaaaaga tatatttaag      60
   ataataattg tttaagtttt ttgttagttt atttttaata ggggtttgtg ttaatgtagt     120
   tttatttaaa tttatattgt gtgggtgttt ggttttaaga gtgtttgtaa atgttgttta     180
   gagttgaatt gaaggttttt tttgtaattt gagtttagag gagaaggatt tggtaatttg     240
   tttattaaaa tatgaatttg aggttagata attgtgtaaa gttaaataat ttatagttta     300
   tttgtatatg tgattaaaag tattgttaaa taaagtaaaa tagagttaat tttattaatg     360
   tttgggatag atgtaaagaa aaaagatttt tgttagtgtt agaaagtaag tttttttttt     420
   atattttttt ttttttttat gtagtttttt tttatgttta ggttattgag aagggaaagg     480
   attgatagga tggaagagta aaggagggag ttttaaggtg gtaaagtatt aaggggatgg     540
   aatgaaaata aatgtgaaag ggtggaataa tgtgaaagtt aggttatata tgaaggagtg     600
   ggagaaggtg attgtttatt tttgttttta taatgatata tataagttaa tattttaaaa     660
   atagatatag tgtattagag atataagggt tgggttagag gaggtttttt gatggaggag     720
   atgagagatg gtggtttttt tgttttagtt ttattttgtt ggataaaatg tttttgttgg     780
   agatgatatt aggttgttgg ttggtgtaat tggtgatgat ggttttgtag gggttgtgtt     840
   tagtgtttgt attttgtgaa gggttgtagg gtttaagaaa tattaggttg gttttggtgg     900
   atttaggggt taggtatatt tgatagtagt aattttggtt gtggtggtgg gagttaaagt     960
   ttttggattt ttttattggt atttgggttg ggttattggg tatattggag ttttgtatta    1020
   gtatgatgtt tgatttgttg agtttttttt tgtgtgtttg ggtttggtgg ttatagtagg    1080
   ttgaattttt gttattgtag tagtagtagt agaggtagta attgttggtt agataagtat    1140
   agatgttgag tttttttttt ttttggtaat gtatggttag tatgattatg gttagtagga    1200
   agatgaagga tattgagttt aatgtgatga tgaggatgag ggtgaggttt agtgaggttt    1260
   ttttgttgtt agagtgattg gggtgttggt gtttttttga ttttttgttt ttgtttttgt    1320
   ttttgttttg gggttttatg gtgttattta ttagttgaat tattagggtg gtggtggagg    1380
   aaaggggtgg ttgtttatgg ttgtgtattt tgattattag tttataaggt tgttgggggt    1440
   tgtgtttggt tgggatttgg tgtgttgtgt gtagtttttt ggtgtgttag tttatgtgaa    1500
   agaggtttat tttgttgtta tgtatgatgt tgtaagtgag ttgggtgttt ttgttgttgt    1560
   ttgtgtttat ggtggttatg tgggtgagta ggtaatttgg ttttgttgag tggggtagta    1620
   ttttatgtgt tggagttttg ttgtgttttg gtagaggtgt tatgatggta ggggtgttgt    1680
   tattttgatt tattatgagg atgttgatag tggtgttatt agttagtgtt tgggggttgt   1740
   tagtgttttg ggtttttatt tgaaaattga agttttttag ttgtttatag ttgaaggagt    1800
   gtagggtgta taagtagttg tttttagagt tgatagaaat gtaggtgaag atgtttatgt    1860
   tttggatttg gtatttgagg atagagtagg taagttgggt gttggtgttt ttattttggt    1920
   tggtggtgtt tattgtgtag atgtaggtgt taggtatgtt gtttttagtt atatatatgt    1980
   tgtagattgg ttggttgaaa tgtggtgtgt tgttgtttat atttgatatt tgtatttgga    2040
   ttgatttatt ggtggagagt gtaggtttgt tttggttttg agttattata gttagggtgt    2100
   aggagttttt tgttttttgg tttagggggg ttttggtaat gatggtgtag taatttttaa    2160
   aggaagattt gaggtggaaa ggtatgtttt ttagtagttt gtattgtatt tgtttatttt    2220
   tttttgagtt gtggttagtt atgttgaaaa g                                   2251
<210> 61
   <211> 2553
   <212> DNA
   <213> Artificial Sequence
<220>
   <223> chemically treated genomic DNA (Homo sapiens)
<400> 61
   ttggttattt ttgttttttt ttagattttt agagttttta gtgtagttat agaggatgtt 60
   gttggtttta gttttaagaa gatgttgttt tttttagagg gttaagtaag tgggaatttt 120
   tttttttatt tgttttgggt tttaggtagg gtttttggtg taaggtttgg ggttggaagt 180
   tgatttattt aggtttaggt tttggggtag aattgaaatt ttttggttat tgttggttgt 240
   agtttgggag taggttattg ttaaagttgt gggttttttt aggatagttt ttttatgagg 300
   ttggtttttt atttgttgtt tttttatatt tggtggttag ggatgtggtt ttgggtagaa 360
   tgatgatttt ttatttttgt tattatggaa gttattgttg ttttttagtt tagttagtta 420
   tttgggttgt agagtatttt ttttatgttt tttgggtgtt tttttttttt tttgttttag 480
   tttggttttg ttttattttg tttttaggga ggggtatttt ggagtggggt tagggtatgg 540
   tttttttttg agggagtttt tttttggttg tttttagggt agttttgtat agttttagta 600
   tttggtgtat tttttttgat atttttttta gggatagtta ggtattttgt gtggggtatt 660
   taagagagtt aggtttgtta gtttttagtt tttgttagaa tgtaggtttg aggggtgagg 720
   ggtggggtag gggtagggat aggaattttg gtgtgttttt tatttgtaaa ggtttattga 780
   ggttttgagt tttagttatt gagttattaa gttagtttgg gttaggtttg ggtgttttgt 840
   ttgtaatgga ggtagagatg gggttttggg gtagttttga ggatgttggg tgtatagtgg 900
   gggttttgtt ggtaggaatt atttatgttt ttttttgggt taagttttgt ggatgtttag 960
   tttggggttg tggggagttg gtaggttagt ggtagatatt ggtgggtaga tttagtgttt 1020
   ggtagaatag gtattaagga agtggtgatt ggagggaagt taagtgtatt taaatttttg 1080
   ggtgagttat tattgttggg ttttttatag ttgttgaaag tgagtaatag tgatgaaggt 1140
   ttgtgagttt ttgtgtgagt gagtgaatgg attagtagta gtttttaggt tgtggaagag 1200
   tgtttttttt ttgggatggg gatatttggt tatagtaatt tttaattttt tatttattta 1260
   ttgtttattg tagaggtatg tgggggtttt gttttttgta ggtaggagtg aggggtattt 1320
   ttgtgatgtg gtatttttgt gattgaggtt atgtgtgatt ggtgtaaggg taggaagtga 1380
   gttattggtt tgtattaggt gtgggggttt ttgtgagggt aggatttaaa gttggtttgg 1440
   ttttttggtt gtagtatttt tttttttttt gaattaggtt agagttttgg gatgggaggt 1500
   gttttgtaga ttattttttt tattaatttt tgttttttgt tttatttttg tggtgatttg 1560
   gtgaattgtt ggttttttgt tgtgtattga gtggggtagt gattttgatg tggtgttttt 1620
   tgttgttttt gttattgtta ttatttttgg tggtttagtt tttgtatttt ttatttttat 1680
   ggaggaatgt attaggtttt tttttttgga tgtatttttt atttatatgt ttttaaattt 1740
   tggtattttt tgtttttttt ttatttttat tttttttttt aggtttttag ataaagggga 1800
   agtggttgga tttttttaaa gggatagtgt tttattagtt tattgttgaa tttttttttt 1860
   taattttagt tttttagtta tagttaatta gtattagtag atagtttatg agtgatattt 1920
   atgtaggttt taggttgtgg agagtttttt gggtaggaaa tagtttttaa ggttttttat 1980
   tttatttagg ttttagtttt ttttatttgt ttttttttta gattgtggtt ttttggagtt 2040
   tggttttttt gtttttgtgt gattgatata tagtatttaa atagtggtag agtgggatgg 2100
   attttttagt ttgttttttg tgtgggtttg tattttgatt tagatatgtt tttttatagt 2160
   aggatttagg ggggtatatg tgtgtttgtg ggtttattgg ggtatttgta tttggtttat 2220
   tttatttttt agagagaggg ttttgttgtg ttatttagtt ggagtgtagt ggtgtaatta 2280
   tagtatattg tagtttttaa tttttgggtt taagtgattt ttttttttta gtttttttag 2340
   tagttgggag tataggattt attgtatttt ggttaatttt ttaataattt tttaagagat 2400
   ggggttttat tgtgttgttt aggttggttt taaatttttg gttttaagtg atttttttat 2460
   ttttgttttt tgaagtgttg agattatagg tatgagttat tatgtttatt ttagattgat 2520
   atttttatat ttgtttattt tggttgggta ggg 2553
<210> 62
   <211> 2553
   <212> DNA
   <213> Artificial Sequence
<220>
   <223> chemically treated genomic DNA (Homo sapiens)
<400> 62
   ttttgtttag ttaggatgaa taaatataga aatgttagtt tgggatgggt atggtggttt      60
   atgtttgtaa ttttagtatt ttaggaggtg aaggtgggag gattatttga ggttagaggt     120
   ttgaggttag tttgggtaat atagtaagat tttatttttt aaaaaattat taaaaaatta     180
   gttaggatat agtgggtttt gtatttttag ttattaggga ggttggggag ggaggattgt     240
   ttgagtttag gagttgaagg ttgtagtgtg ttatgattat attattgtat tttagttggg     300
   tgatatagta agattttttt tttaaaaaat aaaataaatt aaatgtgggt gttttagtga     360
   atttgtaggt atatatgtgt ttttttgggt tttgttgtgg gggggtatgt ttgggttagg     420
   gtatagattt atatagagaa taggttaggg ggtttgtttt gttttgttat tgtttgggtg     480
   ttatgtgttg gttatatagg gatagaagaa ttaggtttta aagggttata atttaggaga     540
   gaggtaggta ggaaagattg ggatttggat gagatgaggg attttaaggg ttgtttttta     600
   tttaggaaat tttttatagt ttggggtttg tatgggtatt atttatgggt tgtttgttaa     660
   tgttaattaa ttgtaattag ggaattgggg ttgaggaggg gagtttagta gtaagttggt     720
   gggatattgt ttttttaaga ggatttagtt attttttttt tgtttgggag tttaggggaa     780
   ggggtgggag taaaggggga gtagaaaatg ttagggtttg gaagtatgtg ggtgaggggt     840
   gtatttagga agggaggttt ggtgtatttt tttatggggg tggggaatgt ggaggttggg     900
   ttattggagg tggtggtggt ggtaggggtg gtaggagatg ttatgttagg gttattgttt     960
   tatttggtgt atagtagggg gttggtagtt tattggatta ttgtgggggt ggggtggagg    1020
   atggaagttg gtgagggggg tggtttatag ggtatttttt gttttagggt tttaatttaa    1080
   tttgaaaggg aaaggagtgt tgtagttggg aggttaggtt gattttgggt tttgtttttg    1140
   tagaagtttt tatgtttggt gtagattaat gatttgtttt ttgtttttat attgattata    1200
   tatgattttg gttatagggg tgttatatta taggagtgtt ttttattttt gtttgtagga    1260
   agtagggttt ttgtatattt ttgtagtggg tggtgggtgg gtgggggatt aggaattgtt    1320
   gtaattaagt gtttttattt tggggaggga atgttttttt ataatttgga aattgttatt    1380
   ggtttattta tttgtttatg tagaaattta taaattttta ttattgttgt ttatttttag    1440
   tagttgtgaa agatttggtg gtgatgattt atttgagggt ttgagtgtat ttggtttttt    1500
   tttggttatt atttttttga tgtttgtttt attagatatt aggtttgttt attagtgttt    1560
   gttattgatt tgttagtttt ttgtggtttt aggttgggta tttataaagt ttggtttagg    1620
   agagagtata agtgattttt gttggtgagg tttttgttgt gtatttagta tttttagaat    1680
   tgttttgaga ttttgttttt gtttttattg tagatagggt atttaggttt ggtttaggtt    1740
   gatttggtgg tttagtggtt ggggtttggg gttttagtga atttttgtgg atggagagta    1800
   tgttggagtt tttgtttttg tttttgtttt gttttttatt ttttaggttt gtattttggt    1860
   aggagttaga ggttgatggg tttggttttt ttgagtgttt tatatagagt gtttgattgt    1920
   ttttaagaag gatgttaagg gaggtgtgtt aggtattgag gttgtgtaga gttgttttgg    1980
   ggatagttag agaggaattt ttttggggga gagttatgtt ttggttttat tttagggtat    2040
   ttttttttga gagtagggta ggataaagtt aggttggggt aggagaaggg ggaggtattt    2100
   ggagggtatg aaaggggtgt tttgtagttt aggtggttgg ttgggttggg agatagtggt    2160
   ggtttttata atgataggag tggagaatta ttgttttatt tagggttata tttttggtta    2220
   ttaggtgtga agaaatagta ggtggaggat tggttttatg gggagattgt tttggaagga    2280
   tttatagttt tggtagtggt ttgtttttag gttgtagttg atagtaatta aggagtttta    2340
   gttttgtttt agagtttgga tttaggtggg ttggttttta gttttaggtt ttatattagg    2400
   ggttttgttt ggagtttagg ataagtaggg aggggatttt ttatttattt agttttttgg    2460
   aggaagtggt gtttttttgg ggttgaagtt aatagtattt tttgtggtta tattgggggt    2520
   tttggaggtt taagggaggg taggggtggt tag                                 2553
<210> 63
   <211> 2001
   <212> DNA
   <213> Artificial Sequence
<220>
   <223> chemically treated genomic DNA (Homo sapiens)
<400> 63
   ggtaaaagtt tgtttggatt ttttggttat tagaaatatg agtatggtgg tggtttttag      60
   ttttttattt atgtttgggt ttaagagatt gggagtttag gtttattgat tttttgagaa     120
   agattaagat tttgtatttt agaaagaggt ttggggattt ttgttttgtg taagggtaga     180
   aggattagtt gtttttttga gtattttaat ttggaatttt ggtttgaagt tgagatagga     240
   gattggatgt gaggtttttt tagagttggt tttttttaaa taatttttaa aatttttaga      300
   ttttaggggt atgttgaaat tttttaaagt agtttaaaga atataatgag agttttaata     360
   ttttaggtgg tggtgtgttg gttttttgga gtggggtggg atgtggttgt gtggatttat    420
   gtgtataatt gtgtgggatg gggttatgtg gatttatgtg tataattgtg ggattttagt     480
   gttagtggga ttttagtgtt agtgggattt tagtgttagt gggattttag tgttagtggg     540
   attttagtgt tagtgggatt ttagtgttag tgggatttta gtgttagtgg gtttgtggtt     600
   tagtggagtg agtggagtgt tggtgatttg agtggagatt gtgttttgga tgttttagtt     660
   tagatgttaa gttatagttt gtgtagtagt agtaaagggg aaggggtagg agttgggtat     720
   agttggattt ggaggttgtg atttagggga aagtgtgggt ggttgattta gggtagttgt     780
   ggtggtgagg taggtgggtt ttttgttttt tggagttgtt tttttttata tttgtttttg    840
   gtgtttttag tagtttttat tttggttttt tgtggttatt gtgggatttg gtgttgttgt     900
   tagtttagtg gggagtgaat tagtgttttt ttttgttttt ggtttttttg atggtatgag     960
   gaatttttgt tttgttttat agatttttgg tttttgttga gtgtggtatt ggagtttgtt    1020
   ttgttagggt tttggaatta gagaaagttg ttttttggtt atttgaagtg ttggattttt   1080
   atagtgtttt ttagtttggg tgggagtggt ggttgtgttg ttgaaggttg gggtttttgg    1140
   tgtgaaaggg aggtagttgt agttttagtt ttattttaga agtggttttt gtattgttgt    1200
   ggtgggtgtt tttgggtttt gattttgtta gtgttgtggg gtagaggtat ttggagtttg    1260
   tagggtttag atttgggttg gaaaagtttt gttgattgta ggtaagtgtt tgggaggggt    1320
   ggttaggtga agttttggtg ttttattata tatttttggg ttttatgtta gttgtatttg    1380
   tggtattggg taggaaatgg tagggttgag gttgatttta ggagtataag ggagtttttt    1440
   attttttgtt tatatttgtt atttttagtt ttgtaattta ttttagatat atagaaagta   1500
   agtaggattg gtggggagat ggagtttaat aggaatattt tttagtagtg agtaggggtt    1560
   gtatgggatg tgggaggagt ttagaggagg tgtggagagt gtttgaggtt gggtgagtgt    1620
   ttagagggga gatagttgaa ttgggtttaa gaggtgttta gtgggtgttt gttgaatgaa    1680
   tgagtgatgg gttttgaagt ttgagtgtat tgaaagaggg ggtgtgtaaa aagggttttt    1740
   tttattatat aggatatagt atatgtaaat tttttttttg tggaaaagtt agataggtta    1800
   aaaaggttat aaataaatta gttgggtatg gtggtgtgtg tttgtagttt tagttattag    1860
   ggaggttgag ttaggggaat tgtttgaatt tgggaggtgg agattgtagt gagttaagat    1920
   tgtgttattg tattttagtt tggaaataga gtgagatttt gttttggaaa aaaaaaaaaa    1980
   aagttataaa ttgtgtgtgg g                                              2001
<210> 64
   <211> 2001
   <212> DNA
   <213> Artificial Sequence
<220>
   <223> chemically treated genomic DNA (Homo sapiens)
<400> 64
   tttatatatg gtttgtaatt tttttttttt ttttttgaga tggagttttg ttttgttttt      60
   aggttggagt gtagtggtgt gattttggtt tattgtaatt tttgtttttt gggtttaagt     120
   gatttttttg gtttagtttt tttagtagtt gggattatag atgtgtatta ttatgtttgg     180
   ttaatttgtt tgtaattttt ttaatttgtt tggttttttt atagggagag gatttgtata    240
   tgttgtgttt tgtgtgatga aaggagtttt ttttatatat tttttttttt aatgtattta     300
   gattttaaag tttattattt atttatttaa taaatattta ttaagtattt tttgaatttg     360
   gtttaattat ttttttttta ggtatttatt taattttggg tattttttgt gttttttttg    420
   agtttttttt gtgttttata tagtttttgt ttattgttgg aaaatatttt tgttaagttt    480
   tgttttttta ttagttttgt ttgttttttg tgtgtttggg ataggttgta aaattggagg   540
   tgataaatgt gggtaggaaa tggagggttt ttttatattt ttagggttgg ttttagtttt     600
   gttatttttt gtttaatatt gtggatgtaa ttggtatggg atttggaagt gtgtggtaaa     660
   gtgttggggt tttgtttggt tgtttttttt ggatgtttgt ttgtagttag tgaagttttt     720
   ttaatttagg tttgggtttt gtgagtttta ggtgtttttg ttttgtggtg ttggtgaagt     780
   tgaagtttga gaatgtttat tgtagtgatg tgaaggttgt ttttggggtg gggttgaggt     840
   tgtagttgtt ttttttttgt attaaggatt ttaattttta gtgatgtagt tgttgttttt     900
   gtttaggttg ggaggtattg tagggatttg atgttttagg tggttaaaga gtgatttttt     960
   ttgattttag ggttttggtg gggtaggttt tagtattgta tttggtggag gttgaaggtt    1020
   tgtggggtag gataggagtt ttttgtgttg ttggaagggt tgaggatgaa ggagggtgtt    1080
   aatttatttt ttattgggtt ggtggtaatg ttgaattttg tagtgattgt ggagggttaa    1140
   ggtgaaaatt gttgggggtg ttgagggtag gtgtggggag gggtggtttt agggagtaag    1200
   gagtttattt gttttgttgt tgtagttgtt ttgggttgat tgtttatgtt tttttttggg    1260
   ttatgatttt tggatttaat tgtgtttggt ttttgttttt ttttttttgt tgttgttgtg    1320
   tgggttgtaa tttgatgttt aggttggggt gtttagggtg tagtttttgt ttaggttgtt    1380
   agtgttttat ttgttttatt gggttataga tttgttggtg ttggggtttt gttggtgttg    1440
   gggttttgtt ggtgttgggg ttttgttggt gttggggttt tgttggtgtt ggggttttgt    1500
   tggtgttggg gttttgttgg tgttggggtt ttgtggttgt gtatgtgagt ttgtgtggtt    1560
   ttgttttgtg tggttgtgta tgtgagtttg tgtggttgtg ttttgttttg ttttagggag    1620
   ttagtgtgtt gttatttggg atgttaggat ttttgttgtg ttttttggat tgttttgggg    1680
   gattttggtg tatttttagg atttaggagt tttggaagtt gtttgagaga aattagtttt    1740
   gggagggttt tgtatttagt tttttgtttt ggttttggat tggggttttg ggttaaggtg    1800
   tttagaggaa tagttgattt ttttattttt gtgtagggta gagattttta aatttttttt    1860
   taaaatgtag ggttttagtt ttttttaggg agttagtgaa tttagatttt tagttttttg    1920
   agtttaagta tgaataggga attggggatt attattatgt ttatattttt ggtggttagg    1980
   aagtttaggt aggtttttgt t                                              2001
<210> 65
   <211> 219909
   <212> DNA
   <213> Artificial Sequence
<220>
   <223> chemically treated genomic DNA (Homo sapiens)
<400> 65
   tgttatttga gttgtaaagg gtggtttttt gtgtttgttt tgttgtattg atatattgag 60
   ttgtgtatgg tgtttagtgt agggagaata aatgattatt tgtttaatgt gtttatttat 120
   aggtgaggaa attaaggttt taatttaatt gatgtatttt gtttttttga ttattagaaa 180
   agtagtagga taggtgtttt gttttgtttt attttggttt attaagttgg tattttggtt 240
   ttgatttttg gttgtgtttg gtgttgttgt ggtgtttggt gttgttgttt tgtttggtgg 300
   ggttgtttgg agtgtttgta tttttgtttg tttttatttg gttgggtttg ttttgtttgg 360
   atttgggatt gggaagtgtg gtgatttttg gaattagtta ttggtgttag tgtggggagt 420
   tgggggtgta gagttgtggg tgtggtgggt tatgtaggtg gtttttattt ttggtttggt 480
   ttggtttggt ttggtttgtg ttgttgtgtg ggggtgtttt tttttaggtt tggtgtttgt 540
   tagttttgtt ttgttaggtg tagtgtagtg taggggtggg tgggggtggg gtttggtgtg 600
   tatgtttatg gggtggggag ggggtgggtt aggggtggga ttatagttgg ttgggttggg 660
   gttttatgtg tatttttggg gaggggtggg gtgggggtgg ggttggggtg gggtttggtt 720
   ggtgtatttt agatggtggg ttgttttttt tttttgtttt tttattattg gtttaggatt 780
   agtgttttgg gagtgtgtgt tttgttgttt tgttgttata tttttttggg agtggtggtt 840
   atggaggtat tatgaagaag ttttatttag gtgggttttg tgtttggggt ggggaggggt 900
   tggtgttttg ggattagtgt tgtttatttg agtgtttgtg gttgggagtg gtgaggtgtt 960
   tttggagttg agtgagtttt tgtggtgggt atattgtagg ttgagttttt tttaggatag 1020
   ggttgttgtt gggttgtttt tgatttgagt tgattgtttt ttgtgttgtt tttagttttt 1080
   gtttgagtgt tggaggggtt gttttggggg atgttttttt ttttttgttt tttgtatttt 1140
   tgtaggaatt gttgattttt taggttggtt gggtgttttg ggtttttgtg tgtttgtgtg 1200
   ggtgaatggg gttggggtta ggtggagggg tgtttttggg tttagttttt agggttggtg 1260
   gtttaggttg tagtattttt tttttggatt ttttttggtt ttttttttat ttagtggggt 1320
   atgggatggt ttttagatgg gattgtttag tagtgtttaa atttggtgat ttgggtttat 1380
   gttttgtgtt taggatgttg tttgtggttg atatttttat tttattttag tagggtttgt 1440
   tttaagtagg tgaagggggt ttgatttggg ttttattatg agttggttta ggagttttta 1500
   ttttataaga ggtttttgtt ttggtatttt gaagttttat tttatagatg ggtatattga 1560
   ggttataagg gtaggttaag agaaatttag atttttatta tttgtttttt tttttttttt 1620
   tttttttttt tgtggttttt tttaaaggat ttaggtaggt aggatttttt gtttttttta 1680
   gtttttaaat tataaatagt gttttttttt ttaaatttag aaattgtttg tttaatgttt 1740
   tttttttaat ttatttagat agaaaggtat ttgttaagga tttatgaatt aatttttatt 1800
   gataggagaa tagaattatg ggttttagtt ggaggtgaag gagttattgg ggttattttt 1860
   ttgtttttgg gttattgttg gttttttttg gttttttata tttaggaatt aaaatgagtg 1920
   ataaagtatt ttgttttaaa gaggattatt tagaattttt ttgaaaatat ttttttgggt 1980
   tagtagttta gaattttaat ttttatttgg ttgttgtgaa taagatggtt ttttatgtat 2040
   ttggagtata gttttgttag gagtgtgggt gggttggtgt gttggtgggt gggagtgggt 2100
   ttttaggtgg tatatgtttg gttttggtat tggggttttt tttttaatat gttttaaata 2160
   tttgagattg ttgtggggat tgtgtggtgt atggtttttt ttaggggatt tttagtttgt 2220
   ttagggaggg ttttttgttt ttggtttttt atattttatt ttttattggt gggtttagat 2280
   agtgggggtg attggggttt ttttaggttg tgggttttgg gttttttttg tggagatttt 2340
   tttgtataga tgttggaagg gttttgaggt aggtttggag tttttgtatt tgtggtttat 2400
   tgggaggtgg ggagattatt atttgttttt tttttttttt ttttatatat atttatttta 2460
   ttttttattg ggtttttttt tttttttttt ttttaaaaaa aaggatagaa ttttttgtag 2520
   taggtggaag tagagtttgt ggagtgagta ggaattaatt tttgtatagt agttttggtg 2580
   tttttttgtg tttatttaaa ggggtaggtt tttttgagga tgggggaagg gagggtttag 2640
   attatttttg gtttgttttt tgtttggtag tggtttaagg tgtgttgggt tattgtgttg 2700
   tagttgtatg gagtgggatt ggagggggtt gatggtgggg gtggtttttt gtttttattt 2760
   tgagtgtttt ggagttgttt tggtattagt ttttttttgg gtatgggtat ttgtaggagt 2820
   taggttgatt ttttaataag ttattgatat tatgaaatta aggaaagtag taaaagatgt 2880
   taaaaatttt ttaaggtttt gataatttaa ggataatagg aaggttttta tttttttatt 2940
   tagattgtgt gggattattg tttttagatt tgtatgtggg ttttagaata aatggaatga 3000
   ttataatgtt gtttttggta gatttggttt gaatatttgt gagtttttga aaaggagaaa 3060
   gattttagaa ggatgtgttg gggtaatttt aagtattgtg ggtttataga tagttattga 3120
   agatatttta tgtgttgggt atatatggtt aagtttgagt gggtatagaa atggtaatga 3180
   gatatagttt ttgtttgtag agaatttata tttttattgg gagttaggat gagggtgaga 3240
   tattagttta ttaatatttt ttttttttga gtagtttagt ttttaaatat tataggaatg 3300
   ttttggaggt tttaggtgga ggtgagattg agttgggttt tgaggaagga gaagttttga 3360
   gtagtggaga ataaaaaggg atttggttag gtgtgtttga agttttagtt atttaggagg 3420
   ttgagatatg aggattgttt gagtttagga ggttatttag gaggttgaga tatgaggatt 3480
   gtttgagttt aggaggttat ttaggaggtt gagataggag gattgtttga agttaggagt 3540
   ttaaggttgt agttttgtga tagtgtttgg aaatagatat ggtattttag tttgggtggt 3600
   gttatgagat tttatttgta agaaggtttt ttttaatgtg gaaggtagta agggtttata 3660
   tagggtagtt gtaggattta gggggatatt ggggataggt tttttttggt agttttattg 3720
   ttgttttagg tttttttgat tgttttattt ggttttttaa tggggtggag gtagtggtag 3780
   tttataggta gtgtggttta gtggttatat ttgggggttt agagttgtta gttttttggg 3840
   attaaatttt attaggttgt gttttttttt gtggtataag gtttttattt tatgtatttt 3900
   atttgtttta tttgtttttt tgtgtgtgtg gtgtttttta attttattta tttatttatt 3960
   tttgagatgg agttttattt tgttttttag gttggagtgt aatggtatga ttttagttta 4020
   ttgtaatttt tgttttttag gtttaagtga tttttttgtt ttagtttttt gagtggttgg 4080
   gattgtagga atgtgttatt gtgtttagtt aatttttata tttttattag agatggggtt 4140
   ttattatgtt ggttaggttg attttgaatt tttgattttg tgatttgttt gttttggttt 4200
   tttaaagtgt tgtgattata ggtatgagtt attatgttta gtttgtttta tttgttaaat 4260
   gggatagtgt tatgttgttg taaaggttaa atgagtttat atttgaaaaa gttgagaaat 4320
   gtttgttttg taatttgtta gttgttgtta tgattatgat tgttattgat aggaggatgg 4380
   agaaggttgg ggttatgtta ttgttaggat gttttagaag tttttatgat ttttttttat 4440
   tgtggttaga gtatggtgta gggttttttt tttagttttt tagtattgga gttagttgta 4500
   ttgtatttgt tttggttttt taggttttga gttttgggat tttttttgta tattaggtta 4560
   tatggaattt gttgttattt ttaggttttt ttggaaattt tttgtgggtg gtagtggtag 4620
   gaggatttat ttgaggttga tgagttatag aagtgggatt gaggttgtgt tatattttta 4680
   ttggtatttg ggaattattt tggagatttt ttttttattt tttatttttt tgagatggag 4740
   ttttgttttg ttgtttaggt tggagtgtag tggtatgatt ttggtttatt gtaatttttg 4800
   ttttttaggt ttaagtaatt tttatgtttt agttttttga gtagttggga ttataggtat 4860
   gtgttattgt gtttagttaa tttttgtatt ttttagtaga gatggggttt tattatattg 4920
   gttaggttgg ttttgaattt tggattttag gtgatttatt tgttttagtt ttttaaagtg 4980
   ttgggattat aggtatgagt tattgtgttt agttattttg gagttttttt gtggttttta 5040
   ggagattttt gaaggggtat tagttatgat taaaaagttt tatgttgttg tgatttttga 5100
   gttttgggta gggagggagg ttgtatttat ataggaagta gtttttgttg tgatttagtg 5160
   tgttggtttg ttgtggtgga tagggttgta tttttattta gtgttttggg agttgattta 5220
   tgggaagggt aaggaattgt atttttagag aatatttttt tagttagtta atttggtgat 5280
   aggtgagtgt gggaagggta taagtatagg gttttttttt tttttttttt agttttgttt 5340
   ttgtgtttgt ttttgttgtt ttttgttttt ttatgtttaa gtttgtttgt tttggtgtat 5400
   ttaggggatt tggattggag agaaaaagtt attttgtatg gttagtttgt ggttttgttt 5460
   gatttgtgtt ttttgttgag gttttttagt tgtgagatag ttggttgagt ttaagttttg 5520
   gggttagttt tttttttagt ttgttgtaaa ggtagaaagg tatgtttagg taggggttat 5580
   ttgttttgtt tgtttatgtt agattgtagg tatttgtttg ttagattttt ttggtttgtt 5640
   tagagggatt ttgatagttg agggtaatgt atttggtagt ggagtattgt gtttgtgata 5700
   gtttattttt tttttggaat tattttttat aagtgtgatt agtttaagtt aggataggta 5760
   tagggggatt taggattgtt atttaggggt agtggtttgg aatttaggtt taggtgatat 5820
   gggagttaat tttttgtttg gagattttag gtgggtgttt gtagaggata ttttatttga 5880
   ggatttatgg aaggggatat tttttatttt ttatttggtt tttgggttgt ttttttttga 5940
   tatttttttg ttattttagg tagttttgat agttttatgt gattgggatt tagaggtggt 6000
   ttttgttttt gttttagtgt ttgtttatag gtagtgttgt gtgggttttg aagtttgttt 6060
   aagatggatt tgataagggg agtttagtat atattagtgt aggtttggtt agggggtgat 6120
   gatggtagtt tttgggtaga tttagttggt tttagggtag gaggggtttt agagagaggt 6180
   tttaggtttt taggggtgtg aatttttggg gtgaggtggg aagtgatgtg ttgggttttt 6240
   gtgtttttgt ttgttggagt tattattttg gaaatatttt agttttaggt ggttgttagg 6300
   gatttatggg gattagtggg tgttaatttt tgggtgaagt ttttggggtt tgtatatagt 6360
   atatagtgtt gtggttttat ggtttatttt gttgtgtgaa gtggtttagt ttttgggaga 6420
   gtttttagtt aggtaggttt gatggtaatt gttaggttat ggtgatttgg aattgttgat 6480
   taaagggagt tagttttggt gggtggaggg atttggtggg tagttattgt gatgttaggt 6540
   atggggattg tgtgttattt aggtttgtgg gtgttgagtt tttgtgggag tagtgaagtg 6600
   tttttgtggt tgatgggttg gttatttttg tttttttttt gatgtgatag ttgtgtttgg 6660
   gttttgggaa atttgttagg aggtttggta ttggtttgtt ttattggagt ggttttgttg 6720
   agttgtgagt tgtttgtttt tttgaggttt ttagtttatt ttaatttttt atgtggattt 6780
   ggttatttat tttagaagtg tttgttgggt gttgttgagt gttaggtttt gtttgttttt 6840
   ttttttgtat gttggaggtt ttgggtatta gttttgtagg gtggtttggt tttgggtttt 6900
   tagggtagag tatggtattt tttgttttta gtttagtttt tttttgtttt atttttttga 6960
   ttttattttg ggtatgggtt tatttgtagt ttttgtgaag ggagatattg agttgttgtt 7020
   ggtagaggag gggtggtttt tgggtattgt ttgttaatgg ttttttggta gttttgtgat 7080
   agttgagttt tgtttttagt ttaatttgat ttggtttttt aaagtatgat tgttggtagt 7140
   ttgaaaattt ttaggatagg aaataggagt ggtgtaggga atgtaagatg attttagttt 7200
   taagttattg ggtggttttt agatttaggt tgtttgttgt ttttggtagg aatttaagtt 7260
   gttggttatt tttagagtgg tgttggtttg gggtttttag tggtttttgt gtttgggtga 7320
   gaggaatttt ggatggttat tttgttttga gtgtgtgggt ttttagaagt gttgggttag 7380
   ggggtatgga gggttagaaa gttttttttg gagtgttgga tttttttgtt gatttttatt 7440
   ttattttggt ttggggtttt agagaagggg tttaggtagg agtgttattt ttttttaatg 7500
   gggatgtggt tttagttttt gtttaggtgg gtgtattgtt ttttttttgg ttttttttag 7560
   gttgtttaaa tgatttgtga tgaggtttgt gttatggttg gggttttttg ttttttttag 7620
   tgttgtattt gggaattagg ggttgggttt tggaagttga ggatgttttt gaatgttatg 7680
   tttgggtatt tgttttggtt aggtgaggtg tgttggggta gtggttgaga tgggagtagt 7740
   agtattgttt gttaggtttg agatgggttt tgggtgagtg tggttattag gaagggttgg 7800
   gtagagtagg tgggtgggtt gtttttgggg ttttgttttg tagattaatt tgatttgtat 7860
   tttggggttg gggttttttt ttatagtttt tttatgaatt atggtttttg tttttatttt 7920
   aaattagatt ttagaagagg aataatgttt atgtaattta agttagttgg aagtgggtta 7980
   tatttaattt ttgttatttt ttagtttgtt aaggatttga ttaatttaat ttagagggat 8040
   ttggagggtt gtttgtaaaa tttattaata atagggaatt ttagttttgt agttataggg 8100
   tttatagagg tgttgggttg ttgggttatt gttgggaggt gagttggtgg tgtagatatt 8160
   atggaggtat tggtagtgga agagttgtaa gtgtatatag aggttggtat tgaggggtgg 8220
   ggaggaggag gtggtggatt ttttgttagt gttggtatta tgttgttttt agtattagag 8280
   atttaaggtg tggttgattt tggttggggt gatgagttat tggatagtag agtttttttg 8340
   gtagttgggg gttaatgttt taattgttgg gattttgtat tatgttgttt tttttttttg 8400
   ttttgtttgg tttggtttgg tttggttttt gagatggagt tttattttgt tatttaggtt 8460
   ggagtgtggt ggtttaattt tggtttattg taatttttgt tttttgggtt taagtaattt 8520
   ttttgtttta gttttttgaa tagttgggat tataggtatg tgttattatg tttggttaat 8580
   ttttgtattt ttagtagaga tggggttttg ttatgttggt tgggttggtt ttaaattttt 8640
   gattttaggt gatttatttg ttttggtttt ttaaagtgtt gggattatag gtatgagtta 8700
   ttgtgtttag tttgtattta tgtttttttt tttttttttt tgagatggag ttttgttttt 8760
   gttgtttagg ttggagtgta aaggtgtgat tttagtttat tgtaattttt attttttggg 8820
   tttaagtgat ttttttgttt tagtttttta agtagttggg attataggtt tgtgttatta 8880
   tgtttggtta atttgtattt tttagtatag atggggtgtt tttatgttgg ttaggttggt 8940
   tttgaatttt tgattttagg tgatttgttt gttttggttt tttaaagtgt tgggattata 9000
   ggtgtgagtt attgtatttg gtttatgttt tttattatta ttatttaatt ttttaaggaa 9060
   agtaatttaa attgtatatt gtattttttt tttttttttt gttttttgtt gttagttttt 9120
   tgtattattt attgttaaat aatatgaata ggaaattgaa aggaaaattt taataattat 9180
   tggttgtagt attttttttg tgttttattt agtgtttgtt tttttaggaa tatgatatag 9240
   ggtttgattt aattatgaaa ggtgtatatt tttagagttt tatatttttt atagaatgaa 9300
   tgttgaattt tgtttttttt atttgaaatt agattttagg tatggatttt atgattatta 9360
   ttataatggt tttttagagt ggatattgta ttttgtaggg aattattttt tttttagttg 9420
   ttggtgtgga tgtttttttt tgatggtgtg ggtttgggtt tttgaagggt ggtgttttga 9480
   gtaaagatgt tatagtagtt atgtttatag ttttgggttt ggggttaggt ggagattggt 9540
   tttaatttta gagtttgttg ttttaaaata aatttttttt tttttttttt tgagatggag 9600
   ttttgttttg ttgtttaggt tggagtgtag tggtgtgatt tttgtttatt gtaagttttg 9660
   ttttttgggt ttatattatt tttttgtttt agtattttgt atagttggga ttataggtat 9720
   ttgtttttat gtttggttaa ttttttgtat ttttttagta gaggtggggt tttattatgt 9780
   tagttaggat ggttttaatt ttttgatttt gtgatttatt tgttttagtt tttaaagtgt 9840
   tgggataaaa taaattttta gggttgggag tggtggttta tatttgtaat tttagtattt 9900
   tgagaggttg aggtgggtag attataaggt tagaagattg agattatggt gaaattttgt 9960
   ttttattaaa aatataaaaa attagttggg tgtagtggtg ggtgtttgta gttttagtta 10020
   tttgggaggt tgaggtagga gaatggtatg aatttggaag gtggagtttg tagtgagttg 10080
   agattgtgtt agtgtatttt agtttgggtg atagagtgag attttgtttt ataaataaat 10140
   aaataaataa ataaataaat aaatttttat atttatttgt attatttatt attattatta 10200
   ttaaataggg ttttgttgtg ttgtttaggt tggagtgtag tggtgtgatt atgatttttt 10260
   agttttgatt ttttagtttt aattggtttt ttttttttag ttttttgagt agttgggatt 10320
   ataggtgtgt gttgtatgtt tggttaattt tttttttttt tttttttttg agatagagtt 10380
   ttattttgtt gtttaggttg gagtgttgtg gtgtaatttt ggtttattgt aatttttggt 10440
   ttttggatta aagtaatttt tttgttttag ttttttgaat agttgtgatt ataggtatgt 10500
   gttgttatat ttggttaatt tttgtatttt tagtagggat ggggttttat tatgttggtt 10560
   aggttggttt taaatttttg attttgtgat ttatttgttt tggtttttta aagtgttggg 10620
   attataggtg ggagttattg tgtttggtat gtttggttaa tttttaaaaa atttttgtag 10680
   tagttgggtg tggtggttta tgtttgtaat tttagtattt tgggaggtta aggaggttgg 10740
   gagtttgaga ttagtttgat taatatggag aaattttgtt tttattaaaa ttataaaatt 10800
   agttgggtat ggtggtatat gtttgtaatt ttagttattt aggaggttga ggtaggagaa 10860
   ttgtttgaat ttaggaggtg ggggttgtag tgagttgaga ttgtgttatg gtattttagt 10920
   ttgggtaaag agagtgaaat tttgttttaa aaaatttttg tttttgtttt tgtagtgata 10980
   gggttttgtt ttgttgttgt ttaggttggt attgaatttt tagttttaag tgattttttt 11040
   gttttagttt tttaaagtgt tgggagtata ggtgtgagtt attgtgtttg gtttttattt 11100
   gttttggttg tttttttttg ttttaggtaa ttatatattt ttttaggtaa ttaagaaaag 11160
   attgtgtatg atgagttttt tttttggtat ttttgggatt gtttttaaaa ttagataagt 11220
   gtttttattt tgatatttta ttaagatttt aatgaataga atgttgtttg tttttagttt 11280
   gttggttttt ttgtgaaggt ttagggtttt tttgtttgaa aagttatttt tttgttgagg 11340
   tttttgattt ttttttatta tggtagttat gttgagggtt tatagagtta tttttttttt 11400
   attttttttt tggtttttta agatgtattt gtatttttgg agatgttttt ttgttttttt 11460
   gggttatttt ttttggttgt tatttatttt gttgatttat tatttgtgta atagtttttt 11520
   ttggaatttt taggtttttg ttatttattt tttgtagttt ttgttttgtt ttttttgttt 11580
   tagttttttt ttttattatt tttttatttg ttggttttta tttttgttta tggtatattt 11640
   ttgtgttata ttttgatggt tggataaagg ttgagtgaaa tgtatttatt tttttggtga 11700
   ttggggtgtt tattaattag aagggattta aggttggtgt agtttttttt tgtattttgg 11760
   ggtattgttt tgtggttttt ttttgttgga gtttgtagtt ttttgttttt ttttttttaa 11820
   attttttgag atggagtttt gttttgttgt ttaggttgga gtgtagtggt gagattttag 11880
   tttattgtaa tttttgtttt ttgggtttaa ttaatttttt tgttttagtt ttttaagtag 11940
   ttgggattat aggtgtttgt tattatgttt ggttaatttt ggtattttta gtagagatgg 12000
   ggttttttta tattggttgg gttggttttg aatttttgat tttaggtgat aggtttgtgt 12060
   tggtttttta aagtgttgag attatgggta tgagttatta ggtttggttg ttgagattat 12120
   ttttataagt tattaggttt ggttgtagtt ggtagttttt gattattggt tttgttttat 12180
   gtgatttgtg attttttttt tggttttttg gttttgtttt gtttatttgt ttattttttt 12240
   tatgtatgtg gtttgtttat agggtatgta atggggttta attaggaaat ggaaattgtt 12300
   tgagtattta tttatttatt tatttattta tttatttatt tatttttttt aagataaggt 12360
   tttattttgt tatgtaagtt gaagtgtagt ggtgtgatta tagtttatgg tagttttgaa 12420
   tttttgagtt taagtgattt ttttttttta gttttttaag tagtttggat tataggtgta 12480
   tattatatgt ttgtttaatt ttttttttta tttttgtaga gatagggttt tgttttgttg 12540
   tttaggttgg ttttgaattt ttaattttaa gtaatttttt tgttttagtt ttttaaagta 12600
   ttggagttat aggtatgtgt tattatgttt tgttaatttt tgtatttttt gtgtgtgtgt 12660
   aaagataagg ttttattatg ttgtttaggt tggttttgta tttttgggtt tatgtgattt 12720
   ttttgtttgg gttttttaaa gtgttgggat tataggtggg agttatagtg tttggtttat 12780
   ttgagtattt agaatagaag gatttaaagg tggggaatta gttatataga tgatgggaga 12840
   ggtgaaggtt agatagggaa tagagaggta attagtagga aattaaagtg gtgtgggttt 12900
   atatagtttt ttaaaggtat ggatgtaatg atttatttag aatgagaaaa taaataatag 12960
   taaattataa attataagta gttgttaaat attataaata ttattatatg tagaagagta 13020
   ttagttaatt gttagttata tttttaaagt tttatttttt ttatattttt ggtattattt 13080
   ttttaatatg atgattttgt aatattgttt tttattttga gaatagaaaa ttgattaggt 13140
   tggatatagt ggtttgtgtt tgtaatttta gtattttggg aggttaaggt aggtggatta 13200
   tttgaggtta ggagtttaag attggtttgg gtagtgtggt gaaattttgt ttttattaaa 13260
   aatataaaaa ttagtagggt atggtggtat atatttgtag ttttagttat ttaggaggtt 13320
   gaggtgggag gattgtttga gtttaggagg ttgaggttgt agtgagttgt gattatgtta 13380
   ttgtatttta gtttgggtga gagagtgaga ttatgtttta aaaaaaaaaa aaaaataata 13440
   aaaaaaaata taggttgggt gtggtggttt atgtttgtaa ttttagtatt gtgggaggtt 13500
   gagatgggtg gattatttga ggttaggagt ttgaaattag tttggttaat atggtgaaat 13560
   tttattttta ttaaaaatat aaaaaattag ttaggtgtga tgatgtatgt ttgtaatttt 13620
   agttatttgg gaggttgagg taggagaatg gtttgaattt aggaggtaga ggttgtagtg 13680
   ggttaagatt gtgttattgt attttagttt gggtaataga gtgagatttt attttaaaaa 13740
   taaataaata aataaagtga atgaattttt ttagttgatt aaattttgtt tttatttttg 13800
   gtagttggga tgtatataaa agtggtggtt aatatggagt tggtggtgtt gttataattg 13860
   ttttgtttaa gatatatagg aattttggta aattttgttt tttttttttt ttttaaatgt 13920
   gatttttatt aagaaagaag taggaaaatg ggggagattt ttaattgtat atgaggtatt 13980
   tgtgtatgta tttttgatta gagagatttt tttgttttga ttttatattg aaggaattga 14040
   attttttgtt tataatttta tttttggtga tgggagtaat tttttataga tttgtttttg 14100
   gatttgtgta ttttaaattt tgtttttttt tattgtgtat gtttttttgg tgttaggtat 14160
   tgtaggatat gtttgtgttt tgtgattttt gattttgtgt ggtaggtgga attggaatgg 14220
   tgagtggagt gagtattgtt ggaatttgtt tttatattgg gatagtgagt agtaattgaa 14280
   ttatatatgg aaataaatgg gaattatata aatataattt gattaaagtt aaattaaata 14340
   tttttttagt ttaatttttt tttagtagga gtttttatgt ggtagtaagt ttttgttttt 14400
   attttatata agagaaaatg tgatgggtag gaatttaaga ttggaaatga atggggatgt 14460
   taattgattg tagggaaaat attttttatt ttgtaaattt tataaaaaat gtttgatttt 14520
   gtggatatgt ggttagggtt ttttttagtg ttttgagagg gaattatgta ggtagaggga 14580
   gggtttttag tattgtgggg gtgatttgga gattggtagt agggtgttat tattattttt 14640
   aggtggggat gagagggagg aagttttgtg attagagttt aggggttaga gttatttgtt 14700
   gggagtttaa ataaaggtgg gattttggta ggagttggaa ttatatagag gattattttt 14760
   tttgttggag aagttgtttg aggtagagga ggtggagagt ttttgtgttt ttttttgttt 14820
   ttatttttta ggtttttttt aagttttttg ttggttgaat ttagtttaaa gttagtaggt 14880
   ttggggattt ggatatttga gttagtagag gttggtgttt tttttatata aaggagggta 14940
   taagaagtgg gaggtatgga gggggattag gttaggattt atataggatg tgtaaatagg 15000
   agtttttttt tttttttatt ttaaatggta tttgtagttg gttttatttt tttgttgtta 15060
   gataggaaga ggaattttgt ttttggtatt agttgttttt attgtttttt attttatgga 15120
   ggtttttttt ttttttggtt tttgtttgta attattggtg atgttgatgg attatatttt 15180
   ttggggagtt tggttatgaa tatggttttt tatttgttgt ggattaaatt gtgttttttt 15240
   ttttgaattt ttgtgttgaa gttttaattt ttaatgtagt tgtaattgaa gatagggttt 15300
   ttagggaggt aattaaggtt aaatgaagtt atagggtagg attttaattt aataggattg 15360
   atgtttttat aagaagaggg agagatatta gagatttttt tttttttttt atttgtaaat 15420
   agaggaaaaa tgatggagga tatagtaggg aggtgggtat aagttaggaa gaggggtttt 15480
   gttgggaatt aattttgatg gtattttgat ttgggatttt gggtttttta aattgagaaa 15540
   tatatgtttg ttgtttaagt tatttttttt atagtaattt ggatggtagt ttgagtttat 15600
   taaggtattg tttgtattag ttagggtttt ttagggagat agttaatagg agatggatgg 15660
   atggatggat ggttagatag atagataagt ggatagagag agagagagat aggtagatat 15720
   agatagatag gtagataaat ggttagatag ataagtagat agagagatag agatagatag 15780
   atataaatag gtaaatagat aggtagataa atggatagat agatgataga tagttaggta 15840
   ggtagataga tatagatagg tagatagata gatagataga tgataggttg ggtttagtga 15900
   tttttatttg taataattag tatattggta ggttgaggtt ggtggattat ttgaggttag 15960
   gagtttgaga ttagtttggt taatagggtg aaattttatt tttattaaaa atagaaaaat 16020
   tagttgggtg tggtggtgta tgtttgtaat tttagttatt tgggaggttg aggtatgaga 16080
   attatttgaa tttgggaggt ggaggttgta gtgagttgag attatgttat tgtattttag 16140
   tttaggtgat aaagtgagat tttattttaa aaagaaaaaa aagatagata gatgaatgga 16200
   tagatagtta gatagatgat agatggatga taggtagata gataattgat aaatagatat 16260
   aaatgattga tggataggta gatatagata tattgattag gtagatgata gatggataga 16320
   tagaagatag atggtggatg ggtgggtaga taggtagata gatagttaga aagatagatg 16380
   atagatagat aggaaggaat ttattagggg aattggttta tattattgtg gaggttaaga 16440
   tgtattatag aaggttgttt gtaagttgga ggtttaggaa agttagtatt gtggtttagt 16500
   ttaagtttgt aggttttagg attgggaagt tgttgatgta atttttagtt taaggttaaa 16560
   ggtttgagag tttggggaat tgttggagaa ttgggagttt taagtttaag ggtaggagag 16620
   gaaggtattt tatttttagg agaaagaggg aatttatttt ttttttgttt ttttgttgtt 16680
   tggtaatgga atggtgttat ttatattgag ggaggatttt tattatttag tttattaatt 16740
   tatatgttta ttttttttag aaatattttt atagatatat ttagaagtga tgtgttatta 16800
   gttatttttt aatttagtta aattgatatt tgttgttagt tattatatag gtttagaaga 16860
   attgtggttg tatggtgatg gtttttattt tgtttaaaga agttgaagat gaattgttgg 16920
   tataagttat tgttttttta ggtttttttt agttgtttat ttttttaaaa atgtatttat 16980
   tgagtatttg ttgtatgtta ggtgttgtga taagtattgg ggtatagtag tgagtaaata 17040
   aggatttttg ttttttggag tttatatgta gtagaaggaa atagaattaa tgaatttatt 17100
   agttttagaa attattaaaa taggattaag agttgtggtg agaattaaga taaggtgatt 17160
   gtatagaaag tgattgatgg ttgttttgga atgagtggtg aggggttata gagatgatga 17220
   tagttgagtt gagattggga agataggaag gagttagtag tgtaaggatt aggttggaag 17280
   taagtttggt gtattttagg aatagtaagg aggttagagg ggttgtagta gagtaggtga 17340
   ggaggaggag ggagatgtag gttaaagagg agtttggggt tagatggagg attttttttt 17400
   taaagtttag ttattttttt tttaaaggag aatgtgttat ttttaaaagt tatatatgtt   17460
   agtatagtgt ttggtttatt atatgttttt agtaggtggt gagtatggtt gtttttgtta   17520
   ttattatatt ataattatta ggaataatgg agttattagg ttttgtattt atagtaatgt   17580
   gagggagtta gtaagttttt ttggggaatt tatttttttt tattaagttt ataagagttt   17640
   gataattagg ttaatgtagt gagagttata gttgtttagt gtattggatt ttttagttat   17700
   gggttaggat atttttattt tagtagaaat ttttaagggg attggggagt gtgttgtgga   17760
   tgtttttggg ttatttagat tttttgggga gtttttttta ttgtagttgt tgtagggagt   17820
   ttagttgtag tgatgaattg tggtttagtt ttgtttatgg gaaaatttta tatgggagat   17880
   gggatggttt tttgttggtt tttttggttt taggttgttt ttataagtgt agggattttt   17940
   atttgggtgt gtgaatgggg tttaggatgt tttaagggta tgtggagatg gggttagttt   18000
   gttttttttg atgtttttga attttttttt tttgtttttt tgtattttat tgttttttgg   18060
   ttttttttat tttgggtgtt ttggaataat atgtaattta atatataggt tgattttatt   18120
   attttgtttt gttttatttt attttatttt ggaaataaag gttttgttat ttagtttgga   18180
   gtgtagtggt gtaattgtaa tttattgtag ttttgaattt ttgggtttaa gtgatttttt   18240
   tgttttagtt ttttaaagtg ttgggattat aggtatgagt tgttgtgttt ggtttaggtt   18300
   ggttttaaaa gttttttatt ttttttgtat tttgatgata tttatttttt ggagtaggtt   18360
   tttagttatt ggttttggat attttgtagt tattattggt tagttgtggt atgtgaagtt   18420
   gaggtttagg agaagggtag ttttgtttaa ggttatatgg tttagtgtta atatttagat   18480
   tttagtgttt ttattttttt tttggttgtt tttattttta gtgtattttt ggaggtttaa   18540
   gggagttaaa ggttaggttt tggttagtta agaatttaaa aaagttgttt ttgtgttttt   18600
   ttgttaattt attattttat tatttttttt gataatgaat ttagtttgaa ttttagataa   18660
   ataaggatta atttttagta taagtatatt ttataataga tttgggatat atttatgttt   18720
   aaaaatgatt tattgtttgt tggttttagt agtttatgtt tataatttta gtgttttggg   18780
   aggttatagt tggagaattg tttgagttta ggagtttaag gttatagtga gttatgatta   18840
   tgttattgag ttttagtttg ggtgaagtag taagattttt atttttttaa aaaattgttg   18900
   attgtttatt tgaaatttag gtttaattga gtattttgta ttttatatga gaattttata   18960
   taggtgagat ttagagtttt aatgtttatt ggtaagtttt tgtagggagg ttttgtgggt   19020
   ttttggttgg tattgatgtt ttgtgatgag tagggttatt tggtaaggtg gatgtttgtt   19080
   gtttatgtag gtagagtaag gttttgggta ggtatgagtt gattttattt tttttagttt   19140
   gggagttaga tattgttggt agtttgttgt taagagaggg gttggttatt aagagagggg   19200
   tggtttttat gggttttttt tggtttttgg ttttttttta gttttttttt tattttaaat   19260
   agaggttaat ggtattgtat tagaattagt gtgttgtttt ttttggttgg tattttgtag   19320
   ttgggtagat tgtttttttt ttataggagt tggtggtgtt ttttttgttt tatagggagg   19380
   atggttgaga tttttgattt tgatataggg tgttgttttt tttttgtttt gagatagagt   19440
   tttggttttt ttatttaggt tggagtgtag tggtatgatg ttggtttatt gtagttttta   19500
   ttttttgggt tttagtaatt tttttgtttt agttttttga gtagttgaga ttataggtgt   19560
   gtattattat tgagaataat gttttttata gtgtttattt ttttgtagga gtaagttttt   19620
   aaggtaagga ggttgttttt tttatggatg aataaattga ttttgattta tagttttatg   19680
   tgttagtttt gagggtgggg aaaggttgtt ttgtgtttag gtagggggtg ggggtaaggt   19740
   taggagggtt tggtgtttag tgtatttttt taggatataa ttttaggtag tagaggttaa   19800
   ggatagaggg gaggagaaga gggaggtggt tattattttt ttattttagt tttttttgga   19860
   gataagtttg agtattttag ttgtttatta agtttggaga taagtttgga atttaaattt   19920
   ttgattttga tttagttttt tatagttttt gtgagtaaat tgagttttag aaaaagggag   19980
   tgaagtttgt gtattttgga tgtttttatt ttaggtttaa attgttttta ttttaggttt   20040
   aaattgtttt tattttagtt taaattgttt ttattttagg tttaaagtgt ttttatttta   20100
   gtttaaagtg tttttatttt aggtttaaag tgtttttatt ttaggtttaa agtgttttta   20160
   ttttaggttt aaattgtttt tattttagtt taaattgttt ttattttagt ttaaattatt   20220
   tttaatatag gtttaaatta tttttaattt aggtttaaag tatttttatt ttaggtttaa   20280
   agtattttta ttttaggttt aaagtgtttg taatttaggt ttaaagtgtt tttattttag   20340
   tttaaattgt tttattttag gtttaaattg tttttatttt aggtttaaat tatttttatt   20400
   ttaggtttaa attgttttta ttttaggttt aaagtgggaa ggagttatag ggaaataaat   20460
   gggggaggta tggatgatta ggatgagggt ttttatgatt tatttttttt taggggttat   20520
   tttaagagtt ttttaaattt ttattatttt ttaatagaga tggggttttg ttgtgttgtt   20580
   taggttgatt tttaattttt agttttaagt tattttttta ttttagtttt ttaaattgtt   20640
   gaggttatag gtgtgagtta ttgtatttgg ttttttttag gagttttaag aaaaaaaatg   20700
   ggaatagatt tgtgtgtgtg tgtgtgtgtg tgtgtgtgtg tgtgtgtgtg tgtgttttga   20760
   gaaagagttt tgttttgtta tttaggttgg agtgtaatgg tatgattttg gtttattgta   20820
   atttttgttt tttgggttta agtgtttttt ttgttttagt tttttgagta gttgggatta   20880
   taaatatgta ggttgatttt gtatttttat tagagatggg gttttattat gttggttagg   20940
   ttgattttga attttggatt ttagatgatt tgtttgtttt ggttttttaa agtgttggga   21000
   ttataggttt gagttattgt gtttggttag gaatagattt ttttttgtag atgatttttg   21060
   ttattattga aaatggatgt ttatgtataa ttgaattgtt ataatgtttt attttaaaaa   21120
   gttttttttt gagaaaatgt gatttttgat agaggagaga ttgtttaggt tttatttatt   21180
   gttagtataa aggtagagta ggggtgtggt ggtgttattt ttttttgaga tagttttatt   21240
   tttgttttgt ttaggggttt agaagtttgg tttatttgag tttatagagg ggtttggagt   21300
   ggttgtttta tattataggt ggggtttgta aggatttgaa atgatttttg agaaggtggg 21360
   tgtggtgggt gtattttaat tattaaagta ataataatga tatattaaat agtgtttgtt 21420
   tagattttga gtgtttgttt aaatattttt attttgtgat ttgttagatt ttagtataat 21480
   aatttaggta aaagaaaaaa tagttgggtg tggtggtttg tgtttgtaat tttagtgtga 21540
   ggttaaggta ggaggattgt ttgagattag gagtttgaga ttagtttggg ttatttgaga 21600
   ttttattttt gaaaataaat aaattaataa aagattttta tgttttatgt tttttttata 21660
   tttaggtttt ttaaattata tgtattgggg aaagtttatt aagagaagtt tttggttggg 21720
   tgtggtggtt tatgtttgta attttagtat tttgggaggt taaggtggga ggattataag 21780
   gttaggagtt tgagattagt ttggttaata tggtgaaatt ttatttttat taaaaatata 21840
   aaaattagtt gggtgtggta gtgtatgttt gtaattttag ttatgtagga ggttgaggta 21900
   ggagaattat ttgaatttgg gaggtagagg ttgtagtgag ttaagttggt gttattgtat 21960
   tttagtttgg gtgatagaaa aagattttat ttttaaataa ataaataaag ggagagaagt 22020
   ttttgggttt ttagatatat gggttagtgt ttatttttgt aaagttgttg tgtttaaaga 22080
   ggggttgtat ttggtggaat tggggtaaaa gagtagggag ggtattgggt agttgatttg 22140
   gtttttatta ttggaaaggg gttgtgttgt tggtgttttg agttggtttt ataaggaaga 22200
   tattgtatag ggtgttttaa gtttgttttt tttgtttagg tttatttttt tttttttagg 22260
   attagggagt tttgtttgtt ttttgttttt ggtatttgtt ttttggtttg tgagtattgt 22320
   attttttttt gtttggaatg ttggtttatg gtgggttttt ggatttttgt tttttgttgt 22380
   tttttgttat attttgttaa agtgtgtttt atttatttat tgttattatt ttttgaaata 22440
   gagttttgtt ttgttgttta ggttggagtg taatggtgtg attttggttt attgtaattt 22500
   ttgttttttg ggtttaagtg atttttttgt tttggttttt tgagtagttg ggattatagg 22560
   tgaatgttat tatgtttggt taatttttgt atttttagta gagatggggt ttttttatgt 22620
   tggttaggtt ggttttgaat tgattttggg tgattggttt atgttagttt tttaaattgt 22680
   tgggattata ggtgtgagtt attatgtttg gttaattatt ataatattga tattataata 22740
   atattatagt tagataatgg tgattaatgt ttgtaatttg aatattttgg gaggttgagg 22800
   taggtagatt atttgaggtt agtttgagat tagtttggtt aatgtggtga aattttgttt 22860
   ttattgaata tataaaaatt agttggagtg tgatggtagg tgtttgtaat tttagttatt 22920
   tgggaggtgg aggttgtggt gaattgagat tgtgttattg tagtttaatt tgggtgatag 22980
   agtaagattt tattttaaaa gtagtaataa taataataat aataataata ttatttattg 23040
   agttttaatt ttgtgttagg tattgtgtta gtatttgtgt tatgtgattt tattgatttg 23100
   gaataattta tgagtaagta ttatttttgt ttttaaggaa gttgagattt aggaaatgga 23160
   agttattttt tagggtttat agtaagggtt tggtgaggtt tgtgtttaaa ttttgattat 23220
   ttgattttag gtttttttgt tttttttgtt ttttaaaaat ttaaatattt ttatttggga 23280
   tgattttaga tagttagatt ttaattgttt tttttttttt tgagtatgtt ttattttaaa 23340
   attatataaa gataatttat tttttaattg gagttatgga attgttagat gttttggaga 23400
   tttttttagt tatttttttg ttggatttag atataggttt gaataattag gtttaaggag 23460
   ttgaattgga gttgtttgtg gttagaggtt tttttttggg aatagtagtg gtggtagtgg 23520
   ttgtgttgag tttggtaatt tgtttaagag taggggatgt ttaggttgtg tttgtttttt 23580
   ttttatgaat ggtttgtttt gtgttttttt tgtgttaagg gttgttaggt attagatgtt 23640
   tattttagga aattagaaaa ataaatagtg taaataaaat aaaaatttta tttaagaaat 23700
   ttgagataga tgttgttttt tttattgttt ttttattttt atgttgggtt tggagagagt 23760
   gggttagtat gttttttatg gataagaggt tttggggtat ttaatttgga tggaattttt 23820
   tttatttttt ttttgtttgg tgttaggggg tagaaggtag ggtggtgttt tatttggttt 23880
   agggaggagg atagggtagt ttttgttgtt ggtatttttt gtagagatgt tttagttaga 23940
   agtgatttta tttttaggaa tgtgtgtttt gttttttgtt gggtggtttt tttttatttt 24000
   ttattgtttt tatagtgggg tgtgttagat ttatgggtag agttttggtt tatttttagt 24060
   tttggatagt agaatgtttt agtagtgtat tgatggtgtg tgtgtgggga tgtgtggaat 24120
   aggggatagt gattttgtgt gtgttatatg tagtgtggtt tttagttggg tgtgtgaagg 24180
   gggtgtttat ggttattgtg gaagtgagtt tagttggggt tggagggttt aggtttattg 24240
   tatgttattt agtagttttg ggagtggggt ttattttttt ttttgttttt tttttttttt 24300
   ggttttgggt agtttattgg agttgtattt tgggttattt tttttggttt taaggaggtt 24360
   gttatttttg ggaggtagat gttggttttt ttatttttgt tttttggaga tttgtgttga 24420
   ggtttgtttt tttgtttgga gggattggtt aggaattttt gggttttttt ggtggggttt 24480
   ttgttgttta gtagtgagta gagtgagtgg gtattaagag gaggatgttg ggtgaggtta 24540
   tggatatggt tggggtaggt tgggggtgtt ggaggtgaat tttgtagttg tgtgattttg 24600
   ggtagtttgt ttagttttgg gttttgtttt tttttttatg aaatggggtg ggagtatttg 24660
   tttgttgggg ttgtgggttg ggtgaattgg agttaaggta tagtttatag tttgttgtta 24720
   gatttttgta agtagaagtg ggtgtttttg tttagtgttt tttggttttt tttttaaggt 24780
   ttagttttgt ttttattttt tttatgaagt ttttttggtt attttaggag ttatgattta 24840
   tatttttttt ggagttttgt gttatatgta ttttttatgg tgttgttttt tttggaggat 24900
   gggtttaatt aaaattgggg tttatttggg ggtttttttt agtattagag aagtttaggt 24960
   ttagtagaga tgggtatttt gttaatatta gttttgtgaa ggatatattg tgtttatttt 25020
   gttgttagtt gttgagtttg gtgttttgta gttatggaat atgtgtatag gtttgggttg 25080
   ttggatatga aggttagatg ggtagtttat tttgtttttg ttttgagttt ttagaaataa 25140
   gttggttgta tttattttta tttggttata gtttaggagt ggggttgtgg aatggatgga 25200
   aggaaggatg agtgggtggg tgggggggtg ggtgggtggg tggatggatg gatggatgat 25260
   gggtggatgg gttgatggat gggtggatta ggtggatagg tgaatggatt ggtgggtggg 25320
   tgggtgggtg aagggttgaa taggtgggtt gggtggatgg gtggatggat gtgtggaggg 25380
   gtggtggggt ggatggggtt tatgggtgaa tggatggatg gatgggtgaa tggagtaaat 25440
   gagtgggtgg atggatggat ggatgttgta agagtttttt tggagggttt tagggaggaa 25500
   ttggtatttt tgattagtag ttagtatggt gattagatat agggaggtgt ttgggtttgt 25560
   ttgatttttt attttattgt tggtgggatt gggttggtta tgtgtttttg aagttttttg 25620
   aagggtggtt gtatggtatt gtttagggga tatttttaga gtgttgtgtt ttatagtaat 25680
   agggtggttg tttgtgtttt tagggttgtt ttttttggta gttttttggg tttgtttggt 25740
   ttagtatttt agtaaatttt tttgagttag gatttgtttg ttatgagttt ttttaataag 25800
   tagttttagg ttaaggtttg tgtgtgggta gagggagtgt atgttgtggg aggttgtatt 25860
   aatttgaggt tttgtttttg tttttttttg gggtaaaagg taaaaatagt ttgttttatt 25920
   ttatttttaa atttagtttg gttgtattta gttttgggag tgaagggggt tgggttagga 25980
   ttgtttgtga gttgtagatt ttggtgtagg gaaattttga tttataagat gggtggatat 26040
   ggaggaagga ggagattttg tttagtagag atatgtttgg gtgtagttta gtttggagtt 26100
   tgatggatgt gggtttattt gtgtgtaatt tttatttatt gtgtggttat agggaagtga 26160
   tattttgtgg agatttagat tttttatttg taaaataggg gtagtagggt ttgggggagg 26220
   atgtgtgtga aggtttggtt tagggttttt ttagtagtta gatagtagtg gttgttattt 26280
   ttttagtgtt tagggttatt tgggggagta gggtttttgt ttttagatgg gttttgtttt 26340
   tggttttgga atttatagta ggagtaaagg tgaggatatt tttggtgtta attatttatt 26400
   tggagggaga gtgttagtgg ttttgtgtga tttttgtttt ttgtttttgt ttttaggagg 26460
   tatgtggatt tttagtggtt ggttttggag gtttggtgat tttagtggtt taggtaggtg 26520
   gtttgttttg ttttggtttt atttagttta tgggtgtgtt tgtgttgggg tttttttatt 26580
   ttggtttggg atttgttttt ttatttggtt tttttggatg agtggggtta tttgtttatt 26640
   ttttagggag atgtagtttt tgatggtttt ggatttaggt ggtttttagg tttgttgatg 26700
   ttgttttgtt ggagatgatt tttgtggggg taggggtagt tatttttagg tttagggagt 26760
   aagtttgtgt gtgtgggata gaggagagaa taggagatag atagggttgg atgatttggt 26820
   ttgagatgtt gaggggatag atgtgttatg gataggtggt tttggttgtg agttttttgg 26880
   tgttggttta gtatttgtgg gttttttgta attttgtagg atggaagatg ttttaggata 26940
   gagtagatat gtttggtgtt atttaggagg aaataagtgg gtttttggta gtggttattg 27000
   tgttattttt tgtgtttttt ttatgtgttt ggaaattttt tatattaatt agtagggaag 27060
   ggaaatagat gtatttatta gagttttggg gttggttgtt taggatatga gttatagttt 27120
   ataggtttga agggatttta ttaatattgg gtgtagtttg ggtaagggag tgggatagag 27180
   aggttagggt tttgttttta gtttttgttt tttagggttg agtttgtgtg ttattttttt 27240
   tttattgggt gtttttgatt ttttttgttt gattttagtg tggttatgtt agtgtttttg 27300
   tttttgggag tttatgttgt ttttggttgt tatttttagt ggttaggttg ggataggtgt 27360
   ttatttagtg gtttttttat ttggagagga tgatgggtta tagtaatgaa tggatttatt 27420
   ttttttagga gatgaggagt tttgagaaga gtagagatgg tgtggttatt tgtgtatatg 27480
   tgttgggggt taattgggtg ttagattttt ggttgttatt atggtggtag ataggtaggg 27540
   attgtgtttt tatgaattag ttattttaga ggagataaag ttattaaagg atatggattt 27600
   ggagttaaat ttatataaat agagtgtttg tggattaggg ttgtttttag aataaggtga 27660
   gatagttgtt ttgttaggat aaggtgttta tgggggtaag ggtatgtgtt tatttggagt 27720
   tgttattttt atgttttaga ttagttttgt ttaatagaat tttttgtgat ggtgggtttg 27780
   ttttatatat ttgtattttg ttattttgta tggtagttgt ttagttgttt gggtttattg 27840
   tatatttaaa gtataaatgt ttatgtttat tttatttatt tatttatttg tatagaatta 27900
   tttatttatt ttaaatggat ttttatttat atttatgtgt ggttagtggt tatgaaattg 27960
   aatagtttta gattatattg gtatttgaga ttttagaatt ttagatttaa agtttgtttt 28020
   tgattttgag tttgtttttg tggggatagt atagatgttt tttaagtttg ttttttagag 28080
   gtggatgtag tgtttgtgga ttgtgtatta ttaggtttgg atagtggaga gggtggtttt 28140
   ttatagggag tgggagtgga ggggatgttg tgtttatatt gagtgtatgt gaggtttttt 28200
   gtagtatagg atgggattgt atgtgggaag aggggggtga tgttttggtt ggaggttttt 28260
   gggtagttgt atgatttagt gttgtggtta agaggggttt tgtttattgt agttatgttg 28320
   ggtattaatt attggatagt ttagttgttg ttgtgttttt ggggagtttt tttttggttt 28380
   tgtatttgat ttgttttttt attttttttt tttttattgg ttagtttttg gtggaatggg 28440
   tggggagggt tttttagttt ttgtgggggt tgtattttgg gaatttggta ttttgggttg 28500
   tatggatata gttttttttg gtggttgttt ttttttgttt agattggttg gttttgtgtt 28560
   gttagtattt ggtagttatg attatagttt agtttggaag tttaaggtat aatttgttga 28620
   attttaaagt tgtaagttgg tgatttgggt ttgtgtggga ttgtttgttg atatatgggg 28680
   tttttttttt gtgggttttt gtgggttttg gtttgagata tttttttttt atttttttgg 28740
   tttttttttt tttttttgtt ttttttttgt taagattttg gtttaggtag gagtttattt 28800
   ttagatgttt tagtattttt ggttttttta tttgatattt tttagatgat tgtttgttat 28860
   tttatttttt tagtttagtt tttttgtaga tttatatttg gttaattttt tatgtttggg 28920
   atttttgttt tttttagaag gatgaaggtt ttttttgagg ttgtgtttgg gattgggtta 28980
   gggatgatat ttttggtaaa agtttaaatg atattatgat taatgttttg gttagtttat 29040
   atattttttt tttttttaaa aaaaaaatgg aatttttaga gttataaaat aaaaatgtaa 29100
   agaataaggt aataaaatat ttgtgtattt attattttgt ttaagaagtg agatattgta 29160
   ggtgtagttg tagttttggg tgattttttt tattttagtt ttatttttgt tgtggtgttt 29220
   gtttttttgg attgggattt ggttttgggt aggtatattt gtgttgggta gtggtgtgtt 29280
   tgagaagttt aggttttttt tttttttttt ttgagataga gttttgtttt attgtttagg 29340
   ttggagtgta gtggtattat tttggtttat tgtaattttt gttttttggg tttaagtgat 29400
   ttttttgttt tagttttttt agtagttagg attataggta tttgttatta tgtttggtta 29460
   attttttttg tatttttagt agatataggg ttttattatg ttggttaggt tggttttata 29520
   tttttgattt taggtgattt atttgttttg gttttttaaa gtgttgggat tataggtgtg 29580
   agttattgta tttggtttgg tttttttttg tatatggtta ttagtattgt gtgattgagt 29640
   agtttaggtt tgtttttttt tgaatgttgt tattagtatt atatttttgg tgttttattg 29700
   gagttagtgg ttgttttgtt tgtgtttata atgtgatggt gtggagattt atttgtgtag 29760
   atttatgaag ttttggttta tttattttgg tagttagtga gtgttttttt gtgtggggag 29820
   gtagagtttg tttgttttgt ggtttattgg tgatattgtt gaaggttttt tttgttatta 29880
   gtgttgttgg gaatgtgtgt ttttttaggt ggatttggtt gggttgtgga gatgtgtttt 29940
   ttagtttttt tagatgttat tattatgttt ttttgagtgg ttttttggta ggttttttta 30000
   tgtttgttta ttttttgtgt tttaggtagt tgtttttttg attgagtgtt tagtggattt 30060
   ttgttttgtt ttttatggtg tatggagtat tttattttag tggtgtttga gttttttttt 30120
   ttttggtttt tagattttgg ttgggatttt tggagttaat ttttttgttt gatattttgt 30180
   gttgttaggt tttgtttttt tattttttaa tatttttggg gtgtgtggtt ttttttttag 30240
   tgtttttatt attttttttg ttttaggttg aattgtgttt tttataaatt tatgtttatt 30300
   aaaaatttta gaatgtgatt tgggagttgg agatggggtt tttgtagatg taagtagtta 30360
   aggattttga gatgaggtta ttttggatta gggtgggttt taaatttaat aattggtgtt 30420
   tttaaaagaa gtagagaggt tatagatata ttggggagag ttatgtgaag gtaggagtag 30480
   atgatggagt gatgtagttt taggttaggg agtgtttggg aagaggtaag gaaggatttt 30540
   ttttttattt tattttagag tttttttagg gagtgtggtt ttgttgatat tttgattttg 30600
   gatttttggt ttttagtatg ggagagaatt tgtttttatt tttttaagtt ttatagttgt 30660
   ggtttattat ggtagttata ggatattgat gtatttttta tttggttgtt gtggatggat 30720
   gattttgttt gtaggaagtt ttttttaagt attgtaggtg tttttgtttt tataggggat 30780
   gtttgtataa gtaggtgtga aaaaagggtt ttgtgggggt aaggtttggg ttggtgtttg 30840
   ttgttttttt ttattttatg ttagttttta tgtttttttt ggtaggagtg tttggtttta 30900
   tttggtgaat tagagttaaa tttttttgat aattggttgt tatttattta attgttatta 30960
   tttttaggag atagaaaatg ttagaagggt ggggtttgtg tagtgttttt ttgttatttt 31020
   tttagggtat tgtgttatgt tgttttaatg tatagagtag ttgtttaaag tgtgttggat 31080
   gaataggtgt tttatgttta gttatgtaga taaaagtgtg ttatgtggag ggagttggtt 31140
   gtggaagaga ttattagtat tttggttgtt tgaggatttt gtttttgggt ttttgtaggt 31200
   atttttagtt tattattttt ttttttattt atagttattt attttttttt tatttttttg 31260
   atattagttt tgttatttat ttaggtttgt aagtaggaaa ttttagagtt gattttgatt 31320
   tttgtttttt tagtattttt aggttttaat aggttgttaa gattttatta atttgttttt 31380
   tgtatattga tttttggata ggttgttttg gttatggtta tgttttggtt gggtttttat 31440
   tatttttttt tggattagta ggtgttattt tttttatttt tgttttgttg tggttttttt 31500
   ttttttatgg ttaatattta gaggatgatt attatttttt tttttgtgtg ttggggattg 31560
   ggtgatagga tttttttatt tgattatttt tattagattt gggttgggtt tttttattta 31620
   tagagttttt agtttttgtt tttaaagttt tgagggtgga tttgtgttgt tgtttagttt 31680
   ggttttgata ttgtttttta gtttggagtt ttttgttttt gtgtttttgg ttttgagtgt 31740
   ttgggttttt tttggatggg ttgttatttg ttttttggaa agtagggttt ttatgttttt 31800
   ggatttttta gttgatttag tgattttggg ttattttgtt atattttttt tattagtttg 31860
   gttttggtta tttttgtgtt ttttatagtt ttggattaga gttatggtgg gatgtttttt 31920
   tggtgtgtgg taggtagtta ggaaatattg ttggattgat tttgatagga gatgtttggg 31980
   agaattgttt aaaaaattag tttaaaatgt aaatattggt ttttttttgt gttggttaaa 32040
   gagtagtatt tttttgtttg ttttttgggt aagaaattga tggtgaatgt aatgttttta 32100
   gggaggagaa gaaggtgata aaaagggttt gtataatggg gtatatgtta ggtatttgtg 32160
   attatttaag tttaaattat aatataaatg tgaaattttt tgttttggta gtttttttat 32220
   attgtaagtg tttattgtta tatgtggttg ttatattgat tatggtagat atagaatatt 32280
   gatattattg tggagagttt tatggggggg tgttggttta gattattttt atgttggatt 32340
   tataaattta ttgtttattt agtgaatggt tattgggtat ttattgagta agtaggatga 32400
   aagatatttg tttttggggg agttttttgt tggttgtaga gaagtttaaa tttaggggat 32460
   ttggggtaaa ttttgtaagg gtttttggtt tttgtatttt tagatttttt ttgttttgta 32520
   ggggtagttg ggggtttttt ttaggaatgg agtgttagtt ttagtttttg ttaggttata 32580
   gtttagatgg ttggttgagt atgagtatgt gagagtggaa ttgggtttaa ttgggttgtt 32640
   tttttttgtt gttttggttt ttttttatgt tgatttggaa ggatttttaa agtaataggt 32700
   ttgtttaggt ggtttgtatt agtaaatttt gtagtggttt taagggtaga gatagttgga 32760
   ttgtatttat ttaggaatag ttaaagtttg aggtataata aaaagtggag aaagatttta 32820
   gagaggaatg tagttggttt tagtttggga atttgttttg tttgggaggt tgtaggtggg 32880
   ttaggttggg gttagtttgg agggttttgg ttggttttgt tggtaggtat agtagtaggg 32940
   ttagtaggag ggagtttagg atggagtttt tttattttta atttagggaa gtaaatttta 33000
   gtggttgagt ttgaggatga gtaggaaatg aatggaaaag tttttttttt tttttttttt 33060
   tttttaggta gggttttttt ttgtttttta ggttggagtg tagtggtata attatagttt 33120
   attgtagttt taatttgtta ggtttaaggg atttttttgt tttagttttt taagtagttg 33180
   ggattatagt tgtgtgttat tatgattggt taatttttaa aagaaatgtt tttaggttag 33240
   gtatggtgat ttatgtttgt aattttagta tttttggagt tgaggtggga ggattatttg 33300
   agtttaggtg ttttagatta gtttgggtaa tatgatgaga ttttgttttg attaaaaata 33360
   taaaaagtta gttgggtgtg gtggtatagt tttgtagttt tagttatttg ggaagttgag 33420
   gttgtagtga gttgtgatta tgttattgtt ttttagtttg ggtaaaggga gtaaggtttt 33480
   gttttaaaaa aaaaaatttt tttttttttt gaggtggagt tttgttttgt tgtttaggtt 33540
   ggagtgtagt ggtatgattt tggtttattg taatttttgt tttttaggtt taagtgattt 33600
   ttttgtttta gttttttaag tagttgggat tataggtatg agttattgtg tttggatttt 33660
   tttttttttt tttttttttt gtagagatgg gattttttta tgttgtttag gttggttttg 33720
   aatttttggt tttaagtgat ttttttattt tggtttttta aagtgttgga attataggtg 33780
   tgagttatta tatttagtag gaaaagttat tttttaaagt gagaggttgt gtgtttttta 33840
   tagtttggtg tttattagta tggaggattt attggaatgt gtgttaggtg aggggatatg 33900
   gaagatggta gtaaatttga gtttagagta ttagaaagta tgtttttagt taaagatgtg 33960
   ttttttttat ttttgtgtag tatttttttt tagtgtgtat ttttggagta aggtggggtt 34020
   gattttatga agattttttt ttgaaggttt ttgttgttta tattagtgtt atttggattg 34080
   tttgtttttt tttttggatg gttggattag ttttagggtt ttttggtttg gtgtggggag 34140
   tatagtttat tttggggaat gtttggtatg tgtttttttt ttattgggta ttaggggatg 34200
   aagttgtgtt ttgtggaagg ggatagaggt gttggtgttt aggatggtgt atttggggtg 34260
   gttgaattgg atttggtttt tattgggttt ttgtggagtt tagattgttg tgagaagggg 34320
   tggggaaagg taggtgattt gtatttttta tttttgtttt atatgggaat aggggtttat 34380
   tttgtatata tatttagttt ggagtatgaa tttttatgaa gaggaaaatt tttattaggt 34440
   tttgatgtta ggattgtttg tgagggttgg gttgtgtttt ggggtaagga taggagtgga 34500
   gggggtaatt agggaaggtt ttttaatgga ggaggtattt gatagtattt tgaaaaagaa 34560
   gtagttttta ggttaagtta gagattgaag aatgattatt taattttttt tttttttttt 34620
   tgagatggaa ttttgtgttg ttgtttaggt tggagtgtag tggtgtgatt taggtttatt 34680
   gtaattttta ttttttaggt ttaagtaatt tttttgtttt agttttttga atagttggga 34740
   ttataggtat gtgttattat gtttagttaa tttttgtatt tttagtagag atggggtttt 34800
   attatgttgg ttaggttggt ttggaatttt tgattttgtg atttatttgt tttggttttt 34860
   taaagtgttg ggattatagg tatgagttat tgtatttggt tatgattatt taattttttt 34920
   ttataagatg ttttttggtt gatgtaaagt aggttgagtg agtgtgatat tttttttttg 34980
   tttgtgggtt ttggtttttt tagagtttgg ttagtgttat tggagataaa gtggtttttg 35040
   tttttttttt ttgttttgtt aggtttagtt tgtgttgttg agttttattt tgtaattaag 35100
   aggagttgtg aattagtgat gttgatgttg tagaaaagtg gttaggggtt tatggggggt 35160
   gaaggatagg gatgttgttt tgagaattgt ttggaagggg tttttgggta atagtgttta 35220
   ttttagggtg tgggtaggtt ggggtggttg ggagttgggt ttataggttg gtataattag 35280
   agttttttaa agttgtgggt ttgagggttt tgtttttttg ggatagtaat ttttggttta 35340
   atttgggaaa aggtgggtta aattaagttt ggttttttta ttgtaggatt atttagtgtg 35400
   tttagtatgt aaatgtgttt ttgaatttaa gataggatta tagaaatttt tttagtgttt 35460
   ttttggaggg agggattgtg ggggatttat tgtaggggtt tgggtttgta ttggtttggt 35520
   gaagtgtttt tttagtgttt atattatgtg tggtgagagg ttggtttttg ttttttttat 35580
   tttttgttgg ttttatttta ttatttttgt atttgttttt tagttggttt atgattaatt 35640
   tttgtttttt tagagttaat tggtgttgtt aggtttttta ttttaagtga ttttttgttt 35700
   gggttaaaat gtttggattt ttaggtatgg aggggagatg gattttaggt taaattatgt 35760
   tgggtaaaat tgagggttaa ggttgtgttt agtaagtgtt ttagttttgg ttgttaaatt 35820
   tatagtgtgt atagtgtgta ggaggtggtt gggtgaatga atttagttat agtttggttt 35880
   tttatttggg gttgttttta agtttttatt gttagttagg ttgggttaga gttttgtggg 35940
   gtttttttgg gtttttttgt tttatagtag gagtttgtat ggagagtttt gagtgaggtt 36000
   aatgggtttt tttttttttt tttttttttt tttttaattt gagatagggt tttatttttt 36060
   ttatttagtt agggtgtagt agtgtaatta tagtttatag tagttttgaa tttttggttt 36120
   taagtaattt ttttgttgta gttttttaaa gtgttgagat tataggtatg tgttattttg 36180
   tttagttggg gtttttgttt tttatttttt tttttgtttt taaatttaat ataaagaaag 36240
   atattatatt tataattaga aaaattaata aggaatatta ttttaaaatt ggaaaaggga 36300
   ttaaagaaat aaattaagaa atttttgtga tttttgttta ttggagttag tggttgttag 36360
   tggttggttt aatataatgt tttaggaaga aatagaataa tagaaagtaa ttttagtgag 36420
   ttgagttttg gttttgtttg tggtgttgta ttagtttttt gtagttgttg taatgagtgg 36480
   ttataagttg gttgggttat aataatgttt agttattttt ttatagtttt gtaggttata 36540
   tatttgagat tagggttatt aggttgaaat tagggtgttt attgggtata gttttttgga 36600
   ggttttaggg taggggtttt tagttttgtg tgttttatta ggaattggtt tgtatagtag 36660
   gaggtgagtg attggtaagt gagtgaagtt ttatttgtat ttatagttat tttttattgt 36720
   ttgtattatt gtttaagttt tgttttttgt ttgattagtt atggtattgg atttttatag 36780
   gagagtgaat tgtattgtga atggtatatg tgagggattt aggttgtgtg ttttttatga 36840
   gaatttgatg tttgatgatt tgttattgtt ttttattatt attagatggg attgtttggt 36900
   tgtaggaaaa taagtttagg gttttaattg attttatatt atggtgagtt gtataattat 36960
   tttattatat attataataa taatagaaat aaagtgtata attaatataa tgtgtttgtg 37020
   gttgggtgta gtggtttata tttgtaattt tagagtgaaa ttttgtttta aaaaataaaa 37080
   aataaaggtt aggtatagtg gtttatgttt gtaattttag tattttggga ggttaaggtt 37140
   ggtggattat gaggttagga gtttaagatt agtttggtta atatggtgaa attttgtttt 37200
   tattaaaaat ataaaaatta gttggatatg gtggtaggtg tttgtagttt tagttttttg 37260
   ggaggttgag gtagaagaat tgtttgaatt taggtggtgg aggttgtagt gaattgagat 37320
   agtgttattg tattttagtt tgggtggtag agtgagattt tgttttaata aaataaaata 37380
   aaataaaata ataaatataa tatatttgaa ttattttaaa attatttttt tattttttgg 37440
   tttgtggaaa aattgttttt tttgaaattg attttaggtg ttaaaaaggt tgggggttat 37500
   tgtttttatt gtttgttttt ttgagttttt ggtggttgtg ggtgtttttt ggtttgtggt 37560
   tgtatggttt ttatttttaa tattagtatt tatagatttt tttgggtttt atttttattt 37620
   ggttattttt gtttgtgtta aatttttttt tgtttttgtt ttgtatgagt attgagattg 37680
   tttttaggga ttatttagat tatttagggt gtttttgtta tagtaagatt tgtaattgaa 37740
   ttatagatgt aaaaattttt gttttaaata aggttttatt tgtaggttta gggattagga 37800
   tatgttattt ttggggttat tatttagttt ttttttgtga ttatgatttt tttttttttt 37860
   tttagttttt tattttggtt gagatgttgt ttttattttt tttattgttt ggttgtttgt 37920
   ggggttgttt tttaggtttt ttgggaggag gtggttgttt gatgtttttt agaaggttgg 37980
   gaatggtttt ttttattttt taggaagatg ttttttttgt taaggagatt gtatttgggg 38040
   ttttatttta ggtagagagt ggatgggatt gtggtttgaa gtggtatttt agggtttttg 38100
   gttttgttat ttagttgagg gttgtgtatg ggttttattt ggttataggt aatttatttg 38160
   gttttgtggg gtagggattg taaatttaga ggtttgtggg gtgtgttgag gtagaggggt 38220
   gtaaatattg taaatttgtt gggggttttg gggagtagta tagattgaaa agtgttttgt 38280
   ttgaagagta gaattgtttt atattagttt tgtgtggtta ggtaggaagg tttttgtggt 38340
   gtggttatgt gttttttttt ttttaataga agttgatagt ttggagtttt tagagagatt 38400
   gtttgagttt ttagttttgg tataaatata tatttttttt aaataggttt gggttaatag 38460
   aattagtttt tgtgttttgg gtttgaatga atttttgagg tgaagtttaa agatagagag 38520
   gatgtttttg atttttattt tagggattta tattgtaaat ttttatttgt tttgttgagt 38580
   ttttggtttt agtatggatt ttgattatgt gttggtgggg attttttagg taagtaagta 38640
   gtttttgagg attttggatg aatagtgggg aatagtattg attttttagt ggggttttga 38700
   gtttttggag aggaagattt gttttttttt aggggatggt tagagattta ttgatttatg 38760
   tgtgtgtggt aggaattttt taggaagttt ttgttttgtt tgtttttttt gtttgggtgg 38820
   ggatggtaat tgtttttgtt attgtagatt aatgggatgg aggggggtga ttttttaggg 38880
   ttttttttgg gtaggtgttt tggaattttt ttttagtatg ttggtttggg gtatggttgt 38940
   tttttttgtt tagttatgtt ggggtatagg gtgaagaagg gttggggtta gtttaggata 39000
   gaggaaggtg aggtaggtat gtaggaattg ggttttttaa atttttaagt ttaaggaaat 39060
   tgtagtattg tgggataggg aaaatgaaag attttggaag ttgttaggaa tttgattttg 39120
   tgagttggtt tttaagagtt taagttaagt atttttaagt ggatattaaa aaggagtagt 39180
   aagttttggg gtggtggggg ttggaggagg tggagagagg aggttgttgg aagttgtatt 39240
   tgggattttt gtagttattg attagattgg gtggttggga ttttgggatt tttgtaggta 39300
   gatttggtgg tttgttggat tttttggggt ttattttggg tttttttttt tgttttttat 39360
   ttttggattt tttttttttg tttttttttt ttttttgttt tgaagagata atgttatttt 39420
   agtttggagt ataaatatat gattagtata tggaaatgtg gtaatttgga tgtatttgtg 39480
   attgtaatag attgaagaaa ttagattaga taaagagtgt ttttagagga ggaggaggag 39540
   gaggaggagg ttgagagagg gagggtgatg ggggtgagaa aggggaggtt gtttttgagt 39600
   gggatgttgg gatttttgtt gttgttaaat atatttgtag gaatggagag ggattggatt 39660
   ttaggtatgt agatagtttt ttttttattg gttgtttttt attgttttgg atttggggtt 39720
   ttatttatgt ttgggggaat aagtatgtaa agggtgtttt tgtttatttt ttatagaaat 39780
   attttttttt tttttgatgg ttttaagttt gtgggatgaa ttttgtttta gaagttaagt 39840
   ttttttgagt ttggttttta tttaaatttg gttttttttt tttttggtta tttttttttt 39900
   attttttagt gtgttatttt tgtatgttat ggttattgtt aatgttgagg ttgttggtgt 39960
   tgaaattttg ggagggtgtg ggggatttgt ttttttggtt tgggtttggg gtggtggtgt 40020
   ttggatttgg ttggtggttg gtttgtttat tgtgtggttt gttttttgtt tttagttaaa 40080
   gtttgttttt aaggttgttt tggtttttta tttaaaatag tatttttgtg tttttttggt 40140
   agttgattat agagggggaa gtttttggtg tttagaggag tttggtgtgt gtgtgtatgt 40200
   gtgtgtgtat gtgtgtgtgt gtgtttattt atgttggtag tttttttgtt gtaggtatag 40260
   atttattagg gtgtgaatag gtaaatgaag agaaaggggg tttttttgtt tttagtttat 40320
   tttagttttt taggtttttt tttttaggtt tagttttttt ggttttttta tttatatgtg 40380
   gtgttgtggt tgttatttta tgtggttagt gttgttattg gtaattagta gtgatgatag 40440
   tttttgttgt ttatgttgtt ttttgagttt agtgttggga gatatggggt ggttgagtta 40500
   agttttaggt tttgggtatt ttgaagtgat ttatgggtta tggagtggga ggttgttaag 40560
   agtgttaatt tttatgtgtt ttgggttttg gtttgttggt tgttattatg tgtgtgttgg 40620
   ggggtttttg agaggtttga gagagggagg tttattttgg tggttttttt ttaggatgtg 40680
   tggttttttt tattattgtt tttagggtta tatttttttt tttttttttt tttgagatgg 40740
   agtttggttt tgttgtttag gttggagtgt agtggtgtga ttttagttta ttgtaagttt 40800
   tgttttttta gtttatgtta tttttttgtt ttagtttttt aagtagttgg gattataggt    40860
   gtttgttata tttttttgta tttttagtag agatggggtt ttattgtgtt aattaggatg    40920
   gttttgattt tttgattttg tgatttgttt gttttagttt tttaaagtgt tgggattata    40980
   ggtgtgagtt atggtgtttg gttttagggt tatattttga ttgttatgtt gggattggtg    41040
   gagtgggtgt gtgttgtgtt gttgagtata tttagttggg tatagtaggt ggattttagg    41100
   agggggttag gggagatggt agaaggtttg ggtggtagtg aaattgtatt ttatttgttt    41160
   gagtttgggg gtttaggttt gttgggatgg gtgggtgtgg agtttagtgt tttgtttttt    41220
   gtttagtagg agttgtaggg tagggagtgt tgagagggat ttttgttagg tttgtatagt    41280
   ggagttgttt ttggagtgat tattttatta ggtagtttgg gtagattata gtgggttttt    41340
   tgttttttgg gttattagtt ttaggaggat gttattttat ttgtttggtt ggtaggggtt    41400
   ttggggagtt ttgggtttgg tttttatttt atagatgatt tagttgaggt ttagatgggt    41460
   gattagtagg ggatttgaga gttgagtaga gaggtgtggg tttgggggtg gtgttttttg    41520
   gggagttgga agtgatggta tttttttagg tttataaaat atgggttttt tatgaattga    41580
   ataaaatatt ttttgaaagt agttagtgtt gtgttttaga gattattttg atttttgttg    41640
   tttttggttg aggatagtat agttagggat gaagtgggga tttttttttg ggttgtgttt    41700
   tgatgaaaga tgttttttat aaaattttat ttggatgtgt ttttatgtaa gaaaatgtat    41760
   atttttagaa atttagttgt aaattggttg ttttttgaat gaatatgata gttttggagt    41820
   tgtttttttt aatgaggttt ttgttttggg taaattttag tgttggtttt taattgttat    41880
   tgaatttttg tatagttttt ggggaagtga tttttttttt gtttttgttg ggtggttttg    41940
   agtaagttat ttggtagttt tgggttgtag ttttgttatt tgtaaggtga ggggtgggag    42000
   tggtatggtt tttaggtagt gtttttattt tttgtgggtt tttgtgtgtg ttttgagttt    42060
   tgtgggtgtt ttttttttta ttgaggggtt tttttttttt gtagggtatg gtttatgttg    42120
   tgttgttagg ttgtaagtta ttgtgagagt tgttttgatt tttaagtatg ttttggtttg    42180
   aggaggagtt atggggagga tgttgtgttt gtgtgataga ggtttgtttg gtagatgttg    42240
   gtttgttgat ttttagatgt ggttgtgggt gttttatgtt ttattgggtt ggtgtgtgag    42300
   gtgtgtttag tttaggttgt tttatagttg tttaggaagg gtttgtggga gggggtggtt    42360
   ttattttggt gagtttatgg tggtttgtta gttttaggga aggtttagga gttttaagat    42420
   ttgttttttt gttttttttt tggtagtgtt tggttttggt ttggtttgtg aatgggggtt    42480
   tgggtatttt tttgtttttt ttgggggttg tggttttttt ttgggaaggt gttggagggt    42540
   ttgttagtag ggttggtatg agttatggag tggtgattgg ttgttgaggt atggaaggtt    42600
   atttttttta gttaagggtt ggagtttttt ttttgttttg gaatggtttt gtggttttta    42660
   taaattaagt agttttattt tgttttaaga gtttgtattt tagtagggag agagttttta    42720
   agaagggaat tttttattgg ggagttttgt tgggttggga ggttaggaat aggttatgag    42780
   ggagggttgt aggtagggga agtggttgtt tgtgtatggt tatgaatgta ggtttggggt    42840
   tttggagggg gtttggttat gttgttgttg gatgtttgtt tttattaggg tttatttgat    42900
   gtagtatggt atagggtaaa gtttagtttt ttttatgggg tttttgggtt attagtgggt    42960
   ggggaattga ggttttttgg agggtggtgt tttagttttt attttttttt tttgtttttt    43020
   gttgtgttgt ttttgatggt ttttgttatt ttttgggagt ttttattttt ggatgtttgg    43080
   tgtttgtttg gttttaattt ttttagtttt gtttttttat ttttgaggtg agaggtttgg    43140
   tttgatgtgt tttgtatttg ttttttgttt agttttgtgg gaattttggt ttttgtagtg    43200
   gttttttggg gttgttatta ttaagtgtta taaattgggt ggtttaaaat attagatatt    43260
   tttttttttg ttgttttgga ggttagaagt ttgaaattat ggtattagta ggtttggttt    43320
   tttttggagg ttttgagggg gagtttgttt tttgtttttg gtagtttttg gtggtttttg    43380
   aaatttttgg tgttttttgg tttgtggtta tattatttga tttttgtttt tgttgttatg    43440
   gggttttttt ttgtgtttta aatttttttt tgtttgtttt ttatatggat agtttttttt    43500
   ggatttaggg tttattgggt aatttaaaat ggttttattt taagattttt aattttataa    43560
   tgtttgtaaa gatttttttt ttaaataggt tatgtttata ggttttgggg tggatgtaaa    43620
   ttttaggggt tattttttat ttttttgttg tttttgtgtg tttggatgta tagtatggtt    43680
   ttatgtttta gattttttgt tggatttttt tggttttttg gggtggtttt tggttttttt    43740
   tatttgtttt tgtgtttttg gtgaggtgtt ggggttgggt ttagtttttg taggaatttt    43800
   ggggatagta tggagttggt aattttttat taggtgtttt tttatttgtt tagagtttat    43860
   ggttgtgagg ggtttttttt tgtggttttg atttgtaatt ttttagtggt tagtgatgtt    43920
   gagtattttt ttgtgtttat tgtttatttg tatatatttt tgtagaaatg tttgtttaag    43980
   ttttttattt gtgtttgatt tggatgattg ttgttgttga gtgttaggag atttgtagat    44040
   taatatttag atattaattt tatatttgat ttatgatttg ttgttatttt atggattgtt    44100
   ttttttttat gttgttagtg ttttttgata tgtaattttt tttttttttt ttgagatgga    44160
   gtttgatttt gttgtttagg ttggagtgta gtggtatgat tttggtttat tgtaattttt    44220
   gtttttttgg ttttagtgat ttttttgttt tagttttttg agtagttggg attataggta    44280
   tttgttatta tatttagtta atttttgtat ttttagtaga gatggggttt tattgtgttg    44340
   attaagttgg ttttgaattt ttgattttaa gtaatttatt tgttttggtt ttttaaagtg    44400
   ttggagttat aggtgtgagt tattgtattt aggttgatat gtaaatattt taaattttta    44460
   tgatgtttta ttttatttat ttgttttttt gttgtttgtg tttttggtgt tatatttaag    44520
   aaattattgt taaatgtaat gttaggaagt tttttttttg tgtttttttt taagagtttt    44580
   gtggttttag tttttgagtt taggtttttg atgtaagttg agttgatttt tgtatgtggt    44640
   gtaagggttg gtttagtttt atgttttggg tttttgattt attttttttt tttttttatg    44700
   tttagttggt tttgttgggt ggtggggagg agtggggaag ttttgggttg ggtttgtatt 44760
   tgattatttt tttttaggtt agttagggag ttttagtttt tgtttaggat ttggttggat 44820
   gtgtttttta ttgggaaagt ttgggttgtt tttttaggtt gatggtaggg ttagtttggg 44880
   gtgttttgag gtttgttttg tggatatgtt ttttgtttta ggtggtgtgg ttgtttggtt 44940
   tgtgtgtgag attagttgtt tgtgttttag gttatggagg ttgagtgttt ttagtttgtt 45000
   tttttgtttg gttttttttt tggggaagtt tttgtagttt attttttgtt tttgtttttg 45060
   ttatttgtgt ttttgtttgt tttttgtttg gaggtggtta tttttggggt tattttttat 45120
   gatttggata taagttttta ttttgaagtt attatttaaa tttttgtgtt ttaaattttt 45180
   tttatttatt atatgggttt ttattgtgat taatttagta gttgatgaag ttttttttgg 45240
   ggttttttta gtaatgggga gttggttttt ttggaggttt ggtttttttt taagtggaag 45300
   tggggtgtga gggtgttagt ttttttttgt tgtttggtgt tttaggttgg tttgttattt 45360
   ttggaagtat ttgttatttt tatatagtat tttatattta tttttttgtt ttttattttt 45420
   ttttttaagg ggttattttt gttttttttt ttatttaatt ttatttatgt ggtttgttta 45480
   gtaatttttg atttttaata tgataaaata aatttttttt gttggttatt tatttattta 45540
   tttagtattg ggtgtttttt gtggatttgg tgttggggtt ttgtggagga taaagttaga 45600
   tatagttttt gtttttatgg gattgtataa gtgtaagatt atattagtaa atgtgaaata 45660
   taggaagtga taggtgtgat aaaggggatt agtggtagga tagaatttgt ggtttgtagg 45720
   attaggttag gagggttgtt ttggggggat atttttgggt tgagtgtaga aggatgaggg 45780
   gagtaaatta ggtttaaatt tagtaggtag aggtgattgt tgtaggtaat tggtaatgtg 45840
   tttaaaggtt ttggggtgtg gggggttgag gttggtagta tggtaggaag taagattggg 45900
   gttgaaagag attgattgtt atgttgtgaa atatatattt ggtttttatt tttatttttt 45960
   ggtatataat ttttaaaatt tttggaattt ttaaagtgat gtttttttgg atgtttatga 46020
   ttgatagatt agttggtagt tttaggatgg tttttaggga agattaggta gaattataag 46080
   gtttagtatt attttgtaat ttttaggtag gggagagggg ttgaaggtta agtagattat 46140
   tagtggttaa tgattgaatt aattatgttt ttgtaatgag gtttttgtga atatttagaa 46200
   ggatggggtt ttgggagttt ttggatggat gagtatgtgg aggtttttgg agggtggagt 46260
   gtttggggag tatatggaag ttttgtgttt tttttttata ttttgtttta tatatttttt 46320
   tttttgtatt ttttgtaata ttttttataa taaattagta aattttatga gttttaggaa 46380
   tatgtgtaaa agaaaaaaaa aagaataaat tatagttgtt gtagtaagtt aattttatta 46440
   gtattggttt ttatttgttg attttatttt tggtaatgtt tttttttttt gggtttttgg 46500
   gatttttttg gttttattat tttttttgta gtggttttgt tggttgttgt ttttaggtta 46560
   gtttattttt atttggtttg tttttattag gggttgaggt ttttttgttt attttagttt 46620
   tttggtttgt tttttgtttt gggtattttt ttattttgaa tggttagagt ttttgaagtt 46680
   gtttgttgtg tgtttttgtt tggttttatg tattttgtat ggtgtttttt agggtttttt 46740
   attggtaagg gtattggtat agattttggg gttgttttgg tttaagttta ttttgatttg 46800
   gagtttgagg gtttttgagg gggttttgaa ggttagggta ggtaaggttg gtatgttttg 46860
   ggtagtgggg gttgggtatg ggaggggatt gtgggtgggg agattttttt taattagtgg 46920
   tttatgtttg gagattgatt tgggttttag gttatgtttt gaaattggga tagagagtgg 46980
   tgtagatttt agtttggaga ttttttttgt ttgtggatgt ttttatattt ttatttgtgg 47040
   agttattttt tttttttttt ttttaaatgg ggaaattttg atttgaatat tgaataaata 47100
   atatattttt gaaattagtg aagaggtatt ggttttgaaa ttttgttatg aaagtttgaa 47160
   aatgtttttt ggattgattt gttggaatag atgggtgtga tggggttgtg gggttttagg 47220
   tggtaggagg tgtttttgtg tttagggtat tttgatgttt agagtttttt tgtgtttagt 47280
   gtgtttttgt atttagggta tttttatgtt tagggtgttt ttgtgtttag tgtgtttttg 47340
   tatttagggt atttttatgt ttagggtttt tttgtgttta gggtgttttt gtatttagag 47400
   tatttttatg tttagggtgt ttttgtattt agggtgtttt tgtttttagg gtgttttgtg 47460
   tttagggtgt ttttgtgttt agtgtgtttt tgtatttagg gtgtttttat gtttagggtg 47520
   tttttatgtt taggtattgt ttttttttat ttttttaatt tttttttatt aataggtgtt 47580
   agttgtgaaa tgtttgttgt gtgtgttttt tggttgagtt tattttgtgt ttttaagttt 47640
   ttgttagata taggtaggtt gttttagttt ttttggtaaa tgttttgttg ttgttgtttg 47700
   atatggggtt ttattttgtt atttaggttg gagtgtagtg gtgttatttt gttttattgt 47760
   aatttttgtt ttttaggttt aagtgatttt tttgttttag ttttttaaat agttgggatg 47820
   ataggtgttt gttattatgt ttggttaatt tttgtatttt tagtagagat ggggttttat 47880
   tatgttggtt aggttggttt tgattttttg attttaagtg atttatttag tttggttttt 47940
   taaagtgttg ggattatagg tatgagttat tgtgtttggt ttgtttggtg aatgtttatt 48000
   gaggtttatt atgtgtagtt ttttttttag gtgtttggtt tgtaatgagg agtaataaag 48060
   atatagttgt tggttgtttg ttatttaata gttggaattg agaggtagtt tataaatgag 48120
   taaatttata ggtggtttta gatgaggatt gtgaagaagt taagaatagg ttgttggggg 48180
   tgtatatttg tgtgtgtggt gaggggatta atggaagttt tttttgagtg gtgatattga 48240
   gttgaagttg taatgattag aaggatttag ttaggttaaa atttaaggga agagtaattt 48300
   ggtaaaggaa atagtaggtg tgaaggtttg aggttggagt ttggggaagg tgggagtggt 48360
   tggagtgtgt ttttagagat gggttatggt gatattaatg tgataggtag ggttaggttg 48420
   tgttgggttt tggaggtttt ggtgagggtt tgattttatt tttattatgg tgggatttta 48480
   gtgttttgtg gttgttgaga atggttaggg gtaggtaggg aggtttgtta gggaggaggt 48540
   tgttgtaggt tttgtagttg gagagggagg attaggattt ttttagagag gaagttggag 48600
   agggaggatt tttttgattg ttggatggat ttatttgttt ggttgttttt gttagtattt 48660
   agtagaggtt attggatgtt gataggtggt tggttttgtt tagttttgta ttgttttgtg 48720
   ttgttttggt ggttgttggt tttaggagga gtgtttttgg tgtttgagtt tagtggtttt 48780
   ggttgtggtt tggtatattt ggttgtgggt gaggttaagg tggtttggat tttgggaagg 48840
   ttgggtattt ggtagtttta aggtaggagt tggggtgttg gaggaggaag tatgtttatt 48900
   ttttgttttt ttttgttttg tgggatgttg agtttttggg tagggatatg gtgagttttg 48960
   gtgtgatgta tttaatattt gttggtgttt ttatttttaa gtaggaattt tggtgtttgg 49020
   tgtttgagga ttgttgttta ttgttttatt attttttagg ttttttttta gtttttgatt 49080
   tggggtaagg tttagatatt tagaatattt ttggttggtt tttggagttg gggtgaggta 49140
   gaattttttt tttatattta tagtattagt taaggttttg gggtttggat attatttttg 49200
   ggaggggtga gtaggataag tgaggttttt agatttgggg tggttttgga ttggttaggt 49260
   aggtgggtgg gggttgtagt ttatgttttt gttttaggta atatagttga gttgtagttt 49320
   ttgttttatt tttagttggg gttttttgtg tttatttttt gttttttttt ttttttttgg 49380
   aatggggggt ggatatttta agattagttt tttagttatt ttttttgttt tttgtttttt 49440
   tttttttggg atagattttt gtgattgttg ggtgtttttg atgttttgtt ttttttgtgt 49500
   tttgtgttgt gggtgagaga gggttttggg gggatttttt ttttttggag tatttgttgt 49560
   tttgggtgtt tttgggggtg ggtttttatt ttatattgtg gagttggtgt aggaaggaag 49620
   gtgttgggag ttagttttaa tttatgtgtt ggttgtggtt tttaaggtgt ggatggtagg 49680
   aggtggtgag atagggattt attgattatg ggattgatat ttttttatgt tgatgtgggt 49740
   tttgagttat aattttatta gtttttgagt tttggtgttg tttttggtga tggtgtggat 49800
   ttagttaggt ttttgggtag ttttttttgg attttttttt tgtttttttt tgggtagtgt 49860
   ttttgtttat ttggaaagga tgggtaggat ttggagataa tagggattta aagaagttgg 49920
   gagtgggggt atttgtgtag tttattttgt agaatagata atttggttaa agtttaaagt 49980
   ttgttgttag gagttttgtt tgttttttag agagtatatg gtttgattat tatggggatt 50040
   gggggagagg tataggtatg tttgtttggt agtagaaggg aattttgggt ttgggttttt 50100
   ttattttgtg gtgttgtttt tttttagagt tgtattgttt tttttattgt ttttgtttgg 50160
   ttttagtgtt tatgtggttt gtggttttta gtttgggttt ggttttttgt aatttatttt 50220
   gtttagtttt atatagtttg tttttttagt tgggaattgg agtttatttg gagttttagg 50280
   tagtagattt gatgtagtga tgtagggttg ttagttatgt tagtaggttt tgttgtttat 50340
   agagggatgg gtattgttgt tgataataat taaagaatta tggttttagg ttgggtatag 50400
   tggtttatat ttgtaatttt agaattttgg gaggttgagg taggagaatt aattgaattt 50460
   aggagtttta gattaggttg ggtaatatag taaggttttg tttttataaa aaataaaaaa 50520
   taataataat aataaaaatt atagttttag aatgattgat tttagtatgt ttataaagtt 50580
   aagggttttt agtgttttgg gtttatgtta ttgtgttaga gggagagagg aagaggttgg 50640
   ggtgatgggt attggggttt atgttttttt tggaatgggt ggttttggat tgtgtttgtg 50700
   tgatatgttt gttgagtggg ggtggtgttt attgaggatt ggatgttgag gtgtgggtgg 50760
   ggggaggtta tgaagtattg ggtatttttt gtgtattagt gttttgagat tgtagagagg 50820
   ggatttgttt agtttgggta gggaggtggg tggtagtgag gaaggggaga ggaatttggg 50880
   tgtagaaagt ttttttttta gtaggtggtg ttgtaagttt ttgagagttt agtttttgtg 50940
   gggtttgtat ttttattgaa tttgtgggta gaggttgttt gggtgtagtt tttgttaatt 51000
   gtgattgttt attgtgggtg aggggaagtg gagtgtagag aatgtattgg agggtagatg 51060
   attagttttt gtataggtat gggggtgttt ttttttttgt ataggtatgg ggggtttgag 51120
   tttttggggt ttttgtttta attgggtggg gaaagggagg gttggaagtt gagttagggg 51180
   gaaagggggt ggttttttgt tatttttttt attttatttt ttttattttt ggttgatagg 51240
   ttttaatttt ttttatattt ttttaatttt ttgttgtaaa attttttaaa tatagaaaag 51300
   atgaaagagg gttgagtgtg gtggtttatg attgtaattt tagtattttg ggaggttgag 51360
   gtaggaggat tatttgagtt taggagttta agattagttt gggtaatata gtgagatttt 51420
   atttttataa aggatataaa aaattagtta ggtatggtgg tgtaggtttg gagttttagt 51480
   tatttggaag gttgaggtga gaggattgtt tgaatttggg aggttgaggt tgtagtgagt 51540
   tgggattgtg ttaatgtatt ttagtttggg tgatagaata aaattttgtt ttaaaaataa 51600
   aataaaataa aattataaaa aagaaattat gaaagatttt aaagtaaata tttatgtatt 51660
   tattatttag attttatagt aattattttg ttgtttgttt tattataaat ttttttggtt 51720
   attagtattt atgttggttt tgatgtattt aaaataagtt gtagaaatta gtatataata 51780
   ttttgttaat attttagttt gtattttatt aattagagtt tggtatttgt ttgtgattat 51840
   ttttgttttt ttttgaagta aaatgtatat attttgagat gtataggtgt gaaatgtttt 51900
   gttttttgaa ttttaataaa ggtatttatg tgtattttag tttgattaga atatagaata 51960
   ttattattat tttagtaagt tttttatgtt tttttaggtg tattatagtt tttatttttt 52020
   ttagggtaat tataattgtg tttttttttt attatgaaat aattttgtta gtttgagtgt 52080
   tttttagagt tatttatgtt gttgtaggtg ttagtgattt gttttgtttt attgttgagt 52140
   aatgtttatt gtgtgaatat tttgtgggtt tttagttatt tttttgttga aagatttttg 52200
   gttgttttta tttggaggtt tttatgagta aatttgttat gttggttttt tttgtataag 52260
   tttttttgtg tatatgggtg tttatttatt ttgtagaaat atttagagta ggtatagggt 52320
   aggtatatat ttagttttat aagaaattgt tagatttttt tttaaagtgg tttttttatt 52380
   ttatattttt attagtaatg gatatgagtt ttagttgttt tatattttgg ttaatatttg 52440
   gtgttgttat tttgtgaaat tttagttatt ttggtgggtg tgtagtggta ttattttatt 52500
   atggttttaa tttgttttgg tttgatgatt aatgtggttg agtatatttt tatgattggt 52560
   tttttatata tttttttttt gtgaaatgtt tttttaaatt tgttgtttat tttaaaattg 52620
   ggttgtttga tttttttttt tggatattga ttttttgtta gatatgtgtt ttataaatat 52680
   tttattttag tttgtggttt gtttgtttat tttttttttt tttttaagaa taaggttttg 52740
   ttgtattgtt taagtaggtt tgaaattttt gggtttaagt tgtttttttg tttttgtttt 52800
   tttaagagtt ggttttatag gtatgagtta ttatgtttgg tttgtttatt tttttagtgg 52860
   tattttttaa taagtaaaag tgtgttattt taatttttat gaagtttaat ttaataaatt 52920
   tttttttttg tggttttttt tttgtgtttt ttgtaagaaa tttttgttta ttttgttaaa 52980
   ttgtaaagat atttatgttt ttttttagaa ggtttagagt tatatagttt ttatgaatta 53040
   atttttgtat atggtgtgag gtagagggtt agggtttatt ttgttgttgt tttatgtgtt 53100
   tatttagttg ttttaaattt tatttttttt taaagttttt attttgtatt tttttggtga 53160
   tttatgaggt tgtataattt tttaagattt ttgttggtta tttatgtatt tttttttgta 53220
   aaattatttg tttatgtttt attttttttt aaaattatgt tgttttaaga ttgagtgatt 53280
   tttgaagttt ttattttata atgtttgaaa gaatgaatat tttgtttttt agttataggt 53340
   ttttgttttt tgttttgtag ttgtatagtg gggtaagatt tgggttttgt tttgttgttg 53400
   ttttgttgtt tttttatttg attgtttttt tatgggtttt atttaaaaaa aaaattggtt 53460
   gggtatagtg gtttatgttt gtaattttag tattttggga ggttgaggtg ggtagattat 53520
   gaggttagga gattaagatt attttggtta atatggtgaa attttatttt tattaaaaat 53580
   ataaaaaatt agttgggtgt ggtggtgggt gtttgtagtt ttagataatt gggaggttga 53640
   ggtaggaaaa tggtgtgaat ttgggaggtg gagtttgtag tgagttgaga ttgtattatt 53700
   gtattttagt ttaggtgata gagtaagatt ttgttttaaa aaaaaaaaaa aaaaaaaatg 53760
   ttgttttata tgttttttat atattttgga tataagtttt ttattagata tatgtattat 53820
   aagttttttt ttttatattg tttttttttt tttggttttt ttaatgatgt tatttaatga 53880
   gtaagagtat tttgttttta taaagtttaa tttattaatt ttttttagtt ttgatttatg 53940
   atttttgtga tttatggagg aaatttttgt ttatattaag gttaaaagaa gtttttttat 54000
   tttttagaaa ttttatagtt gtatatttta tgattttttt tattttttaa ttttttaaat 54060
   ttttatattt tttaatttat tatagttgtt aggatgttgt atgatttatt ttaaattagt 54120
   ttttgtattt ggtttgaggt tagggttaat ttttgttttt ttatatttat atttagtttt 54180
   agtattgttt gttgaaaaga tatattttat tttattgaat tatttgatat ttttgttaaa 54240
   aattattgat tgtatatgtg gttttatatt agtatatgta tgtatatata tgtaggtttt 54300
   gtgttgattg tatgtgtggt tttatattag tatatgtatg tatatatata ttggttttat 54360
   gttgattgta tatgtggttt tgtattagta tatatatatt agttttatat tgattgtata 54420
   tgtggttttg tattagtata tgtatatata tatatgtagg ttttatgttg attgtatatg 54480
   tggttttata ttagtatatg tatatttata tatgtaggtt ttgtgttgat tgtatatgtg 54540
   gttttgtatt agtatatgta tatatatata tataggtttt gtgttgattg tatatgtggt 54600
   tttgtattag tatatgtata tatatatatg taggttttat gttgattgta tatgtggttt 54660
   tgtattagta tatgtatata tatatatata ggttttgtgt tgattgtata tgtggttttg 54720
   tattagtata tgtatatata tatatgtagg ttttatgttg attgtatatg tggttttgta 54780
   ttagtatatg tatatatata tatgtaggtt ttatgttgat tgtatatgta gttttatatt 54840
   agtatatgta tatatatata tgtaggtttt atgttgattg tatatgtagt tttatattag 54900
   tatatgtata tatatatatg taggttttat gttgattgta tatgtggttt tgtattagta 54960
   tatgtatata tatatatgta ggttttatgt tgattgtata tgtggttttg tattagtata 55020
   tgtatatata tatatgtagg ttttatgttg attgtatatg tggttttgta ttagtatatg 55080
   tatatatata tatgtaggtt ttatgttgat tgtatatgtg gttttgtatt agtatatgta 55140
   tatatatata tgtaggtttt atgttgattg tatatatata tgtggttttg tattagtata 55200
   tgtatatata tatatgtagg ttttgtgttg attgtatatg tggttttgta ttagtatatg 55260
   tatatatata tatgtaggtt ttatgttgat tgtatatgtg gttttatatt agtatatgta 55320
   ttgtatatat gtaggtttta tgttgattgt atatgtggtt ttatattagt atatgtattg 55380
   tatatatgta ggttttgtgt tgagatatta ttgtgttttt ttgatatttt gtttattttt 55440
   ttgttgatgt tatattttga ttaatgtggt tttatagtaa gttttaatat aaagtttttt 55500
   aaatatttaa aatattattt tgaatatttt aggttttttg agttttttta taaatttaag 55560
   aagtagtttg ttgatgttta taaaaatgta gtgtgaattt tggttgagat tgtgttgaat 55620
   ttgtagatta attaaggaag aattggtatt ttaataatat tgagtttttt gtatagatgg 55680
   tatatttttg ttttaaaggt ttttttgtat tttttttatt tttttattga aaaaaaattt 55740
   ttatagaatt atattgtttt gtagttttta agaaaaagat tttgtatgtt atttgttaaa 55800
   tttattttta agaattttat ttttggtatt attttaagtt gaaatagatt ttaaatttta 55860
   tttttaatta tttgttgtta gtatgtaaaa atataataga tttttgtatg taaattttat 55920
   attttatgat tttttttaaa gttatttatt attttggtaa tggttttgta ggttttttag 55980
   gatttaaata tatagtttta tttttttttg attttgatgt tttgtttttt ttttttgttt 56040
   tattgtattg gttagagttg ttagtttagt attgaatagt agtgattagt ggatattttt 56100
   gtttgtgtta aatttataat gttaatttta gatttgttat atatgatttt tgttagattg 56160
   aggaaatttt tttttatttt taatttgttg aaatttttta atatgaatgg gtatgatttt 56220
   agtaaatgtt ttttttgtat ttatggaaat gattattgtt ttgtttttta tttgtttaat 56280
   aggataaatt ataatgattg atttttgttt tttgtttttt tttttttttt ttttaaagaa 56340
   atagagtttt atttttgttt aggttgtagt atagtggtgt tattgtagtt tattgtagtt 56400
   tgaatttttg gatttaagta atttttttat tttagttttt taagtagtag ggattatagg 56460
   tatatgttat tatgttaagt taatttttaa tttttaaaat tttttgtata gatggggttt 56520
   tattgtgttg tttaggttgg tttgtaattt ttgagtttaa gtaatatttt tatttttgtt 56580
   ttttaaagtg ttgagattat aggtgtgagt tattgtgtag gtttgatttt ttaatattga 56640
   attaattttg tatttttgga agaagtttaa tttgtttatg agatattatt ttttttatat 56700
   attgttaaat ttgatttgtt tagtttttaa ggatttttgt gtgttgattt atgagggata 56760
   ttggtgtggt attttttttg tttgttagtt tgttttggta atgtttttgt tagttttatt 56820
   ttggttttat aaaattagtt tggaagtgtt tttttttttt tgttttttgt gtttttataa 56880
   ggttgatgtt gttttttttt taaatgtttg atagaattta ttaagaaaat tgtttggatt 56940
   tagaattttt tattaggaaa ggtgattgtt tgtttttaat tataaattta atttaatgta 57000
   tagaaagagt tatttaggtt tttttatttt tttttgtttt atttttggta agttgtattt 57060
   tgaaggaatt tgtttttttt aggtaagttg ttgaatttat ttatttaaag ttgtttgtaa 57120
   tataagtata tttagggttt gtttttagat tttggtaata aagtaaatat tgtaataaag 57180
   tatgttatat aaattttttg gttttttagt atatataaaa gttatgttta ggttaggtat 57240
   ggtggtttat gtttgtaatt ttagtttttt gggaggttaa ggtgggtgga ttatttgagg 57300
   ttaggagttt gagaatagtt tggttaatat ggtgaaattt tatttttatt agaaatataa 57360
   aaattagttg ggtgtggtgg tatatgtttg tagttttagt tgtttgggag gttgaggtgg 57420
   gagaattgtt tgaatttggg aggtggaggt tgtggtgagt tgagattata ttattgtatt 57480
   ttagattggg tgatagggtg agattttatt ttaaaaaaaa aagaaaaaag ttatgtttat 57540
   attatattat agtttattaa agtgtataag attattatgt ttttttgtta aaaaatgtta 57600
   ataattattt gagtttttag taagttataa tttttttttt ggtagaaggt ttgttttaat 57660
   gttgatggtt gttgattagg tggtggttgt tgaaagttga aggtagttgt ggtagtagtt 57720
   tttttgtttt tttttttttt tttttttttt ttgagataga gttttgtttt attgtttagg 57780
   ttggagtgta gtggtatgat tttagtttat tgtaattttt gttttttggg tttaagtaat 57840
   ttttttgttt tagttttttg agtagttggg attataggtg tttgttatta tgtttggtta 57900
   atttttgtat ttttagtaga gatagggttt tattttgttg gttaggttgg ttttaaattt 57960
   ttgattttag gtgatttatt tgttttggtt ttttaaagtg ttgggattat aggtgtgagt 58020
   tattgtattt ggttggtagt tttttaattg aagtttttga tatgggttga tttttttttt 58080
   tatgaaagat ttttttgtgg tatgtattat gtttgatagt attttattta taatagagtt 58140
   ttttttaaaa ttagagttaa tttttttaat tttggttatg gattgattaa ataagttgat 58200
   gtaatatttt aaattttttg ttgttatttt aataatgttt atagtatttt tgtttggagt 58260
   agattttatt tttaggaatt attttgtttg tttatttata agaagtaatt ttttatttgt 58320
   ttaagtttga ttatgggatt gtagtaattt agttttattt ttaggtttta tttttaattt 58380
   tagttttttg ttatttttat tatatttgta gtgatttttt ttgttgaagt tttaaattgt 58440
   ttatttatga gggttggaat taattttttt taaatttttg ttaatgttga tattttaatt 58500
   tttttttgtg aattataagt gtttttaatg gtatttagaa tggggaattt tttttagaag 58560
   gtttttaatt tattttgttt agatttatta gaggaattat taattatggt agatattgtt 58620
   ttataaaata tattttttaa ataataagat ttgaaaatta aatttatttt ttttttatgg 58680
   gttgtagaat gggtgttata ttagtaggta tgaaaataat atttattttt atatatattt 58740
   ttgttagagt ttttgggtta taaggtgtat tgttaatgag taataatatt ttgaaaggat 58800
   tttttttttt tgagtagtag gttttaatga tttttttttt tttttgatgg agttttgttg 58860
   tgttatttag aatggaatgt ggtggtatga ttttggttta ttgtaatttt tgttttttgg 58920
   gtttaagtga tttttgtgtt ttagtttttt gagtagttgg gattataggt atatattatt 58980
   atgtttggtt agtttttgta ttttttgtag aaatagggtt ttattatgtt ggttaggttg 59040
   gttttgaatt tttgatttta ggtgatttat ttgttttagt tttttaaagt attgggatta 59100
   taggtatgag ttattgtatt gggttttatt tgtttttttt ttgttgttgt tttttaattg 59160
   agatagagtt ttgttttgtt gtttagattg gagtgtagta gtgttatttt agtttattgt 59220
   agtttttgtt ttttaggttt aagtgatttt tttgttttag ttttttgagt agttgggatt 59280
   gtaggtgtgt attattatgt ttggttaatt tttgtatttt taatagagat gaggttttat 59340
   tattatgttg gttaggttgg ttttgaattt ttgaatttag gtgatttgtt tgttttggtt 59400
   ttttaaaatg ttgggattat agttgtgagt tattgtgttt ggtttttttt tgtttttttg 59460
   ttttttttta aattgagata gagttttttt ttgttgttta ggttggagta tagtggtatg 59520
   attttggttt attgtaattt ttgttttttg ggtttaagtg gtttttttgt tttagttttt 59580
   taagtagttg gaattatagg tgtgtgatat tatgtttgaa taatttttgt atttttagta 59640
   gagatgtggt tttattatgt tggttaggtt ggttttgaat ttttgatttt aagtgatttt 59700
   tttgttttag tttttaaaag tgttgggatt ataggtgtga gttgttgtat taggttttaa 59760
   tgggttttta atatttagta aattatgttg taaatagatg tgttgttaag tagggtttgt 59820
   tattttattt ttgaagtata gatagaatag atttggtaga atttttaagg gttttaggat 59880
   ttttggaatg gttaatgagt attggtttta atttatggtt aatagttgtg ttagtttttt 59940
   atgagagtta gtttgttttt tgaagtttta aagataggta ttgatttttt ttttttagtt 60000
   atgaaaattt tagatgatat ttttttttag tagaaggtgg gatatttatg ttgaaatttt 60060
   gttgtttagt gtagttattt ttattagtgt tttgagttag attttttggg taatttgttg 60120
   ttgttttttt attagtattt gttgttttat tttgtatttt tatattatag agatggtttt 60180
   tttttttaaa ttttatgatt tgatttttgt tagttttaaa tttttttttt ttagtttttt 60240
   tatttttttt agtttttata ttattgaaga gagttagggt tttgttttgg attaggtttt 60300
   tgtttaaggg aatgttgtgg ttgatttgat tttttattta gattatttaa atttttttta 60360
   tattagtatt aaggttgttt tgtttttttt ttttttatgt gtgtattgga attgtatttt 60420
   taattttttt taataatttt tttttggtat ttataatttg gttagttatt tggtataaga 60480
   gattttattt ttgtttgttt tggtttttga tatgtttttt ttatttagtt taattatttt 60540
   tagtttttga tttaaagtga gagatgggtg attttttttt ttatttgaat atttagaggt 60600
   tattgtaggg ttattaattg gtttaatttt aatattgttg agttttaggg aatagggatg 60660
   tttgagtaga gggagagaga gtggggaata gttagttagt ggagtagtta gaatatataa 60720
   tgtttattga ataagtttat tgttttattt ggatgtggtt tgttatgttt taaaaataat 60780
   tgtaataatg gtattgaaga ttattgatta ttgggtgtgg tggtttatgt ttgtaatttt 60840
   agtattttgg gaggttgagg taggtggatt atttgaggtt aggagtttga gattagtttg 60900
   gttaatatgg tgaaattttg tttttattaa aaatattaaa attagttagg tgtggtggtg 60960
   ggtgtttgta gttttagtta ttggggaagt tggggtagaa gagttgtttg aatttgggag 61020
   gtggaggttg tagtgagttg agattatatt attgtatttt attgtatttt agtttgggta 61080
   atagagtgag attttatttt aaaaaaaaaa aaaaagggtt gggtgtagtg gtttatgttt 61140
   gtaattttag tattttggga ggttgaggtg ggtagattat gaggttaggg gattgagatt 61200
   attttggtta atatgatgaa attttgtttt tattaaagat atgaaaaaaa tagttgggtg 61260
   tggtggtggg tgtttgtagt tttagttatt tgggaggttg aggtaggaga atggtgtgaa 61320
   tttgggaggt ggagtttgta gtgagttgag attgtgttgt tgtattttag atagagtaag 61380
   attttgtttt aaaaaaaaaa aaataaagaa agaaaaaaga aaaaagagat tatagattat 61440
   tatagtagat ataataataa tgaaaaagtt tgaattattt taagaattat taaattttta 61500
   tatagagata tagtgttaat atgttgttgg aaaaatggtg ttagtagatt tatttgatgt 61560
   agggttgtta tgaattttta atttgtaaaa agtgtagtat ttgtgaagta taatgaaatg 61620
   aggtgtgttt gtgttttttg ttattgtaat gttggtagga tttgtaagga aaatatttta 61680
   ttttttttat tgtgatttta tgtttgttat ttttttttta ttagtagttt ggttaggagg 61740
   ttattaattt agattttttt aaagaattag tattggattt tttttttttg gtgtttattt 61800
   ttttgagttt ttttgttatt tttattattt ttttttttgt atttgtttta gtgtattagt 61860
   tagggtttag ttaggaaata aaaattatat tggtaatttg aatagtaatt tgtaatataa 61920
   agtgttttta attgtaagag gtagttaatt attaaaggga ttataaaggg gtgatattgt 61980
   gatataagaa agatgtattt gggttgggtt tggtgtagtg gtttatgttt gtaattttag 62040
   tattttggga ggttgaggta ggtggatttt ttgagtttag gagtttgaga ttagtttggg 62100
   taatatggtg aaattttatg tttattaaaa atataaaaaa ttattttggt gtggtggtgt 62160
   gtgtttgtgg ttttagttat ttgggaggtt gaggtgagag gatagtttga gtttgggaag 62220
   gtagatgttg tggtgagttg agattgtatt attgtatttt agtttggatg atagagggag 62280
   attttgtttt aaaaattaat gtatttgttt gtttgtttgt ttatttattt atttatttat 62340
   ttatttattt atttatttag tttttgtttt ttaattttgt ttggtatgta gtttttaaaa 62400
   tttttgaaat ttttaaagtg atgggtgttt ttttgtttgt taaggaggtg attggtggtt 62460
   ggaggttttt ggatagtttg gggataaggg ttggttgtta ggggatttag ttttgtgatt 62520
   agaaggttag aatttgtagt tttttttatt tattttttgt gaggtaagag gggatgaagg 62580
   ttgagttgat tattaatggt taaagatttt atgttatgtt tatgtagtga agtttttttg 62640
   aaaatttaaa aggatgaggt ttgtagagtt ttaggttgtt gaatatttgt aggtgttggg 62700
   agggtagtgt atttttttgt ggaagtttta tgttgttttt tttatatttt ttttatgtat 62760
   tttttttagt tggttgttta tttgtatttt ttgaaatatt atttgtaata agttagtaat 62820
   tttaagtaaa ttgatttttt gggttttgtg agttatttaa gtaaattatt gaatttgaga 62880
   agggagttgt gaagatttta aatttgtagt taagttagat agaagttatg ggtaatttgg 62940
   tgttttatta tttgtgattg atttttgaag ttgagggtag ttttgtgaga tggaattttt 63000
   aatttatggg atttgtatta attttggttg gtgttagaat tgaattgaat tgtaggatat 63060
   ttagttgggg attgttgagt attggagaat tgtttggggg ttggtgggag gaaattatat 63120
   atttggtgtt agagttgaag tattgagtgt tgtgaatgag agtggagaga tagagtttgt 63180
   tttgttttat gtaagaggat tttaaggagt atagaaagag taaatatgag aagtagttat 63240
   tttagaagaa aatataggag gaaagttttg tgatattgga gttggtaatg tttttttgga 63300
   tgtgatattg aaaatataat aaaagaaata aatgataaat tggattttat taaaatttaa 63360
   aatttttgtg tattaaggaa tattattaat ggtgtgaaaa ggaaatttat agaatggaag 63420
   aaagtatttg aaaattgtat atttgataaa ggattagtat ttagaatata taaagaattt 63480
   ttataattaa gttataaaat aaaaaaaaat aatttagtga atagatattt ttttatagaa 63540
   gatatattaa tggttaagaa gtataggaag agatgtgtag tgttattaat tattagggaa 63600
   atgaaaatta aagttataat aaaatatgat tttatatttt ttaaggtggt tgttagttaa 63660
   aatatggata atagtaagtg ttggtaagga tatggagaaa ttggagtttt gtgtattgtt 63720
   ggtgggaatg taaaatggtg tagttgttgt ggaaaagata taataattgt ttaaaaaatt 63780
   aaatttagaa tgattataat atttagtgat tttattttta gatgtatatt ttaaagaatt 63840
   gatagtaggg atttgaatag gtgtgttata tttttgtttg tagtagtatt ttttttataa 63900
   ttaaaagatg taggtaattt aggtgtttat tagtggatga atgaatagat aaaatgtggt 63960
   ttatttgtat agtggaatat tattttgttt taaaaaggaa ggagaggtta ggtgtggtgg 64020
   tttatgtttg taattttagt gttttggaag gttgaggtgg gtagattatt tgaggttagg 64080
   agtttgagat tagtttggtt agtatggtga aattttgttt ttgttaaaaa tataaaaatt 64140
   agttaggtat gggggtatgt gtttgtaatt ttagttatta gggaggttga ggtaggagaa 64200
   ttgtttgaat tagggaggtg gaggttgtag tgagttgaga ttatgttatt gtattttagt   64260
   ttgggtgata gagtaagatt gttttaaaaa aaaaaaaaaa aaaaaaaaaa aaggagattt   64320
   tgatatatgt tatagtatag atgagttttg aagatatgtt aagtgataga agttagatat   64380
   gaaaagataa atattgtatg attttatttt tatgaggttt ttagagtagt taaatttaga   64440
   gagatagaaa gtgggatgga ggttgttaga ggttggggga agggagaatg aatgggaagt   64500
   tggtgtttaa tggggataga attgtagttt gggaagatga gatggatggt ggtgatggtt   64560
   atataatagt gtgaatgtgt tttatgttat taaattgtat atttgaaaat ggttaaaatg   64620
   gtaaattttt tgtgatggat attttaaggt atgtatgtaa atatatagaa gtagttatta   64680
   ttattggggt tgatggaaaa gaaattaaga atttggagtg ttttttatat tttgttatgt   64740
   tttatgttta ggttttgttg gagagtgtgt gattgttata gaaatttgta gtttattggt   64800
   aataaaaagg tttttgttag aaggtgtaga aagagtggat ttgtaggatt agtttattgg   64860
   gtagttgaga aatttattag agggtatggg tgggtaggag ttgggtgttt gagaagtttg   64920
   ttgaggtttt attgggttag agttggggtg ttggagaaat ttagtataga gtaggtttta   64980
   ttgggtatat gttattggta gttgtgtttt tagaggaaaa agagattttg gaattaggaa   65040
   gagaagttgt tattttagta aggtttggta gttttttata gtgttgtttt ttgtggatag   65100
   agtttgatat gattgtattt ggttaaggag aaagatttat aaggttttgg tttagtatta   65160
   tggagtgggg tttggtagga tgtatgtaga gttaagaggt aatatatggg taattggtat   65220
   atttttttag attttttgtt ttttttgttg ttttgttttg ttttgttttg ttttgttttg   65280
   ttttgagaga gtattttgtt ttgttgttta ggttggagtg tagtggtatg atttaggttt   65340
   attgtaattt ttattttttg ggtttaagtt atttttttgt tttagttttt taagtagttg   65400
   gagatgttta ttatgtttag ttaatttttg tatttttagt agagataggg ttttattatg   65460
   ttggttaggt tggttttgaa tttttgattt taggtgattt atttgttttg gttttttaaa   65520
   gtgttgggat tataggtgtg agttatattg tatttggttt atttttttag atttttaaag   65580
   tagattttta ggtttttttt tttttttttt tttttttttt tttatttttt ttttaatata   65640
   agtatttata gttttttttt aagtattagt tttagtttat gtattttagt atgttgtgtt   65700
   tttattatta ttttatttga aatattgtaa aatttttttt gtgatttttt ttttgattta   65760
   tgggttattt agaagtatgt tgtttaattt gtaaatagtt aaggtttttt tgagtatttt   65820
   agagttattg atttttaatt taattttatt ttggttagat aagtatattt tgtatgtttt   65880
   taaaatttga gatttatggt ttagaatgtg tattttggtg aatgtattgt ttttatttga   65940
   gaaaaaaata tgtatatata ttttagattt tttgagtata gagttttata attgttgatt   66000
   aggttaattg ttagttatta gtgtttttta gattatttga tgtttgtttt ttagatgttg   66060
   agagaatggt gtttgttatg gtttatttgt ttgtgttttt taaaatttat atgttggagt   66120
   ttaatttttg gtgtagtggt gttgggaatt ggggtattag ggaagtattg gtttatgagg   66180
   gtagagttat tgtgaatggg attagttttt tttgttatgt ggggatatag taggaaaagg   66240
   atatttttga agtagggagt tagtttttat tagatattga gtattttgat tttggatttt   66300
   ttagttttta gaattgtgag tattataatt tgtttataaa ttatttagtt taaggtattt   66360
   tgttttaata ttttgaatga attgaggtgg tgttaaaatt tttaattatg tatttaggtg   66420
   tagtggttta tatttgtaat tttagtattt taggaggtta aggtgggtgg attatttgag   66480
   gttaggagtt tgagattagt ttggataata tggtgaaatt ttgtttttat taaaaatata   66540
   aaaattagtt gggtgtggtg gtaggttttt gtaattttag ttatttggga ggttgaggta   66600
   ggagaattgt ttgaatttag gaggtggagg ttgtagtgag ttgagattgt attattgtat   66660
   tttagtttgg gtaataagag tgaaattttg ttttaaaaaa aaaaaaaaag agtttttatt   66720
   tatgattgtg gaattgttta tttttttttt taattttttt aggttttata ttatgtattt   66780
   tgaagttttt ttggttaggt gtatatatat ttatgattat aatggtgttt tgatgaattg   66840
   attatttaat tatgatgaat gtattttttt attttgtaat aattttattt tggatttaat   66900
   tttattgata tttatgtagt ttttttagtt tttttaaata tgttgtttgg gatgtataag   66960
   aaggttggag aggtttatgg agaaatttat gatggtttga tttatagttt tttaatttta   67020
   ttatggtgtg aatgtggtat gtatatagta gaaattgtat tttaagtatg tgtgaaatta   67080
   ttgtgttttt tatttttagt atagtattta ataatttata tgagatagtt aatattttat   67140
   tataaaatag gttttgtgtt tgttgatttt gtttaattgt aggttgaagt aagtgtttta   67200
   attatgttaa ggtaggttag gttaagttag aatgtttggt aagttagatg tattaagtgt   67260
   atttttgaga tgatatttat tttttattta tgatgggttt gttaagatat aattttatta   67320
   ttatttaagt agtattagta tgtttttttt taaatagaaa attttaattt gtgtttatat   67380
   tgaaatagta ttttttgtag atagtatata gttgagtttt gttattttat ttattttgat   67440
   agtttttgtt ttttaattgg aatatttagg ttatttttgt ttaatatagt tatagatatg   67500
   attgggttta gagttattat tttgtttttt gatatttatt ttttttattt tgtttttttg   67560
   gtttttttta tgttaattaa gtatttttta gaattttatt ttaatttatg tttttttttt   67620
   tttttttttt ttaagataga gttttatttt gttgtttagg ttggagtgta atagtatgat   67680
   tttggtttat tgtaattttt gttttttggg tttaagtgat ttttttgttt tagttttttt   67740
   agtatattgg gattatagat gtttgttatt atggttggtt aatttttgta tttttagtaa   67800
   agatggggtt ttattatgtt ggttaggttg gttttgaatt tttgatttta ggtgatttgt   67860
   ttattttggt tttttaaagt gttgggatta taagtgtgag ttattgagtt tggtttgttt   67920
   tgatttttta attgtatttt tttgtattgt tattttagta gttattttat agattttatt   67980
   gtatattttt agttttttat agtttattta gaattaatat tgtttttttt tatggaaaaa   68040
   aagtgtaaga attaggtaat tttatgggtt tatttattat tttttatttt gtatgtgatg   68100
   gttgttagat atttattata tttatatata ttgtaaatta gataatataa taaggaaatt 68160
   tttgttttaa atagttttat gtattttaaa gaaattaaaa gtaaaataga atttttttta 68220
   tttgtttatg tatttgttat attttgtgtt ttttattttt ttttgaagag ttgagttttt 68280
   atttggtttt attttttatg attttggaga atttgtttta tggtttttgt agtgtaggtt 68340
   tgtagatagt taattttttt ttatttttat ttatttgtaa atttttattt tgttgttatt 68400
   tttttttttt ttatttttat ttttattttt gagatagagt tttattttgt ttttttatag 68460
   gttatagtgt aatggtgtga ttttaaaatt tttgtttaat tttaatttat taattttttt 68520
   tttttgggtt taaatgattt ttttgtttta gttttttaag tagttgggat tataggtatg 68580
   tgttattata tttagttaat ttttgtattt ttagtagaga tgggatttta ttatgatggt 68640
   taggttggtt ttgaattttt gattttaagt gatttgtttg ttttggtttt ttaaaatgtt 68700
   gggattatag gtgtgagtta ttgtgtttag tttgttgtta tttttgaatt ataattttat 68760
   tggttataga attttgagtt gttagttttt ttttttagga ttttaaaata tagggtttta 68820
   gtgtttttgg tttttgttgt ttttgttaag gaattagtat ttatttagtt atttttttgt 68880
   agattatatg ttgttttttt ttggatgttt ttaagatttt ttatttaagt ttgattgttt 68940
   agtatttatt tttgggtaaa aaagttttta gaaattagaa atttatttag ttttattttt 69000
   ttttgttagg tgtagatttt attttagttt ttatttattt gagatatttt atattgattt 69060
   taggttgttg ttattttgtt tttttaaata ttttgtttag agttataatg ttttttgtag 69120
   aattgattag atagtagtta ttttttgtga ttaggagttt tgatttttta gatttgttga 69180
   tgggtttata aagtttttgt ttgtggtttt tagttggtag tggttttttt gttttgaagt 69240
   tgttatgagt gtgtaaaatt tataggttta atttttttta ttagtatagt ttagaaaagt 69300
   ggaatttttg gtttgggtgg ttatggtgtt ttagattatt ggttagatta gggtggtgat 69360
   tgttagttgg ttgaatgtgg ggtttttagt ttagtatttt tggattttat tttaggagtt 69420
   atggttgttt tagggttagg ggtttggttt tagtttttgt tttttttttt ttttttttaa 69480
   gttgtttagt attaggtttt aattagtatt ttttgtagat agggtgggtt ggttttttat 69540
   tagaggggta gggttgggtg ggaagtttgt tgagtttttt tttttgtgta gtttatagtt 69600
   ttggggttag ttttagaggg gtgtagaaag gagtttttaa aggtagaaat ttattttttt 69660
   ttttgttttt gtgtttttag ggataaagga gaggtggttt tagttggggg gttattagtg 69720
   gtatgtagga ggtttatatg attattgttt gttggggttt tttttttttt gtgagagaga 69780
   tgagagagag gagagagagt gtgagttagg gtagtgtttt tttttagtaa tgtagatttt 69840
   tgtatgtgtt ttttaggaga aattttgtta tttgttagag ttttagattt ggaagaagtt 69900
   ttgttttttt tttttttttt agtgtagtgt gtgtgtgtgt gtgtgtgtgt gtgtttttat 69960
   atgtattata tttttttata gaggaaataa tttttgttta agtgtaaagg ttgggattgg 70020
   agtaggtagt atgtgggtta aaggagtaga gaggaggaaa tgggggtggg ggtgagggtg 70080
   ggaataggag gtgggtagat gttatgaggt atgtttttta taattagaag ggttggtgga 70140
   gatgattaga atgttttaga tagatttttt attaaatttt taaaatttaa ttttgtttta 70200
   tgaagtttga ggattttgta gagtgaaaga taaggaaggg tttttttgta ggggggtgtg 70260
   tgtgtttgtg tgtgtttgtg tgttgtgtgt ttttatgtgt gtgtatgtgt ggatttgtgt 70320
   ggtgtgtgtt tgtatatgtg tgtatgtgtg gatttgtgtg gtgtgtgttg agtgtgtata 70380
   tgtgtgtgtt tgtgtgttgt gtatgtattt gtatatgtgt atatagattt tatttttttt 70440
   ttttttggtt tattaagtgt tagaaagaat ttttgtgagt ttttttattt tttttaaggt 70500
   tgtatatgga gggatttgtg taggtatatg tgtgtgtgta tataatatat atatatatat 70560
   atatatatat atatatatat atatatgagt taggggtgag tatttttgtg gattgtattt 70620
   aggtttggta ggaaattggg ttttgggaaa ttgtaggttt tatttttggt agaaaattat 70680
   gtttttgttg ttgtttgttg aagggaatat taagtggggt tggggatagt ttatgttgag 70740
   aagagtaaat tttgtttatg gtattgggga tattgagtgg tttttgtttt gggttttgga 70800
   gggtttggga gatatgtttg tagttttggg tattttttaa ggttatgggt ggttatattt 70860
   aaggttaggt ttttttttgt gtgagttgtg gttagtagga aggttgattt tagaggtggt 70920
   tttagggttt ttggaggttt tgtttttgag atatgggtta aggttagaga gttgagaagg 70980
   ttgttgttga ggattttggt tggtgtttag tttagtggtt ttttaggtgg gttttgtgta 71040
   ggggttgatg aagttagttg ggaaatttgt gaaataaagt ataggttttt taggtttgtt 71100
   tagagttttg gtattagatg aggtgtagag aagggaattg agttgttaag tgtgaatagg 71160
   agagaggagt tttaggatta tataggggtt ttgatatggt aggtgtttgg ttagtagtgg 71220
   tgttgttttt attgttggat gtgggaaaat gtattgatat ttgttgatta gagttggggt 71280
   gagtatttgg gtgattttgt agatgagata aaataagggt tgttgtgggg gaggggtttt 71340
   tgttgggtta ttttgttgag gttttgtgtt attgttttgt ggttgttata ataaaggatt 71400
   ataaatgagg tgatttaaaa tatttgaaat tggttatgtg tggtggttta tgtttgtaat 71460
   tttagtattt tgggaggttg aggtggttgg attatgaggt taggagattg agattatttt 71520
   ggttaatatg gtgaaatttt gtttttatta aaaatataaa aaaaaaaaaa aaattagttg 71580
   ggtgtggtgg tgggtgtttg tagttttagt tgtttaggag gttgaggtag gagaatggta 71640
   tgaattttgg aggtggaggt tgtagtgagt tgagattgtg ttattgtatt ttagtttggg 71700
   tgatagagta agattttgtt ttaaaaataa aaataaaaat aaaaataaaa atatttgaaa 71760
   tgtttttttt aaagtttttg gagttagaaa ttttagattg aggtgtttga aggtttattt 71820
   tttttggggg ttttgaggga gattgtttta tgtttttttt tgagttttgt ggtggttagt 71880
   aatttttggt attttttggt ttgtagtttg gtttattttt tatttggttt ttattttgtg 71940
   tttttttttg tgtttttgtt taaattgttt tttttgttgt ttttttaatt tatttttagg 72000
   gataggtttt tattttgttt tttaggttgg agtgtagtgg tgtgattatg gtttattgta 72060
   gttttagttt tttaggttta agtaattttt ttattttagt tttttaagta gttgggatat 72120
   aggtgtatgt tattatgttt ggttaatttt ttaatttttt tgtagagatg agggtttagg 72180
   ttggttttaa atttttgtgt ttaggtaatg agattttgtt ttttaaaata ttgggattag 72240
   agatatgagt tgttgtgttt agtttatttt tgttttataa agatatttgt tattggattt 72300
   agggtttatt ttgatttaat atgtttttaa ataatttaat tatattttta aagattttat 72360
   gtttatataa agttatattt ataggtattg gggggttagg atttgtatat gttttttggg 72420
   ggaatataat ttaatttata atgggtttta tttatagaat gaatggtttt tttagggtgt 72480
   gtgtaggtgg gtgaggggtt taggagaggg atttgtgtgg taagagggtt tgtgtttatg 72540
   ttagagattt tagaaggaga ttggatgggg tggttgtatg gagtaagggg ttatagttat 72600
   tgtttttaag tagttggtgg gttgtttggt agaaagggag tatttatttt gagttttttt 72660
   aagatatgaa gttagtatta ggtatttgga aggtgttagt gagtagagtt tgatttttta 72720
   tagaaagagg tgtttttttt ataatgttta tttgaggatg gtggaatagg ttattttata 72780
   aggtagtgag ttttttatgt ttggaagtat ttaagtaggg gttatgtggg agttgtttag 72840
   gagtgtatag aagggatttt agaggttttt gttaatatta tttttgtttg ttttatttta 72900
   tgataatttg aattttagaa tgtaatgtta ttggggatat gtttattaga ggggttttta 72960
   gtattaggtt ttatatatta tgtattattt ttagggtagg gaatgagagt aggttttatt 73020
   ttttttggtt gtttgtgagg attaggaggt ttgttgttga ggtagttttt tgtggaaata 73080
   gtgttttagt tttgtaattt atttattgga gttttaatat tttaatgaaa tattgtgtta 73140
   gatgggtttt gggtatgtgt gtgtagattg ttataagttt atgagaatgt atatatgtat 73200
   attgtatgtg tgtgtatata tgtatgtata tatgtataag aatatgaaga tttgaggtag 73260
   gtatagtttg tgagatagat ttttttaggt tgaaagattt tttataaggg tttttagaat 73320
   aaggttttag ttttggtagt tttttttatt tttggtggga ggatttttat agagaagaag 73380
   ggttaagaag tatagttttt gatgtttatg gtgtattaag ttttgtttta agtttgtgtt 73440
   ttatttttta tttatttagt aaatttgttg tttttgtttt gtgttagatg ttgtttttag 73500
   tattttataa atattgatgt atgtttgttt tatgataatt ttgtgaagta ggtagtgtta 73560
   ttttgtttta tagatgagga aataggtgta gagggataaa gggatttgtt taaggtttgt 73620
   ttttatttta tatgtatatg aatgtttatt tatgtggtag gtgttagttt ttttttgatg 73680
   tgagttaggt tttgagttgg ttggggtttg ggttttattt tggttgtgtt ttgttgtggg 73740
   tattaaggaa gtttttagta gttttatttt tttggtatga tttagtttta gggattggat 73800
   ttttttaaat gtttttgttt agtttggagt ttttttttta gttgggggag ggttgaatga 73860
   ttttttattt ttggaggttg ttggagtttt ttttgtttgt atgtggttgt tgttgggtgg 73920
   ataggtttgt ttgtgggttg aggtttttag tagttggggt ggattatttt tttttttaag 73980
   aagttttggg agaagtagtt gagggggtta gtgttttttt gaggttaatt ttttgttttt 74040
   tttagtgtag gtgtgaagtt atttttgttt gtgtgtttgt tatagatagt ggagggtggt 74100
   gtaattgtga tgtttgtttg ttgtattttg tgtttttgag gatgttgatt tttggttggt 74160
   gttgggtgtt tgttttttag ttattttgtt ttgatttggt gtggtaggtt tgagtattgt 74220
   ttttttataa ggaaataggt ttagaggttt tgagttattt gtttgggttt ttttagttaa 74280
   taattggttg agttaggatt tttatttagt tttgtgattt tagaagttta agtttttttt 74340
   attatgttag ggttaggaaa ggattagaat gtttttttat gtgttgatta tttggatgat 74400
   ttgtgtagtt tagtttgttg tagttgttta ataaaaatgt ggaatgaatg ggtttatatg 74460
   ttttattgtg tgatttagag ttaatgattt tggttgattt ttttgttttt attttgtggt 74520
   ttagtatttt tttttggggt gtgtgatttt ggaagtagtg atttttttta ttttattttg 74580
   aatggtgttt gtgttgtagt aggggagtag ttggggattg ttagtgtttg ttgttggtgg 74640
   gttgtgtgtt agattttgtt ttggatgtgt gggtgattgt tagttatatg tgtgtgaagg 74700
   gttgtgtgtg taggatttgt gtaaatgtgt ttttttttgt tgttagttta tgggaggttg 74760
   ttaagatttt gtttttgtat attagtttat attttttttg tggtttattg ttgtggttgt 74820
   ttttttgtgt tatgtggttg taggtatagt ttttaagtgg atggggattg tgggttgaat 74880
   gtgtattaga tgtttttgtt ttttggtggg tggagatttt taatttatgt attgtggttg 74940
   ttgtatttga aaggtttttt atggtgaagg atttggattt gagtaatttt ttgtagaggt 75000
   tttagaagtg ttttttggtt gggtgtggtg gtttatattt gtaattttag tattttggga 75060
   ggttgaggtg ggtggattat aaggttagga gattgagatt attttggtta atatggtgaa 75120
   attttgtttt tattaaaaat ataaaaaatt agttgggtgt ggtggtgggt gtttgtagtt 75180
   ttagttattt gggaggttga ggtaggagaa tggtgtgaat ttaggaggtg gagtttgtag 75240
   tgagttgaga ttgtgttatt gtattttagt ttgggtgata gagtgagatt ttgttttaaa 75300
   aaaaaaaaaa aaatgaagtg tttttagttt ttttttttgt ggtgaggttt tgagttttta 75360
   tttttagaag aaaatttggt tggggagggt atttttagat ttggatataa atgttaggag 75420
   attagggttg tttttttagg tgattaatta tagagattta gggaaagtta tgtttggaag 75480
   aggtgatatt gtgttttagt tttatttatt tttttatttt atagatattg tttggtgttt 75540
   agtttgtatt gtgaggtttt ttaagtagtt ttgggtattg tgtatttggt atggttttta 75600
   gttttatttt tttgttttgg gagatttggt ggggattttg ggttgagttt ttgtggttgt 75660
   ggaattttgg tggtttattg tggtggttga gaatagggat gtggggttgg atgggttgag 75720
   tgtaattttt agtggttttg tgaattgttt gggagttttg ggtagttttt ttttattttg 75780
   tgtttttgtt ttttgatgga aaaatggggg ttagggggga gtgattttga aggtatttgt 75840
   gggaattaag tgaggtgata aatgtagtgt ttttggtaga gaattggata tagagtttat 75900
   ttttatgaag ggggttgttt tttttttttt tatgtttttt tttgttttta tttttttttt 75960
   tgtatttttt ttttttgttt tttttttttt tttttttttt ttttatatgt aggtatattt 76020
   tatttggtgt tttaattgtt tttttttatt gtttttttgt aagtggtttt ttaagtggga 76080
   atttaggttt ggaaagggtg ggtattttgt ttggtgggtt ttggtaggtt aggtgttgag 76140
   tagttttagg agatataatt ttaggaaaag gtttatgtag gttggttgtt atgatggatt 76200
   gttggtggga gaggggaagg tgttgtggta ggggaagttt tggaaatatt tattagtggt 76260
   tttatttatg atttaggata tttgttaggt tattagaata tagttgagga agttgttggt 76320
   tagatgttta gaagtattaa tttggtggtt gggttgtggg gggatttttt tggataggta 76380
   gataaggtgt tggaggaagg ggttgatatt tatttagtgt ttgttatgta ttgagggtat 76440
   aaagggtagg atggagagtt ggtttatgtt atttgtggaa ttttttaggt gtgggggtag 76500
   atggggaagt aagggggttt gaagatatat gtgtttatta aatgttttta aattttaaga 76560
   agaagaataa ggatatattt ataggggtag ggttgaaagt ttgatgtatg gtgttttggg 76620
   gtttgttttt gttgtaatag ttatagtggt ttttttttat tttttttttt tttttatttt 76680
   gttgtgaaaa tgtttaaaat atatggttaa gttgaaagta ttttataggg aatatattta 76740
   gttattattt agattttatt attaatatta agttatattt gttttattat tttattgttt 76800
   atttattttt tttttgtttg gttatgaatt tattttattt ttattttttg gtgtatttta 76860
   aagtaatttt aggttatttt gattattttt ggaattattg ttagaaattt tttttttttt 76920
   tttttttttt tttttttttt ttttttttga gatgggattt tgttaggtta tttaggttga 76980
   agtgtggtgg tgtagttata gtatattgta gtttttaatt tttgagttta agtaattttt 77040
   ttgttttagt tttttgagta gttgggattg taggtattta ttattatatt tagttttatt 77100
   tttaaaattt tttattaatt ttttattttt tgtatttatt taggattatt ttttataaag 77160
   ttagggtgtt gtaggagggg ttagaatttt aagttttaat tttggttttg ttattaatat 77220
   tgtgtgattt ttagtaaatt attttgtttg tttaggtttt gtttttattt ttatgaaatg 77280
   agggtgttaa attgtatgtt ataaataatg agggttatta gttattaagt ttttatttta 77340
   ggttaggtat tttttatatg ttattatgag tttttttaat ttaggtattg tttttaatgg 77400
   atagtttagt taatggaagt ttagagaggt ggtgtaattt gttaaaagtt ttattattta 77460
   gtgaatgttt ttatggggtt tgtatttagg agtttgatat agagtttagg ttttagtatt 77520
   tggtgatgtt ttgggggtgt gagtagttta gtttattttg ggtatgtgtt tatttgttgt 77580
   tttttttgtt ttatgtttgt gtttgttttt tttgaagttt agattgttat tttttagttt 77640
   taaatattaa aattggttag gtatggtggt ttatgtttgt aattttagta ttttgggaga 77700
   ttaaggtggg tggattattt gaagttagga gtttaagatt agtttggtta atatggtaaa 77760
   attttatttt tataaaaaat ataaaaatta gttagatatg gtggtgtgta tttgtagttt 77820
   tagttattta aggagaattt agttatttag ttattgaggt atgagaatta tttaaatttg 77880
   ggaggtggag gttgtagtga gttgagattg tattattttt ttttaggtta ggtaataagt 77940
   gtgagatttt gttttaaaaa taataaataa ataaaataaa ataaaaatta aaattaaatg 78000
   ttaaaattaa tttataaaat aaattttggt taaagtagtt tgtttatttt gttttaatta 78060
   tgtatattgt attagatttt gttaggggtt tagtgtataa agatgagatt tttgttttgt 78120
   tagataggtg ggatgatggt tatattttga gttagttaag gtattatttg gttgtgtaat 78180
   aaaagttaaa atattagagg ttgagtagta tttggattta tgttttagtt taagtgttag 78240
   ttgttatggg ggtttaggat agtttggatt tagtttattt ttttggtgta tttttgatat 78300
   atagttttta ttttttgagt tgagatggtt ttagtttttg ttgttatgtt tgtattttag 78360
   ttagagggaa gggaagaagg gagaggggaa agggtaagtg ttttttttta tttttaaggg 78420
   tatgatttga aagttaaata ttttagtttt tttttattgg ttagaattta gttgtatggt 78480
   tgtttttgtt attagggagg ttgagtaatg tattttttag ttgggttttt ttgtgtataa 78540
   ttaaaatttt agttattata gaaaaaaggg agtttagata ttggggaaat aattagtatt 78600
   tgtattatat gtattgatag ttttttttgt atgaatagag ttgtggtagg ttttatgtta 78660
   gggagaaagt aagattggaa aagagtttat taaggaggtg gtatttgtat tgtgtttaag 78720
   gggtaagaaa aatgtttttt aattaggagt tataaatgtt tggttgaagt gttattgttt 78780
   ttttattttg gagttggaat agatgttatt agttaattat gatggttgtt gggtgtattg 78840
   gttaatattt ataattttag tattttgtga ggttgagggt gggaagattg tttggggtta 78900
   ggagtttgag attagtttgg gtaaattgta agattttgtt tttgtaaaaa aatataaaat 78960
   gtagttgagt gtggtggtat ttgtagattt agttttagtt atttgagagg ttgagatggg 79020
   aggattgttt gggtttagga gtttgaggtt gtagtgagtt atgattgtat tattgtattt 79080
   tagtttgggt gatagagtag gatttgtttt taaaaataat aaaataaaat taaagtttta 79140
   aaatggaaaa aaaattatga gtatataatt ttagatgtat ttttaagata gtaaatttgg 79200
   gtgggtgtag tggtttatgt ttgtaatttt ggtattttgg gaggttaagg tgggtggatt 79260
   atttgaggtt aggagtttga gattagtttg gttaatagtt ttatttgtat tataaatata 79320
   aaattagttg ggtatggtgg tgggtatttg tgattttagt tatttggaag gttgaggtag 79380
   gagaattgtt tgaatttagg aggtggaggt tgtagtgagt taagattata ttattgtatt 79440
   ttagtttgga gaaaaaaagt aaaattttgt tttaaaaaaa aaaaaaaaaa gtaaatttag 79500
   ggttaggttt gtagttatat ggttattttt ttttttgagt atggaggtag ttttagaaat 79560
   ttgttttata ttatattagt gggtagttga ttgggttagt ttgttagttg aattttattt 79620
   gttatagtta ttttttatag taaggggtat tttagatata tttttttttt tagggaagaa 79680
   taagttgtag ttgtttgagt gttttattta gtggtgtgta tgtttttttt taattttgtg 79740
   tttagtgatg ttttgtttgt agtttgaaat ttgttatagt gggtgtgttt atattatagg 79800
   aattggtaaa tattatatat taggttttat tttttgtttt ttattagggt aggttgtttt 79860
   ttttttgttg tttttgtttt ttgttttttt ttttttttga gatggagttt tgttttgttg 79920
   tttaggttgg tgtggagtga tgtgattttt gtttattgta agttttgttt ttttggttta 79980
   tattattttt ttgtttgagt ttttagaata gttgggatta taggtgtttg ttattatatt 80040
   tggttaattt tttgtatttt tagtagagat ggggttttat tgtgttagtt aggatggttt 80100
   tgattttttg attttgtaat ttgttttttt tggtttttta aaatgttggt attatgttat 80160
   tgtgtttggt ttagggtagg ttgttagaaa tgtattagtt tgttagtttt agttttgttt 80220
   gttttttatt tttttttttt tttttttttt ttttgagatg gggttttttt ttgttgtttt 80280
   ttaggttgga gtgtagtggt gtgattttgg tttattgtaa tttttgtttt ttgggattaa 80340
   gtgatttttt tgttttagtt ttttaagtag ttgggattat aggtgtttat tattatgttt 80400
   agttaaattt tgtattttta gtagagatgg ggttttatta tattggttag gttggtattg 80460
   aattttttat ttaaggtgat ttatttgttt tggtttttta aagtgttggg attataggtg 80520
   tgagttattg tattgggttt tttttgtttt tttttaatga aaattttttt gaaagatttg 80580
   tttattgatg tttgtgttaa taggtattgt ttgtgggata gagtttgttt tttttttgaa 80640
   ggtttttttt tgtttgggtg attatttgta tgttattttt tgttgtttag aattaaagat 80700
   gtagttatgt ttggggtagg ttgggaaaat ttgtgggaat tatagagttt gttgttatgt 80760
   tgtgttttgg atatttgaag tttggtaggt aggtgggatt gagggtgggt attaggaaag 80820
   tgatgtggtt ttataggaag gggatatagg ttttgagatt ttaggttttt aattggataa 80880
   agttgttttt tttttttgga taaatgtttt agtttattta taattgagtg gttagttttt 80940
   tttttttttt tttttttttt ttttttgaga tggagttttg tttttttgtt taggttggag 81000
   tgtagtggtg ttattttggt ttattgtaag ttttgttttt taggtttatg ttattttttt 81060
   gttttagttt ttggagtagt tgggattata ggtgtttgtt attgtgtttg gttaattttt 81120
   tgtattttta gtagagatag ggttttattg tgttagttag gatggttttg attttttgat 81180
   tttgtgattt gtttgttttg gttttttaaa gtgttgggat tataggtgtg agttattgtg 81240
   tttggttaag tggttagttt ttttagttgt tgggttttgt atattttgtt ggttttggag 81300
   attatatgta ttatgtagat tttatttgtt ttttattttt agtgttgggg gaggggttaa 81360
   gggtttgttg atgtgttgat atttggttat ttattggttt tgaatgttta tatttatttt 81420
   tttgaaattg atgttttgta ttggaggttt tatgtggatt tgggggtaga ggtttgtatt 81480
   ttagatggtg gggtggtttt tattaattag gtagattttt tgttttgttt tgttttttgg 81540
   gttttgtagt tttagatgtg tgtttaagat ttgtggttat atggatggga taagttatag 81600
   agatttagat tttgtggttt ttgtgattag agtagttgtg gttttgtttg ggaatgtttt 81660
   tattttgttg ttgaaggtga gaaatttttt tgtgttggag aatagttgtg ttgagaagtt 81720
   ggtttatttg gtttgtagga ttgttggggt aagggaggtt tgttgttggt tttttttttt 81780
   tttttttgat tgggtgtttt ttgtttaggt tttttgtagt aaaagaattt tttaatttat 81840
   tttgtttttt tattgagttt ttattttatg aagtttattt tttaatttta gttgattggt 81900
   ttttattttt ttaagaggaa ggttaggggt agatgaggtt gaaggaggtt gatttttttg 81960
   tgtttttgta gtttgagttg atgttgttgt atgtttatag ggttttttta gtttttaaaa 82020
   aggttttaat atgtttgtta tatgtttgtg gtttttttgg atgttatatt aatttagtaa 82080
   ggtttgagtt agaggttgtt tttgaggttg tgaaaataga agaagggttt agaggagaat 82140
   ttttttattt gatgatttta ggaaggtttt ttagaagaag tagtttttga gttgagattt 82200
   gaagatagat taggtttgag ttaggttagg gagggtgtga ggggtttatt aggtagaggg 82260
   aataggatag ggtaggtata gaggtttggt atttggtttg tggggaagtg tgagtttttg 82320
   ggtatgatta aggtggggag aagtggtgtg agaggaggta gatgtggttg gtaggagatt 82380
   ggtggttttg gttttgttga gttttatttt gtatgtatta ggtgatagag gttgtttagg 82440
   atggtgggta gggaagtgat aggttatatg gttattttat taatagttgg gagtttagtt 82500
   tggggggatt taagattttg gggaaggtat gagttagggg tttgttagag ttaggagtta 82560
   gtgggagaag gaaatagtgt tgtttatgaa ttataaatag atatttttgt aatttttttt 82620
   gatgtgatat aattttaaag tgggttattt agaggtggga aggtgatttt tggtttttta 82680
   ggttttgggt ttgttttttt tttttttttt tttttttttt tgagatggag ttttattttg 82740
   ttgtttaggt tggagtgtag tggtgtaatt ttggtttatt attatttttg ttttttgtgt 82800
   ttaagtaatt tttttgtttt agttttttga gtagttggga ttataggtat ttgttattat 82860
   atttggttaa tttttgtatt tttagtagag atggtgtttt attatgttgg ttaggttggt 82920
   tttgaatttt tgattttagg tgatttgttt attttggttt tttaaagtgt tgggattata 82980
   ggtatgagtt attgtgttta gttgagttta tttttaagtt ttttttggtt tttagtgttt 83040
   ttgtattagt ttattttttt ggttttttgt ttttaagagt tttttttaag attgttttta 83100
   agtggatagg tttttagaat taaggagtag ttttaggagt ttttagttat agatatttta 83160
   gggggtttgt gtggttttta gtttttggtt tggttttggt agttttggtt ttttgtgttg 83220
   ttttttatat agtttggtta gagttggtta ggttttttgg ttgggttgag ttattgtggt 83280
   tagtgattta tgtaggggga gaaaagtgag gagggggaat agagtgtgag ggggattgtg 83340
   ttaataataa atttattagg tttttgaggt tattttatag gagggataaa tggtttattt 83400
   aggttgattt ggttgttggt tggtttttag agatatttag ggtggggaag attttattat 83460
   ttggggaaag taatattaga ttgtttggag gggtttagtt agtgtatttt gtgttttttg 83520
   tttgtttttt atgtagagtg ttttgttttt aaggtggtag gttaagagtg tgagttgagg 83580
   tttaggtgta aagaggggtg tggtatgggg gtattgtagg gtatggggtg tatgtatttt 83640
   tatttatttt tttattgagt gttagggtaa tttgttaatg gggtatattt ggtgatataa 83700
   aattttttgt tgatttatgg tagtttggaa tggagtggaa gtgttttatt tttggggaat 83760
   ttttatatat atttggttgt gaatgttggt gtttttgtat tggagttttt ttggagtgtg 83820
   ttgggtagag tggagaggtt tttttttttt tttttttatt attttgtgga ggtgttaata 83880
   tgtagttaga gatggttagt ggtagtgagg agaggggggt aggaagagat tagggttggg 83940
   ggtttgttag tttatagttt tatttttgaa tatttatggt ttttttgttt aggttagtga 84000
   ggttgggggt agagatgggt aggggttttt taatgttggg tatttttttg tttttttata 84060
   ttttggattt gttttttgga gaaagttttg gagtatttag aagttttggg gttgttggtt 84120
   gaggtgggga gtagagttta gttgtgtttg gaggtttttt atttttattt agtttttagg 84180
   gatagtttta gttgtgtagg tttaggttgt tgaggttggt tttagaaatt tttttataat 84240
   tattattgga tggaaaatta gttttttaaa gggttttaga gttagttttg tttttttttt 84300
   tataggtttt tatagttaga gtattgggtt tttgttagag atgtagaaga gatgatgttt 84360
   ttagttttta tgtggtttgg tttttattgt ggatgttggt gattttagtt ttttttttaa 84420
   aattaagagg gttgatgttt tatattttag gtttttagaa agagttgtta tttatttata 84480
   ttgttatatt ttttttttat ttttttgttt tggaaagagt agagtggata tgtgtttttg 84540
   tagaaattta tttatagttt gattgtagat gtattttgtt tttttattga gtttttgttt 84600
   tatgaagttt gttttgtttg tatttttttt tttttttttt tttttttttt tttttttttt 84660
   tttttttttt gttttttttt tttttttttt ttttttgttt tatttttttt tttttttttt 84720
   tttttttttt tttttttttt tttttttttt gtttttttta tttttttttt tttttttttt 84780
   tttttttttt gaatagttaa agatagaatg aatggtatag gtaggtattg agttttttgt 84840
   aattggaggt gtttaagtgt agggtggatg ggtatttata gggtaggtat tgaggaagga 84900
   tttttttggg attgtggatg gtaggatagg ttttgtggag ttttttgagt gggtgtagaa 84960
   gagggagggt atgtttttgt tttttgtttt ttttgggttg ttaggttgtt atttttaatt 85020
   tttatttttt ttttttttat ttgttgggtt ttatgggatg ggtagaggta ttgttagaat 85080
   atggagttgt ggtttataga gggttagtag ggaggagagt tttttgggga ttatgttgtt 85140
   ttagttattg tggtgatttt gaaaataaat gttttttttt ttgtttgagt ttttgttgtt 85200
   tttgttgatg tggaattagg gtaggttggt gatttagttt ttatttttgt attgtattgg 85260
   tggtaagttg tttttagttt tagattttat agagtagggt gtttttttta gaaaaatagt 85320
   agggaggtta ggatattaag ggttttaggg atagtttgtt ttttttggtt ggtatgagtt 85380
   ttagttaagt tttttggtat tttgggggtg aggaggtatt agttgtgtgt tttagttatt 85440
   ggttgttttg aggttgggtt ggtgttaggt tggttatttt tttttgtttt ttagtgtgat 85500
   tttgtagagt ttttggaagg gttagagggt ttttttttat tattggtgtt tatgggatat 85560
   tgtgttttta ttttattttt gtattttagt tttagatggg tgttaggaag ttggagattt 85620
   tagttatttt atgattgtgt tttgtggggt tggagtagga ataggagtag gaggaatgtt 85680
   ttggttatgg gattgtgtga agagagaagg attaggaaga aaatatgagt tattatgttt 85740
   gggttatatt ttagtagagt tgttggtggt tttttaggta tttgaattta ttaattataa 85800
   agtttattag gtgtgtaaat aaataaatgt tgtttaggag gtatagttag ggttttgttg 85860
   gagagggatt attttggagg ggttatattt tttatttagt tttttagttt ttttgttttg 85920
   tgtgtgtgta gtttttgatt attattattt ttgtttttgt tatttgtttt tagggtttta 85980
   ttaggttgga tgttattgta ataaaatgta gtaatagaag gtttaggtag tgttgtagtt 86040
   tttggtggta ttattagagt tagtaggtag tagggttggg gaattttaag ttgatttagt 86100
   tttaagtgtt ggagtgggga gtttggatga ggtgggggtg gggaattggg gagttatgga 86160
   gtagagtatt tgttgtgttg aaggttagga gtttttttgg gagattatga atttagaata 86220
   ttaatatttt gtttaaaaag ttttttgttt tggagataag agtttaggtt gttgtggtta 86280
   ggttttgttg taaatgtttt gtataaagta ggttgggggg attattggga agtttgggga 86340
   atagattggt tatgtgtttt tttgagtgaa attttatggt tgtattgaag tggttaggta 86400
   gagggtgtga tggggtgtgg tagttgtata ttggtttagt taggttgaat ggggaaatgt 86460
   aaatttaggt ggtttgaaag agatttagtg gtttatagtt tggtggttgt ttttagtata 86520
   ggtgatgtaa agagagaagg ggttttgggg gtttggttta ggtggaaatt atgtattgtt 86580
   tgtgattttg ggtatgttga ggttggagta ggtttgtttg gtggttggta gaatgtatat 86640
   gaagaattta tgtgtgtttt gaatgttttt tttgtttttt tgggtttatg ttttattagg 86700
   agttttttga ttttgttatt ttgtaaagga aattgtgttt tgttattgta taggattgaa 86760
   taggtgtgtg tggagttttt aaagttattg ttttaagggg ttttatggga taatgggaga 86820
   gttgttgtga tgtttaagat taaagtgatg gttgggtgta gtggtttatg tttgtaattt 86880
   tagtattttg ggaggttgag gaggatggat tatttgaggt taggagtttg agattatttt 86940
   ggttaatatg gtgaaatttt atttttatta aaaaaataaa aattagttgg gtatggtagt 87000
   gggtgtttgt aattttagtt atttgggagg ttgaggtagg agaattgttt tgatttggga 87060
   ggtggaggtt gtagtgagtt gagattatgt tattatatta tagtttgggt gatagagtga 87120
   gattgtttaa aataataata atgttaataa taaataaaat aataataata aaataataaa 87180
   agtaaggatt tgaataaata tttgtatatt tatgtttgta gtagttttat ttttaattgt 87240
   ttaaatgtgg aaggaatgga tatataaagt gtgtgtatat atagtggaat attatttagt 87300
   ttttaaaagg aatggaattt tgttattata tgtgaatttt gaataatgaa ttttgaagat 87360
   tttatgttat gtaagtttgt tttataagga taaatattgt atgattttat ttatgtgaag 87420
   ttaaatttat agagaaattg gaatgatggt tgttagtagt taggggaagg aaggatgggg 87480
   agttattgtt taatgaggat agagttttag tttgggaagt tggaagaagt tttggaggtg 87540
   gatggtgttg atgggtgtat aatgtgaatg tatttaatgt tattaatatg gaaatttggt 87600
   taaaatggta aatttgtttt ttttgttttt gttttttttt tttttgagat ggagttttgt 87660
   tttgttgttt aggttggagt gtattggtgt gattttggtt tattgtaatt tttgtttttt 87720
   aggtttaggt gatttttgtg ttttagtttt ttagagagtt gggattatag gtgtttgtta 87780
   ttatgtttgg ttgatttttg tatttttagt agagataggg ttttattatg ttggttaggt 87840
   tggttttgaa tttttgattt taggtgattt atttattttg gttttttaaa gtgttgggat 87900
   tataggtgtg agttatggtg gttggttaaa aatggtaaat tttatattat atatatttta 87960
   ttatagtaaa aatgtattgt tttattagaa aaatgataga tttttataat atgaattaaa 88020
   ttataagtta tgaaggaaaa attaagtaaa atattaaaaa attatttata agtaattata 88080
   tttaaaataa aagataataa atatataatt tttattattt tttaggtttt ttgtaatatt 88140
   taattattgt ttgttttgtt gtggttatgt ttttgaggtt gtatttgtat gatagaaata 88200
   ttatatttgt ggtgattaaa tatttttttt tagttttatt tttaatgatg ttatatagat 88260
   aatttgaaat tggttggttg ggtatgatgg tttatgtttg taattttagt attttgggag 88320
   gttaaggtag gtagattatt tgagtttaaa attagtttgg ttagtagggt gaatttttat 88380
   ttttagtaaa aatataaaaa ttagttagga gtggtggtgg gtgtttgtaa tttgagttat 88440
   ttgggaggtt gaggtaggag aattatttga atttgggagg tggagtttgt agtgagttag 88500
   gattgtttta ttgtatttta gattgggtga tagagtgaga ttttgttaaa aaagaaaaga 88560
   aaagaaagaa attggttaag gtaggaatat tgatattata ggaataggta agtgttataa 88620
   attgggaaat tgagtgttgt tgtttttgtt ggttttaggt ggttggttgt tgattgttaa 88680
   ttggtatatt attggaggag ggaggtttag attgatttgt agagagatta gaggtagaaa 88740
   tgtattatgg tttggatgtt gagggtgagg gaaagagagg agttaatagt ggtgtttgga 88800
   gatttggttt gagtaattag gtggatggta gtattgtttt ttaagatgag gggttgtggg 88860
   aatttgaggg gttgtggaag gttttgttat tgatggattt agggtttggt agttttggga 88920
   ggtttatttt gtgttaggtt atgtagagat aagattattt gggttttgtt tttgggaagt 88980
   ttttgtttat gaggatatag tttgttattt aatggggtgt tttgtttgga gttttagtgt 89040
   tgttgggaga aagtttttta tttttttttg ttttttgagt tttttttgtt tttttgttag 89100
   ttgagttttt ttatggaggt tgtgtttgga ggggggttat ggatatggat gtgtgtgtta 89160
   aatgttgttt ttttgtggat ttttttttta ttttgttttt atgttttgta agttttattt 89220
   ttttggggtt tgatttgtgg ggtttttttt tagatttgtt tttttttatt tttagaaagg 89280
   tgtgtagggg ttttttatgg tttttttttt tgtttttatt tttttttttt tgggatagtg 89340
   atttgttgga tgggtgggtt atttataggg tttgtgattg tttagatatt tttgttgtat 89400
   gaagttttga ttttaaggta tgatgtagat gggaataagg tatgagtttt ttttggttgt 89460
   tgtaataatt gttgaaattg ggtggtttat agtaagggga atgtgttttt ttatagtttt 89520
   ggaggttaga agtttgaaat taaggtgtta gtagggttag ttttttttga agattttagg 89580
   ggaaaatttt tttttgtttt ttttagtttt tggtggtttt aggtgttttt tggtttgtgg 89640
   ttgtattatt ttggtttttg tttttgtttt tataaggttt ttttttgtgt ttttgtattt 89700
   ttttttttgt tttttagaag gatatagtat tgaatttagg gtttatttta ttttgagatt 89760
   tttgtttaaa ttttttttgt agagattttt aaatagaatt aggttatatt tgaggtttta 89820
   agtgggtata tttttgtttt gggggggatt attggtttat tgtaggtttt gttgtgtatt 89880
   gaagatgtag gtggtttttt tggttaggtt tgtagttggt tttgtttttt tttgtgattg 89940
   tggaagaatg tattttgttg gatgtatagg tttttatagt ttttttgttt ttgttaaatt 90000
   ttagtttagt tttttttttg ttgttggtgt gtattttttt tgtttagtgt tttttttggg 90060
   attgtgtgtt taggttttga ggttttagaa aaggggggtg gagggttttt ttattatttt 90120
   gtagtaatga ggttttatta aaattttgga gttttagatt ggatttagtt tttagttatg 90180
   attaaaggat agtttgggat atgattaaag ggttagagaa agatgattta ggttgagaaa 90240
   gaggaggtta aggagtaatt ggattgtttt tgaatgtgga aaaggtaatt atgatttgaa 90300
   gggtggtgtt tagttgtttt ttattttttt tgaggatagg aaaaaaaagg aaataaatga 90360
   tgtataatta gagtagttgg ttagatatga gggtgaattt tttggttggg agtatggtta 90420
   agttttgatt tgggtttttt gaaagagttt ggggaatttt tttatttgga ggtttttagg 90480
   tttggttatg tggttttgag tgattatatt gaaggggtag tggttggggt tggagttggt 90540
   gggttttgag tatagttatg aaggtatgtg tattttttgg ttgttttttt gttattgttt 90600
   tttgggtttt tattttggtg aggtgtgtat tttggtggtg ttttttaggg aattaaatat 90660
   gtttgttgta ttgtgtgtgg agatggagaa tgtataattg gttgattttg tgttaatttg 90720
   gtggaatttt atgttagttt tgggaaagaa taattgtatg ggtgtgttta tatttattag 90780
   gtgtttttta gaaaaatatt tgagaataat gttgtggttt aggatggttg ttgtgttgga 90840
   tttggtattt ttttaggggg gttgtgttgt tgggttgagt tttttaggta ttggattttt 90900
   aaatttttaa atatggtgtg gataggtggt ttagtagggg ttggattatt tgataggttt 90960
   aggtgttgga gtttagataa gatatatttt ggtttggtgt ggaagatatg gggtgttatt 91020
   aatggtagta atggttgtat ttttgaaatt tgggttttta ggttgatgag ggtgtgtatg 91080
   tatttgaaat gtttgtggtt ttgtagtttt tatgtttata aatttatttg gttgaaaata 91140
   gtttaaaata tttaaagtat gagggaggga gtgtttgttt tttttaaaaa ggaaggattt 91200
   gattttattt atttaaaaag ttatttaaat ttagaatatt ttttgtaaga gattttttgt 91260
   tttttgtttt tttagaatgg ttggagagtt ttagtatttt tgtatatttg ggatatttta 91320
   gagggggtgg ggaggggtaa gtgggtagtg agtgatttta gatttaggat gagttgttag 91380
   gtgttttttg gttatatatt taagggattg gagtgtagtt gtagtgttgt ggtttgttgt 91440
   tttgggggtg ggggtgttgt tttatgttgt gaatttttat atggtttttg attttgggta 91500
   gaggttgagg gtttaaggga tggggtgata gggagagtat gtaggagtgg gtttttggtt 91560
   ttttagggtg agtggaagaa gtgttttttt tttttgtagg tgatagattt gggggttttt 91620
   ttttgaggat gagagtttgt tgttttttaa gttttgtgtt taatttaggt ttttaggttt 91680
   attttagttt tttggttttg tttgttttgt ggatgatata gtttaagggt agagattgtt 91740
   ggtttggagg gaaggttagg ttttaggtta gggtttagaa gggagggaga agtttttggg 91800
   gtagtttttt ttttgtttat ttattgttta gttttttttt ttatattttt ttttggaaat 91860
   gtttgttttt gataaggttt attttttgtt tttaggaggt ttttattgtg gaggaaggga 91920
   ggtgttgttt gtttttggtt tttttgatag ttgtgtttta tttttgtttt gtgttttttt 91980
   ttttggatag tgtttttttt agggtttatt taggagggtg tagtggtggt ttttggggtg 92040
   gtggttgtgg tgggggtgtt agttgtaggg gtgtttttgg tgggtgggag ttggtggttt 92100
   tttgttggtg ttatgggatt tgtatgtttg ttttgtgttt tttggttttt gagtttatag 92160
   gttgggattt tgtttgttag ttgtgtgtgt tgttgtttaa tttttgtagg tgtagagtgt 92220
   gtggtggtgg tgatagagaa ttttgtttgg ttgtttaaat atagtttttt gtagaaggat 92280
   tttgtgtttg gggaagggga ggaatttttt tttttttggg tgtttgtttt ttttgttatg 92340
   gtttggtttt tatatttgtt tatatttggt tgtagtgggg tgtttggggg gaggggttga 92400
   ggttgtgttt tttgttgttt tttgggtgtg ggttaggtgg ggaggagggg ggtgttttgg 92460
   ttgtgtgttt aggattgttt tttagtggtt atttgggttt tagtttttta ggtttggttt 92520
   tgataggtgg gtggagtagt tagtgtgaga tagggaggtt ggtgtgggtg tgggaatttg 92580
   atttgtttgg gaggtggggg tggggtgggg gtgtagtgtg tggggagggg ttggtgtttg 92640
   tttttttttt ttatttattt agttgagtta gggggtttag gggttttttt ggtggttagt 92700
   tttgtattgt aggagtgtgg gtgtggtgtt ttagttagtg tgtagggttt gggttttgtt 92760
   gggggtgttt ttttgttgtt gttttttgtg tgatttgttg tttattagtt attatgttgg 92820
   attttgtggt taatgtgtag ttggatggga ttattttgga ttttgaaggt gggtgttggg 92880
   ttggttgttg tggttgtgga tgtgttggag aggattttgt gggtgggttt ggtgtgggat 92940
   gggggtgtgt tgaggggaga tgggagtgtg ttgaggggag atgggatttt taatttaggt 93000
   gtttttttgt tgagagtgtt gtgtgttttt ggttttgtgt ttgtgttgtt tatgtggggg 93060
   attttgttag gggtatttgt gtagattttg tgtgttttta taggattttg tgtttgtttt 93120
   gtgtattgtt gtttgggttt tttttttttt attgttgttt gtgtttgtta agtgatagtg 93180
   atttttttga gggtttgtga ggttgttttg gaatttttta ggatgtatag ttttattttg 93240
   ggaaatttat tggttttttt tttttggttt tttttggtgg tttttgggtt ttgtttggat 93300
   ttggtaatgg gatagggagg ttgtttttta tttttgattg agtggatagt tgtgttttgt 93360
   ttgggtggat agtttttttt ttttttatgt tagttttggg gttgttaagt tgtgtagttt 93420
   gtgggttggg agtattgaat ggatatagtt taggttgtgg tagggtttag agtgggatgt 93480
   tttatggttt ttatttaggt ttggggatat ttttatttgt tttttagaat tgggttgtgg 93540
   gggatagaag gggtttgtgt gtgggtaggg agagtatttt ggtttttttt tgtttttggg 93600
   gtttataaag tgtgttggga tttgtggggt tgttttgttt aagtttgggt ttggtgtttg 93660
   tgtttttgag tttgtgagtg tgtgtgtttt tttgtgtttt tttgattgtt ggtgttgggg 93720
   ttttgtgttt tgtgtttgtg ggagtaaata tagtaggtga aggggaagtt tatataatgg 93780
   tttttagtgt tttggggtag ggtttttgag gggtgggttt gtttttgttg ggatttggag 93840
   tttttgtttt ttggagaggt ttttaggttg atttgggtag agttttttgg tgggttggga 93900
   gggggaaagg ttgtgttgaa atgagtaaat tgtttaggtg ttaggttaag ttgggaggtg 93960
   attagtttga ggtttttttt gttttatggt tagaattagg gttgatattt gggtgttttg 94020
   agtttagttg tttatatggt ttatttgggg ttagttttat ttgagtgggg gaggtggggt 94080
   tttttggggg attagaattt tggttggatg ttaagtagag tgttagtggt tgttttttag 94140
   ggttgggttt gaggagggtg tggggtggtg aagggatggg agggggttgt gatttagtgg 94200
   ttattggtgt tgtgtagagt gtgagttgga aatattgtag ttattttgtt agtttagtgg 94260
   tgaaagtttt tttttaggtt ttattttttt gtatttttgt tttttagagg gaggggaggt 94320
   ttgggtttgt agagttggga gggtttgttg tttttgtttt ttttttttat aatatttttt 94380
   tatttggata tttttgggta tatgtttata ttggggtttt tttaggttta ttgtgttttg 94440
   ttgagttttt tgtagttttt gagtgaatgt gatttttttg tttttgtttt tttgtaattt 94500
   ttttttgtga ttgttttttt aggggttttt tttgttttaa atgtttaagt ggtatgattt 94560
   agttggtttg attatttttt agtaagtttt tatggagaga ggttttgtgt tgtgtagagt 94620
   tttttttttg tttgtgggat tgaggttttt gtttttagtt tttaatagaa agtgttgggt 94680
   ttttagtggg atttttgggg aagaattttt gtgttttaat gggagttttg tggtgggagg 94740
   ggaggttagg gtttggggtt gtgtttgttg tatagttgtt attatttgta ttatgaaagt 94800
   tgttagtgtt ttttttttgg gtttttgggt gtaattttat ttttgttttt atgtgttttt 94860
   atttggagtt gtttttgtgg ttgtttttta agttagtttt gtgattttgt aatttagttt 94920
   aagataatgg gtttattgag attattttgg tgtagtagtt ggtaattttt tggttttggg 94980
   ggaaggtttt ttagttttgg ggagtggggt tttaatttgt tggttttttg tgtttattag 95040
   tttttttttt gtgtgttttg aatggttttg ttgggaattt tggttttaga gttattaggt 95100
   ggtttgagtt gataggtgtg agagagtgtg tgtgtgtatg agtgtgtatg tgtatggggg 95160
   ttgatttggg gtatggaaag gtggtttttt ttggtgttta aggagtttgg agtatagttg 95220
   gagggtgtgg gggtgtgtat atgggagttg gataattttg ggtggataga tagatgtggg 95280
   gaagggatga ttgaaggagg tggaggagag agtgtgattt agtttagtta ggggtgatgt 95340
   ggataggtag tttttgaatt agggtagaga aaagttatta ttagttagta ggggagaagt 95400
   tagtatggag gaggtggatt ttgagggaga gtaggaattg gattgtaaga ggaaggagag 95460
   ttttttggtt agtagtagtt agtagtagtg ggggaggttg gaatgagttg gttggagagg 95520
   gggttggggt ataaggaggg gtttgtttgt gaagattata tgggttaggt tgtggagggt 95580
   taggtatgtt tgttgggagt gtagttggtt tatgggaagt atttggagtg gttgggaatg 95640
   ggtgtaggag tagtgttgtg ggagtatagg tttttttttt ggggtggttt atttggtgtt 95700
   ttggtttttg taaggtaggt tgaaagggtg gggaggaaat tgttagtttt ttatagtgtt 95760
   gggatggtgg ttttagggtt tttgaggtta gtggatgtgg gtgtttgtta ttatgtgggt 95820
   tgttgagggg tggagatttt aggggttatt ttaaagtagg atgagttttg agttatggta 95880
   tttttggggg tagtttttta attgagtaga tgtttaggtt tggaattttg taatagaggt 95940
   tatagggttt tgattagggt gttttgggag gtttagaatt agtggtagta tatagggtag 96000
   atggtaagtg atttggtatg gggaaagagg taggtgttta ggttggtata gtatatttgt 96060
   aaggaatagg tagatgggaa gttgtttgtg ggtttgtgtg tgtgtttgga gttaaaattt 96120
   tgttaatgtt ttatgttttg ggtatattta tttttttttt ggggagtatt ttttttttta 96180
   tttttttttt ttttgtttgt tttttttatt tagggttttt tttatttttt ttgttttggg 96240
   gattgagggt attatggttt tatgttttat tattgatgag ttgtataggg atttagtttt 96300
   tttgttgttt aggttgggtt ttttaggttt agggttttta ggaatggaga gggtattagt 96360
   gttttttatg gatttaaatt ttttgtattt tgtttttgtt ttttttttaa gataggtttt 96420
   tgagttttaa ggttttaggg ttttgtggag gttgttatgt agtagtaagg agaatgtttt 96480
   gtatttggtt gatgagattt ttagagtttt attttttatt tttttattgt ataaatgggt 96540
   ttttaggtga ttgtagtatt tgttattgtt tgtaataggg tgataagagg gatgattttt 96600
   tttttttttt tttttttggt tggtggaggt atggggttgg tggatggtat gtgtttttgt 96660
   gaatttaggt taaatttgtt attgtaaata tgattataat ttgggttttt gtgtaataaa 96720
   agttttttta agtattagtt gttggtttgt tttgtttagt ggtgtttgtt gtaattagat 96780
   ttgtatattg agaaagaatt taaaagtttt tgatgtttgt tgaaataatt tggtttagga 96840
   tttatgtgtt tagattttag agttgtgtgg tatttgagtt ttttttgagt ttttattgtt 96900
   gtttgaggag gatttttaga tttgtgtttt ggaggtagag taggttgtgg gatgggtttt 96960
   tgggtgggaa ggattatgtg gatatgtttt tttgtttgag agttttaata tttttgggat 97020
   gtgggagttg gtgtgttggt aggatttagg tgtttttttt ttttttagag aaaaaggttt 97080
   tgttgtttgg taataggtgt agatttgttt ttaattaatg ttagtaggtt ttttgtgtga 97140
   tgaattttgt tttttagtta agatttaagg tattttgtga atattgtttt tttgtagttt 97200
   gagtttttgt ggtgggaggt aggagttatg gggagtgggg gtaggttttt tatatgggtt 97260
   ttatagttat tggtagtatt gatttgatgt tttttgagtt tagagtttag ggttagatag 97320
   atttattgtt ttgattatga gttggtttat ttagaggggg gtggatatag tatttaggta 97380
   gtagatgtat tgtgattagt tttgtagtgg ggttgtgggt tttttgggtt ggatgtttgg 97440
   gaagaggtag gtggaggtaa atgttaggat atttttgtag tgattgggtg attgtaggtt 97500
   ggaaatgttt tttgtgggtt gtggttgttt aggaaggttt tgaatggggt tagtggatag 97560
   agtttgtatt tagaggggta gtgttttgga ggagtgaggg gtatggtagt gtagggatgt 97620
   ttaggttgtt tttattttgt tattggaaag ttgggtggtt ttggtttttt tagttttttt 97680
   gtttgttttt ttgtttgtaa agtggggtta gaaatagttt tttttgaggg ttgttggggg 97740
   attttgagat gtagtttatg gtgttgagta tgggttttgt tttttatggg tgtggtgggt 97800
   gttgtggttg gtgtggtatt tgggtgggaa aagggggtat ttgtaaagga taggtaggtt 97860
   tggatgttta aatatgtaga tttggggatg ggaggtttta ggtaagggtt tgtgtgatgt 97920
   tattgtatga atgaggttgg atagtatggt tattattaat tatggaaagt atggtagata 97980
   ttagtgttag tagtgtttgt ttgggggttt ttttgagttt gataggtatt taggttttta 98040
   gtgtagttat gggatttttt tttttttgtt tggttgatat tttggaggtt ggtgttatgt 98100
   tggttgtagg ttttttttat tgggaggttg ttttggtttt ttttgttatt atttattttt 98160
   atttttattt tggagtgtta tagatgggtg gattatatgt ttatttgtag ggttaagtgt 98220
   tgataatgag gtgagtattt gttggatgat tggattatga ggatagtggg ggtgttggta 98280
   gtttgtgttt ggttttgtag gttggttttt ttttgaaaag tggttgtggt agagtgtgta 98340
   ttttagggag tggtgtttgt tgttttgaat atttttattt agtaggtttt tgtaagggtt 98400
   tagttaagtg tggtgtagag ttgggttggt agttggaata gttttatttt gtttagtggt 98460
   gagattaagg gttgatgggt taaggttgtg tgttgtttgt ttagtttgtg gatttattta 98520
   ttgagtattt ttatattttt atattgggtt tggggtgtta ggaggtggag gggggattgg 98580
   gtgtttggaa aaataagttt attgagagtt tgagttttta ggggttagta ttaggtagtt 98640
   attatggtgt ttggtatata gtggttattt ggttttgttg gttgttttgg tggtggagag 98700
   tgtgtatggt ggttgtatgt gatgggtatt atttagggta atatagatat gtagattgtg 98760
   tatttgtttg taggagttag ggatttttta gtttggtaat ttgtgatttt tgtttatttg 98820
   ttgggagaga ggtgttgttt tttgatagtt tggtgttggg agagtagaat tagggttttt 98880
   tttttttttt tttatttttt attttttata tgtttaggta ttaggggagg ttatatttag 98940
   ggatgagttt tggttattgg gtttagggtt ttattgttgt tatttttttt ttttgttagt 99000
   tagttaaatg tttgtttatt attgtgttgg ggatggggtt ggtttgtttt tttgaagttt 99060
   ttttggtgta tagtttaggt tggagttttg gatgattttg ttattttttt ttttgatttt 99120
   ttttttttat tttgatttat tttagaaatt tagttaaaaa ttaagttttt gtgttttttt 99180
   ttgataaagt tattgaggaa aaatggttaa atattgtttt tttttttaaa gtttttttgg 99240
   ttttttttaa atttggaggt tttgagttgt ttatgagatt aaagggtttg gaattttttg 99300
   gaatatagtt ttagaagtgg gaggatttgt tattttttta gttaaataga tttattagtt 99360
   ggtttttaga tatttttagg tgtttggggt atttttgggt ttaggttgtt taatttggtt 99420
   tgtagagttt tgaggttttg tttagattgg gttagtagat agttttagag attttaagtt 99480
   tgttgttgtt tttaaaagtt ttgttaatgt ttagaaattt agagggataa gaagatgatt 99540
   ttgatgttag ttgggattat aaatagtgag gtttgttttt ttttttattt atgtttgttt 99600
   ttattgtttt tgtagttggg tttttagggg attgtttgtg gttatggggg aggttgggaa 99660
   tgtggggttt agattttatt gggttttttg tatttttttg tttttttttg tttatttttt 99720
   tttttggaaa tttttgaata tttttttata gtttttggta ttttgttatt tttggtattt 99780
   ttgggttaat tgggaaagtt attatttgtt taatatttag aaaattaagg ggtttggttt 99840
   taaatatatt gtagtgtttt tttttttata tggtattgaa ttttttgtgt ttttaaagtt 99900
   aggagtttgt gttttggatg tgggtgatgg agttttgtag atatttggag tggttttttt 99960
   gggagagttt tttttgtttt tttatttatt tgtttttttt tttttgtttt aagttttggt 100020
   tatagttttg gaggggttat ttgagtgtag gttgttttta ggtgtatagt tggagggggg 100080
   tagttatttt atattttttt gtttgttagg ggtattttta gggatttagg ttttgaggga 100140
   tagtggaagg gtggggtggg gagtaggttg ttgttttgtg ttatttgggt gttatgtaga 100200
   ttttgaatgg gtagtttttg ggagttggtt tttttttttt atttttttta tagtgttgtt 100260
   tttttgaggg gttggagttt tttggagttt tttgtagttt gtggtaggtt tttttgattt 100320
   tgtgtttggt tttgtagttt ttaatttata gttagtggta tgaagaatat ttttggttgt 100380
   ttttatttgt ggtttttgta ggtagtagtt ttttggggta gggtttgggg gttttggtat 100440
   ttttttgtgg gattttttgg gtatgaggga gggagttatt tgggttatag atagattttt 100500
   tttttgtatt taggaggttt aggatggatt tgttttattt ttttttttgg tgggaagaga 100560
   gtatttttgg ttatggggaa tgtggtttgg atagagttat ttagtttttt ttttttttta 100620
   ggtagggttt tgaaagatgg agatggtttt gtttttgtta ggttaatagt tagtagtttg 100680
   gtggattttt atgtagttta ggtaggattt ttttttgttt ttatttttta ttttatgtgt 100740
   aggaagttgg ttttaagtag ggatttttgg ttttgttttt tatgtgggtg ggtagttttt 100800
   ttgtgagggg taggttaggt ttttgtttta tgttttttag agattgatgt tatttatagt 100860
   aagagtatat aagttttttt ggttttaaat taggtttgga ggggtggttt ttttttttgt 100920
   tttttgtttt ttgtttttta ttaaaggtta gggaatggag tgttttgtat gtttgttggg 100980
   gttgggtttt tttttttggt ttaaaatgaa gggttggaat ttttggtgta gattgtatgg 101040
   tgagaggtgt tttttgggat ttatttgagg agtattattg ttgaatgttt tgttaggtta 101100
   ggagtattgt tgggggtggg agaggtagtt ggtttatttg tttatttaat agttatttat 101160
   gggtatttta agggttagtt ttgttttagg tattgggggt ttaggtatgg gtttaggaga 101220
   aggggtagaa tgggagggtt ttttggagga aggtttttta attagagatt ggggtaggag 101280
   tttgttaggt aggtgatgtt ggttagtttt ttttgttatt tttttttttt tttttttttt 101340
   tttttttttt tttttttttt gagatagagt tttgtttttg ttgtttaggt tgtagtgtag 101400
   tggtgtgatt ttggtttatt gtattttttg ttttttgggt ttaagtgatt tttttgtttt 101460
   agttttttga gtagttgaga ttttaggtat gtattattat gtttagttaa ttttttgtat 101520
   ttttagtaga gataaggttt tattttgttg gttaggttgg ttttaaattt tttattttag 101580
   gtgatttgtt tattttggtt ttttaaagtg ttgggattat aggtgtgagt tattgtgttt 101640
   ggtttttttt tgttattttt gtagatttat agtgtgtgtg gaagattgta tgtttgtgtt 101700
   tggtatagtt gtttttggta tttaggtggg aggtggggga gagggttgtt ggtgttagta 101760
   taggttatgg tatgagatag gtttttgtag ttaagtgggg tgtattgtag tattggaggg 101820
   gttttgttgt gtatgagatt taggttttgt atttgttttt ttttttagga ggtttggagg 101880
   gttgggaggg attagggtgg tgaaataatt taggtgggag agaggtattg ttggagaggt 101940
   agagggagtt gtaggtttgt agtagtgagt tttaaatttt gggtttttat tagaaaattt 102000
   tagaaagggt tgttttgttt ttttttttaa attataaaag aaatttttgg ttgtttgggt 102060
   tttttggggt tgggtggggg aggagatttt ttatttgtat ttttgtaggt ttagtattgt 102120
   gtagagggag gagggtggag gtaaaggtga gggaggaggg tggaagtaaa ggtggggttt 102180
   ttttttagtt ttgttgtttt gtttggttgt tgtttttaga tttttatttt tggtttggaa 102240
   atatggaatt ttgggatagt tggggtaggg ggtatagaat ggagattttg gtgagggagg 102300
   aatttttttt ttagttttgg ttgtggttat agtaaggtta ggttgggagg attttattta 102360
   aatatttata ttttttttaa tatttaaatg atgaagtagg tttttgtata tagttgtttt 102420
   ggagttagtt gttgtttgtt ttattttttt tttttttttt tttttttttt tttttttttt 102480
   tttttttttt tttttttttt tttttttttt ttttttttgt attttttgtt tttgatttaa 102540
   gtttaggttt ttttagttgt ttttttttta tttataggta ttttttgtga ttgagttttt 102600
   ttttgttatt ttttataatt atttttttta attgagtatt ttttaggtgt taggttttgg 102660
   gttggtattt tgtatataag gtgtttttta atttttatga gagtttaata aggtagattg 102720
   ggttgttgag gttttagtgt tggaaggttg gtttgggttt ttttagtttg tatttgtatt 102780
   tatttattat ttttttattg tggagatttg ttttttggtt tttggttgag tatgatggaa 102840
   atttttgttt attagttttt ggagagtggg gatggttgag ggttttgatt ttggggtttg 102900
   atgggggttg tttttgtttt attttagttt ggttgttttt ttatttaatg attgagtttg 102960
   taaaattaga ataatagtgt tatttggttg gtattttggg tggttgggag gtgtgggaga 103020
   gggtgtatgg aggggttgag tgaggtgtta gtattgtggg gatttttgag ggtagttttt 103080
   ttaggtttag ggtggagtgt aggtagaggg tgtgatggtt tagtgtgttg gtttatattt 103140
   ggttttgttt ttgtttatat ggtaatgtgg gggagttatt agttttttag tgttttagtt 103200
   ttttatttgt gtaatgggga taagagtaaa ttattgtttt taggtatggt aagtatttat 103260
   atatattgat tattgtagaa tgaatatagt gagttttttt taggttttta ttatatgtta 103320
   ggtagggtgt taggttttag tggagagggg ataatgatgg gtaagttggt ttttgttttt 103380
   aagggttgtt gagtagggag gaaggtggtg gttgttttga ttgtgatttg gagtggggtg 103440
   atttgtaaag aggttggtgg gaggttggtt gggggttggt tgggggttgt tttttttttt 103500
   gtttgtgttt tgtggtaggg tattaaattg gtttttttta tggatttttg aggttttgat 103560
   tgtgggattt tgggtaggtt ttgggatttt gtgattttgt ttttgtttgt tatttaggtt 103620
   tttgttttta gtagtgttta gtatatggtg atttttgtat gttttttagt gtgtgattga 103680
   ttttggagtt gagatttggt agttgaggtt ggatattgtt tttggataat ttttggggag 103740
   gtgggggtag ggtggggttg tgagttggta attttgagtt tttagtagtt tttgaatgga 103800
   ggtttttttt ttgtaggatt tttggggagg aaggagggga tgggggagat gaggtttgtg 103860
   agtttttagg agggtagagg tttatatttg tagtgtttta gaaatatttt ggggtgttta 103920
   ggtttattta gtttttttga gttagaatta ttaagagttg gggattagta gtttattttt 103980
   tagtaagatt tagaaatgat ttttttgaat agtttgagtt aagaatgata tatttggaat 104040
   tagaatatta gaattataga ggaattgaga gaatgggttt aaaatgtgta gggattttag 104100
   ttatatggtg ttatatggag gaaagtattt aatattatta ttgtatgaat ttgtttaaat 104160
   atatatatat atatatatat atatatatat atatatatat atatttttta gtaaagagtg 104220
   gaaagatatt tgttagaata ttgatggaag ttatttgggt tgtgggttta tagatttttt 104280
   ttttttgttt ttgtgtattt tgtaaatttt tggttttgtg tttaaatttt ttttatagtt 104340
   aggaataaag taatagatgt tattttttaa agtttatggg tttgtgtttg taattttaat 104400
   attttgggag gttgaggtag gaggattttt taagtttagg agtttgagat tagtttgggt 104460
   aatataggga gatttttatt tttttttttt tttttttttg atagagtttt attgtgtttt 104520
   aggttgaagt ggtggtggta tgtttttggt ttattgtaat ttttgttttt taggtttagg 104580
   tgatttttat gttttagttt tttgagtagt tggattatag atatttatat ttgtaggatt 104640
   tgagaatttt ataagaggaa agtttttatt tggaggttgt tggggatttg gagttgttag 104700
   tttatatatt ttttagtaga gatagggttt tattatgttg attaggttgg ttttaaattt 104760
   ttgtttttaa gtgatttatt tattttggtt ttttaaggtt ttaggattat aggtgtgagt 104820
   tattgtgttt ggttatggga gatttttttt taaaataaga ataaaaataa aaataaataa 104880
   aagtttattg ttaaaaagaa aaatatatta tttaatttaa ttttttttta ggattgagag 104940
   atgaggaagt agaggttgga aagtgttata ttttttggtt gttggtagtt aggggatttg 105000
   aggtttgggt tggttttttt gtgttttagg tttggggtgt taggtggagt ttttgttttg 105060
   tttaaaggtt taggtgtttt tgttagtatt gtttgttttt gtatttttgg ttataaggaa 105120
   tgtgggttaa aggtagggtt tttgattttt gtttaggagg gtgaagtggg ttggtttttt 105180
   ttgtttttgt tttaaggttt ttgtggttgt gtttttggaa attgttggtt tgagttagtg 105240
   tgtggagttg ggatagattt tattgtggtt tttgtggggt tattttatgt ttgggttgga 105300
   agggattttt tggtttagtt ttttttgttt tttattagga aattgagatt agagaggtta 105360
   tttgtttggt ttggggtggg gtgtagatgg tgtttgtgat ttttgtatgt tatgtgtaga 105420
   tagtttttta gttgtgggtt ttttatgtaa gtagttatgt tggggataat ttggttgaaa 105480
   tttgtgattt aatgaagaga agaatgattt tttttgtagt tttgtgtttt ataggttttt 105540
   ttttgttttt attttttttg tagaggggtt ttatgttggt gggtgggttt ttgttgttgt 105600
   aatgggttta ggtagttttt gggtagtgga ggtttggttg tttttttgtt taatgttttt 105660
   tgtagtttat gttgtttggg aagggtgggg aagtatttag ttattgggga ttttataaga 105720
   tgggtatttg ggagatgttg attggtaggt taggtttttg tggtttatag gtaggttaga 105780
   ggttatagtg ttgagttggg ttgagttggt agtttagggt agtgggggtg ggggtatttt 105840
   gtttagttat gggttttttt ggtataaggg tttggagttt attgtaaata atttttttag 105900
   tttttttttt ttttataggt tattagtaga attgaggggt tttttgttga tgaggtgtgg 105960
   tttgggaggg gttagttgtt tgtttatttg atttttgtgg ataggtgatt ataggttttt 106020
   tgttttaggg ggttggggga aagattgggg aggttttttt ggttttttta tttggaagga 106080
   aaatatgggt ataggggaga tattttatag ggatatattt tagttagtat tttttggttg 106140
   tgttgggggt atagtgtttg ttttgtttat aagaggggtt agtttttttt aggatttaat 106200
   tttagttttt agtgtagggt agatgagtat ttagaggttt agaggattta tattatgaga 106260
   gagaagttgt gattatatag gtattgaagg ttaagtagaa gagtgggata tatgggtgat 106320
   ttgaaagttt tagggttagt gtggttagtt ttagatagtt ggggagtttt gggtattagg 106380
   tttagtaggg gaggagggtt tttgtgatag tttgttgggg ttgttgtaat aaagttttat 106440
   gaattttggg ggttgaagat ggttttagag gttagaagtt taaaattaag gtgttgatta 106500
   ggttgatttt ttggggtttt tgtgggaggt tttggtttag gttttttttt tagtttttgg 106560
   tggtttttgg gtgtttttgg gtatgtagat gtgttatgtt attttttgtt ttgtttttat 106620
   atggtttttt ttttgtgttt ttgtgttttg taaggatgtt tgttattgta tttagagttt 106680
   attttaaatt taggatattt tttttttgag attttttatt gtatttgtaa agattttatt 106740
   ttaatttttg ttatatttat aggtttgagg gttagggttt tggtatgttt tttgggggtt 106800
   attatttagt ttattgtggt agttgatttt aggggaagta gaatgtttta agtttggggt 106860
   gagtggaggg aatgttgttg ggtggggagt gggagattag gtttggagat attgtggatt 106920
   ttttttgttt gatgagggtg tgatgaggtt tttattttag gttttttgaa tggtggttgt 106980
   ttttgaggag attttttttt ttggatttgt ttttttagaa atttttattt aggtttaggt 107040
   tttaaggtgg atggtaagag gtggttgttt ttgttatatt tgatgggtga gtgtgtgaat 107100
   ggggttggga gtaggatatt tgttattttt gtggatagat tttagaggtt tatgaatttg 107160
   ttgttgggga aggtgtttga tttagtttga ttttttattt ttattttttt ttttttgttt 107220
   ttttttatta gttttgtttt tagagtttta tttttttaaa ggtaggtgtt gttttttgga 107280
   ttttttagta gttatttttt attttagatt ttttttttgt aggttttttt ggttttagtg 107340
   tttatttttt tttgggtttt tgttttttta ttgtgaggtt tttgggtaga agtgtggggt 107400
   ttttttgttg tgtttggttt ttgtggtata ttaggggttt ttttaggggt ttgttttgtt 107460
   tgttaggttt ttagggagtt ttttaagttt ttagatgggt ttgatggagt tttttaatgt 107520
   gttgttttag agtaggaggg gttttggttg agtttgagag tttttttagt tatattttgg 107580
   gttgtttggg gtagttaggt agtttgtttt ttggaggtgt tgtttttttt tggttatttt 107640
   ttttggaatt ttttgtgttt agttgtggtt ttttttatat agtatattta ttattttttt 107700
   ttatttgtga tttttttttg ttaatttttt tatgtttttg agaatggaga ttaggttttt 107760
   tttattttgg agtttagtag aatttaaatt aggtagattt gtgtaggttt atgtgggggt 107820
   tttgtggtta ttgtggtttt gttgttattt ttagtttagt ttggtgtgtt ttggattatg 107880
   ttatttagtg ttggtttttt aaattttttt tttggttttg taggtttggg aagtagttgg 107940
   tttttgggtg tagttgtgat tggagtttga attttgtttt ttggggttat tgttttttgt 108000
   aattaggttg tagtttatat tggtttgggt agaggttatt ggttgtttgt tttggggtta 108060
   taatatagag agttttgtga ttaatggagg ttttatttgg gttaagtggt ttttggtgtt 108120
   aggtgttttt tttttttttt tttttttttt gtatttttta agtgtttgta atattttttt 108180
   taatggataa tgagatgagg agggagggag gggtggtttt tttgtttagg tagattagga 108240
   atggttattg tttgtgtgtg tagtgatagg tttgggaata gataagggag agtttgtttg 108300
   ggaggttgga tttttatatt gatgttgttt gtgtttggta gttttgtttt gggttttaga 108360
   atagatattg attaatatgt tttttagggt ttttaggttt gggttttggt ttttggtaga 108420
   gttttttatt tatgggaaga agtattgagt agttttgttg gttttttgta gggagttggg 108480
   atgtattttt aagagtagta tgtgtgaagt ttggggtttg ggtaaaggag tgtttgggga 108540
   agagtttgtt gttgttggag ggttggaagt agaggggaag gaggggtatg tggtaggttg 108600
   aggaggtggt tggtgatttt gtggtgggga tgagggggtt atgagtagat taggagttag 108660
   tattatgttt ttttgttttt agaattgagg atggagagtt gttagagagg ttttggtggg 108720
   gtgtttgggg ttatttaaag ggtgtgggtg gattaaagtt gaggaaagtt ttattttggg 108780
   ggattttgta tttaattttt gaggttttta gagttgtagt ttgtggtttt ttgaagttaa 108840
   ttatttttta gggttttgtt tttgttttta gtgatagtta tttattaggg agtttttgat 108900
   ttaaggagat ttgaaaggtt atttggtttt ttttttattt ttaggtaggt tatatttaga 108960
   tttttttata tagatttgtt ttattttttt gttgtttttt attagggatg gagggtgggt 109020
   ggttttttta tttaggtttt ggtggtttta ttttagttag gaaatttttt ttttttattt 109080
   gttttatatt tttttttttg tggtttttgt ttattttttt ttaatttgtt tttagtggag 109140
   tagttagtag ttggtttaga atttgggggt ggggggagtg ttagattagt gtagtatatt 109200
   tgagagtgtg ttagaggtgt ttttattttt atagttgatt tagtaaagaa aagaggaagt 109260
   ttagtaattt agtgttattt taaagtttgt ttggaatgat tttgtggttg ttggaattag 109320
   gggtatgtgg tagatgatgg gagtaggtat agagaggttt ttgttttgga ttagttgttg 109380
   ttgttttttt ggaggttggg ttgtttttta agaagaggat ttaaggtttt gtgagtggtg 109440
   gttttttttg gagtttttat tgtggttttg gttttgtttt gtgtaaagag agatggtttg 109500
   aagtagaagt tgttatgtgg ttgttttagg taggtgtttg tagttttagt tatttgggag 109560
   gtggaggtag gagaatggta tgaatttggg aggtggagtt tgtagtgagt agagatggta 109620
   ttattgtatt gtagtttggg tgatagagta agattttgtt ttaaaaaaaa aaaaaaaaaa 109680
   aaggagtaga gaaatttttt ttagaaggtt tgtagttatt ttttttttat gttttagagg 109740
   taaaaaaaaa aaaaattgta tattttgttt tttttttttt tttttgagat ggagttttgt 109800
   tttgttgttt aggttggagt gtagtggtgt gattttggtt tgttgtaagt tttgtttttt 109860
   gggtttatgt tatttttttg ttttagtttt ttgagtagtt gggattatag gtgtttgtta 109920
   ttatgtttgg ttaatttttt gtgtttttag tagagatggg gttttattgt gttagttagg 109980
   atggttttga ttttttgatt ttgtgatttg tttgtttttg ttttttaaag tgttgggatt 110040
   ataggtgtga gttgttgtgt ttggttatat gtttttgttt ttgaataggt ggttttttga 110100
   ttttgggtgt ggttatttta agattttgtt ttggtggggt ataattttgg ttttaggtag 110160
   ggtgattgta taatttattg ttttttgggg gatatgttag tgaaagggat ttttgaatga 110220
   ttatggtggg ataatagatg tgagttgggt ttgttttagg aaggttggat gtatgggttt 110280
   tttgagttag aggagataat agtagaaagt tttgtggttg atgggattga gtaggagaag 110340
   ggttggtgga ggttggggta ataatgtatt tgtttattgt gaagggtttg tgtgagttga 110400
   tggttgatga gagggtagtg gggtgaggtt gttttttgtt aggttagatt agttttagga 110460
   tatttatatt ttttttttag aattggtgat ttagtggtta gaaggggttt tagaattgat 110520
   gggatattga gggagttgaa tggaatggtt ggagtggtta tagtaggttt taggtttttt 110580
   tttgtttttt ttttgtgttt tgtttttggg gtttatattt tggggttgtt gtaatagagt 110640
   gttataattt gggggtttat aaatatagtt tttggtggtt tttggagatt tttggtgttt 110700
   tttggtttgt agatgtatta ttattatttt tgatttttgg tttttgtttt tatgtggttt 110760
   tttttttttg tttgtgtgtt tttttttttt tttgtttttt ataaagatgt ttattattgg 110820
   atttagggtt tattaaattt agaatgattt tattttgaga tttttatttt aattatattt 110880
   gtaaagattt gttttttaaa tgtgattatg tttgtaggtt ttaggagtta ggatttaaga 110940
   tatattgttt tgggggatat tgttgaattt attatattta ataaatttag agttggtgtt 111000
   aatgtttttt gtggatatta tagtagattt tggaggtttt ttgtaaagga atttgtagga 111060
   tttttttttt tttttggagg gatgagattg tagttgggtt ggggaaagtt gttggtttaa 111120
   gttggggttg gtttagttgg tgttggtttg ttaggttgtt agagagtttt atttagggag 111180
   aaattaatat tataaagttg ggggataaga gaaggtttgg aattttaagg gttggttgtt 111240
   gaggttagtt gtttattttt ttgtgtttta ttttataggg atttaggttt gttagtgttt 111300
   tgtttttgga aggaatgttg tgggttgttg tgttttatag gtagggtgga gggttttttt 111360
   gtagttagtg agttgttttt tttgtagtgt gtagagggaa ggaggaggtt tgtttatttt 111420
   ttttggtttt agttgaaaat ttaattatag gttttggtat taggtagatt tgtttatttt 111480
   tatattatta ttgtagagtg agttaggttt tttgggtggg gttgtgatga gtaggaagga 111540
   tttttggagt gtttgggatt ttttgggttt agggttgtta gaggtagaga gaaagaagtt 111600
   tttttttaat atttgagtaa agatagttat tagaatttat tagagtggtt ttttgtggtt 111660
   tttttttggg tgttgatttt tttggtgtat ttgaagtttt tagggatatt attgatgttt 111720
   tttgtatttt tatttagggg ttagtggaaa tggttttttt tttttttgtt tagaggtatt 111780
   gtggatgatg attttaggtt ttagatttta gttattgggt tatgtgtgta gataagtttg 111840
   ggtagttagt aatttttggt tattaagttg gttttttgtg gattgggttg gtattggagg 111900
   tgggttttag ttgttagtat tgtggtttag ttaggtttgg ttttgggaag gtagaggggg 111960
   aggggattgg gaaagggatt gagttggggg tggggtggta ttttggtttt ttttttttag 112020
   atttttggta ggtttgaatt atttggttgt ggttttttaa ggagggagtg ttttttttgt 112080
   tttgatgtgg attttgtttt ttgtttatgt tgtttttgtt tttaaataaa tgtggttttt 112140
   tgtggggagt ttgggggttg ttttttattg atattgttgt tttttggttt ttttttgtag 112200
   gttttgttta gttttattgt gtttattttt aagttttttt ttttggatgg ttttgttttt 112260
   tgagaatata ttttttttta ttattttaat tgtgtttttt tttttatttg gagtttgttt 112320
   tatttgtttt aggagttatt tttttttttt attaagtagt ttaggttaat tttggagggt 112380
   taatttgtgt ttttttagaa gtaggttttg agatagggat tttagtggga ggggatatta 112440
   ggggatgttt gagtgtggga atgggttagg tagggaattt agttaataaa gggtgtttta 112500
   ttagtttatt gtgattatag gtggttgttt agtttttata ttgggtagta tggggagatt 112560
   gtagattgtg tttttagttt tgttttattt ggggttgggt aagggagttg gggtttttat 112620
   attttagttt ttggttaagg attgtattgt ttattttttt tagttttttg agggtttggg 112680
   tattaggaaa ggttttgggt ggagatatgg aaataggtta tgtgaagtta gtgggtgtga 112740
   ttggtagtag taaggttagg atagttttgg ttattagtaa ggtgattata tattttagta 112800
   tgtatagttt ttatagatat atatttagga gatggtaatt atattagtaa tagttatttt 112860
   tgttgtgtgt tatgtttgta tttagggaat tttatttttt atttatttat ttatttttat 112920
   ttgagatgga gttgtgtttt tgttgtttag gatagagtgt aatggtataa ttttggttta 112980
   ttataatttt tgttttttgg gtttaagtta ttttgtttta gttttttgag tagttgggat 113040
   tataggtatg tattattatg tttgattatt tttgtatttt tagtagagat ggggtttttt 113100
   tattttggtt aggttggttt tgaatttttg attttaggtg atttatttat tttagttttt 113160
   taaagtgtta ggattatagg tatgagttat tgtgtttggt tttaggggta ttttattatt 113220
   ttattatttt ttatagtagg tgatattgag gatattgagg tttagagtag attgtggtta 113280
   tgttattttt ttgtttggtt tttttgtttt tagttttgaa ataaagagta tggtttttgt 113340
   gtaggtttat atgttttgtt tttagttttg gagtagtttt taagtttttt gaaggtatat 113400
   taggtttttg ggttttgttt tttaagtttt ggataaatag gaggtgaagg agtttttgta 113460
   attggatttt attttgttag tgttaaggtt ttgtttgatt atttatttta ttttatatag 113520
   attagggatt tgtgttaagg tttagtgtta taggagagtg aagataagga ggtgtttatg 113580
   gtgtatatgt tgttggattt attttgtaga ttttaagggt tatggggagt tatttgttag 113640
   gtgaggtttt tttgtggttt ttttttgtag gtttagggta gtatataggg aggtgttttt 113700
   ttattttttg gtattatttg tttggttttt ttgagttttg attttggttt ttagatagtt 113760
   agtgtttttt tagggataga atttgttttg ttgttgttta gaggagattt agtttgatta 113820
   gggttagtgt ttttggaatt tgagtttgga attttatttt gttttgggta gaggttatgt 113880
   tgtttttttt tatgagagtt gagttgtgta tgagattatt ttgattttgg gttttattat 113940
   ttttttgtat gatatttgtt ttaggttgga gtagggtttt gtggtttgtt tggtgttttt 114000
   tttttagatt gaggtttgat atgtatatta ggaagtgttt gatttggttt taggagttgg 114060
   gagtggtttg ttaggtagtt tgtggttaga gtaggggttt taataatggt tttttgtgtg 114120
   gtatattttt ttttaaatag gtttaggttt aggttgtttt tgttttgatt ttgttatggg 114180
   tttggagggg aaagtttgta tttggagggt ggtttattgt ggtttttttt tgtgtagtag 114240
   ttgagattgt aggtgtttga gaataggaga agttttgtgt ttattttggg ttgtgagatg 114300
   tttttttaga ggtggggatg gggatgggtt agtatgtagt tatttatatt ttttagtttg 114360
   ttggtattaa ggtttgtgta tgtgtgtgtt tttttttttt taattttgtt tttttaatat 114420
   ggagttttgt tatttgttta ggttggtttt ggatttttgg tttaagtgat tttttagttt 114480
   taggttttta aagtgttggg attataggtg tgagttatta tgtttagtta gtattaaggt 114540
   gtgtttttta agagtaatat tggttgggtg tagtggttta tgtttgtaat tgtagtattt 114600
   tgggaggttg aggtaggtgg attatgaggt tagtagattg agattatttt ggttaatatg 114660
   gtgaaattgt gtttttatta aaaatataaa aaaattagtt agatgtggtg gttgggtatt 114720
   tgtagtttta gttatttggg aggttgaggt aggagaatgg tgtgaatttg ggaggtggag 114780
   tttgtggtga gttgagattg tgttattgta ttttagtttg ggtgatagag tgagattgtt 114840
   ttaaaaaaaa aaaaaaaaaa gaataatatt gatggtttta ttttttgaat atgttaagtg 114900
   atttttatgt agtataagta ttttagaagg taggaaaatg taatgaataa tatttattgt 114960
   tatttatttt atttagagat tgttattttt tgtttgggtg tgagtgtgtg tgtgtgtgta 115020
   aattgtatat gtgtgtatga aatggaatta ggatttttgt atatattttg tgttttgtta 115080
   tttttattta atatttttgg tattttttat ttttaaaaat gtgattttaa tgatggtgta 115140
   ggtttttgat atatgggttt agaaaatttg tggaaatttg gattgttttt aggttttgtt 115200
   agtttaatgt tgatgtaaat gtttttgtat gtaaattttt ttgtattttt gattattttt 115260
   ttgggataaa tttttagaag tgggttaaag tatatgaatg gtttttaggt ttttggagta 115320
   agttgagatt ggtggggatt ggagaaggga tttagggagg tttttgggag tttgtgagat 115380
   tggagtttgg ttttagggga tttgaattta tagatgaagg agatggttgg taaaggtggg 115440
   aattttttgt tttatgggtg ggggggtgat attgttttat tttttagtga tagtatgggg 115500
   atttttttaa tatgatttgg ttttgtattt ttttgatatt gtgtatggtt tatagttgtg 115560
   atgtttagtt gggagttttt tttgtgtttt ttagttgtat taattttttg tagatagagg 115620
   aggaagttgt ttggtgtttt tttttgttgt gtgggttttt tgaatttgtt tagttgggtt 115680
   tgtggatttg tgtagttatg tatggggttt taaaatttgt aaaatgataa gtttatgaat 115740
   atttttgttt ttgtgggtga atgtggatat tttttggttg tgtttttttt tatagggaag 115800
   atggagatgt gtaaagtgat aaggatttgt tatttttggg taggttatgt tggttttatt 115860
   gtgaggatgg tggtaggggg ttttttagga ggtttgtata ttgggaagag aggggtgatt 115920
   gtgtgtgatt gtaggattta gggtgtagtt ttaggatttt gatgggggag gagatgttgg 115980
   ggagttttgt tttgttagtt tggtgataga tattttttaa tgttttttgg ttgggtataa 116040
   tttataatgt tttttgtggg gaggtaattg tttatgaggt atttttggta gggatgattt 116100
   tgttttattg ttatatattt tagtaatgaa ataagaattt agagtgatgt gatttttttg 116160
   atgatttttt taattatttg gggtttttgt gtgattttaa gaagggggaa ggaggtagtt 116220
   tttgtttttt tgggtttttg atgtggtagt tttggaatgg ttatgtgttt gttggtttat 116280
   agtgggttat ttgttgaatg tggttgtggt gggattgggg ttgggttgga gagtgggtga 116340
   ttatattagt tagttttatt ttgggttatt gtgtagttag gggttttatt tttttagggg 116400
   ttttgttgtg agtgttttgg ggttgttgga ataaattatt ggaaattaag tggtttaata 116460
   aagattgatt tgtttatggt tttggaggtt ataagtttga tattaaggta ttggtaggat 116520
   tatatttttt ttgatagttt gaggggagga tttttttttg ttttttttag tttgtgttgg 116580
   tttaggtgtt ttttggtttt ggtagtattt gtagtttttg tttttattat gtagtttttt 116640
   ttattgtgtt tttttgtgtt ttaagttttt ttttgttttt tttttataag gatattgagt 116700
   attggattta gagtttattt taaatttagg atagttttat tttgggattt ttaattatat 116760
   ttgtaaagat ttttttttta aataaggtta tatttgtggg tttttggggt taggtattag 116820
   atatgttttt ttgggggtta ttagttaatt ttttatagaa ttttagatga aagtgaattt 116880
   ttataggttg aaatgggggt gaaggtttag tgtgtttttt ttttttggga gtttttttag 116940
   ggtttgtgtt tattttgtga ttttgtgatt tagttttttt tttgagatga agttttgttt 117000
   tgttgtttag gttggagtat tgtggtatga tttgggttta ttgtaatttt tgttttttga 117060
   atttaagtga tttttttgtt ttagtttttt aagtagttgg gattataggt gtttattttt 117120
   atgtttagtt aattttttgt gtttttagta gagatagggt tttattatgt tggttaggtt 117180
   ggttttgaat ttttgatttt aggtgatttg tttgttttgg gtttgttttg gttttttaaa 117240
   gtgttgggat tataggtatg agttattatg tttggtattt agtaggtttt ggttgttttt 117300
   tttttttgag atagagtttt attttgttat ttaggttgga gtgtagtggt gtgattatgg 117360
   tttattgtag ttttgatttt ttggttttaa gtgatttttt tattttagtt ttttgagtag 117420
   ttgggattat aggtatatgt tattattttt ggttaatttt tgtattgttt agtggagttg 117480
   gggttttgtt atgttgttta ggtttgtttt gaatttttgg gtttaagtga tttgtttatt 117540
   ttggtttttt aaagtgttgg ggttataggt gtgagttatt atgtttggtt tagtaggttt 117600
   tttagaagtg tttgaggttt ggtgaaggta tggtgttatg gagtagagaa tatttaattt 117660
   tgtttttggg gagttggaat atattttttg tagttaattt taatagttat gatgtggtta 117720
   ggatgatatg gggagggggt ggtgtggatt gtgtgggagg ttagtttttt gggaaggggg 117780
   ttggagtggg tttggttttg aggaggattt ttaagaggag gaggaggtgg tggtatgttt 117840
   gtttgggggg atggtgtgag ttagggtggg tattttttta tttaggtgtt atttttttga 117900
   tattttagtt gggatatggt ttttttgggg aagatggata tttttagagt ggggtatttt 117960
   ttggggaaga tggatatttt tggaatgggg tgttttttgt ttttattgtt tttttagtat 118020
   atagttgtat ttttttgtag ttttggaggt tgtgagattt tttggttgtt atttagaata 118080
   aatttgtttt tgagtagtgt tgttttgatg gtggggtggt ttttgtgggt ggaatagttt 118140
   ttgttgtttt agagtttatt gagagttagt gggtatggga gtggtgttgg attgaaatat 118200
   ttagagggat tatagagtta ggtagtaagt atagaggtta gttagtgggg agggagggat 118260
   ttatttatga aaagatatta agtattgaaa gagaggagag gatggttggg gattggtata 118320
   tatgtgtatt ttgtgtagat ggttaaggtg gtgaggtgtt agttatgtag ataattggag 118380
   gaaggaggag tgttttaggg gaggggttag ttttggtttt agaatgattt gtttgtgtat 118440
   gtgggtttgg ttggaataaa ttattgtatt tgtattgtag atttaattgt aagtttatat 118500
   tagggtagtt tttgttttgg tgtagtgttt ttgattatga gttggttatg gtgggggtga 118560
   ttttaagggg atttttaagg agggggttta ttggattttt tagggaaggt tatttgtttt 118620
   tttgggttgg atttgggata aggttttttt ttgtttagtt tttgtttttt aatttgattg 118680
   tatggaagga tgttgaggat atttggaagt tggtttttgt tttatagtat ttataggata 118740
   gaggtatatt atgtggggat agtttggata gtgtttagga ggtagggagt agattagagg 118800
   attgtgggag gttttgggaa ttgtatgagg atttaggtat tgagttttgt attttttggt 118860
   attagtttga tgtatagttt ggttttgtgt ttattttgta ttttattttt tttatttgta 118920
   tgggggtggt aataatattt tttatagttt aggtttaagt gggtgtagat gtgatgagtt 118980
   tggtaagttt attttattat atttagtttt gggtattgaa ttttggattt tttggttggt 119040
   agaagtggat atttaatttt tttttggggt tttggggttt ttgtagaatt agaatatagt 119100
   tagtagtttg tatgtagggt gtagagggat agagggatat tatgttggga gtttatattt 119160
   ggtatatttt tgattttggg gtttagtgtt ttgttgtttt gtggttatgt gattagttag 119220
   aggttatggt tggatttata ggtgaatata gtttgtggat ttgattttgt ttggttttgt 119280
   ggtgttttat tttattttta gttttagttt gtggagatag agttttgttt tgttagttag 119340
   gttgagtgta gtgatatgat tttagttttg taatttttgt tttttgggtt taagtgattt 119400
   ttttgtttta gttttttgag tagttgggat tataggtgag tgttattatg tttagttaat 119460
   tttttatttt tagtaaagat ggggttttat tatattagtt aggttgttta gtgttttaaa 119520
   aagggtttgt tttgttttgt gttagtattt aaagattagg agatattata taaagattta 119580
   gatttttatt tttttgagaa gtgaatagtt ttggttatgt tgggtttata ttttttaggg 119640
   tagtgatgga gttgagatag ggtttgtgat aattgagagt tttttggagt ttatatggtt 119700
   ttttgtttag tttttggtgg aggttgagtt tagtgattta ggattttgtt tgtgtttatt 119760
   aagaaggaga ggtgtttgtg gtttagagag ggtgtttata ggagtgtttt gtttaggaag 119820
   ttggtagttg aggtggttag ggttaggggg ttgggttttt tttttttttt tttagtgttt 119880
   gagtgtattt ggaggaagag agatgggtga ggggaaggta ttgaaattgg ttttggagtg 119940
   ttattattgg gggttgggga ttagttgtta tttttttttt tgtgggtagg ggaggtagtg 120000
   gtgggatttt atgagatttg gattttgtgg tttggtgagg tagggagtgg ttggggagat 120060
   attgtgggtt tttatagggg tgtgttgtgg gtttggtttg gggaaggagt tggtgaatgt 120120
   gtttgttatg tgggtttttt tttgttttgg ttttgataat taggagtggt tggtttttat 120180
   ttggagtgtg gttgtgggga ttgtggaggt ggtagttttt taggtattat ttttattgtt 120240
   tttgagggag tagattttgg tggagaggta ggatggttgt agggtgggag ttggtggttg 120300
   gtattattgt atgggtttgt agtagtggat gggaagatgt aggagaggtt gatgattgag 120360
   aaagttggtt tttgtggatt aagttggggg agttggataa ttaagtggtt agaattttgg 120420
   gttttggtat tagtttgatg tatagtttgg ttttgtgttt atttgtgttt ttattttttt 120480
   tatttgtatt gggggtattg gtatttttta tattttaggg ttaagtggtt gtagatgtga 120540
   tgagtttggt aggtttggtg ttttgttggt attgaaagtt ttagtgtttg tttttagtgt 120600
   tattgatggg gttttgtttt tgttgtggtt ttgattggtt atagttgtgg ttatttatta 120660
   ttggaggtat gttttttgtt gtggttgatg gtagaggtgt gtttgttggt ggtgttttag 120720
   tgtgtgtttt ggttggaggt gaggagggat aaaaatagtt gtgataagaa ggaaattaag 120780
   gttgggtatg gtggtgtatg tttgtaattt tagtattttg ggaggttgag gtgggtggat 120840
   tgtttgaggt taggagtttg agattagatt gattaatatg gtgaaatttt atttttatta 120900
   aaaatataaa aattagttag gtatggtggt ttgtgtttat aattttagtt attggggagg 120960
   ttgaggtagg agaattgttt gaatttggga ggtgttgttg tattttagtt agggtaatag 121020
   agtaagattt tattttaaaa aaaaaaagaa ggaaataaaa gttttttttg gaattagaat 121080
   gtttttatgt agatgtagtt ggagggggag gttgtgtggt ttgtgttgtg gttatggtaa 121140
   gatatgatgg gtagtatttt tttattttag tttgtatttt tgttttagaa tttatgatgg 121200
   tttttttagg atggggttgt tttttttgtt tgttgagata tgaaggatgg gaagagattt 121260
   ttggttttta ttgttgtatt gtttgttttt ttttgttgtt ttttagtagg aaatatgttg 121320
   gagtgttttt tagttattta tttattaatt gtattttttt tttttatttt ttagttttga 121380
   aaagattttt tgaggttgag gaggttgaga tatttaattt tattttattt tggagggttt 121440
   agattttttt attttgagtt attgtggtta gttttattta gaaattttag gatttgggtg 121500
   tgaagaattt agaatttttg gtttgttatg tggatttttt aagttaatgt ttttttaagg 121560
   tgtttttgtg gagggtggag tttttgggtt ttaaggtggt tgagttggtg ttttggtgta 121620
   ttgagttgtt tattgatatt ttgtttaagt aggtggagaa tgttggggtt attggtttgt 121680
   tttggtttgg gtttaagagg gttgaggtgt tgggttataa gatgttagaa ttggtttttt 121740
   ggaggatgga gattattatt gttaaatttt aggagttagt ttattggagg atggagtttt 121800
   ttgtttttaa ggtttttgag gtgtttattg tttttgttat tgatgtagtt tttaagaggg 121860
   tggagattta gatgtttaag tttgttgagg tgtttattgt ttttagttta gtttagattt 121920
   tggagaattt agagtttgtt tttgtgtttt agttgtagag taggttggag tttaagtttt 121980
   agttttttgt ggttgaggtt atattttgga gttaggaggg tgagttgtag agtgttaggt 122040
   tttggatgtt gtggtaatgg ggggtttggt tgttggtttt gttatagaat tttggaggaa 122100
   gaagttggat atggggtgga gggtgttatt ttggagattt agaaagattg gaatgtatgg 122160
   gggtgggggt ttgtaagttt aggagaggtg gttttttttg tttgatggga atatgttaag 122220
   atgttttaag tgggatttga atgagagttt ggaaagattt tttgggtttt aaggagtttt 122280
   ttttatgggt tattgtgggt tttgttggga tttttgtttg ttgtgtgttt ttggagggtt 122340
   tgggaggttt agagaatgag gagttatgta ggaattaggg taggtttatg taggggtgta 122400
   ggatagggtg gagttgagat ttttagaagg gttgttatat tttggtggtt tttgtttgag 122460
   gaattgtagg ttgtttggat atgtttagta gaggttgagg gtttggggaa gggaggaggg 122520
   atgtatgttt tttgaggttt gtattggagt ttatggtttg gttttttttt tttaggaggg 122580
   atggttgtgg tagagggagg ggaatatgag gagtatttta gtttgtagga taaaattttt 122640
   agggtattgg gaaggggtgt ttggattagg gggttgtgtg atagttgtat atggttttat 122700
   ttgggggtgt ttttttggtg agaggagaga ggttatgggg ttttagtagt gttttgggag 122760
   ggtttaagtt tatagttgtg ttgatatgga gtttataggt aggggattgg tgttagtttt 122820
   gtgggatata gggtttgttt ggaaatgtta ggttgtggga tatggttggg ttttttttta 122880
   gaagtgtgtt ttttggtttt agtagggatt gtggttttat ggggttatag taaggtgtta 122940
   gttgggttag aagtttttta agagttaggt ttgattttgg gtataggatt agaatttagt 123000
   aatgtttagt aagttttttt taggttggat aaatagtata gtttgggttt ttgatttagt 123060
   tgttgttttt tatttttaat tgtggtgaaa tatataatat ataatgtgta attttagtta 123120
   ttttgtagtg tatgttttag tggtatttag tatatttatg gtgttgtgta gttgttattg 123180
   ttgtttattt ttggtatttt ttttattttt ttaaattgag ttttgttttt gttagattaa 123240
   gttttttatg tttttttttt agttgttggt agttattatt ttattttttg tttttattat 123300
   gaatttattt gattatttta ggaattttgt gtgtgtggaa aaatataata attgtttatt 123360
   ttgtgtttgg tttattttat ttagtatgat gttttaaagg tttatttgtt gtgttgtagt 123420
   atggggtaga attttttttt ttttagaggt tgaataatat tttgttgtgt gatgtatttg 123480
   tgttgtgtag ttgttttttt attggtggat ttttgggttg tttttatatt tttttaagtt 123540
   ttttttttta ttttttttga tttttttttg atttttttga gatagggttt tgttttgttt 123600
   tttaggttgg tgtgtagtgg tgtgattatt gtttattgta atttttgttt tttgggttta 123660
   agtgattttt tagttttagt tttttaagta tttaggatta taggttgtgt gttattatgt 123720
   ttggttaatt tttaaatttt ttttgtagag ataggttttt attaggttgt ttaggttggt 123780
   tttaaatttt tgggtttaaa tgagtttttt gttttggttt tttaaagtgt tgggattata 123840
   ggtatgagtt atttaatttt tttttggttt ttttttttga atgagagttt tttagtggtt 123900
   agtgggtttg ggggttggga ggatgtgggg tttggagagg gtgtgggttt ggggggagat 123960
   tagtgttttt tatgttttta tgggtatgtg gagtgtatat ttgttttggt gggtttgggg 124020
   tttgggattt tgtatttttt atagaggggg tattggaata tattgagtag ttagaggtta 124080
   gtgggttttg gaaggttttt tttggttttt agtgtgtgag ttttgtgttt ttttttttat 124140
   ttttttaagt ttgattttga agagttattt tgttttagtt atttagattt agtgatagtg 124200
   gttgttggtg gtatggtgtt aggttaaagg tatgggtagt ttgtagagtg gtaggaggtt 124260
   gttttttttt ttgttttttg ttggtgtagg atttttgttt gatgtgttgg atatattttt 124320
   attttgggtt gatgtgttta tatttagttg agttaaatag gatgtggttg gggaggtggt 124380
   tgttattgag ttatttaaat tttggtgatg gttggtgttg gatgtatagg gatgtggttt 124440
   tggttttggg ggataggtag ggggatgttt atggggatgt ataagaatat ttttttttag 124500
   gggtagatat agtttagttt ttaaattgtg ttagagattg tgttgggagt taggtttagg 124560
   gatataattt gtgggttttg ttttttggag tttattgagt tgtgagtagg atggttggga 124620
   tataaggagt gtttagggag tttttttagt agtggaagta taattttatt aggttaggaa 124680
   ggggagagaa aggtaggtta tgtggaggta gtagtgttta gagttgttag gaatttggtg 124740
   ttttgggggg atggtggttt tttttatgga ataggatgta tatgttggag ttggggtatg 124800
   ggagaaaatt attagtattt tagatttggt tatttatgga tagggatttg tgtaggtggg 124860
   agtttagttt atgtggagag gtttgtgggt tgggttgata gttttgaggg tttaagtaag 124920
   tttaggtgga ggaaatgtag ggatagatga gggtagggtt tttttaatgg aggagtgttt 124980
   ttgaaatttg atgggagtgg ttttttgggg ggtttaggtt tgagttttat tagttttttt 125040
   tttgaggtat tatttgtttg tttttttgtt tttttttttt gtgttatgat tgttttggat 125100
   atggatttag ttttttttaa tttagttttt tgtttatttt ttttttaggt ttattttttt 125160
   ttgttttttt atgtggggtt gggttgggag ttttttgagt tttgatatgt atagttttga 125220
   ttaatttttg tggttttttt gttttttgag tattagggat ttagttggta ttatggggaa 125280
   aggaattatg gagggagttt tgtgtttttt gagtagatgt ttagtttttt ggtttttgtt 125340
   tgtgggaggg tatttgtgtt gtggtttgtt tttattttag agttgtgggt gtagggtagg 125400
   gttttttggg gttttagttg aattgttttt agattgattt ttttaaagta tagtattgtt 125460
   gagatttttt tttgtgttga gttttttttg tttatttatt ataggttttt ttatggttga 125520
   tttatttttt tgtttagttt atattttttt tttttttagt tttttttgtt ttggtttttt 125580
   tttggttttt tatatttttt tttataattt ttttgttttg attttttgaa ttttgatttt 125640
   tgtttttttt tatattattg ttttggtgaa tttttatgta ttttttaaaa tttggtttaa 125700
   atattttgtg tttggtttgg gatataaatg atttgtgtat aaattttttt ttttttgtta 125760
   gatttttttt tttaaatgtg aaagtagttt tttagattat aaaaagtaat tgagtatttt 125820
   ttttttaaaa tgtaggaagt atagaaatga gaaagaattt aaaatttatt tgtaagttag 125880
   tgatatttat gtttgaggtt ttgttgtata gtttttaaaa ttttttttat gtttttatta 125940
   gattttgagg gtagggttgt tgtttttgtt tttttttgtg tttttttttt gttttttgtt 126000
   ttttgagatg gagttttttt ttattattta ggttggagtg tagtggtatg attttagttt 126060
   attgtaattt ttgtttttta ggtttaagtg atttttttgt tttagttttt tgagtagttg 126120
   ggattatagg tgtgggttat tatgtttggt taatttttgt atttttagta gagatggggt 126180
   tttattatat aggttaggtt ggttttgaat ttttgatttt gtgatttatt tattttagtt 126240
   tttttgattt tttaaagtgt tgggattatg ttattgtgtt tagtttgttt tggtttttat 126300
   aaagaatttt ataaggggtt aggtgtagtg gtttatgttt gtaaatttag tattttggag 126360
   gttgaagtag gtagattgtt tgagtttagg agtttgggat tagtttgggt aatatagaga 126420
   aattttattt ttataaaaaa tataaaaaat tagttaggta tgatagtatg tgtttgtagt 126480
   tttagttatt ggggaggttg aggtgggagg attatttaag tatgggaggt tgaggttgtg 126540
   gtgagttgag attgtgttat tgtattttag tttaggagat agagtgagat tttgttttaa 126600
   aaaaaaaaaa aaaaaaaaaa aaaattttat aagatattaa gtaagtgatt tggatataat 126660
   aggtgtgtta tgaaattatg ttgaaggagt gagttattgg tggtgagttg ggatggtttt 126720
   tttgtggttt ttgtaggtag atgtagtgat ttttatgggt tgtttttaga gattaagtag 126780
   aaattttttt ggttaatttt atttttagga tatttggttt tttttttgga ggttgtgttg 126840
   gagggtttag tgggtgggtg ttggttttag tgtgggggtt ttggaaggag ttagtatttt 126900
   aggagggagt gtgggtattt agatggttga aggggaggga gaggaagaga ggtgttgtag 126960
   agggtagagg tatttttgtt tgtttttttg ttttagggtt tggtgaggtt tgagttttat 127020
   gttggtgggg tggatttttg gtttttgagg ttttggtttt gtttttggat ttggtatttt 127080
   ttttgttgtt tttttataga agggtgttta ttttttgggg ttatttttgg tttttttagg 127140
   ttttgggtgg ggatggtttt gttttttttt ttggattttt gttttttttt tgttttttaa 127200
   tatgggtgtt tttgggtgga gtggggtggg gttgggttgt agtatttagt attgagtttg 127260
   taagggttgg ttgttggttt agagtttttt ttattagagt tatttttttt tggtttttta 127320
   gttatattag ttaagttttt ttagagttgt tttataaggt gggtgtttag tgtttgttta 127380
   tgggtttggt tttttgtttt tttgggatgt ttattggggg taagagggag gttaggttta 127440
   gtgtttagtt tagttttggg tatgttttag ttttgtttta tgagttaatt ggttttgaat 127500
   tattatttta ttttttttag ttttaatttt ttgatttgtt aaagaggtat ttgtagaggt 127560
   ggttgggggt tgagttttta tgtatttatt tattttaaga attaagaagg gagtttatag 127620
   tggttttggg gttatttgtg aatatggatg tgaaattggg gtggggtaga tatggtagta 127680
   tgttttagta gtttttttgt gatttagagg gtttggaaat ttttgttatg tgggtggagt 127740
   gttgagggag tagggggtta ggtttttttg agttgatgat ggtgggtgtg gttttttgta 127800
   tatttggttt gtattgggtt gtgtgtttta ggggtagaat agggaggaat tttagtatag 127860
   atagtagggt tgttagtata gtgttgagta tagggttaat taaagtatag ggttttttgg 127920
   aattatttaa tatagagtag tgaagaggtg gttttgggga aagtggggtt agagttgagt 127980
   ttgagtgggg ggtggggagg gtgggtatta gtttgtaagg gagggatatt tattgggttt 128040
   tagaagtttt tggtggggat tagggattgt gaagttgggg agagggtgat tattgtgatt 128100
   tagggaggaa gggttttata gatagtttag tttttggttt tgtgtttata ggatatttta 128160
   tataggtttt gagtttattt ttggattgtg gattttttga ggtagggtgt gtaggtttgt 128220
   ttagtattag gtgtaaggta gggggttttg gtaagtgttg agggatttat tggtgtttga 128280
   tgaagtagtg agtttggatt gtgttatgtt ttgggttttg agtttgtagg atttttttgt 128340
   ggggttgtgt gtttttgttt ttggaggtgt ttagaaggta ttgatgtttt tgttgggatg 128400
   ttttatttgg aaggttttgt tgtttttatt tttttttatt tttttgagtt gtttgtagag 128460
   ggtatttagg ttttttttta ttttgggatt gggttttttt tttttttttt ttttaagaat 128520
   aagtattttt tttttttgaa ttgtttttgg agttttttag atttaggttt agagtgggaa 128580
   gttttgtatg tgttggggga ataggaatta atgtgggagg attggttagg tttggtgtgt 128640
   tttttttgtg gtgggatggg gtggggttag agaatagagt ttggttttag agtttaggaa 128700
   gagaattttt ttttttgttg gtttgttaag tattgtgtag tggtgagagg aaaggagggt 128760
   tggtttggga atgtagtggg tgttgagtag ttgtggaata ttttaaatag agtgtagttg 128820
   tggggaaggg tggggttagg aggtaggtgt tgggggtggt ttttttgttt tgttttttgt 128880
   agttttgtgg ttttgattat ttagtttggt tttaggatgg agttttggtt tttagatata 128940
   tggtattggg tgaggttgtt ttttagtatt tgttttagga agttagagta gttgttagaa 129000
   gagagaggag ttgtgtttgt atttgttgtt ttttaggttt gggatagttg ttgggattga 129060
   gggtttgtat aatggtgagt ttgtttggag gggtgtgttg tggttagata gttatgtttt 129120
   gtgtggtgtg gatagagggt atatttagtt ttggaggagt gtatttagat tttttgtttt 129180
   ggggtttaaa gtgtgggtag tttttaatta gattttggat tttagtagat agtttgggag 129240
   gatgttatgt ggtttatttt gatatattgt tttatttgtg attttatttt tgagagttgt 129300
   agggaggtgt ttttttagtt ggattttatt ttttttttgg ggttttttgt gggtgttata 129360
   agtgattttg ttttttttag tttaaggggg gtttttgggg tatgggttgt aattgatagg 129420
   ttagagtagt ttatggttag ggttgagttt tttttttgtt ttttttgaat attgggttta 129480
   ggaaggggtt aaagtggaga tgttttgatt aaagttgagg gaggtatgga gtttttttga 129540
   ttttgttttt ttattttatt gttgttattg ggtgtatttt gtggagtttt aggattttta 129600
   aagaatttag agtggagatt tttggtttta tttagtgtag gtgaggggga aagtgatttg 129660
   tttatagtta tatggttatt attagtatat ttgggttaag tttgatttta ttttatggtt 129720
   tttgtttgaa atttggaatt ggaaatggtt ttggaaatta ttgattttat ttgttgtttt 129780
   ttaaaatggt ttgtagaggt taagatatag atttttgtaa tgttttgttt atttgtgtgt 129840
   ggggaaatgg tagaggagta ttggttttgt ttttatattg tttgggttgt tgtttttgtt 129900
   ttgtttagtg gttttttttt ttggtttgtt tttttattgt ggaagtgggt ataatttttt 129960
   ttgtgtagtg tggttagagt ggatagtgat gatttgtgaa gtagggtgga gtgtttgggg 130020
   gatttgtgtt aaataagtat ataaataaaa gttaatataa tttttattta attagtaagt 130080
   aggttttgtt tgaagttttt gggtagtatt ttatgttatt tttaggtttt aggttggtgg 130140
   tgttgttttg tgtttttttt atttgtaagt tggtgtttag aagttttgtt ttgtgttttg 130200
   gtttttattt tttttgtgat tttttttttt ttttatttta aaaagtaatg tatgatgggg 130260
   tgtggtggtg ggtatttgta gttttagttg tttgggaggt tgaggtagga gaattatttg 130320
   aatttaggag gtggaggttg tagtgagttg agattgtgtt agtgtagttt agtttgggta 130380
   atagaataag atttttattt gggaaaaaaa aaaaaaagtg atgtttagtt atttttagat 130440
   agaaaatgaa gattatatga aatttggaaa gaagtttttt ttttttattt tttaattttt 130500
   ttttaagggg tggttatgtt gatagttggg tgtggtattt ttttgaattt tatttttgta 130560
   gagttattta gggtttgtgt aaaaaaagtt aatgtgtaaa tatagatttt ttaagaatat 130620
   aaaggaaatt agatttttgt tattagtttt tatttttttt atttaataat attgttgtta 130680
   tttgtagatg tagtatgttt tttttttttt tttttttttt ttttttttga tagagtgtgg 130740
   tttaggttgg agtgtagtgg tgtgatttta gtttattgtg gtttattgta atttttattt 130800
   tttgtttttt gtttttaagt gatttttttg ttttagtttt ttaagtagtt gaaattatag 130860
   gtgtttgtta ttatatttgg ttaatttttg tatttttagt agagatgggg ttttattatg 130920
   ttggttaggt tgtttttgaa tttttgattt tgagatttgt ttattttggt tttttaaagt 130980
   agtatgtttt ttttaatggt tgtgttatta gttgttttat ggttttgtta ggtttgttta 131040
   gttttttatt gtgattgaga ggttgtaata gatgtttttg tatttgtata tgtgtatata 131100
   tgattgaagg atgagttttt agtgaatttt tgtgttaagg atgtgtgtat ttaagttttg 131160
   atttgttggg tatagtggtt tatatttgta attttagtat tttgggaggt taaggtgggt 131220
   tgattatttg aggttaggag tttaagatta gtttggttaa tatggtgaaa ttttattttt 131280
   attaaaaata taaaaattag ttgggtatgg tggtttgtgt ttgtagtttt agttatttag 131340
   gaggttaagg taggagattt gtttgaattt agtaggtaga ggttgtagtg agttaagatt 131400
   gtgttattgt attttagttt gggtgataga gtgagatttt ttattaaaaa aaaaaaaaaa 131460
   aaaaaaaaat tgatttttgt ttttgaattg gtttttaaag tggttattag aattttttag 131520
   gatttttttt tttgttgatt tgtttagttg tttgttgggt tttgtgtagt gatggtttag 131580
   tagggaggtt ttatgttttt tatttttggt tgagggttaa atgtttttta attggtgttt 131640
   tattttgtgt attttggtta agtgagaggg attatttttt tgtatgttat aggttagggt 131700
   tttgtgtttt ttttttattt ggaaggtgta tttttttttt gttgaaattt tttaaaaaga 131760
   gttttttgta agttaggtgt gatggttaat atttataatt ttagtatttt gggaggttga 131820
   agtgggtgga ttgtttgagt ttaggagttt gagattagtt taggtaatat ggtaaaattg 131880
   tatttttgta aaaattagtt gggtgaggtg gtgtgtttgt agttttggtt atttgggagg 131940
   ttgatgtggg aggattattt gagtttggga gtttgaggtt gttgtgagtt gtggttatat 132000
   tattgtattt tagtttgggt gatagagtga gattttgttt aaaaaaaaaa aagagttatt 132060
   tgtatatttt tattatagta ggtatgagta ttttgttttt agttgtttat ttttgatttt 132120
   gtttttggga tgttttttta tatagaatat tttaattttg atattgttaa gtttttgttt 132180
   ttttttttag tttttggaat ttatattttg tttagaggtt ttttgtgttt tgaaattata 132240
   ggaaagaatt ttatttatgt ttttttttag tatttttgtg gttttatttt tatatgttga 132300
   aattatgaat ttattggaat gtattttggt tttagtagtg aggtaggggt agatggtatt 132360
   gtttggatat atagtagtga gtttggtttg gagtgggtgg ggtgtgtgtg tgtgtgtgtg 132420
   tgtgtgtgtg tgtgtgtgtt atttttatta gttttggtgt ttgtattgtg ttggtttagt 132480
   aaaaataatt tggatgtatt ttttttattt tttgtttttt ggaatatttt aagtagtaat 132540
   ataataattt ttttgttaaa attttttaag aatttatttg tgaaattatt tgagtttgat 132600
   atttttgggg gagtttagta ttttaaaaat ttaaattttt tttatgggtt tgtgagtttg 132660
   ggagtttttt ttttttttta aaatttatta ttattttttt tttagaaaag tgttttttag 132720
   tttatttaaa atttgttagt ttattttgag gggttgtttt tagtttgttt agtgaggata 132780
   gtgtgtagtg tggtgattga tatgattgat tagtagagta tgattgatta gttggtgtgt 132840
   aaatatgatt gattgtggtt agtgttttgg gatgttttat gttgaggtat atgtgtgtag 132900
   tgtagggtga tgtgtatttt gagaggggat gaagttatag gatttttgta tttttaggag 132960
   agagtttttt tttttaaggt tgtagttttg agtaggtagt gatgggattg ggtttagggg 133020
   gttgagtggt tttagtaggt taagggtttt aggaattatg agaagtttgg agttgtttag 133080
   agagtagttt ttaaaattag tttggattgt agtttttaga aagatatgtg attgatatta 133140
   tgaattagga tatatttata tatggggtgg gggtggggat gggatgtatt aatgaaatta 133200
   aatttttgtg atggaatttt tatttttatt atgtgtgtgt tatatttagt attttttttt 133260
   tttttttttt tttttttttt tttttttttt ttttttgttt gtttgttttg gagagaggga 133320
   aggttttttt ttattttttt ttaaatagtt ttattgagat ttaagtaagt tattatataa 133380
   tttattaatt tatttattta tttatttttt tgagatggag ttttattttt gttgtttagg 133440
   ttggagtgta gtggtgttat tttggttgat tgtaattttt gttttttggg tttaattaat 133500
   ttttttgttt tagttttttg agtagttgag attatagata tgtattatta tgtttggtta 133560
   attttgtatt tttagtagag atggggtttt tttatgttga ggttggtttt gaatttttga 133620
   ttttaggtga tttatttgtt ttagtttttt aaagtgttgg gattataggt atgagttatt 133680
   gtgtggttta atttatttat ttaaagtgta taattttatg gtttttagtt tatttagaga 133740
   gtagtgtaat tattgttatt ataattaatt ttagaaaatt gaaaatttga gaaatttaga 133800
   aaattttttt tttaagaaga aattttgtag tttttagtta ttaattttgt tttttttttt 133860
   tatatttttt ttttatttgt gtggattttt tttttttttt tttttttttt tgagataggg 133920
   ttttattttg ttatttaggt tggggtgtaa tggtttaatt tttgtttatt gtaattttta 133980
   ttttttaggt ttaagttatt tttttattat ggttttttga gtagttgaga ttataggttt 134040
   gtgttattat gtttggttaa tttttgtatt ttttgtagag atggggtttt attatgttgt 134100
   ttaggttggt tttgaatttt tgggtttaaa tgattttttt gttttggttt tttaaagttt 134160
   tggaattata ggtgtgagtt attgtgtttg gttgatttta gatatttttt gtatagagaa 134220
   ttgtatgttt ggtttttgtg tttggttttt ttatttggtg ttatgttttt aaggtttatt 134280
   tatattgtgg taggtgttag tgtgtttttg agtaatattt tattatggta tggattattt 134340
   tattgtggat agatatgttg tttttattag ttgagggata ttgggttgtt gttattgttg 134400
   gtgagtgtat tttttttaaa ttaaaaaaaa aatgttggtt attatttatt agattgtttt 134460
   tttggtttat agggtgggat aagttggttt gggtgagggt taggaggtgg ttaggtggag 134520
   gtggttggag tggttttggg ttgtttttgg gtgtagtagg tagaggtgta atggtagagt 134580
   ttgagtaggt tttttgggtg gatttttatg ttggtaatgt ttgtttattt aagaagtttt 134640
   ggttaggtgt ggtgttttat atttgtaatt ttagtatttt gggaagttga ggtaggtaga 134700
   ttatttgagg ttaggagttt gagattagtt tggttaatat ggtaaaattt tgtttttatt 134760
   aaaaatataa aaattagttg ggtgtggtgg tgtgtatttg tagttttagt tatttggtag 134820
   gttgaggtag gagaatttgt ttgattttag gaggtagagt ttgaatttag gaggtggagg 134880
   ttgtagtgag ttaaaattgt gttattgtat tttagtttgg gtgatagagt gagattatgt 134940
   tttaaaaaaa aaaaaaaaaa gaaaaaaaaa gaaattttta gtgttgtttt gtgtttggta 135000
   ttgaagttga gggaaggtag tattagtatg agtttttagt ttattgggtt tttgtttgga 135060
   gttgatgtgt gattaaggta tatgagggaa gtatggtgag gtggatggtg tggaggtata 135120
   aggagtaaaa taaggtagat gtagtggttt agtggttttt ggtggttgtg aggagtgtgg 135180
   gtaggatagt tttaggaggg tatttggaga gtagggttgg ttggggtaag gtgtgttagg 135240
   tatttgttag gagtaaaatg taaggggata ttaaagagtt taggaattaa tatagatagt 135300
   atttaatgta gtatttttta atattgaaat gatgtatagt aatttttatg atgaataaat 135360
   gttggaattt taaattaaga taggtttagt tttgtatgtg tatagttttg ttagagtttg 135420
   tttagtgtgg aatttgattt gtttaaaatg ttgttttaaa atattgtttg atttttggat 135480
   tttttggtat tgttttaatt tttgtatttg gatgaaggtt ttattttaat tttagttgga 135540
   gagggttaag ggttggtgaa tttggttttg gatttgtaga gtttgtggta tttgagaggt 135600
   ggggttagga ataggaagga gttggtgaga atggtatttg gtatgggatg gggttagatt 135660
   agagtagtat tgtggttttt tttaatttgt ggttttgttg ttggatgttt ggtgttggtg 135720
   ttgagttttt ggttttttga atggagtgtg atgaataatg gggttgggga gtttttgggt 135780
   ttttttttga agtagtagtg aagttaggga tatgttggta gtaggtaggg agagggatgt 135840
   ttggtttgtg tttggagtta gttgttttat ggaggagttg ggtgaggatg tggtgggagg 135900
   aagatttttt tagttttgta ttttttgaag tataattgag tgtttgtgtg gtttgggggt 135960
   tattttggtt tgaggatagt tttgttttgt ttagttaggt tttgaagagt tattgggata 136020
   tggatagtag ttttgttggg tggtaggttg tttatttttg tttatttaat gttggaattg 136080
   gatgtagagt atttggatgg ttagttttag agtgtttttg atatttttag ttattttatt 136140
   ttttagatta ggataagatg tgggatttta attttgagtt ttttatgggt tttattttat 136200
   gtgtttttat aagttattgg gtttattttt tggttttagg gttgtttggt tatttggtat 136260
   tttggttttt ttatttgtat aatgaaaata atagttttag tttttttgag gagttataag 136320
   atgttttttg tgtgaaaaat tgggatttgg tattagatta tttggatttg aattttggtt 136380
   ttattttttt taatttgtgg ttttgggtta atttggtttt tttgttttta gtttttttat 136440
   ttatagagtt attttattga gttgttggga ggatgaatat aaggaagtgt atttatgtgt 136500
   ttagagtatt gtgattttat ttattattat taaatgaggt atataataag tgtttaatag 136560
   atgtttagtg ttattttatg gattttgtgt gttttgtgtg tttttagtag ttaagttttg 136620
   gaggtgggga agttgttgat gagttgtatg aggtaattag aaagaggagg ttataggtgt 136680
   aagggtttgg gtgatatttg tggatagaga gaggttgatg gattttggta gaggggaagg 136740
   gggtttttgt atatagaaaa tgttttttgt atttaagatt tttgtgattt tggaatagag 136800
   gtagtagagg tttgtaaatt ttaaattata aatttagaaa gtatagaata tttgtagaga 136860
   ggattagaaa tattatatat tttagtgtgt atagaatttt ttatatagtt aaataaaagt 136920
   atgtttttaa ttgaagttgt tggagagttt ttgaggattt tatattagat aagggttggt 136980
   aagtggaagt ttaatatgta ggagttattt tttttgttta taaatgtttg ttgttttttt 137040
   tgtttatatt tgtatttatg tgagggtatt tataggttta ttgtttagtt ttatgttttg 137100
   tttgttatta aaaaattttt taagaatttt ttagttgtaa atttttttat tggagggggg 137160
   gttttaggaa tttagttttg tttggggttg gggtatttta gtatttgttg tataggtgtt 137220
   ggttattaaa ggttatgtgt tgtatttgtg ggtttttggg gtttggtggt atttgggtat 137280
   atagtttagt ggtggttttt tatttgttag atattgggag tgggggtttt tttatttttg 137340
   ttttttggtg gtggggggag tgttttgagt tgttatatat tagaatttat tgatttatat 137400
   gttgtggtat tttatagttt tttgatagtt ttttagtttg agagattatt aaattgagtt 137460
   tttggagagg gtggttgttg aggtttggat ataggtgtgg ataggatagt tttgttttat 137520
   tttggagttt tgttttggtt tagttttttg tgggtttgaa gtttaggttg ggttagtttt 137580
   tgttttttag atggttggaa aggtagttta gttttatggt ttttttgtta ggttttgaga 137640
   gttgtttggt ttttagtttt gttttttttt ttttttaaga tggagtttta ttttgttgtt 137700
   taggttggag tgtagtggtg ttattttggt ttattgtaat ttttattttt tgggtttaag 137760
   taattttttt gttttagttt tttgagtagt tgggattata ggtgtgtgtt attatggtag 137820
   gttagttttt gtatttttag tagggattgg tttttattat gttggttagg ttggttttga 137880
   atttttgatt ttaagtgatt tgtttatttt ggttttttaa agtgttggga ttataggtgt 137940
   gagttgttat gtttagtttt tagttttgtt ttgatttttt tttaaggttg tgaggatgtt 138000
   tatgtttttt ttttttttta tggtttattg atgtttttag atgttttgta aagttatttt 138060
   ttttttggga gttttttagt tggtgatggg ggattttttg gggattattg tttaggtagt 138120
   tatgtaggtt tgtgggttgt gtttttaggt tggattgaga tttggtgtat ggggtgtttt 138180
   tggtttgagt tgaggttttt ggaagttata gttaggaggt agtaagggat ggttgttatg 138240
   tttttttttt tttgtttttt agggtttttg tgggatagat ttttttgttt gagttttttg 138300
   gttgtttata ttttggtttt ttaaagttta gtttgaagtt ttaggtattt tataatttgt 138360
   tttttttagt agtttgtttt gttttttttt tgttgatgtt tttgaaattt ttttgttttt 138420
   ttttattttt tggttagaga gagattggag attttgtggg aatttttgag gggagggaga 138480
   aggttgttgt ttgaatggag gtggtttttt tttttgtttt tttttaggtg ggttatattt 138540
   gtaggtttag ttttttgttg tggtagtgtt tggggtgttg gttgtttttt gggtatgagt 138600
   tgggagttga gggaggtgtt tttaggattg gagtggtaat ttagttggag gaggagtaat 138660
   aggtttgtgg ttagtagtag ttgttgtagg gattttggtg ttgtatttgt gttttagttg 138720
   attttttttg tttttttagg gtgggttggg gatggtggtt tgttgtttag tttaggaaga 138780
   agaggggttg taattagtag tagttatttt aggggatttt agtgttgtat ttgtgattta 138840
   gttgattttt tttttttttt tagggttggt tgggaatggt ggtttgttgt ttagtttagg 138900
   aggaagaggg gttgtggttt ttattttaga ggtttttttt ttggtttttt ttttggtgtt 138960
   ggtttaattg taggtttgtt ttaggttgtg ttggtatttg ttgattgatg tttagtggat 139020
   gttgatagga gagggttggg atggggaagt agtaagtttt ggttattgtg ggtttgggtt 139080
   atagatggtt gaggtatttt ttggttattt ttaggaagta aggttttatg tagtttttga 139140
   tttatttatt gtttgtgtgt attttaggag gttgtatttt ttaaggtttt tattaggtaa 139200
   ttttattggg ttgggtgttg ttgtaaaatt atttattggg tgtggtggtt ttttggtggg 139260
   gggtatggtt ggggaggatg tggtggagga gaagttagtt ttgtggttgg ggggagggtg 139320
   gattatttgt tttttgtttg ttttttgaat tggagtttat ggtttttttt ttaggtgttt 139380
   gggttatgga agaggttata gttttgtttt atttttgtgg tagtttaggg gagttttggt 139440
   tagttttgtg ttttttggga gggtgatagt ttggttaggt tgtagggtag gattggaatg 139500
   tagatttttg tttggttatg ttgttgtttg gttgttttgt ttggggattt tttgtttagg 139560
   aagagagaag tgggttggat agtttggttt agtgattgga agtttagttt tttatgttta 139620
   gtttagtagg aagtttgttt ttttggaata tttgtgtgtt tttgtatgtg tttttttggt 139680
   tggtggtttt gtgaggtttt tattgggttg tgttatgttt tttggttatt ttggttttgg 139740
   tgtttggttt ttgtgaaata gagatatttg ggtagttttt tatttgtgtt tgtaagtttg 139800
   tgtggtttta gtgatggttg attttgtttt atttagagga tttgggaatt tttagtttaa 139860
   ttgtttagag gagttttttt tagtggggag agtttgagtt gtttttttgg tggatagttt 139920
   tttagatttt aggtggattt ttttgttggt tgtttttttt gttgtttttg tttttttgtt 139980
   ttttatttgt tttttatttt tttttttgta gtagttagtt ggtatgggga tttttttttt 140040
   tttttgggat tgtgaatgag atgatttttt tggttttttt gggtttagat ttttttggta 140100
   atagttagtg aaatttttgt tttaggtggg aggagaaggt ttagtatgtg gttataataa 140160
   tggttttaat tatattattt ttaaatgttg gtttttattt attttttgtt ttgtttaata 140220
   gttattttta ttattttatt ttttagatta ggaaattaag gtgtagagag ttttagttat 140280
   ttgtttgagg tttttatagt taagtgtttg gatttgaatt ttggagtagt ttggttgttg 140340
   agttgttttg gagatggggt attgtgtagt ggttggggta ggggtggagt tttgttgttg 140400
   ttgatttttg ggttatttgg agtgataata tagtttagtg tttgggtttt tgtttgagat 140460
   tttggttgga tgttaggagt ttgagatttt ttagtttttt tttgtgtaag gtgttatggg 140520
   gtgaaatgaa agaaaatttt agttagaagg tgtttttttt tttttttagt tttttagttg 140580
   ttttggattt tgaaataaga tattgtttgt ttgtggggtg agtaggaata gattggaatt 140640
   gtattttttt ttttttagtt atagttgtta ggagtttatt tttttttgtt tttttttttt 140700
   gttgtttttt tttataagat tgggttggat gttgttggga aagttaatag gaagtttttt 140760
   ggttgtggtg gaggtggggg tttgtgtttt tttggggatt gagatgaggg ttttaggttg 140820
   gttttggtaa gagtagggag gtttatgttt ttgtttgttg gttttgtttt ttgttttggg 140880
   agaaggtatt tatatgtttt agaatgtatt ttttgtgtgt atagggatgg tttttgggtt 140940
   atttatttga ggtttattga tttttttttt tggaattgga gtggtttttt tatggtggat 141000
   tgggttgagt tttttgtttt ggttgatgtt tttttttagg tatttttttt tattgtgtta 141060
   gggtgtaggt tgttagtgga tgggttttta tttttgggga ttggtagttg ttatgtttat 141120
   tttatagttg agatattttg gttgaaggtt ttgttaagtt ttatggagtg tagggatttt 141180
   ttttgagatg gttttttttt gttgtttagg ttggagttta atggtttaat gttttgttta 141240
   ttgtgatttt gattttttag gtttagtagt ttttttattt tagtttatta agtagttggg 141300
   attataggtg tgtgttatta tgtttggtta attttttaat tttaattttt ttttgtagaa 141360
   atggagtttt attatatttt ttaggttgaa ttttaatttt tgggtttaaa tgattttttt 141420
   gttttggttt gttaaagtgt tgggatttta ggtatgagtt attgtgttta gttggggttt 141480
   ttttttaatt ttttttggtt ttttttaggt gtaggatttg ggttttagtt ttatgtgttt 141540
   ttgaatagtt ttgggttttg ttttgtattt tttgggtgtg tggttaggtt ttttgtgata 141600
   ttagtaggag atttaggtga agaagtaggt agaatggaat gtttgtttat ttagttttgg 141660
   ggttaggtgg ggtggttagg atagtagtag tggttggtgt ggttttttgg gaggttagtt 141720
   tattggtgtg gttagtgaat ggggagggga gttttttttt ttgttttttt ttatttttgg 141780
   tttttgggtt tttagggtta ggtttttaga gttttaggtg gaggatggag gttagggaag 141840
   ttggggtaag agtgggttgg gggtgggtgg ttaggtggat tttggaaaat tattttttgt 141900
   aggttttgtt ttttttaggg agtatttagg gttggttttg gggagttggg ttaatggtta 141960
   ttttggatgg tgtaggggtt tatttttatg aaattgggtt tatttttatg aaattgggtt 142020
   tatagaagtt tttggttttg gttttgtttt gttttttggt ttttgttttt aaagtaggat 142080
   ttaggatagt tttgaatttt ttgttgaaat gtagtttttt gggttttaat ttagtttttt 142140
   agaattagag tttttggggt gggttttttg tttgtttagt agtttttagg ggatatttgt 142200
   gtttgttaga gtaagtttaa taagtattgt tgtaaatttt ttgtttttat agttttttgg 142260
   tttgtttttt tttttttgtt gggatttgta ttaggattaa atgttaatat tagggattag 142320
   gttatatttg tgaattttga gataatattt gtattttttt tttttttgag gttgttttgt 142380
   ttgttttgtt atttaggttg aagtataatg gtgtgatatt tatttatggt aatttttatt 142440
   tttagggttt aagtaatttt tttgttttag ttttttgagt agttgggatt ataggtttat 142500
   attattatat ttggttaatt tttgtatttt tagtagagat ggagttttat tatgttggtt 142560
   aggttggttt tgaatttttg gttttaagtg atttgtttgt tttagttttt taaagtgttg 142620
   ggattatagg tgtgagttat tgtgtttggt ttgatattta tattttgatt aagttgttgt 142680
   ggaaatattg agtttttttt gatagttttg tttatagagt gaatgtttag gaattggtga 142740
   ggtatttttt atttttattt agatggtggt ttggaggtag tgtttttgat tttgtgatgg 142800
   gatttgtttt ttttgtttaa gagattttag agatgtggtt tttgaatgtg ggggtttttg 142860
   ttggggtggg aatgggagtt ggttttggtt attgtttagt gattttattt tagtgttttg 142920
   attttagtat ttttgatttt agtgtttttt tttttttggt tttgtgtgga gattatgtgg 142980
   tttgtgttga gtaatttttt attgtttttg ttgtgttttt gttgtaggga agttttttta 143040
   gggagttagt tgatatattt ggtttttatt atttgttttt gaataggaat gaaaggagag 143100
   agtgagtaag aaagtggtag gaggtggggg ttgtagagta ggggaaggtt agagagaggg 143160
   gaggaaagat ttggaaagtt taggtatgtg ttttggaagt aggtgttagt gatgagttta 143220
   tttggagggt ggttgtttgt tttgagtttt gtttggggtt agtagggaat attttgttag 143280
   ggtatgttgt tgggagtttg tttttggttt attttttgtt gttttgagtg ttgagtagat 143340
   ttgtttttat tttagggata atgtaaggag gttgttattt tattttttat tttattaata 143400
   ttttatgaat tataagtgat tatttattgt gttttgatgt attttaggtg ttgggtttga 143460
   agttttatgt gttttatggg atgtatttgt attattgttg tagaaaatgg tttttgttat 143520
   tattgtagag aaggtattga ggtatagaga ggttgaggaa ttggttttag gttatatagt 143580
   tagaagtggt tgattttggt tgtgaatata gggttgtttg attttaaagt ttgagttggt 143640
   ttgtagtgag gtttatggtt tttgtgttat tgtttgagtt tttggttgag ttttttggtt 143700
   tagtgggggg tttagtgatt tattagttgt tttattatat gttgtggata tttttatggt 143760
   ttatttgggt gggagggaag gggttttagg aggtgggagg ggtataggga atatagttta 143820
   gtttgtttgg gtttaatatt ttgattttta aggtattttt tgagggtagt atgaggttaa 143880
   gtttttgttt ttttttagga ttttgttttt gtgagggttt tggggttttt tgtttttttt 143940
   tgggggtttt gttggttagg attatagaga agttttttgt tgttgatttt tttgtttttg 144000
   tattttttgg taagttattt tatgatttag ttgttttgtt tgtaaaatgg gttgaataat 144060
   attgttttgt tttggagggt ttttgggagg attaaatgag atttgttgta tagaagtttt 144120
   ttgtaagtta tgttgattgg tggttgttgg gtttttaagt gaatgggtat ttgggtattg 144180
   agtgtgtgga tttagtattt ggaaggaggg gttggggggt atttagaatt tttgggaggt 144240
   ttggattttt gttttgttta gattttgagt ttttttttgg ttagtttaat ttttattagt 144300
   agggtgaggg taattaggat ttatgtatag ttgggtgaag ttgagtgatt atatgtggaa 144360
   gtttagtgtt ttttatgtaa agtgggtagg tggttaaagt gagttttgtt ttagtttttt 144420
   tatttttggg gttgtttttg gtgttttgtt tttttttaga gttttattga tttgtattat 144480
   gtgatttggg tttgggaaga tgggtttagt tgtgggaggt tgtgtgggtt ggggattgag 144540
   agtgttggag gagggttttt taggaaaatt tagttgtata ggaagttggg ttagtggttt 144600
   ttggaagttt ttgggttttg taagttggat tttttagatt tgtggttgtg gggttttttt 144660
   ttgtttgtag ttgagagttt ttgatttgta tttgagggtt ttagaggttt gggaggggga 144720
   tgtgattgtg tttttagggt gaagtttatt tgagtgttat tttggatata gtttttattt 144780
   ttttttggtt ttttttttgg ttatggggat gttgtttggg gggggttttt ttatatggaa 144840
   ggagttgtag ttgagttggt gggttttgta tatttttaag gttgtttgat ttttagtttt 144900
   taagtggggt tgtatttatt atttaggatt tggagtttta gttagagagg gattttttat 144960
   tgtttttttt tttgtttttt gggaaaggta gggtttttgt gttttttttt atttagggat 145020
   ttggtatgtt gggaggttaa gaggaagttg ttttttgttt atttattttt taattttttt 145080
   agttagtaga gttttatttg tgtgtttaga atttgtgtgt ggagttagaa gttataaggt 145140
   tggtttagtt gtgtttgatt ttggttgtgt ggttttggat agttttagtt tttttggagt 145200
   tgagtatttt gatttgtaga ggagaggttg ttgtgttggt tttgtagggt tttgtgtaga 145260
   ggggagtgtg tgtggttgtg agtttaggtt atgtttttag attggaggat aggattggaa 145320
   tagtgggtta ggatggttag agggttttag ttttaggagg aggtttttat attttttagt 145380
   atttttatat taggaaatgt agttaggttt ttaggttttt tatgttagga agtagtattt 145440
   tgttttttat ttttggttgt ttgggtttga gaagttgagt attttttttg aatttgagtg 145500
   ttttttgatt tttgtttggg tagttgtttt ttttgaattg attgtttgtt ttatatagtt 145560
   ttttgtggtt tgtttgatag ggtggttagt gagttttttg tatagtatat tttttttagg 145620
   agtgttgttt gtgatgatgg tgtaaagggt gtttttttga gtttttggag gtatgtagtg 145680
   gtgtaatagt gtgagtttag gagttttggt gagtttttgg atggagaatt tgtttagatt 145740
   attttttagt ttaaattttt tttgggtgtt tgttgtgttt ggggtgttga tttattgtgg 145800
   ggtggtttgt gagattttat gttatttggt tatttttttt agtttttgtt tattgtattg 145860
   gttttttggt ttggttgttt ttggaatatg agggtttatt tttatggaag aatatttatt 145920
   tgtttttttt tggaaagttt ttttttagat tttttggttg ttttatttag gttttgttga 145980
   agtgttgtta ttttggagag gtttttttgt tatttgtagt taaaatagtt tttttatgtt 146040
   tgtgggtggt tttttatggg gtatggatga gtattggaga gttttgttgt ttatttgttt 146100
   atatgttttt tgggagggta ggggatattg atttgtgttg tgggtgtttt gtatgttatg 146160
   ggtgtttagt gaatgtttgt taagtattag gggagtataa aggagggaag tttttgttta 146220
   attggagtag ttagggaaga ttttttggag gaagtggtat ttggtttgtt tagaatgtgg 146280
   tgatttgttt gtatgtagat attagattag agggtaaagg ttattattat aatttagtag 146340
   atagttataa gtagttaagg aattgttatg atatggattg tttgtgattg gagttaagaa 146400
   ggagttagag atagaggttt ttgtgttttg gtgtgagttt ttgggggagg ttgagttttt 146460
   agaatgtggg gttgtttgtg tagaggttgg aggtgggaga tatatttgtg agtgttggtg 146520
   agtgtggggt gagttattgg ggttggatta gaagatggga atttttaaat ttattaatta 146580
   gttttggtgt tttgggagtg ataggagatg gggtttttgg gggattaatt tagtataagt 146640
   ggatgagatg ggatgtaggg gaaaggataa agttttttga ggttttggga agttgagtta 146700
   gggtagagtt atgatagggt gagttttaga agtaatataa gaaaaaatag ttagattggg 146760
   tttttttttt attgtgggag atgttttatt ttattttttt tgttttttgg gttttggtaa 146820
   tgggttgggt atagtgaatg ggttttattt gtaggagtgg ttatattttt tgggtatagt 146880
   tgttttatat attgtattga gatgggattt gaggttggga agaatagttt tttttgtagg 146940
   ttgatattgt tagtttttgg ggagggaagg atggtagggg gtttggagta tttttttttg 147000
   ttatttatat tttgtgtttg tttgtagggt tttgagtttt gtttgttttg ttttagtttt 147060
   ggttaggaat ttgttggagt ttataggggt agttaggttg gaataggagg agattaggtt 147120
   ggtaggggag ttgagagggg gttttggata gtatttaggg ttgagttatt ttgggtttgt 147180
   ttgatttgtt atttggagtt gtttttgttt gtttattatg tgtaaggttt tgatttattt 147240
   gggtttgttt aattagtgtt tttttttagg tttagttagt ttggtttttg tttgatttat 147300
   ttattgttat tagtgttatt tttgtattat gtaattagtt tttgtagtat gttagttatt 147360
   aagtttaggg ttttttgtgt attttttgta atttttatag taatttggga ggtatagaga 147420
   ggttgagttg ttggtttata gttatatagt aggtatgggg tattgttagg atttagttgg 147480
   atttttgatt ttagggtttt tttagggttt tgggtgggat attggtaagg aagtaaggta 147540
   tgaatattgg ttttgttttg gatagagttt ggggatggtg gtttggtagg tttagttgat 147600
   atagtgattt tggtgtaggt ttaattattg gtaaagtgtt taagtatata tagaatttta 147660
   attggggaga taggtgaagt ggttaaggga gggttgtgag tgataggtgg atattgtatt 147720
   tattggtaga ttagttgatt ttggtgtttt ttggagtttt ttaatttttt tatttagaaa 147780
   aagaggaatt aatagtttta tttttaaatt gggtgggagt attatatttg aaattgaagt 147840
   aaagttggtt gtgttatagg tttggtagat gttagttgtg taggtaattt agtgattatt 147900
   tgaagtaggt tgtgtggtgg tatttttatt ttgtagatgt agtttagaga ggttagggta 147960
   ttaattttgg gttatatagt gatagtttag gttatagaga gtagtgttgg atgggagggt 148020
   gttggtagtt ttaggtatag gttgggaggg ttgtggggtt agtagaggga gggtatattt 148080
   tttagttggt ggggtttttt tgtgttttag tatttatgtg ggtttaaagg agttggaagg 148140
   agggaggtta gagttattta tgggtttaga agttgataag taggttattg attttgaatt 148200
   tgttttattg tatgtaaatt ggggataaat ttggggattt aatgatataa tgtaagaagt 148260
   agatatgtta tagttttggt tatatagtta gtgtataata agtggtaagt tgttagagga 148320
   ggtagttggt gagaatggga gtattttagt agttttttgt tttttgtttt tggttgtaga 148380
   tgtatttgtt tttggttatt tgtgttttag ttttgtgttt tttggtgtgt gtgaggttaa 148440
   gtttttgggg tggggatttg gggtgtgttt tgtagttttt gaggttaaga ttaaggttga 148500
   ggttgaggtt gaggttgagg ttattttggt gaggaggaaa ttgtaggtgg agaagtttgt 148560
   tgattgtgat tttgatttga ttgtaatttt gatggtgttg atgttgttag tatgtaggtt 148620
   ttttgttttt gttatgattg gtttttgtag tttatttggt tgagaggtgt gttggttttt 148680
   gtaggttgta aaatggggat attattgttt gtttgggtta tagtgtgttt gttgattgta 148740
   tttggggagg gattgagggt tgattgtgtt gtggggatgt tggtagagaa ttagagaggg 148800
   tatttaaaaa ggttaatagt ttggtgggga gaggaggtgt aaggttggtt tttgtttttt 148860
   gggttttgat tgaaagggaa taggggatat atttggggat agtttttttt ttggaggaag 148920
   aagggtttag ggtgttggtg tggagtttgt atagatgggg ataatgagtt ggagggtgtt 148980
   gggtaggagt tggttagtaa taggttgatg agggaggttt tgttggttgg gtttggtttt 149040
   taagtgagta ttttgagttg tggggattta gggggtttta gagattttag agttgagatt 149100
   tgggattata gaggggtggg ttgtgtttag ggtaatatag tggatggggt agatgttgtg 149160
   aaatttgttt ttttgttttt tttttttttg ttatagaatt ggattttttt agtttatggt 149220
   tgttaaattt tttagatgtt tttgggtttt atgggtgttt tatattggtt tttttgtagg 149280
   atagtagatt ttgggggttg gttttgatgg tatttattat ggggtatttt atgtaagtat 149340
   agtgttttta tgtttagttt tttagtttgt ggagtaggtt tgtgtttggt tagttttatt 149400
   ttgtggttag aggagggaga tagtggtttt ggagatagga gttggtttgg ggttgttttt 149460
   ttagtttttg tttttttagt ttattttttt gttttgtggg tgttggttat tttttttttg 149520
   ttagttttat ttttagtttt ttttgtttga gttgtgattt gttagtaggg ggtagtggag 149580
   gttttgaaag tttttggaga ggattttgaa gtagttttta taaatttgtt tttggaaagt 149640
   tttttttgtt atatttttag tggttttttt ttttttaata ttttttttgg ggttgttatg 149700
   tagtgggggg tggaggaatt tattaggttg tggatagagt agagttatgt tgatataaat 149760
   tttggttgaa tttatatgtt ttttatagtt agtggttgat tattggaatt agggaagtga 149820
   ttttatagta agggtttgta gagtagttat tatgtatttt tgattttggt ttttggattt 149880
   agttttggaa aagaagttag attttagtta tttggagttg tttagggaag attttagttt 149940
   ggtgttggtt gatagtttat tggaattttt tatttgtttg gttaggtgat gttaaggggt 150000
   agaggttaga agtgttaaga agtaggttga gggtggtggt ttatgtatgt aattttaata 150060
   ttttgggagg ttaaggtggg tgaatttttt aaggttagga gtttaagatt aggttggtta 150120
   atatggtgaa attttatttt tattgaaaat ataaaaatta gttaggtatg gtggtgtatg 150180
   tttgtaattt tagttatttt ggaggttgag gtaggagaat tttttgaatt tgggaggtag 150240
   aggttgtagt gagttgagat tgtgttattg tattttagtt tgggtaatag agtaatattt 150300
   tgtattaaaa aaaaaaaaaa gaagaagaat aagaagtgtt aagaagtatt tgtttttttg 150360
   attgaaaagt aatttttgtt gggtgtggtg gtttatgttt gtaattttag ttttgggagg 150420
   ttgtggtggg agagttgttt gaggttaggt gtttgagatt agtttgggta atagaggtgt 150480
   taggggtagt ggtgtttgtt tgtagtttta gttttttagg aggtttaggt aggaggattg 150540
   tttgagttag gagtttgagg ttgtagtgag ttatgattat attattgtat tttagttttt 150600
   gggtgatagg atgagatttt gttttaaaaa ataaaatata aataaaaaat taatgtttat 150660
   tttttgtgat tttaaaaatt atatttattt gtgataaaat aattaaatta gaaaatgtaa 150720
   aggaaatgat taataataat tgtgtttttt agataaatat tgttaatatg tttatgtttt 150780
   tttttagttt ttttttatat agatatttaa tatttattaa tttgttttga attaaagatg 150840
   gttttttttg ttttgtatga gaattatttt agttaggtat ggtggtttat gtttataatt 150900
   ttagaaagtt gaggtaggta gattgtttga gtttatgagt ttgagattag tttgggtaat 150960
   atggtgaaat tttattttta taaaaaaata taaaaattag tttggtgtgg tggtatatgt 151020
   ttgtgttttt agttttttag gaggttgagg taggagaata gtttgagttt gggaggtaga 151080
   ggttgtagtg agttaagatt gtgttattgt attttagttt aggtaatggg agtgaaatgt 151140
   tgttttaaaa aaaaaaaaaa aaaaaaaaaa gttgttatgt tgttattttt tttagggatt 151200
   tttatttttt ttattttttg tttaagattt gttttgtaaa aggatgaggt taaatagtta 151260
   tgggagggtg gtattggatg tttaaaaaaa tgttttgtgg ttggtgggat ttagaaatag 151320
   gttggggagg ttttagtaga gggtggattt tagttggggg tggggggtag taggggggtt 151380
   ttttatatgt tttttttgtg tgttttatgg ggttgtttgt gaggttgtgt gaattagagt 151440
   ttggttggga agttatatgt tatttggatt ttgatttttt ttggtagtat gattttagtt 151500
   aatatgttta attatgtttt tttttgtaaa atgggttaat agaaatgttt atttgagata 151560
   tgatgagaaa taaaggtgtt tatagagtaa agtgtttaga atggttttgg tatgatagga 151620
   gtttatatgt tagtgttgtt gttggtattg ttattattat ggtggtattg gttgttttgt 151680
   tttttttttg tttttgttat tttttttttt tagtaggttt ttatttattt gtttatttat 151740
   ttatggttga ggttttgttt gtgttaggtt tggtgttgag aatgggggta gagagatggg 151800
   ggttgtgttt tgttttttga tttaagggtt ttttttgtaa tgtgaagttt ttggagattg 151860
   gatttttagg ttgttttatt agtttttttt taggttagtt taattttttt gtgtttgttt 151920
   tttgagttgt tagaggaaga gagtgggagg aaggatggag tggggttttt gtggggattg 151980
   agtggggggt gtgtgaggtt ttggaaatgg tgtttgtggt atttatgtag tattagtttt 152040
   tgttgtagtt gttgtttgga gaatattttt ggattttagg atattgatga tagaaggtgg 152100
   aatgtttttt ttggaagtta ttaagagggg attattgtgg tggttgagta gagttgttga 152160
   tttgtgagtt gtggtttgat tatagttatt gtttttatga gtgaggtttt ttgtggtttt 152220
   ttttttagga atttttggtt ttttggtagg ggatagttta gggttggggg tgggtagtgg 152280
   tgtttgtagg tttttttgga gttagagaat tttttgttgt tgtggtgggg ttgttttttt 152340
   ttgttttttt ttttttgttg tgtttttagt tgttttgata ggatgttttt ggtttgagag 152400
   atagaaattt taattttgaa tggttttagt ttttaagaga atttggagtt tttttgtgta 152460
   aaatgagatg ttttgagtat tgaggatgtt gtgggggatt ttttatttta ttatttttag 152520
   ttgtggtgtt ttttttggtt ataggatgtt gtagttatgt tgtagggaag gagagttttt 152580
   ttttttgtga gtttagggag tttttttatg tagttttttt attggttatt gtttgagatt 152640
   tgatttggtg gtttttgttt taaggagtag aaagggggtt tgttaggtta gggtggggtt 152700
   gggtgtgagg attttttttg gttaataggg tttgtttagg tgtttgtttt gttggggtag 152760
   tagggaaggg atttatatgt ggtattggga tatggagagg tttttgtgtt tgtgatggtt 152820
   aaaagggatg gtgttttagt tatgttttta tttattttga ggtttagtat ggttatgttg 152880
   gaaatagtag tattgttttt tttattgttt tggtttgttt ttaataggtt agaggggtgg 152940
   ggttggagtg ggtgtttgat ttgtggttgg atttagttgt ttgtggatag gttgtgagtg 153000
   aatttgtaga ggaagggaga tgggaatgtt gggatttttt tttgtatttt ttgaattttt 153060
   gttttttgtg agttagatgg ggagtgtatt tttgtttttt ttttttgggt attttttgtg 153120
   ttttttgttt taggtatttg atgttgtttg tttatttttt ttttttgttt tttttgttgg 153180
   ttttttttat atggttttaa ttaggatttt ttgtttgttg gtggatgggt agagagggga 153240
   gtttgttgtg gggttttttt tgattggggg attttttagt atagagtttt ttggtttttg 153300
   tagggatgtt gtgtgtgttt gtgtgagttt ggtgtgttag aagtgttaag ttgtagttgt 153360
   tttgtgtgtt ttttatattt tttatttttt aaaggttttg ggtttggtgt ttgtgggttg 153420
   gggatttgtg ttggggtttt taggaggttt ttggtgggag aggtagggtt tttttttttt 153480
   taatttgggt aattttgttt tggtttgttg tttgtggaaa gtgttttagg atatggtggt 153540
   aggattggtt ggggttttgg ttttaggttt agttttagtt tttgttagat ttattttttt 153600
   tgtgtttttg tttattttgt ttatttgtag ggtttgtagg agtattttga gagttggtag 153660
   ggtggtttgt ttggtgtttg tatagggttt gttggtgggt ggttgaggtt gtggtgaggt 153720
   ttttatggtt tttttgtttt gtatattttt tttttgttgt gttgttttgt tagtatatga 153780
   gtttgtattg tttttgagtt tgtagtttgt tttttttatt aggtggttaa gtagggtggt 153840
   agttagggtg gtagagtttt attggtttgt aggggatttt ttgtgggttg taggtagttt 153900
   tttgtggttg aaggtgtttt ggtaggattt gtattatgat ttttgttaaa gtttatgttt 153960
   aaggtatttg tgggtatttt tgttttagat gtgagggttg gtattttttt ttgtatgtga 154020
   agtaaaggtt tgtttgaagg agtgtgtgga agtttgggtg tgatgaaggt aaggattggt 154080
   ggtttatttt ttttgtattt ggattttggg tggtaggttt ggggttttta gtttttaatt 154140
   taggagggta gttagttgtt ggaattaaga gtgaatttta gttgttgttg ttatagttgg 154200
   agggaagagg ggaaaggagg tgttgtaggg gagtagagat tttatttttt ttttgttgat 154260
   attaggttgt tggtgttgga tggggttttg gtaattgtgg taggagtggt gatgtttgat 154320
   gatggtaata agggtttttt gaggattttg agttgtttta agggtttttt atttggagta 154380
   agtatttttt ttaatttttt taataatatt gtgaaattga ttttgttgtt atttttattt 154440
   tattgatgag gaatttatag tttagagatg ttaagtaatt tgtttaagtt tttttttgag 154500
   tatggtagag ggagggtttg aatgtggaaa gtttggtttt agtgttttta tttttaatta 154560
   ttgttattta tttttttggt tagttttttg attgtaaaat ttgaggttta gaggagtgta 154620
   gtgatttggt tagttttgta tggtaggtta gtggtagagt tgagtagtgt tttttatgtt 154680
   ttgattttgt ggttttttga ggtggtttat ttttaaatat gttttttatg gtggttttgt 154740
   tttttttttt tttttgaatt tgttagtttg tgtttaggta gggggagaag tagagtaatt 154800
   atgttgggtg ggttgttgtg ttttagtgtt gtttgtttta tatttgattg gtagtagagt 154860
   ggtttttttt tggtgtggtt tggaggtgtt agtggttatt tattagtata gtttagtttt 154920
   atatatgtta tttttaaagg atgttgatat agaatgaagt agttagggga ggtggatgtt 154980
   ttggtatgag ggtggtgttt ttggtaattt gtagtgttgt tgtttgtggt tgtttttggt 155040
   tggtatttta tttttttttg gttgttttgt agttatttga gggaggttag tatgttgttt 155100
   ttattttagg atagtgtgaa tgtagtagtg taagtttgtt agtgttgtat tgtgggattt 155160
   ggaggttttt gtttttgagt tttgtttttg tgtaattttt aaagtatgga ttttttaggg 155220
   gtttttattt ttttatttgg aaaaatttta tattgtttag ggtttgaggt agggttagag 155280
   tatggagatg ttgttggagg attaagggag aattgagtgt gtgttttttt ggttttagtt 155340
   ttgtgtgtgt gtttatgtat gggtattttg ttatttatat tggtttttat ggttgggtgg 155400
   gagttgagtt gagtttggtg gttgtttttt tattttgtgg gtttggttgg ggagtgtgag 155460
   gatgtttgtt ggaagttaat gtgtatgaaa gagttaatgg tggattttat ttttttagtt 155520
   ttttttttag tttttttagg gaaagggtag gaatagagag gttttatttt tagaggaagg 155580
   ggtgggtggg gatatagtgt tgtttggtat agtttttttt gagttatttt gtatgtttta 155640
   ttttattgat ttttgtagtt tagttgttta gagtggattt agaaggtgga gagagggttt 155700
   ttaataagta gaggatggag tgaatgtgag atttattatt gggatttggt aaagtaaaga 155760
   gttttatttt ttttttgtag atgggatggg ggtggggttg gagttagtgg ggtgggggta 155820
   tttagtaaga gttttttttt atttttttat tttttttgtt atgggttttt ttttttatgt 155880
   agatttttag tttggaatga ttagtgttta gttttttttg tttttttttt gttttgttgg 155940
   agggagtgat ttggtttttg gttttggtta tttatatttt ttgttttgtt gtattagttt 156000
   tgtggtaggg gttgtggtgg aggtgggtaa aagttgtttt tgggtagtta gtttttattt 156060
   tgggagtgat tgaaagtttt tgggggtttt tgtagggtga gttgtttatt ttggttttgg 156120
   taaggatatt taggagaggg ttagggtgtt tgtatttggg gtatagtata aagaatagtt 156180
   gtaagtgttt tttaggtagg agggtgttga agtattggga agttgggatt tagaagtatt 156240
   gtttggggtg gtgagtggaa gatgtaggat atagatttat gtgattgtgt agattatttg 156300
   gttttatttt tttggtttat agtggaggaa attagaggtt gagagaggtg aagtgagttt 156360
   tttggggtta tatagtttag ttaggtttag ggtgttgatt ttttgttttg tttttttgtg 156420
   tttatttggt tggtattagt atttttgttt tgatattttt gtgtggtagt tgttgtgatt 156480
   ttgtgatttt tttttatttt attttagtgg ggttttgtga gggaaagaag aatgttttat 156540
   gtagaagtgg ttggaaagta agaagtttgg agagaagtga ggtttttgtg gtttggtttg 156600
   agtttaggat ttatggtttg gtttttaggg atggagtatt gatttttttt ttttatattt 156660
   ttattttatg tttttaggag ttttttatgg ggaagttgga atggatttgg gtttagagat 156720
   tgtaaattta ttttgttgga tgtgtggttg tttttatgta attaattttt ttatggagaa 156780
   gttaggggat ggtttttagt atttttgggg gttttttagt gaggtgggag gattttgaga 156840
   ggttaggagg ttgtttaaga ttttttgttt tttagggatt aggtgagggt ttgttgattg 156900
   ttttgatttt ttgatgtttt tggtggggag attttagagg ggtttgttgt tattattgtt 156960
   agtggttttg gtttgtttag ttattttttt ggggtggtta aattttatgt tgtaaaagtt 157020
   taagtgaatt ttagtagtgg tttgggagaa ttatttgtta tgagttaagg atggagtttt 157080
   tggggaggat tttttgttgt tttttaattt tttttgggta tatttttttg tagttttatt 157140
   ttttgtaaga aagttaggtg ttttttgaag tttttttttg gttttaggtt agaggtgata 157200
   gtgggggttt tttgtttgtg ttggtgattg tggttggttg ttggtgtttg gttttgtttt 157260
   gtattgtgta tttattgaag gtggtattat ttgggttttt ttggtggtta tgtatgggtg 157320
   agtagtttat gatatatttg tttttagtat tgtgtgggtt ggggttttta gaggggttga 157380
   gggtttggat ggttttgtag gggggtgttt ttggttttgt gattgaaagg agttgaaggt 157440
   ttggagaagt tgttgggttg aggttagttt ttatagattt ggtgaggagg gaatagatgt 157500
   ttatggagtt gttttaggta ttgtatatat tgttttattt tgtgttgata gttaagaggt 157560
   tggttttttg ttgattttta ttttatagat aatgtgtttt tttagttttt tagtggtgga 157620
   gttaggattt gaatttatgt aggtagtttt ttgggtttgt agttttattt tttatttttt 157680
   tttaggattt atgttttggg gttggtagga taatgatttt tagagttgag tatattttaa 157740
   tttttagtat ttgggattat gttaggtgat atggtagggt tagttaaggt tattaattag 157800
   ttggttttaa aatagggaga tgattttgga tgatttatat gggtttgttg ttattataag 157860
   agtttttaaa agtgtaatag attatttgag gggtgtagtg ggagaggttt ggaaggagga 157920
   aggaggttat aagttaagaa atgtaggtga atagaaaatg taggaaaatg tagttttttt 157980
   tgtagttttg ggagggtgtg tagttttgtg gatatttttt ttgtagttta gtgaggtgga 158040
   gtgtgggatt aatttgtgtt gtttgatatt ataagtgtgt ggttatttgt tatagtagtg 158100
   atgggaattg aatgttatta ggtagtagtg tttttatttt ggaagaatta ggataaattt 158160
   ttttgtggat tttaagtttt gggagatttt gggttttttt ggattgtttt agttttgtaa 158220
   ttataggaaa ggttttttat gtttgtttgg gagaagaaaa ttttgtagta aatttatttt 158280
   tttttttgat aaatgaaggt gagggtgtgg gttggatata tgtttttttt ttgtggattt 158340
   tttttgtttt aatatttaga attttgtagt tgaaggagat tttggggggt tgtttgtttg 158400
   atttgtaagt gaagaaatgg aggtttaggg aagttaagtg attggtttag ggttatgtag 158460
   agttatgttg gaatattaag ttagatagtt tagtttttgt tttaaagtgt tttttttttt 158520
   ggttggggag gagggttgga gggagtggat tttgagtaat agtagtattg gtaggggttg 158580
   gaagagtagt tattgtattt aggtgttgga gtttttggtt attttttgta ggaattaggt 158640
   gatttattat ggtataggtt tttttttgtt tatttgtagt tttgggggtt ttagggatta 158700
   gaggatattt tggtttgagg gttggggttt gagagaagag atggaagtat ttttagtttg 158760
   gtttgttttt gtggtgtttt gtaggtatat ttggtagttt aggggtttta gagaatttgg 158820
   ttggtgggtg tttttatttt gttgtagttg tattttgtgg ttagtagttt gtaggatttg 158880
   ggttgttgtt tgtggttttg aaatagttat tttttgagtt gagtggggat taggtgagta 158940
   gatttttata ttttaattgt tgtgattttt ttgttttgga ggttggtggg tagtttggtt 159000
   ggtgttttat ggtattttat gattggtttt gttattaggt ttgtgagttg tttttttttt 159060
   tttgggtatt ttgaatggtt tttttgagag ttttgttttg ggtttttgtt tttgtaggtt 159120
   tttgttgttt ggggtgtttt ttttttttgg aagttgggtt tttttgggga tttattaaga 159180
   agggttttgt tgagtgttgt taaattgttt tatttggttt ttatttgttt tatttattat 159240
   tttattttaa tttttatgtt ttttttttgg ttttaagatt tgagtaggta gggtttgtga 159300
   ggtattttag gttttttgat gttgttagta tttaggggat tgttagtata tatgtaggga 159360
   gggaaggaat gaaggaagag atgatttggg gagtttttta gtttttattt gtaattggta 159420
   tattgtgata aaatatttat ggtattttat tttttttata tatttttttt ttaaatttat 159480
   ttttatttga gatggagttt tgttttgtta tttaggttgg agtgtagtag tataattttg 159540
   gtttattgta atttttattt tttgggttta agtgagtttt ttgttttagt tttttaagta 159600
   gttgggatta taggtgttta ttattatgtt tggtgaattt ttgtattttt agtagagatg 159660
   gggttttatt aagttggtta ggttggtttt gaatttttga ttttaggtga tttgtttgtt 159720
   tttgtttttt aaagtgttgg gattataggt gtgagttatt gtatttggtt tttgtatatt 159780
   tataaaagag aatttgttta gaaaattttt tagatgtatt tttagggttt taaggtatag 159840
   atatatgaag tttgttagtg agtagttgtt gtttattttt tttttttttt taatttagag 159900
   tggtttgtgt tttaagtgga tagttgtttt tttttttttg gttttttgtt gtttatgttt 159960
   ttatagaagt agggagaaga ggtaggaagg tgatgtgtga ttatttgttg ggtgtttgta 160020
   tagtgttgtt tgtataggtg ttattttttt tatttgttat tttttttgtt gagttttttt 160080
   ttttttttgt agatgagaga gtattgtttt ttagaggtta agaaatgggt ttagagttat 160140
   ttagttgatt agtgtgtagt tagatttgaa ttatattttg ttgattttta tgtttgtagg 160200
   tttggtttga tgagatggat agttgggtgg tggagatttt gagattttgg atggttttta 160260
   aatagggttt gtagtttttg gatagggaga gtttggtata aagggtttgt ggagtgagtt 160320
   gggggagaag gtggtttagg gtttttgttt ttgaataaat agatggaggg gttattttag 160380
   tgttttttta gtttttggtg tgttgggttt ttggttttgg gaggagagaa tggagttggt 160440
   ttttattttg ggttgggttg gtttgggttt aatttagata agttttttag tttgttatga 160500
   gtgatatttt tgtttgtttt ggtgtttttg ttaaaaatat gaagggtttt agaattttta 160560
   ttttggtttg tttttttttt agatttggag tgggtttggg gttgataata gttttatgtt 160620
   agatagtttg agtagttatt agtgttatta ggtttgatat tgtggttttt gttttggagg 160680
   gaaaaataag gttttttttg ggggattttt tgttagtttg tgaaaatagg tttggagtta 160740
   gttagtgttt ttatgtttag atattttgtg tggtttttaa agtgttttta tgttgttagt 160800
   ttatatgatt ttttttaaaa ttttataagg gagggtagga agtttgagta atagtttgtt 160860
   aagtatttat atagtaggtg gttttattgt tttttattta tagataagta aattgagttt 160920
   tagggtggtt attgtttgtt ttttaagtta tttagttatt tagttatgga agaggttatg 160980
   attttttata ttttagtgaa gttgaagatt tttttttttg tttttaaaaa tgtttatttt 161040
   ttagggtagt gaatatttgg tgtttttgtt tagtgtaaga ggtgaatgtt tgttattttt 161100
   gttttttttt ttttattaga tttgattttt tttttgtgat gggaatattt tgtaggtagt 161160
   ttgttttgtt tttatagatt ttgagaaatg gggtttaaat tgttggttgt gggtgggata 161220
   ggtgatattg tagtgaagag gtggagtatt tttgtagtgg ggaagaaata tatttttttt 161280
   atttttttag gtttagtgtt tgggggtttg tgaattaaat ttatttaaga taaattaatg 161340
   agagaaaaga tagatttaat tatatatgta tgggatttta ttgaaaaatg tgatttaaaa 161400
   gagtaggtag aaggtggggt ttgtgattgg tttgaaggta gtgagttatt ttagttgatt 161460
   gtttatagtt agttatagat tagatttttt gttttattgt tttttttttt tttatttgtt 161520
   ttgttttttt tgagttagag ttttgtattg ttgtttgggt tggagtgtaa tggtatgatt 161580
   ttggtttatt ttaatttttg ttttttgggt ttaagtgatt tttttgtttt agttttttga 161640
   gtagttagga ttataggtat ttattattat gtttggttaa ttttttgtat ttttagtaga 161700
   gatagggttt tattatgttg gttaggttgg ttttaaattt ttgattttgt gatttgttta 161760
   ttttggtttt ttaaagtggt gggattatag gtatgagtta ttgtatttag ttaattagtt 161820
   ttaaaaaaaa aaaaaaaaaa aaaagaattt agggtttgta tattatttta ataggaaaaa 161880
   aggaaggggt gaaagtatat attatgggtt gggtgtgatg gtttatgttt gtaattttgg 161940
   tattttggga ggttgaggta ggtggattat ttgaggttgg gaatttaaga ttagtttggt 162000
   taatatggtg aaattttgtt tttattaaaa atataaaaat tagttgggta tggtggtgta 162060
   tatttgtaat tttagttatt tgggaagttg aggtaggaga attgtttgaa tttatgaggt 162120
   ggaggttgta gtgagttgag attatattat tgtattttag tttgggtaat agagtgagat 162180
   tgtgttttaa aaaaataaat aaaaaaaaaa aagtatatat tatggtaaaa taaatgagtt 162240
   tttggaaaga taaataggtt tttaggagtt gagatgggag atgtgaagtt ttgtgataat 162300
   gtttttttag gtgtggtgtg gaaattattt atatttttta gggaagagtt agttgttttt 162360
   agggagtaga tttgggtttg tatattattt taatagggga aatggaaggg gtaaaagtat 162420
   atattatggg ttgggtgtaa tgtgtttagt gtaggaggta gatgtggttt atggttgttg 162480
   ttgaaattag tgtagggttt gattgttttg tattgagtgt tgaggatttt ggttttgtat 162540
   tagttatgag tttgtagttt tgggatatat ttgatgttgg tttaagggga aatgaggttg 162600
   gaggggtagg gtggttggat tggatgtggg tttggggtta ttgttgattt ataaagtggt 162660
   tattatatgg gaataataga gttggttata gattataatt agtaatatta tttaaaagta 162720
   aggttatatt tttaataata agttatattt taagttatta ttaatttata aaatatattg 162780
   ttggtataat gatagttttt taagaaggat ataaaaaggt atgttgggtt gggtgtagtg 162840
   atttatgttt ataattttag tattttgaga ggttgaggtt ggtggattat tttaagttag 162900
   gagtttgaga ttagtttggt taatatggtg aaattttgtt tttgttaaaa atataaaaaa 162960
   aaattagttt ggtgtggtgg tgtatgtttg taattttagt tatgggggag gttgtggtag 163020
   aagaattatt taaaatttgg gaggtggagg ttgtagtgag ttgagattgt gttattatat 163080
   tttagtttgg gtaatagagt tagattttgt tttaaaaaaa aagaaaaaaa agaaaaaaag 163140
   taaaaaggta tgttgaaggt atttgtttgt ggaaagttgt aatttgatgg gaggtagggt 163200
   gagtattttt tagaaaggtg gaaatgggtt tttttgttat agagatttat aaatatagat 163260
   tgaggtttgt agtttaggag atttaaaaga ggtaaaggtg aataagagtt aaggttttta 163320
   agttagggtt tgattttggg ggtgggggtt atatatgatg gaggatgtta ggagggtttt 163380
   ttggaggtgg tgatatttat taggggggat tttaggtggg gtttgtgaga tagtaggatt 163440
   tggttgaaga gtgtaggttt tttagatagg agtgggaatt taggtttgaa aatgagttgg 163500
   ttagattgta ggggtttttg aatgtaaggg aagaatttta attttaatag aggagtttgg 163560
   gaagttgttt gggaaaagga gtgataaagt gggaggggtt ttttagtaga gttttgtatg 163620
   ggtaggaggg agtgagatgg gaggtgggtg ttggtaaggg atgattttag tggtttaggg 163680
   gagtagttat agggtggaaa ttaattttgt ggggtttggg taggaaggga ttgatttgag 163740
   atattgggaa gaaatagtta gtaggatttt ggtgagaggg ttgggggttg gggagggggg 163800
   tgtggagagt gggagtttga gtttttgaaa atagatagtg ttttaagggg gagatttgag 163860
   gtaagatgtg gagaggtagt tgagttggga agttggaatg gaggtgtggt tattttagga 163920
   gattaagtta gggggtgagg ggatttttgg gagttattta gattttagta ttagggaata 163980
   tggaggggag aaggagggga gaagaattag ttggtgtgtg ttaggtattg tttttgaggt 164040
   tagatggggt ggggttggat attggttatg aggatttttt taagagttgg gttaatagtg 164100
   agatgggtgg atattagatg gtttgggtta agaagtaagt gggtggtggg aagttagaag 164160
   ttgtggtgta gatttatttt gagaggttgg gttgtttaga tagggagaaa gtttatagtt 164220
   tgaggaggta gataggttaa aggaagaggg tttgtttgtt tttgagattg gggtattttg 164280
   aatgtttttt taggttaagt gagaagaaat tagaagagga atatgttatt taaatgtgtt 164340
   gaaatagttt agtagttgtg ttgttttata gaaaatgtgt agaggttttt ttgttttatt 164400
   atgtttgttt ggggatggtt tttatttggg ttatttaggg tttttttagt ttgttaattt 164460
   aagggtggta ggagttgtgg gtgtttttag ttttttttta aggttggttt gtgggtagaa 164520
   atgtgggttg ttttggggtg ttatagttat aaatgtatat tagtttttag aattatattt 164580
   ttgtttttta gttttttttt ttgtattttt atgtagaagt gtggttgtta gtttataaag 164640
   gatagggagg gatattgttt ttggtatttg atgggaagta tttgttgtat tttattgggg 164700
   tgttgtttag gatggattgg aaaagagttg gtaggaaggt tgagttttgt gtttataatt 164760
   tggtttggtg gtggttgagg agtttggtag gagtagagtg taggatttgg gaattggggg 164820
   ttggtgtatg tgtgtatgta tgtgtgtgtg tgtgtgtgtg tgtgttgggt gggtagggag 164880
   gaagttgtga aattatattt tttttttttt gttgttgtgt tgttgtgtgt tttagtagta 164940
   tgtgggtgtt attatatttt ttagtaggtg ttaattttta agattgtttt gggttttttt 165000
   tttagttggt tgagttggag gtgggagttt taattgtttt ttgtggtttt tagagtggga 165060
   ttttgttgtg ggataggttg gttaatggtg tttttttttt tgtgattttt ttgttgggtg 165120
   ggttatgagg aaggattgtg ggtgttgttt atagataggt ggatatgtgg taaggatatt 165180
   ttgggatttt ttttttgatg ttttttgaag ggggtatttt tttagttttg agatgagttt 165240
   ttgtggatgt gggaagttta ttattttaag agtagtagtt ttggaaaatt taatatagaa 165300
   ttttgagtag gggtgggaag gggttttgtt ttgtttattg gttgtttggt agagttttgt 165360
   ataaggaagt gtttgtgttg ttgtgggtgg aggaatggat tgagggttat atttgttttt 165420
   tgttgttgtt gtaattgttt attataaatt tagtggttta aagtaataga ggtttttttt 165480
   tttatagtgt taagggttag aagttgatta gttttattgg attaaagtta aggtgttggt 165540
   agaatttatt tttgtttttt ttagttttgg gtgggaggtt ttgttggagt tttttggttt 165600
   gtggttgtat ttttttagtt tttgttttta tttttttata gttttttttt tttttgtagt 165660
   tagatttttt tttgtttttt tttttttttt ttttgagatg gagttattta ggttggagtg 165720
   tagtggtata attttggttt attgtagttt ttgttttttg ggtttaagtg atttttttgt 165780
   tttagttttt tgagtagttg ggattatagg tatgtgttat tatatttggt taatttttgt 165840
   atttttagta gagatagggt tttgttatgt tggttaggtt ggttttgaat ttttgatttt 165900
   aggtgatttg tttgttttag ttttttaaag tgttgggatt gtagttatga gttattatat 165960
   ttggtttgtt ttttttttaa aggatgtttg tgatttgggg tttatttggg taattttttt 166020
   attttaatat ttttagttat atttatagag tttttgttgt tatatgaggt aatatagttt 166080
   gggaagggag agttatttag tttattttag gggtttgtgg tgtattttag ggtttttttg 166140
   attttaagat ataaagtaag aaaataaatt ggtttaaggg gaaaaaagga tatgttgaat 166200
   tttgttgttt taaatgtata tttttttatt gtgttaaaat gtatagaata taaaatttgt 166260
   tattagtaat attgagtata tttatagtgt tgtgtaatta ttagtattgt ttagtgttag 166320
   aattttttta ttattttaaa gggaaatttt gtatttatga aggatttatt ttttatttgt 166380
   tttttttagt ttttggtagt tattagaatg ttttttgttt ttataaattt atttttaata 166440
   agtgtaattt tgtgtgattt taaaataaat aaatatgagt atgatgagtt gtttattgga 166500
   aggatattta tgtggggagg ttggtgtgtg gagtgtgtag gtttttggat gggtaggagt 166560
   tgaaggggtg tggatgtgtt gttttttttt ttttttgttt tttttttggg gttattgttt 166620
   gagtattttt ttttgtaaat ggttttaaat aatgttttag tttttatgtt tgtattgttt 166680
   tttagtttta ggatttttta ttaaaaaata ttttaagttt tagatttatt tttgggaaaa 166740
   ttttaatggt tttatttttt tttttgagtt agttagattt tatggtttgt ggtgggttgt 166800
   ttttgagttt tgagtatttg tgagttaggg aagtaggata ggtatattta gggaagggga 166860
   agagttgttg ttagttatag taattgatat ttttggaatt gtttaagaga tatttagtgt 166920
   gtgtttaaaa tatttatttt tttttttgtt tgttttttga gatgaagttt tgttttgttg 166980
   tttaggttgg agtgtagtgg tgtgatttta gtttattgta attttttttt tttgggttta 167040
   agtgattttt ttgttttaat tttttgagtg gttgggatta taggtatata ttattatgtt 167100
   tggtgaattt ttgtattttt agtagtgatt gggttttatt atgttggtta ggttggtttt 167160
   gaatttttga ttttaggtaa tgtgtttgtt ttggtttttt aaagtgttgg gatgataggt 167220
   gtgagttaat atatttggtt atatttattt tttaggagaa gttttgtatt ttttgaaagt 167280
   gaaatgtttt taggtgggtg atattgatgg tgggatttgt ggttttatta gtgttttatg 167340
   agagatgttg ttgtgtgttt tattaagttg tattttattg ttttttaaat gtggtttgta 167400
   gatatggtgt tgagaatgat ttgggaggtt taaattataa tatggtttaa attaatagtt 167460
   ttttgatgat ttggtattat tttaggatta aattgagatt atttgaggat gggtgtggtg 167520
   gtttatgtgt gtaattttag tattttagga ggttgaggtg ggtggattaa ttgaggtttg 167580
   tagtttgaga ttagtttggt taatatggtg aaattttatt tttattaaaa atataaaaag 167640
   tagttgttgg atatggtggt gtatgtttgt agttttagtt atttgggagg ttgaggtggg 167700
   agaattgttt gaatttagga gatggaggtt gtagtgagtt aagattatgt tattgtgttt 167760
   tagtttgggt gatagagtaa gattttgttt tttggtaatt aaaaaaaaaa aaaaaaattg 167820
   agattttttg agttgtattg tttagtatga tagttattgg ttgtatgggg ttatttgaat 167880
   atttgaaatg tggtgatttt aagtggagag gtgttgtgtg tgtgaagtgt attttgggtt 167940
   ttagagtttt agtaaggggg aaagaaaaga atattaaata tttgttattt ttatattgat 168000
   tatatgttga attgataata ttttgggttt tgttaagtga atgatattaa aattaatatt 168060
   atttgttttt atatttttta atgatttttt tttttttttt tttttttttt ttttttaaat 168120
   atggggtttt atattgttgt ttaggttgga gtgtagtggt atgattttgg tttattgtaa 168180
   tttttgtttt ttaggtttga gtgatttttt tgttttagtt ttttaagtag ttgggattat 168240
   aggtggttgt tattatgttt ggttaatttt tgtattttta gtagagatag ggttttgtta 168300
   tgttggttag gttggttttg aatttttgat tttaggtgat ttgtttattt tagtttttta 168360
   aagtgttggt atgataggtg tgagttattg tgtttggttt gtttttatat tttttattgt 168420
   aggtgttgga aaatgtaagg ttgtgtttgt ggtttatgtg ttgtttttgt tggagggttg 168480
   gattgagggt tagttgagaa ggttttgggg ttgttttatt tgaatgttga gtgtgtgttg 168540
   agtggtagag aattttgaga agtgttgaag gatagaggtt ttgtgtgggg gatgtagttt 168600
   taggttggag atgttttttt tgtgttttgg agatttttat ttggtgatag taagaggaat 168660
   tattgtaaga ggggtggtta ttttagtaaa gtatttagga tgttggtttt ttagtttagg 168720
   aatttagagg tagaggaggg gaggaggttg ggtttggagg agagtagggt tttgggtttt 168780
   gggaaaagtt tggggggtta gaaagttggg ggatagattg aggttagagg taaagagaag 168840
   aaggtttgga gaggagggtt aaggaagaag ggttttgttt atggggaggg agaggtttat 168900
   ggggtagggg tgtggtggga gttgtatttt tgaggttttt tgttgatttt gatatttggt 168960
   tagaaaggag ttttttttgg tattttttgt tagtggtttt tggagttgtt ttttaggggt 169020
   tagtgatgag ggtgttttgg gttgggttgt agatagtgga aaagataggt tttttttatt 169080
   tttagtgttg gggaaattag ttttttggag gaagggggag ttgtattttt tgagtggttg 169140
   tattttttgg tttagatgtg tgtgttttgt tgtaattttg gtgattggtg ttggtggtgt 169200
   ttattttatg gatggggaag gttaaatttt atagatattg agtgataggt ttagggtttt 169260
   ggattttagt gtttgattta gaatttaggt ttttgatttg ttattagttt tttttttatt 169320
   tagatgggaa tagggatttt gggtatgtag ttttttttta gatatttttt tggttttggt 169380
   ttggtgtttg gagattagta gtagtagttt tttatggttg tttattgtga gggtggggag 169440
   ggttgtggga ttaggaggtt ttagggggaa agttggttat gaggagtggg aagtgatggg 169500
   ttagatgtta gtgattgttg gttggggagt tgttggatgt ggttggtttg tttggttagt 169560
   gtggtgggtg ttttggggtt ttagttattt ttagttatgt gtgggggtgt gggatgggaa 169620
   aggtaagagt gtgtggagtt tagttagtat ttaatatgag gttttgtggg ggttgtttga 169680
   gggtgtagtt tttagaattt agtttttggt ttttagaagg tgttattaaa ggtttttttt 169740
   tatttgagtg aatttttttt agtttttagt aagtttttgg aatgagttta tttttaggtg 169800
   ttttttttta gtgtgggagt ggttatgttt ttgttgggag tgatttattt gggtttagag 169860
   gttgtggtat tgagatgggt ttggtagatt ttagtgttta ggtttagttt ttatagtgtt 169920
   agtttttttt tttggggatt tttttgtttg tgtttttttg ggttttagta tattttaggt 169980
   ttgtagggag ggggagagga agagattgat ttattggtta ggttttttag gggttggaga 170040
   ggttggagag gtaggagttg gattagattt gaatttagag gtttttggag gaagagttta 170100
   gggtgagttg tgtttttatt ttttgttttt ttttttttgt tttttttttt ttttatggtt 170160
   ttagttagta agtatttggg gtagagggga taaatttagg tggttgtgtt ttagttttgg 170220
   ttgtaggttt gaatggtttt ttggggtggt tggttatgtt ttttgagagt ttagttgtgg 170280
   tgatgtttga gtaggtaggt gggggagtat ttaggaagta ggggtgttag gtagagtata 170340
   aggagagagg gtgtttaggt tagttttagg atttggttga gaggaggggg ttttttatgg 170400
   gtattgtttt tggtaagtat agggataagg gtaaggatgg tatggttaga ggtttttggg 170460
   agtttttttt tttttttttt ttttagtagt ttttttttat tttttttagg gattttgtta 170520
   ttttttttta gtgtgtggta gttttttggt gttttttttg tgtttttagg ttgtttttgt 170580
   gttgggtttg ttgttgggtg tttttatttt tgtttgtttt ttttttttgt tttttttgtt 170640
   ttgttttttt ggagtaattt tttattgagt tttttttttt aggtagttga ggtttttttt 170700
   tatttttgtt ttgtgttttg ggaggttttt tgttttgtga ttataaagtt tttttgattt 170760
   tttgtttggt tttgagttat gtgatttggt gggtgggttg tggtttttgg tgtgtttagt 170820
   gtagtttgat gttttgttgt tggggtgagt tttgtttttt tgtttttttt agttttgtat 170880
   tattggtttg ggggttttta ggtggtgtgg ttgtgaggtg gattttgatg gttatggtgg 170940
   tggtgttggg agttatgttg tttttgggtt ttggtttgag gttggtagga ttgagtgggg 171000
   tttttaggag aggggtggtg gggagtttga tttttatgtg gggattagat ttttggtttt 171060
   aaaatagaag aatagggttt tgtgtggtta tagttatttt tttgtaaata tttggttaat 171120
   taagttgagt ggttaagttt ttttgttgtt tggagttttt tggaatatgt tttttttttg 171180
   taaggggttt ttttggtttt taggagggtt aggaagaaat ttgaattggt tattgttttt 171240
   tttaaaatta ttttgtttaa gttattattt ttttgggttt ttgaagattg gttggttgga 171300
   ggttggagat agttttaatg tttgaaatgt tgtaattgaa gttttttgtg gtgttggtat 171360
   tgggatttag ggagttgttg ttatagtgta gttttggatt tttggatgtg ttggatatgt 171420
   gtagggtgtt tttgggagga gtggggaggg agggtgttgt tggtggggtt ggtttgtgtg 171480
   tgttttgttt ttttataatg gtatgttgtg tgttggttat gtagaggtat gttagtgagt 171540
   aggggttgag ggattttttt aatggatttg tttttagagg gtttttttat gttgggagaa 171600
   ttttagagat taaattatgt agttaatggg gtggtttttg gttttaaagt agggaggggt 171660
   gaggagtttt gtaggtaatg ttatttgttt ttgaaatgtt gttttagtga ttttggggat 171720
   tgatttagtt tttagtttgt tgtattttat ttttggtttt tttttttttg tttttttatg 171780
   tgaaatttgt tgtgttttgg ttagaggttt ggggaatagt tttttgttta tttaagataa 171840
   gtttgtaatt ttttttttgg agaaaaatat gttatttgga agtaggttga gtagtggttt 171900
   tttggtaggt ttgtttgggg ttgtggagat agtatttgtt ggattaggat ggagttggaa 171960
   tttggtttgg attttatatg agaaaatttg ttggaagagg aggaagtaga aaaaggtttt 172020
   attttttaat aatgttgtgg gttttttttt tttatttatt gttattttgt tttgaaattt 172080
   aatggggtta tgtgtgtttt tatgaatgtt attttttttg gttaattttt ttggtagttt 172140
   taggagtttt tttttaggaa aaggaagaag gtgtttgttt agtatttagt atatgagttt 172200
   ttgttggaaa ggtgggattt ttgttttttt ttgggttttt tttaggttga gatgtggtta 172260
   aggaaggttt gtggtgtggt ttgttttttt taatgtttgt ttttggagat ttgagttgtt 172320
   gttgattttt agtttgtttt ttttgttttt tggttatttt tgtgttgggg tggttgagat 172380
   attattgtat tggttatttt gtgtttttat tagtttttgt ttttgtaggt tgttattgtg 172440
   atggtgatgg ggttaattta gttgtggtgg ggaggattta ttgtgtgtgt ttggggggtg 172500
   gtgtggggta ttttgggatg agaagatgtg atttttgttt tgagagtttt gttttaatgg 172560
   aaggtggggg tatagttgta ggtagaagtt attaagtatt gtggttagat tagtatttta 172620
   tgttaaaaat ggttttgttg gttgggtgta gtggtttatg tttgtaattt taatatttta 172680
   ggaggttaag gtaggtagat tatttgaggt taagagtttg agattagttt ggttaatatg 172740
   gtgaaatttt gtttttatta aaaatattgg ttaatttggg tatggtggtg tgtgtttgta 172800
   atggtagtta tttgggaggt tgagatagga gaattatttg aatttgggag gtggaggttg 172860
   tagtgagttg agattgtgtt attgtatttt agtttgggta ataagagtga aattttgttt 172920
   taaaaaaaaa aaaaaaaagt tttatttggg tttgggattt gttttttgaa tattgggttt 172980
   attgtgtttt ataaggattt ttattttgga agttttagat ttatttgggt aattagagtt 173040
   tttgggttga ttatttttta gttttggtta gttttttggg ttattgtatt tttttaattt 173100
   tttgttgtgt tatggttggt ttaagttgtt tttattagat aatgtgaggt atttttatag 173160
   gaggggataa ttttgttttt aggagttttt tttttttgtt tttaggtttg gtgtagtgtg 173220
   tggttttttt gttggtgtta aaggttttaa ggttttttta agaagtttgt tattattatt 173280
   agtagttttt gttatttggt ttttttgtat atgtaagttt tttttatata ttttggatgt 173340
   ttagggtttt tgtttttttt ttttggttgg gagtgtggga agagggtttt attttaatgt 173400
   ttgagagtag ggttaatggt tagggtaagt tgtgggggtt tttttttgtt ggtttttttt 173460
   tgtagttttt attttaatat agagtattga tattatttgg gttttgggaa gatttggagg 173520
   ttgattattt tggaggtttt tagtgtttta tagttatttt ttggggaaga ggttttttga 173580
   gggatttgtg gtttttggtt gggattttag ttttagagtt ttgttttttg gtgttgagtt 173640
   ttgatttgtt tattgagtgt tagtttggtt tttggatagg tagggatttg tttttggagt 173700
   ttagtagagt tttgggaagt ttttttagga gggtttttga gggtggtggt tatgtttagt 173760
   ttgttgtatg tgtgagtttg tttgatgagg aattggttgg gaaggtgtgg gtttaattat 173820
   aggtttatga gggttttttt tgtttttatt taggaagtag tggagatttt ggtatattta 173880
   gtatttgggt ttgtgtttgt ttagttgttg gtggaattat gggagttttg ttttggggtt 173940
   tgtagagtag tagtgggaat tttttttatt ttagtttttt ttagagttgg ttgtttaaat 174000
   ttgttgttgt ttagtttttt tttttttttt tttattaatt ttgggaattg tatattgttt 174060
   attttattgg gttttggagt gggtgtagga agagagagtt taatatttag aggtttgggt 174120
   tttttgtagg tgtttgagtt gttgtggttt gagttgggtt ttttgttgga attattatag 174180
   tagatttaat gatttgtttg taggagttgt tttattttag ttgattttgg ggttgtggga 174240
   aggggttggg ttttggtttt gttgggggtg gggggttagt gtaaagggtt tgaaaagtgg 174300
   tagaggtgat ggtttatttg tttttttttt ggttggttgg tttaaatgtg attggttttt 174360
   tttttttttt tttttttttt tttttttttt tataaataat attaaatgtg atttgttaat 174420
   gtatttattt gggtttagta gttggattat gggtagaagt attttagatt tgttattttt 174480
   tttaagttaa gggtggggaa tattttttgt gagattttgg tttttgagtg tttaagattg 174540
   tttttggaat tttaattttg ggttggtttt aggggattga tgttgatgga gttgttgata 174600
   ttgatttttg gtttttgtat ggtttttagg aagggggttt tttgtttttt gttttgttga 174660
   gtttggttgg tagtattttt tttttaggtt tgtgtttttg gtagggtttg ttgtttttgt 174720
   tttgttttgt gggtgttagg ttttaagtat taggtgattt ggttattgaa tggttattga 174780
   atagttattt ttgttggtgt ttttagtgga atgtgtatgg atgttttttt ttttttagat 174840
   ttttgttttt tattttttag tttttgtttt aggggttatt ataggattag gaggtatgta 174900
   gatgtgggtg gtatatttat tgggtagatt tagttttgaa tttgtatgtt ttttttaagg 174960
   gggtttttag tgggtggggt tgatggtgtt gttttttttt ttgttattag aatggatatg 175020
   aggattgtgt tttgttgatt tatgttttga gtttttagat gttataggtg tttgtttaat 175080
   gagtgtttga atgaatggtt agtattgtgt gattgtagat ttttaatttt tatttaaata 175140
   gtttgtggtt tttgtgaggt tattttgaga tagttttata agaattttta ttttttttgt 175200
   atgttttgta taaggttttt ttggggggtt ttttagaaaa agggttaaag agtagtattt 175260
   ttttggggat ttgaatatga ttttttttgg ggtaggggat tgttttggaa gatttttggt 175320
   attgtttttt atgtgttatg tgattaggag ggttttgggg taggatggtg ttttttgtgt 175380
   tttgtgtgtt ggggttggag gaaatattgt ttgtggttgg tgttatttgt tagttttgaa 175440
   gattaatgat gggttggata gtggttttat tgagtgggag ggagaattgt tgttgtttgt 175500
   agttgggggg ttgggtgggt gggagttgag gttagggaga atagtgtaga tttggtatgg 175560
   agaggtttgg aggaggagta gggggtttta gttatttagg gtgtagggat gttatatgag 175620
   gagtaggtgt ggtgtgtgtt tttttgtggg ttttttattt tttggttttg ttatttgtat 175680
   taggttttgt ggtgagaggg tatagagtgg aggaaggtgt tgtggttagg gtaggttata 175740
   tgttgtgtta atggattgtt gttatttgtt gtttttgtgg ttttgttgtt aggatttttt 175800
   agtattaggt ttttattatt aggtatgggt tttataggta ttattggttt tttgaagtta 175860
   gggatagatt ttatagataa tattgagttt ttgtagttag ggatgggttt ttatggttag 175920
   tattagattt tagggattag tattgggtat ttatagttag ggataggttt ttatggttag 175980
   tattaggttt tagggattag tattgggtat ttatagttag ggatgggttt ttatggttag 176040
   tattagattt tagggattag tattgggtat ttatagttag ggatgggttt ttatggttag 176100
   tattaggttt tagggattag tgttgggttt ttatagttaa ggataagttt ttatggttaa 176160
   tattagattt tagtgattag tattagatgt ttatagttag agatgggttt ttatggttag 176220
   tattaggttt tagggattag tgttgggatt ttggatgatt gaaggagggt atggttgatt 176280
   aaagttgtag gagttagttt ggttattttg aggtattaag ttattattgt ggttttaggg 176340
   gatttagatt tttttatttg tttttttgaa ttttggaatt agatatattt gggtttagtt 176400
   tttgtttttg gggttgtgtg tttgggatgg ttgttttatt tttttgaagt ttagtgagat 176460
   ggggatggtt gtatagggtt tggtatagat ggtatttgat ggtatagggt tatttgtttg 176520
   tatttatata gttttggttt ttgttttggt tgtgtattga gagttttatg tttagatttt 176580
   tgttgagttt ttaagttttt ggttatgttt taggtttttt gtgagaagta ttattgtatg 176640
   gattatgttt tgggatttta ttttgggttg ggaagtttgt tttatttttt taagtttttt 176700
   ttagaggtgt tttatgtttt attattttta ttgtttgagg ggttagatag gggaatttag 176760
   tgtgggaggt ttagaggtgt tggagtagat gttaagtgag ttttgtgttt atgtgttggg 176820
   ttttggtaga tattttgaat gagttttttt tttaggaagg agattggggt tttagtttaa 176880
   gttgtggttt taaattttgt gattaagagt ggagaaaagg taagattttt ttttttagtt 176940
   ttttagaata gttgatagtg gtggaaatat ggggtttata ttgaattttt ttgtaatttg 177000
   ttgaagagaa tttagtttat atttttaggg gtggaggttt ggttgatttg gtagtgtgtt 177060
   tgtttttttg gttatagttt attttggatt gggtattttg gtgttttttt ttagtgatgt 177120
   agtgaagtta ttatgatgtg tttagttggt gagaaagtgt tttttgtaat gttttatttt 177180
   ttttagaggg tgaataagtt ttgtttatta taaataatta tttatagaat atatgaggtt 177240
   taatttaatt ttgggttatg gtagtgtttt ttgggtaaat ttatgtgtta ttttagttag 177300
   agtttgtgtt tttataggtt ttttggattt attttgaagg ggggttggat tttttgtttg 177360
   ttttgatgag agggttgtag gatagttttt agtgagtttt tttgagggtt gtaatttttt 177420
   tttttggtat ttttttgttt gttaggaatg ttttaagttt ttagtagaag tttatttatt 177480
   atagttgttt tttattttgt ttattatttt ttttgttttt atttatttag tagattttgt 177540
   gtatagttta gtgtaggatt tagggtggga aggtgttttg ggtgggttgg agtaaggttt 177600
   ttagttaggg tggggtttta aggtgggttt atagttagga ttgtgatttg ggaggttttt 177660
   ttttgttttg ttttagtttt ggtattgttg ttgtttttgt gtttagtgga gatttattgg 177720
   ttttaggttt taaaatttta gtgaggtttg tagataggga ggagttttat ttgagggtag 177780
   agtgattgtt gggtttattt gtttgttatg aattatgtgg ttttagattt gttatttttt 177840
   tttttttaga gttatggttt tttgtttgaa aaatggataa tgatggttat tatgttaggg 177900
   attattttga ggataaatgg ggatgaaatt gaagaggttt agtatggaat ttggatatag 177960
   aagtgttaat tgtgtgttat tagttgtggt ggttgttaag gtttaattat atggtagtta 178020
   tgggttgagt ggtgggtggg aagtggggag taggagtatg taagatgggt atttttgaag 178080
   tttttgattt agtgagataa gtatatattt agttttaaga tggagtgttt taggaaggtg 178140
   gagattgtaa aggttttgtg gtgtaggagt gtgagttgta tttttgggtt ttttagaatt 178200
   ttattagagt ttagggagga ggttagttta gatatagggg taatgggaga gatgggagtg 178260
   agaaaatggt ttggtgtttg gaagtggagt ttggaaaggt gggtgtgggg atagaggagg 178320
   gttttggggg ttatgggttt atgtgtttag gaggtggagg tagtgaatgg ggttatggtt 178380
   ggttttatat gtggtattat tagttttgtt gagtttgtat ttgtttttta ggttttttga 178440
   gtgttattat tatttggtat tttttggttt gatttttgtt ttttgtttgt ttgggaagag 178500
   tgagattatt tgtttaaagt tttttagttg tttttaatta ttttggttgt tttttggttt 178560
   tgtttagggt tagttgttga tttgaattgt ttggtgttat ttttttagat ttttggggtt 178620
   ttgttttggt attaatagtt atttttattt ttattattta tttgtatatt tatttagtag 178680
   tgttttttgg gtatttattt agtaatatta aggtgttgag atgtggttgt ggttaagatt 178740
   tgggtttggt ttgtgaggat tttgtagttg ttattttttt agagtatagt tttagttagg 178800
   tatattgtgg ggtttagaaa gagaaattaa tatattgtgt ttgtttattt tgagttgtta 178860
   taaggaatag ttgaggttgg ggggttattt gatttatggt tttgtaggtt gtataagtgg 178920
   tgttagtatt tgtttggttt ttggggaggt tttaggaagt ttttttttat ggtagaaggt 178980
   gaagggggag ttggtatgtt atatggtgag agagagaatt tttagatttt tttttttttt 179040
   gaaatggagt tttgttgtgt tagttaggtt ggagtgtagt ggtataattt ttggtttatt 179100
   gtaattttag ttttttgagt agttgggatt ataggtgtat gttattgtgt ttggttaatt 179160
   tttttgtatt tttattagag atggggtttt attatgttgg ttgggttggt tttaaatttt 179220
   taattttgtg atttatttat tttggttttt taaagtgttg ggattatagg tgtgagttat 179280
   tttatttagt ttttttagat tttatagtaa ttagattttt tggattttgt gattgtgggg 179340
   agggtattaa attatttatg agggatttat ttttatgatt taagttttat tttttttagg 179400
   ttttattttt aatattaggg attatatttt agtatgagat ttggagggga taatgtttaa 179460
   attatattat atgtgatttt tttgtgtgtg tgttagtttt tatatttggt taagtagtag 179520
   aagttttttt tttgtttttt gttgagagag tggtggggga gaaatgattt agggatggga 179580
   ggttggtttt ttgaggggtt ttggtgtttg tggagtggga gtgtgggaag gtttttatgt 179640
   tgtgtgtgtt gtgttgggtt ggttttttat agtttattgt ggtgtttttt gaggattgtt 179700
   ggggaggaaa gagttaatta atttggggat ttagttgttg aagttattat taaatttgag 179760
   tagtttaggg gttttttttt ttttgggttt tatgttattt agtatagtgg ggtttaattg 179820
   aatttttttt tattttttaa ggtttttggt tggtgtggtt ggggtttggg atatggggag 179880
   tgagagggtt tgggtttttt ttagatttgg agtgggttta ttagaaatgg tttaggaaat 179940
   tgtgggggtt atttttagta gttgggggtt tttgtggtta tttttttttg gttttgttga 180000
   gtagttttgg tttttggagt ttttagtaag ggatgtgggg gtttgttagg ttagggtttt 180060
   tattgttgtt gttatgtata gttgtatttt gtatttgggg agtggggtgt tgagtggggt 180120
   gtttgggagg taggtttttt tttgtttttt ttgtttagag attttgggga tgagttgagt 180180
   gagatttttt tggtttttga ttggttgtgt ttttgttgtt attttttatt ttttttaagt 180240
   ggaggtttat gatttttttt tttatttaaa agggttattt agtgatttag tagattttag 180300
   tttgtgttat ttagggtttt tttaaaaatt tgtggggagg ttgggtttgg tggtttatat 180360
   ttttaatttt agtattttgg gaggttgagg tgggtggatt atttgaagtt aggagtttga 180420
   gattagtttg gttaatatgg agaaatttta tttttagtaa aaatataaaa aattggttgg 180480
   gtgtggtggt atgtgtttgt aattttagtt atttgggagg ttgaggtagg agaattattt 180540
   aaatttagga ggtggaggtt gtagtgagtt gagattatat tattgtattt tagtttggat 180600
   gatagagtga gattttgttt taaaaaaata aaaataaata aataaaaata aaaatttatg 180660
   gggggatttt tgtgtttatt ggttggtgtg aggagagggt gaggagttgt agagaaggta 180720
   ttaggttgag gattgttttt atttttgggt tatagaattg aaggagggga tatatgaaag 180780
   atgtttttag atttgggttg gaggttgggg atatttgagg gtaaggatga tgttgttttt 180840
   gtttttgttt gtagaggagt gtatagggtt tattagtttt ttgttgttgt ttaagtgata 180900
   gttttatgga gatagaattt atatattgtg atgttgattt ttttaaaatt tatagtttaa 180960
   tggttttgtt gtatttataa agtgattgta ttagggttat ttagagaaat agaattaata 181020
   gaaggtgtgt atgtgtgttt agataaagag atttattatt atgaggagtt ggtttatttt 181080
   attatagagg ttggtaagtg ttaagattgg tagagtaaat tagtaggttg gagatatggg 181140
   agagtttatg ttgtggtttt ggtgtttatt tgaaggttta agatttagga gagttaatgg 181200
   tgttgtttta gttttgagat ttaggaaaag tgtatgtgtt agtttgagtt tgaagtagga 181260
   aaaatttgat gttttaggtt gaaggtggtt aagtgggaag aatttttttt tatttaggtt 181320
   ttgtgttttt ttatttagat ttttaggttt ttgttataaa attataaaga tttgattaga 181380
   ataaaatttg agaaaaaatt tttttttttt attggttttg gatttattgg ttgggatttt 181440
   ggaaattaga ttgatagaga ttaataggaa ataagtataa taatttaagt aagttttatt 181500
   tgatattgag tttttataag gaaatgaaga tttagagaaa ttgttagttt gagtggtttt 181560
   tgttttgttt tgagataaag ttttattttg ttgtatgttt aggttggaga gtagtggttt 181620
   gattatggtt tattgtagtt ttgatttttt gggtttaagt tggggtttag ttgttggttt 181680
   tgggagttgt tttgaattta tttttaagtt ttagtttaga tttttaagta gttgggatta 181740
   taggtatgtg ttattttgtt tggttaattt tttatttttt gtagagatag ggttttatta 181800
   tgttgtttgg gttggtttta aatttttggg tttgagtaat ttttttgttt tggtttttta 181860
   aagtgttggg gttataggtg tgagttattg tgtttggttt aggtttgaat agtttttatt 181920
   tgttttgttt gttttttaaa tttgtttgtt gtttgttttg agtagttttt ttgtttattt 181980
   tttttgtttg ttttttgttt aggttggagt gtagtggtat gatttttgtt tattgtaatt 182040
   tttgtttttt gggtttaagt tatttttatg ttttagtttt ttaagtagtt gtgattatag 182100
   gtgtgtgtta ttatatttgg ttgatttttg tatttttagt agggatgggg ttttattatg 182160
   ttggttaggt tgttttttaa tttttgattt taagtgattt ggttattttg gttttttaaa 182220
   gtgttgggat tgtaggtgtg agttgttgtg tttagtttag gtttgagtgt ttttatgttg 182280
   ggtttgatga agagtggaaa gttgtgggaa attgtgatag agtaataaaa gatgagttga 182340
   atagtgagtt tgtgggggtt ttggtaagat ttgtgtattg gggttttttt tagatttttt 182400
   gtttttggag atgaggttgt ttttttttgt tgggtgtagg gagggtattt tttatgtgag 182460
   gggtttatga tttattttag agaaggggta ggttagtgag ttttttttgg atttgttatt 182520
   ttttaaattt ttttagttga aaatatttat tatgttaagg tattgtattt tggagtgtgt 182580
   gttttaagtt ttgttgtttt taattgattg ggtgaggttt atttttatta gagtatatag 182640
   tttgttttat ttaggttttt gattgtattt tttttttgtt tgttttgaga atatttatta 182700
   gtagtgtttg tggttgtagt gtttatttta agataaattt gttgggaagt attttgtttt 182760
   tattattatt tttatattat tttagtatat tgattttgga aataaaagat attattttgt 182820
   ttatagtatt ttatttttag tagtggtatt ttttttaaaa aaaaatggta atttttagtt 182880
   gggtgtggtg gtttatgttt gtaattttag tatttgggaa agttgaggta ggttgattat 182940
   ttgaggttag gagttggaga ttagtttggt taatatggtg aaattttatt tttattaaaa 183000
   atataaaaat tagttaggtg tggtggtagg tatttgtaat tttagttatt tgggaggttg 183060
   aggttggaga attgtttgaa tttgggaggt agaggttgta gtgagttgag attgtgttat 183120
   tgtattttag tttggatgat agagtaagat tttattttaa aaaataaata aataaataaa 183180
   atttaaaaaa aaatagtaat ttttgattgt tgaaaatgtt aaattttaga gaatatagta 183240
   tttttataag tgatgttaat attgtttttg attagttgtt ggttgaagat ttatttgatg 183300
   aatttgattt ttttagaata gatgatttag atgattttgt tgttttgttt agaaataatt 183360
   gtaggaataa tttaaaaaat tttttttttt tttaattttt ttgttttttg agatggattt 183420
   tttttgttgt ttaggttgga gtgtagtggt atgattttgg tttattgtaa tttttgtttt 183480
   ttgggtttaa gtgatttttt agttttagtt ttttgagttt agttgggatt ataaggtgtt 183540
   tgttattata tgtggttaat tttttgtatt tttagtagag atggtgtttt attaggttga 183600
   ttaggttagt tttgaatttt tgattttaaa taattggttt atgttggttt tttaaagtgt 183660
   tgggattata ggtgtgagtt attatgtttg gttagttttt atatttaatt tttatataga 183720
   gtatttagtt agatttgttt tagttttaaa gagtgtgttt atgtaaaatt aagtgagtgt 183780
   tggtagtgag ttgaattttt taaaaggtaa aagggttaaa tattaaattt atttaaaaat 183840
   atttttatag atatatttag aataatgttt gattggttat ttaggtgttt tatagtttag 183900
   tttagttgat atagaaattt tttttttttt ttttgagatg gagttttgtt ttgttgttta 183960
   ggttggagtg tagtggtgtg attttggttt attgtaagtt ttgtttttta ggtttatgtt 184020
   atttttttgt tttagttttt tgagtagttg ggattatagg tgtttattat tatatttggt 184080
   taattttttt ttttgtattt ttagtagaga tggggtttta ttgtggtttt aattttttga 184140
   ttttgtgatt tatttgtttt agttttttaa agtgttggga ttataggtat gagttattgt 184200
   gtttggttga tataggaaat taattattat agttatatag ttattgttat taattttagt 184260
   atattttatt atttttaaaa gagattttgt ttttattagt taatttttat ttttaatttt 184320
   gggtttttag tttttagtta ttgtttattt gtttttgttt ttatgaattt atttgtttta 184380
   gatagtttgt ataagtggaa ttgtatagta tgtggttttg gtgtttggtt ttttttattt 184440
   ggtagaatat ttttaaggtt tatttatatt gtagtaggtg ttggtgtttt attttttttt 184500
   atggttgaat aatattttat tgtggataga ttatattgtg tattttttta tttgttgatg 184560
   gatatttggg ttgtttttgt tttttggtat aattttgttg tgaagaattt tgtagtgagt 184620
   gttggtttat aggtttttgt gtgaatatgt ttttattttt tttgggtaat tgtaagatta 184680
   tgtggtttag ttaattagtt tttaagttat gtgtttgttg agggatttag gtttggtggg 184740
   ggtgtattgt tgtgtttggt tagttaggtg ttgtgagttt gtgttgtagt tatttttttg 184800
   tgttgtgttt tgaggttgtt ttgttggtat tgggtattgt gagttttagg aggttaaagt 184860
   tttttttgtt ttattttgag tttaggtttt gttttatgaa ttaggatgaa tggatattga 184920
   attaatgtag ggatgggtgg ggttggggtg gggatgtaga gtgattttgg gggtttgtag 184980
   ttgggttttg gttgttgatt tttagttttt tttgtttgtt ggagtttaga ataagttaga 185040
   aaggggaaat gtaaagtttg agtgtttttt ttgtggtaga ttggtaggtt tgggtgggtg 185100
   agttatttgt agttagttgt agtgtgagag aggaagtggg aggggaggga gagggtgagt 185160
   ttggtgtaat ttatttttgg aaggaggttg ggttgaggtt gggggaagtg atttttattt 185220
   ttttgagagg attttttttt ggggttaatt ttaaattaag ggagataaga tttttttggg 185280
   ggaggttgga agtgttgggg tttttaggtg ggtaggtttt ttagggttaa tgttttgggg 185340
   aagttataag ttattatgtt tttggattgt tatatatata tatatatata tatatatata 185400
   tatatatata tatatatata tatgagtaaa ttgtaatata aatgtgtgtt tatgaaagtg 185460
   tttattttaa attattatat agtttgtaaa gtgaaggttt tggtttgtat ttgggtattt 185520
   attgtttagt tagttggtag gtagtttgta ttgtagtttg ttattatttt ttagagatgt 185580
   agttgaggag tttagttggt ttggagggtt tagtgggtgt agttttagga aagtggttag 185640
   gtttgggtta ggtttttttt tttttttagt tttgggagaa attattatat gaatgtagtt 185700
   tttttttgga gtttgggttg gaagggtttg ggaggggata ggtttttatt ttaggagttt 185760
   ttaggttttg gttgttgttt gtgttagttg tgtgagtgtg agtgagttag gggttattat 185820
   tgggtggaag gaagaagtgg gtattttttt taggaggtta ttgttggagt tttatagtag 185880
   gttaatttta ggttttgggt ttttgggagg aggagaaaga agagaagtag gaggtgtgga 185940
   gggtaggtta ggattagaag gtatataggg ttggggtagt gtgggagatt ttgggggttt 186000
   ttttatttaa ttagttaata ttatagtgtt tataagaata tgttattatt gtagttttat 186060
   tattttgttt tgattttaga aggtataatt gatgatttta gggaatggag aatttgagga 186120
   aggttttgta ggagagggtt agatgtgatt gagtaaatgt gagttgtttg taaattgtag 186180
   agtgtatgta tagtattggt tagttttggg gttagtgttt ttttggtgtt tagtttggtt 186240
   tttgtatttt ttagtgtttg gttgggtttt gtgggggtta gggttgggtt tggtatatta 186300
   gaggattttt attaagtttt tggtagtgtt ttagttatta aatttagttt gttagttatt 186360
   aaatttgttt tgttggtttt tgaattattt ttttgttggg gttggttatg ttatgttagg 186420
   ttttttatgt aggatggtag atggatagat gttgggatta agttattttt gggttgtgag 186480
   atgtttgatt ttttgttggt gttttagaag atgttagttt tttgtggtat gttttttatg 186540
   tggtgtttgg ggattagtgg tttttaaggg gtttttttgt tttagtgtgg ggagtggttt 186600
   ggttaaggtt ttagtgtttg gttggagtta ggtataggtt taggtgtatt ggttggaggt 186660
   tttttgttta tgggttttgt ttgttttgtg tttttggttt ttattgtgtt ttttgtgttt 186720
   ttgggagaga ttttaggggt tttgagttta gtattggggt tggggagtgg gtggggaaga 186780
   gggtggggat tagggttttt ggttgtagaa tagttaggtt ttaagttttt attttatttt 186840
   ttttttattg ggtgttttat tttatattga ttattttttg aagaaaattt tttgaattgg 186900
   ttttttattt tggttgtttt aggaagttta tttatttttt tgttttaatg ttttgtggtt 186960
   ttagaaggat ggtggaagtt ggaaattatg agttttattt ataggtggtt atatattgaa 187020
   gttgtggaga tgttgtggga aaggattttg gttagtgtgg ttggtataga gggagtggtg 187080
   ttattggggt agatgggaat tgtggttgtt tttatgttgg tttgggagag atgaggggtt 187140
   ggatgaaagg atagtagatt ggttatttat ttatttattt atttatttat ttatagtgtt 187200
   ttattgggtg ttttggatgt gtaaggtttt gtgggtagtg aatgtgatag attttagata 187260
   gtaattggag ggttatgtgt gtaagaattg agggatttag gttgggtgtg gtgttttaag 187320
   tttgtaattt tagtattttg ggaggtagag gtgggtggat tatgaggtta ggagattgag 187380
   attattttgg ttaatatggt gaaattttgt ttttattaaa aatataaaaa attagttggg 187440
   tgtggtggtg ggtgtttgta attttagtta tttaggaggt tgaggtagga gaatggtgtg 187500
   aatttgggag gtagagtttg tagtgagttt agatagtgtt attgtatttt agtttgggtg 187560
   atagagtgag attttatttt aaaaaaaata aaataaaata aaataaaata aaaattgagg 187620
   aatttagtgt agatgagtta ttttgggaaa ataagtggga gttttaggaa tagtatgggg 187680
   aagggatggt tttggtaggg aggatttttg tgggtagttt tgttggaaga gttgaaaggt 187740
   agttagagag gttgtgtgtg gatggaagtt gaagagtagg ttaggggtgt gtgttttgtt 187800
   aataaagatt ttaagtattt attgattagg tataatatta tgtgttttat aaataaatat 187860
   tttatttgat ttttagtatt tttatgagat agatgttatt attttatttt atatgtaaga 187920
   atattgaggt atagagtggt taagttattt gtttagggtt atatagtatg ttggtggtag 187980
   gtaggatttt ggagttatag gaatttggta tattttaggt gtaatggaaa ttagttatgg 188040
   ttttatagag ggaaattatt agattagtaa attttatata tgttttttgg tgttttgagg 188100
   aggagggatt tggatagggg tagttaatgg tggtggttgt gagatgtgtt gggttaatat 188160
   tgatgagagt ttgggatgga tgagtggatt tggagtaatg gaaatggagg gtttaggttt 188220
   ggtttttgga gatttagttg taatagttgt tggttggaga ggttgtttat ttagatgggg 188280
   gatggggtgg gaggtaggtg agatgagagt tttagagttt agtttggggt atggttattg 188340
   ggtttaagat gtttttagat gttggggttt gatagtgata gtggttgtag atatttagga 188400
   tttagtagag ggtttggtat agtggggttt agggatatgt ttgtgagtta ggttggtatt 188460
   taagtgtgaa tttggataaa gaaggtggta ttgttggtat tgaatgttta ggggttgggt 188520
   gaagttagtt agggagaggg gtttttaagg tattaggatt gattttgtgg gtgagggtgg 188580
   aggaggagat ggtagaggtt gagagaatag ttagagatag gaggaaggtt agtagagtta 188640
   tgtatagaag tttggagaag atttgaggaa aggaagggtt gggtggttgt tattttttgt 188700
   agttaggagg taagggtgat ttttgtgtgt tttatttttt agaggattgg tggttttatt 188760
   tggttagaat tttaagttta gagatgtatt tgagttttat gttatggtgt aggtagtagg 188820
   agttatggag taggtttgtg gggttatggt ttgtttgttg ttttttggtg tttttatttt 188880
   gagtttaggg gttgtttgga tgttttgtgt tgtatttttt taggtggtgt tttagagagg 188940
   atgaaagtgg ttattttaag gtgtgattag gtagtggttt atgtatattg tttttttttg 189000
   gggagagttt gggatggaag ggtatgtgtg tgtttttgag attttttgaa taggttttat 189060
   gtatttattt atttatttta ttatttattt atttatttat ttatttattt atttatgtat 189120
   ttatttattt atttatttat ttatttattt gttaatttat ttatttattt atttatttat 189180
   ttatttattt atttatttat agttatttat ttatttattt atttatttat ttatttattt 189240
   atttatttat ttatttattt gtttatttat ttatttattt atttatttat ttatttattt 189300
   atttatttat ttatgtattt atttatttat ttatttattt atttatttat ttatttattt 189360
   atttatttat ttatttattt atttatttat ttatttattt atttatttat ttatttattt 189420
   atttatttat ttatttgttt atttatttat ttatttattt atttatttat ttatttattt 189480
   atttatttaa ttatttattt atttatttat ttatttatta tttgtttatt tatttattta 189540
   tttatttatg tatttattta tttatttatt tatttattta tttgtttatt tatttattta 189600
   tttatttatt tatttattta tttatttatt tatttattta tttatttttt tatttattta 189660
   tttatttatt tatttattta tttatttatt tatttattta tttgtttatt tatttattta 189720
   tgtatttatt tatttattta tttatttatt tatttattta tttatttatt tgtttattta 189780
   tttatttatt tatttattta tttatttatt tatttattta tttatttatt tatttattta 189840
   tttatttatt tatttattaa tttatttatt tatttattta tttatttatt tatttattta 189900
   gttatttatt tatttgttta tttatttgtt aatttattta tttatttgtt tgtttattta 189960
   tttatttatt tatttattta tttgtttatt tatttattta tttatttatt tatttatata 190020
   tttatatatt tatttattta tttaattatt tatttttatt tttattttag aaagtatttg 190080
   aagttgttgg ggtgtgtatt tggaaaatgt gttataagtt tttataggtt tttttttggt 190140
   ttttttattt ttttgattgg tttttttttt ggatatgtag gattttgagt agtttttttg 190200
   ggaggtgggt gtagtttagg taggttgttt gtaaagttag gattgaggga gttatattta 190260
   aggtattgtg ggtttttaag gtgatggtat tgtgggttta ttgtttttag atttttttaa 190320
   tttttaggtt tttgagtagg aggatttttg gggtagggtt tggagatggg aagagaaaat 190380
   aattttagtt ttttaatttt tgttagttta ggtgggtgtt gtatggattg ggggtatttg 190440
   gaatagttgg tttttttatt tttttttttt atttttttag tttttttggt tataattaga 190500
   tagttgtgtg tgatgttttt tttttttttg aaggggttat ttagttaggt ttttatttat 190560
   ggtttaggaa tgtgtttttt tttgtttgtt ttttattttt aagtaggttg tgtttttagt 190620
   tttttgttgt ttttgaaggg tttgggtatg gggtagtttt tgtagtattt ttagggtttg 190680
   agttgatttg tatagtagaa gggaattggg ggaaagtttt ttttttattt agtatttggt 190740
   agggggttat tttgttgtat tttagagatt tttgtttaag ttttgttatt ttagatgatt 190800
   ttataagttg ggggaggtgg agttttagtt tttatagttt ttgttgttgt tttattgttg 190860
   agttttgttg tgtatttgtt gataggtttg ttgatagtag aaggggttgg agagagggaa 190920
   gttttgggtt ttgttttttt taatttttgt ttaggggata tttttgataa gaggaaattt 190980
   tagtttgtga tagatggtta gtgtttattt atttatgggg gaattttttt ttttttttgt 191040
   aagtttgtag atttgttagg aattaaagga atttttggtt gataggttat tgtggatatg 191100
   gttggagggt tggtatgggt tggattttgg ggttgttttg aggtaggggt tgtttttgtt 191160
   ggaaggtttt ttggggtggg gaaggtatta gggttatttt ttgatttttt ttttgtagtg 191220
   gatttatttt agagtttgag ataaggtaag ggggtagtag aaaatagaat ataaaatagt 191280
   tttttttatt atatattgta ttagaagtta gaggatttat ttttggtttg ttttttttgt 191340
   agtttaagtt ttgagagtgg ttttttagga agtgaatggt gatggtttgt aggttagagt 191400
   ttgttttggt ttaagattga tgttttaata ggttgtggag tttgggtgaa aaaatttagg 191460
   ttagggaggt ggtgggttgt aaatgaaaga atagaggagg gtttttttta gaaagtggtg 191520
   ggtggtgtga ggagtttttg gattttgttt aaatttgatt ttattgattg gggttaattg 191580
   aaattgtgtt ggtagaaagg taaaattgtt tgagattttt ttttttataa tagaggagag 191640
   gtttgtttga aagggtttgg tgagggaaaa gttggaggaa ttttggttgg tagtaaaagt 191700
   ggggattttt gttttttgtt ttgggggtgt tttggttttt gaatgaatga aagaggaggt 191760
   tggatggaga tagatttata ttatttgttt tttttaggtt ttgtaaagga gtatataggg 191820
   tttttgttgg tttttttatt ttgttagtta ttaagaagga gaaaatatta ataggggaat 191880
   tgtaaagttg tagttttgtt taagttttat agtggaaatt ggaaagagtt ttagtttgga 191940
   gaaaaatgta gttttttgtt tttgtaaatt tgtgtttttt tttgggaggt tagagttatt 192000
   gtgtttgatt tttaagtagg gttttagagt gttttaggag gggaaggtag agttttagat 192060
   ttttggtatt ttaggtttta attaggtttt gaggagatgt tttttgtttt atataaagtg 192120
   tgaagtgtaa tttgatttgg agttagttaa taaattagat gagttgtgtg tattttaaaa 192180
   atttagtttt tgatttttta tttatatata tttgtataat ttggttattt atatttagtg 192240
   tttttatttt tagaagagaa taggaagttt ttagtgatat ttatggagtg tttgtagtag 192300
   tttgggtagg ttagtaggaa tataatgtag tgaaaagtag agttgagatg ggaaaatatt 192360
   ttgtttttag agttttttag ggatgatatt ttttattgtt tttgtttatt tgttttggag 192420
   tttattttgt aaatagtaat ttgtttttat ttgagagttt ttttttgttg gagtttaggg 192480
   atggagagta gttggtttta gttttttttt tttatttttt tgttttttag atttattttt 192540
   tattttgaat aattgttttt aatttgtttt tttttttttt tagttgtaga atttagaatt 192600
   ttgtgtgtgt ggttaaaagt ttagagagtg ttgtaggtaa tgttgagata attttattaa 192660
   attttgtaag tttttattat attaagtttg tttgtttttt ttaattttta ttttatttta 192720
   ttttatttta tagggatggg gttttgttat gttgtttagg ttggttttga atttttgggt 192780
   ttaaatgatt tttttgtttt ggttttttag agtgttaagt tattttttta attgggattt 192840
   atgggtttag ggattttttg atgaattgtt taaaatatgt gtgtgtttgt ttgtttgttt 192900
   ttaatgtata tttttttagg gagagttttt attttttatt agaggtttgt ggtatatata 192960
   tgttttgtat tattgtatta gattgtgaat tttttatatg ttaaagattg ttttaaattt 193020
   gtttggaagt tttggttaat aaatatttaa ataaatgaat tttaaatttt tatgtgttat 193080
   tattgttttt attttgatta tttttaagtt tgtagtgtta tttttttgtt attgtgaagt 193140
   agatatttaa taaatatttg ttaaatgaat taatgttatg gttttggttt attaggattt 193200
   taggattatt gtttattatt attgtttata gtttttttat tattttgttt tagagtagtt 193260
   ttttttttga ggtttgttta tttttgtatg tgtgtttagt ttttagtttt gttgttttat 193320
   tatttttttt tttttatttg tattttttga attgttaata gttaataata gttttttttt 193380
   ttatttattt atttatattt gaagtgagaa gttattattg aggtttttgt ggattttgtt 193440
   agtttaattt ggggtggttt tttggaggtg gagtttttag atgagggatg gatattttgg 193500
   tgtttgttgg gtagatttat ttgggagttt tggtaggggt tttagggtag ttttgttttt 193560
   ttattttggt tgtgggattt ttggtgtatt agggtttttt aagtaagtat ttgtgttttg 193620
   ggagagttat aggtaggttt gagagggttt ttggaatttg ggatttttag tttggtttag 193680
   tagttggtat tattattttt tttgtaaggg aagttatgga aattaagatg ttaatgtgtt 193740
   ttgataggtt tggggtggtt ttaagttttg ggaagagttt tggtaatttt ttttggttgg 193800
   tgggtagaat ttttttttgg tagtttaggg ttttgttttt agttttttgg gggagagtaa 193860
   ttgagggagg agttggaggt taaggtttta gtgttgagtt gtttgtattg tagttgtatt 193920
   ttttgggttt tggttgtagg aattttatat ttttttagtt tttttatggt ttttgaagat 193980
   ttggtttggg gagggggtta gtagtttggg gtttatttat ttggttttaa attttggttt 194040
   tattgtttat ttgttttgtg atttttataa ttgataaaag tttttttggt tttggttttt 194100
   ttatttgtga aatgggataa taatagtggt tattttatag gattgtgtga tgatttatta 194160
   gttataatag tttttgagta gtgtttagag taatatttgg ggtttggtgt agtggtttat 194220
   gtttataatt ttagtatttt gggaggttaa ggtgggagga ttgtttgagg ttaggagttt 194280
   gaaattagtt tgattaatat tagtgagatt tattttttaa aaaaaaaaat tagagtaatg 194340
   tttggtatag agagttttta gtaaattttg gttgttgtga tgagttttgt ttttttattt 194400
   gtttatttat ttattagatt ttttgttggg gtttgttgtg agttttatgt tgttttgaag 194460
   aaagttgggt tggggtgagt gtgatggatg aggtgtttgg ttttataaat tttgtgtggg 194520
   ggggttgtgg tgagaggaaa ttgtatatat tattatttag ttattattgt ggggagattg 194580
   tttggatgta gagagtaggt tttgtgtggg agtggggagg gtaagttttg gttgtgaggt 194640
   agggtttttt taggatttag tagggatttg aaggattttg taggtattta gggagatagg 194700
   agaggaggag ggagtagttt tggttggata ttgttttttt agtattgttt gtattaggga 194760
   tttatttagt tttaagaagt ttttttgtgg gggataggga gtatttgtta gtttatagga 194820
   tttgaagtgt ttttatgggt ttaagttttt gggaaggttt gtaggtggtt gtagggttgt 194880
   tgtaggggtg ttggttttag ggtttggatt taggggagtt tgtagaggga gggtagttgg 194940
   aggttgtttt agtgtgtatt gttatgaggt aaagtaagga gattgttggg tttatgttgg 195000
   gttggggtgg atggaggtaa ggaagttttt gttggggtaa gggtattagt tgtagatgtt 195060
   ggtagttttt ttttggatat gggtttggaa ggttgatagg gtgtggtgag tgttattggt 195120
   ttttttgttg tggtttggtt agtggtttta taggtttttg tgggtaggag gaggatgatt 195180
   ttgtattttg tttggttatt attggtagtg atagataagg tgtgtgggga tgtggttatt 195240
   ttggtttgtg ttttgagagt taggtgtggg ttgggtttgg ggaagatagg ggaatggtat 195300
   tgatttgatt ttgagtattt tgttttgggg tgtaggtttg gttgttattt ttttggagtt 195360
   gggaatggta ttgggtggtg gtttagggtt ttttgtttta gtggtattat ttttgtattt 195420
   ggttagtaag gttgatggga tttgtgtgga tatttttggg atgtagatta ggtagtatga 195480
   tatataggga ggggtaggta gagaagggtg gtttttgggt tttggttttt aagttaggtt 195540
   ttgtaagttt gttgtgttaa gttttttttt ggtattaggt taggtgggtt agggttagtt 195600
   ttggggttgt gatgagtgtg agtgttttta aagtgtgtta tgtttaatag ttgttttttt 195660
   tttgggtatt gttttttagt tagatatttt atagtttttg ttttagttat agatagtaat 195720
   ttgatttttt tatttttggt aggttttggg gttggggtaa gaatttgtta ttaagtgtag 195780
   atgtttttag agggtgttgg tttagggttg tgtggattat agttttttta tttttgtttt 195840
   agatttattt gtaattttgg agaaaagatg gtttagtgga tgtttgtggg gaggagggag 195900
   ttggttttta ggatgagtag atttgggggt agtttttatt atgggattgg tgatttattg 195960
   ttattagtgt tagtgaatgg tagaggggtt ttggtataag gtttgggttt agaatgggaa 196020
   tttgtagaga aattgttaag gttttttgtt atttgatatt tttgtttggt tgttatttta 196080
   gaaatatttt tgttgtaaga agatggggga gtttgtttgg ggtatggttt ggtgatatta 196140
   atttgagaat tttaggttta agtgttggtt tttgggtttt agttgttttt tgagttgtaa 196200
   agttggtttt tgggtatttg tggaaaatat tggatttttt attttatttt attttatatt 196260
   ttgggatata tgtgtagaat gtgtaggttt gttatatagg aatataagtg ttatagtggt 196320
   ttgttgtatt tattggattt tttaatttag ttttattaga aatgattgtt tttttttttt 196380
   ttttttgttt tttttttttt tttttttttt tttttttttt tttttgtttt tttttttttt 196440
   tttttttttt tttttttttt tttttttttg tttttatggg gatataattt ttatattata 196500
   ttatttgttg atttagaaag tatagttgag tggtattttt ttataatttt ttataagtat 196560
   tatttttaat tttagaatgt tttttttatt ttaaaaggaa atgttatttt tggtatatag 196620
   ttattttttt ttttttagtt tttggaaatt attaatttgt gttttgtttt tatagatgta 196680
   tttattttga atatatgatt agaattttaa aagatgtgat tttttgtgtt tggttttttg 196740
   tagttggaat gtttttagag tttatttgtg ttggtgtatg tttgtgtttt tttatggttg 196800
   agaaatagtt tgttgtgttg atttagtatg ttgggttttt ttatgtattt gttggtggat 196860
   gtgttagtta tttttggagt tgggtgtgtt gtgttattta tgggttgtat taggagttgg 196920
   ggatattatt gagtaagtta tagttttggg tatttaggag tttgtagagt aggagttggg 196980
   agggaggtta ggaggtgtat ggtttgtttg ttaggttgga gagatttgta aaaagttatt 197040
   ttgagttaga aattagagtt tgaggttttt gatttttagt tgtggagttg ggagggaagt 197100
   tttgtatttt gttttgattg tggggtgatg tgttagttat tgtttaaatt agaatttttt 197160
   tgagagttga tgtgggtatt gttgataatt agtttggata tttgaaagaa tggaaagtag 197220
   ggatttagat agatttttgt ataattgtgt ttgtagtagg gtgaggtttg gaggtgtgat 197280
   ggggatttta atggggtagt tgttgttttg ttggagttta tggtttttgg ggggggttat 197340
   aggtgttaat tgtatattat ataagtagga gtgtgaaatt ttagggttgt taaatttagt 197400
   gagggttttg tgttggtttg gttatttata gtggtatttt aggaaaaaaa attattttat 197460
   ggattttagt ttttttatta gtaaagagta ggttggattg gaattttgta ttatttagta 197520
   tagttgttat ttatgaaatg tggttattta aatttaaata aaaatagaaa ttttgttttt 197580
   tagttggatt agtttagtgt taagtgttta gaagttattt gtgaatatat tgtggtaatt 197640
   ttttaaaaaa tgaaaattag aattattgta gtattttata attttatttt tgggtgtgga 197700
   tttaaaagaa tgtatagtat tttaaagagt tagagatatt tgtatattta tatttatagt 197760
   ggttttgttt atagtagtta gtgggtagaa ataatttgaa tgtttgtggt taaatgaatg 197820
   gagaaattaa aggtgggata ggttgggtgt ggtggtttat gtttgtaatt ttagtatttt 197880
   gggaggttga ggtgggtgga ttatttgagg ttataagttt aagattagtt tgattagtat 197940
   ggagaaattt tgtttttatt aaaataaaat tagttgggta tggtggtggg tgtttgtaat 198000
   tttagttatt tgagaggttg aggtaggaga attgtttgaa tttggggagg tagaggtttt 198060
   ggtgagttga gattgtgtta ttgtatttta gtttgggtaa taagagtaaa attttgttta 198120
   aaaaaaaaaa aaaaaagggt ggaatatata aggaatttga tttagttgta aagaggaagt 198180
   taggatattt gtaatagtaa gggtgaattt tttgggtatt atgttaagtg aaataatttg 198240
   gttataaaag gataattttg taggatttta tttatattag gtttgtagag tagttagatt 198300
   tagaaaaata gtagaatgga ggttgttaga ggtttggggg atggggttgg ggagttgttt 198360
   aatgaggata gtttgtttta taagatgaaa aagttttgga gatttgttat ataataatgt 198420
   gaatgtattt aatattattg gattgtatat tttaaaatag tgattgtggt aaatgttatg 198480
   ataattaaaa ggaaaagtta tgtggttagt ggttattatg ttgagtagtg taggttttgg 198540
   atggtgttgt ggttatagat aatggagtgg gtgattttta ttattgtttt tagttgtttg 198600
   agtattatgg tgttttgggg ttaggatgaa agtgtttttg ggtatggggg tttttgtttt 198660
   ttttatttgg gagttttgaa ggtagatttt aaagtttatt tggttaggta ggagagtgag 198720
   gtgggttttt gtattgtttt ttgtgtgtgt gtttgtttgt gggttagttg gggtttgtaa 198780
   ggttgttttt tgagggaggg gttgtaggaa tggtttttat ttgtgtagaa ggtaagttat 198840
   tgttttatgt taatggtaaa gatggggttt ggttgtgttg tattattatt ttttaagtgg 198900
   ggtttttggt gggtaggatg tttggtttga tttgagtttt tgtagtgagt gttggtgttt 198960
   gtggagggat ttagttgtag ttgagttggt tttttttttg ttttgggttt taggggtgtt 199020
   gggggtgaga aaggagagtt tagggggtag tgtttttttt taggttttgt tggggagggt 199080
   tgagggatgt tggggggagg tgggggtggg taggggttgg ttttagtttt aggtaaaatg 199140
   gtttttagat ttataggtag atggaagtga tttttggtta tgtgggtggg gattgaattg 199200
   ttgttttgta aggtaaggtt ttatgtttgg ttttaggaag tggttttaaa gttatatggt 199260
   ttatggttag atgtagggtt gtgtttttgt ttatttaaag gttttggaag tttaggggag 199320
   agtatttttg aagggggagt aggagtagga gtaaagtttt gaaggttttt aggttgtgtg 199380
   gtttgggtgg gggttatgtg ggggtataga gagaggagtt ttggttttta ggtttgtttg 199440
   tgggtattgt ttttagtagt ggagatgtgt ggattttttt ttggtatttt ttaataagga 199500
   attatattgt ttttttgttt ttttaatttt ataagatagg aatttttgtg ttttgtggag 199560
   gttgagaaga tggggaggtg tttgtgagga taaattagtt tttagttttt ggtttttaga 199620
   gttttttttt tttttatttt tgatgtttta ggtttgggga gttttttttt gatatttttt 199680
   gattgtttta gtgaaatagg ttgagaggga tggtttgaat tggttgggga tgtaggttgt 199740
   agttaggggg tttagtgggt attttttgag tttggttttt gttttgtaag tggaagggta 199800
   gttgaattga agttttttat tgtttttttt ggtttggaag tttgtttttt attttttggg 199860
   tagagtgagg agtgggtgtg ggttgggagg gttgtttttt gtttgggagg gttgtttatt 199920
   tgtttgttat gggggatgag ttttttgtag ttttttgtgt tgtggttttg gtttttatgt 199980
   tggttttatt tttttgtggt tgtttttgtt gggtgtttat tggttttagg gaaaagttat 200040
   tttttttttg gaggattggt gggaggggtt ttgtttggga gtgtgttgtg tggtttttgt 200100
   tttagtgtag gatgtttgag gttagatttt ttttgggagg taggatgggg taaaagtttt 200160
   tttgtaggta gagtagggtt tggatgggtt taagtaggga tgggggtata gaatagtagt 200220
   tgtttagttt gtgtgggagt tgagggttat taagggtagt tttagtttgt ggggattagt 200280
   agaggtatta tttttgtgat tagtgagttg tttgtatttg tatgtgtatg tattagtgag 200340
   tgtggtgagt ttgaggtgtg ttgggatttg gtttgtgtat tagttagttt tgatgggaat 200400
   tagttttatt ttgggtattt ttggaatttt attgtgtggg agtatttggg tttttagtag 200460
   gtgttttttg ggtgtttgtt tttagtttta ggtttttgat ttgtgtgtgg tgttttaata 200520
   gtagttagga gtagggttat tgaagggatt tatagataga agaggtttta gtgattttgg 200580
   gaagtttagt tttatgtgtt ttatatattt aagagtaaaa tttggttttg gtttgtgggt 200640
   gtgtagtttg tgggtgttgg ttaggtatat aagatggttg tttgggttag tttaggggga 200700
   ggtagatgtg gtgtaggggt tttttttttt tatgagttta ggaatagttt tgtatttagg 200760
   tatttgtttt gtatttagta tttttttttt ttttaggatt gttggtttgt tttatgggat 200820
   tgaatatata attttatggg gattgatgtt ggttttattt ttttttttta taagattatt 200880
   tatggggatg tgggaggtgg ggatatatgt tggttttatt tttgtttttt ataagattat 200940
   tttttttgtg ttttggtttg tggattggtt tttaaggttt ttgagtggta gtaggtggta 201000
   tagtttattt gagtagaggt gaaggagagg gttaggttta tttgggtggt ttgaggtagt 201060
   gtggagggtt aggagaggag agtatttttt atttttgttt gagtagtttt ttagggtatg 201120
   gagataggag tttagggtta gttttgtttt ggagtgtagg gggtttttag tttggtatag 201180
   tttttagaag aggagatgaa aaagtttggt tttgggagtt tggggatgga gttttgttgg 201240
   ggaggtagtg tggttgtagg atggtagttt tttagaggat attatggtta gggatattta 201300
   tttgggggaa tttttagggg ttgtttgggt aagttgttta gtttttttga gttttagtgt 201360
   tttagttttt ttatttaagg atgggtgtga taattatttg gtaggtgttt gtgtgtgagt 201420
   ttttgttttg ttttaatttt tttgttgttt tttttttagt tattgaggtg gtttttagtt 201480
   gtgttggtga tatggttgat atttttagag atgttgggtt taagtaggtg tttgtattat 201540
   ggaatgagaa ggttttggtg gattttggtt atgtggggat tgattttatt ttggagtaga 201600
   tgtgttggaa ggttatgaag tagggttttg agtttaatat tatggtggtt ggtgagtttt 201660
   tattttgtat gttatttaat taatgttgga aattagtttt agtttttagg gtggtttata 201720
   agtttttggg tttggttttt ttttttgtaa aatggggtag taattttgat tttaaggatg 201780
   gattgttagt gatattgtgt gtgtatgtat gtgtttggga ggagtttata gtttttagtg 201840
   gttttttaga ggggtttgtt ttgtatattt agggtttttg agatgatttt ggggagttta 201900
   ggttggtttg gagtaggttt tgatgagtgg ttgttgtttt tgagtaatga tgttagtttg 201960
   tgtagagggt tttgtggtag ttgttttttt attgttgttt tagtttggtt ttttaggttt 202020
   tttattgtat tttattgtat tttggttaga ggttttttag tagtttagtt ttagttttgt 202080
   tttggaattg gggttttgtg gtttttatat tttttggtgg ttttggagag ttggaggtta 202140
   ttatgggagg ttgttgatta gtatgatttg ggtagggtga gggggttagg ttggagttta 202200
   tagtgggttg tttgttgttt ttaaagtttt ttaggtttta tttaaatttt atagggattt 202260
   tgtgaagggg gtgttttgtt ttttaggtag aagtgaggtt tagggaaggt tgttaaggtt 202320
   ttttagttta tagggtagag aattgtagtt attgttttga tttttagaat tgtgttttgt 202380
   ttattttatg gggtttaggg aggggtttgg ggaagtgttt ttgttaggtt atattttaaa 202440
   taaaggtgag tttgtggggt ttttgtaagg tgtttgggta ttttttaggt tgggtttgtt 202500
   tttttgattt ttgttagttg tgtatgtttt ttttgtttag ttttgtggat gtaaatttat 202560
   atttgttagt ttgatatttt tttttttttt tttgtattga tttgggtttt ggtttttttg 202620
   gggttttggt gttttttttg ttttttgtag ttttatggga tttggttgtt tatggttttg 202680
   ttgtgtttat agttgtttgg aagttggtag tttgttttaa gggagggtgg aggtgtgggg 202740
   ttttttttta tggtttttat tttgttttgt ttgttgtggg ggatgtgttt gtattttgag 202800
   gatttagggt tgttttttgg gaagatggtt ggagagaaag agaggggaga ggtttaaagg 202860
   tagtggttag gttgggattt atttttgtgg gttatggagg ggttgttttg ttttttagtt 202920
   agggataggg tgagaaggat ttgggaattt ttgtgtttta gttatggtat aggttttttt 202980
   ttggagtttt ggttttaagg agagggagag gtagtttagt agtttttgtt ttttgtggtt 203040
   gttattgtga tagttttgtt ttttgggaat ggtgtgtagg aggttatttt atttgtttgg 203100
   gtttttttgt ttttttaatt ttgagaagaa tagtggtttt gattttttat aaaattattt 203160
   ttttttaggg atgttgagtg aggaataaga tgtggggtag ttttgttttt tttttttttt 203220
   ttttttatgg tagttgtgtt tatttgtaga ggtgtaggta aggtggaggg gtatttgttt 203280
   ggaggtttat agattttggg gagaatttta gttttgtttt aatttagtgg ggtgaggttt 203340
   gtgtgttttt ttttgggttt tttttttgtg agttttagtt tttttgttta taaaatggga 203400
   ttattttata aagtgagatt aagtgagtta ggttttgggt agggttttta ataggttgtg 203460
   gttgtttgag aatttgtagt agtggtgatg gtggttgtgg tgatgaggat gatgggggtg 203520
   gtaatgatgg tgatgatgat ggggtgatgg tgttagtgga gatgatgggg atgatggtga 203580
   tggtggtggt gatagtggtg ggatggtagt ggtgatgtgg gtgatggtga ttgtgatggt 203640
   ggtggtggtg gtgatggagg tgatggtgat agtgattatg agggtgatgg ggatggtggt 203700
   ggtggtgatg gtgatagtgg tgggatggta gtgatgatgt gggtgatggt gatggtggtg 203760
   atggtggtgg tggtgattgt ggtggtggtg gtgatgatgg tggtgatgat ggtgattgtg 203820
   gtgatggtga tggtggtaat tgtgatggtg gtggtggtga tggtggtgat gatggtgatt 203880
   gtggtgatgg tggttatgat ggtggtaatt gtgatggtgg tggtgatggt ggtgatgggg 203940
   atgatgatgg tgattgtgat gggggtggtg gtggtgattg tgatggtggt gatgtgggtg 204000
   gtggtggtga ttgtgatggt ggtgatgtgg gtggtggtgg tgattgtgat ggtggtggtg 204060
   gtggttgtga tggtggtgat gtgggtggtg gtggtgattg tgatggtggt gatgtgggtg 204120
   gtggtggtga ttgtgatggt ggtggtgatg gtggttgtga tggtggtgat gtgggtggtg 204180
   gtgattgtga tggttgtgat tgtgatggtg gtggtggtga tggtgatgat ggtggtgatt 204240
   gtgatggtgg tgatgggagt gatgttggtg attgtggtgg tgaagggggt gatggtggtg 204300
   attgtgatgg tggtgaaggg ggtgatggtg gtgattgtga tggtggtgaa gggggtgatg 204360
   gtggtgattg tggtggtggt aatggtgatg gtggtaaagg ggtgatggtg gtgattgtgg 204420
   tgatgtgggt ggtggtggtg atgtgggtgg tggtggtgat tgtgatgggg gtgatgatgg 204480
   taatgtgggt gatggtgatt gtggtggtgg tggtgatggt gatgattgtg atggtggtga 204540
   agggggtgat ggtggtgatt gtgatggtgg tgattgtgat ggtgatgtgg gtgatggtgg 204600
   tgttgatggg ggtgatgatg ttagtagtgg tggtgatagt gatggggatg atggtggtgt 204660
   tgatggtgat agtggtggga tggtagtggt gatgtggttg atgatggtgt tggtgttgat 204720
   gattgtgatg atagtggtgg tggtgatgag ggtgatggaa gtggtgatga tggtgggagt 204780
   gatgggatga tggtaatagt agtgatggta ttgtgatgat ggtgttgatg gggatggtgg 204840
   tgatgataag ggtgatgatg atggtgatga taataataaa agtaataata ataaagggat 204900
   atattttgta gtattttagt tggtattttt tagatttaag gtgttgtggt tggaatattg 204960
   atataatttt tgatttgagg ttggttagga tatagatttg gtggatagag agagatggat 205020
   gaggagtgtt tgtttttggg ttattaaata gtttaggtta gggattttta gtattagttt 205080
   ttgttttttt aggagtttat atttttttta gaaaggtgtt attaaagtta gaatattagt 205140
   gtaatttagg gtgttttttg tgttgtgttt ttttgagata gggttttatt gttgtttagg 205200
   ttggagtgta gtggtatggt tttgatttat tgtagttttt atattttggg tttaagtgat 205260
   ttttgtgttt taggtatgtg ttattatatt tggttaattt ttgtgttttt tggtagagat 205320
   agggttttat tatgttggtt aagttggttt tgaatttttg attttaagta atttattttt 205380
   tttagttttt taaagtgttg ggattatagg tgtgagttat tttgtttggt tgatttttgt 205440
   ttttttaaaa gatagggttt ttgttatttt ggagtggtaa tgattttagt ttattgtagt 205500
   tttgtatttt tgggtttgag taattttttt attttagttt tttgtgtagt tgggattata 205560
   ggtgtttgtt attatattta gttaatgttt atattttgat gtagagatag ggtttttttg 205620
   tgttgtttag gttggttttg aatttttggg tttaagtaat ttttttgttt tagtttttta 205680
   aagtgttggg attataggta tgagttatta tatttggttt agggtatttt ttttggtgtg 205740
   ttgttagtgt agtttaaggt agttttgtgt agggtaaagt aatggggttt tgagtttgga 205800
   ggggtgtgtg tgtgtgtgtg tgtgtgtgtg tgtgtgtgtg tgtatgtgtg tgtgtgttat 205860
   atgtgatttg tttgtgtgtg ttgtgagagt tgtgtgtgag gtatgtgtaa gaggtatgtt 205920
   tgaggtgagt tgtgtgtgtg ttgtgagttg tgtatgtgaa gtgtgtgtgt tgtgaggtgt 205980
   gtgtatgagg tgtatgtgag ttgtgtgtgt tgtgttgtat atgaatgtta tttttttatt 206040
   tttgtttgtt ttagttattt gtggattggt tgtttgtata tatggttatt gttttttgag 206100
   tggtattatt ttgtaggtta gtgtttttta gtatatggtt ttatagattt tatgggatat 206160
   gtgtgagttt gtttgatgtt tatattagtt ttatggggga aggattgttt attttatttt 206220
   gtagattagg aaattgagat atggtgaggt taggtgattt gtttaggttt tggttggtta 206280
   gggaggagta gtttgaatat ttgggttttg gttgtaatta ttttggtgaa ttgtaggaat 206340
   tttggttatt tggtgtattt tgggtagttt tgggtttttt tgttagtttg gtttttgttt 206400
   tgtttaaaat ttgagttatt aggagatttt ttgtttagaa agtataaggg gtataagaga 206460
   tgttgatttt tttaggtttg gtttattttt taggagagga ttgtggtttg ggttgtgttg 206520
   gtattagagt ttggttgtgt gggtttatat agttattgga tatttggttt gagggtgaag 206580
   tttttggggg ttgtttggta gggatattgt tgggaggtag tttaggtatt gttgaatttt 206640
   agattggaga gttttgtgtt tgggtgggag gggttttatg tgtagatttt gttggttttt 206700
   ttggagagat ttttgattgg ttttttattt tttttttagg gtagagtggt ttgggtaaat 206760
   ttattttaat taatattttt tttaaattta aaattagttg gaagttggtg tagtttattt 206820
   tagaggagtg tatttttaag attattgaga ttaagtttat tatgtatggt tagtggttgg 206880
   gagtgggttg ggggtgtagg atgtttttgt ttttttggag tataggggtt gggggttaag 206940
   attattatat atagttagtg gttaggggtg ggttgggggt gtaggatttt tttgtttttt 207000
   tggagtatag agtggggggt taagattatt atatatagtt agtggttggg agtgggttgg 207060
   ggatgtagga tgtttttgtt tttttggagt ataggggttg ggggttaaga ttattatata 207120
   tggttagtgg ttggggatgg gttggggatg tgggatattt atgttttttt ggagtattgg 207180
   tgtggtgtga gggtgtttga gttaggtaat tttaggtttt taagatgggg atttgttgtg 207240
   tttattattt ttagtatgtt gagtatgttg gggttttggt tgtgatgggt tagtgttttg 207300
   gatttttgtg ggttttgtta tattgagttt tttatagata attgagtttt ttattaaaag 207360
   tttgataggt aaggggtttt taggggaatt gttattggtt tgtgttggag ttggttgagg 207420
   aaggttatta attttttggt gtttttttgg ttgttattgt tgtttatttg gggttgtttt 207480
   ggggttagtt aggtgtgggg tgtttgtgag ggttttttgt tttttttttg attttgtgtt 207540
   tgtggttttg tgtagatatt gaggagaaag gtgtttggat gaagttgata gtgattgata 207600
   tattagggtt tggggattat attaataatg agaattggtg aggttttttt agggggagga 207660
   gtattagtgg gggttttagg gttttttgga ttttatttag agttagtttt agaggttttt 207720
   tggtttgtgg gggtgtaggg tttatttttt gggatttgat atgttgttaa agttgtatgt 207780
   tttaggggtt tgaaaatttt ggattaaagt agaattattt ggggaatttt gttgttttta 207840
   atttattatt tgaggttagt ttggggaggt tggtttggga ggttgagggt aaggttgtgg 207900
   tatttttgtt tagtaagtta gattttttag ggtatgtatt ttattggaag gtggggtgtt 207960
   gggttttgag ttttgggaat gtatgatttg tttggggagg gtattttatg tgtttgttga 208020
   tttgttttta ttttttatgt agttggtagt ttattatgaa gtttattaat gattagtatg 208080
   agaaatattt gtaggaggag gttaatatta attgtaagaa gtgtattttg gatatttgtg 208140
   tttattgttg tttttatttt atttttgtta ttggttattt gtatgttttt gtagtgttgg 208200
   tgtttttatg ttgggtgttt tgggtttttt ttgttttttg ggattgtaag atggtgttgt 208260
   ttgttttgtt gttgggtgta gaggggttga gttaggggtt atggttgtta gttatgaagt 208320
   tgggggtgtg ttatgtttgt atttttaaag ttttatattg atttttatgt aagtgttttg 208380
   tttagttttt attttttagt tgtgttttgg ggagttgagg gttaggtagg ttttgggttg 208440
   ttttggagtt tggtggtttg tgttttgttt ttttaggtta ggttttggta ggtggagtgg 208500
   gagtttttag ttaagtattt gtagtggtga ttgatttttt tatttgggtt ttgttttatg 208560
   ggatttttta gagggtttgg tgtgttttta gatgggaagt atagagatta gtgttggttt 208620
   tttttagggt tggatggggt tgttgttggt gttggtgaga gttttttttt tttgttggtt 208680
   ttgtttgtat ttgatgttgt ttttagagat tagtaagggt gggaggttgt atgtattttg 208740
   gtaggtggat tttttatggt tttatggatt ttttgttttt agttttttgt gtattttttg 208800
   ttagatattt gtggtttatt ttgagttagg gttgggagga ggaggattag aggttagtag 208860
   gagtggttgg tggaggtagt tgtgggtggt ttgtttgtat gggtgggtgg agaaggtttt 208920
   ttgtatttgt tgttttagat gtattttttt gggtggagga agtttagggg tttggggaaa 208980
   ttatattttg tgggtattag gtgtaggaag gagtttggtt ggtgggtagt gggttttatt 209040
   tagagtttgt gggaaaattt aagttggagt ttttgagttt attttttggt attatttttt  209100
   gtgggttagt aggagttagg ggtagggggt tagtttgttg tttatgggtt ttttttgaat 209160
   tgaatatttg tttttttagg tagaatggtt tatttgtggt tttgtggtag ttgttttagt 209220
   tgttttaagt tttgttttag ttgaggttga tggtaggagg tagaagtttt ttttgatgtt 209280
   agttgggtaa gttgagggtg ggtgggtggt ttgtggtatt tttgttattt tttagtattg 209340
   attttttggt tgggatttta gttaggtatg ttgtttggtt ttgtttaagg tgtttagttt  209400
   tatggggtag gggtgggttt tggttatttt tgtttaaatt tttgtttgga gtgggttttt 209460
   ttttagttat gtgtgtgatt gatttatttt tattgtggga aaagtttggt tttagaattg 209520
   gggaagatgg tgttttagtt ttagggtttt gttagttttt gttatttata gtttttttta 209580
   tattagattt taggtttatt ggggttaagt aggtaagggg agtagtttgt ttggtgtagg 209640
   ggagtagttt gtttggtgta ggggtattgt atattggtgt tagtttttat tttttttgtt 209700
   tttttttttt tagttaaagt tttatagtaa gatggttgtt gagttaatga tagttttttt 209760
   gtgtggatat gtaggggtgt ggttgggatt ttagaattta tggatgaggg tgtttggagt 209820
   tggttttggt ttgtttggtt tgtgtagtgg tgttatttgt gtttgggata tatttgtgtt 209880
   ttttttttgt tttatattta tagtgtggtt gatttggttt ggggttttgt ttttgttagt 209940
   agggattttt gtgagttttg agttagtatt tgagagtgtt gatatttgtt tgggggtggg 210000
   tgttttttta ttgttttgtt tttttagatg agttttatgt atattttttt gttttagttt 210060
   taggtttttg gatattgagt ttatgaaatg tttgagtaag gtggttaata ttgtttttgt 210120
   tattgttaag gtggatatat ttattttgga ggagagggtt tattttaaat agtgggtagg 210180
   gttttatttt tatttgtttt agtttttttg tgtaatttgg agatgttgta ggtttggtag 210240
   gggttatttt gtttaaagga gttatattgt tagggagatt gaattgttgg ttattttttt 210300
   tagtgggtgg ttggtttggt ggtttttggt ttaggtggtt gttgggggtt gtttttgaga 210360
   tggttttttt agttttttta tattttaaaa gtagttttgt gggagtgtta attttttagt 210420
   agagtttttg aggggggttt tggagaagat ttggattagg tagatggtga attttttttt 210480
   tagggagtga ggttgagtag gggttgtgtg atatggttta ttttgtttta tgtttttttt 210540
   tttttttttt gagaaagggt ttggtttagt tgttaaggtt gtaatgtagt ggtataatta 210600
   tggtttattg tagtttttat tttttaggtt tagtttttga gtagttggaa ttatagatgt 210660
   atattattat gtttggttaa tttttgtatt tttagtagag atgaggtttt gttatgttgt 210720
   ttaggttggt ttttaatttt tgggtttaag ggattttttt gttttagttt tttaaagtgt 210780
   tggggttata ggtatgagtt attttatttt gttttgtttg gtttttatta gatggaagag 210840
   aagttgggtt aggtgtggtg gtttatgttt gtaattttag tattttggga ggttgaggtg 210900
   ggtagattat ttgaggttag gagtttgagg ttaatatgat gaaattttat ttttattaaa 210960
   aatataaaaa tattagttgg gtatggtggt gggtgtttgt agttttagtt atttgggagg 211020
   ttgaggtagg agaatttttt gaatttggga ggtggaggtt ttagtgagtt gagattgagt 211080
   tattgtattt tagtttgggt gatagagtga gattttattt taaaaaaaaa aaaaaaaaaa 211140
   aaagttttag gagggatggt ttttgttatt tttttttttg ttattgtttg aggtttttgt 211200
   tgtgggatag ggaggggtat ttaagtaaga gggtagtttt gggttttttt ttttgatagg 211260
   gtagtatatt agagttatat ggggtgtttt ttaaaatgaa attgaggtgt ttgggttgtg 211320
   gtattatatt tgttttggtg ggtgtgggtg gggtttgtaa tttattttgt tttggggtta 211380
   ggtgttaggg atttttttag tatgtgtagg ggaagatagt ttatataaag tgggtgtgtt 211440
   tgttggaggt tatatatggg ttgtggtttg tgtttgggta ggtgagtagt tgtttattgg 211500
   gatgtttttg ttgtatgtat ttatgttggg ttgttggtag ggagttgaga ggttattgta 211560
   ggtggggttt ttgttggaat tggggattgt gatgagggtt ttttaggaag ggttttagga 211620
   ggggaggttt tggtaatttt gagtattttt ttggggttgt gatgagtagg agaagagagg 211680
   tgggaggtgt ttaggatgat gattttaagt ttttggggag ttgtagtgtt gtttaggata 211740
   gtaggtgtat ttgtagttgt tttttttttt tttaggaggt ataggagttg gaggtgattg 211800
   gtgttatagt tttttagagt ttgtttttga atttgagttt ggggtagtat atagtgtgga 211860
   ggttatgtgg taaatattag tgggtgggta gagtttgtta tagggatggg tttatttttt 211920
   ttttttttta ttttagatta ttgtagattt gttgtttaat ggtattgatg tgtattttta 211980
   gaaggaattt gatgaggatt tggaggattg gttggtgaat gagaagtttt gggtgagtgg 212040
   atttattagg atgttgtttt taggggattt ttagtttttg ttgaagggtg ggttgggggt 212100
   ttggaggatt ttaagttatg aaattatatt tttggggtta gagggtattg agtttaggtg 212160
   tttgtattta gtgttgttag gttgaggttt ttgtttttgg gggattttag gtttagggta 212220
   gttttttttg ggggtttagg ggagggaatt gtttttttgt atatgtgtaa ttaatattgt 212280
   ttgtttgttt tttaggagat gattttattt gttgtggtgg gtagtgatta tgagtattag 212340
   gttaatggta agaggatttt tgggaggaag attaagtggg gtattattga aggtatttgt 212400
   tgtaggtgtt ggggttttag atagatggga agatagtttt ttttgttgat ttagagtttg 212460
   tgggttatgt ttgagttagg gatttgtgga attttattta ttgttttgtt ttatttagag 212520
   ggaggggttt ttttgttttt atttaatttt tgggaaagtg agagggtaag tggtttgtgt 212580
   agatgttttt attttagatt tttttttggg tagggaaaga tttagggaga taggagttgg 212640
   gatgggggtt gttaagtttt ggattttggt gtaggttttt ttataggtat ttgtgtggtt 212700
   ttggataaat tgtttaattt ttttgggttt agaaaagggg tgggttgtag ttttgtgagt 212760
   tagatggatt tttggttttt tattttgttg aagtttttgg gatttttata ttttagtttt 212820
   ttgagtggtt gttgtaatta attagtataa atgaggggat ttaaaataat ataaatgtat 212880
   ttttatagtt ttagaggtta gaggtttgaa attaggggtt gttttttttt gaggttttga 212940
   ggattagttt tatttatgtt tttttttttt tggtggtgtt tggtattttt agttgtattg 213000
   taaaggtatt atatgtttta atttttgttt tattgttata gggttttttt ttttgtgttt 213060
   ttgttttgtt tttttttttt tttttttttt tttttttttt ttgtttgaga tggagttttg 213120
   ttttgttatt taggttggag tgtagtgttg taattttagt ttattgtaat ttttgttttt 213180
   tgggtttaag taattttttt gttttagttt tttgagtatt tgggattata gttatgtatt 213240
   atttatttga tttaattttt gtatttttag tagagatagg attttgttat gttggttagg 213300
   ttggttttaa atttttgatt ttaggtgatt tgtttatttt gtttggtttt ttaaagtgtt 213360
   gggattatag gaatgagtta ttgagtttgt tttttttttt ttttgttttt ggataaggat 213420
   gtttgttttt ggatttaggg tttattttaa gtggatgatt ttatttggag atttttaatg 213480
   aatttataaa gaaagatgtt ttttaagtaa ggttataatt atgggtttta aggtttggat 213540
   tttgattatg tttttgaatg gatgggagtt tttggggggt tatagtttaa tttatttttg 213600
   gtggtgatat tttgggaggg atgggtttga atgttttttt tttttttttt tttttgtttt 213660
   tttatttttt attagaatgg aagaggggaa ggtgtagagg gaaatgtagt aggaaaagtt 213720
   attttgtttt gggagagtat ttggttgaaa ggtttagtag agtaggaagt ataagttttt 213780
   taatttttta gggttttagt ttttattatt tataaagtgg gtgtagtgtg ttaagatttt 213840
   agtgagttga gagattgttt taaagattat agagtttttt gggaagttgt tgttttaaaa 213900
   aaatggttat aatgataatt attaggaggt attagatatt tttgtgttat tggttagata 213960
   tgggttattt tgttttatgt ttgaagtttt ttgtatttat tttttttgta gagttgaaga 214020
   ggtgttgtga gtagaaaatt tgttaaggga tttagaatgg gaggtggttt tagaatttat 214080
   agttttagtt ttgtattaga tttttttgag atgggagttt atgtttggag gagggtgtgg 214140
   agaggtatat gggttttttt tttgtttgta tttttttttt attattattt ttgttgggtt 214200
   tattttgtgg gagttgttta gttgggtgta gtttgggtgt tttttagtat ttgtttgtgg 214260
   tgttaggttg gtttgttttt ttttggtttt tttgtttgta gttttttttt ttttttttgt 214320
   ttaaagagtt tttgttgttt aggaaatttt ttatgaaagg taggtttggg agagttaggg 214380
   atggatgggt ttgggatggg ttggtggttt tgttatgggt gtgtttttgt tgtattttgg 214440
   tggttaattt tgagtattgt aggtggttgg tgatagaaat tgttaggaaa tgtataagag 214500
   agaggttttt gtattttttt gatttgtgtg ttgttgagta ttattggttt tagttgagga 214560
   agagtttgga atttgttagg atagttgtga ggtggtgggg tttatttttt agtagtttta 214620
   tatgaggatt ttggaaatgt gtttttagta ttgttgggtt tttatattag gggttttatg 214680
   agtttatagt tgatttgata tttggattag taagggttgt gttttgtttt tgttttttat 214740
   ttggtgaata gtattttgag gtattttttt tatttttaga aattatttta agtattgtta 214800
   atagaaatgt ggggtgtaat tgattatgtt atgaatttat ttaattttta tgtttttgag 214860
   aggtgggtga tgttattatt tatattttat ggatgaggaa attgagggaa tgagagtaat 214920
   ttgtttgagt tagggagtgg tgattatata ttttaatttt agtttgagag ttttattttt 214980
   tttgttggtt tttgttgttt gttagtgtta gatgagagtg ttgttggtta gtttgtagga 215040
   ggttttagtg tggatattgt tttatatagg gttgtggtag gagggtttag gtaaaggtaa 215100
   ttggtatagt attgagagta tttgagggtt gggtaaggtg gaggttttgg ttttgtttgt 215160
   tggtttatta agtaaaaatt agatattttt aatttgggga aaagttatgt ggttttgtga 215220
   tggggtagtt tggagttagg tgtatgggtt ttgtattttg ttttagtttt atgtattgtg 215280
   gagttgagat agatatagtt tttgtgtttt ttatgtttta tttttttttt tttttttttt 215340
   tttttttttt tttttttttt ttgagatgaa gttttatttt tttgtttagg ttggagtgtt 215400
   gtggtgtgat tttggtttat tataattttt attttttggg ttttagtgat ttttttgttt 215460
   tagttttttg agtagttggg attataggtg tttggtatta ggtttggtta atttttgtat 215520
   ttttaataga gatgagattt tattatgttg gttaggttgg ttttgaattt ttgattttgt 215580
   gatttattta ttttagtttt ttaaagtgtt gggattgtag atgtgagttg tggtgtttag 215640
   tgtttatgtt ttatttttgt aagataatgg ggataataaa ggaattataa gagatatttg 215700
   taggtgtgtg aagatgaagt ttattatatg ggagggtatt gggtgttttt atttgttttt 215760
   tttgtttggt tttttatttt tagtgatgtt gtttattgtt ttttttgtta aaaggttttt 215820
   tgttatataa tagttatata tgttaggtat tgtgtgttta tataggagtt atgaagtata 215880
   gtaataaaat gattattttg agatggttat tagtttgagt ttgagggtat ttgtgggtgt 215940
   ttgtggtttt ttgtttagtt tttttgagaa gtggttattg ttttgagttt gggtttgtat 216000
   gtattgtgtg aagtgtatat gtttttgagt aatgtttagt attgttgatt tttgagtttg 216060
   tgaatggttg tttatagtat gtagtttttg tgtgtgtttt tgtgtgtgtg tgttttgagt 216120
   gtatataagg tggatggggt tgtgggggta atattttgtg ggttataggt tttttttaaa 216180
   attttagttt tgatttttgt tttattttat ttttagtagt ttagtttttt gtttgttttt 216240
   aggtttttgt tgggatgttt tgtttttttt ggttatttta gtggtgtggg ggtttattgt 216300
   tttggttttt ttattgtggt ttttttttat gtttttattt ggtttggtta ttgtgttatt 216360
   ttttattagt taggaatttt ttggagagta ggagttgagt tgtttaggag taggagttgg 216420
   tgttggttat tttagtattt tttagattga gggtagtgtt aggtgtttta tatttagttg 216480
   tagagtaggt ggtttttttg tttttttgtt tatggggttg tttttagatt ttgtttttgg 216540
   tattagtatt tttgggtagg gggagagagg tggggtttgg gtagagtttt tttattggga 216600
   tattaaagtt ttttttgttt ttttttttag ttgaaaatat tatatattgt gagtttgttt 216660
   atttgtggga tttttttatt aggtgagaga tagggtgttt gggtggggtt gatggtttta 216720
   tttttaagag ggttttatag tgggtggggg tagtgggtgt ggggttgaag ttttgggtag 216780
   agtgggtgtt ttttgttatt tgtttgtttt gtttttggga agattttgga agtttatttt 216840
   ttgaaaaaga aaatttattg ggaaatgaag taggtagtga agattttttt taatttttgt 216900
   gaagttggtt ggatgggaag gttgagtttt gatttattgt ggtttggttt gttagagttg 216960
   tttggtggtt attaagggtt tagtgaggtt ttatgtgtag gttttgttgg tagtgtgggg 217020
   tgtttttagt tttgatttta ttggtttttg atttttattt tttttatttg gttgaggttt 217080
   ttttttgttt tttttgagtt taggtaagga ggggaagttt tgatggagag ggtgttgggt 217140
   gggtggtttt tggagtgtgg gttttggttt gggtaggttt ttttttagta agtagttggt 217200
   atggatgatg ggaatgttat taagaggagg ggtttttagg ttggtggggg taggggtggg 217260
   gggattttgg gagttgtttt gttattgtgg ggatattggg ttttttaatt tttttttaga 217320
   ttattttttt tgagttgtag aggaatgaaa atttgagttt aggatttttt ttttttgggg 217380
   ataagggtaa tttagttttt tgttttaggt tatttgtttg ttataggtta tagttttgga 217440
   ggttggtggt tatttattgt ttttattttg ttagtatttt ggatattggt ttggagtaat 217500
   aatgttttag ttttgttttt ttgagtttat gtattgtttt tgggttttgg atttagggtt 217560
   atagggtgga tttagaggga gatatatgta ttggaatatg tgtgttttga ttgagtttgg 217620
   gtttattagg gggattgatt ttttttttaa aatgtgtatt agatttggtt taatatggtg 217680
   ggtttggggg attttatggg gagttaagta gttgggatgg ggagtggggg tgggggtagg 217740
   tgggtttgag tttgaggtag gttgagtagg gtttttgttt tgttgttttt attttgttgt 217800
   gtttatttta ttgatttgtt tgttttttat ttttatagga tgtatatgta gaatattaag 217860
   gatattatta gtagtattta ttttgaggtg tattgtgtga agtgttttaa tgagggtagt 217920
   agtgttatgg ttaatggtat ggaggagaag gagttagaag ttttggagat gtagatgtta 217980
   ttttgtttat ttttgggatt ttgtttttaa gttatttttg ttttttttta ggttttttta 218040
   ttattttatt ttattttata tgattttttt tatttgttat tgttttttat ttttttgata 218100
   tgttgttagg aaataaggga aggggttttt ttttgagtga gttagtgatg aggttgtggt 218160
   ttttttgagg ttgtggggag gttgtattgg agttataggt aggggtgaga gtatttattg 218220
   aattgatatg attttttgtt tttaggtttg gttttttgag ggtttagaag agtagttttg 218280
   gtgtgtagat tatttgtttg tgtggggttt ttagtgttgg aggttttggg gtgggggtta 218340
   ggttttgtat ttgtagagga gtttagtggg ttgtatgttt ttttttattt ttattggttt 218400
   tgttttttgg agtgtgttag agtttagggg agaatgtagt ttattaggag tatttggatt 218460
   ttttgtttgt tatatggtgt ggttattatt tagtttttat ttttgttttg tttttaaggg 218520
   tagaaaattt tagggttttt tgttattgat tgtttagtta ttttaagttt tttggtagtt 218580
   gttttttttg gagtagaaag tgtttttatt ttagttattt gtagattgtt tggttagatg 218640
   tggggatagg ttggaatgag ggaggtgttt ttattttttt gttatttttt ttttatgtta 218700
   tttttgtttt tattgggttg taggtgttgt tgatgtgttg ggatttgatt gaggataaaa 218760
   aggaaggaga gatgattttt atttttttat tttttagttt tgaaaaggtt taagtaagtg 218820
   agtttggtgg aggagttgag ggagggagtt atggaaggtg ttagaaggaa ggttggtggg 218880
   ggtatgtgtg ggttgtggtt tgggttggtt agagtggtgt gagttgtttg gtgtttgttt 218940
   tgtttaagtg attagggaag tgtgtgtgtg tttatgtgta tgtgtgtttg tttgtttgtt 219000
   tagtgtttgg gtttggttta agattgggtt gtagttatat taatgtttag ttagttattt 219060
   ttgttattta ttttttttgg tttaggtttt gttgattttt tgagttgggg aggtgggagg 219120
   ttaggtgagt ttgattttgt tgatttattt gtgtttgggt tttttttttt agagtttatg 219180
   gtaatgaatt tttagaaagg agagaatggg tgttaggggt gtatagttta tagttgaaga 219240
   gtaaggtttt gtggtagtat ggtttggttt tttatttttt gtttttatga ggggatttat 219300
   gggggttgtt tttgtagggt atagatgatt aaagtttttt tttgtttttt gttatttgtt 219360
   ttgtttttgg ggagaaagag gggtttgatg agattttatt taggtgtata tattattagg 219420
   tgtatttgta ggtattgggt tggttgtttg tagttaggag aaggttagtg agaaggagtg 219480
   tatgagtgtg agtgtgtgtg tatggaagtt ggggtattgg gtgtttgatt ttttttttat 219540
   ttaagagagg aaggattttt tattattttt attggtgaga tagtttattt tgttaatttg 219600
   ttatgttttt gttaaaatta agattttgaa ggaaatgagt ggttagtgtt agggtttagg 219660
   ttatgtggtt tgtttagttt taatgttatt tggtggtggg gtggggtggg ggtgggtagt 219720
   agtattttag ttttgagatg ttttattttt tttttttgta attagatttt gaaaaattgt 219780
   ttgttttatt aggttttgtt ttttaatggt tttttgttat gtgttttttg agaaatttgt 219840
   ttttttgtat aaatgataaa gtgttgaaaa tgtatttttt gaaataaatg ttttaaatgt 219900
   agaaattta 219909
<210> 66
   <211> 219909
   <212> DNA
   <213> Artificial Sequence
<220>
   <223> chemically treated genomic DNA (Homo sapiens)
<400> 66
   tgggtttttg tatttgaaat atttatttta ggaaatatat ttttaatatt ttgttattta      60
   tataaaaaga taaatttttt gggaggtatg tagtaaaagg ttattgagga atagagtttg     120
   atgaaatgaa taatttttta aagtttggtt atagagaagg aaagtgaagt attttaaggt     180
   tgggatgttg ttgtttattt ttattttatt ttgttattaa gtgatattga ggttgggtag     240
   gttatatggt ttgggttttg gtgttggtta tttatttttt ttaaaatttt ggttttggta     300
   aaaatatggt aagttggtaa agtaaattgt tttattagtg ggggtggtga ggggtttttt      360
   tttttttggg tggggaggga gttagatgtt tagtgtttta atttttatgt atatatattt      420
   atatttatat attttttttt gttgattttt ttttggttgt aagtagttag tttggtgttt     480
   gtagatgtat ttggtgatgt gtgtatttga gtggagtttt attaggtttt tttttttttt      540
   taggagtaag ataggtaata ggaagtagga agggattttg gttatttgtg ttttgtaaag     600
   atagttttta tgggtttttt tgtggggata gagggtgagg ggttgggttg tgttgttatg     660
   aaattttgtt ttttagttgt ggattgtgta tttttgatgt ttgttttttt ttttttaggg      720
   gtttgttatt atgggttttg aggggagggg tttaggtatg ggtagattaa tagagttagg     780
   tttgtttggt tttttatttt tttagtttag agagttagta aggtttgggt taggaagggt     840
   gagtggtagg ggtggttggt tgggtattaa tgtaattata gtttagtttt gggttaaatt     900
   tagatattag atagatagat ggatatgtat atatatggat atatatatat ttttttggtt     960
   atttgggtag ggtaggtgtt gggtagttta tgttattttg attagtttaa gttatggttt    1020
   atatgtgttt ttgttaattt tttttttggt attttttatg gttttttttt ttagtttttt    1080
   tattagattt atttgtttag atttttttaa aattggggga tgagggggta ggggttattt   1140
   tttttttttt tttattttta attagatttt agtgtattag tagtatttgt agtttggtgg    1200
   gggtgggggt ggtgtgagag ggggatggta gggagatgaa gatgtttttt ttattttagt     1260
   ttgtttttgt atttggttaa gtagtttgtg gatggttgag ataaaggtat tttttgtttt    1320
   aggaggggta gttgttaggg ggtttggagt ggttgggtag ttggtggtag gggattttgg    1380
   agttttttat ttttaggagt agggtagggg taggggttga gtgatggtta tattatgtgg   1440
   tgggtagggg gtttaggtgt ttttggtggg ttgtattttt ttttgggttt tgatatattt    1500
   taggggatag ggttgatggg gatggagggg agtgtgtagt ttattgggtt tttttgtaag    1560
   tgtgaggttt ggtttttatt ttaaggtttt tggtattgag aattttatat agatggatga    1620
   tttgtatatt gaagttgttt ttttgagttt ttggggagtt aggtttgggg atagagggtt    1680
   atgttaattt agtgggtgtt tttatttttg tttgtggttt tagtgtagtt tttttataat   1740
   tttggggagg ttgtggtttt attattgatt tatttggagg gaggtttttt tttttgtttt    1800
   ttggtagtat gttgaagggg tggggaatga tgataaatgg agaaaattat ataaaataaa    1860
   atggggtggt gggagggttt ggggggggat ggaaatgatt tgggggtagg attttggggg    1920
   tgggtagggt ggtgtttata tttttggggt ttttggtttt ttttttttta tgttgttggt   1980
   tatggtgttg ttgtttttgt tgaggtgttt tatatggtat gttttgaagt ggatgttgtt    2040
   ggtgatgttt ttgatgtttt gtatgtgtgt tttgtggggg tggggggtgg gtgggttagt     2100
   gaggtgggtg tagtggggtg ggggtagtgg ggtaggggtt ttgtttggtt tgttttggat    2160
   ttaggtttgt ttgtttttat ttttattttt tattttgatt gtttggtttt ttatggggtt    2220
   ttttaggttt attatgttgg attagatttg gtatatgttt tgggggaagg gttagttttt    2280
   ttggtaagtt tagatttggt tagaatatat atattttagt gtatgtgttt ttttttgggt    2340
   ttattttatg gttttgggtt tagggtttag ggatagtgta tagatttaga gggatagggt     2400
   tggagtattg ttgttttagg ttggtgttta gggtgttggt agggtggaag tagtgggtga    2460
   ttattggttt ttaggattat ggtttgtggt aggtaggtgg tttggggtaa agggttgggt    2520
   tgtttttgtt tttaggaaaa aaaagttttg ggtttggatt tttatttttt tgtagtttag    2580
   gaggaataat ttggagggag gttggaaagt ttagtatttt tataatggta gaatggtttt    2640
   tggaattttt ttatttttgt ttttgttaat ttggaggttt ttttttttga taatattttt    2700
   attatttatg ttagttgttt gttgaggaag aatttgttta ggttagggtt tatattttag    2760
   ggattattta tttagtattt tttttattag ggtttttttt ttttgtttgg atttaaggga    2820
   aataaggaga agttttagtt aggtgaaagg ggtaggggtt agaggttagt gggattggag    2880
   ttggagatgt tttatgttgt tggtagggtt tgtatatgag gttttattgg gtttttagtg    2940
   gttattgggt agttttaatg ggttagatta tagtggatta aagtttagtt tttttattta    3000
   gttaattttg taggggttga aaaggatttt tattgtttgt tttatttttt agtgggtttt     3060
   tttttttaga agatgggttt ttaagatttt tttgagggta gggtaggtag gtggtagggg     3120
   gtatttattt tgtttagggt tttggtttta tatttattgt ttttatttgt tgtagggttt    3180
   ttttaggggt gaagttgtta gttttattta agtattttgt tttttatttg atgagaaggt    3240
   tttgtaggta ggtaaattta tagtgtgtgg tgtttttaat taggagagga gataggaggg    3300
   gttttaatgt tttagtggag aaattttgtt tggattttat tttttttttt ttgtttagaa     3360
   atgttggtgt taaaagtaga gtttgagggt agttttatgg gtaggagggt aggggaatta    3420
   tttgttttgt agttgagtat aggatatttg gtattgtttt tagtttgggg gatattggag    3480
   tgattagtat tagtttttgt ttttggatag tttagttttt gttttttaga aagtttttgg 3540
   ttaatgggag atggtatagt gattaggtta aataggagta tggaaggaag ttatagtgga 3600
   gaagttaggg tagtgggttt ttgtattatt ggggtgatta gggagagtag gatgttttag 3660
   taggggtttg aagataggta gggggttggg ttgttgggaa tgggatgaag taggaattgg 3720
   agttgggatt ttaaagaaaa tttgtggttt atagggtgtt atttttatgg ttttatttat 3780
   tttgtgtgtg tttagagtat atatatatag ggatatatat aggggttatg tattgtggat 3840
   gattatttat aagtttaaga attagtaatg ttaaatattg tttagggata tgtgtatttt 3900
   atataatata tataagttta agtttgggat agtggttatt ttttagggag attgggtagg 3960
   agattatggg tatttataag tgtttttggg tttgagttgg tggttatttt agagtggtta 4020
   ttttattgtt gtgttttgta atttttatat gaatatgtgg tgtttggtat gtgtaattgt 4080
   tatataatag aaggtttttt aataaggagg gtagtgaatg gtattattgg aggtggggag 4140
   ttgggtaggg aaggtaaata aagatatttg gtgttttttt atgtgatgaa ttttgttttt 4200
   atatatttgt aagtgttttt tgtgattttt ttgttatttt tattatttta tgaaaatggg 4260
   gtgtagatgt tgggtattat ggtttatgtt tgtaatttta gtattttggg aggttgaggt 4320
   gggtggatta tgaggttagg agtttgagat tagtttggtt aatatgatga aattttgttt 4380
   ttgttaaaaa tataaaaatt agttaggttt ggtattaggt gtttgtaatt ttagttattt 4440
   aggaggttga ggtaggagaa ttattggaat ttaggaggtg gaggttgtgg tgagttaaga 4500
   ttatgttatg gtattttagt ttgggtaaaa gagtgaaatt ttgttttaaa aaaaaaaaaa 4560
   aagaaaaaga aaaagaaaag aaaagaaaat gaggtataga gaatgtaaga attgtgtttg 4620
   ttttagtttt atagtgtata aggttggagt gggatgtaga atttatatat ttggttttag 4680
   gttgttttat tgtagagtta tatggttttt ttttaagtta aaaatgttta gtttttattt 4740
   ggtaggttag taggtagggt tggggttttt attttgttta gtttttaagt gtttttagtg 4800
   ttatgttggt tgtttttgtt taggtttttt tgttatggtt ttgtgtaggg tggtgtttat 4860
   attagaattt tttgtagatt gattagtagt atttttattt aatattgatg ggtagtgaga 4920
   gttggtagga agaatggggt ttttaggttg gagttggagt gtgtgattgt tattttttgg 4980
   tttgggtaag ttatttttat ttttttagtt tttttattta taaaatgtgg ataataatgt 5040
   tatttgtttt ttagggatat gaggattaaa tgagtttatg gtgtgattaa ttatatttta 5100
   tatttttatt gataatattt ggggtggttt ttaaggatgg gaaagatatt ttaagatgtt 5160
   gtttattaag tgggaggtaa gaataaggta tagtttttat tagtttgggt gttaggttag 5220
   ttatgaattt atggggtttt tgatataaaa atttagtagt gttgaaggta tatttttagg 5280
   gtttttatat aagattgttg gaaagtgggt tttattattt tataattgtt ttgataggtt 5340
   ttagattttt ttttagttgg agttagtggt gtttaataat gtataggtta gagaggtgtg 5400
   gggatttttt ttttgtgtat tttttgatag tttttgttat tggttatttg tggtatttaa 5460
   ggttggttat tagggtgtag tagaggtatg tttatagtaa agttgttggt ttgttttagg 5520
   tttatttatt tttaattttt ttaaatttgt tttttataaa aagtttttta aatagtaaga 5580
   attttttggg tagagaggaa gagaggaatt gtaggtagag gggttaaggg gaggtaagtt 5640
   agtttgatat tataggtgga tattggaaaa tatttaggtt gtatttagtt gggtagtttt 5700
   tgtagggtgg gtttggtagg ggtggtggta ggggaggagt gtagataggg gaaaggtttg 5760
   tgtgtttttt tatatttttt tttaggtatg aatttttatt ttgagggagt ttggtgtagg 5820
   gttggagttg tagattttag agttgttttt tgttttgggt tttttggtag gttttttgtt 5880
   tatagtattt ttttaatttt gtaaggaggg tgggtgtggg gagttttgga tatgaaatga 5940
   gataatttat gtttagttag tggtatagga gtgtttgatg ttttttggta attgttatta 6000
   tgattatttt tttagagtaa tagtttttta aaaagttttg tggtttttgg gatagttttt 6060
   taatttatta aaattttggt atattgtatt tattttgtgg atgatgaaaa ttgaggtttt 6120
   ggaagattaa gagatttgtg ttttttgttt tattgggttt tttagttaag tgttttttta 6180
   gaataaagtg gttttttttg ttgtattttt ttttgtattt tttttttttt tattttaatg 6240
   agaaatgaag ggatagagag aggaagggga aggaaatgtt tgggtttatt ttttttgggg 6300
   tgttattgtt aggagtgggt tgaattgtgg ttttttaggg gtttttattt atttaaagat 6360
   atggttaagg tttaggtttt ggaatttatg attgtgattt tatttggaaa atgttttttt 6420
   ttatagattt attaaggatt tttaaatggg attatttatt tagggtaggt tttaagttta 6480
   aagataggta tttttgttta aagataagaa ggaaagaggg taggtttggt ggtttatttt 6540
   tgtaatttta gtattttggg aggttaagta aggtgggtag attatttgag gttaggagtt 6600
   taagattagt ttggttaata tggtgaaatt ttgtttttat taaaaatata aaaattaagt 6660
   taggtgggtg gtatgtgatt gtaattttag atatttggga ggttgaggta ggagaattat 6720
   ttgaatttag gaggtagagg ttgtagtgag ttgagattgt aatattgtat tttagtttgg 6780
   gtgatagaat gagattttgt tttaaataaa aaaaaaaaaa aaaaaaaagg aagaggaggg 6840
   atgggataga gatataggga agaaggtttt gtgataatgg ggtagagatt agagtatgtg 6900
   atgtttttat aatataatta ggaatgttag gtgttattag aaggaggaag gtatggatga 6960
   ggttggtttt tagggtttta gaaggaaata gtttttgatt ttggattttt ggtttttgga 7020
   attgtgaaaa tatgtttgtg ttgttttaag tttttttgtt tgtgttaatt ggttataata 7080
   gttatttaag agattaaggt gtgagggttt taggaatttt agtagggtaa ggggttgagg 7140
   gtttatttgg tttatagagt tatagtttat ttttttttta ggtttagaga ggttgggtga 7200
   tttgtttagg gttatatagg tgtttatgaa agagtttgta ttaggattta gaatttggtg 7260
   gtttttattt tagtttttgt ttttttgaat ttttttttgt ttggggggag gtttggggtg 7320
   gaggtatttg tataggttat ttgttttttt atttttttaa gggttgaatg gggataagga 7380
   gatttttttt tttgggtggg gtaaggtagt ggatgaggtt ttatgagttt ttggtttagg 7440
   tgtggtttat aggttttggg ttagtagaag agattgtttt tttatttgtt tggagttttg 7500
   gtgtttgtgg tgagtatttt tgatggtatt ttatttggtt ttttttttaa ggattttttt 7560
   gttgttgatt tggtatttgt ggttattgtt tattatagta aatgggatta ttttttgggg 7620
   aatgggtaga tggtattggt tatatatgtg tgggaaggta attttttttt ttgggttttt 7680
   gggaggagtt gttttgagtt tggggttttt taaaaatgag agttttagtt tgatagtatt 7740
   gggtatagat atttgggttt agtgtttttt ggttttaaga atataatttt atggtttaag 7800
   gttttttaaa tttttaattt attttttagt aggaattggg ggttttttgg gaataatatt 7860
   ttagtggatt tatttatttg gaattttttg tttattagtt ggttttttga gtttttatta 7920
   aatttttttt gggggtatat gttgatgttg ttggatagta ggtttgtggt gatttgggat 7980
   aaggagggag agagatgggt ttatttttgt ggtaagtttt atttatttat tggtgtttgt 8040
   tatatgattt ttatattgtg tgttgtttta ggtttgggtt taagggtagg ttttgggggg 8100
   ttgtgatatt aattattttt aatttttgtg ttttttggag ggaagagggg tagttgtagg 8160
   tgtatttgtt gttttgagtg atgttgtaat tttttagggg tttaaggtta ttgttttgag 8220
   tattttttat tttttttttt ttgtttatta tagttttaag aaagtattta gggttattga 8280
   gatttttttt tttgaaattt tttttaaaaa atttttgtta tagtttttag ttttagtagg 8340
   ggttttgttt gtagtaattt tttagttttt tgttagtagt ttgatatggg tatgtgtaat 8400
   agaaatattt tagtgagtgg ttgtttgttt gtttaggtat agattatagt ttgtgtgtag 8460
   tttttggtag atatatttat tttgtgtgga ttgttttttt ttgtatatgt tgggaaggtt 8520
   tttgatattt ggttttagga tggagtaggt tataggtttt atttatattt attgaggtag 8580
   atgtggtgtt gtagtttagg tattttggtt ttattttgaa aggtatttta tgtggttttg 8640
   atgtgttgtt ttgttaggag gaagagttta gggttgtttt tttgtttgga tgtttttttt 8700
   tattttatag taggggtttt agatggtggt ggagaagaag gtggtagaga ttattttttt 8760
   tgaaattttt tttttttttt ttttttttga gatggagttt tgttttgtta tttaggttgg 8820
   agtgtagtgg tttaattttg gtttattgaa gtttttgttt tttgggttta agagattttt 8880
   ttgttttagt tttttaagta gttgggatta taggtgtttg ttattatgtt tgattaatat 8940
   ttttgtattt ttagtagaga tggggtttta ttatgttggt tttaaatttt tgattttaag 9000
   tgatttgttt gttttggttt tttaaagtgt tgggattata ggtgtgagtt attatatttg 9060
   gtttagtttt ttttttattt aataaaaatt aggtagggtg aggtgaggtg gtttatgttt 9120
   gtaattttag tattttggga ggttgaggta ggaggatttt ttgagtttaa gggttagaga 9180
   ttagtttggg taatatggta aaattttgtt tttattaaaa atataaaaat tagttagata 9240
   tggtggtgtg tgtttgtaat tttagttatt tggggattga gtttgggagg tggaggttgt 9300
   agtgagttat gattgtgtta ttatattgta attttggtaa ttgagttaga ttttttttta 9360
   aaaaaaaaaa gggggggtat aaaataaggt gggttatgtt gtatggtttt tgtttggttt 9420
   tattttttgg aagagagatt tattatttgt ttggtttagg ttttttttaa aatttttttt 9480
   agagattttg ttggagaatt ggtattttta taaagttgtt tttgagatat gggggagttg 9540
   gagaggttat tttagggatg gtttttagta gttatttggg ttagggatta ttaggttagt 9600
   tatttgttgg ggagagtgat tagtggtttg gtttttttga tagtgtgatt tttttagatg 9660
   gggtggtttt tgttaggttt gtagtgtttt taggttgtat agaagggttg gggtaagtag 9720
   agatggaatt ttatttgttg tttgaagtgg attttttttt ttagggtgag tgtgtttgtt 9780
   ttggtgatga tagggatgat gttgattatt ttgtttaggt gttttataaa tttgatgttt 9840
   aggggtttga ggttgaagta gagggatgtg tgtgaggttt atttggggga gtggggtagt 9900
   gggggggtat ttatttttaa gtaggtgttg atatttttag gtgttggttt ggggtttatg 9960
   ggggtttttg ttggtaagga tagggttttg ggttgagttg gttatgttgt gagtgtgggg 10020
   tagaggaagg atatgggtgt gttttagata taggtgatat tattgtatag gttaggtggg 10080
   ttagggttag ttttaagtat ttttgtttat gagttttggg gttttaatta tatttttata 10140
   tgtttatata gaagggttgt tgttggttta gtagttgttt tgttgtgggg ttttggttgg 10200
   gaggggagag gtaagagaaa tagaagttag tattagtgtg tggtgttttt gtattaggtg 10260
   ggttgttttt ttgtattagg tgggttgttt tttttgtttg tttggtttta gtgagtttgg 10320
   ggtttgatgt ggggaaagtt gtgggtggta ggggttggta gggttttgga attggggtat 10380
   tatttttttt agttttggag ttgagttttt tttatagtga gaataaattg gttatatata 10440
   tggttgggga gaagtttgtt ttgggtagaa gtttaagtag agatggttaa ggtttgtttt 10500
   tgttttatgg gattggatgt tttgggtggg attaggtaat atgtttagtt ggagttttag 10560
   ttaaagggtt agtgttgggg agtggtagaa gtgttataga ttgtttgttt gtttttggtt 10620
   tgtttagttg gtattaggag aggtttttgt tttttgttat tagttttagt tgaggtaaag 10680
   tttggagtag ttggaatagt tgttatagga ttgtaggtga gttgttttgt ttgggagggt 10740
   agatgtttag tttaggagag gtttatggat agtgggttgg ttttttgttt ttgatttttg 10800
   ttggtttata gaggatgatg ttagagggtg ggtttgaaaa ttttggtttg ggttttttta 10860
   tagattttgg gtgaggtttg ttgtttatta gttaggtttt tttttgtatt tggtgtttat 10920
   agagtatggt ttttttaaat ttttgggttt tttttgttta gggaagtata tttgggatag 10980
   taggtgtgaa ggattttttt tatttattta tgtagataag ttatttatgg ttgtttttat 11040
   tagttatttt tgttggtttt tggttttttt ttttttagtt ttggtttagg gtgagttata 11100
   ggtgtttggt agggggtgta tagggagttg aggatagagg gtttgtgagg ttatgaagag 11160
   tttatttgtt agggtatatg tagttttttg tttttgttga tttttagggg tgatgttagg 11220
   tgtggataga gttggtggga ggaaagggtt tttattaata ttaatagtag ttttgtttag 11280
   ttttggaagg ggttagtatt gatttttgtg ttttttgttt aagaatgtat taaatttttt 11340
   aaggggtttt atgaggtagg gtttggatga gaaggttagt tattgttatg ggtgtttggt 11400
   tgggggtttt tattttattt gttagagttt ggtttgagaa ggtaggatat agattattag 11460
   gttttagaat agtttagggt ttgtttgatt tttagttttt taagatgtag ttgaaggatg 11520
   ggggttgggt aggatatttg tataggggtt agtgtgaggt tttggaggtg tagatatggt 11580
   atatttttaa ttttatagtt ggtggttatg gtttttgatt tagttttttt gtatttagta 11640
   gtagggtaga tagtattgtt ttgtagtttt aggggataga gggaatttag ggtatttggt 11700
   atgaggatgt tgatattgta gggatgtatg agtggttggt ggtggggatg aagtagaggt 11760
   agtagtggat gtgggtgttt gggatgtgtt ttttgtggtt gatgttgatt tttttttgta 11820
   ggtatttttt gtattggtta ttgatgaatt ttatgatggg ttgttagttg tgtgggggat 11880
   ggggatgagt tagtaggtat atgagatgtt ttttttaagt aggttgtgta tttttaggat 11940
   ttagagttta atattttatt ttttaatgaa atgtatgttt tgggaggttt ggtttgttga 12000
   gtggagatgt tatagtttta tttttggttt tttaaattag tttttttggg ttggttttag 12060
   gtgatgagtt ggggatagta agatttttta ggtaattttg ttttggttta gaatttttag 12120
   gtttttgaga tgtgtggttt tggtagtatg ttaggtttta ggaggtgggt tttgtatttt 12180
   tgtgggttgg gaaattttta gggttgattt tgggtggggt ttagggagtt ttgaagtttt 12240
   tgttagtgtt tttttttttg gaggggtttt attagttttt gttgttgatg tggtttttga 12300
   attttggtgt gttaattatt gttagtttta tttggatgtt ttttttttta atatttgtat 12360
   agagttatgg atgtagagtt agaggggaga tggaagattt ttatagatat tttatatttg 12420
   attgattttg gggtaatttt aggtaggtag tgatgataat taaggagata ttgggaaatt 12480
   aatggttttt tttagttaat tttggtatag gttggtgatg gtttttttgg gagttttttg 12540
   tttgttagat ttttggtgga aaatttagtt gtttgtgggg agtttagtgt gatggggttt 12600
   ataggggttt gggatattga tttattatag ttagagtttt aatatgttta atatgttggg 12660
   gatggtgggt atagtgagtt tttgttttgg ggatttggga ttgtttgatt tgggtatttt 12720
   tatattgtat tagtgtttta ggaaggtatg ggtattttat atttttagtt tatttttagt 12780
   tattgattgt gtgtgatggt tttgattttt aatttttgtg ttttaggaag gtaggggtgt 12840
   tttgtatttt tagtttattt ttggttattg attgtgtgtg atggttttga ttttttattt 12900
   tgtgttttag gaaggtagag gagttttgta tttttagttt gtttttggtt attgattgtg 12960
   tgtgatggtt ttgattttta atttttgtgt tttaggaagg taggggtgtt ttgtattttt 13020
   agtttatttt tggttattga ttgtgtgtga tggatttgat tttgatggtt ttggggatgt 13080
   gtttttttga ggtgggttgt attgattttt ggttgatttt ggatttgaag agggtgttga 13140
   ttaaggtgga tttatttaag ttgttttgtt ttggggagag gatgggaggt tggttagggg 13200
   tttttttgga gagattagta gggtttgtat atggggtttt ttttatttag gtatgaggtt 13260
   ttttagtttg agatttagta gtgtttgggt tattttttgg taatattttt gttaagtagt 13320
   ttttgaggat tttatttttg agttgggtgt ttagtgattg tgtaaattta tatagttggg 13380
   ttttgatgtt agtataattt aggttatagt ttttttttga gaggtgagtt gggtttggga 13440
   gagttggtat tttttgtgtt ttttgtgttt tttggatagg aggttttttg gtggtttaga 13500
   ttttggatag gatagaggtt gggttggtgg aggagtttag ggttgtttag ggtgtattag 13560
   gtggttggga tttttgtagt ttattagggt ggttgtaatt aaagtttagg tgtttaaatt 13620
   gttttttttt gattggttag ggtttgggta agttatttga ttttattgtg ttttagtttt 13680
   ttgatttgta aaatgggata aatagttttt tttttgtgga gttggtgtga gtattaggta 13740
   ggtttatgta tgttttatgg ggtttgtggg gttatgtgtt agggggtgtt ggtttgtagg 13800
   gtggtgttat ttgagggata gtgattgtgt gtgtagatag ttagtttata ggtgattgag 13860
   ataaatgagg atgaggaagt agtatttata tgtaatataa tatatataat ttatatatat 13920
   tttatatata tattttataa tatatatatt ttatatgtat aatttataat atatatataa 13980
   tttattttaa atatattttt tatatatatt ttatatataa tttttataat atatataaat 14040
   aaattatata taatatgtgt gtgtgtgtgt gtatatatat atatatatat atatatatat 14100
   atatattttt ttagatttgg agttttattg ttttattttg tatagagttg ttttgggtta 14160
   tgttagtaat atattaaaaa aaatatttta ggttaggtgt ggtggtttat gtttgtagtt 14220
   ttagtatttt gggaggttga ggtaggagga ttgtttgagt ttaggagttt aagattagtt 14280
   tgggtaatat agagagattt tgtttttata ttaaaatgta aatattggtt aggtgtagtg 14340
   gtgagtattt atagttttag ttatgtagga ggttgaagta ggagaattgt ttaagtttag 14400
   gagtgtgagg ttgtagtgag ttaggattat tgttatttta gagtgataga gattttgttt 14460
   tttaaaaaaa taaaaattgg ttaggtgaga tggtttatgt ttgtaatttt agtattttgg 14520
   gaggttgagg agggtggatt atttgaggtt aggagtttga gattagtttg gttaatatgg 14580
   tgaaattttg tttttattaa aaaatataaa aattagttag gtatggtggt atatgtttga 14640
   ggtatgagaa ttatttgaat ttaggatgtg gaggttgtag tgagttaaga ttatgttatt 14700
   gtattttagt ttgggtaata atgagatttt gttttaaaaa aatataatat aaaaaatatt 14760
   ttaaattata ttgatgtttt gattttgata atattttttt aggaaaagtg tgggtttttg 14820
   ggaggataga ggttggtgtt gggaattttt ggtttgaatt gtttagtagt ttaaggatgg 14880
   atattttttg tttatttttt tttgtttatt aggtttgtgt tttggttaat tttaggttag 14940
   gagttatgtt aatgttttag ttatgatatt ttagatttag gagatgttaa ttgggatatt 15000
   gtaaggtgta tttttttatt attgttattt ttattattat tattattatt attattattt 15060
   ttattattat tattattttt attagtatta ttattataat attattattg ttattattat 15120
   tattttatta tttttgttat tattattatt tttgttattt ttattattat tattattgtt 15180
   attataatta ttagtattag tattattatt aattatatta ttattattat tttattatta 15240
   ttattattaa tattattatt atttttatta ttattattat tattattaat attattattt 15300
   ttattaatat tattattatt tatattatta ttataattat tattattata attattatta 15360
   ttattttttt tattattatt ataattatta ttattattat tattattata attattatta 15420
   tttatattat tattattatt tttattataa ttattattat tatttatatt attattatta 15480
   tttatattat tataattatt attattattt ttttattatt attattatta ttattattat 15540
   aattattatt attatttttt ttattattat tataattatt attattattt tttttattat 15600
   tattataatt attattatta ttttttttat tattataatt attagtatta tttttattat 15660
   tattattata attattatta ttattattat tattattatt attattataa ttataattat 15720
   tataattatt attatttata ttattattat tataattatt attgttatta ttattataat 15780
   tattattatt atttatatta ttattattat aattattatt attatttata ttattattat 15840
   tataattatt attattatta ttataattat tattattatt tatattatta ttattataat 15900
   tattattatt atttatatta ttattattat aattattatt attattttta ttataattat 15960
   tattattatt tttattatta ttattattat tattattata attattatta ttataattat 16020
   tattattata attattatta ttattattat tattattatt attattataa ttattattat 16080
   tattattatt ataattatta ttattattat tattattatt attattatta taattattat 16140
   tattattatt attattatta ttattattta tattattatt attattttat tattgttatt 16200
   attattatta ttattatttt tattattttt atgattatta ttattattat ttttattatt 16260
   attattatta ttattataat tattattatt tatattatta ttattatttt attattatta 16320
   ttattattat tattattatt tttattattt ttattagtat tattatttta ttattattat 16380
   tgttattatt atttttatta tttttattat tataattatt attattattg ttgtaaattt 16440
   ttgaataatt ataatttgtt ggggattttg tttggggttt agtttgtttg attttatttt 16500
   atgggatgat tttattttat ggatgaggaa gttaaggttt atagaggagg agtttagggg 16560
   aagatatata agttttattt tgttgagtta ggataaagtt gggatttttt ttggggtttg 16620
   tggattttta aataggtgtt tttttatttt gtttgtattt ttgtaggtgg gtatagttgt 16680
   tatgggaggg aggagggagg agagtagagt tgttttatgt tttgtttttt atttggtatt 16740
   tttgggaggg agtgattttg tggggaatta ggattattgt tttttttagg gttgaaaggg 16800
   tgaagaggtt tagataggtg aagtggtttt ttgtgtatta tttttagagg gtaaggttgt 16860
   tatagtggta gttatggaag gtgagggttg ttgggttgtt tttttttttt tttggggtta 16920
   aagttttgga agaaggtttg tgttatggtt gggatgtaga ggtttttagg ttttttttat 16980
   tttgtttttg gttagggagt agggtagttt ttttgtagtt tatagagatg agttttagtt 17040
   tggttattgt ttttgggttt tttttttttt ttttttttta gttatttttt tgggaggtag 17100
   ttttgaattt ttagggtata agtatgtttt ttataatagg tagaataggg tgggggttgt 17160
   aggaggggat tttatatttt tgtttttttt tgaagtaggt tgttagtttt taggtagttg 17220
   tgagtatagt agggttatga gtagttgggt tttatgaggt tataggaagt agagaggata 17280
   ttaagatttt aggggagtta gggtttagat tagtgtagaa gaggaaagaa aggtgttggg 17340
   ttggtaggtg tgggtttgtg tttatagagt tgggtaggaa ggatgtgtat ggttggtaaa 17400
   ggttagagaa ataggtttgg tttgagaggt gtttagatat tttgtagaaa ttttgtaagt 17460
   ttatttttgt ttagggtgtg atttggtggg ggtatttttt tagatttttt tttggatttt 17520
   gtggagtggg tagaatatag ttttgggagt taaaatggtg gttgtggttt tttgttttgt 17580
   gagttaagga attttggtag ttttttttga gttttgtttt tgtttggaaa atgggatgtt 17640
   ttttttatag ggtttttgtg aggtttgaat gggatttggg gggttttgga aataataggt 17700
   ggtttattgt agattttagt ttggtttttt tattttgttt aggttgtgtt gattgatgat 17760
   tttttgtggt gatttttagt tttttggggt tattaggggg tgtggaagtt atgaggtttt 17820
   aattttaggg tagagttgaa gttgagttgt tgggaagttt ttggttgggg tgtggtgggg 17880
   tgtggtgggg agtttggagg gttaggttgg agtaatagtg aggggatagt tgttatagaa 17940
   ttttttgtat aagttgatgt tattgtttag aggtagtgat tatttattag gatttgtttt 18000
   aggttggttt gggtttttta aagttatttt aggggttttg gatgtgtagg ataggttttt 18060
   ttgaggagtt gttggaggtt gtgggttttt tttaggtgta tgtatgtata tgtaatgtta 18120
   ttggtaattt gtttttgagg ttagggttgt tgttttattt tatagaaggg aagattaagt 18180
   ttaaaggttt gtgggttgtt ttgggggttg aggttggttt ttggtattgg ttgagtggta 18240
   tgtaaggtga ggatttattg attattatga tgttgaattt gaagttttgt tttatggttt 18300
   tttggtgtat ttgttttagg atggagttaa tttttatgta gttgaagttt attggggttt 18360
   ttttgttttg tgatgtaggt gtttgtttga gtttggtatt tttgggggtg ttggttatgt 18420
   tgttaatgta gttgggagtt gttttagtgg ttggggaagg aatagtagag gagttagagt 18480
   ggaatagggg tttatatata ggtgtttgtt aggtggttgt tatatttatt tttggatggg 18540
   gaaattgagg tattgaggtt tagaaaggtt aagtgatttg tttaggtgat ttttgagaat 18600
   ttttttgggt ggatgttttt ggttgtggtg ttttttaaaa agttgttatt ttgtagttat 18660
   gttgtttttt tagtaaagtt ttatttttag atttttaagg ttaggttttt ttattttttt 18720
   ttttaggagt tgtgttaagt tgggggtttt ttgtgtttta aggtagggtt gattttgaat 18780
   ttttgttttt gtattttgga gggttgttta gataagggtg gagggtgttt tttttttttg 18840
   gttttttata ttgttttggg ttatttaggt ggatttggtt ttttttttta tttttattta 18900
   gataagttgt attgtttgtt gttatttaaa ggttttagaa gttgatttat gagttaagat 18960
   ataggaggga tggttttgta agaaatgggg atagaattag tgtgtgtttt tgttttttat 19020
   atttttatgg atggttttgt aagaaagggg gatagaatta gtgttggttt ttatggagtt 19080
   gtgtgtttaa ttttatagaa tgagttagta gttttgggag aaggaaaagt gttgggtgta 19140
   gggtgggtgt ttggatgtag agttgttttt gggtttatgg aggagggggg tttttgtatt 19200
   atatttgttt ttttttggat tgatttaggt ggttgttttg tgtgtttggt tagtgtttat 19260
   gggttgtata tttataggtt aagattaagt tttatttttg ggtatgtggg gtatgtggag 19320
   ttgaattttt tgaaattatt aaagtttttt ttgtttgtag gtttttttga tggttttgtt 19380
   tttggttgtt gttaagatat tatatatggg ttagaggttt ggggttgggg gtggatgttt 19440
   ggaagatgtt tgttgaggat ttaggtgttt ttatatagtg gggttttagg ggtatttagg 19500
   atggggttgg tttttattag ggttggttgg tatatgggtt aggttttagt atattttaga 19560
   tttgttatat ttattggtgt atgtgtatgt aggtgtagat aatttgttgg ttatagaggt 19620
   ggtgtttttg ttagttttta taggttggag ttgtttttgg tggtttttag tttttatgta 19680
   ggttgggtgg ttgttgtttt gtgtttttgt ttttgtttgg gtttatttag gttttgtttt 19740
   gtttgtgggg agatttttgt tttattttgt tttttaggga ggatttggtt ttaggtattt 19800
   tgtgttaaga tagggattat atggtatgtt tttaggtagg gttttttttg ttagtttttt 19860
   gggaggggag tggttttttt ttgggattgg tgggtattta gtaggggtag ttatagggga 19920
   gtggagttgg tgtgggagtt agggttatag tatgggaggt tgtagggggt ttattttttg 19980
   tgataggtaa gtgagtagtt tttttagata ggaggtagtt tttttggttt atgtttattt 20040
   tttattttgt ttagaaggtg ggaggtaggt ttttaggtta ggaggggtag tgggagattt 20100
   tagtttaatt gtttttttat ttgtggggta agaattgaat ttaggaaatg tttattgagt 20160
   tttttggttg tagtttgtat ttttagttga tttaagttat tttttttggt ttattttatt 20220
   ggagtggtta gagggtgttg gagggaggtt ttttaggttt gaggtattag aggtgagaga 20280
   ggaaaagatt ttggaagtta gaaattgggg gttggtttat ttttataggt atttttttgt 20340
   ttttttagtt tttatagggt ataggaattt ttattttatg gaattggggg aataaaggaa 20400
   taatataatt ttttgttggg aaatgttaag ggggggttta tatattttta ttgttgagag 20460
   taatatttat aggtggattt ggaggttagg gttttttttt ttgtgttttt atgtggtttt 20520
   tatttaggtt atgtagtttg gaagttttta gggttttgtt tttgtttttg tttttttttt 20580
   agaaatattt ttttttgggt ttttaggatt tttaaataaa taaagatata gttttgtatt 20640
   tggttatgga ttatgtggtt ttggggttgt tttttgaagt tagatatggg gttttgtttt 20700
   gtggggtagt agtttagttt ttatttatgt gattgaaaat tgtttttatt tgtttgtggg 20760
   tttgagagtt attttgtttg ggattgggat tagtttttat ttgtttttat ttttttttag 20820
   tattttttaa ttttttttag taggatttgg ggggaggtgt tgttttttga attttttttt 20880
   tttgttttta atgtttttgg agtttagagt gagggagggg ttggtttagt tgtagttggg 20940
   tttttttata ggtattagta tttattgtag aaatttaaat taaattaaat attttgttta 21000
   ttaggggttt tgtttgagaa ataataatat aatgtagttg agttttattt ttgttattga 21060
   tgtagggtag tggtttgttt tttgtatagg tgagagttat ttttgtaatt ttttttttga 21120
   gaggtggttt tgtaggtttt ggttggtttg taggtaggta tgtatgtaga gagtaatgta 21180
   agagtttgtt ttgttttttt atttggttag gtgagttttg gggtttgttt ttaggatttt 21240
   taagtgggaa aagtagagat ttttatattt agagatattt ttattttggt tttaggatat 21300
   tatggtgttt agatagttgg aaataatggt aaagattatt tattttattg tttgtgatta 21360
   tgatattgtt tgaggtttgt gttgtttagt gtggtagtta ttggttatgt gatttttttt 21420
   tttaattgtt ataatgtttg ttatggttat tattttgaag tgtgtagttt agtggtgtta 21480
   agtatatttg tattgttgtg taataaattt ttagaatttt tttattttgt aaaataaatt 21540
   gtttttatta aataattttt tagttttatt ttttaagttt ttggtaattt ttattttgtt 21600
   gtttttttga atttgattat tttatggatt tggtataagt ggaattttat aggattgttt 21660
   ttttgtgatt aaattatttt atttagtgta atatttgaaa ggtttatttt tgttgttgta 21720
   ggtattttaa tttttttttt atggttgagt taaatttttt gtatgtttta tttttttttt 21780
   tttttttttt agatagagtt ttgtttttgt tgtttaggtt ggagtgtaat ggtgtgattt 21840
   tggtttattg aaatttttgt ttttttaggt ttaagtaatt tttttgtttt agttttttaa 21900
   gtagttggga ttataggtat ttgttattat gtttggttaa ttttgtttta gtagagatgg 21960
   ggttttttta tgttggttag gttggttttg aatttgtgat tttagatgat ttatttgttt 22020
   tggtttttta aagtgttgag attataggtg tgagttatta tgtttggttt gttttgtttt 22080
   tgattttttt atttatttga ttgtgggtat ttgggttgtt tttatttgtt ggttattgtg 22140
   aatagagttg ttatgaatgt gggtgtataa atatttttag ttttttaaga tgttgtgtat 22200
   ttttttgaat ttatatttag aagtggaatt atgggatgtt atggtggttt tagtttttgt 22260
   tttttgagga attgttatag tgtgtttata agtgattttt gagtatttga tattgagtta 22320
   gtttaattga ggaatggaat ttttattttt atttaaattt aaatagttat attttataaa 22380
   tggtaattat attggatggt atagaatttt agtttaattt attttttatt aatggggaaa 22440
   ttgaggttta tagagtgatt tttttttttg aaatgttatt gtaagtggtt aggttagtat 22500
   aagattttta ttgaatttgg taattttgaa attttatatt tttgtttgta tgatatgtgg 22560
   ttaatatttg tgattttttt taaagattgt gggttttagt aagatggtag ttgttttgtt 22620
   gagattttta ttgtattttt aggttttgtt ttgttatgaa tatggttgtg taaggatttg 22680
   tttgggtttt tgttttttat ttttttgggt atttagatta attattagta gtatttatgt 22740
   tgatttttag aaaggttttg atttggatag taattgatat gttattttat agttaaagta 22800
   aaatgtagag tttttttttt ggttttgtag ttgggagtta gaggttttag gttttggttt 22860
   ttggtttaaa gtgatttttt gtaagttttt ttagtttggt aaataggtta tgtatttttt 22920
   gatttttttt ttagtttttg ttttgtgagt ttttgggtgt ttgaggttgt ggtttattta 22980
   gtgatatttt tagtttttag tgtagtttgt gagtagtata atatatttgg ttttagaaat 23040
   aattaatata tttgttaata gatgtgtgga gaaatttaat gtgttgagtt aatatagtgg 23100
   attatttttt agttataaaa aggtatgagt gtgtgttaat atagatgaat tttgaaaata 23160
   ttttaattgt aagaaattaa gtataaaaag ttatattttt tagggtttta gttatatgtt 23220
   tagaataagt atatttgtag ggataaagtg tagattggtg atttttaggg gttgggggag 23280
   ggggagtgat tgtgtattga ggatggtgtt tttttttggg gtgaggaaaa tgttttggaa 23340
   ttagaggtga tgtttataga aggttgtgaa aaaatgttat ttaattgtgt tttttgaatt 23400
   ggtggatggt gtaatatgga aattatattt ttatgaggat gggagaggaa gggaaagaag 23460
   ggaaggaggg agggggaagg aaatagggaa ggaaggaaaa aagggagggg aggagggagg 23520
   gatggaagaa gggaaagaag gtagttattt ttagtggagt tggattgaag agtttaatgg 23580
   gtgtagtaaa ttattatggt atttgtattt ttatgtaata aatttgtatg ttttgtatat 23640
   gtattttaga atgtaaagta aaataaaata aagagtttag tgttttttat aagtgtttag 23700
   gagttagttt tatagtttag aggatagttg aggtttagag gttaatattt aggtttgagg 23760
   tttttaagtt ggtgttgtta gattatgttt tagataaatt tttttatttt tttgtagtaa 23820
   gggtgttttt agggtggtag ttaggtggag gtgttaggtg gtgggagatt ttgatagttt 23880
   ttttgtaggt ttttattttg aatttaggtt ttatgttagg gtttttttgt tatttattgg 23940
   tgttggtggt agtaggttat tggttttgtg atggggattg tttttaaatt tgtttatttt 24000
   ggggattagt tttttttttt ttgtaggtgt ttattgggtt attttttttt tagagttgta 24060
   ggtgggttta aggtagggat gaaagggttg taatttatgt agttttgagt tagtattttt 24120
   tgggaatatt tgtatttagt gataaatttt tattttaatt ttaggatttg ttgaggatga 24180
   gggaattaga ttgttgtttg tggttgggat gggggttgta ggatatttgg ttgggaggta 24240
   gtgtttagag aggaggtagt tattggatat gatatatttt aggggtattt atatttatta 24300
   tagttttagg gttgattttg gtttatttgg tttggtgttg agagagggtt tgatatagta 24360
   ggtttgtaaa gtttgatttg gaaattaaga tttaggggtt gttttttttt gtttattttt 24420
   ttttgtgtgt tatgttgttt ggtttatgtt ttagaaatgt ttatataagt tttattagtt 24480
   ttgttggtta aatgtagaga tggtgttgtt ggggtaaggg attttgagtt attgtttaat 24540
   gttattttta gttttagggg agtgatagtt gggtttgtat tttaggataa agtgtttagg 24600
   gttaagttaa tgttattttt ttgttttttt taaatttaat ttatatttgg tttttagagt 24660
   atggattgaa atggttatat ttttatatat tttgtttgtt attgttaatg gtggttaagt 24720
   agaatgtaag gttatttttt ttttgtttat ggaggtttgt gaagttattg attaggttat 24780
   ggtaggggag ttggtggtat ttattatatt ttgttagttt tttaggtttg tgtttaggag 24840
   aaagttgttg gtatttatag ttggtgtttt tgttttggtg aagatttttt tgtttttatt 24900
   tattttggtt tagtgtgggt ttagtagttt ttttattttg ttttgtaata atgtatattg 24960
   gagtagtttt tagttgtttt ttttttgtag gtttttttga atttagattt tggggttagt 25020
   atttttgtgg tagttttatg gttatttgta ggttttttta gaaatttgag tttatggggg 25080
   tattttaggt tttataaatt aatagatgtt ttttgttttt tataaagggg ttttttaaag 25140
   ttgagtgagt ttttgatgta ggtaatgtta ggaggatagt gtttggttag ggttgttttt 25200
   tttttttttt ttattttttt gggtgtttgt aaagtttttt agatttttgt tgaattttgg 25260
   ggaagttttg ttttgtaatt agggtttgtt ttttttgttt ttatatagag tttgtttttt 25320
   gtgtttagat agttttttta tgatggtaat tgaataatga tgtgtgtggt ttttttttat 25380
   tataattttt ttatataggg tttgtgaagt tagatatttt atttattatg tttattttag 25440
   tttggttttt tttagagtag tgtagggttt atggtaagtt ttggtaaaaa gtttggtgaa 25500
   tgaatgaata aatgaagaaa taaagtttat tatagtagtt gaggtttatt gggggttttt 25560
   tgtgttaggt gttattttaa tttttttttt tagagagtgg gttttattaa tgttggttag 25620
   gttggtttta aatttttggt tttaagtgat ttttttattt tggtttttta aagtgttggg 25680
   attatagata taagttattg tattgagttt taggtgttat tttaaatgtt gtttaggagt 25740
   tattataatt gatggattat tatataattt tataaggtag ttattgttat tgttttattt 25800
   tatagataag gaaattgaga ttgagagggt ttttgttaat tatgagggtt gtaaagtgag 25860
   taagtggtag agttaggatt tgaagttagg taggtgagtt ttaggttgtt gatttttttt 25920
   ttaggttagg tttttgggaa ttatggaaag gttggagaag tatggggttt ttgtagttag 25980
   gatttagaag gtgtagttgt ggtgtaggta gtttagtatt gaggttttgg tttttagttt 26040
   tttttttagt tgtttttttt tagggagttg gaggtagagt tttgaattgt taggagaaga 26100
   ttttatttgt tagttaggga aagttgttag ggtttttttt agggtttaga gttattttag 26160
   gtttattaaa gtatattagt attttagttt ttataatttt ttttgtaaga aagatgatgg 26220
   tattagttat tgggttgggt tgggggtttt agattttagg ggttttttta ggtttgttta 26280
   tggttttttt aggatataga tgtttatttg agaaatttta gtgtattagg ggttttatag 26340
   ttaaaataaa gaggtagagt tgttttgggg tttttgttaa agtttttagg tagatttatt 26400
   tagtagatat taaaatgttt attttttatt tgaaaatttt atttttagga ggttgtttta 26460
   ggttgggttg ataaaattta tagagatttt aatgatgatt ttttatttta ggtatgagtg 26520
   agtgggtgga gggagaagtt attgttaatt attaatagtt taggaaatgt aggtaaagga 26580
   aagagaatgg taaggtgata gggttggaaa ttagatatat gtgtagaggt gagtagattt 26640
   tagagaaaga attgttttgg aataagatgg taaagaaatt atagataata atggtagatg 26700
   atgattttgg ggttttagtg gattaggatt ataatattga tttatttgat aagtatttat 26760
   tgagtgttta ttttatagta ataaaaagat gatattatag gtttgagagt agttagaatg 26820
   aaaatagtgg tggtatatgg gaatttgaag tttatttatt taaatgttta ttagttgaga 26880
   tttttggata gatttgaagt agtttttaat atgtaaggaa tttataattt gatatagtag 26940
   tataggatat gtgtgtgtta taaatttttg gtgaaaagta aagatttttt ttagaaaagt 27000
   atatattgaa gatagataga tagatatata tgtattttga atggtttatt aaagaatttt 27060
   taggtttgtg gattttagtt aaaaaaataa tttggtattt tgggaggttg aggtgggagg 27120
   attgtttgag tttaggagtt taagattagt ttgggtaatg tagtgagatt ttatttttat 27180
   aaaatgaaat aaaataaaat gagaattaaa aaaaataaat aaatttggtg tgatgagaat 27240
   ttatagaatt taatagggtt attttagtat tatttgtagt attttttaaa tttttgatta 27300
   tatatataaa gttttgggtt ttatagttag agaagaggga gagtaggtta aaaataatta 27360
   tttaaggtgg aagatgagtt tgagaaataa agaggtaaga ggagaagatt gaggttagtt 27420
   gttttttatt tttgagtttt ggtaggagga agtttttaag tgagaataag ttgttgtttg 27480
   tggagtggat tttagagtgg gtggatagag atgataagga atgttatttt tggggggttt 27540
   tgaaaatagg atattttttt attttaattt tgttttttat tgtattgtgt ttttattgat 27600
   ttatttaagt tattataaat attttataaa tgttattggg gattttttgt ttttttttaa 27660
   agatggaaat attgaatata aatgattaaa ttgtgtaaat gtgtgtgggt gggggattaa 27720
   ggattgaatt tttaaaatgt atatagttta tttaatttgt taattaattt taaattaaat 27780
   tatattttat attttgtatg ggataggaag tgttttttta aagtttggtt aggatttggg 27840
   atgttggaaa tttgagattt tgtttttttt ttttgagata ttttgaaatt ttgtttagaa 27900
   attgagtata atggttttgg ttttttagag aaagatatgg atttgtaaag gtaaagagtt 27960
   gtattttttt ttgagttgga gttttttttg gtttttatta tggggtttaa ataaaattgt 28020
   agttttatag tttttttgtt ggtgtttttt tttttttggt gattaataag gtggaagagt 28080
   taataagaat tttgtgtgtt tttttgtagg atttggaagg gataggtggt gtgagtttgt 28140
   ttttatttag tttttttttt tatttattta ggggttgagg tgtttttgag gtagggggtg 28200
   ggggttttta tttttattgt tggttaaggt ttttttagtt ttttttttgt taaatttttt 28260
   taagtaaatt ttttttttat tgtggaggaa ggaattttaa gtagttttgt ttttttgtta 28320
   gtatggtttt aattggtttt agttggtaaa gttaaatttg gataaggttt aagggttttt 28380
   tatattattt gttgtttttt ggaagaggtt tttttttgtt tttttatttg taatttatta 28440
   tttttttagt ttgagttttt ttatttaggt tttgtggttt attgagatat taattttggg 28500
   ttagagtaag ttttgatttg taaattatta ttatttattt tttaaagagt tatttttaga 28560
   atttaggttg tagggagggt aggttaagga tgaatttttt gatttttggt gtggtgtgtg 28620
   gtgggggggg ttattttata ttttgttttt tgttgttttt ttgttttgtt ttaggttttg 28680
   aagtggattt attgtagagg gagggttagg gaatggtttt gatgtttttt ttattttaag 28740
   gaatttttta gtaggagtgg tttttatttt aaagtaattt taagatttag tttgtgttag 28800
   ttttttagtt atgtttatag tggtttgtta attagagatt tttttagttt ttggtaaatt 28860
   tgtaaattta taaagagaaa gaaaagtttt tttgtgggtg ggtggatatt ggttatttgt 28920
   tataggttgg aatttttttt tattagggat gttttttaag taggggttgg ggagggtagg 28980
   gtttagagtt tttttttttt tagttttttt tgttattagt agatttgtta gtagatatat 29040
   agtagagttt agtagtgagg taataatagg ggttgtgggg gttggggttt tgtttttttt 29100
   aatttatgaa attatttgaa ataataaaat ttagataagg gtttttgggg tgtaataggg 29160
   tggttttttg ttaggtgttg ggtagaaaaa aagttttttt ttaatttttt tttgttgtgt 29220
   agattagttt gggttttggg gatgttgtag aggttgtttt atgtttaggt tttttagaga 29280
   tagtaggggg ttggaggtat ggtttgtttg ggggtagggg ataggtaggg aggggtatat 29340
   ttttgggtta tgagtaggag tttgattaaa tgattttttt aggagggaga aagatattgt 29400
   atataattgt ttgattatag ttagaggggt tggaaaggtg ggggaaaggg gtgaaggggt 29460
   taattgtttt aggtgttttt aatttatgta gtatttattt ggattgataa gagttgagag 29520
   attggaattg tttttttttt ttgtttttag gttttgtttt aggagttttt ttgtttagga 29580
   gtttgggagt tgaggaggtt tggaagtagt gggtttatag tgttattgtt ttgggaattt 29640
   atagtgtttt ggatgtggtt tttttggttt tagttttgta ggtagtttat ttggattgta 29700
   tttatttttt agaagagttg tttagaattt tgtatgtttg aaagaaagat taattaaaga 29760
   agtgaggaag ttaaagagag gtttatgaaa gtttatgatg tattttttaa gtgtatattt 29820
   tagtaatttt aaatgttttt tgaagtaagg atggggatgg atggttggat aaatggatgg 29880
   gtgtatgggt gtgtgaatgg gtgagtgggt gggtggatgg atggatagat ggatgaatgg 29940
   atgggtaggt ggatgaatag gtaggtgaat ggatgggttg gtggatgaat gagtaaatgg 30000
   atgaatgatt ggatggatgg gtggatgagt agataggtgg atgagtgaat tggtggatga 30060
   gtggatagat ggatgagtgg atgagtagat gagtgaatgg gtggatgagt ggatgggtgg 30120
   gtgagtggat gagtagataa gtggatgggt ggatgagtgg atgggtagat ggatgggtga 30180
   gtggatgtgt ggatgagtgg atggatggat gagtggatgg atggataagt ggatgggtag 30240
   atgagtggat ggatagatga gaggatgggt agatggatgg gtgagtggat gggtgggtga 30300
   gtagatgggt ggataagtgg atggatagat gagtggatgg gtggatgagt ggatggatat 30360
   atgggtggat agatgggtga gtggatggat gatgagtgga tgggtggatg agtggatggt 30420
   tgagtggatg gataaatggg tgaatgagtg gatgggtaga tgagtggatg gatagatgag 30480
   tgaatgagtg gatgggtgga tgagtggatg gatagatggg tgaatgagtg gatgggtgga 30540
   tgagtgaatg agtgaatgag tggataggtg gatgagtgga tgggtggatg agtgtataga 30600
   tagatgggtg gatgggtgga tgagtggatg ggtgggtgag tggatgggta ggtggatgag 30660
   tggatggata gatgggtaaa tgagtggatg ggtggatgag tgaatgattg tagataggtg 30720
   gatgagtgga tgggtgaatg agtggataga tgggttgata ggtggatgag tggatgggtg 30780
   gatgggtggg tgtgtggatg ggtgagtgag tggatgggtg ggtgggtggt aagatgggtg 30840
   gataggtata tggagtttat ttaaggagtt ttagaaatat atatatattt ttttatttta 30900
   gatttttttt aggaaaaagt agtgtgtatg gattgttatt tggttatatt ttgagatagt 30960
   tatttttatt ttttttggaa tattgtttgg ggggatgtag tatagggtat ttaagtaatt 31020
   tttgagttta gggtagagat attaggaagt agtaggtagg ttgtgatttt gtaggtttgt 31080
   tttatggttt ttgttgtttg tattatggta tgaagtttag atgtattttt ggatttgggg 31140
   ttttgattag atggagttat taattttttg aagaatgaaa tatatagggg ttatttttgt 31200
   tttttagttg tagggaatga tgattattta gttttttttt tttttaaatt ttttttaggt 31260
   ttttgtgtat ggttttgttg attttttttt tgtttttggt tattttttta gtttttgttg 31320
   tttttttttt tgtttttatt tatggagtta gttttggtgt tttgagagtt ttttttttta 31380
   gttgatttta tttagttttt gagtatttaa tgttaataat gttatttttt ttgtttagat 31440
   ttatatttga gtgttagttt gatttataga tgtgtttttg ggttttgttg tgttaggttt 31500
   tttgttgggt tttgggtatt tgtagttatt gttattgtta agttttaata tttgaaggta 31560
   ttttgaattt aatgattgtg ttttaggttg aattttggag tttttatttt atttgttttt 31620
   tattttattt tttatttaag tgaatagttt ttttagttaa tagttattgt agttagattt 31680
   ttaggggttg ggtttgggtt ttttattttt attattttga atttatttat ttattttaaa 31740
   tttttattaa tgttaattta atatatttta tagttattat tattagttgt ttttgtttga 31800
   attttttttt tttaaagtat tagggggtgt gtgtaaaatt tattaatttg atgatttttt 31860
   tttatgaagt tgtgattggt ttttattgta tttgggatat attagatttt tgtggtttta 31920
   gaattttgtt tattattgat gtgttgtgtg attttgagta agtaatttaa ttattttgtg 31980
   ttttaatgtt tttatatgtg aaatgggata ataatatttg ttttataaga atgttgggga 32040
   ttaaatgaga tatttattta tgaagtatat agtgttgtgt ttggttagta aatgtttgaa 32100
   gtttttgtta ataaaatata tgtttttaat ttatttttta gtttttattt atgtatagtt 32160
   tttttggttg ttttttagtt tttttaataa ggttatttat agaggttttt tttgttggga 32220
   ttattttttt tttatgttgt ttttagaatt tttatttgtt tttttgggat agtttattta 32280
   tattgggttt tttagttttt gttttgtttt gttttgtttt gttttttttg agatggagtt 32340
   ttattttgtt gtttaggttg gagtgtagtg gtattatttg ggtttattgt aagttttgtt 32400
   ttttgggttt atgttatttt tttgttttag ttttttgagt agttgggatt ataggtgttt 32460
   gttattatgt ttggttaatt ttttatattt ttagtagaaa tggggtttta ttgtgttagt 32520
   taggatggtt ttgatttttt gattttgtaa tttgtttgtt tttgtttttt aaagtgttga 32580
   gattataggt ttgagatatt atgtttagtt taggtttttt agtttttata tatgtggttt 32640
   tttgattgtt gtttggggtt tgttgtattt attatttata aaattttata tgtttgaagt 32700
   atttagtaag atgttgtgaa tgagtgaatg aataaatgaa tgaatggtta gtttgttgtt 32760
   tttttgtttg gttttttgtt ttttttagat tagtatgggg gtagttatgg tttttgttta 32820
   ttttggtaat attgtttttt ttgtgttggt tgtgttggtt agagtttttt tttatggtgt 32880
   ttttgtggtt ttggtgtgtg gttgtttgtg aatgagattt atgatttttg gtttttatta 32940
   tttttttgga gttataaagt attaaggtgg aaggatggat gggttttttg ggatagttgg 33000
   ggtgggaggt tggtttagga aatttttttt aggaaatggt tagtgtggga tgaggtgttt 33060
   ggtgggaggg gagtggagtg gggatttggg gtttggttgt tttgtagtta gggattttaa 33120
   tttttatttt tttttttgtt tattttttaa ttttagtgtt gggtttaggg tttttggggt 33180
   tttttttagg aatatagaag atatagtaag gattagggat ataggatagg tagagtttat 33240
   gggtaggagg tttttagtta gtatatttga gtttatgttt ggttttaatt agatattgag 33300
   attttggtta ggttattttt tatgttgagg taggggggtt ttttagaggt tattggtttt 33360
   taagtattgt gtggaagata tgttatggag agttagtatt ttttgagata ttagtagaag 33420
   gttagatgtt ttgtggttta aggatgattt ggttttagtg tttatttatt tgttgttttg 33480
   tatgaaaagt ttgatgtgat atggttggtt ttagtggaag agtgatttgg gaattggtag 33540
   agtggattta gtaattggta aattggattt agtaattgag atattgttag gggtttggta 33600
   gaagtttttt aatgtattag atttagtttt gatttttata gagtttagtt gagtattaaa 33660
   aggtgtagag gttgagttga gtattagggg aatattgatt ttagggttga ttggtgttgt 33720
   gtgtgtattt tatagtttat agatggttta tatttatttg attgtatttg attttttttt 33780
   gtgaggtttt ttttagattt tttgtttttt aaagttatta gttgtgtttt ttgaaattag 33840
   agtaaagtaa taaaattgta gtaatagtgt gtttttatga atgttgtgat gttagttggt 33900
   taaatgaggg agtttttagg gttttttata ttgttttgat tttgtgtgtt ttttggtttt 33960
   ggtttatttt ttatattttt tgtttttttt tttttttttt tttttgagga tttagggttt 34020
   ggggttgatt tattgtggga ttttaatggt gattttttgg aagaaatgtt tgtttttttt 34080
   ttttatttag tagtgatttt tggtttgttt atgtttatgt agttaatata ggtagtagtt 34140
   ggaatttgga agtttttggg gtgggagttt gttttttttt aggttttttt agtttgaatt 34200
   ttgggaagga gttatattta tatggtaatt ttttttaagg ttgggagggg gaaggggttt 34260
   ggtttgggtt tgattatttt tttgaggttg tgtttattgg gttttttaag ttagttgggt 34320
   tttttggttg tatttttgag ggatggtaat agattatagt gtaaattgtt tattggttgg 34380
   ttgggtagtg ggtgtttaga tgtgggttag ggtttttgtt ttataggttg tgtggtggtt 34440
   taaagtgggt atttttatgg gtatatattt gtgttatagt ttatttgtgt gtgtgtgtgt 34500
   gtgtgtgtgt gtgtgtgtgt gtgtgtgtgt gtgtgtgtga tagtttagaa atatggtaat 34560
   ttatagtttt tttaagatgt tggttttggg agatttgttt atttgggggt tttagtattt 34620
   ttagtttttt ttaggaaggt tttgtttttt ttggtttgaa gttggtttta gggaagaatt 34680
   tttttagaaa aataaaagtt atttttttta gttttagttt agtttttttt taggggtgag 34740
   ttatattaaa tttatttttt tttttttttt ttattttttt ttttatgttg taattagttg 34800
   taagtggttt atttatttag gtttgttagt ttattataga aaagatattt gaattttgta 34860
   tttttttttt ttgatttgtt ttgggtttta ataggtaaag gaaattggaa gttagtagtt 34920
   aggatttagt tgtaggtttt tggggttatt ttgtgttttt attttagttt tatttatttt 34980
   tgtgttgatt taatgtttat ttattttagt ttatggagtg aggtttggat ttaagataaa 35040
   ataaaggggg ttttggtttt ttggggttta tagtgtttgg tgttggtgag ataattttag 35100
   agtatagtat agagaaatgg ttgtgatatg gatttgtagt atttggttgg ttaagtatag 35160
   taatgtattt ttattaggtt tgggtttttt agtagatata tggtttaaag gttggttggt 35220
   taaattatat gattttgtaa ttatttaaga gaaatgaaaa tatgtttata taaaaatttg 35280
   tagattagta tttattgtag aattttttat aatagaatta tgttaaaaag tagaaataat 35340
   ttaaatgttt attagtagat gaagagatat atagtgtggt ttatttatga tggaatatta 35400
   tttggttatg aaaaagaatg aagtattagt atttgttata atgtggatga gttttgaaaa 35460
   tattttatta agtgaaagga gttagatatt aaggttatat attgtatgat tttatttata 35520
   taaattattt agaataggtg aatttataga gatgaaggta gatgagtggt ggttaggggt 35580
   tgagagtttg gagttgggaa tggggattgg ttaatggggg tagggttttt tttggggatg 35640
   atgaaatgtg ttgggattaa tggtgatggt tgtgtaattg tgatggttaa ttttttgtgt 35700
   tagttgggtg tagtggttta tgtttgtaat tttagtattt tgggaggttg aggtgggtgg 35760
   attatgaggt taggagattg agattatggt gaaattttat ttttattaaa aatataaaaa 35820
   aaaaaattag ttgggtgtgg tggtgggtat ttgtagtttt agttatttgg gaggttgagg 35880
   taggagaatg gtgtgaattt gggaggtgaa gtttgtagtg agttaagatt gtgttattgt 35940
   attttagttt gggtgataga gtgagatttt gttttaaaaa aaaaaaaaaa aatttttgtg 36000
   ttaattggat tgggttatgg ggtatttagg taattggtta aatattattt tgagtgtgtt 36060
   tgtgaaggtg tttttggatg agtttaatat ttaatttttt tattttttag aaagtttagt 36120
   ttattgttag tatttattta attttatata aatatgtttt ttgaggttga agtaaatttg 36180
   attggatgtt ttgtgtgaaa attaaatata aaaattggtt aggtgtggtg gtttatgttt 36240
   gtaattttag tgttttggga agttgatatg ggttaattat ttgaggttag gggtttgaga 36300
   ttagtttggt taatttggtg aaatattgtt tttattaaaa atataaaaaa ttagttatgt 36360
   gtggtggtag gtattttgta attttagtta aatttaggag gttaagattg gagaattatt 36420
   tgaatttagg aggtagaggt tgtagtaagt tgagattgtg ttattgtatt ttagtttggg 36480
   taatagagag agtttgtttt aaaaaataaa aaaattaaaa agaaaaaaaa ttttttaaat 36540
   tgtttttgta gttattttta agtaaaataa tagagttatt tgagttgttt attttggaaa 36600
   aattagattt attaaatgaa tttttagtta ataattgatt aagaataatg ttaatattat 36660
   ttgtaggaat gttgtgtttt ttaggatttg atatttttag tgattgagaa ttattatttt 36720
   tttttaaatt ttatttattt atttattttt tgagatggaa ttttgttttg ttgtttaggt 36780
   tggaatgtaa tggtgtgatt ttggtttatt gtaatttttg ttttttgggt ttaagtaatt 36840
   ttttggtttt ggttttttaa gtagttggga ttataggtgt ttattattat gtttggttaa 36900
   tttttgtatt tttagtagag atgggatttt attatgttgg ttaggttggt ttttaatttt 36960
   tggttttagg tgattagttt gttttagttt ttttaagtgt taggattata ggtatgagtt 37020
   attatattta gttgagaatt attatttttt tttaaaggaa atattattat taaaaatgga 37080
   atgttataaa tagaatgatg ttttttgttt ttaaagttga tatattagag tgatgtgaaa 37140
   ataataataa aagtaagata ttttttggta aatttatttt ggggtgaatg ttgtagttat 37200
   aagtattgtt ggtgagtatt tttggggtaa ataggaaaag ggtgtaatta ggagtttggg 37260
   taaagtagat tgtgtgtttt gatgggggtg ggttttattt gattagttga aggtaatagg 37320
   gtttggaatg tatattttaa gatatagtgt tttggtatag taaatatttt tagttgaagg 37380
   aatttaagaa atggtaggtt taagaagaat ttattgattt gttttttttt tgaagtagat 37440
   tataagtttt ttatgtaaga ggtgtttttt ttatatttag tggaaaggaa tagttttatt 37500
   tttagagata gaggatttga gaggggtttt agtatatagg ttttgttgag atttttatgg 37560
   gtttattgtt tagtttattt tttgttgttt tgttatagtt ttttatgatt ttttattttt 37620
   tattaaattt agtataaaaa tgtttaggtt taggttaggt atagtagttt atatttataa 37680
   ttttagtatt ttggaaggtt aaggtggtta gattatttga ggttaggagt tgaagaatag 37740
   tttggttaat atggtaaaat tttattttta ttaaaaatat aaaaattagt tgggtgtggt 37800
   ggtatatgtt tgtagttgta gttatttggg aggttgaggt atgagaatgg tttgaattta 37860
   ggagatagag gttgtagtga gtagagatta tgttattgta ttttagtttg ggtaaaagat 37920
   aagtaaaaga aataaataaa aaaattgttt aaaataagta ataaataaat ttaaaaaata 37980
   agtaaaataa ataaaaattg tttaggttta ggttgggtat agtggtttat atttgtaatt 38040
   ttagtatttt gggaggttaa ggtaggagga ttgtttgagt ttaggagttt gagattagtt 38100
   tgggtaatat agtgggattt tgtttttata aaaaataaaa aattagttag gtggggtggt 38160
   atatgtttgt agttttagtt atttgggagt ttgagttggg atttaagagt gggtttagag 38220
   tagtttttgg ggttggtagt tgggttttag tttgagttta ggaagttaag gttgtagtga 38280
   gttgtgattg ggttattgtt ttttagtttg gatgtgtgat agagtgagat tttgttttaa 38340
   aataaaataa aaattgttta ggttaatggt ttttttgggt ttttattttt ttatgaaggt 38400
   ttggtgttaa gtaaaattta tttgaattgt tatgtttgtt ttttgttaat ttttgttagt 38460
   ttaattttta gggttttagt taatgaattt aaaattgata gaaggaaagg gttttttttt 38520
   agattttgtt ttgattaggt ttttgtaatt ttgtgataaa ggtttgaagg tttgaatagg 38580
   aggatataag gtttgagtga gagagaattt tttttgttta attgttttta atttggaata 38640
   ttagattttt tttgttttag atttaaattg atatatgtgt tttttttggg ttttgggatt 38700
   ggaatgatgt tattggtttt tttgggtttt gggtttttag gtggatatta gagttgtaat 38760
   atgggttttt ttgtgttttt agtttgttga tttattttgt taattttggt atttgttaat 38820
   ttttgtaatg aggtgagtta attttttata ataataaatt tttttattta aatatatgta 38880
   tatatttttt attggttttg tttttttgga tgattttaat ataattattt tgtgaatata 38940
   gtaaaattat tgaattgtag attttaaaaa ggttaatgtt atagtatgtg aattttattt 39000
   ttataaagtt attatttaaa tagtaataag aagttggtga gttttgtgta tttttttgta 39060
   aataagagta gggatagtgt tgtttttgtt tttggatgtt tttagttttt agtttgagtt 39120
   taggaatatt ttttatgtgt tttttttttt aattttgtag tttaagggtg gggtggtttt 39180
   ttagtttggt attttttttg tagtttttta tttttttttt atattagtta atggatatag 39240
   aggttttttt atggattttt atttttattt atttattttt atttttttga gatagagttt 39300
   tattttgttg tttaggttgg agtgtagtgg tgtgattttg gtttattgta atttttgttt 39360
   tttggatttg agtgattttt ttgttttagt tttttgagta attgggatta taggtatgtg 39420
   ttattatatt tagttaattt tttgtatttt tattagaggt agggtttttt tatgttggtt 39480
   aggttggttt tgaatttttg attttaggtg atttatttgt tttagttttt taaagtgttg 39540
   ggattagagg tgtgagttat taagtttagt ttttttatag atttttaaaa gggttttggg 39600
   taatatagat tgggatttgt taagttatta aatgattttt ttaaataaag aaggaagtta 39660
   taaattttta tttaaaagga gtgggggatg gtaatagaaa tatagttagt tagaagttgg 39720
   aagggtttta tttaatttat ttttaaggtt tttgggtaga agggataagg gaaaatttgt 39780
   tttttaaata ttttatttgg tattttattt tttggatgta agatgtagtt gtgtatagtg 39840
   gtagtagtga ggattttggt ttggtaggtt tttatatttt ttgttgaggg ttttaggggt 39900
   tagggttgtt taataggatt aagaggaaat gattatagga gtttttagtt gttgggggtg 39960
   gtttttgtgg ttttttgggt tatttttggt gaatttattt taaatttgga agaggtttaa 40020
   gtttttttgt tttttgtatt ttaaatttta gttatgttag ttaggagttt tgggggatga 40080
   gaaaaaattt aattaagttt tattgtgttg ggtgatatag gatttagaag gagaggggtt 40140
   tttggattgt ttaagtttaa tgataatttt agtagttggg tttttaagtt aattagtttt 40200
   ttttttttta gtagtttttg gggagtatta tggtgagttg tgaggagtta gtttagtatg 40260
   gtatatataa tgtaggggtt tttttgtatt tttattttat aaatattagg gttttttggg 40320
   ggattagttt tttatttttg agttattttt tttttattgt ttttttaata aggaatagag 40380
   aaggagtttt tattgtttaa ttagatgtag aaattgatgt atgtatagag aggttatgtg 40440
   tgatatggtt tggatgttgt tttttttaaa ttttatgttg aaatgtgatt tttgatgttg 40500
   gaggtaaggt ttgggggggg tggggtttgg gttatggggg tggatttttt atggatggtt 40560
   tggtgttttt tttatggtta taaagtttgg gagatttgat tgttatagag tttgggaggg 40620
   ttgggtgagg tggtttatat ttgtaatttt agtattttgg gaggttaagg tgggtggatt 40680
   ataaggttag gagtttggga ttagtttgat taatatggtg aaattttatt tttagtaaaa 40740
   atataaaaaa attagttggg tgtggtggta tgtgtttgta attttagtta tttaggaggt 40800
   tgaggttgta gtgagttgag gattgtgtta ttgtatttta gtttggttga tatagtgaga 40860
   ttttatttta aaaaaaaaaa gagtttggga gttttttttt ttgttatgtg atgtgttagt 40920
   ttttttttta ttttttatta tgaggaaaag ttttttgggg tttttttaga agttaagtag 40980
   atgttggtat tatttgtata gtttgtagaa ttgtgagtta aataattttt tagttttggt 41040
   tattttttat agtaatttaa aatggatgaa tataatgtat tagttttttt ttttggattt 41100
   tatagtgtgt ttggttgggg ttgtgttttg gggaaatggt aattgtgagg tttttataga 41160
   ttgggtttgg gttttaatta tagttgtatt ttagtgtttt gatattgttg agtggatgtt 41220
   taaaaagtat tgttgggtga atgtgtgagt gaatgatgaa gatggaggtg gttgttggtg 41280
   ttagggtaag gttttaggag tttggggagg tggtattagg tagtttaggt taatggttaa 41340
   ttttggatag ggttaggagg tagttagaat ggttaaggat agttaaggga ttttaggtaa 41400
   gtggttttat tttttttagg tgggtaggaa gtaggaatta gattaagggg tgttaggtgg 41460
   tagtggtatt tagggggttt gaggagtagg tgtaggttta gtagggttgg tggtgttatg 41520
   tgtggggttg attgtggttt tgtttattgt ttttattttt tggatgtatg ggtttatggt 41580
   ttttaaggtt tttttttgtt tttatgttta tttttttagg ttttattttt agatgttaag 41640
   ttattttttt atttttattt tttttgttgt ttttatattt gagttgattt tttttttgag 41700
   ttttggtgaa attttagaga gtttagaggt gtagtttata tttttgtgtt ataaggtttt 41760
   tgtagttttt atttttttgg gatattttat tttagagttg ggtgtgtatt tattttattg 41820
   gattagaaat tttaggaatg tttattttgt atgtttttgt tttttatttt ttgtttgtta 41880
   tttagtttgt ggttgttatg tggttgggtt ttggtagtta ttgtagttgg taatatataa 41940
   ttagtatttt tgtgtttagg ttttgtgttg agttttttta gttttatttt tgtttgtttt 42000
   taaaatggtt tttgatatgg taattattat tatttatttt ttagataaaa gattatagtt 42060
   ttaggagagg aggaatggta ggtttgaggt tatgtggttt gtaataggta aatggatttg 42120
   gtagttattt tatttttgag tggggttttt ttttgtttgt agattttgtt gaggttttag 42180
   agtttggagt tagtgagttt ttattgagta taaggatagt ggtggtgttg gggttggaat 42240
   aaggtaggga ggggtttttt aaattatagt tttggttgtg gatttatttt gggattttgt 42300
   tttggttggg aattttgttt tagtttattt gggatatttt tttattttga gttttgtatt 42360
   gggttgtgta tggggtttgt tgaatgagtg agggtaggag ggatggtgag tagggtggaa 42420
   agtgattatg gtgggtaggt tttgttggaa agtttgaaat atttttggtg agtaggaaaa   42480
   tattagggga agaggttata gtttttagaa gggtttgttg gggattgttt tgtagttttt   42540
   ttattgggat aggtagaaaa tttagttttt ttttagggtg agtttaggaa gtttgtgggg 42600
   gtataggttt tagttagggt ggtgtgtggg tttatttagg ggatgttgtt gtggtttaga 42660
   gttaggttaa attttatgta ttttgtaaat aattatttat aataaataag gtttatttat 42720
   tttttaagaa gagtaaaata ttataggagg tattttttta ttaattaaat atgttatagt 42780
   gattttattg tgttattgag ggagggtatt agggtgtttg gtttagagta aattgtggtt 42840
   agggggatgg gtatattgtt aaattggtta gatttttatt tttgaaaatg tgggttaaat 42900
   tttttttaat aagttataag gaaatttagt gtgaatttta tgtttttatt attattggtt 42960
   gttttggaag attgggagga ggggttttgt ttttttttta tttttggtta tagggtttga 43020
   ggttataatt tggattggga ttttaatttt ttttttgggg aagaggttta tttagggtgt 43080
   ttgttaaggt ttagtgtata aatatggaat ttgtttaatg tttgttttgg tatttttggg 43140
   ttttttatat tgggtttttt tatttgattt tttaagtagt gagagtgatg ggatatgggg 43200
   tgtttttagg ggagatttgg ggagatggag taagtttttt agtttaaggt gaggttttga 43260
   ggtatggttt atgtagtgat gttttttatg ggaggtttga ggtatgatta ggagtttgga 43320
   gatttagtag aggtttgggt atgggatttt tggtatgtag ttagagtgag ggttgaggtt 43380
   gtatggatgt aagtaggtga ttttgtgtta ttgggtgtta tttgtgttgg gttttgtgta 43440
   gttgttttta ttttattgag ttttagggag gtgaggtaat tattttaggt atatagtttt 43500
   gggggtaaag gttggattta ggtgtgtttg attttagaat ttaagaagat aagtagagga 43560
   gtttgagttt tttgagattg tagtgatggt ttagtgtttt aggatggtta ggttggtttt 43620
   tgtagtttta gttaattgtg ttttttttta gttatttagg gttttagtgt tggtttttga 43680
   gatttggtat tggttatagg ggtttatttt tggttgtgga tatttagtgt tggttattgg 43740
   gatttggtat tggttgtggg gatttgtttt tggttgtggg gatttagtgt tggtttttga 43800
   gatttggtat tggttatggg gatttgtttt tggttgtggg tatttggtgt tggtttttga 43860
   gatttggtat tggttatggg ggtttgtttt tggttgtggg tatttggtgt tggtttttga 43920
   gatttggtat tggttatgga ggtttgtttt tggttgtggg tatttggtgt tggtttttga 43980
   gatttggtat tggttgtggg ggtttatttt tggttgtagg gatttagtat tgtttatggg 44040
   gtttgttttt ggttttgggg agttagtagt gtttgtgggg tttatatttg gtggtgggga 44100
   tttggtgttg gaggattttg atagtaaaat tatagaggtg ataggtaata gtaatttatt 44160
   agtatagtgt gtggtttgtt ttagttatgg tatttttttt tattttgtgt ttttttatta 44220
   taaagtttag tgtaggtaat gaagttaaga ggtggagaat ttgtaggagg atgtatatta 44280
   tatttgtttt ttgtgtggtg tttttgtatt ttgggtggtt gagatttttt gttttttttt 44340
   taggtttttt tgtgttaggt ttgtgttgtt ttttttggtt ttggttttta tttatttggt 44400
   tttttagtta tagatagtag tgattttttt ttttatttaa tggggttatt gtttagttta 44460
   ttattagttt ttagggttgg tagatgatat tggttatggg tagtgttttt tttggtttta 44520
   gtatataagg tataaaagat attattttgt tttaagattt ttttagttat gtggtgtatg 44580
   ggaagtggtg ttgggaattt tttagggtag ttttttgttt tgggggagat tgtgtttaaa 44640
   tttttgagga aatgttgttt tttggttttt tttttgaaaa attttttaag gagattttgt 44700
   gtggagtgtg tagggagaat ggagattttt gtgagattgt tttgggatag ttttatagaa 44760
   attataaatt atttggataa aagttgaaaa tttgtggtta tataatgttg attatttatt 44820
   taaatatttg ttgagtaagt atttatgatg tttgggaatt taggatgtgg attgataaga 44880
   tataattttt atgtttgttt tagtgataga gagagagatg gtattattaa ttttgtttgt 44940
   tgggggtttt tttggagggg gtgtgtaggt ttagggttga atttgtttag tggatgtgtt 45000
   atttatgttt gtgtattttt tggttttgtg gtggtttttg gaataaaggt tgagagataa 45060
   aagataagga tttgggggag ggaggatgtt tatgtatatt ttattgggga tattagtaag 45120
   gatgattgtt tagtgattat ttagtgattg agttatttgg tgtttggagt ttggtattta 45180
   tagagtaggg tgggggtagt aggttttgtt gggggtatag gtttgagaag ggagtgttgt 45240
   tagttaggtt tgatgggata gagagtgaag ggtttttttt ttgaaggtta tgtagaggtt 45300
   ggaggttaat gttagtaatt ttattaatgt tagttttttg agattgattt agagttgggg 45360
   ttttaagagt agttttgggt gtttaggagt taagatttta tagagggtgt tttttatttt 45420
   tagtttaggg aaagtgataa atttggagtg tttttgttta tggtttaatt attgaattta 45480
   aatgaatgta ttgataaatt gtatttagtg ttatttatag ggaaaaggga gggaaaaaaa 45540
   aaaaagaaag agagttaatt gtgtttaggt tggttggtta gggggaggat agatgggtta 45600
   ttatttttgt tattttttag gttttttgta ttgatttttt atttttagta aaattaaggt 45660
   ttagtttttt tttgtagttt tggagttggt tgggatgaag taatttttgt aggtagatta 45720
   ttaggtttat tgtgatggtt ttagtaggga atttagttta ggttatagta gtttaggtgt 45780
   ttgtagaagg tttagatttt tgggtgttaa gttttttttt tttgtatttg ttttggggtt 45840
   tggtggggtg ggtagtgtgt gatttttagg gttgatgaaa gagaggagga agaggttgag 45900
   tggtggtgag tttgaatagt taattttggg aggggttgag gtggggaagg tttttattgt 45960
   tgttttatag gttttggagt aaggttttta tgattttatt agtagttggg taggtatagg 46020
   tttaggtgtt gagtgtgtta ggatttttgt tgttttttgg gtgggggtga gagggatttt 46080
   tatgggtttg taattagatt tgtgtttttt tagttaattt tttattaaat aaatttatat 46140
   gtatagtgga ttgggtatgg ttgttatttt tgggagtttt tttgggaaag ttttttaagg 46200
   ttttgttgag ttttgggaat aggtttttat ttgtttggga attgggttgg tatttaataa 46260
   ataaattaaa gtttggtgtt ggaaggtaga attttgggat tagagtttta gttaagggtt 46320
   ataagttttt taggaagttt tttttttaaa gagtggttgt gggatgttgg gagtttttag 46380
   ggtggttagt ttttaggttt ttttaaagtt taggtggtat tgatgttttg tgttaaggtg 46440
   agggttgtag agaggggttg gtggggagga gtttttatag tttattttgg ttattgattt 46500
   tatttttaag tattgaaata aagttttttt tttatatttt tggttaggga gagagggtgg 46560
   aagttttaaa tatttggggt gtatgggaaa ggtttatatg tatagaagaa ttagataatg 46620
   gaagttgttg gtggtggtga taggtttttt aaaggggttt tgagattttt gatattggta 46680
   gggaagttat atattgtatt aggtttgggg gtagggagaa ggggttttta aggatggggt 46740
   tgtttttttt tgtggaagtg ttttgtattg tttgataaga gtagtttgga ttagttgtgg 46800
   tataatagga agttgggaga atatggtggt ttaggaggtt ggttagggtt ggggagtggt 46860
   taatttagag attttggttg tttaagtggg tttagggttt ttggggtgaa agtttttgtg 46920
   gagtatagtg gatttagtgt ttggaggata gattttaggt ttagataagg tttttttttt 46980
   ttttttttga gatagagttt tatttttgtt gtttagattg gagtgtagta gtataatttt 47040
   agtttattgt aatttttatt ttttaggttt aagtgatttt tttgttttag ttttttgagt 47100
   agttgttatt ataggtatgt gttattatgt ttggattagt tagtattttt agtagagata 47160
   gggttttatt atgttggtta ggttggtttt gaatttttga ttttaggtga tttgtttgtt 47220
   ttggtttttt gaagtgttgg gattataggt atgagttatt gtgtttggtt aataaggtta 47280
   tttttgatat ggaatgttgg tttgattata gtgtttgatg gtttttgttt gtaattatgt 47340
   ttttgttttt tattaaagta gggtttttag ggtagaagtt gtattttttt attttagagt 47400
   gttttatgtt attttttagg tgtatatagt gggttttttt tattgtagtt gaattggttt 47460
   tattattatt ataatgatag tttatagggg tgggggttag tgggaatata aagtggttaa 47520
   tgtaatagtg ttttagttat tttgatatag aaatggttag gggatagaag gggtaagttg 47580
   ggagttagta gtaatttagg tttttagaga tgagtattaa aaaaggtgag ttatattata 47640
   ggtttttttt gattatattt tagtttaaaa gagatttaag gagaaataag aattttattt 47700
   ttttaataag gatttgtata ttagatgtta agtaaatatt tttttttttt ttttgaaaag 47760
   gagtttttgg ggttgttggg ggagttggtt aaagagaatg atatttatgg aaatatatgt 47820
   ggttttatta agttttaagg taaggtggta atgaatggag gaggaaaatt tatagtatta 47880
   ttagggaatg gggttttttt ttgttttttt tttttttaat gggttttttt atgtgggatt 47940
   taaattaaat tttaatttta ttttgattta gtaggtgttg tttttatagt tttgaataga 48000
   tttgttagaa aattattgtt taatttgttt ttgggtggta tatttttttt taggaaagga 48060
   attataaatt tattttagat gagtagagag ttgtttttta aatttttgat taaaatatag 48120
   tagattttat atgaaaaagt aaagagagag gggttaggga tgaagtgtag taggttggag 48180
   gttgagttag tttttagagt tattgggatg gtgttttagg agtagatggt attgtttata 48240
   aagttttttg tttttttttg ttttgaggtt ggggattatt ttgttggttg tatgatttag 48300
   tttttggggt ttttttagta tgaaaagatt ttttgaaagt aggtttgtta gggagatttt 48360
   ttagtttttg tttattgata tgtttttgta tggttgatat gtagtatgtt attgtagaga 48420
   agtaaagtat atgtagatta gttttgttag tagtattttt tttttttgtt ttttttagga 48480
   atgttttgta tatatttaat atatttagga atttagagtt gtgttgtagt agtagttttt 48540
   tggattttag tgttggtgtt gtggggagtt ttggttatgg tattttgagt attgagatta 48600
   tttttagttt ttagttagtt ggtttttggg agtttagagg ggtgataatt tgagtggagt 48660
   gattttagag aaagtggtgg ttaatttgaa tttttttttg gtttttttgg aagttaggga 48720
   aattttttgt gaaaaggaaa tatgttttaa gaagttttgg gtagtaggag aatttagtta 48780
   tttagtttag ttggttaaat atttataaag agatagttgt gattatataa agttttattt 48840
   ttttattttg aggttgaaaa tttggttttt gtgtggagat tgagtttttt gttatttttt 48900
   ttttggggat tttgtttggt tttgttggtt ttgggttggg gtttagggat agtgtggttt 48960
   ttggtattgt tattatggtt attagggttt gttttgtggt tgtgttattt gagagttttt 49020
   gagttagtgg tgtaaggttg ggaagaataa aggagtagga tttattttag tagtggaatg 49080
   ttgggttgtg ttggatgtgt tgagagttgt ggtttgttta ttgggttata tggtttagag 49140
   ttgagtagag gattaaaggg gttttgtggt tgtggaataa ggagtttttt agagtgtggg 49200
   gtagaggtag ggagggattt tgattgtttg ggaagggagg tttggtgggg aattgtttta 49260
   agggggtagg gtgaggggag taggaggagg gggtaggtag agataggggt gtttagtggt 49320
   aggtttggtg tagaagtaat ttgaaggtat gggagggatg ttaaggagtt gttatatgtt 49380
   aaaggaaagt gatagggttt ttgggaagag tgagggggag ttgttaggaa gaagagaggg 49440
   gaagaggttt ttagggattt ttggttatgt tgtttttgtt tttgtttttg tgtttgttag 49500
   aggtggtgtt tgtgaggagt tttttttttt tagttaggtt ttgaaattga tttggatatt 49560
   tttttttttt gtgttttgtt tgatattttt gttttttagg tgttttttta tttatttgtt 49620
   tagatattgt tatagttggg tttttaggga gtatggttag ttattttaga aagttattta 49680
   gatttgtagt tagggttgga atatagttat ttgggtttgt tttttttatt ttaggtgttt 49740
   gttggttggg attatggaag ggagaaggag taggagagga ggggtagggg atgggggtgt 49800
   agtttatttt gagttttttt tttgagagtt tttggattta gatttgattt agtttttgtt 49860
   tttttagttt ttttagtttt tgggggattt ggttagtgag ttagtttttt tttttttttt 49920
   tttttgtagg tttgggatgt gttgggattt agaggggtat aagtaagggg gtttttagag 49980
   gagggagttg atattatagg ggttgggttt ggatgttggg atttgttaga tttattttag 50040
   tgttatgatt tttgaattta agtgagttat ttttagtagg ggtgtggtta tttttatatt 50100
   aggagagagt gtttgggagt gggtttgttt tgagggtttg ttgggggttg ggagggattt 50160
   atttaaatgg aaagaagttt ttggtgatat tttttgaggg ttaggggttg aattttggaa 50220
   gttatgtttt taggtagttt ttatgggatt ttatgttgag tgttgattgg gttttatata 50280
   tttttatttt ttttattttg tatttttata tgtggttggg ggtgattgaa gttttggggt 50340
   atttgttata ttggttaaat aggttagtta tgtttagtgg ttttttagtt agtagttatt 50400
   ggtatttgat ttgttgtttt ttatttttta tggttaattt tttttttaag gttttttggt 50460
   tttatggttt tttttatttt tatagtgaat ggttgtgggg agttgttgtt attggttttt 50520
   gggtgttagg ttagagttgg gggagtgttt gggagaagat tgtatgtttg gagtttttgt 50580
   ttttatttga atgggaggga ggttggtggt agattagaga tttgggtttt gagttaaata 50640
   ttgaaattta aggttttggg tttgttattt aatatttgtg gaatttgatt ttttttattt 50700
   ataaaatggg tattattaat attagttgtt aaaattatag taagatatat atgtttaggt 50760
   taggaagtgt ggttatttag aaaatatagt tttttttttt tttaagaagt taattttttt 50820
   agtattggga gtgagaagag tttgtttttt ttattgtttg tagtttagtt tagggtattt 50880
   ttattattgg tttttaaagg gtagttttag aaattgttga taaaggatgt tagaaggaat 50940
   ttttttttgg ttaggtgtta gggttaatag aaagttttga ggatgtagtt tttattatgt 51000
   ttttgttttg tgggtttttt tttttttgtg ggtagaattt tttttttttg gttttttttt 51060
   ttgaattttt ttttttttgt ttttggtttt aatttgtttt ttagtttttt ggttttttag 51120
   gtttttttta gggtttaaga ttttgttttt ttttaagttt agtttttttt ttttttttgt 51180
   ttttggattt ttgagttggg aggttagtat tttgggtgtt ttgttgaggt ggttgttttt 51240
   tttgtagtgg ttttttttgt tgttattaaa tgagggtttt tagagtatag agaaggtatt 51300
   tttggtttgg ggttgtattt tttatataaa gtttttgttt tttagtattt tttaaagttt 51360
   tttattgttt agtatatatt tagtatttag gtagagtagt tttagagttt ttttggttgg 51420
   tttttggttt aattttttag tagaaatggt atatgggtta tagatgtaat tttatatttt 51480
   ttagtattta taataaaaaa tgtaagaata agttaggtat agtggtttat gtttgttata 51540
   ttagtatttt gggaggttga ggtgggtaga ttatttgagg ttaggagttt aaaattagtt 51600
   tggttaatat ggtaaaattt tgtttttatt aaaaatataa aaattagtta ggtatggtag 51660
   tagttatttg taattttagt tatttgggag gttgaggtag gagaattgtt taaatttggg 51720
   aggtaaaggt tgtagtgagt tgagattgtg ttattgtatt ttagtttggg taatagtgtg 51780
   agattttata tttaaaaaaa aaaagaaaag aaaagaaaaa ggaaattatt aaaaaatgta 51840
   agaataggtg atattaattt taatattatt tatttaatag agtttaaaat attattaatt 51900
   taatatgtaa ttaatgtaaa aatgatagat atttgatatt tttttttttt ttttttgtta 51960
   ggattttgaa atttagagtg tattttatat atatggtatt tttttatttg gaattattat 52020
   attttaggtg tttaaatggt tttgtgtagt tagtggttat tatgttggat agtgtagttt 52080
   ggagagtttt aatttttttt tttttttttg gttgttgggg aatagagttt tgttttgtta 52140
   tttaggttgg agtgtaatgg tgtgattttg gtttattgta atttttgttt tttgagttta 52200
   agtaattttt ttgttttagt tttttaagta gttgggatta taggtgtata ttattatgtt 52260
   tggtggttat tttttgtatt tttagtagag atggggtttt attatgttgg ttaggttggt 52320
   tttaaattat agattttaat tgatttgttt gttttagttt tttgaagtgt tgagattata 52380
   tgtgtgagtt attatgttta tttttggata gttttaattt gattttagga taatattagg 52440
   ttattagaga gttgttgatt tggattatat tatgatttga attttttaga ttatttttgg 52500
   tattatgttt ataagttata tttagaggat aataggatat agtttggtga aatatatagt 52560
   ggtatttttt ataagatatt ggtgaggtta tagattttat tattagtgtt atttgtttgg 52620
   ggatatttta tttttagaaa atgtagagtt ttttttaaga aatgaatgtg gttaggtgtg 52680
   ttggtttata tttgttattt tagtattttg ggaggttaag gtgagtatat tgtttgaggt 52740
   taggagttta agattagttt gattaatatg gtgaaattta gttattatta aaaatataaa 52800
   aatttgttag gtgtggtagt gtgtgtttgt agttttagtt atttaggagg ttgaggtagg 52860
   agaattgttt gaatttggga ggaggaggtt gtggtgagtt gagattatgt tattgtattt 52920
   tagtttgggt gatagagtga gattttgttt taaaaaataa ataaaaaaag aaatgaatgt 52980
   tttaggtata tgttaagtat tttttagatg attttaaaga tattaattat tgtgattgat 53040
   gatgattttt tttttttttt gggtgtgttt gttttgtttt tttagtttat aggtgtttag 53100
   gatttaaagg tagtttgtta taggttatgg gatttggttg gtttaaaagg gagagtgagg 53160
   ttattggaat ttttttagga gtgagtttga agtttggaat gttttttggt ggagggtttt 53220
   gaagttagga ggtgatgtag atgtgggggt tgagatatta tttggggtta tttgtaaaga 53280
   gggatattta ggtagtgatt ttaaggaaag agtaagggga ggagagggtg gtatatttat 53340
   gtttttttaa tttttgtttg tttggaggtt tatgtatttt atatgttggt ttttttgtat 53400
   ggatattttt ttaataagta atttattgtg tttatgttta tttattttaa agttatatag 53460
   aattgtattt attaaaaatg aatttatgga gatagaaagt attttggtgg ttgttaaggg 53520
   ttggagaggg tgaatgggga gtgagttttt tatggatatg gggttttttt ttggggtgat 53580
   gaaaaagttt tggtgttaga tagtgttgat ggttgtatga tattgtgaat gtatttagtg 53640
   ttattaatgg taaattttat gttttgtgta ttttagtata ataaaaaaat atatatttaa 53700
   agtaatagaa tttaatgtgt tttttttttt ttttgagtta gtttgttttt ttgttttata 53760
   ttttaaaatt agaagggttt tgagatatat tataggtttt tagggtaggt tgaataattt 53820
   ttttttttta aattgtgtta ttttatgtgg taatagggat tttgtagatg taattgaaga 53880
   tgttgagatg aagagattat ttaggtgggt tttaaattat aagtgttttt taagagggag 53940
   gtaggttagg tgtgatggtt tatggttgta attttagtat tttgggaggt tgaggtaggt 54000
   agattatttg aggttaggag tttaagatta gtttggttaa tatggtaaaa ttttgttttt 54060
   attaaaaata taaaaattag ttaggtgtag tagtatatgt ttgtaatttt agttatttgg 54120
   gaagttgagg taggagaatt gtttgaattt aggaggtgga ggttgtagtg agttaagatt 54180
   gtgttattgt attttagttt gggtgatttt gttttaaaaa aaaaaaagag gaaggtagag 54240
   ggagatttga ttgtagagaa ggaggaggtt gtaggaggat ggaggtagag gttggaggga 54300
   tgtagttgta agttaaggaa ttttagtaga gttttttatt taaagttgga agaggtagga 54360
   atggattttg ttaatatttt gattttagtt tggtgagatt gattggtttt tgatttttag 54420
   tattgtaaag gaaggagttt ttgttgtttt aagttattga gtttgtgata agtagttata 54480
   gtggtaatag gaagtgaatg tagtttttag tttatttttt tgtttatagt aatataggtg 54540
   tttttttgtg tagaattttg ttaggtagtt agtgagtggg ataggatttt tttttgtttt 54600
   tatttggggt tttgtgttgg attttttaag gttgttgttt ttgagatagt gaatttttta 54660
   tatttataga gatttatttt aaagttgaga aagtgttttt tttaaggggt gttagaaaga 54720
   aggttttaag gtgtttttgt tatatgttta tttgtttgtg ggtaatattt atagtttttt 54780
   tttgtgattt atttaataga agggttatag gggagggagt attattggtt agtttatttt 54840
   atagtaaggt tttattttgg aagttatagg ggatagttgg gatttttatt tttaatttag 54900
   ttaattggga gaagagttta gaatagtttt ggaggttgat atttgttagg aagtgtggtg 54960
   atatttatgt gttgttgaaa tatatagtaa tatagtagta gagaggaggg gatgtggttt 55020
   tatagttttt tttttattta tttagtatgt atgtatatat atatatatgt gtgtgtatat 55080
   atgtattaat ttttagtttt taggttttgt attttgtttt tgttaagttt tttggttatt 55140
   attaagttag gttgtgagta tagagtttag tttttttgtt aatttttttt taatttattt 55200
   tgggtaatat tttaatggga tgtagtagat gttttttatt aaatgttaag agtaatattt 55260
   ttttttattt tttgtgagtt ggtggttgtg tttttgtatg ggggtgtgga ggaaagggtt 55320
   ggggagtagg aatgtggttt tgagggttgg tatgtatttg tagttgtgat gttttgaggt 55380
   agtttatatt tttatttata gattaatttt gagaaggggt tgggagtatt tatagttttt 55440
   gttatttttg ggttggtagg ttggggaaat tttgagtagt ttgagtgaag gttgttttta 55500
   ggtaggtatg gtgggataga ggggtttttg tatatttttt gtgaggtagt gtagttgttg 55560
   ggttgtttta gtatatttgg gtgatatgtt ttttttttgg ttttttttta tttggtttgg 55620
   gagaatgttt aggatgtttt aattttaaaa ataagtaaat tttttttttt tgatttattt 55680
   atttttttaa attgtgaatt ttttttttgt ttgaatagtt taatttttta aagtaggttt 55740
   atattatggt ttttagtttt ttattgttta tttatttttt agtttgagtt atttggtgtt 55800
   tgtttgtttt attattaatt tagtttttga aaaaattttt atggttagta tttagtttta 55860
   ttttatttga ttttgagggt agtgtttgat atgtattagt tggttttttt tttttttttt 55920
   ttttttttgt attttttagt gttggggttt agatagtttt taagggtttt tttgtttttt 55980
   gatttagttt tttggagtga ttatattttt gttttagttt tttagtttag ttgttttttt 56040
   atattttgtt ttaggttttt tttttggaat attgtttgtt tttagagatt taagttttta 56100
   ttttttatat tttttttttt gatttttagt ttttttatta aagttttatt ggttgttttt 56160
   ttttggtgtt ttaaattagt ttttttttgt ttagatttta taaggttagt ttttatttta 56220
   tgattgtttt tttggattat tgggattatt ttttattaat atttattttt tattttgttt 56280
   ttttttgttt atgtagggtt ttgttggagg attttttttg ttttattatt ttttttttta 56340
   aatagttttt taggtttttt tgttaaagtt gaaatttttt ttttgtattt gaggattttt 56400
   gtaatttggt taatttattt ttaagtttga atttttattt ttgtttaagg agtttatgtt 56460
   ttttagttag gttttattgt tttgtaaatt ttatttaaaa ttttttttgg taaatgttat 56520
   tatttttagg aagttttttt gatatttttt attatatgtg atttttattt ttagagttag 56580
   attttgattt ggagattttg atttttattt atttttgttt tttttgagtt ttttaggttg 56640
   taggttttag tttatgtttg tgggtttttg tagtaggagg gtttattttt atttttttaa 56700
   gaagtgtttg ttttattttt tgttaagttg taatttttta taagtaggta tttttaatat 56760
   gtttttttat tttttttttt tttttttttt ttttttgaga tggagtttgg ttttgttgtt 56820
   taggttggag tgtagtggtg tgattttagt ttattgtaat ttttattttt taggttttaa 56880
   gtgatttttt tgttatagtt ttttttgtag ttgggattat aagtatgtgt tgttatgtta 56940
   agttaatttt tttttgtatt tttagtagag atagggtttt attatgttgg ttaggttggt 57000
   tttaaatttt tggtttgaag tgatttgttg attttagttt tttaaagtgt tgggattata 57060
   ggtgtgagtt attgtattta gtttaatatg tttttttata ttttttttga aaagttatta 57120
   ttatattaat agtgtgtttt ataaattgat aataatttaa aatgtgattt attattaaaa 57180
   gtgtgatttt atttttaaat aatattgttg gttgtggttt gtagttagtt ttattatttt 57240
   tgtgtgatga ttattttgta gattagtaat ggttttaggt ttatatttaa tttaattatt 57300
   ttgttttttt aattttattt ttttttgagt taatattaag tgtgttttag agttgtaaat 57360
   ttatggttga tataaaatta ggatttttaa tatttaatat aaagtaattg ggttttgtat 57420
   tggttttaat agtaattata aattatgttt gttttttgta ttgggtatat tgtatttagt 57480
   ttataatatg tgtttttgtt ttttttattt tttttattaa gatggtatat aggtttaaat 57540
   ttgttttttg ggagtaattg attttttttt ggaagatgtg agtagttttt atattatatt 57600
   taagaagata ttgttataag attttatatt ttttatttta gtttttaagg gtttatttat 57660
   ttttttaaaa atttatttgt tttgttataa tatgtgtttt ttttttttta tttgtttttt 57720
   taagatatag ttttattttg ttgtttagat tggaatgtaa tggtatgatt ttagtttatt 57780
   gtaatttttg ttttgtgggt ttaagtaatt tttttgtttt agttttttaa gtagttggga 57840
   ttataggtgt gtattattat atttggttaa tttttgtatt tttagtagag atggggtttt 57900
   attatgttgg ttaggttggt tttgaatttt tgattttagg tgatttattt gttttggttt 57960
   tttgaagtgt tgggattata ggtgtgagtt attgtatttg gtttataata tgtgttttta 58020
   tttttttttt tttttttatt aagatagtat atgggtttta agtttttttt tttttttttt 58080
   tttttttaag attagttggt tgggtgtagt ggtttatgtt tgtaatttta ttattttggg 58140
   aggttgaggt aggtagatta tgaggttagg agtttgagat tagtttggtt aatatagtga 58200
   aattttgttt ttattaaaaa tataaaaaat tagttaggtg tggtggtggg tgtttgtaat 58260
   tttagttatt tgggaggttg aggtagggga attgtttgaa tttgggaggt ggaggttaga 58320
   gtgagttgag attgtgttat tgtattttag tttaggtaat agtgtaagat tttgatttaa 58380
   aaaaaataaa ataagtggga agaggggaga tagtggaata aggagtttaa tttgtaattg 58440
   attgtgaata attaattgag ataatttatt atttttggat tagttatggg ttttattttt 58500
   tatttgtttt tttgagttat gttttttagt gaaattttat atgtatgtga ttaaatttgt 58560
   tttttttttt attaatttat tttgagtgag tttaatttat agatttttaa atattgaatt 58620
   taagagggta gaggaaatgt attttttttt tgttataaaa atattttatt tttttattgt 58680
   agtgttgttt gttttattta tagttggtaa tttggatttt gttttttaaa gtttatgaaa 58740
   gtaggataga ttgtttgtag gatgtttttg ttataaaagg agggttaggt ttggtaggag 58800
   gaaagaaatg gaagtggtaa atgtttattt tttgtgttag atagaggtat taggtgttta 58860
   ttgttttggg gagtaaatat ttttaaaaat ggaaagaaaa atttttagtt ttattggggt 58920
   gtaaaaagtt atgatttttt ttgtggttaa atggttgggt ggtttgggag ataagtggta 58980
   gttattttga agtttagttt atttgtttgt aaatggaggg taatgaaatt atttgttgta 59040
   tgggtgtttg ataggttgtt gtttaaattt tttgtttttt tttatggggt tttggggaag 59100
   attatatgag ttagtaatat aaaagtattt tgaaaattat atgaagtgtt taagtatggg 59160
   ggtgttggtt gattttaggt ttgtttttat aagttggtag aaaatttttt agagagggtt 59220
   ttgttttttt ttttggggta ggggttgtag tgttagattt gatggtattg gtggttgttt 59280
   aggttatttg gtatgagatt gttgttagtt ttagatttat tttaagtttg ggagggaagt 59340
   aggttggggt gggggttttg aaatttttta tatttttggt agagatatta gggtaggtaa 59400
   aagtgttatt tatggtaaat taagaagttt gtttgaattg ggtttaaatt agtttagttt 59460
   agaatggagg ttagttttat tttttttttt tagaattagg agtttagtat attagaggtt 59520
   gagaaaatgt tggaatagtt tttttatttg tttgtttaga gatagggatt ttaagttatt 59580
   ttttttttta gtttatttta taaatttttt gtgttagatt ttttttgttt aggagttata 59640
   ggttttattt gggagttatt tagggtttta aggtttttat tatttagttg tttattttat 59700
   taggttaggt ttgtaggtat ggagattagt aggatgtggt ttaaatttgg ttgtatgttg 59760
   attagttgag tgattttggg tttatttttt aatttttggg aggtaatgtt tttttatttg 59820
   taaaaagggg aaagggttta gtgaggagag tgataagtaa aggagatggt atttgtgtag 59880
   atggtattgt gtgggtgttt agtggatgat tatgtgttat ttttttgttt tttttttttg 59940
   tttttgtggg agtgtgggta gtagggagtt aagaggaagg gagtagttgt ttgtttggga 60000
   tgtaggttgt tttgaattgg aagagaaaaa ggaataagta gtagttgttt attgataagt 60060
   tttatgtatt tgtgttttaa aattttaaag gtgtatttga aagatttttt gggtaaattt 60120
   ttttttatag atatatgaaa gttaggtgta gtggtttatg tttgtaattt taatattttg 60180
   ggaggtgaag gtaggtggat tatttgaagt tgggagtttg agattagttt ggttaatttg 60240
   gtgaaatttt atttttatta aaaatataaa aatttgttgg gtgtggtggt gggtgtttat 60300
   aattttagtt atttaggagg ttgaggtagg agatttgttt gaatttgaga ggtggaggtt 60360
   gtagtgagtt aagattgtgt tattgtattt tagtttaggt gatggagtga gattttattt 60420
   taaataaaaa taaatttaaa aaaaaggtat atgaaaaagg taaaatgtta tgagtgtttt 60480
   gttgtaatgt gttagttata aatgggaatt ggaggatttt ttaagttatt ttttttttta 60540
   tttttttttt ttttgtatat gtattaatag ttttttgggt gttggtagta ttaggggatt 60600
   tggggtgttt tataggtttt gtttgtttag attttagagt taaggaggag atataaaagt 60660
   taaaataggg tggtaagtgg aataaatggg gattagatgg ggtaatttgg tagtatttgg 60720
   taaggttttt tttaatgagt ttttaaagag gtttagtttt taggggaagg aggtgtttta 60780
   ggtagtgggg gtttgtagag gtgaaggttt agggtggggt ttttagggaa gttgtttagg 60840
   atgtttgaaa ggaggggagt aatttatgag tttggtgata gggttggtta taaagtgtta 60900
   tgaagtatta attaggttgt ttattagttt ttaggataga aagattatag tagttaaggt 60960
   gtggggattt gtttatttga tttttattta gtttagaaaa tgattgtttt agggttatag 61020
   gtaatagttt aagttttgta agttgttggt tatagggtgt agttgtggta aggtggggat 61080
   gtttattagt taagtttttt aggatttttg ggttgttgga tatgtttgta ggatgttatg 61140
   ggggtaggtt agattagggg tatttttatt ttttttttta ggttttagtt tttaggttag 61200
   agtgtttttt ggtttttggg gtttttaggg ttgtagatag atagggagag gtttgtgtta 61260
   tggtgggtta tttggttttt gtaggaagtg gttagaggtt ttagtatttg gatatagtag 61320
   ttattttttt aatttttgtt aatgttgttg ttgtttgggg tttatttttt ttaatttttt 61380
   tttttaatta gggaggaaag tattttggag taggaattgg gttatttagt ttgatatttt 61440
   agtatgattt tgtatgattt tgggttagtt atttaatttt tttgggtttt tattttttta 61500
   tttgtaaatt aggtagataa ttttttaaag ttttttttag ttatagaatt ttaagtgtta 61560
   aaataaaaaa aatttataaa aaggaaatat gtgtttagtt tatatttttg tttttatttg 61620
   ttaaaagggg aaatggattt gttatggggt tttttttttt taaatagata tgggagattt 61680
   tttttatgat tgtagaattg gagtagttta gaggagttta gaatttttta gagtttgaga 61740
   tttatagggg aatttatttt aatttttttg ggatagaagt attgttgttt aatggtattt 61800
   aatttttatt attgttgtag taaatgatta tatatttgtg atgttaaata atatagatta 61860
   gttttatatt ttattttatt aggttatagg gaaggtgttt atagggttgt gtattttttt 61920
   gaggttgtag gggagattgt gtttttttgt attttttgtt tatttgtatt ttttggtttg 61980
   tggttttttt ttttttttta agtttttttt attgtatttt ttagatggtt tattgtgttt 62040
   ttaaaaattt ttgtgatgat agtgggtttg tgtggattat ttaggattat ttttttattt 62100
   taagattagt tgattagtga ttttagttgg ttttgttatg ttatttaatg tagttttagg 62160
   tgttggggat taggatgtgt ttaattttgg gagttattat tttgttaatt ttaagatata 62220
   ggttttggag ggagatgaag ggtaggatta taggtttagg aggttgtttg tgtgggtttg 62280
   aattttggtt ttattattaa ggaattgggg gagtatatta tttgtaaaat gggggttggt 62340
   ggggaattag ttttttggtt gttagtgtag agtgaggtag tgtgtgtagt gtttggagta 62400
   gttttgtgag tatttgtttt ttttttgtta aatttataag aattgatttt ggtttaatgg 62460
   tttttttaaa tttttagttt tttttggtta taaggttagg agtatttttt tgtagggtta 62520
   tttaagtttt tagttttttt gaaagttttg gtttatatga tgttggaagt agatgtgttg 62580
   tgggttgttt atttatgtat ggttattgga gaggtttgga tgatgttgtt tttaatgggt 62640
   gtatagtgta gggtagggtt aggtattaat aattagttat agttattagt gtaagtaagg 62700
   ggtttttatt gttatttttg gtttggggtt agggagaagt tttgggaggt atttgatttt 62760
   tttataggga gtggggttat aggagaatgt atttgaaagg ggttgggggg tagtagaaag 62820
   ttttttttag aaattttatt tttggtttgt gatagatgat ttttttaggt tattattggg 62880
   gtttatttgg gtttttgtag tatggagttt ggttatttta gaaggatgat tgggtaggtt 62940
   agggttattg gtgatggtga tggtaggttt ttttgaggtt tttttattag aagtattagg 63000
   aggttagagt agttgataag tttttatttg gtttttgagg ggtagaaggt tttgagtagt 63060
   tttttgattt tttgaggttt ttttatttta ttgagagatt tttgagagta ttgagagtta 63120
   ttttttggtt tttttgtgag agagttggtt atataaaggt agttatatat ttagtaagat 63180
   gaatttataa tttttgagtt taggtttatt ttggtttttt tatagggagt ttttgggggt 63240
   gtggggtggg ggtgtagaag ggaggggttg gtgttttatt tttgggaatt aggttatgag 63300
   ttttggattt aggttaaatt atagaggttt tatttttttt taggtttttt gttttttaat 63360
   tatttttgta tagggtattt tttttttttt tgtagggttt tattggggtg gggtgaggag 63420
   gagttataga attatagtag ttgttatatg ggggtgttaa ggtagggatg ttgatgttaa 63480
   ttgaatgggt atgggaaggt agagtaggag gttggtgttt tgggtttggt taggttgtgt 63540
   gattttaggg aatttatttt attttttttg gtttttagtt ttttttattg taaattgagg 63600
   gagtgaggtt aggtgatttg tataattgtg tgaatttgtg ttttgtattt tttatttatt 63660
   attttaaata atgtttttaa attttagttt tttaatgttt tggtattttt ttgtttgaga 63720
   aatatttatg gttgtttttt atgttgtgtt ttaaatatag gtattttggt ttttttttgg 63780
   gtgtttttat tagggttaga atgggtagtt tattttgtag gaatttttaa ggatttttag 63840
   ttatttttag gatgggggtt ggttgtttgg aagtggtttt tgtttatttt tgttgtagtt 63900
   tttattatga ggttagtgta gtaaaatagg aggtgtaggt ggttggggtt aggggttagg 63960
   ttattttttt tagtggggta ggagaggggt gagaaaagtt ggatattgat tattttaggt 64020
   tgagggtttg tgtggaggag ggggtttatg gtaaaggaag tgggaggatg ggaaggagtt 64080
   tttgttggat atttttattt tgttggtttt agttttgttt ttgttttatt tgtggggaga 64140
   aagtgaggtt ttttgttttg ttaaatttta gtaatgaatt ttatatttat tttatttttt 64200
   gtttattgga aatttttttt ttattttttg gatttgtttt gaatagttgg gttgtagaag 64260
   ttggtggaat gaaatatgta gggtggttta gaggggattg tgttgaatag tgttgtgttt 64320
   ttgtttattt ttttttttga agataaggtt tttttgtttt tgtttttttt ttgggaaagt 64380
   tggagagggg gttgaggaaa tgggatttat tattaatttt tttgtgtatg ttggttttta 64440
   gtaggtattt ttatattttt taattaggtt tgtagggtgg aaaggtagtt gttgagttta 64500
   atttaatttt tatttagttg tggaagttag tgtggatggt aaagtattta tgtataaata 64560
   tatatatgag gttgaggtta agagaatatg tgtttagttt ttttttggtt ttttagtaat 64620
   gtttttatgt tttagttttg ttttaagttt taaatggtgt aaggtttttt taggtggagg 64680
   aatggaagtt tttaagaagt ttgtgtttta gggattatat agaggtaggg tttaggagta 64740
   aaggttttta gattttgtag tgtggtgttg gtaagtttgt gttgttgtat ttatattgtt 64800
   ttgggatgaa ggtgatatgt tgattttttt taggtggttg taggatagtt aggaggaggt 64860
   gagatgttag ttaaaggtag ttatagataa tagtattatg gattattaga aatgttattt 64920
   ttgtgttaaa gtgtttgttt tttttagttg ttttattttg tgttaatatt ttttgggagt 64980
   gatatgtgtg agattgggtt gtgttgatga gtgattattg gtatttttag attatattaa 65040
   ggaggggtta ttttgttgtt aattaggtgt agggtaggtg gtattggggt atgatgattt 65100
   gtttggtatg gttgttttat tttttttttt gtttgagtat aggttggtag gtttagagga 65160
   gaggaggaaa tagagttatt atggggggtg tgtttagggg tgagttattt taagaagtta 65220
   tagaattagg gtatgggaga tattgtttag ttttgttatt aatttgttgt gtgaagttgg 65280
   ttaagttatt gtattttttt gggttttagg ttttgtaatt agagggttgg ttgaagaggt 65340
   aaatgatagt ggttagggat gggggtattg aagttagatt ttttgtattt ggattttttt 65400
   tttgttatgt ttagagggag gtttgggtag gttatttaat atttttgggt tgtgggtttt 65460
   ttattggtga aatgggaata ataatagaat taattttata gtgttgttgg gaggattaaa 65520
   ggggatattt attttaggta aagagttttt aagatagttt ggggttttta ggaagttttt 65580
   gttattatta ttggatatta ttatttttgt tatggttgtt agggttttgt ttagtattgg 65640
   tagtttgatg ttggtagagg aagggtgagg tttttgtttt tttgtagtgt tttttttttt 65700
   tttttttttt tagttatggt aataatagtt gaagtttatt tttagtttta gtgattggtt 65760
   atttttttga attaaggatt gaaggtttta ggtttgttat ttaaggttta ggtgtaagag 65820
   gggtgagtta ttggtttttg tttttattat atttaggttt ttatatgttt ttttaaatag 65880
   gtttttgttt tatgtgtaag agaagatgtt aatttttatg tttggggtgg gggtgtttat 65940
   aaatgttttg gatgtgagtt ttgatggggg ttatggtgta ggttttgtta gggtattttt 66000
   aattataggg gattatttgt agtttataga gggttttttg taggttaatg ggattttgtt 66060
   attttggttg ttattttgtt tagttatttg gtaaggaaaa taggttgtag atttagaggt 66120
   agtatgggtt tatatgttag tgagataata taataaggga aggatgtgta ggatgaggga 66180
   gttatggaga ttttattgta gttttagtta tttattagta gattttgtgt ggatgttggg 66240
   taggttattt tattaatttt taggatgttt ttgtaggttt tgtgggtgga tagggtgaat 66300
   ggaggtataa ggagggtaag tttgataggg gttggggttg ggtttggagt tagaatttta 66360
   gttagttttg ttattgtatt ttagggtatt ttttgtagat agtaggttaa agtaaggttg 66420
   tttagattaa aggggaaggg gttttgtttt ttttattaag ggttttttgg agattttaat 66480
   atagattttt agtttataga tattaaattt agggtttttg agaagtgaga aatgtgaaaa 66540
   atgtatagaa tagttgtagt ttagtgtttt taatatgtta gatttatata ggtatatata 66600
   gtgtttttgt agaggttaga aagttttgtg ttgggaagtt ttttgattgg gggaggtttt 66660
   atagtaagtt tttttttttg tttatttatt aatgggtgga aggttttaat taggattata 66720
   tgggaagggt tagtgggagg ggtagagagg gaaaatgaat aggtaatatt aagtgtttgg 66780
   ggtggggggt gtaggggata tttaggaggg gagggtaggg gtgtattttt tatttaattt 66840
   atagggaatg ggggtttggg aggtgtagga gaaggttttg gtatttttat tttttttttt 66900
   ttgtagattt atttatagtt tgtttatggg tagttagatt tagttatagg ttagatgttt 66960
   gttttagttt tatttttttg gtttattgaa agtaagttag gatagtgagg gggatgatgt 67020
   tgttattttt ggtatggtta tgttgggttt tggggtgggt gggaatgtgg ttggggtgtt 67080
   attttttttg gttgttatag gtataaaagt ttttttgtgt tttagtgtta tatgtgggtt 67140
   ttttttttgt tgttttaata ggatggatat ttaagtagat tttgttggtt ggggggggtt 67200
   tttgtattta gttttatttt gatttagtga attttttttt tgttttttgg ggtaggagtt 67260
   attaggttgg gttttaggta atggttagtg aaggggttgt atggaggggt tttttaggtt 67320
   tataggaagg gaggtttttt ttttttgtaa tgtggttgta gtattttgtg attgagaggg 67380
   atattatggt tggagatggt gggatggagg attttttata gtatttttga tgtttagaat 67440
   attttatttt gtataaaaaa gttttaaatt tttttgaggg ttgaagttat ttagagttgg 67500
   ggtttttgtt ttttaggtta aaagtatttt gttggagtag ttgagagtat agtgggaggg 67560
   gaggggtagg agggaatagt tttgttatgg tgatagggga ttttttgatt ttaagagggt 67620
   ttgtagatat tattatttat ttttagtttt gaattgtttt ttgttaaggg gttagaaatt 67680
   tttgggaggg ggattatagg gggttttgtt tatgagaata gtgattgtgg ttaggttata 67740
   gtttataagt tagtaatttt gtttagttgt tgtggtggtt tttttttgat ggtttttaga 67800
   aaaagtgttt tattttttgt tattagtgtt ttgaaattta agaatatttt ttaaatagta 67860
   attgtagtaa aagttaatgt tgtgtgagtg ttgtaggtat tatttttagg gttttatata 67920
   ttttttgttt agtttttata aaaattttat tttatttttt tttttgtttt ttttttttaa 67980
   tagtttagaa aatgagtatg gaagggttaa gttggtttga ggaggggttg gtggaatagt 68040
   ttgagggttt agtttttaag aattttatat tgtaaaggga atttttgggt tagggagtag 68100
   ggtatagttt ttattttttt atttttgttt ttagtattag gtttgatata gatggggttt 68160
   tggttatgaa taaatgaata agtgaatgag agtttgttga gggaagaagg tgatagggat 68220
   aagggagaaa taaagtagtt aatgttgttg tgatgatgat gatgttaatg gtagtattga 68280
   tatgtgggtt tttgttatgt taggattgtt ttaagtattt tgttttatga atatttttgt 68340
   tttttattat gttttgagtg gatattttta ttagtttatt ttatagaggg gaatatagtt 68400
   gagtgtattg gttaaggtta tgttgttagg aaaggttaga gtttaggtag tatgtggttt 68460
   tttagttggg ttttggttta tatggtttta taggtagttt tatgaaatat gtagagagag 68520
   tatgtgaggg gtttttttgt tgttttttat ttttagttgg ggtttatttt ttgttgaaat 68580
   ttttttaatt tgtttttaag ttttgttagt tatgaaatat ttttttgaat atttagtatt 68640
   atttttttat ggttatttgg ttttattttt ttatagggta agttttgggt gaaaagtaga 68700
   aaggatgaaa atttttgaga gaggtgataa tgtggtaatt tttttttttt tttttttttt 68760
   tttgagatag tattttattt ttgttgttta ggttggaatg tagtggtatg attttggttt 68820
   attgtaattt ttattttttg ggtttaagtt atttttttgt tttagttttt tgaggagttg 68880
   ggagtatagg tatgtgttat tatattaggt taatttttgt gtttttttgt agagatggag 68940
   ttttgttatg ttgtttaggt tggttttaaa tttgtgggtt taagtaattt gtttgtttta 69000
   gttttttggg attatagatg tgaattattg tgtttggttg aggtggtttt tatataaagt 69060
   agagaaaatt atttttagtt taaaataaat taataagtat tgagtgttta tgtagaaaaa 69120
   gattagaagg aaatgtgaat atgttaatag tgtttgtttg gaggatgtaa ttattattga 69180
   ttattttttt tatatttttt aatttaatta ttttgttata agtaggtgtg atttttaaaa 69240
   ttataaaaga taaatgttgg ttttttgttt gtgttttgtt ttttgagata gggttttatt 69300
   ttgttattta ggagttggag tatagtggtg tggttatagt ttattgtagt tttaaatttt 69360
   taatttaagt aattttttta tttgagtttt ttgaggagtt gggattatag gtaagtgtta 69420
   ttatttttag tatttttgtt gtttagattg gttttgaatg tttggtttta agtgattttt 69480
   ttattgtagt tttttaaagt tgggattata ggtgtgagtt attatgttta gtaaagattg 69540
   ttttttaatt aggaagatag atgtttttta gtattttttg tttttttttt tttttttttt 69600
   tttgatatgg agtattgttt tgttgtttag gttggagtgt agtagtatag ttttggttta 69660
   ttgtaatttt tgttttttgg gtttaagaga tttttttgtt ttagttttta gagtagttgg 69720
   gattataagt gtgtattatt atatttggtt aatttttgta tttttagtag agatggggtt 69780
   ttgttatgtt gattagtttg gttttgaatt tttgatttta agggatttat ttgttttggt 69840
   tttttaaagt gttgggatta tatgtgtgag ttattatttt tggtttgttt tttagtattt 69900
   ttaatttttg ttttttggta ttatttggtt aagtagatga aaagttttag tgagttgtta 69960
   gttggtatta ggttggggtt ttttttaggt agttttaagt ggttaggatt tggttttttt 70020
   tttagagttg ggtttagaaa ttaagattgg gaatgtgtga tggttgtttt gtgggttttt 70080
   gttatgaggt tatttttttg gttttagtga ttggttgttg gttgtaggga gtatgtgggt 70140
   ttagttaggg tttgtgttaa tatggttttg ttttatttat ggtttagtgg gtttttttat 70200
   tttttgttgt atgataattt taggaaaaat gttaggaggg agggagttgt tgggggtgtg 70260
   ataggagaga ttttttaggg atagatttgt aaaaattgtt ttaaagtttt ttttaaggat 70320
   ttttaaggtt tttattgttt tttattgata ggttatggtt tgggtagaga aagttgggga 70380
   tggagttggt ggggggaggg tggttagtat ttgtaaggta aggagatgag ttgggagaat 70440
   aggggttggg agggtagttt taggttagtt tttgttttta gaattattgt tttttttttt 70500
   tggttataaa gtgaggttgg ttagatatag gtttatttta taggttggga ggttgggtgt 70560
   gaaagtattg tgtttgtatg gggtatttta tggtgaatat tattagagtt aatttttagg 70620
   gtttgttgtt ttatagggaa gttagtgtaa ggtgtttatg gaatttagaa gtatttaaag 70680
   gatttgatag ttatgagttg ggaggattta gttttgtagt aggagaaaga gaggtagaga 70740
   gataggtttt gtaatatttg ttttatttgt tgtgttgttt tgagtatagt ttattttttt 70800
   gtggttttga gttttagttt tgaggttttt aagatttttt gggtttttgt agtttaggat 70860
   gtttatttga aaattaggtt tagttagtag ggtttttttt gttagtttat tattggttag 70920
   tttttgttta gtatttttta gtttattatt tttatttgtg taaattttat attagtattt 70980
   taagtttttt tttttttagg aaggagattg tttttagatg tgttttttgt ttttttttga 71040
   ttaaagttta gaaaatagga attagttttg tgtttttttt ttttattgaa ttgttggttt 71100
   ttttggatgt tttttttgat tttttgttaa tatttttata atataattag tttttagttt 71160
   ttttttaaat gtaattggtg agtatgttgt agtttaggtg gatggtgatg tttttatttt 71220
   gtaatttgtg agagttagta tattttttag ttaggtgggt tgtaggagtt agttgtggtg 71280
   agggtaggag gtttgtgtgt tgatggtatt ggtattatta aaattatggt tagattaagg 71340
   ttatggttaa tgagtttttt tatttgtaat ttttttttta ttaaggtggt tttagtttta 71400
   gttttggttt tagttttggt tttggtttta ggagttgtag atatattttt aagtttttat 71460
   tttaggagtt tggttttata tatattggag gatatggagt tggggtataa atagttaaga 71520
   ataggtatat ttgtaattag gagtaggggg tggagggttg ttgagatgtt tttattttta 71580
   ttggttgttt tttttgatgg tttattattt attatatatt gattgtataa ttagggttgt 71640
   gatatgtttg ttttttatat tatattatta aatttttaaa tttgttttta atttatatat 71700
   gatgaggtaa atttagagtt aataatttgt ttattagttt ttgaatttat gggtggtttt 71760
   aatttttttt tttttagttt ttttgggttt atatgagtat tgaggtataa gggaatttta 71820
   ttagttggga agtgtgtttt ttttttgttg attttatagt ttttttagtt tgtgtttgaa 71880
   gttgttaata tttttttatt tagtattgtt ttttgtagtt tgagttgttg ttgtgtgatt 71940
   tagggttagt gttttaattt ttttggatta tatttgtaag atgaggatgt tgttgtatag 72000
   tttattttaa atggttatta agttgtttgt atagttaata tttgttgagt ttgtgatatg 72060
   attagttttg ttttaatttt gaatgtggta tttttattta gtttgggggt ggaattgtta 72120
   gttttttttt ttttggataa ggaagttgaa aggttttaga aggtattaag gttagttgat 72180
   ttgttagtag gtatagtgtt tatttgttat ttatagtttt tttttgattg ttttgtttgt 72240
   ttttttagtt gagattttgt gtgtatttga atattttgtt aatggttgaa tttgtattag 72300
   ggttattgtg ttagttgggt ttgttaggtt attgtttttg agttttgttt aaggtagggt 72360
   tagtgtttat attttgtttt tttgttagtg ttttatttaa ggttttggaa aggttttgga 72420
   gttagagatt tggttaaatt ttggtaatgt tttatatttg ttgtgtgatt atgggttagt 72480
   gatttagttt ttttgtgttt tttgggttgt tgtgagggtt ataggagatg tatagagggt 72540
   tttgagtttg gtggttggta tgttgtaaag gttagttatg tggtgtaagg atggtattga 72600
   tgataataaa tgaattgaat gaaaattagg ttaattgggt ttggggaggg gtgttggtta 72660
   aatagatttg agtggattag gattttgtat atgatgagta agtgggaata gttttaaata 72720
   gtaagttggg taggtttagg atgatttagt tttgggtgtt gtttagggtt tttttttggt 72780
   ttttttgttg gtttggtttt tttttgtttt agtttgattg tttttatggg ttttaataag 72840
   tttttgatta ggattgaggt agaatagata gggtttaaga ttttgtaggt aggtatagag 72900
   tgtggatggt aaaaggagat gttttaggtt ttttgttgtt tttttttttt tgaaggttga 72960
   taatgttaat ttatagaaga agttgttttt tttggttttg gattttattt taatatagtg 73020
   tatgaggtag ttgtgtttaa ggggtgtgat tatttttgta gatggaattt atttattgtg 73080
   tttagtttat tgttagagtt tggaggatgg gggaagtgag gtggggtatt ttttatagtg 73140
   aagggaaaat ttagtttagt tatttttttt tgtgttgttt ttggaattta ttttgttatg 73200
   gttttatttt aatttagttt tttaaagttt tagagagttt tgtttttttt tttgtatttt 73260
   attttgttta tttgtgttgg gttagttttt tagaagtttt gttttttgtt atttttaggg 73320
   tattaaggtt ggttggtggg tttagggatt tttatttttt gatttagttt tagtggttta 73380
   ttttatattt attggtattt ataggtgtgt tttttatttt tggtttttgt ataggtggtt 73440
   ttatattttg ggagtttagt tttttttaag gatttatgtt agagtatagg gatttttgtt 73500
   tttgattttt ttttagtttt aattatgagt agtttatgtt atagtaattt tttagttgtt 73560
   tgtagttgtt tgttaagtta tgataatggt ttttgttttt taatttaatg tttgtatata 73620
   gataaattgt tatattttaa gtagattaga tattattttt tttaggaagt tttttttgat 73680
   tgttttagtt ggatagaggt tttttttttt tgtgtttttt taatgtttaa taggtattta 73740
   ttgagtattt gtgatatgta aggtatttat aatataagtt agtgtttttt gtttttttag 73800
   agggtatatg aataaataaa tggtaagatt ttttaatatt tatttatgtt ttatagaaag 73860
   ttatttatag gtatgggagg gttgttttaa ttgtgggtga tagggaggtt tttttaagat 73920
   ggtagtgttt tagtaagatt tgaatgaggt ggttaggagg tttagggagg gattttttag 73980
   gagggaataa gtgaatattt ttttgtgagg atgagttttt atgttttagg agtagttagg 74040
   ttaggggatt ggtgtagtga ataggggttg aggggagtgg ttaggtgatg taggttttta 74100
   taggttgttt tatgatgagt tagtgtttta agtatagtag atatttaggg aaggtttagg 74160
   ttggggagtg atttgaatag attttttatt taggagttta ttggggtttt tgggtttatg 74220
   ttgttgtgtt attgtatgtt tttgggggtt tgggggggta ttttttatat tattattata 74280
   ggtagtattt ttgggggagg tgtgttgtgt aggggatttg ttggttattt tgttgggtaa 74340
   attatagggg attgtgtagg ataggtggtt agtttaggag ggataattat ttaagtaggg 74400
   gttagagggt atttggattt gggaggagtg tttaattttt taggtttagg taattagagg 74460
   tggggggtgg ggtgttgttt tttgatatgg gaggtttggg ggtttggttg tattttttgg 74520
   tgtggaggtg ttgggaggtg tggaggtttt tttttgaggt tgggattttt tggttatttt 74580
   gatttattgt tttgatttta ttttttagtt tgagggtatg gtttgaattt atagttgtat 74640
   atattttttt ttgtataggg ttttgtgggg ttggtatagt agtttttttt ttatagatta 74700
   ggatgtttag ttttaggagg gttgggattg tttaagattg tataattagg gttagatata 74760
   gttgggttaa ttttgtgatt tttggtttta tgtatagatt ttaagtatat aggtaaaatt 74820
   ttgttggttg ggagggttgg ggggtagatg agtggggggt agtttttttt tagtttttta 74880
   atatgttagg tttttgggtg ggagggggta taaagatttt atttttttta agagatagag 74940
   aaagaggtag tggagggttt ttttttggtt gaggttttag gttttgagtg gtgaatgtaa 75000
   ttttatttgg aggttggaag ttaggtggtt ttgggggtgt gtgaggtttg ttagtttagt 75060
   tgtagttttt tttatgtaag ggaatttttt ttaggtagtg tttttatgat tagagagggg 75120
   gttaggaaga ggtgagggtt gtatttagag tgatatttgg gtgggtttta ttttggggat 75180
   atggttatat tttttttttg gatttttgaa gtttttaggt gtgggttaag ggtttttagt 75240
   tgtaggtagg agagggtttt atagttatag atttggggag tttagtttgt aaagtttaga 75300
   ggtttttgga ggttgttggt ttggtttttt gtgtagttgg gtttttttgg gaggtttttt 75360
   tttggtattt ttagttttta gtttgtatga ttttttatag ttgggtttat ttttttaggt 75420
   ttgggttata tggtgtaggt tagtggggtt ttgggggagg gtagggtgtt aggaatagtt 75480
   ttgggggtgg ggggattgga gtagagttta ttttggttgt ttgtttattt tgtatgaagg 75540
   atgttagatt tttatatgtg gttatttgat tttgtttaat tatgtgtaag ttttggttat 75600
   ttttatttta ttgatgggaa ttaaattggt taaggagaag tttagaattt gagtagagta 75660
   ggggtttaag ttttttagga gttttaggta ttttttagtt tttttttttg agtattgagt 75720
   ttatatattt agtgtttgga tgtttattta tttgggggtt tggtgattgt tagttagtat 75780
   ggtttataaa gggtttttgt gtaataggtt ttatttgatt tttttaaaaa ttttttgagg 75840
   taggataatg ttatttaatt tattttatag ataaagtaat tgagttataa aatggtttgt 75900
   taaaagatat agaggtagaa agattaatag taaagagttt ttttgtgatt ttgattagta 75960
   aggtttttag aaaaaagtaa gggattttga ggtttttata ggaatagagt tttggaggag 76020
   aataagagtt tagttttatg ttatttttaa ggggtatttt gaaggttaag gtattgggtt 76080
   taagtaggtt ggattgtgtt ttttgtattt tttttatttt ttgaagtttt ttttttttta 76140
   tttaggtggg ttgtggaggt gtttataata tataatggaa tagttggtag gttattgggt 76200
   tttttattga gttaaggggt ttgattagag gtttaaatag tggtatagaa attatgagtt 76260
   ttgttgtaaa ttagtttagg ttttggagtt aagtagtttt gtgtttatag ttagggttag 76320
   ttgtttttag ttgtgtggtt tgaggttagt tttttaattt ttttgtgttt tggtgttttt 76380
   tttgtagtag taataggaat tgttttttat ggtgatagtg tagatatatt ttatgaagtg 76440
   tataaagttt tgagtttagt gtttggagta tgttaggatg tagtaagtgg ttatttatga 76500
   tttatgaaat attaataagg tgaggggtgg aatgatagtt tttttgtgtt gtttttaaaa 76560
   tggaaataaa tttgtttagt gtttaaagta gtagaaagtg agttaaggat aggtttttag 76620
   tgatatgttt tggtagagtg ttttttgttg gttttaggta gggtttaggg tgggtggtta 76680
   ttttttagat gagtttattg ttggtatttg tttttagagt atatgtttgg attttttaga 76740
   tttttttttt tttttttttg attttttttt attttgtaat ttttattttt tgttattttt 76800
   ttatttattt ttttttttta tttttgttta aaaataaata atggaggtta aatgtgttag 76860
   ttggtttttt ggaaaggttt ttttgtagta ggaatgtgat gggggtagtg gaggattgtt 76920
   tagtgtaagt tatgtgattt ttgtgtggag ttggggaagg ggaggtgttg agattgaggg 76980
   tgttaggatt gaggtattga gatggggtta ttggataatg gttagggtta gtttttgttt 77040
   ttattttaat aggggttttt atatttaggg gttatatttt taaagttttt tggataagag 77100
   agataaattt tattataggg ttagaaatgt tgtttttagg ttgttatttg ggtgggggtg 77160
   agagatgttt tgttggtttt taaatattta ttttgtggat aaaattatta aggggaattt 77220
   aatgttttta tagtagtttg gttaagatat aggtattagg ttgggtgtag tggtttatgt 77280
   ttgtagtttt agtattttgg gaggttgagg tgggtggatt gtttgaggtt aggagtttga 77340
   gattagtttg gttaatatgg tgaaatttta tttttattaa aaatataaaa attagttagg 77400
   tgtggtggtg tgagtttgta gttttagtta tttaggaggt tgagatagga gaattgtttg 77460
   aattttggag gtggaggttg ttatgagtgg atattatatt attgtatttt agtttggatg 77520
   atagagtgag taagatagtt ttaagaaaaa aaaagatgta ggtattgttt tagggtttat 77580
   aagtataatt tgatttttga tgttggtatt taattttgat gtagatttta gtaggaaaag 77640
   ggagatgaat tagaaggttg tggggatgag gggtttatag tagtgtttat tagatttgtt 77700
   ttggtaggta taagtgtttt ttggaggttg ttagataagt agagggttta ttttagagat 77760
   tttggttttg gagggttggg ttagggttta ggaagttgta ttttggtagg gaatttagag 77820
   ttattttgag ttttattttg gaagtaagag ttagggggta gaatagaatt ggaattaaaa 77880
   gtttttgtgg gtttagtttt atggggatgg gtttggtttt atggggatgg atttttgtat 77940
   tatttgaggt gattattagt ttggtttttt gggattagtt ttgagtgttt tttggggaaa 78000
   gtgggatttg tagaggatgg ttttttagga tttgtttgat tatttatttt tagtttgttt 78060
   ttattttagt ttttttgatt tttatttttt atttagaatt ttggagattt ggttttggag 78120
   gtttagagat tagaaatgag gggagataag gggagaagtt ttttttttta tttgttggtt 78180
   atgttaatgg gttgattttt taggaagtta tattagttat tgttattgtt ttggttgttt 78240
   tgtttggttt tgaggttggg tgagtaagta ttttgttttg tttgtttttt tatttgagtt 78300
   ttttgttgat gttatgagag gtttgattat gtgtttagga ggtataggat agagtttaga 78360
   gttgtttaag aatatataag gttgaggttt gggttttgta tttgggaagg gttaggaagg 78420
   gttaaaggaa aattttagtt gagtataatg gtttatgttt ggaattttag tattttggta 78480
   ggttaaggta ggaggattat ttaagtttag gagttggagt ttagtttggg aaatatagta 78540
   agattttatt tttataaaaa aaaattaaaa ttaaaaaatt aattaggtat ggtggtatat 78600
   atttgtagtt ttagttattt ggtaggttga gatgggagga ttgttgagtt taggaggttg 78660
   agattatagt gagtgggata ttgggttatt gaattttagt ttgggtgata gagagagatt 78720
   gttttaaaaa aaatttttat attttatgga gtttagtaga atttttggtt agggtgtttt 78780
   gattgtgaaa tgagtgtggt agttgttaat ttttgagggt gaagatttat ttattagtaa 78840
   tttgtatttt ggtatggtga gaggaagtgt ttggggaaag atgttggttg aggtagagag 78900
   tttagtttaa tttattgtgg aggagttatt ttagttttaa agagaaagat taataaattt 78960
   taggtgaatg gtttaaaagt tatttttgta tatgtagagg gtgtattttg ggatatgtgg 79020
   atattttttt ttaggatagg aagtaaaatt agtaggtaga gatgtgagtt tttttgtttt 79080
   tgttggagtt aatttggagt ttttattttg gtttttaggg aagtatagat ttttattttt 79140
   attgtagtta aaagattttt tgttgatttt tttagtagta tttagtttag ttttgtgggg 79200
   agaggtggta gggggaggag atagagaaag atgagttttt ggtagttgtg attgggggga 79260
   gagaaatgtg gttttaattt gtttttgttt attttataga tgggtggtgt tttattttaa 79320
   aatttagggt agttgaggga ttgaaggaag agggggatat tttttaattg gggttttttt 79380
   ttattttatt ttatggtgtt ttatatagga aagagttgga aggttttggg tttttggtat 79440
   ttagttaaag ttttaggtag gggtttagat attgggttgt gttattattt taggtagttt 79500
   gggagttagt gatggtagag ttttattttt attttgatta ttgtatagta ttttgttttt 79560
   aggatagttt agtagttagg ttgttttagg gtttaaattt agatatttaa ttgtgggggt 79620
   tttaggtaag tggttgaagt tttttgtgtt ttgatttttt aatttggaaa atgggatgat 79680
   gaaagtaatt gttgggtagg gtaaggggta agtgaaagtt agtgtttgag agtgatgtgg 79740
   ttaggattgt tgttgtgatt atatattgag tttttttttt ttatttgaga taagggtttt 79800
   attgattgtt gttagaaaga tttagattta gaaggattag gaaagttgtt ttatttataa 79860
   ttttagaggg aaggggaggt ttttgtgtta gttggttgtt gtaggggaga gggtgggaga 79920
   tagatggggg atagaggagt aggggtagta ggaggaatgg ttggtaggga agtttgtttg 79980
   agatttaaag gattgtttat taggaggata gtttaggttt tttttattga gaaagatttt 80040
   tttggatagt tgggttgagg atttttaggt tttttgggtg gagtagaatt agttattatt 80100
   gaagttatat aggtttgtaa gtataagtgg gggattgttt aggtattttt gttttatagg 80160
   ggttaggtat taaggttagg gtggttagag gatatgatgt agtttagtag gggttttgta 80220
   gaattattag ttaagaagat atatgtagaa gtatgtaagt gttttgagga ggtaaatttt 80280
   ttgttggatt gaatatgggg agttgagttt ttaattattg agttgggttg tttggtttgt 80340
   tttttttttt ttgaatagaa ggtttttaga tagagtagtt aggtaatagt atggttaagt 80400
   aggggtttgt attttagttt tgttttgtag tttgattagg ttgttgtttt tttaggagat 80460
   atgggattgg ttagggtttt tttaggttgt tataggaata aggtagggtt gtgatttttt 80520
   ttatggttta ggtatttggg ggagggatta tgagttttag tttaggggat agataggaaa 80580
   tggatagttt gttttttttt taattatgga gttggttttt tttttattgt atttttttta 80640
   gttatgtttt ttattagaag attattgtat ttaatgggtg gttttgtagt gatatttagt 80700
   ttaatgaagt tatttggtgg gggttttggg ggatgtagtt ttttgaaata tatatgggta 80760
   gtgggtgagt tggaggttgt gtggggtttt gttttttggg gatggttagg aggtgtttta 80820
   attatttgta atttgagttt atagtgattg ggatttgttg tttttttatt ttagtttttt 80880
   tttattagta tttgttaagt gttagttagt aggtgttggt atggtttaag gtaggtttgt 80940
   agttagatta gtattggggg agggattggg agagagattt ttagagtgga agttatagtt 81000
   tttttttttt ttaggttggg taatagatta ttatttttag ttagttttgg ggaaggggga 81060
   ggggttagtt gggttgtagg tatagtgttg gggttttttg aggtggttgt tgttgattat 81120
   ggtttttttt ttttttgggt tgggtaatag attattattt ttggtttatt ttagggaagt 81180
   gggaggggtt agttggggta taggtatagt gttggggttt ttgtggtggt tgttgttgat 81240
   tatgggtttg ttattttttt tttagttggg ttgttatttt ggttttgaaa gtgttttttt 81300
   tagtttttaa tttgtgttta ggaggtagtt gatattttaa gtgttgttat agtaggaggt 81360
   tgggtttgta agtgtgattt gtttggggag gggtagaggg aaagattatt tttgtttaaa 81420
   tagtggtttt tttttttttt ttgggggttt ttatagggtt tttagttttt ttttggttaa 81480
   gaggtaagga gaggtaggaa agttttagga atattggtag agaagagata gggtaggttg 81540
   ttagaggaag taggttgtag ggtgtttaag gttttaggtt gggttttggg aggttagggt 81600
   atgggtaatt aggaggttta ggtagggaaa tttgttttgt agggattttg ggagataggg 81660
   gagaggggat atgatagttg ttttttgttg ttttttggtt gtggttttta ggggttttag 81720
   tttagattag aagtatttta tgtattgggt tttggtttag tttggaagta tggtttatag 81780
   atttatatga ttatttgggt agtgattttt aaaaggtttt ttgttattag ttggggagtt 81840
   tttgagggga gggtggtttt gtagagtatt tggagatatt aatgggttgt gaggaaagga 81900
   ggaggtgtgg atatttttat ggttttaaga gaaaattaag gtagagttgg aggttgggtg 81960
   tggtggttta tatttgtaat tttagtattt tgggaagttg aggtgggtag attatttgag 82020
   gttaggagtt taagattagt ttggttaata tggtgaaaat tggtttttgt taaaaatata 82080
   aaaattagtt tgttatggtg atatgtgttt gtagttttag ttatttagga ggttgaggta 82140
   ggagaattgt ttgaatttag gaggtggaga ttgtagtgag ttaagatggt attattgtat 82200
   tttagtttgg gtaatagagt aaaattttat tttaagaaaa aaaaagagta gagttggagg 82260
   ttaggtggtt tttagagttt gataaggagg ttgtggggtt gggttgtttt tttggttatt 82320
   tgaggggtag ggattggttt agtttgagtt ttagatttat aagaggttga attggagtgg 82380
   gattttgaga tgaggtaggg ttattttgtt tatatttgtg tttaggtttt aatagttatt 82440
   ttttttagga atttagtttg atggtttttt aggttgagag gttgttaagg ggttgtaagg 82500
   tgttgtaatg tgtgagttgg tgggttttga tgtgtgatag tttggggtat tttttttatt 82560
   gttagggaat aggaatgagg agttttttat ttttagtgtt tggtaggtag gaggttgtta 82620
   ttggattgtg tgtttaggtg ttattagatt ttgagggttt atggatatgg tatatgattt 82680
   ttaatgatta atgtttgtgt agtaggtgtt gaggtgtttt aattttgagt aaggttgggt 82740
   ttttggaatt ttttttttgg taggaggatt tgtagttgag gaatttttaa gaaatttttt 82800
   ggtagtaaat aaagtatggg gttggatggt ggatttatga gtgtttttat atgaatgtgg 82860
   atatagatag aaaagatggt aaatatttgt gaataaaagg aatggttttt gtgtattgaa 82920
   tttttattta ttggtttttg tttggtataa agtttttagg aattttttaa tagttttagt 82980
   tagaaatgtg tttttgtttg gttatgtgga aaattttata tatattaaga tatatgatgt 83040
   ttttggtttt ttttatgaat gttttatgtt ttttgggttt atgatttgga atttgtagat 83100
   ttttgttgtt tttgttttag agttataagg attttggatg taggaaatat tttttatgtg 83160
   taagggtttt tttttttttt gttagggttt attggttttt ttttgtttat aggtgttatt 83220
   taaatttttg tatttgtggt tttttttttt taattatttt gtgtaatttg ttaatagttt 83280
   ttttattttt agagtttggt tgttggagat atataaggta tatagaattt gtgaaatgat 83340
   attgagtatt tattaggtat ttattgtgtg ttttgtttgg taataataag taaaattata 83400
   atgttttggg tatatgagta tattttttta tgtttatttt tttagtaatt taatgagatg 83460
   gttttataaa tggggaaatt gaggataggg aggttagatt ggtttaaagt tataggttag 83520
   gaggggtagg gttaaaattt aaatttagat agtttggtat taaattttag ttttttatat 83580
   gaaaggtatt ttgtaatttt ttgagaaggt tgggattatt atttttattg tatagatgag 83640
   gaaattaaga tgttaaatgg ttagatagtt ttggagttag gaggtgggtt tagtggttta 83700
   tgggagtata tggaatggag tttatgggag gtttgaggtt gggattttgt attttatttt 83760
   ggtttaaggg atgaggtgat tgggagtgtt agagatattt tgggattggt tatttaggtg 83820
   ttttgtattt agttttagtg ttgagtgggt gagggtggat aatttgttat ttagtagggt 83880
   tgttgtttgt attttggtgg ttttttaaag tttggttaag taaagtaaga ttgtttttgg 83940
   attagagtga tttttaagtt atataagtat ttagttatgt tttggaggat gtaggattgg 84000
   gaaggttttt tttttattgt atttttattt agttttttta tggggtagtt ggttttaggt 84060
   atagattaga tgtttttttt tttgtttgtt gttggtatgt ttttggtttt attgttgttt 84120
   tagggagggg tttaagagtt ttttggtttt gttatttatt atattttgtt tagagggttg 84180
   ggggtttagt attaatgtta agtatttagt agtaaaatta tgggttggaa agagttatag 84240
   tgttgttttg atttggtttt attttatatt gggtgttgtt tttattagtt tttttttgtt 84300
   tttggtttta ttttttaggt attataagtt ttgtaggttt aaagttaaat ttattagttt 84360
   ttgatttttt ttagttggga ttagggtgag gtttttgttt aggtgtaaaa gttaaggtag 84420
   tgttaaaaaa tttaggaatt aaataatatt ttgaagtaat attttaaata aattaaattt 84480
   tatgttgaat aaattttgat agggttgtgt atgtgtaggg ttgggtttgt tttaatttaa 84540
   aattttaata tttgtttatt atagagatta ttatgtatta ttttgatatt gaaaaatatt 84600
   gtattaaata ttatttgtgt tgatttttga gttttttggt gtttttttat attttgtttt 84660
   tgataagtgt ttgatatatt ttgttttagt tggttttgtt ttttaggtgt ttttttgggg 84720
   ttgttttgtt tatgtttttt gtagttatta ggagttattg agttattgta tttgttttat 84780
   tttgtttttt atgtttttgt attgtttatt ttgttatgtt ttttttgtgt gttttggttg 84840
   tatgttagtt ttagatagga gtttggtggg ttgggagttt atattaatgt tgtttttttt 84900
   tggttttggt gttgggtata gggtagtatt ggagattttt tttttttttt tttttttttt 84960
   ttttttgaga tatagtttta ttttgttatt taggttggag tgtagtggtg taattttggt 85020
   ttattgtaat ttttattttt taggtttaag ttttgttttt tggaattaag tagatttttt 85080
   tgttttagtt tattgagtag ttgggattat aggtatatgt tattatattt agttaatttt 85140
   tatattttta gtagagatgg ggttttgtta tgttggttag gttggttttg aatttttgat 85200
   tttaggtgat ttgtttgttt taatttttta aagtgttggg attataggtg tgaggtatta 85260
   tgtttggttg agattttttg aataaataaa tattattggt atggagattt gtttagggaa 85320
   tttgtttaga ttttgttatt gtatttttgt ttgttgtgtt tagaggtaat ttagggttgt 85380
   tttagttgtt tttatttggt tattttttgg tttttattta ggttggtttg ttttattttg 85440
   tgggttaaga aaatgattta gtgggtggtg attagtattt tttttttaat ttgaaaagaa 85500
   tatatttatt agtggtggta gtaatttaat gttttttagt tgatgaaggt aatatgtttg 85560
   tttataatgg aatggtttat gttatggtgg aatattattt agggatgtat tggtatttgt 85620
   tataatgtgg atggattttg gaaatatgat attaagtgga gaagttagat ataaaggtta 85680
   aatgtgtaat tttttgtata agaaatgttt agaattggtt aggtgtagta gtttatgttt 85740
   gtaattttag aattttggga ggttaaggta ggaggattat ttgagtttag gagtttaaga 85800
   ttagtttggg taatatggtg aaattttatt tttataaaaa atataaaaat tagttgggta 85860
   tggtggtata ggtttgtagt tttagttatt tgggaggtta tggtgggagg atggtttgag 85920
   tttgggaggt ggaggttgta gtgagtagag attgagttat tgtattttag tttgggtgat 85980
   agagtgagat tttattttaa aaaaaaaaaa aaaaaaaaaa agaatttata tagataggaa 86040
   ggaaatatgg aggaggaggg taggattgat agttaaaggt tatggggttt ttttttaaga 86100
   aaaaaatttt ttaaattttt taaattttta attttttaaa attggttgtg gtgatgatgg 86160
   ttgtattatt ttttgaatag attgaaaatt atggaattat gtattttaaa tgggtggatt 86220
   gggttatgtg gtgatttatg tttgtaattt tagtattttg ggaggttgag gtgggtggat 86280
   tatttgaggt taggagtttg agattagttt taatatggag aaattttgtt tttattaaaa 86340
   atataaaatt agttgggtgt agtggtatat gtttgtaatt ttagttattt gggaggttga 86400
   ggtaggagaa ttggttgaat ttaggaggtg gaggttgtag ttagttgaga tggtgttatt 86460
   gtattttagt ttaggtgata agagtgaaat tttgttttaa aaaaataaat aaataaataa 86520
   attggtggat tgtatagtag tttatttgga ttttaataaa gttgtttaaa aaaaaatgaa 86580
   aaaaagtttt tttttttttt aaaataaata aataaaaaga aaaaaagaga gaggggaaaa 86640
   aaaaaaaaaa aaagaaaata ttgagtgtag tatatgtatg gtaaaggtaa gaattttatt 86700
   atggaaattt ggttttatta atatatttta tttttatttt tattttatat ataggtgtgt 86760
   tttggtttgt ggtgttaatt gtatgttttt ttggaggttg tagtttagat tggttttgaa 86820
   agttgttttt tgggtaattt taaatttttt ataatttttg aagtttttgg tttgttggga 86880
   ttatttagtt ttttagattt agttttgtta ttatttattt aagattgtag ttttggggag 86940
   gggggttttt ttttaagggt gtaggggttt tgtggtttta ttttttttta gaatatatat 87000
   tattttgtat tgtatatatg tgttttaata tggagtattt taggatgttg gttatagtta 87060
   gttatgtttg tatattgatt gattgattgt gttttgttga ttaattatgt taattattgt 87120
   gttgtatatt gtttttatta agtgagttaa agtaattttt ttggaataag ttgatggatt 87180
   ttaaataagt taaaagatat ttttttagga aaaaaatagt aatgaatttt aagaagaaag 87240
   aaaaattttt aaatttatag atttatggaa aaagtttaag tttttaaagt gttagatttt 87300
   tttaaaagta ttaggtttag atggttttat gagtgaattt ttgaaaagtt ttaatagaga 87360
   gattattatg ttgttgttta aaatgtttta gaaggtagag ggtaaaagaa atgtatttaa 87420
   attgtttttg ttaagttagt ataatgtaga tattaaaatt gataaaaata gtatatatat 87480
   atatatatat atatatatat atatatgttt tatttgtttt agattaagtt tgttgttgtg 87540
   tatttaagta atattattta tttttatttt gttgttgaaa ttaaaatata ttttaatgga 87600
   tttatgattt taatatgtaa aaatgaaatt atagaagtat tagaagaaaa tgtgggtgaa 87660
   attttttttt ataattttag aatgtaggag atttttaaat aggatgtgaa ttttaaaagt 87720
   taaaagggaa agtagagatt tgataatatt aggattaaaa tgttttgtgt agaagaatat 87780
   tttagaaata aaattaaaag taggtagttg gggataagat atttgtattt gttgtgataa 87840
   agatgtataa ataatttttt ttttttttta gataggattt tattttgtta tttaggttgg 87900
   agtgtagtgg tatgattata gtttatagta gttttaaatt tttagattta aatgattttt 87960
   ttatattagt tttttgagta gttgggatta taggtgtatt attttattta gttaattttt 88020
   gtagagatgt ggttttgtta tgttgtttag gttggttttg aatttttaag tttaagtaat 88080
   ttgtttattt tagtttttta aagtgttggg attataggtg ttagttatta tgtttggttt 88140
   ataaagaatt tttttaaaaa aattttaata agaaaaggat atatttttta aatgaaaaga 88200
   gaatataggg ttttggttta taatatatga agagataatt ttttttgttt gattgagatg 88260
   tataaaataa aatattaatt ggagagtatt tggtttttag ttaggggtaa ggggtatagg 88320
   atttttttgt tggattatta ttgtataggg tttagtagat agttgagtag gttgatgagg 88380
   ggaggggttt taaaaaattt tagtaattat tttagaggtt aatttaggaa taaggattaa 88440
   tttttttttt tttttttttt ttttgatagg gagttttgtt ttgttattta ggttggagtg 88500
   taatggtatg attttggttt attgtaattt ttgtttgttg ggtttaagta agttttttgt 88560
   tttagttttt tgagtaattg ggattatagg tataagttat tatgtttagt taatttttgt 88620
   atttttagta gagatggggt tttattatgt tggttaggtt ggttttgaat ttttgatttt 88680
   aagtgattag tttgttttgg ttttttaaag tgttgggatt ataggtgtga gttattgtgt 88740
   ttagtagatt aaaatttaaa tgtatatatt tttgatatag aaatttatta agagtttatt 88800
   ttttagttat gtgtgtatat gtataagtgt gagaatattt gttgtagttt tttaattata 88860
   gtgggaagtt ggataagttt ggtagggtta tgggatagtt gatgatataa ttattaaaag 88920
   gaatgtgtta ttttgggagg ttgaggtggg tggattttaa ggttaggagt ttaagagtag 88980
   tttggttaat atggtgaaat tttgttttta ttaaaaatat aaaaattagt taggtgtggt 89040
   ggtaagtatt tgtgatttta gttatttggg aggttgaggt aggagaattg tttgaaagtg 89100
   gaaggtgaaa ggtggaggtt atagtgagtt atagtgagtt gagattgtat tattgtattt 89160
   tagtttgggt tatgttttgt taaaaaaaaa aaaaaagaaa aagaaaaagg aatgtgttat 89220
   atttataagt ggtaatagtg ttgttaaatg gaaaaaataa aagttggtag tagaagtttg 89280
   attttttttg tgtttttgaa aagtttatat ttatatattg atttttttta tataagtttt 89340
   gaatgatttt gtaggaataa aatttaggag aatgttatat ttagttgtta atatggttat 89400
   tttttgggag agagttgggg aatgggagag aaaaattttt ttttaaattt tatgtgattt 89460
   ttatttttta tttaagaatg attgggtatt gttttttttt ttttttttta gatgggagtt 89520
   ttgttttgtt gtttaggttg gattgtattg gtataatttt agtttattgt aatttttgtt 89580
   ttttgggttt aagtgatttt tttgttttag ttttttaagt agttgggatt ataggtgttt 89640
   gttattatgt tttgttgtgt attgtttttt aggatgaaga aagggaaagg ttatagggag 89700
   aatgggagtt agaatataga gtagggtttt taaatattag tttgtgggtg agagagatat 89760
   ggggtgatat tattggtttg gaatttaggg atggtatgag gtgttgttta ggggttttag 89820
   ataaggtttg tttgttgatt aagtaagggt tgtgttggtt tttgtttatg tgtttgttta 89880
   atatgaattt tttaggtgtt ttattttgtt ttatgaatta ttattgttta ttttaattat 89940
   gttgtatagg aagaattgtg tttattttta taatgagaaa atggattaga gaggaaagtt 90000
   attaggtagg gtaggggtgg tagtttaggt agtgtgaggg taaagttggt gtttttttgt 90060
   tattttttta tatatgagtg ggtaaggtat tataggagtt tatattttaa tttttgtggg 90120
   ttattttgga aggtggtaga tggaattagt gatttttagg gttattttta gttttaggtt 90180
   ttagataggg gttgtggagt ggaattgagt ttgatttagg tgtgttgatg gtagttatgt 90240
   aattgtggat aaattatttt tttttttatt tgtattggat gagattggga atttttattt 90300
   tgggtttttt aagagttttg gggttttata gagtatattt agtagtagta gtgggatggg 90360
   gggatagggt taggggagtt ttatgttttt tttaatttta attagagtat ttttatttta 90420
   attttttttt gggtttagtg tttagagaga atgggaagga agtttagttt tggttatggg 90480
   ttattttagt ttgttagtta tagtttatat tttaggagtt ttttttggat tgaaggagat 90540
   agagttattt ataatattta tagaaaattt tggagaggaa gtggagttta attgaggaag 90600
   tgtttttttg tagtttttag aaatgaggtt ataggtgaga tagtgtgtta gggtggatta 90660
   tatggtattt ttttaggttg tttgttggaa tttagggttt ggttaggggt tgtttatatt 90720
   ttgggttttg aggtggggag tttgagtgtg tttttttagg gttgaatgtg ttttttgttt 90780
   atattgtata gagtatggtt gtttggttgt ggtgtgtttt tttggatagg tttgttattg 90840
   tgtagatttt taattttggt agttgttttg ggtttgggag gtggtgggta tagatatagt 90900
   tttttttttt tttggtagtt gttttgattt tttgggataa gtgttgagag gtggttttat 90960
   ttagtgttat gtgtttgggg gttggggttt tattttgggg ttaggttggg tggttagggt 91020
   tatagagttg tagggggtgg agtagagaaa ttgtttttag tgtttgtttt ttggttttat 91080
   ttttttttgt agttgtgttt tatttaaggt attttatagt tgtttggtat ttgttgtatt 91140
   tttggattag tttttttttt tttttattat tgtatggtgt ttggtaggtt ggtgagggga 91200
   ggggtttttt ttttgggttt tggagttagg ttttgttttt tggttttatt ttgttttatt 91260
   atgaaggaga tatattgggt ttggttaatt tttttgtatt gatttttgtt tttttagtat 91320
   atgtgggatt ttttatttta gatttaggtt tgagaggttt tagggatagt ttggagaagg 91380
   agggtatttg tttttagaag gggaaagaga aagagattta attttaaagt aaaaggggat 91440
   ttgggtgttt tttgtaagta gtttaggaaa atgggaggag gtgggagtag tgggattttt 91500
   tgagtaaggt attttggtag gaatgttagt gttttttgga tatttttaga agtagaagta 91560
   tatagtttta tagaaaaatt ttgtaggttt aggatttgag gtatgatatg atttaaattt 91620
   attgttttat tagatattag tgagtttttt gatgtttatt aaggtttttt gttttatatt 91680
   tggtattgga tgaatttgta tattttgttt tagggagttt atagtttagg gataggttta 91740
   gagtttatgt ggggtgtttt gtaaatatgg ggttagaagt tgggttgttt atggggtttt 91800
   ttttttttaa attatagtgg ttattttttt tttaatttta tagtttttga tttttattag 91860
   gaatttttgg ggtttagtag gtgttttttt tttgtaggtt gatgtttgtt ttttttattt 91920
   tttatttagg tttagttttg attttatttt ttttaaagtt atttttttat tgttttgtgt 91980
   tgggtggttt tagggagttt tgtgttttga ttaattttat atttaatgtt gtattggtag 92040
   ttttgttgtt tgtgttgaga tttttttttg ttttgttttt ggaatatatg gtttggtgtg 92100
   gattaagtat gtgaagagtt atgtttatta ttattagttt gggggggttt gattttttgt 92160
   ttttttaatg ttttatttat atgataaaga tttttagatt ttttgggtta taggaaaatt 92220
   attgagatat gttgttatgt ttattttgtt ttaattttat atttatgttt ataagtggtt 92280
   ttagaattat tgtggatttt ttttttggtt tttggggtgg gtggatatat gagagtttag 92340
   tttttagtta tttttgtaga tgtttttttg atagattaag aaattgaggt tgggaggggt 92400
   ggaatgatag tttagaatta gttgatttat ggggtagagt tgggatatgt ttagaattga 92460
   gttgggtatt gggtttggtt ttttttttgt ttttagtgag tattttaaga aggtagagaa 92520
   ttagatttat gaatgagtgt tgagtatttg ttttataagg tagttttgga gaaatttggt 92580
   tggtgtggtt gaggagttgg aggggggtaa ttttggtggg gggggttttg agttagtagt 92640
   tagtttttgt aggtttagta ttgggtgttg tagtttagtt ttattttatt ttatttagag 92700
   gtatttatgt tggggggtag gaaaagagta gggatttagg agaggaggta aggttgtttt 92760
   tatttagagt ttgagaggat tagggatgat tttaaaggat gagtgttttt ttgtgggagg 92820
   atagtaagga gggtgttaag tttaggggta gggttagagt tttggaaatt gagaatttat 92880
   tttattagta taaagtttag attttattag gttttggggt ggaggagtag gtagggatgt 92940
   ttttgttttt tgtagtgttt tttttttttt tttttttttt taattattta ggtgtttata 93000
   tttttttttg aaatgttgat tttttttaga atttttatat tgaagttaat atttatttat 93060
   tgagtttttt agtataattt ttagaggaga gattaggtgt tttgggggta aagttgatta 93120
   ggaaagtttt tgtttggttt ttagggatga tttatggggg ttattatatt tgtttataag 93180
   aattataaaa aagttatttt aatttattat tagtggttta tttttttagt ataattttat 93240
   ggtatattta ttatgtttaa gttatttatt tggtatttta tgggattttt tttttttttt 93300
   tttttttttt tgagataggg ttttattttg ttttttaggt tggagtgtag tggtataatt 93360
   ttagtttatt gtagttttaa ttttttatgt ttaggtggtt tttttatttt aattttttta 93420
   gtagttggga ttataagtgt atgttattat gtttggttaa ttttttgtat tttttgtaaa 93480
   gatagggttt ttttatgttg tttaggttag ttttaaattt ttgggtttaa gtaatttgtt 93540
   tgttttggtt tttaaagtgt tggatttata ggtatgagtt attgtatttg gttttttatg 93600
   ggatttttta taaaaattaa gataggttgg gtatggtggt atgattttag tattttggga 93660
   ggttagggaa gttgaggtgg gtgggttatg aggttaggag tttgagatta gtttggttta 93720
   tatggtgaaa ttttgttttt attaaaaata taaaagttag ttaggtgtgg tagtttatgt 93780
   ttatagtttt agttatttgg gaggttgagg tagaagaatt gtttgaattt gggaggtaga 93840
   ggttgtagta agttgagatt atgttattgt attttagttt gggtgataga gagagatttt 93900
   attttaaaaa ataaaaaata aaaaaaaaat ataaaaaaaa ataaagataa tagttttatt 93960
   tttagaattt agtgggggta tgaaaggaat tttgaagatt atatagtaaa attttaagta 94020
   tggatattgt tggtttatag gtgaatttta ggtttttttt tatttttgta ttttttgtat 94080
   tttaaagaaa gggtatttga ttatttttta tgatttgaaa aattattttt atatttaaaa 94140
   aaaaaagttt agtagaggaa agaaagttta tatataaatt atttatattt taagttaaat 94200
   atgaggtatt tggattaggt tttgagggat gtataggagt ttattaaggt agtgatgtaa 94260
   agagaagtag aggttagggt ttaggaggtt agggtaaagg ggttgtgagg aaaggtgtga 94320
   gagattaagg gaagattaaa gtaggagaag ttgggaagga gagaggtgtg agttgagtag 94380
   gagagtgaat taattgtgga ggggtttatg gtgagtgagt agagaaggtt tagtatggga 94440
   agagatttta gtagtgttgt gttttgggga agttagtttg ggagtaattt agttggggtt 94500
   ttaggagatt ttgttttgta tttatagttt tggggtggag ataaattata gtatagatgt 94560
   ttttttgtag gtagaagtta aggggttggg tgtttgttta gagggtatgg aatttttttt 94620
   atgatttttt tttttatggt gttagttgga tttttggtgt ttgggggata gaggggttgt 94680
   agagattggt tagagttgtg tatgttgggg tttagggaat ttttagttta gttttatatg 94740
   gaggggtaga aggggatggg tttgaaggaa agatgggtag gaagttgagt tggagagaat 94800
   tggatttgtg tttagaatgg ttgtgatata gagagggagg atggagaagt aagtaggtgg 94860
   tgttttggag gaagaattag tggggtttag gtttgggttt tttgagaagt tatttttatt 94920
   ggattttaag agtatttttt tattgggaag gttttgtttt tgtttgtttt tgtatttttt 94980
   ttatttggat ttgtttaggt ttttaggatt gttagtttag tttatggatt tttttatgtg 95040
   agttggattt ttatttgtat aggtttttgt ttgtgggtgg ttgggtttgg gatgttgatg 95100
   gttttttttt atgttttggt tttaatgtgt atattttatt ttatggagaa agttattgtt 95160
   tttttagagt gttaggtttt tggtagtttt gaatgttgtt gtttttatgt ggtttgtttt 95220
   tttttttttt ttttggtttg atgaaattgt atttttattg ttggggaagt tttttggata 95280
   ttttttgtgt tttagttatt ttgtttatgg tttggtgagt tttagaaagt agagtttgta 95340
   ggttgtgttt ttgggtttgg tttttggtat agtttttggt ataatttagg ggttaaattg 95400
   tgtttgtttt tggaggagaa tgtttttgta tatttttatg gatatttttt tatttgtttt 95460
   ttagagttag gattatgttt ttgtgtattt agtattagtt attattgaga tttagatggt 95520
   ttggtgataa ttgttttttt agttatattt tgtttgattt agttgagtgt gaatatatta 95580
   atttaggatg ggggtgtatt tggtatgtta aataaaggtt ttgtattaat agaaggtagg 95640
   ggaggaaata gttttttgtt attttgtaaa ttgtttatgt ttttggtttg atattatgtt 95700
   attagtaatt attgttattg agtttgggtg gttggggtag ggtggttttt tagagttaag 95760
   tttagagaga tgggagaggg aatatgggat ttgtatattg agggttggaa aggatttttt 95820
   aaggtttatt agtttttggt tatttagtgt gttttgatgt tttttttgta ggaaatgtag 95880
   agttttaggt tttagatttg ttaagataaa tgtgtatttt atatgtttat gaaagtatga 95940
   ggagtattgg ttttttttta aatttatatt ttttttaggt tttatatttt tttagttttt 96000
   aagtttattg attattggag aatttttatt taaaaagaaa aattaaaaag gggttgggtg 96060
   gtttatgttt ataattttaa tattttggga ggttgaggta ggaggtttat ttgaatttag 96120
   gagtttgaga ttagtttggg taatttagtg agaatttgtt tttataaaaa aaatttaaaa 96180
   attagttagg tatggtggtg tgtaatttgt ggttttaggt atttgggagg ttgaggttgg 96240
   ggaattattt gagtttaggg ggtagaggtt gtagtaagta gtgattatat tattgtatgt 96300
   tagtttggag gatagagtaa gattttgttt taaaaaagtt aaagaaaagt taaagaagat 96360
   ggaaagaggg atttagagag atgtggaagt aatttaaggg tttattgatg gaggaatagt 96420
   tatataatat aagtgtattg tataatagaa tattatttag tttttgaaag gaaggaaatt 96480
   ttgttttatg ttatagtatg atggatgaat ttttaggatg ttatgttgag tgaaatgagt 96540
   tagatataaa atgggtagtt attgtatttt tttatatata taaggttttt agagtggtta 96600
   aataaattta taataaagat agaaaataga atggtggttg ttagtagttg ggggaggggt 96660
   atggggagtt tggtttaatg gggatggagt ttagtttggg aggatgaaaa gggtattgga 96720
   gatggatagt ggtggtagtt gtgtaatatt gtgaatgtat tgaatgttgt tgaaatgtat 96780
   attgtaaagt ggttaaaatt gtatattgta tgttgtgtat tttattatgg ttagaaataa 96840
   gaagtagtag ttaaattaaa aatttagatt gtgttgtttg tttaatttaa agaaggttta 96900
   ttgagtatta ttgagttttg attttgtgtt tagaattaga tttggttttt gaggggtttt 96960
   taatttagtt ggtattttat tatagtttta tggagttatg atttttgttg gggttagggg 97020
   gtatgttttt gagaagggat ttaattatgt tttatagttt ggtattttta aataggtttt 97080
   gtgttttata ggattggtat taattttttg tttgtaagtt ttatgttagt atggttgtgg 97140
   gtttgagttt ttttaggatg ttgttggagt tttatggttt tttttttttt gttagaaggg 97200
   tatttttaaa taaaattgtg tgtagttatt atatagtttt ttggtttagg tatttttttt 97260
   tagtgttttg agaattttgt tttgtaggtt aaggtgtttt ttatgttttt ttttttttgt 97320
   tataattatt ttttttgggg gaggaggatt agattatggg ttttaatgta agttttagga 97380
   ggtatatgtt tttttttttt tttttaagtt tttggttttt gttgggtata tttaggtagt 97440
   ttgtaatttt ttaagtagga gttattagaa tgtgatagtt tttttagggg ttttaatttt 97500
   attttgtttt atatttttgt atgagtttgt tttggttttt gtgtggtttt ttatttttta 97560
   agttttttag gttttttagg gatatatagt agatagaggt tttagtaagg tttatggtga 97620
   tttataaagg aggttttttg aaatttagaa aattttttta aatttttatt taagttttgt 97680
   ttggggtatt ttggtatgtt tttattagat agagagagtt attttttttg agtttgtaga 97740
   tttttatttt tatgtatttt ggtttttttg ggtttttagg gtagtatttt ttattttata 97800
   tttagttttt ttttttaaga ttttgtggta aagttagtaa ttaggttttt tattattata 97860
   gtatttaaga tttagtgttt tgtgatttat ttttttggtt ttggggtgta gttttagtta 97920
   tagggggttg gggtttgggt tttagtttgt tttgtagttg agatataggg gtaggttttg 97980
   aattttttaa ggtttgggtt gggttggggg tggtgggtgt tttagtaggt ttgggtattt 98040
   ggatttttat ttttttgggg gttgtgttgg tggtaggggt agtgggtatt ttggggattt 98100
   tggaggtagg gggttttatt ttttggtggg ttgatttttg gggtttgatg atggtgattt 98160
   ttgttttttg aggggttggt tttggtgttt tgtggtttaa tattttggtt tttttgagtt 98220
   tgaattggga tgggttgatg gttttggtgt tttttatttg tttggatgag atgttaatgg 98280
   atagtttagt gtgttgggat attggtttgg ttgttttggg gtttgagagt tttatttttt 98340
   gtagggatgt tttgggggag tgttggttta gggagtttat atggtgggtt gagggttttg 98400
   agttttttat gtttaggttt tggaattttt gggtggagtt ggttatagtg gtttggagta 98460
   ggggagtttg gattttttgg ggtggggtgg agttgggtgt tttgattttt ttgattttaa 98520
   aagatttttt taaggttgaa aaataaagag aggggatgta attggtgaat ggatggttag 98580
   ggaatatttt ggtatgtttt ttgttaagga gtagtagagg aaggtaggtg atgtggtggt 98640
   gagggttggg ggtttttttt tgttttttat gttttagtaa gtggaaggga tagttttatt 98700
   ttaggagaat tattatgagt tttggggtag agatgtaggt tggagtggga agatgttgtt 98760
   tgttgtgttt tgttatggtt atggtgtaaa ttatatagtt ttttttttta gttatgttta 98820
   tataggaatg ttttagtttt agagaagatt tttatttttt tttttttttt tttgagatgg 98880
   agttttgttt tgttgttttg gttggagtgt agtggtattt tttgggttta agtgattttt 98940
   ttattttagt ttttttagta gttgggatta taggtatggg ttattatgtt tggttaattt 99000
   ttgtattttt agtagagatg gggttttatt atgttggtta gtttggtttt ggatttttga 99060
   ttttaagtga tttatttgtt ttggtttttt aaagtgttgg gattataggt gtgtgttatt 99120
   gtgtttggtt ttgatttttt ttttgttgtg gttgttttta ttttttttta tttttagtta 99180
   aggtatatat tgggatatta ttagtaaata tatttttatt attagttata gtgggggata 99240
   tgtttttaat aataagtgat tgtaattgta attaattaga attgtagtag gaataaaatt 99300
   ttattaatga tattaagggt ggatattgga gtttttagtg ttggtgaggt gttgggttta 99360
   ttgagtttat tatatttgta gttgtttaat tttgggatgt aaagaatatt agtattttta 99420
   gtatagatgg ggaaagtggg gtatggggta gatatagagt tagattgtat attaggttga 99480
   tattagagtt taaggtttta attatttggt tgtttggttt ttttagttta gtttatagga 99540
   attaattttt ttagttatta gttttttttg tattttttta tttattatta taagtttgtg 99600
   tagtgatgtt aattattagt ttttgttttg tagttatttt gtttttttat tagggtttgt 99660
   ttttttggag gtagtgggga tggtatttgg gaagttgtta tttttatagt ttttataatt 99720
   gtattttaaa tgagagttag ttgtttttgg ttattagggt tagagtaggg agggatttat 99780
   atggtaggta tatttattag tttttttttt aggttagatt tatagtgtat ttttgtggag 99840
   atttatagtg tttttttagt tgttttttgt tttattaggt tatagagttt gggttttatg 99900
   ggattttatt attgtttttt ttgtttatag gaaaagggat gatagttggt ttttagtttt 99960
   taataatagt gttttaggat taattttagt gttttttttt tatttgtttt ttttttttta 100020
   aatgtattta ggtattgggg aggggaagag gaagtttaat tttttaattt tggttgtttt 100080
   agttgttagt tttttgggta aggtattttt gtgggtgttt tttttaagtt ataggtattt 100140
   tttttttttg gtggatatag atgaaatttt gggttattaa gtttaatttt tattagaagt 100200
   tgggtaagga gttgtgtgga ttttagagaa tttttagttg ttataggttt tgttttagtt 100260
   ttgttattgt tttagaggat atgggtttgg tgtggttaga gttgtttatt ttttaagaga 100320
   gtagaaattt ggatttttat atgatatttt ttgattttta aatgttggta taaagtaaaa 100380
   taagtttttt ttaaaatatt gagtagtttg gttgatgtgg tgaaatttta tttttattaa 100440
   aaataaaaaa ttagttgggt atggtgatat ttgtttatag ttttagttat ttaggaggtt 100500
   gaggtaggag aattgtttga atttgggagg tagaggttgt agagttgaga ttgtgttatt 100560
   gtatttagtt tgattgatag agtgagattt tgtttttata aattaaaatt aaaaataaaa 100620
   taaaatattg tagggttaaa taaaattaaa tttataggtt gtatttgttt atgggtttag 100680
   ttatgatttt tagttgatta tgtggttgta gggtagtagg atattgagtt ttagagttag 100740
   gggtgtgtta ggtgtgggtt tttgatatgg tgtttttttg tttttttgta ttttgtatgt 100800
   aaattgttgg ttgtgttttg attttgtaga agttttaggg ttttaaggaa aggttgaatg 100860
   tttattttta ttagttagaa agtttagggt ttaatgttta gaattgaatg taatgagatg 100920
   ggtttattaa gtttattata tttatatttg tttaaatttg ggttgtaaag aatattatta 100980
   ttgtttttgt atagatgggg aaagtgaagt atggggtaga tatagagtta gattgtatat 101040
   tgggttgatg ttagaaagtg tagagtttaa tgtttgggtt tttgtgtggt ttttagggtt 101100
   ttttgtggtt ttttgatttg ttttttgttt tttgagtatt gtttaaattg tttttatatg 101160
   gtgtgttttt gttttatggg tgttgtagag taggggttgg tttttaggtg tttttagtat 101220
   ttttttgtgt ggttaagttg gagaatagaa attagatagg agaaggtttt gttttaggtt 101280
   taatttaaga aaataaatga ttttttttgg gagatttggt gaattttttt tttgggagtt 101340
   tttttggagt tatttttatt atggttaatt tatgattgaa gatattgtat taaagtagag 101400
   gttgttttgg tgtgagttta tgattgagtt tatggtgtgg atgtaatgat ttattttagt 101460
   taggtttatg tgtataagtg gattatttta gaattggagt tggttttttt tttagggtat 101520
   tttttttttt tttagttgtt tgtgtggttg gtgttttatt attttggtta tttgtgtaaa 101580
   atgtatatgt gtgttggttt ttaattattt tttttttttt tttaatgttt ggtgtttttt 101640
   tatggatggg tttttttttt tttattagtt ggtttttgtg tttattgttt ggttttatga 101700
   tttttttggg tgttttggtt tagtattatt tttatgttta ttagttttta gtaagttttg 101760
   agatagtagg ggttgtttta tttgtagggg ttattttatt attaggatgg tgttgtttaa 101820
   aggtgagttt gttttagata atagttagga gattttatag tttttagaat tgtaagagaa 101880
   tataattgtg tgttagaaga atagtgagaa taggaggtgt tttattttgg aggtgtttat 101940
   tttttttagg agatgtttta ttttgggggt gtttattttt tttaaggaaa ttatgttttg 102000
   gttggaatgt taagaaggtg atatttaaat gaagaggtgt ttattttggt ttatgttatt 102060
   tttttaaatg agtatgttat tgtttttttt tttttttggg aatttttttt aaagttaagt 102120
   ttattttagt tttttttttg gggagttggt tttttatata gtttatatta tttttttttt 102180
   atgttatttt ggttatgtta tgattgttga gattggttat gaggggtgtg ttttggtttt 102240
   ttgggaatag agttgaatgt tttttatttt atgatattat gtttttatta ggttttaggt 102300
   gtttttagaa aatttattaa gttaggtgtg gtggtttatg tttgtaattt taatattttg 102360
   ggaagttaag gtggatagat tgtttgagtt taggagttta agataaattt gggtaatatg 102420
   gtaaagtttt gattttatta aataatataa aaattggtta ggggtggtgg tatatgtttg 102480
   taattttagt tatttgagag gttgaggtga gaggattatt tgaggttagg agattaaggt 102540
   tgtagtgagt tatgattata ttattatatt ttagtttgga tgatagagtg agattttgtt 102600
   ttaaaaaaaa aaaataatta aaatttatta aatgttaggt gtggtggttt atgtttgtaa 102660
   ttttagtatt ttgggaggtt aaggtaggtt taaggtaggt ggattatttg aggttaggag 102720
   tttgaaatta gtttggttaa tatggtaaaa ttttgttttt attaaaaata taaaaaatta 102780
   gttaggtatg ggggtgggtg tttgtaattt tagttatttg gaaggttgag gtagaagaat 102840
   tatttaaatt tgggaggtag aggttgtagt gagtttagat tgtgttatag tattttagtt 102900
   tgggtgataa agtaagattt tattttaaaa aaaaggttgg attataggat tataggatag 102960
   gtataagttt tgagggagtt tttaaggagg agggatgtat tagattttta tttttatttt 103020
   agtttgtgaa gatttatttt tgtttgaggt tttgtagagg gttgattagt gatttttaaa 103080
   aagatatgtt taatatttaa ttttaagaat ttatgaatgt gattttattt ggaaaaaggg 103140
   tttttgtaga tgtgattagg gattttggga tgagattatt ttggatttgg gatgggtttt 103200
   aaatttagtg tttggtgttt ttataggaga aaggtagaag gggatttggg atataaagag 103260
   atatggtggg gagggttatg tgatgagagt agagattgta gatgttgtta gagttaagga 103320
   gtgtttgggt taatgtaagt tggaggaggt aaggaagggt tttttttttg agttgttgga 103380
   gggagtgtgg ttttgttaat attttgatgt taaatttgtg gtttttagaa ttatgagtga 103440
   attaattttt gttgggttat ttggttttta gtaatttgtt ttagtagttt taggatattt 103500
   ataatgagat ttttgggaag atgaaatttt tggttgtata gtggtttagg gtgaggttag 103560
   ttggtgtggt tatttatttt ttagtttagt tttaatttta ttataattat atttgataaa 103620
   tggtttattg tgggttaatg gatatgtggt tattttagag ttgttatatt aggagtttag 103680
   aggagtgaga gttgtttttt tttttttttt tggagttgta taaaaatttt agatggttgg 103740
   agaagttatt ggaggaatta tattatttta gatttttgtt ttattgttgg gatatgtggt 103800
   aatgaaataa gattattttt gttagggatg ttttatgggt aattgttttt ttatggagga 103860
   tattgtgagt tgtatttagt tgggaaatat taggagatgt ttgttattag gttggtagaa 103920
   tagggttttt tagtattttt ttttttatta gagttttaag attgtgtttt gggttttgta 103980
   gttatatatg gttgtttttt tttttttaat gtgtaggttt tttgggaagt tttttgttat 104040
   tatttttata gtggaattgg tataatttgt ttaggggtag taggtttttg ttattttata 104100
   tgtttttatt ttttttgtgg agggagatgt agttaagggg tatttatatt tatttataga 104160
   agtgagagtg tttgtgagtt tgttattttg tagattttag aattttgtat gtgattgtat 104220
   agatttatag gtttaattgg gtagatttaa aggatttatg tagtagggag gagtattgga 104280
   tagttttttt ttttgtttgt aaggagttaa tgtaattgaa ggatatagaa gaggttttta 104340
   gttgagtatt ataattgtgg attgtgtata gtgttagaaa gatgtggagt taggttatgt 104400
   tgggaggatt tttatgttgt tattggaagg taaagtagtg ttattttttt atttatagag 104460
   taggaggttt ttatttttgt tagttatttt ttttatttgt gaatttaagt tttttaaagt 104520
   tagattttaa ttttgtaagt ttttaaaagt ttttttgggt ttttttttta atttttattg 104580
   attttaattt gttttaaaag tttgaagatt gtttatgtgt tttggtttat ttttgggaat 104640
   ttattttaag gaaataatta gaagtataga aagatttatg tatgaggata tttgtattag 104700
   tattaaattg ataaaatttg gaaatagttt gggtttttgt agatttttta aatttatatg 104760
   ttaaaggttt gtattattat taaaattata tttttgaaga tgagaaatgt tgagaatatt 104820
   aagtaaaaat agtaggatat aagatgtata taaagatttt aattttattt tatatatata 104880
   tgtgtagttt gtatgtatat atatatttat atttgggtag aaaataatga tttttggatg 104940
   gaatgaatgg tggtgggtgt tgtttattat atttttttgt tttttgagat gtttatatta 105000
   tatggggatt atttagtatg tttagggaat aaaattattg atattgtttt tttttttttt 105060
   tttttttgag atagttttgt tttgttgttt agattggagt gtagtggtgt aattttggtt 105120
   tattgtaagt tttatttttt aggtttatgt tatttttttg ttttagtttt ttgagtagtt 105180
   gggattatag gtgtttggtt attatgtttg gttaattttt ttgtattttt agtagagata 105240
   tgattttatt gtgttagtta ggatggtttt aatttgttga ttttatgatt tgtttgtttt 105300
   ggttttttaa agtgttgtga ttataggtgt gagttattgt atttggttaa tattgttttt 105360
   aaaaaatata ttttggtatt ggttgggtat ggtggtttat atttataatt ttagtatttt 105420
   gggaatttga ggttagagga ttgtttgagt taggagtttg agattagttt gggtaaatgg 105480
   taagatttta tgttggaaaa ataaaattaa aaaaaaaaaa atatatatat atatagattt 105540
   tgatgttaat aagttggagg gtgtgggtaa ttgtatgttg gtttattttt atttttattt 105600
   ttggaaaaat attttatagt ttaggatggg tatagggttt tttttatttt taagtattta 105660
   tggttttagt tgttatataa aaagaggtta tagtgggtta ttttttagat gtaaattttt 105720
   ttttttaggt ttatggtggg gttgaaatag gagtagtttg gatttgggtt tatttagaga 105780
   ggaatgtgtt gtataaggga ttattgttaa ggtttttgtt ttgattatag gttgtttggt 105840
   aaattgtttt tagtttttga aattagatta agtatttttt ggtgtatata ttaggtttta 105900
   gtttggggag ggaatattaa gtgagttata gagttttgtt ttagtttggg ataaatgtta 105960
   tgtaaagaaa tggtagagtt taaggttaga ataattttat gtgtagttta gtttttgtag 106020
   gagggagtaa tgtggttttt gtttagggta gggtggaatt ttaggtttag gttttaggaa 106080
   tgttagtttt ggttaaattg ggtttttttt gagtagtagt aaagtaggtt ttgtttttga 106140
   agaaatattg gttatttagg agttagggtt agggtttagg gaagttggat agatggtgtt 106200
   aaggagtagg gaagtatttt tttgtatgtt attttgagtt tgtaggaaaa agttatggga 106260
   aaattttatt tggtagataa ttttttataa tttttaggat ttatagagtg agtttagtag 106320
   tatgtatgtt atgggtattt ttttgttttt atttttttgt gatattggat tttggtatag 106380
   gtttttgatt tgtgtagggt ggggtgggta gttaggtagg gttttggtgt tggtagagtg 106440
   gggtttagtt gtaaaggttt ttttattttt tatttattta ggatttgggg gatgaggttt 106500
   aaaggtttgg tgtgttttta aggagtttaa ggattgtttt agggttggag ataggatgta 106560
   tgggtttgta tagggattgt gttttttatt ttagggttga gagtaggaga attaaatagg 106620
   agagtgatat aattatagtt tattttgagt tttagtgttt ttagtgttat ttgttgtagg 106680
   aggtgatggg gtaataaggt gtttttgagg ttaggtgtgg tggtttatgt ttgtaatttt 106740
   aatattttgg gaggttgagg tgggtggatt atttgaggtt aggagtttga gattagtttg 106800
   attaaaatgg agaaatttta tttttattaa aaatataaaa atagttaggt gtggtggtgt 106860
   atgtttgtaa ttttagttat ttgggaggtt gaggtagaat agtttgaatt taggaggtgg 106920
   aggttgtggt gagttgagat tgtgttattg tattttattt tgggtaataa gagtgtaatt 106980
   ttattttaaa taaaaataaa taaataaata aaaaataaga ttttttgggt gtaggtatgg 107040
   tatgtggtag gggtgattgt tgttggtgta attattattt tttgggtgtg tgtttgtggg 107100
   ggttgtgtat gttgaaatgt gtggttattt tgttggtgat taaggttgtt ttggttttat 107160
   tgttattaat tatgtttatt gattttatat ggtttatttt tgtgttttta tttagagttt 107220
   tttttagtgt ttaagttttt ggaaggttgg aaggagtgag tagtgtagtt tttggttaag 107280
   aattgaagta taaaaatttt agttttttta tttaatttta ggtgggataa ggttgggggt 107340
   atggtttgta gtttttttat gttgtttggt atgggggttg ggtagttatt tgtggttata 107400
   atgagttggt gaggtatttt ttattggttg gattttttgt ttggtttatt tttatattta 107460
   ggtatttttt ggtgtttttt tttattgaaa tttttgtttt aggatttgtt tttggaggga 107520
   tataaattag ttttttagaa ttggtttggg ttgtttggtg gaggagggag gtggtttttg 107580
   gggtaggtgg agtaggtttt ggatagggag gaggatatag ttggggtgat ggggaggggt 107640
   gtgtttttag gaagtaaaat tgtttaaaag gaaaggttta aaaataggtg tagtgaagtt 107700
   gagtggagtt tgtaaaggga agttaggaaa tagtagtgtt agtggagggt ggtttttagg 107760
   ttttttatag agagttatat ttgtttaggg atagggataa tgtgaataga aggtaaagtt 107820
   tatattaagg tggaggaggt gttttttttt tagagagttg tagttagata gtttaaattt 107880
   gttaaaggtt tgggagggaa gaattagggt attattttat ttttagttta attttttttt 107940
   tagttttttt tttttttgtt tttttaaggt taggtttggt tgggttatag tgttagtagt 108000
   taagatttgt ttttagtgtt ggtttagttt gtaggaggtt agtttggtgg ttaaaggtta 108060
   ttgattgttt aggtttattt gtatatgtgg tttaatgatt gaggtttggg atttggggtt 108120
   attatttata gtatttttgg ataaggagga aaagaattat ttttattaat ttttggatgg 108180
   aggtgtaggg agtgttaata gtatttttga aaattttaaa tgtattaggg gggttagtat 108240
   ttagggaaga gttataggag gttattttga tggattttga tgattatttt tgtttaagtg 108300
   ttaggaggag gttttttttt ttttgttttt ggtaattttg ggtttaagaa gttttaggta 108360
   ttttggaggt tttttttgtt tattatagtt ttatttaaga ggtttggttt attttgtaat 108420
   gatgatgtga gaatgagtag gtttgtttag tgttagagtt tgtggttgga tttttggttg 108480
   aggttaagag aaataaataa gttttttttt tttttttgta tgttatagga agagtagttt 108540
   gttggttgta aggggatttt ttattttgtt tatggggtat agtagtttat agtatttttt 108600
   ttagaaatgg agtattggta ggtttgggtt tttgtaagat ggaatataaa ggggtgggta 108660
   gttgatttta atagttagtt tttgggattt taaatttttt tttatttttt aattttgtga 108720
   tgttaatttt tttttggata aagtttttta ataatttggt aagttagtat tagttgaatt 108780
   agttttagtt tgaattagta gtttttttta gtttagttat agttttattt ttttagaagg 108840
   gaaaagaagt tttatggatt tttttataag gaatttttag aatttgttat ggtgtttgta 108900
   ggggatattg gtattaattt tgggtttatt aaatgtagtg ggtttaatag tgttttttaa 108960
   aatgatatgt tttaagtttt aatttttgga atttgtgaat atgattatat ttggaaaatg 109020
   gatttttgta gatgtaatta aggtaagggt tttaaggtga gattattttg aatttagtgg 109080
   gttttaaatt taatggtgag tatttttata aaagatagaa aaggagaaag atatatagat 109140
   agggggagaa agttatgtgg agataggggt tagagattag agatgatggt gatgtgtttg 109200
   taggttaagg aatgttagga atttttagga gttgttggaa gttatgtttg taagttttta 109260
   ggttgtggta ttttgttata gtaattttag gatatagatt ttaggaatag aatatagaaa 109320
   agaaatggga ggaggtttag agtttgttgt ggttatttta gttattttat ttgatttttt 109380
   tagtgttttg ttaattttga agtttttttt agttattgag ttattggttt tgaaaaggag 109440
   atatgggtgt tttggagttg gtttagtttg gtaggaggta gttttgtttt attgtttttt 109500
   tattagttat tagtttatat aggtttttta tagtgggtaa gtgtgttgtt gttttagttt 109560
   ttgttggttt tttttttgtt tggttttatt agttgtagag ttttttgttg ttgttttttt 109620
   tggtttaaga ggtttatata tttagttttt ttgggataaa tttagtttat atttgttgtt 109680
   ttattgtagt tatttaaggg ttttttttat taatgtgttt tttaagggat agtaagttat 109740
   atggttgttt tatttggggt tagaattgtg ttttattaga gtaaggtttt ggagtaatta 109800
   tatttagggt tagggagtta tttgtttaag aatagaaatg tgtggttggg tgtggtggtt 109860
   tatgtttgta attttagtat tttgggaggt agaggtgggt agattatgag gttaggagat 109920
   tgagattatt ttggttaata tagtgaaatt ttgtttttat taaaaatata aaaaattagt 109980
   tgggtgtggt ggtgggtgtt tgtagtttta gttatttggg aggttgaggt aggagaatgg 110040
   tgtgaatttg ggaggtggag tttgtagtga gttgagattg tgttattgta ttttagtttg 110100
   ggtgatagag tgagattttg ttttaaaaaa aaaaaaaaaa agtgaaatgt gtagtttttt 110160
   ttttttttat ttttgaaata tgaaggggag gtgattgtag gttttttggg aaagattttt 110220
   ttattttttt tttttttttt tttttttaag atggagtttt gttttgttgt ttaggttgtg 110280
   gtgtagtggt gttatttttg tttattgtaa gttttgtttt ttgggtttat gttatttttt 110340
   tgtttttgtt ttttgagtag ttgggattat gggtgtttgt ttgggatagt tatgtagtag 110400
   tttttgtttt aagttatttt tttttatgta gagtagaatt aggattatag taaaggtttt 110460
   agaggaggtt attgtttata gggttttggg tttttttttt gaaaggtagt ttagttttta 110520
   agagggtaat agtgattgat ttgggatgaa ggtttttttg tgtttgtttt tgttatttat 110580
   tgtgtgtttt tggttttggt agttataggg ttattttagg tggattttaa agtggtgtta 110640
   agttgttgga tttttttttt tttttattaa attagttatg ggaatgggag tatttttggt 110700
   atatttttag atgtgttgta ttggtttagt gtttttttta tttttgggtt ttaaattagt 110760
   tgttggttgt tttattagga gtagattgag gggaaatggg tggaagttgt aggggaagag 110820
   atgtgaggta gataaagagg aagaattttt tggttggggt ggggttgtta gggtttgggt 110880
   gggagggttg tttatttttt gtttttggtg gaaggtagta gaagggtggg atggatttgt 110940
   gtgggagggt ttaggtgtgg tttgtttggg ggtaggagag ggattagatg attttttagg 111000
   tttttttggg ttaaagattt tttgataaat ggttgttgtt gaaggtagga gtgaggtttt 111060
   gggaggtgat tgattttgaa aaattatagg ttgtagtttt gagggtttta aaggttaagt 111120
   atggagtttt ttaaaatgag gtttttttta gttttaattt atttatgttt tttaggtggt 111180
   tttagatatt ttgttagggt ttttttggta gttttttgtt tttgattttg gagatagaga 111240
   aatgtgatgt tggtttttgg tttgtttata gtttttttgt ttttattata gggttgttag 111300
   ttattttttt agtttattgt gtgttttttt ttttttttta tttttgattt tttaataata 111360
   gtagattttt ttttaaatat ttttttgttt agattttggg ttttatgtat gttgttttta 111420
   gaaatgtatt ttagtttttt gtgaggagtt agtaaggttg tttggtgttt ttttttatgg 111480
   atgggaagtt ttgttgggga ttaggatttg gatttggaga ttttgaggag tgtgttggtt 111540
   agtgtttgtt ttgaggtttg ggatagagtt gttaggtata ggtagtattg gtgtgagggt 111600
   ttagtttttt ggataagttt ttttttgttt gtttttagat ttgttattgt atatataggt 111660
   ggtgattatt tttgatttgt ttgggtagag aagttatttt tttttttttt tttgttttat 111720
   tgtttattag gggaaatgtt ataagtattt aaaaaatgta aaaaaaaaaa aaaaaaaaaa 111780
   aaagtgttta atattagggg ttatttggtt taggtggagt ttttattaat tatggggttt 111840
   tttgtgttgt ggttttaagg tgggtagttg atggtttttg tttgagttaa tatgagttgt 111900
   agtttaatta taggggatgg tgattttagg gagtagagtt taggttttgg ttatagttgt 111960
   atttaggagt tagttgtttt ttagatttgt agagttaggg gagaggtttg gaggattggt 112020
   attgaatgat atgatttaaa gtgtgttaag ttgggttgga ggtgatggta gagttatagt 112080
   ggttatagag tttttatgtg aatttatatg gatttgtttg gtttaggttt tgttgaattt 112140
   tagggtgagg aggatttggt ttttattttt aaagatatga agaggttgat aaagaagagt 112200
   tataggtaaa agggggtggt gggtgtgttg tgtaggaggg gttgtaattg gatgtagaag 112260
   attttagaag gggtaattag agagaggtag tatttttaga agatgggttg tttggttgtt 112320
   ttaaatagtt tagggtgtga ttgaggggat ttttggattt agttggggtt ttttttgttt 112380
   tgagataatg tattgggaaa ttttattaag tttatttaag aatttgaaaa gttttttgga 112440
   aatttagtgg gtagggtggg tttttgggag gatttttggt atattgtgag agttagatat 112500
   agtgaggggg ttttatattt ttgtttagaa gttttatggt agaggggtag aagtttaggg 112560
   aggagtaggt gttgagatta gaggggttta taagagaggg gtttggggta gggggtagtt 112620
   gttgagaagt ttaggaaatg gtgtttgttt ttggggaagt gaagttttgg gaatagggtt 112680
   ggtgaagggg ggtgggaagg gggaggtggg ggtggagggt taggttagat taggtatttt 112740
   ttttagtagt agatttatga gtttttggaa tttgtttata gaagtgataa gtgttttgtt 112800
   tttagttttg tttatatatt tatttattaa gtgtggtagg gatagttgtt ttttattatt 112860
   tattttggga tttggattta agtggaggtt tttggagggg tagatttagg ggaaagggtt 112920
   tttttagaag tagttgttat ttagaggatt tgagatgaga attttattat gtttttatta 112980
   gatagaggag atttgtggtg tttttgagtt tggtttttta ttttttattt agtagtgttt 113040
   tttttgttta ttttaagttt agaatatttt gtttttttta agattagttg ttgtagtgga 113100
   ttgaatagtg atttttaaaa gatatgttag agttttagtt tttggatttg tgaatgtgat 113160
   aggagttgga atagggtttt tgtagatgta ataaaggatt ttaagaggag agtattttgg 113220
   atttagggtg ggttttaaat gtaatgataa gtatttttat gagatataga gatatagaga 113280
   aaaggttatg tgaagatgga gtagggagtg gtgtgatgta tttatatgtt taggagtatt 113340
   tgggagttat tagaagttgg gagagaggtt tgggttggag ttttttgtag aggttttaaa 113400
   ggattagttt ggttgatatt ttgattttgg atttttggtt tttagaatta tttttagttt 113460
   ttaaagtttg tggggttttg ttgtagtagt tttagtaaat tgttatgggg gttttttttt 113520
   tttgttaagt ttggtattta aagtttttta gttatttggg gttaattata ttgattttgg 113580
   gatttttagg ttatttgtgt gttttatttt tttgtttaat ttttagtgtt tgtgtggtta 113640
   tagttttttt tttgtgatat gagttttttg ggtttttgag tgtttatttg ttttgtattg 113700
   aaggttgaag ttaaattttg gagagaattg attttttttg tgggtagggt aggtattgtg 113760
   tttttagtat agttagaggg tattggttga aatgtgtttt tgtaaagtgt tttttttatg 113820
   tttgtatttt ttttttaagt ggggaagtta gagaggtttt tttgattttt tttttagttt 113880
   tttggggtag gaagtttgtg gttatttgtt tgtaaggatt aggtggatag ataattagtt 113940
   ttttttaggt tatattttat taatagaggg ttttttaatt ttgttggtgg tttatgagag 114000
   aaggaaaaat tgaaggagtt gtttatagta aattttaaat ttttgtgtta gagaggttta 114060
   tggttgagta aggtattttt atttttattg ttttgagttg ttaatttagt ttagtttagt 114120
   attgtgattt ttgatttgtt tgtgggttat gggagtttgg tttgttaatt agtatttttt 114180
   agatatttat tttgtgaagt ttttagtggt tgagtgtttt tttgtttttt ttggatagtg 114240
   tgggttatag gggatattgg gtaggaagat agttaagttt ttattgttta gaaattgttt 114300
   gggtttattg tagtaatagg gatttattta ttagtatgag gtttttttgt aggaggggtg 114360
   ggggtaggag ggagtttgtg gggtataggg ttgtaggagg agttattttt ttttttatta 114420
   aattgtaggt tttagttaag ttatttttag tatggttgtt tgtgtgggaa gtttatagtt 114480
   gagggattgt ttgtatgtgg tatgtaggag ttataagtgt tatttatatt ttattttggg 114540
   ttaggtagat gatttttttg gttttagttt tttgatgagg aataggaagg gttggattga 114600
   aagatttttt ttagtttgga tatggaatga ttttatgagg gttatggtga ggtttgtttt 114660
   agttttgtat attagtttgg gttagtagtt tttaaaggta tagttgtagg ggttttggag 114720
   tagggataag gaggattagt ttattttatt tttttgggta gaggttaagg attttgtttt 114780
   tggtttatat tttttgtggt taaaaatgta gaagtaagta gtgttggtag aggtatttag 114840
   atttttgggt aaagtagggg ttttatttga tattttaggt ttggagtatg agaaaattag 114900
   tttaggtttt aggttttttg attgttagtg attagggaat gtgatatttt ttggtttttg 114960
   tttttttatt ttttaatttt agggggagat taggttgggt gatgtgtttt tttttttgat 115020
   agtaaatttt tatttatttt tatttttatt tttgttttaa gagggggttt tttatggttg 115080
   ggtgtagtgg tttatgtttg taattttgga attttgggag gttgagatgg atggattatt 115140
   tgaggatagg agtttgagat tagtttggtt aatatggtga aattttgttt ttattaaaaa 115200
   atatgtgagt tggtaatttt aagtttttag tagtttttga atggaggttt tttttttgta 115260
   ggatttttaa attttataaa tatgagtgtt tgtaatttag ttatttagga ggttgaggta 115320
   tgagaattat ttgaatttgg gaggtagagg ttgtagtgaa ttaagaatgt gttattgtta 115380
   ttttagtttg ggatatagtg aaattttgtt aaaaaaaaaa aaaaaaaaag gtgggggttt 115440
   ttttatgttg tttaggttgg ttttaaattt ttgggtttaa ggaatttttt tgttttagtt 115500
   ttttagagtg ttgggattat gggtatgagt ttatgaattt taaaaaataa tatttattgt 115560
   tttgtttttg attgtaaaag gaatttaggt ataaagttga aaatttgtaa aatgtataaa 115620
   aataaaggga aaaaatttat aaatttataa tttaggtaat ttttattaat attttgataa 115680
   atgttttttt attttttatt ggggagtgtg tgtgtgtgtg tgtgtgtgtg tgtgtgtgtg 115740
   tgtgtgtgtg tttaaatagg tttatatagt gatgatgttg ggtgtttttt tttatgtggt 115800
   gttatgtggt tgaggttttt atatattttg ggtttgtttt tttgattttt ttgtagtttt 115860
   gatgttttga ttttagatat gttatttttg gtttggattg tttaggagaa ttatttttgg 115920
   attttgttaa aaaatggatt gttggttttt aatttttggt ggttttggtt taggagggtt 115980
   gggtaagttt gggtattttg gagtgttttt ggggtgttgt aggtgtgaat ttttgttttt 116040
   ttgaagattt ataagtttta ttttttttat tttttttttt ttttttgaga attttataag 116100
   aggaaagttt ttatttggag gttgttaggg atttggagtt gttagtttat agttttattt 116160
   tgtttttatt tttttgggag ttatttaagg ataatgttta gttttagttg ttaagtttta 116220
   gttttagggt tagttatata ttgaaggata tgtagaagtt gttgtgtgtt gggtattgtt 116280
   gaggataaag gtttgaatga taagtgggaa taaagttata aggttttaaa atttgtttga 116340
   ggttttatag ttagggtttt ggaaatttat gaaaaaagtt agtttaatat tttgttatgg 116400
   aatataggta ggagagggag tagtttttag ttagttttta gttagttttt tgttagtttt 116460
   tttgtgaatt attttatttt gggttatagt tagggtagtt gttgtttttt tttttgtttg 116520
   atagtttttg agggtaggag ttagtttgtt tattattgtt ttttttttat tagggtttgg 116580
   tattttgttt ggtatgtagt agaagtttaa gaagggttta ttgtgtttat tttgtaatag 116640
   ttaatgtgta tgagtgttta ttatatttga agatagtgat ttatttttgt ttttattgta 116700
   tagatgggaa attgaggtat tgagaggttg atgatttttt tatattgttg tgtgggtaag 116760
   ggtaaggtta gatgtgagtt ggtatgttgg gttattgtgt tttttgttta tattttgttt 116820
   tgggtttggg aaggttattt ttggagattt ttatgatgtt gatattttgt ttagtttttt 116880
   tgtgtatttt tttttatgtt ttttagttat ttaaagtatt agttaggtag tattgttatt 116940
   ttggttttgt aaatttagtt attgagtgga agagtagtta ggttgaagtg gggtagaggt 117000
   agtttttgtt agattttagg gttgggattt ttagttattt ttatttttta aggattagta 117060
   gatagagatt tttattgtgt ttagttaggg gttagggaat aggtttttat agtgggggag 117120
   tgatggatgg atgtaggtgt ggattgggag gatttgggtt ggttttttag tgttggagtt 117180
   ttagtagttt aatttatttt gttgggtttt tgtggggatt aaaagatatt ttgtatataa 117240
   agtattagtt tagggtttgg tatttaggag gtgtttaatt aaaggaaatg gttatgaaaa 117300
   ataataagga gaagtttagt tataggaggt gtttgtgggt aaggagagaa tagttggaag 117360
   ggtttggatt tgggttagag ataaagaatg taagagggag ggagggagag aaggagggag 117420
   ggagagaaag agggagggaa agaaagaggg agggagagag ggagggtagg gtaggtaata 117480
   gttggtttta gggtagttgt gtgtaggagt ttgttttatt atttaaatat taggagagat 117540
   gtaaatattt gagtgaggtt tttttagttt ggttttgttg tggttatagt tagggttgga 117600
   gaaagagttt tttttttatt agggtttttg ttttgtgttt tttattttag ttgttttaag 117660
   attttatgtt tttgggttag gagtaggggt ttgagagtag taattaggta gggtagtggg 117720
   gttagggagg gattttgttt ttgtttttat tttttttttt tgtttttgtt tttatttttt 117780
   tttttttgta tagtattggg tttgtaaagg tgtgaatgaa ggattttttt ttttatttga 117840
   ttttaagggg tttaggtggt taagggtttt ttttgtagtt tagaaaaaaa agtaaagtag 117900
   ttttttttga ggttttttgg tggaggttta gggtttaaag tttgttgtta taaatttgta 117960
   gttttttttg tttttttaat agtgtttttt ttttatttgg gttattttat tattttggtt 118020
   ttttttagtt ttttaggttt tttggggagg aggatagatg tagagtttgg attttgtgtg 118080
   tagtagggtt tttttaatgt tgtggtgtat tttgtttggt tgtgggagtt tgttttatgt 118140
   tatgatttgt gttggtatta atagtttttt tttttatttt ttatttgagt gttgagggta 118200
   gttgtgttag gtatagatat atagtttttt atatatatta tgagtttgta gaaatggtaa 118260
   gagaaggttg agtgtagtgg tttatatttg taattttagt attttgggaa gttaaggtgg 118320
   gtggattatt tgaggtaagg agtttgagat tagtttggtt aataggatga aattttgttt 118380
   ttattaaaaa tataaaaaat tagttgggtg tggtggtgtg tgtttggagt tttagttatt 118440
   taggaggttg aggtaggaga attgtttgaa tttaggaggt ggaggatgta gtgagttgag 118500
   attatgttat tgtattgtag tttgggtaat aagagtgaaa ttttgtttta aaaaaaaaga 118560
   aagaaagaaa aagaaaaaaa gaaaaggaaa atggtaagag aagttggtta gtattatttg 118620
   tttggtaaat ttttattttg gtttttggtt aggaagtttt tttttaggaa gtttttttgt 118680
   tttatttttt tttttgagtt tatgtttaga tttttagtgt ttagaataga gttggttttt 118740
   ggggtattta tggatggttg ttggataaat aaataagtta attgtttttt ttatttttag 118800
   tgatgttttt ggtttggtag ggtatttagt agtaatgttt tttgagtgga ttttaaggag 118860
   tattttttat tgtgtagttt gtattaggga ttttagtttt ttattttaaa ttaaaggaaa 118920
   aaatttaatt ttagtaaata tatagagtat tttatttttt gatttttaat ggggggtaag 118980
   aggtagagga tagaggagaa gattattttt ttaggtttgg tttggagtta aaaagatttg 119040
   tatgttttta ttgtagataa tattggtttt tgggggatat agggtagggg tttggtttgt 119100
   tttttgtagg gaaattgttt atttatgtgg aaagtggggt taagggtttt tgtttggaat 119160
   tagttttttg tatatggggt ggggggtggg gatagaagga gattttgttt ggattgtatg 119220
   gaaatttatt agattgttgg ttgttgattt ggtaggagta gagttatttt tattttttag 119280
   ggttttgttt gggggagggg agaggttagg tggttttgtt taggttatat tttttatggt 119340
   taggaatgtt ttttttttat tagaaagaaa gatgagatag gtttattttg aattttttgg 119400
   atataggaag gggtttattt tgtggtttag gtggtttttt tttttatatt taaggggttt 119460
   tataaggagg tgttaggatt tttaggtttt gttttaggga attgttgttt gtaaaggtta 119520
   taggtagaga tagttaggaa tgttttttat gttattggtt atgggttgga ggttgtgaaa 119580
   ttaggtatag aattagaaag atttgttata ggttgtaaag ggttttagag agttttagtt 119640
   ttttagaggg gtagtattgt agaaggggtg agaaggaaaa attagttttt aggaattgtt 119700
   tatttagagt ttatataata tttaggtggt atgagataat ggtttatttt ttattttatt 119760
   tttttgttgt tttttaagat ttgagttttt gggggtgttt ttagtagata gggggatgtg 119820
   gggtaattgt ttttttttaa ttgtatattt ggaggtaatt tatatttaga tgattttttt 119880
   gaggttgtga ttagagtttg gggtagggga ggggaagtgg gtggatggga aggtgaggga 119940
   gattttttta ggaaagttgt tttaggtgtt tgtaaggttt tattgtttat atttaaggta 120000
   taggtttttg gttttgagga tataggaaat ttgatgttat gtggggagga gggtattgta 120060
   atgtatttaa ggttaggttt tttgattttt tggatattgg ataaatgatg gtttttttag 120120
   ttggtttaga gatgttagga gtgatagaat gttaaaggtt gtagggggat atttagaaat 120180
   ttttgagaaa gaagatggat aaggaaaaat aaaaaaatat gagaagttta gtagagtttg 120240
   agttttgtat ttttggtttt ttttatggtt atgggtaatt ttttggagat ttagttatgg 120300
   gagtagtgga ggtaggtatg ggtggggggg ggggtgggtt ttattgttta tggttttggt 120360
   tgatattgag gttatttttt tgttttttta agtttttggg tattggtagg atttttaaaa 120420
   atgataataa atttgaagtt tttagaattg tttgttggtt tagtttaaat ggagttttgg 120480
   ggttttgtag attaaattaa atagtttgag tttagaaatg ttttaggtgt ttgggggtgt 120540
   ttaggggtta attggtgggt ttgtttggtt ggagagatga taaatttttt tatttttggg 120600
   attgtgtttt agagagtttt ggattttttg gttttataag taatttagag tttttaaatt 120660
   tgggaaaaat taagagggtt ttggaaagaa aggtagtgtt tgattatttt tttttagtgg 120720
   ttttgttaag ggagagtata ggaatttggt ttttggttgg gtttttggga tgaattaaaa 120780
   taaaaagaaa aagttggggg agggggtggt ggaattgttt ggggttttag tttaggttat 120840
   atattaagga gattttagag gagtaggtta gttttatttt tggtatgatg atggatgggt 120900
   atttggttgg ttggtaaagg gaagagatgg taatagtgag gttttgaatt tagtggttaa 120960
   gatttatttt tgagtgtgat tttttttgat gtttgggtat gtaagaaatg ggaggtggag 121020
   aaaggaggag gagattttag ttttgttttt ttggtattag gttattaggg aatagtattt 121080
   tttttttaat gggtaggtag aggttatagg ttgttaggtt gagaagtttt tggtttttat 121140
   agataaatgt atagtttgta tatttgtatt attttgggtg atgtttatta tgtatggtta 121200
   ttatgtatgt tttttattat taagatagtt gatgagatta ggtggttatt gtgtattggg 121260
   tattgtggtg attgtttgat attgattttt gggggtttag gtttttggtg ggtttgtttt 121320
   tttaggtatt tagttttttt tttatttttt gatattttgg gtttagtgta ggggtatggg 121380
   ggtgtttagt ggatgggttt atgggttggg taagtagtat gtagttttgg tttattagtt 121440
   tttggtttta ttattagata agatgagatt gttttagttg ttagtttagt tttgtattat 121500
   atttgattgg gtttttgtag aggtttgttg gatgagaatg tttagggtag taggtattat 121560
   tttttgggat gtatattttg ttataattgt tttttaggga aaggttagtt tgtagagttg 121620
   aatgtaggtt gttagtgttt ttgttgtttt tatggtttgg ttatttagta agtgtttatt 121680
   ttattgttag tgtttggttt tgtagatggg tatatggttt atttatttgt gatattttag 121740
   ggtgggggtg ggggtagatg gtggtaggga agattaggat aattttttag taagaggaat 121800
   ttgtagttaa tatgatgtta gtttttagaa tgttagttag gtaggaaaag gggggtttta 121860
   tggttgtatt gggagtttga gtatttgttg ggtttagagg gatttttagg tgggtgttat 121920
   tggtattagt gtttgttgtg ttttttataa ttagtggtga ttatgttgtt tggttttatt 121980
   tatatagtgg tattatatag gtttttgttt gagatttttt atttttagat ttgtatattt 122040
   aagtatttaa atttatttgt tttttgtaaa tgtttttttt ttttgtttag atgttatatt 122100
   agttgtgata tttattatat ttgtaggagg taggatttgt gtttagtgtt atgggttgta 122160
   ttttagagtt ttttagtaat ttttaaagag agttgttttt aattttattt tgtaggtagg 122220
   gaaataggtg aagaaattga agaaattgaa gttgtttagt tttttaatgg tagagtgagg 122280
   gtggtttggg tatttttata ttgttatatt ttttattttt ttaaagtatt gtttttttgg 122340
   atgtaaattt tgtttattag ttttatttaa aatttttttg gatagttata gtttatagaa 122400
   agtattttta gtttgtagtt atttagttat tgtaggggtg ttttggtgtt tatttttatt 122460
   tgtttttttt tggatattta gtttagagaa tttatagttt tgttgtaagg ttgattatag 122520
   tgtatttgtt gtttgggtgt tgtgtttgtt tttttttaaa tgaattaatt tgtagttggg 122580
   atagtgggtt tgtttggttt tgggttttga atttaaagag tattaagtta gtgttgttag 122640
   tggttgtggg atttgtgtaa gaggtttgtt tttatttttt atggtttttg ttttttatta 122700
   taaaagttta aattgtagag aggtagtatt tataggatgt tttgagtttt ggttgaaagg 122760
   tagggtttat tatgtaagag gtttattggt attaattagg gataggtttg tatttattat 122820
   taagtaatgg ggtttttttt tttagagagg aaagaggtat ttgaattttg ttagtgtgtt 122880
   agtttttatg ttttggaggt gttgggattt ttagataaga aggtatattt atatggtttt 122940
   ttttatttag aagtttgttt tatagtttgt tttgttttta gaatatagat ttgggggttt 123000
   tttttgggtg atagtggggg tttgggagaa gtttagatgt tatatagttt tgggatttga 123060
   gtatgtgggt tttgggttag attgttttaa taaatattaa ggatttttgg attttttttt 123120
   ggtgtgtaaa tttggttata gtaaatattg ttgagtaaag taggttaata gttggtattt 123180
   ggaaaggttt ttgttatata aagatttgag ttgtaattgt gtttgtgata atagatttgg 123240
   tttgaattta tgaaagtata tgttgtttgt tagttttgtg tttttattaa ttagaaggga 123300
   gaaagaggaa agggttattt tttttgttat tttattatgg gtagtggtgg gtgttgtagt 123360
   tgtttggagg tttgtttgta tagtggaaaa gtagaaaatg agattttaaa aattttatta 123420
   gttaaatata aaatattttt tttgttgtta tgtggtggtt tttatagagt tttaaaattt 123480
   tgaagtttgg agatttattt tgaaggggga gtaaaggtgg gatgtgggaa gtttgagttt 123540
   atggaagata ttgatatttt ttttattttt gggaattttg agtttgaagg atttggtttg 123600
   aatagtggaa ggattgaatt tttgtgtaat ttattggtga tggaatataa agttatggtg 123660
   tttttggttt ttaagatggg agggatgggg aaggttttgg gtgaagggga taggtgggga 123720
   aggaaaaggt gaagagaaag gtgtttttta gggagggggt agatgtgttt aggatatggg 123780
   atattggtag aattttaatt ttgagtatat atataggttt atggatggtt ttttgtttat 123840
   ttgtttttta tgggtgtgtt gtgttggttt gggtatttgt tttttttttt atgttaggtt 123900
   atttgttgtt tattttgtat attgttatta gttttaagtt ttttagaata ttttgattag 123960
   ggttttgtgg tttttgttat aggattttag gtttagatgt ttgtttgatt ggaaaattgt 124020
   ttttagagat gttgtggttt agagtttgtt ttgttttggg gtggtttttg gagtttttgt 124080
   tttttagtaa tttatatggt gataggtatt tatatttgtt ggttttagga attttggggt 124140
   tattatttta gtgttgtaag gagttggtag tttttttttt atttttttgg tttattttgt 124200
   aggaattaag atattaggtg agttgttttg ggaaagagat ttgtgttttt atggtgttgt 124260
   ttttgtgttt atttttagtt attttagatg ttttttgtgg attagttgta tttttggtgg 124320
   gtatgtttgg ttttttgtag tttggtttat atgattttta taggtaggtt tttttttatg 124380
   ttttagtttt ttttttagtt agtttatttt agtttttttt attgttgttg gttgttgttg 124440
   gttagaaggt tttttttttt tttgtaattt aatttttatt tttttttaag gtttgttttt 124500
   tttatattga tttttttttt gttagttagt ggtgattttt ttttattttg atttggaagt 124560
   tgtttgttta tattattttt ggttgggttg aattatattt ttttttttat tttttttaat 124620
   tatttttttt ttatgtttgt ttgtttattt aaggttgttt aatttttata tatatatttt 124680
   tatatttttt aattatattt taggtttttt gggtattagg gagggttatt tttttatatt 124740
   ttaggttagt ttttatgtat atgtgtattt atgtatatat atattttttt atgtttattg 124800
   atttgggtta tttgatggtt ttaagattag aatttttagt aaaattattt aggatatata 124860
   agggggagat tgataaatat agggaattag tagattgagg ttttattttt tgagattgag 124920
   aaattttttt ttagagttaa aggattgtta attgttgtat taggatggtt ttaatgagtt 124980
   tattgttttg gattaaatta tagggttata aaattggttt gggagatagt tgtagaagta 125040
   gttttagatg gaggtatatg ggggtaaggg tggagttata tttagagatt taaggaaggg 125100
   gtattaataa tttttatagt gtaaatggtg atagttgtat aatgaatata attttaaatt 125160
   ttggtttttt tttttgttat agggtttttg ttgagatatg agagtttttt tttagaaatt 125220
   ttattggggg tttgatattt tttgttagga attgagagta gagattttga ttttgtaggt 125280
   aggaggagag ttttgtataa tatagggttt ttttttataa gggtttattg gaaagtggtt 125340
   agattggtta agttgtgtta tttggatatt tgggataagg aaggtttttg gaggagtggt 125400
   tgtagggagg agttgtaaag aggtaggggt agggaggtta tatttatttg gggattgtag 125460
   gaggtttaat ggaatatagt ggatttaggg agattttagt atgagtatgt gtttaaagat 125520
   gtttagatga ggaggtgttg tgggggaggg gggtgggaat agtaagtttt tttagttttg 125580
   tagatttaga tttttttttt ttttgggaag tagggatgta aagggataga gtttgaaaaa 125640
   gggtttttat tattaagttg ataaagtaat tatgatgttt ttagtttata ttttgtatag 125700
   tgttagtggt tattggatta taattttttt ttgttttttt gttgttttat atttttttta 125760
   ggtttagttt tgaagaatag ttattggtat tttgtttggt gtttagttaa agttttggtt 125820
   ttttaggagg ttttgttttt tttatttaga tagggttgat tttaggtggg ttgtgtgggt 125880
   agttgggttt aggatattta gatgttagtt ttggttttgg ttatggagtg gggaggattt 125940
   taggttggtt attttttagt ttggtttgat atttggatag tttgtttatt ttaatatagt 126000
   tttttttttt tttggtttat tagagggttt tgtttaagtt agtttaggag tttttttgag 126060
   gggtgggggt tttaggtttt ggtagaggta ggtttgtttt ttagaagttt tgttttagag 126120
   tgttggagat tattgtgtga gttttttttt tgtttgttgt atttattttt gtggatgtag 126180
   gatgtagagt tttagtattg gtagttaaga ggatgtagga aagtgtatgt atttataggt 126240
   ttagagatgt ggatgttaga tttaggtttg gatagagtag ttttgtaagt tttaatatat 126300
   tttgtaaatt ttgaagatag gagagagtta aaatattttt tttgtttgta tgtaggtttt 126360
   ttttgttttt tatggtttga ttttgggagg tggatgagga tatttttagg tttggatggg 126420
   ggttatggga tattttattt tagattttgt tatgatttgg gttgtgtttg tttggtgttt 126480
   ttggtttatg ggttgtataa tttggtggtt ttgaaattgg tgtgggggag gggagggttg 126540
   tttatttgag taggatgtgg ttgtttattt agttggaggt gaggaatgat ttttttattt 126600
   tgttgttggg tttaagtggg gtttgagagt tgttggggag agttaaaggg aggggattga 126660
   tggatttttt agagtgaaat tgtgtgtttt ggagagtttt gaggtagttt tgtgagtttt 126720
   tgaggaggtt gttgttgttt ggtaaatata aataataata aaaggaagga aatttaggtg 126780
   gtagtgtgta gaatgagtat agggttttgt gagggtgtgt agggtttatg tgggtgtttt 126840
   taatagggtt ttttatgtag gtggtgtggg tatggggttg ggggtgtgtg gtgtttttag 126900
   tgggagggtg tttggattag gggttttgtt tttttttagt gtatttttgt ttttttttag 126960
   tgtatttttg ttttgtgtta ggtttatttg tagggttttt tttagtatgt ttgtggttgt 127020
   agtagttagt ttagtattta tttttgaagt ttgaaatgat tttatttagt tgtgtgttga 127080
   ttgtggggtt tgatatgatg gttggtgggt agtgggttgt gtggagggta gtggtgagga 127140
   agtgtttttg gtggggtttg ggttttgtgt gttggttggg gtgttgtgtt tgtgtttttg 127200
   tagtgtagag ttagttgttg gaggagtttt taggtttttt ggtttagttg aatgaatggg 127260
   ggaggaaggt gggtgttggt ttttttttgt gtgttgtgtt tttgttttgt ttttgttttt 127320
   tgggtggatt aggtttttgt atttgtattg gtttttttgt tttgtattgg ttgttttgtt 127380
   tgtttgttaa ggttaggttt ggggggttgg ggtttgagtg gttgttgggg gatagttttg 127440
   ggtatatgat tggagtgttt tttttttttt tgtttggttt gtgtttaggg gatggtgaga 127500
   gatgtggttt tagttttttt ttttgggtgt tttgttgtgg ttagatgtgg gtggatgtgg 127560
   aggttgggtt atggtgagga gggtgggtgt ttagagggga agagattttt tttttttttt 127620
   gggtgtaggg tttttttgta ggaggttgta tttgggtagt taaataaagt tttttgttat 127680
   tgttgttgtg tgttttgtgt ttgtaagggt taaatggtag tgtatgtggt tggtaggtag 127740
   gattttggtt tgtgggttta agagttgagg ggtgtagggt gaatatgtga gttttatggt 127800
   attagtgaga ggttattaat ttttatttat tgagggtatt tttgtagtta atatttttat 127860
   tatgattatt gttttggggg ttattgttgt atttttttgg gtgagttttg gagaaggtat 127920
   tgtttgggag aaggggtata gggtggggat ggaatatggt tgttagagaa gttagggatg 127980
   ggtgatgttt tttttttttt ataataaggg ttttttgaga gtaggaagta gattttgtta 128040
   ggagtagatg tttttgaagg aaggtgtgaa ggaaggagtt aggtagtgag tgagtagaaa 128100
   gggagttgtt ttaagggttt tttttttttt tttgggtttt aatttgaggt ttagtttttt 128160
   ttttaggtta gtggttttta tttttaaatt atattattta taaggtaggt agggttgagg 128220
   ggttggggta gatttgggga tttgggttag atatagaatt tgagaagtaa taggttttta 128280
   tttttaagaa gggtttttta ggtttgttat ttataagaga gagaggtgtt ttttttattt 128340
   gttttggaaa gttagaaatt tatttttgta tgtttttttt gttattttgt tttttagatt 128400
   tttggttttt gtttagagtt aggggttatg tgagaattta tagtatggaa taatattttt 128460
   atttttgaag tagtaggttg taatgttata attgtatttt ggttttttga atgtgtggtt 128520
   ggggagtgtt tgatagttta ttttgggttt gaggttgttt gttgtttatt tgtttttttt 128580
   tatttttttt gaaatatttt aaatgtgtag gagtgttgag attttttagt tattttggag 128640
   aggtgggggg tagggggttt tttgtggaaa atgttttagg tttgggtggt tttttaagta 128700
   gatgaaatta agtttttttt ttttaagaaa agtaggtatt ttttttttta tgttttggat 128760
   attttgaatt atttttaatt aagtgagttt gtggatatgg gaattgtaaa attatagata 128820
   ttttagatgt gtgtatattt ttgttggttt gggagtttgg gttttagaaa tgtagttatt 128880
   gttgttatta atagtatttt gtatttttta tgttaggtta gggtatgttt tgtttgaatt 128940
   ttagtatttg ggtttattgg atagtttagt ttttattggg ttatttgttt atgttgtatt 129000
   tggggatttg ggggtttagt atttaagaaa tttagtttaa taatatagtt tttttgggaa 129060
   gatgttgggt ttggtataat agttatttta agttatagta ttatttttga gtgttttttt 129120
   aaaaagtatt tggtgagtgt ggatatattt atgtaattgt ttttttttag ggttggtatg 129180
   gagttttatt agattagtat agggttagtt aattgtatat tttttatttt tatgtatagt 129240
   gtagtaaata tatttagttt tttggggagt gttgttaggg tgtatgtttt attaaaatgg 129300
   gaatttaagg gataatggta ggagagtagt tagaaagtgt gtatgttttt gtgattgtgt 129360
   ttagagttta ttggttttag ttttagttat tgttttttta atgtaattat ttagagttat 129420
   atgattaggt ttagaaattt ttaggtaagg aggttttttg agttttttta ggaaatttaa 129480
   attagggttt ggttatattt ttagttagga agtttatttt tgtatttaat taattgtttt 129540
   aattgtatat tatttatttt tttttttttt ttgtttttag aggagatggg aaatagttgg 129600
   gtattatttt ttaagttata attatttttt ttatatttaa ggataattta gttgtttttt 129660
   ggtttttttt tttttagttt aaattatttt tttttagttt tttaattata ttttgagttg 129720
   ttttttgatt atagttaagg gttgggttta gtttggggtt ttaggatttt aatgaggttt 129780
   tattgttata ggatagtgag gaggtttttt attttttttt tttagggttt tagggtttgg 129840
   atatatagtt ttagaagggg tattgggtag agaagatgta tgttggtagt agagaggaaa 129900
   ttgagttgaa atttggtaag aataaaagaa ttataaaggt ttgtgtattt agtaaggtgt 129960
   atttttttat ggttataaaa gaaggtaaga ttgattgtag gtttggttaa agggattgtt 130020
   tgtgttttta gtgtataatg agatttatag tgggttggtg atttttttta aaataaagat 130080
   atgtttattt ggaattttaa atgtgatttg attttgttta agggtttttg tagaaagaat 130140
   ttaggtaagg attttaagat aaggtgggtt ttaaatttaa tgttgtgttt ttttaagaga 130200
   tagaaaagaa agtatagaga tatagaaaaa ggttttgtga agatagaggt agagattgga 130260
   gtgatgtagt tataggttaa ggaatatttg gggttattag aagttggaag aggtaaagaa 130320
   aggttttttt ttggagtttt tagaggaagt tggttttgtt ggtattttga ttttagattt 130380
   ttggttttta gaattgtgag aggatatatt ttttttgtta taagttattt agttttgata 130440
   gttgttatag tagttaggag aaatttgtgt tttattttta tttgtattat gttttgaggt 130500
   taaagtttta tatagtaaag atgtttaagt aattataaat tttgtgggta gtttatttgt 130560
   ttagtagatt attgttttaa gggaaggggg atggggatag ggaagagagt tataggaaat 130620
   ttttgtatgt ttttttggaa atgggaagga gtaggtttgg agagaaattt tataggttag 130680
   gttttgggga aatgaagttt ataaagtgtg ggaatggagt ggggaggaaa tttatgagga 130740
   agtagtgttt ggtgtatata tttatattta tgattttttt ttaggtatag tttttatgga 130800
   gaggtttagt tggtagggaa gtaaaagagg tttggagagt gaaggaaggt gaggggtttt 130860
   tttttagtaa tattgaggtt ttagatggga tattttatta ggtggtaggt tgtgttttta 130920
   tagatagaga ttttttaagg gtagggttta gatggtttta tttttgtgtg gtttgatata 130980
   aagtaggttt tttagagttg ttgagttttg ggtttgttag tggtagagtt ttttatagtt 131040
   ttttaagttt ttatagtttt ttattttagg aaatggtgtt attatttatt tagttgttta 131100
   agttaagttt ttgggtatta ttgttgattt tttttttttt tttattttta gtatttaaat 131160
   tatggtgtgt ttttattttt agtttttttg tgaattagtt tgaatttttt ttttttagta 131220
   gtgtgttagt taatggttaa taattggtta tttggggtta gtaaaagtaa taatatttag 131280
   ttttttgatt tgtagtgttt gtttattttt gtggtgttaa tatttttgtt ttggttaatt 131340
   tttttttttt tttttttttt ttgatagggt tttattttgt tatttggttt ggagtgtagt 131400
   ggggtaattt tggtttattg taaattttat tttttaggtt taagtgattt ttttgtttta 131460
   gttttttaag tagtttggat tataggtatt tgttattatt tttggttaat ttttgtattt 131520
   ttattagaga tgaggattta ttttgttggt taggttggtt ttgaatttag atgatttgtt 131580
   tgttttggtt ttttaaagtg ttgggattat aggtatgagt tattgtgttt agttggttaa 131640
   ttttaagtta tttgtatgat attattggag gtgagattga gaagagatgt ttagttatta 131700
   tgaatatagt gtttttatta tataaatata attttaagaa tataattgta ataggataga 131760
   taatagttaa atgttgtaaa aaatttaaga ggtgatgaaa gttgtgtatt tattattttt 131820
   tgttttaaat ataattattt ataagtaatt ttttaatgtt ttgtttaatt ttttttttat 131880
   aatttgtaat ttaatttata ttgtaaaaat ttattatttt tttaatagag taatgtattt 131940
   ttattgtgat aaaatatatg taatataaaa tttattattt ttggttagtt attatggttt 132000
   atgtttgtaa ttttagtatt ttggaaggtt aaggtgggtg gattatttga ggttaggagt 132060
   ttaagattag tttggttaat atggtgaaat tttgttttta ttaaaaatat aaaaattagt 132120
   tgggtgtggt ggtaaatgtt tataatttta gttttttggg aggttgaggt atgagaattg 132180
   tttgaatttg ggaggtggag gttatagtga gttaagatta tgttagtgta ttttagtttg 132240
   ggtgatagag tgagattttg ttttaaaaaa aaaaaaaata aaaataaaaa agataaattt 132300
   attattttaa ttaagttttt atgttagtga tattaagtat atttatattg tatatttatt 132360
   aatattattt atttttgaaa ttttttttaa ttttttaaat tgaaattttg tttttattaa 132420
   ataataattt tttatttttt ttttttttag ttgttggtaa ttgttatttt aattttttta 132480
   tgaatttgat tttatataag tgaaattata taatatttgt ttttgtgaga taggtttatg 132540
   tagtatgagg tttttaaggt ttattattta aggtttatat gtagtgatag aattttgttt 132600
   tttttgaagg ttgaatgata ttttattgtg tgtatatata ttttgtatat ttattttttt 132660
   tatatttgag taattgagag taaagttgtt ataaatatgg gtgtgtaaat atttgtttaa 132720
   gtttttgttt ttattatttt attattatta ttttatttat tattagtatt attattattt 132780
   tagatagttt tattttgttg tttgggttgt agtgtagtgg tgtgattttg gtttattgta 132840
   atttttgttt tttagattgg agtaattttt ttgttttagt tttttgagta gttgggatta 132900
   taggtatttg ttattatgtt tggttaattt ttgttttttt agtagagatg gggttttatt 132960
   gtgttggtta gggtagtttt gaatttttgg ttttaagtga tttatttttt ttagtttttt 133020
   aaagtgttgg gattatagat gtgagttatt gtgtttagtt attattttag ttttaaatat 133080
   tatagtaatt tttttattgt tttatgagat tttttgagat agtggtttta gggattttat 133140
   atatatttgt ttgattttgt gtaatgataa agtgtagttt tttttgtaga atggtagagt 133200
   tgggagattt ttgatggggt atgagtttaa aggggtagag gggatgttta gaatatatat 133260
   ggatttttta tgtgtgtttt gttagttatt aagtagattt gttttagttt tgatatgttt 133320
   aggattatag atagtgtatg gtttttattt gggttaagtt tttggaattt tttttttttt 133380
   tatattattt gtattgggag tagttattag gttgtggatt attaagtttt ttttaggtta 133440
   tttgagtttg tattttttta tttagtttga ttgagttggt gtgtagttat tatattttat 133500
   tatatttttt gtttggttgt tttaatgtaa ttataaggtt ttatttaaag aagtatatga 133560
   ttaatttgtt ttttagattt tttagtgatt tttttagttt gttttgtgta gaatatttat 133620
   agtaaaattt gattatagtg gtttgagttt ttatttttaa ggtaggggat tttttaaata 133680
   aagtattagt gttttaaatt tatgattttt taaggaggtt tttggttttt agtatagtaa 133740
   atgttttgtt ttatggtttt ttggtttttt atttttattt tatttgagtt ttttgttttg 133800
   gtatttggga ttgaattagt ttggagtttt ttagttttgt tgtttgttgg ttttgatgat 133860
   attattaggg attgtagtat tgtttggatt ttttgttgtt atattttatt gtaataatat 133920
   ttaatttgat ggaattttgg gagtaagtgg taggggtagg gatggtggtg gttaggggtt 133980
   gtatatatat aggatagaag gattgaagga ttgggtggaa agtatgattt ttttaagatg 134040
   attttttttt agtagaattt tggttgtgtt ttttgaatag tatttgttta tttgtatatt 134100
   tggtgagttt tgtgattagt aaatttagat gtttgggagg ttattagtgg ttttgttgag 134160
   gtataattta gatatgatag tttatgtttt ttttttgatt tttttttttt tatatagttt 134220
   tatgattaga gtgttttttt tatttttgtt tttattttag ttttgtaaga tatagttgtg 134280
   aaatggttaa agtttttaat tttttagtat ttgtttgaga ttggagtata aaaatgagat 134340
   ggggatatag tattttatgg atattagtga tggaaaaaaa ttttttgatt tttttgggag 134400
   ttttgtaaag ttatattagg ggataagaag gagtggttag tttggtattg atttagtttt 134460
   aaagtagtta gtgattaaag tatatagttg gtattttttt atttttaggg tattaggagg 134520
   tttggttaga atttatgtta gttaggagaa ataggttgtt tttggggttt ttggtgtttt 134580
   ggttttttta ttgttttttt gaagaaggta ttttgttttg tggggtttga ggttggaggt 134640
   aatttgttat tgatgtagtg tagggatgag ggttgagtta ttagtttgtt ttagttttat 134700
   gttagtagga ataataggga tttaagtaga gaagaaagta tttattttta aagttattat 134760
   agtgattgag gtgatgtggt ttttagggag tttttttttt tgttggtttt ttgtggattg 134820
   tggttttatg ttttaataat gtttttgttt attttgtaag gtttagtagg tggaaggaaa 134880
   gagggtggag gttgggagtg gtggtttggt aatttgggaa gggtagggag taggaatatg 134940
   tttttttttt tttgtatttg tttagaaggt tttatagggt ttgttttgtt atttataatt 135000
   ttagggagat ttttttttaa tgtttgtttt ataaatgttt atttatttta tatttgaata 135060
   tttttagtta tagggagttt agtatttatt tatgttattt attttatttt tggttgttta 135120
   aaaaagagga aagagagaga aagaaaagat aggaaagata gagaaagggg gaaagagaga 135180
   gagaggaaag aaagaaagag gaaagaataa gataggaagg aaagaaaaag gaagggaatg 135240
   gaagggaagg aagagaggga agaaagagag aaagagagag aaagaggtat aaataaggta 135300
   ggttttatgg agtagagatt taataaggga gtaaaatata tttgtagtta ggttgtagat 135360
   ggatttttat aggagtgtat atttatttta ttttttttgg aatagaaaag tggaaagaag 135420
   atgtagtggt gtgggtgggt ggtggttttt tttgggggtt tggaatgtgg ggtattagtt 135480
   tttttggttt tgagaaggag attaagatta ttaatattta tagtggagat tgaattatat 135540
   aagggttggg gatattgttt tttttgtatt tttaataaag atttgatgtt ttaattatgg 135600
   gaatttgtgg aagaagaaat aaaattggtt ttggggtttt ttagagaatt ggttttttat 135660
   ttagtggtgg ttgtgaagaa atttttggag ttagttttag tggtttgggt ttgtatagtt 135720
   gagattgttt ttggaagttg agtggaagtg gggagttttt aggtatagtt gggttttgtt 135780
   ttttatttta gttagtagtt ttaggatttt tgggtgtttt agagtttttt ttagagggta 135840
   aatttagagt atgagggagt ggggagatgt ttaatattgg gggatttttg tttgtttttg 135900
   tttttagttt tgttgatttg aatagggagg ttatgggtgt ttaggggtag aattgtgggt 135960
   tggtaagttt ttagttttgg tttttttttg tttttttttt tttattgtta ttgattattt 136020
   ttggttatat gttgatattt ttataggatg atggagaaga gagaagagga atttttttgt 136080
   tttgtttagt atattttaaa gaggttttag tgtaaaggta ttgatgttta tagttaaatg 136140
   tgtgtggggg ttttttagga gtgggatgtt tttattttgt tttagattgt tatgaattga 136200
   tagaaggttt tatgttatta aatatatttt attaataaat tattttaata tttagtaaaa 136260
   aaataaataa aaatgtatgt attttgtgtt ttgtagtgtt tttatgttgt attttttttt 136320
   gtatttgagt tttggtttgt gtttttggtt tgttgttttg ggaatagaat gttttgtgtg 136380
   ggaggtagat aggaagtgtg gggtgtgttg gttgggtttt tttagataat ttggtattgt 136440
   tttttttaga taatgaaatt ttttttattt tgagtgtttt tggaagttag ttggtagtta 136500
   gattaattta aatgaattat ttgttttttt tgtgaagtga ttttagggat ttaatgggtt 136560
   tgttattaat atagtttttt ttgtattttg tttttttttt ttattttttt ttttttgtat 136620
   gagttattgg ttatgatgat ttggtttagt taagggattt ggttagtttt ggttaggtta 136680
   tgtgagagat agtataaagg attaaggttg ttggaattag gttagaggtt gagggttata 136740
   tggatttttt ggagtgtttg taattgggga tttttaaggt tgttttttgg ttttggggat 136800
   ttgtttattt ggggatagtt ttaagggaga tttttaaaag tagggagtta aaagggtgaa 136860
   ttaatataga aatattgagg gttagggaga atttgaagat gggtttggtt gggtatagtg 136920
   gtttatgttt gtaattttag tattttggga ggttaaggtg ggtggattat ttgaggttag 136980
   gaatttgaga ttagtttggt taatatggtg aaatattatt tttattaaaa atataaaaat 137040
   tagttgggtg tggtggtagg tgtttgtaat tttagttatt taggaggttg aggtaggagg 137100
   attgtttgaa tgtaggaggt agaggtggta gtgagttaag attatattat tgtattttag 137160
   tttgggtaat agagtgagat tttattttaa aaaaaaaaaa aaaaaaaaaa aaaagatagg 137220
   tttaaggttt ggagagttag aagttatttt tttatttttg ggtggtttat tttgagattg 137280
   tattatgtta gagaaagttg tgggagtgtt tgtttataat ttataagtaa tattgttttt 137340
   tttttttatt agtttttggt tttagtaggt ttttgatttg tgtttttttt agggttttgg 137400
   gtttttttaa gttggatttt tggttgttgg tgggatgatt atgtgatttg ttattttttt 137460
   gtttattgtt ttgggtagtt tttgttattt ggtgtatata gagtggagtt tagtaaggtt 137520
   ggggttgttg attttttgtt agttatattt gttttttttt atgttatttt tttttgtttt 137580
   ggttatgttt aggggtttat atttttttat aagttaggtg ttagattttt gtgtttgttt 137640
   tattttgttt tttttgtttg gtgggttttt tatatttttt ttggtttgat ttaggtttga 137700
   tttgttttta ggttttagtt tagaggttgt tttttttagg aagttttttt gggattgtta 137760
   gatgaaggga tttttttttg gatttttttt ttatttttat agttttagga atagttttta 137820
   atttaggttt tattgagtta gtgtggtatt taggagagtt ataagtgtgt gatgaatgtg 137880
   ttgaagtttt tttaaaagtt gaaggagttt tgtgaatata tagtagtatt agtttagatt 137940
   gtgaaggtat aggggagtta gtttttttta gttttgtttg tttttggttt tttttttgag 138000
   gaggtgggag ttagttagtt gggattagga ggtgggtttt atggagtaga gatttaataa 138060
   gggaataaaa taaattagag aatttttttg ttgtaaggaa tttagataag gagtatttag 138120
   ttagaggagg aaggaagaga ttggtagtag attttttttg ttttagtagt tttgtaagtt 138180
   agatgggtta attttttagt atagttgttt tttagtatag agaaattttt tatttttagt 138240
   aatagggtgg gaatattttt agatagggtt atggttattt tggttatagg gattatggag 138300
   tttgagtttt tgtggtttgt tttatttatg tggttataga ttttgagtat atatttggag 138360
   ttatagagtt taaagggtag ggtagggtag ggaatttgtt tgattggtga aggttatttt 138420
   attatttgga gtataggttt ttgtttttaa atttatatgg agtttttgat atggggtatt 138480
   gattttaagg aggtgaatgt ggatatttaa ggttagtgaa tggttaggtg ttaatatatt 138540
   gataggtttt tggttttttt tttaatattg agggtaggaa ataaatgggg tttatatggt 138600
   gtatatggtt tttagagtta gtaaggtgtg taagatttgg tagttgagaa aattgattat 138660
   ttggttgggt gtggtggttt atgtttgtaa ttttagtatt ttgggaggtt gaggtgggtg 138720
   gattatgagg ttaagagatt gagattattt tggttaatat ggtgaaattt tgtttttatt 138780
   aaaaatataa aaaattagtt ggatgtggtg gtaggtgttt gtagttttag ttattttgga 138840
   ggttgaggtg ggagaatggt gtgaatttgg gaggtggagt ttgtagtgag ttgagatagt 138900
   gttattgtat tttagtttgg gtgaaagagt gagattttat tttaaaaaaa aaaagaaaag 138960
   aaaagaaaag aaaattgatt atttgattat gaataagtta gaatatttat ttagaaaaaa 139020
   aaaatagttt tgtttaattg gggatttaaa attttaggat ttatgttttt tttttataga 139080
   gttatattat ttttttaatg tttatttttg attttatttg tttgttaagt tttaggtatt 139140
   tagaatatag tatgatagta agttttgtga tttttatagg tttttttaat ttattttagg 139200
   tatagttata tttttagttt tgagtagtag ggagtgatat gtagatggtt atttagatag 139260
   agaaagattt ttaaaaggag ggtaggtttt gttttgtagg tagtatttat tgatatagat 139320
   attagtaggt aaatttttta ggaagatttt tattaagaaa gaatgaaaga ggtttagtgt 139380
   ggtggtttat gtttgtaatt ttagtatttt gggaggttaa ggtgggtgga ttattttagg 139440
   taaggagttt gatattagtt tggttaatgt ggtgaaattt tatttttatt aaaaatataa 139500
   aatttagttg gatgtggtgg tgggtatttg taattttagt tatttgggag gttgaggtag 139560
   gagaattatt tgattttagg aggtagaggt tgtagtgagt taggattata ttattgtatt 139620
   ttagtttggg gggtaataga gagagatttt gttttaaaaa aaaaaaaaaa aaaaaaaagg 139680
   atgaaagata aataaggttg aaattggtag gttggtatat ttttaataat ttattttagg 139740
   ttgggtgtag tggtataatg ttagtatttt gggaggttga ggagggtaga ttgtgaggtt 139800
   aggagattga gattattttg gttaatatgg tgaaattttg tttttattaa aaatataaaa 139860
   aattagttgg gtgtggtggt gggtgtttgt agttttagtt attttggaag tttaggtagg 139920
   agaatggtgt gaattaggga ggtggagttt gtagtgagtg gagattgtat tattttatgt 139980
   tagtttgggt gatagagtga gattttgttt taaaaaaaaa aagaaataaa aaataaaaat 140040
   aataaaaaaa aaataattta ttttagtagg gagtaggagg taaggtttga tatgtagtgt 140100
   ttgttaattt ttgtggtgta aatatattta ttgtggtaaa ttttaagtta taaataggat 140160
   attattaaat atagagttgg gaaaagatat gtatattatt ggataaaatg tttaggtagt 140220
   tatggtttat ttttttttgg gaaggggggt atgtttagaa tgttttttat tatggagaat 140280
   gattgtggta aatagggttt aattggtagg ttgatttaat taattgttta ttgatgtgat 140340
   gtggggtagg tttttgaggt tgtttttatg tttagggaag aaggtggtta tgtggttata 140400
   gatttggttt tgggtttgtt tttttttttt ttttttgaga tagagttttg tttttttttt 140460
   ttaggttgga gtgtaatggt gtgattttgg tttattgtaa tttttgtttt ttgggtttaa 140520
   gtaatttttt tgttttagtt ttttaagtag ttgggattat aggtgtttat tattatgttt 140580
   agttaatttt gtatttgtag tatagatggg gttgttggtt aggttagttt taaatttttg 140640
   attttaggtg atttatttat tttggttttt taaagtgtta ggattatagg tatgagttat 140700
   tgtgtttatt tgggtttgtt attttgagga tatatttgga gttatgtgtt tatgattttt 140760
   tttttatttt aaaattttga ttttatttta ttgtttttag agataggttt tgttttgtta 140820
   tttaggttgg agtgtagtgg tgtaattata gtttattgta gttttgaatt tttaggttta 140880
   agtgattttt ttattttagt tttttgagta gttggggttg ggtttatagg tgttattata 140940
   tttagttata ttttatattt ttttgtagag atagggtttt gtaatttgtt taaattggtt 141000
   ttaaattttt ggttttaagt aattttttta tttttagttt tataaagtgt tgggattata 141060
   gatgttagtt agtgtattta gtagttatta tgattgattg gtgatgtttg ttttagtttt 141120
   aggatgaaag agtagtagta ttttagttaa gtatttgtgg tttttgattg gaagatgttt 141180
   tttttgtttt ttaagtatag tgtaaatgtt atttttttgg taagtttttt tttaatttta 141240
   tttttttttt ggtatagggt ttgttatagt tttgtttata taaagagagt tattagtgta 141300
   tgtggtatag atgttagttg tttttttaat atttgagttt tttttttttt atagtaattg 141360
   aagttttggt tgtgtatagg gaagtttagt tgaagaatat attgtttagt ttttttgatg 141420
   ataggagtgg ttatataatt gggttttggt tagtgggaag gagttgaagt gtttaatttt 141480
   taggttatgt ttttaaaaat aaagagggat atttgttttt tttttttttt tttttttttt 141540
   tttttttggt tggagtgtag atatgatggt aggagttgga attattttag tttaagagat 141600
   ggaagttgtg tgttgaggat gtattaaagg aatgaattag gtttgggttg ttttgggttt 141660
   ttataatgat tgatatttgg attgggatgt gggtttgagt gttgtttagt ttttgatgtt 141720
   ttagtttttg ttatatagtt agatggtgtt ttgattaatt taaggtatgg ttattatttt 141780
   atttgtttgg tagggtaggg gttttatttt tatatgttga gtttttggta gaatttggtg 141840
   taatatatat agttaaagta aaatgaatag gttattttaa ttaaggttta ttttgtgggt 141900
   tggttttggt atttggtttt aatttttatt ttattttatt tatttattat ttttgagata 141960
   gagttttata tttgttgttt agtttagaga ggagtggtgt gattttggtt tattgtaatt 142020
   tttatttttt aggtttaagt gatttttgtg ttttagtagt tgggtagttg ggtttttttt 142080
   gagtagttgg gattataggt gtatgttatt atgtttggtt aatttttgta ttttttgtag 142140
   agatggggtt ttattatgtt ggttaggttg gttttgaatt tttgatttta agtgatttat 142200
   ttgttttggt tttttaaagt gttgggatta taggtatgag ttattgtgtt tagttagttt 142260
   tggtatttgg aattggaaaa taatagtttg agttttagag gggataaata taaatatgag 142320
   gtagaaggaa tagtaagtaa atatgtgttt agagtaggtt gggttgttta tatttttaaa 142380
   atattgttag gtgttgaggt ttgggttttg tgttagattt ttgggtgtag attttgtggg 142440
   gatatttatt gggtagtggg atttttggta agttatatta tttttttgag tttttgttga 142500
   ttaagttgtt tattgagaat aatatttaga ttgaaaggat ttataataat atgtgaaaag 142560
   tgtttggttt aaaataggag tttggtaatt ggtagttttt attatttata atatatagtt 142620
   taatgttttt gttttatagg aatgaaaata ggatttagat aggtaaaatg atttgttgag 142680
   ggttgtatag tgttaatgat agagttagga ttagggtttg gggttttgat tttttttgta 142740
   gtattttggt tttgtgagaa gtggttttgg gtgaatataa ggagtgagga gttggtgaga 142800
   aattttgaaa atgaagttgg gtgtggtggt ggatgtttgt ggttttagtt atttaggagg 142860
   ttgaggtagg aggattgttt gagtttggga gttggaggtt gtagtgtgtt atgattgtat 142920
   tattgtattt tagtttaggt gatttagtaa gattttattt tagaaaagga aaggaaagga 142980
   gaggggaggg gaagggaggg aatttttgat agtgatttta gagataatta ggatgatttg 143040
   aaattatttt gaaatgtatt aaaaaataaa aataaaatgg atttatggtt aggtgaagga 143100
   gggatagatg gatggtgagg tgataaagta ggtgtagttt ggtgttagtg gtagaattta 143160
   ggtgatggtt gaatgtgttt tttgtaaaat gtttttaatt tgattatgtg ttttgaatat 143220
   ttttataata gaatggaggg aagagaaagg taagagggag ttattgtagt tgttgtagta 143280
   ggagtagatt ttaaaatatt atgtattaga tttttagttt tgtttttgtg gatgtgtttt 143340
   tgtttttttt tttgggattt aaggatattt aatgagtgta tgtgttttta agtttttttg 143400
   tttttttatt tgtttttata tttggaagat tttataggtg gtgtgggtta gttttttatt 143460
   ttgttttttg tgtttttagt gtatgataga tattgagtga gtgttagttt ttttttttga 143520
   tattttgttt gtttgtttag agaggttttt ttatagttta gttattaggt tgatgttttt 143580
   ggatgtttgg ttgatagttt ttttagttgt gttttagtga tttggtaggt gttttgagtt 143640
   ataaataagg ttattagtgg atgtttttaa agtttttttt attatggtgt tttttttttt 143700
   tttattaata gtttgttatg atagttagtt tgtatgagtt tttttttagg gttgtgtttt 143760
   ttgaggttgt ttagtgtttg gtttgttaga gtttattaga tagggtgttt atttttttta 143820
   ggtttgagtt tttatttaga gaattattta tagaaggata atggagaaag gtagttgggg 143880
   tattaaatgg agtgtgtttg tatgtggggg gggaggggag ggggagggaa agtaggaaga 143940
   gaagatataa gaggagaaat aagaataaga ggaaatatag aaggaggagg agtagttttt 144000
   tttatggggg tggattttgt gtttagtttt ttattaagga tattatattt attattttgt 144060
   ttggttttta tgaatatttt taaggttgtt tttttttggt ttttattttt ttattaggaa 144120
   gtggaggtat agagtgagaa agagttgttt aaggttttta ggtagtttat ggagttattg 144180
   ggagttatat ttaatttgtt taattttatg tttttgtttt tagttattat aataaattat 144240
   taaggtttta tagttatagg agtttagttt ggagttttta ttgggttttt taaggtaggg 144300
   agatggggtt ggaggttatg ttaggtgtat ggtgtttaga gttgtttagg aagttttgtg 144360
   gtgtgggttg ggtattaggt agtgtttgta ggatggagaa atgaatgagg ttgagatata 144420
   gtgttgtttt ttttaaatgt gatttttttt gagtttttgt gattggttat ttgagagggt 144480
   agttttggtt ttttggtgtt tatgtttggg tttagagatg ttttttttag ttaggttttt 144540
   ttttgaggat ggaaatttag aattttatta taggaggaag agttgggagt attttatttt 144600
   tttttttttt ttgagatgga gttttgtttt gttgtttagg ttggagtgta gtgatgtgat 144660
   tttggtttat tgtaagtttt gttttttggg tttatgttat ttttttgttt tagttttttg 144720
   agtagttggg attataggta tttgttatta tgtttggtta attttttgta tttttagtag 144780
   agatggggtt ttattgtgtt agttaggatg gttttgattt tttgattttg tgatttgttt 144840
   attttggttt tttaaagtgt tgggattata ggtgtgagtt attgtgtttg gttagggagt 144900
   atttttaaga tttttgtgga gggttgttta ggtttaagtt ttttattatg gaaggttttt 144960
   tagatgtggt agttatggtg tatgggttga gggtttttat ttattaagaa gtaggagtat 145020
   ttgatgtata tttagtttat gatttttatt tatttggggg ttgtgtttgt agttatgtga 145080
   tataagggaa tagttatagt agtgggttat aaagagagtg tgggttaatg tgtaggggta 145140
   gaattttgat agttttttat gggttggtag tagggagggg tatatttgta taaattttat 145200
   atgtatagtt ttttatatat atgtggttga taattattta tatatttaag atgggatttg 145260
   gtgtatagtt tattagtaat aggtgttggt aatttttagt tgtttttttg ttgtggtgta 145320
   gatattattt aaggtgggat ggagagagtt attgttttta aggttatata ttttaaaggg 145380
   agatgttggg ttatggaatg aaaataggag gattagttag agttattggt tttgagttat 145440
   ataatgggat atataggttt atttatttta tatttttatt gagtaattat aatgagttaa 145500
   gttgtataga ttatttaaat aattggtatg tagaaaagta ttttaatttt tttttggttt 145560
   tgatgtgatg ggaagaattt ggatttttgg agttatagag ttggatggaa attttggttt 145620
   agttaattat tggttggggg agtttgggta agtgatttaa ggtttttgag tttgtttttt 145680
   tgtaaaagag tagtgtttgg gtttgttatg ttaggttagg gtaagatgat tgaagagtaa 145740
   gtgtttagta ttggttaggg gttaatgttt ttaaggatgt aaagtgtaat aagtaggtgt 145800
   tatgattgta ttgtttttta ttgtttgtgg taaatatata agtaaagatg gttttgtgtt 145860
   tgtgttgggg gaggtaggga attggtttta ggaggatgtt ggttttttta gttgtttttt 145920
   ttaaagtttt ttgggaggag aaatgattta ttttagttgt tgggagtttt aatttataag 145980
   taggtttgtt tatttggtag tggttgtatg taggtaggag gggttttggt agtttttaag 146040
   agtgaggggt tgtttagttt ttttttagtt ggaaaggagg ttttaggttg ggtaggagta 146100
   tttggggaaa tttagttttt ggagttaggt tatattaggg agatgaggtt gttagaagtt 146160
   tttttggtgt ttatagtaaa atataattaa agtgggattt agattttaat tagtttaagg 146220
   tttgatttat attagaggga aattagtgtt tgttgtatga gtgagtgttt atgtgtgtgt 146280
   aaggtagaga tagattttgg gtaagttttt ttattttttt atgtttgttt ttttatttat 146340
   aaaatggggt aatattattt attttatagg gttgttatga aataaatatg tattaatatt 146400
   tgtaaagtgt tgagaatagt atttggtata aaataagagt gatagatttg ttaaataagt 146460
   aaaaaataag atataagttt aaagtagagt ttggtatatt gtgagtatta agagttgtgt 146520
   tttttgattt tttttttttg tggagatttt tttattagaa gtgggaggga ttgttgaggt 146580
   tggagttttg ttttagggat ttttgtgggg agttttttag tttagaggga tttgttttat 146640
   ggattatatt tgttttgggt ttttgtgttt ttgtgtatgt gtgtatatat gtgtgtatat 146700
   atatgtaatg tgtgtgtgtg tatttttatg gatttgtgat aatttgtata tatatgttta 146760
   gaatttattt gatatagtgt tttattaaga tgttggagtt ttgatgaatg gattgtgggg 146820
   ttgagatatt gtttttatag gaagttgttt tagtagtaag ttttttggtt tttatagata 146880
   attaaaaaga atggagtttg tttttatttt ttgttttaaa aatgatgtat agtgtgtggg 146940
   gtttgatgtt ggaggttttt ttagtagata tatttttaat aatattatat tttgaaattt 147000
   aagttgttgt aaaataagat aagtaaagat ggtattggta gggatttttg aggttttttt 147060
   tatgtatttt tgaataattt ttatatggtt tttgtttgaa tgtttttagg tatggggaat 147120
   ttattatttt gtgaaatagt ttgttttatt gtttttaagt gaatattgtg agaaaaatat 147180
   ttttttttgt aaagagttaa gttttatttg ttagtatttt ttaggtattt ggtattagtt 147240
   ttatattttg ggggaattta aaataaatgt ttttttttta ttagataatt tattaattat 147300
   ttggagatag tggttgtagt tttttgtttt atgtagttat tttatttagt ttttttttgg 147360
   aatttttgat gtgaatataa gtttttttgt tatgtaggtt ttttttttgg gttttttatt 147420
   tatttgtgta tattttggag aggttgtttg ttttgtgggt gagatttatt gtgggttgaa 147480
   ttgtgttttt ttaaaagata tatgtaagtt ttgatttttt agtatttatg aatgtgattt 147540
   tatatggata tagggttttt gaagatgtaa ttgggttatt tgagggtata ttgaattaga 147600
   gtgggtttta aatttaatga taggtgtttt tataagatag aggtaggttg ggtatagtag 147660
   tttatgtttt taattttagt gttttgggag gtaaagtttt attgtttgag tgtaggagtt 147720
   tgagattagt ttgggttttt gtttttataa aaaaattaaa aaattagtta ggtatggtgg 147780
   tgtgtgtttg tgttttagtt atttggaagg ttgaggtggg aggattgttt gagtttggga 147840
   ggttgaggtt gtagtgagtt atgattatgt tattgtattt tagtttggag gatagaatga 147900
   gaatttgttt ttaaaaataa attaaaaaga taatagagag ggtagtttgg atagagatat 147960
   aagaagagat atagggtgaa ggttaggtgg ggggtaggtt gagttgtaag ttgaggaata 148020
   ttaggggttg ttggttgttg tagagtttgg agaaaggtat gggatagttt tttttggaat 148080
   ttttaggagg aatgaatttt tagatatttt ggtttgggat ttttggtttt agaaatttta 148140
   gggaagatat tttggatgtt tttgtttttg tttttgtttt tgtttttgag atggagtttt 148200
   gttttgttat ttaggttgga gtgtaatggt gtgattttgg tttattgtaa tttttatttt 148260
   taaggtttat gttatttttt tgttttagtt ttttgagtag ttgggattat aggtatttat 148320
   tattatgttt ggttaatttt tttttttttt ttgtattttt agtagagatg gggttttatt 148380
   atgttagtta ggatggtttt gattttttga ttttgtgatt taattgtttt ggttttttaa 148440
   agtgttggga ttataggtat gagttattgt atgtggttga ttttggatgt tttgagttat 148500
   tttatttgtg gttttttgtt atgatagttg taggatagta atatagggtt ttagtagggt 148560
   ggtttggtaa gggttttttt tttatggtag tttttatttt gttttatttg tagggttatt 148620
   tagatgttta ttttggtttt ggttagtaag tgttagtgta tttttttatg tttagtagta 148680
   agggtagtat tgttgttgat taagtatttg ttatgttggg gtttttgtgt ggttttgggg 148740
   tttttttttt ttgtttatat ttggtagttt agtttttttt tttgtatttt atttgatgtt 148800
   ggggttttgg gtaagtttgg gggatttgtg ttttatttta tagatttttt agttgatttt 148860
   attagttttt gtatagggtt tgtttgaagg gttgttgggt tgggtgttaa ttagagtttt 148920
   tagtagtagt ttttttggtt ttttgatttt ggtttatgtt ttaggggtag ggtttttaag 148980
   ggttttgggg ttgtttttga ggttagtttt tttgttgatt gtagtttata tagaggaggg 149040
   tttaattttg agtgtggttg tttgtgattt tgagagatgt ttagagttgt agatgtgttt 149100
   tttaagtttt ttagagtttg ggataggagt tatttaatgt ttttagtgtt ataggtaaaa 149160
   tttgtttttt ttaatataaa ttgtttttag ttttatttaa tgtttttttt aatgagtagt 149220
   aataaaaata tggttttttg ttaaaggtgg ggtttatagt tttttagaat ttggtttttt 149280
   gttaggttta ggtataattt ataggagtgt ttgtttttga tttgtgtgtg tgtgtgtgtg 149340
   tgtgtgtgtg tgtgtgtgtg tgtgttgtgt gtatgtgtat atgtgtttgt gtaagttttt 149400
   ttatgtgtag ttttgggaag ggtggagagg tttatggaaa ttttttttgg tatttagtaa 149460
   gttagaaaaa aggaaaatga gatttgtgta tatatgtgta agtgtatata tagtatatag 149520
   gtatatatat atatatattt agtatatatt atataggttt atatgtgtat atatgtgtag 149580
   gtatatatta tataggttta tatgtgtata tatatggggg tatatagtat ataggtatat 149640
   ataggtatat atattttttt gtaggaaagt ttttttttgt tttttatttt gtagggtttt 149700
   taggttttat gaaataagat tgaattttgg aagtttgata aaaggtttgt ttggagtatt 149760
   ttggttattt ttattagttt ttttagttgt ggaaggtatg ttttgtggta tttatttatt 149820
   ttttgttttt atttttattt ttgtttttgt tttttttttt ttgttttttt agtttatatg 149880
   ttgtttgttt taattttaat ttttgtattt ggatgggaat tatttttttt gtagaagggt 149940
   gtaatgtatg tggggatata tatatatata tatatatata tattgtattg gaggagagaa 150000
   gagaggtaga gtttttttta gatttagagt tttagtgagt gatgggattt tttttagaga 150060
   atatgtgtag aagtttgtgt tattggaaga agatattatt ttgatttata tttttttttt 150120
   ttttttttgt tttttttata gagaaggaaa ggttttagta aatagtggtt atgtgggttt 150180
   tttgtatgtt attggtagtt ttttagttag gattattttt tttttatttt tggaagtata 150240
   agaatagaga gaagggtgag tttttgtttt tgggggtttt tttttgtatt tttttggagt 150300
   taattttagg gttgtgggtt gtataggaag gagggtttaa taggtttttt gtttaatttt 150360
   gtttttttag tgaggagttg atttattttg tttgtaaaag atgttggttg ggatttgatg 150420
   ttgggtaatt tgaggagaag agaaggggga taggggttga ggttaggttt ttggttttga 150480
   aatagttata atttttgggg tagagtttaa gggtgttagg ttggagattt tatatttggt 150540
   tagttggtag ttattatttt ggtttagtta gtggtttagg gtattatagt tatttagatt 150600
   aggaatttta tttttttgga ttgtattaat gaggaaggtt gagtttgtag gttttatata 150660
   tttatggtaa ttttaaggta gagaggttat tgttaattga gggttatggg taggagtttt 150720
   gtgagtttat tagtaagttt ggaagattaa ggtttttggt tatagagagt agttgttatt 150780
   tggttaattt tgtagaaaat attatagttt tggataaaat atttgaaaga ataaaataat 150840
   aatagtttgg agttagtata gaatatttta agtaggtaga agttggagta gagtttatat 150900
   ttgatagagg ggaataaaat tagataagtt tttggttttt ggaggttttt ttgtttagag 150960
   gtaggtattg agtaattaaa tttggataaa aagttttaaa agtatttagg gaaaaataat 151020
   atgtggttta taggggaata gttagatgaa tgttgatttt ttgatagaaa taatggaggt 151080
   taaagatatt ggaattttat attttaaagt tttgaaagaa aaaattgata atttagaatt 151140
   ttataattag tgaaattatg gtttaagaat gatagtaggt tgggtatagt ggtttatatt 151200
   tataatttta gtattttagg aggttaaagt aggtagatta tttgaggtta ggagtttgag 151260
   attagtttgg ttattatggt gaaattttat ttttattaaa aatataaaaa ttagttgggt 151320
   atgatggtat atgtttgtag ttttagttat ttgggaggtt gaggtaggag aattgtttga 151380
   gtttgggagg agaaggttgg tgagttgaga ttgggtagga gttttgaaat tatattattg 151440
   tattgtagtt tgtggggaga tagagtgaga ttttgtttta aaaataaaaa taaaaataaa 151500
   aaaagaaaga atgatagtaa aataaagatt tgtagataag tgaaaatgaa agggaattag 151560
   ttgtttgtaa atttgtatta taaaaattgt gaagtaagtt ttttaagatt atgggaaatg 151620
   agattagatg ggaatttagt tttttaggaa ggaatgaaga gtataggata tagtaaatat 151680
   atgggtaaat agaaaagatt ttgttttatt tttaattttt ttaaaatgta tggggttgtt 151740
   taaagtaaaa atttttttat tgtattgttt agtttataat gtgtatggat gtagtaaata 151800
   tttaataatt attatatata ggatggaaag tggtaaatga atttatagga ttgtttagtt 151860
   tttatatttt ttttttatga agaaaaataa tgttaatttt aagtagattg tgaaaagtta 151920
   agaatgtgta atagaattta taaagtaatt attaaaatag taatataaag agatataatt 151980
   aaaaagttaa aataggttgg gtttagtggt ttatgtttgt aattttagta ttttgggagg 152040
   ttagggtggg tggattgttt gaggttagga gtttgaggtt agtttggtta gtatggtgaa 152100
   attttgtttt tattaaaaat ataaaaatta gttggttgtg gtggtaggtg tttgtaattt 152160
   tagtatatta gggaagttga ggtaggagaa ttatttgaat ttaggaggta gaggttgtag 152220
   tgagttaaga ttatgttatt gtattttagt ttgggtaata gagtgagatt ttgttttaaa 152280
   aaaaaaaaaa aaaaaaaaat ataaattaaa atagaatttt aaaaaatatt taattaatat 152340
   aaaagaagtt aagaaagtaa gatgaaagaa ataaatatta aagagtaagg tagtagtttt 152400
   aaatttagtt atatttataa ttatgttaaa tagaaatagt ttgaatattt taattaaaag 152460
   gtggaggttg ttagaatgaa taaaatagta agatttaatt atatattatt tataaaagat 152520
   gttgttttag tataaatata ggttgaaatt ttttgtttgg aaaaagatat attgatgttg 152580
   tttgaatgat gatggagtta tgttttgata aatttattat aagtggaaaa taaatattat 152640
   tttaaaaatg tatttaatat atttaattta ttgaatattt tagtttagtt tagtttattt 152700
   taatatgatt agaatattta ttttagttta tagttgagta aaattaataa atataaagtt 152760
   tattttataa taaagtgttg attattttat gtaagttatt gaatattgta ttgaaagtga 152820
   gaaatataat ggttttatat atatttaaag tatagttttt attgtatata tattatgttt 152880
   gtattatggt aaagttgaaa aattgtaagt taaattatta taagtttttt tataagtttt 152940
   tttagttttt ttatgtattt taaatagtat gtttaagaag gttggagagg ttatataaat 153000
   attaataaag ttgagtttaa gataaaatta ttatgagata aaggaatata tttattatgg 153060
   ttaaatgatt agtttattga gatattatta taattataaa tgtgtatgta tttaattaaa 153120
   ggagttttaa aatatatgat ataaaatttg aaagaattaa agggagaaat gggtaatttt 153180
   ataattatag gtggagattt tttttttttt tttttgagat ggagttttat ttttgttgtt 153240
   taggttggag tataatggtg taattttggt ttattgtaat ttttattttt tgggtttaag 153300
   tgattttttt gttttagttt tttgagtagt tgggattata ggagtttgtt attatgttta 153360
   gttaattttt gtatttttag tagagataag gttttattat gttgtttagg ttggttttaa 153420
   atttttgatt ttaggtgatt tatttgtttt ggttttttga agtgttggga ttataggtgt 153480
   gagttattgt gtttggatgt atagttggag attttaatat tattttagtt tatttaggat 153540
   gttaaaataa aatgttttag gttgggtaat ttataaatag attgtagtgt ttatagtttt 153600
   agaggttggg aagtttaaaa ttagggtgtt tagtgtttgg tgagagttag tttttttgtt 153660
   tagagatgtt ttttttttgt tgtgttttta tatggtagaa gggattaatt ttatttatga 153720
   tgattttgtt tttatgagtt aatatttttt tgatgtttta atttttaata ttattgtatt 153780
   agagattaag ttttaatata tgaattttgg gggatataaa taggtaggtt atagtaaata 153840
   ttattttttt aatatttgag aaataagtat tagataattt gaagaatatt ggtaattagt 153900
   aattgattta attagtagtt ataaagtttt atatttagga agtttagaat atatatatat 153960
   attttttttt taagtgaaaa tagtgtattt attaagatat atattttggg ttataagttt 154020
   taaattttaa aagtatgtaa aatatgtttg tttggttaaa gtaagattaa attagaaatt 154080
   aatagtttta agatatttag aggagttttg attatttgta aattaaataa tatattttta 154140
   aataatttat gagttaaaga ggaaattata agaaaaattt tataatattt taaatgaaat 154200
   gataatgaaa atatagtata ttaaaatatg taggttgaag ttagtgttta gagggaaatt 154260
   gtaagtgttt atattagaaa agaagtaaaa aaaaaaaaaa aaaaaaaaaa aaaaagattt 154320
   aaggatttgt tttaagaatt tggaaaaatg ggttaggtgt agtgtagttt atgtttgtaa 154380
   ttttagtatt ttgggaggtt aaggtaggtg gattatttga gattaggagt ttaagattag 154440
   tttggttaat atggtgaaat tttgttttta ttaaaaatat aaaaattagt tgggtatggt 154500
   gggtattttt agttatttag aaggttgagg taggagaatg gtttgaattt gggaggtgga 154560
   ggttgtagtg agtttagatt atgttattgt attttagttt gggtaataga gtaggatgtt 154620
   tttttaaaat aaaataaaat aaaataaaat aaaataaaat aataaaaaaa ataaagaatt 154680
   tggaaaaata tgttagttat ttatgtattg ttttttaatt ttgtatatat tttgttaggt 154740
   tttgttttgt gatattggat tgaggtttta tgagtttttt tttttggtta aatatagtta 154800
   tgttaggttt tatttatagg ggataatatt gtggggagtt gttaggtttt gttgaggtag 154860
   tagttttttt ttttggtttt agggtttttt ttttttttga aggtatagtt gttagtggtg 154920
   tgtgtttagt ggggtttgtt ttgtattgag tttttttagt attttagttt tagtttaatg 154980
   ggattttagt aaatttttta gatatttagt ttttgtttat ttatattttt tagtaggttt 155040
   tttggttatt tagtaagttg attttgtaga tttatttttt ttatattttt tggtaagaat 155100
   ttttttgtta ttagtaggtt gtaggttttt gtggtagtta tatatttttt aataaggttt 155160
   gaatgtaaga tgtggtaagg tgtggaggat gttttgagtt tttggttttt tttttattaa 155220
   ttttagtggt agtaattgtt tttgtgtgtt tgtatatatg ttttaaagta tttattatag 155280
   aaaatttatt attttaatta tttttaggtg tgtagtttgg tggtatgaag tatatttata 155340
   ttgttgtgta attattatta ttatttattt tattttttta aattgtaatt ttatttttat 155400
   taaatattaa ttttttattt attttttttt tttttagttt ttggtaattt ttattttatt 155460
   ttttgttttt ttgaatttga ttattttagg gattttataa aagtgaaatt atatagtatt 155520
   tgttttttta tgtttggttt ttgttattta gtatgttttt aaggtttatt tatgttatag 155580
   tatgtgttag aatttttttt tttttttttt tttttttttt ttttgaggta gttttgtttt 155640
   gttgtttaag ttggagtgta gtggtatggt tttggtttat tgtaattttt atttttttag 155700
   tttaagtgat ttttttgttt tagttttttt agtagttggg attataggta tatgttttta 155760
   tgtttggtta atttttgtat ttttagtaga gatagggttt tgttatgttg gttaggttag 155820
   ttttaaattt ttgattttaa gtgatttgtt tattttggtt ttttaaagtg ttgggattat 155880
   aggtgtgagt tattatattt ggtttttttt ttttttttaa ggtagaataa tattttattg 155940
   tatagatgga ttatattttg tttgtttatt tatttgttgg tggatatttg ggttgtttgt 156000
   attttttggt tatgggaaaa gtgttgttat aaataagaat gtagtatatt tgtttaagtt 156060
   tttgttgtta gttttttgga gtatatattt agaagtggaa ttattggatg ttatggttat 156120
   tttgagttta attttttgag taattgttat atttttttta tagtagttgt attattttat 156180
   atttttatta gtagtgtatg gggttttaat ttttttatat ttttgttaat atttgttgtt 156240
   atttatgttt tggttaatag ttattttaaa gggtgtgaaa ttatatttta ttgtggtttt 156300
   gatttttatt tttttgatga ttagtgatat tgtgtatttt tttttgtgtt ttttggttat 156360
   tggtatgttt tttgtggaaa aatgtttgtt tattgggttg tttttttttg ttttgtggtt 156420
   tagttgtagg agttttttat atattttgga tattagtttt ttattggata tatgattttt 156480
   aaatattttt ttttattttg tggatttttt ttttatgtta ttggtagtat tttttgatgt 156540
   ataaaagttt taagttttgg tgaagtttaa tttattattt attttttttg ttgtattttt 156600
   ggtgttatat ttaagaaagt attgttaatt ttaatgttat gaagtttttt ttttatgttt 156660
   ttttttaaag tagttgtttt ttatatttgt tttttttata ttttttagag tttttttgta 156720
   taaggtaaaa taaattttat ttttttattt ttatttataa tatttagtgt tttagttttg 156780
   atattagatg tgtggttttt ttttgttgat ttttaagtaa ttttttaata tttagtagtt 156840
   tttaattggg tgttttgtaa tttagtttaa ttttgatatt aattagagtt agtgtagatt 156900
   ttataggtta agggttttat tttataagat tgtttttaat tttagagatt agttgtaagt 156960
   ggtaagatat taggttattt ataatttttg tttgatttgg ttataaattt gaggttttta 157020
   taattttttt tttaggttta ataatttgtt tgagtgattt atagaattta ggaaattagt 157080
   ttatttaaaa ttgttgattt attataaatg atattttaaa ggatataaat gaatagttaa 157140
   ttggaagaga tgtataagaa aggtatgaag gaagtggtat ggagttttta tgggagggta 157200
   tattgttttt ttagtatttg taggtattta ataatttgaa attttataaa ttttattttt 157260
   ttgggttttt agggaggttt tattatataa gtatgatatg aaatttttgg ttattgatga 157320
   ttaatttaat ttttattttt ttttgttttg taggaggtgg atgggaaagg ttgtaagttt 157380
   tagtttttta attataaggt tgggtttttt ggtaattagt ttttattttt aggttgttta 157440
   ggagttttta gttattagtt gtttttttag taaataaaaa gatatttatt attttggaga 157500
   ttttaagagt tttaggagtt atatgttaaa taaagttggg ggatagagat tagatagata 157560
   gatagataga tagatagata gatagataga tagatagata gatagatata ttgatttttg 157620
   agatagagtt tttttttgtt atttaggttg gagtgtagta gtgtaatttt ggtttattat 157680
   aatgtttgtt tttttgggtt taaattattt ttttatttta attttttaag tagttggaat 157740
   tgtaggtata tattgttatg ttaggataat tttttgtatt tttggtagat atgaggtttt 157800
   attatgttgt ttaggttggt tttaaatttt tgagtttaag ggatttgttt gttttagttt 157860
   tttaaagtgt tgggattata aatatgagtt attgtattga gtttagttta aatatatttt 157920
   ttttatatta taatgttatt tttttgtgat ttttttaata gttaattatt ttttatagtt 157980
   aagaatattt tatattataa gttgttattt aaattgttgg tgtggttttt gttttttgat 158040
   tgggttttga ttaatgtgtt gaagtaagta taaggaagga agtaatgaag ataatagaga 158100
   aatttaagga aatggatatt agaaaagggg agtttaatgt tggtttttta aaaagattta 158160
   gattgataat tttttagtta ggttgttgat gagagggaga tggtgaatat aaagttataa 158220
   taaaaggaat gaagtgtttt ttttatggat tttattgata ttatagtgat aaggaatata 158280
   ggtatatttt attttattgt gttttataga tattgtattt tttataaatt gaaggtttgt 158340
   ggtagttttg tattaagtaa gtttgttggt attatttttt taataatatg ttaatattgt 158400
   gtttttgtgt aggagtttga tgatttttga aatagtttaa atttttttat tattattata 158460
   tttgttatgg tgatttgtga tttttttttt tttttttttt tttttgtttt tttttttttg 158520
   agatagagtt ttgttttgtt tggagtgtag tggtgtgatt ttggtttatt gtaagttttg 158580
   ttttttgggt ttatgttatt tttttgtttt agttttttga gtagttggga ttataggtat 158640
   ttattattat gtttggttat tttttttgta tttttagtag agatggggtt ttattgtgtt 158700
   agttaggatg gttttaattt tttgatttta tgatttgttt gttttggttt tttaaagtgt 158760
   tgggattata ggtgtgagtt attgtgtttg gttttttttt tttttttttg agatggagtt 158820
   ttgttttgtt gtttaggttg gagtgtagtg gagtgtagtg gtgtgatttt ggtttattgt 158880
   aatttttatt ttttaggttt aagtgatttt tttgttttag tttttttagt agttgggatt 158940
   ataggtattt gttattatgt ttggttaatt ttggtatttt tagtagagat gaggttttat 159000
   tatgttggtt aggttggttt taaatttttg attttaggtg atttatttgt tttggttttt 159060
   taaagtgttg ggattatagg tatgggttat tgtgtttagt gattagtgat ttttaatgtt 159120
   attattgtag ttgtttttgg ggtatgatga gttatattta gataagatag tgaatttatt 159180
   taataaatgt tgtatgtttt gattgtttta ttgattggtt gttttttgtt tttttttttt 159240
   ttgtttaggt atttttattt tttgagattt aataatattg aaattaggtt aattaataat 159300
   tttataatgg tttttaagtg tttaagtgaa aggaagagtt atttgttttt tattttaaat 159360
   taaaagttag aaatgattaa gttgagtgag gaaggtgtgt tgaaagttaa gataggtaaa 159420
   agtgagattt tttatgttaa atagttagtt aagttgtgaa tgttaagaaa aagttattga 159480
   aagaaattaa aagtgtgatt ttagtgtata tatgaagaag aagaaagtaa aatagtttta 159540
   atgttgatat gaagaaagtt tgagtggttt ggatagaaga ttaaattagt tataatattt 159600
   ttttaaataa aagtttaatt tagggtaaag ttttaatttt ttttaatggt atgaaggtta 159660
   agagaagtga ggaagttgga gaaggaaagt ttgaggttag tagaggttag gttatgaagt 159720
   ttaaggaaag aagttatttt tataatgtaa aagtgtaagg tgaagtagta ggtgttgatg 159780
   gagaagtggt agtaagttat ttagaagatt tagtttagag tattgatgaa ggtggttata 159840
   ttaagtaata gaattttagt atagatgttt tattttttat tggaagaaga tgttatttag 159900
   gatttttata gttagagggg agaagttaat gtttgttttt aaagttttaa aggataggtt 159960
   gatttttatg agaggttaat gtagttgttg attgtaagtt aaagttaata tttattgatt 160020
   gttttaaaaa ttttagggtt tttaagaatt ttgttaaatt tattttgttt atgttttaga 160080
   aatggaataa taggttttat ttgatagtat atttgtttat agtatggttt attgaatatt 160140
   aaaaatttat tgaggtttgg tgtggtaatt tatgtttgta attttagtat ttttgaaggt 160200
   tggggtgggg ggattatttg aggttaggag tttgagatta gtttggttaa tatggtgaaa 160260
   ttgtattttt attaaaaata taaaaattat ttaggtatgg tgttatatgt ttgtaatttt 160320
   agttatttgg gaggttgagg taggagaatt gtttgagttt aggaggtgga ggttgtagtg 160380
   agttgagatt gtgttattgt attttagttt gggtaataga gggagatttt gttttaattt 160440
   gaaaaaaaat aaaaaaataa aaaagggtta agtatggtgg tttatagttg taattttagt 160500
   attttgggag gttaaggtag gtaaattatt tgagtttagg agtttaagat tagtttggtt 160560
   aatatggtgg tgaaatttta tttttattaa aaatataaaa attagttagg tatggtagtg 160620
   tatatttgta gttttagtta tttaggaggt tgaggtagga gaattatttg aatttgggag 160680
   gtggaggttg tggtgagttg agatagtgtt gttgtatttt agtttaggta ataaagtgag 160740
   attttgtttt aattaaaaaa taataataaa aaaaaaataa atagggtttg gtgtagtggt 160800
   ttatgtttgt agttttaatg ttttgagagg ttgaggtagg tggattattt gaggttagga 160860
   gtttgagatt agtttggtta atatggtgaa attttgtttt tataaaaaat ataaaaatta 160920
   gttaagtatg gtggtgtgtg tttgtaattt taattattta ggaggttgag gtataagaat 160980
   tatttgaatt tgggaggtag aggttataat gagttgagat tatgttattg tattttattt 161040
   tgggtgatat agtgagattt tattaaaaaa aaaaaaaaat tattgagatt tattgtttag 161100
   gaaaaaagaa tttttttaaa atattattgt ttattgataa tgtattttgt gatttaagag 161160
   ttttgatgga gatgtatgta aagatgaatg ttgtttttat gtttgttaat gtaatattta 161220
   ttttgtagtt tatggagaag ggataaattt gatttttaag ttttattatt taagaaatat 161280
   attttataaa gtagtatttg ttatagttag tgattttttt aatggatttg ggtaaagtaa 161340
   attgaaaatt ttttggaaag gattttttat tttagatgtt attaagaata tttgtgattt 161400
   ataagaagag gttgaaatat taatattaat aggagtttgg aagaagttga ttttaatttt 161460
   tatggatgag tggtttaaga ttttagtaga ggaagttatt gtagatgtgg tggaaatagt 161520
   agagaattag aattagaagt ggagtttgga gatgggattg gattgttgta attttatgat 161580
   taaatttgaa tagatgagga gttgtttttt atggatgagt agataaagtg gtttttggag 161640
   atgggattta ttttaggtga agatgttgtg aatattgttg aaatgataat aaaagattta 161700
   gaatattata ttaatttatt tgattaattt gtggttaggg ttgaaagaat tgattttaat 161760
   tttgaaggaa gttttattgt gggtaagatg ttattaaata taatgtgtgt tatagaaaaa 161820
   ttttttatga aagaaagagt taatttatgt taaaaatttt agttaagaaa ttgttggtta 161880
   ggtgtagtgg tttatgtttg taattttagt attttgggag gttaaggtag gtggattatt 161940
   tgaggttagg agtttgagat tagtttggtt aataaggtga attttgtttt ttattaaaaa 162000
   tataaaaatt agttaggtgt ggtggtaggt atttgtagtt ttagttattt gggaggttga 162060
   ggtaggagaa ttgtttgaat ttgggaggta gaggttgtag tgagttgaaa ttgtgttatt 162120
   gtattttagt ttgggtaata gagtaagatt ttgttttaaa aaaaaagaaa aagaaaaaag 162180
   aaataaagaa attgttgtta tagttatttt taatttttag taattattat ttggttagta 162240
   gttattaata ttgaggtaga ttttttatta agaaaaagat tatggtttgt tgaaggttta 162300
   gatgattatt agtatttttt agtaaaaaga tataatgatt ttatatattt taatagatta 162360
   tagtatagtg taaatataat tttttttttt tttttttgag atggagtttt gttttgttat 162420
   ttagtttgga gtatagtggt gtgattttag tttattgtaa tttttatttt ttaggtttga 162480
   gtaatttttt tattttagtt ttttaagtag ttgggattat aggtatgtgt tattatattt 162540
   agttaatttt tgtattttta atagagatga ggttttatta tgttggttag gttgttttta 162600
   aatttttaat tttaaatgat ttgtttattt tggtttttta aagggttagg attataggta 162660
   tgagttatta tgtttggttt aaatataatt tttatatgta ttgggaaatt aaaaagtttg 162720
   tgtgatgtgt tttattgtaa tatttgtttt attgttgagg tttggaagta aattttaggt 162780
   atgtttgtat tatgaataat tttaaatgag taaatttaat aatttatttg aaagggataa 162840
   atttttttaa agtataattt attaaaagta agataagggg aagtaaaaaa atttgaataa 162900
   ttttttttat atattaaatt gaatttgtga ttaaaaataa ataattattt tttttagtag 162960
   aaaattttag gtttagatgg tttttttggt ggattttatt aaatatttaa ggaagaaata 163020
   atattaattt tatgaaaata tagaaaataa agagaaggga atatttttaa attaatttta 163080
   tgaagttaaa atgaggttga taaagatatt attgaaataa attaataaat aaagaaaata 163140
   ttatattaat attttttatg aattaatatg taaaaatttt taaaaattgg gtaaattaaa 163200
   tttagtaata tataaaaagg atgatatttt ataagtaagt tggatttttt ttagaaatgt 163260
   aaggttggtt taatattaaa aaattaggtt tgtatagtgg tttatatttg taattttagt 163320
   attttgggag gtagaggtgg gagtattgtt taagtttaag aattgtagat tagtttgagt 163380
   aatataatga gattttattt gtataaaaaa ttttaaaaat taaaaattag tttggtatgg 163440
   tggtatgtgt ttatagtttt tgttatttag gaggttgagg tgggaggatt gtttaagttt 163500
   aggagtttag gttgtagtga gttatgatgg tattattgtg ttatagtttg ggtaagagtg 163560
   agattttgtt tttttaaaaa aaaaaaaaaa aaaaatgggg ggtaaaaatt aattattgta 163620
   atttatttta ttaaatgaat aaaagataaa ataatgatta tttttataga tgtagaaaaa 163680
   gtatttatta aaattatatt tatttatgtt aaaaaatttt agtaaattaa gaatagaagg 163740
   gaattttttt aatttgataa aggttatata tgataaattt aaagttaata ttataagttt 163800
   ggtataggta aggatgttta ttaattatta ttatttaata ttgaattggt agttttaatt 163860
   agtgtaataa agtaagaaaa aaaaataagg tattaagatt agaaagaagt aaaattatgt 163920
   atttaaattt taaaagattt ataaaattat tattagagta ataaatgatt ttggaaaaga 163980
   ttataggata taagatttat atataaaaat ttattgtatt tttatatatt agtagtaaat 164040
   aattaaaaat gaaatttaaa atttatttta gtttaaaata gtgttaaaag taaaattttt 164100
   aggaataaat ttaataaatg gtatgtaaga tttttttttt gaaaattata aaataatgta 164160
   attttgtaga gatttttttt tagtagagaa ataaaagaaa tataaaagga tttttaaaat 164220
   ggagatatat tatttatata aaagatttaa tattgttaaa atgttaattt ttttttaatt 164280
   gatttataga tttaatataa ttttaattaa aatttatatt gtatttttgt agatattaat 164340
   aagttatttt ttaaatttat aaggaaattt aaaggattta gaatatttaa agtaatattt 164400
   tagatatttg agagatttta tattaagatt tattgtaaag ttatattaat taagatgtgg 164460
   tattagtaaa ggaatggata aaatattaag aaaatataat agtgttttaa tatagggttt 164520
   atatgtatat agtatatata ttaatatagg attatatata tagttaatat aggatttata 164580
   tgtatatagt atatatatta atataggatt atatatatag ttaatatagg atttatatgt 164640
   atatatgtat atatattaat ataggattat atatatagtt aatatggggt ttatatgtat 164700
   atatgtatat atattaatat aggattatat atatatatat agttaatata gggtttatat 164760
   gtatatatgt atatatatta atataggatt atatatatag ttaatatagg gtttatatgt 164820
   atatatgtat atatattaat ataggattat atatatagtt aatatagggt ttatatgtat 164880
   atatgtatat atattaatat aggattatat atatagttaa tatagggttt atatgtatat 164940
   atgtatatat attaatatag gattatatat atagttaata tggggtttat atgtatatat 165000
   gtatatatat taatatagga ttatatatat agttaatata gggtttatat gtatatatgt 165060
   atatatatta atataggatt atatatatag ttaatatagg gtttatatgt atatatgtat 165120
   atatattaat ataagattat atatatagtt aatatggggt ttatatgtat atatgtatat 165180
   atattaatat aggattatat atatagttaa tatggggttt atatgtatat atgtatatat 165240
   attaatatag gattatatat atagttaata tggggtttat atgtatatat gtatatatat 165300
   taatatagga ttatatatat agttaatatg gggtttatat gtatatatgt atatatatta 165360
   atataggatt atatatatag ttaatatagg gtttatatgt ataaatgtat atatattaat 165420
   ataggattat atatatagtt aatatagggt ttatatgtat atatgtatat atattaatat 165480
   aggattatat atatagttaa tatagggttg atatgtatat attaatatag gattatatat 165540
   atagttaata tagggttaat atgtatatat gtatatatat taatatagga ttatatatat 165600
   agttaatata gggtttatat gtatatatgt atatatatta atatagggtt atatatatag 165660
   ttaatgattt ttagtaaagg tgttaggtaa tttaatagag taaggtatgt ttttttaata 165720
   aatggtgtta gaattggata tgagtataga aaaataagaa ttaattttga ttttaaatta 165780
   aatataaaaa ttgatttaaa atggattata taatattttg atggttataa taggttaaaa 165840
   aatgtaaaaa tttaaaaaat taaaaaataa aaggaattat agaatatata attataaaat 165900
   ttttagaaaa taagagaatt ttttttgatt ttagtgtagg taaagatttt ttttataggt 165960
   tataaaaatt ataaattgaa attggaaaaa gttgataaat tggattttat gaaaataaaa 166020
   tatttttgtt tattaaataa tattattaag aaaattaaaa ggaaaggagt aatgtggaaa 166080
   aaaaaattta tgatatatat atttgataaa ggatttatat ttagaatata taaaaaatat 166140
   atgaaataat attttttttt tttttttttt ttgagataga gttttgtttt gttatttagg 166200
   ttggagtgta gtggtgtaat tttggtttat tgtaagtttt gttttttagg tttatattat 166260
   ttttttgttt tggttttttg attatttggg attataggtg tttgttatta tgtttggtta 166320
   attttttgta tttttagtag agatggggtt ttattgtgtt agttaggatg gttttgattt 166380
   tttgattttg tgatttgttt gttttggttt tttaaagtgt tgggattata ggtgtgagtt 166440
   attgtgtttg gttaattttt tttttaaatg agatttatgg ggaaatagtt aaatgaagaa 166500
   ataataaagt agtaataaaa taaaatttaa gttttatttt attgtgtagt tgtaggataa 166560
   agagtagaag tttgtagttg gggaataagg tgtttatttt tttaagtatt ataaaatgaa 166620
   ggttttagaa attatttgat tttgaaataa tataatttta aaaagaagta agatgtggat 166680
   agataatttt ataaaggaaa atatgtaaat gattaataag gattttggaa aattgtgtga 166740
   ttttataagt tattaagaaa atataaaata aaagttttaa aaaaaaatga agtttggaat 166800
   aattgaataa atatataaaa tgataataaa atgaattttg attttttatt ttatattata 166860
   tgtaaaagtt aatttataaa agttatataa ttttaagttt tttaaaggaa aatataaata 166920
   tttttgtaat ttggtagggt aggtaaaggt tttttataaa gaatatagag agagaattat 166980
   aaagaaagga gtttgttaaa ttagatttta taaaaattaa agtgatatat ttttgtttat 167040
   tggaaaatat tattaagaaa atgaataggt tgggtatggt ggtttatgtt tgtaaaattg 167100
   gtttttgggg aggtagaggt aggaggatag tttgagttta ggagttttag atttgtttgg 167160
   gtaatatagt gagattttgt ttttgaaaaa ggaaagaaaa tgaataggta agttatagat 167220
   tggaataaaa tatttgtaaa atatgtattt gataaaagat tagtatttag aagaaaaagt 167280
   taagtaattt aattttaaaa tgagtaatag atttgaaaag atattttata aaggaaaaat 167340
   atatgaagag ttaattatga aaatgtgttt aattgtatta gttattaggt taaagtaaat 167400
   taaagttata atgagatgat attattatat gtttattaga atgattaaga ttttatagag 167460
   tgatagtatt aaatgttggt taggatgtgg agtaattaga atttgtattt attgttggtg 167520
   ggagtgtgaa atggaagggt tattttggga aaagatttgg tagtttttta taaaattaaa 167580
   tgtatattta ttttatattt attttaggtg tttttataga ataaatgaat gtttatatgt 167640
   atagaaaaat ttgtataaga agaattagtg taataggttt atttataaga gtttttagat 167700
   ggaaatagtt aggggttttt taataagaga atgattaaaa atttatagaa tgtttatata 167760
   gtggatgtta tttagtaata aaatagaata aattgttggt atttgtagta atatgggtag 167820
   ttttaagaaa tatttaaatt ggtaaaatta ttttgtggtg gaaaaaaagt ataattgtgg 167880
   ttgttttggg gaaggtgggg attgtgatgt atttggaggg gtatgaggaa tttattgggg 167940
   tgatagtgat gttttatgtt ttggttaggt tgggatatat gtggatattt ttgttaaaat 168000
   ttaaagaatg gaatgtttta tatttgtgta ttttagggta tatatatttt attttaaaga 168060
   aaaataaaaa taattataga taaatgttgg attttagtta atgaaatgta aattgaggtg 168120
   ttgataggat gttgtgtgtt gatttttgta gtttatttta agtgtattaa aattagtgtg 168180
   gatgttggtg gttagaggga tttgtggtga agtaaatagt aaaatggttg ttgtggaatt 168240
   taggtggtag gtgtatgggt gtttatttta gaatttttta tggttttttt tttgtggttt 168300
   tgttttgttt tgtttttgag atagggtttt gttttgttgt ttaggttgga gtgtattggt 168360
   ataattttag tttattgtag ttttaatttt ttaggtttaa gtaatttttt tattttaatt 168420
   ttttgagtag ttgggatttt aagtttgtat tattatgttt gattaatttt ttgtattttt 168480
   tatagagatg ggattttatt atattgtttg ggttggtttt gaatttttgg gtttaagtga 168540
   tttttttatt ttaatttttt aaagtgttgg gattatagtt atgagttatt atatttagtt 168600
   tttttttatt ttttttatgt ttgaaaagtt ttatggtaaa aagttggaaa aatgtaaaaa 168660
   agattgaaat ttattaatta aaaataggaa aaataaaata aaagagatgg taggagatta 168720
   tttttttttt ttttaattta gtttttaatt tttttttttt ttgtttagtt aggataggaa 168780
   ttttaagggt ttgggttttt tgtgtttgtg tggggaagga ggtatttttg tgtttgtgtg 168840
   ggggttggtt atttgttttt tggtgtattt tttgtatttt attttttttt gtttgtagtg 168900
   agtagttata gttggtggaa attatattta ggtagttttt gtttataaat ttagtgaaaa 168960
   tgtaaatttt gtaggagttg gatttttgag agtttgtggt gttatttgtt ggaggaaagg 169020
   ttttttgtat ttgagttttt tttttttttt ttgttgttat ttgttttttt gtttaggttg 169080
   ggtgggtttt ttttttgtaa ttttggggta ttggtgtatg ggaaatgttt agtgttttat 169140
   aatttttttt tatttatatt ttaatattta gtttttagtg gatattgttt ttgtttgata 169200
   aatatattat ataggtatag tttaagatta tttattttag gaggagtata agttttagtg 169260
   tttattattt tagttttttt tttttttttt ttgatgtagt gatataggtt taggatattg 169320
   aagatttttg attttataaa tatgttgaag ttaattattt tgaagttgta gtttttatta 169380
   ttattattat tttttatttt ttgtaaagat aaagttttgt tatgttgttt agtttggttt 169440
   ggaatttttg gatttaattg atttttttgt tttggttttt taaagttttg ggattgtagg 169500
   tgtgagttat tgtatttagt ttgaagttgt agttttttaa ttattgttaa tagtaatatt 169560
   tatttttttg tgagtagtag ggtttgttgg tatggttgat agttttgtat tattgtattg 169620
   aatttgttgt ttgggatttt aggtgagttt tgatttttag ttggagggat aagttgtgtg 169680
   gggttgggta gggtgaattg tgggagatta agtttagatt gagggttata agttatatga 169740
   gtattggggt taggtaggga tagtgaagga gatagtgtaa ttttgaggaa agataatgtt 169800
   atagaatgga gaggtttagg tttggggttt ttttttgttg ttggataggt atgtttgtgt 169860
   ttttttttta gtttttgtga tggttaagtt atgtgttttt tgagaggtaa gtgaggtttt 169920
   tgatagtagg ttttaggttt taattaaatt gtttgttttg tagggtgagt tgtataggtg 169980
   tttttatttt tgattttttt gaatttttgt tgtttttaag ttttatttat tttttttagg 170040
   tgaatagaaa tattgtttag gggagaatgg aggaagaatt taggaaaggt tgtttgagag 170100
   tttggttgag tttatgttat tgttaagagt agtgttaaaa tttaggagtt ggtgagatta 170160
   tggtttaggg tttatattaa tatgaggggg tgttagtttt atgattaatg agtttttgtt 170220
   ttattatttt ttgttattta tattttgagg gttatagtta gtatgtgaat tggagttggt 170280
   ttttggtatt ttttttttta tattagtttt ataatgtgag gtgggggttt gtttttagag 170340
   gtatttggag tagtaggtgt tttaagaggg ggaagttttt ttgaagtttt tttttattta 170400
   tggtatagga tgtagagggg atagagtatt aggaatattt agtgattata ggggtttgtt 170460
   ttgaaggaga ggaggtagga ggtagggaga gtggttggga ggttagtttt ggagtgttta 170520
   ttttttattt tgagggagag gaaggaagta gaaggtgagt atagggagtt ttagttgggg 170580
   atggagtggg ggttgtagtt tgattgtgtt gtttggagtg ggggtatgga ttgtagtttt 170640
   tatttatttg tttggttagt ttgaagttat tttaagtttg gagattttat ttgttttgtt 170700
   tatttttttt agggatggtg tttaaatttt gaagttttgg ttggtgttgt aaatatggga 170760
   gggaggtttt gttttatttt agttttaaga gttagttaag aatgttttgg atatttgaat 170820
   tttgttttga gttagaggtt gggaagaaat ttgaaggatg gtgggatggt ggatagtagt 170880
   ttttaggtat taggtattag ggtttttgtt tggggatggg ggtattagta ggtgttaagt 170940
   gtattatatt agggtttatt atgtttttgt ttagagattt agtattttat gaaataggaa 171000
   ggaatgaggg atgagtgtgt tttttttttt aatattttag tttttgtttt gaggttgtta 171060
   ggtatttaat ttttttagga tttaaattat tttggtttta tttatggtta agtgtattaa 171120
   attatagtta ggattattgg gtttagatat taaaagtgtt ttttttaaag ttggtagtta 171180
   ttagggtagt atagggtagt gtaaggttgg gtagggttgg ttatttgtta gtatttagtg 171240
   gtttttgttg gatgttgatg ggggtagtta ggtaggtggg tttatttagt aattaaagag 171300
   attttttttt tttaattttt tttttaaaga gattttgatt tttttttttt agttgtaggg 171360
   tttgtagtag tttttttttt agtaggtttt tttgtttgtt tttggttatt tttagtagtt 171420
   atagggtatt gaggttttat tatagtgaga ataaaattaa gtttttatta gggtttttaa 171480
   ggtttggtat gatttggttt tgtttgttgt attggtgtta ttatggtttg tttttgaaaa 171540
   tatattttag ttatttttat tttttttagg ttttagtttt gggtttttat atttgttgtt 171600
   ttttttgtta ggttgttttt tttttggatt ttggtttggt tgggtttttt tggttattat 171660
   agttttagtt tagtgttatt atttagaaag ggtttttatt aatttttttg ttatatatat 171720
   agatatgtat ttttagtaat ttgtttttag tttttttata gtttttattt aaaattattt 171780
   atggatttgt ttatttgtgg attgtttttt agttttaatt gttaagtaat aagtgattag 171840
   tgattgtgtt tttgttgttt tttattatag gttaagtatt taagagaggg gttgtatata 171900
   gtaggtttta ataaatattt attagatggg ttaggtatgg tggtttatgt ttgtaatttt 171960
   agtattttgg gaggttaagt tgggtggatt atttgaggtt aggagattgg gattagtttg 172020
   attaatatgg tgaaatttta tttttattaa aaatataaaa attaattagg tatggtggtg 172080
   gatgtttgtt attttagtta tttgggaggt tgaggtagga gaattgtttg aatttgggag 172140
   gtggaggttg tagtgaggtg agatggtgtt attgtatttt agtttgggtg atggagtgag 172200
   attttgtatt aaataataat aataaaatat ttgttagaag aattaaagta gtttgtttgt 172260
   gtttaatagg aatttaagag tataagatga atttaattaa aggatatata tagtaggtat 172320
   tttatagttg atatttgttg gtggagggag attaggaaag tggggagagg tagtgtttgg 172380
   gtatggggat attttgggta tggggatgtt ttggatatag ggatatattg ggtatgggga 172440
   tattttgggt ataggatgtt ttgggaatgg ggatattttg ggtatgggga tattttgggt 172500
   atggggatgt tttggatata gggatatttt gggtatgggg aaattttggg tatggggatg 172560
   ttttggatat agggatatat taggtatggg gatattttgg gtatggggat gttttggata 172620
   tagggatata ttgggtatgg ggaaattttg agtattggga tgttttaggt atggggatgt 172680
   tttttgttat ttgaaatttt gtagttttat tatatttgtt tgttttagta aattggttta 172740
   aagagtattt ttaagttttt atggtaaagt tttagagtta gtgttttttt gttgattttg 172800
   agggtgtgtt gtttatttag tgtttaagtt agagtttttt tatttagaaa aaaaaagagg 172860
   agggtggttt tatggatgga ggtatgaggg tgtttatagg tagagggagt ttttgggttg 172920
   agatttgtgt tattttttgt tttaatttta aggtgtagtt tggagtttaa attagttttt 172980
   aaatgtgggt tgttggttaa gaggaatttt tttatttatg attttttttt atatttagtt 173040
   tttattgttt agagtatgtt agttttgttt gttttggttt ttaggatttt tttagggatt 173100
   tttaggtttt aagttagggt gggtttgaat taaggtagtt ttagaattta tgttaatgtt 173160
   tttgttagtg ggagattttg gggagtattg tgtgggatgt atggggttag gtgggggtat 173220
   atagtgagtg gttttagaag ttttggttgt ttggagtggg ggggtgttta gagtaagaga 173280
   tgggttagga agttggagtg agtaaggagg ttttagtttt tggtgggagt aggttgggta 173340
   aaaataaatt aatttgaggg tagtagttag tgagattatt ataggagggg tggtggggtt 173400
   ggaggggttt taggagttta ggagagggag gtattgttag aggtaaggtt ggtagatgag 173460
   agttagtgtt ggtaggatta atttgttata gtagttatgg tttatttttt tttttttttt 173520
   ttatatatgt ttttggggtt tatggaattt attagtttat tataaaggat attataaagg 173580
   atatagggga agagatgtgt agggtaaggt atgggggagg gatgtagagt ttttatgtgt 173640
   tttttaggtg ttttattttt taggagtttt tatatgttta tttatttaga agtttttgga 173700
   attttatttt tttgagtgtt tatggaggtt ttattatgaa ggtatgattg atttaattat 173760
   tggttgttga tgatttgttt aatttttagt tttttttttt tatttggagg ttgtggggtg 173820
   gtgttgaatt ttgtgatttt atttggtttt ttttgggaat tattttgaag ttgttagttg 173880
   gtttgttaat tatgagtatt taaaaagata ttattttgga gattttaagg attttaggag 173940
   ttgtgtgtta ggaaatggag atgaaaatta agtgtatatt ttataatatg atagttagtt 174000
   ttttttaatt ttagttttat tttttgttgt gttgttggtt ttagtttttt gtgttttagg 174060
   gtttttgaat atattgttgg ttgtttgtag tgattgtttt tgtttgttgg gtttgagttt 174120
   ggtttatttt ttttattttt ttgtgtttag tttgaaggtg tttttttgag gtagtttttt 174180
   tgatttggtt ttatgaattg taggttttgt tttgttattg gttttttttg ttatatttgt 174240
   tattttttgt gttttatgtt tattgatgtg gttttgtatt tgtgtagttt tatgagggta 174300
   aggattgtgt ttggttttgt tttttatggg attttagtgt taagtttata gggagtattt 174360
   aatgttgaat gaatgaatga gtgattggta agggaggttt attttgttat gttgggggtt 174420
   aaaggttgtt gggtggatta tatgggtgag gttgggtggg gagggaagta gagatgattt 174480
   tttggaagaa ggggtaggag gtggggaggt gggtgtgggg tgttgtataa ggatggtaag 174540
   tgtttttagg ggtgataagt taatttagag tattaggtag tagaaaaagg ttgatatttt 174600
   tatgttttat ttttatttag ggagaggtta ggtttttggg aaagttagtt ttttgttgtt 174660
   aggagagttt taagggaagt tttattagtt gttgagttgg ttatagtgga aatttatgtg 174720
   gtgggtggga ggggtttgag gtataggggt ttgggtggtg gttttaggat ggagatttgt 174780
   gtttagatta tgaggggtgg ttttagggat gattattttt aaataggaag tagataaagg 174840
   tatagatggt agaagtggag gtagagaatg ggttgtaggg gtttttttag agggagagtt 174900
   gagtaagggg ataggttgga aatatttagt ttttatggtt tgaagtatag atagttggtt 174960
   ttatatgtag gttgggtagt tatattattt agaataaggg gtatgtttgt agggtaggtt 175020
   ttaggatgtt ttgggttggt tttgttatta gtttagaaag atggtttagg tttttttggt 175080
   agggagtatg tttagttagg ttttgggtag gagttgagat tttttggttg gtttgagagg 175140
   ggatgattga gtgtaggttt agtttgggat ttttttattt ttttttgtta tttagtggaa 175200
   ttagttgggt gtgagaaaag aagagaagtg aattaagagt ttagagtatg aggttggatt 175260
   agtttttata ttatatgtaa aaattaattt aatttgtatt aaagatttaa atttaagagt 175320
   taaaattata aaatttttag aagaaaatat aggggaaaag ttttttgatg ttgtatttgg 175380
   taatgatttt ttggatatgg tgttaaaagt ataggtaata gaagaataaa tagataaagt 175440
   ggaatgttat agaagtttaa aatatttgta tattagtttg ggtgtggtga tttatgtttg 175500
   taattttggt attttgggag gttgagatgg gtggattatt tgaggttagg agtttgagat 175560
   taatttagtt aatatagtga aattttgttt ttattaaaaa tataaaaatt agttgggtgt 175620
   ggtggtgggt gtttgtaatt ttaattattt aggaggttga ggtaggaaaa ttgttggaat 175680
   tagggaggtg gaggttgtag tgagttaaga ttatgttatt gtattttagt ttgggtaata 175740
   gagttagatt ttattttaaa aaaaaaaaaa aaaattgtat attaaagggt attaataatg 175800
   tggggagaag gtaatttatg gaatggtagt aaattatagg ttagatatgg gattagtatt 175860
   tggatattaa tttatagatt ttttaatatt tagtaataat aattatttaa attaaatata 175920
   ggtaaaggat ttggatagat atttttgtaa gaatatgtgt aaatgagtaa taaatatgaa 175980
   aagatgttta atattattaa ttattgggga attgtaaatt aaaattataa ggagagattt 176040
   tttataatta tgagttttgg gtaggtagga agatgtttgg tggggagttg ttggttttat 176100
   gttgttttta gaatttttgt aggggttaag tttgattttg atattttatt aggagtatag 176160
   aaatgagtgg aaaaggttaa aggttatttt aaagggttag aggggtttag taaggagttt 176220
   gggatatgga attatgttgt gtatttagat atatggggat aataggggga tgaaaggtgg 176280
   tttttaaaat ttatatttat tttagagttt atgaatatgg tttatttgga aaaagggttt 176340
   ttgtagatgt tatgaagtta aggattttga gatgggatta ttttgggtta tttagtgggt 176400
   tttaaatttg agaaagattg tttgtatgag agatagatag ggggagattt agggtataga 176460
   aggaggtttt gtgataatgg aggtagaggt tgagtggtgt ggttatgagt taaggaatgt 176520
   taagggtttt gggagttgtt agaagttgtt agaggtgaga agtaggtttt tttttggagt 176580
   ttttggaaag aattaggttt gttgatgttg tgattttgga tttttggttt ttagaatggt 176640
   gagagaagaa atgtttggtg ttttgagtta tttagtttgt ggtatttggt gatggtagtt 176700
   ttaggaaatt attataggga ttagagtttt tatggggttg ggtaggaagt gggtgtagag 176760
   tgtgttaggt taggtttttt attttggaga tggaaaagta gagttggaga ggttgagatt 176820
   ggataggtat tgagtgttta ggggtaagaa tttttagggg atggtagagg ttattgggga 176880
   tagtatagtg agggatagga gggaggggtg agggttgagg tattgttttt taggaggttt 176940
   tggttttttg tttattgatg atttaggaat tttgtaagag gagttgggtt ttattttgtg 177000
   ttgtattgta ttagatggat tttggtgggg atagatgttt agtgatagtg tggttaagtt 177060
   ttttttagga ttttgggttt gtatttatga ttatgtatag atggttattt tttttgtttg 177120
   tagttttttt ttgtggtttg tttttagttt tttagtttaa tagagttttt tagtggaaag 177180
   tttttttttt ggagattttt tttttgttga aatataagtt tttggaataa gataaaattg 177240
   tttaatttgt aaaagttata aagttgtttt agaataaaaa aaaagttttg atttttggtt 177300
   ggaaggagtg gttttttatg ttttagtggt tagttattat tttatgattt atgttggttt 177360
   tgttgataaa ttttttagta tttttttagg ggaaggttat agtttttagg gagggtggga 177420
   gagtatttag gtttttgttt ataaattaga ttggagttag atattgttga gggagggata 177480
   agagggtagg ttttggggtt tttaagtttt ttttggggtt ggtagattat tgtggattta 177540
   ttagggtagg attatttttt tttataaatt ttttttgggt agttgtaggg tagtttgagt 177600
   tggatatgtt ttatatgtta gtttagtggg gtatgaggta tttatagtta tgtttggaag 177660
   ttagtgggtt ggtatttgtt gggtaggttt ttgttatata ggtatagtgt tttttttatg 177720
   gttttttttt aggttagagt atgtttgggg attagagtag tttttgtagt ggtttgtagt 177780
   ttgataatgt ggtgtgagtt atgttttgta gagaagaaga gttttttagt gagggaagag 177840
   gtgtttataa aatttaggat atgtgtagaa atttgtagga ggtgggggtg ttgtttaggg 177900
   gttgtgttgt ttttattttt tattttgtag atgatggaat tgtggtttag agttgttaag 177960
   tggtttgttt aaggttattt agtagggata ggaaggaagt tattttttta gggattgtgt 178020
   gggagtttag tgataattaa agattagtgt taaagtttgt ttaggatgag aattttatta 178080
   agggaaatag ttttgggatt gttgtgttta tttggaaggt agttagttta taattgagtt 178140
   tttaaaaatg tatgtttttt tatatgaaaa tatatttgaa tgggattttg tagaaagtat 178200
   tttttattaa ggtatagttt gaggggaggt ttttgtttta tttttggttg tgttgttttt 178260
   agttaagggt agtagggatt agagtggttt ttggggtata gtattggttg tttttaaaga 178320
   gtgttttgtt tagtttatga aaagtttgtg ttttgtgagt ttgggagggt gttattattt 178380
   ttagtttttt agggagtatt atttttaaat ttatgttttt ttgtttagtt tttagatttt 178440
   ttgttggtta tttatttggg ttttggttag attatttata aagtgaaggt tgaatttgag 178500
   gttttttgag gtttttgtta gttaggtggg tggagtgatg tttttttggg gttggtggtt 178560
   taggaggtag gggatttatt gtggtttgtt taggttgttt ggtggggtgg ttattttgag 178620
   gatggtttta ttgtgtaagt ttgatgaagg ttttttttag tgttttttgt tttgtggttt 178680
   ttgttgggtg gagagtagga tgttgggttt tatatttatt tgttttagtg ggtttgagtt 178740
   tttgagttta aatagataag atgtagtttt attgttattt aagttttttg ttgttttttt 178800
   tggttttttt ttgaggtttg tttgttgtgt ttgattgggt gtgtttagtg gtataatata 178860
   tatttgtttt attagtttta atgtgataat taagatgtgg ttttgaggtt gggtgttgtg 178920
   gtttatgttt gtaattttag tattttggga ggttgaggtg ggtggattat gagattagga 178980
   gattgagatt attttggttg atatggtgaa attttatttt tattaaaaat ataaaaaaat 179040
   atggtgggtg tttatagttt tagttgtttg ggaggttgag gtaggagaat ggtatgaatt 179100
   agggaggtgg agtttgtagt gagttgagat tgtgttattg tattttagtt tgggtaatag 179160
   agttagattt tgttttaaag aaaaaaaaaa aaaaagatgt ggttttggga gtagtggtgg 179220
   aggggattat atgttttaga gaggagttat tgagatgggt tttttttttt taggtttttt 179280
   agggattttt taatatatat atagtggtaa ttaatagatt agggtttaag gtatgtaggg 179340
   gttgatattt ttagtgattt tttgttttat ggtttgtagg ttattttaga atatttaaag 179400
   tttggagttt ggtttagtta ttttatgttt tttagtgttg ggtttagagg gtggtatgag 179460
   tggtagaaat tgttattatt gttaattatt gatgatggtg ttggttatgt gagatggtag 179520
   ttatggtatt gtatgtgagt gaagggatta aggaggttgg gtttgggagg gagggtttgg 179580
   aaagttgggg tgaattaaag gtaggggagt tttttttttt tttatttgtt tgtttatgtt 179640
   ttgatgggtt tgtgtttgtg gtaagggggt tgttagtgtg aatggatata tgtatatata 179700
   tgtatatata tgtgtatata tatgttaggt ttttttgagt gttgggagtt ttttttttta 179760
   taattaattg ttaggaaggt atagaaatgt tattttaggt aggagattga ggtagttttg 179820
   aagatagatt ttggttggaa gtaaaaaata aattatatag tggataaatt aattattagt 179880
   taggtttaag tgttgttgtt ttagatttga gttagggggg taggtttttt atattttttt 179940
   aaggttttgg tgttaatagt tttagtattg atagtggtta tgatatgtaa gaatggtata 180000
   ttggaaaatg aaagggaagt agttaggaga gaggagagtt gagtttaaat aaaagttaga 180060
   tttaggagag tttagttttt gagatggagt ttattttatg agtttggaat tgttaaaaag 180120
   aaaagaaata tttttgtgaa aaatgagtaa aagtgttttt tgtatgttta tttttttagg 180180
   tataaataaa gttttgagtt tagggtagtg gggggtggtt gatggggagg gggttgtttg 180240
   tgtatttgag atttggtttt tttttatttt tatggatata tttagtagtg gtgggagttt 180300
   tggtgttttg tttagaggtg gttttttttt ttttattttt gttgtttttt tttttttagt 180360
   tttttttttt tttttttttt tttttaaaaa tattttttgt ttggtttaat ttttttaatt 180420
   tgttgtaatt atgaatgtat ttgaattatt atatttttat gtgttgatta tatgtttgtg 180480
   ttttaaattg aagtagtatt gtttttttaa aatgggagga aaaagggagt aaaggagaga 180540
   aatttaaaaa taggaggtag gagagagggt ttgaagtggg ttttgaggag tttggtagat 180600
   tattgggttt gtttgtgaga gttttagagt tttggttgtt tggtttggtt ggtggttgta 180660
   gaggttttga gtgtggtttt tggtagtttt ttttttttat tttttttagt ttttgttgtt 180720
   ttgaggtttg ttgttttttt ttaatattta tttggagatg tttaatttag atttttggaa 180780
   attaatttat agagttaaat ttttgatgat ttttaaagtt ttttattttt tttgttttgt 180840
   gatgttatga tttttttggg tttaagggtt taaaaagttt agtttttgtg tgtttgtttt 180900
   gttttttttt gttttgggtt ggttttagtt tttttttatt ttgtatttta atgtggttgg 180960
   gtaggaggaa tggttgtgtt ttaggttagt gtgttgagaa aaggttttgg agtatttgtt 181020
   taggaggaat tttgagaagt tatttttttt tgttttatta gtttgtagtg atagaggtag 181080
   ttgttgtttt tgtttaggta gagagggtga atgggatggg gattttttgg aagattttta 181140
   ttatatatgt atgagttagt gagtttttag ttgttttttg ggagggagtg agtttttttt 181200
   tttgggaatt tggggtttta ttgggggatt agtgttgttt tttattgttt atttggggtt 181260
   tttagaggtt gtttatttat ttggggggtt tttattagta tatggttagg gtttatgttg 181320
   aggttaaggg tttagtagag taggtggggg tttataatgt gggtttttgg ggtgggaatt 181380
   aggggtattt tttttgtttt taagttttat tttaagggtt tatttaaatt tgagatatgg 181440
   aggttggttt tgttggttta agtttgttta aagagaatgt gtgtttgtgt taaggttgag 181500
   aatttaggta atttttttga aaattttaga ttgttagttt ttgttgagaa agggaggatg 181560
   tgtggttgta ttatggaagt ttttttgttt ggttgtatag ggttggtata agatagtttt 181620
   gttttttaga taaaatattt tttagtttgt gttgtttttt aaggttttta ataaatttat 181680
   aatatttgta tttttttatt ttgatatatt ttgtaggttt ttgagtttat aatttttgtt 181740
   ttatagagtt aggtgagtta tttgtgatta ggtgaggttt atgtatagtt tttggttaga 181800
   tgatagagtt aaaggttttg aagtgttatt ttaggttata gttttgtttg ttttttgttt 181860
   gggataggat tttaaatgta atttttttgg tagaaaggat atttttttgg ggggtgggga 181920
   ggattatttt tagttttttg ggagatatta agtaattatt tttttttaaa gagtttggag 181980
   gatagtttta taggtagtta gatagtgagg gaggtgggga tagtatttta gttgaggtgg 182040
   gaggttgggg aggaagggag ggattatagt tatgagagaa ggttgaataa tggttttagg 182100
   gatagtatgt tttaattttt agatttgtaa atgggatttt gtttggaata agagtttttg 182160
   tatttgtgat ttagttgtag attttgttgt ggtgagagta ttttggatgg tttaggtggt 182220
   ttttaaaggt agttttggtg tttatataag atgggggtag agggagattt gatatagata 182280
   gggatggtta agtggagatg gggtttggag agatttgtag atgttggtgt tgaaggtaag 182340
   ggttatgtag ttataagtta agggatattt atagttatta gaggtttaaa ggggtaggtg 182400
   atgaaggtag tggtttttaa tttttttggt atttgggatt ggttttaggg aagataattt 182460
   ttttatagat tagggagtgg ggagatggtt ttgagatgat ttaagtgtat tatatttatt 182520
   attttatttt attttatttt attgagatag agttttgttt tgttatttag gttggagtat 182580
   agtggtatta ttttagttta ttgtaatttt tgttatttgg gtttaagtaa tttttttgtt 182640
   ttagtttttt aagaagttgg gattatagat gtttgttatt gtgtttggtt aatttttgta 182700
   tttttagtag aaatggggtt ttattatatt ggttaggttg attttgaatt tttgattttg 182760
   tgatttgtta gttttggttt tttaaagtgt tgggattata ggtataagtt attgtgtttg 182820
   gtttttattt tttatttttt gagatagagt tttattttgg gattataggt gtgagttatt 182880
   gtatttggtt ataagtatgt tgtattgatt gtgtatttta tttttattat tattataata 182940
   tataatgaga taattatata atttattata atgtagaatt agttggagtt ttgagtttat 183000
   ttttttgtaa ttagatggtt ttatttggtg gtgatgggag atggtgatag attattaggt 183060
   attagatttt tataagaagt atgtaattta gattttttat atgtgttgtt tataatatgg 183120
   tttatttttt tataagaatt taatgttatg gttgattaga taggaggtag agtttgggta 183180
   gtaatgtgag taatggggag tgattgtaaa tatagatgaa gttttgtttg tttgttagtt 183240
   atttattttt tgttgtgtag gttagttttt aataggatat atggggttgg gaatttttgt 183300
   tttagagttt ttagagggtt atgtttagtg gatattttga ttttagtttg gtgattttga 183360
   ttttggatgt gtggtttgta gagttgtgag aggatggttg ggtgttgttg tgatttagtt 183420
   agtttgtggt tatttgttat agtagttgta ggaaattgat gtagtgttat agatggagtt 183480
   agggtttagt ttattggagt tgttttttgt tattttattt ttttttaaaa tattgtgtta 183540
   agttaattat taataattat tgattttgat gggtaggaat tgtagaggtt ttttggtttg 183600
   tttttttaat ttttttttta attttgaaat aatatttttt gttggttttt ttgattataa 183660
   atgtaatatt tttttttgta ttgagtttag aaataagaga aaaagtaaaa agtagaaatt 183720
   ttagttgggt aaggtggtat gtgtttgtaa ttttagtatt ttgggaggtt gtggtggaag 183780
   gattgtttga agttaggagt ttgaggttgt tgtgagttat aattgtatta ttgtattttg 183840
   gttgggtaaa aagagtgaga ttttgtttta aattaaaaaa aaaaaaaaag aagaggaaga 183900
   aaatttatta attttatttg agatttttta tatgaatttt tgttgtgggg tagggaagtt 183960
   taggaaggtt ttatgaggtt ttgatttagt ttggttggtg gtgggaattt gggaatagtt 184020
   ttgagtgagg ggttgggtta tgattgagtt tatttattta gttatttttt gtatgttgtg 184080
   tatattatgg gtttgatggt tagggttgga gtatttgttg agtatagttt tgatttttag 184140
   ttttatttag tatggtttgg tttgaggttt attttttttt tgtgtttgaa aatttaaata 184200
   ttttggttta ggtgggggat tatttgaaat ggaaaatttg gtagtattga ttggttttga 184260
   agaggtagga gttagttatg agttggttgg gaggtgagtg tagaggtggt gaggtaaggt 184320
   tagtagaggg tgggggaggt gagggttaat tttttattgt gtatggtgta agtattaagg 184380
   gggtatttta ttaggttagt gtagatttag gtttttgtgg tggatttttt gtggtttttt 184440
   tttttagagg aatattgaag aggtttttgt ggttttgttt tagatttaga ggtatatttg 184500
   tatattaaat gtattgagta attttatagt gagggagttg ggtttggttt aatttatttt 184560
   tttttaaatt ggattaaagg ttgttgtttt aggaaggtag ggtttttagg tttatggttt 184620
   tgggaaattt tggttgtgtt agtttatgag tttagttttt agttatttta gtttgtttat 184680
   attttggagt gggtattgtt atttaaaagt tttttttaga tagtttttag ggtggtattt 184740
   ttgtttttta ttttttatag atttttggtt atttttttgt gatattagta ttattggttt 184800
   atagtttttt ttaattgtaa agtggggttt agtggtatag gttgggtttg gtagggtggg 184860
   aaggaagagt gaaggttatt ttgtttttaa tagtattggt tgggttttgg aaaggttgga 184920
   atttatagat aggaaggaag tgttatattt atttaatttg ttttgtgtta gttagggggt 184980
   attttgtaga aagaagttag gtggttgtgg ttgggtgtag tggtttatgt ttgtaatttt 185040
   agtattttgg gaggttgagg taggtggatt ataaggttag gggttttaga ttagtttgat 185100
   taatatggtg aaattttgtt tttattaaaa atataaaaat tagttgggta tggtggtgta 185160
   tgtttgtaat tttagttatt tagaaggttg aggtaggaga attgtttgaa tttgggaggt 185220
   ggaggttgta gtgagtttag attatgttat tgtattttag tttgggtgat agtgtgagat 185280
   tttattttaa aaaaaaaaaa aagaagttag gtggttattt tttagttttt ggtttggttt 185340
   aggaattgtt ttttttttaa gatgttatta aatgtttttt ttattaggaa gttttttttg 185400
   gttgtttttt ttatttttgt ttttgtttta gaatatggtt tggtttttat ggataatttt 185460
   gatattagag tttaatagag attttttttt ttatggaaat ttatatttta aattgaatat 185520
   gtgtgtagga taagtttttg tttttatgtg aaataaaagt gaaaggtgtg gattatttgt 185580
   ttttttttat ttttttttat aataatttgg gttttatagg ggtttagtag gagttaggtt 185640
   tagtttagtt gttttaggtg tattattttg ggtattagtg tttttgtttt tttttatgaa 185700
   gtatagtttt attttttggt atttagtggg gaggggatat atattaggta ttttttaggg 185760
   tggattgtgt tttttatatt aggttaggag gttttggggt tggtttagtt atttagaaaa 185820
   gggagtaggt ggtttaggtg atattgatgt gggtagtagg ggtttttggg aaagggtttt 185880
   tgtaggattg gttttatttt gttttaggag tatatattgg ggaagggtgt tgtgtggagg 185940
   tggagagaat gtatttttgg ttgaaagtat gttttttgat gttttgggtt tggatttgtt 186000
   gttatttttt atgttttttt atttaatata tgttttagta aattttttat gttgatgagt 186060
   gttaagttat agaggatgta taattttttg ttttaaaaag tgattttttt tgttgggtgt 186120
   ggtggtttat atttgtaatt ttaatatttt gggaggttaa agtgggagga ttatttgagg 186180
   ttaagagttt aagattagtt tgggtaatat agagaaattt tatttttata aaaaaaaaaa 186240
   aaaaaaaaaa aagtttaggt atagtggttt atgtttgtaa ttttagttat ttgggaggtt 186300
   gaggtaggag aattatttga atttgggagg tagagattgt agtgagttaa gattatgtta 186360
   ttgtatttta gtttgggtaa tagagtaaga ttttatttta aaaaaaaaaa aaattttttt 186420
   ttttgaaata gggttttatt ttttttgttt aggttggagg atagtgatgt gattataatt 186480
   tattgtagtt ttagtttttt gggtagttgg aattataggg ttgtattatt atgtttagtt 186540
   aattttttgt atttttagtt aagatggggt tttgttatgt tgtttaggtt ggtttggagt 186600
   atttgagttt aagtgatttt tttattttgg ttttaaaagt attgggatta taggtatggg 186660
   ttattatgtt tagtttgaaa atattttttt taagaattag ttagttatgg tggtgtataa 186720
   ttgtagtttt agttatttag gaggttaagg taggaggatt ttttgagttt ggtaggttga 186780
   ggttgtagtg agttataatt gtgttattgt attttagttt gggggataga gaaagatttt 186840
   gtttagaaag aaaaaagaaa aaaaaaagat ttttttattt attttttgtt tatttttaga 186900
   tttaattatt gaagtttgtt tttttgaatt aaaaatgaag gagttttgtt ttggattttt 186960
   ttttgttgat tttgttattg tatttgttag tagagttagt taagattttt taggttggtt 187020
   ttagtttggt ttgtttatag ttttttaagt aagatggatt tttaggttga ggttagttgt 187080
   attttttttt ggaatttttt tttatttttt attgtgtttt agattttggt tgtttttaaa 187140
   taaatataat ttagttattt ttgtttttgg ggttgttgta gggtttattg gtgtaaattg 187200
   tttgggtagg tttgttgttt taggagtttt tttaggttag tgtggaagga aattaagata 187260
   atggggagaa gtagtttggt tgggtttaat tttattttta tatgtttgtg tttagttaat 187320
   tatttgggtt gtagtttgat aggagttgga gttggtgttt tatttttaga ggggtttttt 187380
   gattgttttt gtagagtgag ggaggtttgg gggtgtagag gttaagggtt tttgtagagt 187440
   ttgttttagg ttttttgagt ttaagttttt ttgtagttaa tggaagattt ttttgagagt 187500
   gggtgttttt tattttgttt gtttagtagg tgtttagtaa ttgtttattg gataaataat 187560
   gaatttgtgg atttaatgtg aagatgattt aaattagtgt ttttttatag aattttttgt 187620
   aatgatgtta atgttttgta tttgttgtag ttaatatggt agttatatgt ggtagtgaat 187680
   gtttgtaatg tggaagggtt attaaggtaa agagttttgt atttatgtta taatttaaat 187740
   ttaaatagtt ataaatgttt agtgtgtgtt ttgttatata gatttttttt gttatttttt 187800
   tttttttttt agagatattg tatttgttat taatttttta tttgggaggt aaatggagaa 187860
   atgttatttt ttagttaatg taaaagaagg ttaatatttg tattttaagt tagttttttg 187920
   ggtaattttt ttaagtattt tttattaagg ttagtttagt aatatttttt gattgtttgt 187980
   tatgtattag gaaagtattt tgttgtggtt ttggtttgag gttatggagg atatagaaat 188040
   aattaaggtt aagttgatag aaggggtgtg ataagatggt ttaaagttat tgagttagtt 188100
   aggaggttta ggagtatgga agttttgttt tttagaggat aagtggtaat ttatttaggg 188160
   ggggtttgag tatttagagt taggagtgta gaagtagggg gttttaggtt ggaaagtggt 188220
   attagggtta ggttgggtgg tagtatgaat ttatttttga ggttttgggg gtggaaatta 188280
   agggttttat gagtaagaag atttggttta ggtttaatgg aaatgattgg gtggagggat 188340
   tttattattt ggtttttagt atatagggag ggaagtggta attgtttttt aaatgttggt 188400
   tgtggagggg aaggagttat agtagggtag gggtggggag gatggtattt gttggtttaa 188460
   ggagggatga tggaggttta attaagatat gattgtgatt ggggtagttt gtttaagggt 188520
   tagtatgtag agggtgagtt gatgagattt tggtagtttg ttgagatttg ggggtgttgg 188580
   ggaggtagag gttaaggtta attttgagat tttttgtttg taaatttggg tgggtggtaa 188640
   ggttgatatt agagaaatag aggaagagtg ggtagttatg ggtgagagag gaagtaatga 188700
   attaggggtg tttgtaagag tttaggggta aggtttttgg gtagttaaga tgttggtggt 188760
   tttttttatg attagttttt tttatgtggt atatttttgt ttgtatggtt gggtatgggg 188820
   tatttgtttg tttgatatat tttgagtagt tattttgtgt attgaggtaa tgtggtatgg 188880
   tgttttgggg aaatagtgag gaggtattgt ataggtttta tttttttggt attttttgtt 188940
   ttttggggat ggtggtagtt aaataagtga tagttaattg ttaggagagt ttggttttga 189000
   tttattaggt ggagttaggt atttttgttg gaagaggtat gggaattagt ataggatgaa 189060
   gggaggtagt gagtattagt ttaggttttg tttttatagg gttttttttt tatatttatt 189120
   tgtgtaggtg ttttttgtga gggtagagat atttgtagtg tttgagggag attttttgtg 189180
   ggtggggttg tttatttata gtggttaggt gggaggtgta ttagtgtttt gtggttgtta 189240
   taatggatta tagttgtggg gtttgaaaaa atggaagtgg gttttttttt atgttggagg 189300
   ttagaggttt aaaattaagg tgttagtaag gttatatttt ttagggaggt tttggggtgg 189360
   ggtggggggg gggttttttt ttgttttttt ttagatgttt tttggtttgg ggttgtgtta 189420
   ttttattatt tgtttttgtt tttatgtggt tttttttggt gtgtttgtgg tttttttgtt 189480
   ttttttaagg atattagttg ttggatttag ggtttatttt aatttagaat gattttattt 189540
   taagattttt aattatttat atttgtaaag attttatttt taatttttag gttatatttt 189600
   gaggtttttg gtggatatga gtttgtgggg gatatggttt aatttaggat agaagggatg 189660
   atgggggtat tggggaaaag gttatatatt ttagaggtat tggaagatgg aagagtagga 189720
   tttagtgata tgggatgtta ggtagaggag ttggttttaa ggattttagt tggggtttga 189780
   gggttagagg gagggaagtt taggtattat tgagatgaaa tgttttgtgt attgtgggaa 189840
   atagggtaaa agtttgttgg atgtttagtt agagaggtgg ttgtttgagg tatagaaaat 189900
   gagtgaatat gggagagttt attagaaggt tatttgggag ggtgtggtgg taatatttag 189960
   aagggttgag aagtgtattt ttatagttta gttaaattta tttggggaaa tatatgtttt 190020
   tggtagtatt agtaatagag aagatttttg gtgatgttat tagtgggtta tgggatgggt 190080
   aaattttgtt tttttatata gaggaatatt tgttagttgt taaaataagt gaattaaggt 190140
   tttgtggatt tatatggata agtttttatg ttgttatgtt gtgggtatag gtagggtagt 190200
   tattgatttt agtgaggtat taaggatgtg atgttgatga tggtatttaa gaggagatag 190260
   atttaggttg tttagttata tgatgttgat gattgtatgt aagaggagat taggttggat 190320
   gtggtggttt atatttataa ttttagtatt ttgggaggtt aaggtaggtg gattatttga 190380
   ggttaggggt atgagattag tttgattaat atggtgaaat tttgtgttta ttaaaaatat 190440
   aaaaaaaatt agttagatat ggtggtgggt gtttgtaatt ttggttattg aggaggttga 190500
   ggtagaagaa ttgtttgaat ttaggaggta gaggttgtag tgagttgaga tggtgttatt 190560
   gtattttagt ttgggtgata gagtgagatt ttattttaaa aaaaaaaaaa aggagattta 190620
   ggttttttag atatattatt gtttagtata gatgtgtttg tttggggtta gattttaatt 190680
   tagaggggtg ggtattgtag taggagtggg gttaggatgg agaaggttat ttagggttgt 190740
   ggttatattt gtaatgtttt attttttaag tgggatggtg agtatatagg tgttttatta 190800
   ttttattttt tatatttttg ttttgtagtt ttaagaattt tatttttttt ttaaaaggaa 190860
   agaaagtgtg tgagttgatt aaagtgttga ttatggtgtt atttaagttt ttgttagaaa 190920
   tgttattttt ggtttagttt taaatataat tttgagaaga gtttttgttt ttttggggag 190980
   ggtaagagtt ttagatatag gagattggtt agatgtggat ttatagagaa gttgggttgg 191040
   gaggatggga tggtagatag ttgtttgggg agtgttaaat ggggaggttg gagatgttgg 191100
   ggtgtttagg aatgaatttt tgtttggatt agggttttag taaaagggag gtagggagaa 191160
   agaaagaagt taagaagatg aggaaagggt gttttgggtt agagtttata gaggtttata 191220
   gagggaggat tttgtgtgtt agtaggtagt tttatgtaaa tttagattat tagtttgtag 191280
   ttttgggatt tagtaaatta tgttttaggt ttttaagttg agttgtgatt gtggttgtta 191340
   agtgttgata gtatagaatt agttagtttg agtagaggga agtaattgtt gggggaggtt 191400
   gtgtttatgt agtttagggt gttaggtttt taagatgtaa tttttataag aattggggga 191460
   ttttttttat ttattttatt agaagttggt taatgagagg gaaggagatg gagaggtaga 191520
   ttagatgtgg agttggagaa aagtttttta gaaatatagt agtggttggg ttgtttagtg 191580
   gttagtgttt aatatgattg tgatgggtgg agtttttttt aattatagtg ttgaattatg 191640
   tagttgttta ggggttttta gttagagtat tatttttttt tttttatata tagttttatt 191700
   ttgtattgta aggggtttta tatgtattta atgtggatat ggtatttttt ttatttttat 191760
   tttttgtaaa aagttatttt ttttattgtt taggtttggt ggtgtatagt ttatgggtat 191820
   tgtatttatt tttggaaggt agatttagga ggtgtttgtg ttgtttttat agaagtaagt 191880
   ttaggattag aagtaggttt tgggtttaga attttgaggt tttaggtgtt agtgtggttt 191940
   ggaattgttt agttttatag ttattagtta tatgtagata taagtggaaa tttatttaaa 192000
   ataaataaat aattttatgt aagtaaatga gtgaataaag taaatgtaaa tatttatatt 192060
   ttgagtgtgt aatagattta ggtggttaag tggttattat ataggatggt ggagtgtaga 192120
   tgtatagaat agatttatta ttatagaagg ttttattaga tggagttggt ttagagtatg 192180
   gaggtggtgg ttttagatgg gtatatgttt ttgtttttgt aggtgttttg ttttggtagg 192240
   atggttgttt tattttgttt tgaaggtagt tttggtttat aggtgtttta tttgtgtgga 192300
   tttgattttg ggtttgtgtt ttttggtaat tttgtttttt ttagaatggt taatttatga 192360
   aggtatagtt tttgtttgtt tgttattatg atggtagtta ggggtttggt atttagttgg 192420
   tttttagtat atatgtatag gtgattatgt tatttttgtt ttttttagag ttttttgttt 192480
   tttaggggag gtaagtttat ttattgttgt gatttattat tttttttaaa tagaaaagtt 192540
   attagatggg tatttgtttt agtttggttg ttggaagtga tagttaagaa taatgtaggt 192600
   ttttagaaat agaggtatta gtatggttat attggggtta ggtaggggag attagggata 192660
   tttagtgggg agaagatgat gtgtagattt agttttgggg ggtaagggtt ggaggtagag 192720
   ttttgatttt tttgttttat ttttttgttt aggttgtatt tagtgttggt agagtttttt 192780
   taagtttata agttgtaatt tgtgttttgg atggttagtt ttagagtttt gatgggtgta 192840
   tttgtttttt ttttttattg gttagtgtgg aaaattttta aatatatgga aaaagtataa 192900
   ggagtagtat agtgattatt gttagaggtt tatttatttt tttttagata atattggatg 192960
   tatttgtttt attttggagt attttttatt ttgtaaggtt gtagggagtt tatagatatt 193020
   gaattaatat tgagaggttt atggttaaag ttatttgttt atggtatatt tgttttttta 193080
   gtattttaaa ttaaattatt tagttttatt tattttttgt tttttttttt gttttgtata 193140
   tatgggtttg ttttttgagt ttgggggtgg ttgtttttgt ttttataagg attattttta 193200
   gtagggtagt gttgataggt ttggaagtta tttgagttta aagttgttaa agattgtgtt 193260
   tttttgggaa atgggtaagt ggttttattt atttagggaa gttaggtggg gaggtaggtt 193320
   ttagattaga atgaagggat tttagtataa atatatttat gagttgggtg gggttagagt 193380
   ggagtgagtt atttatttgg gttattggag ttattaagtt tttggagttg gttattggag 193440
   gtttgtgtgt tttttaaaga tagagataaa gggtaaaggt tatataaggt tattggtgtt 193500
   ttttttttgg gtggatgatt aatattagga atgtttttgt ttttgttttt gttgtgagtt 193560
   ttagagttag aaatggggtt tatttgggga tagaggtttt atttttttag gtaattttgg 193620
   atgttggagg gatgatagtt attgttgttt ggttgttgga ggggttttgg gttaaatttt 193680
   tatatatatt tttttttaag ttttattatt tttgttttgt agataaggaa tttgagtttt 193740
   tatagagtgt tatttttttg tggttatata gtgaatgaga gttgtatata ggtgagttta 193800
   tgtttattag attttaagtt gggttgtatt taagtgtgtt tttgttggat ggagtttttt 193860
   ttttttttta tgtttatttg ttttatgaat tagggttttt ttatattagg gtttataatt 193920
   tatgggtagt tttgatttag ttttttttat ttttagggtt gggtgtagtt gggttggatt 193980
   tggggatgga gtgggatagg ttatttttat tttttgtttt aggagggaat aaaaatagaa 194040
   ttttagatta atgtagtttt ttgtggtatg tgtttttttt gtttatatat aggttttggt 194100
   ttggggttgt ttgttggaga ggtttatggt aggtaagttt taatttgagg agatttgttg 194160
   aggtgttaag ttaagtaaat ttgaaaggtt gttaggagga gtggttttgg gagtataggt 194220
   ggttatttta ttgttgtaga gtatggtatt ttggggatgt tttttgggta atgttatata 194280
   gttatttttt agagagtttt agggatatat aattagttta gttttattaa tagtgagata 194340
   agggattagg tagatttaga tatttttttg tatttgatta ttatgttggt tgttggttaa 194400
   agatgttagt ttttttttgg aattttttag aaaggttttt gtagtattta tttatttatt 194460
   tatttattta tttattttat ttatttattt atttatttat ttatttatgg attttattta 194520
   ttttattatt tttttatata tttatttatt tatttattta atttatttat ttagtttttt 194580
   atttatttat ttatttatta atttatttat ttgtttattt aatttattta tttattaatt 194640
   tatttattta ttatttattt atttatttat ttatttattt atttttttat ttatttattt 194700
   attttttttt ttatttattt tataatttta tttttaaatt gtgattagat ggaaatgggt 194760
   gtagttagtt tgtttttaaa ggtttagagt ggaggtagag tgaattgttt atttggtttt 194820
   tgtgtttagt agtttaggtt tgtatatatg ttttataatt ataggatatt agatttagta 194880
   attggtagta gaatgagtat agtgtatttt ttgtggaatt agtattgata gggtatttat 194940
   ttttattaag tttgagtttt tttggtgttg gagggaattt ttagatgggt tttaatttta 195000
   gttgaattta ttttttaggg aaagtaatat tgtgagaaat gtgtgtgata taaggtttta 195060
   aagagggtgt gggttgtgat ttttggagta attagggagg ttttatggag gagatgagga 195120
   taaagttgag ttttgaagga gggattggag gatgttgagt agggatattt atttttgttt 195180
   gtgagggttt gatggtaggt tgtgagttgt attttagttt tgatttattt aatttataat 195240
   tttagtaggt aggtgttttt gttttatttt atagaggagg aagtgaggtt tagggttggg 195300
   taaattgttt aaggttgtat agttgtggaa tttattttta gtatttttag tttgttttag 195360
   ttatgtttgt gattttattt agtatttttt ttttggtgtt tatttatttt gtttattgtt 195420
   aagtaataaa aattttatta ggaaaattta ggggtttttg gttagttttt ttagatagaa 195480
   aggtagattt tggtgtagat ttttaggaag taaggatgga agggttagta tttgtttttt 195540
   agaaatgata gtttttttaa gattaggaga gatggtttag agtgtagttt tggtgggttg 195600
   tttaggatta ggaggggaga ggagtgggga ggaggatgaa ttttattttt aaggttgttg 195660
   agtggtatat agtgagtttg gattttttag ttttagttga gtttattttt atgatggtta 195720
   tgagtatttt ttttatatat ttgattggaa gttgtattgt atataatgta tatggggtta 195780
   ttattttttg ttttatatat ttttatgtat atattattag tgtattattg ggatattttg 195840
   ttgtttgggg ttgagagtga attagaattt tgtttataaa tttagtatat tttattgtgg 195900
   aaataatggg gggtaaggag gggttgttta gtaaagaata ggatatatgt ttttgaggat 195960
   ggagttattt ttggttgaga tatttttgta ggggatgttg gtgataaagg ttgttttatt 196020
   tttttttttg ggttagatgg atattatttt tgttttttgt tttttagtat taggtaaaag 196080
   gaaaatagaa gaggttttat ttaggttaaa tattttaggg ttttttattt gtaggaaata 196140
   tgttggttta ttttttttaa atttagtatg ggggtggaga aatggtgggg aaaatagtat 196200
   ttgttttaga ttttttgggt ggagtttttg ttttatttat attgtttatt tttttgattt 196260
   tttaaaatgg gtatttggtg tttggtagtt tttggtatag agaagatgta aagtaaattg 196320
   tttataagaa gggggtgagt gtagtttgag tattttttgt ttttgaataa attgttaggt 196380
   ttagtgtagt tgttgttgtt attgttgttt ttaggaaggg atttttggtt atgagtggtt 196440
   ttagtttagt tttttggatt taattattta ggtttgtgtt tgagtttagt aaagaggtag 196500
   ttagaaaagt ttttaagata tttaatgatt ttgtggtttt agttgagaag tgagttattt 196560
   ttatgtgatt ttaaaataaa atatatttag aaggagagaa gataattgaa atttagttgt 196620
   ttggagttat tttagatgaa agtatttgag tttttgggag gtaaaggagg taggaaagtt 196680
   tggagttaga tgattagaat ttgaatgtag attttgttag gtttttgttg tgggttttgg 196740
   gagatgattt ttattttttg agttttagtt tttttgggga tagagatggt gtttatttat 196800
   aggttgtttt gagttaatga ggttatatgg tgtaagtatt agtatagtgt ttggtataaa 196860
   attaaaattt aataagtgat attattatta ttattattat tattattatt tttgagatgg 196920
   agttttgttt tgttatttag gttggattgt agtggtatga ttttagttta ttgtaatttt 196980
   tgttttttaa gtagttgaga ttataggtat ttgttattat gttttggtta atttttgtat 197040
   atttggtaga gatagggttt tattatgttg gttaggttgg ttttgaattg attttaggtg 197100
   atttgtttgt tttagttttt taaagtgttt ggattatagg tgttagttat tgttatttgg 197160
   ttgtgatatt attataatat taatattata atagttggtt aggtatggtg atttatgttt 197220
   gtaattttag taatttggga ggttgatgtg ggttagttat ttgaggttag tttgagatta 197280
   gtttgattaa tatagggaaa ttttattttt attaaaaata taaaaattag ttgggtgtgg 197340
   tggtatttgt ttgtaatttt agttatttgg gaggttaagg taggagaatt gtttgaattt 197400
   aggagatgga ggttgtagtg agttgagatt atattattgt attttagttt gggtgataga 197460
   gtaagatttt gttttaaaaa ataataataa taaataaata aaatatattt tgatgagata 197520
   tggtaaaaga tagtagaagg taggagtttg gggatttatt atgggttggt gttttaagta 197580
   gaaagaaatg tagtgtttat aagttagaga gtagatgttg aagataggag atgagtgggg 197640
   ttttttggtt ttggagagga aagggtgagt ttaggtagaa agagtaaatt tagggtgttt 197700
   tgtgtagtgt tttttttgtg gaattagttt agggtattag tagtatagtt tttttttagt 197760
   agtggagatt aggttaattg tttagtgttt tttttgtttt tttgttttaa ttttattaag 197820
   tatagttttt ttttgggtat agtagttttg taggggtgga tattgattta tgtatttagg 197880
   agtttagagg tttttttttt tttatttatt tatttagaga tggagttttt ttttgttgtt 197940
   taggttggag tgtagtggta ttaatttggt ttattgtaat ttttattttt taggtttaag 198000
   tgattttttt gttttaattt tttgtgtagt tgggattata agtatgtatt attatgtttg 198060
   gttaattttt gtatttttag tagagatggg gttttattat gttggttagg ttggttttga 198120
   atttttgatt ttgtgatttt tttattttgg ttttttaaag tgttgggatt atagatgtga 198180
   gttattatgt ttggttaggg gtttttttta atggattttt tttaatgtat ataatttggg 198240
   gaatttagat ataagaaaga tatagagtat gaaggttttt tattggtttg tttattttta 198300
   gaggtggggt tttaggtggt ttaggttggt tttaaatttt tggttttaag taattttttt 198360
   gttttggttt tatgttggga ttataggtat aagttattat gtttagttat tttttttttt 198420
   atttgggttg ttatgttaga atttggtaag ttataagatg gaaatgttta aataggtatt 198480
   tagaatttga gtagatatta tttgatgtat tattgttgtt attttaatga ttgaaatgta 198540
   tttattatat ttgttttttt aaaggttatt ttagattttt atagatttta tttgtgatgt 198600
   aagatagtta ttttaaattt ttttgtgggt ttgaatagat tagatttttg aatttttggg 198660
   tagagtaggg atggggttgt tttagaggag ggtgatattg ttatattttt gttttgtttt 198720
   tgtgttgata atgaatgagg tttggatagt ttttttttta ttaaaagtta tattttttta 198780
   aaaaggagtt ttttaggatg aggtgttatg ataatttaat tgtgtataaa tatttatttt 198840
   taataatgat agaagttatt tatagaaaag aatttatttt tggttgggta tggtggttta 198900
   ggtttgtaat tttagtattt tgagaggtta aggtaggtaa attatttgag gtttggagtt 198960
   tgagattagt ttgattaata tggtgaaatt ttgtttttaa tgaaaatata aaattagttt 199020
   gtatgtttgt aattttagtt atttaggaag ttgaggtaga agaagtattt gagtttgaga 199080
   ggtggaggtt gtagtgagtt gagattgtgt tattgtattt tagtttgggt gatagagtga 199140
   aatttttttt taaaatatat atatatatat atatatatat atatatatat atatatataa 199200
   atttattttt attttttttt ttgaaatttt tgagagaggt taggtgtagt ggtttatatt 199260
   tgtaatttta gtagtttggg aggttgaggt gggaggatgg tttgaggtta ggagttggag 199320
   attagtttgg gtaatatagt aagattttat ttttattaaa aaataataaa agtttaaaaa 199380
   gtttttgaga tagtttttgg ggaaaggtgg gttatggaga tttttatttt ggttatttgt 199440
   gttttttttg tttgtttttt tgtggttttt ttttattttg agtttggggt gggggtggtt 199500
   tgagtttagg atgggggtgg tttgagtttg gggtgggagt ggtttgagtt tgggataggg 199560
   tggtttgagt tggggtgggg gtgttttaag tttgggttgt gggtattttg agtttggggt 199620
   ggggatgttt tgagtttggg gtgggggtgt tttgagtttg ggttgagggt ggtttgagtt 199680
   tgtgttgggg gtggtttgag ttgggatggg ggtggtttga gttgggatgg gggtggtttg 199740
   agtttggggt gggggtgttt tgagtttggg atgggggtat tttgagtttg ggatgggggt 199800
   gttttgagtt gggatggggg tgttttgagt ttgggatggg ggtggtttga gttgggatgg 199860
   gggtggtttg agtttggggt gggggtggtt tgagtttggg gtgggggtgt ttaggatgta 199920
   taggttttat tttttttttt tgaggtttag tttgtttata ggagttgtga agggttggat 199980
   taaagttaaa ggtttaaatt ttagatttat ttttagattt agtggataat taaaatattt 200040
   aagtttattt ttaaaggaag ttgagataag gagataatgg ttattttttt tttttttttt 200100
   tttttgtttt tggtttttgt tatttggagt tgtgttttga agaggtatat tgggtattag 200160
   gtttttttgg ttttgttttt attttttgtt tagatataga ataatttttt tttattttta 200220
   aggttgatat gtggggttgt ggattaaagt taatttattt atttatgagg ggagtaattt 200280
   ttttgtttta ggaatttgtt tttataggag aataaatatt gtagaaggta ttgtttttag 200340
   tggtggtgta tgtttgtaat tttagttatt taggaggttg aggtagaaga attgttagaa 200400
   tttaggaggt ggaggttgta gtgagttaat attatgttat tgtattttaa tttgggtgaa 200460
   aagattaaga ttttgtttta aaataaaaaa agaatagtat tttgtgttaa ggttaggggt 200520
   tttagttatt ttttttgtga agtggaaggg gtattattag tttttgtgag aaggaaatag 200580
   tttatttaat tataaaatgt taattaaagg aggtagtata ttggttttgg tgtggtgttg 200640
   ttggtttttg tttggggtag gaggagggtt gggggagagt taaaagttaa agaaggttta 200700
   tggaggttgt tttttttttg gtgattagtt ttttttttga tggtaagttg ttaatagtat 200760
   ttggttttta ggttggggaa ggtgagatta gtttatattt gtttagggtt ttgttttgtt 200820
   tgtatgggta gtaggtgttt attttgttag atggttttgt ttattatagg gtattagtgt 200880
   tagttaagga tttatagagt ttttttgtaa aggtttatta gtgggtattg ggattttggg 200940
   ttttatttat atagggtttt tatataaaat ataggatgtt tagttaaatt tggattttag 201000
   ataaataatt aataattttt tgaagagata aggattttgt tgttttattt aagttggagt 201060
   ttagtggtat gattatggtt tattgtaatt ttgaattttt ggatttaagt aattttttaa 201120
   ttgtagtttt ttaaagtatt gggattgtag gtatgagtta ttaaggttag taaataatga 201180
   attattttta ggtataagta tattttaagt ttgttgtggg atatgtttat attaaaaatt 201240
   aatttttgtt tatttgaaat ttaaattgaa tttattgttg ggggagataa tggagtgatg 201300
   gattgataga ggagtataga gatagttttt ttaaattttt agttgattaa agtttggttt 201360
   ttagtttttt tgggttttta gagatgtatt aagggtggaa gtgattagga gggagataaa 201420
   aatattgaag tttgagtgtt agtattgagt tgtgtggttt tggataggat tgtttttttt 201480
   ttgagtttta gttttatatg ttataattgg ttaataatag ttataaagta tttgaagtta 201540
   atggttggga gtttgtttta ggaggtaggt attattagga tgtagagaga gtgaggagtt 201600
   tttaaagtta gtttaggtta ggtatagtag tttatgtttg taattttagt attttgagag 201660
   gttgaggtga gagaattatt tgagtttaga agtttaaggt tgtagtgagt tgtaattgta 201720
   ttgttgtatt ttaggttgaa tgatagagtt tttgttttta aaataaaata aaataaaata 201780
   aaataaaata ataaagttag tttgtgtatt ggattgtatg ttgttttaaa gtatttaaag 201840
   tgagagaggt taggaagtaa tgagatgtag aaaggtaggg ggaggaggtt tagaggtatt 201900
   agagaagata gttgattttt atttttatat atttttgggg tattttgagt tttatttata 201960
   tatttaggtg agaatttttg tatttgtgaa agtaatttga ggttgagaga attaatagga 202020
   aattgtttta ttttttatgt agggtttttt tataaataag gttgagttgt agtttattat 202080
   tgtagttgag ttttttgtag tagttgtggt ggggaaggtt ttttaggagg tttgagtaat 202140
   ttagaaatat ttatagtata ttttttagtt tttttagagg tttttgttga gatgaaagtg 202200
   ttttggtttg tggttaaaga gtttagtata ttgggtagtt atagttttta ttatattgat 202260
   ttggttatta ggtttttatg aatttagtga ggggagatgg gttttttagg gaagtttgtt 202320
   gattttttta tattgttgta aatgtaaagt ttggtgattt tattattttt agtaattgtg 202380
   atgtagtaat aataagaata gttatgttta ttgtttattg aaggtatatg atgagttaga 202440
   tattgtgttg gtatatgtga tttttggagg taatatattt ttttttaagg aggaaatgat 202500
   tgagttttgg agggagggtt ttttatttgg ttttaggttt ttttttgatt tgtgtttttt 202560
   tttttttttt tgtttgtttt attgtagttt ttttggtttt tttgttgttt ttggaatgta 202620
   ttaagtttgt ttttagttta gtttttgtat tgttggtttt tttttgtttt tttaatttta 202680
   atttagttgt tattattttt gtgatttttt attatttatt ttaaagtagt tattagttat 202740
   tttttatgta attattttat tttaattttt attatagttt ttagttttat tttgatagtt 202800
   tttagaattg atgaatttat tgattttgtt tttttttatt gtatgtaaat tttaagaaat 202860
   aagagttttt gtttgtttat tgttgtgttt tagtgtttgt tatagtattt ggtatgtagt 202920
   aggtgtttaa tgaatatatt tttaaaagaa tgaatgattg gaaggagttt gaaagaatga 202980
   tggtttgtgt taatagttta tttttaattt ttttagatgg gatggagatt attattatgt 203040
   aattatgatt tttttgagtt tgtgtggtgg ttgatggtag gtgttagttt ggttggatta 203100
   agggatggtt ggtaatgtat tgtttttggg tgtgtttgtg agggtgtttt tggaggagat 203160
   gggtatgtga gttggtgggt tgagtggtaa agattttttt ttaatatgga tgatattatt 203220
   ttattgttag ataataaaaa ggtagagaaa aggtgaattt tttttttttt ttggaggtgg 203280
   gatgtttttt ttttttgttt ttggatttag aatttttagt tttttagtag ttttttaggt 203340
   ttttaaattt ttggttttaa attaagagtt atattaatag ttttttagtt ttgaggtttg 203400
   tagatttgga ttgagttatg atattggttt ttttgggttt ttagtttgtg gatggttttt 203460
   tgtggtatat tttggttttt ataatggtat gagttaattt ttttaataaa ttttttttta 203520
   tttatttatt atttattttt ttaattgttt atttgtttat ttatttattt atttattatt 203580
   tattttttat ttatttattt attatttatt taattaatgt atttatgttt gtttgtttat 203640
   tggttattta tatttattta ttaattattt atttatttat tatttatttg ttatttattt 203700
   agttatttat ttatttattt atttattttt tttttttttt tgagatggag ttttattttg 203760
   ttgtttaggt tggagtgtag tggtgtgatt ttagtttatt gtaattttta ttttttaggt 203820
   ttaagtgatt tttgtgtttt agttttttga gtggttggga ttatagatat gtattattat 203880
   gtttagttaa tttttttatt tttagtagag atggggtttt attttgttag ttaggttggt 203940
   tttaaatttt tggttttaag tgatttattg gttttagttt attaatgtgt tggttattat 204000
   aggtaagagt tattgaattt aatttattat ttgtttgttt gtttgtttgt ttgtttgtgt 204060
   ttatttgttt gtttggttat ttattattta tttatttatt tgtttgtttg tttgtttgtt 204120
   tgtttgtgtt tgtttgtttt tatttttttg tttgtttgtt tatttagtta tttgtttgtt 204180
   tgtttgtttg tgtttgtttg tttttttttt tttttgtttg tttgtttgtt tatttagtta 204240
   tttatttatt tatttatttt ttattggttg tttttttgga gagttttggt tgatgtagat 204300
   ggtgttttgg taagtttagg ttgttattta gattattgtg gagggagtga tttaaataat 204360
   agatatgtat tttttagttt gagaggttta aagttttgga ttaaggtgtt gttagggtta 204420
   gtttttggtg aggttttttt ttttggtttg tatttatttt tttgttgtgt tttttgttat 204480
   tttttttttg tttataagtg aggggggagg agagattttt ggtgtttttt ttttttttta 204540
   taaggatatt aattttatta gattagggtt ttgttttatg attttattta attttaatta 204600
   tttttttaaa agttttattt ttaattatag ttgtattggg agttagggtt ttaatgtagg 204660
   aatttaaggg gaagggtata gtttggttta taataaatgg gaaattatgt ttatgattaa 204720
   attttttaga agatatggtt tattagtgtt gttaatgatt gtaagtaaag gttagagagg 204780
   aaaggggttt ttgtggggtg gggggtggtg ggggtagttg gtgttgggaa taaggttttt 204840
   ttttttgttt aatagtaggg aagtgaggtt ggttgtagat gttatttggg gtgggaagag 204900
   aggggagttt ttgtttgtat attttgtgtg ggttttggtt tgattttttt ttgtgttttt 204960
   tattttttgt gttttttttt gtgtgggaga aatgttgatt tttgttggtt taggtgttta 205020
   ggtttttagg tttgttggtt ttgggttggg tttaattaat gagaggtttg ggaggaattt 205080
   gggaggtgga agtaagggag ggtgtgggag ttttttattt tttttgtttt agatagtttt 205140
   tttagtagga gaggtggttt tttgtgtagt tttagttttt gttagagttt tatttttgtt 205200
   tgggttttta gtaggtgatt ttggtttttg gattttggtt ataggatttt ttttttttta 205260
   tttttattta ggggtggtgg tggtattttg ttgttaattt ttaggttatt tttataatgt 205320
   taagaatttt ttttttattt gtatggtttt tttttaaggt attgagaagg gttttaatta 205380
   tatgtttata gggttaaata ttttttgtaa aatttataaa atggaaaata ttttttttat 205440
   aattaattaa tgtttttgtt tatttttagt tttgagtttt tgtttgttat attttttttt 205500
   gtgtgaggtg gaaatggagg tttgttgtta tgtgggggtt tttgttaagg agaagttgaa 205560
   ttggaaagat atttagtttg gttttggttg ggttatattt atgtagtttt tatttatttt 205620
   tatgtatagt ttagttattg tttgttattt tggtgtggaa atggatttta gtaatatttg 205680
   ttttatttat tgttttaatt ttatttattg tgtagaatta aaaattataa gatatgaata 205740
   tattttgtgg tgtttggtgt tggaaaaatg tgtgtagtgg agagaagtaa gatttgaaat 205800
   gtatgggttt agaagtaaat ttgtggaaaa ttatttttat tattagaggt gaagttgtaa 205860
   gtaaaggatt tagttttttt aatgtggagt taaaatagaa aagttttttt ggttagaaat 205920
   atatgtataa atgttttata tgtagttaga aatttttttt atttttttat tttttttttg 205980
   atgggaatta tatttaaaaa aaaagaaaaa aataggattt attagaattt ttgtgtattt 206040
   tgaataaaat aattgtagta gtattattaa ttttatgttg attgttattt ttgtatatat 206100
   tttaattatt aaaaataaaa ataaaatttg attaattaag gagatttatt tgttttattt 206160
   atttatttat ttttgagatg gagttttatt ttgttgttta ggttggagtg taatggtatg 206220
   attttggttt attgtaattt ttgttttttg ggtttaagtt atttttttgt tttagttttt 206280
   taagtagttg ggattatagg tgtgtgttat tatatttggt taatttttta tgtttttggt 206340
   agagatgggg ttttattatg ttgattaggt tggttttaaa tttttgattt taagtgattt 206400
   gtttatttta attttttata gtgttggaat tataggtgtg agttattatg tttagtttat 206460
   gttttttttt gttgtttttt tttttttttt gagatatggt tttatttttt tatttaggtt 206520
   ggagtgtagt ggtatgatta tggtttattg tagttttgat tttttgggtt taagtaattt 206580
   ttttatttta gttttttaag taattgggat tataggtgta tgttattatg ttttgttagt 206640
   ttttgtattt ttagtagaga tggggttttg ttatgttgtt tggattggtt ttgaattttt 206700
   ggttttaagt gatttatttg ttttggtttt ttaaagtgtt gggattatag gtatgagtta 206760
   ttgtgtttag tttgattagt tttttgtttt tgaaatagaa aatgatatta taaagttatt 206820
   atattgaaga agtaatgtta aaaatgtggg agaaatagaa ttttggagat atgattgata 206880
   gttgattggt atttttttgt atgtagtgat gtttgtgata tttaatagtt gtttataatt 206940
   tataatttgt tgttatttat ttttttattt taaatgaatt attgtattta tatttttaga 207000
   gggttgtata agtttatatt attttgattt tttgttgatt gtttttttgt tttttgtttg 207060
   gtttttattt tttttattat ttgtgtaatt agttttttgt ttttaagttt ttttgtttta 207120
   aatgtttaag tggattaggt attgtggttt ttgtttgtaa ttttagtatt ttgggaggtt 207180
   taggtaggag gattatataa atttaggagt ataagattaa tttgggtaat atagtaagat 207240
   tttgttttta tatatatata aaaaatataa aaattagtaa ggtgtgatgg tatatgtttg 207300
   tagttttagt attttgggag gttgaggtag gaggattgtt tgaggttggg agtttaggat 207360
   tagtttgggt aataaagtaa gattttgttt ttataaaaat aaaagaaaaa attaggtagg 207420
   tatatagtgt gtatttatag tttgagttat ttgggaggtt gagaagggag gattgtttga 207480
   gtttaggagt ttaaggttgt tatgagttgt gattatatta ttgtatttta gtttgtgtga 207540
   tagagtgaga ttttgttttg ggaaaaataa ataaataaat aaataaataa ataaataaat 207600
   aaatgtttaa gtggttttta ttttttggtt agattttatt atatatttta tgaataaatt 207660
   gtatgtatga gaaaggtgag tagatgaata gggtagggtt agggggttaa ggggaagatt 207720
   atagattatg tggagtgaga ttagtggtta aaaattgtta attatagtta ggtttggtgg 207780
   tttatgaagg taattttaat ggttgggttt ggtggtttat gtttgtaatt ttagtatttt 207840
   gggaggttga tgtgagttta ttatttgagg ttaagagttt gagattagtt tggttaatgt 207900
   agggaaattt tgtttttatt aaaaatatta aaattggtta ggtatggtgg tgggtgtttg 207960
   tagttttagt tatttgggag gttgaagtag gagaattggt tgaatttggg aggtggaggt 208020
   tgtagtgagt tgggatttta ttattgtatt ttagtttggg tgatagagtg agattttatt 208080
   ttaaaaaatt taaaaaaaaa aaaataaaaa attgtagatt ttaataagga agagttatag 208140
   gatgatattt taggatgtgg gaaaaggtta tgttagtttt ggattttttt tgattaatgg 208200
   gtattttagt tattaaaggg gtagatgtgt tttatttaat ttttatttag ttattagagt 208260
   ataatatagg gatgtgttat agatagagat ggagattaat aggtagaggg gtgatgggga 208320
   ggaagattga ggtaagagga ataaaatggg gattgtagag ggtaggtggt aggagtttga 208380
   gggttttaga agaaattgtt gtataagtaa tgagttagta gggtgagtga tggttaggag 208440
   aggtggttta agaaaataaa gagatatttt tgggggtata gatgtgtttt gaggggttga 208500
   ggaagagatg gggaaggggt ggttttgtgg atttttggta tgattgttgt ggtgaaaagg 208560
   aattaaagat tttgataagg ggatgatttt ttaaataagg aagttttggg tttttgtagg 208620
   gggattagta gattgagggt aaatggtatt ttgtttatta aagttttaat aaaatgttaa 208680
   ggtaagaata tttgtttggt tttgggggta attttagaaa tattagaaaa gggatttatt 208740
   atgtgtaatt tttttttaat tatttggaga aatgtatggt tatttaagat agagaaaggt 208800
   agttaaagta agtggaggtt aggtatagtg atttatgttt gtatttttag tgttttggga 208860
   ggttgaggta ggaggattgt ttgaggttgg aaatttagta ttagtttagg taataataaa 208920
   ataagatttt gttgttataa aaataaaaat aaaaattttt tgagatggag ttttattttt 208980
   tttgtttagg ttggagtgtt gtggtatgat tttagtttat tgtaattttt gttttttaga 209040
   tttaagtaat ttttttgttt tagttttttg agtagttggg attataggta tgtgttatta 209100
   tgtttagtta attttgtagt tttagtagag atagggtttt tttatgttgg ttaggttggt 209160
   tttgaatttt tgattttttt ggttttttaa agtgttagga ttataggtat gagttattgt 209220
   gtttggttgt tataaaaatt ttttaaaaat tagttaggta tgttaggtgt ggtggttttt 209280
   atttgtaatt ttagtatttt gggaggttaa ggtaggtgga ttatgaggtt aggagtttga 209340
   gattagtttg attaatatgg tgaaattttg tttttattaa aaatataaaa attagttagg 209400
   tgtggtggta tatgtttgta attgtagtta tttaggaggt tgaggtagga gaattgtttt 209460
   aatttaggag ttgggggttg tagtgagttg agattgtatt atggtatttt agtttgggta 209520
   atagagtaag attttgtttt aaaaaaaaaa aaaaaaaaaa aattagttag gtatgtagtg 209580
   tatatttgta gttttagtta tttaggaggt tgagaaggga ggattaattg aaattgggag 209640
   gttgagattg ggaggttatg attatgttat tgtattttaa tttgggtaat atagtaagat 209700
   tttgtttaat aataataata ataaataatg taagtgggta taagaattta tttatttatt 209760
   tatttattta tttatttatg agatagggtt ttattttgtt gtttaggttg gagtgtattg 209820
   gtgtaatttt ggtttattgt aagttttgtt ttttgggttt atgttatttt tttgttttag 209880
   ttttttgagt agttgggatt ataggtgttt gttattgtat ttagttaatt ttttgtattt 209940
   ttagtagaga tggggtttta ttatggtttt gattttttga ttttgtgatt tgtttgtttt 210000
   ggttttttaa agtgttggga ttataggtgt gagttattgt ttttggtttt aagaatttat 210060
   tttattttag tattttggag gttgaggtga gtggattatg aggttagaag attgagatta 210120
   ttttggttaa tatggtgaaa ttttgttttt attaaaaaaa tataaaaaat tagttgggtg 210180
   tgggggtggg tgtttgtagt tttagttatg tgggatgttg aggtaggaga atggtgtgaa 210240
   tttgggaggt ggagtttgta gtgagtggag attgtgttat tgtattttag tttgggtaat 210300
   agagtgagat tttattttaa aaaaaaaaaa aaaagaattt attttaagat agtaagtttt 210360
   agggttgaag ttagttttta tttagtttta ggtttaaagt tataagtatg gttattgtaa 210420
   tatttttatt taggatatta ttttttaggg gtttagattt atattattaa ggaggagtgt 210480
   ttatattagt agttggggag agggtggttt tttataagat gtaatgttta ttttgaagag 210540
   ttattatagt gataattatg aaatttatgt ttgagattta attttaagtg aaaagaatag 210600
   aatttaatat ttattttatg gggagtataa aattttgaaa atatatgttt tttataatta 210660
   aattaaattt tatgttatgt ttttgaggag ataagtattg aatggaatat agaagaaatg 210720
   ttatagttaa tgattgttga agtttttttt ttaatttttt gtttatgttg tttggtaata 210780
   agtaatatag aaaattgata ataaaaagta aaagagaaag aaaagatata gtgtatagtt 210840
   tagattgttt tttttaaaaa attaaataat aataataaaa agtataggtt gggtgtggtg 210900
   gtttatgttt gtaattttag tattttggga ggttaaggtg ggtggattat ttgaggttgg 210960
   gagtttgaga ttagtttgat taatatgggg atattttgtt tgtattaaaa atataaaatt 211020
   agttgggtat ggtgatgtag gtttgtaatt ttagttattt gggaggttga ggtaagagaa 211080
   ttgtttgaat ttgggaggtg gaggttgtag tgagttgaga ttgtgttttt gtattttagt 211140
   ttgggtaata agagtgaaat tttattttaa aaaaaaaaaa aaaaaagtat aaatgtaagt 211200
   tgggtgtggt agtttatgtt tgtaatttta gtattttggg aggttggggt aggtggatta 211260
   tttgaggtta ggagtttgaa attaatttgg ttaatatggt gaaattttat ttttattaaa 211320
   aatataaaaa ttagttaggt atggtggtgt gtgtttgtaa ttttagttat ttaggaggtt 211380
   gaggtaggag aattgtttga atttaggagg tagaggttgt agtgagttaa gattgagtta 211440
   ttatatttta gtttgggtga tagagtgaga ttttgtttta aaaattaaat taaattaaat 211500
   taaataaaat aaaaaaaaga agtggtataa tataaagttt tggtagttaa agtgttaatt 211560
   tttagttatt aggaaggttt tgttatttaa tgatttattg ttttagttaa ggttagttat 211620
   attttgggtt tttgatgttg gaggtaatat gatgttaata ttggtgagaa atttattatt 211680
   tttttttttt tgttttttgg tgttggtttt tgtgtgtatt tgtagttttt ttattattgg 211740
   tgtttttatg gtgtttatgt tattggttta ttttttagta ataatttgat agtttgatat 211800
   ttttgtagat tttgtggtta tagagttaaa gttttttgtt attagtgggt tttgtagata 211860
   attttttaaa tttttttggg ttgagttagt taagttttta ataagttgga agatgatagg 211920
   ggttggatgt aatttatttt taattggttt gggttatatg gatattattt tttttttggg 211980
   atttgatttg ggatggaaat aggagttata gtttatggga aggttgtgaa aggaagtttt 212040
   ggttttaggg tgtggattag gttgatttgt ggaataaggt tttaggggtg gtttatttgt 212100
   ttgttttgtt tagttttttt tggtaattat atttatttgg ggtttatttt aaatttggta 212160
   ggtaatgttg ttgtttttat tttagttgtt gttttaatat gttttgtttg gttaggatag 212220
   gtgtttaggt atggtattta gggatatttt tggtttttgg ggtttaattt ttggttttta 212280
   ggtgtagtgt tgggaaaagt agagggtttt ggttatggtg taggttttgt tataggttat 212340
   ttaagtagtt tgggaggagt tggaaagaga gtaatgtatt tatttggata gagattgaag 212400
   ttatattttt attgagaaaa gatgatattt ttgtttggat tttttttttg aaattttagg 212460
   ttggggtggg gtaggagtta gtgagagagt ttagtgtttt aaaggggatt ttttggtttt 212520
   ttgtgttttt taatttagta tttttgggga tttatatatt tagggtagag tggttattta 212580
   gggttttttt tatttagata taaaggttat tgaaggtttt gggttaatat tgttttgagg 212640
   atgattagtg atttgagttt ttgttagaga tagtaggtag tttgggttta agggttgttt 212700
   agtgatttga agttgggatt attttatatt ttttgtgtta tttttgtttt ttgttttgga 212760
   ggtttttagg ttgttaatag ttatgttttg gagaattagg ttagattagg ttgggaataa 212820
   agtttagttg ttatagagtt gttgagaggt tattggtaag tagtgtttag gagttgtttt 212880
   ttttttgtta gtaatagttt aatgtttttt tttatagggg ttgtaaatgg gtttatgttt 212940
   agggtggggt tagggagata aagtagagag aaattgggtt gggagtagag agtgttatgt 213000
   tttattttgg agatttaagg ttaggttatt ttgtagagtt ggtgtttaga gtttttagtg 213060
   tataggagag gaggtaaata gaatttgata tttagtagtg tttggtagat gtttttggaa 213120
   tgaatgatta aatttatatg gagagttgaa atgggttagg aattttaggg agatgggtag 213180
   tttatagttt agtagggttg ttttagtaga gtaggttaat gttaggtttt ttggtaaatt 213240
   ttttggggtt taggtataat tgttatatta ggaaaggggt aggggtgatt agtttattgg 213300
   ttatgggggt attttattgt ttttatagaa gtttggtatt tataggtttg ggtgatatat 213360
   gatttttatg tttggtatta tgatggttgt ttattaagtt tttttattta ttaggattga 213420
   ttttttttgg ttagtgattt tagattatta tgatttggta gttgttgtta ggtttgtttg 213480
   gttaaggatt tttttggaga ttgagttatt ttatatagta aagtgagtta tggggttata 213540
   gtgttgtgtg ttgtgtataa gttttgaggg ttttatttag ggattggtat ttattgattt 213600
   ttataaattt ttggtaattg tttagagttg ggatgttttt aaggtaatag ttttaataaa 213660
   tagaggtgta ggaatttagt gtgttatttt ttgttttatt ttaaaggttt atatttttag 213720
   gggtttggag tttttttttg gagttttttt tattttaaag ttagttggat ttgtttagga 213780
   attgttatta ttattttttg gttgggtttg tattgatgtg tattgaattt tttttattag 213840
   gtttatttta ggtaaatttt agagtttatg taatattgtt tgtggataaa tattggggtg 213900
   gaaatagggg ttatttttag gttttagtta tatggggttg ttagggttgt ttgggatggt 213960
   aggagagtgt tagaggaggg tagtttaagg attaggtagg gggtgggaag tgtttttttt 214020
   tatgggtttt taagtgagat gttttttgta aatatttatt tgggattttt aagtagaggg 214080
   ttggttttta tgttatttgg atttggattt tagattattg tttttaagtg gtaattttgg 214140
   gtttttttgt atttgttttg gtttgagttg gttatatttg tggggggtgg ttttaggaag 214200
   aaggtggatt gttgtaaatg taatgttttg ttgttaggtg tattgttttt ggttattaga 214260
   atttttttgg gtaggttggg gagatttgat gggtaagtgt ttgtggtttg gtgtgagtgg 214320
   gtagggtagg tggtttttgt ttgggtatgt ttttttattt ttgtaataga ttagggagag 214380
   ggttggtttt aggatttagg tttagttagt tgttttataa ttgaggggtt ttggtggagg 214440
   atataagtta gatagagtta tagattaatt gtatgaggtg gttttttttt tttagtttag 214500
   gttttttgaa tgtgttagag tagataagtt tggatgtgag gaggtaagag gtagtgagaa 214560
   taggtgtgga gatggggttg ggggagaaga ggaagaaatt ttgtgtttgt gtttttttta 214620
   tgtttgtttg ttattgggtt agttgattgg ggaagtgttt tttgagggtg taattttttg 214680
   ttttttttgt aagttagttt ttggggtgtt gggtgagggt gtagttttgt ttattatggt 214740
   gagttagtat attgaattat ggtagaagtt gttttttgtg tgggtgtagt tttttttttt 214800
   gtttaggatt tagaagttat gatagtatag agttttttaa ttgtggttgg tgttttttta 214860
   ggaatttttt gaaagttata gagaaatttt agagtggttg ggtatggtgg tttatgtttg 214920
   tgattttagt attttgggag gttgaggtgg gtggattatt tgaggtttgg agtttgagat 214980
   tagtttggtt agtatggtaa aattttattt ttattaaaaa atataaaaat tagttgggta 215040
   tggtggtata tgtttgtaat tttagttatt taggaggttg aggtatgaga attgtttgaa 215100
   tttgggaggt agaggttgta gtgaattgag attgtgttat tgtattttag tttgggtaat 215160
   agagtaagat tttattttaa aaaaataaaa aataaaaaaa gaatttttag agtaattttt 215220
   aggtgttagt aggggtgtga tgtaatttta gttttgtttt tgtggtttat tggttttggg 215280
   taagtttttt tgttgttgtt atttatagag agtttttagg ggagtttggg aatgatggta 215340
   agttttatgt gatttagtat gtaaagggag ttttaaaatt tagaatttag agagttaggg 215400
   tgggtatagt gtagttggtt ttagtgttga ggggttgggg gagagatttt gtattatgtt 215460
   ttgattatag taagggaaga ttatggaggt ttttggaatg ttttagtaat ggtgtaattt 215520
   tagttttttt tatttttttg ttaatgataa ttatgattat gatagtagtt aataggttat 215580
   agagtaggta ttttttagtt tttttaaata tgaatttgtt taatttttat aataatatag 215640
   tattgtttta tttaataaat gaggtaggtt gggtgtggtg gtttatgttt gtaattatag 215700
   tattttggga ggttaaggta ggtagattat gaggttagga gtttgagatt agtttgatta 215760
   atatggtgaa attttatttt tagtaaaaat ataaaaatta gttgggtgta gtggtatgtt 215820
   tttataattt tagttattta ggaggttgag gtaggagaat tatttgaatt tgggaggtgg 215880
   aggttgtagt aagttgagat tatgttattg tattttagtt tgggagatag agtgagattt 215940
   tgttttaaaa ataaataaat aaataaaatt aaaaaatatt atatatataa aaaaataaat 216000
   gaggtaaatg agatgtatga ggtgagaatt ttgtgttata gggagaggtg tagtttggtg 216060
   agatttaatt ttaaggggtt ggtggttttg agtttttaag tgtaattatt ggattatatt 216120
   gtttgtagat tattattatt tttattttat tggaaggttg ggtgaggtaa ttgggaaagt 216180
   ttggagtagt agtggagttg ttagagagaa tttgttttta gtgttttttt ggattttgta 216240
   gttgttttgt gtgggttttt gttgtttttt gtattggaaa agattttttt atagatgggg 216300
   ttttatgatg ttatttaggt tggagtgttg tgtttatttt taggtgttgt tatagggttg 216360
   tagttttgaa tttttggttt taagtgattt ttttgtttta gttttttgag tagttttttg 216420
   ggtttaagtg atttttgtgt tttagttttt tgagtagttt tttgggttta agtgattttt 216480
   gtgttttagt tttttgagta gttgggattt taagtgtatt tggttaagtt tttttttatt 216540
   ttttattatt taagattttt ttttttttaa ggtttagttt aattttattt ttatttaaaa 216600
   tttttggggt atttttgtag tatttggagg ttgaattgtt taggagggaa gggtgttaat 216660
   gagttggtgt tttgttttta ttttgatttt tggtgagaat gtaagttttt tgtaggtaag 216720
   gattatattt tattattatt tttgtattta tttagatttg gttatgtgtg tttagtatat 216780
   aaagtgtttt tgatgattgt ttatagattt atggtatttg agattgtttt aatatatttt 216840
   tttaaaattt tttttttttt tagaggttta tgaatgttta aattaggttt attgggaata 216900
   atattatggt tattttgttt attttgaagt ttatatatag gtttggaagt aataattttg 216960
   tatagtttag atgaaaaaat aaagattttt ttgttgtttt tggattgtta gagttttggg 217020
   ggatttttaa tattttttgt tgtttttttt aattttatgg tgttgatggt ttgttggggg 217080
   gttagtttga tttttgtgag tgtttgtatt tggaaaaggg ttggtgttaa gatagtttta 217140
   ggatatttag aatgagggta agaggttatt tttattatta gtttttttta gttttatttt 217200
   atataattgt aatatagtga tttagtatat tttgggttat tgttaagtag ggagtaaatt 217260
   agaaatggtt tgagtttttt ttttttttat ttttagaggg gtttgttttt ttaggtaaat 217320
   ataaggaggt attgaggttg ttgtataaga gttggttttt gtttatttta taaattttat 217380
   ttttatttat tgtaaaaggt tttgtttttt tttttaaaaa aaaaaaaaaa aaagaaattt 217440
   agtgaggggt ggggtaggtg tgtgtggagg agaggagaaa aggataagtg atggtttttt 217500
   tattttttag taagttgtag gtgtagagat tttaagtttg ttttagggtt tttttggtat 217560
   ttgtgtagga gggtttttgt agaaaaaatt taggatttat gatttgggag gattttggtt 217620
   atttttattg tttggattta ttagtgggag gtaaagtgta agaaattaag ggtaaagggt 217680
   tttttttggg tgggttagag attttttgga aagggttatg tattgtgtga tttttataat 217740
   aattttaagt atttggagta tgttggggag gaagttttgg tgttaaaatt aagtatgtgt 217800
   tatttggaag tttgtttttg tttattagta tattaattta tttatgtttt tgatagagtt 217860
   gtgttttggg tgtgtagagg gttattttgt ttgtaataat tgggtgaaag ttgaaatttt 217920
   aagttattga tttagaaggg tatttttggg gaagttttga gtaatttttt ttgagataag 217980
   gtattttatt atttattttg gtttttaaat atggaaagtt agaggaaatt gataatggtt  218040
   taaaggtagg gggatggttt tgatgatttt tttattttta attaaggttt atgattttgt  218100
   ttttttatta ataaaaatta atttatggat ttttggtagg tattttttta tttgaatgaa  218160
   ttaaaggagg gatattaaat aaatggtttt tagatttaga agaggagatg ttatttataa  218220
   tttgaggatt gaggaggata aaggattttg tttgtttgga ttttttggag aaaattataa  218280
   aggaaaaaaa aaaggaagga aaagataaat aatgaagatt tggatttttt ttaatttgtt  218340
   tttgtggttt tggtgtgttt atttgtgaaa tggaatttta gagtgttaga gtgggagttt  218400
   tttatgaaat gaaagttttt gaattggttt atggtgaggt ttaagttagg ttttttttgt  218460
   ttatttaagg tgggttttgt tagggtggag tgaggatgtt agttgtgggt ggtgttttga  218520
   gtgtaaaatg tgaatttgag ttgttaagtt tgggtgttgt tggatggttt tatttggagg  218580
   ttgttttgtg ttttattaag tagaggagaa attgaagaga atttgaagaa aggatgttgt  218640
   ggtttggatt attagtttta gaggttgaat ttaaggatat ttttttattt agttttgatt  218700
   ttatttattt atatagatgt atagggattt aaagtatttg gttgatttgg aaagttagtg  218760
   atttttgtga gggtgtaagg ggtgaggaag agggagtgtt ttttagggta gtttttttga  218820
   tatttgagta agggttggag atagtgtagg ggatggttgg tttaggttga aagtggtttg  218880
   atagtggttt tgttttggga ggaatttgat ttgtagtgtg tttgttgtgg ggatttgttt  218940
   agttttgggg gtgttttgtt atttttggtt gtaggtattt aggtgagtag tgttggtttt  219000
   gggatattga ttttttttta ttttgggtgt gaagtttatt tgagtaagat ttttttatgg  219060
   tgtttttgtg gttgttgttt ttaggaaagt gtggtggtga ggtagtgggg tgtgtgtttt  219120
   tagggtgtta attttgggtt ggtagtagga aggtgggaag agggggtggt ttgttgtttg  219180
   gagtgtattg attgggtttt gttttggttt tgtttttgtt ttgttttttt ttggagatgt  219240
   gtatagaatt ttggtttagt tggttgtggt tttgtttttg atttgttttt tttttgtttt  219300
   gtgaatgtgt gtgttgagtt ttatttttgt ttatttttgt gttgtgttgt atttggtgga  219360
   gtggggttgg tgggtgttgg gtttgggagg gggtgttttt gtgtggtagt gtagattaga  219420
   ttagattagg ttaggttggg ggtgggggtt gtttgtgtgg tttgttgtgt ttgtagtttt  219480
   gtatttttag ttttttgtgt tggtgttaat ggttggtttt gggagttgtt gtatttttta  219540
   gttttgagtt tgggtggggt gggtttggtt aggtggaagt gggtggaggt gtgggtgttt  219600
   tgggtggttt tgttgggtga ggtggtggtg ttgggtattg tggtggtgtt gggtatagtt  219660
   gggggttgga gttggggtgt tggtttagtg ggttggggta gggtgggata ggatatttgt  219720
   tttgttattt ttttggtaat taaaagggta gagtgtgttg attgagttgg agttttagtt  219780
   tttttatttg taaatgggtg tgttggatag atgattattt gttttttttg tgttgggtat  219840
   tgtgtgtagt ttggtgtatt ggtgtagtgg ggtagatata gggagttgtt ttttataatt  219900
   tgggtaatg                                                          219909
<210> 67
   <211> 1405
   <212> DNA
   <213> Artificial Sequence
<220>
   <223> chemically treated genomic DNA (Homo sapiens)
<400> 67
   tgttatttga gttgtaaagg gtggtttttt gtgtttgttt tgttgtattg atatattgag      60
   ttgtgtatgg tgtttagtgt agggagaata aatgattatt tgtttaatgt gtttatttat     120
   aggtgaggaa attaaggttt taatttaatt gatgtatttt gtttttttga ttattagaaa     180
   agtagtagga taggtgtttt gttttgtttt attttggttt attaagttgg tattttggtt     240
   ttgatttttg gttgtgtttg gtgttgttgt ggtgtttggt gttgttgttt tgtttggtgg     300
   ggttgtttgg agtgtttgta tttttgtttg tttttatttg gttgggtttg ttttgtttgg     360
   atttgggatt gggaagtgtg gtgatttttg gaattagtta ttggtgttag tgtggggagt     420
   tgggggtgta gagttgtggg tgtggtgggt tatgtaggtg gtttttattt ttggtttggt     480
   ttggtttggt ttggtttgtg ttgttgtgtg ggggtgtttt tttttaggtt tggtgtttgt     540
   tagttttgtt ttgttaggtg tagtgtagtg taggggtggg tgggggtggg gtttggtgtg     600
   tatgtttatg gggtggggag ggggtgggtt aggggtggga ttatagttgg ttgggttggg     660
   gttttatgtg tatttttggg gaggggtggg gtgggggtgg ggttggggtg gggtttggtt     720
   ggtgtatttt agatggtggg ttgttttttt tttttgtttt tttattattg gtttaggatt     780
   agtgttttgg gagtgtgtgt tttgttgttt tgttgttata tttttttggg agtggtggtt     840
   atggaggtat tatgaagaag ttttatttag gtgggttttg tgtttggggt ggggaggggt     900
   tggtgttttg ggattagtgt tgtttatttg agtgtttgtg gttgggagtg gtgaggtgtt     960
   tttggagttg agtgagtttt tgtggtgggt atattgtagg ttgagttttt tttaggatag    1020
   ggttgttgtt gggttgtttt tgatttgagt tgattgtttt ttgtgttgtt tttagttttt    1080
   gtttgagtgt tggaggggtt gttttggggg atgttttttt ttttttgttt tttgtatttt    1140
   tgtaggaatt gttgattttt taggttggtt gggtgttttg ggtttttgtg tgtttgtgtg    1200
   ggtgaatggg gttggggtta ggtggagggg tgtttttggg tttagttttt agggttggtg    1260
   gtttaggttg tagtattttt tttttggatt ttttttggtt ttttttttat ttagtggggt    1320
   atgggatggt ttttagatgg gattgtttag tagtgtttaa atttggtgat ttgggtttat    1380
   gttttgtgtt taggatgttg tttgt                                          1405
<210> 68
   <211> 1405
   <212> DNA
   <213> Artificial Sequence
<220>
   <223> chemically treated genomic DNA (Homo sapiens)
<400> 68
   gtgggtggtg ttttgagtgt aaaatgtgaa tttgagttgt taagtttggg tgttgttgga      60
   tggttttatt tggaggttgt tttgtgtttt attaagtaga ggagaaattg aagagaattt     120
   gaagaaagga tgttgtggtt tggattatta gttttagagg ttgaatttaa ggatattttt     180
   ttatttagtt ttgattttat ttatttatat agatgtatag ggatttaaag tatttggttg     240
   atttggaaag ttagtgattt ttgtgagggt gtaaggggtg aggaagaggg agtgtttttt     300
   agggtagttt ttttgatatt tgagtaaggg ttggagatag tgtaggggat ggttggttta     360
   ggttgaaagt ggtttgatag tggttttgtt ttgggaggaa tttgatttgt agtgtgtttg     420
   ttgtggggat ttgtttagtt ttgggggtgt tttgttattt ttggttgtag gtatttaggt     480
   gagtagtgtt ggttttggga tattgatttt tttttatttt gggtgtgaag tttatttgag     540
   taagattttt ttatggtgtt tttgtggttg ttgtttttag gaaagtgtgg tggtgaggta     600
   gtggggtgtg tgtttttagg gtgttaattt tgggttggta gtaggaaggt gggaagaggg     660
   ggtggtttgt tgtttggagt gtattgattg ggttttgttt tggttttgtt tttgttttgt     720
   ttttttttgg agatgtgtat agaattttgg tttagttggt tgtggttttg tttttgattt     780
   gttttttttt tgttttgtga atgtgtgtgt tgagttttat ttttgtttat ttttgtgttg     840
   tgttgtattt ggtggagtgg ggttggtggg tgttgggttt gggagggggt gtttttgtgt     900
   ggtagtgtag attagattag attaggttag gttgggggtg ggggttgttt gtgtggtttg     960
   ttgtgtttgt agttttgtat ttttagtttt ttgtgttggt gttaatggtt ggttttggga    1020
   gttgttgtat tttttagttt tgagtttggg tggggtgggt ttggttaggt ggaagtgggt    1080
   ggaggtgtgg gtgttttggg tggttttgtt gggtgaggtg gtggtgttgg gtattgtggt    1140
   ggtgttgggt atagttgggg gttggagttg gggtgttggt ttagtgggtt ggggtagggt    1200
   gggataggat atttgttttg ttattttttt ggtaattaaa agggtagagt gtgttgattg    1260
   agttggagtt ttagtttttt tatttgtaaa tgggtgtgtt ggatagatga ttatttgttt    1320
   tttttgtgtt gggtattgtg tgtagtttgg tgtattggtg tagtggggta gatataggga    1380
   gttgtttttt ataatttggg taatg                                          1405
<210> 69
   <211> 1818
   <212> DNA
   <213> Artificial Sequence
<220>
   <223> chemically treated genomic DNA (Homo sapiens)
<400> 69
   tgttgtttgt ttttggtttt tttgatagtt gtgttttatt tttgttttgt gttttttttt      60
   ttggatagtg ttttttttag ggtttattta ggagggtgta gtggtggttt ttggggtggt     120
   ggttgtggtg ggggtgttag ttgtaggggt gtttttggtg ggtgggagtt ggtggttttt     180
   tgttggtgtt atgggatttg tatgtttgtt ttgtgttttt tggtttttga gtttataggt     240
   tgggattttg tttgttagtt gtgtgtgttg ttgtttaatt tttgtaggtg tagagtgtgt     300
   ggtggtggtg atagagaatt ttgtttggtt gtttaaatat agttttttgt agaaggattt      360
   tgtgtttggg gaaggggagg aatttttttt tttttgggtg tttgtttttt ttgttatggt     420
   ttggttttta tatttgttta tatttggttg tagtggggtg tttgggggga ggggttgagg     480
   ttgtgttttt tgttgttttt tgggtgtggg ttaggtgggg aggagggggg tgttttggtt    540
   gtgtgtttag gattgttttt tagtggttat ttgggtttta gttttttagg tttggttttg    600
   ataggtgggt ggagtagtta gtgtgagata gggaggttgg tgtgggtgtg ggaatttgat     660
   ttgtttggga ggtgggggtg gggtgggggt gtagtgtgtg gggaggggtt ggtgtttgtt     720
   tttttttttt atttatttag ttgagttagg gggtttaggg gtttttttgg tggttagttt     780
   tgtattgtag gagtgtgggt gtggtgtttt agttagtgtg tagggtttgg gttttgttgg     840
   gggtgttttt ttgttgttgt tttttgtgtg atttgttgtt tattagttat tatgttggat     900
   tttgtggtta atgtgtagtt ggatgggatt attttggatt ttgaaggtgg gtgttgggtt     960
   ggttgttgtg gttgtggatg tgttggagag gattttgtgg gtgggtttgg tgtgggatgg    1020
   gggtgtgttg aggggagatg ggagtgtgtt gaggggagat gggattttta atttaggtgt    1080
   ttttttgttg agagtgttgt gtgtttttgg ttttgtgttt gtgttgttta tgtgggggat    1140
   tttgttaggg gtatttgtgt agattttgtg tgtttttata ggattttgtg tttgttttgt    1200
   gtattgttgt ttgggttttt ttttttttat tgttgtttgt gtttgttaag tgatagtgat    1260
   ttttttgagg gtttgtgagg ttgttttgga attttttagg atgtatagtt ttattttggg    1320
   aaatttattg gttttttttt tttggttttt tttggtggtt tttgggtttt gtttggattt    1380
   ggtaatggga tagggaggtt gtttttattt tttgattgag tggatagttg tgttttgttt    1440
   gggtggatag tttttttttt ttttatgtta gttttggggt tgttaagttg tgtagtttgt    1500
   gggttgggag tattgaatgg atatagttta ggttgtggta gggtttagag tgggatgttt    1560
   tatggttttt atttaggttt ggggatattt ttatttgttt tttagaattg ggttgtgggg    1620
   gatagaaggg gtttgtgtgt gggtagggag agtattttgg tttttttttg tttttggggt    1680
   ttataaagtg tgttgggatt tgtggggttg ttttgtttaa gtttgggttt ggtgtttgtg    1740
   tttttgagtt tgtgagtgtg tgtgtttttt tgtgtttttt tgattgttgg tgttggggtt    1800
   ttgtgttttg tgtttgtg                                                  1818
<210> 70
   <211> 1818
   <212> DNA
   <213> Artificial Sequence
<220>
   <223> chemically treated genomic DNA (Homo sapiens)
<400> 70
   tgtgaatgta aaatgtaaaa ttttaatatt gataattaaa aaaatgtaaa aaaatgtatg      60
   tatttataaa tttaaaaatg tgaatgttaa atttaaattt aaataaaata attttgtaaa     120
   ttttaatata ttttataaat tttgaaaata aaaaaaaatt aaaatatttt ttttatttgt     180
   atgtaaattt tttttatttt ttatgatttg attttaaaaa atgaataaaa atatttttaa    240
   atttaaataa aaattataaa atattttatt ttaaatttta ttatgattta aattatattt     300
   gtttgatatt tttgatttat gaattatata atttaatgat tttgaaatta atgtaaaaaa     360
   aaaaaaaatt atttatttga ataaaatgtg attatttatt taattgaaaa taaaaaatga     420
   tttttttatt ttgttattga atttaaatga aatttgaaaa ttgttgaaaa aaattaaaaa    480
   aaaaaaattg ataaattttt taaaataaaa ttatatgttt taaaaaattt tgaaataatt     540
   ttgtgaattt ttgaaaaaat tgttattgtt taataaatat aaataataat aaaaaaaaaa     600
   aaatttaaat gataatatgt aaaatgaata taaaatttta taaaaatgtg taaaatttat     660
   gtgaatattt ttaataaaat tttttatgta aatgatgtga atatgaaatt gaaaatgtgt     720
   gatgttttta atgaaaaaat gtttaaatta aaaattttgt ttttttttaa tgtatttttg      780
   ttttttttta atgtattttt gttttgtgtt aaatttattt gtaaaatttt ttttaatatg     840
   tttgtgattg taataattaa tttaatattt atttttgaaa tttgaaataa ttttatttaa     900
   ttatgtgtta attgtgaaat ttgatataat aattaataaa taatgaattg tgtgaaaaat     960
   aatgatgaaa aaatgttttt gatgaaattt gaattttatg tgttaattaa aatgttgtgt    1020
   ttgtgttttt ataatataaa attaattgtt gaaaaaattt ttaaattttt taatttaatt    1080
   aaataaataa aaaaaaaaaa tgaatgttga tttttttttg tgtgttatgt ttttgttttg    1140
   tttttgtttt ttgaatgaat taaatttttg tatttgtatt gattttttta ttttgtatta    1200
   attattttgt ttgtttatta aaattaaatt taaaaaatta aaatttgaat aattgttaaa    1260
   aaataatttt aaatatatga ttgaaatgtt tttttttttt ttgtttaatt tgtgtttaaa    1320
   aaatgatgaa aaatgtgatt ttaatttttt tttttgaatg ttttgttatg attaaatata    1380
   aatgaatata aaaattgaat tataatgaaa aaaatgaatg tttaaaaaaa aaaaaatttt    1440
   tttttttttt tgaatgtaaa atttttttat aaaaaattat atttaaataa ttaaataaaa    1500
   ttttttatta ttgttgttgt gtgttttatg tttataaaaa ttaaatgata atgtatgtga    1560
   ttaataaata aaattttgat ttataaattt aaaaattgaa aaatgtaaaa tgaatatatg   1620
   aattttataa tattaatgaa aaattattaa tttttattta ttgaaaatat ttttataatt    1680
   aatattttta ttatgattat tgttttgaaa attattgtta tattttttta aataaatttt    1740
   aaaaaaaata ttatttgaaa aaaaaaatat aaaatgaaaa taaaatatga ttattaaaaa    1800
   aattaaaaat gaatgatg                                                  1818
<210> 71
   <211> 20
   <212> DNA
   <213> Artificial Sequence
<220>
   <223> chemically treated genomic DNA (Homo sapiens)
<400> 71
   ggagtggagg aaattgagat 20
<210> 72
   <211> 22
   <212> DNA
   <213> Artificial Sequence
<220>
   <223> chemically treated genomic DNA (Homo sapiens)
<400> 72
   ccacacaaca aatactcaaa ac 22
<210> 73
   <211> 33
   <212> DNA
   <213> Artificial Sequence
<220>
   <223> chemically treated genomic DNA (Homo sapiens)
<400> 73
   tgggtgtttg taatttttgt tttgtgttag gtt 33
<210> 74
   <211> 23
   <212> DNA
   <213> Artificial Sequence
<220>
   <223> chemically treated genomic DNA (Homo sapiens)
<400> 74
   tttcgtttcg gtttttagcg tag 23
<210> 75
   <211> 17
   <212> DNA
   <213> Artificial Sequence
<220>
   <223> chemically treated genomic DNA (Homo sapiens)
<400> 75
   aaccaaccgc gactccg 17
<210> 76
   <211> 22
   <212> DNA
   <213> Artificial Sequence
<220>
   <223> chemically treated genomic DNA (Homo sapiens)
<400> 76
   cgatttcaac cgcgcgatcg aa 22
<210> 77
   <211> 16
   <212> DNA
   <213> Artificial Sequence
<220>
   <223> chemically treated genomic DNA (Homo sapiens)
<400> 77
   cgtggtttcg gggacg 16
<210> 78
   <211> 18
   <212> DNA
   <213> Artificial Sequence
<220>
   <223> chemically treated genomic DNA (Homo sapiens)
<400> 78
   cgtctacacg cgaaacgc 18
<210> 79
   <211> 25
   <212> DNA
   <213> Artificial Sequence
<220>
   <223> chemically treated genomic DNA (Homo sapiens)
<400> 79
   cacatcgaaa tcgtacgcgc tctcg 25
<210> 80
   <211> 19
   <212> DNA
   <213> Artificial Sequence
<220>
   <223> chemically treated genomic DNA (Homo sapiens)
<400> 80
   caccttcccg aaaaacgaa 19
<210> 81
   <211> 27
   <212> DNA
   <213> Artificial Sequence
<220>
   <223> chemically treated genomic DNA (Homo sapiens)
<400> 81
   atggtagtag agatttcgag taaacgg 27
<210> 82
   <211> 28
   <212> DNA
   <213> Artificial Sequence
<220>
   <223> chemically treated genomic DNA (Homo sapiens)
<400> 82
   cgaatccgac tctaacgaaa cgctacga 28
<210> 83
   <211> 22
   <212> DNA
   <213> Artificial Sequence
<220>
   <223> chemically treated genomic DNA (Homo sapiens)
<400> 83
   gaagtagtcg gggtcgttta cg 22
<210> 84
   <211> 20
   <212> DNA
   <213> Artificial Sequence
<220>
   <223> chemically treated genomic DNA (Homo sapiens)
<400> 84
   gcaaaatacg cgaaaaccgt 20
<210> 85
   <211> 23
   <212> DNA
   <213> Artificial Sequence
<220>
   <223> chemically treated genomic DNA (Homo sapiens)
<400> 85
   acgtcttctc tcgccccgaa cga 23
<210> 86
   <211> 20
   <212> DNA
   <213> Artificial Sequence
<220>
   <223> chemically treated genomic DNA (Homo sapiens)
<400> 86
   tcgtgtgtgt cgtttttcgg 20
<210> 87
   <211> 19
   <212> DNA
   <213> Artificial Sequence
<220>
   <223> chemically treated genomic DNA (Homo sapiens)
<400> 87
   attcccgacg actccgaat 19
<210> 88
   <211> 33
   <212> DNA
   <213> Artificial Sequence
<220>
   <223> chemically treated genomic DNA (Homo sapiens)
<400> 88
   caacgaaaac gaaaaaatac gactacgtct cga 33
<210> 89
   <211> 20
   <212> DNA
   <213> Artificial Sequence
<220>
   <223> chemically treated genomic DNA (Homo sapiens)
<400> 89
   tttgaagcgg ttttcgtatc 20
<210> 90
   <211> 20
   <212> DNA
   <213> Artificial Sequence
<220>
   <223> chemically treated genomic DNA (Homo sapiens)
<400> 90
   ccgaacgctt acctacaatc 20
<210> 91
   <211> 22
   <212> DNA
   <213> Artificial Sequence
<220>
   <223> chemically treated genomic DNA (Homo sapiens)
<400> 91
   ttgcggtggg cgttttcggg tt 22
<210> 92
   <211> 19
   <212> DNA
   <213> Artificial Sequence
<220>
   <223> chemically treated genomic DNA (Homo sapiens)
<400> 92
   tttgtagtcg cgtcgtcgt 19
<210> 93
   <211> 20
   <212> DNA
   <213> Artificial Sequence
<220>
   <223> chemically treated genomic DNA (Homo sapiens)
<400> 93
   ctaactaaaa cgcgccgaaa 20
<210> 94
   <211> 22
   <212> DNA
   <213> Artificial Sequence
<220>
   <223> chemically treated genomic DNA (Homo sapiens)
<400> 94
   tcgacgtaaa cttccccgcg ct 22
<210> 95
   <211> 20
   <212> DNA
   <213> Artificial Sequence
<220>
   <223> chemically treated genomic DNA (Homo sapiens)
<400> 95
   atttcggaaa gcgtttttcg 20
<210> 96
   <211> 25
   <212> DNA
   <213> Artificial Sequence
<220>
   <223> chemically treated genomic DNA (Homo sapiens)
<400> 96
   caactccgaa taaattacca acgac 25
<210> 97
   <211> 33
   <212> DNA
   <213> Artificial Sequence
<220>
   <223> chemically treated genomic DNA (Homo sapiens)
<400> 97
   ttacgataac acttacacga ccaaaacgac gaa 33
<210> 98
   <211> 21
   <212> DNA
   <213> Artificial Sequence
<220>
   <223> chemically treated genomic DNA (Homo sapiens)
<400> 98
   cgtttttcgt tttattttcg c 21
<210> 99
   <211> 18
   <212> DNA
   <213> Artificial Sequence
<220>
   <223> chemically treated genomic DNA (Homo sapiens)
<400> 99
   gacaaaaaac gccacgtc 18
<210> 100
   <211> 22
   <212> DNA
   <213> Artificial Sequence
<220>
   <223> chemically treated genomic DNA (Homo sapiens)
<400> 100
   ccgacaattc accgaatcac cg 22
<210> 101
   <211> 22
   <212> DNA
   <213> Artificial Sequence
<220>
   <223> chemically treated genomic DNA (Homo sapiens)
<400> 101
   tcgcgagttt agttattcgt tg 22
<210> 102
   <211> 22
   <212> DNA
   <213> Artificial Sequence
<220>
   <223> chemically treated genomic DNA (Homo sapiens)
<400> 102
   ccactctcga aatatacgtc ga 22
<210> 103
   <211> 35
   <212> DNA
   <213> Artificial Sequence
<220>
   <223> chemically treated genomic DNA (Homo sapiens)
<400> 103
   cgataaacga aatctataat atacgaacgc gaaca 35
<210> 104
   <211> 17
   <212> DNA
   <213> Artificial Sequence
<220>
   <223> chemically treated genomic DNA (Homo sapiens)
<400> 104
   tagtcgtttt ggcggcg 17
<210> 105
   <211> 18
   <212> DNA
   <213> Artificial Sequence
<220>
   <223> chemically treated genomic DNA (Homo sapiens)
<400> 105
   aacgcacgac caacacga 18
<210> 106
   <211> 30
   <212> DNA
   <213> Artificial Sequence
<220>
   <223> chemically treated genomic DNA (Homo sapiens)
<400> 106
   aaaaaacacc gaacccaacg cgataataaa 30
<210> 107
   <211> 26
   <212> DNA
   <213> Artificial Sequence
<220>
   <223> chemically treated genomic DNA (Homo sapiens)
<400> 107
   gtagtagtta gtttagtatt tatttt 26
<210> 108
   <211> 24
   <212> DNA
   <213> Artificial Sequence
<220>
   <223> chemically treated genomic DNA (Homo sapiens)
<400> 108
   gatttagagt tgaatgtaaa gtaa 24
<210> 109
   <211> 18
   <212> DNA
   <213> Artificial Sequence
<220>
   <223> chemically treated genomic DNA (Homo sapiens)
<400> 109
   ttttttggag ttgaaagg 18
<210> 110
   <211> 20
   <212> DNA
   <213> Artificial Sequence
<220>
   <223> chemically treated genomic DNA (Homo sapiens)
<400> 110
   ttaggaaatg aaattggaga 20
<210> 111
   <211> 20
   <212> DNA
   <213> Artificial Sequence
<220>
   <223> chemically treated genomic DNA (Homo sapiens)
<400> 111
   aaacccaaac ctaaattaaa 20
<210> 112
   <211> 17
   <212> DNA
   <213> Artificial Sequence
<220>
   <223> chemically treated genomic DNA (Homo sapiens)
<400> 112
   cccaccaacc atcatat 17
<210> 113
   <211> 18
   <212> DNA
   <213> Artificial Sequence
<220>
   <223> chemically treated genomic DNA (Homo sapiens)
<400> 113
   ctaaaacctc aacctaac 18
<210> 114
   <211> 23
   <212> DNA
   <213> Artificial Sequence
<220>
   <223> chemically treated genomic DNA (Homo sapiens)
<400> 114
   aaattactac catctccaac tac 23
<210> 115
   <211> 23
   <212> DNA
   <213> Artificial Sequence
<220>
   <223> chemically treated genomic DNA (Homo sapiens)
<400> 115
   tataaaaaat tacttcccac atc 23
<210> 116
   <211> 17
   <212> DNA
   <213> Artificial Sequence
<220>
   <223> chemically treated genomic DNA (Homo sapiens)
<400> 116
   ggaagtgtgt ggtaaag 17
<210> 117
   <211> 32
   <212> DNA
   <213> Artificial Sequence
<220>
   <223> chemically treated genomic DNA (Homo sapiens)
<400> 117
   accatcatat caaaccccac aatcaacaca ca 32
<210> 118
   <211> 31
   <212> DNA
   <213> Artificial Sequence
<220>
   <223> chemically treated genomic DNA (Homo sapiens)
<400> 118
   cctaacatct tctctcaccc caaacaaaac a 31
<210> 119
   <211> 31
   <212> DNA
   <213> Artificial Sequence
<220>
   <223> chemically treated genomic DNA (Homo sapiens)
<400> 119
   caactacaac ccaacccaat ctacaaacat t 31
<210> 120
   <211> 37
   <212> DNA
   <213> Artificial Sequence
<220>
   <223> chemically treated genomic DNA (Homo sapiens)
<400> 120
   ccacatcaaa atcatacaca ctctcaaaca aaaaaca 37
<210> 121
   <211> 28
   <212> DNA
   <213> Artificial Sequence
<220>
   <223> chemically treated genomic DNA (Homo sapiens)
<400> 121
   taaagtgttg gggttttgtt tggttgtt 28
<210> 122
   <211> 25
   <212> DNA
   <213> Artificial Sequence
<220>
   <223> chemically treated genomic DNA (Homo sapiens)
<400> 122
   gttcgaaatg attttattta gttgc 25
<210> 123
   <211> 23
   <212> DNA
   <213> Artificial Sequence
<220>
   <223> chemically treated genomic DNA (Homo sapiens)
<400> 123
   aacgaaacaa ataccgtaaa cga 23
<210> 124
   <211> 22
   <212> DNA
   <213> Artificial Sequence
<220>
   <223> chemically treated genomic DNA (Homo sapiens)
<400> 124
   ggttgggttt tttaacgttc gt 22
<210> 125
   <211> 16
   <212> DNA
   <213> Artificial Sequence
<220>
   <223> chemically treated genomic DNA (Homo sapiens)
<400> 125
   ggtttcgggg acgcgt 16
<210> 126
   <211> 23
   <212> DNA
   <213> Artificial Sequence
<220>
   <223> chemically treated genomic DNA (Homo sapiens)
<400> 126
   aaacaaacgt ccgaaaaaaa cga 23
<210> 127
   <211> 17
   <212> DNA
   <213> Artificial Sequence
<220>
   <223> chemically treated genomic DNA (Homo sapiens)
<400> 127
   cgttgatcgc ggggttc 17
<210> 128
   <211> 24
   <212> DNA
   <213> Artificial Sequence
<220>
   <223> chemically treated genomic DNA (Homo sapiens)
<400> 128
   ccgactactt ctacatttcg aacg 24
<210> 129
   <211> 20
   <212> DNA
   <213> Artificial Sequence
<220>
   <223> chemically treated genomic DNA (Homo sapiens)
<400> 129
   atcgggtcgg gttgtagttg 20
<210> 130
   <211> 17
   <212> DNA
   <213> Artificial Sequence
<220>
   <223> chemically treated genomic DNA (Homo sapiens)
<400> 130
   ttcgttcgag agcgcgt 17
<210> 131
   <211> 18
   <212> DNA
   <213> Artificial Sequence
<220>
   <223> chemically treated genomic DNA (Homo sapiens)
<400> 131
   caaacgaaac cccgacgc 18
<210> 132
   <211> 4333
   <212> DNA
   <213> Homo Sapiens
<400> 132
   atgtatatct taatgaatta tagaaagcta tctaaatgaa ctaattcatg ctagggtaca      60
   gatacacttc attcatttta aaagatgagc ttcttatcaa aaaggaattt ctaattagtg     120
   gcattacaac tttgaagcca acatggtatg tatattcaac caatgtaata aaacactcca     180
   gttctgatat gagacaaatg tttctaatca ttttcgggct gaaattctac cctagagaag     240
   cttgctttat gaaacaatca ggaatgagat ttcaggaaat tatgagaact tgacatgatc     300
   ctttgaattg atcttcgaat gagttgtctt tcatttattt tctatgggga attttaggat     360
   aatgaaggag caaatatttc tccttttgaa atgtatgttt ttgttcatta ctgttcttgg     420
   gaggggtgaa gaataaggtg gttcgtgtct aggagtctgc aagtacataa gcctactaca     480
   tacagtgctt tgggtaaaaa ctgacatcaa atttgctttg gcatacacct gtattgtcct     540
   caacaactgc cttaaaattt ggctttccca gtcattaccc tcactcagca tctccttgat     600
   ctctcctctc catattcaaa cacattttga aggcttcctg tcttctctct ctctctctct     660
   ctctctctct gtatacacac cccattctca gggaagaaaa ctatcttgtt aacctgacaa     720
   ccttacctaa acttattgga gagagcagaa ttcaaaccct atggcaatct tttggcaatg     780
   tcctggagga tttgtgtccc caagacaggg atttcagaaa ggggaatata caaatctagc     840
   agtaagatac tttttctcag caggagtttc tagtctcttt caaatacctt gcaatagagg     900
   atggcttgct ccaatacaca cccgcggttc tatgagtctg tactgaagtc ggatatccat     960
   aatattttcc ccaacatcct atcatttttc tcccttgaca accatgtttt gagaacttga    1020
   agcaacagat atgaagagtc ttaagggatt gcatcaatgt acgctaagat tagtctctac    1080
   cacatagtcc aatgacacgg ttttaggttc agaagggatg ctctacaccc acagaatccc    1140
   accgagttca ggtgccagct cagagaactc atcaccttcc tgtttccctt caagagttaa    1200
   ctgatgggat agtatgcaaa gcacatgcgt tcttgctgcc aaaatagcag ccaaaactgc    1260
   ctgcctagat gtgtaagatc ccatgaaatg tcttggcaaa agctagttta ggagtgttgg    1320
   ccaaggtgct gaagcgaagc aaggggagtc aggaccacct ttgccactga atctcccctc    1380
   ccccacctct ttttaaagac agcgagctta ttcattttga caagataccc taggatttca    1440
   aatctgacat aattttggag aaagcagtgc gtgtgtgtgt gtgtgtgtgt gtgtgtgtgt    1500
   gtggtgtaga agggtatctg ggatacataa tctttataga aattctagtg ccacaaaaat    1560
   tctaggagga tctacattta aaaaaacaca caacagttta agctgtttat tccaattgaa    1620
   cgctgtgtgt ttccatcaga tcctcagtga gattcagtcc aatgagtcaa ttgcaaaata    1680
   aagtttttgt caagggaatg gcagaaaatg acaaaaccac aattctgaat gatcattcaa    1740
   aagtaaaaag tactttctac tactgtgtgg agattttctt atctctactt ttactcgaat    1800
   tgaatctttt cttctaatgt tcttttgaac agtttgtctt tggtgtcctg aaaatgtgca    1860
   cgctgatagc agtaccacat gctgtccctg gcacagagtg agctaacagt tgaaccacct    1920
   ctgggtaact gcggactgca gctagtgtaa gtgcaagaga aggcaccctg gccaccaatt    1980
   cgtttcacac tccaatcacc cccgcccgct ctacaccccc tgggcacccg atgcctcttc    2040
   cctctgccag gcaatttgct tccgttctgt tcaactttcc gacctctggc tttagagaat    2100
   agtcctggct gtgggaactg gctctgggaa gcttttggcg ccctcctcat tggtgactgg    2160
   caagagtaac actttcacac tcttaaaaag agccgtttag ttcctaatga tattttctgg    2220
   caaaaaagaa aatgaaacac acaacccgga ttccggtcca ccaaacgcag gccaagagcg    2280
   cggctgctga gccccggtct tgctgtgagg ctgttgttcc cactcgcttc ccgtttccac    2340
   tagagttgat ttgtctcttg ccacctctcc ccggcagtgt ggacctactg caacgcagga    2400
   acatctgggt tggtatggat aggatcctag catcagatgc tggctgctgc cccagggact    2460
   tcccgcggaa atcacccagc acttagaagc cgtttcaata aggaagtaac cagtaaagca    2520
   ttgctggtgg gagaaccccg cgaaacatct tagaaaaata tcagaaaccg ctggagacct    2580
   aatgacccac gtctcctcca gcctgtgttc ctcagccctt tctggaggag tattaagtgg    2640
   tttgtgggtt tttaccctgc ggtgggaggg ctgttccaag tacatctatt tctgcgatag    2700
   ttaacgtcgg ggggccccag ctctaaccag ggcgaggacc acggactgca tccctctctc    2760
   tcagcgcact ggtctgttta ctggagggtg agagggacgc gcagacctcc ggactcaggg    2820
   gttctgttct gtagatcccc ccaccccacc cggggtctct ggcactcgta tcacttagca   2880
   ccagtgtgac tggcggtgca ttccggcccc ctcgcgcccc attaaagccc ctgcacatcc   2940
   tctcccacgc tgcgcttctg ctacccacgc agctgctcct ctgagcgtcc cgcgtgcaca   3000
   aacgcgcccc cggtccttac gcgtcggtgg cgctgcctcg ctgaggtggc gccgggggag   3060
   cccgcagagg cggcggggga gggagtgcgg aggagaggac ccgtgagctg cggggacctg   3120
   ggcccgccga gcttttgggg aagctgggct ccgttccccc cgggcgcctg gggcgcgcgg   3180
   gaaggcgcgg aggacgcgcc gcactcaccc gctgctgctg ccggctgctg actgccaccg   3240
   cggcggcgga cgcggcgctg tgccggagct cggccgcctg cccaggtctc aacaggctcc   3300
   gggtgaacct gcgggtccgc tcaccggcca tcgccggtca cgccaatcgc gccgcgggcc   3360
   cggggcgcgc ctcccgccgg tcttccccgc gccaaccttc tctatccacc cgccgttcag   3420
   gaagcggaac tcggagcctt cccccccctc ttcctcgctc aactcgcaag aggcgggttt   3480
   ttctctcttg cacccccact cttcccaccc ccttccctcc tctgaagctt ctcgaagact   3540
   ttccaaactc tgcgctcccc cgggcgcgcg cggccaactc ggctggcggc cgggccctgc   3600
   agcggcgcat ctggagcggg cgtcgcggga cgcgtgtggg gcggctcctg cccgcgccgc   3660
   ccacacgggc tcagctggcc ggccggggct gtccacgccg cccgggggaa ctgcaggctg   3720
   cgggggaggg cggggggcgg ccgccgaggg gctccgggcg gggcctgtcc aaggccgtcg   3780
   cctgtccaag gccgtcagcc tcatttggag gttgctggga gagttggagc cctggatgca   3840
   tttgccgttt agacttctat tttctgggtc cggctagtgg gtgggggcca agctcggttc   3900
   gcccttccac cctcgggaag gggtcagagg cttattaaag atctggtggc tgggatccca   3960
   gcagcatcgc ccaagccagc gtttggaagg ctcccttgcc agaagtccat gttcagggcc   4020
   ttggaataag cttccccatt cattcactcc ttcaatgagt atttattaag cgcctactat   4080
   atgccagctc acactgttct ggaaactgag gatacggaga ggaacttagc aactacactt   4140
   taaggctgtg tagagctgat aggttttttc ggggtagggg tgggagacag caagtaacca   4200
   agcgaacaaa taaacaaata cagagaaatc aaggagacag tttaagctac taacacgaac   4260
   tcaaaaccaa taaatcagcg ttctgggggt aagagacttt gggtggcatt aggatgagta   4320
   ggcaagaacc aac                                                       4333
<210> 133
   <211> 2333
   <212> DNA
   <213> Homo Sapiens
<400> 133
   accatgtttt gagaacttga agcaacagat atgaagagtc ttaagggatt gcatcaatgt      60
   acgctaagat tagtctctac cacatagtcc aatgacacgg ttttaggttc agaagggatg     120
   ctctacaccc acagaatccc accgagttca ggtgccagct cagagaactc atcaccttcc     180
   tgtttccctt caagagttaa ctgatgggat agtatgcaaa gcacatgcgt tcttgctgcc     240
   aaaatagcag ccaaaactgc ctgcctagat gtgtaagatc ccatgaaatg tcttggcaaa     300
   agctagttta ggagtgttgg ccaaggtgct gaagcgaagc aaggggagtc aggaccacct     360
   ttgccactga atctcccctc ccccacctct ttttaaagac agcgagctta ttcattttga     420
   caagataccc taggatttca aatctgacat aattttggag aaagcagtgc gtgtgtgtgt     480
   gtgtgtgtgt gtgtgtgtgt gtggtgtaga agggtatctg ggatacataa tctttataga     540
   aattctagtg ccacaaaaat tctaggagga tctacattta aaaaaacaca caacagttta     600
   agctgtttat tccaattgaa cgctgtgtgt ttccatcaga tcctcagtga gattcagtcc     660
   aatgagtcaa ttgcaaaata aagtttttgt caagggaatg gcagaaaatg acaaaaccac     720
   aattctgaat gatcattcaa aagtaaaaag tactttctac tactgtgtgg agattttctt     780
   atctctactt ttactcgaat tgaatctttt cttctaatgt tcttttgaac agtttgtctt     840
   tggtgtcctg aaaatgtgca cgctgatagc agtaccacat gctgtccctg gcacagagtg     900
   agctaacagt tgaaccacct ctgggtaact gcggactgca gctagtgtaa gtgcaagaga     960
   aggcaccctg gccaccaatt cgtttcacac tccaatcacc cccgcccgct ctacaccccc     1020
   tgggcacccg atgcctcttc cctctgccag gcaatttgct tccgttctgt tcaactttcc    1080
   gacctctggc tttagagaat agtcctggct gtgggaactg gctctgggaa gcttttggcg    1140
   ccctcctcat tggtgactgg caagagtaac actttcacac tcttaaaaag agccgtttag    1200
   ttcctaatga tattttctgg caaaaaagaa aatgaaacac acaacccgga ttccggtcca    1260
   ccaaacgcag gccaagagcg cggctgctga gccccggtct tgctgtgagg ctgttgttcc    1320
   cactcgcttc ccgtttccac tagagttgat ttgtctcttg ccacctctcc ccggcagtgt    1380
   ggacctactg caacgcagga acatctgggt tggtatggat aggatcctag catcagatgc    1440
   tggctgctgc cccagggact tcccgcggaa atcacccagc acttagaagc cgtttcaata    1500
   aggaagtaac cagtaaagca ttgctggtgg gagaaccccg cgaaacatct tagaaaaata    1560
   tcagaaaccg ctggagacct aatgacccac gtctcctcca gcctgtgttc ctcagccctt    1620
   tctggaggag tattaagtgg tttgtgggtt tttaccctgc ggtgggaggg ctgttccaag    1680
   tacatctatt tctgcgatag ttaacgtcgg ggggccccag ctctaaccag ggcgaggacc    1740
   acggactgca tccctctctc tcagcgcact ggtctgttta ctggagggtg agagggacgc    1800
   gcagacctcc ggactcaggg gttctgttct gtagatcccc ccaccccacc cggggtctct    1860
   ggcactcgta tcacttagca ccagtgtgac tggcggtgca ttccggcccc ctcgcgcccc    1920
   attaaagccc ctgcacatcc tctcccacgc tgcgcttctg ctacccacgc agctgctcct    1980
   ctgagcgtcc cgcgtgcaca aacgcgcccc cggtccttac gcgtcggtgg cgctgcctcg    2040
   ctgaggtggc gccgggggag cccgcagagg cggcggggga gggagtgcgg aggagaggac    2100
   ccgtgagctg cggggacctg ggcccgccga gcttttgggg aagctgggct ccgttccccc    2160
   cgggcgcctg ggcgcgcggg gaaggcgcgg aggacgcgcc gcactcaccc gctgctgctg    2220
   ccggctgctg actgccaccg cggcggcgga cgcggcgctg tgccggagct cggccgcctg    2280
   cccaggtctc aacaggctcc gggtgaacct gcgggtccgc tcaccggcca tcg           2333
<210> 134
   <211> 4333
   <212> DNA
   <213> Artificial Sequence
<220>
   <223> chemically treated genomic DNA (Homo sapiens)
<400> 134
   atgtatattt taatgaatta tagaaagtta tttaaatgaa ttaatttatg ttagggtata      60
   gatatatttt atttatttta aaagatgagt tttttattaa aaaggaattt ttaattagtg     120
   gtattataat tttgaagtta atatggtatg tatatttaat taatgtaata aaatatttta     180
   gttttgatat gagataaatg tttttaatta ttttcgggtt gaaattttat tttagagaag     240
   tttgttttat gaaataatta ggaatgagat tttaggaaat tatgagaatt tgatatgatt     300
   ttttgaattg attttcgaat gagttgtttt ttatttattt tttatgggga attttaggat     360
   aatgaaggag taaatatttt tttttttgaa atgtatgttt ttgtttatta ttgtttttgg     420
   gaggggtgaa gaataaggtg gttcgtgttt aggagtttgt aagtatataa gtttattata     480
   tatagtgttt tgggtaaaaa ttgatattaa atttgttttg gtatatattt gtattgtttt     540
   taataattgt tttaaaattt ggttttttta gttattattt ttatttagta tttttttgat     600
   tttttttttt tatatttaaa tatattttga aggttttttg tttttttttt tttttttttt     660
   tttttttttt gtatatatat tttattttta gggaagaaaa ttattttgtt aatttgataa     720
   ttttatttaa atttattgga gagagtagaa tttaaatttt atggtaattt tttggtaatg     780
   ttttggagga tttgtgtttt taagataggg attttagaaa ggggaatata taaatttagt     840
   agtaagatat ttttttttag taggagtttt tagttttttt taaatatttt gtaatagagg     900
   atggtttgtt ttaatatata ttcgcggttt tatgagtttg tattgaagtc ggatatttat     960
   aatatttttt ttaatatttt attatttttt ttttttgata attatgtttt gagaatttga    1020
   agtaatagat atgaagagtt ttaagggatt gtattaatgt acgttaagat tagtttttat    1080
   tatatagttt aatgatacgg ttttaggttt agaagggatg ttttatattt atagaatttt    1140
   atcgagttta ggtgttagtt tagagaattt attatttttt tgtttttttt taagagttaa    1200
   ttgatgggat agtatgtaaa gtatatgcgt ttttgttgtt aaaatagtag ttaaaattgt    1260
   ttgtttagat gtgtaagatt ttatgaaatg ttttggtaaa agttagttta ggagtgttgg    1320
   ttaaggtgtt gaagcgaagt aaggggagtt aggattattt ttgttattga attttttttt    1380
   ttttattttt ttttaaagat agcgagttta tttattttga taagatattt taggatttta    1440
   aatttgatat aattttggag aaagtagtgc gtgtgtgtgt gtgtgtgtgt gtgtgtgtgt    1500
   gtggtgtaga agggtatttg ggatatataa tttttataga aattttagtg ttataaaaat    1560
   tttaggagga tttatattta aaaaaatata taatagttta agttgtttat tttaattgaa    1620
   cgttgtgtgt ttttattaga tttttagtga gatttagttt aatgagttaa ttgtaaaata    1680
   aagtttttgt taagggaatg gtagaaaatg ataaaattat aattttgaat gattatttaa    1740
   aagtaaaaag tattttttat tattgtgtgg agattttttt atttttattt ttattcgaat    1800
   tgaatttttt tttttaatgt ttttttgaat agtttgtttt tggtgttttg aaaatgtgta    1860
   cgttgatagt agtattatat gttgtttttg gtatagagtg agttaatagt tgaattattt    1920
   ttgggtaatt gcggattgta gttagtgtaa gtgtaagaga aggtattttg gttattaatt    1980
   cgttttatat tttaattatt ttcgttcgtt ttatattttt tgggtattcg atgttttttt    2040
   tttttgttag gtaatttgtt ttcgttttgt ttaatttttc gatttttggt tttagagaat    2100
   agttttggtt gtgggaattg gttttgggaa gtttttggcg ttttttttat tggtgattgg    2160
   taagagtaat atttttatat ttttaaaaag agtcgtttag tttttaatga tattttttgg    2220
   taaaaaagaa aatgaaatat ataattcgga tttcggttta ttaaacgtag gttaagagcg    2280
   cggttgttga gtttcggttt tgttgtgagg ttgttgtttt tattcgtttt tcgtttttat    2340
   tagagttgat ttgttttttg ttattttttt tcggtagtgt ggatttattg taacgtagga    2400
   atatttgggt tggtatggat aggattttag tattagatgt tggttgttgt tttagggatt    2460
   tttcgcggaa attatttagt atttagaagt cgttttaata aggaagtaat tagtaaagta    2520
   ttgttggtgg gagaatttcg cgaaatattt tagaaaaata ttagaaatcg ttggagattt    2580
   aatgatttac gtttttttta gtttgtgttt tttagttttt tttggaggag tattaagtgg    2640
   tttgtgggtt tttattttgc ggtgggaggg ttgttttaag tatatttatt tttgcgatag    2700
   ttaacgtcgg ggggttttag ttttaattag ggcgaggatt acggattgta tttttttttt    2760
   ttagcgtatt ggtttgttta ttggagggtg agagggacgc gtagattttc ggatttaggg    2820
   gttttgtttt gtagattttt ttattttatt cggggttttt ggtattcgta ttatttagta    2880
   ttagtgtgat tggcggtgta tttcggtttt ttcgcgtttt attaaagttt ttgtatattt    2940
   ttttttacgt tgcgtttttg ttatttacgt agttgttttt ttgagcgttt cgcgtgtata    3000
   aacgcgtttt cggtttttac gcgtcggtgg cgttgtttcg ttgaggtggc gtcgggggag    3060
   ttcgtagagg cggcggggga gggagtgcgg aggagaggat tcgtgagttg cggggatttg    3120
   ggttcgtcga gtttttgggg aagttgggtt tcgttttttt cgggcgtttg gggcgcgcgg    3180
   gaaggcgcgg aggacgcgtc gtatttattc gttgttgttg tcggttgttg attgttatcg    3240
   cggcggcgga cgcggcgttg tgtcggagtt cggtcgtttg tttaggtttt aataggtttc    3300
   gggtgaattt gcgggttcgt ttatcggtta tcgtcggtta cgttaatcgc gtcgcgggtt    3360
   cggggcgcgt ttttcgtcgg tttttttcgc gttaattttt tttatttatt cgtcgtttag    3420
   gaagcggaat tcggagtttt tttttttttt tttttcgttt aattcgtaag aggcgggttt    3480
   tttttttttg tatttttatt ttttttattt tttttttttt tttgaagttt ttcgaagatt    3540
   ttttaaattt tgcgtttttt cgggcgcgcg cggttaattc ggttggcggt cgggttttgt    3600
   agcggcgtat ttggagcggg cgtcgcggga cgcgtgtggg gcggtttttg ttcgcgtcgt    3660
   ttatacgggt ttagttggtc ggtcggggtt gtttacgtcg ttcgggggaa ttgtaggttg    3720
   cgggggaggg cggggggcgg tcgtcgaggg gtttcgggcg gggtttgttt aaggtcgtcg    3780
   tttgtttaag gtcgttagtt ttatttggag gttgttggga gagttggagt tttggatgta    3840
   tttgtcgttt agatttttat tttttgggtt cggttagtgg gtgggggtta agttcggttc    3900
   gtttttttat tttcgggaag gggttagagg tttattaaag atttggtggt tgggatttta    3960
   gtagtatcgt ttaagttagc gtttggaagg tttttttgtt agaagtttat gtttagggtt    4020
   ttggaataag tttttttatt tatttatttt tttaatgagt atttattaag cgtttattat    4080
   atgttagttt atattgtttt ggaaattgag gatacggaga ggaatttagt aattatattt    4140
   taaggttgtg tagagttgat aggttttttc ggggtagggg tgggagatag taagtaatta    4200
   agcgaataaa taaataaata tagagaaatt aaggagatag tttaagttat taatacgaat    4260
   ttaaaattaa taaattagcg ttttgggggt aagagatttt gggtggtatt aggatgagta    4320
   ggtaagaatt aat                                                       4333
<210> 135
   <211> 4333
   <212> DNA
   <213> Artificial Sequence
<220>
   <223> chemically treated genomic DNA (Homo sapiens)
<400> 135
   gttggttttt gtttatttat tttaatgtta tttaaagttt tttattttta gaacgttgat      60
   ttattggttt tgagttcgtg ttagtagttt aaattgtttt tttgattttt ttgtatttgt     120
   ttatttgttc gtttggttat ttgttgtttt ttatttttat ttcgaaaaaa tttattagtt     180
   ttatatagtt ttaaagtgta gttgttaagt tttttttcgt atttttagtt tttagaatag    240
   tgtgagttgg tatatagtag gcgtttaata aatatttatt gaaggagtga atgaatgggg     300
   aagtttattt taaggttttg aatatggatt tttggtaagg gagtttttta aacgttggtt     360
   tgggcgatgt tgttgggatt ttagttatta gatttttaat aagtttttga ttttttttcg     420
   agggtggaag ggcgaatcga gtttggtttt tatttattag tcggatttag aaaatagaag     480
   tttaaacggt aaatgtattt agggttttaa tttttttagt aatttttaaa tgaggttgac      540
   ggttttggat aggcgacggt tttggatagg tttcgttcgg agtttttcgg cggtcgtttt     600
   tcgttttttt tcgtagtttg tagttttttc gggcggcgtg gatagtttcg gtcggttagt      660
   tgagttcgtg tgggcggcgc gggtaggagt cgttttatac gcgtttcgcg acgttcgttt     720
   tagatgcgtc gttgtagggt tcggtcgtta gtcgagttgg tcgcgcgcgt tcgggggagc     780
   gtagagtttg gaaagttttc gagaagtttt agaggaggga agggggtggg aagagtgggg    840
   gtgtaagaga gaaaaattcg ttttttgcga gttgagcgag gaagaggggg gggaaggttt     900
   cgagtttcgt tttttgaacg gcgggtggat agagaaggtt ggcgcgggga agatcggcgg     960
   gaggcgcgtt tcgggttcgc ggcgcgattg gcgtgatcgg cgatggtcgg tgagcggatt    1020
   cgtaggttta ttcggagttt gttgagattt gggtaggcgg tcgagtttcg gtatagcgtc    1080
   gcgttcgtcg tcgcggtggt agttagtagt cggtagtagt agcgggtgag tgcggcgcgt    1140
   ttttcgcgtt ttttcgcgcg ttttaggcgt tcggggggaa cggagtttag tttttttaaa   1200
   agttcggcgg gtttaggttt tcgtagttta cgggtttttt ttttcgtatt ttttttttcg    1260
   tcgtttttgc gggttttttc ggcgttattt tagcgaggta gcgttatcga cgcgtaagga   1320
   tcgggggcgc gtttgtgtac gcgggacgtt tagaggagta gttgcgtggg tagtagaagc    1380
   gtagcgtggg agaggatgtg taggggtttt aatggggcgc gagggggtcg gaatgtatcg    1440
   ttagttatat tggtgttaag tgatacgagt gttagagatt tcgggtgggg tggggggatt   1500
   tatagaatag aatttttgag ttcggaggtt tgcgcgtttt ttttattttt tagtaaatag    1560
   attagtgcgt tgagagagag ggatgtagtt cgtggttttc gttttggtta gagttggggt    1620
   ttttcgacgt taattatcgt agaaatagat gtatttggaa tagttttttt atcgtagggt 1680
   aaaaatttat aaattattta atattttttt agaaagggtt gaggaatata ggttggagga 1740
   gacgtgggtt attaggtttt tagcggtttt tgatattttt ttaagatgtt tcgcggggtt 1800
   tttttattag taatgtttta ttggttattt ttttattgaa acggttttta agtgttgggt 1860
   gattttcgcg ggaagttttt ggggtagtag ttagtatttg atgttaggat tttatttata 1920
   ttaatttaga tgtttttgcg ttgtagtagg tttatattgt cggggagagg tggtaagaga 1980
   taaattaatt ttagtggaaa cgggaagcga gtgggaataa tagttttata gtaagatcgg 2040
   ggtttagtag tcgcgttttt ggtttgcgtt tggtggatcg gaattcgggt tgtgtgtttt 2100
   attttttttt ttgttagaaa atattattag gaattaaacg gtttttttta agagtgtgaa 2160
   agtgttattt ttgttagtta ttaatgagga gggcgttaaa agttttttag agttagtttt 2220
   tatagttagg attatttttt aaagttagag gtcggaaagt tgaatagaac ggaagtaaat 2280
   tgtttggtag agggaagagg tatcgggtgt ttagggggtg tagagcgggc gggggtgatt 2340
   ggagtgtgaa acgaattggt ggttagggtg tttttttttg tatttatatt agttgtagtt 2400
   cgtagttatt tagaggtggt ttaattgtta gtttattttg tgttagggat agtatgtggt 2460
   attgttatta gcgtgtatat ttttaggata ttaaagataa attgtttaaa agaatattag 2520
   aagaaaagat ttaattcgag taaaagtaga gataagaaaa tttttatata gtagtagaaa 2580
   gtatttttta tttttgaatg attatttaga attgtggttt tgttattttt tgttattttt 2640
   ttgataaaaa ttttattttg taattgattt attggattga attttattga ggatttgatg 2700
   gaaatatata gcgtttaatt ggaataaata gtttaaattg ttgtgtgttt ttttaaatgt 2760
   agattttttt agaatttttg tggtattaga atttttataa agattatgta ttttagatat 2820
   ttttttatat tatatatata tatatatata tatatatata tacgtattgt tttttttaaa 2880
   attatgttag atttgaaatt ttagggtatt ttgttaaaat gaataagttc gttgttttta 2940
   aaaagaggtg ggggagggga gatttagtgg taaaggtggt tttgattttt tttgtttcgt 3000
   tttagtattt tggttaatat ttttaaatta gtttttgtta agatatttta tgggatttta 3060
   tatatttagg taggtagttt tggttgttat tttggtagta agaacgtatg tgttttgtat 3120
   attattttat tagttaattt ttgaagggaa ataggaaggt gatgagtttt ttgagttggt 3180
   atttgaattc ggtgggattt tgtgggtgta gagtattttt tttgaattta aaatcgtgtt 3240
   attggattat gtggtagaga ttaattttag cgtatattga tgtaattttt taagattttt 3300
   tatatttgtt gttttaagtt tttaaaatat ggttgttaag ggagaaaaat gataggatgt 3360
   tggggaaaat attatggata ttcgatttta gtatagattt atagaatcgc gggtgtgtat 3420
   tggagtaagt tattttttat tgtaaggtat ttgaaagaga ttagaaattt ttgttgagaa 3480
   aaagtatttt attgttagat ttgtatattt ttttttttga aatttttgtt ttggggatat 3540
   aaatttttta ggatattgtt aaaagattgt tatagggttt gaattttgtt ttttttaata 3600
   agtttaggta aggttgttag gttaataaga tagttttttt ttttgagaat ggggtgtgta 3660
   tatagagaga gagagagaga gagagagaga agataggaag tttttaaaat gtgtttgaat 3720
   atggagagga gagattaagg agatgttgag tgagggtaat gattgggaaa gttaaatttt 3780
   aaggtagttg ttgaggataa tataggtgta tgttaaagta aatttgatgt tagtttttat 3840
   ttaaagtatt gtatgtagta ggtttatgta tttgtagatt tttagatacg aattatttta 3900
   ttttttattt tttttaagaa tagtaatgaa taaaaatata tattttaaaa ggagaaatat 3960
   ttgttttttt attattttaa aattttttat agaaaataaa tgaaagataa tttattcgaa 4020
   gattaattta aaggattatg ttaagttttt ataatttttt gaaattttat ttttgattgt 4080
   tttataaagt aagttttttt agggtagaat tttagttcga aaatgattag aaatatttgt 4140
   tttatattag aattggagtg ttttattata ttggttgaat atatatatta tgttggtttt 4200
   aaagttgtaa tgttattaat tagaaatttt tttttgataa gaagtttatt ttttaaaatg 4260
   aatgaagtgt atttgtattt tagtatgaat tagtttattt agatagtttt ttataattta 4320
   ttaagatata tat 4333
<210> 136
   <211> 2333
   <212> DNA
   <213> Artificial Sequence
<220>
   <223> chemically treated genomic DNA (Homo sapiens)
<400> 136
   attatgtttt gagaatttga agtaatagat atgaagagtt ttaagggatt gtattaatgt      60
   acgttaagat tagtttttat tatatagttt aatgatacgg ttttaggttt agaagggatg     120
   ttttatattt atagaatttt atcgagttta ggtgttagtt tagagaattt attatttttt     180
   tgtttttttt taagagttaa ttgatgggat agtatgtaaa gtatatgcgt ttttgttgtt     240
   aaaatagtag ttaaaattgt ttgtttagat gtgtaagatt ttatgaaatg ttttggtaaa     300
   agttagttta ggagtgttgg ttaaggtgtt gaagcgaagt aaggggagtt aggattattt     360
   ttgttattga attttttttt ttttattttt ttttaaagat agcgagttta tttattttga     420
   taagatattt taggatttta aatttgatat aattttggag aaagtagtgc gtgtgtgtgt     480
   gtgtgtgtgt gtgtgtgtgt gtggtgtaga agggtatttg ggatatataa tttttataga     540
   aattttagtg ttataaaaat tttaggagga tttatattta aaaaaatata taatagttta     600
   agttgtttat tttaattgaa cgttgtgtgt ttttattaga tttttagtga gatttagttt     660
   aatgagttaa ttgtaaaata aagtttttgt taagggaatg gtagaaaatg ataaaattat     720
   aattttgaat gattatttaa aagtaaaaag tattttttat tattgtgtgg agattttttt     780
   atttttattt ttattcgaat tgaatttttt tttttaatgt ttttttgaat agtttgtttt     840
   tggtgttttg aaaatgtgta cgttgatagt agtattatat gttgtttttg  gtatagagtg     900
   agttaatagt tgaattattt ttgggtaatt gcggattgta gttagtgtaa gtgtaagaga     960
   aggtattttg gttattaatt cgttttatat tttaattatt ttcgttcgtt ttatattttt    1020
   tgggtattcg atgttttttt tttttgttag gtaatttgtt ttcgttttgt ttaatttttc    1080
   gatttttggt tttagagaat agttttggtt gtgggaattg gttttgggaa gtttttggcg    1140
   ttttttttat tggtgattgg taagagtaat atttttatat ttttaaaaag agtcgtttag    1200
   tttttaatga tattttttgg taaaaaagaa aatgaaatat ataattcgga tttcggttta    1260
   ttaaacgtag gttaagagcg cggttgttga gtttcggttt tgttgtgagg ttgttgtttt    1320
   tattcgtttt tcgtttttat tagagttgat ttgttttttg  ttattttttt tcggtagtgt    1380
   ggatttattg taacgtagga atatttgggt tggtatggat aggattttag tattagatgt    1440
   tggttgttgt tttagggatt tttcgcggaa attatttagt atttagaagt cgttttaata    1500
   aggaagtaat tagtaaagta ttgttggtgg gagaatttcg cgaaatattt tagaaaaata    1560
   ttagaaatcg ttggagattt aatgatttac gtttttttta gtttgtgttt tttagttttt    1620
   tttggaggag tattaagtgg tttgtgggtt tttattttgc ggtgggaggg ttgttttaag    1680
   tatatttatt tttgcgatag ttaacgtcgg ggggttttag ttttaattag ggcgaggatt    1740
   acggattgta tttttttttt ttagcgtatt ggtttgttta ttggagggtg agagggacgc    1800
   gtagattttc ggatttaggg gttttgtttt gtagattttt ttattttatt cggggttttt    1860
   ggtattcgta ttatttagta ttagtgtgat tggcggtgta tttcggtttt ttcgcgtttt    1920
   attaaagttt ttgtatattt ttttttacgt tgcgtttttg ttatttacgt agttgttttt    1980
   ttgagcgttt cgcgtgtata aacgcgtttt cggtttttac gcgtcggtgg cgttgtttcg    2040
   ttgaggtggc gtcgggggag ttcgtagagg cggcggggga gggagtgcgg aggagaggat    2100
   tcgtgagttg cggggatttg ggttcgtcga gtttttgggg aagttgggtt tcgttttttt    2160
   cgggcgtttg gggcgcgcgg  gaaggcgcgg aggacgcgtc gtatttattc gttgttgttg    2220
   tcggttgttg attgttatcg cggcggcgga cgcggcgttg tgtcggagtt cggtcgtttg    2280
   tttaggtttt aataggtttc gggtgaattt gcgggttcgt ttatcggtta tcg           2333
<210> 137
   <211> 2333
   <212> DNA
   <213> Artificial Sequence
<220>
   <223> chemically treated genomic DNA (Homo sapiens)
<400> 137
   cgatggtcgg tgagcggatt cgtaggttta ttcggagttt gttgagattt gggtaggcgg      60
   tcgagtttcg gtatagcgtc gcgttcgtcg tcgcggtggt agttagtagt cggtagtagt     120
   agcgggtgag tgcggcgcgt ttttcgcgtt  ttttcgcgcg ttttaggcgt tcggggggaa     180
   cggagtttag tttttttaaa agttcggcgg gtttaggttt tcgtagttta cgggtttttt    240
   ttttcgtatt ttttttttcg tcgtttttgc gggttttttc ggcgttattt tagcgaggta     300
   gcgttatcga cgcgtaagga tcgggggcgc gtttgtgtac gcgggacgtt tagaggagta     360
   gttgcgtggg tagtagaagc gtagcgtggg agaggatgtg taggggtttt aatggggcgc     420
   gagggggtcg gaatgtatcg ttagttatat tggtgttaag tgatacgagt gttagagatt     480
   tcgggtgggg tggggggatt tatagaatag aatttttgag ttcggaggtt tgcgcgtttt     540
   ttttattttt tagtaaatag attagtgcgt tgagagagag ggatgtagtt cgtggttttc      600
   gttttggtta gagttggggt ttttcgacgt taattatcgt agaaatagat gtatttggaa     660
   tagttttttt atcgtagggt aaaaatttat aaattattta atattttttt agaaagggtt    720
   gaggaatata ggttggagga gacgtgggtt attaggtttt tagcggtttt tgatattttt    780
   ttaagatgtt tcgcggggtt tttttattag taatgtttta ttggttattt ttttattgaa   840
   acggttttta agtgttgggt gattttcgcg ggaagttttt ggggtagtag ttagtatttg    900
   atgttaggat tttatttata ttaatttaga tgtttttgcg ttgtagtagg tttatattgt    960
   cggggagagg tggtaagaga taaattaatt ttagtggaaa cgggaagcga gtgggaataa   1020
   tagttttata gtaagatcgg ggtttagtag tcgcgttttt ggtttgcgtt tggtggatcg   1080
   gaattcgggt tgtgtgtttt attttttttt ttgttagaaa atattattag gaattaaacg  1140
   gtttttttta agagtgtgaa agtgttattt ttgttagtta ttaatgagga gggcgttaaa   1200
   agttttttag agttagtttt tatagttagg attatttttt aaagttagag gtcggaaagt   1260
   tgaatagaac ggaagtaaat tgtttggtag agggaagagg tatcgggtgt ttagggggtg   1320
   tagagcgggc gggggtgatt ggagtgtgaa acgaattggt ggttagggtg tttttttttg   1380
   tatttatatt agttgtagtt cgtagttatt tagaggtggt ttaattgtta gtttattttg   1440
   tgttagggat agtatgtggt attgttatta gcgtgtatat ttttaggata ttaaagataa   1500
   attgtttaaa agaatattag aagaaaagat ttaattcgag taaaagtaga gataagaaaa   1560
   tttttatata gtagtagaaa gtatttttta tttttgaatg attatttaga attgtggttt   1620
   tgttattttt tgttattttt ttgataaaaa ttttattttg taattgattt attggattga   1680
   attttattga ggatttgatg gaaatatata gcgtttaatt ggaataaata gtttaaattg   1740
   ttgtgtgttt ttttaaatgt agattttttt agaatttttg tggtattaga atttttataa   1800
   agattatgta ttttagatat ttttttatat tatatatata tatatatata tatatatata  1860
   tacgtattgt tttttttaaa attatgttag atttgaaatt ttagggtatt ttgttaaaat  1920
   gaataagttc gttgttttta aaaagaggtg ggggagggga gatttagtgg taaaggtggt   1980
   tttgattttt tttgtttcgt tttagtattt tggttaatat ttttaaatta gtttttgtta   2040
   agatatttta tgggatttta tatatttagg taggtagttt tggttgttat tttggtagta   2100
   agaacgtatg tgttttgtat attattttat tagttaattt ttgaagggaa ataggaaggt   2160
   gatgagtttt ttgagttggt atttgaattc ggtgggattt tgtgggtgta gagtattttt   2220
   tttgaattta aaatcgtgtt attggattat gtggtagaga ttaattttag cgtatattga   2280
   tgtaattttt taagattttt tatatttgtt gttttaagtt tttaaaatat ggt          2333
<210> 138
   <211> 4333
   <212> DNA
   <213> Artificial Sequence
<220>
   <223> chemically treated genomic DNA (Homo sapiens)
<400> 138
   atgtatattt taatgaatta tagaaagtta tttaaatgaa ttaatttatg ttagggtata     60
   gatatatttt atttatttta aaagatgagt tttttattaa aaaggaattt ttaattagtg    120
   gtattataat tttgaagtta atatggtatg tatatttaat taatgtaata aaatatttta    180
   gttttgatat gagataaatg tttttaatta tttttgggtt gaaattttat tttagagaag    240
   tttgttttat gaaataatta ggaatgagat tttaggaaat tatgagaatt tgatatgatt    300
   ttttgaattg atttttgaat gagttgtttt ttatttattt tttatgggga attttaggat    360
   aatgaaggag taaatatttt tttttttgaa atgtatgttt ttgtttatta ttgtttttgg    420
   gaggggtgaa gaataaggtg gtttgtgttt aggagtttgt aagtatataa gtttattata    480
   tatagtgttt tgggtaaaaa ttgatattaa atttgttttg gtatatattt gtattgtttt    540
   taataattgt tttaaaattt ggttttttta gttattattt ttatttagta tttttttgat    600
   tttttttttt tatatttaaa tatattttga aggttttttg tttttttttt tttttttttt    660
   tttttttttt gtatatatat tttattttta gggaagaaaa ttattttgtt aatttgataa    720
   ttttatttaa atttattgga gagagtagaa tttaaatttt atggtaattt tttggtaatg    780
   ttttggagga tttgtgtttt taagataggg attttagaaa ggggaatata taaatttagt    840
   agtaagatat ttttttttag taggagtttt tagttttttt taaatatttt gtaatagagg    900
   atggtttgtt ttaatatata tttgtggttt tatgagtttg tattgaagtt ggatatttat    960
   aatatttttt ttaatatttt attatttttt ttttttgata attatgtttt gagaatttga   1020
   agtaatagat atgaagagtt ttaagggatt gtattaatgt atgttaagat tagtttttat   1080
   tatatagttt aatgatatgg ttttaggttt agaagggatg ttttatattt atagaatttt   1140
   attgagttta ggtgttagtt tagagaattt attatttttt tgtttttttt taagagttaa   1200
   ttgatgggat agtatgtaaa gtatatgtgt ttttgttgtt aaaatagtag ttaaaattgt   1260
   ttgtttagat gtgtaagatt ttatgaaatg ttttggtaaa agttagttta ggagtgttgg   1320
   ttaaggtgtt gaagtgaagt aaggggagtt aggattattt ttgttattga attttttttt   1380
   ttttattttt ttttaaagat agtgagttta tttattttga taagatattt taggatttta   1440
   aatttgatat aattttggag aaagtagtgt gtgtgtgtgt gtgtgtgtgt gtgtgtgtgt   1500
   gtggtgtaga agggtatttg ggatatataa tttttataga aattttagtg ttataaaaat   1560
   tttaggagga tttatattta aaaaaatata taatagttta agttgtttat tttaattgaa   1620
   tgttgtgtgt ttttattaga tttttagtga gatttagttt aatgagttaa ttgtaaaata   1680
   aagtttttgt taagggaatg gtagaaaatg ataaaattat aattttgaat gattatttaa   1740
   aagtaaaaag tattttttat tattgtgtgg agattttttt atttttattt ttatttgaat   1800
   tgaatttttt tttttaatgt ttttttgaat agtttgtttt tggtgttttg aaaatgtgta   1860
   tgttgatagt agtattatat gttgtttttg gtatagagtg agttaatagt tgaattattt   1920
   ttgggtaatt gtggattgta gttagtgtaa gtgtaagaga aggtattttg gttattaatt   1980
   tgttttatat tttaattatt tttgtttgtt ttatattttt tgggtatttg atgttttttt   2040
   tttttgttag gtaatttgtt tttgttttgt ttaatttttt gatttttggt tttagagaat   2100
   agttttggtt gtgggaattg gttttgggaa gtttttggtg ttttttttat tggtgattgg   2160
   taagagtaat atttttatat ttttaaaaag agttgtttag tttttaatga tattttttgg   2220
   taaaaaagaa aatgaaatat ataatttgga ttttggttta ttaaatgtag gttaagagtg   2280
   tggttgttga gttttggttt tgttgtgagg ttgttgtttt tatttgtttt ttgtttttat     2340
   tagagttgat ttgttttttg ttattttttt ttggtagtgt ggatttattg taatgtagga     2400
   atatttgggt tggtatggat aggattttag tattagatgt tggttgttgt tttagggatt     2460
   ttttgtggaa attatttagt atttagaagt tgttttaata aggaagtaat tagtaaagta     2520
   ttgttggtgg gagaattttg tgaaatattt tagaaaaata ttagaaattg ttggagattt     2580
   aatgatttat gtttttttta gtttgtgttt tttagttttt tttggaggag tattaagtgg     2640
   tttgtgggtt tttattttgt ggtgggaggg ttgttttaag tatatttatt tttgtgatag     2700
   ttaatgttgg ggggttttag ttttaattag ggtgaggatt atggattgta tttttttttt     2760
   ttagtgtatt ggtttgttta ttggagggtg agagggatgt gtagattttt ggatttaggg     2820
   gttttgtttt gtagattttt ttattttatt tggggttttt ggtatttgta ttatttagta     2880
   ttagtgtgat tggtggtgta ttttggtttt tttgtgtttt attaaagttt ttgtatattt     2940
   ttttttatgt tgtgtttttg ttatttatgt agttgttttt ttgagtgttt tgtgtgtata     3000
   aatgtgtttt tggtttttat gtgttggtgg tgttgttttg ttgaggtggt gttgggggag     3060
   tttgtagagg tggtggggga gggagtgtgg aggagaggat ttgtgagttg tggggatttg     3120
   ggtttgttga gtttttgggg aagttgggtt ttgttttttt tgggtgtttg gggtgtgtgg     3180
   gaaggtgtgg aggatgtgtt gtatttattt gttgttgttg ttggttgttg attgttattg     3240
   tggtggtgga tgtggtgttg tgttggagtt tggttgtttg tttaggtttt aataggtttt     3300
   gggtgaattt gtgggtttgt ttattggtta ttgttggtta tgttaattgt gttgtgggtt     3360
   tggggtgtgt tttttgttgg ttttttttgt gttaattttt tttatttatt tgttgtttag     3420
   gaagtggaat ttggagtttt tttttttttt ttttttgttt aatttgtaag aggtgggttt     3480
   tttttttttg tatttttatt ttttttattt tttttttttt tttgaagttt tttgaagatt     3540
   ttttaaattt tgtgtttttt tgggtgtgtg tggttaattt ggttggtggt tgggttttgt     3600
   agtggtgtat ttggagtggg tgttgtggga tgtgtgtggg gtggtttttg tttgtgttgt     3660
   ttatatgggt ttagttggtt ggttggggtt gtttatgttg tttgggggaa ttgtaggttg     3720
   tgggggaggg tggggggtgg ttgttgaggg gttttgggtg gggtttgttt aaggttgttg     3780
   tttgtttaag gttgttagtt ttatttggag gttgttggga gagttggagt tttggatgta     3840
   tttgttgttt agatttttat tttttgggtt tggttagtgg gtgggggtta agtttggttt     3900
   gtttttttat ttttgggaag gggttagagg tttattaaag atttggtggt tgggatttta     3960
   gtagtattgt ttaagttagt gtttggaagg tttttttgtt agaagtttat gtttagggtt     4020
   ttggaataag tttttttatt tatttatttt tttaatgagt atttattaag tgtttattat     4080
   atgttagttt atattgtttt ggaaattgag gatatggaga ggaatttagt aattatattt     4140
   taaggttgtg tagagttgat aggttttttt ggggtagggg tgggagatag taagtaatta     4200
   agtgaataaa taaataaata tagagaaatt aaggagatag tttaagttat taatatgaat     4260
   ttaaaattaa taaattagtg ttttgggggt aagagatttt gggtggtatt aggatgagta     4320
   ggtaagaatt aat                                                        4333
<210> 139
   <211> 4333
   <212> DNA
   <213> Artificial Sequence
<220>
   <223> chemically treated genomic DNA (Homo sapiens)
<400> 139
   gttggttttt gtttatttat tttaatgtta tttaaagttt tttattttta gaatgttgat 60
   ttattggttt tgagtttgtg ttagtagttt aaattgtttt tttgattttt ttgtatttgt 120
   ttatttgttt gtttggttat ttgttgtttt ttatttttat tttgaaaaaa tttattagtt 180
   ttatatagtt ttaaagtgta gttgttaagt ttttttttgt atttttagtt tttagaatag 240
   tgtgagttgg tatatagtag gtgtttaata aatatttatt gaaggagtga atgaatgggg 300
   aagtttattt taaggttttg aatatggatt tttggtaagg gagtttttta aatgttggtt 360
   tgggtgatgt tgttgggatt ttagttatta gatttttaat aagtttttga tttttttttg 420
   agggtggaag ggtgaattga gtttggtttt tatttattag ttggatttag aaaatagaag 480
   tttaaatggt aaatgtattt agggttttaa tttttttagt aatttttaaa tgaggttgat 540
   ggttttggat aggtgatggt tttggatagg ttttgtttgg agttttttgg tggttgtttt 600
   ttgttttttt ttgtagtttg tagttttttt gggtggtgtg gatagttttg gttggttagt 660
   tgagtttgtg tgggtggtgt gggtaggagt tgttttatat gtgttttgtg atgtttgttt 720
   tagatgtgtt gttgtagggt ttggttgtta gttgagttgg ttgtgtgtgt ttgggggagt 780
   gtagagtttg gaaagttttt gagaagtttt agaggaggga agggggtggg aagagtgggg 840
   gtgtaagaga gaaaaatttg ttttttgtga gttgagtgag gaagaggggg gggaaggttt 900
   tgagttttgt tttttgaatg gtgggtggat agagaaggtt ggtgtgggga agattggtgg 960
   gaggtgtgtt ttgggtttgt ggtgtgattg gtgtgattgg tgatggttgg tgagtggatt 1020
   tgtaggttta tttggagttt gttgagattt gggtaggtgg ttgagttttg gtatagtgtt 1080
   gtgtttgttg ttgtggtggt agttagtagt tggtagtagt agtgggtgag tgtggtgtgt 1140
   tttttgtgtt tttttgtgtg ttttaggtgt ttggggggaa tggagtttag tttttttaaa    1200
   agtttggtgg gtttaggttt ttgtagttta tgggtttttt tttttgtatt tttttttttg    1260
   ttgtttttgt gggttttttt ggtgttattt tagtgaggta gtgttattga tgtgtaagga    1320
   ttgggggtgt gtttgtgtat gtgggatgtt tagaggagta gttgtgtggg tagtagaagt    1380
   gtagtgtggg agaggatgtg taggggtttt aatggggtgt gagggggttg gaatgtattg    1440
   ttagttatat tggtgttaag tgatatgagt gttagagatt ttgggtgggg tggggggatt    1500
   tatagaatag aatttttgag tttggaggtt tgtgtgtttt ttttattttt tagtaaatag    1560
   attagtgtgt tgagagagag ggatgtagtt tgtggttttt gttttggtta gagttggggt    1620
   tttttgatgt taattattgt agaaatagat gtatttggaa tagttttttt attgtagggt    1680
   aaaaatttat aaattattta atattttttt agaaagggtt gaggaatata ggttggagga    1740
   gatgtgggtt attaggtttt tagtggtttt tgatattttt ttaagatgtt ttgtggggtt    1800
   tttttattag taatgtttta ttggttattt ttttattgaa atggttttta agtgttgggt    1860
   gatttttgtg ggaagttttt ggggtagtag ttagtatttg atgttaggat tttatttata    1920
   ttaatttaga tgtttttgtg ttgtagtagg tttatattgt tggggagagg tggtaagaga    1980
   taaattaatt ttagtggaaa tgggaagtga gtgggaataa tagttttata gtaagattgg    2040
   ggtttagtag ttgtgttttt ggtttgtgtt tggtggattg gaatttgggt tgtgtgtttt    2100
   attttttttt ttgttagaaa atattattag gaattaaatg gtttttttta agagtgtgaa    2160
   agtgttattt ttgttagtta ttaatgagga gggtgttaaa agttttttag agttagtttt    2220
   tatagttagg attatttttt aaagttagag gttggaaagt tgaatagaat ggaagtaaat    2280
   tgtttggtag agggaagagg tattgggtgt ttagggggtg tagagtgggt gggggtgatt    2340
   ggagtgtgaa atgaattggt ggttagggtg tttttttttg tatttatatt agttgtagtt    2400
   tgtagttatt tagaggtggt ttaattgtta gtttattttg tgttagggat agtatgtggt    2460
   attgttatta gtgtgtatat ttttaggata ttaaagataa attgtttaaa agaatattag    2520
   aagaaaagat ttaatttgag taaaagtaga gataagaaaa tttttatata gtagtagaaa    2580
   gtatttttta tttttgaatg attatttaga attgtggttt tgttattttt tgttattttt    2640
   ttgataaaaa ttttattttg taattgattt attggattga attttattga ggatttgatg    2700
   gaaatatata gtgtttaatt ggaataaata gtttaaattg ttgtgtgttt ttttaaatgt    2760
   agattttttt agaatttttg tggtattaga atttttataa agattatgta ttttagatat    2820
   ttttttatat tatatatata tatatatata tatatatata tatgtattgt tttttttaaa    2880
   attatgttag atttgaaatt ttagggtatt ttgttaaaat gaataagttt gttgttttta    2940
   aaaagaggtg ggggagggga gatttagtgg taaaggtggt tttgattttt tttgttttgt    3000
   tttagtattt tggttaatat ttttaaatta gtttttgtta agatatttta tgggatttta    3060
   tatatttagg taggtagttt tggttgttat tttggtagta agaatgtatg tgttttgtat    3120
   attattttat tagttaattt ttgaagggaa ataggaaggt gatgagtttt ttgagttggt    3180
   atttgaattt ggtgggattt tgtgggtgta gagtattttt tttgaattta aaattgtgtt    3240
   attggattat gtggtagaga ttaattttag tgtatattga tgtaattttt taagattttt    3300
   tatatttgtt gttttaagtt tttaaaatat ggttgttaag ggagaaaaat gataggatgt    3360
   tggggaaaat attatggata tttgatttta gtatagattt atagaattgt gggtgtgtat    3420
   tggagtaagt tattttttat tgtaaggtat ttgaaagaga ttagaaattt ttgttgagaa    3480
   aaagtatttt attgttagat ttgtatattt ttttttttga aatttttgtt ttggggatat    3540
   aaatttttta ggatattgtt aaaagattgt tatagggttt gaattttgtt ttttttaata    3600
   agtttaggta aggttgttag gttaataaga tagttttttt ttttgagaat ggggtgtgta    3660
   tatagagaga gagagagaga gagagagaga agataggaag tttttaaaat gtgtttgaat    3720
   atggagagga gagattaagg agatgttgag tgagggtaat gattgggaaa gttaaatttt    3780
   aaggtagttg ttgaggataa tataggtgta tgttaaagta aatttgatgt tagtttttat    3840
   ttaaagtatt gtatgtagta ggtttatgta tttgtagatt tttagatatg aattatttta    3900
   ttttttattt tttttaagaa tagtaatgaa taaaaatata tattttaaaa ggagaaatat    3960
   ttgttttttt attattttaa aattttttat agaaaataaa tgaaagataa tttatttgaa    4020
   gattaattta aaggattatg ttaagttttt ataatttttt gaaattttat ttttgattgt    4080
   tttataaagt aagttttttt agggtagaat tttagtttga aaatgattag aaatatttgt    4140
   tttatattag aattggagtg ttttattata ttggttgaat atatatatta tgttggtttt    4200
   aaagttgtaa tgttattaat tagaaatttt tttttgataa gaagtttatt ttttaaaatg    4260
   aatgaagtgt atttgtattt tagtatgaat tagtttattt agatagtttt ttataattta    4320
   ttaagatata tat                                                        4333
<210> 140
   <211> 2333
   <212> DNA
   <213> Artificial Sequence
<220>
   <223> chemically treated genomic DNA (Homo sapiens)
<400> 140
   attatgtttt gagaatttga agtaatagat atgaagagtt ttaagggatt gtattaatgt      60
   atgttaagat tagtttttat tatatagttt aatgatatgg ttttaggttt agaagggatg     120
   ttttatattt atagaatttt attgagttta ggtgttagtt tagagaattt attatttttt     180
   tgtttttttt taagagttaa ttgatgggat agtatgtaaa gtatatgtgt ttttgttgtt     240
   aaaatagtag ttaaaattgt ttgtttagat gtgtaagatt ttatgaaatg ttttggtaaa     300
   agttagttta ggagtgttgg ttaaggtgtt gaagtgaagt aaggggagtt aggattattt     360
   ttgttattga attttttttt ttttattttt ttttaaagat agtgagttta tttattttga     420
   taagatattt taggatttta aatttgatat aattttggag aaagtagtgt gtgtgtgtgt     480
   gtgtgtgtgt gtgtgtgtgt gtggtgtaga agggtatttg ggatatataa tttttataga     540
   aattttagtg ttataaaaat tttaggagga tttatattta aaaaaatata taatagttta     600
   agttgtttat tttaattgaa tgttgtgtgt ttttattaga tttttagtga gatttagttt     660
   aatgagttaa ttgtaaaata aagtttttgt taagggaatg gtagaaaatg ataaaattat     720
   aattttgaat gattatttaa aagtaaaaag tattttttat tattgtgtgg agattttttt     780
   atttttattt ttatttgaat tgaatttttt tttttaatgt ttttttgaat agtttgtttt     840
   tggtgttttg aaaatgtgta tgttgatagt agtattatat gttgtttttg gtatagagtg       900
   agttaatagt tgaattattt ttgggtaatt gtggattgta gttagtgtaa gtgtaagaga     960
   aggtattttg gttattaatt tgttttatat tttaattatt tttgtttgtt ttatattttt     1020
   tgggtatttg atgttttttt tttttgttag gtaatttgtt tttgttttgt ttaatttttt    1080
   gatttttggt tttagagaat agttttggtt gtgggaattg gttttgggaa gtttttggtg    1140
   ttttttttat tggtgattgg taagagtaat atttttatat ttttaaaaag agttgtttag    1200
   tttttaatga tattttttgg taaaaaagaa aatgaaatat ataatttgga ttttggttta     1260
   ttaaatgtag gttaagagtg tggttgttga gttttggttt tgttgtgagg ttgttgtttt    1320
   tatttgtttt ttgtttttat tagagttgat ttgttttttg ttattttttt ttggtagtgt     1380
   ggatttattg taatgtagga atatttgggt tggtatggat aggattttag tattagatgt    1440
   tggttgttgt tttagggatt ttttgtggaa attatttagt atttagaagt tgttttaata    1500
   aggaagtaat tagtaaagta ttgttggtgg gagaattttg tgaaatattt tagaaaaata     1560
   ttagaaattg ttggagattt aatgatttat gtttttttta gtttgtgttt tttagttttt    1620
   tttggaggag tattaagtgg tttgtgggtt tttattttgt ggtgggaggg ttgttttaag    1680
   tatatttatt tttgtgatag ttaatgttgg ggggttttag ttttaattag ggtgaggatt    1740
   atggattgta tttttttttt ttagtgtatt ggtttgttta ttggagggtg agagggatgt    1800
   gtagattttt ggatttaggg gttttgtttt gtagattttt ttattttatt tggggttttt    1860
   ggtatttgta ttatttagta ttagtgtgat tggtggtgta ttttggtttt tttgtgtttt    1920
   attaaagttt ttgtatattt ttttttatgt tgtgtttttg ttatttatgt agttgttttt     1980
   ttgagtgttt tgtgtgtata aatgtgtttt tggtttttat gtgttggtgg tgttgttttg    2040
   ttgaggtggt gttgggggag tttgtagagg tggtggggga gggagtgtgg aggagaggat    2100
   ttgtgagttg tggggatttg ggtttgttga gtttttgggg aagttgggtt ttgttttttt    2160
   tgggtgtttg gggtgtgtgg gaaggtgtgg aggatgtgtt gtatttattt gttgttgttg    2220
   ttggttgttg attgttattg tggtggtgga tgtggtgttg tgttggagtt tggttgtttg    2280
   tttaggtttt aataggtttt gggtgaattt gtgggtttgt ttattggtta ttg           2333
<210> 141
   <211> 2333
   <212> DNA
   <213> Artificial Sequence
<220>
   <223> chemically treated genomic DNA (Homo sapiens)
<400> 141
   tgatggttgg tgagtggatt tgtaggttta tttggagttt gttgagattt gggtaggtgg      60
   ttgagttttg gtatagtgtt gtgtttgttg ttgtggtggt agttagtagt tggtagtagt     120
   agtgggtgag tgtggtgtgt tttttgtgtt tttttgtgtg ttttaggtgt ttggggggaa      180
   tggagtttag tttttttaaa agtttggtgg gtttaggttt ttgtagttta tgggtttttt     240
   tttttgtatt tttttttttg ttgtttttgt gggttttttt ggtgttattt tagtgaggta     300
   gtgttattga tgtgtaagga ttgggggtgt gtttgtgtat gtgggatgtt tagaggagta     360
   gttgtgtggg tagtagaagt gtagtgtggg agaggatgtg taggggtttt aatggggtgt     420
   gagggggttg gaatgtattg ttagttatat tggtgttaag tgatatgagt gttagagatt     480
   ttgggtgggg tggggggatt tatagaatag aatttttgag tttggaggtt tgtgtgtttt      540
   ttttattttt tagtaaatag attagtgtgt tgagagagag ggatgtagtt tgtggttttt     600
   gttttggtta gagttggggt tttttgatgt taattattgt agaaatagat gtatttggaa     660
   tagttttttt attgtagggt aaaaatttat aaattattta atattttttt agaaagggtt     720
   gaggaatata ggttggagga gatgtgggtt attaggtttt tagtggtttt tgatattttt     780
   ttaagatgtt ttgtggggtt tttttattag taatgtttta ttggttattt ttttattgaa     840
   atggttttta agtgttgggt gatttttgtg ggaagttttt ggggtagtag ttagtatttg     900
   atgttaggat tttatttata ttaatttaga tgtttttgtg ttgtagtagg tttatattgt     960
   tggggagagg tggtaagaga taaattaatt ttagtggaaa tgggaagtga gtgggaataa    1020
   tagttttata gtaagattgg ggtttagtag ttgtgttttt ggtttgtgtt tggtggattg    1080
   gaatttgggt tgtgtgtttt attttttttt ttgttagaaa atattattag gaattaaatg    1140
   gtttttttta agagtgtgaa agtgttattt ttgttagtta ttaatgagga gggtgttaaa     1200
   agttttttag agttagtttt tatagttagg attatttttt aaagttagag gttggaaagt    1260
   tgaatagaat ggaagtaaat tgtttggtag agggaagagg tattgggtgt ttagggggtg    1320
   tagagtgggt gggggtgatt ggagtgtgaa atgaattggt ggttagggtg tttttttttg     1380
   tatttatatt agttgtagtt tgtagttatt tagaggtggt ttaattgtta gtttattttg    1440
   tgttagggat agtatgtggt attgttatta gtgtgtatat ttttaggata ttaaagataa    1500
   attgtttaaa agaatattag aagaaaagat ttaatttgag taaaagtaga gataagaaaa    1560
   tttttatata gtagtagaaa gtatttttta tttttgaatg attatttaga attgtggttt      1620
   tgttattttt tgttattttt ttgataaaaa ttttattttg taattgattt attggattga     1680
   attttattga ggatttgatg gaaatatata gtgtttaatt ggaataaata gtttaaattg    1740
   ttgtgtgttt ttttaaatgt agattttttt agaatttttg tggtattaga atttttataa     1800
   agattatgta ttttagatat ttttttatat tatatatata tatatatata tatatatata    1860
   tatgtattgt tttttttaaa attatgttag atttgaaatt ttagggtatt ttgttaaaat    1920
   gaataagttt gttgttttta aaaagaggtg ggggagggga gatttagtgg taaaggtggt   1980
   tttgattttt tttgttttgt tttagtattt tggttaatat ttttaaatta gtttttgtta    2040
   agatatttta tgggatttta tatatttagg taggtagttt tggttgttat tttggtagta    2100
   agaatgtatg tgttttgtat attattttat tagttaattt ttgaagggaa ataggaaggt    2160
   gatgagtttt ttgagttggt atttgaattt ggtgggattt tgtgggtgta gagtattttt    2220
   tttgaattta aaattgtgtt attggattat gtggtagaga ttaattttag tgtatattga    2280
   tgtaattttt taagattttt tatatttgtt gttttaagtt tttaaaatat ggt           2333
<210> 142
   <211> 26
   <212> DNA
   <213> Artificial Sequence
<220>
   <223> chemically treated genomic DNA (Homo sapiens)
<400> 142
   ggtttttacg tacgttgttt ttttcg 26
<210> 143
   <211> 25
   <212> DNA
   <213> Artificial Sequence
<220>
   <223> chemically treated genomic DNA (Homo sapiens)
<400> 143
   tcactaccga aaacgaaaat tacga 25
<210> 144
   <211> 19
   <212> DNA
   <213> Artificial Sequence
<220>
   <223> chemically treated genomic DNA (Homo sapiens)
<400> 144
   atcgccaacg ccgcgcaaa 19
<210> 145
   <211> 20
   <212> DNA
   <213> Artificial Sequence
<220>
   <223> chemically treated genomic DNA (Homo sapiens)
<400> 145
   tcctctaaac gtcccgcgta 20
<210> 146
   <211> 20
   <212> DNA
   <213> Artificial Sequence
<220>
   <223> chemically treated genomic DNA (Homo sapiens)
<400> 146
   caaacgcgcc cccgatcctt 20
<210> 147
   <211> 20
   <212> DNA
   <213> Artificial Sequence
<220>
   <223> chemically treated genomic DNA (Homo sapiens)
<400> 147
   gcgaggtagc gttatcgacg 20
<210> 148
   <211> 21
   <212> DNA
   <213> Artificial Sequence
<220>
   <223> chemically treated genomic DNA (Homo sapiens)
<400> 148
   ttttttttcg tcgtttttgc g 21